(19) Europäisches Patentamt
European Patent Office
Office européen des brevets

(11) **EP 1 900 818 A2**

(12) **EUROPEAN PATENT APPLICATION**

(43) Date of publication:
**19.03.2008 Bulletin 2008/12**

(21) Application number: **07075379.3**

(22) Date of filing: **09.10.1998**

(51) Int Cl.:
***C12N 15/31*** *(2006.01)* ***C07K 14/22*** *(2006.01)*
***C07K 16/12*** *(2006.01)* ***G01N 33/53*** *(2006.01)*
***A61K 39/095*** *(2006.01)* *C12R 1/36* *(2006.01)*

(84) Designated Contracting States:
**AT BE CH CY DE DK ES FI FR GB GR IE IT LI LU MC NL PT SE**

(30) Priority:
**06.11.1997 GB 9723516**
**14.11.1997 GB 9724190**
**18.11.1997 GB 9724386**
**27.11.1997 GB 9725158**
**10.12.1997 GB 9726147**
**14.01.1998 GB 9800759**
**01.09.1998 GB 9819016**

(62) Document number(s) of the earlier application(s) in accordance with Art. 76 EPC:
**98946675.0 / 1 029 052**

(71) Applicant: **Novartis Vaccines and Diagnostics S.r.l.**
**53100 Siena (SI) (IT)**

(72) Inventors:
- **Grandi, Guido**
  **53100 Siena (IT)**
- **Masignani, Vega**
  **53100 Siena (IT)**
- **Pizza, Mariagrazia**
  **53100 Siena (IT)**
- **Rappuoli, Rino**
  **53100 Siena (IT)**
- **Scarlato, Vincenzo**
  **53100 Siena (IT)**

(74) Representative: **Marshall, Cameron John et al**
**Carpmaels & Ransford**
**43-45 Bloomsbury Square**
**London WC1A 2RA (GB)**

Remarks:
This application was filed on 21 - 05 - 2007 as a divisional application to the application mentioned under INID code 62.

# (54) Neisserial antigens

(57) The invention provides proteins from *Neisseria meningitidis* (strains A & B) and from *Neisseria gonorrhoeae,* including amino acid sequences, the corresponding nucleotide sequences, expression data, and serological data. The proteins are useful antigens for vaccines, immunogenic compositions, and/or diagnostics.

EP 1 900 818 A2

Printed by Jouve, 75001 PARIS (FR)

## Description

This invention relates to antigens from *Neisseria* bacteria.

## BACKGROUND ART

*Neisseria meningitidis* and *Neisseria gonorrhoeae* are non-motile, gram negative diplococci that are pathogenic in humans. *N.meningitidis* colonises the pharynx and causes meningitis (and, occasionally, septicaemia in the absence of meningitis); *N.gonorrhoeae* colonises the genital tract and causes gonorrhea. Although colonising different areas of the body and causing completely different diseases, the two pathogens are closely related, although one feature that clearly differentiates meningococcus from gonococcus is the presence of a polysaccharide capsule that is present in all pathogenic meningococci.

*N.gonorrhoeae* caused approximately 800,000 cases per year during the period 1983-1990 in the United States alone (chapter by Meitzner & Cohen, "Vaccines Against Gonococcal Infection", In: New Generation Vaccines, 2nd edition, ed. Levine, Woodrow, Kaper, & Cobon, Marcel Dekker, New York, 1997, pp.817-842). The disease causes significant morbidity but limited mortality. Vaccination against *N.gonorrhoeae* would be highly desirable, but repeated attempts have failed. The main candidate antigens for this vaccine are surface-exposed proteins such as pili, porins, opacity-associated proteins (Opas) and other surface-exposed proteins such as the Lip, Laz, IgA1 protease and transferrin-binding proteins. The lipooligosaccharide (LOS) has also been suggested as vaccine (Meitzner & Cohen, *supra*).

*N.meningitidis* causes both endemic and epidemic disease. In the United States the attack rate is 0.6-1 per 100,000 persons per year, and it can be much greater during outbreaks (see Lieberman et al. (1996) Safety and Immunogenicity of a Serogroups A/C Neisseria meningitidis Oligosaccharide-Protein Conjugate Vaccine in Young Children. JAMA 275(19):1499-1503; Schuchat et al (1997) Bacterial Meningitis in the United States in 1995. N Engl J Med 337 (14):970-976). In developing countries, endemic disease rates are much higher and during epidemics incidence rates can reach 500 cases per 100,000 persons per year. Mortality is extremely high, at 10-20% in the United States, and much higher in developing countries. Following the introduction of the conjugate vaccine against *Haemophilus influenzae, N. meningitidis* is the major cause of bacterial meningitis at all ages in the United States (Schuchat *et al* (1997) *supra).*

Based on the organism's capsular polysaccharide, 12 serogroups of *N.meningitidis* have been identified. Group A is the pathogen most often implicated in epidemic disease in sub-Saharan Africa. Serogroups B and C are responsible for the vast majority of cases in the United States and in most developed countries. Serogroups W135 and Y are responsible for the rest of the cases in the United States and developed countries. The meningococcal vaccine currently in use is a tetravalent polysaccharide vaccine composed of serogroups A, C, Y and W135. Although efficacious in adolescents and adults, it induces a poor immune response and short duration of protection, and cannot be used in infants [eg. Morbidity and Mortality weekly report, Vol.46, No. RR-5 (1997)]. This is because polysaccharides are T-cell independent antigens that induce a weak immune response that cannot be boosted by repeated immunization. Following the success of the vaccination against *H.influenzae,* conjugate vaccines against serogroups A and C have been developed and are at the final stage of clinical testing (Zollinger WD "New and Improved Vaccines Against Meningococcal Disease" in: New Generation Vaccines, supra, pp. 469-488; Lieberman *et al* (1996) *supra;* Costantino et al (1992) Development and phase I clinical testing of a conjugate vaccine against meningococcus A and C. Vaccine 10:691-698).

Meningococcus B remains a problem, however. This serotype currently is responsible for approximately 50% of total meningitis in the United States, Europe, and South America. The polysaccharide approach cannot be used because the menB capsular polysaccharide is a polymer of $\alpha$(2-8)-linked *N*-acetyl neuraminic acid that is also present in mammalian tissue. This results in tolerance to the antigen; indeed, if an immune response were elicited, it would be anti-self, and therefore undesirable. In order to avoid induction of autoimmunity and to induce a protective immune response, the capsular polysaccharide has, for instance, been chemically modified substituting the *N*-acetyl groups with N-propionyl groups, leaving the specific antigenicity unaltered (Romero & Outschoorn (1994) Current status of Meningococcal group B vaccine candidates: capsular or non-capsular? Clin Microbiol Rev 7(4):559-575).

Alternative approaches to menB vaccines have used complex mixtures of outer membrane proteins (OMPs), containing either the OMPs alone, or OMPs enriched in porins, or deleted of the class 4 OMPs that are believed to induce antibodies that block bactericidal activity. This approach produces vaccines that are not well characterized. They are able to protect against the homologous strain, but are not effective at large where there are many antigenic variants of the outer membrane proteins. To overcome the antigenic variability, multivalent vaccines containing up to nine different porins have been constructed (*eg.*

Poolman JT (1992) Development of a meningococcal vaccine. Infect. Agents Dis. 4:13-28). Additional proteins to be used in outer membrane vaccines have been the opa and opc proteins, but none of these approaches have been able to overcome the antigenic variability (*eg.* Ala'Aldeen & Borriello (1996) The meningococcal transferrin-binding proteins 1 and 2 are both surface exposed and generate bactericidal antibodies capable of killing homologous and heterologous strains. Vaccine 14(1):49-53).

A certain amount of sequence data is available for meningococcal and gonoccocal genes and proteins (*eg*. EP-A-0467714, WO96/29412), but this is by no means complete. The provision of further sequences could provide an opportunity to identify secreted or surface-exposed proteins that are presumed targets for the immune system and which are not antigenically variable. For instance, some of the identified proteins could be components of efficacious vaccines against meningococcus B, some could be components of vaccines against all meningococcal serotypes, and others could be components of vaccines against all pathogenic *Neisseriae.*

**THE INVENTION**

The invention provides proteins comprising the Neisserial amino acid sequences disclosed in the examples. These sequences relate to *N.meningitidis* or *N.gonorrhoeae.*

It also provides proteins comprising sequences homologous (*ie*. having sequence identity) to the Neisserial amino acid sequences disclosed in the examples. Depending on the particular sequence, the degree of identity is preferably greater than 50% *(eg.* 65%, 80%, 90%, or more). These homologous proteins include mutants and allelic variants of the sequences disclosed in the examples. Typically, 50% identity or more between two proteins is considered to be an indication of functional equivalence. Identity between the proteins is preferably determined by the Smith-Waterman homology search algorithm as implemented in the MPSRCH program (Oxford Molecular), using an affine gap search with parameters *gap open penalty=12* and *gap extension penalty=l.*

The invention further provides proteins comprising fragments of the Neisserial amino acid sequences disclosed in the examples. The fragments should comprise at least n consecutive amino acids from the sequences and, depending on the particular sequence, *n* is 7 or more *(eg.* 8, 10, 12, 14, 16, 18, 20 or more). Preferably the fragments comprise an epitope from the sequence.

The proteins of the invention can, of course, be prepared by various means (*eg*. recombinant expression, purification from cell culture, chemical synthesis *etc.)* and in various forms *(eg.* native, fusions *etc.*). They are preferably prepared in substantially pure or isolated form (*ie.* substantially free from other Neisserial or host cell proteins)

According to a further aspect, the invention provides antibodies which bind to these proteins. These may be polyclonal or monoclonal and may be produced by any suitable means.

According to a further aspect, the invention provides nucleic acid comprising the Neisserial nucleotide sequences disclosed in the examples. In addition, the invention provides nucleic acid comprising sequences homologous (*ie*. having sequence identity) to the Neisserial nucleotide sequences disclosed in the examples.

Furthermore, the invention provides nucleic acid which can hybridise to the Neisserial nucleic acid disclosed in the examples, preferably under "high stringency" conditions (*eg*. 65°C in a 0.1XSSC, 0.5% SDS solution).

Nucleic acid comprising fragments of these sequences are also provided. These should comprise at least *n* consecutive nucleotides from the Neisserial sequences and, depending on the particular sequence, *n* is 10 or more (*eg* 12, 14, 15, 18, 20, 25, 30, 35, 40 or more).

According to a further aspect, the invention provides nucleic acid encoding the proteins and protein fragments of the invention.

It should also be appreciated that the invention provides nucleic acid comprising sequences complementary to those described above (*eg*. for antisense or probing purposes).

Nucleic acid according to the invention can, of course, be prepared in many ways (*eg.* by chemical synthesis, from genomic or cDNA libraries, from the organism itself *etc.*) and can take various forms (*eg.* single stranded, double stranded, vectors, probes *etc.*).

In addition, the term "nucleic acid" includes DNA and RNA, and also their analogues, such as those containing modified backbones, and also peptide nucleic acids (PNA) *etc.*

According to a further aspect, the invention provides vectors comprising nucleotide sequences of the invention (*eg*. expression vectors) and host cells transformed with such vectors.

According to a further aspect, the invention provides compositions comprising protein, antibody, and/or nucleic acid according to the invention. These compositions may be suitable as vaccines, for instance, or as diagnostic reagents, or as immunogenic compositions.

The invention also provides nucleic acid, protein, or antibody according to the invention for use as medicaments (*eg*. as vaccines) or as diagnostic reagents. It also provides the use of nucleic acid, protein, or antibody according to the invention in the manufacture of: (i) a medicament for treating or preventing infection due to Neisserial bacteria; (ii) a diagnostic reagent for detecting the presence of Neisserial bacteria or of antibodies raised against Neisserial bacteria; and/or (iii) a reagent which can raise antibodies against Neisserial bacteria. Said Neisserial bacteria may be any species or strain (such as *N.gonorrhoeae,* or any strain of *N.meningitidis,* such as strain A, strain B or strain C).

The invention also provides a method of treating a patient, comprising administering to the patient a therapeutically effective amount of nucleic acid, protein, and/or antibody according to the invention.

According to further aspects, the invention provides various processes.

A process for producing proteins of the invention is provided, comprising the step of culturing a host cell according to the invention under conditions which induce protein expression.

A process for producing protein or nucleic acid of the invention is provided, wherein the the protein or nucleic acid is synthesised in part or in whole using chemical means.

A process for detecting polynucleotides of the invention is provided, comprising the steps of: (a) contacting a nucleic probe according to the invention with a biological sample under hybridizing conditions to form duplexes; and (b) detecting said duplexes.

A process for detecting proteins of the invention is provided, comprising the steps of: (a) contacting an antibody according to the invention with a biological sample under conditions suitable for the formation of an antibody-antigen complexes; and (b) detecting said complexes.

A summary of standard techniques and procedures which may be employed in order to perform the invention (*eg*. to utilise the disclosed sequences for vaccination or diagnostic purposes) follows. This summary is not a limitation on the invention but, rather, gives examples that may be used, but are not required.

*General*

The practice of the present invention will employ, unless otherwise indicated, conventional techniques of molecular biology, microbiology, recombinant DNA, and immunology, which are within the skill of the art. Such techniques are explained fully in the literature eg. Sambrook Molecular Cloning; A Laboratory Manual, Second Edition (1989); DNA Cloning, Volumes I and ii (D.N Glover ed. 1985); Oligonucleotide Synthesis (M.J. Gait ed, 1984); Nucleic Acid Hybridization (B.D. Hames & S.J. Higgins eds. 1984); Transcription and Translation (B.D. Hames & S.J. Higgins eds. 1984); Animal Cell Culture (R.I. Freshney ed. 1986); Immobilized Cells and Enzymes (IRL Press, 1986); B. Perbal, A Practical Guide to Molecular Cloning (1984); the Methods in Enzymology series (Academic Press, Inc.), especially volumes 154 & 155; Gene Transfer Vectors for Mammalian Cells (J.H. Miller and M.P. Calos eds. 1987, Cold Spring Harbor Laboratory); Mayer and Walker, eds. (1987), Immunochemical Methods in Cell and Molecular Biology (Academic Press, London); Scopes, (1987) Protein Purification: Principles and Practice, Second Edition (Springer-Verlag, N.Y.), and Handbook of Experimental Immunology, Volumes I-IV (D.M. Weir and C. C. Blackwell eds 1986).

Standard abbreviations for nucleotides and amino acids are used in this specification.

All publications, patents, and patent applications cited herein are incorporated in full by reference.

In particular, the contents of UK patent applications 9723516.2, 9724190.5, 9724386.9, 9725158.1, 9726147.3, 9800759.4, and 9819016.8 are incorporated herein.

*Definitions*

A composition containing X is "substantially free of" Y when at least 85% by weight of the total X+Y in the composition is X. Preferably, X comprises at least about 90% by weight of the total of X+Y in the composition, more preferably at least about 95% or even 99% by weight.

The term "comprising" means "including" as well as "consisting" *eg.* a composition "comprising" X may consist exclusively of X or may include something additional to X, such as X+Y.

The term "heterologous" refers to two biological components that are not found together in nature. The components may be host cells, genes, or regulatory regions, such as promoters. Although the heterologous components are not found together in nature, they can function together, as when a promoter heterologous to a gene is operably linked to the gene. Another example is where a Neisserial sequence is heterologous to a mouse host cell. A further examples would be two epitopes from the same or different proteins which have been assembled in a single protein in an arrangement not found in nature.

An "origin of replication" is a polynucleotide sequence that initiates and regulates replication of polynucleotides, such as an expression vector. The origin of replication behaves as an autonomous unit of polynucleotide replication within a cell, capable of replication under its own control. An origin of replication may be needed for a vector to replicate in a particular host cell. With certain origins of replication, an expression vector can be reproduced at a high copy number in the presence of the appropriate proteins within the cell. Examples of origins are the autonomously replicating sequences, which are effective in yeast; and the viral T-antigen, effective in COS-7 cells.

A "mutant" sequence is defined as DNA, RNA or amino acid sequence differing from but having sequence identity with the native or disclosed sequence. Depending on the particular sequence, the degree of sequence identity between the native or disclosed sequence and the mutant sequence is preferably greater than 50% *(eg.* 60%, 70%, 80%, 90%, 95%, 99% or more, calculated using the Smith-Waterman algorithm as described above). As used herein, an "allelic variant" of a nucleic acid molecule, or region, for which nucleic acid sequence is provided herein is a nucleic acid molecule, or region, that occurs essentially at the same locus in the genome of another or second isolate, and that, due to natural variation caused by, for example, mutation or recombination, has a similar but not identical nucleic acid sequence. A coding region allelic variant typically encodes a protein having similar activity to that of the protein encoded by the gene to which it is being compared. An allelic variant can also comprise an alteration in the 5' or 3' untranslated regions of the gene, such as in regulatory control regions (*eg.* see US patent 5,753,235).

*Expression systems*

The Neisserial nucleotide sequences can be expressed in a variety of different expression systems; for example those used with mammalian cells, baculoviruses, plants, bacteria, and yeast.

i. Mammalian Systems

Mammalian expression systems are known in the art. A mammalian promoter is any DNA sequence capable of binding mammalian RNA polymerase and initiating the downstream (3') transcription of a coding sequence (*eg.* structural gene) into mRNA. A promoter will have a transcription initiating region, which is usually placed proximal to the 5' end of the coding sequence, and a TATA box, usually located 25-30 base pairs (bp) upstream of the transcription initiation site. The TATA box is thought to direct RNA polymerase II to begin RNA synthesis at the correct site. A mammalian promoter will also contain an upstream promoter element, usually located within 100 to 200 bp upstream of the TATA box. An upstream promoter element determines the rate at which transcription is initiated and can act in either orientation [Sambrook et al. (1989) "Expression of Cloned Genes in Mammalian Cells." In Molecular Cloning: A Laboratory Manual, 2nd ed.*].*

Mammalian viral genes are often highly expressed and have a broad host range; therefore sequences encoding mammalian viral genes provide particularly useful promoter sequences. Examples include the SV40 early promoter, mouse mammary tumor virus LTR promoter, adenovirus major late promoter (Ad MLP), and herpes simplex virus promoter. In addition, sequences derived from non-viral genes, such as the murine metallotheionein gene, also provide useful promoter sequences. Expression may be either constitutive or regulated (inducible), depending on the promoter can be induced with glucocorticoid in hormone-responsive cells.

The presence of an enhancer element (enhancer), combined with the promoter elements described above, will usually increase expression levels. An enhancer is a regulatory DNA sequence that can stimulate transcription up to 1000-fold when linked to homologous or heterologous promoters, with synthesis beginning at the normal RNA start site. Enhancers are also active when they are placed upstream or downstream from the transcription initiation site, in either normal or flipped orientation, or at a distance of more than 1000 nucleotides from the promoter [Maniatis et al. (1987) Science 236:1237; Alberts et al. (1989) Molecular Biology of the Cell, 2nd ed.]. Enhancer elements derived from viruses may be particularly useful, because they usually have a broader host range. Examples include the SV40 early gene enhancer [Dijkema et al (1985) EMBO J. 4:761] and the enhancer/promoters derived from the long terminal repeat (LTR) of the Rous Sarcoma Virus [Gorman et al. (1982b) Proc. Natl. Acad Sci. 79:6777] and from human cytomegalovirus [Boshart et al. (1985) Cell 41:521]. Additionally, some enhancers are regulatable and become active only in the presence of an inducer, such as a hormone or metal ion [Sassone-Corsi and Borelli (1986) Trends Genet. 2:215; Maniatis et al. (1987) Science 236:1237].

A DNA molecule may be expressed intracellularly in mammalian cells. A promoter sequence may be directly linked with the DNA molecule, in which case the first amino acid at the N-terminus of the recombinant protein will always be a methionine, which is encoded by the ATG start codon. If desired, the N-terminus may be cleaved from the protein by *in vitro* incubation with cyanogen bromide.

Alternatively, foreign proteins can also be secreted from the cell into the growth media by creating chimeric DNA molecules that encode a fusion protein comprised of a leader sequence fragment that provides for secretion of the foreign protein in mammalian cells. Preferably, there are processing sites encoded between the leader fragment and the foreign gene that can be cleaved either *in vivo* or *in vitro.* The leader sequence fragment usually encodes a signal peptide comprised of hydrophobic amino acids which direct the secretion of the protein from the cell. The adenovirus triparite leader is an example of a leader sequence that provides for secretion of a foreign protein in mammalian cells.

Usually, transcription termination and polyadenylation sequences recognized by mammalian cells are regulatory regions located 3' to the translation stop codon and thus, together with the promoter elements, flank the coding sequence. The 3' terminus of the mature mRNA is formed by site-specific post-transcriptional cleavage and polyadenylation [Birnstiel et al. (1985) Cell 41:349; Proudfoot and Whitelaw (1988) "Termination and 3' end processing of eukaryotic RNA. In Transcription and splicing (ed. B.D. Hames and D.M. Glover); Proudfoot (1989) Trends Biochem. Sci. 14:105]. These sequences direct the transcription of an mRNA which can be translated into the polypeptide encoded by the DNA. Examples of transcription terminater/polyadenylation signals include those derived from SV40 [Sambrook et al (1989) "Expression of cloned genes in cultured mammalian cells." In Molecular Cloning: A Laboratory Manual].

Usually, the above described components, comprising a promoter, polyadenylation signal, and transcription termination sequence are put together into expression constructs. Enhancers, introns with functional splice donor and acceptor sites, and leader sequences may also be included in an expression construct, if desired. Expression constructs are often maintained in a replicon, such as an extrachromosomal element (*eg.* plasmids) capable of stable maintenance in a host, such as mammalian cells or bacteria. Mammalian replication systems include those derived from animal viruses, which require trans-acting factors to replicate. For example, plasmids containing the replication systems of papovaviruses, such as SV40 [Gluzman (1981) Cell 23:175] or polyomavirus, replicate to extremely high copy number in the presence of the appropriate viral T antigen. Additional examples of mammalian replicons include those derived from bovine papillomavirus and Epstein-Barr virus. Additionally, the replicon may have two replicaton systems, thus allowing it to be maintained, for example, in mammalian cells for expression and in a prokaryotic host for cloning and amplification. Examples of such mammalian-bacteria shuttle vectors include pMT2 [Kaufman et al. (1989) Mol. Cell. Biol. 9:946] and pHEBO [Shimizu et al. (1986) Mol. Cell. Biol. 6:1074].

The transformation procedure used depends upon the host to be transformed. Methods for introduction of heterologous polynucleotides into mammalian cells are known in the art and include dextran-mediated transfection, calcium phosphate precipitation, polybrene mediated transfection, protoplast fusion, electroporation, encapsulation of the polynucleotide(s) in liposomes, and direct microinjection of the DNA into nuclei.

Mammalian cell lines available as hosts for expression are known in the art and include many immortalized cell lines available from the American Type Culture Collection (ATCC), including but not limited to, Chinese hamster ovary (CHO) cells, HeLa cells, baby hamster kidney (BHK) cells, monkey kidney cells (COS), human hepatocellular carcinoma cells *(eg.* Hep G2), and a number of other cell lines.

ii. Baculovirus Systems

The polynucleotide encoding the protein can also be inserted into a suitable insect expression vector, and is operably linked to the control elements within that vector. Vector construction employs techniques which are known in the art. Generally, the components of the expression system include a transfer vector, usually a bacterial plasmid, which contains both a fragment of the baculovirus genome, and a convenient restriction site for insertion of the heterologous gene or genes to be expressed; a wild type baculovirus with a sequence homologous to the baculovirus-specific fragment in the transfer vector (this allows for the homologous recombination of the heterologous gene in to the baculovirus genome); and appropriate insect host cells and growth media.

After inserting the DNA sequence encoding the protein into the transfer vector, the vector and the wild type viral genome are transfected into an insect host cell where the vector and viral genome are allowed to recombine. The packaged recombinant virus is expressed and recombinant plaques are identified and purified. Materials and methods for baculovirus/insect cell expression systems are commercially available in kit form from, *inter alia,* Invitrogen, San Diego CA ("MaxBac" kit).

These techniques are generally known to those skilled in the art and fully described in Summers and Smith, Texas Agricultural Experiment Station Bulletin No. 1555 (1987) (hereinafter "Summers and Smith").

Prior to inserting the DNA sequence encoding the protein into the baculovirus genome, the above described components, comprising a promoter, leader (if desired), coding sequence of interest, and transcription termination sequence, are usually assembled into an intermediate transplacement construct (transfer vector). This construct may contain a single gene and operably linked regulatory elements; multiple genes, each with its owned set of operably linked regulatory elements; or multiple genes, regulated by the same set of regulatory elements. Intermediate transplacement constructs are often maintained in a replicon, such as an extrachromosomal element (*eg.* plasmids) capable of stable maintenance in a host, such as a bacterium. The replicon will have a replication system, thus allowing it to be maintained in a suitable host for cloning and amplification.

Currently, the most commonly used transfer vector for introducing foreign genes into AcNPV is pAc373. Many other vectors, known to those of skill in the art, have also been designed. These include, for example, pVL985 (which alters the polyhedrin start codon from ATG to ATT, and which introduces a BamHI cloning site 32 basepairs downstream from the ATT; see Luckow and Summers, virology (1989) 17:31.

The plasmid usually also contains the polyhedrin polyadenylation signal (Miller et al. (1988) Ann. Rev. Microbiol., 42:177) and a prokaryotic ampicillin-resistance *(amp)* gene and origin of replication for selection and propagation in *E. coli.*

Baculovirus transfer vectors usually contain a baculovirus promoter. A baculovirus promoter is any DNA sequence capable of binding a baculovirus RNA polymerase and initiating the downstream (5' to 3') transcription of a coding sequence (*eg.* structural gene) into mRNA. A promoter will have a transcription initiation region which is usually placed proximal to the 5' end of the coding sequence. This transcription initiation region usually includes an RNA polymerase binding site and a transcription initiation site. A baculovirus transfer vector may also have a second domain called an enhancer, which, if present, is usually distal to the structural gene. Expression may be either regulated or constitutive.

Structural genes, abundantly transcribed at late times in a viral infection cycle, provide particularly useful promoter sequences. Examples include sequences derived from the gene encoding the viral polyhedron protein, Friesen et al., (1986) "The Regulation of Baculovirus Gene Expression," in: The Molecular Biology of Baculoviruses (ed. Walter Doerfler); EPO Publ. Nos. 127 839 and 155 476; and the gene encoding the p10 protein, Vlak et al., (1988), J Gen. Virol. 69:765.

DNA encoding suitable signal sequences can be derived from genes for secreted insect or baculovirus proteins, such as the baculovirus polyhedrin gene (Carbonell et al. (1988) Gene, 73:409). Alternatively, since the signals for mammalian cell posttranslational modifications (such as signal peptide cleavage, proteolytic cleavage, and phosphorylation) appear to be recognized by insect cells, and the signals required for secretion and nuclear accumulation also appear to be conserved between the invertebrate cells and vertebrate cells, leaders of non-insect origin, such as those derived from genes encoding human $\alpha$-interferon, Maeda et al., (1985), Nature 315:592; human gastrin-releasing peptide, Lebacq-Verheyden et al., (1988), Molec. Cell. Biol. 8:3129; human IL-2, Smith et al., (1985) Proc. Nat'l Acad. Sci. USA, 82:8404; mouse IL-3, (Miyajima et al., (1987) Gene 58:273; and human glucocerebrosidase, Martin et al. (1988) DNA, 7:99, can also be used to provide for secretion in insects.

A recombinant polypeptide or polyprotein may be expressed intracellularly or, if it is expressed with the proper regulatory sequences, it can be secreted. Good intracellular expression of nonfused foreign proteins usually requires heterologous genes that ideally have a short leader sequence containing suitable translation initiation signals preceding an ATG start signal. If desired, methionine at the N-terminus may be cleaved from the mature protein by *in vitro* incubation with cyanogen bromide.

Alternatively, recombinant polyproteins or proteins which are not naturally secreted can be secreted from the insect cell by creating chimeric DNA molecules that encode a fusion protein comprised of a leader sequence fragment that provides for secretion of the foreign protein in insects. The leader sequence fragment usually encodes a signal peptide comprised of hydrophobic amino acids which direct the translocation of the protein into the endoplasmic reticulum.

After insertion of the DNA sequence and/or the gene encoding the expression product precursor of the protein, an insect cell host is co-transformed with the heterologous DNA of the transfer vector and the genomic DNA of wild type baculovirus -- usually by co-transfection. The promoter and transcription termination sequence of the construct will usually comprise a 2-5kb section of the baculovirus genome. Methods for introducing heterologous DNA into the desired site in the baculovirus virus are known in the art. (See Summers and Smith *supra;* Ju et al. (1987); Smith et al., Mol. Cell. Biol. (1983) 3:2156; and Luckow and Summers (1989)). For example, the insertion can be into a gene such as the polyhedrin gene, by homologous double crossover recombination; insertion can also be into a restriction enzyme site engineered into the desired baculovirus gene. Miller et al., (1989), Bioessays 4:91.The DNA sequence, when cloned in place of the polyhedrin gene in the expression vector, is flanked both 5' and 3' by polyhedrin-specific sequences and is positioned downstream of the polyhedrin promoter.

The newly formed baculovirus expression vector is subsequently packaged into an infectious recombinant baculovirus. Homologous recombination occurs at low frequency (between about 1% and about 5%); thus, the majority of the virus produced after cotransfection is still wild-type virus. Therefore, a method is necessary to identify recombinant viruses. An advantage of the expression system is a visual screen allowing recombinant viruses to be distinguished. The polyhedrin protein, which is produced by the native virus, is produced at very high levels in the nuclei of infected cells at late times after viral infection. Accumulated polyhedrin protein forms occlusion bodies that also contain embedded particles. These occlusion bodies, up to 15 $\mu$m in size, are highly refractile, giving them a bright shiny appearance that is readily visualized under the light microscope. Cells infected with recombinant viruses lack occlusion bodies. To distinguish recombinant virus from wild-type virus, the transfection supernatant is plaqued onto a monolayer of insect cells by techniques known to those skilled in the art. Namely, the plaques are screened under the light microscope for the presence (indicative of wild-type virus) or absence (indicative of recombinant virus) of occlusion bodies. "Current Protocols in Microbiology" Vol. 2 (Ausubel et al. eds) at 16.8 (Supp. 10, 1990); Summers and Smith, *supra;* Miller et al. (1989).

Recombinant baculovirus expression vectors have been developed for infection into several insect cells. For example, recombinant baculoviruses have been developed for, *inter alia: Aedes aegypti , Autographa californica, Bombyx mori, Drosophila melanogaster, Spodoptera frugiperda,* and *Trichoplusia ni* (WO 89/046699; Carbonell et al., (1985) J Virol. 56:153; Wright (1986) Nature 321:718; Smith et al., (1983) Mol. Cell. Biol. 3:2156; and see generally, Fraser, et al. (1989) In Vitro Cell. Dev. Biol. 25:225).

Cells and cell culture media are commercially available for both direct and fusion expression of heterologous polypeptides in a baculovirus/expression system; cell culture technology is generally known to those skilled in the art. *See, eg.* Summers and Smith *supra.*

The modified insect cells may then be grown in an appropriate nutrient medium, which allows for stable maintenance of the plasmid(s) present in the modified insect host. Where the expression product gene is under inducible control, the host may be grown to high density, and expression induced. Alternatively, where expression is constitutive, the product will be continuously expressed into the medium and the nutrient medium must be continuously circulated, while removing the product of interest and augmenting depleted nutrients. The product may be purified by such techniques as chromatography, eg. HPLC, affinity chromatography, ion exchange chromatography, etc.; electrophoresis; density gradient centrifugation; solvent extraction, or the like. As appropriate, the product may be further purified, as required, so as to remove substantially any insect proteins which are also secreted in the medium or result from lysis of insect cells, so as to provide a product which is at least substantially free of host debris, eg. proteins, lipids and polysaccharides.

In order to obtain protein expression, recombinant host cells derived from the transformants are incubated under conditions which allow expression of the recombinant protein encoding sequence. These conditions will vary, dependent upon the host cell selected. However, the conditions are readily ascertainable to those of ordinary skill in the art, based upon what is known in the art.

iii. Plant Systems

There are many plant cell culture and whole plant genetic expression systems known in the art. Exemplary plant cellular genetic expression systems include those described in patents, such as: US 5,693,506; US 5,659,122; and US 5,608,143. Additional examples of genetic expression in plant cell culture has been described by Zenk, Phytochemistry 30:3861-3863 (1991). Descriptions of plant protein signal peptides may be found in addition to the references described above in Vaulcombe et al., Mol. Gen. Genet. 209:33-40 (1987); Chandler et al., Plant Molecular Biology 3: 407-418 (1984); Rogers, J Biol. Chem. 260:3731-3738 (1985); Rothstein et al., Gene 55:353-356 (1987); Whittier et al., Nucleic Acids Research 15:2515-2535 (1987); Wirsel et al., Molecular Microbiology 3:3-14 (1989); Yu et al., Gene 122: 247-253 (1992). A description of the regulation of plant gene expression by the phytohormone, gibberellic acid and secreted enzymes induced by gibberellic acid can be found in R.L. Jones and J. MacMillin, Gibberellins: in: Advanced Plant Physiology,. Malcolm B. Wilkins, ed., 1984 Pitman Publishing Limited, London, pp. 21-52. References that describe other metabolically-regulated genes: Sheen, Plant Cell, 2:1027-1038(1990); Maas et al., EMBOJ. 9:3447-3452 (1990) ; Benkel and Hickey, Proc. Natl. Acad Sci. 84:1337-1339 (1987)

Typically, using techniques known in the art, a desired polynucleotide sequence is inserted into an expression cassette comprising genetic regulatory elements designed for operation in plants. The expression cassette is inserted into a desired expression vector with companion sequences upstream and downstream from the expression cassette suitable for expression in a plant host. The companion sequences will be of plasmid or viral origin and provide necessary characteristics to the vector to permit the vectors to move DNA from an original cloning host, such as bacteria, to the desired plant host. The basic bacterial/plant vector construct will preferably provide a broad host range prokaryote replication origin; a prokaryote selectable marker; and, for Agrobacterium transformations, T DNA sequences for Agrobacterium-mediated transfer to plant chromosomes.

Where the heterologous gene is not readily amenable to detection, the construct will preferably also have a selectable marker gene suitable for determining if a plant cell has been transformed. A general review of suitable markers, for example for the members of the grass family, is found in

Wilmink and Dons, 1993, Plant Mol. Biol. Reptr, 11(2):165-185.

Sequences suitable for permitting integration of the heterologous sequence into the plant genome are also recommended. These might include transposon sequences and the like for homologous recombination as well as Ti sequences which permit random insertion of a heterologous expression cassette into a plant genome. Suitable prokaryote selectable markers include resistance toward antibiotics such as ampicillin or tetracycline. Other DNA sequences encoding additional functions may also be present in the vector, as is known in the art.

The nucleic acid molecules of the subject invention may be included into an expression cassette for expression of the protein(s) of interest. Usually, there will be only one expression cassette, although two or more are feasible. The recombinant expression cassette will contain in addition to the heterologous protein encoding sequence the following elements, a promoter region, plant 5' untranslated sequences, initiation codon depending upon whether or not the structural gene comes equipped with one, and a transcription and translation termination sequence. Unique restriction enzyme sites at the 5' and 3' ends of the cassette allow for easy insertion into a pre-existing vector.

A heterologous coding sequence may be for any protein relating to the present invention. The sequence encoding the protein of interest will encode a signal peptide which allows processing and translocation of the protein, as appropriate, and will usually lack any sequence which might result in the binding of the desired protein of the invention to a membrane. Since, for the most part, the transcriptional initiation region will be for a gene which is expressed and translocated during germination, by employing the signal peptide which provides for translocation, one may also provide for translocation of the protein of interest. In this way, the protein(s) of interest will be translocated from the cells in which they are expressed and may be efficiently harvested. Typically secretion in seeds are across the aleurone or scutellar epithelium layer into the endosperm of the seed. While it is not required that the protein be secreted from the cells in which the protein is produced, this facilitates the isolation and purification of the recombinant protein.

Since the ultimate expression of the desired gene product will be in a eucaryotic cell it is desirable to determine whether any portion of the cloned gene contains sequences which will be processed out as introns by the host's splicosome machinery. If so, site-directed mutagenesis of the "intron" region may be conducted to prevent losing a portion of the genetic message as a false intron code, Reed and Maniatis, Cell 41:95-105, 1985.

The vector can be microinjected directly into plant cells by use of micropipettes to mechanically transfer the recombinant DNA. Crossway, Mol. Gen. Genet, 202:179-185, 1985. The genetic material may also be transferred into the plant cell by using polyethylene glycol, Krens, et al., Nature, 296, 72-74, 1982. Another method of introduction of nucleic acid segments is high velocity ballistic penetration by small particles with the nucleic acid either within the matrix of small beads or particles, or on the surface, Klein, et al., Nature, 327, 70-73, 1987 and Knudsen and Muller, 1991, Planta, 185:330-336 teaching particle bombardment of barley endosperm to create transgenic barley. Yet another method of introduction would be fusion of protoplasts with other entities, either minicells, cells, lysosomes or other fusible lipid-surfaced bodies, Fraley, et al., Proc. Natl. Acad. Sci. USA, 79, 1859-1863, 1982.

The vector may also be introduced into the plant cells by electroporation. (Fromm et al., Proc. Natl Acad. Sci. USA 82:5824, 1985). In this technique, plant protoplasts are electroporated in the presence of plasmids containing the gene construct. Electrical impulses of high field strength reversibly permeabilize biomembranes allowing the introduction of the plasmids. Electroporated plant protoplasts reform the cell wall, divide, and form plant callus.

All plants from which protoplasts can be isolated and cultured to give whole regenerated plants can be transformed by the present invention so that whole plants are recovered which contain the transferred gene. It is known that practically all plants can be regenerated from cultured cells or tissues, including but not limited to all major species of sugarcane, sugar beet, cotton, fruit and other trees, legumes and vegetables. Some suitable plants include, for example, species from the genera *Fragaria, Lotus, Medicago, Onobrychis, Trifolium, Trigonella, Vigna, Citrus, Linum, Geranium, Manihot, Daucus, Arabidopsis, Brassica, Raphanus, Sinapis, Atropa, Capsicum, Datura, Hyoscyamus, Lycopersion, Nicotiana, Solanum, Petunia, Digitalis, Majorana, Cichorium, Helianthus, Lactuca, Bromus, Asparagus, Antirrhinum, Hererocallis, Nemesia, Pelargonium, Panicum, Pennisetum, Ranunculus, Senecio, Salpiglossis, Cucumis, Browaalia, Glycine, Lolium, Zea, Triticum, Sorghum,* and *Datura.*

Means for regeneration vary from species to species of plants, but generally a suspension of transformed protoplasts containing copies of the heterologous gene is first provided. Callus tissue is formed and shoots may be induced from callus and subsequently rooted. Alternatively, embryo formation can be induced from the protoplast suspension. These embryos germinate as natural embryos to form plants. The culture media will generally contain various amino acids and hormones, such as auxin and cytokinins. It is also advantageous to add glutamic acid and proline to the medium, especially for such species as corn and alfalfa. Shoots and roots normally develop simultaneously. Efficient regeneration will depend on the medium, on the genotype, and on the history of the culture. If these three variables are controlled, then regeneration is fully reproducible and repeatable.

In some plant cell culture systems, the desired protein of the invention may be excreted or alternatively, the protein may be extracted from the whole plant. Where the desired protein of the invention is secreted into the medium, it may be collected. Alternatively, the embryos and embryoless-half seeds or other plant tissue may be mechanically disrupted to release any secreted protein between cells and tissues. The mixture may be suspended in a buffer solution to retrieve soluble proteins. Conventional protein isolation and purification methods will be then used to purify the recombinant protein. Parameters of time, temperature pH, oxygen, and volumes will be adjusted through routine methods to optimize expression and recovery of heterologous protein.

iv. Bacterial Systems

Bacterial expression techniques are known in the art. A bacterial promoter is any DNA sequence capable of binding bacterial RNA polymerase and initiating the downstream (3') transcription of a coding sequence (*eg.* structural gene) into mRNA. A promoter will have a transcription initiation region which is usually placed proximal to the 5' end of the coding sequence. This transcription initiation region usually includes an RNA polymerase binding site and a transcription initiation site. A bacterial promoter may also have a second domain called an operator, that may overlap an adjacent RNA polymerase binding site at which RNA synthesis begins. The operator permits negative regulated (inducible) transcription, as a gene repressor protein may bind the operator and thereby inhibit transcription of a specific gene. Constitutive expression may occur in the absence of negative regulatory elements, such as the operator. In addition, positive regulation may be achieved by a gene activator protein binding sequence, which, if present is usually proximal (5') to the RNA polymerase binding sequence. An example of a gene activator protein is the catabolite activator protein (CAP), which helps initiate transcription of the lac operon in Escherichia coli (E. coli) [Raibaud et al. (1984) Annu. Rev. Genet. 18:173]. Regulated expression may therefore be either positive or negative, thereby either enhancing or reducing transcription.

Sequences encoding metabolic pathway enzymes provide particularly useful promoter sequences. Examples include promoter sequences derived from sugar metabolizing enzymes, such as galactose, lactose *(lac)* [Chang et al. (1977) Nature 198:1056], and maltose. Additional examples include promoter sequences derived from biosynthetic enzymes such as tryptophan *(trp)* [Goeddel et al. (1980) Nuc. Acids Res. 8:4057; Yelverton et al. (1981) Nucl. Acids Res. 9:731; US patent 4,738,921; EP-A-0036776 and EP-A-0121775]. The g-laotamase *(bla)* promoter system [Weissmann (1981) "The cloning of interferon and other mistakes." In Interferon 3 (ed. 1. Gresser)], bacteriophage lambda PL [Shimatake et al. (1981) Nature 292:128] and T5 [US patent 4,689,406] promoter systems also provide useful promoter sequences.

In addition, synthetic promoters which do not occur in nature also function as bacterial promoters. For example, transcription activation sequences of one bacterial or bacteriophage promoter may be joined with the operon sequences of another bacterial or bacteriophage promoter, creating a synthetic hybrid promoter [US patent 4,551,433]. For example, the *tac* promoter is a hybrid *trp-lac* promoter comprised of both *trp* promoter and *lac* operon sequences that is regulated by the *lac* repressor [Amann et al. (1983) Gene 25:167; de Boer et al. (1983) Proc. Natl. Acad. Sci. 80:21]. Furthermore, a bacterial promoter can include naturally occurring promoters of non-bacterial origin that have the ability to bind bacterial RNA polymerase and initiate transcription. A naturally occurring promoter of non-bacterial origin can also be coupled with a compatible RNA polymerase to produce high levels of expression of some genes in prokaryotes. The bacteriophage T7 RNA polymerase/promoter system is an example of a coupled promoter system [Studier et al. (1986) J. Mol. Biol. 189:113; Tabor et al. (1985) Proc Natl. Acad. Sci. 82:1074]. In addition, a hybrid promoter can also be comprised of a bacteriophage promoter and an *E. coli* operator region (EPO-A-0 267 851).

In addition to a functioning promoter sequence, an efficient ribosome binding site is also useful for the expression of foreign genes in prokaryotes. In *E. coli,* the ribosome binding site is called the Shine-Dalgarno (SD) sequence and includes an initiation codon (ATG) and a sequence 3-9 nucleotides in length located 3-11 nucleotides upstream of the initiation codon [Shine et al. (1975) Nature 254:34]. The SD sequence is thought to promote binding of mRNA to the ribosome by the pairing of bases between the SD sequence and the 3' and of *E. coli* 16S rRNA [ Steitz et al. (1979) "Genetic signals and nucleotide sequences in messenger RNA." In Biological Regulation and Development: Gene Expression (ed. R.F. Goldberger)]. To express eukaryotic genes and prokaryotic genes with weak ribosome-binding site [Sambrook et al. (1989) "Expression of cloned genes in Escherichia coli." In Molecular Cloning: A Laboratory Manual].

A DNA molecule may be expressed intracellularly. A promoter sequence may be directly linked with the DNA molecule, in which case the first amino acid at the N-terminus will always be a methionine, which is encoded by the ATG start codon. If desired, methionine at the N-terminus may be cleaved from the protein by *in vitro* incubation with cyanogen bromide or by either *in vivo* on *in vitro* incubation with a bacterial methionine N-terminal peptidase (EPO-A-0 219 237).

Fusion proteins provide an alternative to direct expression. Usually, a DNA sequence encoding the N-terminal portion of an endogenous bacterial protein, or other stable protein, is fused to the 5' end of heterologous coding sequences. Upon expression, this construct will provide a fusion of the two amino acid sequences. For example, the bacteriophage lambda cell gene can be linked at the 5' terminus of a foreign gene and expressed in bacteria. The resulting fusion protein preferably retains a site for a processing enzyme (factor Xa) to cleave the bacteriophage protein from the foreign gene [Nagai et al. (1984) Nature 309:810]. Fusion proteins can also be made with sequences from the *lacZ* [Jia et al. (1987) Gene 60:197], *trpE* [Allen et al. (1987) J. Biotechnol. 5:93; Makoff et al. (1989) J. Gen. Microbiol. 135:11], and *Chey* [EP-A-0 324 647] genes. The DNA sequence at the junction of the two amino acid sequences may or may not encode a cleavable site. Another example is a ubiquitin fusion protein. Such a fusion protein is made with the ubiquitin region that preferably retains a site for a processing enzyme (*eg*. ubiquitin specific processing-protease) to cleave the ubiquitin from the foreign protein. Through this method, native foreign protein can be isolated [Miller et al. (1989) Bio/ Technology 7:698].

Alternatively, foreign proteins can also be secreted from the cell by creating chimeric DNA molecules that encode a fusion protein comprised of a signal peptide sequence fragment that provides for secretion of the foreign protein in bacteria [US patent 4,336,336]. The signal sequence fragment usually encodes a signal peptide comprised of hydrophobic amino acids which direct the secretion of the protein from the cell. The protein is either secreted into the growth media (gram-positive bacteria) or into the periplasmic space, located between the inner and outer membrane of the cell (gram-negative bacteria). Preferably there are processing sites, which can be cleaved either *in vivo* or *in vitro* encoded between the signal peptide fragment and the foreign gene.

DNA encoding suitable signal sequences can be derived from genes for secreted bacterial proteins, such as the *E. coli* outer membrane protein gene (*ompA*) [Masui et al. (1983), in: Experimental Manipulation of Gene Expression; Ghrayeb et al. (1984) EMBO J 3:2437] and the *E. coli* alkaline phosphatase signal sequence (*phoA*) [Oka et al. (1985) Proc. Natl. Acad. Sci. 82:7212]. As an additional example, the signal sequence of the alpha-amylase gene from various Bacillus strains can be used to secrete heterologous proteins from *B. subtilis* [Palva et al. (1982) Proc. Natl. Acad. Sci. USA 79:5582; EP-A-0 244 042].

Usually, transcription termination sequences recognized by bacteria are regulatory regions located 3' to the translation stop codon, and thus together with the promoter flank the coding sequence. These sequences direct the transcription of an mRNA which can be translated into the polypeptide encoded by the DNA. Transcription termination sequences frequently include DNA sequences of about 50 nucleotides capable of forming stem loop structures that aid in terminating transcription. Examples include transcription termination sequences derived from genes with strong promoters, such as the *trp* gene in *E. coli* as well as other biosynthetic genes.

Usually, the above described components, comprising a promoter, signal sequence (if desired), coding sequence of interest, and transcription termination sequence, are put together into expression constructs. Expression constructs are often maintained in a replicon, such as an extrachromosomal element (*eg*. plasmids) capable of stable maintenance in a host, such as bacteria. The replicon will have a replication system, thus allowing it to be maintained in a prokaryotic host either for expression or for cloning and amplification. In addition, a replicon may be either a high or low copy number plasmid. A high copy number plasmid will generally have a copy number ranging from about 5 to about 200, and usually about 10 to about 150. A host containing a high copy number plasmid will preferably contain at least about 10, and more preferably at least about 20 plasmids. Either a high or low copy number vector may be selected, depending upon the effect of the vector and the foreign protein on the host.

Alternatively, the expression constructs can be integrated into the bacterial genome with an integrating vector. Integrating vectors usually contain at least one sequence homologous to the bacterial chromosome that allows the vector to integrate. Integrations appear to result from recombinations between homologous DNA in the vector and the bacterial chromosome. For example, integrating vectors constructed with DNA from various Bacillus strains integrate into the Bacillus chromosome (EP-A- 0 127 328). Integrating vectors may also be comprised of bacteriophage or transposon sequences.

Usually, extrachromosomal and integrating expression constructs may contain selectable markers to allow for the selection of bacterial strains that have been transformed. Selectable markers can be expressed in the bacterial host and may include genes which render bacteria resistant to drugs such as ampicillin, chloramphenicol, erythromycin, kanamycin (neomycin), and tetracycline [Davies et al. (1978) Annu. Rev. Microbiol. 32:469]. Selectable markers may also include biosynthetic genes, such as those in the histidine, tryptophan, and leucine biosynthetic pathways.

Alternatively, some of the above described components can be put together in transformation vectors. Transformation vectors are usually comprised of a selectable market that is either maintained in a replicon or developed into an integrating vector, as described above.

Expression and transformation vectors, either extra-chromosomal replicons or integrating vectors, have been developed for transformation into many bacteria. For example, expression vectors have been developed for, *inter alia,* the following bacteria: Bacillus subtilis [Palva et al. (1982) Proc. Natl. Acad. Sci. USA 79:5582; EP-A-0 036 259 and EP-A-0 063 953; WO 84/04541], Escherichia coli [Shimatake et al. (1981) Nature 292:128; Amann et al. (1985) Gene 40: 183; Studier et al. (1986) J Mol. Biol. 189:113; EP-A-0 036 776,EP-A-0 136 829 and EP-A-0 136 907], Streptococcus cremoris [Powell et al. (1988) Appl. Environ. Microbiol. 54:655]; Streptococcus lividans [Powell et al. (1988) Appl. Environ. Microbiol. 54:655], Streptomyces lividans [US patent 4,745,056].

Methods of introducing exogenous DNA into bacterial hosts are well-known in the art, and usually include either the transformation of bacteria treated with $CaCl_2$ or other agents, such as divalent cations and DMSO. DNA can also be introduced into bacterial cells by electroporation. Transformation procedures usually vary with the bacterial species to be transformed. See *eg.* [Masson et al. (1989) FEMS Microbiol. Lett. 60:273; Palva et al. (1982) Proc. Natl. Acad. Sci. USA 79:5582; EP-A-0 036 259 and EP-A-0 063 953; WO 84/04541, Bacillus], [Miller et al. (1988) Proc. Natl. Acad. Sci. 85:856; Wang et al. (1990) J Bacteriol. 172:949, Campylobacter], [Cohen et al. (1973) Proc. Natl. Acad. Sci. 69: 2110; Dower et al. (1988) Nucleic Acids Res. 16:6127; Kushner (1978) "An improved method for transformation of Escherichia coli with ColEl-derived plasmids. In Genetic Engineering: Proceedings of the International Symposium on Genetic Engineering (eds. H.W. Boyer and S. Nicosia); Mandel et al. (1970) J Mol. Biol. 53:159; Taketo (1988) Biochim. Biophys. Acta 949:318; Escherichia], [Chassy et al. (1987) FEMS Microbiol. Lett. 44:173 Lactobacillus]; [Fiedler et al. (1988) Anal. Biochem 170:38, Pseudomonas]; [Augustin et al. (1990) FEMS Microbiol. Lett. 66:203, Staphylococcus], [Barany et al. (1980) J. Bacteriol. 144:698; Harlander (1987) "Transformation of Streptococcus lactis by electroporation, in: Streptococcal Genetics (ed. J. Ferretti and R. Curtiss III); Perry et al. (1981) Infect Immun. 32:1295; Powell et al. (1988) Appl. Environ. Microbiol. 54:655; Somkuti et al. (1987) Proc. 4th Evr. Cong. Biotechnology 1:412, Streptococcus].

v. Yeast Expression

Yeast expression systems are also known to one of ordinary skill in the art. A yeast promoter is any DNA sequence capable of binding yeast RNA polymerase and initiating the downstream (3') transcription of a coding sequence (*eg*. structural gene) into mRNA. A promoter will have a transcription initiation region which is usually placed proximal to the 5' end of the coding sequence. This transcription initiation region usually includes an RNA polymerase binding site (the "TATA Box") and a transcription initiation site. A yeast promoter may also have a second domain called an upstream activator sequence (UAS), which, if present, is usually distal to the structural gene. The UAS permits regulated (inducible) expression. Constitutive expression occurs in the absence of a UAS. Regulated expression may be either positive or negative, thereby either enhancing or reducing transcription.

Yeast is a fermenting organism with an active metabolic pathway, therefore sequences encoding enzymes in the metabolic pathway provide particularly useful promoter sequences. Examples include alcohol dehydrogenase (ADH) (EP-A-0 284 044), enolase, glucokinase, glucose-6-phosphate isomerase, glyceraldehyde-3-phosphate-dehydrogenase (GAP or GAPDH), hexokinase, phosphofructokinase, 3-phosphoglycerate mutase, and pyruvate kinase (PyK) (EPO-A-0 329 203). The yeast *PHO5* gene, encoding acid phosphatase, also provides useful promoter sequences [Myanohara et al. (1983) Proc. Natl. Acad. Sci. USA 80:1].

In addition, synthetic promoters which do not occur in nature also function as yeast promoters. For example, UAS sequences of one yeast promoter may be joined with the transcription activation region of another yeast promoter, creating a synthetic hybrid promoter. Examples of such hybrid promoters include the ADH regulatory sequence linked to the GAP transcription activation region (US Patent Nos. 4,876,197 and 4,880,734). Other examples of hybrid promoters include promoters which consist of the regulatory sequences of either the *ADH2, GAL4, GAL10,* OR *PHO5* genes, combined with the transcriptional activation region of a glycolytic enzyme gene such as GAP or

PyK (EP-A-0 164 556). Furthermore, a yeast promoter can include naturally occurring promoters of non-yeast origin that have the ability to bind yeast RNA polymerase and initiate transcription.

Examples of such promoters include, *inter alia,* [Cohen et al. (1980) Proc. Natl. Acad. Sci. USA 77:1078; Henikoff et al. (1981) Nature 283:835; Hollenberg et al. (1981) Curr. Topics Microbiol. Immunol. 96:119; Hollenberg et al. (1979) "The Expression of Bacterial Antibiotic Resistance Genes in the Yeast Saccharomyces cerevisiae," in: Plasmids of Medical, Environmental and Commercial Importance (eds. K.N. Timmis and A. Puhler); Mercerau-Puigalon et al. (1980) Gene 11:163; Panthier et al. (1980) Curr. Genet. 2:109;].

A DNA molecule may be expressed intracellularly in yeast. A promoter sequence may be directly linked with the DNA molecule, in which case the first amino acid at the N-terminus of the recombinant protein will always be a methionine, which is encoded by the ATG start codon. If desired, methionine at the N-terminus may be cleaved from the protein by *in vitro* incubation with cyanogen bromide.

Fusion proteins provide an alternative for yeast expression systems, as well as in mammalian, baculovirus, and bacterial expression systems. Usually, a DNA sequence encoding the N-terminal portion of an endogenous yeast protein, or other stable protein, is fused to the 5' end of heterologous coding sequences. Upon expression, this construct will provide a fusion of the two amino acid sequences. For example, the yeast or human superoxide dismutase (SOD) gene, can be linked at the 5' terminus of a foreign gene and expressed in yeast. The DNA sequence at the junction of the two amino acid sequences may or may not encode a cleavable site. See *eg.* EP-A-0 196 056. Another example is a ubiquitin fusion protein. Such a fusion protein is made with the ubiquitin region that preferably retains a site for a processing enzyme (*eg.* ubiquitin-specific processing protease) to cleave the ubiquitin from the foreign protein. Through this method, therefore, native foreign protein can be isolated *(eg.* WO88/024066).

Alternatively, foreign proteins can also be secreted from the cell into the growth media by creating chimeric DNA molecules that encode a fusion protein comprised of a leader sequence fragment that provide for secretion in yeast of the foreign protein. Preferably, there are processing sites encoded between the leader fragment and the foreign gene that can be cleaved either *in vivo* or *in vitro.* The leader sequence fragment usually encodes a signal peptide comprised of hydrophobic amino acids which direct the secretion of the protein from the cell.

DNA encoding suitable signal sequences can be derived from genes for secreted yeast proteins, such as the yeast invertase gene (EP-A-0 012 873; JPO. 62,096,086) and the A-factor gene (US patent 4,588,684). Alternatively, leaders of non-yeast origin, such as an interferon leader, exist that also provide for secretion in yeast (EP-A-0 060 057).

A preferred class of secretion leaders are those that employ a fragment of the yeast alpha-factor gene, which contains both a "pre" signal sequence, and a "pro" region. The types of alpha-factor fragments that can be employed include the full-length pre-pro alpha factor leader (about 83 amino acid residues) as well as truncated alpha-factor leaders (usually about 25 to about 50 amino acid residues) (US Patents 4,546,083 and 4,870,008; EP-A-0 324 274). Additional leaders employing an alpha-factor leader fragment that provides for secretion include hybrid alpha-factor leaders made with a presequence of a first yeast, but a pro-region from a second yeast alphafactor. (eg. see WO 89/02463.)

Usually, transcription termination sequences recognized by yeast are regulatory regions located 3' to the translation stop codon, and thus together with the promoter flank the coding sequence. These sequences direct the transcription of an mRNA which can be translated into the polypeptide encoded by the DNA. Examples of transcription terminator sequence and other yeast-recognized termination sequences, such as those coding for glycolytic enzymes.

Usually, the above described components, comprising a promoter, leader (if desired), coding sequence of interest, and transcription termination sequence, are put together into expression constructs. Expression constructs are often maintained in a replicon, such as an extrachromosomal element (eg. plasmids) capable of stable maintenance in a host, such as yeast or bacteria. The replicon may have two replication systems, thus allowing it to be maintained, for example, in yeast for expression and in a prokaryotic host for cloning and amplification. Examples of such yeast-bacteria shuttle vectors include YEp24 [Botstein et al. (1979) Gene 8:17-24], pCl/l [Brake et al. (1984) Proc. Natl. Acad. Sci USA 81:4642-4646], and YRp 17 [Stinchcomb et al. (1982) J. Mol. Biol. 158:157]. In addition, a replicon may be either a high or low copy number plasmid. A high copy number plasmid will generally have a copy number ranging from about 5 to about 200, and usually about 10 to about 150. A host containing a high copy number plasmid will preferably have at least about 10, and more preferably at least about 20. Enter a high or low copy number vector may be selected, depending upon the effect of the vector and the foreign protein on the host. See *eg.* Brake *et al., supra.*

Alternatively, the expression constructs can be integrated into the yeast genome with an integrating vector. Integrating vectors usually contain at least one sequence homologous to a yeast chromosome that allows the vector to integrate, and preferably contain two homologous sequences flanking the expression construct. Integrations appear to result from recombinations between homologous DNA in the vector and the yeast chromosome [Orr-Weaver et al. (1983) Methods in Enzymol. 101:228-245]. An integrating vector may be directed to a specific locus in yeast by selecting the appropriate homologous sequence for inclusion in the vector. See Orr-Weaver *et al., supra.* One or more expression construct may integrate, possibly affecting levels of recombinant protein produced [Rine et al. (1983) Proc. Natl. Acad. Sci. USA 80:6750]. The chromosomal sequences included in the vector can occur either as a single segment in the vector, which results in the integration of the entire vector, or two segments homologous to adjacent segments in the chromosome and flanking the expression construct in the vector, which can result in the stable integration of only the expression construct.

Usually, extrachromosomal and integrating expression constructs may contain selectable markers to allow for the selection of yeast strains that have been transformed. Selectable markers may include biosynthetic genes that can be expressed in the yeast host, such as *ADE2, HIS4, LEU2, TRP1,* and *ALG7,* and the G418 resistance gene, which confer resistance in yeast cells to tunicamycin and G418, respectively. In addition, a suitable selectable marker may also provide yeast with the ability to grow in the presence of toxic compounds, such as metal. For example, the presence of *CUP1* allows yeast to grow in the presence of copper ions [Butt et al. (1987) Microbiol, Rev. 51:351].

Alternatively, some of the above described components can be put together into transformation vectors. Transformation vectors are usually comprised of a selectable marker that is either maintained in a replicon or developed into an integrating vector, as described above.

Expression and transformation vectors, either extrachromosomal replicons or integrating vectors, have been developed for transformation into many yeasts. For example, expression vectors have been developed for, *inter alia,* the following yeasts:Candida albicans [Kurtz, et al. (1986) Mol. Cell. Biol. 6:142], Candida maltosa [Kunze, et al. (1985) J. Basic Microbiol. 25:141]. Hansenula polymorpha [Gleeson, et al. (1986) J. Gen. Microbiol. 132:3459; Roggenkamp et al. (1986) Mol. Gen. Genet. 202:302], Kluyveromyces fragilis [Das, et al. (1984) J. Bacteriol. 158:1165], Kluyveromyces lactis [De Louvencourt et al. (1983) J Bacteriol. 154:737; Van den Berg et al. (1990) Bio/Technology 8:135], Pichia guillerimondii [Kunze et al. (1985) J. Basic Microbiol. 25:141], Pichia pastoris [Cregg, et al. (1985) Mol. Cell. Biol. 5: 3376; US Patent Nos. 4,837,148 and 4,929,555], Saccharomyces cerevisiae [Hinnen et al. (1978) Proc. Natl. Acad. Sci. USA 75:1929; Ito et al. (1983) J. Bacteriol. 153:163], Schizosaccharomyces pombe [Beach and Nurse (1981) Nature 300:706], and Yarrowia lipolytica [Davidow, et al. (1985) Curr. Genet. 10:380471 Gaillardin, et al. (1985) Curr. Genet. 10:49].

Methods of introducing exogenous DNA into yeast hosts are well-known in the art, and usually include either the transformation of spheroplasts or of intact yeast cells treated with alkali cations.

Transformation procedures usually vary with the yeast species to be transformed. See *eg.* [Kurtz et al. (1986) Mol. Cell. Biol. 6:142; Kunze et al. (1985) J. Basic Microbiol. 25:141; Candida]; [Gleeson et al. (1986) J. Gen. Microbiol. 132:3459; Roggenkamp et al. (1986) Mol. Gen. Genet. 202:302; Hansenula]; [Das et al. (1984) J. Bacteriol. 158:1165; De Louvencourt et al. (1983) J. Bacteriol. 154:1165; Van den Berg et al. (1990) Bio/Technology 8:135; Kluyveromyces]; [Cregg et al. (1985) Mol. Cell. Biol. 5:3376; Kunze et al. (1985) J. Basic Microbiol. 25:141; US Patent Nos. 4,837,148 and 4,929,555; Pichia]; [Hinnen et al. (1978) Proc. Natl. Acad Sci. USA 75;1929; Ito et al. (1983) J. Bacteriol. 153:163 Saccharomyces]; [Beach and Nurse (1981) Nature 300:706; Schizosaccharomyces]; [Davidow et al. (1985) Curr. Genet. 10:39; Gaillardin et al. (1985) Curr. Genet. 10:49; Yarrowia].

*Antibodies*

As used herein, the term "antibody" refers to a polypeptide or group of polypeptides composed of at least one antibody combining site. An "antibody combining site" is the three-dimensional binding space with an internal surface shape and charge distribution complementary to the features of an epitope of an antigen, which allows a binding of the antibody with the antigen. "Antibody" includes, for example, vertebrate antibodies, hybrid antibodies, chimeric antibodies, humanised antibodies, altered antibodies, univalent antibodies, Fab proteins, and single domain antibodies.

Antibodies against the proteins of the invention are useful for affinity chromatography, immunoassays, and distinguishing/identifying Neisserial proteins.

Antibodies to the proteins of the invention, both polyclonal and monoclonal, may be prepared by conventional methods. In general, the protein is first used to immunize a suitable animal, preferably a mouse, rat, rabbit or goat. Rabbits and goats are preferred for the preparation of polyclonal sera due to the volume of serum obtainable, and the availability of labeled anti-rabbit and anti-goat antibodies. Immunization is generally performed by mixing or emulsifying the protein in saline, preferably in an adjuvant such as Freund's complete adjuvant, and injecting the mixture or emulsion parenterally (generally subcutaneously or intramuscularly). A dose of 50-200 μg/injection is typically sufficient. Immunization is generally boosted 2-6 weeks later with one or more injections of the protein in saline, preferably using Freund's incomplete adjuvant. One may alternatively generate antibodies by in vitro immunization using methods known in the art, which for the purposes of this invention is considered equivalent to *in vivo* immunization. Polyclonal antisera is obtained by bleeding the immunized animal into a glass or plastic container, incubating the blood at 25°C for one hour, followed by incubating at 4°C for 2-18 hours. The serum is recovered by centrifugation *(eg.* 1,000g for 10 minutes). About 20-50 ml per bleed may be obtained from rabbits.

Monoclonal antibodies are prepared using the standard method of Kohler & Milstein [Nature (1975) 256:495-96], or a modification thereof. Typically, a mouse or rat is immunized as described above. However, rather than bleeding the animal to extract serum, the spleen (and optionally several large lymph nodes) is removed and dissociated into single cells. If desired, the spleen cells may be screened (after removal of nonspecifically adherent cells) by applying a cell suspension to a plate or well coated with the protein antigen. B-cells expressing membrane-bound immunoglobulin specific for the antigen bind to the plate, and are not rinsed away with the rest of the suspension. Resulting B-cells, or all dissociated spleen cells, are then induced to fuse with myeloma cells to form hybridomas, and are cultured in a selective medium (eg. hypoxanthine, aminopterin, thymidine medium, "HAT"). The resulting hybridomas are plated by limiting dilution, and are assayed for the production of antibodies which bind specifically to the immunizing antigen (and which do not bind to unrelated antigens). The selected MAb-secreting hybridomas are then cultured either *in vitro* (*eg.* in tissue culture bottles or hollow fiber reactors), or *in vivo* (as ascites in mice).

If desired, the antibodies (whether polyclonal or monoclonal) may be labeled using conventional techniques. Suitable labels include fluorophores, chromophores, radioactive atoms (particularly $^{32}P$ and $^{125}I$), electron-dense reagents, enzymes, and ligands having specific binding partners. Enzymes are typically detected by their activity. For example, horseradish peroxidase is usually detected by its ability to convert 3,3',5,5'-tetramethylbenzidine (TMB) to a blue pigment, quantifiable with a spectrophotometer. "Specific binding partner" refers to a protein capable of binding a ligand molecule with high specificity, as for example in the case of an antigen and a monoclonal antibody specific therefor. Other specific binding partners include biotin and avidin or streptavidin, IgG and protein A, and the numerous receptor-ligand couples known in the art. It should be understood that the above description is not meant to categorize the various labels into distinct classes, as the same label may serve in several different modes. For example, $^{125}I$ may serve as a radioactive label or as an electron-dense reagent. HRP may serve as enzyme or as antigen for a MAb. Further, one may combine various labels for desired effect. For example, MAbs and avidin also require labels in the practice of this invention: thus, one might label a MAb with biotin, and detect its presence with avidin labeled with $^{125}I$, or with an anti-biotin MAb labeled with HRP. Other permutations and possibilities will be readily apparent to those of ordinary skill in the art, and are considered as equivalents within the scope of the instant invention.

*Pharmaceutical Compositions*

Pharmaceutical compositions can comprise either polypeptides, antibodies, or nucleic acid of the invention. The pharmaceutical compositions will comprise a therapeutically effective amount of either polypeptides, antibodies, or polynucleotides of the claimed invention.

The term "therapeutically effective amount" as used herein refers to an amount of a therapeutic agent to treat, ameliorate, or prevent a desired disease or condition, or to exhibit a detectable therapeutic or preventative effect. The effect can be detected by, for example, chemical markers or antigen levels. Therapeutic effects also include reduction in physical symptoms, such as decreased body temperature. The precise effective amount for a subject will depend upon the subject's size and health, the nature and extent of the condition, and the therapeutics or combination of therapeutics selected for administration. Thus, it is not useful to specify an exact effective amount in advance. However, the effective amount for a given situation can be determined by routine experimentation and is within the judgement of the clinician.

For purposes of the present invention, an effective dose will be from about 0.01 mg/ kg to 50 mg/kg or 0.05 mg/kg to about 10 mg/kg of the DNA constructs in the individual to which it is administered.

A pharmaceutical composition can also contain a pharmaceutically acceptable carrier. The term "pharmaceutically acceptable carrier" refers to a carrier for administration of a therapeutic agent, such as antibodies or a polypeptide, genes, and other therapeutic agents. The term refers to any pharmaceutical carrier that does not itself induce the production of antibodies harmful to the individual receiving the composition, and which may be administered without undue toxicity. Suitable carriers may be large, slowly metabolized macromolecules such as proteins, polysaccharides, polylactic acids, polyglycolic acids, polymeric amino acids, amino acid copolymers, and inactive virus particles. Such carriers are well known to those of ordinary skill in the art.

Pharmaceutically acceptable salts can be used therein, for example, mineral acid salts such as hydrochlorides, hydrobromides, phosphates, sulfates, and the like; and the salts of organic acids such as acetates, propionates, malonates, benzoates, and the like. A thorough discussion of pharmaceutically acceptable excipients is available in Remington's Pharmaceutical Sciences (Mack Pub. Co., N.J. 1991).

Pharmaceutically acceptable carriers in therapeutic compositions may contain liquids such as water, saline, glycerol and ethanol. Additionally, auxiliary substances, such as wetting or emulsifying agents, pH buffering substances, and the like, may be present in such vehicles. Typically, the therapeutic compositions are prepared as injectables, either as liquid solutions or suspensions; solid forms suitable for solution in, or suspension in, liquid vehicles prior to injection may also be prepared. Liposomes are included within the definition of a pharmaceutically acceptable carrier.

*Delivery Methods*

Once formulated, the compositions of the invention can be administered directly to the subject. The subjects to be treated can be animals; in particular, human subjects can be treated.

Direct delivery of the compositions will generally be accomplished by injection, either subcutaneously, intraperitoneally, intravenously or intramuscularly or delivered to the interstitial space of a tissue. The compositions can also be administered into a lesion. Other modes of administration include oral and pulmonary administration, suppositories, and transdermal or transcutaneous applications (*eg*. see WO98/20734), needles, and gene guns or hyposprays. Dosage treatment may be a single dose schedule or a multiple dose schedule.

*Vaccines*

Vaccines according to the invention may either be prophylactic (*ie*. to prevent infection) or therapeutic (*ie*. to treat disease after infection).

Such vaccines comprise immunising antigen(s), immunogen(s), polypeptide(s), protein(s) or nucleic acid, usually in combination with "pharmaceutically acceptable carriers," which include any carrier that does not itself induce the production of antibodies harmful to the individual receiving the composition. Suitable carriers are typically large, slowly metabolized macromolecules such as proteins, polysaccharides, polylactic acids, polyglycolic acids, polymeric amino acids, amino acid copolymers, lipid aggregates (such as oil droplets or liposomes), and inactive virus particles. Such carriers are well known to those of ordinary skill in the art. Additionally, these carriers may function as immunostimulating agents ("adjuvants"). Furthermore, the antigen or immunogen may be conjugated to a bacterial toxoid, such as a toxoid from diphtheria, tetanus, cholera, *H. pylori, etc.* pathogens.

Preferred adjuvants to enhance effectiveness of the composition include, but are not limited to: (1) aluminum salts (alum), such as aluminum hydroxide, aluminum phosphate, aluminum sulfate, etc; (2) oil-in-water emulsion formulations (with or without other specific immunostimulating agents such as muramyl peptides (see below) or bacterial cell wall components), such as for example (a) MF59™ (WO 90/14837; Chapter 10 in Vaccine design: the subunit and adjuvant approach, eds. Powell & Newman, Plenum Press 1995), containing 5% Squalene, 0.5% Tween 80, and 0.5% Span 85 (optionally containing various amounts of MTP-PE (see below), although not required) formulated into submicron particles using a microfluidizer such as Model 110Y microfluidizer (Microfluidics, Newton, MA), (b) SAF, containing 10% Squalane, 0.4% Tween 80, 5% pluronic-blocked polymer L121, and thr-MDP (see below) either microfluidized into a submicron emulsion or vortexed to generate a larger particle size emulsion, and (c) Ribi™ adjuvant system (RAS), (Ribi Immunochem, Hamilton, MT) containing 2% Squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox™); (3) saponin adjuvants, such as Stimulon™ (Cambridge Bioscience, Worcester, MA) may be used or particles generated therefrom such as ISCOMs (immunostimulating complexes); (4) Complete Freund's

Adjuvant (CFA) and Incomplete Freund's Adjuvant (IFA); (5) cytokines, such as interleukins (eg.

IL-1, IL-2, IL-4, IL-5, IL-6, IL-7, IL-12, *etc.),* interferons *(eg.* gamma interferon), macrophage colony stimulating factor (M-CSF), tumor necrosis factor (TNF), etc; and (6) other substances that act as immunostimulating agents to enhance the effectiveness of the composition. Alum and MF59™ are preferred.

As mentioned above, muramyl peptides include, but are not limited to, N-acetyl-muramyl-L-threonyl-D-isoglutamine (thr-MDP), N-acetyl-normuramyl-L-alanyl-D-isoglutamine (nor-MDP), N-acetylmuramyl-L-alanyl-D-isoglutaminyl-L-alanine-2-(1'-2'-dipalmitoyl-*sn*-glycero-3-hydroxyphosphoryloxy)-ethylamine (MTP-PE), *etc.*

The immunogenic compositions (*eg.* the immunising antigen/immunogen/polypeptide/protein/nucleic acid, pharmaceutically acceptable carrier, and adjuvant) typically will contain diluents, such as water, saline, glycerol, ethanol, etc. Additionally, auxiliary substances, such as wetting or emulsifying agents, pH buffering substances, and the like, may be present in such vehicles.

Typically, the immunogenic compositions are prepared as injectables, either as liquid solutions or suspensions; solid forms suitable for solution in, or suspension in, liquid vehicles prior to injection may also be prepared. The preparation also may be emulsified or encapsulated in liposomes for enhanced adjuvant effect, as discussed above under pharmaceutically acceptable carriers.

Immunogenic compositions used as vaccines comprise an immunologically effective amount of the antigenic or immunogenic polypeptides, as well as any other of the above-mentioned components, as needed. By "immunologically effective amount", it is meant that the administration of that amount to an individual, either in a single dose or as part of a series, is effective for treatment or prevention. This amount varies depending upon the health and physical condition of the individual to be treated, the taxonomic group of individual to be treated *(eg.* nonhuman primate, primate, *etc. ),* the capacity of the individual's immune system to synthesize antibodies, the degree of protection desired, the formulation of the vaccine, the treating doctor's assessment of the medical situation, and other relevant factors. It is expected that the amount will fall in a relatively broad range that can be determined through routine trials.

The immunogenic compositions are conventionally administered parenterally, *eg.* by injection, either subcutaneously, intramuscularly, or transdermally/transcutaneously (*eg.* WO98/20734). Additional formulations suitable for other modes of administration include oral and pulmonary formulations, suppositories, and transdermal applications. Dosage treatment may be a single dose schedule or a multiple dose schedule. The vaccine may be administered in conjunction with other immunoregulatory agents.

As an alternative to protein-based vaccines, DNA vaccination may be employed [eg. Robinson & Torres (1997) Seminars in Immunology 9:271-283; Donnelly et al. (1997) Annu Rev Immunol 15:617-648; see later herein].

<u>*Gene Delivery Vehicles*</u>

Gene therapy vehicles for delivery of constructs including a coding sequence of a therapeutic of the invention, to be delivered to the mammal for expression in the mammal, can be administered either locally or systemically. These constructs can utilize viral or non-viral vector approaches in *in vivo* or *ex vivo* modality. Expression of such coding sequence can be induced using endogenous mammalian or heterologous promoters. Expression of the coding sequence in vivo can be either constitutive or regulated.

The invention includes gene delivery vehicles capable of expressing the contemplated nucleic acid sequences. The gene delivery vehicle is preferably a viral vector and, more preferably, a retroviral, adenoviral, adeno-associated viral (AAV), herpes viral, or alphavirus vector. The viral vector can also be an astrovirus, coronavirus, orthomyxovirus, papovavirus, paramyxovirus, parvovirus, picornavirus, poxvirus, or togavirus viral vector. See generally, Jolly (1994) Cancer Gene Therapy 1:51-64; Kimura (1994) Human Gene Therapy 5:845-852; Connelly (1995) Human Gene Therapy 6:185-193; and Kaplitt (1994) Nature Genetics 6:148-153.

Retroviral vectors are well known in the art and we contemplate that any retroviral gene therapy vector is employable in the invention, including B, C and D type retroviruses, xenotropic retroviruses (for example, NZB-X1, NZB-X2 and NZB9-1 (see O'Neill (1985) J Virol. 53:160) polytropic retroviruses *eg.* MCF and MCF-MLV (see Kelly (1983) J Virol. 45:291), spumaviruses and lentiviruses. See RNA Tumor Viruses, Second Edition, Cold Spring Harbor Laboratory, 1985.

Portions of the retroviral gene therapy vector may be derived from different retroviruses. For example, retrovector LTRs may be derived from a Murine Sarcoma Virus, a tRNA binding site from a Rous Sarcoma Virus, a packaging signal from a Murine Leukemia Virus, and an origin of second strand synthesis from an Avian Leukosis Virus.

These recombinant retroviral vectors may be used to generate transduction competent retroviral vector particles by introducing them into appropriate packaging cell lines (see US patent 5,591,624). Retrovirus vectors can be constructed for site-specific integration into host cell DNA by incorporation of a chimeric integrase enzyme into the retroviral particle (see WO96/37626). It is preferable that the recombinant viral vector is a replication defective recombinant virus.

Packaging cell lines suitable for use with the above-described retrovirus vectors are well known in the art, are readily prepared (see WO95/30763 and WO92/05266), and can be used to create producer cell lines (also termed vector cell lines or "VCLs") for the production of recombinant vector particles. Preferably, the packaging cell lines are made from human parent cells (eg. HT1080 cells) or mink parent cell lines, which eliminates inactivation in human serum.

Preferred retroviruses for the construction of retroviral gene therapy vectors include Avian Leukosis Virus, Bovine Leukemia, Virus, Murine Leukemia Virus, Mink-Cell Focus-Inducing Virus, Murine Sarcoma Virus, Reticuloendotheliosis Virus and Rous Sarcoma Virus. Particularly preferred Murine Leukemia Viruses include 4070A and 1504A (Hartley and Rowe (1976) J Virol 19:19-25), Abelson (ATCC No. VR-999), Friend (ATCC No. VR-245), Graffi, Gross (ATCC Nol VR-590), Kirsten, Harvey Sarcoma Virus and Rauscher (ATCC No. VR-998) and Moloney Murine Leukemia Virus (ATCC No. VR-190). Such retroviruses may be obtained from depositories or collections such as the American Type Culture Collection ("ATCC") in Rockville, Maryland or isolated from known sources using commonly available techniques.

Exemplary known retroviral gene therapy vectors employable in this invention include those described in patent applications GB2200651, EP0415731, EP0345242, EP0334301, WO89/02468; WO89/05349, WO89/09271, WO90/02806, WO90/07936, WO94/03622, WO93/25698, WO93/25234, WO93/11230, WO93/10218, WO91/02805, WO91/02825, WO95/07994, US 5,219,740, US 4,405,712, US 4,861,719, US 4,980,289, US 4,777,127, US 5,591,624. See also Vile (1993) Cancer Res 53:3860-3864; Vile (1993) Cancer Res 53:962-967; Ram (1993) Cancer Res 53 (1993) 83-88; Takamiya (1992) J Neurosci Res 33:493-503; Baba (1993) J Neurosurg 79:729-735; Mann (1983) Cell 33:153; Cane (1984) Proc Natl Acad Sci 81:6349; and Miller (1990) Human Gene Therapy 1.

Human adenoviral gene therapy vectors are also known in the art and employable in this invention.

See, for example, Berkner (1988) Biotechniques 6:616 and Rosenfeld (1991) Science 252:431, and WO93/07283, WO93/06223, and WO93/07282. Exemplary known adenoviral gene therapy vectors employable in this invention include those described in the above referenced documents and in WO94/12649, WO93/03769, WO93/19191, WO94/28938, WO95/11984 WO95/00655, WO95/27071, WO95/29993, WO95/34671, WO96/05320, WO94/08026, WO94/11506, WO93/06223, WO94/24299, WO95/14102, WO95/24297, WO95/02697, WO94/28152, WO94/24299, WO95/09241, WO95/25807, WO95/05835, WO94/18922 and WO95/09654. Alternatively, administration of DNA linked to killed adenovirus as described in Curiel (1992) Hum. Gene Ther. 3:147-154 may be employed. The gene delivery vehicles of the invention also include adenovirus associated virus (AAV) vectors. Leading and preferred examples of such vectors for use in this invention are the AAV-2 based vectors disclosed in Srivastava, WO93/09239. Most preferred AAV vectors comprise the two AAV inverted terminal repeats in which the native D-sequences are modified by substitution of nucleotides, such that at least 5 native nucleotides and up to 18 native nucleotides, preferably at least 10 native nucleotides up to 18 native nucleotides, most preferably 10 native nucleotides are retained and the remaining nucleotides of the D-sequence are deleted or replaced with non-native nucleotides. The native D-sequences of the AAV inverted terminal repeats are sequences of 20 consecutive nucleotides in each AAV inverted terminal repeat (*ie*. there is one sequence at each end) which are not involved in HP formation. The non-native replacement nucleotide may be any nucleotide other than the nucleotide found in the native D-sequence in the same position. Other employable exemplary AAV vectors are pWP-19, pWN-1, both of which are disclosed in Nahreini (1993) Gene 124:257-262. Another example of such an AAV vector is psub201 (see Samulski (1987) J. Virol. 61:3096). Another exemplary AAV vector is the Double-D ITR vector. Construction of the Double-D ITR vector is disclosed in US Patent 5,478,745. Still other vectors are those disclosed in Carter US Patent 4,797,368 and Muzyczka US Patent 5,139,941, Chartejee US Patent 5,474,935, and Kotin WO94/288157. Yet a further example of an AAV vector employable in this invention is SSV9AFABTKneo, which contains the AFP enhancer and albumin promoter and directs expression predominantly in the liver.

Its structure and construction are disclosed in Su (1996) Human Gene Therapy 7:463-470. Additional AAV gene therapy vectors are described in US 5,354,678, US 5,173,414, US 5,139,941, and US 5,252,479.

The gene therapy vectors of the invention also include herpes vectors. Leading and preferred examples are herpes simplex virus vectors containing a sequence encoding a thymidine kinase polypeptide such as those disclosed in US 5,288,641 and EP0176170 (Roizman). Additional exemplary herpes simplex virus vectors include HFEM/ICP6-LacZ disclosed in WO95/04139 (Wistar Institute), pHSVlac described in Geller (1988) Science 241:1667-1669 and in WO90/09441 and WO92/07945, HSV Us3::pgC-lacZ described in Fink (1992) Human Gene Therapy 3:11-19 and HSV 7134, 2 RH 105 and GAL4 described in EP 0453242 (Breakefield), and those deposited with the ATCC as accession numbers ATCC VR-977 and ATCC VR-260.

Also contemplated are alpha virus gene therapy vectors that can be employed in this invention. Preferred alpha virus vectors are Sindbis viruses vectors. Togaviruses, Semliki Forest virus (ATCC VR-67; ATCC VR-1247), Middleberg virus (ATCC VR-370), Ross River virus (ATCC VR-373; ATCC VR-1246), Venezuelan equine encephalitis virus (ATCC VR923; ATCC VR-1250; ATCC VR-1249; ATCC VR-532), and those described in US patents 5,091,309, 5,217,879, and WO92/10578. More particularly, those alpha virus vectors described in US Serial No. 08/405,627, filed March 15, 1995,WO94/21792, WO92/10578, WO95/07994, US 5,091,309 and US 5,217,879 are employable. Such alpha viruses may be obtained from depositories or collections such as the ATCC in Rockville, Maryland or isolated from known sources using commonly available techniques. Preferably, alphavirus vectors with reduced cytotoxicity are used (see USSN 08/679640).

DNA vector systems such as eukarytic layered expression systems are also useful for expressing the nucleic acids of the invention. See WO95/07994 for a detailed description of eukaryotic layered expression systems. Preferably, the eukaryotic layered expression systems of the invention are derived from alphavirus vectors and most preferably from Sindbis viral vectors.

Other viral vectors suitable for use in the present invention include those derived from poliovirus, for example ATCC VR-58 and those described in Evans, Nature 339 (1989) 385 and Sabin (1973) J. Biol. Standardization 1:115; rhinovirus, for example ATCC VR-1110 and those described in Arnold (1990) J Cell Biochem L401; pox viruses such as canary pox virus or vaccinia virus, for example ATCC VR-111 and ATCC VR-2010 and those described in Fisher-Hoch (1989) Proc Natl Acad Sci 86:317; Flexner (1989) Ann NY Acad Sci 569:86, Flexner (1990) vaccine 8:17; in US 4,603,112 and US 4,769,330 and WO89/01973; SV40 virus, for example ATCC VR-305 and those described in Mulligan (1979) Nature 277:108 and Madzak (1992) J Gen Virol 73:1533; influenza virus, for example ATCC VR-797 and recombinant influenza viruses made employing reverse genetics techniques as described in US 5,166,057 and in Enami (1990) Proc Natl Acad Sci 87:3802-3805; Enami & Palese (1991) J Virol 65:2711-2713 and Luytjes (1989) Cell 59:110, (see also McMichael (1983) NEJ Med 309:13, and Yap (1978) Nature 273:238 and Nature (1979) 277:108); human immunodeficiency virus as described in EP-0386882 and in Buchschacher (1992) J Virol. 66:2731; measles virus, for example ATCC VR-67 and VR-1247 and those described in EP-0440219; Aura virus, for example ATCC VR-368; Bebaru virus, for example ATCC VR-600 and ATCC VR-1240; Cabassou virus, for example ATCC VR-922; Chikungunya virus, for example ATCC VR-64 and ATCC VR-1241; Fort Morgan Virus, for example ATCC VR-924; Getah virus, for example ATCC VR-369 and ATCC VR-1243; Kyzylagach virus, for example ATCC VR-927; Mayaro virus, for example ATCC VR-66; Mucambo virus, for example ATCC VR-580 and ATCC VR-1244; Ndumu virus, for example ATCC VR-371; Pixuna virus, for example ATCC VR-372 and ATCC VR-1245; Tonate virus, for example ATCC VR-925; Triniti virus, for example ATCC VR-469; Una virus, for example ATCC VR-374; Whataroa virus, for example ATCC VR-926; Y-62-33 virus, for example ATCC VR-375; O'Nyong virus, Eastern encephalitis virus, for example ATCC VR-65 and ATCC VR-1242; Western encephalitis virus, for example ATCC VR-70, ATCC VR-1251, ATCC VR-622 and ATCC VR-1252; and coronavirus, for example ATCC VR-740 and those described in Hamre (1966) Proc Soc Exp Biol Med 121:190.

Delivery of the compositions of this invention into cells is not limited to the above mentioned viral vectors. Other delivery methods and media may be employed such as, for example, nucleic acid expression vectors, polycationic condensed DNA linked or unlinked to killed adenovirus alone, for example see US Serial No. 08/366,787, filed December 30, 1994 and Curiel (1992) Hum Gene Ther 3:147-154 ligand linked DNA, for example see Wu (1989) J Biol Chem 264: 16985-16987, eucaryotic cell delivery vehicles cells, for example see US Serial No.08/240,030, filed May 9, 1994, and US Serial No. 08/404,796, deposition of photopolymerized hydrogel materials, hand-held gene transfer particle gun, as described in US Patent 5,149,655, ionizing radiation as described in US5,206,152 and in WO92/11033, nucleic charge neutralization or fusion with cell membranes. Additional approaches are described in Philip (1994) Mol Cell Biol 14: 2411-2418 and in Woffendin (1994) Proc Natl Acad Sci 91:1581-1585.

Particle mediated gene transfer may be employed, for example see US Serial No. 60/023,867. Briefly, the sequence can be inserted into conventional vectors that contain conventional control sequences for high level expression, and then incubated with synthetic gene transfer molecules such as polymeric DNA-binding cations like polylysine, protamine, and albumin, linked to cell targeting ligands such as asialoorosomucoid, as described in Wu & Wu (1987) J. Biol. Chem. 262:4429-4432, insulin as described in Hucked (1990) Biochem Pharmacol 40:253-263, galactose as described in Plank (1992) Bioconjugate Chem 3:533-539, lactose or transferrin.

Naked DNA may also be employed. Exemplary naked DNA introduction methods are described in WO 90/11092 and US 5,580,859. Uptake efficiency may be improved using biodegradable latex beads. DNA coated latex beads are efficiently transported into cells after endocytosis initiation by the beads. The method may be improved further by treatment of the beads to increase hydrophobicity and thereby facilitate disruption of the endosome and release of the DNA into the cytoplasm.

Liposomes that can act as gene delivery vehicles are described in US 5,422,120, WO95/13796, WO94/23697, WO91/14445 and EP-524,968. As described in USSN. 60/023,867, on non-viral delivery, the nucleic acid sequences encoding a polypeptide can be inserted into conventional vectors that contain conventional control sequences for high level expression, and then be incubated with synthetic gene transfer molecules such as polymeric DNA-binding cations like polylysine, protamine, and albumin, linked to cell targeting ligands such as asialoorosomucoid, insulin, galactose, lactose, or transferrin. Other delivery systems include the use of liposomes to encapsulate DNA comprising the gene under the control of a variety of tissue-specific or ubiquitously-active promoters. Further non-viral delivery suitable for use includes mechanical delivery systems such as the approach described in Woffendin et al (1994) Proc. Natl. Acad. Sci. USA 91(24):11581-11585. Moreover, the coding sequence and the product of expression of such can be delivered through deposition of photopolymerized hydrogel materials. Other conventional methods for gene delivery that can be used for delivery of the coding sequence include, for example, use of hand-held gene transfer particle gun, as described in US 5,149,655; use of ionizing radiation for activating transferred gene, as described in US 5,206,152 and WO92/11033

Exemplary liposome and polycationic gene delivery vehicles are those described in US 5,422,120 and 4,762,915; inWO 95/13796; WO94/23697; and WO91/14445; in EP-0524968; and in Stryer, Biochemistry, pages 236-240 (1975) W.H. Freeman, San Francisco; Szoka (1980) Biochem Biophys Acta 600:1; Bayer (1979) Biochem Biophys Acta 550:464; Rivnay (1987) Meth Enzymol 149:119; Wang (1987) Proc Natl Acad Sci 84:7851; Plant (1989) Anal Biochem 176:420.

A polynucleotide composition can comprises therapeutically effective amount of a gene therapy vehicle, as the term is defined above. For purposes of the present invention, an effective dose will be from about 0.01 mg/ kg to 50 mg/kg or 0.05 mg/kg to about 10 mg/kg of the DNA constructs in the individual to which it is administered.

*Delivery Methods*

Once formulated, the polynucleotide compositions of the invention can be administered (1) directly to the subject; (2) delivered *ex vivo,* to cells derived from the subject; or (3) *in vitro* for expression of recombinant proteins. The subjects to be treated can be mammals or birds. Also, human subjects can be treated.

Direct delivery of the compositions will generally be accomplished by injection, either subcutaneously, intraperitoneally, intravenously or intramuscularly or delivered to the interstitial space of a tissue. The compositions can also be administered into a lesion. Other modes of administration include oral and pulmonary administration, suppositories, and transdermal or transcutaneous applications (*eg.* see WO98/20734), needles, and gene guns or hyposprays. Dosage treatment may be a single dose schedule or a multiple dose schedule.

Methods for the *ex vivo* delivery and reimplantation of transformed cells into a subject are known in the art and described in *eg.* WO93/14778. Examples of cells useful in ex vivo applications include, for example, stem cells, particularly hematopoetic, lymph cells, macrophages, dendritic cells, or tumor cells.

Generally, delivery of nucleic acids for both *ex vivo* and *in vitro* applications can be accomplished by the following procedures, for example, dextran-mediated transfection, calcium phosphate precipitation, polybrene mediated transfection, protoplast fusion, electroporation, encapsulation of the polynucleotide(s) in liposomes, and direct microinjection of the DNA into nuclei, all well known in the art.

*Polynucleotide and polypeptide pharmaceutical compositions*

In addition to the pharmaceutically acceptable carriers and salts described above, the following additional agents can be used with polynucleotide and/or polypeptide compositions.

A.Polypeptides

One example are polypeptides which include, without limitation: asioloorosomucoid (ASOR); transferrin; asialoglycoproteins; antibodies; antibody fragments; ferritin; interleukins; interferons, granulocyte, macrophage colony stimulating factor (GM-CSF), granulocyte colony stimulating factor (G-CSF), macrophage colony stimulating factor (M-CSF), stem cell factor and erythropoietin. Viral antigens, such as envelope proteins, can also be used. Also, proteins from other invasive organisms, such as the 17 amino acid peptide from the circumsporozoite protein of plasmodium falciparum known as RII.

B.Hormones, Vitamins, *etc.*

Other groups that can be included are, for example: hormones, steroids, androgens, estrogens, thyroid hormone, or vitamins, folic acid.

C.Polyalkylenes, Polysaccharides, *etc.*

Also, polyalkylene glycol can be included with the desired polynucleotides/polypeptides. In a preferred embodiment, the polyalkylene glycol is polyethlylene glycol. In addition, mono-, di-, or polysaccarides can be included. In a preferred embodiment of this aspect, the polysaccharide is dextran or DEAE-dextran. Also, chitosan and poly(lactide-co-glycolide)

D.Lipids, and Liposomes

The desired polynucleotide/polypeptide can also be encapsulated in lipids or packaged in liposomes prior to delivery to the subject or to cells derived therefrom. Lipid encapsulation is generally accomplished using liposomes which are able to stably bind or entrap and retain nucleic acid. The ratio of condensed polynucleotide to lipid preparation can vary but will generally be around 1:1 (mg DNA:micromoles lipid), or more of lipid. For a review of the use of liposomes as carriers for delivery of nucleic acids, see, Hug and Sleight (1991) Biochim. Biophys. Acta. 1097:1-17; Straubinger (1983) Meth. Enzymol. 101:512-527.

Liposomal preparations for use in the present invention include cationic (positively charged), anionic (negatively charged) and neutral preparations. Cationic liposomes have been shown to mediate intracellular delivery of plasmid DNA (Felgner (1987) Proc. Natl. Acad. Sci. USA 84:7413-7416); mRNA (Malone (1989) Proc. Natl. Acad. Sci. USA 86: 6077-6081); and purified transcription factors (Debs (1990) J Biol. Chem. 265:10189-10192), in functional form.

Cationic liposomes are readily available. For example, N[1-2,3-dioleyloxy)propyl]-N,N,N-triethylammonium (DOTMA) liposomes are available under the trademark Lipofectin, from GIBCO BRL, Grand Island, NY. (See, also, Felgner *supra*). Other commercially available liposomes include transfectace (DDAB/DOPE) and DOTAP/DOPE (Boerhinger). Other cationic liposomes can be prepared from readily available materials using techniques well known in the art. See, *eg.* Szoka (1978) Proc. Natl. Acad Sci. USA 75:4194-4198; WO90/11092 for a description of the synthesis of DOTAP (1,2-bis(oleoyloxy)-3-(trimethylammonio)propane) liposomes.

Similarly, anionic and neutral liposomes are readily available, such as from Avanti Polar Lipids (Birmingham, AL), or can be easily prepared using readily available materials. Such materials include phosphatidyl choline, cholesterol, phosphatidyl ethanolamine, dioleoylphosphatidyl choline (DOPC), dioleoylphosphatidyl glycerol (DOPG), dioleoylphoshatidyl ethanolamine (DOPE), among others. These materials can also be mixed with the DOTMA and DOTAP starting materials in appropriate ratios. Methods for making liposomes using these materials are well known in the art.

The liposomes can comprise multilammelar vesicles (ML Vs), small unilamellar vesicles (SUVs), or large unilamellar vesicles (LUVs). The various liposome-nucleic acid complexes are prepared using methods known in the art. See *eg.* Straubinger (1983) Meth. Immunol. 101:512-527; Szoka (1978) Proc. Natl. Acad. Sci. USA 75:4194-4198; Papahadjopoulos (1975) Biochim. Biophys. Acta 394:483; Wilson (1979) Cell 17:77); Deamer & Bangham (1976) Biochim. Biophys. Acta 443:629; Ostro (1977) Biochem. Biophys. Res. Commun. 76:836; Fraley (1979) Proc. Natl. Acad. Sci. USA 76:3348); Enoch & Strittmatter (1979) Proc. Natl. Acad. Sci. USA 76:145; Fraley (1980) J. Biol. Chem. (1980) 255:10431; Szoka & Papahadjopoulos (1978) Proc. Natl. Acad. Sci. USA 75:145; and Schaefer-Ridder (1982) Science 215:166.

E.Lipoproteins

In addition, lipoproteins can be included with the polynucleotide/polypeptide to be delivered. Examples of lipoproteins to be utilized include: chylomicrons, HDL, IDL, LDL, and VLDL. Mutants, fragments, or fusions of these proteins can also be used. Also, modifications of naturally occurring lipoproteins can be used, such as acetylated LDL. These lipoproteins can target the delivery of polynucleotides to cells expressing lipoprotein receptors. Preferably, if lipoproteins are including with the polynucleotide to be delivered, no other targeting ligand is included in the composition.
Naturally occurring lipoproteins comprise a lipid and a protein portion. The protein portion are known as apoproteins. At the present, apoproteins A, B, C, D, and E have been isolated and identified. At least two of these contain several proteins, designated by Roman numerals, AI, AII, AIV; CI, CII, CIII.
A lipoprotein can comprise more than one apoprotein. For example, naturally occurring chylomicrons comprises of A, B, C, and E, over time these lipoproteins lose A and acquire C and E apoproteins. VLDL comprises A, B, C, and E apoproteins, LDL comprises apoprotein B; and HDL comprises apoproteins A, C, and E.
The amino acid of these apoproteins are known and are described in, for example, Breslow (1985) Annu Rev. Biochem 54:699; Law (1986) Adv. Exp Med. Biol. 151:162; Chen (1986) J Biol Chem 261:12918; Kane (1980) Proc Natl Acad Sci USA 77:2465; and Utermann (1984) Hum Genet 65:232.
Lipoproteins contain a variety of lipids including, triglycerides, cholesterol (free and esters), and phopholipids. The composition of the lipids varies in naturally occurring lipoproteins. For example, chylomicrons comprise mainly triglycerides. A more detailed description of the lipid content of naturally occurring lipoproteins can be found, for example, in Meth. Enzymol. 128 (1986). The composition of the lipids are chosen to aid in conformation of the apoprotein for receptor binding activity. The composition of lipids can also be chosen to facilitate hydrophobic interaction and association with the polynucleotide binding molecule.
Naturally occurring lipoproteins can be isolated from serum by ultracentrifugation, for instance.
Such methods are described in *Meth. Enzymol. (supra);* Pitas (1980) J Biochem. 255:5454-5460 and Mahey (1979) J Clin. Invest 64:743-750. Lipoproteins can also be produced by *in vitro* or recombinant methods by expression of the apoprotein genes in a desired host cell. See, for example, Atkinson (1986) Annu Rev Biophys Chem 15:403 and Radding (1958) Biochim Biophys Acta 30: 443. Lipoproteins can also be purchased from commercial suppliers, such as Biomedical Technologies, Inc., Stoughton, Massachusetts, USA. Further description of lipoproteins can be found in Zuckermann et al. PCT/US97/14465.

F.Polycationic Agents

Polycationic agents can be included, with or without lipoprotein, in a composition with the desired polynucleotide/polypeptide to be delivered.
Polycationic agents, typically, exhibit a net positive charge at physiological relevant pH and are capable of neutralizing the electrical charge of nucleic acids to facilitate delivery to a desired location. These agents have both in vitro, ex vivo, and in vivo applications. Polycationic agents can be used to deliver nucleic acids to a living subject either intramuscularly, subcutaneously, etc.
The following are examples of useful polypeptides as polycationic agents: polylysine, polyarginine, polyornithine, and protamine. Other examples include histones, protamines, human serum albumin, DNA binding proteins, non-histone chromosomal proteins, coat proteins from DNA viruses, such as (X174, transcriptional factors also contain domains that bind DNA and therefore may be useful as nucleic aid condensing agents. Briefly, transcriptional factors such as C/CEBP, c-jun, c-fos,
**[0182]** AP-1, AP-2, AP-3, CPF, Prot-1, Sp-1, Oct-1, Oct-2, CREP, and TFIID contain basic domains that bind DNA sequences.
Organic polycationic agents include: spermine, spermidine, and purtrescine.
The dimensions and of the physical properties of a polycationic agent can be extrapolated from the list above, to construct other polypeptide polycationic agents or to produce synthetic polycationic agents.
Synthetic polycationic agents which are useful include, for example, DEAE-dextran, polybrene.
Lipofectin™, and lipofectAMINE™ are monomers that form polycationic complexes when combined with polynucleotides/polypeptides.

*Immunodiagnostic Assays*

Neisserial antigens of the invention can be used in immunoassays to detect antibody levels (or, conversely, anti-Neisserial antibodies can be used to detect antigen levels). Immunoassays based on well defined, recombinant antigens can be developed to replace invasive diagnostics methods.

Antibodies to Neisserial proteins within biological samples, including for example, blood or serum samples, can be detected. Design of the immunoassays is subject to a great deal of variation, and a variety of these are known in the art. Protocols for the immunoassay may be based, for example, upon competition, or direct reaction, or sandwich type assays. Protocols may also, for example, use solid supports, or may be by immunoprecipitation. Most assays involve the use of labeled antibody or polypeptide; the labels may be, for example, fluorescent, chemiluminescent, radioactive, or dye molecules. Assays which amplify the signals from the probe are also known; examples of which are assays which utilize biotin and avidin, and enzyme-labeled and mediated immunoassays, such as ELISA assays.

Kits suitable for immunodiagnosis and containing the appropriate labeled reagents are constructed by packaging the appropriate materials, including the compositions of the invention, in suitable containers, along with the remaining reagents and materials (for example, suitable buffers, salt solutions, *etc. )* required for the conduct of the assay, as well as suitable set of assay instructions.

*Nucleic Acid Hybridisation*

"Hybridization" refers to the association of two nucleic acid sequences to one another by hydrogen bonding. Typically, one sequence will be fixed to a solid support and the other will be free in solution. Then, the two sequences will be placed in contact with one another under conditions that favor hydrogen bonding. Factors that affect this bonding include: the type and volume of solvent; reaction temperature; time of hybridization; agitation; agents to block the non-specific attachment of the liquid phase sequence to the solid support (Denhardt's reagent or BLOTTO); concentration of the sequences; use of compounds to increase the rate of association of sequences (dextran sulfate or polyethylene glycol); and the stringency of the washing conditions following hybridization. See Sambrook *et al. [supra]* Volume 2, chapter 9, pages 9.47 to 9.57.

"Stringency" refers to conditions in a hybridization reaction that favor association of very similar sequences over sequences that differ. For example, the combination of temperature and salt concentration should be chosen that is approximately 120 to 200°C below the calculated Tm of the hybrid under study. The temperature and salt conditions can often be determined empirically in preliminary experiments in which samples of genomic DNA immobilized on filters are hybridized to the sequence of interest and then washed under conditions of different stringencies. See Sambrook *et al.* at page 9.50.

Variables to consider when performing, for example, a Southern blot are (1) the complexity of the DNA being blotted and (2) the homology between the probe and the sequences being detected. The total amount of the fragment (s) to be studied can vary a magnitude of 10, from 0.1 to 1 μg for a plasmid or phage digest to $10^{-9}$ to $10^{-8}$ g for a single copy gene in a highly complex eukaryotic genome. For lower complexity polynucleotides, substantially shorter blotting, hybridization, and exposure times, a smaller amount of starting polynucleotides, and lower specific activity of probes can be used. For example, a single-copy yeast gene can be detected with an exposure time of only I hour starting with 1 μg of yeast DNA, blotting for two hours, and hybridizing for 4-8 hours with a probe of $10^8$ cpm/μg. For a single-copy mammalian gene a conservative approach would start with 10 μg of DNA, blot overnight, and hybridize overnight in the presence of 10% dextran sulfate using a probe of greater than $10^8$ cpm/μg, resulting in an exposure time of ~24 hours.

Several factors can affect the melting temperature (Tm) of a DNA-DNA hybrid between the probe and the fragment of interest, and consequently, the appropriate conditions for hybridization and washing. In many cases the probe is not 100% homologous to the fragment. Other commonly encountered variables include the length and total G+C content of the hybridizing sequences and the ionic strength and formamide content of the hybridization buffer. The effects of all of these factors can be approximated by a single equation:

$$\mathrm{Tm}= 81 + 16.6(\log_{10}\mathrm{Ci}) + 0.4[\%(\mathrm{G} + \mathrm{C})]-0.6(\%\mathrm{formamide}) - 600/n-1.5(\%\mathrm{mismatch}).$$

where Ci is the salt concentration (monovalent ions) and *n* is the length of the hybrid in base pairs (slightly modified from Meinkoth & Wahl (1984) Anal. Biochem. 138: 267-284).

In designing a hybridization experiment, some factors affecting nucleic acid hybridization can be conveniently altered. The temperature of the hybridization and washes and the salt concentration during the washes are the simplest to adjust. As the temperature of the hybridization increases *(ie.* stringency), it becomes less likely for hybridization to occur between strands that are nonhomologous, and as a result, background decreases. If the radiolabeled probe is not completely homologous with the immobilized fragment (as is frequently the case in gene family and interspecies hybridization experiments), the hybridization temperature must be reduced, and background will increase. The temperature of the washes affects the intensity of the hybridizing band and the degree of background in a similar manner. The stringency of the washes is also increased with decreasing salt concentrations.

In general, convenient hybridization temperatures in the presence of 50% formamide are 42°C for a probe with is 95% to 100% homologous to the target fragment, 37°C for 90% to 95% homology, and 32°C for 85% to 90% homology. For lower homologies, formamide content should be lowered and temperature adjusted accordingly, using the equation above. If the homology between the probe and the target fragment are not known, the simplest approach is to start with both hybridization and wash conditions which are nonstringent. If non-specific bands or high background are observed after autoradiography, the filter can be washed at high stringency and reexposed. If the time required for exposure makes this approach impractical, several hybridization and/or washing stringencies should be tested in parallel.

*Nucleic Acid Probe Assays*

Methods such as PCR, branched DNA probe assays, or blotting techniques utilizing nucleic acid probes according to the invention can determine the presence of cDNA or mRNA. A probe is said to "hybridize" with a sequence of the invention if it can form a duplex or double stranded complex, which is stable enough to be detected.

The nucleic acid probes will hybridize to the Neisserial nucleotide sequences of the invention (including both sense and antisense strands). Though many different nucleotide sequences will encode the amino acid sequence, the native Neisserial sequence is preferred because it is the actual sequence present in cells. mRNA represents a coding sequence and so a probe should be complementary to the coding sequence; single-stranded cDNA is complementary to mRNA, and so a cDNA probe should be complementary to the non-coding sequence.

The probe sequence need not be identical to the Neisserial sequence (or its complement) - some variation in the sequence and length can lead to increased assay sensitivity if the nucleic acid probe can form a duplex with target nucleotides, which can be detected. Also, the nucleic acid probe can include additional nucleotides to stabilize the formed duplex. Additional Neisserial sequence may also be helpful as a label to detect the formed duplex. For example, a non-complementary nucleotide sequence may be attached to the 5' end of the probe, with the remainder of the probe sequence being complementary to a Neisserial sequence. Alternatively, non-complementary bases or longer sequences can be interspersed into the probe, provided that the probe sequence has sufficient complementarity with the a Neisserial sequence in order to hybridize therewith and thereby form a duplex which can be detected.

The exact length and sequence of the probe will depend on the hybridization conditions, such as temperature, salt condition and the like. For example, for diagnostic applications, depending on the complexity of the analyte sequence, the nucleic acid probe typically contains at least 10-20 nucleotides, preferably 15-25, and more preferably at least 30 nucleotides, although it may be shorter than this. Short primers generally require cooler temperatures to form sufficiently stable hybrid complexes with the template.

Probes may be produced by synthetic procedures, such as the triester method of Matteucci et al. [J. Am. Chem. Soc. (1981) 103:3185], or according to Urdea et at. [Proc. Natl. Acad. Sci. USA (1983) 80: 7461], or using commercially available automated oligonucleotide synthesizers.

The chemical nature of the probe can be selected according to preference. For certain applications, DNA or RNA are appropriate. For other applications, modifications may be incorporated eg. backbone modifications, such as phosphorothioates or methylphosphonates, can be used to increase *in vivo* half-life, alter RNA affinity, increase nuclease resistance *etc. [eg.* see Agrawal & Iyer (1995) Curr Opin Biotechnol 6:12-19; Agrawal (1996) TIBTECH 14:376-387]; analogues such as peptide nucleic acids may also be used *[eg.* see Corey (1997) TIBTECH 15:224-229; Buchardt et al. (1993) TIBTECH 11:384-386].

Alternatively, the polymerase chain reaction (PCR) is another well-known means for detecting small amounts of target nucleic acids. The assay is described in: Mullis et al. [Meth. Enzymol. (1987) 155: 335-350]; US patents 4,683,195 and 4,683,202. Two "primer" nucleotides hybridize with the target nucleic acids and are used to prime the reaction. The primers can comprise sequence that does not hybridize to the sequence of the amplification target (or its complement) to aid with duplex stability or, for example, to incorporate a convenient restriction site. Typically, such sequence will flank the desired Neisserial sequence.

A thermostable polymerase creates copies of target nucleic acids from the primers using the original target nucleic acids as a template. After a threshold amount of target nucleic acids are generated by the polymerase, they can be detected by more traditional methods, such as Southern blots. When using the Southern blot method, the labelled probe will hybridize to the Neisserial sequence (or its complement).

Also, mRNA or cDNA can be detected by traditional blotting techniques described in Sambrook *et al* [*supra*]. mRNA, or cDNA generated from mRNA using a polymerase enzyme, can be purified and separated using gel electrophoresis. The nucleic acids on the gel are then blotted onto a solid support, such as nitrocellulose. The solid support is exposed to a labelled probe and then washed to remove any unhybridized probe. Next, the duplexes containing the labeled probe are detected. Typically, the probe is labelled with a radioactive moiety.

## BRIEF DESCRIPTION OF THE DRAWINGS

**Figures 1-20** show biochemical data obtained in the Examples, and also sequence analysis, for ORFs 37, 5, 2, 15, 22, 28, 32, 4, 61, 76, 89, 97, 106, 138, 23, 25, 27, 79, 85 and 132. M1 and M2 are molecular weight markers. Arrows indicate the position of the main recombinant product or, in Western blots, the position of the main *N.meningitidis* immunoreactive band. TP indicates *N.meningitidis* total protein extract; OMV indicates *N.meningitidis* outer membrane vesicle preparation. In bactericidal assay results: a diamond (♦) shows preimmune data; a triangle (▲) shows GST control data; a circle (•) shows data with recombinant *N.meningitidis* protein. Computer analyses show a hydrophilicity plot (upper), an antigenic index plot (middle), and an AMPHI analysis (lower). The AMPHI program has been used to predict T-cell epitopes [Gao et al. (1989) J. Immunol. 143:3007; Roberts et al. (1996) AIDS Res Hum Retrovir 12:593; Quakyi et al. (1992) Scand J Immunol suppl.11:9) and is available in the Protean package of DNASTAR, Inc. (1228 South Park Street, Madison, Wisconsin 53715 USA).

## EXAMPLES

The examples describe nucleic acid sequences which have been identified in *N.meningitidis,* along with their putative translation products, and also those of *N.gonorrhoeae.* Not all of the nucleic acid sequences are complete *ie.* they encode less than the full-length wild-type protein.
The examples are generally in the following format:

- a nucleotide sequence which has been identified in *N. meningitidis* (strain B)
- the putative translation product of this sequence
- a computer analysis of the translation product based on database comparisons
- corresponding gene and protein sequences identified in *N.meningitidis* (strain A) and in *N.gonorrhoeae*
- a description of the characteristics of the proteins which indicates that they might be suitably antigenic
- results of biochemical analysis (expression, purification, ELISA, FACS *etc.)*

The examples typically include details of sequence identity between species and strains. Proteins that are similar in sequence are generally similar in both structure and function, and the sequence identity often indicates a common evolutionary origin. Comparison with sequences of proteins of known function is widely used as a guide for the assignment of putative protein function to a new sequence and has proved particularly useful in whole-genome analyses.
Sequence comparisons were performed at NCBI (http://www.ncbi.nlm.nih.gov) using the algorithms BLAST, BLAST2, BLASTn, BLASTp, tBLASTn, BLASTx, & tBLASTx [*eg.* see also Altschul et al. (1997) Gapped BLAST and PSI-BLAST: a new generation of protein database search programs. Nucleic Acids Research 25:2289-3402]. Searches were performed against the following databases: non-redundant GenBank+EMBL+DDBJ+PDB sequences and non-redundant
GenBank CDS translations+PDB+SwissProt+SPupdate+PIR sequences.
To compare Meningococcal and Gonococcal sequences, the tBLASTx algorithm was used, as implemented at http://www.genome.ou.edu/gono_blast.html. The FASTA algorithm was also used to compare the ORFs (from GCG Wisconsin Package, version 9.0).
Dots within nucleotide sequences (eg. position 495 in SEQ ID 11) represent nucleotides which have been arbitrarily introduced in order to maintain a reading frame. In the same way, double-underlined nucleotides were removed. Lower case letters (*eg.* position 496 in SEQ ID 11) represent ambiguities which arose during alignment of independent sequencing reactions (some of the nucleotide sequences in the examples are derived from combining the results of two or more experiments).
Nucleotide sequences were scanned in all six reading frames to predict the presence of hydrophobic domains using an algorithm based on the statistical studies of Esposti et al. [Critical evaluation of the hydropathy of membrane proteins (1990) Eur J Biochem 190:207-219]. These domains represent potential transmembrane regions or hydrophobic leader sequences.
Open reading frames were predicted from fragmented nucleotide sequences using the program ORFFINDER (NCBI).
Underlined amino acid sequences indicate possible transmembrane domains or leader sequences in the ORFs, as predicted by the PSORT algorithm (http://www.psort.nibb.ac.jp). Functional domains were also predicted using the MOTIFS program (GCG Wisconsin & PROSITE).

Various tests can be used to assess the *in vivo* immunogencity of the proteins identified in the examples. For example, the proteins can be expressed recombinantly and used to screen patient sera by immunoblot. A positive reaction between the protein and patient serum indicates that the patient has previously mounted an immune response to the protein in question ie. the protein is an immunogen. This method can also be used to identify immunodominant proteins.

The recombinant protein can also be conveniently used to prepare antibodies eg. in a mouse. These can be used for direct confirmation that a protein is located on the cell-surface. Labelled antibody (*eg.* fluorescent labelling for FACS) can be incubated with intact bacteria and the presence of label on the bacterial surface confirms the location of the protein.

In particular, the following methods (A) to (S) were used to express, purify and biochemically characterise the proteins of the invention:

### A) Chromosomal DNA preparation

*N. meningitidis* strain 2996 was grown to exponential phase in 100ml of GC medium, harvested by centrifugation, and resuspended in 5ml buffer (20% Sucrose, 50mM Tris-HCl, 50mM EDTA, pH8). After 10 minutes incubation on ice, the bacteria were lysed by adding 10ml lysis solution (50mM NaCl, 1% Na-Sarkosyl, 50μg/ml Proteinase K), and the suspension was incubated at 37°C for 2 hours. Two phenol extractions (equilibrated to pH 8) and one $ChCl_3$/isoamylalcohol (24:1) extraction were performed. DNA was precipitated by addition of 0.3M sodium acetate and 2 volumes ethanol, and was collected by centrifugation. The pellet was washed once with 70% ethanol and redissolved in 4ml buffer (10mM Tris-HCl, 1mM EDTA, pH 8). The DNA concentration was measured by reading the OD at 260 nm.

### B) Oligonucleotide design

Synthetic oligonucleotide primers were designed on the basis of the coding sequence of each ORF, using (a) the meningococcus B sequence when available, or (b) the gonococcus/meningococcus A sequence, adapted to the codon preference usage of meningococcus as necessary. Any predicted signal peptides were omitted, by deducing the 5'-end amplification primer sequence immediately downstream from the predicted leader sequence.

For most ORFs, the 5' primers included two restriction enzyme recognition sites (*BamH*I-*Nde*I, *BamH*I-*Nhe*I, or *EcoR*I-*Nhe*I, depending on the gene's own restriction pattern); the 3' primers included a *Xho*I restriction site. This procedure was established in order to direct the cloning of each amplification product (corresponding to each ORF) into two different expression systems: pGEX-KG (using either *BamH*I-*Xho*I or *EcoR*I-*Xho*I), and pET21b+ (using either *Nde*I-*Xho*I or *Nhe*I-*Xho*I).

| | | |
|---|---|---|
| 5'-end primer tail: | CGCGGATCCCATATG | (*BamH*I-*Nde*I) |
| | CGCGGATCCGCTAGC | (*BamH*I-*Nhe*I) |
| | CCGGAATTCTAGCTAGC | (*EcoR*I-*Nhe*I) |
| 3'-end primer tail: | CCCGCTCGAG | (*Xho*I) |

For ORFs 5, 15, 17, 19, 20, 22, 27, 28, 65 & 89, two different amplifications were performed to clone each ORF in the two expression systems. Two different 5' primers were used for each ORF; the same 3' *Xho*I primer was used as before:

| | | |
|---|---|---|
| 5'-end primer tail: | GGAATTCCATATGGCCATGG | (*Nde*I) |
| 5'-end primer tail: | CGGGATCC | (*BamH*I) |

ORF 76 was cloned in the pTRC expression vector and expressed as an amino-terminus His-tag fusion. In this particular case, the predicted signal peptide was included in the final product. *Nhe*I*BamH*I restriction sites were incorporated using primers:

| | | |
|---|---|---|
| 5'-end primer tail: | GATCAGCTAGCCATATG | (*Nhe*I) |
| 3'-end primer tail: | CGGGATCC | (*BamH*I) |

As well as containing the restriction enzyme recognition sequences, the primers included nucleotides which hybridizeed to the sequence to be amplified. The number of hybridizing nucleotides depended on the melting temperature of the whole primer, and was determined for each primer using the formulae:

$$T_m = 4\,(G{+}C) + 2\,(A{+}T) \qquad \text{(tail excluded)}$$

$$T_m = 64.9 + 0.41\,(\%\,GC) - 600/N \qquad \text{(whole primer)}$$

The average melting temperature of the selected oligos were 65-70°C for the whole oligo and 50-55°C for the hybridising region alone.

**Table I** (page 485) shows the forward and reverse primers used for each amplification. In certain cases, it will be noted that the sequence of the primer does not exactly match the sequence in the ORF. When initial amplifications were performed, the complete 5' and/or 3' sequence was not known for some meningococcal ORFs, although the corresponding sequences had been identified in gonococcus. For amplification, the gonococcal sequences could thus be used as the basis for primer design, altered to take account of codon preference. In particular, the following codons were changed: ATA→ATT; TCG→TCT; CAG→CAA; AAG→AAA; GAG→GAA; CGA→CGC; CGG→CGC; GGG→GGC. Italicised nucleotides in Table I indicate such a change. It will be appreciated that, once the complete sequence has been identified, this approach is generally no longer necessary.

Oligos were synthesized by a Perkin Elmer 394 DNA/RNA Synthesizer, eluted from the columns in 2ml $NH_4OH$, and deprotected by 5 hours incubation at 56°C. The oligos were precipitated by addition of 0.3M Na-Acetate and 2 volumes ethanol. The samples were then centrifuged and the pellets resuspended in either 100μl or 1ml of water. $OD_{260}$ was determined using a Perkin Elmer Lambda Bio spectophotometer and the concentration was determined and adjusted to 2-10pmol/μl.

### C) Amplification

The standard PCR protocol was as follows: 50-200ng of genomic DNA were used as a template in the presence of 20-40μM of each oligo, 400-800μM dNTPs solution, 1x PCR buffer (including 1.5mM $MgCl_2$), 2.5 units *TaqI* DNA polymerase (using Perkin-Elmer AmpliTaQ, GIBCO Platinum, Pwo DNA polymerase, or Tahara Shuzo Taq polymerase).

In some cases, PCR was optimsed by the addition of 10μl DMSO or 50μl 2M betaine.

After a hot start (adding the polymerase during a preliminary 3 minute incubation of the whole mix at 95°C), each sample underwent a double-step amplification: the first 5 cycles were performed using as the hybridization temperature the one of the oligos excluding the restriction enzymes tail, followed by 30 cycles performed according to the hybridization temperature of the whole length oligos. The cycles were followed by a final 10 minute extension step at 72°C.

The standard cycles were as follows:

| | Denaturation | Hybridisation | Elongation |
|---|---|---|---|
| First 5 cycles | 30 seconds 95°C | 30 seconds 50-55°C | 30-60 seconds 72°C |
| Last 30 cycles | 30 seconds 95°C | 30 seconds 65-70°C | 30-60 seconds 72°C |

The elongation time varied according to the length of the ORF to be amplified.

The amplifications were performed using either a 9600 or a 2400 Perkin Elmer GeneAmp PCR System. To check the results, 1/10 of the amplification volume was loaded onto a 1-1.5% agarose gel and the size of each amplified fragment compared with a DNA molecular weight marker.

The amplified DNA was either loaded directly on a 1% agarose gel or first precipitated with ethanol and resuspended in a suitable volume to be loaded on a 1% agarose gel. The DNA fragment corresponding to the right size band was then eluted and purified from gel, using the Qiagen Gel Extraction Kit, following the instructions of the manufacturer. The final volume of the DNA fragment was 30μl or 50μl of either water or 10mM Tris, pH 8.5.

### D) Digestion of PCR fragments

The purified DNA corresponding to the amplified fragment was split into 2 aliquots and double-digested with:

- *NdeI*/*XhoI* or *NheI*/*XhoI* for cloning into pET-21b+ and further expression of the protein as a C-terminus His-tag fusion

- *BamHI/XhoI* or *EcoRI/XhoI* for cloning into pGEX-KG and further expression of the protein as N-terminus GST fusion.
- For ORF 76, *Nhe*I/*BamH*I for cloning into pTRC-HisA vector and further expression of the protein as N-terminus His-tag fusion.
- *EcoRI/PstI, EcoRI/SalI, SalI/PstI* for cloning into pGex-His and further expression of the protein as N-terminus His-tag fusion

Each purified DNA fragment was incubated (37°C for 3 hours to overnight) with 20 units of each restriction enzyme (New England Biolabs) in a either 30 or 40μl final volume in the presence of the appropriate buffer. The digestion product was then purified using the QIAquick PCR purification kit, following the manufacturer's instructions, and eluted in a final volume of 30 or 50μl of either water or 10mM Tris-HCl, pH 8.5. The final DNA concentration was determined by 1% agarose gel electrophoresis in the presence of titrated molecular weight marker.

**E) Digestion of the cloning vectors (pET22B, pGEX-KG, pTRC-His A, and pGex-His)**

10μg plasmid was double-digested with 50 units of each restriction enzyme in 200μl reaction volume in the presence of appropriate buffer by overnight incubation at 37°C. After loading the whole digestion on a 1 % agarose gel, the band corresponding to the digested vector was purified from the gel using the Qiagen QIAquick Gel Extraction Kit and the DNA was eluted in 50μl of 10mM Tris-HCl, pH 8.5. The DNA concentration was evaluated by measuring $OD_{260}$ of the sample, and adjusted to 50μg/μl. 1μl of plasmid was used for each cloning procedure.
The vector pGEX-His is a modified pGEX-2T vector carrying a region encoding six histidine residues upstream to the thrombin cleavage site and containing the multiple cloning site of the vector pTRC99 (Pharmacia).

**F) Cloning**

The fragments corresponding to each ORF, previously digested and purified, were ligated in both pET22b and pGEX-KG. In a final volume of 20μl, a molar ratio of 3:1 fragment/vector was ligated using 0.5μl of NEB T4 DNA ligase (400 units/μl), in the presence of the buffer supplied by the manufacturer. The reaction was incubated at room temperature for 3 hours. In some experiments, ligation was performed using the Boheringer "Rapid Ligation Kit", following the manufacturer's instructions.
In order to introduce the recombinant plasmid in a suitable strain, 100μl *E. coli* DH5 competent cells were incubated with the ligase reaction solution for 40 minutes on ice, then at 37°C for 3 minutes, then, after adding 800μl LB broth, again at 37°C for 20 minutes. The cells were then centrifuged at maximum speed in an Eppendorf microfuge and resuspended in approximately 200μl of the supernatant. The suspension was then plated on LB ampicillin (100mg/ml).
The screening of the recombinant clones was performed by growing 5 randomly-chosen colonies overnight at 37°C in either 2ml (pGEX or pTC clones) or 5ml (pET clones) LB broth + 100μg/ml ampicillin. The cells were then pelletted and the DNA extracted using the Qiagen QIAprep Spin Miniprep Kit, following the manufacturer's instructions, to a final volume of 30μl. 5μl of each individual miniprep (approximately 1 g) were digested with either *Nde*I/*Xho*I or *BamH*I/*Xho*I and the whole digestion loaded onto a 1-1.5% agarose gel (depending on the expected insert size), in parallel with the molecular weight marker (1Kb DNA Ladder, GIBCO). The screening of the positive clones was made on the base of the correct insert size.
For the cloning of ORFs 110, 111, 113, 115, 119, 122, 125 & 130, the double-digested PCR product was ligated into double-digested vector using *EcoR*I-*Pst*I cloning sites or, for ORFs 115 & 127, *EcoR*I-*Sal*I or, for ORF 122, *Sal*I-*Pst*I. After cloning, the recombinant plasmids were introduced in the *E.coli* host W3110. Individual clones were grown overnight at 37°C in L-broth with 50μl/ml ampicillin.

**G) Expression**

Each ORF cloned into the expression vector was transformed into the strain suitable for expression of the recombinant protein product. 1μl of each construct was used to transform 30μl of *E. coli*
BL21 (pGEX vector), *E.coli* TOP 10 (pTRC vector) or *E.coli* BL21-DE3 (pET vector), as described above. In the case of the pGEX-His vector, the same *E.coli* strain (W3110) was used for initial cloning and expression. Single recombinant colonies were inoculated into 2ml LB+Amp (100μg/ml), incubated at 37°C overnight, then diluted 1:30 in 20ml of LB+Amp (100μg/ml) in 100ml flasks, making sure that the $OD_{600}$ ranged between 0.1 and 0.15. The flasks were incubated at 30°C into gyratory water bath shakers until OD indicated exponential growth suitable for induction of expression (0.4-0.8 OD for pET and pTRC vectors; 0.8-1 OD for pGEX and pGEX-His vectors). For the pET, pTRC and pGEX-His vectors, the protein expression was induced by addition of 1mM IPTG, whereas in the case of pGEX system the final concentration of IPTG was 0.2mM. After 3 hours incubation at 30°C, the final concentration of the sample was checked by OD. In order to check expression, 1ml of each sample was removed, centrifuged in a microfuge, the pellet resuspended in PBS, and analysed by 12% SDS-PAGE with Coomassie Blue staining. The whole sample was centrifuged at 6000*g* and the pellet resuspended in PBS for further use.

**H) GST-fusion proteins large-scale purification.**

A single colony was grown overnight at 37°C on LB+Amp agar plate. The bacteria were inoculated into 20ml of LB+Amp liquid colture in a water bath shaker and grown overnight. Bacteria were diluted 1:30 into 600ml of fresh medium and allowed to grow at the optimal temperature (20-37°C) to $OD_{550}$ 0.8-1. Protein expression was induced with 0.2mM IPTG followed by three hours incubation. The culture was centrifuged at 8000rpm at 4°C. The supernatant was discarded and the bacterial pellet was resuspended in 7.5ml cold PBS. The cells were disrupted by sonication on ice for 30 sec at 40W using a Branson sonifier B-15, frozen and thawed twice and centrifuged again.

The supernatant was collected and mixed with 150μl Glutatione-Sepharose 4B resin (Pharmacia) (previously washed with PBS) and incubated at room temperature for 30 minutes. The sample was centrifuged at 700g for 5 minutes at 4°C. The resin was washed twice with 10ml cold PBS for 10 minutes, resuspended in 1ml cold PBS, and loaded on a disposable column. The resin was washed twice with 2ml cold PBS until the flow-through reached $OD_{280}$ of 0.02-0.06. The GST-fusion protein was eluted by addition of 700μl cold Glutathione elution buffer (10mM reduced glutathione, 50mM Tris-HCl) and fractions collected until the $OD_{280}$ was 0.1. 21 μl of each fraction were loaded on a 12% SDS gel using either Biorad SDS-PAGE Molecular weight standard broad range (M1) (200, 116.25, 97.4, 66.2, 45, 31, 21.5, 14.4, 6.5 kDa) or Amersham Rainbow Marker (M2) (220, 66, 46, 30, 21.5, 14.3 kDa) as standards. As the MW of GST is 26kDa, this value must be added to the MW of each GST-fusion protein.

**I) His-fusion solubility analysis (ORFs 111-129)**

To analyse the solubility of the His-fusion expression products, pellets of 3ml cultures were resuspended in buffer M1 [500μl PBS pH 7.2]. 25μl lysozyme (10mg/ml) was added and the bacteria were incubated for 15 min at 4°C. The pellets were sonicated for 30 sec at 40W using a Branson sonifier B-15, frozen and thawed twice and then separated again into pellet and supernatant by a centrifugation step. The supernatant was collected and the pellet was resuspended in buffer M2 [8M urea, 0.5M NaCl, 20mM imidazole and O.1M $NaH_2$ $PO_4$] and incubated for 3 to 4 hours at 4°C. After centrifugation, the supernatant was collected and the pellet was resuspended in buffer M3 [6M guanidinium-HCl, 0.5M NaCl, 20mM imidazole and 0.1M $NaH_2PO_4$] overnight at 4°C. The supernatants from all steps were analysed by SDS-PAGE.

The proteins expressed from ORFs 113, 119 and 120 were found to be soluble in PBS, whereas ORFs 111, 122, 126 and 129 need urea and ORFs 125 and 127 need guanidium-HCl for their solubilization.

**J) His-fusion large-scale purification.**

A single colony was grown overnight at 37°C on a LB + Amp agar plate. The bacteria were inoculated into 20ml of LB+Amp liquid culture and incubated overnight in a water bath shaker. Bacteria were diluted 1:30 into 600ml fresh medium and allowed to grow at the optimal temperature (20-37°C) to $OD_{550}$ 0.6-0.8. Protein expression was induced by addition of 1mM IPTG and the culture further incubated for three hours. The culture was centrifuged at 8000rpm at 4°C, the supernatant was discarded and the bacterial pellet was resuspended in 7.5ml of either (i) cold buffer A (300mM NaCl, 50mM phosphate buffer, 10mM imidazole, pH 8) for soluble proteins or (ii) buffer B (urea 8M, 10mM Tris-HCl, 100mM phosphate buffer, pH 8.8) for insoluble proteins.

The cells were disrupted by sonication on ice for 30 sec at 40W using a Branson sonifier B-15, frozen and thawed two times and centrifuged again.

For insoluble proteins, the supernatant was stored at -20°C, while the pellets were resuspended in 2ml buffer C (6M guanidine hydrochloride, 100mM phosphate buffer, 10mM Tris-HCl, pH 7.5) and treated in a homogenizer for 10 cycles. The product was centrifuged at 13000rpm for 40 minutes.

Supernatants were collected and mixed with 150μl $Ni^{2+}$-resin (Pharmacia) (previously washed with either buffer A or buffer B, as appropriate) and incubated at room temperature with gentle agitation for 30 minutes. The sample was centrifuged at 700g for 5 minutes at 4°C. The resin was washed twice with 10ml buffer A or B for 10 minutes, resuspended in 1ml buffer A or B and loaded on a disposable column. The resin was washed at either (i) 4°C with 2ml cold buffer A or (ii) room temperature with 2ml buffer B, until the flow-through reached $OD_{280}$ of 0.02-0.06.

The resin was washed with either (i) 2ml cold 20mM imidazole buffer (300mM NaCl, 50mM phosphate buffer, 20mM imidazole, pH 8) or (ii) buffer D (urea 8M, 10mM Tris-HCl, 100mM phosphate buffer, pH 6.3) until the flow-through reached the $O.D_{280}$ of 0.02-0.06. The His-fusion protein was eluted by addition of 700μl of either (i) cold elution buffer A (300mM NaCl, 50mM phosphate buffer, 250mM imidazole, pH 8) or (ii) elution buffer B (urea 8M, 10mM Tris-HCl, 100mM phosphate buffer, pH 4.5) and fractions collected until the $O.D_{280}$ was 0.1. 21μl of each fraction were loaded on a 12% SDS gel.

**K) His-fusion proteins renaturation**

10% glycerol was added to the denatured proteins. The proteins were then diluted to 20μg/ml using dialysis buffer I (10% glycerol, 0.5M arginine, 50mM phosphate buffer, 5mM reduced glutathione, 0.5mM oxidised glutathione, 2M urea, pH 8.8) and dialysed against the same buffer at 4°C for 12-14 hours. The protein was further dialysed against dialysis buffer II (10% glycerol, 0.5M arginine, 50mM phosphate buffer, 5mM reduced glutathione, 0.5mM oxidised glutathione, pH 8.8) for 12-14 hours at 4°C. Protein concentration was evaluated using the formula:

$$\text{Protein (mg/ml)} = (1.55 \times OD_{280}) - (0.76 \times OD_{260})$$

**L) His-fusion large-scale purification (ORFs 111-129)**

500ml of bacterial cultures were induced and the fusion proteins were obtained soluble in buffer M1, M2 or M3 using the procedure described above. The crude extract of the bacteria was loaded onto a Ni-NTA superflow column (Quiagen) equilibrated with buffer M1, M2 or M3 depending on the solubilization buffer of the fusion proteins. Unbound material was eluted by washing the column with the same buffer. The specific protein was eluted with the corresponding buffer containing 500mM imidazole and dialysed against the corresponding buffer without imidazole. After each run the columns were sanitized by washing with at least two column volumes of 0.5 M sodium hydroxide and reequilibrated before the next use.

**M) Mice immunisations**

20μg of each purified protein were used to immunise mice intraperitoneally. In the case of ORFs 2, 4, 15, 22, 27, 28, 37, 76, 89 and 97, Balb-C mice were immunised with $Al(OH)_3$ as adjuvant on days 1, 21 and 42, and immune response was monitored in samples taken on day 56. For ORFs 44, 106 and 132, CD1 mice were immunised using the same protocol. For ORFs 25 and 40, CD1 mice were immunised using Freund's adjuvant, rather than $AL(OH)_3$, and the same immunisation protocol was used, except that the immune response was measured on day 42, rather than 56. Similarly, for ORFs 23, 32, 38 and 79, CD1 mice were immunised with Freund's adjuvant, but the immune response was measured on day 49.

**N) ELISA assay (sera analysis)**

The acapsulated MenB M7 strain was plated on chocolate agar plates and incubated overnight at 37°C. Bacterial colonies were collected from the agar plates using a sterile dracon swab and inoculated into 7ml of Mueller-Hinton Broth (Difco) containing 0.25% Glucose. Bacterial growth was monitored every 30 minutes by following $OD_{620}$. The bacteria were let to grow until the OD reached the value of 0.3-0.4. The culture was centrifuged for 10 minutes at 10000rpm. The supernatant was discarded and bacteria were washed once with PBS, resuspended in PBS containing 0.025% formaldehyde, and incubated for 2 hours at room temperature and then overnight at 4°C with stirring. 100μl bacterial cells were added to each well of a 96 well Greiner plate and incubated overnight at 4°C. The wells were then washed three times with PBT washing buffer (0.1 % Tween-20 in PBS). 200μl of saturation buffer (2.7% Polyvinylpyrrolidone 10 in water) was added to each well and the plates incubated for 2 hours at 37°C. Wells were washed three times with PBT. 200μl of diluted sera (Dilution buffer: 1 % BSA, 0.1% Tween-20, 0.1 % $NaN_3$ in PBS) were added to each well and the plates incubated for 90 minutes at 37°C. Wells were washed three times with PBT. 100μl of HRP-conjugated rabbit anti-mouse (Dako) serum diluted 1:2000 in dilution buffer were added to each well and the plates were incubated for 90 minutes at 37°C. Wells were washed three times with PBT buffer. 100μl of substrate buffer for HRP (25ml of citrate buffer pH5, 10mg of O-phenildiamine and 10μl of $H_2O$) were added to each well and the plates were left at room temperature for 20 minutes. 100μl $H_2SO_4$ was added to each well and $OD_{490}$ was followed. The ELISA was considered positive when $OD_{490}$ was 2.5 times the respective pre-immune sera.

**O) FACScan bacteria Binding Assay procedure.**

The acapsulated MenB M7 strain was plated on chocolate agar plates and incubated overnight at 37°C. Bacterial colonies were collected from the agar plates using a sterile dracon swab and inoculated into 4 tubes containing 8ml each Mueller-Hinton Broth (Difco) containing 0.25% glucose. Bacterial growth was monitored every 30 minutes by following $OD_{620}$. The bacteria were let to grow until the OD reached the value of 0.35-0.5. The culture was centrifuged for 10 minutes at 4000rpm. The supernatant was discarded and the pellet was resuspended in blocking buffer (1% BSA, 0.4% $NaN_3$) and centrifuged for 5 minutes at 4000rpm. Cells were resuspended in blocking buffer to reach $OD_{620}$ of 0.07. 100μl bacterial cells were added to each well of a Costar 96 well plate. 100μl of diluted (1:200) sera (in blocking buffer) were added to each well and plates incubated for 2 hours at 4°C. Cells were centrifuged for 5 minutes at 4000rpm, the supernatant aspirated and cells washed by addition of 200μl/well of blocking buffer in each well. 100μl of R-Phicoerytrin conjugated $F(ab)_2$ goat anti-mouse, diluted 1:100, was added to each well and plates incubated for 1 hour at 4°C. Cells were spun down by centrifugation at 4000rpm for 5 minutes and washed by addition of 200μl/well of blocking buffer. The supernatant was aspirated and cells resuspended in 200μl/well of PBS, 0.25% formaldehyde. Samples were transferred to FACScan tubes and read. The condition for FACScan setting were: FL1 on, FL2 and FL3 off; FSC-H threshold:92; FSC PMT Voltage: E 02; SSC PMT: 474; Amp. Gains 7.1; FL-2 PMT: 539; compensation values: 0.

**P) OMV preparations**

Bacteria were grown overnight on 5 GC plates, harvested with a loop and resuspended in 10 ml 20mM Tris-HCl. Heat inactivation was performed at 56°C for 30 minutes and the bacteria disrupted by sonication for 10 minutes on ice (50% duty cycle, 50% output). Unbroken cells were removed by centrifugation at 5000g for 10 minutes and the total cell envelope fraction recovered by centrifugation at 50000g at 4°C for 75 minutes. To extract cytoplasmic membrane proteins from the crude outer membranes, the whole fraction was resuspended in 2% sarkosyl (Sigma) and incubated at room temperature for 20 minutes. The suspension was centrifuged at 10000g for 10 minutes to remove aggregates, and the supernatant further ultracentrifuged at 50000g for 75 minutes to pellet the outer membranes. The outer membranes were resuspended in 10mM Tris-HCl, pH8 and the protein concentration measured by the Bio-Rad Protein assay, using BSA as a standard.

**Q) Whole Extracts preparation**

Bacteria were grown overnight on a GC plate, harvested with a loop and resuspended in 1ml of 20mM Tris-HCl. Heat inactivation was performed at 56°C for 30 minutes.

**R) Western blotting**

Purified proteins (500ng/lane), outer membrane vesicles (5μg) and total cell extracts (25μg) derived from MenB strain 2996 were loaded on 15% SDS-PAGE and transferred to a nitrocellulose membrane. The transfer was performed for 2 hours at 150mA at 4°C, in transferring buffer (0.3 % Tris base, 1.44 % glycine, 20% methanol). The membrane was saturated by overnight incubation at 4°C in saturation buffer (10% skimmed milk, 0.1% Triton X100 in PBS). The membrane was washed twice with washing buffer (3% skimmed milk, 0.1% Triton X100 in PBS) and incubated for 2 hours at 37°C with mice sera diluted 1:200 in washing buffer. The membrane was washed twice and incubated for 90 minutes with a 1:2000 dilution of horseradish peroxidase labelled anti-mouse Ig. The membrane was washed twice with 0.1% Triton X100 in PBS and developed with the Opti-4CN Substrate Kit (Bio-Rad). The reaction was stopped by adding water.

**S) Bactericidal assay**

MC58 strain was grown overnight at 37°C on chocolate agar plates. 5-7 colonies were collected and used to inoculate 7ml Mueller-Hinton broth. The suspension was incubated at 37°C on a nutator and let to grow until $OD_{620}$ was 0.5-0.8. The culture was aliquoted into sterile 1.5ml Eppendorf tubes and centrifuged for 20 minutes at maximum speed in a microfuge. The pellet was washed once in Gey's buffer (Gibco) and resuspended in the same buffer to an $OD_{620}$ of 0.5, diluted 1:20000 in Gey's buffer and stored at 25°C.

50μl of Gey's buffer/1% BSA was added to each well of a 96-well tissue culture plate. 25μl of diluted mice sera (1:100 in Gey's buffer/0.2% BSA) were added to each well and the plate incubated at 4°C. 25μl of the previously described bacterial suspension were added to each well. 25μl of either heat-inactivated (56°C waterbath for 30 minutes) or normal baby rabbit complement were added to each well. Immediately after the addition of the baby rabbit complement, 22μl of each sample/well were plated on Mueller-Hinton agar plates (time 0). The 96-well plate was incubated for 1 hour at 37°C with rotation and then 22μl of each sample/well were plated on Mueller-Hinton agar plates (time 1). After overnight incubation the colonies corresponding to time 0 and time 1 hour were counted.

**Table II** (page 491) gives a summary of the cloning, expression and prurification results.

**Example 1**

The following partial DNA sequence was identified in *N. meningitidis* <SEQ ID 1>:

```
  1 ATGAAACAGA CAGTCAA.AT GCTTGCCGCC GCCCTGATTG CCTTGGGCTT
 51 GAACCGACCG GTGTGGNCGG ATGACGTATC GGATTTTCGG GAAAACTTGC
101 A.GCGGCAGC ACAGGGAAAT GCAGCAGCCC AATACAATTT GGGCGCAATG
151 TAT.TACAAA GGACGCGCGT GCGCCGGGAT GATGCTGAAG CGGTCAGATG
201 GTATCGGCAG CCGGCGGAAC AGGGGTTAGC CCAAGCCCAA TACAATTTGG
251 GCTGGATGTA TGCCAACGGG CGCGC.GTGC GCCAAGATGA TACCGAAGCG
301 GTCAGATGGT ATCGGCAGGC GGCAGCGCAG GGGGTTGTCC AAGCCCAATA
351 CAATTTGGGC GTGATATATG CCGAAGGACG TGGAGTGCGC CAAGACGATG
401 TCGAAGCGGT CAGATGGTTT CGGCAGGCGG CAGCGCAGGG GGTAGCCCAA
451 GCCCAAAACA ATTTGGGCGT GATGTATGCC GAAAGANCGC GCGTGCGCCA
501 AGACCG...
```

This corresponds to the amino acid sequence <SEQ ID 2; ORF37>:

```
  1 MKQTVXMLAA ALIALGLNRP VWXDDVSDFR ENLXAAAQGN AAAQYNLGAM
 51 YXQRTRVRRD DAEAVRWYRQ PAEQGLAQAQ YNLGWMYANG RXVRQDDTEA
101 VRWYRQAAAQ GVVQAQYNLG VIYAEGRGVR QDDVEAVRWF RQAAAQGVAQ
151 AQNNLGVMYA ERXRVRQD...
```

Further work revealed the complete nucleotide sequence <SEQ ID 3>:

```
  1 ATGAAACAGA CAGTCAAATG GCTTGCCGCC GCCCTGATTG CCTTGGGCTT
 51 GAACCGAGCG GTGTGGGCGG ATGACGTATC GGATTTTCGG GAAAACTTGC
101 AGGCGGCAGC ACAGGGAAAT GCAGCAGCCC AATACAATTT GGGCGCAATG
151 TATTACAAAG GACGCGGCGT GCGCCGGGAT GATGCTGAAG CGGTCAGATG
201 GTATCGGCAG GCGGCGGAAC AGGGGTTAGC CCAAGCCCAA TACAATTTGG
251 GCTGGATGTA TGCCAACGGG CGCGGCGTGC GCCAAGATGA TACCGAAGCG
301 GTCAGATGGT ATCGGCAGGC GGCAGCGCAG GGGGTTGTCC AAGCCCAATA
351 CAATTTGGGC GTGATATATG CCGAAGGACG TGGAGTGCGC CAAGACGATG
401 TCGAAGCGGT CAGATGGTTT CGGCAGGCGG CAGCGCAGGG GGTAGCCCAA
451 GCCCAAAACA ATTTGGGCGT GATGTATGCC GAAAGACGCG GCGTGCGCCA
501 AGACCGCGCC CTTGCACAAG AATGGTTTGG CAAGGCTTGT CAAAACGGAG
551 ACCAAGACGG CTGCGACAAT GACCAACGCC TGAAGGCGGG TTATTGA
```

This corresponds to the amino acid sequence <SEQ ID 4; ORF37-1>:

```
  1 MKQTVKWLAA ALIALGLNRA VWADDVSDFR ENLQAAAQGN AAAQYNLGAM
 51 YYKGRGVRRD DAEAVRWYRQ AAEQGLAQAQ YNLGWMYANG RGVRQDDTEA
101 VRWYRQAAAQ GVVQAQYNLG VIYAEGRGVR QDDVEAVRWF RQAAAQGVAQ
151 AQNNLGVMYA ERRGVRQDRA LAQEWFGKAC QNGDQDGCDN DQRLKAGY*
```

Further work identified the corresponding gene in strain A of *N. meningitidis* <SEQ ID 5>:

```
  1 ATGAAACAGA CAGTCAAATG GCTTGCCGCC GCCCTGATTG CCTTGGGCTT
 51 GAACCAAGCG GTGTGGGCGG ATGACGTATC GGATTTTCGG GAAAACTTGC
101 AGGCGGCAGC ACAGGGAAAT GCAGCAGCCC AAAACAATTT GGGCGTGATG
151 TATGCCGAAA GACGCGGCGT GCGCCAAGAC CGCGCCCTTG CACAAGAATG
201 GCTTGGCAAG GCTTGTCAAA ACGGATACCA AGACAGCTGC GACAATGACC
251 AACGCCTGAA AGCGGGTTAT TGA
```

This encodes a protein having amino acid sequence <SEQ ID 6; ORF37a>:

```
  1  MKQTVKWLAA ALIALGLNQA VWADDVSDFR ENLQAAAQGN AAAQNNLGVM
 51  YAERRGVRQD RALAQEWLGK ACQNGYQDSC DNDQRLKAGY *
```

The originally-identified partial strain B sequence (ORF37) shows 68.0% identity over a 75aa overlap with ORF37a:

```
                      10        20        30        40        50        60
orf37.pep    MKQTVXMLAAALIALGLNRPVWXDDVSDFRENLXAAAQGNAAAQYNLGAMYXQRTRVRRD
             |||||  |||||||||||: ||  |||||||||| ||||||||||  |||:|| :|  ||:|
orf37a       MKQTVKWLAAALIALGLNQAVWADDVSDFRENLQAAAQGNAAAQNNLGVMYAERRGVRQD
                      10        20        30        40        50        60

                      70        80        90       100       110       120
orf37.pep    DAEAVRWYRQPAEQGLAQAQYNLGWMYANGRXVRQDDTEAVRWYRQAAAQGVVQAQYNLG
              | | :|  :  ::|
orf37a       RALAQEWLGKACQNGYQDSCDNDQRLKAGYX
                      70        80        90
```

Further work identified the corresponding gene in *N.gonorrhoeae* <SEQ ID 7 >:

```
  1  ATGAAACAGA CAGTCAAATG GCTTGCCGCC GCCCTGATTG CCTTGGGCTT
 51  GAACCAAGCG GTGTGGGCGG GTGACGTATC GGATTTTCGG GAAAACTTGC
101  AGgcggcaGA ACaggGAAAT GCAGCAGCCC AATTCAATTT GGGCGTGATG
151  TATGAAAATG GACAAGGAGT TCGTCAAGAT TATGTACAGG CAGTGCAGTG
201  GTATCGCAAG GCTTCAGAAC AAGGGGATGC CCAAGCCCAA TACAATTTGG
251  GCTTGATGTA TTACGATGGA CGCGGCGTGC GCCAAGACCT TGCGCTCGCT
301  CAACAATGGC TTGGCAAGGC TTGTCAAAAC GGAGACCAAA ACAGCTGCGA
351  CAATGACCAA CGCCTGAAGG CGGGTTATTA A
```

This encodes a protein having amino acid sequence <SEQ ID 8; ORF37ng>:

```
  1  MKQTVKWLAA ALIALGLNQA VWAGDVSDFR ENLQAAEQGN AAAQFNLGVM
 51  YENGQGVRQD YVQAVQWYRK ASEQGDAQAQ YNLGLMYYDG RGVRQDLALA
101  QQWLGKACQN GDQNSCDNDQ RLKAGY*
```

The originally-identified partial strain B sequence (ORF37) shows 64.9% identity over a 111aa overlap with ORF37ng:

```
orf37.pep    MKQTVXMLAAALIALGLNRPVWXDDVSDFRENLXAAAQGNAAAQYNLGAMYXQRTRVRRD   60
             |||||  |||||||||||: ||  ||||||||| || |||||||:|||:|| :   ||:|
orf37ng      MKQTVKWLAAALIALGLNQAVWAGDVSDFRENLQAAEQGNAAAQFNLGVMYENGQGVRQD   60

orf37.pep    DAEAVRWYRQPAEQGLAQAQYNLGWMYANGRXVRQDDTEAVRWYRQAAAQGVVQAQYNLG  120
              ::||:|||: :||| |||||||| || :|| |||| : | :|  :|  :|
orf37ng      YVQAVQWYRKASEQGDAQAQYNLGLMYYDGRGVRQDLALAQQWLGKACQNGDQNSCDNDQ  120

orf37.pep    VIYAEGRGVRQDDVEAVRWFRQAAAQGVAQAQNNLGVMYAERXRVRQD  168

orf37ng      RLKAGY                                            126
```

The complete strain B sequence (ORF37-1) and ORF37ng show 51.5% identity in 198 aa overlap:

```
                        10        20        30        40        50        60
orf37-1.pep  MKQTVKWLAAALIALGLNRAVWADDVSDFRENLQAAAQGNAAAQYNLGAMYYKGRGVRRD
             ||||||||||||||||||:|||| |||||||||||| |||||||:|||:|| :|:|||:|
orf37ng      MKQTVKWLAAALIALGLNQAVWAGDVSDFRENLQAAEQGNAAAQFNLGVMYENGQGVRQD
                        10        20        30        40        50        60

                        70        80        90       100       110       120
orf37-1.pep  DAEAVRWYRQAAEQGLAQAQYNLGWMYANGRGVRQDDTEAVRWYRQAAAQGVVQAQYNLG
              ::||:|||:|:||| |||||||| || :|||||||
orf37ng      YVQAVQWYRKASEQGDAQAQYNLGLMYYDGRGVRQD------------------------
                        70        80        90

                       130       140       150       160       170       180
orf37-1.pep  VIYAEGRGVRQDDVEAVRWFRQAAAQGVAQAQNNLGVMYAERRGVRQDRALAQEWFGKAC
                                                          ||||:|:||||
orf37ng      ------------------------------------------------LALAQQWLGKAC
                                                             100

                       190       199

orf37-1.pep  QNGDQDGCDNDQRLKAGYX
             |||||::||||||||||||
orf37ng      QNGDQNSCDNDQRLKAGYX
                    110       120
```

Computer analysis of these amino acid sequences indicates a putative leader sequence, and it was predicted that the proteins from *N.meningitidis* and *N.gonorrhoeae,* and their epitopes, could be useful antigens for vaccines or diagnostics, or for raising antibodies.

ORF37-1 (11kDa) was cloned in pET and pGex vectors and expressed in *E.coli,* as described above. The products of protein expression and purification were analyzed by SDS-PAGE. Figure 1A shows the results of affinity purification of the GST-fusion protein, and Figure 1B shows the results of expression of the His-fusion in *E.coli.* Purified GST-fusion protein was used to immunise mice, whose sera were used for ELISA (positive result), FACS analysis (Figure 1C), and a bactericidal assay (Figure 1D). These experiments confirm that ORF37-1 is a surface-exposed protein, and that it is a useful immunogen.

Figure 1E shows plots of hydrophilicity, antigenic index, and AMPHI regions for ORF37-1.

**Example 2**

The following partial DNA sequence was identified in *N.meningitidis* <SEQ ID 9>:

```
  TTCGGCGA CATCGGCGGT TTGAAGGTCA ATGCCCCCGT CAAATCCGCA
GGCGTATTGG TCGGGCGCGT CGGCGCTATC GGACTTGACC CGAAATCCTA
TCAGGCGAGG GTGCGCCTCG ATTTGGACGG CAAGTATCAG TTCAGCAGCG
ACGTTTCCGC GCAAATCCTG ACTTCsGGAC TTTTGGGCGA GCAGTACATC
GGGCTGCAGC AGGGCGGCGA CACGGAAAAC CTTGCTGCCG GCGACACCAT
CTCCGTAACC AGTTCTGCAA TGGTTCTGGA AAACCTTATC GGCAAATTCA
TGACGAGTTT TGCCGAGAAA AATGCCGACG GCGGCAATGC GGAAAAAGCC
GCCGAATAA
```

This corresponds to the amino acid sequence <SEQ ID 10>:

```
  1  FGDIGGLKVN APVKSAGVLV GRVGAIGLDP KSYQARVRLD LDGKYQFSSD
 51  VSAQILTSGL LGEQYIGLQQ GGDTENLAAG DTISVTSSAM VLENLIGKFM
101  TSFAEKNADG GNAEKAAE*
```

Computer analysis of this amino acid sequence gave the following results:

Homology with a hypothetical *H influenzae* protein (ybrd.haein; accession number p45029)

SEQ ID 9 and ybrd.haein show 48.4% aa identity in 122 aa overlap:

```
                20        30        40        50        60        70
yrbd.h LGIGALVFLGLRVANVQGFAETKSYTVTATFDNIGGLKVRAPLKIGGVVIGRVSAITLDE
                                     |::||||||:||:| :||::|||:||:||
N.m                                  FGDIGGLKVNAPVKSAGVLVGRVGAIGLDP
                                              10        20        30

                80        90       100       110       120       130
yrbd.h KSYLPKVSIAINQEYNEIPENSSLSIKTSGLLGEQYIALTMGFDDGDTAMLKNGSQIQDT
       ||| ::|::::: :| ::::: |  | ||||||||||:|  |   |||: | :|: |  |
N.m    KSYQARVRLDLDGKY-QFSSDVSAQILTSGLLGEQYIGLQQG---GDTENLAAGDTISVT
                  40        50        60        70           80

               140       150       160
yrbd.h TSAMVLEDLIGQFL--YGSKKSDGNEKSESTEQ
       :||||||:|||:|:  :::|::||:: ::::|:
N.m    SSAMVLENLIGKFMTSFAEKNADGGNAEKAAEX
           90       100       110       120
```

Homology with a predicted ORF from *N.gonorrhoeae*

SEQ ID 9 shows 99.2% identity over a 118aa overlap with a predicted ORF from *N. gonorrhoeae:*

```
              20        30        40        50        60        70
yrbd   GAAAVAFLAFRVAGGAAFGGSDKTYAVYADFGDIGGLKVNAPVKSAGVLVGRVGAIGLDP
                                     ||||||||||||||||||||||||||||||
N.m                                  FGDIGGLKVNAPVKSAGVLVGRVGAIGLDP
                                              10        20        30

              80        90       100       110       120       130
yrbd   KSYQARVRLDLDGKYQFSSDVSAQILTSGLLGEQYIGLQQGGDTENLAAGDTISVTSSAM
       ||||||||||||||||||||||||||||||||||||||||||||||||||||||||||||
N.m    KSYQARVRLDLDGKYQFSSDVSAQILTSGLLGEQYIGLQQGGDTENLAAGDTISVTSSAM
                  40        50        60        70        80        90

             140       150       160
yrbd   VLENLIGKFMTSFAEKNAEGGNAEKAAEX
       ||||||||||||||||||:||||||||||
N.m    VLENLIGKFMTSFAEKNADGGNAEKAAEX
              100       110       120
```

The complete yrbd *H. influenzae* sequence has a leader sequence and it is expected that the full-length homologous *N.meningitidis* protein will also have one. This suggests that it is either a membrane protein, a secreted protein, or a surface protein and that the protein, or one of its epitopes, could be a useful antigen for vaccines or diagnostics.

**Example 3**

The following partial DNA sequence was identified in *N.meningitidis* <SEQ ID 11>:

```
  1  ..ATTTTGATAT ACCTCATCCG CAAGAATCTA GGTTCGCCCG TCTTCTTCTT
 51    TCAGGAACGC CCCGGAAAGG ACGGAAAACC TTTTAAAATG GTCAAATTCC
101    GTTCCATGCG CGACGGCTTG TATTCAGACG GCATTCCGCT GCCCGACGGA
151    GAACGCCTGA CACCGTTCGG CAAAAAACTG CGTGCCGcCA GTwTGGACGA
201    ACTGCCTGAA TTATGGAATA TCTTAAAAGG CGAGATGAGC CTGGTCGGCC
251    CCCGCCCGCT GCTGATGCAA TATCTGCCGC TGTACGACAA CTTCCAAAAC
301    CGCCGCCACG AAATGAAACC CGGCATTACC GGCTGGGCGC AGGTCAACGG
351    GCGCAACGCg CTTTCGTGGG ACGAAAAATT CGCCTGCGAT GTTTGGTATA
401    TCGACCACTT CAGCCTGTGC CTCGACATCA AAATCCTACT GCTGACGGTT
451    AAAAAAGTAT TAATCAAGGA AGGGATTTCC GCACAGGGCG AACA.aCCAT
501    GCCCCCTTTC ACAGGAAAAC GCAAACTCGC CGTCGTCGGT GCGGGCGGAC
551    ACGGAAAAGT CGTTGCCGAC CTTGCCGCCG CACTCGGCCG GTACAGGGAA
601    ATCGTTTTTC TGGACGACCG CGCACAAGGC AGCGTCAACG GCTTTTCCGT
651    CATCGGCACG ACGCTGCTGC TTGAAAACAG TTTATCGCCC GAACAATACG
701    ACGTCGCCGT CGCCGTCGGC AACAACCGCA TCCGCCGCCA AATCGCCGAA
751    AAAGCCGCCG CGCTCGGCTT CGCCCTGCCC GTACTGGTTC ATCCGGACGC
801    GACCGTCTCG CCTTCTGCAA CAGTCGGACA AGGCAGCGTC GTTATGGCGA
851    AAGCGGTCG..
```

This corresponds to the amino acid sequence <SEQ ID 12; ORF3>:

```
  1  ..ILIYLIRKNL GSPVFFFQER PGKDGKPFKM VKFRSMRDGL YSDGIPLPDG
 51    ERLTPFGKKL RAASXDELPE LWNILKGEMS LVGPRPLLMQ YLPLYDNFQN
101    RRHEMKPGIT GWAQVNGRNA LSWDEKFACD VWYIDHFSLC LDIKILLLTV
151    KKVLIKEGIS AQGEXTMPPF TGKRKLAVVG AGGHGKVVAD LAAALGRYRE
201    IVFLDDRAQG SVNGFSVIGT TLLLENSLSP EQYDVAVAVG NNRIRRQIAE
251    KAAALGFALP VLVHPDATVS PSATVGQGSV VMAKAV..
```

Further sequence analysis revealed the complete nucleotide sequence <SEQ ID 13>:

```
   1  ATGAGTAAAT TCTTCAAACG CCTGTTTGAC ATTGTTGCCT CCGCCTCGGG
  51  ACTGATTTTC CTCTCGCCAG TATTTTTGAT TTTGATATAC CTCATCCGCA
 101  AGAATCTAGG TTCGCCCGTC TTCTTCTTTC AGGAACGCCC CGGAAAGGAC
 151  GGAAAACCTT TTAAAATGGT CAAATTCCGT TCCATGCGCG ACGCGCTTGA
 201  TTCAGACGGC ATTCCGCTGC CCGACGGAGA ACGCCTGACA CCGTTCGGCA
 251  AAAAACTGCG TGCCGCCAGT TTGGACGAAC TGCCTGAATT ATGGAATATC
 301  TTAAAAGGCG AGATGAGCCT GGTCGGCCCC CGCCCGCTGC TGATGCAATA
 351  TCTGCCGCTG TACGACAACT TCCAAAACCG CCGCCACGAA ATGAAACCCG
 401  GCATTACCGG CTGGGCGCAG GTCAACGGGC GCAACGCGCT TTCGTGGGAC
 451  GAAAAATTCG CCTGCGATGT TTGGTATATC GACCACTTCA GCCTGTGCCT
 501  CGACATCAAA ATCCTACTGC TGACGGTTAA AAAAGTATTA ATCAAGGAAG
 551  GGATTTCCGC ACAGGGCGAA GCCACCATGC CCCCTTTCAC AGGAAAACGC
 601  AAACTCGCCG TCGTCGGTGC GGGCGGACAC GGAAAAGTCG TTGCCGACCT
 651  TGCCGCCGCA CTCGGCCGGT ACAGGGAAAT CGTTTTTCTG GACGACCGCG
 701  CACAAGGCAG CGTCAACGGC TTTTCCGTCA TCGGCACGAC GCTGCTGCTT
 751  GAAAACAGTT TATCGCCCGA ACAATACGAC GTCGCCGTCG CCGTCGGCAA
 801  CAACCGCATC CGCCGCCAAA TCGCCGAAAA AGCCGCCGCG CTCGGCTTCG
 851  CCCTGCCCGT TCTGGTTCAT CCGGACGCGA CCGTCTCGCC TTCTGCAACA
 901  GTCGGACAAG GCAGCGTCGT TATGGCGAAA GCCGTCGTAC AGGCAGGCAG
 951  CGTATTGAAA GACGGCGTGA TTGTGAACAC TGCCGCCACC GTCGATCACG
1001  ACTGCCTGCT TAACGCTTTC GTCCACATCA GCCCAGGCGC GCACCTGTCG
1051  GGCAACACGC ATATCGGCGA AGAAAGCTGG ATAGGCACGG GCGCGTGCAG
1101  CCGCCAGCAG ATCCGTATCG GCAGCCGCGC AACCATTGGA GCGGGCGCAG
1151  TCGTCGTACG CGACGTTTCA GACGGCATGA CCGTCGCGGG CAATCCGGCA
1201  AAGCCGCTGC CGCGCAAAAA CCCCGAGACC TCGACAGCAT AA
```

This corresponds to the amino acid sequence <SEQ ID 14; ORF3-1>:

```
  1 MSKFFKRLFD IVASASGLIF LSPVFLILIY LIRKNLGSPV FFFQERPGKD
 51 GKPFKMVKFR SMRDALDSDG IPLPDGERLT PFGKKLRAAS LDELPELWNI
101 LKGEMSLVGP RPLLMQYLPL YDNFQNRRHE MKPGITGWAQ VNGRNALSWD
151 EKFACDVWYI DHFSLCLDIK ILLLTVKKVL IKEGISAQGE ATMPPFTGKR
201 KLAVVGAGGH GKVVADLAAA LGRYREIVFL DDRAQGSVNG FSVIGTTLLL
251 ENSLSPEQYD VAVAVGNNRI RRQIAEKAAA LGFALPVLVH PDATVSPSAT
301 VGQGSVVMAK AVVQAGSVLK DGVIVNTAAT VDHDCLLNAF VHISPGAHLS
351 GNTHIGEESW IGTGACSRQQ IRIGSRATIG AGAVVVRDVS DGMTVAGNPA
401 KPLPRKNPET STA*
```

Computer analysis of this amino acid sequence gave the following results:

Homology with a predicted ORF from *N.meningitidis* (strain A)

ORF3 shows 93.0% identity over a 286aa overlap with an ORF (ORF3a) from strain A of *N. meningitidis:*

```
                                              10        20        30
orf3.pep                              ILIYLIRKNLGSPVFFFQERPGKDGKPFKMVKFR
                                      ||||||||||||||||||||||||||||||||||
orf3a       MSKFFKRLFDIVASASGLIFLSPVFLILIYLIRKNLGSPVFFFQERPGKDGKPFKMVKFR
                    10        20        30        40        50        60

                40        50        60        70        80        90
orf3.pep    SMRDGLYSDGIPLPDGERLTPFGKKLRAASXDELPELWNILKGEMSLVGPRPLLMQYLPL
            ||:|:| |||| |||||||||||||||||| ||||||||:|||:||||||||||||||||
orf3a       SMHDALDSDGILLPDGERLTPFGKKLRAASLDELPELWNVLKGDMSLVGPRPLLMQYLPL
                    70        80        90       100       110       120

               100       110       120       130       140       150
orf3.pep    YDNFQNRRHEMKPGITGWAQVNGRNALSWDEKFACDVWYIDHFSLCLDIKILLLTVKKVL
            |||||||||||||||||||||||||||||||:||||:|||||||||||||||||||||||
orf3a       YDNFQNRRHEMKPGITGWAQVNGRNALSWDERFACDIWYIDHFSLCLDIKILLLTVKKVL
                   130       140       150       160       170       180

               160       170       180       190       200       210
orf3.pep    IKEGISAQGEXTMPPFTGKRKLAVVGAGGHGKVVADLAAALGRYREIVFLDDRAQGSVNG
            |||||||||| ||||||||||||||||||||||||:|||||| | ||||||||:||||||
orf3a       IKEGISAQGEATMPPFTGKRKLAVVGAGGHGKVVAELAAALGTYGEIVFLDDRVQGSVNG

                   190       200       210       220       230       240

               220       230       240       250       260       270
orf3.pep    FSVIGTTLLLENSLSPEQYDVAVAVGNNRIRRQIAEKAAALGFALPVLVHPDATVSPSAT
            | ||||||||||||||||:|:|||||||||||||||||||||||||||:|||:|||||||
orf3a       FPVIGTTLLLENSLSPEQFDIAVAVGNNRIRRQIAEKAAALGFALPVLIHPDSTVSPSAT
                   250       260       270       280       290       300

               280
orf3.pep    VGQGSVVMAKAV
            ||||:|||||||
orf3a       VGQGGVVMAKAVVQADSVLKDGVIVNTAATVDHDCLLDAFVHISPGAHLSGNTRIGEESW
                   310       320       330       340       350       360
```

The complete length ORF3a nucleotide sequence <SEQ ID 15> is:

```
   1  ATGAGTAAAT TCTTCAAACG CCTGTTTGAC ATTGTTGCCT CCGCCTCGGG
  51  ACTGATTTTC CTCTCGCCAG TATTTTTGAT TTTGATATAC CTCATCCGCA
 101  AGAATCTGGG TTCGCCCGTC TTCTTCTTTC AGGAACGCCC CGGAAAGGAC
 151  GGAAAACCTT TTAAAATGGT CAAATTCCGT TCCATGCACG ACGCGCTTGA
 201  TTCAGACGGC ATTCTGCTGC CCGACGGAGA ACGCCTGACA CCGTTCGGCA
 251  AAAAACTGCG TGCCGCCAGT TTGGACGAAC TGCCCGAACT GTGGAACGTC
 301  CTCAAAGGCG ACATGAGCCT GGTCGGCCCC CGCCCGCTGC TGATGCAATA
 351  TCTGCCGCTG TACGACAACT TCCAAAACCG CCGCCACGAA ATGAAACCGG
 401  GCATTACCGG CTGGGCGCAG GTCAACGGGC GCAACGCGCT TTCGTGGGAC
 451  GAACGCTTCG CATGCGACAT CTGGTATATC GACCACTTCA GCCTGTGCCT
 501  CGACATCAAA ATCCTACTGC TGACGGTTAA AAAAGTATTA ATCAAAGAAG
 551  GGATTTCCGC ACAGGGCGAA GCCACCATGC CCCCTTTCAC AGGAAAAACGC
 601  AAACTTGCCG TCGTCGGTGC GGGCGGACAC GGCAAAGTCG TTGCCGAGCT
 651  TGCCGCCGCA CTCGGCACAT ACGGCGAAAT CGTTTTTCTG GACGACCGCG
 701  TCCAAGGCAG CGTCAACGGC TTCCCCGTCA TCGGCACGAC GCTGCTGCTT
 751  GAAAACAGTT TATCGCCCGA ACAATTCGAC ATCGCCGTCG CCGTCGGCAA
 801  CAACCGCATC CGCCGCCAAA TCGCCGAAAA AGCCGCCGCG CTCGGCTTCG
 851  CCCTGCCCGT CCTGATTCAT CCGGACTCGA CCGTCTCGCC TTCTGCAACA
 901  GTCGGACAAG GCGGCGTCGT TATGGCGAAA GCCGTCGTAC AGGCTGACAG
 951  CGTATTGAAA GACGGCGTAA TTGTGAACAC TGCCGCCACC GTCGATCACG
1001  ATTGCCTGCT TGATGCTTTC GTCCACATCA GCCCGGGCGC GCACCTGTCG
1051  GGCAACACGC GTATCGGCGA AGAAAGCTGG ATAGGCACAG GCGCGTGCAG
1101  CCGCCAGCAG ATCCGTATCG GCAGCCGCGC AACCATTGGA GCGGGCGCAG
1151  TCGTCGTGCG CGACGTTTCA GACGGCATGA CCGTCGCGGG CAACCCGGCA
1201  AAACCATTGG CAGGCAAAAA TACCGAGACC CTGCGGTCGT AA
```

This is predicted to encode a protein having amino acid sequence <SEQ ID 16>:

```
  1  MSKFFKRLFD IVASASGLIF LSPVFLILIY LIRKNLGSPV FFFQERPGKD
 51  GKPFKMVKFR SMHDALDSDG ILLPDGERLT PFGKKLRAAS LDELPELWNV
101  LKGDMSLVGP RPLLMQYLPL YDNFQNRRHE MKPGITGWAQ VNGRNALSWD
151  ERFACDIWYI DHFSLCLDIK ILLLTVKKVL IKEGISAQGE ATMPPFTGKR
201  KLAVVGAGGH GKVVAELAAA LGTYGEIVFL DDRVQGSVNG FPVIGTTLLL
251  ENSLSPEQFD IAVAVGNNRI RRQIAEKAAA LGFALPVLIH PDSTVSPSAT
301  VGQGGVVMAK AVVQADSVLK DGVIVNTAAT VDHDCLLDAF VHISPGAHLS
351  GNTRIGEESW IGTGACSRQQ IRIGSRATIG AGAVVVRDVS DGMTVAGNPA
401  KPLAGKNTET LRS*
```

Two transmembrane domains are underlined.

ORF3-1 shows 94.6% identity in 410 aa overlap with ORF3a:

```
                          10        20        30        40        50        60
orf3a.pep         MSKFFKRLFDIVASASGLIFLSPVFLILIYLIRKNLGSPVFFFQERPGKDGKPFKMVKFR
                  ||||||||||||||||||||||||||||||||||||||||||||||||||||||||||||
orf3-1            MSKFFKRLFDIVASASGLIFLSPVFLILIYLIRKNLGSPVFFFQERPGKDGKPFKMVKFR
                          10        20        30        40        50        60

                          70        80        90       100       110       120
orf3a.pep         SMHDALDSDGILLPDGERLTPFGKKLRAASLDELPELWNVLKGDMSLVGPRPLLMQYLPL
                  ||:|||||||| |||||||||||||||||||||||||||:|||:||||||||||||||||
orf3-1            SMRDALDSDGIPLPDGERLTPFGKKLRAASLDELPELWNILKGEMSLVGPRPLLMQYLPL
                          70        80        90       100       110       120
```

```
                130       140       150       160       170       180
orf3a.pep  YDNFQNRRHEMKPGITGWAQVNGRNALSWDERFACDIWYIDHFSLCLDIKILLLTVKKVL
           |||||||||||||||||||||||||||||||:||||:|||||||||||||||||||||||
orf3-1     YDNFQNRRHEMKPGITGWAQVNGRNALSWDEKFACDVWYIDHFSLCLDIKILLLTVKKVL
                130       140       150       160       170       180

                190       200       210       220       230       240
orf3a.pep  IKEGISAQGEATMPPFTGKRKLAVVGAGGHGKVVAELAAALGTYGEIVFLDDRVQGSVNG
           |||||||||||||||||||||||||||||||||||:|||||| | ||||||||:||||||
orf3-1     IKEGISAQGEATMPPFTGKRKLAVVGAGGHGKVVADLAAALGRYREIVFLDDRAQGSVNG
                190       200       210       220       230       240

                250       260       270       280       290       300
orf3a.pep  FPVIGTTLLLENSLSPEQFDIAVAVGNNRIRRQIAEKAAALGFALPVLIHPDSTVSPSAT
           | ||||||||||||||||:|:|||||||||||||||||||||||||||:|||:|||||||
orf3-1     FSVIGTTLLLENSLSPEQYDVAVAVGNNRIRRQIAEKAAALGFALPVLVHPDATVSPSAT
                250       260       270       280       290       300

                310       320       330       340       350       360
orf3a.pep  VGQGGVVMAKAVVQADSVLKDGVIVNTAATVDHDCLLDAFVHISPGAHLSGNTRIGEESW
           ||||:|||||||||| |||||||||||||||||||||:|||||||||||||||:||||||
orf3-1     VGQGSVVMAKAVVQAGSVLKDGVIVNTAATVDHDCLLNAFVHISPGAHLSGNTHIGEESW
                310       320       330       340       350       360

                370       380       390       400       410
orf3a.pep  IGTGACSRQQIRIGSRATIGAGAVVVRDVSDGMTVAGNPAKPLAGKNTETLRSX
           |||||||||||||||||||||||||||||||||||||||||||  || ||
orf3-1     IGTGACSRQQIRIGSRATIGAGAVVVRDVSDGMTVAGNPAKPLPRKNPETSTAX
                370       380       390       400       410
```

<u>Homology with hypothetical protein encoded by *yvfc* gene (accession Z71928) of *B. subtilis*</u>

ORF3 and YVFC proteins show 55% aa identity in 170 aa overlap (BLASTp):

```
ORF3   3   IYLIRKNLGSPVFFFQERPGKDGKPFKMVKFRSMRDGLYSDGIPLPDGERLTPFGKKLRA 62
           I ++R  +GSPVFF Q RPG  GKPF + KFR+M D   S G  LPD  RLT  G+ +R
yvfc  27   IAVVRLKIGSPVFFKQVRPGLHGKPFTLYKFRTMTDERDSKGNLLPDEVRLTKTGRLIRK 86

ORF3   63  ASXDELPELWNILKGEMSLVGPRPLLMQYLPLYDNFQNRRHEMKPGITGWAQVNGRNALS 122
            S DELP+L N+LKG++SLVGPRPLLM YLPLY   Q RRHE+KPGITGWAQ+NGRNA+S
yvfc   87  LSIDELPQLLNVLKGDLSLVGPRPLLMDYLPLYTEKQARRHEVKPGITGWAQINGRNAIS 146

ORF3   123 WDEKFACDVWYIDHFSLCLDXXXXXXXXXXXXXXEGISAQGEXTMPPFTG 172
           W++KF  DVWY+D++S  LD              EGI      T   FTG
yvfc   147 WEKKFELDVWYVDNWSFFLDLKILCLTVRKVLVSEGIQQTNHVTAERFTG 196
```

<u>Homology with a predicted ORF from *N.gonorrhoeae*</u>

ORF3 shows 86.3% identity over a 286aa overlap with a predicted ORF (ORF3.ng) from *N. gonorrhoeae:*

```
orf3          ILIYLIRKNLGSPVFFFQERPGKDGKPFKMVKFR     34
                                        ^^^^^^
                                        :|||||||| ||||||::||||||||||||||||
orf3ng        MSKAVKRLFDIIASASGLIVLSPVFLVLIYLIRKNKGSPVFFIRERPGKDGKPFKMVKFR     60

orf3          SMRDGLYSDGIPLPDGERLTPFGKKLRAASXDELPELWNILKGEMSLVGPRPLLMQYLPL     94
              ||||:| ||||||||:|||| |||||||:| ||||||||:||||||||||||||||||||
orf3ng        SMRDALDSDGIPLPDSERLTDFGKKLRATSLDELPELWNVLKGEMSLVGPRPLLMQYLPL    120

orf3          YDNFQNRRHEMKPGITGWAQVNGRNALSWDEKFACDVWYIDHFSLCLDIKILLLTVKKVL    154
              |::||||||||||||||||||||||||||||||:||||| |:||: ||:|||:|||||||
orf3ng        YNKFQNRRHEMKPGITGWAQVNGRNALSWDEKFSCDVWYTDNFSFWLDMKILFLTVKKVL    180

orf3          IKEGISAQGEXTMPPFTGKRKLAVVGAGGHGKVVADLAAALGRYREIVFLDDRAQGSVNG    214
              |||||||||| |||||:|:|||||:||||||||||:|||||| | ||||||||:||||||
orf3ng        IKEGISAQGEATMPPFAGNRKLAVIGAGGHGKVVAELAAALGTYGEIVFLDDRTQGSVNG    240
```

--

```
orf3          FSVIGTTLLLENSLSPEQYDVAVAVGNNRIRRQIAEKAAALGFALPVLVHPDATVSPSAT    274
              | ||||||||||||||||:|::||||||||||||:|:|||||| ||||:||||||||||
orf3ng        FPVIGTTLLLENSLSPEQFDITVAVGNNRIRRQITENAAALGFKLPVLIHPDATVSPSAI    300

orf3          VGQGSVVMAKAV                                                    286
              :|||||||||||
orf3ng        IGQGSVVMAKAVVQAGSVLKDGVIVNTAATVDHDCLLDAFVHISPGAHLSGNTRIGEESR    360
```

The complete length ORF3ng nucleotide sequence <SEQ ID 17> is:

```
   1 ATGAGTAAAG CCGTCAAACG CCTGTTCGAC ATCATCGCAT CCGCATCGGG
  51 GCTGATTGTC CTGTCGCCCG TGTTTTTGGT TTTAATATAC CTCATCCGCA
 101 AAAACTTAGG TTCGCCCGTC TTCTTCattC GGGAACGCCc cgGAAAGGAc
 151 ggaaaacCTT TTAAAATGGT CAAATTCCGT TCCAtgcgcg acgcgcttGA
 201 TTCAGACGGC ATTCCGCTGC CCGATAGCGA ACGCCTGACC GATTTCGGCA
 251 AAAAATTACG CGCCACCAGT TTGGACGAAC TTCCTGAATT ATGGAATGTC
 301 CTCAAAGGCG AGATGAGCCT GGTCGGCCCC CGCCCGCTTT TGATGCAGTA
 351 TCTGCCGCTT TACAACAAAT TTCAAAACCG CCGCCACGAA ATGAAACCGG
 401 GCATTACCGG CTGGGCGCAG GTCAACGGGC GCAACGCGCT TTCGTGGGAC
 451 GAAAAGTTCT CCTGCGATGT TTGGTACACC GACAATTTCA GCTTTTGGCT
 501 GGATATGAAA ATCCTGTTTC TGACAGTCAA AAAAGTCTTG ATTAAAGAAG
 551 GCATTTCGGC GCAAGGGGAA GCCACCATGC CCCCTTTCGC GGGGAATCGC
 601 AAACTCGCCG TTATCGGCGC GGGCGGACAC GGCAAAGTCG TTGCCGAGCT
 651 TGCCGCCGCA CTCGGCACAT ACGGCGAAAT CGTTTTTCTG GACGACCGCA
 701 CCCAAGGCAG CGTCAACGGC TTCCCCGTCA TCGGCACGAC GCTGCTGCTT
 751 GAAAACAGTT TATCGCCCGA ACAATTCGAC ATCACCGTCG CCGTCGGCAA
 801 CAACCGCATC CGCCGCCAAA TCACCGAAAA CGCCGCCGCG CTCGGCTTCA
 851 AACTGCCCGT TCTGATTCAT CCCGACGCGA CCGTCTCGCC TTCTGCAATA
 901 ATCGGACAAG GCAGCGTCGT AATGGCGAAA GCCGTCGTAC AGGCCGGCAG
 951 CGTATTGAAA GACGGCGTGA TTGTGAACAC TGCCGCCACC GTCGATCACG
1001 ACTGCCTGCT TGACGCTTTC GtccaCATCA GCCCGGGCGC GCACCTGTCG
1051 GGCAACACGC GTATCGGCGA AGAAAGCCGG ATAGGCACGG GCGCGTGCAG
1101 CCGCCAGCAG ACAACCGTCG GCAGCGGGGT TACCgccgGT GCAGGGGgcGG
1151 TTATCGTATG CGACATCCCG GACGGCATGA CCGTCGCGGG CAACCCGGCA
1201 AAGCCCCTTA CGGGCAAAAA CCCCAAGACC GGGACGGCAT AA
```

This encodes a protein having amino acid sequence <SEQ ID 18>:

```
  1 MSKAVKRLFD IIASASGLIV LSPVFLVLIY LIRKNLGSPV FFIRERPGKD
 51 GKPFKMVKFR SMRDALDSDG IPLPDSERLT DFGKKLRATS LDELPELWNV
101 LKGEMSLVGP RPLLMQYLPL YNKFQNRRHE MKPGITGWAQ VNGRNALSWD
151 EKFSCDVWYT DNFSFWLDMK ILFLTVKKVL IKEGISAQGE ATMPPFAGNR
201 KLAVIGAGGH GKVVAELAAA LGTYGEIVFL DDRTQGSVNG FPVIGTTLLL
251 ENSLSPEQFD ITVAVGNNRI RRQITENAAA LGFKLPVLIH PDATVSPSAI
301 IGQGSVVMAK AVVQAGSVLK DGVIVNTAAT VDHDCLLDAF VHISPGAHLS
351 GNTRIGEESR IGTGACSRQQ TTVGSGVTAG AGAVIVCDIP DGMTVAGNPA
401 KPLTGKNPKT GTA*
```

This protein shows 86.9% identity in 413 aa overlap with ORF3-1:

```
                          10        20        30        40        50        60
orf3-1.pep    MSKFFKRLFDIVASASGLIFLSPVFLILIYLIRKNLGSPVFFFQERPGKDGKPFKMVKFR
              |||  ||||||:||||||| ||||||:|||||||||||||||::||||||||||||||||
orf3ng        MSKAVKRLFDIIASASGLIVLSPVFLVLIYLIRKNLGSPVFFIRERPGKDGKPFKMVKFR
                          10        20        30        40        50        60

                          70        80        90       100       110       120
orf3-1.pep    SMRDALDSDGIPLPDGERLTPFGKKLRAASLDELPELWNILKGEMSLVGPRPLLMQYLPL
              |||||||||||||||:|||| |||||||:||||||||||:||||||||||||||||||||
orf3ng        SMRDALDSDGIPLPDSERLTDFGKKLRATSLDELPELWNVLKGEMSLVGPRPLLMQYLPL
                          70        80        90       100       110       120

                         130       140       150       160       170       180
orf3-1.pep    YDNFQNRRHEMKPGITGWAQVNGRNALSWDEKFACDVWYIDHFSLCLDIKILLLTVKKVL
              |::||||||||||||||||||||||||||||||:||||| |:||: ||:|||:|||||||
orf3ng        YNKFQNRRHEMKPGITGWAQVNGRNALSWDEKFSCDVWYTDNFSFWLDMKILFLTVKKVL
                         130       140       150       160       170       180

                         190       200       210       220       230       240
orf3-1.pep    IKEGISAQGEATMPPFTGKRKLAVVGAGGHGKVVADLAAALGRYREIVFLDDRAQGSVNG
              ||||||||||||||||:|:|||||:||||||||||:|||||| | ||||||||:||||||
orf3ng        IKEGISAQGEATMPPFAGNRKLAVIGAGGHGKVVAELAAALGTYGEIVFLDDRTQGSVNG
                         190       200       210       220       230       240

                         250       260       270       280       290       300
orf3-1.pep    FSVIGTTLLLENSLSPEQYDVAVAVGNNRIRRQIAEKAAALGFALPVLVHPDATVSPSAT
              | ||||||||||||||||:|::||||||||||||:|:|||||| ||||:|||||||||| 
orf3ng        FPVIGTTLLLENSLSPEQFDITVAVGNNRIRRQITENAAALGFKLPVLIHPDATVSPSAI
                         250       260       270       280       290       300

                         310       320       330       340       350       360
orf3-1.pep    VGQGSVVMAKAVVQAGSVLKDGVIVNTAATVDHDCLLNAFVHISPGAHLSGNTHIGEESW
              :||||||||||||||||||||||||||||||||||||:|||||||||||||||:||||| 
orf3ng        IGQGSVVMAKAVVQAGSVLKDGVIVNTAATVDHDCLLDAFVHISPGAHLSGNTRIGEESR
                         310       320       330       340       350       360

                         370       380       390       400       410
orf3-1.pep    IGTGACSRQQIRIGSRATIGAGAVVVRDVSDGMTVAGNPAKPLPRKNPETSTAX
              ||||||||||  :|| :| |||||:| |: |||||||||||||  |||:|:|||
orf3ng        IGTGACSRQQTTVGSGVTAGAGAVIVCDIPDGMTVAGNPAKPLTGKNPKTGTAX
                         370       380       390       400       410
```

In addition, ORF3ng shows significant homology with a hypothetical protein from *B.subtilis:*

```
gnl|PID|e238668 (Z71928) hypothetical protein [Bacillus subtilis]
>gi|1945702|gnl|PID|e313004 (Z94043) hypothetical protein [Bacillus subtilis]
>gi|2635938|gnl|PID|e1186113 (Z99121) similar to capsular polysaccharide
biosynthesis [Bacillus subtilis]Length = 202
 Score =  235 bits (594), Expect = 3e-61
 Identities = 114/195 (58%), Positives = 142/195 (72%)

Query: 5    VKRLFDIIASASGLIVLSPVFLVLIYLIRKNLGSPVFFIRERPGKDGKPFKMVKFRSMRD 64
             +KRLFD+ A+    L    S + L   I ++R  +GSPVFF + RPG  GKPF + KFR+M D
Sbjct: 3    LKRLFDLTAAIFLLCCTSVIILFTIAVVRLKIGSPVFFKQVRPGLHGKPFTLYKFRTMTD 62

Query: 65   ALDSDGIPLPDSERLTDFGKKLRATSLDELPELWNVLKGEMSLVGPRPLLMQYLPLYNKF 124
               DS G  LPD  RLT  G+ +R  S+DELP+L NVLKG++SLVGPRPLLM YLPLY +
Sbjct: 63   ERDSKGNLLPDEVRLTKTGRLIRKLSIDELPQLLNVLKGDLSLVGPRPLLMDYLPLYTEK 122

Query: 125  QNRRHEMKPGITGWAQVNGRNALSWDEKFSCDVWYTDNFSFWLDMKILFLTVKKVLIKEG 184
             Q RRHE+KPGITGWAQ+NGRNA+SW++KF  DVWY DN+SF+LD+KIL LTV+KVL+ EG
Sbjct: 123  QARRHEVKPGITGWAQINGRNAISWEKKFELDVWYVDNWSFFLDLKILCLTVRKVLVSEG 182

Query: 185  ISAQGEATMPPFAGN 199
             I       T   F G+
Sbjct: 183  IQQTNHVTAERFTGS 197
```

The hypothetical product of *yvfc* gene shows similarity to EXOY of *R.meliloti,* an exopolysaccharide production protein. Based on this and on the two predicted transmembrane regions in the homologous *N.gonorrhoeae* sequence, it is predicted that these proteins, or their epitopes, could be useful antigens for vaccines or diagnostics, or for raising antibodies.

**Example 4**

The following partial DNA sequence was identified in *N.meningitidis* <SEQ ID 19>:

```
  1  ..AACCATATGG CGATTGTCAT CGACGAATAC GGCGGCACAT CCGGCTTGGT
 51    CACCTTTGAA GACATCATCG AGCAAATCGT CGGCGAAATC GAAGACGAGT
101    TTGACGAAGA CGATAGCGCC GACAATATCC ATGCCGTTTC TTCAGACACG
151    TGGCGCATCC ATGCAGCTAC CGAAATCGAA GACATCAACA CCTTCTTCGG
201    CACGGAATAC AGCATCGAAG AAGCCGACAC CATT.GGCGG CCTGGTCATT
251    CAAGAGTTGG GACATCTGCC CGTGCGCGGC GAAAAAGTCC TTATCGGCGG
301    TTTGCAGTTC ACCGTCGCAC GCGCCGACAA CCGCCGCCTG CATACGCTGA
351    TGGCGACCCG CGTGAAGTAA GC........ .....ACCGC CGTTTCTGCA
401    CAGTTTAG
```

This corresponds to amino acid sequence <SEQ ID 20; ORF5>:

```
  1  ..NHMAIVIDEY GGTSGLVTFE DIIEQIVGEI EDEFDEDDSA DNIHAVSSDT
 51    WRIHAATEIE DINTFFGTEY SIEEADTIXR PGHSRVGTSA RARRKSPYRR
101    FAVHRRTRRQ PPPAYADGDP REVS....XR RFCTV*
```

Further sequence analysis revealed the complete DNA sequence to be <SEQ ID 21>:

```
  1 ATGGACGGCG CACAACCGAA AACGAATTTT TTTGAACGCC TGATTGCCCG
 51 ACTCGCCCGC GAACCCGATT CCGCCGAAGA CGTATTAAAC CTGCTTCGGC
101 AGGCGCACGA GCAGGAAGTT TTTGATGCGG ATACGCTTTT AAGATTGGAA
151 AAAGTCCTCG ATTTTTCCGA TTTGGAAGTG CGCGACGCGA TGATTACGCG
201 CAGCCGTATG AACGTTTTAA AAGAAAAACGA CAGCATCGAG CGCATCACCG
251 CCTACGTTAT CGATACCGCC CATTCGCGCT TCCCCGTCAT CGGCGAAGAC
301 AAAGACGAAG TTTTGGGCAT TTTGCACGCC AAAGACCTGC TCAAATATAT
351 GTTTAACCCC GAGCAGTTCC ACCTCAAATC CATTCTCCGC CCCGCCGTCT
401 TCGTCCCCGA AGGCAAATCG CTGACCGCCC TTTTAAAAGA GTTCCGCGAA
451 CAGCGCAACC ATATGGCGAT TGTCATCGAC GAATACGGCG GCACATCCGG
501 CTTGGTCACC TTTGAAGACA TCATCGAGCA AATCGTCGGC GAAATCGAAG
551 ACGAGTTTGA CGAAGACGAT AGCGCCGACA ATATCCATGC CGTTTCTTCC
601 GAACGCTGGC GCATCCATGC AGCTACCGAA ATCGAAGACA TCAACACCTT
651 CTTCGGCACG GAATACAGCA GCGAAGAAGC CGACACCATT CGGCCTGGTC
701 ATTCAAGAGT TGGGACATCT GCCCGTGCGC GGCGAAAAAG TCCTTATCGG
751 CGGTTTGCAG TTCACCGTCG CACGCGCCGA CAACCGCCGC CTGCATACGC
801 TGATGGCGAC CCGCGTGAAG TAAGCACCGC CGTTTCTGCA CAGTTTAGGA
851 TGACGGTACG GGCGTTTTCT GTTTCAATCC GCCCCATCCG CCAAACATAA
```

This corresponds to amino acid sequence <SEQ ID 22; ORF5-1>:

```
  1 MDGAQPKTNF FERLIARLAR EPDSAEDVLN LLRQAHEQEV FDADTLLRLE
 51 KVLDFSDLEV RDAMITRSRM NVLKENDSIE RITAYVIDTA HSRFPVIGED
101 KDEVLGILHA KDLLKYMFNP EQFHLKSILR PAVFVPEGKS LTALLKEFRE
151 QRNHMAIVID EYGGTSGLVT FEDIIEQIVG EIEDEFDEDD SADNIHAVSS
201 ERWRIHAATE IEDINTFFGT EYSSEEADTI RPGHSRVGTS ARARRKSPYR
251 RFAVHRRTRR QPPPAYADGD PREVSTAVSA QFRMTVRAFS VSIRPIRQT*
```

Further work identified the corresponding gene in strain A of *N. meningitidis* <SEQ ID 23 >:

```
  1 ATGGACGGCG CACAACCGAA AACAAATTTT TTNNAACGCC TGATTGCCCG
 51 ACTCGCCCGC GAACCCGATT CCGCCGAAGA CGTATTGACC CTGTTGCGCC
101 AAGCGCACGA ACAGGAAGTA TTTGATGCGG ATACGCTTTT AAGATTGGAA
151 AAAGTCCTCG ATTTTTCTGA TTTGGAAGTG CGCGACGCGA TGATTACGCG
201 CAGCCGTATG AACGTTTTAA AAGAAAACGA CAGCATCGAA CGCATCACCG
251 CCTACGTTAT CGATACCGCC CATTCGCGCT TCCCCGTCAT CGGTGAAGAC
301 AAAGACGAAG TTTTGGGTAT TTTGCACGCC AAAGACCTGC TCAAATATAT
351 GTTCAACCCC GAGCAGTTCC ACCTCAAATC GATATTGCGC CCTGCCGTCT
401 TCGTCCCCGA AGGCAAATCG CTGACCGCCC TTTTAAAAGA GTTCCGCGAA
451 CAGCGCAACC ATATGGCAAT CGTCATCGAC GAATACGGCG GCACGTCGGG
501 TTTGGTAACT TTTGAAGACA TCATCGAGCA AATCGTCGGC GACATCGAAG
551 ATGAGTTTGA CGAAGACGAA AGCGCGGACA ACATCCACGC CGTTTCCGCC
601 GAACGCTGGC GCATCCACGC GGCTACCGAA ATCGAAGACA TCAACGCCTT
651 TTTCGGCACG GAATACAGCA GCGAAGAAGC CGACACCATC GGCGGCCNTG
701 GTCATTCAGG AATTGGNACA CCTGCCCGTG CGCGGCGAAA AAGTCNTTAT
751 CGGCGNNTTG CANTTCACNG TCGCCNGCGC NGACAACCGC CGCCTGCATA
801 CGCTGATGGC GACCCGCGTG AAGTAAGCTC CGCCGTTTCT GTACAGTTTA
851 GGATGACGGT ACGGGCGTTT TCTGTTTCAA TCCGCCCCAT CCGCCANACA
901 TAA
```

This encodes a protein having amino acid sequence <SEQ ID 24; ORF5a>:

```
  1 MDGAQPKTNF XXRLIARLAR EPDSAEDVLT LLRQAHEQEV FDADTLLRLE
 51 KVLDFSDLEV RDAMITRSRM NVLKENDSIE RITAYVIDTA HSRFPVIGED
101 KDEVLGILHA KDLLKYMFNP EQFHLKSILR PAVFVPEGKS LTALLKEFRE
151 QRNHMAIVID EYGGTSGLVT FEDIIEQIVG DIEDEFDEDE SADNIHAVSA
201 ERWRIHAATE IEDINAFFGT EYSSEEADTI GGXGHSGIGT PARARRKSXY
251 RRXAXHXRXR XQPPPAYADG DPREVSSAVS VQFRMTVRAF SVSIRPIRXT
301 *
```

The originally-identified partial strain B sequence (ORF5) shows 54.7% identity over a 124aa overlap with ORF5a:

```
                                                          10        20        30
orf5.pep                                      NHMAIVIDEYGGTSGLVTFEDIIEQIVGEI
                                              ||||||||||||||||||||||||||||:|
orf5a             FHLKSILRPAVFVPEGKSLTALLKEFREQRNHMAIVIDEYGGTSGLVTFEDIIEQIVGDI
                         130       140       150       160       170       180

                          40        50        60        70        80        90
orf5.pep          EDEFDEDDSADNIHAVSSDTWRIHAATEIEDINTFFGTEYSIEEADTIXRPGHSRVGTSA
                  |||||||:|||||||||:: |||||||||||||:||||||| ||||||   ||| :|| |
orf5a             EDEFDEDESADNIHAVSAERWRIHAATEIEDINAFFGTEYSSEEADTIGGXGHSGIGTPA
                         190       200       210       220       230       240

                         100       110       120       130
orf5.pep          RARRKSPYRRFAVHRRTRRQPPPAYADGDPREVSXXXXXXRRFCTV
                  |||||| ||| | | |:| |||||||||||||||
orf5a             RARRKSXYRRXAXHXRXRXQPPPAYADGDPREVSSAVSVQFRMTVRAFSVSIRPIRXTX
                         250       260       270       280       290       300
```

The complete strain B sequence (ORF5-1) and ORF5a show 92.7% identity in 300 aa overlap:

```
                          10        20        30        40        50        60
orf5a.pep         MDGAQPKTNFXXRLIARLAREPDSAEDVLTLLRQAHEQEVFDADTLLRLEKVLDFSDLEV
                  ||||||||||  |||||||||||||||||:||||||||||||||||||||||||||||||
orf5-1            MDGAQPKTNFFERLIARLAREPDSAEDVLNLLRQAHEQEVFDADTLLRLEKVLDFSDLEV
                          10        20        30        40        50        60

                          70        80        90       100       110       120
orf5a.pep         RDAMITRSRMNVLKENDSIERITAYVIDTAHSRFPVIGEDKDEVLGILHAKDLLKYMFNP
                  ||||||||||||||||||||||||||||||||||||||||||||||||||||||||||||
orf5-1            RDAMITRSRMNVLKENDSIERITAYVIDTAHSRFPVIGEDKDEVLGILHAKDLLKYMFNP
                          70        80        90       100       110       120

                         130       140       150       160       170       180
orf5a.pep         EQFHLKSILRPAVFVPEGKSLTALLKEFREQRNHMAIVIDEYGGTSGLVTFEDIIEQIVG
                  ||||||||||||||||||||||||||||||||||||||||||||||||||||||||||||
orf5-1            EQFHLKSILRPAVFVPEGKSLTALLKEFREQRNHMAIVIDEYGGTSGLVTFEDIIEQIVG
                         130       140       150       160       170       180

                         190       200       210       220       230       240
orf5a.pep         DIEDEFDEDESADNIHAVSAERWRIHAATEIEDINAFFGTEYSSEEADTIGGXGHSGIGT
                  :||||||||:|||||||||:|||||||||||||||:||||||||||||||   ||| :||
orf5-1            EIEDEFDEDDSADNIHAVSSERWRIHAATEIEDINTFFGTEYSSEEADTIRP-GHSRVGT
                         190       200       210       220       230

                         250       260       270       280       290       300
orf5a.pep         PARARRKSXYRRXAXHXRXRXQPPPAYADGDPREVSSAVSVQFRMTVRAFSVSIRPIRXT
                   ||||||| ||| | | |:| |||||||||||||||:|||:||||||||||||||||| |
orf5-1            SARARRKSPYRRFAVHRRTRRQPPPAYADGDPREVSTAVSAQFRMTVRAFSVSIRPIRQT
                240       250       260       270       280       290
```

Further work identified the a partial DNA sequence in *N.gonorrhoeae* <SEQ ID 25> which encodes a protein having amino acid sequence <SEQ ID 26; ORF5ng>:

```
  1  MDGAQPKTNF FERLIARLAR EPDSAEDVLN LLRQAHEQEV FDADTLTRLE
 51  KVLDFAELEV RDAMITRSRM NVLKENDSIE RITAYVIDTA HSRFPVIGED
101  KDEVLGILHA KDLLKYMFNP EQFHLKSVLR PAVFVPEGKS LTALLKEFRE
151  QRNHMAIVID EYGGTSGLVT FEDIIEQIVG DIEDEFDEDE SADDIHSVSA
201  ERWRIHAATE IEDINAFFGT EYGSEEADTI RRLGHSGIGT PARARRKSPY
251  RRFAVHRRPR RQPPPAHADG DPREVSRACP HRRFCTV*
```

Further analysis revealed the complete gonococcal nucleotide sequence <SEQ ID 27> to be:

```
  1 ATGGACGGCG CACAACCGAA AACAAATTTT TTTGAACGCC TGATTGCCCG
 51 ACTCGCCCGC GAACCCGATT CCGCCGAAGA CGTATTAAAC CTGCTTCGGC
101 AGGCGCACGA ACAGGAAGTT TTTGATGCCG ACACACTGAC CCGGCTGGAA
151 AAAGTATTGG ACTTTGCCGA GCTGGAAGTG CGCGATGCGA TGATTACGCG
201 CAGCCGCATG AACGTATTGA AAGAAAACGA CAGCATCGAA CGCATCACCG
251 CCTACGTCAT CGATACCGCC CATTCGCGCT TCCCCGTCAT CGGCGAAGAC
301 AAAGACGAAG TTTTGGGCAT TTTGCACGCC AAAGACCTGC TCAAATATAT
351 GTTCAACCCC GAGCAGTTCC ACCTGAAATC CGTCTTGCGC CCTGCCGTTT
401 TCGTGCCCGA AGGCAAATCT TTGACCGCCC TTTTAAAAGA GTTCCGCGAA
451 CAGCGCAACC ATATGGCAAT CGTCATCGAC GAATACGGCG GCACGTCGGG
501 TTTGGTCACC TTTGAAGACA TCATCGAGCA AATCGTCGGT GACATCGAAG
551 ACGAGTTTGA CGAAGACGAA AGCGccgacg acatCCACTC cgTTTccgCC
601 GAACGCTGGC GCATCCacgc ggctaCCGAA ATCGAAGaca TCAACGCCTT
651 TTTCGGTACG GAatacggca gcgaagaagc cgacaccatc cggcggctTG
701 GTCATTCAGG AATTGGGACA CCTGCCCGTG CGCGGCGAAA AAGTCCTTAt
751 cggcgGTTTG Cagttcaccg tCGCCCGCGC CGACAACCGC CGCCTGCACA
801 CGCTGATGGC GACCCGCGTG AAGTAAGCAG AGCCTGCCcg AccgccgttT
851 CTGCacAGTT TAGGatgACG gtaCGGTCGT TTTCTGTTTC AATCCGCCCC
901 ATCCGCCAAA CATAA
```

This encodes a protein having amino acid sequence <SEQ ID 28; ORF5ng-1>:

```
  1 MDGAQPKTNF FERLIARLAR EPDSAEDVLN LLRQAHEQEV FDADTLTRLE
 51 KVLDFAELEV RDAMITRSRM NVLKENDSIE RITAYVIDTA HSRFPVIGED
101 KDEVLGILHA KDLLKYMFNP EQFHLKSVLR PAVFVPEGKS LTALLKEFRE
151 QRNHMAIVID EYGGTSGLVT FEDIIEQIVG DIEDEFDEDE SADDIHSVSA
201 ERWRIHAATE IEDINAFFGT EYGSEEADTI RRLGHSGIGT PARARRKSPY
251 RRFAVHRRPR RQPPPAHADG DPREVSRACP TAVSAQFRMT VRSFSVSIRP
301 IRQT*
```

The originally-identified partial strain B sequence (ORF5) shows 83.1% identity over a 135aa overlap with the partial gonococcal sequence (ORF5ng):

```
orf5                                          NHMAIVIDEYGGTSGLVTFEDIIEQIVGEI   30
                                              ||||||||||||||||||||||||||||:|
orf5ng        FHLKSVLRPAVFVPEGKSLTALLKEFREQRNHMAIVIDEYGGTSGLVTFEDIIEQIVGDI  182

orf5          EDEFDEDDSADNIHAVSSDTWRIHAATEIEDINTFFGTEYSIEEADTIXRPGHSRVGTSA   90
              |||||||:|||:||:||:: |||||||||||||:||||||: |||||| | ||| :|| |
orf5ng        EDEFDEDESADDIHSVSAERWRIHAATEIEDINAFFGTEYGSEEADTIRRLGHSGIGTPA  242

orf5          RARRKSPYRRFAVHRRTRRQPPPAYADGDPREVSX----RRFCTV  131
              |||||||||||||||| |||||||:||||||||| ||||||
orf5ng        RARRKSPYRRFAVHRRPRRQPPPAHADGDPREVSRACPHRRFCTV  287
```

The complete strain B and gonococcal sequences (ORF5-1 & ORFSng-1) show 92.4% identity in 304 aa overlap:

```
                        10        20        30        40        50        60
orf5ng-1.pep  MDGAQPKTNFFERLIARLAREPDSAEDVLNLLRQAHEQEVFDADTLTRLEKVLDFAELEV
              |||||||||||||||||||||||||||||||||||||||||||||| ||||||||::|||
orf5-1        MDGAQPKTNFFERLIARLAREPDSAEDVLNLLRQAHEQEVFDADTLLRLEKVLDFSDLEV
                        10        20        30        40        50        60

                        70        80        90       100       110       120
orf5ng-1.pep  RDAMITRSRMNVLKENDSIERITAYVIDTAHSRFPVIGEDKDEVLGILHAKDLLKYMFNP
              ||||||||||||||||||||||||||||||||||||||||||||||||||||||||||||
orf5-1        RDAMITRSRMNVLKENDSIERITAYVIDTAHSRFPVIGEDKDEVLGILHAKDLLKYMFNP
                        70        80        90       100       110       120

                       130       140       150       160       170       180
orf5ng-1.pep  EQFHLKSVLRPAVFVPEGKSLTALLKEFREQRNHMAIVIDEYGGTSGLVTFEDIIEQIVG
              |||||||:||||||||||||||||||||||||||||||||||||||||||||||||||||
orf5-1        EQFHLKSILRPAVFVPEGKSLTALLKEFREQRNHMAIVIDEYGGTSGLVTFEDIIEQIVG
                       130       140       150       160       170       180

                       190       200       210       220       230       240
orf5ng-1.pep  DIEDEFDEDESADDIHSVSAERWRIHAATEIEDINAFFGTEYGSEEADTIRRLGHSGIGT
              :||||||||:|||:||:||:|||||||||||||||:||||||:||||||||  ||| :||
orf5-1        EIEDEFDEDDSADNIHAVSSERWRIHAATEIEDINTFFGTEYSSEEADTIRP-GHSRVGT
                       190       200       210       220       230


                        250       260       270       280       290       300
orf5ng-1.pep   PARARRKSPYRRFAVHRRPRRQPPPAHADGDPREVSRACPTAVSAQFRMTVRSFSVSIRP
                ||||||||||||||||| |||||||:|||||||||    ||||||||||||:|||||||
orf5-1         SARARRKSPYRRFAVHRRTRRQPPPAYADGDPREVS----TAVSAQFRMTVRAFSVSIRP
             240       250       260       270           280       290

orf5ng-1.pep   IRQTX
               |||||
orf5-1         IRQTX
                 300
```

Computer analysis of these amino acid sequences indicates a putative leader sequence, and identified the following homologies:

Homology with hemolysin homolog TlyC (accession U32716) of *H.influenzae*

ORF5 and TlyC proteins show 58% aa identity in 77 aa overlap (BLASTp).

```
ORF5    2  HMAIVIDEYGGTSGLVTFEDIIEQIVGEIEDEFDEDDSADNIHAVSSDTWRIHAATEIED 61
           HMAIV+DE+G  SGLVT EDI+EQIVG+IEDEFDE++ AD I  +S  T+ + A T+I+D
TlyC  166  HMAIVVDEFGAVSGLVTIEDILEQIVGDIEDEFDEEEIAD-IRQLSRHTYAVRALTDIDD 224

ORF5   62  INTFFGTEYSIEEADTI 78
            N  F T++  EE DTI
TlyC  225  FNAQFNTDFDDEEVDTI 241
```

ORF5ng-1 also shows significant homology with TlyC:

```
SCORES        Init1:    301 Initn:    419 Opt:    668
Smith-Waterman score: 668;     45.9% identity in 242 aa overlap

                             10        20        30        40        50
orf5ng-1.pep         MDGAQPKTNFFERLIARLAR-EPDSAEDVLNLLRQAHEQEVFDADTLTRLEK
                             |  ||: |::|: : |   : |::::::|:::::::::| :|   :|
tlyc_haein   MNDEQQNSNQSENTKKPFFQSLFGRFFQGELKNREELVEVIRDSEQNDLIDQNTREMIEG
                     10        20        30        40        50        60

                    60        70        80        90       100       109
orf5ng-1.pep VLDFAELEVRDAMITRSRMNVLKENDSIERITAYVIDTAHSRFPVIGE--DKDEVLGILH
             |:::|||:|||  ||  ||::   ::::::::      :|::|||||||||::   |:|:::||||
tlyc_haein   VMEIAELRVRDIMIPRSQIIFIEDQQDLNTCLNTIIESAHSRFPVIADADDRDNIVGILH
                     70        80        90       100       110       120

          110       120       130       140       150       160
orf5ng-1.pep AKDLLKYMF-NPEQFHLKSVLRPAVFVPEGKSLTALLKEFREQRNHMAIVIDEYGGTSGL
             ||||||::   :  |  |  |:|:|||:|:|||:|  :   :||:|| :|  ||||:||:|::|||
tlyc_haein   AKDLLKFLREDAEVFDLSSLLRPVVIVPESKRVDRMLKDFRSERFHMAIVVDEFGAVSGL
                     130       140       150       160       170       180

           170       180       190       200       210       220
orf5ng-1.pep VTFEDIIEQIVGDIEDEFDEDESADDIHSVSAERWRIHAATEIEDINAFFGTEYGSEEAD
             ||:|||:|||||||||||||||:|  ||  |:::|  :  :  ::|  |:|:|:||  |:|::  :||:|
tlyc_haein   VTIEDILEQIVGDIEDEFDEEEIAD-IRQLSRHTYAVRALTDIDDFNAQFNTDFDDEEVD
                     190       200        210       220       230

           230        240       250       260       270       280
orf5ng-1.pep TIRRLGHSGIG-TPARARRKSPYRRFAVHRRPRRQPPPAHADGDPREVSRACPTAVSAQF
             ||   |   :  :|   |  |:
tlyc_haein   TIGGLIMQTFGYLPKRGEEIILKNLQFKVTSADSRRLIQLRVTVPDEHLAEMNNVDEKSE
           240       250       260       270       280       290
```

<u>Homology with a hypothetical secreted protein from *E.coli:*</u>

ORF5a shows homology to a hypothetical secreted protein from *E.coli:*

```
sp|P77392|YBEX_ECOLI HYPOTHETICAL 33.3 KD PROTEIN IN CUTE-ASNB INTERGENIC REGION
```

>gi|1778577 (U82598) similar to *H. influenzae* [*Escherichia coli*] >gi|1786879 (AE000170) f292; This 292 aa ORF is 23% identical (9 gaps) to 272 residues of an approx. 440 aa protein YTFL_HAEIN SW: P44717 [*Escherichia coli*] Length = 292

```
 Score =  212 bits (533), Expect = 3e-54
 Identities = 112/230 (48%), Positives = 149/230 (64%), Gaps = 3/230 (1%)

Query: 2   DGAQPKTNFXXRLIARLAR-EPDSAEDVLTLLRQAHEQEVFDADTLLRLEKVLDFSDLEV 60
           D    K  F   L+++L    EP + +++L L+R + + ++ D DT   LE V+D +D  V
Sbjct: 10  DTISNKKGFFSLLLSQLFHGEPKNRDELLALIRDSGQNDLIDEDTRDMLEGVMDIADQRV 69

Query: 61  RDAMITRSRMNVLKENDSIERITAYVIDTAHSRFPVIGEDKDEVLGILHAKDLLKYM-FN 119
           RD MI RS+M  LK N +++      +I++AHSRFPVI EDKD + GIL AKDLL +M  +
Sbjct: 70  RDIMIPRSQMITLKRNQTLDECLDVIIESAHSRFPVISEDKDHIEGILMAKDLLPFMRSD 129

Query: 120 PEQFHLKSILRPAVFVPEGKSLTALLKEFREQRNHMAIVIDEYGGTSGLVTFEDIIEQIV 179
            E F +   +LR AV VPE K +   +LKEFR QR HMAIVIDE+GG SGLVT EDI+E IV
Sbjct: 130 AEAFSMDKVLRQAVVVPESKRVDRMLKEFRSQRYHMAIVIDEFGGVSGLVTIEDILELIV 189

Query: 180 GDIEDEFDEDESADNIHAVSAERWRIHAATEIEDINAFFGTEYSSEEADT 229
           G+IEDE+DE++  D    +S   W + A   IED N  FGT +S EE DT
Sbjct: 190 GEIEDEYDEEDDID-FRQLSRHTWTVRALASIEDFNEAFGTHFSDEEVDT 238
```

Based on this analysis, including the amino acid homology to the TlyC hemolysin-homologue from *H. influenzae* (hemolysins are secreted proteins), it was predicted that the proteins from *N.meningitidis* and *N.gonorrhoeae* are secreted and could thus be useful antigens for vaccines or diagnostics.

ORFS-1 (30.7kDa) was cloned in the pGex vector and expressed in *E.coli,* as described above. The products of protein expression and purification were analyzed by SDS-PAGE. Figure 2A shows the results of affinity purification of the GST-fusion protein. Purified GST-fusion protein was used to immunise mice, whose sera were used for Western blot analysis (Figure 1B). These experiments confirm that ORF5-1 is a surface-exposed protein, and that it is a useful immunogen.

## Example 5

The following partial DNA sequence was identified in *N.meningitidis* <SEQ ID 29>:

```
  1   ATGCGCGGCG GCAGGCCGGA TTCCGTTACC GTGCAGATTA TCGAAGGTTC
 51   GCGTTTTTCG CATATGAGGA AAGTCATCGA CGCAACGCCC GACATCGGAC
101   ACGACACCAA AGGCTGGAGC AATGAAAAAC TGATGGCGGA AGTTGCGCCC
151   GATGCCTTCA GCGGCAATCC TGAAgGGCAG TTTTTCCCCG ACAGCTACGA
201   AATCGATGCG GGCGGCAGTG ATTTGCAGAT TTACCAAACC GCCTACAAgG
251   GCGATGCAAC GCCGCCTGAA TGAgGGCATG GGAAAGCAGG CAGGACGGGC
301   TGCCTTATAA AAACCCTTAT GAAATGCTGA TTATGGCGAr CCTGGTCGAA
351   AAGGAAACAG GGCATGAAGC CGAsCsCGAC CATGTcGCTT CCGTCTTCGT
401   CAACCGCCTG AAAATCGGTA TGCGCCTGCA AACCgAssCG TCCGTGATTT
451   ACGGCATGGG TGCGGCATAC AAGGGCAAAA TCCGTAAAGC CGACCTGCGC
501   CGCGACACGC CGTACAACAC CTACACGCGC GGCGGTCTGC CGCCAACCCC
551   GATTGCGCTG CCC..
```

This corresponds to the amino acid sequence <SEQ ID 30; ORF7>:

```
  1   MRGGRPDSVT VQIIEGSRFS HMRKVIDATP DIGHDTKGWS NEKLMAEVAP
 51   DAFSGNPEGQ FFPDSYEIDA GGSDLQIYQT AYKAMQRRLN EAWESRQDGL
101   PYKNPYEMLI MAXLVEKETG HEAXXDHVAS VFVNRLKIGM RLQTXXSVIY
151   GMGAAYKGKI RKADLRRDTP YNTYTRGGLP PTPIALP..
```

Further sequence analysis revealed the complete DNA sequence <SEQ ID 31>:

```
  1   ATGTTGAGAA AATTGTTGAA ATGGTCTGCC GTTTTTTTGA CCGTGTCGGC
 51   AGCCGTTTTC GCCGCGCTGC TTTTTGTTCC TAAGGATAAC GGCAGGGCAT
101   ACCGAATCAA AATTGCCAAA AACCAGGGTA TTTCGTCGGT CGGCAGGAAA
151   CTTGCCGAAG ACCGCATCGT GTTCAGCAGG CATGTTTTGA CGGCGGCGGC
201   CTACGTTTTG GGTGTGCACA ACAGGCTGCA TACGGGGACG TACAGATTGC
251   CTTCGGAAGT GTCTGCTTGG GATATCTTGC AGAAAATGCG CGGCGGCAGG
301   CCGGATTCCG TTACCGTGCA GATTATCGAA GGTTCGCGTT TTTCGCATAT
351   GAGGAAAGTC ATCGACGCAA CGCCCGACAT CGGACACGAC ACCAAAGGCT
401   GGAGCAATGA AAAACTGATG GCGGAAGTTG CGCCCGATGC CTTCAGCGGC
451   AATCCTGAAG GGCAGTTTTT CCCCGACAGC TACGAAATCG ATGCGGGCGG
501   CAGTGATTTG CAGATTTACC AAACCGCCTA CAAGGCGATG CAACGCCGCC
551   TGAATGAGGC ATGGGAAAGC AGGCAGGACG GGCTGCCTTA TAAAAACCCT
601   TATGAAATGC TGATTATGGC GAGCCTGGTC GAAAAGGAAA CAGGGCATGA
651   AGCCGACCGC GACCATGTCG CTTCCGTCTT CGTCAACCGC CTGAAAATCG
701   GTATGCGCCT GCAAACCGAC CCGTCCGTGA TTTACGGCAT GGGTGCGGCA
751   TACAAGGGCA AAATCCGTAA AGCCGACCTG CGCCGCGACA CGCCGTACAA
801   CACCTACACG CGCGGCGGTC TGCCGCCAAC CCCGATTGCG CTGCCCGGCA
851   AGGCGGCACT CGATGCCGCC GCCCATCCGT CCGGCGAAAA ATACCTGTAT
901   TTCGTGTCCA AAATGGACGG CACGGGCTTG AGCCAGTTCA GCCATGATTT
951   GACCGAACAC AATGCCGCCG TCCGCAAATA TATTTTGAAA AAATAA
```

This corresponds to the amino acid sequence <SEQ ID 32; ORF7-1>:

```
  1  MLRKLLKWSA VFLTVSAAVF AALLFVPKDN GRAYRIKIAK NQGISSVGRK
 51  LAEDRIVFSR HVLTAAAYVL GVHNRLHTGT YRLPSEVSAW DILQKMRGGR
101  PDSVTVQIIE GSRFSHMRKV IDATPDIGHD TKGWSNEKLM AEVAPDAFSG
151  NPEGQFFPDS YEIDAGGSDL QIYQTAYKAM QRRLNEAWES RQDGLPYKNP
201  YEMLIMASLV EKETGHEADR DHVASVFVNR LKIGMRLQTD PSVIYGMGAA
251  YKGKIRKADL RRDTPYNTYT RGGLPPTPIA LPGKAALDAA AHPSGEKYLY
301  FVSKMDGTGL SQFSHDLTEH NAAVRKYILK K*
```

Computer analysis of this amino acid sequence gave the following results:

<u>Homology with hypothetical protein encoded by *yceg* gene (accession P44270) of *H.influenzae*</u>

ORF7 and yceg proteins show 44% aa identity in 192 aa overlap:

```
ORF7    1  MRGGRPDSVTVQIIEGSRFSHMRKVIDATPDIGHDTKGWSNEKLMA-----EVAPDAFSG 55
           + G+     V+ IEG  F   RK ++  P +    K  SNE++ A     ++  +
yceg  102  LNSGKEVQFNVKWIEGKTFKDWRKDLENAPHLVQTLKDKSNEEIFALLDLPDIGQNLELK 161

ORF7   56  NPEGQFFPDSYEIDAGGSDLQIYQTAYKAMQRRLNEAWESRQDGLPYKNPYEMLIMAXLV 115
           N EG  +PD+Y      +DL++ + + + M++ LN+AW  R + LP  NPYEMLI+A +V
yceg  162  NVEGWLYPDTYNYTPKSTDLELLKRSAERMKKALNKAWNERDEDLPLANPYEMLILASIV 221

ORF7  116  EKETGHEAXXDHVASVFVNRLKIGMRLQTXXSVIYGMGAAYKGKIRKADLRRDTPYNTYT 175
           EKETG        VASVF+NRLK  M+LQT  +VIYGMG  Y G IRK DL   TPYNTY
yceg  222  EKETGIANERAKVASVFINRLKAKMKLQTDPTVIYGMGENYNGNIRKKDLETKTPYNTYV 281

ORF7  176  RGGLPPTPIALP 187
             GLPPTPIA+P
yceg  282  IDGLPPTPIAMP 293
```

The complete length YCEG protein has sequence:

```
  1  MKKFLIAILL LILILAGVAS FSYYKMTEFV KTPVNVQADE LLTIERGTTS
 51  SKLATLFEQE KLIADGKLLP YLLKLKPELN KIKAGTYSLE NVKTVQDLLD
101  LLNSGKEVQF NVKWIEGKTF KDWRKDLENA PHLVQTLKDK SNEEIFALLD
151  LPDIGQNLEL KNVEGWLYPD TYNYTPKSTD LELLKRSAER MKKALNKAWN
201  ERDEDLPLAN PYEMLILASI VEKETGIANE RAKVASVFIN RLKAKMKLQT
251  DPTVIYGMGE NYNGNIRKKD LETKTPYNTY VIDGLPPTPI AMPSESSLQA
301  VANPEKTDFY YFVADGSGGH KFTRNLNEHN KAVQEYLRWY RSQKNAK
```

<u>Homology with a predicted ORF from *N.meningitidis* (strain A)</u>

ORF7 shows 95.2% identity over a 187aa overlap with an ORF (ORF7a) from strain A of *N. meningitidis:*

```
                10        20        30
```

```
orf7.pep                                      MRGGRPDSVTVQIIEGSRFSHMRKVIDATP
                                              ||||||||||||||||||||||||||||||
orf7a           AAYVLGVHNRLHTGTYRLPSEVSAWDILQKMRGGRPDSVTVQIIEGSRFSHMRKVIDATP
                       70        80        90       100       110       120

                       40        50        60        70        80        90
orf7.pep        DIGHDTKGWSNEKLMAEVAPDAFSGNPEGQFFPDSYEIDAGGSDLQIYQTAYKAMQRRLN
                || ||||||||||||||||||||||||||||||||||||||||||:||| ||||||||||
orf7a           DIEHDTKGWSNEKLMAEVAPDAFSGNPEGQFFPDSYEIDAGGSDLRIYQIAYKAMQRRLN
                      130       140       150       160       170       180

                      100       110       120       130       140       150
orf7.pep        EAWESRQDGLPYKNPYEMLIMAXLVEKETGHEAXXDHVASVFVNRLKIGMRLQTXXSVIY
                |||||||||||||||||||||| |:||||||||  |||||||||||||||||||  ||||
orf7a           EAWESRQDGLPYKNPYEMLIMASLIEKETGHEADRDHVASVFVNRLKIGMRLQTDPSVIY
                      190       200       210       220       230       240

                      160       170       180
orf7.pep        GMGAAYKGKIRKADLRRDTPYNTYTRGGLPPTPIALP
                |||||||||||||||||||||||||||||||||||||
orf7a           GMGAAYKGKIRKADLRRDTPYNTYTRGGLPPTPIALPGKAALDAAAHPSGEKYLYFVSKM
                      250       260       270       280       290       300

orf7a           DGTGLSQFSHDLTEHNAAVRKYILKKX
                      310       320       330
```

The complete length ORF7a nucleotide sequence <SEQ ID 33> is:

```
  1 ATGTTGAGAA AATTGTTGAA ATGGTCTGCC GTTTTTTTGA CCGTATCGGC
 51 AGCCGTTTTC GCCGCGCTGC TTTTCGTCCC TAAAGACAAC GGCAGGGCAT
101 ACAGGATTAA AATTGCCAAA AACCAGGGTA TTTCGTCGGT CGGCAGGAAA
151 CTTGCCGAAG ACCGCATCGT GTTCAGCAGG CATGTTTTGA CGGCGGCGGC
201 CTACGTTTTG GGTGTGCACA ACAGGCTGCA TACGGGGACG TACAGACTGC
251 CTTCGGAAGT GTCTGCTTGG GATATCTTGC AGAAAATGCG CGGCGGCAGG
301 CCGGATTCCG TTACCGTGCA GATTATCGAA GGTTCGCGTT TTTCGCATAT
351 GAGGAAAGTC ATCGACGCAA CGCCCGACAT CGAACACGAC ACCAAAGGCT
401 GGAGCAATGA AAAACTGATG GCGGAAGTTG CCCCTGATGC CTTCAGCGGC
451 AATCCTGAAG GGCAGTTTTT CCCCGACAGC TACGAAATCG ATGCGGGCGG
501 CAGCGATTTA CGGATTTACC AAATCGCCTA CAAGGCGATG CAACGCCGAC
551 TGAATGAGGC ATGGGAAAGC AGGCAGGACG GGCTGCCTTA TAAAAACCCT
601 TATGAAATGC TGATTATGGC GAGCCTGATC GAAAAGGAAA CAGGGCATGA
651 AGCCGACCGC GACCATGTCG CTTCCGTCTT CGTCAACCGC CTGAAAATCG
701 GTATGCGCCT GCAAACCGAC CCGTCCGTGA TTTACGGCAT GGGTGCGGCA
751 TACAAGGGCA AAATCCGTAA AGCCGACCTG CGCCGCGACA CGCCGTACAA
801 CACCTACACG CGCGGCGGTC TGCCGCCAAC CCCGATCGCG CTGCCCGGCA
851 AGGCGGCACT CGATGCCGCC GCCCATCCGT CCGGTGAAAA ATACCTGTAT
901 TTCGTGTCCA AAATGGACGG TACGGGCTTG AGCCAGTTCA GCCATGATTT
951 GACCGAACAC AACGCCGCCG TTCGCAAATA TATTTTGAAA AAATAA
```

This is predicted to encode a protein having amino acid sequence <SEQ ID 34>:

```
  1 MLRKLLKWSA VFLTVSAAVF AALLFVPKDN GRAYRIKIAK NQGISSVGRK
 51 LAEDRIVFSR HVLTAAAYVL GVHNRLHTGT YRLPSEVSAW DILQKMRGGR
101 PDSVTVQIIE GSRFSHMRKV IDATPDIEHD TKGWSNEKLM AEVAPDAFSG
151 NPEGQFFPDS YEIDAGGSDL RIYQIAYKAM QRRLNEAWES RQDGLPYKNP
201 YEMLIMASLI EKETGHEADR DHVASVFVNR LKIGMRLQTD PSVIYGMGAA
251 YKGKIRKADL RRDTPYNTYT RGGLPPTPIA LPGKAALDAA AHPSGEKYLY
301 FVSKMDGTGL SQFSHDLTEH NAAVRKYILK K*
```

A leader peptide is underlined.

ORF7a and ORF7-1 show 98.8% identity in 331 aa overlap:

```
                         10        20        30        40        50        60
orf7a.pep   MLRKLLKWSAVFLTVSAAVFAALLFVPKDNGRAYRIKIAKNQGISSVGRKLAEDRIVFSR
            ||||||||||||||||||||||||||||||||||||||||||||||||||||||||||||
orf7-1      MLRKLLKWSAVFLTVSAAVFAALLFVPKDNGRAYRIKIAKNQGISSVGRKLAEDRIVFSR
                    10        20        30        40        50        60

                    70        80        90       100       110       120
```

```
orf7a.pep   HVLTAAAYVLGVHNRLHTGTYRLPSEVSAWDILQKMRGGRPDSVTVQIIEGSRFSHMRKV
            ||||||||||||||||||||||||||||||||||||||||||||||||||||||||||||
orf7-1      HVLTAAAYVLGVHNRLHTGTYRLPSEVSAWDILQKMRGGRPDSVTVQIIEGSRFSHMRKV
                    70        80        90       100       110       120

                   130       140       150       160       170       180
orf7a.pep   IDATPDIEHDTKGWSNEKLMAEVAPDAFSGNPEGQFFPDSYEIDAGGSDLRIYQIAYKAM
            ||||||| ||||||||||||||||||||||||||||||||||||||||||:||| |||||
orf7-1      IDATPDIGHDTKGWSNEKLMAEVAPDAFSGNPEGQFFPDSYEIDAGGSDLQIYQTAYKAM
                   130       140       150       160       170       180

                   190       200       210       220       230       240
orf7a.pep   QRRLNEAWESRQDGLPYKNPYEMLIMASLIEKETGHEADRDHVASVFVNRLKIGMRLQTD
            |||||||||||||||||||||||||||||:||||||||||||||||||||||||||||||
orf7-1      QRRLNEAWESRQDGLPYKNPYEMLIMASLVEKETGHEADRDHVASVFVNRLKIGMRLQTD
                   190       200       210       220       230       240

                   250       260       270       280       290       300
orf7a.pep   PSVIYGMGAAYKGKIRKADLRRDTPYNTYTRGGLPPTPIALPGKAALDAAAHPSGEKYLY
            ||||||||||||||||||||||||||||||||||||||||||||||||||||||||||||
orf7-1      PSVIYGMGAAYKGKIRKADLRRDTPYNTYTRGGLPPTPIALPGKAALDAAAHPSGEKYLY
                   250       260       270       280       290       300

                   310       320       330
orf7a.pep   FVSKMDGTGLSQFSHDLTEHNAAVRKYILKKX
            ||||||||||||||||||||||||||||||||
orf7-1      FVSKMDGTGLSQFSHDLTEHNAAVRKYILKKX
                   310       320       330
```

Homology with a predicted ORF from *N.gonorrhoeae*

ORF7 shows 94.7% identity over a 187aa overlap with a predicted ORF (ORF7.ng) from *N. gonorrhoeae:*

```
orf7       MRGGRPDSVTVQIIEGSRFSHMRKVIDATPDIGHDTKGWSNEKLMAEVAPDAFSGNPEGQ   60
           ||||||||||||||||||||||||||||||||||||||||||||||||||||||||||||
orf7ng     MRGGRPDSVTVQIIEGSRFSHMRKVIDATPDIGHDTKGWSNEKLMAEVAPDAFSGNPEGQ   60

orf7       FFPDSYEIDAGGSDLQIYQTAYKAMQRRLNEAWESRQDGLPYKNPYEMLIMAXLVEKETG  120
           ||||||||||||||||||||||||||||||||| :||||||||||||||||| |:|||||
orf7ng     FFPDSYEIDAGGSDLQIYQTAYKAMQRRLNEAWAGRQDGLPYKNPYEMLIMASLIEKETG  120

orf7       HEAXXDHVASVFVNRLKIGMRLQTXXSVIYGMGAAYKGKIRKADLRRDTPYNTYTRGGLP  180
           |||  |||||||||||||||||||  ||||||||||||||||||||||||||||| ||||
orf7ng     HEADRDHVASVFVNRLKIGMRLQTDPSVIYGMGAAYKGKIRKADLRRDTPYNTYTGGGLP  180

orf7       PTPIALP                                                  187
           || ||||
orf7ng     PTRIALPGKAAMDAAAHPSGEKYLYFVSKMDGTGLSQFSHDLTEHNAAVRKYILKK  236
```

An ORF7ng nucleotide sequence <SEQ ID 35> is predicted to encode a protein having amino acid sequence <SEQ ID 36>:

```
  1 MRGGRPDSVT VQIIEGSRFS HMRKVIDATP DIGHDTKGWS NEKLMAEVAP
 51 DAFSGNPEGQ FFPDSYEIDA GGSDLQIYQT AYKAMQRRLN EAWAGRQDGL
101 PYKNPYEMLI MASLIEKETG HEADRDHVAS VFVNRLKIGM RLQTDPSVIY
151 GMGAAYKGKI RKADLRRDTP YNTYTGGGLP PTRIALPGKA AMDAAAHPSG
201 EKYLYFVSKM DGTGLSQFSH DLTEHNAAVR KYILKK*
```

Further sequence analysis revealed a partial DNA sequence of ORF7ng <SEQ ID 37>:

```
  1 ..taccgaatca AGATTGCCAA AAATCAGGGT ATTTCGTCGG TCGGCAGGAA
 51   ACTTGCcgaA GACCGCATCG TGTTCAGCAG GCATGTTTTG ACAGCGGCGG
101   CCTACGTTTT GGGTGTGCAC AACAGGCTGC ATACGGGGAC gTACAGATTG
151   CCTTCGGAAG TGTCTGCTTG GGATATCTTG CAGAAAATGC GCGGCGGCAG
201   GCCGGATTCC GTTACCGTGC AGATTATCGA AGGTTCGCGT TTTTCGCATA
251   TGAGGAAAGT CATCGACGCA ACGCCCGACA TCGGACACGA CACCAAAGGC
301   TGGAGCAATG AAAAACTGAT GGCGGAAGTT GCGCCCGATG CCTTCAGCGG

351   CAATCCTGAA GGGCAGTTTT TTCCCGACAG CTACGAAATC GATGCGGGCG
401   GCAGCGATTT GCAGATTTAC CAAACCGCCT ACAAGGCGAT GCAACGCCGC
451   CTGAACGAGG CATGGGCAGG CAGGCAGGAC GGGCTGCCTT ATAAAAACCC
501   TTATGAAATG CTGATTATGG CGAGCCTGAT CGAAAAGGAA ACGGGGCATG
551   AGGCCGACCG CGACCATGTC GCTTCCGTCT TCGTCAACCG CCTGAAAATC
601   GGTATGCGCC TGCAAACCGA CCCGTCCGTG ATTTACGGCA TGGGTGCGGC
651   ATACAAGGGC AAAATCCGTA AAGCCGACCT GCGCCGCGAC ACGCCGTACA
701   aCAccTAtac gggcgggggc ttgccgccaa cccggattgc gctgcccggC
751   Aaggcggcaa tggatgccgc cgcccacccg tccggcgaAa aatacctgTa
801   tttcgtgtcC AAAATGGACG GCACGGGCTT GAGCCAGTTC AGCCATGATT
851   TGACCGAACA CAACGCCGCc gTcCGCAAAT ATATTTTGAA AAAATAA
```

This corresponds to the amino acid sequence <SEQ ID 38; ORF7ng-1>:

```
  1 ..YRIKIAKNQG ISSVGRKLAE DRIVFSRHVL TAAAYVLGVH NRLHTGTYRL
 51   PSEVSAWDIL QKMRGGRPDS VTVQIIEGSR FSHMRKVIDA TPDIGHDTKG
101   WSNEKLMAEV APDAFSGNPE GQFFPDSYEI DAGGSDLQIY QTAYKAMQRR
151   LNEAWAGRQD GLPYKNPYEM LIMASLIEKE TGHEADRDHV ASVFVNRLKI
201   GMRLQTDPSV IYGMGAAYKG KIRKADLRRD TPYNTYTGGG LPPTRIALPG
251   KAAMDAAAHP SGEKYLYFVS KMDGTGLSQF SHDLTEHNAA VRKYILKK*
```

ORF7ng-1 and ORF7-1 show 98.0% identity in 298 aa overlap:

```
                     10        20        30        40        50        60
orf7-1.pep   KLLKWSAVFLTVSAAVFAALLFVPKDNGRAYRIKIAKNQGISSVGRKLAEDRIVFSRHVL
                                           ||||||||||||||||||||||||||||||
orf7ng-1                                   YRIKIAKNQGISSVGRKLAEDRIVFSRHVL
                                                   10        20        30

                     70        80        90       100       110       120
orf7-1.pep   TAAAYVLGVHNRLHTGTYRLPSEVSAWDILQKMRGGRPDSVTVQIIEGSRFSHMRKVIDA
             ||||||||||||||||||||||||||||||||||||||||||||||||||||||||||||
orf7ng-1     TAAAYVLGVHNRLHTGTYRLPSEVSAWDILQKMRGGRPDSVTVQIIEGSRFSHMRKVIDA
                     40        50        60        70        80        90

                    130       140       150       160       170       180
orf7-1.pep   TPDIGHDTKGWSNEKLMAEVAPDAFSGNPEGQFFPDSYEIDAGGSDLQIYQTAYKAMQRR
             ||||||||||||||||||||||||||||||||||||||||||||||||||||||||||||
orf7ng-1     TPDIGHDTKGWSNEKLMAEVAPDAFSGNPEGQFFPDSYEIDAGGSDLQIYQTAYKAMQRR
                    100       110       120       130       140       150

                    190       200       210       220       230       240
orf7-1.pep   LNEAWESRQDGLPYKNPYEMLIMASLVEKETGHEADRDHVASVFVNRLKIGMRLQTDPSV
             |||||  :||||||||||||||||||:|||||||||||||||||||||||||||||||||
orf7ng-1     LNEAWAGRQDGLPYKNPYEMLIMASLIEKETGHEADRDHVASVFVNRLKIGMRLQTDPSV
                    160       170       180       190       200       210

                    250       260       270       280       290       300
orf7-1.pep   IYGMGAAYKGKIRKADLRRDTPYNTYTRGGLPPTPIALPGKAALDAAAHPSGEKYLYFVS
             ||||||||||||||||||||||||||| |||||| |||||||:|||||||||||||||||
orf7ng-1     IYGMGAAYKGKIRKADLRRDTPYNTYTGGGLPPTRIALPGKAAMDAAAHPSGEKYLYFVS
                    220       230       240       250       260       270

                    310       320       330
orf7-1.pep   KMDGTGLSQFSHDLTEHNAAVRKYILKKX
             |||||||||||||||||||||||||||||
orf7ng-1     KMDGTGLSQFSHDLTEHNAAVRKYILKKX
                    280       290
```

In addition, ORF7ng-1 shows significant homology with a hypothetical *E.coli* protein:

```
sp|P28306|YCEG_ECOLI HYPOTHETICAL 38.2 KD PROTEIN IN PABC-HOLB INTERGENIC REGION
gi|1787339 (AE000210) o340; 100% identical to fragment YCEG_ECOLI SW: P28306 but
has 97 additional C-terminal residues [Escherichia coli] Length = 340
 Score = 79 (36.2 bits), Expect = 5.0e-57, Sum P(2) = 5.0e-57
 Identities = 20/87 (22%), Positives = 40/87 (45%)

Query:    10 GISSVGRKLAEDRIVFSRHVLTAAAYVLGVHNRLHTGTYRLPSEVSAWDILQKMRGGRPD 69
             G  ++G +L  D+I++    V      +     +     GTYR    +++  ++L+ +  G+
Sbjct:    49 GRLALGEQLYADKIINRPRVFQWLLRIEPDLSHFKAGTYRFTPQMTVREMLKLLESGKEA 108
```

```
Query:    70 SVTVQIIEGSRFSHMRKVIDATPDIGH 96
                ++++EG R S   K +    P I H
Sbjct:   109 QFPLRLVEGMRLSDYLKQLREAPYIKH 135

 Score = 438 (200.7 bits), Expect = 5.0e-57, Sum P(2) = 5.0e-57
 Identities = 84/155 (54%), Positives = 111/155 (71%)

Query:   120 EGQFFPDSYEIDAGGSDLQIYQTAYKAMQRRLNEAWAGRQDGLPYKNPYEMLIMASLIEK 179
             EG F+PD++   A  +D+ + + A+K M + ++ AW GR DGLPYK+  +++ MAS+IEK
Sbjct:   158 EGWFWPDTWMYTANTTDVALLKRAHKKMVKAVDSAWEGRADGLPYKDKNQLVTMASIIEK 217

Query:   180 ETGHEADRDHVASVFVNRLKIGMRLQTDPSVIYGMGAAYKGKIRKADLRRDTPYNTYTGG 239
             ET   ++RD VASVF+NRL+IGMRLQTDP+VIYGMG  Y GK+ +ADL   T YNTYT
Sbjct:   218 ETAVASERDKVASVFINRLRIGMRLQTDPTVIYGMGERYNGKLSRADLETPTAYNTYTIT 277

Query:   240 GLPPTRIALPGKAAMDAAAHPSGEKYLYFVSKMDG 274
             GLPP  IA PG  ++ AAAHP+    YLYFV+   G
Sbjct:   278 GLPPGAIATPGADSLKAAAHPAKTPYLYFVADGKG 312
```

Based on this analysis, including the fact that the *H.influenzae* YCEG protein possesses a possible leader sequence, it is predicted that the proteins from *N meningitidis* and *N.gonorrhoeae,* and their epitopes, could be useful antigens for vaccines or diagnostics, or for raising antibodies.

**Example 6**

The following partial DNA sequence was identified in *N.meningitidis* <SEQ ID 39>:

```
  1  CGTTTCAAAA TGTTAACTGT GTTGACGGCA ACCTTGATTG CCGGACAGGT
 51  ATCTGCCGCC GGAGGCGGTG CGGGGGATAT GAAACAGCCG AAGGAAGTCG
101  GAAAGGTTTT CAGAAAGCAG CAGCGTTACA GCGAGGAAGA AATCAAAAAC
151  GAACGCGCAC GGCTTGCGGC AGTGGGCGAG CGGGTTAATC AGATATTTAC
201  GTTGCTGGGA GGGGAAACCG CCTTGCAAAA GGGGCAGGCG GGAACGGCTC
251  TGGCAACCTA TATGCTGATG TTGGAACGCA CAAAATCCCC CGAAGTCGCC
301  GAACGCGCCT TGGAAATGGC CGTGTCGCTG AACGCGTTTG AACAGGCGGA
351  AATGATTTAT CAGAAATGGC GGCAGATTGA GCCTATACCG GGTAAGGCGC
401  AAAAACGGGC GGGGTGGCTG CGGAACGTGC TGAGGGAAAG AGGAAATCAG
451  CATCTGGACG GACGGGAAGA AGTGCTGGCT CAGGCGGACG AAGGACAG
```

This corresponds to the amino acid sequence <SEQ ID 40; ORF9>:

```
  1  ..RFKMLTVLTA TLIAGQVSAA GGGAGDMKQP KEVGKVFRKQ QRYSEEEIKN
 51    ERARLAAVGE RVNQIFTLLG GETALQKGQA GTALATYMLM LERTKSPEVA
101    ERALEMAVSL NAFEQAEMIY QKWRQIEPIP GKAQKRAGWL RNVLRERGNQ
151    HLDGREEVLA QADEGQ
```

Further sequence analysis revealed the complete DNA sequence <SEQ ID 41>:

```
   1  ATGTTACCTA ACCGTTTCAA AATGTTAACT GTGTTGACGG CAACCTTGAT
  51  TGCCGGACAG GTATCTGCCG CCGGAGGCGG TGCGGGGGAT ATGAAACAGC
 101  CGAAGGAAGT CGGAAAGGTT TTCAGAAAGC AGCAGCGTTA CAGCGAGGAA
 151  GAAATCAAAA ACGAACGCGC ACGGCTTGCG GCAGTGGGCG AGCGGGTTAA
 201  TCAGATATTT ACGTTGCTGG GAGGGGAAAC CGCCTTGCAA AAGGGGCAGG
 251  CGGGAACGGC TCTGGCAACC TATATGCTGA TGTTGGAACG CACAAAATCC
 301  CCCGAAGTCG CCGAACGCGC CTTGGAAATG GCCGTGTCGC TGAACGCGTT
 351  TGAACAGGCG GAAATGATTT ATCAGAAATG GCGGCAGATT GAGCCTATAC
 401  CGGGTAAGGC GCAAAAACGG GCGGGGTGGC TGCGGAACGT GCTGAGGGAA
 451  AGAGGAAATC AGCATCTGGA CGGACTGGAA GAAGTGCTGG CTCAGGCGGA
 501  CGAAGGACAG AACCGCAGGG TGTTTTTATT GTTGGCACAA GCCGCCGTGC
 551  AACAGGACGG GTTGGCGCAA AAAGCATCGA AAGCGGTTCG CCGCGCGGCG
 601  TTGAAATATG AACATCTGCC CGAAGCGGCG GTTGCCGATG TGGTGTTCAG
 651  CGTACAGGGA CGCGAAAAGG AAAAGGCAAT CGGAGCTTTG CAGCGTTTGG
 701  CGAAGCTCGA TACGGAAATA TTGCCCCCCA CTTTAATGAC GTTGCGTCTG
 751  ACTGCACGCA AATATCCCGA AATACTCGAC GGCTTTTTCG AGCAGACAGA
 801  CACCCAAAAC CTTTCGGCCG TCTGGCAGGA AATGGAAATT ATGAATCTGG
 851  TTTCCCTGCA CAGGCTGGAT GATGCCTATG CGCGTTTGAA CGTGCTGTTG
```

```
 901  GAACGCAATC CGAATGCAGA CCTGTATATT CAGGCAGCGA TATTGGCGGC
 951  AAACCGAAAA GAAGGTGCTT CCGTTATCGA CGGCTACGCC GAAAAGGCAT
1001  ACGGCAGGGG GACGGAGGAA CAGCGGAGCA GGGCGGCGCT AACGGCGGCG
1051  ATGATGTATG CCGACCGCAG GGATTACGCC AAAGTCAGGC AGTGGCTGAA
1101  AAAAGTATCC GCGCCGGAAT ACCTGTTCGA CAAAGGTGTG CTGGCGGCTG
1151  CGGCGGCTGT CGAGTTGGAC GGCGGCAGGG CGGCTTTGCG GCAGATCGGC
1201  AGGGTGCGGA AACTTCCCGA ACAGCAGGGG CGGTATTTTA CGGCAGACAA
1251  TTTGTCCAAA ATACAGATGC TCGCCCTGTC GAAGCTGCCC GATAAACGGG
1301  AGGCTTTGAG GGGGTTGGAC AAGATTATCG AAAAACCGCC TGCCGGCAGT
1351  AATACAGAGT TACAGGCAGA GGCATTGGTA CAGCGGTCAG TTGTTTACGA
1401  TCGGCTTGGC AAGCGGAAAA AAATGATTTC AGATCTTGAA AGGGCGTTCA
1451  GGCTTGCACC CGATAACGCT CAGATTATGA ATAATCTGGG CTACAGCCTG
1501  CTGACCGATT CCAAACGTTT GGACGAAGGT TTCGCCCTGC TTCAGACGGC
1551  ATACCAAATC AACCCGGACG ATACCGCTGT CAACGACAGC ATAGGCTGGG
1601  CGTATTACCT GAAAGGCGAC GCGGAAAGCG CGCTGCCGTA TCTGCGGTAT
1651  TCGTTTGAAA ACGACCCCGA GCCCGAAGTT GCCGCCCATT TGGGCGAAGT
1701  GTTGTGGGCA TTGGGCGAAC GCGATCAGGC GGTTGACGTA TGGACGCAGG
1751  CGGCACACCT TACGGGAGAC AAGAAAATAT GGCGGGAAAC GCTCAAACGT
1801  CACGGCATCG CATTGCCCCA ACCTTCCCGA AAACCTCGGA AATAA
```

This corresponds to the amino acid sequence <SEQ ID 42; ORF9-1>:

```
   1  MLPNRFKMLT VLTATLIAGQ VSAAGGGAGD MKQPKEVGKV FRKQQRYSEE
  51  EIKNERARLA AVGERVNQIF TLLGGETALQ KGQAGTALAT YMLMLERTKS
 101  PEVAERALEM AVSLNAFEQA EMIYQKWRQI EPIPGKAQKR AGWLRNVLRE
 151  RGNQHLDGLE EVLAQADEGQ NRRVFLLLAQ AAVQQDGLAQ KASKAVRRAA
 201  LKYEHLPEAA VADVVFSVQG REKEKAIGAL QRLAKLDTEI LPPTLMTLRL
 251  TARKYPEILD GFFEQTDTQN LSAVWQEMEI MNLVSLHRLD DAYARLNVLL
 301  ERNPNADLYI QAAILAANRK EGASVIDGYA EKAYGRGTEE QRSRAALTAA
 351  MMYADRRDYA KVRQWLKKVS APEYLFDKGV LAAAAAVELD GGRAALRQIG
 401  RVRKLPEQQG RYFTADNLSK IQMLALSKLP DKREALRGLD KIIEKPPAGS
 451  NTELQAEALV QRSVVYDRLG KRKKMISDLE RAFRLAPDNA QIMNNLGYSL
 501  LTDSKRLDEG FALLQTAYQI NPDDTAVNDS IGWAYYLKGD AESALPYLRY
 551  SFENDPEPEV AAHLGEVLWA LGERDQAVDV WTQAAHLTGD KKIWRETLKR
 601  HGIALPQPSR KPRK*
```

Computer analysis of this amino acid sequence gave the following results:

Homology with a predicted ORF from *N. meningitidis* (strain A)

ORF9 shows 89.8% identity over a 166aa overlap with an ORF (ORF9a) from strain A of *N. meningitidis:*

```
                      10        20        30        40        50
orf9.pep          RFKMLTVLTATLIAGQVSAAGGGAGDMKQPKEVGKVFRKQQRYSEEEIKNERARLA
                  || :|:||:|:|:|||: ||  ||:| | |||||||||||||||||||||||||||
orf9a         MLPARFTILSVLAAALLAGQAYAA--GAADAKPPKEVGKVFRKQQRYSEEEIKNERARLA
                      10        20          30        40        50

                60        70        80        90       100       110
orf9.pep      AVGERVNQIFTLLGGETALQKGQAGTALATYMLMLERTKSPEVAERALEMAVSLNAFEQA
              |||||||||||||| |||||||||||||||||||||||||||||||||||||||||||||
orf9a         AVGERVNQIFTLLGXETALQKGQAGTALATYMLMLERTKSPEVAERALEMAVSLNAFEQA
              60        70        80        90       100       110

               120       130       140       150       160
orf9.pep      EMIYQKWRQIEPIPGKAQKRAGWLRNVLRERGNQHLDGREEVLAQADEGQ
              |||||||||||||||||||||||||||||||||||||| || |||||| |
orf9a         EMIYQKWRQIEPIPGKAQKRAGWLRNVLRERGNQHLDGLEEXLAQADEXQNRRVFLLLAQ
            120       130       140       150       160       170

orf9a         AAVQQDGLAQKASKAVRRAALRYEHLPEAAVADVVFSVQXREKEKAIGALQRLAKLDTEI
            180       190       200       210       220       230
```

The complete length ORF9a nucleotide sequence <SEQ ID 43> is:

```
  1 ATGTTACCCG CCCGTTTCAC CATTTTATCT GTGCTCGCGG CAGCCCTGCT
 51 TGCCGGGCAG GCGTATGCCG CCGGCGCGGC GGATGCGAAG CCGCCGAAGG
101 AAGTCGGAAA GGTTTTCAGA AAGCAGCAGC GTTACAGCGA GGAAGAAATC
151 AAAAACGAAC GCGCACGGCT TGCGGCAGTG GGCGAGCGGG TTAATCAGAT
```

```
 201 ATTTACGTTG CTGGGANGGG AAACCGCCTT GCAAAAGGGG CAGGCGGGAA
 251 CGGCTCTGGC AACCTATATG CTGATGTTGG AACGCACAAA ATCCCCCGAA
 301 GTCGCCGAAC GCGCCTTGGA AATGGCCGTG TCNCTGAACG CGTTTGAACA
 351 GGCGGAAATG ATTTATCAGA AATGGCGGCA GATTGAGCCT ATACCGGGTA
 401 AGGCGCAAAA ACGGGCGGGG TGGCTGCGGA ACGTGCTGAG GGAAAGAGGA
 451 AATCAGCATC TAGACGGACT GGAAGAANTG CTGGCTCAGG CGGACGAANG
 501 ACAGAACCGC AGGGTGTTTT TATTGTTGGC ACAAGCCGCC GTGCAACAGG
 551 ACGGGTTGGC GCAAAAAGCA TCGAAAGCGG TTCGCCGCGC GGCGTTGAGA
 601 TATGAACATC TGCCCGAAGC GGCGGTTGCC GATGTGGTGT TCAGCGTACA
 651 GGNACGCGAA AAGGAAAAGG CAATCGGAGC TTTGCAGCGT TTGGCGAAGC
 701 TCGATACGGA AATATTGCCC CCCACTTTAA TGACGTTGCG TCTGACTGCA
 751 CGCAAATATC CCGAAATACT CGACGGCTTT TTCGAGCAGA CAGACACCCA
 801 AAACCTTTCG GCCGTCTGGC AGGAAATGGA AATTATGAAT CTGGTTTCCC
 851 TGCACAGGCT GGATGATGCC TATGCGCGTT TGAACGTGCT GTTGGAACGC
 901 AATCCGAATG CAGACCTGTA TATTCAGGCA GCGATATTGG CGGCAAACCG
 951 AAAAGAANGT GCTTCCGTTA TCGACGGCTA CGCCGAAAAG GCATACGGCA
1001 GGGGGACGGG GGAACAGCGG GGCAGGGCGG CAATGACGGC GGCGATGATA
1051 TATGCCGACC GAAGGGATTA CACCAAAGTC AGGCAGTGGT TGAAAAAAGT
1101 GTCCGCGCCG GAATACCTGT TCGACAAAGG TGTGCTGGCG GCTGCGGCGG
1151 CTGTCGAGTT GGACNGCGGC AGGGCGGCTT TGCGGCAGAT CGGCAGGGTG
1201 CGGAAACTTC CCGAACAGCA GGGGCGGTAT TTTACGGCAG ACAATTTGTC
1251 CAAAATACAG ATGTTCGCCC TGTCGAAGCT GCCCGACAAA CGGGAGGCTT
1301 TGAGGGGGTT GGACAAGATT ATCGAAAAAC CGCCTGCCGG CAGTAATACA
1351 GAGTTACAGG CAGAGGCATT GGTACAGCGG TCAGTTGTTT ACGATCGGCT
1401 TGGCAAGCGG AAAAAAATGA TTTCAGATCT TGAAAGGGCG TTCAGGCTTG
1451 CACCCGATAA CGCTCAGATT ATGAATAATC TGGGCTACAG CCTGCTTTCC
1501 GATTCCAAAC GTTTGGACGA AGGCTTCGCC CTGCTTCAGA CGGCATACCA
1551 AATCAACCCG GACGATACCG CTGTCAACGA CAGCATAGGC TGGGCGTATT
1601 ACCTGAAANG CGACGCGGAA AGCGCGCTGC CGTATCTGCG GTATTCGTTT
1651 GAAAACGACC CCGAGCCCGA AGTTGCCGCC CATTTGGGCG AAGTGTTGTG
1701 GGCATTGGGC GAACGCGATC AGGCGGTTGA CGTATGGACG CAGGCGGCAC
1751 ACCTTACGGG AGACAAGAAA ATATGGCGGG AAACGCTCAA ACGTCACGGC
1801 ATCGCATTGC CCCAACCTTC CCGAAAACCT CGGAAATAA
```

This encodes a protein having amino acid sequence <SEQ ID 44>:

```
  1 MLPARFTILS VLAAALLAGQ AYAAGAADAK PPKEVGKVFR KQQRYSEEEI
 51 KNERARLAAV GERVNQIFTL LGXETALQKG QAGTALATYM LMLERTKSPE
101 VAERALEMAV SLNAFEQAEM IYQKWRQIEP IPGKAQKRAG WLRNVLRERG
151 NQHLDGLEEX LAQADEXQNR RVFLLLAQAA VQQDGLAQKA SKAVRRAALR
201 YEHLPEAAVA DVVFSVQXRE KEKAIGALQR LAKLDTEILP PTLMTLRLTA
251 RKYPEILDGF FEQTDTQNLS AVWQEMEIMN LVSLHRLDDA YARLNVLLER
301 NPNADLYIQA AILAANRKEX ASVIDGYAEK AYGRGTGEQR GRAAMTAAMI
351 YADRRDYTKV RQWLKKVSAP EYLFDKGVLA AAAAVELDXG RAALRQIGRV
401 RKLPEQQGRY FTADNLSKIQ MFALSKLPDK REALRGLDKI IEKPPAGSNT
451 ELQAEALVQR SVVYDRLGKR KKMISDLERA FRLAPDNAQI MNNLGYSLLS
501 DSKRLDEGFA LLQTAYQINP DDTAVNDSIG WAYYLKXDAE SALPYLRYSF
551 ENDPEPEVAA HLGEVLWALG ERDQAVDVWT QAAHLTGDKK IWRETLKRHG
601 IALPQPSRKP RK*
```

ORF9a and ORF9-1 show 95.3% identity in 614 aa overlap:

```
                  10        20        30        40        50
orf9a.pep  MLPARFTILSVLAAALLAGQAYAAG--AADAKPPKEVGKVFRKQQRYSEEEIKNERARLA
           ||| || :|:||:|:|:|||: |||  |:| | ||||||||||||||||||||||||||
orf9-1     MLPNRFKMLTVLTATLIAGQVSAAGGGAGDMKQPKEVGKVFRKQQRYSEEEIKNERARLA
                  10        20        30        40        50        60

           60        70        80        90       100       110
orf9a.pep  AVGERVNQIFTLLGXETALQKGQAGTALATYMLMLERTKSPEVAERALEMAVSLNAFEQA
           ||||||||||||| ||||||||||||||||||||||||||||||||||||||||||||||
orf9-1     AVGERVNQIFTLLGGETALQKGQAGTALATYMLMLERTKSPEVAERALEMAVSLNAFEQA
                  70        80        90       100       110       120

          120       130       140       150       160       170
orf9a.pep  EMIYQKWRQIEPIPGKAQKRAGWLRNVLRERGNQHLDGLEEXLAQADEXQNRRVFLLLAQ
           |||||||||||||||||||||||||||||||||||||||||| |||||| ||||||||||
orf9-1     EMIYQKWRQIEPIPGKAQKRAGWLRNVLRERGNQHLDGLEEVLAQADEGQNRRVFLLLAQ
                 130       140       150       160       170       180

          180       190       200       210       220       230
```

```
orf9a.pep    AAVQQDGLAQKASKAVRRAALRYEHLPEAAVADVVFSVQXREKEKAIGALQRLAKLDTEI
             |||||||||||||||||||||:||||||||||||||||| ||||||||||||||||||||
orf9-1       AAVQQDGLAQKASKAVRRAALKYEHLPEAAVADVVFSVQGREKEKAIGALQRLAKLDTEI
                    190       200       210       220       230       240

            240       250       260       270       280       290
orf9a.pep    LPPTLMTLRLTARKYPEILDGFFEQTDTQNLSAVWQEMEIMNLVSLHRLDDAYARLNVLL
             ||||||||||||||||||||||||||||||||||||||||||||||||||||||||||||
orf9-1       LPPTLMTLRLTARKYPEILDGFFEQTDTQNLSAVWQEMEIMNLVSLHRLDDAYARLNVLL
                    250       260       270       280       290       300

            300       310       320       330       340       350
orf9a.pep    ERNPNADLYIQAAILAANRKEXASVIDGYAEKAYGRGTGEQRGRAAMTAAMIYADRRDYT
             ||||||||||||||||||||| |||||||||||||||| |||:|||:||||:|||||||:
orf9-1       ERNPNADLYIQAAILAANRKEGASVIDGYAEKAYGRGTEEQRSRAALTAAMMYADRRDYA
                    310       320       330       340       350       360

            360       370       380       390       400       410
orf9a.pep    KVRQWLKKVSAPEYLFDKGVLAAAAAVELDXGRAALRQIGRVRKLPEQQGRYFTADNLSK
             |||||||||||||||||||||||||||||| |||||||||||||||||||||||||||||
orf9-1       KVRQWLKKVSAPEYLFDKGVLAAAAAVELDGGRAALRQIGRVRKLPEQQGRYFTADNLSK
                    370       380       390       400       410       420

            420       430       440       450       460       470
orf9a.pep    IQMFALSKLPDKREALRGLDKIIEKPPAGSNTELQAEALVQRSVVYDRLGKRKKMISDLE
             |||:||||||||||||||||||||||||||||||||||||||||||||||||||||||||
orf9-1       IQMLALSKLPDKREALRGLDKIIEKPPAGSNTELQAEALVQRSVVYDRLGKRKKMISDLE
                    430       440       450       460       470       480

            480       490       500       510       520       530
orf9a.pep    RAFRLAPDNAQIMNNLGYSLLSDSKRLDEGFALLQTAYQINPDDTAVNDSIGWAYYLKXD
             |||||||||||||||||||||:|||||||||||||||||||||||||||||||||||| |
orf9-1       RAFRLAPDNAQIMNNLGYSLLTDSKRLDEGFALLQTAYQINPDDTAVNDSIGWAYYLKGD
                    490       500       510       520       530       540

            540       550       560       570       580       590
orf9a.pep    AESALPYLRYSFENDPEPEVAAHLGEVLWALGERDQAVDVWTQAAHLTGDKKIWRETLKR
             ||||||||||||||||||||||||||||||||||||||||||||||||||||||||||||
orf9-1       AESALPYLRYSFENDPEPEVAAHLGEVLWALGERDQAVDVWTQAAHLTGDKKIWRETLKR
                    550       560       570       580       590       600

            600       610
orf9a.pep    HGIALPQPSRKPRKX
             ||||||||||||||
orf9-1       HGIALPQPSRKPRKX
                    610
```

Homology with a predicted ORF from *N.gonorrhoeae*

ORF9 shows 82.8% identity over a 163aa overlap with a predicted ORF (ORF9.ng) from *N. gonorrhoeae:*

```
Orf9               RFKMLTVLTATLIAGQVSAAGGGAGDMKQPKEVGKVFRKQQRYSEEEIKNERAR    54
                   || :|:||:|:|:|||: ||  ||:|:: |||||||:||::|||||||||||||
orf9ng       MIMLPARFTILSVLAAALLAGQAYAA--GAADVELPKEVGKVLRKHRRYSEEEIKNERAR    58

orf9         LAAVGERVNQIFTLLGGETALQKGQAGTALATYMLMLERTKSPEVAERALEMAVSLNAFE   114
             |||||||||::|||||||||||||||||||||||||||||||||||||||||||||||||
orf9ng       LAAVGERVNRVFTLLGGETALQKGQAGTALATYMLMLERTKSPEVAERALEMAVSLNAFE   118

orf9         QAEMIYQKWRQIEPIPGKAQKRAGWLRNVLRERGNQHLDGREEVLAQADEGQ           166
             |||||||||||||||||:||| ||||||||:| || |||  ||| ||:|
orf9ng       QAEMIYQKWRQIEPIPGEAQKPAGWLRNVLKEGGNPHLDRLEEVPAQSDYVHQPMIFLLL   178
```

The ORF9ng nucleotide sequence <SEQ ID 45> was predicted to encode a protein having including acid sequence <SEQ ID 46>:

```
  1 MIMLPARFTI LSVLAAALLA GQAYAAGAAD VELPKEVGKV LRKHRRYSEE
 51 EIKNERARLA AVGERVNRVF TLLGGETALQ KGQAGTALAT YMLMLERTKS
```

```
101 PEVAERALEM AVSLNAFEQA EMIYQKWRQI EPIPGEAQKP AGWLRNVLKE
151 GGNPHLDRLE EVPAQSDYVH QPMIFLLLVQ AAVQHGGVAQ KPSKAVRPAA
201 YNYEVLPETA GADAVFCVQG PQYEKAIQSF PPCGRNPQTE NIAPPFNELF
251 RPTARPISPK LLQRFFRTEP NLAKPFRPPG PEMETYQTGF PRPLTRNNPT
```

Amino acids 1-28 are a putative leader sequence, and 173-189 are predicted to be a transmembrane domain.

Further sequence analysis revealed the complete length ORF9ng DNA sequence <SEQ ID 47>:

```
   1 ATGTTACCCG CCCGTTTCAC TATTTTATCT GTCCTCGCAG CAGCCCTGCT
  51 TGCCGGACAG GCGTATGCTG CCGGCGCGGC GGATGTGGAG CTGCCGAAGG
 101 AAGTCGGAAA GGTTTTAAGG AAACATCGGC GTTACAGCGA GGAAGAAATC
 151 AAAAACGAAC GCGCACGGCT TGCGGCAGTG GGCGAACGGG TCAACAGGGT
 201 GTTTACGCTG TTGGGCGGTG AAACGGCTTT GCAGAAAGGG CAGGCGGGAA
 251 CGGCTCTGGC AACCTATATG CTGATGTTGG AACGCACAAA ATCCCCCGAA
 301 GTCGCCGAAC GCGCCTTGGA AATGGCCGTG TCGCTGAACG CGTTTGAACA
 351 GGCGGAAATG ATTTATCAGA AATGGcggca gatcgagcct ataCcgggtg
 401 aggcgcaaaa accgGcgggG tggctgcgga acgtattgaa ggaagggGGa
 451 aaTCAGCATC TGGAcgggtt gaaagaggTG CtggcgcaAT cggacgatGT
 501 GCAAAAAcgc aggaTATTTT TGCTGCTGGT GCAAGCCGCC GTGCagcagg
 551 gTGGGGTGGC TCAAAAAGCA TCGAAAGCGG TTCGCcgtgc GGcgttgaAG
 601 TATGAACATC TGCCcgaagc ggcggTTGCC GATGcggTGT TCGGCGTACA
 651 GGGACGCGAA AAGGAAAagg caaTCGAAGC TTTGCAGCGT TTGGCGAAGC
 701 TCGATACGGA AATATTGCCC CCCACTTTAA TGACGTTGCG TCTGACTGCA
 751 CGCAAATATC CCGAAATACT CGACGGCTTT TTCGAGCAGA CAGACACCCA
 801 AAACCTTTCG GCCGTCTGGC AGGAAATGGA AATTATGAAT CTGGTTTCCC
 851 TGCGTAAGCC GGATGATGCC TATGCGCGTT TGAACGTGCT GTTGGAACAC
 901 AACCCGAATG CAAACCTGTA TATTCAGGCG GCGATATTGG CGGCAAACCG
 951 AAAAGAAGGT GCGTCCGTTA TCGACGGCTA CGCCGAAAAG GCATACGGCA
1001 GGGGGACGGG GGAACAGCGG GGCagggcgg cAATgacggc GGCGATGATA
1051 TATGCCGACC GCAGGGATTA CGCCAAAGTC AGGCAGTGGT TGAAAAAAGT
1101 GTCCGCGCCG GAATACCTGT TCGACAAAGG CGTGCTGGCG GCTGCGGCGG
1151 CTGCCGAATT GGACGGAGGC CGGGCGGCTT TGCGGCAGAT CGGCAGGGTG
1201 CGGAAACTTC CCGAACAGCA GGGGCGGTAT TTTACGGCAG ACAATTTGTC
1251 CAAAATACAG ATGCTCGCCC TGTCGAAGCT GCCCGACAAA CGGGAAGCCC
1301 TGATCGGGCT GAACAACATC ATCGCCAAAC TTTCGGCGGC GGGAAGCACG
1351 GAACCTTTGG CGGAAGCATT GGCACAGCGT TCCATTATTT ACGaacAGTT
1401 cggCAAACGG GGAAAAATGA TTGCCGACCT tgaAACcgcg CTCAAACTTA
1451 CGCCCGATAA TGCACAAATT ATGAATAATC TGGGCTACAG CCTGCTTTCC
1501 GATTCCAAAC GTTTGGACGA GGGTTTCGCC CTGCTTCAGA CGGCATACCA
1551 AATCAACCCG GACGATACCG CCGTTAACGA CAGCATAGGC TGGGCGTATT
1601 ACCTGAAAGG CGACgcggaA AGCGCGCTGC CGTATCTGcg gtattcgttt
1651 gAAAACGACC CCGAGCCCGA AGTTGCCGCC CATTTGGGCG AAGTGTTGTG
1701 GGCATTGGGC GAACGCGATC AGGCGGTTGA CGTATGGACG CAGGCGGCAC
1751 ACCTTAGGGG AGACAAGAAA ATATGGCGGG AGACGCTCAA ACGCTACGGA
1801 ATCGCCTTGC CCGAGCCTTC CCGAAAACCC CGGAAATAA
```

This encodes a protein having amino acid sequence <SEQ ID 48>:

```
  1  MLPARFTILS VLAAALLAGQ AYAAGAADVE LPKEVGKVLR KHRRYSEEEI
 51  KNERARLAAV GERVNRVFTL LGGETALQKG QAGTALATYM LMLERTKSPE
101  VAERALEMAV SLNAFEQAEM IYQKWRQIEP IPGEAQKPAG WLRNVLKEGG
151  NQHLDGLKEV LAQSDDVQKR RIFLLLVQAA VQQGGVAQKA SKAVRRAALK
201  YEHLPEAAVA DAVFGVQGRE KEKAIEALQR LAKLDTEILP PTLMTLRLTA
251  RKYPEILDGF FEQTDTQNLS AVWQEMEIMN LVSLRKPDDA YARLNVLLEH
301  NPNANLYIQA AILAANRKEG ASVIDGYAEK AYGRGTGEQR GRAAMTAAMI
351  YADRRDYAKV RQWLKKVSAP EYLFDKGVLA AAAAAELDGG RAALRQIGRV
401  RKLPEQQGRY FTADNLSKIQ MLALSKLPDK REALIGLNNI IAKLSAAGST
451  EPLAEALAQR SIIYEQFGKR GKMIADLETA LKLTPDNAQI MNNLGYSLLS
501  DSKRLDEGFA LLQTAYQINP DDTAVNDSIG WAYYLKGDAE SALPYLRYSF
551  ENDPEPEVAA HLGEVLWALG ERDQAVDVWT QAAHLRGDKK IWRETLKRYG
601  IALPEPSRKP RK*
```

ORF9ng and ORF9-1 show 88.1 % identity in 614 aa overlap:

```
                          10        20        30        40        50        60
orf9-1.pep     MLPNRFKMLTVLTATLIAGQVSAAGGGAGDMKQPKEVGKVFRKQQRYSEEEIKNERARLA
               ||| || :|:||:|:|:|||: |||   |:|:: |||||||:||::||||||||||||||
```

```
orf9ng-1          MLPARFTILSVLAAALLAGQAYAAG--AADVELPKEVGKVLRKHRRYSEEEIKNERARLA
                          10        20          30        40        50

                          70        80        90       100       110       120
orf9-1.pep        AVGERVNQIFTLLGGETALQKGQAGTALATYMLMLERTKSPEVAERALEMAVSLNAFEQA
                  |||||||::|||||||||||||||||||||||||||||||||||||||||||||||||||
orf9ng-1          AVGERVNRVFTLLGGETALQKGQAGTALATYMLMLERTKSPEVAERALEMAVSLNAFEQA
                  60        70        80        90       100       110

                         130       140       150       160       170       180
orf9-1.pep        EMIYQKWRQIEPIPGKAQKRAGWLRNVLRERGNQHLDGLEEVLAQADEGQNRRVFLLLAQ
                  |||||||||||||||:||| ||||||||:| ||||||||:|||||:|: |:||:||||:|
orf9ng-1          EMIYQKWRQIEPIPGEAQKPAGWLRNVLKEGGNQHLDGLKEVLAQSDDVQKRRIFLLLVQ
                 120       130       140       150       160       170

                         190       200       210       220       230       240
orf9-1.pep        AAVQQDGLAQKASKAVRRAALKYEHLPEAAVADVVFSVQGREKEKAIGALQRLAKLDTEI
                  ||||| |:|||||||||||||||||||||||:||:|||||||||| ||||||||||||
orf9ng-1          AAVQQGGVAQKASKAVRRAALKYEHLPEAAVADAVFGVQGREKEKAIEALQRLAKLDTEI
                 180       190       200       210       220       230

                         250       260       270       280       290       300
orf9-1.pep        LPPTLMTLRLTARKYPEILDGFFEQTDTQNLSAVWQEMEIMNLVSLHRLDDAYARLNVLL
                  ||||||||||||||||||||||||||||||||||||||||||||||:: |||||||||||
orf9ng-1          LPPTLMTLRLTARKYPEILDGFFEQTDTQNLSAVWQEMEIMNLVSLRKPDDAYARLNVLL
                 240       250       260       270       280       290

                         310       320       330       340       350       360
orf9-1.pep        ERNPNADLYIQAAILAANRKEGASVIDGYAEKAYGRGTEEQRSRAALTAAMMYADRRDYA
                  |:||||:|||||||||||||||||||||||||||||||| ||:|||:||||:||||||||
orf9ng-1          EHNPNANLYIQAAILAANRKEGASVIDGYAEKAYGRGTGEQRGRAAMTAAMIYADRRDYA
                 300       310       320       330       340       350

                         370       380       390       400       410       420
orf9-1.pep        KVRQWLKKVSAPEYLFDKGVLAAAAAVELDGGRAALRQIGRVRKLPEQQGRYFTADNLSK
                  ||||||||||||||||||||||||||:|||||||||||||||||||||||||||||||||
orf9ng-1          KVRQWLKKVSAPEYLFDKGVLAAAAAAELDGGRAALRQIGRVRKLPEQQGRYFTADNLSK
                 360       370       380       390       400       410

                         430       440       450       460       470       480
orf9-1.pep        IQMLALSKLPDKREALRGLDKIIEKPPAGSNTELQAEALVQRSVVYDRLGKRKKMISDLE
                  |||||||||||||||| ||::|| |  |:::||  ||||:|||::|:::||| |||:|||
orf9ng-1          IQMLALSKLPDKREALIGLNNIIAKLSAAGSTEPLAEALAQRSIIYEQFGKRGKMIADLE
                 420       430       440       450       460       470

                         490       500       510       520       530       540
orf9-1.pep        RAFRLAPDNAQIMNNLGYSLLTDSKRLDEGFALLQTAYQINPDDTAVNDSIGWAYYLKGD
                   |::|:||||||||||||||||:||||||||||||||||||||||||||||||||||||||
orf9ng-1          TALKLTPDNAQIMNNLGYSLLSDSKRLDEGFALLQTAYQINPDDTAVNDSIGWAYYLKGD
                 480       490       500       510       520       530

                         550       560       570       580       590       600
orf9-1.pep        AESALPYLRYSFENDPEPEVAAHLGEVLWALGERDQAVDVWTQAAHLTGDKKIWRETLKR
                  ||||||||||||||||||||||||||||||||||||||||||||||| ||||||||||||
orf9ng-1          AESALPYLRYSFENDPEPEVAAHLGEVLWALGERDQAVDVWTQAAHLRGDKKIWRETLKR
                 540       550       560       570       580       590

                         610
orf9-1.pep        HGIALPQPSRKPRKX
                  :|||||:||||||||
orf9ng-1          YGIALPEPSRKPRKX
                 600       610
```

In addition, ORF9ng shows significant homology with a hypothetical protein from *P.aeruginosa*:

sp|P42810|YHE3_PSEAE HYPOTHETICAL 64.8 KD PROTEIN IN HEMM-HEMA INTERGENIC REGION (ORF3)
>gi|1072999|pir||S49376 hypothetical protein 3 - Pseudomonas aeruginosa >gi|557259 (X82071) orf3 [Pseudomonas aeruginosa] Length = 576
Score = 128 bits (318), Expect = 1e-28
Identities = 138/587 (23%), Positives = 228/587 (38%), Gaps = 125/587 (21%)

```
Query: 67  VFTLLGGETALQKGQAGTALATYMLMLERTKSPEVAERALEMAVSLNAFEQAEMIYQKWR 126
           +++LL  E A Q+ +   AL+ Y++  ++T+ P V+ERA  +A  L A ++A      W
Sbjct: 53  LYSLLVAELAGQRNRFDIALSNYVVQAQKTRDPGVSERAFRIAEYLGADQEALDTSLLWA 112

Query: 127 QIEPIPGEAQKPAG--------------WLRNVLKEGGNQHLDGLKEVLAQSDDVQKRRI 172
           +  P   +AQ+ A               ++  VL   G+ H D L    A++D   +  +
Sbjct: 113 RSAPDNLDAQRAAAIQLARAGRYEESMVYMEKVLNGQGDTHFDFLALSAAETDPDTRAGL 172

Query: 173 FXXXXXXXXXXXXXXXKASKAVRRAALKYEHLPEAAVADAVFGVQGREKEKAIEALQRLA 232
                              ++      KY +  +     A+   Q    ++A+  L+  +
Sbjct: 173 L------------------QSFDHLLKKYPNNGQLLFGKALLLQQDGRPDEALTLLEDNS 214

Query: 233 KLDTEILPPTLMTLRLTARK-----YPEILDGFFEQTDTQNLSAVWQEMEIMNLVSLRKP 287
               E+ P  L +  L + K      P + G  E  D + +   +  +    LV   +
Sbjct: 215 ASRHEVAPLLLRSRLLQSMKRSDEALPLLKAGIKEHPDDKRVRLAYARL----LVEQNRL 270

Query: 288 DDAYARLNVLLEHNPN---------------------ANLYIQAAI-------------- 312
           DDA A    L++  P+                     A +Y++  +
Sbjct: 271 DDAKAEFAGLVQQFPDDDDDLRFSLALVCLEAQAWDEARIYLEELVERDSHVDAAHFNLG 330

Query: 313 -LAANRKEGASVIDGYAEKAYGRGTGEQRGRAAMTAAMIYADRRDYAKVRQWLKKVSAPE 371
            LA  +K+ A  +D YA+   G G      +   T  ++ A R D A  R    +   P+
Sbjct: 331 RLAEEQKDTARALDEYAQ--VGPGNDFLPAQLRQTDVLLKAGRVDEAAQRLDKARSEQPD 388

Query: 372 YLFDKXXXXXXXXXXXXXXXXXXRQIGRVRKLPEQQGRYFTADNLSKIQMLALSKLPDKR 431
           Y                                        A  L  I+  ALS    +
Sbjct: 389 Y----------------------------------------AIQLYLIEAEALSNNDQQE 408

Query: 432 EALIGLNNIIAKLSAAGSTEPLAEALAQRSIIYEQFGKRGKMIADLETALKLTPDNAQIM 491
           +A   +   + +       E L   L  RS++ E+     +M  DL   +   PDNA  +
Sbjct: 409 KAWQAIQEGLKQYP-----EDL-NLLYTRSMLAEKRNDLAQMEKDLRFVIAREPDNAMAL 462

Query: 492 NNLGYSLLSDSKRLDEGFALLQTAYQINPDDTAVNDSIGWAYYLKGDAESALPYLRYSFE 551
           N LGY+L   + R  E   L+  A+++NPDD A+ DS+GW  Y +G    A  YLR + +
Sbjct: 463 NALGYTLADRTTRYGEARELILKAHKLNPDDPAILDSMGWINYRQGKLADAERYLRQALQ 522

Query: 552 NDPEPEVAAHLGEVLWALGERDQAVDVWTQAAHLRGDKKIWRETLKR 598
             P+ EVAAHLGEVLWA G +  A  +W +    + D  + R T+KR
Sbjct: 523 RYPDHEVAAHLGEVLWAQGRQGDARAIWREYLDKQPDSDVLRRTIKR 569

 gi|2983399 (AE000710) hypothetical protein [Aquifex aeolicus] Length = 545
 Score = 81.5 bits (198), Expect = 1e-14
 Identities = 61/198 (30%), Positives = 98/198 (48%), Gaps = 19/198 (9%)

Query: 408 GRYFTADNL-SKIQMLALSKLPDKREALIGLNNIIAKLSAAGSTEPLAEALAQ------- 459
           G Y  A  L  K ++LA    PDK+E L    +  +K         + + L +
Sbjct: 335 GNYEDAKRLIEKAKVLA----PDKKEILFLEADYYSKTKQYDKALEILKKLEKDYPNDSR 390

Query: 460 ----RSIIYEQFGKRGKMIADLETALKLTPDNAQIMNNLGYSLLS--DSKRLDEGFALLQ 513
                +I+Y+  G        L  A++L P+N    N LGYSLL     +R++E   L++
Sbjct: 391 VYFMEAIVYDNLGDIKNAEKALRKAIELDPENPDYYNYLGYSLLLWYGKERVEEAEELIK 450

Query: 514 TAYQINPDDTAVNDSIGWAYYLKGDAESALPYLRYSF-ENDPEPEVAAHLGEVLWALGER 572
            A +  +P++ A  DS+GW YYLKGD E A+ YL  +  E   +P V  H+G+VL  +G +
Sbjct: 451 KALEKDPENPAYIDSMGWVYYLKGDYERAMQYLLKALREAYDDPVVNEHVGDVLLKMGYK 510

Query: 573 DQAVDVWTQAAHLRGDKK 590
           ++A + + +A  L  + K
Sbjct: 511 EEARNYYERALKLLEEGK 528
```

Based on this analysis, it is predicted that the proteins from *N.meningitidis* and *N.gonorrhoeae,* and their epitopes, could be useful antigens for vaccines or diagnostics, or for raising antibodies.

## Example 7

The following partial DNA sequence was identified in *N.meningitidis* <SEQ ID 49>:

```
  1   AACCTCTACG CCGGCCCGCA GACCACATCC GTCATCGCAA ACATCGCCGA

 51   CAACCTGCAA CTGGCCAAAG ACTACGGCAA AGTACACTGG TTCGCCTCCC
101   CGCTCTTCTG GCTCCTGAAC CAACTGCACA ACATCATCGG CAACTGGGGC
151   TGGGCGATTA TCGTTTTAAC CATCATCGTC AAAGCCGTAC TGTATCCATT
201   GACCAACGCC TCTTACCGCT CTATGGCGAA AATGCGTGCC GCCGCACCCA
251   AACTGCAAGC CATCAAAGAG AAATACGGCG ACGACCGTAT GGCGCAACAA
301   CAGGCGATGA TGCAGCTTTA CACAGACGAG AAAATCAACC CGaCTGGGCG
351   GCTGCCTGCC TATGCTGTTG CAAATCCCCG TCTTCATCGG ATTGTATTGG
401   GCATTGTTCG CCTCCGTAGA ATTGCGCCAG GCACCTTGGC TGGGTTGGAT
451   TACCGACCTC AGCCGCGCCG ACCCCTACTA CATCCTGCCC ATCATTATGG
501   CGGCAACGAT GTTCGCCCAA ACTTATCTGA ACCCGCCGCC GAcCGACCCG
551   ATGCagGCGA AAATGATGAA AATCATGCCG TTGGTTTTCT CsGwCrTGTT
601   CTTCTTCTTC CCTGCCGGks TGGTATTGTA CTGGGTAGTC AACAACCTCC
651   TGACCATCGC CCAGCAATGG CACATCAACC GCAGCATCGA AAAACAACGC
701   GCCCAAGGCG AAGTCGTTTC CTAA
```

This corresponds to the amino acid sequence <SEQ ID 50; ORF11>:

```
  1   ..NLYAGPQTTS VIANIADNLQ LAKDYGKVHW FASPLFWLLN QLHNIIGNWG
 51     WAIIVLTIIV KAVLYPLTNA SYRSMAKMRA AAPKLQAIKE KYGDDRMAQQ
101     QAMMQLYTDE KINPLGGCLP MLLQIPVFIG LYWALFASVE LRQAPWLGWI
151     TDLSRADPYY ILPIIMAATM FAQTYLNPPP TDPMQAKMMK IMPLVFSXXF
201     FFFPAGXVLY WVVNNLLTIA QQWHINRSIE KQRAQGEVVS *
```

Further sequence analysis revealed the complete DNA sequence <SEQ ID 51>:

```
   1   ATGGATTTTA AAAGACTCAC GGCGTTTTTC GCCATCGCGC TGGTGATTAT
  51   GATCGGCTGG GAAAAGATGT TCCCCACTCC GAAGCCAGTC CCCGCGCCCC
 101   AACAGGCAGC ACAACAACAG GCCGTAACCG CTTCCGCCGA AGCCGCGCTC
 151   GCGCCCGCAA CGCCGATTAC CGTAACGACC GACACGGTTC AAGCCGTCAT
 201   TGATGAAAAA AGCGGCGACC TGCGCCGGCT GACCCTGCTC AAATACAAAG
 251   CAACCGGCGA CGAAAATAAA CCGTTCATCC TGTTTGGCGA CGGCAAAGAA
 301   TACACCTACG TCGCCCAATC CGAACTTTTG GACGCGCAGG GCAACAACAT
 351   TCTAAAAGGC ATCGGCTTTA GCGCACCGAA AAAACAGTAC AGCTTGGAAG
 401   GCGACAAAGT TGAAGTCCGC CTGAGCGCGC CTGAAACACG CGGTCTGAAA
 451   ATCGACAAAG TTTATACTTT CACCAAAGGC AGCTATCTGG TCAACGTCCG
 501   CTTCGACATC GCCAACGGCA GCGGTCAAAC CGCCAACCTG AGCGCGGACT
 551   ACCGCATCGT CCGCGACCAC AGCGAACCCG AGGGTCAAGG TTACTTTACC
 601   CACTCTTACG TCGGCCCTGT TGTTTATACC CCTGAAGGCA ACTTCCAAAA
 651   AGTCAGCTTT TCCGACTTGG ACGACGATGC CAAATCCGGC AAATCCGAGG
 701   CCGAATACAT CCGCAAAACC CCGACCGGCT GGCTCGGCAT GATTGAACAC
 751   CACTTCATGT CCACCTGGAT TCTCCAACCT AAAGGCAGAC AAAGCGTTTG
 801   CGCCGCAGGC GAGTGCAACA TCGACATCAA ACGCCGCAAC GACAAGCTGT
 851   ACAGCACCAG CGTCAGCGTG CCTTTAGCCG CCATCCAAAA CGGCGCGAAA
 901   GCCGAAGCCT CCATCAACCT CTACGCCGGC CCGCAGACCA CATCCGTCAT
 951   CGCAAACATC GCCGACAACC TGCAACTGGC CAAAGACTAC GGCAAAGTAC
1001   ACTGGTTCGC CTCCCCGCTC TTCTGGCTCC TGAACCAACT GCACAACATC
1051   ATCGGCAACT GGGGCTGGGC GATTATCGTT TTAACCATCA TCGTCAAAGC
1101   CGTACTGTAT CCATTGACCA ACGCCTCTTA CCGCTCTATG GCGAAAATGC
1151   GTGCCGCCGC ACCCAAACTG CAAGCCATCA AAGAGAAATA CGGCGACGAC
1201   CGTATGGCGC AACAACAGGC GATGATGCAG CTTTACACAG ACGAGAAAAT
1251   CAACCCGCTG GGCGGCTGCC TGCCTATGCT GTTGCAAATC CCCGTCTTCA
1301   TCGGATTGTA TTGGGCATTG TTCGCCTCCG TAGAATTGCG CCAGGCACCT
1351   TGGCTGGGTT GGATTACCGA CCTCAGCCGC GCCGACCCCT ACTACATCCT
1401   GCCCATCATT ATGGCGGCAA CGATGTTCGC CCAAACTTAT CTGAACCCGC
1451   CGCCGACCGA CCCGATGCAG GCGAAAATGA TGAAAATCAT GCCGTTGGTT
1501   TTCTCCGTCA TGTTCTTCTT CTTCCCTGCC GGTCTGGTAT TGTACTGGGT
1551   AGTCAACAAC CTCCTGACCA TCGCCCAGCA ATGGCACATC AACCGCAGCA
1601   TCGAAAAACA ACGCGCCCAA GGCGAAGTCG TTTCCTAA
```

This corresponds to the amino acid sequence <SEQ ID 52; ORF11-1>:

```
  1  MDFKRLTAFF AIALVIMIGW EKMFPTPKPV PAPQQAAQQQ AVTASAEAAL
 51  APATPITVTT DTVQAVIDEK SGDLRRLTLL KYKATGDENK PFILFGDGKE
101  YTYVAQSELL DAQGNNILKG IGFSAPKKQY SLEGDKVEVR LSAPETRGLK
151  IDKVYTFTKG SYLVNVRFDI ANGSGQTANL SADYRIVRDH SEPEGQGYFT
201  HSYVGPVVYT PEGNFQKVSF SDLDDDAKSG KSEAEYIRKT PTGWLGMIEH
251  HFMSTWILQP KGRQSVCAAG ECNIDIKRRN DKLYSTSVSV PLAAIQNGAK
301  AEASINLYAG PQTTSVIANI ADNLQLAKDY GKVHWFASPL FWLLNQLHNI
351  IGNWGWAIIV LTIIVKAVLY PLTNASYRSM AKMRAAAPKL QAIKEKYGDD
401  RMAQQQAMMQ LYTDEKINPL GGCLPMLLQI PVFIGLYWAL FASVELRQAP
451  WLGWITDLSR ADPYYILPII MAATMFAQTY LNPPPTDPMQ AKMMKIMPLV

501  FSVMFFFFPA GLVLYWVVNN LLTIAQQWHI NRSIEKQRAQ GEVVS*
```

Computer analysis of this amino acid sequence gave the following results:

Homology with a 60kDa inner-membrane protein (accession P25754) of *Pseudomonas putida*

ORF11 and the 60kDa protein show 58% aa identity in 229 aa overlap (BLASTp).

```
ORF11   2   LYAGPQTTSVIANIADNLQLAKDYGKVHWFASPLFWLLNQLHNIIGNWGWAIIVLTIIVK  61
            LYAGP+  S +   ++   L+L  DYG + + A P+FWLL  +H+++GNWGW+IIVLT+++K
60K     324 LYAGPKIQSKLKELSPGLELTVDYGFLWFIAQPIFWLLQHIHSLLGNWGWSIIVLTMLIK 383

ORF11   62  AVLYPLTNASYRSMAKMRAAAPKLQAIKEKYGDDRXXXXXXXXXLYTDEKINPLGGCLPM 121
             + +PL+ ASYRSMA+MRA APKL A+KE++GDDR         LY  EKINPLGGCLP+
60K     384 GLFFPLSAASYRSMARMRAVAPKLAALKERFGDDRQKMSQAMMELYKKEKINPLGGCLPI 443

ORF11   122 LLQIPVFIGLYWALFASVELRQAPWLGWITDLSRADPYYILPIIMAATMFAQTYLNPPPT 181
            L+Q+PVF+ LYW L  SVE+RQAPW+ WITDLS  DP++ILPIIM ATMF Q  LNP P
60K     444 LVQMPVFLALYWVLLESVEMRQAPWILWITDLSIKDPFFILPIIMGATMFIQQRLNPTPP 503

ORF11   182 DPMQAKMMKIMPLVXXXXXXXXXPAGXVLYWVVNNLLTIAQQWHINRSIE 230
            DPMQAK+MK+MP++         PAG VLYWVVNN L+I+QQW+I R IE
60K     504 DPMQAKVMKMMPIIFTFFFLWFPAGLVLYWVVNNCLSISQQWYITRRIE 552
```

Homology with a predicted ORF from *N. meningitidis* (strain A)

ORF11 shows 97.9% identity over a 240aa overlap with an ORF (ORF11a) from strain A of *N. meningitidis:*

```
                                                          10        20        30
orf11.pep                              NLYAGPQTTSVIANIADNLQLAKDYGKVHW
                                       |||||||||||||||||||| ||||||||
orf11a       IKRRNDKLYSTSVSVPLAAIQNGAKSXASINLYAGPQTTSVIANIADNLQLXKDYGKVHW
                280       290       300       310       320       330

                    40        50        60        70        80        90
orf11.pep    FASPLFWLLNQLHNIIGNWGWAIIVLTIIVKAVLYPLTNASYRSMAKMRAAAPKLQAIKE
             ||||||||||||||||||||||||||||||||||||||||||||||||||||||||||||
orf11a       FASPLFWLLNQLHNIIGNWGWAIIVLTIIVKAVLYPLTNASYRSMAKMRAAAPKLQAIKE
                340       350       360       370       380       390

                   100       110       120       130       140       150
orf11.pep    KYGDDRMAQQQAMMQLYTDEKINPLGGCLPMLLQIPVFIGLYWALFASVELRQAPWLGWI
             ||||||||||||||||||||||||||||||||||||||||||||||||||||||||||||
orf11a       KYGDDRMAQQQAMMQLYTDEKINPLGGCLPMLLQIPVFIGLYWALFASVELRQAPWLGWI
                400       410       420       430       440       450

                   160       170       180       190       200       210
orf11.pep    TDLSRADPYYILPIIMAATMFAQTYLNPPPTDPMQAKMMKIMPLVFSXXFFFFPAGXVLY
             |||||||||||||||||||||||||||||||||||||||||||||| ||||| |||| |||
orf11a       TDLSRADPYYILPIIMAATMFAQTYLNPPPTDPMQAKMMKIMPLVXSXXFFXFPAGLVLY
                460       470       480       490       500       510

                   220       230       240
orf11.pep    WVVNNLLTIAQQWHINRSIEKQRAQGEVVSX
             ||:||||||||||||||||||||||||||||
orf11a       WVINNLLTIAQQWHINRSIEKQRAQGEVVSX
                520       530       540
```

The complete length ORF11a nucleotide sequence <SEQ ID 53> is:

```
  1  ANGGATTTTA AAAGACTCAC NGNGTTTTTC GCCATCGCAC TGGTGATTAT
 51  GATCGGATNG NAAANGATGT TCCCCACTCC GAAGCCCGTC CCCGCGCCCC
101  AACAGACGGC ACAACAACAG GCCGTAANCG CTTCCGCCGA AGCCGCGCTC
151  GCGCCCGNAN CGCCGATTAC CGTAACGACC GACACGGTTC AAGCCGTCAT
201  TGATGAAAAA AGCGGCGACC TGCGCCGGCT GACCCTGCTC AAATACAAAG
251  CAACCGGCGA CNAAAATAAA CCGTTCATCC TGTTTGGCGA CGGCAAANAA
301  TACACCTACN TCGCCCANTC CGAACTTTTG GACGCGCAGG GCAACAACAT
351  TCTAAAAGGC ATCGGCTTTA GCGCACCGAA AAAACAGTAC AGCTTGGAAG
```

```
 401  GCGACAAAGT TGAAGTCCGC CTGAGCGCAC CTGAAACACG CGGTCTGAAA
 451  ATCGACAAAG TTTATACTTT CACCAAAGGC AGCTATCTGG TCAACGTCCG
 501  CTTCGACATC GCCAACGGCA GCGGTCAAAC CGCCAACCTG AGCGCGGACT
 551  ACCGCATCGT CCGCGACCAC AGCGAACCCG AGGGTCAAGG CTACTTTACC
 601  CACTCTTACG TCGGCCCTGT TGTTTATACC CCTGAAGGCA ACTTCCAAAA
 651  AGTCAGCTTC TCCGACTTGG ACGACGATGC CAANTCCGGN AAATCCGAGG
 701  CCGAATACAT CCGCAAAACC CNGACCGGCT GGCTCGGCAT GATTGAACAC
 751  CACTTCATGT CCACCTGGAT CCTCCAACCC AAAGGCGGAC AAAGCGTTTG
 801  CGCCGCTGGC GACTGCNGTA TNGACATCAA ACGCCGCAAC GACAAGCTGT
 851  ACAGCACCAG CGTCAGCGTG CCTTTAGCCG CTATCCAAAA CGGTGCGAAA
 901  TCCNAAGCCT CCATCAACCT CTACGCCGGC CCACAGACCA CATCNGTTAT
 951  CGCAAACATC GCCGACAACC TGCAACTGGN CAAAGACTAC GGCAAAGTAC
1001  ACTGGTTCGC CTCCCCCCTC TTTTGGCTTT TGAACCAACT GCACAACATC
1051  ATCGGCAACT GGGGCTGGGC GATTATCGTT TTAACCATCA TCGTCAAAGC
1101  CGTACTGTAT CCATTGACCA ACGCCTCTTA CCGTTCGATG GCGAAAATGC
1151  GTGCCGCCGC GCCCAAACTG CAAGCCATCA AAGAGAAATA CGGCGACGAC
1201  CGTATGGCGC AGCAACAAGC CATGATGCAG CTTTACACAG ACGAGAAAAT
1251  CAACCCGCTG GGCGGCTGCC TGCCTATGCT GTTGCAAATC CCCGTCTTCA
1301  TCGGATTGTA TTGGGCATTG TTCGCCTCCG TAGAATTGCG CCAGGCACCT
1351  TGGCTGGGTT GGATTACCGA CCTCAGCCGC GCCGACCCNT ACTACATCCT
1401  GCCCATCATT ATGGCGGCAA CGATGTTCGC CCAAACCTAT CTGAACCCGC
1451  CGCCGACCGA CCCGATGCAG GCGAAAATGA TGAAAATCAT GCCTTTGGTT
1501  NTNTCNNNNA NGTTCTTCNN CTTCCCTGCC GGTCTGGTAT TGTACTGGGT
1551  GATCAACAAC CTCCTGACCA TCGCCCAGCA ATGGCACATC AACCGCAGCA
1601  TCGAAAAACA ACGCGCCCAA GGCGAAGTCG TTTCCTAA
```

This encodes a protein having amino acid sequence <SEQ ID 54>:

```
  1  XDFKRLTXFF AIALVIMIGX XXMFPTPKPV PAPQQTAQQQ AVXASAEAAL
 51  APXXPITVTT DTVQAVIDEK SGDLRRLTLL KYKATGDXNK PFILFGDGKX
101  YTYXAXSELL DAQGNNILKG IGFSAPKKQY SLEGDKVEVR LSAPETRGLK
151  IDKVYTFTKG SYLVNVRFDI ANGSGQTANL SADYRIVRDH SEPEGQGYFT
201  HSYVGPVVYT PEGNFQKVSF SDLDDDAXSG KSEAEYIRKT XTGWLGMIEH
251  HFMSTWILQP KGGQSVCAAG DCXXDIKRRN DKLYSTSVSV PLAAIQNGAK
301  SXASINLYAG PQTTSVIANI ADNLQLXKDY GKVHWFASPL FWLLNQLHNI
351  IGNWGWAIIV LTIIVKAVLY PLTNASYRSM AKMRAAAPKL QAIKEKYGDD
401  RMAQQQAMMQ LYTDEKINPL GGCLPMLLQI PVFIGLYWAL FASVELRQAP
451  WLGWITDLSR ADPYYILPII MAATMFAQTY LNPPPTDPMQ AKMMKIMPLV
501  XSXXFFXFPA GLVLYWVINN LLTIAQQWHI NRSIEKQRAQ GEVVS*
```

ORF11a and ORF11-1 show 95.2% identity in 544 aa overlap:

```
                      10        20        30        40        50        60
orf11a.pep   XDFKRLTXFFAIALVIMIGXXXMFPTPKPVPAPQQTAQQQAVXASAEAALAPXXPITVTT
              |||||| |||||||||||   |||||||||||||:||||||:|||||||||  :||||||
orf11-1      MDFKRLTAFFAIALVIMIGWEKMFPTPKPVPAPQQAAQQQAVTASAEAALAPATPITVTT
                      10        20        30        40        50        60

                      70        80        90       100       110       120
orf11a.pep   DTVQAVIDEKSGDLRRLTLLKYKATGDXNKPFILFGDGKXYTYXAXSELLDAQGNNILKG
             ||||||||||||||||||||||||||| ||||||||||| ||| | ||||||||||||||
orf11-1      DTVQAVIDEKSGDLRRLTLLKYKATGDENKPFILFGDGKEYTYVAQSELLDAQGNNILKG
                      70        80        90       100       110       120

                     130       140       150       160       170       180
orf11a.pep   IGFSAPKKQYSLEGDKVEVRLSAPETRGLKIDKVYTFTKGSYLVNVRFDIANGSGQTANL
             ||||||||||||||||||||||||||||||||||||||||||||||||||||||||||||
orf11-1      IGFSAPKKQYSLEGDKVEVRLSAPETRGLKIDKVYTFTKGSYLVNVRFDIANGSGQTANL
                     130       140       150       160       170       180

                     190       200       210       220       230       240
orf11a.pep   SADYRIVRDHSEPEGQGYFTHSYVGPVVYTPEGNFQKVSFSDLDDDAXSGKSEAEYIRKT
             ||||||||||||||||||||||||||||||||||||||||||||||| ||||||||||||
orf11-1      SADYRIVRDHSEPEGQGYFTHSYVGPVVYTPEGNFQKVSFSDLDDDAKSGKSEAEYIRKT
                     190       200       210       220       230       240

                     250       260       270       280       290       300
orf11a.pep   XTGWLGMIEHHFMSTWILQPKGGQSVCAAGDCXXDIKRRNDKLYSTSVSVPLAAIQNGAK
              ||||||||||||||||||||| |||||||:|  ||||||||||||||||||||||||||
orf11-1      PTGWLGMIEHHFMSTWILQPKGRQSVCAAGECNIDIKRRNDKLYSTSVSVPLAAIQNGAK
                     250       260       270       280       290       300

                     310       320       330       340       350       360
orf11a.pep   SXASINLYAGPQTTSVIANIADNLQLXKDYGKVHWFASPLFWLLNQLHNIIGNWGWAIIV
             : |||||||||||||||||||||||| |||||||||||||||||||||||||||||||||
orf11-1      AEASINLYAGPQTTSVIANIADNLQLAKDYGKVHWFASPLFWLLNQLHNIIGNWGWAIIV
                     310       320       330       340       350       360

                     370       380       390       400       410       420
orf11a.pep   LTIIVKAVLYPLTNASYRSMAKMRAAAPKLQAIKEKYGDDRMAQQQAMMQLYTDEKINPL
             ||||||||||||||||||||||||||||||||||||||||||||||||||||||||||||
orf11-1      LTIIVKAVLYPLTNASYRSMAKMRAAAPKLQAIKEKYGDDRMAQQQAMMQLYTDEKINPL
                     370       380       390       400       410       420

                     430       440       450       460       470       480
orf11a.pep   GGCLPMLLQIPVFIGLYWALFASVELRQAPWLGWITDLSRADPYYILPIIMAATMFAQTY
             ||||||||||||||||||||||||||||||||||||||||||||||||||||||||||||
orf11-1      GGCLPMLLQIPVFIGLYWALFASVELRQAPWLGWITDLSRADPYYILPIIMAATMFAQTY
                     430       440       450       460       470       480

                     490       500       510       520       530       540
orf11a.pep   LNPPPTDPMQAKMMKIMPLVXSXXFFXFPAGLVLYWVINNLLTIAQQWHINRSIEKQRAQ
             |||||||||||||||||||| |  || ||||||||||:||||||||||||||||||||||
orf11-1      LNPPPTDPMQAKMMKIMPLVFSVMFFFFPAGLVLYWVVNNLLTIAQQWHINRSIEKQRAQ
                     490       500       510       520       530       540

orf11a.pep   GEVVSX
             ||||||
orf11-1      GEVVSX
```

Homology with a predicted ORF from *N.gonorrhoeae*

ORF11 shows 96.3% identity over a 240aa overlap with a predicted ORF (ORF11.ng) from *N. gonorrhoeae:*

```
Orf11          NLYAGPQTTSVIANIADNLQLAKDYGKVHWFASPLFWLLNQLHNIIGNWGWAIIVLT   57
               ||||||||||||||||||||||||||||||||||||||||||||||||||||||:|||
orf11ng     MAVNLYAGPQTTSVIANIADNLQLAKDYGKVHWFASPLFWLLNQLHNIIGNWGWAIVVLT   60

orf11       IIVKAVLYPLTNASYRSMAKMRAAAPKLQAIKEKYGDDRMAQQQAMMQLYTDEKINPLGG  117
            |||||||||||||||||||||||||:||:|||||||||||||||||||: ||:||||||
orf11ng     IIVKAVLYPLTNASYRSMAKMRAAAPELQTIKEKYGDDRMAQQQAMMQLFEDEEINPLGG  120

orf11       CLPMLLQIPVFIGLYWALFASVELRQAPWLGWITDLSRADPYYILPIIMAATMFAQTYLN  177
            ||||||||||||||||||||||||||||||||||||||||||||||||||||||||||||
orf11ng     CLPMLLQIPVFIGLYWALFASVELRQAPWLGWITDLSRADPYYILPIIMAATMFAQTYLN  180

orf11       PPPTDPMQAKMMKIMPLVFSXXFFFFPAGXVLYWVVNNLLTIAQQWHINRSIEKQRAQGE  237
            ||||||||||||||||||||  |||||||| |||||||||||||||||||||||||||||
orf11ng     PPPTDPMQAKMMKIMPLVFSVMFFFFPAGLVLYWVVNNLLTIAQQWHINRSIEKQRAQGE  240

orf11       VVS  240
            |||
orf11ng     VVS  243
```

An ORF11ng nucleotide sequence <SEQ ID 55> was predicted to encode a protein having amino acid sequence <SEQ ID 56>:

```
  1  MAVNLYAGPQ TTSVIANIAD NLQLAKDYGK VHWFASPLFW LLNQLHNIIG
 51  NWGWAIVVLT IIVKAVLYPL TNASYRSMAK MRAAAPELQT IKEKYGDDRM
101  AQQQAMMQLF EDEEINPLGG CLPMLLQIPV FIGLYWALFA SVELRQAPWL
151  GWITDLSRAD PYYILPIIMA ATMFAQTYLN PPPTDPMQAK MMKIMPLVFS
201  VMFFFFPAGL VLYWVVNNLL TIAQQWHINR SIEKQRAQGE VVS*
```

Further sequence analysis revealed the complete gonococcal DNA sequence <SEQ ID 57> to be:

```
  1  ATGGATTTTA AAAGACTCAC GGCGTTTTTC GCCATCGCGC TGGTGATTAT
 51  GATCGGCTGG GAAAAAATGT TCCCCACCCC GAAACCCGTC CCCGCGCCCC
```

```
 101  AACAGGCGGC ACAAAAACAG GCAGCAACCG CTTCCGCCGA AGCCGCGCTC
 151  GCGCCCGCAA CGCCGATTAC CGTAACGACC GACACGGTTC AAGCCGTTAT
 201  TGATGAAAAA AGTGGCGACC TGCGCCGGCT GACCCTGCTC AAATACAAAG
 251  CAACCGGCGA CGAAAACAAA CCGTTCGTCC TGTTTGGCGA CGGCAAAGAA
 301  TACACCTACG TCGCCCAATC CGAACTTTTG GACGCGCAGG GCAACAACAT
 351  TCTGAAAGGC ATCGGCTTTA GCGCACCGAA AAAACAGTAC ACCCTCAACG
 401  GCGACACAGT CGAAGTCCGC CTGAGCGCGC CCGAAACCAA CGGACTGAAA
 451  ATCGACAAAG TCTATACCTT TACCAAAGAC AGCTATCTGG TCAACGTCCG
 501  CTTCGACATC GCCAACGGCA GCGGTCAAAC CGCCAACCTG AGCGCGGACT
 551  ACCGCATCGT CCGCGACCAC AGCGAACCCG AGGGTCAAGG CTACTTTACC
 601  CACTCTTACG TCGGCCCTGT TGTTTATACC CCTGAAGGCA ACTTCCAAAA
 651  AGTCAGCTTC TCCgacTTgg acgACGATGC gaaaTccggc aaATccgagg
 701  ccgaatacaT CCGCAAAACC ccgaccggtt ggctcggcat gattgaacac
 751  cacttcatgt ccacctggat cctccAAcct aaaggcggcc aaaacgtttg
 801  cgcccaggga gactgccgta tcgacattaa aCgccgcaac gacaagctgt
 851  acagcgcaag cgtcagcgtg cctttaaccg ctatcccaac ccgggggcca
 901  aaaccgaaaa tggcggTCAA CCTGTATGCC GGTCCGCAAA CCACATCCGT
 951  TATCGCAAAC ATCGCcgacA ACCTGCAACT GGCAAAAGAC TACGGTAAAG
1001  TACACTGGTT CGCATCGCCG CTCTTCTGGC TCCTGAACCA ACTGCACAAC
1051  ATTATCGGCA ACTGGGGCTG GGCAATCGTC GTTTTGACCA TCATCGTCAA
1101  AGCCGTACTG TATCCATTGA CCAACGcctc ctACCGTTCG ATGGCGAAAA
1151  TGCGTGccgc cgcacCcaaA CTGCAGACCA TCAAAGAAAA ATAcgGCGAC
1201  GACCGTATGG CGCAACAGCA AGCGATGATG CAGCTTTACA AAgacgAGAA
1251  AATCAACCCG CTGGGCGGCT GTctgcctat gctgttgCAA ATCCCCGTCT
1301  TCATCGGCTT GTACTGGGCA TTGTTCGCCT CCGTAGAATT GCGCCAGGCA
1351  CCTTGGCTGG GCTGGATTAC CGACCTCAGC CGCGCCGACC CCTACTACAT
1401  CCTGCCCATC ATTATGGCGG CAACGATGTT CGCCCAAACC TATCTGAACC
1451  CGCCGCCGAC CGACCCGATG CAGGCGAAAA TGATGAAAAT CATGCCGTTG
1501  GTTTTCTCCG TCATGTTCTT CTTCTTCCCT GCCGGTTTGG TTCTCTACTG
1551  GGTGGTCAAC AACCTCCTGA CCATCGCCCA GCAGTGGCAC ATCAACCGCA
1601  GCATCGAAAA ACAACGCGCC CAAGGCGAAG TCGTTTCCTA A
```

This encodes a protein having amino acid sequence <SEQ ID 58; ORF1 Ing-I>:

```
  1  MDFKRLTAFF AIALVIMIGW EKMFPTPKPV PAPQQAAQKQ AATASAEAAL
 51  APATPITVTT DTVQAVIDEK SGDLRRLTLL KYKATGDENK PFVLFGDGKE
101  YTYVAQSELL DAQGNNILKG IGFSAPKKQY TLNGDTVEVR LSAPETNGLK
151  IDKVYTFTKD SYLVNVRFDI ANGSGQTANL SADYRIVRDH SEPEGQGYFT
201  HSYVGPVVYT PEGNFQKVSF SDLDDDAKSG KSEAEYIRKT PTGWLGMIEH
251  HFMSTWILQP KGGQNVCAQG DCRIDIKRRN DKLYSASVSV PLTAIPTRGP
301  KPKMAVNLYA GPQTTSVIAN IADNLQLAKD YGKVHWFASP LFWLLNQLHN
351  IIGNWGWAIV VLTIIVKAVL YPLTNASYRS MAKMRAAAPK LQTIKEKYGD
401  DRMAQQQAMM QLYKDEKINP LGGCLPMLLQ IPVFIGLYWA LFASVELRQA
451  PWLGWITDLS RADPYYILPI IMAATMFAQT YLNPPPTDPM QAKMMKIMPL
501  VFSVMFFFFP AGLVLYWVVN NLLTIAQQWH INRSIEKQRA QGEVVS*
```

ORF11ng-1 and ORF11-1 shown 95.1% identity in 546 aa overlap:

```
                          10        20        30        40        50        60
orf11ng-1.pep     MDFKRLTAFFAIALVIMIGWEKMFPTPKPVPAPQQAAQKQAATASAEAALAPATPITVTT
                  |||||||||||||||||||||||||||||||||||||||:||:|||||||||||||||||
orf11-1           MDFKRLTAFFAIALVIMIGWEKMFPTPKPVPAPQQAAQQQAVTASAEAALAPATPITVTT
                          10        20        30        40        50        60

                          70        80        90       100       110       120
orf11ng-1.pep     DTVQAVIDEKSGDLRRLTLLKYKATGDENKPFVLFGDGKEYTYVAQSELLDAQGNNILKG
                  ||||||||||||||||||||||||||||||||:|||||||||||||||||||||||||||
orf11-1           DTVQAVIDEKSGDLRRLTLLKYKATGDENKPFILFGDGKEYTYVAQSELLDAQGNNILKG
                          70        80        90       100       110       120

                         130       140       150       160       170       180
orf11ng-1.pep     IGFSAPKKQYTLNGDTVEVRLSAPETNGLKIDKVYTFTKDSYLVNVRFDIANGSGQTANL
                  ||||||||||:|:|| |||||||||| ||||||||||||| |||||||||||||||||||
orf11-1           IGFSAPKKQYSLEGDKVEVRLSAPETRGLKIDKVYTFTKGSYLVNVRFDIANGSGQTANL
                         130       140       150       160       170       180

                         190       200       210       220       230       240
orf11ng-1.pep     SADYRIVRDHSEPEGQGYFTHSYVGPVVYTPEGNFQKVSFSDLDDDAKSGKSEAEYIRKT
                  ||||||||||||||||||||||||||||||||||||||||||||||||||||||||||||
orf11-1           SADYRIVRDHSEPEGQGYFTHSYVGPVVYTPEGNFQKVSFSDLDDDAKSGKSEAEYIRKT
                         190       200       210       220       230       240
```

```
                         250       260       270       280       290       300
orf11ng-1.pep     PTGWLGMIEHHFMSTWILQPKGGQNVCAQGDCRIDIKRRNDKLYSASVSVPLTAIPTRGP
                  |||||||||||||||||||||| |:||| |:| ||||||||||||:||||||:|| :  |
orf11-1           PTGWLGMIEHHFMSTWILQPKGRQSVCAAGECNIDIKRRNDKLYSTSVSVPLAAIQN-GA
                         250       260       270       280       290

                         310       320       330       340       350       360
orf11ng-1.pep     KPKMAVNLYAGPQTTSVIANIADNLQLAKDYGKVHWFASPLFWLLNQLHNIIGNWGWAIV
                  | : ::||||||||||||||||||||||||||||||||||||||||||||||||||||||:
orf11-1           KAEASINLYAGPQTTSVIANIADNLQLAKDYGKVHWFASPLFWLLNQLHNIIGNWGWAII
                300       310       320       330       340       350

                         370       380       390       400       410       420
orf11ng-1.pep     VLTIIVKAVLYPLTNASYRSMAKMRAAAPKLQTIKEKYGDDRMAQQQAMMQLYKDEKINP
                  ||||||||||||||||||||||||||||||||:||||||||||||||||||||| ||||||
orf11-1           VLTIIVKAVLYPLTNASYRSMAKMRAAAPKLQAIKEKYGDDRMAQQQAMMQLYTDEKINP
                360       370       380       390       400       410

                         430       440       450       460       470       480
orf11ng-1.pep     LGGCLPMLLQIPVFIGLYWALFASVELRQAPWLGWITDLSRADPYYILPIIMAATMFAQT
                  ||||||||||||||||||||||||||||||||||||||||||||||||||||||||||||
orf11-1           LGGCLPMLLQIPVFIGLYWALFASVELRQAPWLGWITDLSRADPYYILPIIMAATMFAQT
                420       430       440       450       460       470

                         490       500       510       520       530       540
orf11ng-1.pep     YLNPPPTDPMQAKMMKIMPLVFSVMFFFFPAGLVLYWVVNNLLTIAQQWHINRSIEKQRA
                  ||||||||||||||||||||||||||||||||||||||||||||||||||||||||||||
orf11-1           YLNPPPTDPMQAKMMKIMPLVFSVMFFFFPAGLVLYWVVNNLLTIAQQWHINRSIEKQRA
                480       490       500       510       520       530

orf11ng-1.pep     QGEVVSX
                  |||||||
orf11-1           QGEVVSX
                540
```

In addition, ORF11ng-1 shows significant homology with an inner-membrane protein from the database (accession number p25754):

```
ID   60IM_PSEPU     STANDARD;      PRT;   560 AA.
AC   P25754;
DT   01-MAY-1992 (REL. 22, CREATED)
DT   01-MAY-1992 (REL. 22, LAST SEQUENCE UPDATE)
DT   01-NOV-1995 (REL. 32, LAST ANNOTATION UPDATE)
DE   60 KD INNER-MEMBRANE PROTEIN. . . .

SCORES      Init1:  1074 Initn:  1293 Opt:  1103
Smith-Waterman score: 1406;    41.5% identity in 574 aa overlap

                           10        20                         30        40
orf11ng-1.pep   MDFKR---LTAFFAIALVIMIGW-----EKMFPT------------PKPVPAPQQAAQKQ
                ||:||   ::|: ::: |::: |     :  :||            |  ||| :::|: :
p25754          MDIKRTILIAALAVVSYVMVLKWNDDYGQAALPTQNTAASTVAPGLPDGVPAGNNGASAD
                        10        20        30        40        50        60

                        50               60        70        80         90
orf11ng-1.pep   AATASAEAALAPATPIT-------VTTDTVQAVIDEKSGDLRRLTLLKYKATGDE-NKPF
                : :|:||:: |  :|::       | ||::: :||  :||: :|:| ||    |: | ||
p25754          VPSANAESSPAELAPVALSKDLIRVKTDVLELAIDPVGGDIVQLNLPKYPRRQDHPNIPF
                        70        80        90       100       110       120

                     100       110       120          130           140
orf11ng-1.pep   VLFGDGKEYTYVAQSELLDAQGNNILKGIG---FSAPKKQYTL-NGD---TVEVRLSAPE
                 || :| | :|:||| |  ::| :  :: |   ::| :|:| | :|:   :|::::|
p25754          QLFDNGGERVYLAQSGLTGTDGPDA-RASGRPLYAAEQKSYQLADGQEQLVVDLKFS---
                       130       140        150       160       170

                  150       160       170       180       190         200
orf11ng-1.pep   TNGLKIDKVYTFTKDSYLVNVRFDIANGSGQTANLSADYRIVRDHS-EPEGQGYF-THSY
```

```
               ||::  | ::| :  | :|| : | | |||: | :    :: || | :| ::    | :|
p25754         DNGVNYIKRFSFKRGEYDLNVSYLIDNQSGQAWNGNMFAQLKRDASGDPSSSTATGTATY
                180       190       200       210       220       230

                  210       220       230       240       250       260
orf11ng-1.pep  VGPVVYTPEGNFQKVSFSDLDDDAKSGKSEAEYIRKTPTGWLGMIEHHFMSTWILQPKGG
               :| :::|    ::|||::|:|   |:: :|      ::  ||:: ::|:|:::||   |:
p25754         LGAALWTASEPYKKVSMKDID---KGSLKE-----NVSGGWVAWLQHYFVTAWI-PAKSD
                240       250           260            270       280

                  270       280       290       300       310       320
orf11ng-1.pep  QNVCAQGDCRIDIKRRNDKLYSASVSVPLTAIPTRGPKPKMAVNLYAGPQTTSVIANIAD
               :||         :: :: ::   |   : : |: ::|: | | : :: ||||:  | : :::
p25754         NNV-------VQTRKDSQGNYIIGYTGPVISVPA-GGKVETSALLYAGPKIQSKLKELSP
               290              300       310       320       330

                 330       340       350       360       370       380
orf11ng-1.pep  NLQLAKDYGKVHWF-ASPLFWLLNQLHNIIGNWGWAIVVLTIIVKAVLYPLTNASYRSMA
               :|:|: ||| : || |:|:||||:::|:::|||||:|:|||:::|::::||: |||||||
p25754         GLELTVDYGFL-WFIAQPIFWLLQHIHSLLGNWGWSIIVLTMLIKGLFFPLSAASYRSMA
             340       350       360       370       380       390

                 390       400       410       420       430       440
orf11ng-1.pep  KMRAAAPKLQTIKEKYGDDRMAQQQAMMQLYKDEKINPLGGCLPMLLQIPVFIGLYWALF
               :|||:|||| ::||::||||: ::||||:||| |||||||||||:|:|:|||::|||:|:
p25754         RMRAVAPKLAALKERFGDDRQKMSQAMMELYKKEKINPLGGCLPILVQMPVFLALYWVLL
              400       410       420       430       440       450

                 450       460       470       480       490       500
orf11ng-1.pep  ASVELRQAPWLGWITDLSRADPYYILPIIMAATMFAQTYLNPPPTDPMQAKMMKIMPLVF
                |||:||||||: ||||||  ||::||||||:|||| |   ||| | ||||||:||:||::|
p25754         ESVEMRQAPWILWITDLSIKDPFFILPIIMGATMFIQQRLNPTPPDPMQAKVMKMMPIIF
              460       470       480       490       500       510

                 510       520       530       540
orf11ng-1.pep  SVMFFFFPAGLVLYWVVNNLLTIAQQWHINRSIEKQRAQGEVVSX
               : :|::||||||||||||| |:|:|||:|:| ||
p25754         TFFFLWFPAGLVLYWVVNNCLSISQQWYITRRIEAATKKAAA
              520       530       540       550       560
```

Based on this analysis, including the homology to an inner-membrane protein from *P. putida* and the predicted transmembrane domains (seen in both the meningococcal and gonoccal proteins), it is predicted that the proteins from *N.meningitidis* and *N.gonorrhoeae,* and their epitopes, could be useful antigens for vaccines or diagnostics, or for raising antibodies.

## Example 8

The following partial DNA sequence was identified in *N.meningitidis* <SEQ ID 59>:

```
  1  ..GCCGTCTTAA TCATCGAATT ATTGACGGGA ACGGTTTATC TTTTGGTTGT
 51    NAGCGCGGCT TTGGCGGGTT CGGGCATTGC TTACGGGCTG ACCGGCAGTA
101    CGCCTGCCGC CGTCTTGACC GNCGCTCTGC TTTCCGCGCT GGGTATTTNG
151    TTCGTACACG CCAAAACCGC CGTTAGAAAA GTTGAAACGG ATTCATATCA
201    GGATTTGGAT GCCGGACAAT ATGTCGAAAT CCTCCGNCAC ACAGGCGGCA
251    ACCGTTACGA AGTT.TTTAT CGCGGTACG. ACTGGCAGGC TCAAAATACG
301    GGGCAAGAAG AGCTTGAACC AGGAACTCGC GCCCTCATTG TCCGCAAGGA
351    AGGCAACCTT CTTATTATCA CACACCCTTA A
```

This corresponds to the amino acid sequence <SEQ ID 60; ORF13>:

```
  1  ..AVLIIELLTG TVYLLVVSAA LAGSGIAYGL TGSTPAAVLT XALLSALGIX
 51    FVHAKTAVRK VETDSYQDLD AGQYVEILRH TGGNRYEVXY RGTXWQAQNT
101    GQEELEPGTR ALIVRKEGNL LIITHP*
```

Further sequence analysis elaborated the DNA sequence slightly <SEQ ID 61>:

```
  1  ..GCCGTCTTAA TCATCGAATT ATTGACGGGA ACGGTTTATC TTTTGGTTGT
 51    nAGCGCGGCT TTGGCGGGTT CGGGCATTGC TTACGGGCTG ACCGGCAGTA
101    CGCCTGCCGC CGTCTTGACC GnCGCTCTGC TTTCCGCGCT GGGTATTTnG
151    TTCGTACACG CCAAAACCGC CGTTAGAAAA GTTGAAACGG ATTCATATCA
201    GGATTTGGAT GCCGGACAAT ATGTCGAAAT CCTCCGACAC ACAGGCGGCA
251    ACCGTTACGA AGTTTTtTAT CGCGGTACGc ACTGGCAGGC TCAAAATACG
301    GGCAAGAAG AGCTTGAACC AGGAACTCGC GCCCTCATTG TCCGCAAGGA
351    AGGCAACCTT CTTATTATCA CACACCCTTA A
```

This corresponds to the amino acid sequence <SEQ ID 62; ORF13-1>:

```
  1  ..AVLIIELLTG TVYLLVVSAA LAGSGIAYGL TGSTPAAVLT XALLSALGIX
 51    FVHAKTAVRK VETDSYQDLD AGQYVEILRH TGGNRYEVFY RGTHWQAQNT
101    GQEELEPGTR ALIVRKEGNL LIITHP*
```

Computer analysis of this amino acid sequence gave the following results:

Homology with a predicted ORF from *N. meningitidis* (strain A)

ORF13 shows 92.9% identity over a 126aa overlap with an ORF (ORF13a) from strain A of *N. meningitidis:*

```
                          10        20        30        40        50
orf13.pep             AVLIIELLTGTVYLLVVSAALAGSGIAYGLTGSTPAAVLTXALLSALGIXF
                      |||||||||||||||||||||||||||||||||||||||| ||||||||| |
orf13a           MTVWFVAAVAVLIIELLTGTVYLLVVSAALAGSGIAYGLTGSTPAAVLTAALLSALGIWF
                         10        20        30        40        50        60

                        60        70        80        90       100       110
orf13.pep        VHAKTAVRKVETDSYQDLDAGQYVEILRHTGGNRYEVXYRGTXWQAQNTGQEELEPGTRA
                 ||||||| ||||||||||||||||:|||||:||||||| |||| ||||||||||||||||
orf13a           VHAKTAVGKVETDSYQDLDAGQYAEILRHAGGNRYEVFYRGTHWQAQNTGQEELEPGTRA
                         70        80        90       100       110       120

                        120
orf13.pep        LIVRKEGNLLIITHPX
                 ||||||||||||::||
orf13a           LIVRKEGNLLIIAKPX
                        130
```

The complete length ORF13a nucleotide sequence <SEQ ID 63> is:

```
  1  ATGACTGTAT GGTTTGTTGC CGCTGTTGCC GTCTTAATCA TCGAATTATT
 51  GACGGGAACG GTTTATCTTT TGGTTGTCAG CGCGGCTTTG GCGGGTTCGG
101  GCATTGCTTA CGGGCTGACC GGCAGCACGC CTGCCGCCGT CTTGACCGCC
151  GCTCTGCTTT CCGCGCTGGG TATTTGGTTC GTACACGCCA AAACCGCCGT
201  GGGAAAAGTT GAAACGGATT CATATCAGGA TTTGGATGCC GGGCAATATG
251  CCGAAATCCT CCGGCACGCA GGCGGCAACC GTTACGAAGT TTTTTATCGC
301  GGTACGCACT GGCAGGCTCA AAATACGGGG CAAGAAGAGC TTGAACCAGG
351  AACGCGCGCC CTAATCGTCC GCAAGGAAGG CAACCTTCTT ATCATCGCAA
401  AACCTTAA
```

This encodes a protein having amino acid sequence <SEQ ID 64>:

```
  1  MTVWFVAAVA VLIIELLTGT VYLLVVSAAL AGSGIAYGLT GSTPAAVLTA
 51  ALLSALGIWF VHAKTAVGKV ETDSYQDLDA GQYAEILRHA GGNRYEVFYR
101  GTHWQAQNTG QEELEPGTRA LIVRKEGNLL IIAKP*
```

ORF13a and ORF13-1 show 94.4% identity in 126 aa overlap

```
                     10        20        30        40        50        60
orf13a.pep   MTVWFVAAVAVLIIELLTGTVYLLVVSAALAGSGIAYGLTGSTPAAVLTAALLSALGIWF
                       |||||||||||||||||||||||||||||||||||||| |||||||| |
orf13-1                AVLIIELLTGTVYLLVVSAALAGSGIAYGLTGSTPAAVLTXALLSALGIXF
                               10        20        30        40        50

                     70        80        90       100       110       120
orf13a.pep   VHAKTAVGKVETDSYQDLDAGQYAEILRHAGGNRYEVFYRGTHWQAQNTGQEELEPGTRA
             ||||||| ||||||||||||||:|||||:|||||||||||||||||||||||||||||||
orf13-1      VHAKTAVRKVETDSYQDLDAGQYVEILRHTGGNRYEVFYRGTHWQAQNTGQEELEPGTRA
                     60        70        80        90       100       110

                    130
orf13a.pep   LIVRKEGNLLIIAKPX
             ||||||||||||::||
orf13-1      LIVRKEGNLLIITHPX
                    120
```

Homology with a predicted ORF from *N.gonorrhoeae*

ORF13 shows 89.7% identity over a 126aa overlap with a predicted ORF (ORF13.ng) *from N. gonorrhoeae*:

```
orf13              AVLIIELLTGTVYLLVVSAALAGSGIAYGLTGSTPAAVLTXALLSALGIXF   51
                   |||||||||||||||||||||||||||||||||||||||| |||||||| |
orf13ng   MTVWFVAAVAVLIIELLTGTVYLLVVSAALAGSGIAYGLTGSTPAAVLTAALLSALGIWF   60

orf13     VHAKTAVRKVETDSYQDLDAGQYVEILRHTGGNRYEVXYRGTXWQAQNTGQEELEPGTRA  111
          ||||||| |||||||||||:|:|:||||:|||||||| |||| ||||||||| :||||||
orf13ng   VHAKTAVGKVETDSYQDLDTGKYAEILRYTGGNRYEVFYRGTHWQAQNTGQEVFEPGTRA  120

orf13     LIVRKEGNLLIITHP  126
          ||||||||||||::|
orf13ng   LIVRKEGNLLIIANP  135
```

The complete length ORF13ng nucleotide sequence <SEQ ID 65> is:

```
  1  ATGACTGTAT GGTTTGTTGC CGCTGTTGCC GTCTTAATCA TCGAATTATT
 51  GACGGGAACG GTTTATCTTT TGGTTGTCAG CGCGGCTTTG GCGGGTTCGG
101  GCATTGCCTA CGGGCTGACT GGCAGCACGC CTGCCGCCGT CTTGACCGCC
151  GCACTGCTTT CCGCGCTGGG CATTTGGTTC GTACATGCCA AAACCGCCGT
201  GGGAAAAGTT GAAACGGATT CATATCAGGA TTTGGATACC GGAAAATATG
251  CCGAAATCCT CCGATACACA GGCGGCAACC GTTACGAAGT TTTTTATCGC
301  GGTACGCACT GGCAGGCGCA AAATACGGGG CAGGAAGTGT TTGAACCGGG
351  AACGCGCGCC CTCATCGTCC GCAAAGAAGG TAACCTTCTT ATCATCGCAA
401  ACCCTTAA
```

This encodes a protein having amino acid sequence <SEQ ID 66>:

```
  1  MTVWFVAAVA VLIIELLTGT VYLLVVSAAL AGSGIAYGLT GSTPAAVLTA
 51  ALLSALGIWF VHAKTAVGKV ETDSYQDLDT GKYAEILRYT GGNRYEVFYR
101  GTHWQAQNTG QEVFEPGTRA LIVRKEGNLL IIANP*
```

ORF13ng shows 91.3% identity in 126 aa overlap with ORF13-1:

```
                                    10        20        30        40        50
orf13-1.pep               AVLIIELLTGTVYLLVVSAALAGSGIAYGLTGSTPAAVLTXALLSALGIXF
                          |||||||||||||||||||||||||||||||||||||||| |||||||| |
orf13ng         MTVWFVAAVAVLIIELLTGTVYLLVVSAALAGSGIAYGLTGSTPAAVLTAALLSALGIWF
                        10        20        30        40        50        60

                       60        70        80        90       100       110
orf13-1.pep     VHAKTAVRKVETDSYQDLDAGQYVEILRHTGGNRYEVFYRGTHWQAQNTGQEELEPGTRA
                ||||||| ||||||||||:|:|:||||:|||||||||||||||||||||||| :||||||
orf13ng         VHAKTAVGKVETDSYQDLDTGKYAEILRYTGGNRYEVFYRGTHWQAQNTGQEVFEPGTRA
                        70        80        90       100       110       120

                      120
orf13-1.pep     LIVRKEGNLLIITHPX
                ||||||||||||::||
orf13ng         LIVRKEGNLLIIANPX
                       130
```

Based on this analysis, including the extensive leader sequence in this protein, it is predicted that ORF13 and ORF13ng are likely to be outer membrane proteins. It is thus predicted that the proteins from *N.meningitidis* and *N.gonorrhoeae,* and their epitopes, could be useful antigens for vaccines or diagnostics, or for raising antibodies.

**Example 9**

The following DNA sequence was identified in *N.meningitidis* <SEQ ID 67>:

```
  1  ATGTwTGATT TCGGTTTrGG CGArCTGGTT TTTGTCGGCA TTATCGCCCT
 51  GATwGtCCTC GGCCCCGAAC GCsTGCCCGA GGCCGCCCGC AyCGCCGGAC
101  GGcTCATCGG CAGGCTGCAA CGCTTTGTCG GcAGCGTCAA ACAGGAATTT
151  GACACTCAAA TCGAACTGGA AGAACTGAGG AAGGCAAAGC AGGAATTTGA
201  AGCTGCCGcC GCTCAGGTTC GAGACAGCCT CAAAGAAACC GGTACGGATA
251  TGGAAGGCAA TCTGCACGAC ATTTCCGACG GTCTGAAGCC TTGGGAAAAA
301  CTGCCCGAAC AGCGGACACC TGCCGATTTC GGTGTCGATG AAAACGGCAA
351  TCCGCT.TCC CGATGCGGCA AACACCCTAT CAGACGGCAT TTCCGACGTT
401  ATGCCGTC..
```

This corresponds to the amino acid sequence <SEQ ID 68; ORF2>:

```
  1  MXDFGLGELV FVGIIALIVL GPERXPEAAR XAGRLIGRLQ RFVGSVKQEF
 51  DTQIELEELR KAKQEFEAAA AQVRDSLKET GTDMEGNLHD ISDGLKPWEK
101  LPEQRTPADF GVDENGNPXS RCGKHPIRRH FRRYAV..
```

Further work revealed the complete nucleotide sequence <SEQ ID 69>:

```
  1 ATGTTTGATT TCGGTTTGGG CGAGCTGGTT TTTGTCGGCA TTATCGCCCT
 51 GATTGTCCTC GGCCCCGAAC GCCTGCCCGA GGCCGCCCGC ACCGCCGGAC
101 GGCTCATCGG CAGGCTGCAA CGCTTTGTCG GCAGCGTCAA ACAGGAATTT
151 GACACTCAAA TCGAACTGGA AGAACTGAGG AAGGCAAAGC AGGAATTTGA
201 AGCTGCCGCC GCTCAGGTTC GAGACAGCCT CAAAGAAACC GGTACGGATA
251 TGGAAGGCAA TCTGCACGAC ATTTCCGACG GTCTGAAGCC TTGGGAAAAA
301 CTGCCCGAAC AGCGGACACC TGCCGATTTC GGTGTCGATG AAAACGGCAA
351 TCCGCTTCCC GATGCGGCAA ACACCCTATC AGACGGCATT TCCGACGTTA
401 TGCCGTCCGA ACGTTCCTAC GCTTCCGCCG AAACCCTTGG GGACAGCGGG
451 CAAACCGGCA GTACAGCCGA ACCCGCGGAA ACCGACCAAG ACCGCGCATG
501 GCGGGAATAC CTGACTGCTT CTGCCGCCGC ACCCGTCGTA CAGACCGTCG
551 AAGTCAGCTA TATCGATACT GCTGTTGAAA CGCCTGTTCC GCACACCACT
601 TCCCTGCGCA AACAGGCAAT AAGCCGCAAA CGCGATTTTC GTCCGAAACA
651 CCGCGCCAAA CCTAAATTGC GCGTCCGTAA ATCATAA
```

This corresponds to the amino acid sequence <SEQ ID 70; ORF2-1>:

```
  1 MFDFGLGELV FVGIIALIVL GPERLPEAAR TAGRLIGRLQ RFVGSVKQEF
 51 DTQIELEELR KAKQEFEAAA AQVRDSLKET GTDMEGNLHD ISDGLKPWEK
101 LPEQRTPADF GVDENGNPLP DAANTLSDGI SDVMPSERSY ASAETLGDSG
151 QTGSTAEPAE TDQDRAWREY LTASAAAPVV QTVEVSYIDT AVETPVPHTT
201 SLRKQAISRK RDFRPKHRAK PKLRVRKS*
```

Further work identified the corresponding gene in strain A of *N.meningitidis* <SEQ ID 71 >:

```
  1 ATGTTTGATT TCGGTTTGGG CGAGCTGGTT TTTGTCGGCA TTATCGCCCT
 51 GATTGTCCTC GGCCCCGAAC GCCTGCCCGA GGCCGCCCGC ACCGCCGGAC
101 GGCTCATCGG CAGGCTGCAA CGCTTTGTCG GCAGCGTCAA ACAGGAATTT
151 GACACGCAAA TCGAACTGGA AGAACTAAGG AAGGCAAAGC AGGAATTTGA
201 AGCTGCCGCT GCTCAGGTTC GAGACAGCCT CAAAGAAACC GGTACGGATA
251 TGGAGGGTAA TCTGCACGAC ATTTCCGACG GTCTGAAGCC TTGGGAAAAA
301 CTGCCCGAAC AGCGCACGCC TGCTGATTTC GGTGTCGATG AAAACGGCAA
351 TCCCTTTCCC GATGCGGCAA ACACCCTATT AGACGGCATT TCCGACGTTA
401 TGCCGTCCGA ACGTTCCTAC GCTTCCGCCG AAACCCTTGG GGACAGCGGG
451 CAAACCGGCA GTACAGCCGA ACCCGCGGAA ACCGACCAAG ACCGTGCATG
501 GCGGGAATAC CTGACTGCTT CTGCCGCCGC ACCCGTCGTA CAGACCGTCG
551 AAGTCAGCTA TATCGATACC GCTGTTGAAA CCCCTGTTCC GCATACCACT
601 TCGCTGCGTA AACAGGCAAT AAGCCGCAAA CGCGATTTGC GTCCTAAATC
651 CCGCGCCAAA CCTAAATTGC GCGTCCGTAA ATCATAA
```

This encodes a protein having amino acid sequence <SEQ ID 72; ORF2a>:

```
  1 MFDFGLGELV FVGIIALIVL GPERLPEAAR TAGRLIGRLQ RFVGSVKQEF
 51 DTQIELEELR KAKQEFEAAA AQVRDSLKET GTDMEGNLHD ISDGLKPWEK
101 LPEQRTPADF GVDENGNPFP DAANTLLDGI SDVMPSERSY ASAETLGDSG
151 QTGSTAEPAE TDQDRAWREY LTASAAAPVV QTVEVSYIDT AVETPVPHTT
201 SLRKQAISRK RDLRPKSRAK PKLRVRKS*
```

The originally-identified partial strain B sequence (ORF2) shows 97.5% identity over a 118aa overlap with ORF2a:

```
                   10        20        30        40        50        60
orf2.pep   MXDFGLGELVFVGIIALIVLGPERXPEAARXAGRLIGRLQRFVGSVKQEFDTQIELEELR
           | |||||||||||||||||||||| |||||:|||||||||||||||||||||||||||||
orf2a      MFDFGLGELVFVGIIALIVLGPERLPEAARTAGRLIGRLQRFVGSVKQEFDTQIELEELR
                   10        20        30        40        50        60

                   70        80        90       100       110       120
orf2.pep   KAKQEFEAAAAQVRDSLKETGTDMEGNLHDISDGLKPWEKLPEQRTPADFGVDENGNPXS
           ||||||||||||||||||||||||||||||||||||||||||||||||||||||||||
orf2a      KAKQEFEAAAAQVRDSLKETGTDMEGNLHDISDGLKPWEKLPEQRTPADFGVDENGNPFP
                   70        80        90       100       110       120

                  130
orf2.pep   RCGKHPIRRHFRRYAV

orf2a      DAANTLLDGISDVMPSERSYASAETLGDSGQTGSTAEPAETDQDRAWREYLTASAAAPVV
                  130       140       150       160       170       180
```

The complete strain B sequence (ORF2-1) and ORF2a show 98.2% identity in 228 aa overlap:

```
orf2a.pep   MFDFGLGELVFVGIIALIVLGPERLPEAARTAGRLIGRLQRFVGSVKQEFDTQIELEELR  60
            ||||||||||||||||||||||||||||||||||||||||||||||||||||||||||||
orf2-1      MFDFGLGELVFVGIIALIVLGPERLPEAARTAGRLIGRLQRFVGSVKQEFDTQIELEELR  60

orf2a.pep   KAKQEFEAAAAQVRDSLKETGTDMEGNLHDISDGLKPWEKLPEQRTPADFGVDENGNPFP 120
            ||||||||||||||||||||||||||||||||||||||||||||||||||||||||||:|
orf2-1      KAKQEFEAAAAQVRDSLKETGTDMEGNLHDISDGLKPWEKLPEQRTPADFGVDENGNPLP 120

orf2a.pep   DAANTLLDGISDVMPSERSYASAETLGDSGQTGSTAEPAETDQDRAWREYLTASAAAPVV 180
            |||||| |||||||||||||||||||||||||||||||||||||||||||||||||||||
orf2-1      DAANTLSDGISDVMPSERSYASAETLGDSGQTGSTAEPAETDQDRAWREYLTASAAAPVV 180

orf2a.pep   QTVEVSYIDTAVETPVPHTTSLRKQAISRKRDLRPKSRAKPKLRVRKSX 229
            ||||||||||||||||||||||||||||||||:||| ||||||||||||
orf2-1      QTVEVSYIDTAVETPVPHTTSLRKQAISRKRDFRPKHRAKPKLRVRKSX 229
```

Further work identified a partial DNA sequence <SEQ ID 73> in *N.gonorrhoeae* encoding the following amino acid sequence <SEQ ID 74; ORF2ng>:

```
  1  MFDFGLGELI FVGIIALIVL GPERLPEAAR TAGRLIGRLQ RFVGSVKQEL
 51  DTQIELEELR KVKQAFEAAA AQVRDSLKET DTDMQNSLHD ISDGLKPWEK
101  LPEQRTPADF GVDEKGNSLS RYGKHRIRRH FRRYAV*
```

Further work identified the complete gonococcal gene sequence <SEQ ID 75>:

```
  1  ATGTTTGATT TCGGTTTGGG CGAGCTGATT TTTGTCGGCA TTATCGCCCT
 51  GATTGTCCTT GGTCCAGAAC GCCTGCCCGA AGCCGCCCGC ACTGCCGGAC
101  GGCTTATCGG CAGGCTGCAA CGCTTTGTAG GAAGCGTCAA ACAAGAACTT
151  GACACTCAAA TCGAACTGGA AGAGCTGAGG AAGGTCAAGC AGGCATTCGA
201  AGCTGCCGCC GCTCAGGTTC GAGACAGCCT CAAAGAAACC GATACGGATA
251  TGCAGAACAG TCTGCACGAC ATTTCCGACG GTCTGAAGCC TTGGGAAAAA
301  CTGCCCGAAC AGCGCACGCc tgccgatttc gGTGTCGATg AAAacggcaa
351  tccccttccc gATACGGCAA ACACCGTATC AGACGGCATT TCCGACGTTA
401  TGCCGTCTGA ACGTTCCGAT ACTtccgcCG AAACCCTTGG GGACGACAGG
451  CAAACCGGCA GTACAGCCGA ACCTGCGGAA ACCGACAAAG ACCGCGCATG
501  GCGGGAATAC CTGactgctt ctgccgccgc acctgtcgta Cagagggccg
551  tcgaagtcag ctaTATCGAT ACTGCTGTTG AAacgcctgT tccgcaCacc
601  acttccctgc gcaAACAGGC AATAAACCGC AAACGCGATT TttgtccgaA
651  ACACCGCGCc aAACCGAAat tgcgcgtcCG TAAATCATAA
```

This encodes a protein having the amino acid sequence <SEQ ID 76; ORF2ng-1>:

```
  1  MFDFGLGELI FVGIIALIVL GPERLPEAAR TAGRLIGRLQ RFVGSVKQEL
 51  DTQIELEELR KVKQAFEAAA AQVRDSLKET DTDMQNSLHD ISDGLKPWEK
101  LPEQRTPADF GVDENGNPLP DTANTVSDGI SDVMPSERSD TSAETLGDDR
151  QTGSTAEPAE TDKDRAWREY LTASAAAPVV QRAVEVSYID TAVETPVPHT
201  TSLRKQAINR KRDFCPKHRA KPKLRVRKS*
```

The originally-identified partial strain B sequence (ORF2) shows 87.5% identity over a 136aa overlap with ORF2ng:

```
orf2.pep    MXDFGLGELVFVGIIALIVLGPERXPEAARXAGRLIGRLQRFVGSVKQEFDTQIELEELR   60
            | |||||||:|||||||||||||| |||||:|||||||||||||||||||:||||||||||
orf2ng      MFDFGLGELIFVGIIALIVLGPERLPEAARTAGRLIGRLQRFVGSVKQELDTQIELEELR   60

orf2.pep    KAKQEFEAAAAQVRDSLKETGTDMEGNLHDISDGLKPWEKLPEQRTPADFGVDENGNPXS  120
            |:|| |||||||||||||| |||:::||||||||||||||||||||||||||||:||
orf2ng      KVKQAFEAAAAQVRDSLKETDTDMQNSLHDISDGLKPWEKLPEQRTPADFGVDEKGNSLP  120

orf2.pep    RCGKHPIRRHFRRYAV  136
            | ||| ||||||||||
orf2ng      RYGKHRIRRHFRRYAV  136
```

The complete strain B and gonococcal sequences (ORF2-1 & ORF2ng-1) show 91.7% identity in 229 aa overlap:

```
                       10        20        30        40        50        60
orf2-1.pep     MFDFGLGELVFVGIIALIVLGPERLPEAARTAGRLIGRLQRFVGSVKQEFDTQIELEELR
               |||||||||:||||||||||||||||||||||||||||||||||||||||:|||||||||
orf2ng-1       MFDFGLGELIFVGIIALIVLGPERLPEAARTAGRLIGRLQRFVGSVKQELDTQIELEELR
                       10        20        30        40        50        60

                       70        80        90       100       110       120
orf2-1.pep     KAKQEFEAAAAQVRDSLKETGTDMEGNLHDISDGLKPWEKLPEQRTPADFGVDENGNPLP
               |:|| ||||||||||||||| |||:::|||||||||||||||||||||||||||||||||
orf2ng-1       KVKQAFEAAAAQVRDSLKETDTDMQNSLHDISDGLKPWEKLPEQRTPADFGVDENGNPLP
                       70        80        90       100       110       120

                      130       140       150       160       170       180
orf2-1.pep     DAANTLSDGISDVMPSERSYASAETLGDSGQTGSTAEPAETDQDRAWREYLTASAAAPVV
               |:|||:||||||||||||| :|||||||: ||||||||||||:|||||||||||||||||
orf2ng-1       DTANTVSDGISDVMPSERSDTSAETLGDDRQTGSTAEPAETDKDRAWREYLTASAAAPVV
                      130       140       150       160       170       180

                       190       200       210       220      229
orf2-1.pep     Q-TVEVSYIDTAVETPVPHTTSLRKQAISRKRDFRPKHRAKPKLRVRKSX
               | :|||||||||||||||||||||||||:||||| |||||||||||||||
orf2ng-1       QRAVEVSYIDTAVETPVPHTTSLRKQAINRKRDFCPKHRAKPKLRVRKSX
                      190       200       210       220       230
```

Computer analysis of these amino acid sequences indicates a transmembrane region (underlined), and also revealed homology (59% identity) between the gonococcal sequence and the TatB protein of *E.coli:*

```
 gnl|PID|e1292181 (AJ005830) TatB protein [Escherichia coli] Length = 171
 Score = 56.6 bits (134), Expect = 1e-07
 Identities = 30/88 (34%), Positives = 52/88 (59%), Gaps = 1/88 (1%)

Query: 1  MFDFGLGELIFVGIIALIVLGPERLPEAARTAGRLIGRLQRFVGSVKQELDTQIELEELR 60
          MFD G  EL+ V II L+VLGP+RLP A +T    I  L+    +V+ EL  +++L+E +
Sbjct: 1  MFDIGFSELLLVFIIGLVVLGPQRLPVAVKTVAGWIRALRSLATTVQNELTQELKLQEFQ 60

Query: 61 -KVKQAFEAAAAQVRDSLKETDTDMQNS 87
            +K+  +A+   +   LK +  +++ +
Sbjct: 61 DSLKKVEKASLTNLTPELKASMDELRQA 88
```

Based on this analysis, it was predicted that ORF2, ORF2a and ORF2ng are likely to be membrane proteins and so the proteins from *N.meningitidis* and *N.gonorrhoeae,* and their epitopes, could be useful antigens for vaccines or diagnostics, or for raising antibodies.

ORF2-1 (16kDa) was cloned in pET and pGex vectors and expressed in *E.coli,* as described above. The products of protein expression and purification were analyzed by SDS-PAGE. Figure 3A shows the results of affinity purification of the GST-fusion protein, and Figure 3B shows the results of expression of the His-fusion in *E.coli.* Purified GST-fusion protein was used to immunise mice, whose sera were used for Western blots (Figure 3C), ELISA (positive result), and FACS analysis (Figure 3D). These experiments confirm that ORF37-1 is a surface-exposed protein, and that it is a useful immunogen.

**Example 10**

The following partial DNA sequence was identified in *N. meningitidis* <SEQ ID 77>:

1 ATGCAAGCAC GGCTGCTGAT ACCTATTCTT TTTTCAGTTT TTATTTTATC
51 CGC.TGCGGG ACACTGACAG GTATTCCATC GCATGGCGgA GkTAAACgCT
101 TTgCGGTCGA ACAAGAACTT GTGGCCGCTT CTGCCAGAGC TGCCGTTAAA
151 GACATGGATT TACAGGCATT ACACGGACGA AAAGTTGCAT TGTACATTGC
201 CACTATGGGC GACCAAGGTT CAGGcAGTTT GACAGGGGGG TCGCTACTCC
251 ATTGATGCAC kGrTwCsTGG CGAATACATA AACAGCCCTG CCGTCCGTAC
301 CGATTACACC TATCCACGTT ACGAAACCAC CGCTGAAACA ACATCAGGCG
351 GTTTGACAGG TTTAACCACT TCTTTATCTA CACTTAATGC CCCTGCACTC
401 TCTCGCACCC AATCAGACGG TAGCGGAAGT AAAAGCAGTC TGGGCTTAAA
451 TATTGGCGGG ATGGGGGATT ATCGAAATGA AACCTTGACG ACTAACCCGC
501 GCGACACTGC CTTTCTTTCC CACTTGGTAC AGACCGTATT TTTCCTGCGC
551 GGCATAGACG TTGTTTCTCC TGCCAATGCC GATACAGATG TGTTTATTAA
601 CATCGACGTA TTCGGAACGA TACGCAACAG AACCGAAATG..

This corresponds to the amino acid sequence <SEQ ID 78; ORF15>:

1 MQARLLIPIL FSVFILSACG TLTGIPSHGG XKRFAVEQEL VAASARAAVK
51 DMDLQALHGR KVALYIATMG DQGSGSLTGG RYSIDAXXXG EYINSPAVRT
101 DYTYPRYETT AETTSGGLTG LTTSLSTLNA PALSRTQSDG SGSKSSLGLN
151 IGGMGDYRNE TLTTNPRDTA FLSHLVQTVF FLRGIDVVSP ANADTDVFIN
201 IDVFGTIRNR TEM..

Further work revealed the complete nucleotide sequence <SEQ ID 79>:

1 ATGCAAGCAC GGCTGCTGAT ACCTATTCTT TTTTCAGTTT TTATTTTATC
51 CGCCTGCGGG ACACTGACAG GTATTCCATC GCATGGCGGA GGTAAACGCT
101 TTGCGGTCGA ACAAGAACTT GTGGCCGCTT CTGCCAGAGC TGCCGTTAAA
151 GACATGGATT TACAGGCATT ACACGGACGA AAAGTTGCAT TGTACATTGC
201 CACTATGGGC GACCAAGGTT CAGGCAGTTT GACAGGGGGT CGCTACTCCA
251 TTGATGCACT GATTCGTGGC GAATACATAA ACAGCCCTGC CGTCCGTACC
301 GATTACACCT ATCCACGTTA CGAAACCACC GCTGAAACAA CATCAGGCGG
351 TTTGACAGGT TTAACCACTT CTTTATCTAC ACTTAATGCC CCTGCACTCT
401 CTCGCACCCA ATCAGACGGT AGCGGAAGTA AAAGCAGTCT GGGCTTAAAT
451 ATTGGCGGGA TGGGGGATTA TCGAAATGAA ACCTTGACGA CTAACCCGCG
501 CGACACTGCC TTTCTTTCCC ACTTGGTACA GACCGTATTT TTCCTGCGCG
551 GCATAGACGT TGTTTCTCCT GCCAATGCCG ATACAGATGT GTTTATTAAC
601 ATCGACGTAT TCGGAACGAT ACGCAACAGA ACCGAAATGC ACCTATACAA
651 TGCCGAAACA CTGAAAGCCC AAACAAAACT GGAATATTTC GCAGTAGACA
701 GAACCAATAA AAAATTGCTC ATCAAACCAA AAACCAATGC GTTTGAAGCT
751 GCCTATAAAG AAAATTACGC ATTGTGGATG GGGCCGTATA AAGTAAGCAA
801 AGGAATTAAA CCGACGGAAG GATTAATGGT CGATTTCTCC GATATCCGAC
851 CATACGGCAA TCATACGGGT AACTCCGCCC CATCCGTAGA GGCTGATAAC

901 AGTCATGAGG GGTATGGATA CAGCGATGAA GTAGTGCGAC AACATAGACA
951 AGGACAACCT TGA

This corresponds to the amino acid sequence <SEQ ID 80; ORF15-1>:

```
  1  MQARLLIPIL FSVFILSACG TLTGIPSHGG GKRFAVEQEL VAASARAAVK
 51  DMDLQALHGR KVALYIATMG DQGSGSLTGG RYSIDALIRG EYINSPAVRT
101  DYTYPRYETT AETTSGGLTG LTTSLSTLNA PALSRTQSDG SGSKSSLGLN
151  IGGMGDYRNE TLTTNPRDTA FLSHLVQTVF FLRGIDVVSP ANADTDVFIN
201  IDVFGTIRNR TEMHLYNAET LKAQTKLEYF AVDRTNKKLL IKPKTNAFEA
251  AYKENYALWM GPYKVSKGIK PTEGLMVDFS DIRPYGNHTG NSAPSVEADN
301  SHEGYGYSDE VVRQHRQGQP *
```

Further work identified the corresponding gene in strain A of *N.meningitidis* <SEQ ID 81>:

```
  1  ATGCAAGCAC GGCTGCTGAT ACCTATTCTT TTTTCAGTTT TTATTTTATC
 51  CGCCTGCGGG ACACTGACAG GTATTCCATC GCATGGCGGA GGTAAACGCT
101  TTGCGGTCGA ACAAGAACTT GTGGCCGCTT CTGCCAGAGC TGCCGTTAAA
151  GACATGGATT TACAGGCATT ACACGGACGA AAAGTTGCAT TGTACATTGC
201  AACTATGGGC GACCAAGGTT CAGGCAGTTT GACAGGGGGT CGCTACTCCA
251  TTGATGCACT GATTCGTGGC GAATACATAA ACAGCCCTGC CGTCCGTACC
301  GATTACACCT ATCCACGTTA CGAAACCACC GCTGAAACAA CATCAGGCGG
351  TTTGACAGGT TTAACCACTT CTTTATCTAC ACTTAATGCC CCTGCACTCT
401  CGCGCACCCA ATCAGACGGT AGCGGAAGTA AAAGCAGTCT GGGCTTAAAT
451  ATTGGCGGGA TGGGGGATTA TCGAAATGAA ACCTTGACGA CTAACCCGCG
501  CGACACTGCC TTTCTTTCCC ACTTGGTACA GACCGTATTT TTCCTGCGCG
551  GCATAGACGT TGTTTCTCCT GCCAATGCCG ATACGGATGT GTTTATTAAC
601  ATCGACGTAT TCGGAACGAT ACGCAACAGA ACCGAAATGC ACCTATACAA
651  TGCCGAAACA CTGAAAGCCC AAACAAAACT GGAATATTTC GCAGTAGACA
701  GAACCAATAA AAAATTGCTC ATCAAACCAA AAACCAATGC GTTTGAAGCT
751  GCCTATAAAG AAAATTACGC ATTGTGGATG GGACCGTATA AAGTAAGCAA
801  AGGAATTAAA CCGACAGAAG GATTAATGGT CGATTTCTCC GATATCCAAC
851  CATACGGCAA TCATATGGGT AACTCTGCCC CATCCGTAGA GGCTGATAAC
901  AGTCATGAGG GGTATGGATA CAGCGATGAA GCAGTGCGAC GACATAGACA
951  AGGGCAACCT TGA
```

This encodes a protein having amino acid sequence <SEQ ID 82; ORF15a>:

```
  1  MQARLLIPIL FSVFILSACG TLTGIPSHGG GKRFAVEQEL VAASARAAVK
 51  DMDLQALHGR KVALYIATMG DQGSGSLTGG RYSIDALIRG EYINSPAVRT
101  DYTYPRYETT AETTSGGLTG LTTSLSTLNA PALSRTQSDG SGSKSSLGLN
151  IGGMGDYRNE TLTTNPRDTA FLSHLVQTVF FLRGIDVVSP ANADTDVFIN
201  IDVFGTIRNR TEMHLYNAET LKAQTKLEYF AVDRTNKKLL IKPKTNAFEA
251  AYKENYALWM GPYKVSKGIK PTEGLMVDFS DIQPYGNHMG NSAPSVEADN
301  SHEGYGYSDE AVRRHRQGQP *
```

The originally-identified partial strain B sequence (ORF 15) shows 98.1 % identity over a 213aa overlap with ORF15a:

```
                     10        20        30        40        50        60
orf15.pep    MQARLLIPILFSVFILSACGTLTGIPSHGGXKRFAVEQELVAASARAAVKDMDLQALHGR
             |||||||||||||||||||||||||||||| |||||||||||||||||||||||||||||
orf15a       MQARLLIPILFSVFILSACGTLTGIPSHGGGKRFAVEQELVAASARAAVKDMDLQALHGR
                     10        20        30        40        50        60

                     70        80        90       100       110       120
orf15.pep    KVALYIATMGDQGSGSLTGGRYSIDAXXXGEYINSPAVRTDYTYPRYETTAETTSGGLTG
             ||||||||||||||||||||||||||   |||||||||||||||||||||||||||||||
orf15a       KVALYIATMGDQGSGSLTGGRYSIDALIRGEYINSPAVRTDYTYPRYETTAETTSGGLTG
                     70        80        90       100       110       120

                    130       140       150       160       170       180
orf15.pep    LTTSLSTLNAPALSRTQSDGSGSKSSLGLNIGGMGDYRNETLTTNPRDTAFLSHLVQTVF
             ||||||||||||||||||||||||||||||||||||||||||||||||||||||||||||
orf15a       LTTSLSTLNAPALSRTQSDGSGSKSSLGLNIGGMGDYRNETLTTNPRDTAFLSHLVQTVF
                    130       140       150       160       170       180

                    190       200       210
orf15.pep    FLRGIDVVSPANADTDVFINIDVFGTIRNRTEM
             |||||||||||||||||||||||||||||||||

orf15a       FLRGIDVVSPANADTDVFINIDVFGTIRNRTEMHLYNAETLKAQTKLEYFAVDRTNKKLL
                    190       200       210       220       230       240
```

The complete strain B sequence (ORF15-1) and ORF15a show 98.8% identity in 320 aa overlap:

```
                     10        20        30        40        50        60
orf15a.pep   MQARLLIPILFSVFILSACGTLTGIPSHGGGKRFAVEQELVAASARAAVKDMDLQALHGR
             ||||||||||||||||||||||||||||||||||||||||||||||||||||||||||||
orf15-1      MQARLLIPILFSVFILSACGTLTGIPSHGGGKRFAVEQELVAASARAAVKDMDLQALHGR
                     10        20        30        40        50        60

                     70        80        90       100       110       120
orf15a.pep   KVALYIATMGDQGSGSLTGGRYSIDALIRGEYINSPAVRTDYTYPRYETTAETTSGGLTG
             ||||||||||||||||||||||||||||||||||||||||||||||||||||||||||||
orf15-1      KVALYIATMGDQGSGSLTGGRYSIDALIRGEYINSPAVRTDYTYPRYETTAETTSGGLTG
                     70        80        90       100       110       120

                    130       140       150       160       170       180
orf15a.pep   LTTSLSTLNAPALSRTQSDGSGSKSSLGLNIGGMGDYRNETLTTNPRDTAFLSHLVQTVF
             ||||||||||||||||||||||||||||||||||||||||||||||||||||||||||||
orf15-1      LTTSLSTLNAPALSRTQSDGSGSKSSLGLNIGGMGDYRNETLTTNPRDTAFLSHLVQTVF
                    130       140       150       160       170       180

                    190       200       210       220       230       240
orf15a.pep   FLRGIDVVSPANADTDVFINIDVFGTIRNRTEMHLYNAETLKAQTKLEYFAVDRTNKKLL
             ||||||||||||||||||||||||||||||||||||||||||||||||||||||||||||
orf15-1      FLRGIDVVSPANADTDVFINIDVFGTIRNRTEMHLYNAETLKAQTKLEYFAVDRTNKKLL
                    190       200       210       220       230       240

                    250       260       270       280       290       300
orf15a.pep   IKPKTNAFEAAYKENYALWMGPYKVSKGIKPTEGLMVDFSDIQPYGNHMGNSAPSVEADN
             ||||||||||||||||||||||||||||||||||||||||||:||||| |||||||||||
orf15-1      IKPKTNAFEAAYKENYALWMGPYKVSKGIKPTEGLMVDFSDIRPYGNHTGNSAPSVEADN
                    250       260       270       280       290       300

                    310       320
orf15a.pep   SHEGYGYSDEAVRRHRQGQPX
             ||||||||||:||:|||||||
orf15-1      SHEGYGYSDEVVRQHRQGQPX
                    310       320
```

Further work identified the corresponding gene in *N.gonorrhoeae* <SEQ ID 83>:

```
  1 ATGCGGGCAC GGCTGCTGAT ACCTATTCTT TTTTCAGTTT TTATTTTATC
 51 CGCCTGCGGG ACACTGACAG GTATTCCATC GCATGGCGGA GGCAAACGCT
101 TCGCGGTCGA ACAAGAACTT GTGGCCGCTT CTGCCAGAGC TGCCGTTAAA
151 GACATGGATT TACAGGCATT ACACGGACGA AAAGTTGCAT TGTACATTGC
201 AACTATGGGC GACCAAGGTT CAGGCAGTTT GACAGGGGGT CGCTACTCCA
251 TTGATGCACT GATTCGCGGC GAATACATAA ACAGCCCTGC CGTCCGCACC
301 GATTACACCT ATCCGCGTTA CGAAACCACC GCTGAAACAA CATCAGGCGG
351 TTTGACGGGT TTAACCACTT CTTTATCTAC ACTTAATGCC CCTGCACTCT
401 CGCGCACCCA ATCAGACGGT AGCGGAAGTA GGAGCAGTCT GGGCTTAAAT
451 ATTGGCGGGA TGGGGGATTA TCGAAATGAA ACCTTGACGA CCAACCCGCG
501 CGACACTGCC TTTCTTTCCC ACTTGGTGCA GACCGTATTT TTCCTGCGCG
551 GCATAGACGT TGTTTCTCCT GCCAATGCCG ATACAGATGT GTTTATTAAC
601 ATCGACGTAT TCGGAACGAT ACGCAACAGA ACCGAAATGC ACCTATACAA
651 TGCCGAAACA CTGAAAGCCC AAACAAAACT GGAATATTTC GCAGTAGACA
701 GAACCAATAA AAAATTGCTC ATCAAACCCA AAACCAATGC GTTTGAAGCT
751 GCCTATAAAG AAAATTACGC ATTGTGGATG GGGCCGTATA AAGTAAGCAA
801 AGGAATCAAA CCGACGGAAG GATTGATGGT CGATTTCTCC GATATCCAAC
851 CATACGGCAA TCATACGGGT AACTCCGCCC CATCCGTAGA GGCTGATAAC
901 AGTCATGAGG GGTATGGATA CAGCGATGAA GCAGTGCGAC AACATAGACA
951 AGGGCAACCT TGA
```

This encodes a protein having amino acid sequence <SEQ ID 84; ORF15ng>:

```
  1 MRARLLIPIL FSVFILSACG TLTGIPSHGG GKRFAVEQEL VAASARAAVK
 51 DMDLQALHGR KVALYIATMG DQGSGSLTGG RYSIDALIRG EYINSPAVRT
101 DYTYPRYETT AETTSGGLTG LTTSLSTLNA PALSRTQSDG SGSRSSLGLN
151 IGGMGDYRNE TLTTNPRDTA FLSHLVQTVF FLRGIDVVSP ANADTDVFIN
201 IDVFGTIRNR TEMHLYNAET LKAQTKLEYF AVDRTNKKLL IKPKTNAFEA
```

```
251 AYKENYALWM GPYKVSKGIK PTEGLMVDFS DIQPYGNHTG NSAPSVEADN
301 SHEGYGYSDE AVRQHRQGQP *
```

The originally-identified partial strain B sequence (ORF15) shows 97.2% identity over a 213aa overlap with ORF15ng:

```
orf15.pep  MQARLLIPILFSVFILSACGTLTGIPSHGGXKRFAVEQELVAASARAAVKDMDLQALHGR   60
           |:|||||||||||||||||||||||||||| |||||||||||||||||||||||||||||
orf15ng    MRARLLIPILFSVFILSACGTLTGIPSHGGGKRFAVEQELVAASARAAVKDMDLQALHGR   60

orf15.pep  KVALYIATMGDQGSGSLTGGRYSIDAXXXGEYINSPAVRTDYTYPRYETTAETTSGGLTG  120
           ||||||||||||||||||||||||||   |||||||||||||||||||||||||||||||
orf15ng    KVALYIATMGDQGSGSLTGGRYSIDALIRGEYINSPAVRTDYTYPRYETTAETTSGGLTG  120

orf15.pep  LTTSLSTLNAPALSRTQSDGSGSKSSLGLNIGGMGDYRNETLTTNPRDTAFLSHLVQTVF  180
           |||||||||||||||||||||||:||||||||||||||||||||||||||||||||||||
orf15ng    LTTSLSTLNAPALSRTQSDGSGSRSSLGLNIGGMGDYRNETLTTNPRDTAFLSHLVQTVF  180

orf15.pep  FLRGIDVVSPANADTDVFINIDVFGTIRNRTEM                            213
           |||||||||||||||||||||||||||||||||
orf15ng    FLRGIDVVSPANADTDVFINIDVFGTIRNRTEMHLYNAETLKAQTKLEYFAVDRTNKKLL  240
```

The complete strain B sequence (ORF15-1) and ORF15ng show 98.8% identity in 320 aa overlap:

```
                        10        20        30        40        50        60
orf15-1.pep     MQARLLIPILFSVFILSACGTLTGIPSHGGGKRFAVEQELVAASARAAVKDMDLQALHGR
                |:||||||||||||||||||||||||||||||||||||||||||||||||||||||||||
orf15ng         MRARLLIPILFSVFILSACGTLTGIPSHGGGKRFAVEQELVAASARAAVKDMDLQALHGR
                        10        20        30        40        50        60

                        70        80        90       100       110       120
orf15-1.pep     KVALYIATMGDQGSGSLTGGRYSIDALIRGEYINSPAVRTDYTYPRYETTAETTSGGLTG
                ||||||||||||||||||||||||||||||||||||||||||||||||||||||||||||
orf15ng         KVALYIATMGDQGSGSLTGGRYSIDALIRGEYINSPAVRTDYTYPRYETTAETTSGGLTG
                        70        80        90       100       110       120

                       130       140       150       160       170       180
orf15-1.pep     LTTSLSTLNAPALSRTQSDGSGSKSSLGLNIGGMGDYRNETLTTNPRDTAFLSHLVQTVF
                |||||||||||||||||||||||:||||||||||||||||||||||||||||||||||||
orf15ng         LTTSLSTLNAPALSRTQSDGSGSRSSLGLNIGGMGDYRNETLTTNPRDTAFLSHLVQTVF
                       130       140       150       160       170       180

                       190       200       210       220       230       240
orf15-1.pep     FLRGIDVVSPANADTDVFINIDVFGTIRNRTEMHLYNAETLKAQTKLEYFAVDRTNKKLL
                ||||||||||||||||||||||||||||||||||||||||||||||||||||||||||||
orf15ng         FLRGIDVVSPANADTDVFINIDVFGTIRNRTEMHLYNAETLKAQTKLEYFAVDRTNKKLL
                       190       200       210       220       230       240

                       250       260       270       280       290       300
orf15-1.pep     IKPKTNAFEAAYKENYALWMGPYKVSKGIKPTEGLMVDFSDIRPYGNHTGNSAPSVEADN
                ||||||||||||||||||||||||||||||||||||||||||:|||||||||||||||||
orf15ng         IKPKTNAFEAAYKENYALWMGPYKVSKGIKPTEGLMVDFSDIQPYGNHTGNSAPSVEADN
                       250       260       270       280       290       300

                       310       320
orf15-1.pep     SHEGYGYSDEVVRQHRQGQPX
                ||||||||||:||||||||||
orf15ng         SHEGYGYSDEAVRQHRQGQPX
                       310       320
```

Computer analysis of these amino acid sequences reveals an ILSAC motif (putative membrane lipoprotein lipid attachment site, as predicted by the MOTIFS program). indicates a putative leader sequence, and it was predicted that the proteins from *N.meningitidis* and *N.gonorrhoeae,* and their epitopes, could be useful antigens for vaccines or diagnostics, or for raising antibodies.

ORF15-1 (31.7kDa) was cloned in pET and pGex vectors and expressed in *E.coli,* as described above. The products of protein expression and purification were analyzed by SDS-PAGE. Figure 4A shows the results of affinity purification of the GST-fusion protein, and Figure 4B shows the results of expression of the His-fusion in *E.coli.* Purified GST-fusion protein was used to immunise mice, whose sera were used for Western blot (Figure 4C) and ELISA (positive result). These experiments confirm that ORFX-1 is a surface-exposed protein, and that it is a useful immunogen.

**Example 11**

The following partial DNA sequence was identified in *N.meningitidis* <SEQ ID 85>:

```
  1  ..GG.CAGCACA AAAAACAGGC GGTTGAACGG AAAAACCGTA TTTACGATGA
 51    TGCCGGGTAT GATATTCGGC GTATTCACGG GCGCATTCTC CGCAAAATAT
101    ATCCCCGCGT TCGGGCTTCA AATTTTCTTC ATCCTGTTTT TAACCGCCGT
151    CGCATTCAAA ACACTGCATA CCGACCCTCA GACGGCATCC CGCCCGCTGC
201    CCGGACTGCC CrGACTGACT GCGGTTTCCA CACTGTTCGG CACAATGTCG
251    AGCTGGGTCG GCATAGGCGG CGGTTCACTT TCCGTCCCCT TCTTAATCCA
301    CTGCGGCTTC CCCGCCCATA AAGCCATCGG CACATCATCC GGCCTTGCCT
351    GGCCGATTGC ACTCTCCGGC GCAATATCGT ATCTGCTCAA CGGCCTGAAT
401    ATTGCAGGAT TGCCCGAAGG GTCACTGGGC TTCCTTTACC TGCCCGCCGT
451    CGCCGTCCTC AGCGCGGCAA CCATTGCCTT TGCCCCGCTC GGTGTCAAAA
501    CCGCCCACAA ACTTTCTTCT GCCAAACTCA AAAAATC.TT CGGCATTATG
551    TTGCTTTTGA TTGCCGGAAA AATGCTGTAC AACCTGCTTT AA
```

This corresponds to the amino acid sequence <SEQ ID 86; ORF17>:

```
  1 ..GQHKKQAVNG KTVFTMMPGM IFGVFTGAFS AKYIPAFGLQ IFFILFLTAV
 51   AFKTLHTDPQ TASRPLPGLP XLTAVSTLFG TMSSWVGIGG GSLSVPFLIH
101   CGFPAHKAIG TSSGLAWPIA LSGAISYLLN GLNIAGLPEG SLGFLYLPAV
151   AVLSAATIAF APLGVKTAHK LSSAKLKKSF GIMLLLIAGK MLYNLL*
```

Further work revealed the complete nucleotide sequence <SEQ ID 87>:

```
  1 ATGTGGCATT GGGACATTAT CTTAATCCTG CTTGCCGTAG GCAGTGCGGC
 51 AGGTTTTATT GCCGGCCTGT TCGGCGTAGG CGGCGGCACG CTGATTGTCC
101 CTGTCGTTTT ATGGGTGCTT GATTTGCAGG GTTTGGCACA ACATCCTTAC
151 GCGCAACACC TCGCCGTCGG CACATCCTTC GCCGTCATGG TCTTCACCGC
201 CTTTTCCAGT ATGCTGGGGC AGCACAAAAA ACAGGCGGTC GACTGGAAAA
251 CCGTATTTAC GATGATGCCG GGTATGATAT TCGGCGTATT CACGGGCGCA
301 CTCTCCGCAA AATATATCCC CGCGTTCGGG CTTCAAATTT TCTTCATCCT
351 GTTTTTAACC GCCGTCGCAT TCAAAACACT GCATACCGAC CCTCAGACGG
401 CATCCCGCCC GCTGCCCGGA CTGCCCGGAC TGACTGCGGT TTCCACACTG
451 TTCGGCACAA TGTCGAGCTG GGTCGGCATA GGCGGCGGTT CACTTTCCGT
501 CCCCTTCTTA ATCCACTGCG GCTTCCCCGC CCATAAAGCC ATCGGCACAT
551 CATCCGGCCT TGCCTGGCCG ATTGCACTCT CCGGCGCAAT ATCGTATCTG
601 CTCAACGGCC TGAATATTGC AGGATTGCCC GAAGGGTCAC TGGGCTTCCT
651 TTACCTGCCC GCCGTCGCCG TCCTCAGCGC GGCAACCATT GCCTTTGCCC
701 CGCTCGGTGT CAAAACCGCC CACAAACTTT CTTCTGCCAA ACTCAAAAAA
751 Tc.TTCGGCA TTATGTTGCT TTTGATTGCC GGAAAAATGC TGTACAACCT
801 GCTTTAA
```

This corresponds to the amino acid sequence <SEQ ID 88; ORF17-1>:

```
  1 MWHWDIILIL LAVGSAAGFI AGLFGVGGGT LIVPVVLWVL DLQGLAQHPY
 51 AQHLAVGTSF AVMVFTAFSS MLGQHKKQAV DWKTVFTMMP GMIFGVFTGA

101 LSAKYIPAFG LQIFFILFLT AVAFKTLHTD PQTASRPLPG LPGLTAVSTL
151 FGTMSSWVGI GGGSLSVPFL IHCGFPAHKA IGTSSGLAWP IALSGAISYL
201 LNGLNIAGLP EGSLGFLYLP AVAVLSAATI AFAPLGVKTA HKLSSAKLKK
251 XFGIMLLLIA GKMLYNLL*
```

Computer analysis of this amino acid sequence gave the following results:

Homology with hypothetical *H.influenzae* transmembrane protein HI0902 (accession number P44070

ORF17 and HI0902 proteins show 28% aa identity in 192 aa overlap:

```
ORF17    3   HKKQAVNGKTVFTMMPGMIFGVFT-GAFSAKYIPAFGLQIF--FILFLTAVAFKTLHTDP 59
             HK  +  + V  + P ++  VF  G F  +         +IF   +++L      ++  D
HI0902   72  HKLGNIVWQAVRILAPVIMLSVFICGLFIGRLDREISAKIFACLVVYLATKMVLSIKKD- 130

ORF17    60  QTASRPLPGLPXLTAVSTLFGTMSSWVGIGGGSLSVPFLIHCGFPAHKAIGTSSGLAWPI 119
             Q  ++ L  L  +      L G  SS  GIGGG   VPFL   G    +AIG+S+     +
HI0902   131 QVTTKSLTPLSSVIG-GILIGMASSAAGIGGGFIVPFLTARGINIKQAIGSSAFCGMLL 189

ORF17    120 ALSGAISYLLNGLNIAGLPEGSLGFLYLPAVAVLSAATIAFAPLGVXXXXXXXXXXXXXX 179
              +SG  S++++G      +PE SLG++YLPAV  ++A +    + LG
HI0902   190 GISGMFSFIVSGWGNPLMPEYSLGYIYLPAVLGITATSFFTSKLGASATAKLPVSTLKKG 249

ORF17    180 FGIMLLLIAGKM 191
             F + L+++A  M
HI0902   250 FALFLIVVAINM 261
```

Homology with a predicted ORF from *N.meningitidis* (strain A)

ORF17 shows 96.9% identity over a 196aa overlap with an ORF (ORF17a) from strain A of *N. meningitidis:*

```
                                                       10        20        30
orf17.pep                               GQHKKQAVNGKTVFTMMPGMIFGVFTGAFS
                                        ||||||||: ||||||||||:||||:||:|
orf17a       QGLAQHPYAQHLAVGTSFAVMVFTAFSSMLGQHKKQAVDWKTVFTMMPGMVFGVFAGALS
                     50        60        70        80        90       100

                   40        50        60        70        80        90
orf17.pep    AKYIPAFGLQIFFILFLTAVAFKTLHTDPQTASRPLPGLPXLTAVSTLFGTMSSWVGIGG
             |||||||||||||||||||||||||||||||||||||||| |||||||||||||||||||
orf17a       AKYIPAFGLQIFFILFLTAVAFKTLHTDPQTASRPLPGLPGLTAVSTLFGTMSSWVGIGG
                  110       120       130       140       150       160

                  100       110       120       130       140       150
orf17.pep    GSLSVPFLIHCGFPAHKAIGTSSGLAWPIALSGAISYLLNGLNIAGLPEGSLGFLYLPAV
             ||||||||||||||||||||||||||||||||||||||||||||||||||||||||||||
orf17a       GSLSVPFLIHCGFPAHKAIGTSSGLAWPIALSGAISYLLNGLNIAGLPEGSLGFLYLPAV
                  170       180       190       200       210       220

                  160       170       180       190
orf17.pep    AVLSAATIAFAPLGVKTAHKLSSAKLKKSFGIMLLLIAGKMLYNLLX
             |||||||||||||||||||||||||||||||||||||||||||||||
orf17a       AVLSAATIAFAPLGVKTAHKLSSAKLKKSFGIMLLLIAGKMLYNLLX
                  230       240       250       260
```

The complete length ORF17a nucleotide sequence <SEQ ID 89> is:

```
  1 ATGTGGCATT GGGACATTAT CTTAATCCTG CTTGCCGTAG GCAGTGCGGC
 51 AGGTTTTATT GCCGGCCTGT TCGGCGTAGG CGGCGGCACG CTGATTGTCC
101 CTGTCGTTTT ATGGGTGCTT GATTTGCAGG GTTTGGCACA ACATCCTTAC
151 GCGCAACACC TCGCCGTCGG CACATCCTTC GCCGTCATGG TCTTCACCGC
201 CTTTTCCAGT ATGCTGGGGC AGCACAAAAA ACAGGCGGTC GACTGGAAAA
251 CCGTATTTAC GATGATGCCG GGTATGGTAT TCGGCGTATT CGCTGGCGCA
301 CTCTCCGCAA AATATATCCC AGCGTTCGGG CTTCAAATTT TCTTCATCCT
351 GTTTTTAACC GCCGTCGCAT TCAAAACACT GCATACCGAC CCTCAGACGG
401 CATCCCGCCC GCTGCCCGGA CTGCCCGGAC TGACTGCGGT TTCCACACTG
451 TTCGGCACAA TGTCGAGCTG GGTCGGCATA GGCGGCGGTT CACTTTCCGT
501 CCCCTTCTTA ATCCACTGCG GCTTCCCCGC CCATAAAGCC ATCGGCACAT
```

```
551  CATCCGGCCT TGCCTGGCCG ATTGCACTCT CCGGCGCAAT ATCGTATCTG
601  CTCAACGGCC TGAATATTGC AGGATTGCCC GAAGGGTCAC TGGGCTTCCT
651  TTACCTGCCC GCCGTCGCCG TCCTCAGCGC GGCAACCATT GCCTTTGCCC
701  CGCTCGGTGT CAAAACCGCC CACAAACTTT CTTCTGCCAA ACTCAAAAAA
751  TCCTTCGGCA TTATGTTGCT TTTGATTGCC GGAAAAATGC TGTACAACCT
801  GCTTTAA
```

This encodes a protein having amino acid sequence <SEQ ID 90>:

```
  1  MWHWDIILIL LAVGSAAGFI AGLFGVGGGT LIVPVVLWVL DLQGLAQHPY
 51  AQHLAVGTSF AVMVFTAFSS MLGQHKKQAV DWKTVFTMMP GMVFGVFAGA
101  LSAKYIPAFG LQIFFILFLT AVAFKTLHTD PQTASRPLPG LPGLTAVSTL
151  FGTMSSWVGI GGGSLSVPFL IHCGFPAHKA IGTSSGLAWP IALSGAISYL
201  LNGLNIAGLP EGSLGFLYLP AVAVLSAATI AFAPLGVKTA HKLSSAKLKK
251  SFGIMLLLIA GKMLYNLL*
```

ORF17a and ORF17-1 show 98.9% identity in 268 aa overlap:

```
                     10        20        30        40        50        60
orf17a.pep   MWHWDIILILLAVGSAAGFIAGLFGVGGGTLIVPVVLWVLDLQGLAQHPYAQHLAVGTSF
             ||||||||||||||||||||||||||||||||||||||||||||||||||||||||||||
orf17-1      MWHWDIILILLAVGSAAGFIAGLFGVGGGTLIVPVVLWVLDLQGLAQHPYAQHLAVGTSF
                     10        20        30        40        50        60

                     70        80        90       100       110       120
orf17a.pep   AVMVFTAFSSMLGQHKKQAVDWKTVFTMMPGMVFGVFAGALSAKYIPAFGLQIFFILFLT
             ||||||||||||||||||||||||||||||||:||||:||||||||||||||||||||||
orf17-1      AVMVFTAFSSMLGQHKKQAVDWKTVFTMMPGMIFGVFTGALSAKYIPAFGLQIFFILFLT
                     70        80        90       100       110       120

                    130       140       150       160       170       180
orf17a.pep   AVAFKTLHTDPQTASRPLPGLPGLTAVSTLFGTMSSWVGIGGGSLSVPFLIHCGFPAHKA
             ||||||||||||||||||||||||||||||||||||||||||||||||||||||||||||
orf17-1      AVAFKTLHTDPQTASRPLPGLPGLTAVSTLFGTMSSWVGIGGGSLSVPFLIHCGFPAHKA
                    130       140       150       160       170       180

                    190       200       210       220       230       240
orf17a.pep   IGTSSGLAWPIALSGAISYLLNGLNIAGLPEGSLGFLYLPAVAVLSAATIAFAPLGVKTA
             ||||||||||||||||||||||||||||||||||||||||||||||||||||||||||||
orf17-1      IGTSSGLAWPIALSGAISYLLNGLNIAGLPEGSLGFLYLPAVAVLSAATIAFAPLGVKTA
                    190       200       210       220       230       240

                    250       260      269
orf17a.pep   HKLSSAKLKKSFGIMLLLIAGKMLYNLLX
             |||||||||| ||||||||||||||||||
orf17-1      HKLSSAKLKKXFGIMLLLIAGKMLYNLLX
                    250       260
```

<u>Homology with a predicted ORF from *N.gonorrhoeae*</u>

ORF17 shows 93.9% identity over a 196aa overlap with a predicted ORF (ORF 17.ng) from *N. gonorrhoeae:*

```
orf17.pep                                                   GQHKKQAVNGKTVFTMMPGMIFGVFTGAFS   30
                                                            ||||||||: ||:|:||||||||||:||:|
orf17ng     QGLAQHPYAQHLAVGTSFAVMVFTAFSSMLGQHKKQAVDWKTIFAMMPGMIFGVFAGALS  102

orf17.pep   AKYIPAFGLQIFFILFLTAVAFKTLHTDPQTASRPLPGLPXLTAVSTLFGTMSSWVGIGG   90
            |||||||||||||||||||||||||||  ||||||||||| |||||||||:|||||||||
orf17ng     AKYIPAFGLQIFFILFLTAVAFKTLHTGRQTASRPLPGLPGLTAVSTLFGAMSSWVGIGG  162

orf17.pep   GSLSVPFLIHCGFPAHKAIGTSSGLAWPIALSGAISYLLNGLNIAGLPEGSLGFLYLPAV  150
            ||||||||||||||||||||||||||||||||||||||:|||||||||||||||||||||
orf17ng     GSLSVPFLIHCGFPAHKAIGTSSGLAWPIALSGAISYLVNGLNIAGLPEGSLGFLYLPAV  202

orf17.pep   AVLSAATIAFAPLGVKTAHKLSSAKLKKSFGIMLLLIAGKMLYNLL  196
            |||||||||||||||||||||||||||:||||||||||||||||||
orf17ng     AVLSAATIAFAPLGVKTAHKLSSAKLKESFGIMLLLIAGKMLYNLL  268
```

An ORF17ng nucleotide sequence <SEQ ID 91> is predicted to encode a protein having amino acid sequence <SEQ ID 92>:

```
  1  MWHWDIILIL LAVGSAAGFI AGLFGVGGGT LIVPVVLWVL DLQGLAQHPY
 51  AQHLAVGTSF AVMVFTAFSS MLGQHKKQAV DWKTIFAMMP GMIFGVFAGA
101  LSAKYIPAFG LQIFFILFLT AVAFKTLHTG RQTASRPLPG LPGLTAVSTL
151  FGAMSSWVGI GGGSLSVPFL IHCGFPAHKA IGTSSGLAWP IALSGAISYL
201  VNGLNIAGLP EGSLGFLYLP AVAVLSAATI AFAPLGVKTA HKLSSAKLKE
251  SFGIMLLLIA GKMLYNLL*
```

Further work revealed the complete gonococcal DNA sequence <SEQ ID 93>:

```
  1  ATGTGGCATT GGGACATTAT CTTAATCCTG CTTGCcgtag gcAGTGCGGC
 51  AGGTTTTATT GCCGGCCTGT Tcggtgtagg cggcgGTACG CTGATTGTCC
101  CTGTCGTTTT ATGGGTGCTT GATTTGCAGG GTTTGGCACA ACATCCTTAC
151  GCGCAACACC TCGCCGTCGG CAcaTccttc gcCGTCATGG TCTTCACCGC
201  CTTTTCCAGT ATGTTGGGGC AGCACAAAAA ACAGGCGGTC GACTGGAAAA
251  CCATATTTGC GATGATGCCG GGTATGATAT TCGGCGTATT CGCTGGCGCA
301  CTCTCCGCAA AATATATCCC CGCGTTCGGG CTTCAAATTT TCTTCATCCT
351  GTTTTTAACC GCCGTCGCAT TCAAAACACT GCATACCGGT CGTCAGACGG
401  CATCCCGCCC GCTGCCCGGG CTGCCCGGAC TGACTGCGGT TTCCACACTG
451  TTCGGCGCAA TGTCGAGCTG GGTCGGCATA GGCGGCGGTT CACTTTCCGT
501  CCCCTTCTTA ATCCACTGCG GCTTCCCCGC CCATAAAGCC ATCGGCACAT
551  CATCCGGCCT TGCCTGGCCG ATTGCACTCT CCGGCGCAAT ATCGTATCTG
601  GTCAACGGTC TGAATATTGC AGGATTGCCC GAAGGGTCGC TGGGCTTCCT
651  TTACCTGCCC GCCGTCGCCG TCCTCAGCGC GGCAACCATT GCCTTTGCCC
701  CGCTCGGTGT CAAAACCGCC CACAAACTTT CTTCTGCCAA ACTCAAAGAA
751  TCCTTCGGCA TTATGTTGCT TTTGATTGCC GGAAAAATGC TGTACAACCT
801  GCTTTAA
```

This corresponds to the amino acid sequence <SEQ ID 94; ORF17ng-1>:

```
  1  MWHWDIILIL LAVGSAAGFI AGLFGVGGGT LIVPVVLWVL DLQGLAQHPY
 51  AQHLAVGTSF AVMVFTAFSS MLGQHKKQAV DWKTIFAMMP GMIFGVFAGA
101  LSAKYIPAFG LQIFFILFLT AVAFKTLHTG RQTASRPLPG LPGLTAVSTL
151  FGAMSSWVGI GGGSLSVPFL IHCGFPAHKA IGTSSGLAWP IALSGAISYL
201  VNGLNIAGLP EGSLGFLYLP AVAVLSAATI AFAPLGVKTA HKLSSAKLKE
251  SFGIMLLLIA GKMLYNLL*
```

ORF17ng-1 and ORF17-1 show 96.6% identity in 268 aa overlap:

```
                        10        20        30        40        50        60
orf17-1.pep     MWHWDIILILLAVGSAAGFIAGLFGVGGGTLIVPVVLWVLDLQGLAQHPYAQHLAVGTSF
                ||||||||||||||||||||||||||||||||||||||||||||||||||||||||||||
orf17ng-1       MWHWDIILILLAVGSAAGFIAGLFGVGGGTLIVPVVLWVLDLQGLAQHPYAQHLAVGTSF
                        10        20        30        40        50        60

                        70        80        90       100       110       120
orf17-1.pep     AVMVFTAFSSMLGQHKKQAVDWKTVFTMMPGMIFGVFTGALSAKYIPAFGLQIFFILFLT
                ||||||||||||||||||||||||:|:||||||||||:||||||||||||||||||||||
orf17ng-1       AVMVFTAFSSMLGQHKKQAVDWKTIFAMMPGMIFGVFAGALSAKYIPAFGLQIFFILFLT
                        70        80        90       100       110       120

                       130       140       150       160       170       180
orf17-1.pep     AVAFKTLHTDPQTASRPLPGLPGLTAVSTLFGTMSSWVGIGGGSLSVPFLIHCGFPAHKA
                |||||||||  |||||||||||||||||||||:|||||||||||||||||||||||||||
orf17ng-1       AVAFKTLHTGRQTASRPLPGLPGLTAVSTLFGAMSSWVGIGGGSLSVPFLIHCGFPAHKA
                       130       140       150       160       170       180

                       190       200       210       220       230       240
orf17-1.pep     IGTSSGLAWPIALSGAISYLLNGLNIAGLPEGSLGFLYLPAVAVLSAATIAFAPLGVKTA
                ||||||||||||||||||||:|||||||||||||||||||||||||||||||||||||||
orf17ng-1       IGTSSGLAWPIALSGAISYLVNGLNIAGLPEGSLGFLYLPAVAVLSAATIAFAPLGVKTA
                       190       200       210       220       230       240

                       250       260      269
orf17-1.pep     HKLSSAKLKKXFGIMLLLIAGKMLYNLLX
                |||||||||: |||||||||||||||||
orf17ng-1       HKLSSAKLKESFGIMLLLIAGKMLYNLLX
                       250       260
```

In addition, ORF17ng-1 shows significant homology with a hypothetical *H.influenzae* protein:

```
sp|P44070|Y902_HAEIN HYPOTHETICAL PROTEIN HI0902 pir||G64015 hypothetical protein
HI0902 - Haemophilus influenzae (strain Rd KW20) gi|1573922 (U32772) H. influenzae
predicted coding region HI0902 [Haemophilus influenzae]Length = 264
 Score = 74 (34.9 bits), Expect = 1.6e-23, Sum P(2) = 1.6e-23
 Identities = 15/43 (34%), Positives = 23/43 (53%)

Query:    55 AVGTSFAVMVFTAFSSMLGQHKKQAVDWKTIFAMMPGMIFGVF 97
             A+GTSFA +V T   S    HK   + W+ +   + P ++  VF
Sbjct:    52 ALGTSFATIVITGIGSAQRHHKLGNIVWQAVRILAPVIMLSVF 94

 Score = 195 (91.9 bits), Expect = 1.6e-23, Sum P(2) = 1.6e-23
 Identities = 44/114 (38%), Positives = 65/114 (57%)

Query:   150 LFGAMSSWVGIGGGSLSVPFLIHCGFPAHKAIGTSSGLAWPIALSGAISYLVNGLNIAGL 209
             L G  SS  GIGGG   VPFL   G    +AIG+S+     + +SG  S++V+G     +
Sbjct:   148 LIGMASSAAGIGGGGFIVPFLTARGINIKQAIGSSAFCGMLLGISGMFSFIVSGWGNPLM 207

Query:   210 PEGSLGFLYLPAVAVLSAATIAFAPLGVKTAHKLSSAKLKESFGIMLLLIAGKM 263
             PE SLG++YLPAV  ++A +   + LG    KL  + LK+ F + L+++A  M
Sbjct:   208 PEYSLGYIYLPAVLGITATSFFTSKLGASATAKLPVSTLKKGFALFLIVVAINM 261
```

This analysis, including the homology with the hypothetical *H.influenzae* transmembrane protein, suggests that the proteins from *N.meningitidis* and *N.gonorrhoeae,* and their epitopes, could be useful antigens for vaccines or diagnostics, or for raising antibodies.

### Example 12

The following partial DNA sequence was identified in *N.meningitidis* <SEQ ID 95>:

```
  1  ..GGAAACGGAT GGCAGGCAGA CCCCGAACAT CCGCTGCTCG GGCTTTTTGC
 51    CGTCAGTAAT GTATCGATGA CGCTTGCTTT TGTCGGAATA TGTGCGTTGG
101    TGCATTATTG CTTTTCGGGA ACGGTTCAAG TGTTTGTGTT TGCGGCACTG
151    CTCAAACTTT ATGCGCTGAA GCCGGTTTAT TGGTTCGTGT TGCAGTTTGT
201    GCTGATGGCG GTTGCCTATG TCCACCGCTG CGGTATAGAC CGGCAGCCGC
251    CGTCAACGTT CGGCGGCTCG CAGCTGCGAC TCGGCGGGTT GACGGCAGCG
301    TTGATGCAGG TCTCGGTACT GGTGCTGCTG CTTTCAGAAA TTGGAAGATA
351    A
```

This corresponds to the amino acid sequence <SEQ ID 96; ORF18>:

```
  1  ..GNGWQADPEH PLLGLFAVSN VSMTLAFVGI CALVHYCFSG TVQVFVFAAL
 51    LKLYALKPVY WFVLQFVLMA VAYVHRCGID RQPPSTFGGS QLRLGGLTAA
101    LMQVSVLVLL LSEIGR*
```

Further work revealed the complete nucleotide sequence <SEQ ID 97>:

```
  1  ATGATTTTGC TGCATTTGGA TTTTTTGTCT GCCTTACTGT ATGCGGCGGT
 51  TTTTCTGTTT CTGATATTCC GCGCAGGAAT GTTGCAATGG TTTTGGGCGA
101  GTATTATGCT GTGGCTGGGC ATATCGGTTT TGGGGGCAAA GCTGATGCCC
151  GGCATATGGG GAATGACCCG CGCCGCGCCC TTGTTCATCC CCCATTTTTA
201  CCTGACTTTG GGCAGCATAT TTTTTTTCAT CGGGCATTGG AACCGGAAAA
251  CAGATGGAAA CGGATGGCAG GCAGACCCCG AACATCCGCT GCTCGGGCTT
301  TTTGCCGTCA GTAATGTATC GATGACGCTT GCTTTTGTCG GAATATGTGC
351  GTTGGTGCAT TATTGCTTTT CGGGAACGGT TCAAGTGTTT GTGTTTGCGG
401  CACTGCTCAA ACTTTATGCG CTGAAGCCGG TTTATTGGTT CGTGTTGCAG
451  TTTGTGCTGA TGGCGGTTGC CTATGTCCAC CGCTGCGGTA TAGACCGGCA
501  GCCGCCGTCA ACGTTCGGCG GCTCGCAGCT GCGACTCGGC GGGTTGACGG
551  CAGCGTTGAT GCAGGTCTCG GTACTGGTGC TGCTGCTTTC AGAAATTGGA
601  AGATAA
```

This corresponds to the amino acid sequence <SEQ ID 98; ORF18-1>:

```
  1  MILLHLDFLS ALLYAAVFLF LIFRAGMLQW FWASIMLWLG ISVLGAKLMP
 51  GIWGMTRAAP LFIPHFYLTL GSIFFFIGHW NRKTDGNGWQ ADPEHPLLGL
101  FAVSNVSMTL AFVGICALVH YCFSGTVQVF VFAALLKLYA LKPVYWFVLQ
151  FVLMAVAYVH RCGIDRQPPS TFGGSQLRLG GLTAALMQVS VLVLLLSEIG
201  R*
```

Computer analysis of this amino acid sequence gave the following results:

<u>Homology with a predicted ORF from *N.meningitidis* (strain A)</u>

ORF18 shows 98.3% identity over a 116aa overlap with an ORF (ORF18a) from strain A of *N. meningitidis:*

```
                                          10        20        30
orf18.pep                       GNGWQADPEHPLLGLFAVSNVSMTLAFVGI
                                ||||||||||||||||||||||||||||||
orf18a     TRAAPLFIPHFYLTLGSIFFFIGHWNRKTDGNGWQADPEHPLLGLFAVSNVSMTLAFVGI
             60        70        80        90       100       110

              40        50        60        70        80        90
orf18.pep  CALVHYCFSGTVQVFVFAALLKLYALKPVYWFVLQFVLMAVAYVHRCGIDRQPPSTFGGS
           |||||||| |||||||||||||||||||||||||||||||||||||||||||||||||||
orf18a     CALVHYCFSXTVQVFVFAALLKLYALKPVYWFVLQFVLMAVAYVHRCGIDRQPPSTFGGS
             120       130       140       150       160       170

              100       110
orf18.pep  QLRLGGLTAALMQVSVLVLLLSEIGRX
           ||||||||||||| |||||||||||||
orf18a     QLRLGGLTAALMQXSVLVLLLSEIGRX
             180       190       200
```

The complete length ORF18a nucleotide sequence <SEQ ID 99> is:

```
  1 ATGATTTTGC TGCATTTGGA TTTTTTGTCT GCCTTACTGT ATGCGGCGGT
 51 TTTTCTGTTT CTGATATTCC GCGCAGGAAT GTTGCAATGG TTTTGGGCGA
101 GTATTATGCT GTGGCTGGGC ATATCGGTTT TGGGGGCAAA GCTGATGCCC
151 GGCATATGGG GAATGACCCG CGCCCGCGCCC TTGTTCATCC CCCATTTTTA
201 CCTGACTTTG GGCAGCATAT TTTTTTTCAT CGGGCATTGG AACCGGAAAA
251 CGGATGGAAA CGGATGGCAG GCAGACCCCG AACATCCTCT GCTCGGGCTG
301 TTTGCCGTCA GTAATGTATC GATGACGCTT GCTTTTGTCG GAATATGTGC
351 GTTGGTGCAT TATTGCTTTT CGNGAACGGT TCAAGTGTTT GTGTTTGCGG
401 CACTGCTCAA ACTTTATGCG CTGAAGCCGG TTTATTGGTT CGTGTTGCAG
451 TTTGTGCTGA TGGCGGTTGC CTATGTCCAC CGCTGCGGTA TAGACCGGCA
501 GCCGCCGTCA ACGTTCGGCG GNTCGCAGCT GCGACTCGGC GGGTTGACGG
551 CAGCGTTGAT GCAGNTCTCG GTACTGGTGC TGCTGCTTTC AGAAATTGGA
601 AGATAA
```

This encodes a protein having amino acid sequence <SEQ ID 100>:

```
  1 MILLHLDFLS ALLYAAVFLF LIFRAGMLQW FWASIMLWLG ISVLGAKLMP
 51 GIWGMTRAAP LFIPHFYLTL GSIFFFIGHW NRKTDGNGWQ ADPEHPLLGL
101 FAVSNVSMTL AFVGICALVH YCFSXTVQVF VFAALLKLYA LKPVYWFVLQ
151 FVLMAVAYVH RCGIDRQPPS TFGGSQLRLG GLTAALMQXS VLVLLLSEIG
201 R*
```

ORF18a and ORF18-1 show 99.0% identity in 201 aa overlap:

```
                  10        20        30        40        50        60
orf18a.pep  MILLHLDFLSALLYAAVFLFLIFRAGMLQWFWASIMLWLGISVLGAKLMPGIWGMTRAAP
            ||||||||||||||||||||||||||||||||||||||||||||||||||||||||||||
orf18-1     MILLHLDFLSALLYAAVFLFLIFRAGMLQWFWASIMLWLGISVLGAKLMPGIWGMTRAAP
                  10        20        30        40        50        60

                  70        80        90       100       110       120
orf18a.pep  LFIPHFYLTLGSIFFFIGHWNRKTDGNGWQADPEHPLLGLFAVSNVSMTLAFVGICALVH
            ||||||||||||||||||||||||||||||||||||||||||||||||||||||||||||
orf18-1     LFIPHFYLTLGSIFFFIGHWNRKTDGNGWQADPEHPLLGLFAVSNVSMTLAFVGICALVH
                  70        80        90       100       110       120
```

```
                        130       140       150       160       170       180
orf18a.pep      YCFSXTVQVFVFAALLKLYALKPVYWFVLQFVLMAVAYVHRCGIDRQPPSTFGGSQLRLG
                |||| |||||||||||||||||||||||||||||||||||||||||||||||||||||||
orf18-1         YCFSGTVQVFVFAALLKLYALKPVYWFVLQFVLMAVAYVHRCGIDRQPPSTFGGSQLRLG
                        130       140       150       160       170       180

                        190       200
orf18a.pep      GLTAALMQXSVLVLLLSEIGRX
                |||||||| ||||||||||||
orf18-1         GLTAALMQVSVLVLLLSEIGRX
                        190       200
```

Homology with a predicted ORF from *N.gonorrhoeae*

ORF18 shows 93.1 % identity over a 116aa overlap with a predicted ORF (ORF18.ng) from *N. gonorrhoeae:*

```
orf18.pep                                     GNGWQADPEHPLLGLFAVSNVSMTLAFVGI   30
                                              ||||||||||||||||||||||||||||||
orf18ng     TRAAPLFIPHFYLTLGSIFFFIGYWNRKTDGNGWQADPEHPLLGLFAVSNVSMTLAFVGI  115

orf18.pep   CALVHYCFSGTVQVFVFAALLKLYALKPVYWFVLQFVLMAVAYVHRCGIDRQPPSTFGGS   90
            ||||||||||||||||||||||||||||||||||||||||||||||||||||||||||||
orf18ng     CALVHYCFSGTVQVFVFAALLKLYALKPVYWFVLQFVLMAVAYVHRCGIDRQPPSTFGGS  175

orf18.pep   QLRLGGLTAALMQVSVLVLLLSEIGR  116
            ||||| |:| ||||:| ::||:||||
orf18ng     QLRLGVLAAMLMQVAVTAMLLAEIGR  201
```

The complete length ORF18ng nucleotide sequence is <SEQ ID 101>:

```
  1  ATGATTTTGC TGCATTTGGA TTTTTTGTCT GCCTTACTGt aTGCGGcggt
 51  tttTctgTTT CTGATATTCC GCGCAGGAAT GTTGCAATGG TTTTGGGCGA
101  GTATTGCGTT GTGGCTCGGC ATCTCGGTTT TAGGGGTAAA GCTGATGCCG
151  GGGATGTGGG GAATGACCCG CGCCGCGCCT TTGTTCATCC CCCATTTTTA
201  CCTGACTTTG GGCAGCATAT TTTTTTTCAT CGGGTATTGG AACCGGAAAA
251  CAGATGGAAA CGGATGGCAG GCAGACCCCG AACATCCGCT GCTCGGGCTT
301  TTTGCCGTCA GTAATGTATC GATGACGCTT GCTTTTGTCG GAATATGTGC
351  GTTGGTGCAT TATTGCTTTT CGGGAACGGT TCAAGTGTTT GTGTTTGCGG
401  CATTGCTCAA ACTTTATGCG CTGAAGCCGG TTTATTGGTT CGTGTTGCAG
451  TTTGTATTGA TGGCGGttgC CTATGTCCAC CGCTGCGGTA TAGACCGGCA
501  GCCGCCGTCA ACGTTCGGCG GTTCGCAGCT GCGACTCGGC GTGTTGGCGG
551  CGATGTTGAT GCAGGTTGCG GTAACGGCGA TGCTGCTTGC CGAAATCGGC
601  AGATGA
```

This encodes a protein having amino acid sequence <SEQ ID 102>:

```
  1  MILLHLDFLS ALLYAAVFLF LIFRAGMLQW FWASIALWLG ISVLGVKLMP
 51  GMWGMTRAAP LFIPHFYLTL GSIFFFIGYW NRKTDGNGWQ ADPEHPLLGL
101  FAVSNVSMTL AFVGICALVH YCFSGTVQVF VFAALLKLYA LKPVYWFVLQ
151  FVLMAVAYVH RCGIDRQPPS TFGGSQLRLG VLAAMLMQVA VTAMLLAEIG
201  R*
```

This ORF18ng protein sequence shows 94.0% identity in 201 aa overlap with ORF18-1:

```
                      10        20        30        40        50        60
orf18-1.pep   MILLHLDFLSALLYAAVFLFLIFRAGMLQWFWASIMLWLGISVLGAKLMPGIWGMTRAAP
              ||||||||||||||||||||||||||||||||||| |||||||||:|||||:||||||||
orf18ng       MILLHLDFLSALLYAAVFLFLIFRAGMLQWFWASIALWLGISVLGVKLMPGMWGMTRAAP
                      10        20        30        40        50        60

                      70        80        90       100       110       120
orf18-1.pep   LFIPHFYLTLGSIFFFIGHWNRKTDGNGWQADPEHPLLGLFAVSNVSMTLAFVGICALVH
              ||||||||||||||||||:|||||||||||||||||||||||||||||||||||||||||
orf18ng       LFIPHFYLTLGSIFFFIGYWNRKTDGNGWQADPEHPLLGLFAVSNVSMTLAFVGICALVH
                      70        80        90       100       110       120

                     130       140       150       160       170       180
orf18-1.pep   YCFSGTVQVFVFAALLKLYALKPVYWFVLQFVLMAVAYVHRCGIDRQPPSTFGGSQLRLG
              ||||||||||||||||||||||||||||||||||||||||||||||||||||||||||||
```

```
orf18ng       YCFSGTVQVFVFAALLKLYALKPVYWFVLQFVLMAVAYVHRCGIDRQPPSTFGGSQLRLG
                     130       140       150       160       170       180

                     190       200
orf18-1.pep   GLTAALMQVSVLVLLLSEIGRX
               |:| ||||:| ::||:|||||
orf18ng       VLAAMLMQVAVTAMLLAEIGRX
                     190       200
```

Based on this analysis, including the presence of several putative transmembrane domains in the gonococcal protein, it is predicted that the proteins from *N.meningitidis* and *N.gonorrhoeae,* and their epitopes, could be useful antigens for vaccines or diagnostics, or for raising antibodies.

**Example 13**

The following partial DNA sequence was identified in *N.meningitidis* <SEQ ID 103>:

```
  1   ATGAAAACCC CACTCCTCAA GCCTCTGCTN ATTACCTCGC TTCCCGTTTT
 51   CGCCAGTGTT TTTACCGCCG CCTCCATCGT CTGGCAGCTA GGCGAACCCA
101   AGCTCGCCAT GCCCTTCGTA CTCGGCATCA TCGCCGGCGG CCTTGTCGAT
151   TTGGACAACC NCNTGACCGG ACGGCTNAAA AACATCATCA CCACCGTCGC
201   CCTGTTCACC CTCTCCTCGC TCACGGCACA AAGCACCCTC GGCACAGGGC
251   TGCCCTTCAT CCTCGCCATG ACCCTGATGA CTT.CG.CTT CACCATTTTA
301   GGCGCGGNCG ...
```

This corresponds to the amino acid sequence <SEQ ID 104; ORF19>:

```
  1   MKTPLLKPLL ITSLPVFASV FTAASIVWQL GEPKLAMPFV LGIIAGGLVD
 51   LDNXXTGRLK NIITTVALFT LSSLTAQSTL GTGLPFILAM TLMTXXFTIL
101   GAX...
```

Further work revealed the complete nucleotide sequence <SEQ ID 105>:

```
   1  ATGAAAACCC CACTCCTCAA GCCTCTGCTC ATTACCTCGC TTCCCGTTTT
  51  CGCCAGTGTT TTTACCGCCG CCTCCATCGT CTGGCAGCTA GGCGAACCCA
 101  AGCTCGCCAT GCCCTTCGTA CTCGGCATCA TCGCCGGCGG CCTTGTCGAT
 151  TTGGACAACC GCCTGACCGG ACGGCTGAAA AACATCATCA CCACCGTCGC
 201  CCTGTTCACC CTCTCCTCGC TCACGGCACA AAGCACCCTC GGCACAGGGC
 251  TGCCCTTCAT CCTCGCCATG ACCCTGATGA CCTTCGGCTT CACCATTTTA
 301  GGCGCGGTCG GGCTCAAATA CCGCACCTTC GCCTTCGGTG CACTCGCCGT
 351  CGCCACCTAC ACCACACTTA CCTACACCCC CGAAACCTAC TGGCTGACCA
 401  ACCCCTTCAT GATTTTATGC GGCACCGTAC TGTACAGCAC CGCCATCCTC
 451  CTGTTCCAAA TCGTCCTGCC CCACCGCCCC GTCCAAGAAA GCGTCGCCAA
 501  CGCCTACGAC GCACTCGGCG GCTACCTCGA AGCCAAAGCC GACTTCTTCG
 551  ACCCCGATGA GGCAGCCTGG ATAGGCAACC GCCACATCGA CCTCGCCATG
 601  AGCAACACCG GCGTCATCAC CGCCTTCAAC CAATGCCGTT CCGCCCTGTT
 651  TTACCGCCTT CGCGGCAAAC ACCGCCACCC GCGCACCGCC AAAATGCTGC
 701  GTTACTACTT TGCCGCCCAA GACATACACG AACGCATCAG CTCCGCCCAC
 751  GTCGATTATC AGGAAATGTC CGAAAAATTC AAAAACACCG ACATCATCTT
 801  CCGCATCCAC CGCCTGCTCG AAATGCAGGG ACAAGCCTGC CGCAACACCG
 851  CCCAAGCCCT GCGCGCAAGC AAAGACTACG TTTACAGCAA ACGCCTCGGC
 901  CGCGCCATCG AAGGCTGCCG CCAATCGCTG CGCCTCCTTT CAGACAGCAA
 951  CGACAGTCCC GACATCCGCC ACCTGCGCCG CCTTCTCGAC AACCTCGGCA
1001  GCGTCGACCA GCAGTTCCGC CAACTCCAGC ACAACGGCCT GCAGGCAGAA
1051  AACGACCGCA TGGGCGACAC CCGCATCGCC GCCCTCGAAA CCAGCAGCCT
1101  CAAAAACACC TGGCAGGCAA TCCGTCCGCA GCTAAACCTC GAATCAGGCG
1151  TATTCCGCCA TGCCGTCCGC CTGTCCCTCG TCGTTGCCGC CGCCTGCACC
1201  ATCGTCGAAG CCCTCAACCT CAACCTCGGC TACTGGATAC TACTGACCGC
1251  CCTTTTCGTC TGCCAACCCA ACTACACCGC CACCAAAAGC CGCGTCCGCC
1301  AGCGCATCGC CGGCACCGTA CTCGGCGTAA TCGTCGGCTC GCTCGTCCCC
1351  TACTTCACCC CGTCTGTCGA AACCAAACTC TGGATTGTCA TCGCCAGTAC
1401  CACCCTCTTT TTCATGACCC GCACCTACAA ATACAGTTTC TCCACCTTCT
1451  TCATTACCAT TCAAGCCCTG ACCAGCCTCT CCCTCGCAGG TTTGGACGTA
1501  TACGCCGCCA TGCCCGTACG CATCATCGAC ACCATTATCG GCGCATCCCT
1551  TGCCTGGGCG GCAGTCAGCT ACCTGTGGCC AGACTGGAAA TACCTCACGC
1601  TCGAACGCAC CGCCGCCCTT GCCGTATGCA GCAACGGTGC CTATCTCGAA
```

```
1651  AAAATCACCG AACGCCTCAA AAGCGGCGAA ACCGGCGACG ACGTCGAATA
1701  CCGCGCCACC CGCCGCCGCG CCCACGAACA CACCGCCGCC CTCAGCAGCA
1751  CCCTTTCCGA CATGAGCAGC GAACCCGCAA AATTCGCCGA CAGCCTGCAA
1801  CCCGGCTTTA CCCTGCTCAA AACCGGCTAC GCCCTGACCG GCTACATCTC
1851  CGCCCTCGGC GCATACCGCA GCGAAATGCA CGAAGAATGC AGCCCCGACT
1901  TTACCGCACA GTTCCACCTC GCCGCCGAAC ACACCGCCCA CATCTTCCAA
1951  CACCTGCCCG AAACCGAACC CGACGACTTT CAGACAGCAC TGGATACACT
2001  GCGCGGCGAA CTCGACACCC TCCGCACCCA CAGCAGCGGA ACACAAAGCC
2051  ACATCCTCCT CCAACAGCTC CAACTCATCG CCCGACAGCT CGAACCCTAC
2101  TACCGCGCCT ACCGCCAAAT TCCGCACAGG CAGCCCCAAA ATGCAGCCTG
2151  A
```

This corresponds to the amino acid sequence <SEQ ID 106; ORF19-1>:

```
  1 MKTPLLKPLL ITSLPVFASV FTAASIVWQL GEPKLAMPFV LGIIAGGLVD
 51 LDNRLTGRLK NIITTVALFT LSSLTAQSTL GTGLPFILAM TLMTFGFTIL
101 GAVGLKYRTF AFGALAVATY TTLTYTPETY WLTNPFMILC GTVLYSTAIL
151 LFQIVLPHRP VQESVANAYD ALGGYLEAKA DFFDPDEAAW IGNRHIDLAM
201 SNTGVITAFN QCRSALFYRL RGKHRHPRTA KMLRYYFAAQ DIHERISSAH
251 VDYQEMSEKF KNTDIIFRIH RLLEMQGQAC RNTAQALRAS KDYVYSKRLG
301 RAIEGCRQSL RLLSDSNDSP DIRHLRRLLD NLGSVDQQFR QLQHNGLQAE
351 NDRMGDTRIA ALETSSLKNT WQAIRPQLNL ESGVFRHAVR LSLVVAAACT
401 IVEALNLNLG YWILLTALFV CQPNYTATKS RVRQRIAGTV LGVIVGSLVP
451 YFTPSVETKL WIVIASTTLF FMTRTYKYSF STFFITIQAL TSLSLAGLDV
501 YAAMPVRIID TIIGASLAWA AVSYLWPDWK YLTLERTAAL AVCSNGAYLE
551 KITERLKSGE TGDDVEYRAT RRRAHEHTAA LSSTLSDMSS EPAKFADSLQ
601 PGFTLLKTGY ALTGYISALG AYRSEMHEEC SPDFTAQFHL AAEHTAHIFQ
651 HLPETEPDDF QTALDTLRGE LDTLRTHSSG TQSHILLQQL QLIARQLEPY
701 YRAYRQIPHR QPQNAA*
```

Computer analysis of this amino acid sequence gave the following results:

Homology with predicted transmenbrane protein YHFK of *H. influenzae* (accession number P44289)

ORF19 and YHFK proteins show 45% aa identity in 97 aa overlap:

```
orf19  6   LKPLLITSLPVFASVFTAASIVWQLGEPKLAMPFVLGIIAGGLVDLDNXXTGRLKNIITT 65
           L    +I+++PVF +V  AA  +W        +MP +LGIIAGGLVDLDN  TGRLKN+  T
YHFK   5   LNAKVISTIPVFIAVNIAAVGIWFFDISSQSMPLILGIIAGGLVDLDNRLTGRLKNVFFT 64

orf19  66  VALFTLSSLTAQSTLGTGLPFILAMTLMTXXFTILGA 102
           +  F++SS   Q  +G  + +I+ MT++T  FT++GA
YHFK   65  LIAFSISSFIVQLHIGKPIQYIVLMTVLTFIFTMIGA 101
```

Homology with a predicted ORF from *N.meningitidis* (strain A)

ORF19 shows 92.2% identity over a 102aa overlap with an ORF (ORF19a) from strain A of *N. meningitidis:*

```
                      10     .  20        30        40        50        60
orf19.pep   MKTPLLKPLLITSLPVFASVFTAASIVWQLGEPKLAMPFVLGIIAGGLVDLDNXXTGRLK
            |||| |||||||||||||||||||||||||||||||||||||||||||||||||  |||||
orf19a      MKTPPLKPLLITSLPVFASVFTAASIVWQLGEPKLAMPFVLGIIAGGLVDLDNRLTGRLK
                    10        20        30        40        50        60

                    70        80        90       100
orf19.pep   NIITTVALFTLSSLTAQSTLGTGLPFILAMTLMTXXFTILGAX
            |||:||||||||||:|||||||||||||||||||  |||:||
orf19a      NIIATVALFTLSSLVAQSTLGTGLPFILAMTLMTFGFTIMGAVGLKYRTFAFGALAVATY
                    70        80        90       100       110       120

orf19a      TTLTYTPETYWLTNPFMILCGTVLYSTAIILFQIILPHRPVQENVANAYEALGSYLEAKA
                   130       140       150       160       170       180
```

The complete length ORF19a nucleotide sequence <SEQ ID 107> is:

```
  1 ATGAAAACCC CACCCCTCAA GCCTCTGCTC ATTACCTCGC TTCCCGTTTT
 51 CGCCAGTGTC TTTACCGCCG CCTCCATCGT CTGGCAGCTG GGCGAACCCA
101 AGCTCGCCAT GCCCTTCGTA CTCGGCATCA TCGCTGGCGG CCTGGTCGAT
```

```
 151  TTGGACAACC GCCTGACCGG ACGGCTGAAA AACATCATCG CCACCGTCGC
 201  CCTGTTCACC CTCTCCTCAC TTGTCGCGCA AAGCACCCTC GGCACAGGTT
 251  TGCCATTCAT CCTCGCCATG ACCCTGATGA CTTTCGGCTT TACCATCATG
 301  GGCGCGGTCG GGCTGAAATA CCGCACCTTC GCCTTCGGCG CACTCGCCGT
 351  CGCCACCTAC ACCACACTTA CCTACACCCC CGAAACCTAC TGGCTGACCA
 401  ACCCCTTTAT GATTCTGTGC GGAACCGTAC TGTACAGCAC CGCCATCATC
 451  CTGTTCCAAA TCATCCTGCC CCACCGCCCC GTTCAAGAAA ACGTCGCCAA
 501  CGCCTACGAA GCACTCGGCA GCTACCTCGA AGCCAAAGCC GACTTTTTCG
 551  ATCCCGACGA AGCCGAATGG ATAGGCAACC GCCACATCGA CCTCGCCATG
 601  AGCAACACCG GCGTCATCAC CGCCTTCAAC CAATGCCGTT CCGCCCTGTT
 651  TTACCGCCTT CGCGGCAAAC ACCGCCACCC GCGCACCGCC AAAATGCTGC
 701  GCTACTACTT CGCCGCCCAA GACATACACG AACGCATCAG CTCCGCCCAC
 751  GTCGACTACC AAGAGATGTC CGAAAAATTC AAAAACACCG ACATCATCTT
 801  CCGCATCCAC CGCCTGCTCG AAATGCAGGG ACAAGCCTGC CGCAACACCG
 851  CCCAAGCCCT GCGCGCAAGC AAAGACTACG TTTACAGCAA ACGCCTCGGC
 901  CGCGCCATCG AAGGCTGCCG CCAATCGCTG CGCCTCCTTT CAGACAGCAA
 951  CGACAATCCC GACATCCGCC ACCTGCGCCG CCTTCTCGAC AACCTCGGCA
1001  GCGTCGACCA GCAGTTCCGC CAACTCCAGC ACAACGGCCT GCAGGCAGAA
1051  AACGACCGCA TGGGCGACAC CCGCATCGCC GCCCTCGAAA CCGGCAGCCT
1101  CAAAAACACC TGGCAGGCAA TCCGTCCGCA GCTAAACCTC GAATCAGGCG
1151  TATTCCGCCA TGCCGTCCGC CTGTCCCTTG TCGTTGCCGC CGCCTGCACC
1201  ATCGTCGAAG CCCTCAACCT CAACCTCGGC TACTGGATAC TACTGACCGC
1251  CCTTTTCGTC TGCCAACCCA ACTACACCGC CACCAAAAGC CGCGTCCGCC
1301  AGCGCATCGC CGGCACCGTA CTCGGCGTAA TCGTCGGCTC GCTCGTCCCC
1351  TACTTTACCC CCTCCGTCGA AACCAAACTC TGGATCGTCA TCGCCAGTAC
1401  CACCCTCTTT TTCATGACCC GCACCTACAA ATACAGCTTC TCGACATTTT
1451  TCATCACCAT TCAAGCCCTG ACCAGCCTCT CCCTCGCAGG GTTGGACGTA
1501  TACGCCGCCA TGCCCGTACG CATCATCGAC ACCATTATCG GCGCATCCCT
1551  TGCCTGGGCG GCAGTCAGCT ACCTGTGGCC AGACTGGAAA TACCTCACGC
1601  TCGAACGCAC CGCCGCCCTT GCCGTATGCA GCAACGGCGC CTATCTCGAA
1651  AAAATCACCG AACGCCTCAA AAGCGGCGAA ACCGGCGACG ACGTCGAATA
1701  CCGCGCCACC CGCCGCCGCG CCCACGAACA CACCGCCGCC CTCAGCAGCA
1751  CCCTTTCCGA CATGAGCAGC GAACCCGCAA AATTCGCCGA CAGCCTGCAA
1801  CCCGGCTTTA CCCTGCTCAA AACCGGCTAC GCCCTGACCG GCTACATCTC
1851  CGCCCTCGGC GCATACCGCA GCGAAATGCA CGAAGAATGC AGCCCCGACT
1901  TTACCGCACA GTTCCACCTC GCCGCCGAAC ACACCGCCCA CATCTTCCAA
1951  CACCTGCCCG AAACCGAACC CGACGACTTT CAGACAGCAC TGGATACACT
2001  GCGCGGCGAA CTCGACACCC TCCGCACCCA CAGCAGCGGA ACACAAAGCC
2051  ACATCCTCCT CCAACAGCTC CAACTCATCG CCCGGCAGCT CGAACCCTAC
2101  TACCGCGCCT ACCGACAAAT TCCGCACAGG CAGCCCCAAA ACGCAGCCTG
2151  A
```

This encodes a protein having amino acid sequence <SEQ ID 108>:

```
  1  MKTPPLKPLL ITSLPVFASV FTAASIVWQL GEPKLAMPFV LGIIAGGLVD
 51  LDNRLTGRLK NIIATVALFT LSSLVAQSTL GTGLPFILAM TLMTFGFTIM
101  GAVGLKYRTF AFGALAVATY TTLTYTPETY WLTNPFMILC GTVLYSTAII
151  LFQIILPHRP VQENVANAYE ALGSYLEAKA DFFDPDEAEW IGNRHIDLAM
201  SNTGVITAFN QCRSALFYRL RGKHRHPRTA KMLRYYFAAQ DIHERISSAH
251  VDYQEMSEKF KNTDIIFRIH RLLEMQGQAC RNTAQALRAS KDYVYSKRLG
301  RAIEGCRQSL RLLSDSNDNP DIRHLRRLLD NLGSVDQQFR QLQHNGLQAE
351  NDRMGDTRIA ALETGSLKNT WQAIRPQLNL ESGVFRHAVR LSLVVAAACT
401  IVEALNLNLG YWILLTALFV CQPNYTATKS RVRQRIAGTV LGVIVGSLVP
451  YFTPSVETKL WIVIASTTLF FMTRTYKYSF STFFITIQAL TSLSLAGLDV
501  YAAMPVRIID TIIGASLAWA AVSYLWPDWK YLTLERTAAL AVCSNGAYLE
551  KITERLKSGE TGDDVEYRAT RRRAHEHTAA LSSTLSDMSS EPAKFADSLQ
601  PGFTLLKTGY ALTGYISALG AYRSEMHEEC SPDFTAQFHL AAEHTAHIFQ
651  HLPETEPDDF QTALDTLRGE LDTLRTHSSG TQSHILLQQL QLIARQLEPY
701  YRAYRQIPHR QPQNAA*
```

ORF19a and ORF19-1 show 98.3% identity in 716 aa overlap:

```
                      10        20        30        40        50        60
orf19a.pep    MKTPPLKPLLITSLPVFASVFTAASIVWQLGEPKLAMPFVLGIIAGGLVDLDNRLTGRLK
              |||| |||||||||||||||||||||||||||||||||||||||||||||||||||||||
orf19-1       MKTPLLKPLLITSLPVFASVFTAASIVWQLGEPKLAMPFVLGIIAGGLVDLDNRLTGRLK
                      10        20        30        40        50        60

                      70        80        90       100       110       120
orf19a.pep    NIIATVALFTLSSLVAQSTLGTGLPFILAMTLMTFGFTIMGAVGLKYRTFAFGALAVATY
              |||:||||||||||:|||||||||||||||||||||||||:|||||||||||||||||||
orf19-1       NIITTVALFTLSSLTAQSTLGTGLPFILAMTLMTFGFTILGAVGLKYRTFAFGALAVATY
```

```
                          70        80        90       100       110       120

                         130       140       150       160       170       180
orf19a.pep    TTLTYTPETYWLTNPFMILCGTVLYSTAIIILFQIILPHRPVQENVANAYEALGSYLEAKA
              |||||||||||||||||||||||||||||:||||:||||||||:|||||:|||:||||||
orf19-1       TTLTYTPETYWLTNPFMILCGTVLYSTAILLFQIVLPHRPVQESVANAYDALGGYLEAKA
                         130       140       150       160       170       180

                         190       200       210       220       230       240
orf19a.pep    DFFDPDEAEWIGNRHIDLAMSNTGVITAFNQCRSALFYRLRGKHRHPRTAKMLRYYFAAQ
              |||||||| |||||||||||||||||||||||||||||||||||||||||||||||||||
orf19-1       DFFDPDEAAWIGNRHIDLAMSNTGVITAFNQCRSALFYRLRGKHRHPRTAKMLRYYFAAQ
                         190       200       210       220       230       240

                         250       260       270       280       290       300
orf19a.pep    DIHERISSAHVDYQEMSEKFKNTDIIFRIHRLLEMQGQACRNTAQALRASKDYVYSKRLG
              ||||||||||||||||||||||||||||||||||||||||||||||||||||||||||||
orf19-1       DIHERISSAHVDYQEMSEKFKNTDIIFRIHRLLEMQGQACRNTAQALRASKDYVYSKRLG
                         250       260       270       280       290       300

                         310       320       330       340       350       360
orf19a.pep    RAIEGCRQSLRLLSDSNDNPDIRHLRRLLDNLGSVDQQFRQLQHNGLQAENDRMGDTRIA
              ||||||||||||||||||:|||||||||||||||||||||||||||||||||||||||||
orf19-1       RAIEGCRQSLRLLSDSNDSPDIRHLRRLLDNLGSVDQQFRQLQHNGLQAENDRMGDTRIA
                         310       320       330       340       350       360

                         370       380       390       400       410       420
orf19a.pep    ALETGSLKNTWQAIRPQLNLESGVFRHAVRLSLVVAAACTIVEALNLNLGYWILLTALFV
              ||||:|||||||||||||||||||||||||||||||||||||||||||||||||||||||
orf19-1       ALETSSLKNTWQAIRPQLNLESGVFRHAVRLSLVVAAACTIVEALNLNLGYWILLTALFV
                         370       380       390       400       410       420

                         430       440       450       460       470       480
orf19a.pep    CQPNYTATKSRVRQRIAGTVLGVIVGSLVPYFTPSVETKLWIVIASTTLFFMTRTYKYSF
              ||||||||||||||||||||||||||||||||||||||||||||||||||||||||||||
orf19-1       CQPNYTATKSRVRQRIAGTVLGVIVGSLVPYFTPSVETKLWIVIASTTLFFMTRTYKYSF
                       430       440       450       460       470       480

                         490       500       510       520       530       540
orf19a.pep    STFFITIQALTSLSLAGLDVYAAMPVRIIDTIIGASLAWAAVSYLWPDWKYLTLERTAAL
              ||||||||||||||||||||||||||||||||||||||||||||||||||||||||||||
orf19-1       STFFITIQALTSLSLAGLDVYAAMPVRIIDTIIGASLAWAAVSYLWPDWKYLTLERTAAL
                         490       500       510       520       530       540

                         550       560       570       580       590       600
orf19a.pep    AVCSNGAYLEKITERLKSGETGDDVEYRATRRRAHEHTAALSSTLSDMSSEPAKFADSLQ
              ||||||||||||||||||||||||||||||||||||||||||||||||||||||||||||
orf19-1       AVCSNGAYLEKITERLKSGETGDDVEYRATRRRAHEHTAALSSTLSDMSSEPAKFADSLQ
                         550       560       570       580       590       600

                         610       620       630       640       650       660
orf19a.pep    PGFTLLKTGYALTGYISALGAYRSEMHEECSPDFTAQFHLAAEHTAHIFQHLPETEPDDF
              ||||||||||||||||||||||||||||||||||||||||||||||||||||||||||||
orf19-1       PGFTLLKTGYALTGYISALGAYRSEMHEECSPDFTAQFHLAAEHTAHIFQHLPETEPDDF
                         610       620       630       640       650       660

                         670       680       690       700       710
orf19a.pep    QTALDTLRGELDTLRTHSSGTQSHILLQQLQLIARQLEPYYRAYRQIPHRQPQNAAX
              |||||||||||||||||||||||||||||||||||||||||||||||||||||||||
orf19-1       QTALDTLRGELDTLRTHSSGTQSHILLQQLQLIARQLEPYYRAYRQIPHRQPQNAAX
                         670       680       690       700       710
```

<u>Homology with a predicted ORF from *N.gonorrhoeae*</u>

ORF19 shows 95.1 % identity over a 102aa overlap with a predicted ORF (ORF19.ng) from *N. gonorrhoeae:*

```
orf19.pep    MKTPLLKPLLITSLPVFASVFTAASIVWQLGEPKLAMPFVLGIIAGGLVDLDNXXTGRLK   60
             ||||||||||||||||||||||||||||||||||||||||||||||||||||||  |||||
orf19ng      MKTPLLKPLLITSLPVFASVFTAASIVWQLGEPKLAMPFVLGIIAGGLVDLDNRLTGRLK   60
```

```
orf19.pep    NIITTVALFTLSSLTAQSTLGTGLPFILAMTLMTXXFTILGAX                    103
             |||:||||||||||||||||||||||||||||||  ||||||
orf19ng      NIIATVALFTLSSLTAQSTLGTGLPFILAMTLMTFGFTILGAVGLKYRTFAFGALAVATY  120
```

An ORF19ng nucleotide sequence <SEQ ID 109> is predicted to encode a protein having amino acid sequence <SEQ ID 110>:

```
  1  MKTPLLKPLL ITSLPVFASV FTAASIVWQL GEPKLAMPFV LGIIAGGLVD
 51  LDNRLTGRLK NIIATVALFT LSSLTAQSTL GTGLPFILAM TLMTFGFTIL
101  GAVGLKYRTF AFGALAVATY TTLTYTPETY WLTNPFMILC GTVLYSTAII
151  LFQIILPHRP VQESVANAYE ALGGYLEAKA DFFDPDEAAW IGNRHIDLAM
201  SNTGVITAFN QCRSALFYRL RGKHRHPRTA KMLRYYFAAQ DIHERISSAH
251  VDYQEMSEKF KNTDIIFRIR RLLEMQGQAC RNTAQAIRSG KDYVYSKRLG
301  RAIEGCRQSL RLLSDGNDSP DIRHLSRLLD NLGSVDQQFR QLRHSDSPAE
351  NDRMGDTRIA ALETGSFKNT *
```

Further work revealed the complete nucleotide sequence <SEQ ID 111>:

1 ATGAAAACCC CACTCCTCAA GCCTCTGCTC ATTACCTCGC TTCCCGTTTT
51 CGCCAGTGTC TTTACCGCCG CCTCCATCGT CTGGCAGCTA GGCGAACCCA
101 AGCTCGCCAT GCCCTTCGTA CTCGGCATCA TCGCCGGCGG CCTGGTCGAT
151 TTGGACAACC GCCTGACCGG ACGGCTGAAA AACATCATCG CCACCGTCGC
201 CCTGTTTACC CTCTCCTCGC TCACGGCGCA AAGCACCCTC GGCACAGGGC
251 TGCCCTTCAT CCTCGCCATG ACCCTGATGA CCTTCGGCTT TACCATTTTA
301 GGCGCGGTCG GGCTGAAATA CCGCACCTTC GCCTTCGGCG CACTCGCCGT
351 CGCCACCTAC ACCACGCTTA CCTACACCCC CGAAACCTAC TGGCTGACCA
401 ACCCCTTCAT GATTTTATGC GGCACCGTAC TGTACAGCAC CGCCATCATC
451 CTGTTCCAAA TCATCCTGCC CCACCGCCCC GTCCAAGAAA GCGTCGCCAA
501 TGCCTACGAA GCACTCGGCG GCTACCTCGA AGCCAAAGCC GACTTCTTCG
551 ACCCCGATGA GGCAGCCTGG ATAGGCAACC GCCACATCGA CCTCGCCATG
601 AGCAACACCG GCGTCATCAC CGCCTTCAAC CAATGCCGTT CCGCCCTGTT
651 TTACCGTTTG CGCGGCAAAC ACCGCCACCC GCGCACCGCC AAAATGCTGC
701 GCTACTACTT CGCCGCCCAA GACATCCACG AACGCATCAG CTCCGCCCAC
751 GTCGACTACC AAGAGATGTC CGAAAAATTC AAAAACACCG ACATCATCTT
801 CCGCATCCGC CGCCTGCTCG AAATGCAGGG GCAGGCGTGC CGCAACACCG
851 CCCAAGCCAT CCGGTCGGGC AAAGACTAcg tTTACAGCAA ACGCCTCGGA
901 CGCGCCATcg aaggctgCCG CCAGTCGCtg cgcctCCTTt cagacggcaA
951 CGACAGTCCC GACATCCGCC ACCTGAGccg CCTTCTCGAC AACCTCGgca
1001 GCGTcgacca gcagtTCcgc caactCCGAC ACAgcgactC CCCCGCcgaa
1051 Aacgaccgca tgggcgacaC CCGCATCGCC GCCCtcgaaa ccggcagctT
1101 caaaaaCAcc tggcaggCAA TCCGTCCGCa gctgaaCCTC GAATCatgCG
1151 TATTCCGCCA TGCCGTCCGC CTGTCCCTCG TCGTTGCCGC CGCCTGCACC
1201 ATCGTCgaag cCCTCAACCT CAACCTCGGC TACTGGATAC TGCTGACCGC
1251 CCTTTTCGTC TGCCAACCCA ACTACACCGC CACCAAAAGC CGCGTGTACC
1301 AACGCATCGC CGGCACCGTA CTCGGCGTAA TCGTCGGCTC GCTCGTCCCC
1351 TACTTCACCC CCTCCGTCGA AACCAAACTC TGGATTGTCA TCGCCGGTAC
1401 CACCCTGTTC TTCATGACCC GCACCTACAA ATACAGTTTC TCCACCTTCT
1451 TCATCACCAT TCAGGCACTG ACCAGCCTCT CCCTCGCAGG TTTGGACGTA
1501 TACGCCGCCA TGCCCGTGCG CATCATcgaC ACCATTATCG GCGCATCCCT
1551 TGCCTGGGCG GCGGTCAGCT ACCTGTGGCC AGACTGGAAA TACCTCACGC
1601 TCGAACGCAC CGCCGCCCTT GCCGTATGCA GCAGCGGCAC ATACCTCCAA
1651 AAAATTGCCG AACGCCTCAA AACCGGCGAA ACCGGCGACG ACATAGAATA
1701 CCGCATCACC CGCCGCCGCG CCCACGAACA CACCGCCGCC CTCAGCAGCA
1751 CCCTTTCCGA CATGAGCAGC GAACCCGCAA AATTCGCCGA CAGCCTGCAA
1801 CCCGGCTTTA CCCTGCTCAA AACCGGCTAC GCCCTGACCG GCTACATCTC
1851 CGCCCTCGGC GCATACCGCA GCGAAATGCA CGAAGAATGC AGCCCCGACT
1901 TTACCGCACA GTTCCACCTT GCCGCCGAAC ACACCGCCCA CATCTTCCAA
1951 CACCTGCCCG ACATGGGACC CGACGACTTT CAGACGGCAT TGGATACACT
2001 GCGCGGCGAA CTCGGCACCC TCCGCACCCG CAGCAGCGGA ACACAAAGCC
2051 ACATCCTCCT CCAACAGCTC CAACTCATCG CccgGCAACT CGAACCCTAC
2101 TACCGCGCCT ACCGACAAAT TCCGCACAGG CAGCCCCAAA ACGCAGCCTG
2151 A

This corresponds to the amino acid sequence <SEQ ID 112; ORF19ng-1>:

1 MKTPLLKPLL ITSLPVFASV FTAASIVWQL GEPKLAMPFV LGIIAGGLVD
51 LDNRLTGRLK NIIATVALFT LSSLTAQSTL GTGLPFILAM TLMTFGFTIL
101 GAVGLKYRTF AFGALAVATY TTLTYTPETY WLTNPFMILC GTVLYSTAII
151 LFQIILPHRP VQESVANAYE ALGGYLEAKA DFFDPDEAAW IGNRHIDLAM

```
201  SNTGVITAFN QCRSALFYRL RGKHRHPRTA KMLRYYFAAQ DIHERISSAH
251  VDYQEMSEKF KNTDIIFRIR RLLEMQGQAC RNTAQAIRSG KDYVYSKRLG
301  RAIEGCRQSL RLLSDGNDSP DIRHLSRLLD NLGSVDQQFR QLRHSDSPAE
351  NDRMGDTRIA ALETGSFKNT WQAIRPQLNL ESCVFRHAVR LSLVVAAACT
401  IVEALNLNLG YWILLTALFV CQPNYTATKS RVYQRIAGTV LGVIVGSLVP
451  YFTPSVETKL WIVIAGTTLF FMTRTYKYSF STFFITIQAL TSLSLAGLDV
501  YAAMPVRIID TIIGASLAWA AVSYLWPDWK YLTLERTAAL AVCSSGTYLQ
551  KIAERLKTGE TGDDIEYRIT RRRAHEHTAA LSSTLSDMSS EPAKFADSLQ
601  PGFTLLKTGY ALTGYISALG AYRSEMHEEC SPDFTAQFHL AAEHTAHIFQ
651  HLPDMGPDDF QTALDTLRGE LGTLRTRSSG TQSHILLQQL QLIARQLEPY
701  YRAYRQIPHR QPQNAA*
```

ORF19ng-1 and ORF19-1 show 95.5% identity in 716 aa overlap:

```
                    10        20        30        40        50        60
orf19-1.pep  MKTPLLKPLLITSLPVFASVFTAASIVWQLGEPKLAMPFVLGIIAGGLVDLDNRLTGRLK
             ||||||||||||||||||||||||||||||||||||||||||||||||||||||||||||
orf19ng-1    MKTPLLKPLLITSLPVFASVFTAASIVWQLGEPKLAMPFVLGIIAGGLVDLDNRLTGRLK
                    10        20        30        40        50        60

                    70        80        90       100       110       120
orf19-1.pep  NIITTVALFTLSSLTAQSTLGTGLPFILAMTLMTFGFTILGAVGLKYRTFAFGALAVATY
             |||:||||||||||||||||||||||||||||||||||||||||||||||||||||||||
orf19ng-1    NIIATVALFTLSSLTAQSTLGTGLPFILAMTLMTFGFTILGAVGLKYRTFAFGALAVATY
                    70        80        90       100       110       120

                   130       140       150       160       170       180
orf19-1.pep  TTLTYTPETYWLTNPFMILCGTVLYSTAILLFQIVLPHRPVQESVANAYDALGGYLEAKA
             |||||||||||||||||||||||||||||:|||||:|||||||||||||:||||||||||
orf19ng-1    TTLTYTPETYWLTNPFMILCGTVLYSTAIILFQIILPHRPVQESVANAYEALGGYLEAKA
                   130       140       150       160       170       180

                   190       200       210       220       230       240
orf19-1.pep  DFFDPDEAAWIGNRHIDLAMSNTGVITAFNQCRSALFYRLRGKHRHPRTAKMLRYYFAAQ
             ||||||||||||||||||||||||||||||||||||||||||||||||||||||||||||
orf19ng-1    DFFDPDEAAWIGNRHIDLAMSNTGVITAFNQCRSALFYRLRGKHRHPRTAKMLRYYFAAQ
                   190       200       210       220       230       240

                   250       260       270       280       290       300
orf19-1.pep  DIHERISSAHVDYQEMSEKFKNTDIIFRIHRLLEMQGQACRNTAQALRASKDYVYSKRLG
             |||||||||||||||||||||||||||||:||||||||||||||||:|::||||||||||
orf19ng-1    DIHERISSAHVDYQEMSEKFKNTDIIFRIRRLLEMQGQACRNTAQAIRSGKDYVYSKRLG
                   250       260       270       280       290       300

                   310       320       330       340       350       360
orf19-1.pep  RAIEGCRQSLRLLSDSNDSPDIRHLRRLLDNLGSVDQQFRQLQHNGLQAENDRMGDTRIA
             |||||||||||||||:||||||||| ||||||||||||||||:|:   ||||||||||||
orf19ng-1    RAIEGCRQSLRLLSDGNDSPDIRHLSRLLDNLGSVDQQFRQLRHSDSPAENDRMGDTRIA
                   310       320       330       340       350       360

                   370       380       390       400       410       420
orf19-1.pep  ALETSSLKNTWQAIRPQLNLESGVFRHAVRLSLVVAAACTIVEALNLNLGYWILLTALFV
             ||||:|:|||||||||||||||| ||||||||||||||||||||||||||||||||||||
orf19ng-1    ALETGSFKNTWQAIRPQLNLESCVFRHAVRLSLVVAAACTIVEALNLNLGYWILLTALFV
                   370       380       390       400       410       420

                   430       440       450       460       470       480
orf19-1.pep  CQPNYTATKSRVRQRIAGTVLGVIVGSLVPYFTPSVETKLWIVIASTTLFFMTRTYKYSF
             |||||||||||| |||||||||||||||||||||||||||||||||:|||||||||||||
orf19ng-1    CQPNYTATKSRVYQRIAGTVLGVIVGSLVPYFTPSVETKLWIVIAGTTLFFMTRTYKYSF
                   430       440       450       460       470       480

                   490       500       510       520       530       540
orf19-1.pep  STFFITIQALTSLSLAGLDVYAAMPVRIIDTIIGASLAWAAVSYLWPDWKYLTLERTAAL
             ||||||||||||||||||||||||||||||||||||||||||||||||||||||||||||
orf19ng-1    STFFITIQALTSLSLAGLDVYAAMPVRIIDTIIGASLAWAAVSYLWPDWKYLTLERTAAL
                   490       500       510       520       530       540

                   550       560       570       580       590       600
orf19-1.pep  AVCSNGAYLEKITERLKSGETGDDVEYRATRRRAHEHTAALSSTLSDMSSEPAKFADSLQ
             ||||:|:||:||:||||:||||||:||| |||||||||||||||||||||||||||||||
orf19ng-1    AVCSSGTYLQKIAERLKTGETGDDIEYRITRRRAHEHTAALSSTLSDMSSEPAKFADSLQ
```

```
                          550       560       570       580       590       600

                          610       620       630       640       650       660
orf19-1.pep    PGFTLLKTGYALTGYISALGAYRSEMHEECSPDFTAQFHLAAEHTAHIFQHLPETEPDDF
               |||||||||||||||||||||||||||||||||||||||||||||||||||||:  ||||
orf19ng-1      PGFTLLKTGYALTGYISALGAYRSEMHEECSPDFTAQFHLAAEHTAHIFQHLPDMGPDDF
                          610       620       630       640       650       660

                          670       680       690       700       710
orf19-1.pep    QTALDTLRGELDTLRTHSSGTQSHILLQQLQLIARQLEPYYRAYRQIPHRQPQNAAX
               ||||||||||| ||||:||||||||||||||||||||||||||||||||||||||||
orf19ng-1      QTALDTLRGELGTLRTRSSGTQSHILLQQLQLIARQLEPYYRAYRQIPHRQPQNAAX
                          670       680       690       700       710
```

In addition, ORF19ng-1 shows significant homology to a hypothetical gonococcal protein previously entered in the databases:

```
sp|O33369|YOR2_NEIGO  HYPOTHETICAL  45.5  KD  PROTEIN  (ORF2)  gnl|PID|e1154438
(AJ002423) hypothetical protein [Neisseria gonorrh] Length = 417
 Score = 1512 (705.6 bits), Expect = 5.3e-203, P = 5.3e-203
 Identities = 301/326 (92%), Positives = 306/326 (93%)

Query:   307 RQSLRLLSDGNDSPDIRHLSRLLDNLGSVDQQFRQLRHSDSPAENDRMGDTRIAALETGS 366
             RQSLRLLSDGNDS DIRHLSRLLDNLGSVDQQFRQLRHSDSPAENDRMGDTRIAALETGS
Sbjct:     1 RQSLRLLSDGNDSXDIRHLSRLLDNLGSVDQQFRQLRHSDSPAENDRMGDTRIAALETGS 60

Query:   367 FKNTWQAIRPQLNLESCVFRHAVRLSLVVAAACTIVEALNLNLGYWILLTALFVCQPNYT 426
             FKNTWQAIRPQLNLES VFRHAVRLSLVVAAACTIVEALNLNLGYWILLT LFVCQPNYT
Sbjct:    61 FKNTWQAIRPQLNLESGVFRHAVRLSLVVAAACTIVEALNLNLGYWILLTRLFVCQPNYT 120

Query:   427 ATKSRVYQRIAGTVLGVIVGSLVPYFTPSVETKLWIVIAGTTLFFMTRTYKYSFSTFFIT 486
             ATKSRVYQRIAGTVLGVIVGSLVPYFTPSVETKLWIVIAGTTLFFMTRTYKYSFSTFFIT
Sbjct:   121 ATKSRVYQRIAGTVLGVIVGSLVPYFTPSVETKLWIVIAGTTLFFMTRTYKYSFSTFFIT 180

Query:   487 IQALTSLSLAGLDVYAAMPVRIIDTIIGASLAWAAVSYLWPDWKYLTLERTAALAVCSSG 546
             IQALTSLSLAGLDVYAAMPVRIIDTIIGASLAWAAVSYLWPDWKYLTLERTAALAVCSSG
Sbjct:   181 IQALTSLSLAGLDVYAAMPVRIIDTIIGASLAWAAVSYLWPDWKYLTLERTAALAVCSSG 240

Query:   547 TYLQKIAERLKTGETGDDIEYRITRRRAHEHTAALSSTLSDMSSEPAKFADSLQPGFTLL 606
             TYLQKIAERLKTGETGDDIEYRITRRRAHEHTAALSSTLSDMSSEPAKFAD+   P
Sbjct:   241 TYLQKIAERLKTGETGDDIEYRITRRRAHEHTAALSSTLSDMSSEPAKFADTCNPALPCS 300

Query:   607 KTGYALTGYISALGAYRSEMHEECSP 632
             K   ALTGYISALG   ++  +  +P
Sbjct:   301 KPATALTGYISALGHTAAKCTKNAAP 326
```

Based on this analysis, including the presence of several putative transmembrane domains in the gonococcal protein (the first of which is also seen in the meningococcal protein), and on homology with the YHFK protein, it is predicted that the proteins from *N.meningitidis* and *N.gonorrhoeae,* and their epitopes, could be useful antigens for vaccines or diagnostics, or for raising antibodies.

**Example 14**

The following DNA sequence, believed to be complete, was identified in *N.meningitidis* <SEQ ID 113>:

```
   1  ATGAATATGC TGGGAGCTTT GGCAAAAGTC GGCAGCCTGA CGATGGTGTC
  51  GCGCGTTTTG GGATTTGTGC GCGATACGGT CATTGCGCGG GCATTCGGCG
 101  CGGGTATGGC GACGGATGCG TTTTTTGTCG CGTTCAAACT GCCCAACCTG
 151  CTTCGCCGCG TGTTTGCGGA GGGGGCGTTT GCCCAAGCGT TTGTGCCGAT
 201  TTTGGCGGAA TACAAGGAAA CGCGTTCAAA AGAGGCGG.C GAAGCCTTTA
 251  TCCGCCATGT GGCGGGGATG CTGTCGTTTG TACTGGTTAT CGTTACCGCG
 301  CTGGGCATAC TTGCCGCGCC TTGGGTGATT TATGTTTCCG CACCCGAGTT
 351  TTGCCCAAGA TGCCGACAAA TTTCAGCTCT CCATCGATTT GCTGCGGATT
```

```
 401  ACGTTTCCTT ATATATTATT GATTTCCCTG TCTTCATTTG TCGGCTCGGT
 451  ACTCAATTCT TATCATAAGT TCGGCATTCC GGCGTTTACG CCAC.GTTTC
 501  TGAACGTGTC GTTTATCGTA TTCGCGCTGT TTTTCGTGCC GTATTTCGAT
 551  CCGCCCGTTA CCGCGCyGGC GTGGGCGGTC TTTGTCGGCG GCATTTTGCA
 601  ACTCGrmTTC CAACTGCCCT GGCTGGCGAA ACTGGGCTTT TTGAAACTGC
 651  CCAAACtGAG TTTCAAAGAT GCGGCGGTCA ACCGCGTGAT GAAACAGATG
 701  GCGCCTGCgA TTTTgGGCGT GAgCGTGGCG CAGGTTTCTT TGGTGATCAA
 751  CACGATTTTc GCGTCTTATC TGCAATCGGG CAGCGTTTCA TGGATGTATT
 801  ACGCCGACCG CATGATGGAG CTGCCCAGCG GCGTGCTGGG GGCGGCACTC
 851  GGTACGATTT TGCTGCCGAC TTTGTCCAAA CACTCGGCAA ACCaAGATAC
 901  GGaACAGTTT TCCGCCCTGC TCGACTGGGG TTTGCGCCTG TGCATGCtgc
 951  TGACGCTGCC GGCGgcGGTC GGACTGGCGG TGTTGTCGTT cCCgCtGGTG
1001  GCGACGCTGT TTATGTACCG CGwATTTACG CTGTTTGACG CGCAGATGAC
1051  GCAACACGCG CTGATTGCCT ATTCTTTCGG TTTAATCGGC TTAATCATGA
1101  TTAAAGTGTT GGCACCCGGC TTCTATGCGC GGCAAAACAT CAAwAmGCCC
1151  GTCAAAATCG CCATCTTCAC GCTCATCTGC mCGCAGTTGA TGAACCTTGs
1201  CTTTAyCGGC CCACTrrAAC rCasTCGGAC TTTCGCTTGC CATCGGTCTG
1251  GGCGCGTGTA TCAATGCCGG ATTGTTGTTT TACCTGTTGC GCAGACACGG
1301  TATTTACCAA CCTGG.CAAG GGTTGGGCAG CGTTCTT.AG CAAAAATGCT
1351  GcTCTCGCTC GCCGTGA
```

This corresponds to the amino acid sequence <SEQ ID 114; ORF20>:

```
   1  MNMLGALAKV GSLTMVSRVL GFVRDTVIAR AFGAGMATDA FFVAFKLPNL
  51  LRRVFAEGAF AQAFVPILAE YKETRSKEAX EAFIRHVAGM LSFVLVIVTA
 101  LGILAAPWVI YVSAPSFAQD ADKFQLSIDL LRITFPYILL ISLSSFVGSV
 151  LNSYHKFGIP AFTPXFLNVS FIVFALFFVP YFDPPVTAXA WAVFVGGILQ
 201  LXFQLPWLAK LGFLKLPKLS FKDAAVNRVM KQMAPAILGV SVAQVSLVIN
 251  TIFASYLQSG SVSWMYYADR MMELPSGVLG AALGTILLPT LSKHSANQDT
 301  EQFSALLDWG LRLCMLLTLP AAVGLAVLSF PLVATLFMYR XFTLFDAQMT
 351  QHALIAYSFG LIGLIMIKVL APGFYARQNI XXPVKIAIFT LICXQLMNLX
 401  FXGPLXXIGL SLAIGLGACI NAGLLFYLLR RHGIYQPXQG LGSVLXQKCC
 451  SRSP*
```

These sequences were elaborated, and the complete DNA sequence <SEQ ID 115> is:

```
   1  ATGAATATGC TGGGAGCTTT GGCAAAAGTC GGCAGCCTGA CGATGGTGTC
  51  GCGCGTTTTG GGATTTGTGC GCGATACGGT CATTGCGCGG GCATTCGGCG
 101  CGGGTATGGC GACGGATGCG TTTTTTGTCG CGTTCAAACT GCCCAACCTG
 151  CTTCGCCGCG TGTTTGCGGA GGGGGCGTTT GCCCAAGCGT TTGTGCCGAT
 201  TTTGGCGGAA TACAAGGAAA CGCGTTCAAA AGAGGCGGCG GAGGCTTTTA
 251  TCCGCCATGT GGCGGGGATG CTGTCGTTTG TACTGGTTAT CGTTACCGCG
 301  CTGGGCATAC TTGCCGCGCC TTGGGTGATT TATGTTTCCG CACCCGGTTT
 351  TGCCCAAGAT GCCGACAAAT TTCAGCTCTC CATCGATTTG CTGCGGATTA
 401  CGTTTCCTTA TATATTATTG ATTTCCCTGT CTTCATTTGT CGGCTCGGTA
 451  CTCAATTCTT ATCATAAGTT CGGCATTCCG GCGTTTACGC CCACGTTTCT
 501  GAACGTGTCG TTTATCGTAT TCGCGCTGTT TTTCGTGCCG TATTTCGATC
 551  CGCCCGTTAC CGCGCTGGCG TGGGCGGTCT TTGTCGGCGG CATTTTGCAA
 601  CTCGGCTTCC AACTGCCCTG GCTGGCGAAA CTGGGCTTTT TGAAACTGCC
 651  CAAACTGAGT TTCAAAGATG CGGCGGTCAA CCGCGTGATG AAACAGATGG
 701  CGCCTGCGAT TTTGGGCGTG AGCGTGGCGC AGGTTTCTTT GGTGATCAAC
 751  ACGATTTTCG CGTCTTATCT GCAATCGGGC AGCGTTTCAT GGATGTATTA
 801  CGCCGACCGC ATGATGGAGC TGCCCAGCGG CGTGCTGGGG GCGGCACTCG
 851  GTACGATTTT GCTGCCGACT TTGTCCAAAC ACTCGGCAAA CCAAGATACG
 901  GAACAGTTTT CCGCCCTGCT CGACTGGGGT TTGCGCCTGT GCATGCTGCT
 951  GACGCTGCCG GCGGCGGTCG GACTGGCGGT GTTGTCGTTC CCGCTGGTGG
1001  CGACGCTGTT TATGTACCGC GAATTTACGC TGTTTGACGC GCAGATGACG
1051  CAACACGCGC TGATTGCCTA TTCTTTCGGT TTAATCGGCT TAATCATGAT
1101  TAAAGTGTTG GCACCCGGCT TCTATGCGCG GCAAAACATC AAAACGCCCG
1151  TCAAAATCGC CATCTTCACG CTCATCTGCA CGCAGTTGAT GAACCTTGCC
1201  TTTATCGGCC CACTGAAACA CGTCGGACTT TCGCTTGCCA TCGGTCTGGG
1251  CGCGTGTATC AATGCCGGAT TGTTGTTTTA CCTGTTGCGC AGACACGGTA
1301  TTTACCAACC TGGCAAGGGT TGGGCAGCGT TCTTAGCAAA AATGCTGCTC
1351  TCGCTCGCCG TGATGTGCGG CGGACTGTGG GCAGCGCAGG CTTACCTGCC
1401  GTTTGAATGG GCGCACGCCG GCGGAATGCG GAAAGCGGGG CAGCTCTGCA
1451  TCCTGATTGC CGTCGGCGGC GGACTGTATT TCGCATCACT GGCGGCTTTG
1501  GGCTTCCGTC CGCGCCATTT CAAACGCGTG GAAAACTGA
```

This corresponds to the amino acid sequence <SEQ ID 116; ORF20-1>:

```
  1  MNMLGALAKV GSLTMVSRVL GFVRDTVIAR AFGAGMATDA FFVAFKLPNL
 51  LRRVFAEGAF AQAFVPILAE YKETRSKEAA EAFIRHVAGM LSFVLVIVTA
101  LGILAAPWVI YVSAPGFAQD ADKFQLSIDL LRITFPYILL ISLSSFVGSV
151  LNSYHKFGIP AFTPTFLNVS FIVFALFFVP YFDPPVTALA WAVFVGGILQ
201  LGFQLPWLAK LGFLKLPKLS FKDAAVNRVM KQMAPAILGV SVAQVSLVIN
251  TIFASYLQSG SVSWMYYADR MMELPSGVLG AALGTILLPT LSKHSANQDT
301  EQFSALLDWG LRLCMLLTLP AAVGLAVLSF PLVATLFMYR EFTLFDAQMT
351  QHALIAYSFG LIGLIMIKVL APGFYARQNI KTPVKIAIFT LICTQLMNLA
401  FIGPLKHVGL SLAIGLGACI NAGLLFYLLR RHGIYQPGKG WAAFLAKMLL
451  SLAVMCGGLW AAQAYLPFEW AHAGGMRKAG QLCILIAVGG GLYFASLAAL
501  GFRPRHFKRV EN*
```

Computer analysis of this amino acid sequence gave the following results:

Homology with the MviN virulence factor of *S. typhimurium* (accession number P37169)

ORF20 and MviN proteins show 63% aa identity in 440aa overlap:

```
Orf20   1   MNMLGALAKVGSLTMVSRVLGFVRDTVIARAFGAGMATDAFFVAFKLPNLLRRVFAEGAF 60
            MN+L +LA V S+TM SRVLGF RD ++AR FGAGMATDAFFVAFKLPNLLRR+FAEGAF
MviN    14  MNLLKSLAAVSSMTMFSRVLGFARDAIVARIFGAGMATDAFFVAFKLPNLLRRIFAEGAF 73

Orf20   61  AQAFVPILAEYKETRSKEAXEAFIRHVAGMLSFVLVIVTALGILAAPWVIYVSAPSFAQD 120
            +QAFVPILAEYK  + +EA   F+ +V+G+L+  L +VT  G+LAAPWVI V+AP FA
MviN    74  SQAFVPILAEYKSKQGEEATRIFVAYVSGLLTLALAVVTVAGMLAAPWVIMVTAPGFADT 133

Orf20   121 ADKFQLSIDLLRITFPYILLISLSSFVGSVLNSYHKFGIPAFTPXFLNVSFIVFALFFVP 180
            ADKF L+  LLRITFPYILLISL+S VG++LN++++F IPAF P FLN+S I FALF  P
MviN    134 ADKFALTTQLLRITFPYILLISLASLVGAILNTWNRFSIPAFAPTFLNISMIGFALFAAP 193

Orf20   181 YFDPPVTAXAWAVFVGGILQLXFQLPWLAKLGFLKLPKLSFKDAAVNRVMKQMAPAILGV 240
            YF+PPV A AWAV VGG+LQL +QLP+L K+G L LP+++F+D    RV+KQM PAILGV
MviN    194 YFNPPVLALAWAVTVGGVLQLVYQLPYLKKIGMLVLPRINFRDTGAMRVVKQMGPAILGV 253

Orf20   241 SVAQVSLVINTIFASYLQSGSVSWMYYADRMMELPSGVLGAALGTILLPTLSKHSANQDT 300
            SV+Q+SL+INTIFAS+L SGSVSWMYYADR+ME PSGVLG ALGTILLP+LSK  A+ +
MviN    254 SVSQISLIINTIFASFLASGSVSWMYYADRLMEFPSGVLGVALGTILLPSLSKSFASGNH 313

Orf20   301 EQFSALLDWGLRLCMLLTLPAAVGLAVLSFPLVATLFMYRXFTLFDAQMTQHALIAYSFG 360
            +++  L+DWGLRLC LL LP+AV L +L+ PL  +LF Y  FT FDA MTQ ALIAYS G
MviN    314 DEYCRLMDWGLRLCFLLALPSAVALGILAKPLTVSLFQYGKFTAFDAAMTQRALIAYSVG 373

Orf20   361 LIGLIMIKVLAPGFYARQNIXXPVKIAIFTLICXQLMNLXFXXXXXXXXXXXXXXXXXCI 420
            LIGLI++KVLAPGFY+RQ+I  PVKIAI TLI  QLMNL F                 C+
MviN    374 LIGLIVVKVLAPGFYSRQDIKTPVKIAIVTLIMTQLMNLAFIGPLKHAGLSLSIGLAACL 433

Orf20   421 NAGLLFYLLRRHGIYQPXQG 440
            NA LL++ LR+  I+ P  G
MviN    434 NASLLYWQLRKQNIFTPQPG 453
```

Homology with a predicted ORF from *N.meningitidis* (strain A)

ORF20 shows 93.5% identity over a 447aa overlap with an ORF (ORF20a) from strain A of *N. meningitidis:*

```
                    10        20        30        40        50        60
orf20.pep   MNMLGALAKVGSLTMVSRVLGFVRDTVIARAFGAGMATDAFFVAFKLPNLLRRVFAEGAF
            |||||||:||||||||||||||||||||||||||||||||||||||||||||||||||||
orf20a      MNMLGALVKVGSLTMVSRVLGFVRDTVIARAFGAGMATDAFFVAFKLPNLLRRVFAEGAF
                    10        20        30        40        50        60

                    70        80        90       100       110       120
orf20.pep   AQAFVPILAEYKETRSKEAXEAFIRHVAGMLSFVLVIVTALGILAAPWVIYVSAPSFAQD
            |||||||||||||||||||:|||||||||||||||||||||||||||||||||||:||:|
orf20a      AQAFVPILAEYKETRSKEATEAFIRHVAGMLSFVLVIVTALGILAAPWVIYVSAPGFAKD
                    70        80        90       100       110       120
```

```
                    130       140       150       160       170       180
orf20.pep   ADKFQLSIDLLRITFPYILLISLSSFVGSVLNSYHKFGIPAFTPXFLNVSFIVFALFFVP
            ||||||||||||||||||||||||||||||||||||:||||||:||||||||||||||
orf20a      ADKFQLSIDLLRITFPYILLISLSSFVGSVLNSYHKFSIPAFTPTFLNVSFIVFALFFVP
                    130       140       150       160       170       180

                    190       200       210       220       230       240
orf20.pep   YFDPPVTAXAWAVFVGGILQLXFQLPWLAKLGFLKLPKLSFKDAAVNRVMKQMAPAILGV
            ||||||| ||||||||||| ||||||||||||||||||||||||||||||||||||||||
orf20a      YFDPPVTALAWAVFVGGILQLGFQLPWLAKLGFLKLPKLSFKDAAVNRVMKQMAPAILGV
                    190       200       210       220       230       240

                    250       260       270       280       290       300
orf20.pep   SVAQVSLVINTIFASYLQSGSVSWMYYADRMMELPSGVLGAALGTILLPTLSKHSANQDT
            ||||:|||||||||||||||||||||||||||||||:|||||||||||||||||||||||
orf20a      SVAQISLVINTIFASYLQSGSVSWMYYADRMMELPGGVLGAALGTILLPTLSKHSANQDT
                    250       260       270       280       290       300

                    310       320       330       340       350       360
orf20.pep   EQFSALLDWGLRLCMLLTLPAAVGLAVLSFPLVATLFMYRXFTLFDAQMTQHALIAYSFG
            |||||||||||| ||||||||||:|||||||||||||||| |||||||||||||||||||
orf20a      EQFSALLDWGLRXCMLLTLPAAVGMAVLSFPLVATLFMYREFTLFDAQMTQHALIAYSFG
                    310       320       330       340       350       360

                    370       380       390       400       410       420
orf20.pep   LIGLIMIKVLAPGFYARQNIXXPVKIAIFTLICXQLMNLXFXGPLXXIGLSLAIGLGACI
            |||||||||||||||||||| :||||||||||||:||||| | |||  :|||||||||||
orf20a      LIGLIMIKVLAPGFYARQNIKTPVKIAIFTLICTQLMNLAFIGPLKHVGLSLAIGLGACI
                    370       380       390       400       410       420

                    430       440       450
orf20.pep   NAGLLFYLLRRHGIYQPXQGLGSVLXQKCCSRSPX
            ||||||||||||||||| :| :: | :
orf20a      NAGLLFYLLRRHGIYQPGKGWAAFLAKMLLSLAVMGGGLYAAQIWLPFDWAHAGGMQKAA
                    430       440       450       460       470       480
```

The complete length ORF20a nucleotide sequence <SEQ ID 117> is:

```
   1  ATGAATATGC TGGGAGCTTT GGTAAAAGTC GGCAGCCTGA CGATGGTGTC
  51  GCGCGTTTTG GGATTTGTGC GCGATACGGT CATTGCGCGC GCATTCGGCG
 101  CAGGCATGGC GACGGATGCG TTCTTTGTCG CGTTCAAACT GCCCAACCTG
 151  CTTCGCCGCG TGTTTGCGGA GGGGGCGTTT GCCCAAGCGT TTGTGCCGAT
 201  TTTGGCGGAA TATAAGGAAA CGCGTTCTAA AGAGGCGACG GAGGCTTTTA
 251  TCCGCCATGT GGCGGGGATG CTGTCGTTTG TACTGGTCAT CGTTACCGCG
 301  CTGGGCATAC TTGCCGCGCC TTGGGTGATT TATGTTTCCG CACCCGGTTT
 351  TGCCAAAGAT GCCGACAAAT TTCAGCTCTC TATCGATTTG CTGCGGATTA
 401  CGTTTCCTTA TATCTTATTG ATTTCACTTT CCTCTTTTGT CGGCTCGGTA
 451  CTCAATTCCT ATCATAAATT CAGCATTCCT GCGTTTACGC CCACGTTCCT
 501  GAACGTGTCG TTTATCGTAT TCGCGCTGTT TTTCGTGCCG TATTTCGATC
 551  CTCCCGTTAC CGCGCTGGCT TGGGCGGTTT TTGTCGGCGG CATTTTGCAA
 601  CTCGGCTTCC AACTGCCCTG GCTGGCGAAA CTGGGTTTTT TGAAACTGCC
 651  CAAACTGAGT TTCAAAGATG CGGCGGTCAA CCGCGTGATG AAACAGATGG
 701  CGCCTGCGAT TTTGGGCGTG AGCGTGGCGC AGATTTCTTT GGTGATCAAC
 751  ACGATTTTCG CGTCTTATCT GCAATCGGGC AGCGTTTCAT GGATGTATTA
 801  CGCCGACCGC ATGATGGAAC TGCCCGGCGG CGTGCTGGGG GCGGCACTCG
 851  GTACGATTTT GCTGCCGACT TTGTCCAAAC ACTCGGCAAA CCAAGATACG
 901  GAACAGTTTT CCGCCCTGCT CGACTGGGGT TTGCGCNTGT GCATGCTGCT
 951  GACGCTGCCG GCGGCGGTCG GAATGGCGGT GTTGTCGTTC CCGCTGGTGG
1001  CAACCTTGTT TATGTACCGA GAATTCACGC TGTTTGACGC GCAGATGACG
1051  CAACACGCGC TGATTGCCTA TTCTTTCGGT TTAATCGGTT TAATCATGAT
1101  TAAAGTGTTG GCGCCCGGCT TTTATGCGCG GCAAAACATC AAAACGCCCG
1151  TCAAAATCGC CATCTTCACG CTCATTTGCA CGCAGTTGAT GAACCTTGCC
1201  TTTATCGGCC CACTGAAACA CGTCGGACTT TCGCTTGCCA TCGGTCTGGG
1251  CGCGTGTATC AATGCCGGAT TGTTGTTTTA CCTGTTGCGC AGACACGGTA
1301  TTTACCAACC TGGCAAGGGT TGGGCAGCGT TCTTGGCAAA AATGCTGCTC
1351  TCGCTCGCCG TGATGGGAGG CGGCCTGTAT GCCGCCCAAA TCTGGCTGCC
1401  GTTCGACTGG GCACACGCCG GCGGAATGCA AAAGGCCGCC CGGCTCTTCA
1451  TCCTGATTGC CGTCGGCGGC GGACTGTATT TCGCATCACT GGCGGCTTTG
1501  GGCTTCCGTC CGCGCCATTT CAAACGCGTG GAAAGCTGA
```

This encodes a protein having amino acid sequence <SEQ ID 118>:

```
  1  MNMLGALVKV GSLTMVSRVL GFVRDTVIAR AFGAGMATDA FFVAFKLPNL
 51  LRRVFAEGAF AQAFVPILAE YKETRSKEAT EAFIRHVAGM LSFVLVIVTA
101  LGILAAPWVI YVSAPGFAKD ADKFQLSIDL LRITFPYILL ISLSSFVGSV
151  LNSYHKFSIP AFTPTFLNVS FIVFALFFVP YFDPPVTALA WAVFVGGILQ
201  LGFQLPWLAK LGFLKLPKLS FKDAAVNRVM KQMAPAILGV SVAQISLVIN
251  TIFASYLQSG SVSWMYYADR MMELPGGVLG AALGTILLPT LSKHSANQDT
301  EQFSALLDWG LRXCMLLTLP AAVGMAVLSF PLVATLFMYR EFTLFDAQMT
351  QHALIAYSFG LIGLIMIKVL APGFYARQNI KTPVKIAIFT LICTQLMNLA
401  FIGPLKHVGL SLAIGLGACI NAGLLFYLLR RHGIYQPGKG WAAFLAKMLL
451  SLAVMGGGLY AAQIWLPFDW AHAGGMQKAA RLFILIAVGG GLYFASLAAL
501  GFRPRHFKRV ES*
```

ORF20a and ORF20-1 show 96.5% identity in 512 aa overlap:

```
                     10        20        30        40        50        60
orf20a.pep   MNMLGALVKVGSLTMVSRVLGFVRDTVIARAFGAGMATDAFFVAFKLPNLLRRVFAEGAF
             |||||||:||||||||||||||||||||||||||||||||||||||||||||||||||||
orf20-1      MNMLGALAKVGSLTMVSRVLGFVRDTVIARAFGAGMATDAFFVAFKLPNLLRRVFAEGAF
                     10        20        30        40        50        60

                     70        80        90       100       110       120
orf20a.pep   AQAFVPILAEYKETRSKEATEAFIRHVAGMLSFVLVIVTALGILAAPWVIYVSAPGFAKD
             |||||||||||||||||||:||||||||||||||||||||||||||||||||||||||:|
orf20-1      AQAFVPILAEYKETRSKEAAEAFIRHVAGMLSFVLVIVTALGILAAPWVIYVSAPGFAQD
                     70        80        90       100       110       120

                    130       140       150       160       170       180
orf20a.pep   ADKFQLSIDLLRITFPYILLISLSSFVGSVLNSYHKFSIPAFTPTFLNVSFIVFALFFVP
             |||||||||||||||||||||||||||||||||||||:||||||||||||||||||||||
orf20-1      ADKFQLSIDLLRITFPYILLISLSSFVGSVLNSYHKFGIPAFTPTFLNVSFIVFALFFVP
                    130       140       150       160       170       180

                    190       200       210       220       230       240
orf20a.pep   YFDPPVTALAWAVFVGGILQLGFQLPWLAKLGFLKLPKLSFKDAAVNRVMKQMAPAILGV
             ||||||||||||||||||||||||||||||||||||||||||||||||||||||||||||
orf20-1      YFDPPVTALAWAVFVGGILQLGFQLPWLAKLGFLKLPKLSFKDAAVNRVMKQMAPAILGV
                    190       200       210       220       230       240

                    250       260       270       280       290       300
orf20a.pep   SVAQISLVINTIFASYLQSGSVSWMYYADRMMELPGGVLGAALGTILLPTLSKHSANQDT
             ||||:|||||||||||||||||||||||||||||||:|||||||||||||||||||||||
orf20-1      SVAQVSLVINTIFASYLQSGSVSWMYYADRMMELPSGVLGAALGTILLPTLSKHSANQDT
                    250       260       270       280       290       300

                    310       320       330       340       350       360
orf20a.pep   EQFSALLDWGLRXCMLLTLPAAVGMAVLSFPLVATLFMYREFTLFDAQMTQHALIAYSFG
             |||||||||||| |||||||||||:|||||||||||||||||||||||||||||||||||
orf20-1      EQFSALLDWGLRLCMLLTLPAAVGLAVLSFPLVATLFMYREFTLFDAQMTQHALIAYSFG
                    310       320       330       340       350       360

                    370       380       390       400       410       420
orf20a.pep   LIGLIMIKVLAPGFYARQNIKTPVKIAIFTLICTQLMNLAFIGPLKHVGLSLAIGLGACI
             ||||||||||||||||||||||||||||||||||||||||||||||||||||||||||||
orf20-1      LIGLIMIKVLAPGFYARQNIKTPVKIAIFTLICTQLMNLAFIGPLKHVGLSLAIGLGACI
                    370       380       390       400       410       420

                    430       440       450       460       470       480
orf20a.pep   NAGLLFYLLRRHGIYQPGKGWAAFLAKMLLSLAVMGGGLYAAQIWLPFDWAHAGGMQKAA
             ||||||||||||||||||||||||||||||||||| |||:||| :|||:||||||:||:
orf20-1      NAGLLFYLLRRHGIYQPGKGWAAFLAKMLLSLAVMCGGLWAAQAYLPFEWAHAGGMRKAG
                    430       440       450       460       470       480

                    490       500       510
orf20a.pep   RLFILIAVGGGLYFASLAALGFRPRHFKRVESX
             :| ||||||||||||||||||||||||||||:|
orf20-1      QLCILIAVGGGLYFASLAALGFRPRHFKRVENX
                    490       500       510
```

Homology with a predicted ORF from *N.gonorrhoeae*

ORF20 shows 92.1% identity over a 454aa overlap with a predicted ORF (ORF20ng) from *N. gonorrhoeae:*

```
orf20.pep   MNMLGALAKVGSLTMVSRVLGFVRDTVIARAFGAGMATDAFFVAFKLPNLLRRVFAEGAF   60
            ||||||||||||||||||||||||||||||||||||||||||||||||||||||||||||
orf20ng     MNMLGALAKVGSLTMVSRVLGFVRDTVIARAFGAGMATDAFFVAFKLPNLLRRVFAEGAF   60

orf20.pep   AQAFVPILAEYKETRSKEAXEAFIRHVAGMLSFVLVIVTALGILAAPWVIYVSAPSFAQD  120
            |||||||||||||||||||:|||||||||||||||::||||||||||||||||||:|::|
orf20ng     AQAFVPILAEYKETRSKEATEAFIRHVAGMLSFVLIVVTALGILAAPWVIYVSAPGFTKD  120

orf20.pep   ADKFQLSIDLLRITFPYILLISLSSFVGSVLNSYHKFGIPAFTPXFLNVSFIVFALFFVP  180
            ||||||||:||||||||||||||||||||:||||||||||||||:|||:|||||||||||
orf20ng     ADKFQLSISLLRITFPYILLISLSSFVGSILNSYHKFGIPAFTPTFLNISFIVFALFFVP  180

orf20.pep   YFDPPVTAXAWAVFVGGILQLXFQLPWLAKLGFLKLPKLSFKDAAVNRVMKQMAPAILGV  240
            |||||||| |||||||||||| |||||||||||||||||:||||||||||||||||||||
orf20ng     YFDPPVTALAWAVFVGGILQLGFQLPWLAKLGFLKLPKLNFKDAAVNRVMKQMAPAILGV  240

orf20.pep   SVAQVSLVINTIFASYLQSGSVSWMYYADRMMELPSGVLGAALGTILLPTLSKHSANQDT  300
            ||||:||||||||||||||||||||||||||||||:||||||||||||||||||||||||
orf20ng     SVAQISLVINTIFASYLQSGSVSWMYYADRMMELPGGVLGAALGTILLPTLSKHSANQDT  300

orf20.pep   EQFSALLDWGLRLCMLLTLPAAVGLAVLSFPLVATLFMYRXFTLFDAQMTQHALIAYSFG  360
            ||||||||||||||||||||||:||||||||||||||||| |||||||||||||||||||
orf20ng     EQFSALLDWGLRLCMLLTLPAAAGLAVLSFPLVATLFMYREFTLFDAQMTQHALIAYSFG  360

orf20.pep   LIGLIMIKVLAPGFYARQNIXXPVKIAIFTLICXQLMNLXFXGPLXXIGLSLAIGLGACI  420
            ||||||||||| |||||||| :|||||||||||:||||| | |||   ||||||||||||
orf20ng     LIGLIMIKVLASGFYARQNIKTPVKIAIFTLICTQLMNLAFIGPLKHAGLSLAIGLGACI  420

orf20.pep   NAGLLFYLLRRHGIYQPXQGLGSVLXQKCCSRSP  454
            ||||||:|:|:||||:| ||||:   :|||||||
orf20ng     NAGLLFFLFRKHGIYRPGQGLGQPSWRKCCSRSP  454
```

An ORF20ng nucleotide sequence <SEQ ID 119> was predicted to encode a protein having amino acid sequence <SEQ ID 120>:

```
  1  MNMLGALAKV GSLTMVSRVL GFVRDTVIAR AFGAGMATDA FFVAFKLPNL
 51  LRRVFAEGAF AQAFVPILAE YKETRSKEAT EAFIRHVAGM LSFVLIVVTA
101  LGILAAPWVI YVSAPGFTKD ADKFQLSISL LRITFPYILL ISLSSFVGSI
151  LNSYHKFGIP AFTPTFLNIS FIVFALFFVP YFDPPVTALA WAVFVGGILQ
201  LGFQLPWLAK LGFLKLPKLN FKDAAVNRVM KQMAPAILGV SVAQISLVIN
251  TIFASYLQSG SVSWMYYADR MMELPGGVLG AALGTILLPT LSKHSANQDT
301  EQFSALLDWG LRLCMLLTLP AAAGLAVLSF PLVATLFMYR EFTLFDAQMT
351  QHALIAYSFG LIGLIMIKVL ASGFYARQNI KTPVKIAIFT LICTQLMNLA
401  FIGPLKHAGL SLAIGLGACI NAGLLFFLFR KHGIYRPGQG LGQPSWRKCC
451  SRSP*
```

Further DNA sequence analysis revealed the following DNA sequence <SEQ ID 121>:

```
   1 ATGAATATGC TTGGAGCTTT GGCAAAAGTC GGCAGCCTGA CGATGGTGTC
  51 GCGCGTTTTG GGATTTGTGC GCGATACGGT CATTGCGCGG GCATTCGGCG
 101 CGGGTATGGC GACGGATGCG TTTTTTGTCG CGTTCAAACT GCCCAACCTG
 151 CTTCGCCGCG TGTTTGCGGA GGGGGCGTTT GCCCAAGCGT TTGTGCCGAT
 201 TTTGGCGGAA TATAAGGAAA CGCGTTCTAA AGAGGCGAcg gAGGCTTTTA
 251 TCCGCCACGt tgcgggAatg CTGTCGTTTG TGCTGATcgt cGttacCGCG
 301 CTGGGCATAC TTGCCGCgcc tTGGGTGATT TATGTTtccg CgcccGGCTT
 351 TACCAAAGAC GCGGACAAGT TCCAACTTTC CATCAGCCTG CTGCGGATTA
 401 CGTTTCCTTA TATATTATTG ATTTCTTTGT CTTCTTTTGT CGGCTCGATA
 451 CTCAATTCCT ACCATAAGTT CGGCATTCCC GCGTTTACGC CCACGTTTTT
 501 AAACATCTCT TTTATCGTAT TCGCACTGTT TTTCGTGCCG TATTTCGATC
 551 CGCCCGTTAC CGCGCTGGCG TGGGCGGTTT TTGTCGGCGG TATTTTGCAG
 601 CTCGGTTTCC AACTGCCGTG GCTGGCGAAA CTGGGCTTTT TGAAACTGCC
 651 CAAACTGAAT TTCAAAGATG CGGCGGTCAA CCGCGTCATG AAACAGATGG
 701 CGCCTGCGAT TTTGGGCGTG agcgTGGCGC AAATTTCTTT GgttATCAAC
```

```
 751 ACGATTTTCG CGTCTTATCT GCAATCGGGC AGCGTTTCAT GGATGTatta
 801 cgCCGACCGC ATGATGGAGc tgcgccGGGG CGTGCTGGGG GCTGCACTCG
 851 GTACAATTTT GCTGCCGACT TTGTCCAAAC ACTCGGCAAA CCAAGATACG
 901 GAACAGTTTT CCGCCCTGCT CGACTGGGGT TTGCGCCTGT GCATGCTGCT
 951 GACGCTGCCG GCGGCGGccg GACTGGCGGT ATTGTCGTTC CCGCTGGTGG
1001 CGACGCTGTT TATGTACCGA GAATTCACGC TGTTTGACGC ACAAATGACG
1051 CAACACGCGC TGATTGCCTA TTCTTTCGGT TTAATCGGTT TAATTATGAT
1101 TAAAGTGTTG GCATCCGGCT TTTATGCGCG GCAAAACATC AAAACGCCCG
1151 TCAAAATCGC CATCTTCACG CTCATCTGCA CGCAGTTGAT GAACCTCGCC
1201 TTTATCGGTC CGTTGAAACA CGCCGGGCTT TCGCTCGCCA TCGGCCTGGG
1251 CGCGTGCATC AACGCCGGAT TGTTGTTCTT CCTGTTGCGC AAACACGGTA
1301 TTTACCGGCC cggcaggggt tgggcggcgt TCTTGGCGAA AATGCTGCTC
1351 GCGCTCGCCG TGATGTGCGG CGGACTGTGG GCGGCGCAGG CTTGCCTGCC
1401 GTTCGAATGG GCGCACGCCG GCGGAATGCG GAAAGCGGGG CAGCTCTGCA
1451 TCCTGATTGC CGTCGGCGGC GGACTGTATT TCGCATCTCT GGCGGCTTTG
1501 GGCTTCCGTC CGCGCCATTT CAAACGCGTG GAAAGCTGA
```

This encodes the following amino acid sequence <SEQ ID 122; ORF20ng-1>:

```
   1 MNMLGALAKV GSLTMVSRVL GFVRDTVIAR AFGAGMATDA FFVAFKLPNL
  51 LRRVFAEGAF AQAFVPILAE YKETRSKEAT EAFIRHVAGM LSFVLIVVTA
 101 LGILAAPWVI YVSAPGFTKD ADKFQLSISL LRITFPYILL ISLSSFVGSI
 151 LNSYHKFGIP AFTPTFLNIS FIVFALFFVP YFDPPVTALA WAVFVGGILQ
 201 LGFQLPWLAK LGFLKLPKLN FKDAAVNRVM KQMAPAILGV SVAQISLVIN
 251 TIFASYLQSG SVSWMYYADR MMELRRGVLG AALGTILLPT LSKHSANQDT
 301 EQFSALLDWG LRLCMLLTLP AAAGLAVLSF PLVATLFMYR EFTLFDAQMT
 351 QHALIAYSFG LIGLIMIKVL ASGFYARQNI KTPVKIAIFT LICTQLMNLA
 401 FIGPLKHAGL SLAIGLGACI NAGLLFFLLR KHGIYRPGRG WAAFLAKMLL
 451 ALAVMCGGLW AAQACLPFEW AHAGGMRKAG QLCILIAVGG GLYFASLAAL
 501 GFRPRHFKRV ES*
```

ORF20ng-1 and ORF20-1 show 95.7% identity in 512 aa overlap:

```
                       10        20        30        40        50        60
orf20-1.pep    MNMLGALAKVGSLTMVSRVLGFVRDTVIARAFGAGMATDAFFVAFKLPNLLRRVFAEGAF
               ||||||||||||||||||||||||||||||||||||||||||||||||||||||||||||
orf20ng-1      MNMLGALAKVGSLTMVSRVLGFVRDTVIARAFGAGMATDAFFVAFKLPNLLRRVFAEGAF
                       10        20        30        40        50        60

                       70        80        90       100       110       120
orf20-1.pep    AQAFVPILAEYKETRSKEAAEAFIRHVAGMLSFVLVIVTALGILAAPWVIYVSAPGFAQD
               |||||||||||||||||||:|||||||||||||||::||||||||||||||||||||::|
orf20ng-1      AQAFVPILAEYKETRSKEATEAFIRHVAGMLSFVLIVVTALGILAAPWVIYVSAPGFTKD
                       70        80        90       100       110       120

                      130       140       150       160       170       180
orf20-1.pep    ADKFQLSIDLLRITFPYILLISLSSFVGSVLNSYHKFGIPAFTPTFLNVSFIVFALFFVP
               ||||||||:||||||||||||||||||||:||||||||||||||||||:|||||||||||
orf20ng-1      ADKFQLSISLLRITFPYILLISLSSFVGSILNSYHKFGIPAFTPTFLNISFIVFALFFVP
                      130       140       150       160       170       180

                      190       200       210       220       230       240
orf20-1.pep    YFDPPVTALAWAVFVGGILQLGFQLPWLAKLGFLKLPKLSFKDAAVNRVMKQMAPAILGV
               |||||||||||||||||||||||||||||||||||||||:||||||||||||||||||||
orf20ng-1      YFDPPVTALAWAVFVGGILQLGFQLPWLAKLGFLKLPKLNFKDAAVNRVMKQMAPAILGV
                      190       200       210       220       230       240

                      250       260       270       280       290       300
orf20-1.pep    SVAQVSLVINTIFASYLQSGSVSWMYYADRMMELPSGVLGAALGTILLPTLSKHSANQDT
               ||||:|||||||||||||||||||||||||||||  ||||||||||||||||||||||||
orf20ng-1      SVAQISLVINTIFASYLQSGSVSWMYYADRMMELRRGVLGAALGTILLPTLSKHSANQDT
                      250       260       270       280       290       300

                      310       320       330       340       350       360
orf20-1.pep    EQFSALLDWGLRLCMLLTLPAAVGLAVLSFPLVATLFMYREFTLFDAQMTQHALIAYSFG
               ||||||||||||||||||||||:|||||||||||||||||||||||||||||||||||||
orf20ng-1      EQFSALLDWGLRLCMLLTLPAAAGLAVLSFPLVATLFMYREFTLFDAQMTQHALIAYSFG
                      310       320       330       340       350       360

                      370       380       390       400       410       420
orf20-1.pep    LIGLIMIKVLAPGFYARQNIKTPVKIAIFTLICTQLMNLAFIGPLKHVGLSLAIGLGACI
               ||||||||||| |||||||||||||||||||||||||||||||||||:||||||||||||
orf20ng-1      LIGLIMIKVLASGFYARQNIKTPVKIAIFTLICTQLMNLAFIGPLKHAGLSLAIGLGACI
                      370       380       390       400       410       420

                      430       440       450       460       470       480
orf20-1.pep    NAGLLFYLLRRHGIYQPGKGWAAFLAKMLLSLAVMCGGLWAAQAYLPFEWAHAGGMRKAG
               ||||||:|||:||||:||:|||||||||||:||||||||||||| |||||||||||||||
orf20ng-1      NAGLLFFLLRKHGIYRPGRGWAAFLAKMLLALAVMCGGLWAAQACLPFEWAHAGGMRKAG
                      430       440       450       460       470       480

                      490       500       510
orf20-1.pep    QLCILIAVGGGLYFASLAALGFRPRHFKRVENX
               |||||||||||||||||||||||||||||||:|
orf20ng-1      QLCILIAVGGGLYFASLAALGFRPRHFKRVESX
                      490       500       510
```

In addition, ORF20ng-1 shows significant homology with a virulence factor of *S. typhimurium:*

```
sp|P37169|MVIN_SALTY VIRULENCE FACTOR MVIN pir||S40271 mviN protein - Salmonella
typhimurium gi|438252 (Z26133) mviB gene product [Salmonella typhimurium]
gnl|PID|d1005521 (D25292) ORF2 [Salmonella typhimurium] Length = 524
 Score = 1573 (750.1 bits), Expect = 1.1e-220, Sum P(2) = 1.1e-220
 Identities = 309/467 (66%), Positives = 368/467 (78%)

Query:     1 MNMLGALAKVGSLTMVSRVLGFVRDTVIARAFGAGMATDAFFVAFKLPNLLRRVFAEGAF 60
             MN+L +LA V S+TM SRVLGF RD ++AR FGAGMATDAFFVAFKLPNLLRR+FAEGAF
Sbjct:    14 MNLLKSLAAVSSMTMFSRVLGFARDAIVARIFGAGMATDAFFVAFKLPNLLRRIFAEGAF 73

Query:    61 AQAFVPILAEYKETRSKEATEAFIRHVAGMLSFVLIVVTALGILAAPWVIYVSAPGFTKD 120
             +QAFVPILAEYK  + +EAT  F+ +V+G+L+  L VVT  G+LAAPWVI V+APGF
Sbjct:    74 SQAFVPILAEYKSKQGEEATRIFVAYVSGLLTLALAVVTVAGMLAAPWVIMVTAPGFADT 133

Query:   121 ADKFQLSISLLRITFPYILLISLSSFVGSILNSYHKFGIPAFTPTFLNISFIVFALFFVP 180
             ADKF L+  LLRITFPYILLISL+S VG+ILN++++F IPAF PTFLNIS I FALF  P
Sbjct:   134 ADKFALTTQLLRITFPYILLISLASLVGAILNTWNRFSIPAFAPTFLNISMIGFALFAAP 193

Query:   181 YFDPPVTALAWAVFVGGILQLGFQLPWLAKLGFLKLPKLNFKDAAVNRVMKQMAPAILGV 240
             YF+PPV ALAWAV VGG+LQL +QLP+L K+G L LP++NF+D    RV+KQM PAILGV
Sbjct:   194 YFNPPVLALAWAVTVGGVLQLVYQLPYLKKIGMLVLPRINFRDTGAMRVVKQMGPAILGV 253

Query:   241 SVAQISLVINTIFASYLQSGSVSWMYYADRMMELRRGVLGAALGTILLPTLSKHSANQDT 300
             SV+QISL+INTIFAS+L SGSVSWMYYADR+ME  GVLG ALGTILLP+LSK  A+ +
Sbjct:   254 SVSQISLIINTIFASFLASGSVSWMYYADRLMEFPSGVLGVALGTILLPSLSKSFASGNH 313

Query:   301 EQFSALLDWGLRLCMLLTLPAAAGLAVLSFPLVATLFMYREFTLFDAQMTQHALIAYSFG 360
             +++  L+DWGLRLC LL LP+A  L +L+ PL  +LF Y +FT FDA MTQ ALIAYS G
Sbjct:   314 DEYCRLMDWGLRLCFLLALPSAVALGILAKPLTVSLFQYGKFTAFDAAMTQRALIAYSVG 373

Query:   361 LIGLIMIKVLASGFYARQNIKTPVKIAIFTLICTQLMNLAFIGPLKHAGLSLAIGLGACI 420
             LIGLI++KVLA GFY+RQ+IKTPVKIAI TLI TQLMNLAFIGPLKHAGLSL+IGL AC+
Sbjct:   374 LIGLIVVKVLAPGFYSRQDIKTPVKIAVTLIMTQLMNLAFIGPLKHAGLSLSIGLAACL 433

Query:   421 NAGLLFFLLRKHGIYRPGRGWXXXXXXXXXXXXXVMCGGLWAAQACLP 467
             NA LL++ LRK  I+ P  GW             VM   L+     +P
Sbjct:   434 NASLLYWQLRKQNIFTPQPGWMWFLMRLIISVLVMAAVLFGVLHIMP 480

 Score = 70 (33.4 bits), Expect = 1.1e-220, Sum P(2) = 1.1e-220
 Identities = 14/41 (34%), Positives = 23/41 (56%)

Query:   469 EWAHAGGMRKAGQLCILIAVGGGLYFASLAALGFRPRHFKR 509
             EW+     + +  +L  ++  G   YFA+LA LGF+ + F R
Sbjct:   481 EWSQGSMLWRLLRLMAVVIAGIAAYFAALAVLGFKVKEFVR 521
```

Based on this analysis, including the homology with a virulence factor from *S.typhimurium,* it is predicted that these proteins from *N.meningitidis* and *N.gonorrhoeae,* and their epitopes, could be useful antigens for vaccines or diagnostics, or for raising antibodies.

**Example 15**

The following partial DNA sequence was identified in *N.meningitidis* <SEQ ID 123>:

```
  1 atGATTAAAA TCAAAAAAGG TCTAAACCTG CCCATCGCGG GCAGACCGGA
 51 GCAAGCCGTT tACGACGGCC CGGCCaTTAC CGAAGtCGCG TTGCTTGGCG
101 AAGAATATGC CGGTATGCGC CCCTCGATGA AAGTCAAGGA AGGCGATGCC
151 GTcAAAAAAG GCCAAGTGCT GTTTGAAGAC AAAAAGAATC CGGGCGTGGT
201 GTTTACTGCG CCGGCTTCAG GcAAAATCGC CGCGATTCAC CGTGGCGAAA
251 AGCGCGTACT TCAGTCAGTC GTGATTGCCG TTGAArGCAA CGACGAAATC
301 GAGTTTGAAC GCTACGCACC TGAAGCGCTG GCAAACTTAA GCGGCGAAGA
351 AGTGCGCCGC AACCTGATCC AATCCGGTTT GTGGACTGCG CTGCGCACCC
401 GTCCGTTCAG CAAAATTCCT GCCGTCGATG CCGAGCCGTT CGCCATCTTC
451 GTCAATGCGA tGGACACCAA TCCG..
```

This corresponds to the amino acid sequence <SEQ ID 124; ORF22>:

```
  1  MIKIKKGLNL PIAGRPEQAV YDGPAITEVA LLGEEYAGMR PSMKVKEGDA
 51  VKKGQVLFED KKNPGVVFTA PASGKIAAIH RGEKRVLQSV VIAVEXNDEI
101  EFERYAPEAL ANLSGEEVRR NLIQSGLWTA LRTRPFSKIP AVDAEPFAIF
151  VNAMDTNP..
```

Further work revealed the complete nucleotide sequence <SEQ ID 125>:

```
   1  ATGATTAAAA TCAAAAAAGG TCTAAACCTG CCCATCGCGG GCAGACCGGA
  51  GCAAGCCGTT TACGACGGCC CGGCCATTAC CGAAGTCGCG TTGCTTGGCG
 101  AAGAATATGC CGGTATGCGC CCCTCGATGA AAGTCAAGGA AGGCGATGCC
 151  GTCAAAAAAG GCCAAGTGCT GTTTGAAGAC AAAAAGAATC CGGGCGTGGT
 201  GTTTACTGCG CCGGCTTCAG GCAAAATCGC CGCGATTCAC CGTGGCGAAA
 251  AGCGCGTACT TCAGTCAGTC GTGATTGCCG TTGAAGGCAA CGACGAAATC
 301  GAGTTTGAAC GCTACGCACC TGAAGCGCTG GCAAACTTAA GCGGCGAAGA
 351  AGTGCGCCGC AACCTGATCC AATCCGGTTT GTGGACTGCG CTGCGCACCC
 401  GTCCGTTCAG CAAAATTCCT GCCGTCGATG CCGAGCCGTT CGCCATCTTC
 451  GTCAATGCGA TGGACACCAA TCCGCTGGCT GCCGACCCTA CGGTCATTAT
 501  CAAAGAAGCC GCCGAGGATT TCAAACGCGG CCTGTTGGTA TTGAGCCGTT
 551  TGACCGAACG CAAAATCCAT GTTTGTAAGG CAGCTGGCGC AGACGTGCCG
 601  TCTGAAAATG CTGCCAACAT CGAAACACAT GAATTCGGCG GCCCGCATCC
 651  TGCCGGTTTG AGTGGCACGC ACATTCATTT CATCGAGCCG GTCGGCGCGA
 701  ATAAAACCGT GTGGACCATC AATTATCAAG ATGTAATTAC CATTGGCCGT
 751  TTGTTTGCAA CAGGCCGTCT GAACACCGAG CGCGTGATTG CCCTAGGTGG
 801  TTCTCAAGTC AACAAACCGC GCCTCTTGCG TACCGTTTTG GGTGCGAAAG
 851  TATCGCAAAT TACTGCGGGC GAATTGGTTG ACACAGACAA CCGCGTGATT
 901  TCCGGTTCGG TATTGAACGG CGCGATTACA CAAGGCGCGC ACGATTATTT
 951  GGGACGCTAC CACAATCAGA TTTCCGTTAT CGAAGAAGGC CGCAGCAAAG
1001  AGCTGTTCGG CTGGGTTGCG CCGCAGCCGG ACAAATACTC CATCACGCGT
1051  ACAACCCTCG GCCATTTCCT GAAAAACAAA CTCTTCAAGT TCAACACAGC
1101  CGTCAACGGC GGCGACCGCG CCATGGTGCC GATTGGTACT TACGAGCGCG
1151  TGATGCCCTT GGATATCCTG CCCACCCTGC TTTTGCGCGA TTTAATCGTC
1201  GGCGATACCG ACAGCGCGCA GGCATTGGGT TGCTTGGAAT TGGACGAAGA
1251  AGACCTCGCT TTGTGCAGCT TCGTCTGCCC GGGCAAATAC GAATACGGCC
1301  CGCTGTTGCG CAAAGTGCTG GAAACCATTG AGAAGGAAGG CTGA
```

This corresponds to the amino acid sequence <SEQ ID 126; ORF22-1>:

```
  1  MIKIKKGLNL PIAGRPEQAV YDGPAITEVA LLGEEYAGMR PSMKVKEGDA
 51  VKKGQVLFED KKNPGVVFTA PASGKIAAIH RGEKRVLQSV VIAVEGNDEI
101  EFERYAPEAL ANLSGEEVRR NLIQSGLWTA LRTRPFSKIP AVDAEPFAIF
151  VNAMDTNPLA ADPTVIIKEA AEDFKRGLLV LSRLTERKIH VCKAAGADVP
201  SENAANIETH EFGGPHPAGL SGTHIHFIEP VGANKTVWTI NYQDVITIGR
251  LFATGRLNTE RVIALGGSQV NKPRLLRTVL GAKVSQITAG ELVDTDNRVI
301  SGSVLNGAIT QGAHDYLGRY HNQISVIEEG RSKELFGWVA PQPDKYSITR
351  TTLGHFLKNK LFKFNTAVNG GDRAMVPIGT YERVMPLDIL PTLLLRDLIV
401  GDTDSAQALG CLELDEEDLA LCSFVCPGKY EYGPLLRKVL ETIEKEG*
```

Further work identified the corresponding gene in strain A of *N.meningitidis* <SEQ ID 127>:

```
  1  ATGATTAAAA TCAAAAAAGG TCTAAACCTG CCCATCGCGG GCAGACCGGA
 51  GCAAGTCATT TATGACGGGC CCGTCATTAC CGAAGTCGCG TTGCTTGGCG
101  AAGAATATGC CGGTATGCGC CCCTNGATGA AAGTCAAGGA AGGCGATGCC
151  GTCAAAAAAG GCCAAGTGCT GTTTGAAGAC AAAAAGNATC CGGGCGTGGT
```

```
 201  GTTTACCGCG CCNGTTTCAG GCAAAATCGC CGCCATCCAT CGCGGCGAAA
 251  AGCGCGTACT TCAGTCGGTC GTGATTGCCG TTGAAGGCAA CGACGAAATC
 301  GAGTTCGAAC GCTACGCGCC CGAAGCGTTG GCAAACTTAA GCGGCGANGA
 351  ANTNNGNNGC AATCTGATCC AATCCGGTTT GTGGACTGCG CTGCGTANCC
 401  GTCCGTTCAG CAAAATCCCT GCCGTCGATG CCGAGCCGTT CGCCATCTTC
 451  GTCAATGCGA TGGACACCAA TCCGCTNGCG GCAGACCCTG TGGTTGTGAT
 501  CAAAGAAGCC GNCGANGATT TCAGACGANG TNTGCTGGTA TTGAGCCGTT
 551  TGACCGAGCG TAAAATCCAT GTGTGTAAGG CAGCTGGCGC AGACGTGCCG
 601  TCTGAAAATG CTGCCAACAT CGAAACACAT GAATTCGGCG GCCCGCATCC
 651  GGCCGGTTTG AGTGGCACGC ACATTCATTT CATTGAGCCG GTCGGTGCAA
 701  ACAAAACCGT TTGGACCATC AATTATCAAG ATGTAATTGC CATCGGACGT
 751  TTGTTTGCAA CAGGCCGTCT GAACACCGAG CGCGTGATTG CTTTGGGTGG
 801  TTCTCAAGTC AACAAACCAC GCCTCTTGCG TACCGTTTTG GGTGCGAAAG
 851  TATCGCAAAT TACTGCGGGC GAATTGGTTG ACGCAGACAA CCGCGTGATT
 901  TCCGGTTCGG TATTGAACGG CGCGATTACA CAAGGCGCGC ACGATTATTT
 951  GGGACGCTAC CACAATCAGA TTTCCGTTAT CGAAGAAGGC CGCAGCAAAG
1001  AGCTGTTCGG CTGGGTTGCG CCGCAGCCGG ACAAATACTC CATCACGCGT
1051  ACGACCCTCG GCCATTTCCT GAAAAACAAA CTCTTCAAGT TCACGACAGC
1101  CGTCAACGGT GGCGACCGCG CCATGGTGCC GATTGGTACT TACGAGCGCG
1151  TAATGCCGCT AGACATCCTG CCTACCCTGC TTTTGCGCGA TTTAATCGTC
1201  GGCGATACCG ACAGCGCGCA AGCATTGGGT TGCTTGGAAT TGGACGAAGA
1251  AGACCTCGCT TTGTGCAGCT TCGTCTGCCC GGGCAAATAC GAATANGGCC
1301  CGCTGTTGCG TAAGGTGCTG GAAACCNTTG AGAAGGAAGG CTGA
```

This encodes a protein having amino acid sequence <SEQ ID 128; ORF22a>:

```
  1  MIKIKKGLNL PIAGRPEQVI YDGPVITEVA LLGEEYAGMR PXMKVKEGDA
 51  VKKGQVLFED KKXPGVVFTA PVSGKIAAIH RGEKRVLQSV VIAVEGNDEI
101  EFERYAPEAL ANLSGXEXXX NLIQSGLWTA LRXRPFSKIP AVDAEPFAIF
151  VNAMDTNPLA ADPVVVIKEA XXDFRRXXLV LSRLTERKIH VCKAAGADVP
201  SENAANIETH EFGGPHPAGL SGTHIHFIEP VGANKTVWTI NYQDVIAIGR
251  LFATGRLNTE RVIALGGSQV NKPRLLRTVL GAKVSQITAG ELVDADNRVI
301  SGSVLNGAIT QGAHDYLGRY HNQISVIEEG RSKELFGWVA PQPDKYSITR
351  TTLGHFLKNK LFKFTTAVNG GDRAMVPIGT YERVMPLDIL PTLLLRDLIV
401  GDTDSAQALG CLELDEEDLA LCSFVCPGKY EXGPLLRKVL ETXEKEG*
```

The originally-identified partial strain B sequence (ORF22) shows 94.2% identity over a 158aa overlap with ORF22a:

```
                    10        20        30        40        50        60
orf22.pep   MIKIKKGLNLPIAGRPEQAVYDGPAITEVALLGEEYAGMRPSMKVKEGDAVKKGQVLFED
            ||||||||||||||||||::||||:|||||||||||||||| ||||||||||||||||||
orf22a      MIKIKKGLNLPIAGRPEQVIYDGPVITEVALLGEEYAGMRPXMKVKEGDAVKKGQVLFED
                    10        20        30        40        50        60

                    70        80        90       100       110       120
orf22.pep   KKNPGVVFTAPASGKIAAIHRGEKRVLQSVVIAVEXNDEIEFERYAPEALANLSGEEVRR
            || ||||||||:||||||||||||||||||||||| ||||||||||||||||||| |   
orf22a      KKXPGVVFTAPVSGKIAAIHRGEKRVLQSVVIAVEGNDEIEFERYAPEALANLSGXEXXX
                    70        80        90       100       110       120

                   130       140       150
orf22.pep   NLIQSGLWTALRTRPFSKIPAVDAEPFAIFVNAMDTNP
            ||||||||||||:|||||||||||||||||||||||||
orf22a      NLIQSGLWTALRXRPFSKIPAVDAEPFAIFVNAMDTNPLAADPVVVIKEAXXDFRRXXLV
                   130       140       150       160       170       180
```

The complete strain B sequence (ORF22-1) and ORF22a show 94.9% identity in 447 aa overlap:

```
                    10        20        30        40        50        60
orf22a.pep   MIKIKKGLNLPIAGRPEQVIYDGPVITEVALLGEEYAGMRPXMKVKEGDAVKKGQVLFED
             ||||||||||||||||||::||||:|||||||||||||||||| ||||||||||||||||
orf22-1      MIKIKKGLNLPIAGRPEQAVYDGPAITEVALLGEEYAGMRPSMKVKEGDAVKKGQVLFED
                    10        20        30        40        50        60

                    70        80        90       100       110       120
orf22a.pep   KKXPGVVFTAPVSGKIAAIHRGEKRVLQSVVIAVEGNDEIEFERYAPEALANLSGXEXXX
             || ||||||||:||||||||||||||||||||||||||||||||||||||||||| |   
orf22-1      KKNPGVVFTAPASGKIAAIHRGEKRVLQSVVIAVEGNDEIEFERYAPEALANLSGEEVRR
                    70        80        90       100       110       120
```

```
                   130       140       150       160       170       180
orf22a.pep   NLIQSGLWTALRXRPFSKIPAVDAEPFAIFVNAMDTNPLAADPVVVIKEAXXDFRRXXLV
             ||||||||||||:||||||||||||||||||||||||||||||:|:||||  ||:|  ||
orf22-1      NLIQSGLWTALRTRPFSKIPAVDAEPFAIFVNAMDTNPLAADPTVIIKEAAEDFKRGLLV
                   130       140       150       160       170       180

                   190       200       210       220       230       240
orf22a.pep   LSRLTERKIHVCKAAGADVPSENAANIETHEFGGPHPAGLSGTHIHFIEPVGANKTVWTI
             ||||||||||||||||||||||||||||||||||||||||||||||||||||||||||||
orf22-1      LSRLTERKIHVCKAAGADVPSENAANIETHEFGGPHPAGLSGTHIHFIEPVGANKTVWTI
                   190       200       210       220       230       240

                   250       260       270       280       290       300
orf22a.pep   NYQDVIAIGRLFATGRLNTERVIALGGSQVNKPRLLRTVLGAKVSQITAGELVDADNRVI
             ||||||:||||||||||||||||||||||||||||||||||||||||||||||:||||||
orf22-1      NYQDVITIGRLFATGRLNTERVIALGGSQVNKPRLLRTVLGAKVSQITAGELVDTDNRVI
                   250       260       270       280       290       300

                   310       320       330       340       350       360
orf22a.pep   SGSVLNGAITQGAHDYLGRYHNQISVIEEGRSKELFGWVAPQPDKYSITRTTLGHFLKNK
             ||||||||||||||||||||||||||||||||||||||||||||||||||||||||||||
orf22-1      SGSVLNGAITQGAHDYLGRYHNQISVIEEGRSKELFGWVAPQPDKYSITRTTLGHFLKNK
                   310       320       330       340       350       360

                   370       380       390       400       410       420
orf22a.pep   LFKFTTAVNGGDRAMVPIGTYERVMPLDILPTLLLRDLIVGDTDSAQALGCLELDEEDLA
             ||||:|||||||||||||||||||||||||||||||||||||||||||||||||||||||
orf22-1      LFKFNTAVNGGDRAMVPIGTYERVMPLDILPTLLLRDLIVGDTDSAQALGCLELDEEDLA
                   370       380       390       400       410       420

                   430       440
orf22a.pep   LCSFVCPGKYEXGPLLRKVLETXEKEGX
             ||||||||||| |||||||||| |||||
orf22-1      LCSFVCPGKYEYGPLLRKVLETIEKEGX
                   430       440
```

Further work identified a partial gene sequence <SEQ ID 129> from *N.gonorrhoeae,* which encodes the following amino acid sequence <SEQ ID 130; ORF22ng>:

```
  1 MIKIKKGLNL PIAGRPEQVI YDGPAITEVA LLGEEYVGMR PSMKIKEGEA
 51 VKKGQVLFED KKNPGVVFTA PASGKIAAIH RGEKRVLQSV VIAVEGNDEI
101 EFERYVPEAL AKLSSEKVRR NLIQSGLWTA LRTRPFSKIP AVDAEPFAIF
151 VNAMDTNPLA ADPTVIIKEA AEDFKRGLLV LSRLTERKIH VCKAAGADVP
201 SENAANIETH EFGGPHPAGL SGTHIHFIEP VGANKTVWTI NYQDVIAIGR
251 LFVTGRLNTE RVVALGGLQV NKPRLLRTVL GAKVSQLTAG ELVDADNRVI
301 SGSVLNGAIA QGAHDYLGRY HN*
```

Further work identified complete gonococcal gene <SEQ ID 131>:

```
   1  ATGATTAAAA TCAAAAAAGG TCTAAATCTG CCCATCGCGG GCAGACCGGA
  51  GCAAGTCATT TATGACGGCC CGGCCATTAC CGAAGTCGCG TTGCTTGGCG
 101  AAGAATATGT CGGCATGCGC CCCTCGATGA AAATCAAGGA AGGTGAAGCC
 151  GTCAAAAAAG GCCAAGTGCT GTTTGAAGAC AAAAAGAATC CGGGCGTAGT
 201  ATTTACTGCG CCGGCTTCAG GCAAAATCGC CGCTATTCAC CGTGGCGAAA
 251  AGCGCGTACT TCAGTCAGTC GTGATTGCCG TTGAAGGCAA CGACGAAATC
 301  GAGTTCGAAC GCTACGTACC TGAAGCGCTG GCAAAATTGA GCAGCGAAAA
 351  AGTGCGCCGC AACCTGATTC AATCAGGCTT ATGGACTGCG CTTCGCACCC
 401  GTCCGTTCAG CAAAATCCCT GCCGTAGATG CCGAGCCGTT CGCCATCTTC
 451  GTCAATGCGA TGGACACCAA TCCGCTGGCT GCCGACCCTA CGGTCATCAT
 501  CAAAGAAGCC GCCGAAGACT TCAAACGCGG CCTGTTGGTA TTGAGCCGCC
 551  TGACCGAACG TAAAATCCAT GTGTGTAAAG CAGCAGGCGC AGACGTGCCG
 601  TCTGAAAATG CTGCCAATAT CGAAACACAT GAATTTGGCG GCCCGCATCC
 651  TGCCGGCTTG AGTGGCACGC ACATTCATTT CATCGAGCCA GTCGGCGCGA
 701  ATAAAACCGT GTGGACCATC AATTATCAAG ACGTGATTGC TATCGGACGT
 751  TTGTTCGTAA CAGGCCGTCT GAATACCGAG CGCGTGGTTG CCTTGGGCGG
 801  CCTGCAAGTC AACAAACCGC GCCTCTTGCG TACCGTTTTG GGTGCGAAGG
 851  TGTCTCAACT TACCGCCGGC GAATTGGTTG ACGCGGACAA CCGCGTGATT
 901  TCCGGTTCGG TATTGAACGG TGCGATTGCA CAAGGCGCGC ATGATTATTT
 951  GGGACGCTAC CACAATCAGA TTTCCGTTAT CGAAGAAGGC CGCAGCAAAG

1001  AGCTGTTCGG CTGGGTTGCG CCGCAGCCGG ACAAATACTC CATCACGCGC
1051  ACCACTCTCG GCCATTTCCT AAAAAACAAA CTCTTCAAGT TCACGACAGC
1101  CGTCAACGGC GGCGACCGCG CCATGGTACC GATCGGCACT TATGAGCGCG
1151  TAATGCCGTT GGACATCCTG CCTACCTTGC TTTTGCGCGA TTTAATCGTC
1201  GGCGATACCG ACAGCGCGCA GGCTTTGGGT TGCTTGGAAT TGGACGAAGA
1251  AGACCTCGCT TTGTGCAGCT TCGTCTGCCC GGGCAAATAC GAATACGGCC
1301  CGCTGTTGCG CAAAGTGCTG GAAACCATTG AGAAGGAAGG CTGA
```

This encodes a protein having amino acid sequence <SEQ ID 132; ORF22ng-l>:

```
  1  MIKIKKGLNL PIAGRPEQVI YDGPAITEVA LLGEEYVGMR PSMKIKEGEA
 51  VKKGQVLFED KKNPGVVFTA PASGKIAAIH RGEKRVLQSV VIAVEGNDEI
101  EFERYVPEAL AKLSSEKVRR NLIQSGLWTA LRTRPFSKIP AVDAEPFAIF
151  VNAMDTNPLA ADPTVIIKEA AEDFKRGLLV LSRLTERKIH VCKAAGADVP
201  SENAANIETH EFGGPHPAGL SGTHIHFIEP VGANKTVWTI NYQDVIAIGR
251  LFVTGRLNTE RVVALGGLQV NKPRLLRTVL GAKVSQLTAG ELVDADNRVI
301  SGSVLNGAIA QGAHDYLGRY HNQISVIEEG RSKELFGWVA PQPDKYSITR
351  TTLGHFLKNK LFKFTTAVNG GDRAMVPIGT YERVMPLDIL PTLLLRDLIV
401  GDTDSAQALG CLELDEEDLA LCSFVCPGKY EYGPLLRKVL ETIEKEG*
```

The originally-identified partial strain B sequence (ORF22) shows 93.7% identity over a 158aa overlap with ORF22ng:

```
orf22.pep   MIKIKKGLNLPIAGRPEQAVYDGPAITEVALLGEEYAGMRPSMKVKEGDAVKKGQVLFED   60
            ||||||||||||||||||::||||||||||||||||:|||||||:|||:|||||||||||
orf22ng     MIKIKKGLNLPIAGRPEQVIYDGPAITEVALLGEEYVGMRPSMKIKEGEAVKKGQVLFED   60

orf22.pep   KKNPGVVFTAPASGKIAAIHRGEKRVLQSVVIAVEXNDEIEFERYAPEALANLSGEEVRR  120
            ||||||||||||||||||||||||||||||||||| |||||||||:|||||:||:|:|||
orf22ng     KKNPGVVFTAPASGKIAAIHRGEKRVLQSVVIAVEGNDEIEFERYVPEALAKLSSEKVRR  120

orf22.pep   NLIQSGLWTALRTRPFSKIPAVDAEPFAIFVNAMDTNP                        158
            ||||||||||||||||||||||||||||||||||||||
orf22ng     NLIQSGLWTALRTRPFSKIPAVDAEPFAIFVNAMDTNPLAADPTVIIKEAAEDFKRGLLV  180
```

The complete sequences from strain B (ORF22-1) and gonococcus (ORF22ng) show 96.2% identity in 447 aa overlap:

```
                      10        20        30        40        50        60
orf22-1.pep   MIKIKKGLNLPIAGRPEQAVYDGPAITEVALLGEEYAGMRPSMKVKEGDAVKKGQVLFED
              ||||||||||||||||||::||||||||||||||||:|||||||:|||:|||||||||||
orf22ng-1     MIKIKKGLNLPIAGRPEQVIYDGPAITEVALLGEEYVGMRPSMKIKEGEAVKKGQVLFED
                      10        20        30        40        50        60

                      70        80        90       100       110       120
orf22-1.pep   KKNPGVVFTAPASGKIAAIHRGEKRVLQSVVIAVEGNDEIEFERYAPEALANLSGEEVRR
              |||||||||||||||||||||||||||||||||||||||||||||:|||||:||:|:|||
orf22ng-1     KKNPGVVFTAPASGKIAAIHRGEKRVLQSVVIAVEGNDEIEFERYVPEALAKLSSEKVRR
                      70        80        90       100       110       120

                     130       140       150       160       170       180
orf22-1.pep   NLIQSGLWTALRTRPFSKIPAVDAEPFAIFVNAMDTNPLAADPTVIIKEAAEDFKRGLLV
              ||||||||||||||||||||||||||||||||||||||||||||||||||||||||||||
orf22ng-1     NLIQSGLWTALRTRPFSKIPAVDAEPFAIFVNAMDTNPLAADPTVIIKEAAEDFKRGLLV
                     130       140       150       160       170       180

                     190       200       210       220       230       240
orf22-1.pep   LSRLTERKIHVCKAAGADVPSENAANIETHEFGGPHPAGLSGTHIHFIEPVGANKTVWTI
              ||||||||||||||||||||||||||||||||||||||||||||||||||||||||||||
orf22ng-1     LSRLTERKIHVCKAAGADVPSENAANIETHEFGGPHPAGLSGTHIHFIEPVGANKTVWTI
                     190       200       210       220       230       240

                     250       260       270       280       290       300
orf22-1.pep   NYQDVITIGRLFATGRLNTERVIALGGSQVNKPRLLRTVLGAKVSQITAGELVDTDNRVI
              ||||||:|||||:|||||||||:|||| ||||||||||||||||||:|||||||:|||||
orf22ng-1     NYQDVIAIGRLFVTGRLNTERVVALGGLQVNKPRLLRTVLGAKVSQLTAGELVDADNRVI
                     250       260       270       280       290       300

                     310       320       330       340       350       360
orf22-1.pep   SGSVLNGAITQGAHDYLGRYHNQISVIEEGRSKELFGWVAPQPDKYSITRTTLGHFLKNK
              |||||||||:||||||||||||||||||||||||||||||||||||||||||||||||||
orf22ng-1     SGSVLNGAIAQGAHDYLGRYHNQISVIEEGRSKELFGWVAPQPDKYSITRTTLGHFLKNK
                     310       320       330       340       350       360

                     370       380       390       400       410       420
orf22-1.pep   LFKFNTAVNGGDRAMVPIGTYERVMPLDILPTLLLRDLIVGDTDSAQALGCLELDEEDLA
              ||||:|||||||||||||||||||||||||||||||||||||||||||||||||||||||
orf22ng-1     LFKFTTAVNGGDRAMVPIGTYERVMPLDILPTLLLRDLIVGDTDSAQALGCLELDEEDLA
                     370       380       390       400       410       420

                     430       440
orf22-1.pep   LCSFVCPGKYEYGPLLRKVLETIEKEGX
              ||||||||||||||||||||||||||||
orf22ng-1     LCSFVCPGKYEYGPLLRKVLETIEKEGX
                     430       440
```

Computer analysis of these sequences gave the following results:

Homology with 48kDa outer membrane protein of *Actinobacillus pleuropneumoniae* (accession number U24492).

ORF22 and this 48kDa protein show 72% aa identity in 158aa overlap:

```
Orf22  1   MIKIKKGLNLPIAGRPEQAVYDGPAITEVALLGEEYAGMRPSMKVKEGDAVKKGQVLFED 60
           MI IKKGL+LPIAG P Q +++G  + EVA+LGEEY GMRPSMKV+EGD VKKGQVLFED
48kDa  1   MITIKKGLDLPIAGTPAQVIHNGNTVNEVAMLGEEYVGMRPSMKVREGDVVKKGQVLFED 60

orf22  61  KKNPGVVFTAPASGKIAAIHRGEKRVLQSVVIAVEXNDEIEFERYAPEALANLSGEEVRR 120
           KKNPGVVFTAPASG +   I+RGEKRVLQSVVI VE +++I F RY    LA+LS E+V++
48kDa  61  KKNPGVVFTAPASGTVVTINRGEKRVLQSVVIKVEGDEQITFTRYEAAQLASLSAEQVKQ 120

orf22  121 NLIQSGLWTALRTRPFSKIPAVDAEPFAIFVNAMDTNP 158
           NLI+SGLWTA RTRPFSK+PA+DA P +IFVNAMDTNP
48kDa  121 NLIESGLWTAFRTRPFSKVPALDAIPSSIFVNAMDTNP 158
```

ORF22a also shows homology to the 48kDa *Actinobacillus pleuropneumoniae* protein:

```
gi|1185395 (U24492) 48 kDa outer membrane protein [Actinobacillus pleuropneumoniae]
Length = 449

 Score =  530 bits (1351), Expect = e-150
 Identities = 274/450 (60%), Positives = 323/450 (70%), Gaps = 4/450 (0%)

Query: 1   MIKIKKGLNLPIAGRPEQVIYDGPVITEVALLGEEYAGMRPXMKVKEGDAVKKGQVLFED 60
            MI IKKGL+LPIAG P QVI++G  + EVA+LGEEY GMRP MKV+EGD VKKGQVLFED
Sbjct: 1   MITIKKGLDLPIAGTPAQVIHNGNTVNEVAMLGEEYVGMRPSMKVREGDVVKKGQVLFED 60

Query: 61  KKXPGVVFTAPVSGKIAAIHRGEKRVLQSVVIAVEGNDEIEFERYAPEALANLSGXEXXX 120
            KK PGVVFTAP SG +   I+RGEKRVLQSVVI VEG+++I F RY    LA+LS  +
Sbjct: 61  KKNPGVVFTAPASGTVVTINRGEKRVLQSVVIKVEGDEQITFTRYEAAQLASLSAEQVKQ 120

Query: 121 NLIQSGLWTALRXRPFSKIPAVDAEPFAIFVNAMDTNPLAADPVVVIKEAXXDFRRXXLV 180
            NLI+SGLWTA R RPFSK+PA+DA P +IFVNAMDTNPLAADP VV+KE   DF+   V
Sbjct: 121 NLIESGLWTAFRTRPFSKVPALDAIPSSIFVNAMDTNPLAADPEVVLKEYETDFKDGLTV 180

Query: 181 LSRL--TERKIHVCKAAGADVP-SENAANIETHEFGGPHPAGLSGTHIHFIEPVGANKTV 237
            L+RL   ++ +++CK A +++P S    I    F G HPAGL GTHIHF++PVGA K V
Sbjct: 181 LTRLFNGQKPVYLCKDADSNIPLSPAIEGITIKSFSGVHPAGLVGTHIHFVDPVGATKQV 240

Query: 238 WTINYQDVIAIGRLFATGRLNTERVIALGGSQVNKPRLLRTVLGAKVSQITAGELVDADN 297
            W +NYQDVIAIG+LF TG L T+R+I+L G QV  PRL+RT LGA +SQ+TA EL   +N
Sbjct: 241 WHLNYQDVIAIGKLFTTGELFTDRIISLAGPQVKNPRLVRTRLGANLSQLTANELNAGEN 300

Query: 298 RVISGSVLNGAITQGAHDYLGRYHNQISVIEEGRSKELFGWVAPQPDKYSITRTTLGHFL 357
            RVISGSVL+GA   G  DYLGRY  Q+SV+ EGR KELFGW+ P  DK+SITRT LGHF
Sbjct: 301 RVISGSVLSGATAAGPVDYLGRYALQVSVLAEGREKELFGWIMPGSDKFSITRTVLGHFG 360

Query: 358 KNKLFKFTTAVNGGDRAMVPIGTYERVMXXXXXXXXXXXXXXVGDTDSAQXXXXXXXXXX 417

            K KLF FTTAV+GG+RAMVPIG YERVM              GDTDSAQ
Sbjct: 361 K-KLFNFTTAVHGGERAMVPIGAYERVMPLDIIPTLLLRDLAAGDTDSAQNLGCLELDEE 419

Query: 418 XXXXXSFVCPGKYEXGPLLRKVLETXEKEG 447
                 ++VCPGK   GP+LR  LE  EKEG
```

ORF22ng-1 also shows homology with the OMP from *A.pleuropneumoniae:*

```
 gi|1185395  (U24492)  48  kDa  outer  membrane  protein  [Actinobacillus
pleuropneumoniae] Length = 449
 Score =  555 bits (1414), Expect = e-157
 Identities = 284/450 (63%), Positives = 337/450 (74%), Gaps = 4/450 (0%)

Query: 27  MIKIKKGLNLPIAGRPEQVIYDGPAITEVALLGEEYVGMRPSMKIKEGEAVKKGQVLFED 86
            MI IKKGL+LPIAG P QVI++G  + EVA+LGEEYVGMRPSMK++EG+ VKKGQVLFED
Sbjct: 1   MITIKKGLDLPIAGTPAQVIHNGNTVNEVAMLGEEYVGMRPSMKVREGDVVKKGQVLFED 60

Query: 87  KKNPGVVFTAPASGKIAAIHRGEKRVLQSVVIAVEGNDEIEFERYVPEALAKLSSEKVRR 146
            KKNPGVVFTAPASG +   I+RGEKRVLQSVVI VEG+++I F RY    LA LS+E+V++
Sbjct: 61  KKNPGVVFTAPASGTVVTINRGEKRVLQSVVIKVEGDEQITFTRYEAAQLASLSAEQVKQ 120

Query: 147 NLIQSGLWTALRTRPFSKIPAVDAEPFAIFVNAMDTNPLAADPTVIIKEAAEDFKRGLLV 206
            NLI+SGLWTA RTRPFSK+PA+DA P +IFVNAMDTNPLAADP V++KE   DFK GL V
Sbjct: 121 NLIESGLWTAFRTRPFSKVPALDAIPSSIFVNAMDTNPLAADPEVVLKEYETDFKDGLTV 180

Query: 207 LSRL--TERKIHVCKAAGADVP-SENAANIETHEFGGPHPAGLSGTHIHFIEPVGANKTV 263
            L+RL   ++ +++CK A +++P S     I    F G HPAGL GTHIHF++PVGA K V
Sbjct: 181 LTRLFNGQKPVYLCKDADSNIPLSPAIEGITIKSFSGVHPAGLVGTHIHFVDPVGATKQV 240

Query: 264 WTINYQDVIAIGRLFVTGRLNTERVVALGGLQVNKPRLLRTVLGAKVSQLTAGELVDADN 323
            W +NYQDVIAIG+LF TG L T+R+++L G QV  PRL+RT LGA +SQLTA EL   +N
Sbjct: 241 WHLNYQDVIAIGKLFTTGELFTDRIISLAGPQVKNPRLVRTRLGANLSQLTANELNAGEN 300

Query: 324 RVISGSVLNGAIAQGAHDYLGRYHNQISVIEEGRSKELFGWVAPQPDKYSITRTTLGHFL 383
            RVISGSVL+GA A G  DYLGRY  Q+SV+ EGR KELFGW+ P  DK+SITRT LGHF
Sbjct: 301 RVISGSVLSGATAAGPVDYLGRYALQVSVLAEGREKELFGWIMPGSDKFSITRTVLGHFG 360

Query: 384 KNKLFKFTTAVNGGDRAMVPIGTYERVMXXXXXXXXXXXXXXVGDTDSAQXXXXXXXXXX 443
            K KLF FTTAV+GG+RAMVPIG YERVM              GDTDSAQ
Sbjct: 361 K-KLFNFTTAVHGGERAMVPIGAYERVMPLDIIPTLLLRDLAAGDTDSAQNLGCLELDEE 419

Query: 444 XXXXXSFVCPGKYEYGPLLRKVLETIEKEG 473
                 ++VCPGK  YGP+LR  LE IEKEG
Sbjct: 420 DLALCTYVCPGKNNYGPMLRAALEKIEKEG 449
```

Based on this analysis, including the homology with the outer membrane protein of *Actinobacillus pleuropneumoniae,* it was predicted that these proteins from *N.meningitidis* and *N.gonorrhoeae,* and their epitopes, could be useful antigens for vaccines or diagnostics, or for raising antibodies. ORF22-1 (35.4kDa) was cloned in pET and pGex vectors and expressed in *E.coli,* as described above. The products of protein expression and purification were analyzed by SDS-PAGE. Figure 5A shows the results of affinity purification of the GST-fusion protein, and Figure 5B shows the results of expression of the His-fusion in *E.coli.* Purified GST-fusion protein was used to immunise mice, whose sera were used for ELISA (positive result) and FACS analysis (Figure 5C). These experiments confirm that ORF22-1 is a surface-exposed protein, and that it is a useful immunogen.

**Example 16**

The following partial DNA sequence was identified in *N.meningitidis* <SEQ ID 133>:

```
   1  ..GCGnCGnAAA TCATCCATCC CC..nACGTC GTAGGCCCTG AAGCCAACTG
  51    GTTTTTTATG GTAGCCAGTA CGTTTGTGAT TGCTTTGATT GGTTATTTTG
 101    TTACTGAAAA AATCGTCGAA CCGCAATTGG GCCCTTATCA ATCAGATTTG
 151    TCACAAGAAG AAAAAGACAT TCGGCATTCC AATGAAATCA CGCCTTTGGA
 201    ATATAAAGGA TTAATTTGGG CTGGCGTGGT GTTTGTTGCC TTATCCGCCC
 251    TATTGGCTTG GAGCATCGTC CCTGCCGACG GTATTTTGCG TCATCCTGAA
 301    ACAGGATTGG TTTCCGGTTC GCCGTTTTTA AAATCGATTG TTGTTTTTAT
 351    TTTCTTGTTG TTTGCACTGC CGGGCATTGT TTATGGCCGG GTAACCCGAA
 401    GTTTGCGCGG CGAACAGGAA GTCGTTAATG CGmyGGCCGA ATCGATGAGT
 451    ACTCTGGsGC TTTmTTTGsw CAkcATCTTT TTTGCCGCAC AGTTTGTCGC
 501    ATTTTTTAAT TGGACGAATA TTGGGCAATA TATTGCCGTT AAAGGGGCGA
 551    CGTTCTTAAA AGAAGTCGGC TTGGGCGGCA GCGTGTTGTT TATCGGTTTT
 601    ATTTTAATTT GTGCTTTTAT CAATCTGATG ATAGGCTCCG CCTCCGCGCA
 651    ATGGGCGGTA ACTGCGCCGA TTTTCGTCCC TATGCTGATG TTGGCCGGCT
 701    ACGCGCCCGA AGTCATTCAA GCCGCTTACC GCATCGGTGA TTCCGTTACC
 751    AATATTATTA CGCCGATGAT GAGTTATTTC GGGCTGATTA TGGCGACGGT
 801    GrkCmmmTAC AAAAAAGATG CGGGCGTGGG TaCGcTGATT wCTATGATGT
 851    TGCCGTATTC CGCTTTCTTC TTGATTGCgT GGATTGCCTT ATTCTGCATT
 901    TGGGTATTTg TTTTGGGCCT GCCCGTCGGT CCCGGCGCGC CCACATTCTA
 951    TCCCGCACCT TAA
```

This corresponds to the amino acid sequence <SEQ ID 134; ORF12>:

```
   1  ..AXXIIHPXXV VGPEANWFFM VASTFVIALI GYFVTEKIVE PQLGPYQSDL
  51    SQEEKDIRHS NEITPLEYKG LIWAGVVFVA LSALLAWSIV PADGILRHPE
 101    TGLVSGSPFL KSIVVFIFLL FALPGIVYGR VTRSLRGEQE VVNAXAESMS
 151    TLXLXLXXIF FAAQFVAFFN WTNIGQYIAV KGATFLKEVG LGGSVLFIGF
 201    ILICAFINLM IGSASAQWAV TAPIFVPMLM LAGYAPEVIQ AAYRIGDSVT
 251    NIITPMMSYF GLIMATVXXY KKDAGVGTLI XMMLPYSAFF LIAWIALFCI
 301    WVFVLGLPVG PGAPTFYPAP *
```

Further sequence analysis revealed the complete DNA sequence <SEQ ID 135> to be:

```
   1  ATGAGTCAAA CCGATACGCA ACGGGACGGA CGATTTTTAC GCACAGTCGA
  51  ATGGCTGGGC AATATGTTGC CGCATCCGGT TACGCTTTTT ATTATTTTCA
 101  TTGTGTTATT GCTGATTGCC TCTGCCGTCG GTGCGTATTT CGGACTATCC
 151  GTCCCCGATC CGCGCCCTGT TGGTGCGAAA GGACGTGCCG ATGACGGTTT
 201  GATTTACATT GTCAGCCTGC TCAATGCCGA CGGTTTTATC AAAATCCTGA
 251  CGCATACCGT TAAAAATTTC ACCGGTTTCG CGCCGTTGGG AACGGTGTTG
 301  GTTTCTTTAT TGGGCGTGGG GATTGCGGAA AAATCGGGCT TGATTTCCGC
 351  ATTAATGCGC TTATTGCTCA CAAAATCGCC ACGCAAACTC ACTACTTTTA
 401  TGGTTGTTTT TACAGGGATT TTATCTAATA CCGCTTCTGA ATTGGGCTAT
 451  GTCGTCCTAA TCCCTTTGTC CGCCATCATC TTTCATTCCC TCGGCCGCCA
 501  TCCGCTTGCC GGTCTGGCTG CGGCTTTCGC CGGCGTTTCG GGCGGTTATT
 551  CGGCCAATCT GTTCTTAGGC ACAATCGATC CGCTCTTGGC AGGCATCACC
 601  CAACAGGCGG CGCAAATCAT CCATCCCGAC TACGTCGTAG GCCCTGAAGC
 651  CAACTGGTTT TTTATGGTAG CCAGTACGTT TGTGATTGCT TTGATTGGTT
 701  ATTTTGTTAC TGAAAAAATC GTCGAACCGC AATTGGGCCC TTATCAATCA
 751  GATTTGTCAC AAGAAGAAAA AGACATTCGG CATTCCAATG AAATCACGCC
 801  TTTGGAATAT AAAGGATTAA TTTGGGCTGG CGTGGTGTTT GTTGCCTTAT
 851  CCGCCCTATT GGCTTGGAGC ATCGTCCCTG CCGACGGTAT TTTGCGTCAT
 901  CCTGAAACAG GATTGGTTTC CGGTTCGCCG TTTTTAAAAT CGATTGTTGT
 951  TTTTATTTTC TTGTTGTTTG CACTGCCGGG CATTGTTTAT GGCCGGGTAA
1001  CCCGAAGTTT GCGCGGCGAA CAGGAAGTCG TTAATGCGAT GGCCGAATCG
1051  ATGAGTACTC TGGGGCTTTA TTTGGTCATC ATCTTTTTTG CCGCACAGTT
1101  TGTCGCATTT TTTAATTGGA CGAATATTGG GCAATATATT GCCGTTAAAG
1151  GGGCGACGTT CTTAAAAGAA GTCGGCTTGG GCGGCAGCGT GTTGTTTATC
1201  GGTTTTATTT TAATTTGTGC TTTTATCAAT CTGATGATAG GCTCCGCCTC
1251  CGCGCAATGG GCGGTAACTG CGCCGATTTT CGTCCCTATG CTGATGTTGG
1301  CCGGCTACGC GCCCGAAGTC ATTCAAGCCG CTTACCGCAT CGGTGATTCC
1351  GTTACCAATA TTATTACGCC GATGATGAGT TATTTCGGGC TGATTATGGC
1401  GACGGTGATC AAATACAAAA AAGATGCGGG CGTGGGTACG CTGATTTCTA
1451  TGATGTTGCC GTATTCCGCT TTCTTCTTGA TTGCGTGGAT TGCCTTATTC
1501  TGCATTTGGG TATTTGTTTT GGGCCTGCCC GTCGGTCCCG GCGCGCCCAC
1551  ATTCTATCCC GCACCTTAA
```

This corresponds to the amino acid sequence <SEQ ID 136; ORF12-1>:

```
  1 MSQTDTQRDG RFLRTVEWLG NMLPHPVTLF IIFIVLLLIA SAVGAYFGLS
 51 VPDPRPVGAK GRADDGLIYI VSLLNADGFI KILTHTVKNF TGFAPLGTVL
101 VSLLGVGIAE KSGLISALMR LLLTKSPRKL TTFMVVFTGI LSNTASELGY
```

```
151 VVLIPLSAII FHSLGRHPLA GLAAAFAGVS GGYSANLFLG TIDPLLAGIT
201 QQAAQIIHPD YVVGPEANWF FMVASTFVIA LIGYFVTEKI VEPQLGPYQS
251 DLSQEEKDIR HSNEITPLEY KGLIWAGVVF VALSALLAWS IVPADGILRH
301 PETGLVSGSP FLKSIVVFIF LLFALPGIVY GRVTRSLRGE QEVVNAMAES
351 MSTLGLYLVI IFFAAQFVAF FNWTNIGQYI AVKGATFLKE VGLGGSVLFI
401 GFILICAFIN LMIGSASAQW AVTAPIFVPM LMLAGYAPEV IQAAYRIGDS
451 VTNIITPMMS YFGLIMATVI KYKKDAGVGT LISMMLPYSA FFLIAWIALF
501 CIWVFVLGLP VGPGAPTFYP AP*
```

Computer analysis of this amino acid sequence gave the following results:

<u>Homology with a predicted ORF from *N.meningitidis* (strain A)</u>

ORF12 shows 96.3% identity over a 320aa overlap with an ORF (ORF12a) from strain A of *N. meningitidis:*

```
                                                 10        20        30
orf12.pep                                AXXIIHPXXVVGPEANWFFMVASTFVIALI
                                         |  ||||  |||||||||||||||||||||
orf12a     AAAFAGVSGGYSANLFLGTIDPLLAGITQQAAQIIHPDYVVGPEANWFFMVASTFVIALI
                   180       190       200       210       220       230

                   40        50        60        70        80        90
orf12.pep  GYFVTEKIVEPQLGPYQSDLSQEEKDIRHSNEITPLEYKGLIWAGVVFVALSALLAWSIV
           ||||||||||||||||||||||||||||||||||||||||||||||||||||||||||||
orf12a     GYFVTEKIVEPQLGPYQSDLSQEEKDIRHSNEITPLEYKGLIWAGVVFVALSALLAWSIV
                  240       250       260       270       280       290

                  100       110       120       130       140       150
orf12.pep  PADGILRHPETGLVSGSPFLKSIVVFIFLLFALPGIVYGRVTRSLRGEQEVVNAXAESMS
           |||||||||||||||||||||||||||||||||||||||||||||||||||||| |||||
orf12a     PADGILRHPETGLVSGSPFLKSIVVFIFLLFALPGIVYGRVTRSLRGEQEVVNAMAESMS
                  300       310       320       330       340       350

                  160       170       180       190       200       210
orf12.pep  TLXLXLXXIFFAAQFVAFFNWTNIGQYIAVKGATFLKEVGLGGSVLFIGFILICAFINLM
           || | |  ||||||||||||||||||||||||||||||||||||||||||||||||||||
orf12a     TLGLYLVIIFFAAQFVAFFNWTNIGQYIAVKGATFLKEVGLGGSVLFIGFILICAFINLM
                  360       370       380       390       400       410

                  220       230       240       250       260       270
orf12.pep  IGSASAQWAVTAPIFVPMLMLAGYAPEVIQAAYRIGDSVTNIITPMMSYFGLIMATVXXY
           |||||||||||||||||||||||||||||||||||||||||||||||||||||||||  |
orf12a     IGSASAQWAVTAPIFVPMLMLAGYAPEVIQAAYRIGDSVTNIITPMMSYFGLIMATVIKY
                  420       430       440       450       460       470

                  280       290       300       310       320
orf12.pep  KKDAGVGTLIXMMLPYSAFFLIAWIALFCIWVFVLGLPVGPGAPTFYPAPX
           |||||||||| ||||||||||||||||||||||||||||||||||||||||
orf12a     KKDAGVGTLISMMLPYSAFFLIAWIALFCIWVFVLGLPVGPGAPTFYPAPX
                  480       490       500       510       520
```

The complete length ORF12a nucleotide sequence <SEQ ID 137> is:

```
   1 ATGAGTCAAA CCGATACGCA ACGGGACGGA CGATTTTTAC GCACAGTCGA
  51 ATGGCTGGGC AATATGTTGC CGCACCCGGT TACGCTTTTT ATTATTTTCA
 101 TTGTGTTATT GCTGATTGCC TCTGCCGCCG GTGCGTATTT CGGACTATCC
 151 GTCCCCGATC CGCGCCCTGT TGGTGCGAAA GGACGTGCCG ATGACGGTTT
 201 GATTCACGTT GTCAGCCTGC TCGATGCTGA CGGTTTGATC AAAATCCTGA
 251 CGCATACCGT TAAAAATTTC ACCGGTTTCG CGCCGTTGGG AACGGTGTTG
 301 GTTTCTTTAT TGGGCGTGGG GATTGCGGAA AAATCGGGCT TGATTTCCGC
 351 ATTAATGCGC TTATTGCTCA CAAAATCTCC ACGCAAACTC ACTACTTTTA
 401 TGGTTGTTTT TACAGGGATT TTATCTAATA CCGCTTCTGA ATTGGGCTAT
 451 GTCGTCCTAA TCCCTTTGTC CGCCATCATC TTTCATTCCC TCGGCCGCCA
 501 TCCGCTTGCC GGTCTGGCTG CGGCTTTCGC CGGCGTTTCG GGCGGTTATT
 551 CGGCCAATCT GTTCTTAGGC ACAATCGATC CGCTCTTGGC AGGCATCACC
 601 CAACAGGCGG CGCAAATCAT CCATCCCGAC TACGTCGTAG GCCCTGAAGC
 651 CAACTGGTTT TTTATGGTAG CCAGTACGTT TGTGATTGCT TTGATTGGTT
 701 ATTTTGTTAC TGAAAAAATC GTCGAACCGC AATTGGGCCC TTATCAATCA
 751 GATTTGTCAC AAGAAGAAAA AGACATTCGA CATTCCAATG AAATCACGCC
```

```
 801 TTTGGAATAT AAAGGATTAA TTTGGGCTGG CGTGGTGTTT GTTGCCTTAT
 851 CCGCCCTATT GGCTTGGAGC ATCGTCCCTG CCGACGGTAT TTTGCGTCAT
 901 CCTGAAACAG GATTGGTTTC CGGTTCGCCG TTTTTAAAAT CAATTGTTGT
 951 TTTTATTTTC TTGTTGTTTG CACTGCCGGG CATTGTTTAT GGCCGGGTAA
1001 CCCGAAGTTT GCGCGGCGAA CAGGAAGTCG TTAATGCGAT GGCCGAATCG
1051 ATGAGTACTC TGGGGCTTTA TTTGGTCATC ATCTTTTTTG CCGCACAGTT
1101 TGTCGCATTT TTTAATTGGA CGAATATTGG GCAATATATT GCCGTTAAAG
1151 GGGCGACGTT CTTAAAAGAA GTCGGCTTGG GCGGCAGCGT GTTGTTTATC
1201 GGTTTTATTT TAATTTGTGC TTTTATCAAT CTGATGATAG GCTCCGCCTC
1251 CGCGCAATGG GCGGTAACTG CGCCGATTTT CGTCCCTATG CTGATGTTGG
1301 CCGGCTACGC GCCCGAAGTC ATTCAAGCCG CTTACCGCAT CGGTGATTCC
1351 GTTACCAATA TTATTACGCC GATGATGAGT TATTTCGGGC TGATTATGGC
1401 GACGGTGATC AAATACAAAA AAGATGCGGG CGTGGGTACG CTGATTTCTA
1451 TGATGTTGCC GTATTCCGCT TTCTTCTTGA TTGCGTGGAT TGCCTTATTC
1501 TGCATTTGGG TATTTGTTTT GGGCCTGCCC GTCGGTCCCG GCGCGCCCAC
1551 ATTCTATCCC GCACCTTAA
```

This encodes a protein having amino acid sequence <SEQ ID 138>:

```
  1 MSQTDTQRDG RFLRTVEWLG NMLPHPVTLF IIFIVLLLIA SAAGAYFGLS
 51 VPDPRPVGAK GRADDGLIHV VSLLDADGLI KILTHTVKNF TGFAPLGTVL
101 VSLLGVGIAE KSGLISALMR LLLTKSPRKL TTFMVVFTGI LSNTASELGY
151 VVLIPLSAII FHSLGRHPLA GLAAAFAGVS GGYSANLFLG TIDPLLAGIT
201 QQAAQIIHPD YVVGPEANWF FMVASTFVIA LIGYFVTEKI VEPQLGPYQS
251 DLSQEEKDIR HSNEITPLEY KGLIWAGVVF VALSALLAWS IVPADGILRH
301 PETGLVSGSP FLKSIVVFIF LLFALPGIVY GRVTRSLRGE QEVVNAMAES
351 MSTLGLYLVI IFFAAQFVAF FNWTNIGQYI AVKGATFLKE VGLGGSVLFI
401 GFILICAFIN LMIGSASAQW AVTAPIFVPM LMLAGYAPEV IQAAYRIGDS
451 VTNIITPMMS YFGLIMATVI KYKKDAGVGT LISMMLPYSA FFLIAWIALF
501 CIWVFVLGLP VGPGAPTFYP AP*
```

ORF12a and ORF12-1 show 99.0% identity in 522 aa overlap:

```
                    10        20        30        40        50        60
orf12a.pep   MSQTDTQRDGRFLRTVEWLGNMLPHPVTLFIIFIVLLLIASAAGAYFGLSVPDPRPVGAK
             ||||||||||||||||||||||||||||||||||||||||||:|||||||||||||||||
orf12-1      MSQTDTQRDGRFLRTVEWLGNMLPHPVTLFIIFIVLLLIASAVGAYFGLSVPDPRPVGAK
                    10        20        30        40        50        60

                    70        80        90       100       110       120
orf12a.pep   GRADDGLIHVVSLLDADGLIKILTHTVKNFTGFAPLGTVLVSLLGVGIAEKSGLISALMR
             ||||||||::||||:|||:|||||||||||||||||||||||||||||||||||||||||
orf12-1      GRADDGLIYIVSLLNADGFIKILTHTVKNFTGFAPLGTVLVSLLGVGIAEKSGLISALMR
                    70        80        90       100       110       120

                   130       140       150       160       170       180
orf12a.pep   LLLTKSPRKLTTFMVVFTGILSNTASELGYVVLIPLSAIIFHSLGRHPLAGLAAAFAGVS
             ||||||||||||||||||||||||||||||||||||||||||||||||||||||||||||
orf12-1      LLLTKSPRKLTTFMVVFTGILSNTASELGYVVLIPLSAIIFHSLGRHPLAGLAAAFAGVS
                   130       140       150       160       170       180

                   190       200       210       220       230       240
orf12a.pep   GGYSANLFLGTIDPLLAGITQQAAQIIHPDYVVGPEANWFFMVASTFVIALIGYFVTEKI
             ||||||||||||||||||||||||||||||||||||||||||||||||||||||||||||
orf12-1      GGYSANLFLGTIDPLLAGITQQAAQIIHPDYVVGPEANWFFMVASTFVIALIGYFVTEKI
                   190       200       210       220       230       240

                   250       260       270       280       290       300
orf12a.pep   VEPQLGPYQSDLSQEEKDIRHSNEITPLEYKGLIWAGVVFVALSALLAWSIVPADGILRH
             ||||||||||||||||||||||||||||||||||||||||||||||||||||||||||||
orf12-1      VEPQLGPYQSDLSQEEKDIRHSNEITPLEYKGLIWAGVVFVALSALLAWSIVPADGILRH
                   250       260       270       280       290       300

                   310       320       330       340       350       360
orf12a.pep   PETGLVSGSPFLKSIVVFIFLLFALPGIVYGRVTRSLRGEQEVVNAMAESMSTLGLYLVI
             ||||||||||||||||||||||||||||||||||||||||||||||||||||||||||||
orf12-1      PETGLVSGSPFLKSIVVFIFLLFALPGIVYGRVTRSLRGEQEVVNAMAESMSTLGLYLVI
                   310       320       330       340       350       360

                   370       380       390       400       410       420
orf12a.pep   IFFAAQFVAFFNWTNIGQYIAVKGATFLKEVGLGGSVLFIGFILICAFINLMIGSASAQW
             ||||||||||||||||||||||||||||||||||||||||||||||||||||||||||||
orf12-1      IFFAAQFVAFFNWTNIGQYIAVKGATFLKEVGLGGSVLFIGFILICAFINLMIGSASAQW
                    370       380       390       400       410       420

                    430       440       450       460       470       480
orf12a.pep   AVTAPIFVPMLMLAGYAPEVIQAAYRIGDSVTNIITPMMSYFGLIMATVIKYKKDAGVGT
             ||||||||||||||||||||||||||||||||||||||||||||||||||||||||||||
orf12-1      AVTAPIFVPMLMLAGYAPEVIQAAYRIGDSVTNIITPMMSYFGLIMATVIKYKKDAGVGT
                    430       440       450       460       470       480

                    490       500       510       520
orf12a.pep   LISMMLPYSAFFLIAWIALFCIWVFVLGLPVGPGAPTFYPAPX
             |||||||||||||||||||||||||||||||||||||||||||
orf12-1      LISMMLPYSAFFLIAWIALFCIWVFVLGLPVGPGAPTFYPAPX
                    490       500       510       520
```

Homology with a predicted ORF from *N.gonorrhoeae*

ORF12 shows 92.5% identity over a 320aa overlap with a predicted ORF (ORF12.ng) from *N. gonorrhoeae:*

```
orf12.pep                                       AXXIIHPXXVVGPEANWFFMVASTFVIALI   30
                                                |  ||||  |||||||||||:|||||||||
orf12ng         AAAFAGVSGGYSANLFLGTIDPLLAGITQQAAQIIHPDYVVGPEANWFFMAASTFVIALI  232

orf12.pep       GYFVTEKIVEPQLGPYQSDLSQEEKDIRHSNEITPLEYKGLIWAGVVFVALSALLAWSIV   90
                ||||||||||||||||||||||||||||||||||||||||||||||||||||||||||||
orf12ng         GYFVTEKIVEPQLGPYQSDLSQEEKDIRHSNEITPLEYKGLIWAGVVFVALSALLAWSIV  292

orf12.pep       PADGILRHPETGLVSGSPFLKSIVVFIFLLFALPGIVYGRVTRSLRGEQEVVNAXAESMS  150
                ||||||||||||||:|||||||||||||||||||||||||:|||||||:||||| |||||
orf12ng         PADGILRHPETGLVAGSPFLKSIVVFIFLLFALPGIVYGRITRSLRGEREVVNAMAESMS  352

orf12.pep       TLXLXLXXIFFAAQFVAFFNWTNIGQYIAVKGATFLKEVGLGGSVLFIGFILICAFINLM  210
                || | |  |||||||||||||||||||||||||:|||:  ||||||||||||||||||||
orf12ng         TLGLYLVIIFFAAQFVAFFNWTNIGQYIAVKGAVFLKKFRLGGSVLFIGFILICAFINLM  412

orf12.pep       IGSASAQWAVTAPIFVPMLMLAGYAPEVIQAAYRIGDSVTNIITPMMSYFGLIMATVXXY  270
                ||||||||||||||||||||||| ||:||||||||||||||||||||||||||||||  |
orf12ng         IGSASAQWAVTAPIFVPMLMLAGNAPQVIQAAYRIGDSVTNIITPMMSYFGLIMATVIKY  472

orf12.pep       KKDAGVGTLIXMMLPYSAFFLIAWIALFCIWVFVLGLPVGPGAPTFYPAP  320
                |||||||||| |||||||||||||||||||||||||||||||:|||||:|
orf12ng         KKDAGVGTLISMMLPYSAFFLIAWIALFCIWVFVLGLPVGPGTPTFYPVP  522
```

The complete length ORF12ng nucleotide sequence <SEQ ID 139> is:

```
   1  ATGAGTCAAA CCGACGCGCG TCGTAGCGGA CGATTTTTAC GCACAGTCGA
  51  ATGGCTGGGC AATATGTTGC CGCACCCGGT TACGCTTTTT ATTATTTTCA
 101  TTGTGTTATT GCTGATTGcc tctgCCGTCG GTGCGTATTT CGGACTATCC
 151  GTCCCCGATC CGCGTCCTGT TGGGGCGAAA GGACGTGCCG ATGACGGTTT
 201  GATTCACGTT GTCAGCCTGC TCGATGCCGA CGGTTTGATC AAAATCCTGA
 251  CGCATACCGT TAAAAATTTC ACCGGTTTCG CGCCGTTGGG AACGGTGTTG
 301  GTTTCTTTAT TGGGCGTGGG GATTGCGGAA AAATCGGGCT TGATTTCCGC
 351  ATTAATGCGC TTATTGCTCA CAAAATCCCC ACGCAAACTC ACTACTTTTA
 401  TGGTTGTTTT TACAGGGATT TTATCCAATA CGGCTTCTGA ATTGGGCTAT
 451  GTCGTCCTAA TCCCTTTGTC CGCCGTCATC TTTCATTCGC TCGGCCGCCA
 501  TCCGCTTGCC GGTTTGGCTG CGGCTTTCGC CGGCGTTTCG GGCGGTTATT
 551  CGGCCAATCT GTTCTTAGGC ACAATCGATC CGCTCTTGGC AGGCATCACC
 601  CAACAGGCGG CGCAAATCAT CCATCCCGAC TACGTCGTAG GCCCTGAAGC
 651  CAACTGGTTT TTTATGGCAG CCAGTACGTT TGTGATTGCT TTGATTGGTT
 701  ATTTTGTTAC TGAAAAAATC GTCGAACCGC AATTGGGCCC TTATCAATCA
 751  GATTTGTCAC AAGAAGAAAA AGACATTCGG CATTCCAATG AAATCACGCC
 801  TTTGGAATAT AAAGGATTAA TTTGGGCAGG CGTGGTGTTT GTTGCCTTAT
 851  CCGCCCTATT GGCTTGGAGC ATCGTCCCTG CCGACGGTAT TTTGCGTCAT
 901  CCTGAAACAG GATTGGTTGC CGGTTCGCCG TTTTTAAAAT CGATTGTTGT
 951  TTTTATTTTC TTGTTGTTTG CGCTGCCGGG CATTGTTTAT GGCCGGATAA
1001  CCCGAAGTTT GCGCGGCGAA CGGGAAGTCG TTAATGCGAT GGCCGAATCG
1051  ATGAGTACTT TGGGACTTTA TTTGGTCATC ATCTTTTTTG CCGCACAGTT
1101  TGTCGCATTT TTTAATTGGA CGAATATTGG GCAATATATT GCCGTTAAAG

1151  GGGCGGTGTT CTTAAAAGAA GTCGGCTTGG GCGGCAGTGT GTTGTTTATC
1201  GGTTTTATTT TAATTTGTGC TTTTATCAAT CTGATGATAG GCTCCGCCTC
1251  CGCGCAATGG GCGGTAACTG CGCCGATTTT CGTCCCTATG CTGATGTTGG
1301  CCGGCTACGC GCCCGAAGTC ATTCAAGCCG CTTACCGCAT CGGTGATTCC
1351  GTTACCAATA TTATTACGCC GATGATGAGT TATTTCGGGC TGATTATGGC
1401  GACGGTAATC AAATACAAAA AAGATGCGGG CGTAGGCACG CTGATTTCTA
1451  TGATGTTGCC GTATTCCGCT TTCTTCTTAA TTGCATGGAT CGCCTTATTC
1501  TGCATTTGGG TATTTGTTTT GGGTCTGCCC GTCGGTCCCG GCACACCCAC
1551  ATTCTATCCG GTGCCTTAA
```

This encodes a protein having amino acid sequence <SEQ ID 140>:

```
  1  MSQTDARRSG RFLRTVEWLG NMLPHPVTLF IIFIVLLLIA SAVGAYFGLS
 51  VPDPRPVGAK GRADDGLIHV VSLLDADGLI KILTHTVKNF TGFAPLGTVL
101  VSLLGVGIAE KSGLISALMR LLLTKSPRKL TTFMVVFTGI LSNTASELGY
151  VVLIPLSAVI FHSLGRHPLA GLAAAFAGVS GGYSANLFLG TIDPLLAGIT
201  QQAAQIIHPD YVVGPEANWF FMAASTFVIA LIGYFVTEKI VEPQLGPYQS
251  DLSQEEKDIR HSNEITPLEY KGLIWAGVVF VALSALLAWS IVPADGILRH
301  PETGLVAGSP FLKSIVVFIF LLFALPGIVY GRITRSLRGE REVVNAMAES
351  MSTLGLYLVI IFFAAQFVAF FNWTNIGQYI AVKGAVFLKK FRLGGSVLFI
401  GFILICAFIN LMIGSASAQW AVTAPIFVPM LMLAGNAPQV IQAAYRIGDS
451  VTNIITPMMS YFGLIMATVI KYKKDAGVGT LISMMLPYSA FFLIAWIALF
501  CIWVFVLGLP VGPGTPTFYP VP*
```

ORF12ng shows 97.1% identity in 522 aa overlap with ORF12-1:

```
                      10        20        30        40        50        60
orf12-1.pep   MSQTDTQRDGRFLRTVEWLGNMLPHPVTLFIIFIVLLLIASAVGAYFGLSVPDPRPVGAK
              |||||::|:|||||||||||||||||||||||||||||||||||||||||||||||||||
orf12ng       MSQTDARRSGRFLRTVEWLGNMLPHPVTLFIIFIVLLLIASAVGAYFGLSVPDPRPVGAK
                      10        20        30        40        50        60

                      70        80        90       100       110       120
orf12-1.pep   GRADDGLIYIVSLLNADGFIKILTHTVKNFTGFAPLGTVLVSLLGVGIAEKSGLISALMR
              ||||||||::||||:|||:|||:|||||||||||||||||||||||||||||||||||||
orf12ng       GRADDGLIHVVSLLDADGLIKILTHTVKNFTGFAPLGTVLVSLLGVGIAEKSGLISALMR
                      70        80        90       100       110       120

                     130       140       150       160       170       180
orf12-1.pep   LLLTKSPRKLTTFMVVFTGILSNTASELGYVVLIPLSAIIFHSLGRHPLAGLAAAFAGVS
              |||||||||||||||||||||||||||||||||||||:||||||||||||||||||||||
orf12ng       LLLTKSPRKLTTFMVVFTGILSNTASELGYVVLIPLSAVIFHSLGRHPLAGLAAAFAGVS
                     130       140       150       160       170       180

                     190       200       210       220       230       240
orf12-1.pep   GGYSANLFLGTIDPLLAGITQQAAQIIHPDYVVGPEANWFFMVASTFVIALIGYFVTEKI
              ||||||||||||||||||||||||||||||||||||||||||:|||||||||||||||||
orf12ng       GGYSANLFLGTIDPLLAGITQQAAQIIHPDYVVGPEANWFFMAASTFVIALIGYFVTEKI
                     190       200       210       220       230       240

                     250       260       270       280       290       300
orf12-1.pep   VEPQLGPYQSDLSQEEKDIRHSNEITPLEYKGLIWAGVVFVALSALLAWSIVPADGILRH
              ||||||||||||||||||||||||||||||||||||||||||||||||||||||||||||
orf12ng       VEPQLGPYQSDLSQEEKDIRHSNEITPLEYKGLIWAGVVFVALSALLAWSIVPADGILRH
                     250       260       270       280       290       300

                     310       320       330       340       350       360
orf12-1.pep   PETGLVSGSPFLKSIVVFIFLLFALPGIVYGRVTRSLRGEQEVVNAMAESMSTLGLYLVI
              ||||||:||||||||||||||||||||||||||:|||||||:||||||||||||||||||
orf12ng       PETGLVAGSPFLKSIVVFIFLLFALPGIVYGRITRSLRGEREVVNAMAESMSTLGLYLVI
                     310       320       330       340       350       360

                     370       380       390       400       410       420
orf12-1.pep   IFFAAQFVAFFNWTNIGQYIAVKGATFLKEVGLGGSVLFIGFILICAFINLMIGSASAQW
              |||||||||||||||||||||||||:||||||||||||||||||||||||||||||||||
orf12ng       IFFAAQFVAFFNWTNIGQYIAVKGAVFLKEVGLGGSVLFIGFILICAFINLMIGSASAQW
                     370       380       390       400       410       420

                     430       440       450       460       470       480
orf12-1.pep   AVTAPIFVPMLMLAGYAPEVIQAAYRIGDSVTNIITPMMSYFGLIMATVIKYKKDAGVGT
              ||||||||||||||||||||||||||||||||||||||||||||||||||||||||||||
```

```
orf12ng         AVTAPIFVPMLMLAGYAPEVIQAAYRIGDSVTNIITPMMSYFGLIMATVIKYKKDAGVGT
                        430       440       450       460       470       480

                        490       500       510       520
orf12-1.pep     LISMMLPYSAFFLIAWIALFCIWVFVLGLPVGPGAPTFYPAPX
                |||||||||||||||||||||||||||||||||||:|||||:||
orf12ng         LISMMLPYSAFFLIAWIALFCIWVFVLGLPVGPGTPTFYPVPX
                        490       500       510       520
```

In addition, ORF12ng shows significant homology with a hypotehtical protein from *E.coli:*

```
sp|P46133|YDAH_ECOLI HYPOTHETICAL 55.1 KD PROTEIN IN OGT-DBPA INTERGENIC REGION
>gi|1787597 (AE000231) hypothetical protein in ogt 5'region [Escherichia coli]
Length = 510
 Score =  329 bits (835), Expect = 2e-89
 Identities = 178/507 (35%), Positives = 281/507 (55%), Gaps = 15/507 (2%)

Query: 8   RSGRFLRTVEWLGNMLPHPVTXXXXXXXXXXXASAVGAYFGLSVPDPRPVGAKGRADDGL 67
            +SG+    VE +GN +PHP              +A+ + FG+S  +P         D
Sbjct: 13  QSGKLYGWVERIGNKVPHPFLLFIYLIIVLMVTTAILSAFGVSAKNP--------TDGTP 64

Query: 68  IHVVSLLDADGLIKILTHTVKNFTGFAPXXXXXXXXXXXXIAEKSGLISALMRLLLTKSP 127
            + V +LL  +GL   L + +KNF+GFAP             +AE+ GL+ ALM  + +
Sbjct: 65  VVVKNLLSVEGLHWFLPNVIKNFSGFAPLGAILALVLGAGLAERVGLLPALMVKMASHVN 124

Query: 128 RKLTTFMVVFTGILSNTASELGYVVLIPLSAVIFHSLGRHPLAGLAAAFAGVSGGYSANL 187
             +  ++MV+F    S+ +S+   V++ P+ A+IF ++GRHP+AGL AA AGV  G++ANL
Sbjct: 125 ARYASYMVLFIAFFSHISSDAALVIMPPMGALIFLAVGRHPVAGLLAAIAGVGCGFTANL 184

Query: 188 FLGTIDPLLAGITQQAAQIIHPDYVVGPEANWFFMAASTFVIALIGYFVTEKIVEPQLGP 247
             + T D LL+GI+ +AA    +P    V    NW+FMA+S  V+ ++G  +T+KI+EP+LG
Sbjct: 185 LIVTTDVLLSGISTEAAAAFNPQMHVSVIDNWYFMASSVVVLTIVGGLITDKIIEPRLGQ 244

Query: 248 YQSDLSQEEKDIRHSNEITPLEYKGLIWAGVVFVALSALLAWSIVPADGILRHPETGLVA 307
            +Q +   ++ + +   S          GL  AGVV +    A +A  ++P +GILR P    V
Sbjct: 245 WQGNSDEKLQTLTESQRF------GLRIAGVVSLLFIAAIALMVIPQNGILRDPINHTVM 298

Query: 308 GSPFLKSIVVFIFLLFALPGIVYGRITRSLRGEREVVNAMAESMSTLGLYLXXXXXXXXX 367
             SPF+K IV  I L F +   + YG  TR++R + ++ + M E M   +  ++
Sbjct: 299 PSPFIKGIVPLIILFFFVVSLAYGIATRTIRRQADLPHLMIEPMKEMAGFIVMVFPLAQF 358

Query: 368 XXXXNWTNIGQYIAVKGAVFLKEVGLGGSVLFIGFILICAFINLMIGSASAQWAVTAPIF 427
                NW+N+G++IAV     L+  GL G   F+G  L+ +F+ + I S SA W++ APIF
Sbjct: 359 VAMFNWSNMGKFIAVGLTDILESSGLSGIPAFVGLALLSSFLCMFIASGSAIWSILAPIF 418

Query: 428 VPMLMLAGYAPEVIQAAYRIGDSVTNIITPMMSYFGLIMATVIKYKKDAGVGTLISMMLP 487
            VPM ML G+ P   Q  +RI DS    + P+   +   L +  + +YK DA +GT   S++LP
Sbjct: 419 VPMFMLLGFHPAFAQILFRIADSSVLPLAPVSPFVPLFLGFLQRYKPDAKLGTYYSLVLP 478

Query: 488 YSAFFLIAWIALFCIWVFVLGLPVGPG 514
            Y   FL+ W+ +   W +++GLP+GPG
Sbjct: 479 YPLIFLVVWLLMLLAW-YLVGLPIGPG 504
```

Based on this analysis, including the presence of several putative transmembrane domains and the predicted actinin-type actin-binding domain signature (shown in bold) in the gonococcal protein, it is predicted that the proteins from *N.meningitidis* and *N.gonorrhoeae,* and their epitopes, could be useful antigens for vaccines or diagnostics, or for raising antibodies.

**Example 17**

The following partial DNA sequence was identified in *N.meningitidis* <SEQ ID 141>:

```
  1  ..ACAGCCGGCG CAGCAGGTTn CnCGGTCTTC GTTTTCGTAA CGGACAGTCA
 51    GGTGGAGGTG TTCGGGAACA TCCAGACCGC AGTGGAAACA GGTTTTTTTC
101    ATGGCATTTC GGTTTCGTCT GTGTTTGGTG CGGCGGCACA AGACTCGGCA

151    ATgGCTTCGC GCAGTGCGTC TATACCGGTA TTTTCAGCAA CGGAAATGCG
201    GACGGcGgCA ATTTTTCCCG CAGCGTCGCG CCATATGCCC GTGTTTTgTT
251    CTTCAGACGG CAGCAGGTCG GTTTTGTTGT ACACCTTgAT GCACGGAaTA
301    TCGCCGGCAT GGATTTCTTG CAGTACGTTT TCCACGTCTT CAATCTGCTG
351    TCCGCTGTTC GGAGCGGCGG CATCGACGAC GTGCAGCAGC ACATCgGcTT
401    gCGCGGTTTC TTCCAGCGTG GCgGAAAAGG CGGAAATCAG TTTgTGCGGC
451    agATyGCTnA CGAATCCGAC GGTATCGGTC AGGATAATGC TGCATTCGGG
501    ACT..
```

This corresponds to the amino acid sequence <SEQ ID 142; ORF14>:

```
  1  ..TAGAAGXXVF VFVTDSQVEV FGNIQTAVET GFFHGISVSS VFGAAAQDSA
 51    MASRSASIPV FSATEMRTAA IFPAASRHMP VFCSSDGSRS VLLYTLMHGI
101    SPAWISCSTF STSSICCPLF GAAASTTCSS TSACAVSSSV AEKAEISLCG
151    RXLTNPTVSV RIMLHSG..
```

Computer analysis of this amino acid sequence gave the following results:

<u>Homology with a predicted ORF from *N.meningitidis* (strain A)</u>

CRT14 shows 94.0% identity over a 167aa overlap with an ORF (ORF14a) from strain A of *N. meningitidis:*

```
                                                    10        20        30
orf14.pep                                 TAGAAGXXVFVFVTDSQVEVFGNIQTAVET
                                          |:||||  |||||||:|::||||:| ||||
orf14a        GRQLGFLRVGGALFVITAQARVNNALCDCLTTGAAGFAVFVFVTDGQMQVFGNVQPAVET
                150       160       170       180       190       200

                      40        50        60        70        80        90
orf14.pep     GFFHGISVSSVFGAAAQDSAMASRSASIPVFSATEMRTAAIFPAASRHMPVFCSSDGSRS
              ||||||||||||||||| ||||||||||||||||||||||||||||||||||||||||||
orf14a        GFFHGISVSSVFGAAAQYSAMASRSASIPVFSATEMRTAAIFPAASRHMPVFCSSDGSRS
                210       220       230       240       250       260

                     100       110       120       130       140       150
orf14.pep     VLLYTLMHGISPAWISCSTFSTSSICCPLFGAAASTTCSSTSACAVSSSVAEKAEISLCG
              ||||||||||||||||||||||||||||||||||||||||||||||||||||||||||||
orf14a        VLLYTLMHGISPAWISCSTFSTSSICCPLFGAAASTTCSSTSACAVSSSVAEKAEISLCG
                270       280       290       300       310       320

                     160
orf14.pep     RXLTNPTVSVRIMLHSG
              | |||||||||||||||
orf14a        RSLTNPTVSVRIMLHSGLMYSRRAVVSSVAKSWSFAYMPDLVSRLNRLDLPTLVX
                330       340       350       360       370       380
```

The complete length ORF14a nucleotide sequence <SEQ ID 143> is:

```
   1 ATGGAGGATT TGCAGGAAAT CGGGTTCGAT GTCGCCGCCG TAAAGGTAGG
  51 TCGGCAGCGC GAACATCATC GTCTGCATCA TCCCCAGCCC GGCAACGGCG
 101 AGGCGGACGA TGTATTGTTT GCGTTCTTTT TGGTTGGCGG CTTCGATTTT
 151 TTGCGCGTCA TAGGGTGCGG CGGTGTAGCC TATCTGCCTG ATTTTCAACA
 201 GAATGTCGGA AAGGCGGATT TTGCCGTCGT CCCAGACGAC GCGGCAGCGG
 251 TGCGTGCTGT AATTGAGGTC GATGCGGACG ATGCCGTCTG TACGCAAAAG
 301 CTGCTGTTCG ATCAGCCAGA CGCAGGCGGC GCAGGTGATG CCGCCGAGCA
 351 TTAAAACCGC CTCGCGCGTG CCGCCGTGGG TTTCCACAAA GTCGGACTGG
 401 ACTTCGGGCA GGTCGTACAG GCGGATTTGG TCGAGGATTT CTTGGGGCGG
 451 CAGCTCGGTT TTTTGCGCGT CGGCGGTGCG TTGTTTGTAA TAACTGCCCA
 501 AGCCCGCGTC AATAATGCTT TGTGCGACTG CCTGACAACC GGCGCAGCAG
 551 GTTTCGCGGT CTTCGTTTTC GTAACGGACG GTCAGATGCA GGTTTTCGGG
 601 AACGTCCAGC CCGCAGTGGA AACAGGTTTT TTTCATGGCA TTTCGGTTTC
 651 GTCTGTGTTT GGTGCGGCGG CACAATACTC GGCAATGGCT TCGCGCAGTG
 701 CGTCTATACC GGTATTTTCA GCAACGGAAA TGCGGACGGC GGCAATTTTT
 751 CCCGCAGCGT CGCGCCATAT GCCCGTGTTT TGTTCTTCAG ACGGCAGCAG
 801 GTCGGTTTTG TTGTACACCT TGATGCACGG AATATCGCCG GCATGGATTT
 851 CTTGCAGTAC GTTTTCCACG TCTTCAATCT GCTGTCCGCT GTTCGGAGCG
 901 GCGGCATCGA CGACGTGCAG CAGCACATCG GCTTGCGCGG TTTCTTCCAG
 951 CGTGGCGGAA AAGGCGGAAA TCAGTTTGTG CGGCAGATCG CTGACGAATC
1001 CGACGGTATC GGTCAGGATA ATGCTGCATT CGGGACTGAT GTACAGCCGC
```

```
1051 CGCGCCGTCG TGTCGAGTGT GGCGAAAAGC TGGTCTTTCG CATATATGCC
1101 CGACTTGGTC AGCCGGTTGA ACAGACTGGA TTTGCCGACA TTGGTATAG
```

This encodes a protein having amino acid sequence <SEQ ID 144>:

```
  1 MEDLQEIGFD VAAVKVGRQR EHHRLHHPQP-GNGEADDVLF AFFLVGGFDF
 51 LRVIGCGGVA YLPDFQQNVG KADFAVVPDD AAAVRAVIEV DADDAVCTQK
101 LLFDQPDAGG AGDAAEH*NR LARAAVGFHK VGLDFGQVVQ ADLVEDFLGR
151 QLGFLRVGGA LFVITAQARV NNALCDCLTT GAAGFAVFVF VTDGQMQVFG
201 NVQPAVETGF FHGISVSSVF GAAAQYSAMA SRSASIPVFS ATEMRTAAIF
251 PAASRHMPVF CSSDGSRSVL LYTLMHGISP AWISCSTFST SSICCPLFGA
301 AASTTCSSTS ACAVSSSVAE KAEISLCGRS LTNPTVSVRI MLHSGLMYSR
351 RAVVSSVAKS WSFAYMPDLV SRLNRLDLPT LV*
```

It should be noted that this sequence includes a stop codon at position 118.

Homology with a predicted ORF from *N.gonorrhoeae*

ORF14 shows 89.8% identity over a 167aa overlap with a predicted ORF (ORF14.ng) from *N. gonorrhoeae:*

```
orf14.pep                                   TAGAAGXXVFVFVTDSQVEVFGNIQTAVET   30
                                            || |||  ||:||:|:|::||||:| ||||
orf14ng     GRQFGFFRVGGASFVITAQAGIDDALCDCLTADAAGFAVFAFVADGQMQVFGNVQPAVET  208

orf14.pep   GFFHGISVSSVFGAAAQDSAMASRSASIPVFSATEMRTAAIFPAASRHMPVFCSSDGSRS   90
            ||||||||||||||||| ||||||||||||||||||||||||||||||||||||||||||
orf14ng     GFFHGISVSSVFGAAAQYSAMASRSASIPVFSATEMRTAAIFPAASRHMPVFCSSDGSRS  268

orf14.pep   VLLYTLMHGISPAWISCSTFSTSSICCPLFGAAASTTCSSTSACAVSSSVAEKAEISLCG  150
            ||||||||||| |||||||||||||||||| |||||||||||||:|||:|||||||||||
orf14ng     VLLYTLMHGISWAWISCSTFSTSSICCPLFRAAASTTCSSTSACTVSSKVAEKAEISLCG  328

orf14.pep   RXLTNPTVSVRIMLHSG                                         167
            | |||||||||||||:|
orf14ng     RSLTNPTVSVRIMLHAGLMYSRRAVVSRVAKSWSFAYMPDLVSRLNRLDLPTLV    382
```

The complete length ORF14ng nucleotide sequence <SEQ ID 145> is predicted to encode a protein having amino acid sequence <SEQ ID 146>:

```
  1 MEDLQEIGFD VAAVKVGRQR EHHRLHHTQS GNGKADDVLF AFFLVGGFDF
 51 LRVIGCGGVA CLPDFQQNVG EADFAVVPDD AAAVRAVIEV DADDAVCAQK
101 LLFDQPDAGG AGNAAEHQHC FVRAIMGFHK VGLDFGQVVQ ADLVEDFLGR
151 QFGFFRVGGA SFVITAQAGI DDALCDCLTA DAAGFAVFAF VADGQMQVFG
201 NVQPAVETGF FHGISVSSVF GAAAQYSAMA SRSASIPVFS ATEMRTAAIF
251 PAASRHMPVF CSSDGSRSVL LYTLMHGISW AWISCSTFST SSICCPLFRA
301 AASTTCSSTS ACTVSSKVAE KAEISLCGRS LTNPTVSVRI MLHAGLMYSR
351 RAVVSRVAKS WSFAYMPDLV SRLNRLDLPT LV*
```

Based on the putative transmembrane domain in the gonococcal protein, it is predicted that the proteins from *N.meningitidis* and *N.gonorrhoeae,* and their epitopes, could be useful antigens for vaccines or diagnostics, or for raising antibodies.

**Example 18**

The following partial DNA sequence was identified in *N.meningitidis* <SEQ ID 147>:

```
  1 ..GGCCATTACT CCGACCGCAC TTGGAAGCCG CGTTTGGNCG GCCGCCGTCT
 51   GCCGTATCTG CTTTATGGCA CGCTGATTGC GGTTATTGTG ATGATTTTGA
101   TGCCGAACTC GGGCAGCTTC GGTTTCGGCT ATGCGTCGCT GGCGGCTTTG
151   TCGTTCGGCG CGCTGATGAT TGCGCTGTTA GACGTGTCGT CAAATATGGC
201   GATGCAGCCG TTTAAGATGA TGGTCGGCGA CATGGTCAAC GAGGAGCAGA
251   AAA.NTACGC CTACGGGATT CAAAGTTTCT TAGCAAATAC GGGCGCGGTC
301   GTGGCGGCGA TTCTGCCGTT TGTGTTTGCG TATATCGGTT TGGCGAACAC
351   CGCCGANAAA GGCGTTGTGC CGCAGACCGT GGTCGTGGCG TTTTATGTGG
401   GTGCGGCGTT GCTGGTGATT ACCAGCGCGT TCACGATTTT CAAAGTGAAG
451   GAATACGANC CGGAAACCTA CGCCCGTTAC CACGGCATCG ATGTCGCCGC
501   GAATCAGGAA AAAGCCAACT GGATCGCACT CTTAAAA.CC GCGC..
```

This corresponds to the amino acid sequence <SEQ ID 148; ORF16>:

```
  1 ..GHYSDRTWKP RLXGRRLPYL LYGTLIAVIV MILMPNSGSF GFGYASLAAL
 51   SFGALMIALL DVSSNMAMQP FKMMVGDMVN EEQKXYAYGI QSFLANTGAV
101   VAAILPFVFA YIGLANTAXK GVVPQTVVVA FYVGAALLVI TSAFTIFKVK
151   EYXPETYARY HGIDVAANQE KANWIALLKX A..
```

Further work revealed the complete nucleotide sequence <SEQ ID 149>:

```
   1  ATGTCGGAAT ATACGCCTCA AACAGCAAAA CAAGGTTTGC CCGCGCTGGC
  51  AAAAAGCACG ATTTGGATGC TCAGTTTCGG CTTTCTCGGC GTTCAGACGG
 101  CCTTTACCCT GCAAAGCTCG CAAATGAGCC GCATTTTTCA AACGCTAGGC
 151  GCAGACCCGC ACAATTTGGG CTGGTTTTTC ATCCTGCCGC CGCTGGCGGG
 201  GATGCTGGTG CAGCCGATTG TCGGCCATTA CTCCGACCGC ACTTGGAAGC
 251  CGCGTTTGGG CGGCCGCCGT CTGCCGTATC TGCTTTATGG CACGCTGATT
 301  GCGGTTATTG TGATGATTTT GATGCCGAAC TCGGGCAGCT TCGGTTTCGG
 351  CTATGCGTCG CTGGCGGCTT TGTCGTTCGG CGCGCTGATG ATTGCGCTGT
 401  TAGACGTGTC GTCAAATATG GCGATGCAGC CGTTTAAGAT GATGGTCGGC
 451  GACATGGTCA ACGAGGAGCA GAAAGGCTAC GCCTACGGGA TTCAAAGTTT
 501  CTTAGCAAAT ACGGGCGCGG TCGTGGCGGC GATTCTGCCG TTTGTGTTTG
 551  CGTATATCGG TTTGGCGAAC ACCGCCGAGA AAGGCGTTGT GCCGCAGACC
 601  GTGGTCGTGG CGTTTTATGT GGGTGCGGCG TTGCTGGTGA TTACCAGCGC
 651  GTTCACGATT TTCAAAGTGA AGGAATACGA TCCGGAAACC TACGCCCGTT
 701  ACCACGGCAT CGATGTCGCC GCGAATCAGG AAAAAGCCAA CTGGATCGAA
 751  CTCTTGAAAA CCGCGCCTAA GGCGTTTTGG ACGGTTACTT TGGTGCAATT
 801  CTTCTGCTGG TTCGCCTTCC AATATATGTG GACTTACTCG GCAGGCGCGA
 851  TTGCGGAAAA CGTCTGGCAC ACCACCGATG CGTCTTCCGT AGGTTATCAG
 901  GAGGCGGGTA ACTGGTACGG CGTTTTGGCG GCGGTGCAGT CGGTTGCGGC
 951  GGTGATTTGT TCGTTTGTAT TGGCGAAAGT GCCGAATAAA TACCATAAGG
1001  CGGGTTATTT CGGCTGTTTG GCTTTGGGCG CGCTCGGCTT TTTCTCCGTT
1051  TTCTTCATCG GCAACCAATA CGCGCTGGTG TTGTCTTATA CCTTAATCGG
1101  CATCGCTTGG GCGGGCATTA TCACTTATCC GCTGACGATT GTGACCAACG
1151  CCTTGTCGGG CAAGCATATG GGCACTTACT TGGGCTTGTT TAACGGCTCT
1201  ATCTGTATGC CTCAAATCGT CGCTTCGCTG TTGAGTTTCG TGCTTTTCCC
1251  TATGCTGGGC GGCTTGCAGG CCACTATGTT CTTGGTAGGG GGCGTCGTCC
1301  TGCTGCTGGG CGCGTTTTCC GTGTTCCTGA TTAAAGAAAC ACACGGCGGG
1351  GTTTGA
```

This corresponds to the amino acid sequence <SEQ ID 150; ORF16-1>:

```
   1  MSEYTPQTAK QGLPALAKST IWMLSFGFLG VQTAFTLQSS QMSRIFQTLG
  51  ADPHNLGWFF ILPPLAGMLV QPIVGHYSDR TWKPRLGGRR LPYLLYGTLI
 101  AVIVMILMPN SGSFGFGYAS LAALSFGALM IALLDVSSNM AMQPFKMMVG
 151  DMVNEEQKGY AYGIQSFLAN TGAVVAAILP FVFAYIGLAN TAEKGVVPQT
 201  VVVAFYVGAA LLVITSAFTI FKVKEYDPET YARYHGIDVA ANQEKANWIE
 251  LLKTAPKAFW TVTLVQFFCW FAFQYMWTYS AGAIAENVWH TTDASSVGYQ
 301  EAGNWYGVLA AVQSVAAVIC SFVLAKVPNK YHKAGYFGCL ALGALGFFSV
 351  FFIGNQYALV LSYTLIGIAW AGIITYPLTI VTNALSGKHM GTYLGLFNGS
 401  ICMPQIVASL LSFVLFPMLG GLQATMFLVG GVVLLLGAFS VFLIKETHGG
 451  V*
```

Computer analysis of this amino acid sequence gave the following results:

Homology with a predicted ORF from *N. meningitidis* (strain A)

ORF16 shows 96.7% identity over a 181aa overlap with an ORF (ORF 16a) from strain A of *N. meningitidis:*

```
                                                    10        20        30
orf16.pep                                   GHYSDRTWKPRLXGRRLPYLLYGTLIAVIV
                                            |||||||||||| |||||||||||||||||
orf16a       IFQTLGADPHSLGWFFILPPLAGMLVQPIVGHYSDRTWKPRLGGRRLPYLLYGTLIAVIV
                    50        60        70        80        90       100
```

```
                     40        50        60        70        80        90
orf16.pep   MILMPNSGSFGFGYASLAALSFGALMIALLDVSSNMAMQPFKMMVGDMVNEEQKXYAYGI
            |||||||||||||||||||||||||||||||||||||||||||||||||||||| |||||
orf16a      MILMPNSGSFGFGYASLAALSFGALMIALLDVSSNMAMQPFKMMVGDMVNEEQKGYAYGI
                 110       120       130       140       150       160

                    100       110       120       130       140       150
orf16.pep   QSFLANTGAVVAAILPFVFAYIGLANTAXKGVVPQTVVVAFYVGAALLVITSAFTIFKVK
            |||||||||||||||||||||||||||| |||||||||||||||||||||||||||||||
orf16a      QSFLANTGAVVAAILPFVFAYIGLANTAEKGVVPQTVVVAFYVGAALLVITSAFTIFKVK
                 170       180       190       200       210       220

                    160       170       180
orf16.pep   EYXPETYARYHGIDVAANQEKANWIALLKXA
            || |||||||||||||||||||||| |||:|
orf16a      EYNPETYARYHGIDVAANQEKANWIELLKTAPKAFWTVTLVQFFCWFAFQYMWTYSAGAI
                 230       240       250       260       270       280

orf16a      AENVWHTTDASSVGYQEAGNWYGVLAAVQSVAAVICSFVLAKVPNKYHKAGYFGCLALGA
                 290       300       310       320       330       340
```

The complete length ORF16a nucleotide sequence <SEQ ID 151> is:

```
   1  ATGTCGGAAT ATACGCCTCA AACAGCAAAA CAAGGTTTGC CCGCGCTGGC
  51  AAAAAGCACG ATTTGGATGC TCAGTTTCGG CTTTCTCGGC GTTCAGACGG
 101  CCTTTACCCT GCAAAGCTCG CAGATGAGCC GCATCTTCCA GACGCTCGGT
 151  GCCGATCCGC ACAGCCTCGG CTGGTTCTTT ATCCTGCCGC CGCTGGCGGG
 201  GATGCTGGTG CAGCCGATTG TCGGCCATTA CTCCGACCGC ACTTGGAAGC
 251  CGCGTTTGGG CGGCCGCCGT CTGCCGTATC TGCTTTATGG CACGCTGATT
 301  GCGGTTATTG TGATGATTTT GATGCCGAAC TCGGGCAGCT TCGGTTTCGG
 351  CTATGCGTCG CTGGCGGCTT TGTCGTTCGG CGCGCTGATG ATTGCGCTGT
 401  TAGACGTGTC GTCAAATATG GCGATGCAGC CGTTTAAGAT GATGGTCGGC
 451  GACATGGTCA ACGAGGAGCA GAAAGGCTAC GCCTACGGGA TTCAAAGTTT
 501  CTTAGCGAAT ACGGGCGCGG TCGTGGCGGC GATTCTGCCG TTTGTGTTTG
 551  CGTATATCGG TTTGGCGAAC ACCGCCGAGA AAGGCGTTGT GCCGCAGACC
 601  GTGGTCGTGG CGTTTTATGT GGGTGCGGCG TTGCTGGTGA TTACCAGCGC
 651  GTTCACGATT TTCAAAGTGA AGGAATACAA TCCGGAAACC TACGCCCGTT
 701  ACCACGGCAT CGATGTCGCC GCGAATCAGG AAAAAGCCAA CTGGATCGAA
 751  CTCTTGAAAA CCGCGCCTAA GGCGTTTTGG ACGGTTACTT TGGTGCAATT
 801  CTTCTGCTGG TTCGCCTTCC AATATATGTG GACTTACTCG GCAGGCGCGA
 851  TTGCGGAAAA CGTCTGGCAC ACCACCGATG CGTCTTCCGT AGGTTATCAG
 901  GAGGCGGGTA ACTGGTACGG CGTTTTGGCG GCGGTGCAGT CGGTTGCGGC
 951  GGTGATTTGT TCGTTTGTAT TGGCGAAAGT GCCGAATAAA TACCATAAGG
1001  CGGGTTATTT CGGCTGTTTG GCTTTGGGCG CGCTCGGCTT TTTCTCCGTT
1051  TTCTTCATCG GCAACCAATA CGCGCTGGTG TTGTCTTATA CCTTAATCGG
1101  CATCGCTTGG GCGGGCATTA TCACTTATCC GCTGACGATT GTGACCAACG
1151  CCTTGTCGGG CAAGCATATG GGCACTTACT TGGGCCTGTT TAACGGCTCT
1201  ATCTGTATGC CGCAAATCGT CGCTTCGCTG TTGAGTTTCG TGCTTTTCCC
1251  TATGCTGGGC GGCTTGCAGG CCACTATGTT CTTGGTAGGG GGCGTCGTCC
1301  TGCTGCTGGG CGCGTTTTCC GTGTTCCTGA TTAAAGAAAC ACACGGCGGG
1351  GTTTGA
```

This encodes a protein having amino acid sequence <SEQ ID 152>:

```
   1  MSEYTPQTAK QGLPALAKST IWMLSFGFLG VQTAFTLQSS QMSRIFQTLG
  51  ADPHSLGWFF ILPPLAGMLV QPIVGHYSDR TWKPRLGGRR LPYLLYGTLI
 101  AVIVMILMPN SGSFGFGYAS LAALSFGALM IALLDVSSNM AMQPFKMMVG
 151  DMVNEEQKGY AYGIQSFLAN TGAVVAAILP FVFAYIGLAN TAEKGVVPQT
 201  VVVAFYVGAA LLVITSAFTI FKVKEYNPET YARYHGIDVA ANQEKANWIE
 251  LLKTAPKAFW TVTLVQFFCW FAFQYMWTYS AGAIAENVWH TTDASSVGYQ
 301  EAGNWYGVLA AVQSVAAVIC SFVLAKVPNK YHKAGYFGCL ALGALGFFSV
 351  FFIGNQYALV LSYTLIGIAW AGIITYPLTI VTNALSGKHM GTYLGLFNGS
 401  ICMPQIVASL LSFVLFPMLG GLQATMFLVG GVVLLLGAFS VFLIKETHGG
 451  V*
```

ORF16a and ORF16-1 show 99.6% identity in 451 aa overlap:

```
                       10        20        30        40        50        60
orf16a.pep     MSEYTPQTAKQGLPALAKSTIWMLSFGFLGVQTAFTLQSSQMSRIFQTLGADPHSLGWFF
               ||||||||||||||||||||||||||||||||||||||||||||||||||||||:|||||
orf16-1        MSEYTPQTAKQGLPALAKSTIWMLSFGFLGVQTAFTLQSSQMSRIFQTLGADPHNLGWFF


                       10        20        30        40        50        60

                       70        80        90       100       110       120
orf16a.pep     ILPPLAGMLVQPIVGHYSDRTWKPRLGGRRLPYLLYGTLIAVIVMILMPNSGSFGFGYAS
               ||||||||||||||||||||||||||||||||||||||||||||||||||||||||||||
orf16-1        ILPPLAGMLVQPIVGHYSDRTWKPRLGGRRLPYLLYGTLIAVIVMILMPNSGSFGFGYAS
                       70        80        90       100       110       120

                      130       140       150       160       170       180
orf16a.pep     LAALSFGALMIALLDVSSNMAMQPFKMMVGDMVNEEQKGYAYGIQSFLANTGAVVAAILP
               ||||||||||||||||||||||||||||||||||||||||||||||||||||||||||||
orf16-1        LAALSFGALMIALLDVSSNMAMQPFKMMVGDMVNEEQKGYAYGIQSFLANTGAVVAAILP
                      130       140       150       160       170       180

                      190       200       210       220       230       240
orf16a.pep     FVFAYIGLANTAEKGVVPQTVVVAFYVGAALLVITSAFTIFKVKEYNPETYARYHGIDVA
               ||||||||||||||||||||||||||||||||||||||||||||||:|||||||||||||
orf16-1        FVFAYIGLANTAEKGVVPQTVVVAFYVGAALLVITSAFTIFKVKEYDPETYARYHGIDVA
                      190       200       210       220       230       240

                      250       260       270       280       290       300
orf16a.pep     ANQEKANWIELLKTAPKAFWTVTLVQFFCWFAFQYMWTYSAGAIAENVWHTTDASSVGYQ
               ||||||||||||||||||||||||||||||||||||||||||||||||||||||||||||
orf16-1        ANQEKANWIELLKTAPKAFWTVTLVQFFCWFAFQYMWTYSAGAIAENVWHTTDASSVGYQ
                      250       260       270       280       290       300

                      310       320       330       340       350       360
orf16a.pep     EAGNWYGVLAAVQSVAAVICSFVLAKVPNKYHKAGYFGCLALGALGFFSVFFIGNQYALV
               ||||||||||||||||||||||||||||||||||||||||||||||||||||||||||||
orf16-1        EAGNWYGVLAAVQSVAAVICSFVLAKVPNKYHKAGYFGCLALGALGFFSVFFIGNQYALV
                      310       320       330       340       350       360

                      370       380       390       400       410       420
orf16a.pep     LSYTLIGIAWAGIITYPLTIVTNALSGKHMGTYLGLFNGSICMPQIVASLLSFVLFPMLG
               ||||||||||||||||||||||||||||||||||||||||||||||||||||||||||||
orf16-1        LSYTLIGIAWAGIITYPLTIVTNALSGKHMGTYLGLFNGSICMPQIVASLLSFVLFPMLG
                      370       380       390       400       410       420

                      430       440       450
orf16a.pep     GLQATMFLVGGVVLLLGAFSVFLIKETHGGVX
               ||||||||||||||||||||||||||||||||
orf16-1        GLQATMFLVGGVVLLLGAFSVFLIKETHGGVX
                      430       440       450
```

<u>Homology with a predicted ORF from *N.gonorrhoeae*</u>

ORF16 shows 93.9% identity over a 181aa overlap with a predicted ORF (ORF16.ng) from *N. gonorrhoeae:*

```
orf16.pep                                  GHYSDRTWKPRLXGRRLPYLLYGTLIAVIV   30
                                           |:|||||||||| ||||||||||||||||
orf16ng    HFSNARRRPAQFGLVFHPAAAGGDAGSADSGYYSDRTWKPRLGGRRLPYLLYGTLIAVIV  131

orf16.pep  MILMPNSGSFGFGYASLAALSFGALMIALLDVSSNMAMQPFKMMVGDMVNEEQKXYAYGI   90
           |||||||||||||||||||||||||||||||||||||||||||||||||||||| |||||
orf16ng    MILMPNSGSFGFGYASLAALSFGALMIALLDVSSNMAMQPFKMMVGDMVNEEQKSYAYGI  191

orf16.pep  QSFLANTGAVVAAILPFVFAYIGLANTAXKGVVPQTVVVAFYVGAALLVITSAFTIFKVK  150
           ||||||| ||||||||||||||||||||| ||||||||||||||||||||:||||||| |||
orf16ng    QSFLANTDAVVAAILPFVFAYIGLANTAEKGVVPQTVVVAFYVGAALLIITSAFTISKVK  251

orf16.pep  EYXPETYARYHGIDVAANQEKANWIALLKXA                               181
           || |||||||||||||||||||||: |||:|
orf16ng    EYDPETYARYHGIDVAANQEKANWFELLKTAPKVFWTVTPVQFFCWFAFRYMWTYSAGAI  311
```

The complete length ORF16ng nucleotide sequence <SEQ ID 153> is:

```
   1  ATGATAGGGG ATCGCCGCGC CGGCAACCAT TTCGGATTTT CCAAAGCAAA
  51  TACTTTTCAA ATCAAAAAAA AGGATTTACT TTATGTCGGA ATATACGCCT
 101  CAAACAGCAA AACAAGGTTT GCCCGCGCCG GCAAAAAGCA CGATTTGGAT
 151  GTTGAGCTTC GGCTATCTCG GCGTTCAGAC GGCCTTTACC CTGCAAAGCT
 201  CGCAGATGAG CCGCATTTTT CAAACGCTAG GCGCAGACCC GCACAATTTG
 251  GGCTGGTTTT TCATCCTGCC GCCGCTGGCG GGGATGCTGG TTCAGCCGAT
 301  AGTGGCTACT ACTCAGACCG CACTTGGAAG CCGCGCTTGG GCGGCCGCCG
 351  CCTGCCGTAT CTGCTTTACG GCACGCTGAT TGCGGTCATC GTGATGATTT
 401  TGATGCCGAA CTCGGGCAGC TTCGGTTTCG GCTATGCGTC GCTGGCGGCC
 451  TTGTCGTTCG GCGCGCTGAT GATTGCGCTG TTGGACGTGT CGTCGAATAT
 501  GGCGATGCAG CCGTTTAAGA TGATGGTCGG CGATATGGTC AACGAGGAGC
 551  AGAAAAGCTA CGCCTACGGG ATTCAAAGTT TCTTAGCGAA TACGGACGCG
 601  GTTGTGGCAG CGATTCTGCC GTTTGTGTTC GCGTATATCG GTTTGGCGAA
 651  CACTGCCGAG AAAGGCGTTG TGCCACAAAC CGTGGTCGTA GCATTCTATG
 701  TGGGTGCGGC GTTACTGATT ATTACCAGTG CGTTCACAAT CTCCAAAGTC
 751  AAAGAATACG ACCCGGAAAC CTACGCCCGT TACCACGGCA TCGATGTCGC
 801  CGCGAATCAG GAAAAAGCCA ACTGGTTCGA ACTCTTAAAA ACCGCGCCTA
 851  AAGTGTTTTG GACGGTTACT CCGGTACAGT TTTTCTGCTG GTTCGCCTTC
 901  CGGTATATGT GGACTTACTC GGCAGGCGCG ATTGCAGAAA ACGTCTGGCA
 951  CACTACCGAT GCGTCTTCCG TAGGCCATCA GGAGGCGGGC AACCGGTACG
1001  GCGTTTTGGC GGCGGTGTAG
```

This encodes a protein having amino acid sequence <SEQ ID 154>:

```
  1  MIGDRRAGNH FGFSKANTFQ IKKKDLLYVG IYASNSKTRF ARAGKKHDLD
 51  VELRLSRRSD GLYPAKLADE PHFSNARRRP AQFGLVFHPA AAGGDAGSAD
101  SGYYSDRTWK PRLGGRRLPY LLYGTLIAVI VMILMPNSGS FGFGYASLAA
151  LSFGALMIAL LDVSSNMAMQ PFKMMVGDMV NEEQKSYAYG IQSFLANTDA
201  VVAAILPFVF AYIGLANTAE KGVVPQTVVV AFYVGAALLI ITSAFTISKV
251  KEYDPETYAR YHGIDVAANQ EKANWFELLK TAPKVFWTVT PVQFFCWFAF
301  RYMWTYSAGA IAENVWHTTD ASSVGHQEAG NRYGVLAAV*
```

ORF16ng and ORF16-1 show 89.3% identity in 261 aa overlap:

```
                    30        40        50        60        70        80
orf16-1.pep  MLSFGFLGVQTAFTLQSSQMSRIFQTLGADPHNLGWFFILPPLAGMLVQPI-VGHYSDRT
                                         | ::|  |   |  ||   :    |:||||| 
orf16ng      DVELRLSRRSDGLYPAKLADEPHFSNARRRPAQFGLVF-HPAAAGGDAGSADSGYYSDRT
            50        60        70        80         90       100

                    90       100       110       120       130       140
orf16-1.pep  WKPRLGGRRLPYLLYGTLIAVIVMILMPNSGSFGFGYASLAALSFGALMIALLDVSSNMA
             ||||||||||||||||||||||||||||||||||||||||||||||||||||||||||||
orf16ng      WKPRLGGRRLPYLLYGTLIAVIVMILMPNSGSFGFGYASLAALSFGALMIALLDVSSNMA
            110       120       130       140       150       160

                   150       160       170       180       190       200
orf16-1.pep  MQPFKMMVGDMVNEEQKGYAYGIQSFLANTGAVVAAILPFVFAYIGLANTAEKGVVPQTV
             ||||||||||||||||:||||||||||||| |||||||||||||||||||||||||||||
orf16ng      MQPFKMMVGDMVNEEQKSYAYGIQSFLANTDAVVAAILPFVFAYIGLANTAEKGVVPQTV
            170       180       190       200       210       220

                   210       220       230       240       250       260
orf16-1.pep  VVAFYVGAALLVITSAFTIFKVKEYDPETYARYHGIDVAANQEKANWIELLKTAPKAFWT
             ||||||||||:||||||| |||||||||||||||||||||||||||||:||||||||:|||
orf16ng      VVAFYVGAALLIITSAFTISKVKEYDPETYARYHGIDVAANQEKANWFELLKTAPKVFWT
            230       240       250       260       270       280

                   270       280       290       300       310       320
orf16-1.pep  VTLVQFFCWFAFQYMWTYSAGAIAENVWHTTDASSVGYQEAGNWYGVLAAVQSVAAVICS
             || ||||||||:|||||||||||||||||||||||||:||||| |||||||
orf16ng      VTPVQFFCWFAFRYMWTYSAGAIAENVWHTTDASSVGHQEAGNRYGVLAAVX
            290       300       310       320       330       340
```

Based on this analysis, including the presence of several putative transmembrane domains in the gonococcal protein, it is predicted that the proteins from *N.meningitidis* and *N.gonorrhoeae,* and their epitopes, could be useful antigens for vaccines or diagnostics, or for raising antibodies.

## Example 19

The following partial DNA sequence was identified in *N.meningitidis* <SEQ ID 155>:

```
  1 ATGTTGTTCC GTAAAACGAC CGCCGCCGTT TTGGCGCATA CCTTGATGCT
 51 GAACGGCTGT ACGTTGATGT TGTGGGGAAT GAACAACCCG GTCAGCGAAA
101 CAATCACCCG NAAACACGTT GNCAAAGACC AAATCCGNGN CTTCGGTGTG
151 GTTGCCGAAG ACAATGCCCA ATTGGAAAAG GGCAGCCTGG TGATGATGGG
201 CGGAAAATAC TGGTTCGTCG TCAATCCCGA AGATTCGGCG AA.NTGACGG
251 GNATTTTGAN GGCAGGGCTG GACAAACCCT TCCAAATAGT TNAGGATACC
301 CCGAGCTATG C.TGCCACCA AGCCCTGCCG GTCAAACTCG GATCGNCTGG
351 CAGCCAGAAT...
```

This corresponds to the amino acid sequence <SEQ ID 156; ORF28>:

```
  1 MLFRKTTAAV LAHTLMLNGC TLMLWGMNNP VSETITRKHV XKDQIRXFGV
 51 VAEDNAQLEK GSLVMMGGKY WFVVNPEDSA XXTGILXAGL DKPFQIVXDT
101 PSYXCHQALP VKLGSXGSQN...
```

Further work revealed the complete nucleotide sequence <SEQ ID 157>:

```
  1  ATGTTGTTCC GTAAAACGAC CGCCGCCGTT TTGGCGGCAA CCTTGATGCT
 51  GAACGGCTGT ACGTTGATGT TGTGGGGAAT GAACAACCCG GTCAGCGAAA
101  CAATCACCCG CAAACACGTT GACAAAGACC AAATCCGCGC CTTCGGTGTG
151  GTTGCCGAAG ACAATGCCCA ATTGGAAAAG GGCAGCCTGG TGATGATGGG
201  CGGAAAATAC TGGTTCGTCG TCAATCCCGA AGATTCGGCG AAGCTGACGG
251  GCATTTTGAA GGCAGGGCTG GACAAACCCT TCCAAATAGT TGAGGATACC
301  CCGAGCTATG CTCGCCACCA AGCCCTGCCG GTCAAACTCG AATCGCCTGG
351  CAGCCAGAAT TTCAGTACCG AAGGCCTTTG CCTGCGCTAC GATACCGACA
401  AGCCTGCCGA CATCGCCAAG CTGAAACAGC TCGGGTTTGA AGCGGTCAAA
451  CTCGACAATC GGACCATTTA CACGCGCTGC GTATCCGCCA AAGGCAAATA
501  CTACGCCACA CCGCAAAAAC TGAACGCCGA TTACCATTTT GAGCAAAGTG
551  TGCCTGCCGA TATTTATTAC ACGGTTACTG AAGAACATAC CGACAAATCC
601  AAGCTGTTTG CAAATATCTT ATATACGCCC CCCTTTTTGA TACTGGATGC
651  GGCGGGCGCG GTACTGGCCT TGCCTGCGGC GGCTCTGGGT GCGGTCGTGG
701  ATGCCGCCCG CAAATGA
```

This corresponds to the amino acid sequence <SEQ ID 158; ORF28-1>:

```
  1  MLFRKTTAAV LAATLMLNGC TLMLWGMNNP VSETITRKHV DKDQIRAFGV
 51  VAEDNAQLEK GSLVMMGGKY WFVVNPEDSA KLTGILKAGL DKPFQIVEDT
101  PSYARHQALP VKLESPGSQN FSTEGLCLRY DTDKPADIAK LKQLGFEAVK
151  LDNRTIYTRC VSAKGKYYAT PQKLNADYHF EQSVPADIYY TVTEEHTDKS
201  KLFANILYTP PFLILDAAGA VLALPAAALG AVVDAARK*
```

Computer analysis of this amino acid sequence gave the following results:

Homology with a predicted ORF from *N.meningitidis* (strain A)

ORF28 shows 79.2% identity over a 120aa overlap with an ORF (ORF28a) from strain A of *N. meningitidis:*

```
                     10        20        30        40        50        60
orf28.pep    MLFRKTTAAVLAHTLMLNGCTLMLWGMNNPVSETITRKHVXKDQIRXFGVVAEDNAQLEK
             |||||||||||| ||||||||:|:||||:| ||| :|||| ||||| |||||||||||||
orf28a       MLFRKTTAAVLAATLMLNGCTVMMWGMNSPFSETTARKHVDKDQIRAFGVVAEDNAQLEK
                     10        20        30        40        50        60

                     70        80        90       100       110       120
orf28.pep    GSLVMMGGKYWFVVNPEDSAXXTGILXAGLDKPFQIVXDTPSYXCHQALPVKLGSXGSQN
             ||||||||||||||||||||  |||| ||||| ||:|  :| :  :||||||| | :|||
orf28a       GSLVMMGGKYWFVVNPEDSAKLTGILKAGLDKQFQMVEPNPRFA-YQALPVKLESPASQN
                     70        80        90       100       110

orf28a       FSTEGLCLRYDTDRPADIAKLKQLEFEAVELDNRTIYTRCVSAKGKYYATPQKLNADYHF
          120       130       140       150       160       170
```

The complete length ORF28a nucleotide sequence <SEQ ID 159> is:

```
  1 ATGTTGTTCC GTAAAACGAC CGCCGCCGTT TTGGCGGCAA CCTTGATGTT
 51 GAACGGCTGT ACGGTAATGA TGTGGGGTAT GAACAGCCCG TTCAGCGAAA
101 CGACCGCCCG CAAACACGTT GACAAGGACC AAATCCGCGC CTTCGGTGTG
151 GTTGCCGAAG ACAATGCCCA ATTGGAAAAG GGCAGCCTGG TGATGATGGG
201 CGGGAAATAC TGGTTCGTCG TCAATCCTGA AGATTCGGCG AAGCTGACGG
251 GCATTTTGAA GGCCGGGTTG GACAAGCAGT TTCAAATGGT TGAGCCCAAC
301 CCGCGCTTTG CCTACCAAGC CCTGCCGGTC AAACTCGAAT CGCCCGCCAG
351 CCAGAATTTC AGTACCGAAG GCCTTTGCCT GCGCTACGAT ACCGACAGAC
401 CTGCCGACAT CGCCAAGCTG AAACAGCTTG AGTTTGAAGC GGTCGAACTC
451 GACAATCGGA CCATTTACAC GCGCTGCGTC TCCGCCAAAG GCAAATACTA
501 CGCCACACCG CAAAAACTGA ACGCCGATTA TCATTTTGAG CAAAGTGTGC
551 CTGCCGATAT TTATTACACG GTTACGAAAA AACATACCGA CAAATCCAAG
601 TTGTTTGAAA ATATTGCATA TACGCCCACC ACGTTGATAC TGGATGCGGT
651 GGGCGCGGTG CTGGCCTTGC CTGTCGCGGC GTTGATTGCA GCCACGAATT
701 CCTCAGACAA ATGA
```

This encodes a protein having amino acid sequence <SEQ ID 160>:

```
  1 MLFRKTTAAV LAATLMLNGC TVMMWGMNSP FSETTARKHV DKDQIRAFGV
 51 VAEDNAQLEK GSLVMMGGKY WFVVNPEDSA KLTGILKAGL DKQFQMVEPN
101 PRFAYQALPV KLESPASQNF STEGLCLRYD TDRPADIAKL KQLEFEAVEL
151 DNRTIYTRCV SAKGKYYATP QKLNADYHFE QSVPADIYYT VTKKHTDKSK
201 LFENIAYTPT TLILDAVGAV LALPVAALIA ATNSSDK*
```

ORF28a and ORF28-1 show 86.1% identity in 238 aa overlap:

```
                           10        20        30        40        50        60
orf28a.pep         MLFRKTTAAVLAATLMLNGCTVMMWGMNSPFSETTARKHVDKDQIRAFGVVAEDNAQLEK
                   |||||||||||||||||||||:|:||||:| ||| :||||||||||||||||||||||||
orf28-1            MLFRKTTAAVLAATLMLNGCTLMLWGMNNPVSETITRKHVDKDQIRAFGVVAEDNAQLEK
                           10        20        30        40        50        60

                           70        80        90       100        110      119
orf28a.pep         GSLVMMGGKYWFVVNPEDSAKLTGILKAGLDKQFQMVEPNPRFA-YQALPVKLESPASQN
                   |||||||||||||||||||||||||||||||| ||:|| :| :| :||||||||||:|||
orf28-1            GSLVMMGGKYWFVVNPEDSAKLTGILKAGLDKPFQIVEDTPSYARHQALPVKLESPGSQN
                           70        80        90       100       110       120

                 120       130       140       150       160       170      179
orf28a.pep         FSTEGLCLRYDTDRPADIAKLKQLEFEAVELDNRTIYTRCVSAKGKYYATPQKLNADYHF
                   |||||||||||||:|||||||||| ||||:||||||||||||||||||||||||||||||
orf28-1            FSTEGLCLRYDTDKPADIAKLKQLGFEAVKLDNRTIYTRCVSAKGKYYATPQKLNADYHF
                          130       140       150       160       170       180

                 180       190       200       210       220       230
orf28a.pep         EQSVPADIYYTVTKKHTDKSKLFENIAYTPTTLILDAVGAVLALPVAALIAATNSSDKX
                   |||||||||||||::|||||||| || |||  |||||:|||||||:||| |::::: ||
orf28-1            EQSVPADIYYTVTEEHTDKSKLFANILYTPPFLILDAAGAVLALPAAALGAVVDAARKX
                          190       200       210       220       230
```

<u>Homology with a predicted ORF from *N.gonorrhoeae*</u>

ORF28 shows 84.2% identity over a 120aa overlap with a predicted ORF (ORF28.ng) from *N. gonorrhoeae:*

```
orf28.pep   MLFRKTTAAVLAHTLMLNGCTLMLWGMNNPVSETITRKHVXKDQIRXFGVVAEDNAQLEK   60
            |||||||||||| ||:|||||:|| |||||||:||||||| ||||| |||||||||||||
orf28ng     MLFRKTTAAVLAATLILNGCTMMLRGMNNPVSQTITRKHVDKDQIRAFGVVAEDNAQLEK   60

orf28.pep   GSLVMMGGKYWFVVNPEDSAXXTGILXAGLDKPFQIVXDTPSYXCHQALPVKLGSXGSQN  120
            ||||||||||||:|||||||  ||:| |||||||||| |||||  |||||||: : ||||
orf28ng     GSLVMMGGKYWFAVNPEDSAKLTGLLKAGLDKPFQIVEDTPSYARHQALPVKFEAPGSQN  120
```

The complete length ORF28ng nucleotide sequence <SEQ ID 161> is

```
  1 ATGTTGTTCC GTAAAACGAC CGCCGCCGTT TTGGCGGCAA CCTTGATACT
 51 GAACGGCTGT ACGATGATGT TGCGGGGGAT GAACAACCCG GTCAGCCAAA
101 CAATCACCCG CAAACACGTT GACAAAGACC AAATCCGCGC CTTCGGTGTG
151 GTTGCCGAAG ACAATGCCCA ATTGGAAAAG GGCAGCCTGG TGATGATGGG
201 CGGGAAATAC TGGTTCGCCG TCAATCCCGA AGATTCGGCG AAGCTGACGG
```

```
251 GCCTTTTGAA GGCCGGGTTG GACAAGCCCT TCCAAATAGT TGAGGATACC
301 CCGAGCTATG CCCGCCACCA AGCCCTGCCG GTCAAATTCG AAGCGCCCGG
351 CAGCCAGAAT TTCAGTACCG GAGGTCTTTG CCTGCGCTAT GATACCGGCA
401 GACCTGACGA CATCGCCAAG CTGAAACAGC TTGAGTTTAA AGCGGTCAAA
451 CTCGACAATC GGACCATTTA CACGCGCTGC GTATCCGCCA AAGGCAAATA
501 CTACGCCACG CCGCAAAAAC TGAACGCCGA TTATCATTTT GAGCAAAGTG
551 TGCCCGCCGA TATTTATTAT ACGGTTACTG AAAAACATAC CGACAAATCC
601 AAGCTGTTTG GAAATATCTT ATATACGCCC CCCTTGTTGA TATTGGATGC
651 GGCGGCCGCG GTGCTGGTCT TGCCTATGGC TCTGATTGCA GCCGCGAATT
701 CCTCAGACAA ATGA
```

This encodes a protein having amino acid sequence <SEQ ID 162>:

```
  1 MLFRKTTAAV LAATLILNGC TMMLRGMNNP VSQTITRKHV DKDQIRAFGV
 51 VAEDNAQLEK GSLVMMGGKY WFAVNPEDSA KLTGLLKAGL DKPFQIVEDT
101 PSYARHQALP VKFEAPGSQN FSTGGLCLRY DTGRPDDIAK LKQLEFKAVK
151 LDNRTIYTRC VSAKGKYYAT PQKLNADYHF EQSVPADIYY TVTEKHTDKS
201 KLFGNILYTP PLLILDAAAA VLVLPMALIA AANSSDK*
```

ORF28ng and ORF28-1 share 90.0% identity in 231 aa overlap:

```
                        10        20        30        40        50        60
orf28-1.pep     MLFRKTTAAVLAATLMLNGCTLMLWGMNNPVSETITRKHVDKDQIRAFGVVAEDNAQLEK
                |||||||||||||||:|||||:|| |||||||:|||||||||||||||||||||||||||
orf28ng         MLFRKTTAAVLAATLILNGCTMMLRGMNNPVSQTITRKHVDKDQIRAFGVVAEDNAQLEK
                        10        20        30        40        50        60

                        70        80        90       100       110       120
orf28-1.pep     GSLVMMGGKYWFVVNPEDSAKLTGILKAGLDKPFQIVEDTPSYARHQALPVKLESPGSQN
                ||||||||||||:|||||||||||:|||||||||||||||||||||||||||:|:|||||
orf28ng         GSLVMMGGKYWFAVNPEDSAKLTGLLKAGLDKPFQIVEDTPSYARHQALPVKFEAPGSQN
                        70        80        90       100       110       120

                       130       140       150       160       170       180
orf28-1.pep     FSTEGLCLRYDTDKPADIAKLKQLGFEAVKLDNRTIYTRCVSAKGKYYATPQKLNADYHF
                ||| |||||||| :| |||||||| |:|||||||||||||||||||||||||||||||||
orf28ng         FSTGGLCLRYDTGRPDDIAKLKQLEFKAVKLDNRTIYTRCVSAKGKYYATPQKLNADYHF
                       130       140       150       160       170       180

                       190       200       210       220       230      239
orf28-1.pep     EQSVPADIYYTVTEEHTDKSKLFANILYTPPFLILDAAGAVLALPAAALGAVVDAARKX
                ||||||||||||||:||||||||:|||||||:||||||:|||:||  |  ::|:
orf28ng         EQSVPADIYYTVTEKHTDKSKLFGNILYTPPLLILDAAAAVLVLPMALIAAANSSDKX
                       190       200       210       220       230
```

Based on this analysis, including the presence of a putative transmembrane domain in the gonococcal protein, it was predicted that the proteins from *N.meningitidis* and *N.gonorrhoeae,* and their epitopes, could be useful antigens for vaccines or diagnostics, or for raising antibodies.

ORF28-1 (24kDa) was cloned in pET and pGex vectors and expressed in *E.coli,* as described above. The products of protein expression and purification were analyzed by SDS-PAGE. Figure 6A shows the results of affinity purification of the GST-fusion protein, and Figure 6B shows the results of expression of the His-fusion in *E. coli.* Purified GST-fusion protein was used to immunise mice, whose sera were used for ELISA, which gave a positive result. These experiments confirm that ORF28-1 is a surface-exposed protein, and that it may be a useful immunogen.

**Example 20**

The following partial DNA sequence was identified in *N.meningitidis* <SEQ ID 163>:

```
  1 ..GTCAGTCCTG TACTGCCTAT TACACACGAA CGGACAGGGT TTGAAGGTGT
 51   TATCGGTTAT GAAACCCATT TTTCAGGGCA CGGACATGAA GTACACAGTC
101   CGTTCGATCA TCATGATTCA AAAAGCACTT CTGATTTCAG CGGCGGTGTA
151   GACGGCGGTT TTACTGTTTA CCAACTTCAT CGAACATGGT CGGAAATCCA
201   TCCGGAGGAT GAATATGACG GGCCGCAAGC AGCG.ATTAT CCGCCCCCCG
251   GAGGAGCAAG GGATATATAC AGCTATTATG TCAAAGGAAC TTCAACAAAA
301   ACAAAGACTA GTATTGTCCC TCAAGCCCCA TTTTCAGACC GTTGGCTAGA
351   AGAAAATGCC GGTGCCGCCT CTGGT..
```

This corresponds to the amino acid sequence <SEQ ID 164; ORF29>:

```
  1 ..VSPVLPITHE RTGFEGVIGY ETHFSGHGHE VHSPFDHHDS KSTSDFSGGV
 51   DGGFTVYQLH RTWSEIHPED EYDGPQAAXY PPPGGARDIY SYYVKGTSTK
101   TKTSIVPQAP FSDRWLEENA GAASG..
```

Further work revealed the complete nucleotide sequence <SEQ ID 165>:

```
   1 ATGAATTTGC CTATTCAAAA ATTCATGATG CTGTTTGCAG CAGCAATATC
  51 GTTGCTGCAA ATCCCCATTA GTCATGCGAA CGGTTTGGAT GCCCGTTTGC
 101 GCGATGATAT GCAGGCAAAA CACTACGAAC CGGGTGGTAA ATACCATCTG
 151 TTTGGTAATG CTCGCGGCAG TGTTAAAAAG CGGGTTTACG CCGTCCAGAC
 201 ATTTGATGCA ACTGCGGTCA GTCCTGTACT GCCTATTACA CACGAACGGA
 251 CAGGGTTTGA AGGTGTTATC GGTTATGAAA CCCATTTTTC AGGGCACGGA
 301 CATGAAGTAC ACAGTCCGTT CGATCATCAT GATTCAAAAA GCACTTCTGA
 351 TTTCAGCGGC GGTGTAGACG GCGGTTTTAC TGTTTACCAA CTTCATCGAA
 401 CAGGGTCGGA AATCCATCCG GAGGATGGAT ATGACGGGCC GCAAGGCAGC
 451 GATTATCCGC CCCCCGGAGG AGCAAGGGAT ATATACAGCT ATTATGTCAA
 501 AGGAACTTCA ACAAAAACAA AGACTAATAT TGTCCCTCAA GCCCCATTTT
 551 CAGACCGTTG GCTAAAAGAA AATGCCGGTG CCGCCTCTGG TTTTTTCAGC
 601 CGTGCGGATG AAGCAGGAAA ACTGATATGG GAAAGCGACC CCAATAAAAA
 651 TTGGTGGGCT AACCGTATGG ATGATGTTCG CGGCATCGTC CAAGGTGCGG
 701 TTAATCCTTT TTTAATGGGT TTTCAAGGAG TAGGGATTGG GGCAATTACA
 751 GACAGTGCAG TAAGCCCGGT CACAGATACA GCCGCGCAGC AGACTCTACA
 801 AGGTATTAAT GATTTAGGAA AATTAAGTCC GGAAGCACAA CTTGCTGCCG
 851 CGAGCCTATT ACAGGACAGT GCTTTTGCGG TAAAAGACGG TATCAACTCT
 901 GCCAAACAAT GGGCTGATGC CCATCCAAAT ATAACAGCTA CTGCCCAAAC
 951 TGCCCTTTCC GCAGCAGAGG CCGCAGGTAC GGTTTGGAGA GGTAAAAAAG
1001 TAGAACTTAA CCCGACTAAA TGGGATTGGG TTAAAAATAC CGGTTATAAA
1051 AAACCTGCTG CCCGCCATAT GCAGACTTTA GATGGGGAGA TGGCAGGTGG
1101 GAATAAACCT ATTAAATCTT TACCAAACAG TGCCGCTGAA AAAAGAAAAC
1151 AAAATTTTGA GAAGTTTAAT AGTAACTGGA GTTCAGCAAG TTTTGATTCA
1201 GTGCACAAAA CACTAACTCC CAATGCACCT GGTATTTTAA GTCCTGATAA
1251 AGTTAAAACT CGATACACTA GTTTAGATGG AAAAATTACA ATTATAAAAG
1301 ATAACGAAAA CAACTATTTT AGAATCCATG ATAATTCACG AAAACAGTAT
1351 CTTGATTCAA ATGGTAATGC TGTGAAAACC GGTAATTTAC AAGGTAAGCA
1401 AGCAAAAGAT TATTTACAAC AACAAACTCA TATCAGGAAC TTAGACAAAT
1451 GA
```

This corresponds to the amino acid sequence <SEQ ID 166; ORF29-1>:

```
  1 MNLPIQKFMM LFAAAISLLQ IPISHANGLD ARLRDDMQAK HYEPGGKYHL
 51 FGNARGSVKK RVYAVQTFDA TAVSPVLPIT HERTGFEGVI GYETHFSGHG
101 HEVHSPFDHH DSKSTSDFSG GVDGGFTVYQ LHRTGSEIHP EDGYDGPQGS
151 DYPPPGGARD IYSYYVKGTS TKTKTNIVPQ APFSDRWLKE NAGAASGFFS
201 RADEAGKLIW ESDPNKNWWA NRMDDVRGIV QGAVNPFLMG FQGVGIGAIT
251 DSAVSPVTDT AAQQTLQGIN DLGKLSPEAQ LAAASLLQDS AFAVKDGINS
301 AKQWADAHPN ITATAQTALS AAEAAGTVWR GKKVELNPTK WDWVKNTGYK
351 KPAARHMQTL DGEMAGGNKP IKSLPNSAAE KRKQNFEKFN SNWSSASFDS
401 VHKTLTPNAP GILSPDKVKT RYTSLDGKIT IIKDNENNYF RIHDNSRKQY
451 LDSNGNAVKT GNLQGKQAKD YLQQQTHIRN LDK*
```

Computer analysis of this amino acid sequence gave the following results:

Homology with a predicted ORF from *N.meningitidis* (strain A)

ORF29 shows 88.0% identity over a 125aa overlap with an ORF (ORF29a) from strain A of *N. meningitidis:*

```
        10        20        30
```

```
orf29.pep                                 VSPVLPITHERTGFEGVIGYETHFSGHGHE
                                          |:|:||||||||||||:|||||||||||||
orf29a        EPGGKYHLFGNARGSVKNRVYAVQTFDATAVGPILPITHERTGFEGIIGYETHFSGHGHE
                    50        60        70        80        90       100

                      40        50        60        70        80        90
orf29.pep     VHSPFDHHDSKSTSDFSGGVDGGFTVYQLHRTWSEIHPEDEYDGPQAAXYPPPGGARDIY
              ||||||:||||||||||||||||||||||||| ||||||| |||||:: |||||||||||
orf29a        VHSPFDNHDSKSTSDFSGGVDGGFTVYQLHRTGSEIHPEDGYDGPQGSDYPPPGGARDIY
                   110       120       130       140       150       160

                     100       110       120
orf29.pep     SYYVKGTSTKTKTSIVPQAPFSDRWLEENAGAASG
                ||||||||||::|||:||||||||:||||||||
orf29a        XXYVKGTSTKTKSNIVPRAPFSDRWLKENAGAASGFFSRADEAGKLIWESDPNKNWWANR
                   170       180       190       200       210       220

orf29a        MDDIRGIVQGAVNPFLMGFQGVGIGAITDSAVSPVTDTAAQQTLQGXNHLGXLSPEAQLA
                   230       240       250       260       270       280
```

The complete length ORF29a nucleotide sequence <SEQ ID 167> is:

```
   1  ATGAATTNGC CTATTCAAAA ATTCATGATG CTGTTTGCAG CAGCAATATC
  51  GTNGCTGCAA ATCCCNATTA GTCATGCGAA CGGTTTGGAT GCCCGTTTGC
 101  GCGATGATAT GCAGGCAAAA CACTACGAAC CGGGTGGTAA ATACCATCTG
 151  TTTGGTAATG CTCGCGGCAG TGTTAAAAAT CGGGTTTACG CCGTCCAAAC
 201  ATTTGATGCA ACTGCGGTCG GCCCCATACT GCCTATTACA CACGAACGGA
 251  CAGGATTTGA AGGCATTATC GGTTATGAAA CCCATTTTTC AGGACATGGA
 301  CATGAAGTAC ACAGTCCGTT CGATAATCAT GATTCAAAAA GCACTTCTGA
 351  TTTCAGCGGC GGCGTAGACG GTGGTTTTAC CGTTTACCAA CTTCATCGGA
 401  CAGGGTCGGA AATCCATCCG GAGGATGGAT ATGACGGGCC GCAAGGCAGC
 451  GATTATCCGC CCCCCGGAGG AGCAAGGGAT ATATACANNT ANTATGTCAA
 501  AGGAACTTCA ACAAAAACAA AGAGTAATAT TGTTCCCCGA GCCCCATTTT
 551  CAGACCGCTG GCTAAAAGAA AATGCCGGTG CCGCCTCTGG TTTTTTCAGC
 601  CGTGCTGATG AAGCAGGAAA ACTGATATGG GAAAGCGACC CCAATAAAAA
 651  TTGGTGGGCT AACCGTATGG ATGATATTCG CGGCATCGTC CAAGGTGCGG
 701  TTAATCCTTT TTTAATGGGT TTTCAAGGAG TAGGGATTGG GGCAATTACA
 751  GACAGTGCAG TAAGCCCGGT CACAGATACA GCCGCGCAGC AGACTCTACA
 801  AGGTATNAAT CATTTAGGAA ANTTAAGTCC CGAAGCACAA CTTGCGGCTG
 851  CAACCGCATT ACAAGACAGT GCTTTTGCGG TAAAAGACGG TATCAATTCC
 901  GCCAGACAAT GGGCTGATGC CCATCCGAAT ATAACTGCAA CAGCCCAAAC
 951  TGCCCTTGCC GTAGCAGANG CCGCAACTAC GGTTTGGGGC GGTAAAAAAG
1001  TAGAACTTAA CCCGACCAAA TGGGATTGGG TTAAAAATAC NGGCTATAAN
1051  ACACCTGCTG TTCGCACCAT GCATACTTTG GATGGGGAAA TGGCCGGTGG
1101  GAATAGACCG CCTAAATCTA TAACGTCCAA CAGCAAAGCA GATGCTTCCA
1151  CACAACCGTC TTTACAAGCG CAACTAATTG GAGAACAAAT TANNNNNGGG
1201  CATGCTTATA ACAAGCATGT CATAAGACAA CAAGAATTTA CGGATTTAAA
1251  TATCAATTCA CCAGCAGATT TTGCTCGGCA TATTGAAAAT ATTGTTAGCC
1301  ATCCANCAAA TATGAAAGAG TTACCTCGCG GTAGAACTGC GTATTGGGAT
1351  NATAAAACAG GGACNATAGT TATCCGAGAT AAAAATTCTG ACGATGGAGG
1401  TACAGCATTT AGACCAACAT CAGGTAAAAA ATATTATGAT GATTTATAG
```

This encodes a protein having amino acid sequence <SEQ ID 168>:

```
  1  MNXPIQKFMM LFAAAISXLQ IPISHANGLD ARLRDDMQAK HYEPGGKYHL
 51  FGNARGSVKN RVYAVQTFDA TAVGPILPIT HERTGFEGII GYETHFSGHG
101  HEVHSPFDNH DSKSTSDFSG GVDGGFTVYQ LHRTGSEIHP EDGYDGPQGS
151  DYPPPGGARD IYXXYVKGTS TKTKSNIVPR APFSDRWLKE NAGAASGFFS
201  RADEAGKLIW ESDPNKNWWA NRMDDIRGIV QGAVNPFLMG FQGVGIGAIT
251  DSAVSPVTDT AAQQTLQGXN HLGXLSPEAQ LAAATALQDS AFAVKDGINS
301  ARQWADAHPN ITATAQTALA VAXAATTVWG GKKVELNPTK WDWVKNTGYX
351  TPAVRTMHTL DGEMAGGNRP PKSITSNSKA DASTQPSLQA QLIGEQIXXG
401  HAYNKHVIRQ QEFTDLNINS PADFARHIEN IVSHPXNMKE LPRGRTAYWD
451  XKTGTIVIRD KNSDDGGTAF RPTSGKKYYD DL*
```

ORF29a and ORF29-1 show 90.1% identity in 385 aa overlap:

```
                        10        20        30        40        50        60
orf29a.pep      MNXPIQKFMMLFAAAISXLQIPISHANGLDARLRDDMQAKHYEPGGKYHLFGNARGSVKN
                || |||||||||||||| |||||||||||||||||||||||||||||||||||||||||:
orf29-1         MNLPIQKFMMLFAAAISLLQIPISHANGLDARLRDDMQAKHYEPGGKYHLFGNARGSVKK

                        10        20        30        40        50        60

                        70        80        90       100       110       120
orf29a.pep      RVYAVQTFDATAVGPILPITHERTGFEGIIGYETHFSGHGHEVHSPFDNHDSKSTSDFSG
                |||||||||||||:|:||||||||||||:||||||||||||||||||||:||||||||||
orf29-1         RVYAVQTFDATAVSPVLPITHERTGFEGVIGYETHFSGHGHEVHSPFDHHDSKSTSDFSG
                        70        80        90       100       110       120

                       130       140       150       160       170       180
orf29a.pep      GVDGGFTVYQLHRTGSEIHPEDGYDGPQGSDYPPPGGARDIYXXYVKGTSTKTKSNIVPR
                ||||||||||||||||||||||||||||||||||||||||||  ||||||||||:||||:
orf29-1         GVDGGFTVYQLHRTGSEIHPEDGYDGPQGSDYPPPGGARDIYSYYVKGTSTKTKTNIVPQ
                       130       140       150       160       170       180

                       190       200       210       220       230       240
orf29a.pep      APFSDRWLKENAGAASGFFSRADEAGKLIWESDPNKNWWANRMDDIRGIVQGAVNPFLMG
                |||||||||||||||||||||||||||||||||||||||||||||:||||||||||||||
orf29-1         APFSDRWLKENAGAASGFFSRADEAGKLIWESDPNKNWWANRMDDVRGIVQGAVNPFLMG
                       190       200       210       220       230       240

                       250       260       270       280       290       300
orf29a.pep      FQGVGIGAITDSAVSPVTDTAAQQTLQGXNHLGXLSPEAQLAAATALQDSAFAVKDGINS
                |||||||||||||||||||||||||||| | || ||||||||||: ||||||||||||||
orf29-1         FQGVGIGAITDSAVSPVTDTAAQQTLQGINDLGKLSPEAQLAASLLQDSAFAVKDGINS
                       250       260       270       280       290       300

                       310       320       330       340       350       360
orf29a.pep      ARQWADAHPNITATAQTALAVAXAATTVWGGKKVELNPTKWDWVKNTGYXTPAVRTMHTL
                |:|||||||||||||||||::| || ||| |||||||||||||||||||  ||:| |:||
orf29-1         AKQWADAHPNITATAQTALSAAEAAGTVWRGKKVELNPTKWDWVKNTGYKKPAARHMQTL
                       310       320       330       340       350       360

                       370       380       390       400       410       420
orf29a.pep      DGEMAGGNRPPKSITSNSKADASTQPSLQAQLIGEQIXXGHAYNKHVIRQQEFTDLNINS
                ||||||||:| ||:  || |:   |
orf29-1         DGEMAGGNKPIKSLP-NSAAEKRKQNFEKFNSNWSSASFDSVHKTLTPNAPGILSPDKVK
                       370        380        390        400        410
```

<u>Homology with a predicted ORF from *N.gonorrhoeae*</u>

ORF29 shows 88.8% identity over a 125aa overlap with a predicted ORF (ORF29.ng) from *N. gonorrhoeae:*

```
orf29.pep                                        VSPVLPITHERTGFEGVIGYETHFSGHGHE   30
                                                 |:|:||||||||||||||||||||||||||
orf29ng      EPGGKYHLFGNARGSVKNRVCAVQTFDATAVGPILPITHERTGFEGVIGYETHFSGHGHE  102

orf29.pep    VHSPFDHHDSKSTSDFSGGVDGGFTVYQLHRTWSEIHPEDEYDGPQAAXYPPPGGARDIY   90
             ||||||:||||||||||||||||||||||||| ||||||| |||||:: |||||||||||
orf29ng      VHSPFDNHDSKSTSDFSGGVDGGFTVYQLHRTGSEIHPEDGYDGPQGGGYPPPGGARDIY  162

orf29.pep    SYYVKGTSTKTKTSIVPQAPFSDRWLEENAGAASG                          125
             ||::|||||||| : |||||||||||:||||||||
orf29ng      SYHIKGTSTKTKINTVPQAPFSDRWLKENAGAASGFLSRADEAGKLIWENDPDKNWRANR  222
```

The complete length ORF29ng nucleotide sequence <SEQ ID 169> is predicted to encode a protein having amino acid sequence <SEQ ID 170>:

```
  1  MNLPIQKFMM LFAAAISLLQ IPISHANGLD ARLRDDMQAK HYEPGGKYHL
 51  FGNARGSVKN RVCAVQTFDA TAVGPILPIT HERTGFEGVI GYETHFSGHG
101  HEVHSPFDNH DSKSTSDFSG GVDGGFTVYQ LHRTGSEIHP EDGYDGPQGG
151  GYPPPGGARD IYSYHIKGTS TKTKINTVPQ APFSDRWLKE NAGAASGFLS
201  RADEAGKLIW ENDPDKNWRA NRMDDIRGIV QGAVNPFLTG FQGLGVGAIT
251  DSAVSPVTYA AARKTLQGIH NLGNLSPEAQ LAAATALQDS AFAVKDSINS
301  ARQWADAHPN ITATAQTALA VTEAATTVWG GKKVELNPAK WDWVKNTGYK
351  KPAARHMQTV DGEMAGGNKP LESKNTVTTN NFFENTGYTE KVLRQASNGD
401  YHGFPQSVDA FSENGTVIQI VGGDNIVRHK LYIPGSYKGK DGNFEYIREA
451  DGKINHRLFV PNQQLPEK*
```

In a second experiment, the following DNA sequence <SEQ ID 171> was identified:

```
   1  atgAATTTGC CTATTCAAAA ATTCATGATG ctgttggcAg cggcaatatc
  51  gatgctGCat ATCCCCATTA GTCATGCGAA CGGTTTGGAT GCCCGTTTGC
 101  GCGATGATAT GCAGGCAAAA CACTACGAAC CGGGTGGCAA ATACCATCTG
 151  TTTGGTAATG CTCGCGGCAG TGTTAAAAAT CGGGTTTGCG CCGTCCAAAC
 201  ATTTGATGCA ACTGCGGTCG GCCCCATACT GCCTATTACA CACGAACGGA
 251  CAGGATTTGA AGGTGTTATC GGCTATGAAA CCCATTTTTC AGGACACGGA
 301  CACGAAGTAC ACAGTCCGTT CGATAATCAT GATTCAAAAA GCACTTCTGA
 351  TTTCAGCGGC GGCGTAGACG GCGGTTTTAC CGTTTACCAA CTTCATCGGA
 401  CAGGGTCGGA AATACATCCC GCAGACGGAT ATGACGGGCC TCAAGGCGGC
 451  GGTTATCCGG AACCACAAGG GGCAAGGGAT ATATACAGCT ACCATATCAA
 501  AGGAACTTCA ACCAAAACAA AGATAAACAC TGTTCCGCAA GCCCCTTTTT
 551  CAGACCGCTG GCTAAAAGAA AATGCCGGTG CCGCTTCCGG TTTTCTCAGC
 601  CGTGCGGATG AAGCAGGAAA ACTGATATGG GAAAACGACC CCGATAAAAA
 651  TTGGCGGGCT AACCGTATGG ATGATATTCG CGGCATCGTC CAAGGTGCGG
 701  TTAATCCTTT TTTAACGGGT TTTCAAGGGG TAGGGATTGG GGCAATTACA
 751  GACAGTGCGG TAAGCCCGGT CACAGATACA GCCGCTCAGC AGACTCTACA
 801  AGGTATTAAT GATTTAGGAA ATTTAAGTCC GGAAGCACAA CTTGCCGCCG
 851  CGAGCCTATT ACAGGACAGT GCCTTTGCGG TAAAAGACGG CATCAATTCC
 901  GCCAGACAAT GGGCTGATGC CCATCCGAAT ATAACAGCAA CAGCCCAAAC
 951  TGCCCTTGCC GTAGCAGAGG CCGCAGGTAC GGTTTGGCGC GGTAAAAAAG
1001  TAGAACTTAA CCCGACCAAA TGGGATTGGG TTAAAAATAC CGGCTATAAA
1051  AAACCTGCTG CCCGCCATAT GCAGACTGTA GATGGGGAGA TGGCAGGGGG
1101  GAATAGACCG CCTAAATCTA TAACGTCGGA AGGAAAAGCT AATGCTGCAA
1151  CCTATCCTAA GTTGGTTAAT CAGCTAAATG AGCAAAACTT AAATAACATT
1201  GCGGCTCAAG ATCCAAGATT GAGTCTAGCT ATTCATGAGG GTAAAAAAAA
1251  TTTTCCAATA GGAACTGCAA CTTATGAAGA GGCAGATAGA CTAGGTAAAA
1301  TTTGGGTTGG TGAGGGTGCA AGACAAACTA GTGGAGGCGG ATGGTTAAGT
1351  AGAGATGGCA CTCGACAATA TCGGCCACCA ACAGAAAAAA AATCACAATT
1401  TGCAACTACA GGTATTCAAG CAAATTTTGA AACTTATACT ATTGATTCAA
1451  ATGAAAAAAG AAATAAAATT AAAAATGGAC ATTTAAATAT TAGGTAA
```

This encodes a protein having amino acid sequence <SEQ ID 172; ORF29ng-1>:

```
  1 MNLPIQKFMM LLAAAISMLH IPISHANGLD ARLRDDMQAK HYEPGGKYHL
 51 FGNARGSVKN RVCAVQTFDA TAVGPILPIT HERTGFEGVI GYETHFSGHG
101 HEVHSPFDNH DSKSTSDFSG GVDGGFTVYQ LHRTGSEIHP ADGYDGPQGG
151 GYPEPQGARD IYSYHIKGTS TKTKINTVPQ APFSDRWLKE NAGAASGFLS
201 RADEAGKLIW ENDPDKNWRA NRMDDIRGIV QGAVNPFLTG FQGVGIGAIT
251 DSAVSPVTDT AAQQTLQGIN DLGNLSPEAQ LAAASLLQDS AFAVKDGINS
301 ARQWADAHPN ITATAQTALA VAEAAGTVWR GKKVELNPTK WDWVKNTGYK
351 KPAARHMQTV DGEMAGGNRP PKSITSEGKA NAATYPKLVN QLNEQNLNNI
401 AAQDPRLSLA IHEGKKNFPI GTATYEEADR LGKIWVGEGA RQTSGGGWLS
451 RDGTRQYRPP TEKKSQFATT GIQANFETYT IDSNEKRNKI KNGHLNIR*
```

ORF29ng-1 and ORF29-1 show 86.0% identity in 401 aa overlap:

```
                         10        20        30        40        50        60
orf29ng-1.pep    MNLPIQKFMMLLAAAISMLHIPISHANGLDARLRDDMQAKHYEPGGKYHLFGNARGSVKN
                 |||||||||||:|||||:|:|||||||||||||||||||||||||||||||||||||||:
orf29-1          MNLPIQKFMMLFAAAISLLQIPISHANGLDARLRDDMQAKHYEPGGKYHLFGNARGSVKK
                         10        20        30        40        50        60

                         70        80        90       100       110       120
orf29ng-1.pep    RVCAVQTFDATAVGPILPITHERTGFEGVIGYETHFSGHGHEVHSPFDNHDSKSTSDFSG
                 || ||||||||||:|:||||||||||||||||||||||||||||||||:|||||||||||
orf29-1          RVYAVQTFDATAVSPVLPITHERTGFEGVIGYETHFSGHGHEVHSPFDHHDSKSTSDFSG
                         70        80        90       100       110       120

                        130       140       150       160       170       180
orf29ng-1.pep    GVDGGFTVYQLHRTGSEIHPADGYDGPQGGGYPEPQGARDIYSYHIKGTSTKTKINTVPQ
                 |||||||||||||||||||| ||||||||: || | ||||||||::|||||||| | |||
orf29-1          GVDGGFTVYQLHRTGSEIHPEDGYDGPQGSDYPPPGGARDIYSYYVKGTSTKTKTNIVPQ
                        130       140       150       160       170       180

                        190       200       210       220       230       240
orf29ng-1.pep    APFSDRWLKENAGAASGFLSRADEAGKLIWENDPDKNWRANRMDDIRGIVQGAVNPFLTG
                 ||||||||||||||||||:||||||||||||:||:||| ||||||:|||||||||||| |
orf29-1          APFSDRWLKENAGAASGFFSRADEAGKLIWESDPNKNWWANRMDDVRGIVQGAVNPFLMG
                        190       200       210       220       230       240

                        250       260       270       280       290       300
orf29ng-1.pep    FQGVGIGAITDSAVSPVTDTAAQQTLQGINDLGNLSPEAQLAAASLLQDSAFAVKDGINS
                 |||||||||||||||||||||||||||||||||:||||||||||||||||||||||||||
orf29-1          FQGVGIGAITDSAVSPVTDTAAQQTLQGINDLGKLSPEAQLAAASLLQDSAFAVKDGINS
                        250       260       270       280       290       300

                        310       320       330       340       350       360
orf29ng-1.pep    ARQWADAHPNITATAQTALAVAEAAGTVWRGKKVELNPTKWDWVKNTGYKKPAARHMQTV
                 |:|||||||||||||||||::||||||||||||||||||||||||||||||||||||||:
orf29-1          AKQWADAHPNITATAQTALSAAEAAGTVWRGKKVELNPTKWDWVKNTGYKKPAARHMQTL
                        310       320       330       340       350       360

                        370       380       390       400       410       419
orf29ng-1.pep    DGEMAGGNRPPKSI-TSEGKANAATYPKLVNQLNEQNLNNIAAQDPRLSLAIHEGKKNFP
                 ||||||||:| ||: :| ::    :: |: :: :  :::::
orf29-1          DGEMAGGNKPIKSLPNSAAEKRKQNFEKFNSNWSSASFDSVHKTLTPNAPGILSPDKVKT
                        370       380       390       400       410       420

              420       430       440       450       460       470       479
orf29ng-1.pep    IGTATYEEADRLGKIWVGEGARQTSGGGWLSRDGTRQYRPPTEKKSQFATTGIQANFETY

orf29-1          RYTSLDGKITIIKDNENNYFRIHDNSRKQYLDSNGNAVKTGNLQGKQAKDYLQQQTHIRN
                        430       440       450       460       470       480
```

Based on this analysis, including the presence of a putative leader sequence in the gonococcal protein, it is predicted that the proteins from *N.meningitidis* and *N.gonorrhoeae,* and their epitopes, could be useful antigens for vaccines or diagnostics, or for raising antibodies.

**Example 21**

The following partial DNA sequence was identified in *N.meningitidis* <SEQ ID 173>:

```
  1  ATGAAAAAAC AAATCACCGC AGCCGTAATG ATGCTGTCTA TGATTGCCCC
 51  CGCAATGGCA AACGGCTTGG ACAATCAGGC ATTTGAAGAC CAAATGTTCC
101  ACACGCGGGC AGATGCACCG ATGCAG...
```

This corresponds to the amino acid sequence <SEQ ID 174; ORF30>:

```
1  MKKQITAAVM MLSMIAPAMA NGLDNQAFED QMFHTRADAP MQ..
```

Further work revealed the complete nucleotide sequence <SEQ ID 175>:

```
  1  ATGAAAAAAC AAATCACCGC AGCCGTAATG ATGCTGTCTA TGATTGCCCC
 51  CGCAATGGCA AACGGCTTGG ACAATCAGGC ATTTGAAGAC CAAGTGTTCC
101  ACACGCGGGC AGATGCACCG ATGCAGTTGG CGGAGCTTTC TCAAAAGGAG
151  ATGAAGGAGA CAGAGGGGGC GTTTCTTCCA TTGGCTATCT TGGGTGGTGC
201  TGCCATTGGT ATGTGGACAC AGCATGGTTT TAGTTATGCA ACGACAGGCA
251  GACCAGCTTC TGTTAGAGAT GTTGCTATTG CTGGCGGATT AGGCGCAATT
301  CCTGGTGGTG TAGGCGCCGC AGGAAAGGTT GTTTCCTTTG CTAAATATGG
351  ACGTGAGATT AAAATCGGCA ATAATATGCG GATAGCCCCT TTCGGTAATA
401  GAACAGGTCA TCCTATTGGA AAATTTCCCC ATTATCATCG TCGAGTTACG
451  GATAATACGG GCAAGACTTT GCCTGGACAG GGAATTGGTC GTCATCGCCC
501  TTGGGAATCA AAATCTACGG ACAGATCATG GAAAAACCGC TTCTAA
```

This corresponds to the amino acid sequence <SEQ ID 176; ORF30-1>:

```
  1  MKKQITAAVM MLSMIAPAMA NGLDNQAFED QVFHTRADAP MQLAELSQKE
 51  MKETEGAFLP LAILGGAAIG MWTQHGFSYA TTGRPASVRD VAIAGGLGAI
101  PGGVGAAGKV VSFAKYGREI KIGNNMRIAP FGNRTGHPIG KFPHYHRRVT
151  DNTGKTLPGQ GIGRHRPWES KSTDRSWKNR F*
```

Computer analysis of this amino acid sequence gave the following results:

Homology with a predicted ORF from *N.meninigtidis* (strain A)

ORF30 shows 97.6% identity over a 42aa overlap with an ORF (ORF30a) from strain A of *N. meningitidis:*

```
                     10        20        30        40
orf30.pep    MKKQITAAVMMLSMIAPAMANGLDNQAFEDQMFHTRADAPMQ
             |||||||||||||||||||||||||||||||:||||||||||
orf30a       MKKQITAAVMMLSMIAPAMANGLDNQAFEDQVFHTRADAPMQLAELSQKEMKXTXGAFLP
                     10        20        30        40        50        60

orf30a       LXILGGAAIGMWTQHGFSYATTGRPASVRDVAIAGGLGAIPGXVGAAGKVVSFAKYGREI
                     70        80        90       100       110       120
```

The complete length ORF30a nucleotide sequence <SEQ ID 177> is:

```
  1  ATGAAAAAAC AAATCACCGC AGCCGTAATG ATGCTGTCTA TGATTGCCCC
 51  CGCAATGGCA AACGGCTTGG ACAATCAGGC ATTTGAAGAC CAAGTGTTCC
101  ACACGCGGGC AGATGCACCG ATGCAGTTGG CGGAGCTTTC TCAAAAGGAG
151  ATGAAGGANA CAGNGGGGGC GTTTCTTCCA TTGGNTATCT TGGGTGGTGC
201  TGCCATTGGT ATGTGGACAC AGCATGGTTT TAGTTATGCA ACGACAGGCA
251  GACCAGCTTC TGTTAGAGAT GTTGCTATTG CTGGCGGATT AGGCGCAATT
301  CCTGGTGNTG TAGGCGCCGC AGGAAAGGTT GTTTCCTTTG CTAAATATGG
351  ACGTGAGATT AAAATCGGCA ATAATATGCG GATAGCCCCT TTCGGTAATA
401  GAACAGGTCA TCCTATTGGN AAATTTCCCC ATTATCATCG TCGAGTTACG
451  GATAATACGG GCAAGACTTT GCCTGGACAG GGAATTGGTC GTCATCGCCC
501  TTGGGAATCA AAATCTACGG ACAGATCATG GAAAAACCGC TTCTAA
```

This encodes a protein having amino acid sequence <SEQ ID 178>:

```
  1  MKKQITAAVM MLSMIAPAMA NGLDNQAFED QVFHTRADAP MQLAELSQKE
 51  MKXTXGAFLP LXILGGAAIG MWTQHGFSYA TTGRPASVRD VAIAGGLGAI
101  PGXVGAAGKV VSFAKYGREI KIGNNMRIAP FGNRTGHPIG KFPHYHRRVT
151  DNTGKTLPGQ GIGRHRPWES KSTDRSWKNR F*
```

ORF30a and ORF30-1 show 97.8% identity in 181 aa overlap:

```
orf30a.pep   MKKQITAAVMMLSMIAPAMANGLDNQAFEDQVFHTRADAPMQLAELSQKEMKXTXGAFLP   60
             |||||||||||||||||||||||||||||||||||||||||||||||||||| | |||||
orf30-1      MKKQITAAVMMLSMIAPAMANGLDNQAFEDQVFHTRADAPMQLAELSQKEMKETEGAFLP   60

orf30a.pep   LXILGGAAIGMWTQHGFSYATTGRPASVRDVAIAGGLGAIPGXVGAAGKVVSFAKYGREI  120
             | |||||||||||||||||||||||||||||||||||||||| |||||||||||||||||
orf30-1      LAILGGAAIGMWTQHGFSYATTGRPASVRDVAIAGGLGAIPGGVGAAGKVVSFAKYGREI  120

orf30a.pep   KIGNNMRIAPFGNRTGHPIGKFPHYHRRVTDNTGKTLPGQGIGRHRPWESKSTDRSWKNR  180
             ||||||||||||||||||||||||||||||||||||||||||||||||||||||||||||
orf30-1      KIGNNMRIAPFGNRTGHPIGKFPHYHRRVTDNTGKTLPGQGIGRHRPWESKSTDRSWKNR  180

orf30a.pep   FX
             ||
orf30-1      FX
```

<u>Homology with a predicted ORF from *N.gonorrhoeae*</u>

ORF30 shows 97.6% identity over a 42aa overlap with a predicted ORF (ORF30.ng) from *N. gonorrhoeae:*

```
orf30.pep   MKKQITAAVMMLSMIAPAMANGLDNQAFEDQMFHTRADAPMQ                    42
            |||||||||||||||||||||||||||||||:||||||||||
orf30ng     MKKQITAAVMMLSMIAPAMANGLDNQAFEDQVFHTRADAPMQLAELSQKEMKETEGAFLP  60
```

The complete length ORF30ng nucleotide sequence <SEQ ID 179> is

```
  1  ATGAAAAAAC AAATCACCGC AGCCGTAATG ATGCTGTCTA TGATCGCCCC
 51  CGCAATGGCA AACGGATTGG ACAATCAGGC ATTTGAAGAC CAAGTGTTCC
101  ACACGCGGGC AGATGCGCCG ATGCAGTTGG CGGAGCTTTC TCAGAAGGAG
151  ATGAAGGAGA CTGAAGGGGC TTTTCTTCCA TTGGCTATCT TGGGTGGTGC
```

```
201  TGCCATTGGT ATGTGGACAC AGCATGGTTT TAGTTATGCA ACGACAGGCA
251  GACCAGCTTC TGTTAGAGAT GTTGCTGGCG GATTAGGCGC AATTCCTGGT
301  GATGTAGGTG CTGCAGGAAA GGTTGTTTCC TTTGCTAAAT ATGGACGTGA
351  GATTAAAATC GGCAATAATA TGCGGATAGC CCCTTTCGGT AATAGAACAG
401  GTCATCCTAT TGGAAAATTT CCCCATTATC ATCGTCGAGT TACGGATAAT
451  ACGGGCAAGA CTTTGCCTGG ACAGGGAATT GGTCGTCATC GCCCTTGGGA
501  ATCAAAATCT ACGGACAGAT CATGGAAAAA CCGCTTCTAA
```

This encodes a protein having amino acid sequence <SEQ ID 180>:

```
  1  MKKQITAAVM MLSMIAPAMA NGLDNQAFED QVFHTRADAP MQLAELSQKE
 51  MKETEGAFLP LAILGGAAIG MWTQHGFSYA TTGRPASVRD VAGGLGAIPG
101  DVGAAGKVVS FAKYGREIKI GNNMRIAPFG NRTGHPIGKF PHYHRRVTDN
151  TGKTLPGQGI GRHRPWESKS TDRSWKNRF*
```

ORF30ng and ORF30-1 show 98.3% identity in 181 aa overlap:

```
                       10        20        30        40        50        60
orf30ng.pep    MKKQITAAVMMLSMIAPAMANGLDNQAFEDQVFHTRADAPMQLAELSQKEMKETEGAFLP
               ||||||||||||||||||||||||||||||||||||||||||||||||||||||||||||
orf30-1        MKKQITAAVMMLSMIAPAMANGLDNQAFEDQVFHTRADAPMQLAELSQKEMKETEGAFLP
                       10        20        30        40        50        60

                       70        80        90          100       110
orf30ng.pep    LAILGGAAIGMWTQHGFSYATTGRPASVRDVA--GGLGAIPGDVGAAGKVVSFAKYGREI
               ||||||||||||||||||||||||||||||||  |||||||| |||||||||||||||||
orf30-1        LAILGGAAIGMWTQHGFSYATTGRPASVRDVAIAGGLGAIPGGVGAAGKVVSFAKYGREI
                       70        80        90       100       110       120

              120       130       140       150       160       170
orf30ng.pep    KIGNNMRIAPFGNRTGHPIGKFPHYHRRVTDNTGKTLPGQGIGRHRPWESKSTDRSWKNR
               ||||||||||||||||||||||||||||||||||||||||||||||||||||||||||||
orf30-1        KIGNNMRIAPFGNRTGHPIGKFPHYHRRVTDNTGKTLPGQGIGRHRPWESKSTDRSWKNR
                      130       140       150       160       170       180

              180
orf30ng.pep    FX
               ||
orf30-1        FX
```

Based on this analysis, including the presence of a putative leader sequence in the gonococcal protein, it is predicted that the proteins from *N.meningitidis* and *N.gonorrhoeae,* and their epitopes, could be useful antigens for vaccines or diagnostics, or for raising antibodies.

**Example 22**

The following partial DNA sequence was identified in *N.meningitidis* <SEQ ID 181>:

```
  1  ATGAATAAAA CTCTCTATCG TGTAATTTTC AACCGCAAAC GTGGGGCTGT
 51  GrTAGCCGTT GCTGAAACTA CCAAGCGCGA AGGTAAAAGC TGTGCCGATA
101  GTGATTCAGG CAGCGCTCAT GTGAAATCTG TTCCTTTTGG TACTACTCAT
151  GCACCTGTTT GTg.CGTTaC AAATATCTTT TCTTTTTCTT TATTGGGCTT
201  TTCTTTATGT TTGGCTGTAG GtacGGyCAA TATTGCTTTT GCTGATGGCA
251  TT..
```

This corresponds to the amino acid sequence <SEQ ID 182; ORF31>:

```
 1  MNKTLYRVIF NRKRGAVXAV AETTKREGKS CADSDSGSAH VKSVPFGTTH
51  APVCXVTNIF SFSLLGFSLC LAVGTXNIAF ADGI..
```

Further work revealed a further partial nucleotide sequence <SEQ ID 183>:

```
  1  ATGAATAAAA CTCTCTATCG TGTAATTTTC AACCGCAAAC GTGGGGCTGT
 51  GGTAGCCGTT GCTGAAACTA CCAAGCGCGA AGGTAAAAGC TGTGCCGATA
101  GTGATTCAGG CAGCGCTCAT GTGAAATCTG TTCCTTTTGG TACTACTCAT
151  GCACCTGTTT GTCGTTCAAA TATCTTTTCT TTTTCTTTAT TGGGCTTTTC
201  TTTATGTTTG GCTGTAGGTA CGGCCAATAT TGCTTTTGCT GATGGCATT..
```

This corresponds to the amino acid sequence <SEQ ID 184; ORF31-1>:

```
 1  MNKTLYRVIF NRKRGAVVAV AETTKREGKS CADSDSGSAH VKSVPFGTTH
51  APVCRSNIFS FSLLGFSLCL AVGTANIAFA DGI..
```

Computer analysis of this amino acid sequence gave the following results:

<u>Homology with a predicted ORF from *N.gonorrhoeae*</u>

ORF31 shows 76.2% identity over a 84aa overlap with a predicted ORF (ORF31.ng) from *N. gonorrhoeae:*

```
orf31.pep   MNKTLYRVIFNRKRGAVXAVAETTKREGKSCADSDSGSAHVKSVPFGTTHAPVCXVTNIF   60
            ||||||||||||||||| ||||||||||||||| |||::|||| |   ||       :: |
orf31ng     MNKTLYRVIFNRKRGAVVAVAETTKREGKSCADSGSGSVYVKSVSFIPTH------SKAF   54

orf31.pep   SFSLLGFSLCLAVGTXNIAFADGI                                      84
             || ||||||||:|| ||||||||
orf31ng     CFSALGFSLCLALGTVNIAFADGIITDKAAPKTQQATILQTGNGIPQVNIQTPTSAGVSV  114
```

The complete length ORF31ng nucleotide sequence <SEQ ID 185> is:

```
  1  ATGAACAAAA CCCTCTATCG TGTGATTTTC AACCGCAAAC GCGGTGCTGT
 51  GGTAGCTGTT GCCGAAACCA CCAAGCGCGA AGGTAAAAGC TGTGCCGATA
101  GTGGTTCGGG CAGCGTTTAT GTGAAATCCG TTTCTTTCAT TCCTACTCAT
151  TCCAAAGCCT TTTGTTTTTC TGCATTAGGC TTTTCTTTAT GTTTGGCTTT
201  GGGTACGGTC AATATTGCTT TTGCTGACGG CATTATTACT GATAAAGCTG
251  CTCCTAAAAC CCAACAAGCC ACGATTCTGC AAACAGGTaa cGGCATACCG
301  CAAGTCAATA TTCAAACCCC TACTTCGGCA GGGGTTTCTG TTAATCAATA
351  TGCCCAGTTT GATGTGGGTA ATCGCGGGGC GATTTTAAAC AACAGTCGCA
401  GCAACACCCA AACACAGCTA GGCGGTTGGA TTCAAGGCAA TCCTTGGTTG
451  ACAAGGGGCG AAGCACGTGT GGTTGTAAAC CAAATCAACA GCAGCCATCC
501  TTCACAACTG AATGGCTATA TTGAAGTGGG TGGACGACGT GCAGAAGTCG
551  TTATTGCCAA TCCGGCAGGG ATTGCAGTCA ATGGTGGTGG TTTTATCAAT
601  GCTTCCCGTG CCACTTTGAC GACAGGCCAA CCGCAATATC AAGCAGGAGA
651  CTTTAGCGGC TTTAAGATAA GGCAAGGCAA TGCTGTAATC GCCGGACACG
701  GTTTGGATGC CCGTGATACC GATTTCACAC GTATTCTTGT ATGCCAACAA
751  AATCACCTTG ATCAGTACGG CCGAACAAGC AGGCATTCGT AA
```

This encodes a protein having amino acid sequence <SEQ ID 186>:

```
  1  MNKTLYRVIF NRKRGAVVAV AETTKREGKS CADSGSGSVY VKSVSFIPTH
 51  SKAFCFSALG FSLCLALGTV NIAFADGIIT DKAAPKTQQA TILQTGNGIP
101  QVNIQTPTSA GVSVNQYAQF DVGNRGAILN NSRSNTQTQL GGWIQGNPWL
151  TRGEARVVVN QINSSHPSQL NGYIEVGGRR AEVVIANPAG IAVNGGGFIN
201  ASRATLTTGQ PQYQAGDFSG FKIRQGNAVI AGHGLDARDT DFTRILVCQQ
251  NHLDQYGRTS RHS*
```

This gonococcal protein shares 50% identity over a 149aa overlap with the pore-forming hemolysins-like HecA protein from *Erwinia chrysanthemi* (accession number L39897):

```
orf31ng  96  GNGIPQVNIQTPTSAGVSVNQYAQFDVGNRGAILNNSRSN-TQTQLGGWIQGNPWLTRGE 154
             GNG+P VNI TP ++G+S N+Y  F+V NRG ILNN   +   T +QLGG IQ NP L
HecA     45  GNGVPVVNIATPDASGLSHNRYHDFNVDNRGLILNNGTARLTPSQLGGLIQNNPNLNGRA 104

Orf31ng  155 ARVVVNQINSSHPSQLNGYIEVGGRRAEVVIANPAGIAVNGGGFINASRATLTTGQPQYQ 214
             A  ++N++ S + S+L GY+EV G+ A VV+ANP GI  +G GF+N  R TLTTG PQ+
HecA     105 AAAILNEVVSPNRSRLAGYLEVAGQAANVVVANPYGITCSGCGFLNTPRLTLTTGTPQFD 164

Orf31ng  215 -AGDFSGFKIRQGNAVIAGHGLDARDTDF 242
              AG  SG  +R G+ +I G GLDA  +D+
HecA     165 AAGGLSGLDVRGGDILIDGAGLDASRSDY 193
```

Furthermore, ORF31ng and ORF31-1 show 79.5% identity in 83 aa overlap:

```
                      10        20        30        40        50        60
   orf31-1.pep  MNKTLYRVIFNRKRGAVVAVAETTKREGKSCADSDSGSAHVKSVPFGTTHAPVCRSNIFS
                |||||||||||||||||||||||||||||||||| |||::|||| |  ||      |: |
   orf31ng      MNKTLYRVIFNRKRGAVVAVAETTKREGKSCADSGSGSVYVKSVSFIPTH-----SKAFC
                        10        20        30        40        50

                        70        80
   orf31-1.pep  FSLLGFSLCLAVGTANIAFADGI
                ||  |||||||:||:||||||||
   orf31ng      FSALGFSLCLALGTVNIAFADGIITDKAAPKTQQATILQTGNGIPQVNIQTPTSAGVSVN
                   60        70        80        90       100       110
```

On this basis, including the homology with hemolysins, and also with adhesins, it is predicted that the proteins from *N.meningitidis* and *N.gonorrhoeae,* and their epitopes, could be useful antigens for vaccines or diagnostics, or for raising antibodies.

**Example 23**

The following partial DNA sequence was identified in *N.meningitidis* <SEQ ID 187>:

```
  1  ATGAATACTC CTCCTTTTGT CTGTTGGATT TTTTGCAAGG TCATCGACAA
 51  TTTCGGCGAC ATCGGCGTTT CGTGGCGGCT CGCCCGTGTT TTGCACCGCG
101  AACTCGGTTG GCAGGTGCAT TTGTGGACGG ACGATGTGTC CGCCTTGCGT
151  GCGCTTTGCC CTGATTTGCC CGATGTTCCC TGCGTTCATC AGGATATTCA
201  TGTCCGCACT TGGCATTCCG ATGCGGCAGA TATTGATACC GCG..
```

This corresponds to the amino acid sequence <SEQ ID 188; ORF32>:

```
 1  MNTPPFVCWI FCKVIDNFGD IGVSWRLARV LHRELGWQVH LWTDDVSALR
51  ALCPDLPDVP CVHQDIHVRT WHSDAADIDT A..
```

Further work revealed the complete nucleotide sequence <SEQ ID 189>:

```
   1  ATGAATACTC CTCCTTTTGT CTGTTGGATT TTTTGCAAGG TCATCGACAA
  51  TTTCGGCGAC ATCGGCGTTT CGTGGCGGCT CGCCCGTGTT TTGCACCGCG
 101  AACTCGGTTG GCAGGTGCAT TTGTGGACGG ACGATGTGTC CGCCTTGCGT
 151  GCGCTTTGCC CTGATTTGCC CGATGTTCCC TGCGTTCATC AGGATATTCA
 201  TGTCCGCACT TGGCATTCCG ATGCGGCAGA TATTGATACC GCGCCTGTTC
 251  CCGATGTCGT CATCGAAACT TTTGCCTGCG ACCTGCCCGA AAATGTGCTG
 301  CACATTATCC GCCGACACAA GCCGCTTTGG CTGAATTGGG AATATTTGAG
 351  CGCGGAGGAA AGCAATGAAA GGCTGCATCT GATGCCTTCG CCGCAGGAGG
 401  GTGTTCAAAA ATATTTTTGG TTTATGGGTT TCAGCGAAAA AAGCGGCGGG
 451  TTGATACGCG AACGTGATTA CTGCGAAGCC GTCCGTTTCG ATACTGAAGC
 501  CCTGCGAGAG CGGCTGATGC TGCCCGAAAA AAACGCCTCC GAATGGCTGC
 551  TTTTCGGCTA TCGGAGCGAT GTTTGGGCAA AGTGGCTGGA AATGTGGCGA
 601  CAGGCAGGCA GCCCGATGAC ACTGTTGCTG GCGGGGACGC AAATCATCGA
 651  CAGCCTCAAA CAAAGCGGCG TTATTCCGCA AGATGCCCTG CAAAACGACG
 701  GCGATGTTTT TCAGACGGCA TCCGTCCGCC TCGTCAAAAT CCCTTTCGTG
 751  CCGCAACAGG ACTTCGACCA ACTGCTGCAC CTTGCCGACT GCGCCGTCAT
 801  CCGCGGCGAA GACAGTTTCG TGCGCGCCCA GCTTGCGGGC AAACCCTTCT
 851  TTTGGCACAT CTACCCGCAA GACGAGAATG TCCATCTCGA CAAACTCCAC
 901  GCCTTTTGGG ATAAGGCACA CGGTTTCTAC ACGCCCGAAA CCGTGTCGGC
 951  ACACCGCCGT CTTTCGGACG ACCTCAACGG CGGAGAGGCT TTATCCGCAA
1001  CACAACGCCT CGAATGTTGG CAAACCCTGC AACAACATCA AAACGGCTGG
1051  CGGCAAGGCG CGGAGGATTG GAGCCGTTAT CTTTTCGGGC AGCCGTCAGC
1101  TCCTGAAAAA CTCGCTGCCT TTGTTTCAAA GCATCAAAAA ATACGCTAG
```

This corresponds to the amino acid sequence <SEQ ID 190; ORF32-1>:

```
  1  MNTPPFVCWI FCKVIDNFGD IGVSWRLARV LHRELGWQVH LWTDDVSALR
 51  ALCPDLPDVP CVHQDIHVRT WHSDAADIDT APVPDVVIET FACDLPENVL
101  HIIRRHKPLW LNWEYLSAEE SNERLHLMPS PQEGVQKYFW FMGFSEKSGG
151  LIRERDYCEA VRFDTEALRE RLMLPEKNAS EWLLFGYRSD VWAKWLEMWR
201  QAGSPMTLLL AGTQIIDSLK QSGVIPQDAL QNDGDVFQTA SVRLVKIPFV
251  PQQDFDQLLH LADCAVIRGE DSFVRAQLAG KPFFWHIYPQ DENVHLDKLH
301  AFWDKAHGFY TPETVSAHRR LSDDLNGGEA LSATQRLECW QTLQQHQNGW
351  RQGAEDWSRY LFGQPSAPEK LAAFVSKHQK IR*w
```

Computer analysis of this amino acid sequence gave the following results:

<u>Homology with a predicted ORF from *N.meningitidis* (strain A)</u>

ORF32 shows 93.8% identity over a 81aa overlap with an ORF (ORF32a) from strain A of *N. meningitidis:*

```
                        10        20        30        40        50        60
orf32.pep    MNTPPFVCWIFCKVIDNFGDIGVSWRLARVLHRELGWQVHLWTDDVSALRALCPDLPDVP
             ||||||    ||||||||||||||||||||||||||||||||||||||||||||||||||
orf32a       MNTPPFSAGXFCKVIDNFGDIGVSWRLARVLHRELGWQVHLWTDDVSALRALCPDLPDVX
                        10        20        30        40        50        60

                        70        80
orf32.pep    CVHQDIHVRTWHSDAADIDTA
             |||||||||||||||||||||
orf32a       CVHQDIHVRTWHSDAADIDTAPVXDVVIETFACDLPENVLHIIRRHKPLWLXWEYLSAEX
                        70        80        90       100       110       120
```

The complete length ORF32a nucleotide sequence <SEQ ID 191> is:

```
   1 ATGAATACTC CTCCTTTTTC TGCTGGANTT TTTTGCAAGG TCATCGACAA
  51 TTTCGGCGAC ATCGGCGTTT CGTGGCGGCT TGCCCGTGTT TTGCACCGCG
 101 AACTCGGTTG GCAGGTGCAT TTGTGGACGG ACGATGTGTC CGCCTTGCGT
 151 GCGCTTTGCC CTGATTTGCC CGATGTTCNC TGCGTTCATC AGGATATTCA
 201 TGTCCGCACT TGGCATTCCG ATGCGGCAGA TATTGATACC GCGCCTGTTC
 251 NCGATGTCGT CATCGAAACT TTTGCCTGCG ACCTGCCCGA AAATGTGCTG
 301 CACATCATCC GCCGACACAA GCCGCTTTGG CTGAANTGGG AATATTTGAG
 351 CGCGGAGGAN AGCAATGAAA GGCTGCACNT GATGCCTTCG CCGCAGGAGA
 401 GTGTTCNAAA ATANTTTTGG TTTATGGGTT TCAGCGAANN NAGCGGCGGA
 451 CTGATACGCG AACGCGATTA CTGCGAAGCC GTCCGTTTCG ATAGCGGAGC
 501 CTTGCGCAAG AGGCTGATGC TTCCCGAAAA AAACGNCCCC GAATGGCTGC
 551 TTTTCGGCTA TCGGAGCGAT GTTTGGGCAA AGTGGCTGGA AATGTGGCGA
 601 CAGGCAGGCA GTCCGTTGAC ACTTTTGCTG GCNGGGGCGC ANATTATCGA
 651 CAGCCTCAAA CAAAACGGCG TTATTCCGCA AGATGCCCTG CAAAACGACG
 701 GCGATGTTTT TCAGACGGCA TCCGTCCGCC TCGTCAAAAT CCCTTTCGTG
 751 CCGCAACAGG ACTTCGACAA ACTGCTGCAC CTTGCCGACT GCGCCGTCAT
 801 CCGCGGCGAA GACAGTTTCG TGCGCGCCCA GCTTGCGGGC AAACCCTTCT
 851 TTTGGCACAT CTACCCGCAA GATGAGAATG TCCATCTCGA CAAACTCCAC
 901 GCCTTTTGGG ATAAGGCACA CGGTTTCTAC ACGCCCGAAA CCGCATCGGC
 951 ACACCGCCGC CTTTCAGACG ACCTCAACGG CGGAGAGGCT TTATCCGCAA
1001 CACAACGCCT CGAATGTTGG CAAATCCTGC AACAACATCA AAACGGCTGG
1051 CGGCAAGGCG CGGAGGATTG GAGCCGTTAT CTTTTTGGGC AGCCTTCCGC
1101 ATCCGAAAAA CTCGCCGCCT TTGTTTCAAA GCATCAAAAA ATACGCTAG
```

This encodes a protein having amino acid sequence <SEQ ID 192>:

```
  1 MNTPPFSAGX FCKVIDNFGD IGVSWRLARV LHRELGWQVH LWTDDVSALR
 51 ALCPDLPDVX CVHQDIHVRT WHSDAADIDT APVXDVVIET FACDLPENVL
101 HIIRRHKPLW LXWEYLSAEX SNERLHXMPS PQESVXKXFW FMGFSEXSGG
151 LIRERDYCEA VRFDSGALRK RLMLPEKNXP EWLLFGYRSD VWAKWLEMWR
201 QAGSPLTLLL AGAXIIDSLK QNGVIPQDAL QNDGDVFQTA SVRLVKIPFV
251 PQQDFDKLLH LADCAVIRGE DSFVRAQLAG KPFFWHIYPQ DENVHLDKLH
301 AFWDKAHGFY TPETASAHRR LSDDLNGGEA LSATQRLECW QILQQHQNGW
351 RQGAEDWSRY LFGQPSASEK LAAFVSKHQK IR*
```

ORF32a and ORF32-1 show 93.2% identity in 382 aa overlap:

```
                       10        20        30        40        50        60
orf32-1.pep     MNTPPFVCWIFCKVIDNFGDIGVSWRLARVLHRELGWQVHLWTDDVSALRALCPDLPDVP
                ||||||    ||||||||||||||||||||||||||||||||||||||||||||||||||| 
orf32a          MNTPPFSAGXFCKVIDNFGDIGVSWRLARVLHRELGWQVHLWTDDVSALRALCPDLPDVX
                       10        20        30        40        50        60

                       70        80        90       100       110       120
orf32-1.pep     CVHQDIHVRTWHSDAADIDTAPVPDVVIETFACDLPENVLHIIRRHKPLWLNWEYLSAEE
                ||||||||||||||||||||||| ||||||||||||||||||||||||||| ||||||| 
orf32a          CVHQDIHVRTWHSDAADIDTAPVXDVVIETFACDLPENVLHIIRRHKPLWLXWEYLSAEX
                       70        80        90       100       110       120

                      130       140       150       160       170       180
orf32-1.pep     SNERLHLMPSPQEGVQKYFWFMGFSEKSGGLIRERDYCEAVRFDTEALRERLMLPEKNAS
```

```
                 |||||| ||||||:| | |||||||| ||||||||||||||||: |||:||||||||
orf32a           SNERLHXMPSPQESVXKXFWFMGFSEXSGGLIRERDYCEAVRFDSGALRKRLMLPEKNXP
                         130       140       150       160       170       180

                         190       200       210       220       230       240
orf32-1.pep      EWLLFGYRSDVWAKWLEMWRQAGSPMTLLLAGTQIIDSLKQSGVIPQDALQNDGDVFQTA
                 |||||||||||||||||||||||||:||||||: |||||||:||||||||||||||||||
orf32a           EWLLFGYRSDVWAKWLEMWRQAGSPLTLLLAGAXIIDSLKQNGVIPQDALQNDGDVFQTA
                         190       200       210       220       230       240

                         250       260       270       280       290       300
orf32-1.pep      SVRLVKIPFVPQQDFDQLLHLADCAVIRGEDSFVRAQLAGKPFFWHIYPQDENVHLDKLH
                 ||||||||||||||||:|||||||||||||||||||||||||||||||||||||||||||
orf32a           SVRLVKIPFVPQQDFDKLLHLADCAVIRGEDSFVRAQLAGKPFFWHIYPQDENVHLDKLH
                         250       260       270       280       290       300

                         310       320       330       340       350       360
orf32-1.pep      AFWDKAHGFYTPETVSAHRRLSDDLNGGEALSATQRLECWQTLQQHQNGWRQGAEDWSRY
                 ||||||||||||||:|||||||||||||||||||||||||| ||||||||||||||||||
orf32a           AFWDKAHGFYTPETASAHRRLSDDLNGGEALSATQRLECWQILQQHQNGWRQGAEDWSRY
                         310       320       330       340       350       360

                         370       380
orf32-1.pep      LFGQPSAPEKLAAFVSKHQKIRX
                 ||||||| |||||||||||||||
orf32a           LFGQPSASEKLAAFVSKHQKIRX
                         370       380
```

## Homology with a predicted ORF from *N.gonorrhoeae*

ORF32 shows 95.1 % identity over a 82aa overlap with a predicted ORF (ORF32.ng) from *N. gonorrhoeae:*

```
orf32.pep     MNTPPF-VCWIFCKVIDNFGDIGVSWRLARVLHRELGWQVHLWTDDVSALRALCPDLP   57
                |||  | |||||||||||||||||||||||||||||||||||||||||||||||||||
orf32ng     MVMNTYAFPVCWIFCKVIDNFGDIGVSWRLARVLHRELGWQVHLWTDDVSALRALCPDLP   60

orf32.pep   DVPCVHQDIHVRTWHSDAADIDTA                                       81
            ||| ||||||||||||||||||||
orf32ng     DVPFVHQDIHVRTWHSDAADIDTAPVPDAVIETFACDLPENVLNIIRRHKPLWLNWEYLS  120
```

An ORF32ng nucleotide sequence <SEQ ID 193> was predicted to encode a protein having amino acid sequence <SEQ ID 194>:

```
  1  MVMNTYAFPV CWIFCKVIDN FGDIGVSWRL ARVLHRELGW QVHLWTDDVS
 51  ALRALCPDLP DVPFVHQDIH VRTWHSDAAD IDTAPVPDAV IETFACDLPE
101  NVLNIIRRHK PLWLNWEYLS AEESNERLHL MPSPQEGVQK YFWFMGFSEK
151  SGGLIRERDY REAVRFDTEA LRRRLVLPEK NAPEWLLFGY RGDVWAKWLD
201  MWQQAGSLMT LLLAGAQIID SLKQSGVIPQ NALQNEGGVF QTASVRLVKI
251  PFVPQQDFDK LLHLADCAVI RGEDSFVRTQ LAGKPFFWHI YPQDENVHLD
301  KLHAFWDKAY GFYTPETASV HRLLSDDLNG GEALSATQRL ECGVL*
```

Further sequencing revealed the following DNA sequence <SEQ ID 195>:

```
   1 ATGAATACAT ACGCTTTTCC TGTCTGTTGG ATTTTTTGCA AGGTCATCGA
  51 CAATTTCGGC GACATCGGCG TTTCGTGGCG GCTCGCCCGT GTTTTGCACC
 101 GCGAACTCGG TTGGCAGGTG CATTTGTGGA CGGACGACGT GTCCGCCTTG
 151 CGCGCGCTTT GTCCCGATTT GCCCGATGTT CCCTTCGTTC ATCAGGATAT
 201 TCATGTCCGC ACTTGGCATT CCGATGCGGC AGACATTGAT ACCGCGCCCG
 251 TTCCCGATGC CGTTATCGAA ACTTTTGCCT GCGACCTGCC CGAAAATGTG
 301 CTGAACATCA TCCGCCGACA CAAACCGCTT TGGCTGAATT GGGAATATTT
 351 GAGCGCGGAG GAAAGCAATG AAAGGCTGCA CCTGATGCCT TCGCCGCAGG
 401 AGGGCGTTCA AAAATATTTT TGGTTTATGG GTTTCAGCGA AAAAAGCGGC
 451 GGGTTGATAC GCGAACGCGA TTACCGCGAA GCCGTCCGTT TCGATACCGA
 501 AGCCCTGCGC CGGCGGCTGG TGCTGCCCGA AAAAAACGCC CCCGAATGGC
 551 TGCTTTTCGG CTATCGGGGC GATGTTTGGG CAAAGTGGCT GGACATGTGG
 601 CAACAGGCAG GCAGCCTGAT GACCCTACTG CTGGCGGGGG CGCAAATTAT
 651 CGACAGCCTC AAACAAAGCG GCGTTATTCC GCAAAACGCC CTGCAAAAtg
```

```
 701 aaggcgGTGT CTTTCagacG gcatccgTcC gccttGTCAA AAtcCCGTTC
 751 GTGCcGCAAC AGGAcTTCGA CAAATTGCTG CAcctcgcCG ACTGCGCCGT
 801 GATACGCGGC GAAGACAGTT TCGTGCGTAC CCAGCTTGCC GGAAAACCCT
 851 TTTTTTGGCA CATCTACCCG CAAGACGAGA ATGTCCATCT CGACAAACTC
 901 CACGCCTTTT GGGATAAGGC ATACGGCTTC TACACGCCCG AAACCGCATC
 951 GGTGCACCGC CTCCTTTCGG ACGACCTCAA CGGCGGAGAG GCTTTATCCG
1001 CAACACAACG CCTCGAATGT TGGCAAACCC TGCAACAACA TCAAAACGGC
1051 TGGCGGCAAG GCGCGGAGGA TTGGAGCCGT TATCTTTTCG GGCAGCCTTC
1101 CGCATCCGAA AAACTCGCCG CCTTTGTTTC AAAGCATCAA AAAATACGCT
1151 AG
```

This encodes a protein having amino acid sequence <SEQ ID 196; ORF32ng-l>:

```
  1 MNTYAFPVCW IFCKVIDNFG DIGVSWRLAR VLHRELGWQV HLWTDDVSAL
 51 RALCPDLPDV PFVHQDIHVR TWHSDAADID TAPVPDAVIE TFACDLPENV
101 LNIIRRHKPL WLNWEYLSAE ESNERLHLMP SPQEGVQKYF WFMGFSEKSG
151 GLIRERDYRE AVRFDTEALR RRLVLPEKNA PEWLLFGYRG DVWAKWLDMW
201 QQAGSLMTLL LAGAQIIDSL KQSGVIPQNA LQNEGGVFQT ASVRLVKIPF
251 VPQQDFDKLL HLADCAVIRG EDSFVRTQLA GKPFFWHIYP QDENVHLDKL
301 HAFWDKAYGF YTPETASVHR LLSDDLNGGE ALSATQRLEC WQTLQQHQNG
351 WRQGAEDWSR YLFGQPSASE KLAAFVSKHQ KIR*
```

ORF32ng-1 and ORF32-1 show 93.5% identity in 383 aa overlap:

```
                         10        20        30        40        50        59
orf32-1.pep      MNTPPF-VCWIFCKVIDNFGDIGVSWRLARVLHRELGWQVHLWTDDVSALRALCPDLPDV
                 |||  | |||||||||||||||||||||||||||||||||||||||||||||||||||||
orf32ng-1        MNTYAFPVCWIFCKVIDNFGDIGVSWRLARVLHRELGWQVHLWTDDVSALRALCPDLPDV
                        10        20        30        40        50        60

                60        70        80        90       100       110       119
orf32-1.pep      PCVHQDIHVRTWHSDAADIDTAPVPDVVIETFACDLPENVLHIIRRHKPLWLNWEYLSAE
                 | ||||||||||||||||||||||||:||||||||||||||:||||||||||||||||||
orf32ng-1        PFVHQDIHVRTWHSDAADIDTAPVPDAVIETFACDLPENVLNIIRRHKPLWLNWEYLSAE
                        70        80        90       100       110       120

               120       130       140       150       160       170       179
orf32-1.pep      ESNERLHLMPSPQEGVQKYFWFMGFSEKSGGLIRERDYCEAVRFDTEALRERLMLPEKNA
                 |||||||||||||||||||||||||||||||||||||| |||||||||||:||:||||||
orf32ng-1        ESNERLHLMPSPQEGVQKYFWFMGFSEKSGGLIRERDYREAVRFDTEALRRRLVLPEKNA
                       130       140       150       160       170       180

               180       190       200       210       220       230       239
orf32-1.pep      SEWLLFGYRSDVWAKWLEMWRQAGSPMTLLLAGTQIIDSLKQSGVIPQDALQNDGDVFQT
                  ||||||||:|||||||:||:|||| |||||||:||||||||||||||:||||:| ||||
orf32ng-1        PEWLLFGYRGDVWAKWLDMWQQAGSLMTLLLAGAQIIDSLKQSGVIPQNALQNEGGVFQT
                       190       200       210       220       230       240

               240       250       260       270       280       290       299
orf32-1.pep      ASVRLVKIPFVPQQDFDQLLHLADCAVIRGEDSFVRAQLAGKPFFWHIYPQDENVHLDKL
                 |||||||||||||||||:||||||||||||||||||:|||||||||||||||||||||||
orf32ng-1        ASVRLVKIPFVPQQDFDKLLHLADCAVIRGEDSFVRTQLAGKPFFWHIYPQDENVHLDKL
                       250       260       270       280       290       300

               300       310       320       330       340       350       359
orf32-1.pep      HAFWDKAHGFYTPETVSAHRRLSDDLNGGEALSATQRLECWQTLQQHQNGWRQGAEDWSR
                 |||||||:|||||||:|:|| |||||||||||||||||||||||||||||||||||||||
orf32ng-1        HAFWDKAYGFYTPETASVHRLLSDDLNGGEALSATQRLECWQTLQQHQNGWRQGAEDWSR
                       310       320       330       340       350       360

               360       370       380
orf32-1.pep      YLFGQPSAPEKLAAFVSKHQKIRX
                 |||||||| |||||||||||||||
orf32ng-1        YLFGQPSASEKLAAFVSKHQKIRX
                       370       380
```

On this basis, including the RGD sequence in the gonococcal protein, characteristic of adhesins, it is predicted that the proteins from *N.meningitidis* and *N.gonorrhoeae,* and their epitopes, could be useful antigens for vaccines or diagnostics, or for raising antibodies.

ORF32-1 (42kDa) was cloned in pET and pGex vectors and expressed in *E.coli,* as described above. The products of protein expression and purification were analyzed by SDS-PAGE. Figure 7A shows the results of affinity purification of the His-fusion protein, and Figure 7B shows the results of expression of the GST-fusion in *E.coli.* Purified His-fusion protein was used to immunise mice, whose sera were used for ELISA, giving a positive result. These experiments confirm that ORF32-1 is a surface-exposed protein, and that it is a useful immunogen.

**Example 24**

The following partial DNA sequence was identified in *N.meningitidis* <SEQ ID 197>:

```
  1  ..TTGTTCCTGC GTGTNAAAGT GGGGCGTTTT TTCAGCAGTC CGGCGACGTG
 51    GTTTCGGGNC AAAGACCCTG TAAATCAGGC GGTGTTGCGG CTGTATNCGG
101    ACGAGTGGCG GCA.ACTTCG GTACGTTGGA AAATAGNCGC AACGTCGCAC
151    AGCCTGTGGC TCTGCACGCT GCTCGGAATG CTGGTGTCGG TATTGTTGCT
201    GCTTTTGGTG CGGCAATATA CGTTCAACTG GGAAAGCACG CTGTTGAGCA
251    ATGCCGCTTC GGTACGCGCG GTGGAAATGT TGGCATGGCT GCCGTCGAAA
301    CTCGGTTTCC CTGTCCCCGA TGCGCGGTCG GTCATCGAAG GCCGTCTGAA
351    CGGCAATATT GCCGATGCGC GGGCTTGGTC GGGGCTGCTG GTCGNCAGTA
401    TCGCCTGCTA NGGCATCCTG CCGCGCCTG..
```

This corresponds to the amino acid sequence <SEQ ID 198; ORF33>:

```
  1  ..LFLRVKVGRF FSSPATWFRX KDPVNQAVLR LYXDEWRXTS VRWKIXATSH
 51    SLWLCTLLGM LVSVLLLLLV RQYTFNWEST LLSNAASVRA VEMLAWLPSK
101    LGFPVPDARS VIEGRLNGNI ADARAWSGLL VXSIACXGIL PRL..
```

Further work revealed the complete nucleotide sequence <SEQ ID 199>:

```
   1  ATGTTGAATC CATCCCGAAA ACTGGTTGAG CTGGTCCGTA TTTTGGACGA
  51  AGGCGGTTTT ATTTTCAGCG GCGATCCCGT ACAGGCGACG GAGGCTTTGC
 101  GCCGCGTGGA CGGCAGTACG GAGGAAAAAA TCATCCGTCG GCGGAGATG
 151  ATTGACAGGA ACCGTATGCT GCGGGAGACG TTGGAACGTG TGCGTGCGGG
 201  GTCGTTCTGG TTGTGGGTGG TGGCGGCGAC GTTTGCATTT TTTACCGGTT
 251  TTTCAGTCAC TTATCTTCTA ATGGACAATC AGGGTCTGAA TTTCTTTTTG
 301  GTTTTGGCGG GCGTGTTGGG CATGAATACG CTGATGCTGG CAGTATGGTT
 351  GGCAATGTTG TTCCTGCGTG TGAAAGTGGG GCGTTTTTTC AGCAGTCCGG
 401  CGACGTGGTT TCGGGGCAAA GACCCTGTAA ATCAGGCGGT GTTGCGGCTG
 451  TATGCGGACG AGTGGCGGCA ACCTTCGGTA CGTTGGAAAA TAGGCGCAAC
 501  GTCGCACAGC CTGTGGCTCT GCACGCTGCT CGGAATGCTG GTGTCGGTAT
 551  TGTTGCTGCT TTTGGTGCGG CAATATACGT TCAACTGGGA AAGCACGCTG
 601  TTGAGCAATG CCGCTTCGGT ACGCGCGGTG GAAATGTTGG CATGGCTGCC
 651  GTCGAAACTC GGTTTCCCTG TCCCCGATGC GCGGGCGGTC ATCGAAGGCC
 701  GTCTGAACGG CAATATTGCC GATGCGCGGG CTTGGTCGGG GCTGCTGGTC
 751  GGCAGTATCG CCTGCTACGG CATCCTGCCG CGCCTGCTGG CTTGGGTAGT
 801  GTGTAAAATC CTTTTGAAAA CAAGCGAAAA CGGATTGGAT TTGGAAAAGC
 851  CCTATTATCA GGCGGTCATC CGCCGCTGGC AGAACAAAAT CACCGATGCG
 901  GATACGCGTC GGGAAACCGT GTCCGCCGTT TCACCGAAAA TCATCTTGAA
 951  CGATGCGCCG AAATGGGCGG TCATGCTGGA GACCGAGTGG CAGGACGGCG
1001  AATGGTTCGA GGGCAGGCTG GCGCAGGAAT GGCTGGATAA GGGCGTTGCC
1051  ACCAATCGGG AACAGGTTGC CGCGCTGGAG ACAGAGCTGA AGCAGAAACC
1101  GGCGCAACTG CTTATCGGCG TGCGCGCCCA AACTGTGCCG GACCGCGGCG
1151  TGTTGCGGCA GATTGTCCGA CTCTCGGAAG CGGCGCAGGG CGGCGCGGTG
1201  GTGCAGCTTT TGGCGGAACA GGGGCTTTCA GACGACCTTT CGGAAAAGCT
1251  GGAACATTGG CGTAACGCGC TGGCCGAATG CGGCGCGGCG TGGCTTGAGC
1301  CTGACAGGGC GGCGCAGGAA GGGCGTTTGA AAGACCAATA A
```

This corresponds to the amino acid sequence <SEQ ID 200; ORF33-1>:

```
  1  MLNPSRKLVE LVRILDEGGF IFSGDPVQAT EALRRVDGST EEKIIRRAEM
 51  IDRNRMLRET LERVRAGSFW LWVVAATFAF FTGFSVTYLL MDNQGLNFFL
101  VLAGVLGMNT LMLAVWLAML FLRVKVGRFF SSPATWFRGK DPVNQAVLRL
151  YADEWRQPSV RWKIGATSHS LWLCTLLGML VSVLLLLLVR QYTFNWESTL
201  LSNAASVRAV EMLAWLPSKL GFPVPDARAV IEGRLNGNIA DARAWSGLLV
251  GSIACYGILP RLLAWVVCKI LLKTSENGLD LEKPYYQAVI RRWQNKITDA
301  DTRRETVSAV SPKIILNDAP KWAVMLETEW QDGEWFEGRL AQEWLDKGVA
351  TNREQVAALE TELKQKPAQL LIGVRAQTVP DRGVLRQIVR LSEAAQGGAV
401  VQLLAEQGLS DDLSEKLEHW RNALAECGAA WLEPDRAAQE GRLKDQ*
```

Computer analysis or this amino acid sequence gave the following results:

Homology with a predicted ORF from *N.meningitidis* (strain A)

ORF33 shows 90.9% identity over a 143aa overlap with an ORF (ORF33a) from strain A of *N. meningitidis:*

```
                                                  10        20        30
orf33.pep                                 LFLRVKVGRFFSSPATWFRXKDPVNQAVLR
                                          |||||||||||||||||||| ||||||||||
orf33a        LMDNQGLNFFLVLAGVXGMNTLMLAVWLAMLFLRVKVGRFFSSPATWFRGKDPVNQAVLR
             90       100       110       120       130       140

                     40        50        60        70        80        90
orf33.pep     LYXDEWRXTSVRWKIXATSHSLWLCTLLGMLVSVLLLLLVRQYTFNWESTLLSNAASVRA
              || ||||| |||||| ||||||||||||||||||||||||||||||||||::::|||
orf33a        LYADEWRXPSVRWKIGATSHSLWLCTLLGMLVSVLLLLLVRQYTFNWESTLLGDSSSVRL
            150       160       170       180       190       200

                    100       110       120       130       140
orf33.pep     VEMLAWLPSKLGFPVPDARSVIEGRLNGNIADARAWSGLLVXSIACXGILPRL
              ||||||||:|||||||||||:||||||||||||||||||||| |||| ||||||
orf33a        VEMLAWLPAKLGFPVPDARAVIEGRLNGNIADARAWSGLLVGSIACYGILPRLLAWAVCK
            210       220       230       240       250       260

orf33a        ILXXTSENGLDLEKXXXXXXIRRWQNKITDADTRRETVSAVSPKIVLNDAPKWAVMLETE
            270       280       290       300       310       320
```

The complete length ORF33a nucleotide sequence <SEQ ID 201> is:

```
   1 ATGTTGAATC CATCCCGAAA ACTGGTTGAG CTGGTCCGTA TTTTGGAAGA
  51 AGGCGGCTTT ATTTTCAGCG GCGATCCCGT GCAGGCGACG GAGGCTTTGC
 101 GCCGCGTGGA CGGCAGTACG GAGGAAAAAA TCATCCGTCG GGCGAAGATG
 151 ATCGACAGGA ACCGTATGCT GCGGGAGACG TTGGAACGTG TGCGTGCGGG
 201 GTCGTTCTGG TTGTGGGTGG CGGCGGCGAC GTTTGCGTTT NTTACCGNTT
 251 TTTCAGTTAC TTATCTTCTA ATGGACAATC AGGGTCTGAA TTTCTTTTTG
 301 GTTTTGGCGG GCGTGNTGGG CATGAATACG CTGATGCTGG CAGTATGGTT
 351 GGCAATGTTG TTCCTGCGCG TGAAAGTGGG GCGTTTTTTC AGCAGTCCGG
 401 CGACGTGGTT TCGGGGCAAA GACCCTGTCA ATCAGGCGGT GTTGCGGCTG
 451 TATGCGGACG AGTGGCGGCN ACCTTCGGTA CGTTGGAAAA TAGGCGCAAC
 501 GTCGCACAGC CTGTGGCTCT GCACGCTGCT CGGAATGCTG GTGTCGGTAT
 551 TGTTGCTGCT TTTGGTGCGG CAATATACGT TCAACTGGGA AAGCACGCTG
 601 TTGGGCGATT CGTCTTCGGT ACGGCTGGTG GAAATGTTGG CATGGCTGCC
 651 TGCGAAACTG GGTTTTCCCG TGCCTGATGC GCGGGCGGTC ATCGAAGGTC
 701 GTCTGAACGG CAATATTGCC GATGCGCGGG CTTGGTCGGG GCTGCTGGTC
 751 GGCAGTATCG CCTGCTACGG CATCCTGCCG CGCCTCTTGG CTTGGGCGGT
 801 ATGCAAAATC CTTNTGNAAA CAAGCGAAAA CGGCTTGGAT TTGGAAAAGC
 851 NCNNNNNTCN NNCGNTCATC CGCCGCTGGC AGAACAAAAT CACCGATGCG
 901 GATACGCGTC GGGAAACCGT GTCCGCCGTT TCGCCGAAAA TCGTCTTGAA
 951 CGATGCGCCG AAATGGGCGG TCATGCTGGA GACCGAATGG CAGGACGGCG
1001 AATGGTTCGA GGGCAGGCTG GCGCAGGAAT GGCTGGATAA GGGCGTTGCC
1051 GCCAATCGGG AACAGGTTGC CGCGCTGGAG ACAGAGCTGA AGCAGAAACC
1101 GGCGCAACTG CTTATCGGCG TGCGCGCCCA AACTGTGCCC GACCGCGGCG
1151 TGTTGCGGCA GATCGTCCGA CTTTCGGAAG CGGCGCAGGG CGGCGCGGTG
1201 GTGCANCTTT TGGCGGAACA GGGGCTTTCA GACGACCTTT CGGAAAAGCT
1251 GGAACATTGG CGTAACGCGC TGACCGAATG CGGCGCGGCG TGGCTGGAAC
1301 CCGACAGAGC GGCGCAGGAA GGCCGTCTGA AAACCAACGA CCGCACTTGA
```

This encodes a protein having amino acid sequence <SEQ ID 202>:

```
  1 MLNPSRKLVE LVRILEEGGF IFSGDPVQAT EALRRVDGST EEKIIRRAKM
 51 IDRNRMLRET LERVRAGSFW LWVAAATFAF XTXFSVTYLL MDNQGLNFFL
101 VLAGVXGMNT LMLAVWLAML FLRVKVGRFF SSPATWFRGK DPVNQAVLRL
151 YADEWRXPSV RWKIGATSHS LWLCTLLGML VSVLLLLLVR QYTFNWESTL
201 LGDSSSVRLV EMLAWLPAKL GFPVPDARAV IEGRLNGNIA DARAWSGLLV
251 GSIACYGILP RLLAWAVCKI LXXTSENGLD LEKXXXXXXI RRWQNKITDA
301 DTRRETVSAV SPKIVLNDAP KWAVMLETEW QDGEWFEGRL AQEWLDKGVA
351 ANREQVAALE TELKQKPAQL LIGVRAQTVP DRGVLRQIVR LSEAAQGGAV
401 VXLLAEQGLS DDLSEKLEHW RNALTECGAA WLEPDRAAQE GRLKTNDRT*
```

ORF33a and ORF33-1 show 94.1% identity in 444 aa overlap:

```
                    10        20        30        40        50        60
orf33a.pep  MLNPSRKLVELVRILEEGGFIFSGDPVQATEALRRVDGSTEEKIIRRAKMIDRNRMLRET
            ||||||||||||||:|||||||||||||||||||||||||||||||||:|||||||||||
orf33-1     MLNPSRKLVELVRILDEGGFIFSGDPVQATEALRRVDGSTEEKIIRRAEMIDRNRMLRET
                    10        20        30        40        50        60

                    70        80        90       100       110       120
orf33a.pep  LERVRAGSFWLWVAAATFAFXTXFSVTYLLMDNQGLNFFLVLAGVXGMNTLMLAVWLAML
            ||||||||||||:|||||| | ||||||||||||||||||||||| ||||||||||||||
orf33-1     LERVRAGSFWLWVVAATFAFFTGFSVTYLLMDNQGLNFFLVLAGVLGMNTLMLAVWLAML
                    70        80        90       100       110       120

                   130       140       150       160       170       180
orf33a.pep  FLRVKVGRFFSSPATWFRGKDPVNQAVLRLYADEWRXPSVRWKIGATSHSLWLCTLLGML
            |||||||||||||||||||||||||||||||||||| |||||||||||||||||||||||
orf33-1     FLRVKVGRFFSSPATWFRGKDPVNQAVLRLYADEWRQPSVRWKIGATSHSLWLCTLLGML
                   130       140       150       160       170       180

                   190       200       210       220       230       240
orf33a.pep  VSVLLLLLVRQYTFNWESTLLGDSSSVRLVEMLAWLPAKLGFPVPDARAVIEGRLNGNIA
            ||||||||||||||||||||::::||| ||||||||:|||||||||||||||||||||||
orf33-1     VSVLLLLLVRQYTFNWESTLLSNAASVRAVEMLAWLPSKLGFPVPDARAVIEGRLNGNIA
                   190       200       210       220       230       240

                   250       260       270       280       290       300
orf33a.pep  DARAWSGLLVGSIACYGILPRLLAWAVCKILXXTSENGLDLEKXXXXXXIRRWQNKITDA
            ||||||||||||||||||||||||||:|||||  |||||||||      |||||||||||
orf33-1     DARAWSGLLVGSIACYGILPRLLAWVVCKILLKTSENGLDLEKPYYQAVIRRWQNKITDA
                   250       260       270       280       290       300

                   310       320       330       340       350       360
orf33a.pep  DTRRETVSAVSPKIVLNDAPKWAVMLETEWQDGEWFEGRLAQEWLDKGVAANREQVAALE
            ||||||||||||||:||||||||||||||||||||||||||||||||||||:|||||||||
orf33-1     DTRRETVSAVSPKIILNDAPKWAVMLETEWQDGEWFEGRLAQEWLDKGVATNREQVAALE
                   310       320       330       340       350       360

                   370       380       390       400       410       420
orf33a.pep  TELKQKPAQLLIGVRAQTVPDRGVLRQIVRLSEAAQGGAVVXLLAEQGLSDDLSEKLEHW
            ||||||||||||||||||||||||||||||||||||||||| ||||||||||||||||||
orf33-1     TELKQKPAQLLIGVRAQTVPDRGVLRQIVRLSEAAQGGAVVQLLAEQGLSDDLSEKLEHW
                   370       380       390       400       410       420

                   430       440       450
orf33a.pep  RNALTECGAAWLEPDRAAQEGRLKTNDRTX
            ||||:||||||||||||||||||||
orf33-1     RNALAECGAAWLEPDRAAQEGRLKDQX
                   430       440
```

Homology with a predicted ORF from *N. gonorrhoeae*

ORF33 shows 91.6% identity over a 143aa overlap with a predicted ORF (ORF33.ng) from *N. gonorrhoeae:*

```
orf33.pep                                         LFLRVKVGRFFSSPATWFRXKDPVNQAVLR   30
                                                  ||||||||||||||||||| | ||||||||
orf33ng     LMDNQGLNFFLVLAGVLGMNTLMLAVWLATLFLRVKVGRFFSSPATWFRGKGPVNQAVLR  100

orf33.pep   LYXDEWRXTSVRWKIXATSHSLWLCTLLGMLVSVLLLLLVRQYTFNWESTLLSNAASVRA   90
            || |:||  |||||| ||:|||||||||||||||||||||||||||||||||||||||||
orf33ng     LYADQWRQPSVRWKIGATAHSLWLCTLLGMLVSVLLLLLVRQYTFNWESTLLSNAASVRA  160

orf33.pep   VEMLAWLPSKLGFPVPDARSVIEGRLNGNIADARAWSGLLVXSIACXGILPRL         143
            |||||||||||||||||||:||||||||||||||||||||| ||:| ||||||
orf33ng     VEMLAWLPSKLGFPVPDARAVIEGRLNGNIADARAWSGLLVGSIVCYGILPRLLAWVVCK  220
```

An ORF33ng nucleotide sequence <SEQ ID 203> was predicted to encode a protein having amino acid sequence <SEQ ID 204>:

```
  1  MIDRDRMLRD TLERVRAGSF WLWVVVASMM FTAGFSGTYL LMDNQGLNFF
 51  LVLAGVLGMN TLMLAVWLAT LFLRVKVGRF FSSPATWFRG KGPVNQAVLR
101  LYADQWRQPS VRWKIGATAH SLWLCTLLGM LVSVLLLLLV RQYTFNWEST
151  LLSNAASVRA VEMLAWLPSK LGFPVPDARA VIEGRLNGNI ADARAWSGLL
201  VGSIVCYGIL PRLLAWVVCK ILLKTSENGL DLEKTYYQAV IRRWQNKITD
251  ADTRRETVSA VSPKIVLNDA PKWALMLETE WQDGQWFEGR LAQEWLDKGV
301  AANREQVAAL ETELKQKPAQ LLIGVRAQTV PDRGVLRQIV RLSEAAQGGA
351  VVQLLAEQGL SDDLSEKLEH WRNALTECGA AWLEPDRVAQ EGRLKDQ*
```

Further sequence analysis revealed the following DNA sequence <SEQ ID 205>:

```
   1  ATGTTGaatC CATCCCgaAA ACTGgttgag ctGgTCCgtA Ttttgaataa
  51  agggggtTTT attttcagcg gcgatcctgt gcaggcgacg gaggctttgc
 101  gccgcgtgga cggcAGTACG GAggAaaaaa tcttccgtcg GGCGGAGAtg
 151  atcgACAGGg accgtatgtt gcgggACaCg TtggaacGTG TGCGTGCggg
 201  gtcgtTctgG TTATGGGTGG TggtggCAtC gATGATGTtt aCCGCCGGAT
 251  TTTCAGgcac ttatCttCTG ATGGACaatC AGGGGCtGAA TtTCTTTTTA
 301  GTTTTggcgG GAGTGTtggG CATGaatacG ctgATGCTGG CAGTATGGtt
 351  gGCAACGTTG TTCCTGCGCG TGAAAGTGGG ACGGTTTTTC AGCAGTCCGG
 401  CGACGTGGTT TCGGGGCAAA GGCCCTGTAA ATCAGGCGGT GTTGCGGCTG
 451  TATGCGGACC AGTGGCGGCA ACCTTCGGTA CGATGGAAAA TAGGCGCAAC
 501  GGCGCACAGC TTGTGGCTCT GCACGCTGCT CGGAATGCTG GTGTCGGTAT
 551  TGCTGCTGCT TTTGGTGCGG CAATATACGT TCAACTGGGA AAGCACGCTG
 601  TTGAGCAATG CCGCTTCGGT ACGCGCGGTG GAAATGTTGG CATGGCTGCC
 651  GTCGAAACTC GGTTTCCCTG TCCCCGATGC GCGGGCGGTC ATCGAAGGTC
 701  GTCTGAACGG CAATATTGCC GATGCGCGGG CTTGGTCGGG GCTGCTGGTC
 751  GGCAGTATCG TCTGCTACGG CATCCTGCCG CGCCTCTTGG CTTGGGTAGT
 801  GTGTAAAATC CTTTTGAAAA CAAGCGAAAA CGGattgGAT TTGGAAAAAA
 851  CCTATTATCA GGCGGTCATC CGCCGCTGGC AGAACAAAAT CACCGATGCG
 901  GATACGCGTC GGGAAACCGT GTCCGCCGTT TCGCCgaAAA TCGTCTTGAA
 951  CGATGCGCCG AAATGGGCGC TCATGCTGGA GACCGAGTGG CAGGACGGCC
1001  AATGGTTCGA GGGCAGGCTG GCGCAGGAAT GGCTGGATAA GGGCGTTGCC
1051  GCCAATCGGG AACAGGTTGC CGCGCTGGAG ACAGAGCTGA AGCAGAAACC
1101  GGCGCAACTG CTTATCGGCG TACGCGCCCA AACTGTGCCG GACCGGGGCG
1151  TGCTGCGGCA GATTGTGCGG CTTTCGGAAG CGGCGCAGGG CGGCGCGGTG
1201  GTGCAGCTTT TGGCGGAACA GGGGCTTTCA GACGACCTTT CGGAAAAGCT
1251  GGAACATTGG CGTAACGCGC TGACCGAATG CGGCGCGGCG TGGCTTGAGC
1301  CTGACAGGGT GGCGCAGGAA GGCCGTTTGA AAGACCAATA A
```

This encodes a protein having amino acid sequence <SEQ ID 206; ORF33ng-1>:

```
  1  MLNPSRKLVE LVRILNKGGF IFSGDPVQAT EALRRVDGST EEKIFRRAEM
 51  IDRDRMLRDT LERVRAGSFW LWVVVASMMF TAGFSGTYLL MDNQGLNFFL
101  VLAGVLGMNT LMLAVWLATL FLRVKVGRFF SSPATWFRGK GPVNQAVLRL
151  YADQWRQPSV RWKIGATAHS LWLCTLLGML VSVLLLLLVR QYTFNWESTL
201  LSNAASVRAV EMLAWLPSKL GFPVPDARAV IEGRLNGNIA DARAWSGLLV
251  GSIVCYGILP RLLAWVVCKI LLKTSENGLD LEKTYYQAVI RRWQNKITDA
301  DTRRETVSAV SPKIVLNDAP KWALMLETEW QDGQWFEGRL AQEWLDKGVA
351  ANREQVAALE TELKQKPAQL LIGVRAQTVP DRGVLRQIVR LSEAAQGGAV
401  VQLLAEQGLS DDLSEKLEHW RNALTECGAA WLEPDRVAQE GRLKDQ*
```

ORF33ng-1 and ORF33-1 show 94.6% identity in 446 aa overlap:

```
                       10        20        30        40        50        60
orf33-1.pep    MLNPSRKLVELVRILDEGGFIFSGDPVQATEALRRVDGSTEEKIIRRAEMIDRNRMLRET
               |||||||||||||||::||||||||||||||||||||||||||||:|||||||||:||||:|
orf33ng-1      MLNPSRKLVELVRILNKGGFIFSGDPVQATEALRRVDGSTEEKIFRRAEMIDRDRMLRDT
                       10        20        30        40        50        60

                       70        80        90       100       110       120
orf33-1.pep    LERVRAGSFWLWVVVAATFAFFTGFSVTYLLMDNQGLNFFLVLAGVLGMNTLMLAVWLAML
               |||||||||||||||:|:: | :||| |||||||||||||||||||||||||||||||| |
orf33ng-1      LERVRAGSFWLWVVVASMMFTAGFSGTYLLMDNQGLNFFLVLAGVLGMNTLMLAVWLATL
                       70        80        90       100       110       120

                      130       140       150       160       170       180
orf33-1.pep    FLRVKVGRFFSSPATWFRGKDPVNQAVLRLYADEWRQPSVRWKIGATSHSLWLCTLLGML
               |||||||||||||||||||| |||||||||||||:|||||||||||||:|||||||||||
orf33ng-1      FLRVKVGRFFSSPATWFRGKGPVNQAVLRLYADQWRQPSVRWKIGATAHSLWLCTLLGML
                      130       140       150       160       170       180

                      190       200       210       220       230       240
orf33-1.pep    VSVLLLLLVRQYTFNWESTLLSNAASVRAVEMLAWLPSKLGFPVPDARAVIEGRLNGNIA
               ||||||||||||||||||||||||||||||||||||||||||||||||||||||||||||
orf33ng-1      VSVLLLLLVRQYTFNWESTLLSNAASVRAVEMLAWLPSKLGFPVPDARAVIEGRLNGNIA
                      190       200       210       220       230       240

                      250       260       270       280       290       300
orf33-1.pep    DARAWSGLLVGSIACYGILPRLLAWVVCKILLKTSENGLDLEKPYYQAVIRRWQNKITDA
               ||||||||||||:|||||||||||||||||||||||||||||||| ||||||||||||||||
orf33ng-1      DARAWSGLLVGSIVCYGILPRLLAWVVCKILLKTSENGLDLEKTYYQAVIRRWQNKITDA
                      250       260       270       280       290       300

                      310       320       330       340       350       360
orf33-1.pep    DTRRETVSAVSPKIILNDAPKWAVMLETEWQDGEWFEGRLAQEWLDKGVATNREQVAALE
               ||||||||||||||:||||||||:|||||||||:|||||||||||||||||:|||||||||
orf33ng-1      DTRRETVSAVSPKIVLNDAPKWALMLETEWQDGQWFEGRLAQEWLDKGVAANREQVAALE
                      310       320       330       340       350       360

                      370       380       390       400       410       420
orf33-1.pep    TELKQKPAQLLIGVRAQTVPDRGVLRQIVRLSEAAQGGAVVQLLAEQGLSDDLSEKLEHW
               ||||||||||||||||||||||||||||||||||||||||||||||||||||||||||||
orf33ng-1      TELKQKPAQLLIGVRAQTVPDRGVLRQIVRLSEAAQGGAVVQLLAEQGLSDDLSEKLEHW
                      370       380       390       400       410       420

                      430       440
orf33-1.pep    RNALAECGAAWLEPDRAAQEGRLKDQX
               ||||:|||||||||||:||||||||||
orf33ng-1      RNALTECGAAWLEPDRVAQEGRLKDQX
                      430       440
```

Based on the presence of several putative transmembrane domains in the gonococcal protein, it is predicted that the proteins from *N.meningitidis* and *N.gonorrhoeae,* and their epitopes, could be useful antigens for vaccines or diagnostics, or for raising antibodies.

**Example 25**

The following partial DNA sequence was identified in *N.meningitidis* <SEQ ID 207>:

```
  1 ..CAGAAGAGTT TGTCGAGAAT TTCTTTATGG GGTTTGGGCG GCGTGTTTTT
 51   CGGGGTGTCC GGTCTGGTAT GGTTTTCTTT GGGCGTTTCT TT.GAGTGCG
101   CCTGTTTTTC GGGTGTTTCT TTTCGGGGTT CGGGACGGGG GACGTTTGTG
151   GGCAGTACGG GGGTTTCTTT GAGTGTGTTT TCAGCTTGTG TTCC.GGCGT
201   CGTCCGGCTG CCTGTCGGTT TGAGCTGTGT CGGCAGGTTG CG..GTTTGA
251   CCCGGTTTTT CTTGGGTGCG GCAGGGGACG TCATTCTCCT GCCGCTTTCG
301   TCTGTGCCGT CCGGCTGTGC GGGTTCGGAT GAGGCGGCGT GGTGGTGTTC
351   GGGTTGGGCG GCATCTTGTT CCGACTACGC CGTTTGGCAG CCAGAATTCG
401   GTTTCGCGGG GGCTGTCGGT GTGTTGCGGT TCGGCTTGAA GGGTTTTGTC
451   GTCC..
```

This corresponds to the amino acid sequence <SEQ ID 208; ORF34>:

```
  1 ..QKSLSRISLW GLGGVFFGVS GLVWFSLGVS XECACFSGVS FRGSGRGTFV

 51   GSTGVSLSVF SACVXGVVRL PVGLSCVGRL XXLTRFFLGA AGDVILLPLS
101   SVPSGCAGSD EAAWWCSGWA ASCPTTPFGS QNSVSRGLSV CCGSA*RVLS
151   S..
```

Further work revealed the complete nucleotide sequence <SEQ ID 209>:

```
   1 ATGATGATGC CGTTCATAAT GCTTCCTTGG ATTGCkGGTG TGCCTGCCGT
  51 GCCGGGTCAG AATAGGTTGT CCAGAATTTC TTTATGGGGT TTGGGCGGCG
 101 TGTTTTTCGG GGTGTCCGGT TTGGTATGGT TTTCTTTGGG CGTTTCTTTG
 151 GGCTGCGCCT GTTTTTCGGG TGTTTCTTTT CGGGGTTCGG GACGGGGGAC
 201 GTTTGTGGGC AGTACGGGGG TTTCTTTGAG TGTGTTTTCA GCTTGTGTTC
 251 CGGCGTCGTC CGGCTGCCTG TCGGTTTGAG CTGTGTCGGC AGGTTGCGGT
 301 TTGACCCGGT TTTTCTTGGG TGCGGCAGGG GACGGCAGTC CGCTGCCGCT
 351 TTCGTCTGTG CCGTCCGGCT GTGCGGGTTC GGATGAGGCG GCGTGGTGGT
 401 GTTCGGGTTG GGCGGCATCT TGTCCGACTA CGCCGTTTGG CAGCCAGAAT
 451 TCGGTTTCGC GGGGGCTGTC GGTGTGTTGC GGTTCGGCTT GAAGGGTTTT
 501 GTCGCCGTTC GGGTTGAATG TGCTGACGAT GCCTATTGCC AATGCGCCGA
 551 TGGCGGCGAT ACAGATGAGC AATACGGCGC GTATCAGGAG TTTGGGGGTC
 601 AGCCTGAAGG GTTTGTTCGG TTTTTTTGCC ATTTTGATTG TGCTTTTGGG
 651 GTGTCGGGCA ATGCCGTCTG AAGGCGGTTC AGACGGCATT GCCGAGTCAG
 701 CGTTGGACGT AGTTTTGGTA GAGGGTGATG ACTTTTTGTA CGCCGACGGT
 751 GGTGCTGACT TTTTGGGTAA TCTGCGCCTG TTCTTCGGGG GTGAGGATGC
 801 CCATAACGTA GGTTACGTTG CCGTAGGTAA CGATTTTGAC GCGCGCCTGT
 851 GTGGCGGGGC TGATGCCCAA CAGCGTGGCG CGGACTTTGG ATGTGTTCCA
 901 AGTGTCGCCG GCGATGTCGC CGGCAGTGCG CGGCAGGGAG GCGACGGTAA
 951 TATAGTTGTA CACGCCTTCG GCGGCCTGTT CGGAACGTGC AATCTGACCG
1001 ACGAACTGTT TTTCGCCTTC GGTGGCGACT TGTCCGAGCA GCAGCAGGTG
1051 GCGGTTGTAG CCGACGACGG AGATTTGGGG CGTGTAGCCT TTGGTTTGGT
1101 TGTTTTGGCG CAGATAGGAA CGGGCGGTGG TTTCGATACG CAACGCCATA
1151 ACGTTGTCGT CGGTTTGCGC GCCGGTGGTT CGGCGGTCGA CGGCGGATTT
1201 CGCGCCGACG GCGGCGCTTC CGATTACTGC GCTGACGCAG CCGCTAAGGG
1251 CAAGGCTGAA AATGGCGGCA ATCAGGGTGC GGACGGTGTG CGGTTTGGGT
1301 TTCATCGGGT GCTTCCTTTC TTGGGCGTTT CAGACGGCAT TGCTTTGCGC
1351 CATGCCGTCT GA
```

This corresponds to the amino acid sequence <SEQ ID 210; ORF34-1>:

```
  1  MMMPFIMLPW IAGVPAVPGQ NRLSRISLWG LGGVFFGVSG LVWFSLGVSL
 51  GCACFSGVSF RGSGRGTFVG STGVSLSVFS ACVPASSGCL SV*AVSAGCG
101  LTRFFLGAAG DGSPLPLSSV PSGCAGSDEA AWWCSGWAAS CPTTPFGSQN
151  SVSRGLSVCC GSA*RVLSPF GLNVLTMPIA NAPMAAIQMS NTARIRSLGV
201  SLKGLFGFFA ILIVLLGCRA MPSEGGSDGI AESALDVVLV EGDDFLYADG
251  GADFLGNLRL FFGGEDAHNV GYVAVGNDFD ARLCGGADAQ QRGADFGCVP
301  SVAGDVAGSA RQGGDGNIVV HAFGGLFGTC NLTDELFFAF GGDLSEQQQV
351  AVVADDGDLG RVAFGLVVLA QIGTGGGFDT QRHNVVVGLR AGGSAVDGGF
401  RADGGASDYC ADAAAKGKAE NGGNQGADGV RFGFHRVLPF LGVSDGIALR
451  HAV*
```

Computer analysis of this amino acid sequence gave the following results:

Homology with a predicted ORF from *N.meningitidis* (strain A)

ORF34 shows 73.3% identity over a 161aa overlap with an ORF (ORF34a) from strain A of *N. meningitidis:*

```
                                     10        20        30
orf34.pep                  QKSLSRISLWGLGGVFFGVSGLVWFSLGVSXE------CAC
                           || ||| ||||||| |||||||||||||||       |||
orf34a       MMXPXIMLPWIAGVPAVPGQKRLSRXSLWGLGGXFFGVSGLVWFSLGVSXSLGVSXGCAC
                     10        20        30        40        50        60

               40        50        60        70        80        90
orf34.pep    FSGVSFRGSGRGTFVGSTGVSLSVFSACVXGVVRLPVGLSCVGRLXX-----LTRFFLGA
             ||||||||||||||||||||||||||||:       |:: :|::       ||| | ||
orf34a       FSGVSFRGSGRGTFVGSTGVSLSVFSACA------PASSGCLSVXAVSAGCGLTRXFXGA
                     70        80              90       100       110

                    100       110       120       130       140       150
orf34.pep    AGDVILLPLSSVPSGCAGSDEAAWWCSGWAASCPTTPFGSQNSVSRGLSVCCGSAXRVLS
             |||    |||||||||||:|| |  ||||||||||||||||||||||||||||||: ||||
orf34a       AGDGSPLPLSSVPSGCAGADEEAXXCSGWAASCPTTPFGSQNSVSRGLSVCCGSVWRVLS
                   120       130       140       150       160       170

orf34.pep    S

orf34a       PFGXNVLTMPIANAPMAVIQMSNTARIRSLGVSLKGLFXFFAILIVLLGCRAMPSEGGSD
                   180       190       200       210       220       230
```

The complete length ORF34a nucleotide sequence <SEQ ID 211> is:

```
   1 ATGATGATNC CGTTNATAAT GCTTCCTTGG ATTGCGGGTG TGCCTGCCGT
  51 GCCGGGTCAG AAGAGGTTGT CGAGAANTTC TTTATGGGGT TTAGGCGGCN
 101 TGTTTTTCGG GGTGTCCGGT TTGGTATGGT TTTCTTTGGG CGTTTCTNTT
 151 TCTTTGGGTG TTTCTNTGGG CTGTGCCTGT TTTTCGGGTG TTTCTTTTCG
 201 GGGTTCGGGA CGGGGGACGT TTGTGGGCAG TACNGGGGTT TCTTTGAGTG
 251 TGTTTTCAGC TTGTGCTCCG GCGTCGTCCG GCTGCCTGTC GGTTTNAGCT
 301 GTGTCGGCAG GTTGCGGTTT GACCCGGNTT TTCTTNGGTG CGGCAGGGGA
 351 CGGCAGTCCG CTGCCGCTTT CGTCTGTGCC GTCCGGCTGT GCGGGTGCGG
 401 ATGAGGAGGC GTNGTNGTGT TCGGGTTGGG CGGCATCTTG TCCGACTACG
 451 CCGTTTGGCA GCCAGAATTC GGTTTCGCGG GGGCTGTCGG TGTGTTGCGG
 501 TTCGGTNTGG AGGGTTTTGT CNCCGTTCGG GTNGAATGTG CTGACGATGC
 551 CTATTGCCAA TGCGCCGATG GCGGTGATAC AGATGAGCAA TACGGCGCGT
 601 ATCAGGAGTT TGGGGGTCAG CCTGAAGGGT TTGTTCNGTT TTTTTGCCAT
 651 TTTGATTGTG CTTTTGGGGT GTCGGGCAAT GCCGTCTGAA GGCGGTTCAG
 701 ACGGCATTGC CGAGTCAGCG TTGGACGTAG TTTNGGTAGA GGGTGATGAC
 751 TTTTTGTACG CCGACGGTGG TGCTGACTTT TTGGGTAATC TGCGCCTGTT
 801 CTTCGGGGGT GAGGATGCCC ATAACGTAGG TTACGTTGCC GTAGGTAACG
 851 ATTTTGACGC GCGCCTGTGT GGCGGGGCTG ATGCCCAACA GCGTGGCGCG
 901 GACTTTGGAT GTGTTCCAAG TGTCGCCGGC GATGTCGCCG GCAGTGCGCG
 951 GCAGGGAGGC GACGGTAATG TANTTGTACA CGCCTTCGGC GGCCTGTTCG
1001 GAACGTGCAA TCTGACCGAC GAACTGTTTC TCGCCTTCGG TGGCGACTTG
1051 TCCGAGCAGC AGCAGGTGGC GGTTGTAGCC GACAACGGAG ATTTGGGGCG
1101 TGTANCCTTT GGTTTGGTTG TTTTGGCGCA GATAGGAGCG GGCGGTGGTT
1151 TCGATACGCA GCGCCATTAC GTTGTCGTCG GTTNGCGCGC CGGTGGTTCG
1201 GCGGTCGACG GCGGATTTCG CGCCGACCGC CGCGCCGCCG ACGACTGCGC
1251 TGACGCAGCC GCCGAGGGCA AGGCTGAGGA CGGCGGCAGT CAGGGTGCGG
1301 ACGGTGTGCG GTTTGGGTTT CATCGGGTGC TTCCTTTCTT GGGCGTTTCA
1351 GACGGCATTG CTTTGCGCCA TGCCGTCTGA
```

This encodes a protein having amino acid sequence <SEQ ID 212>:

```
  1 MMXPXIMLPW IAGVPAVPGQ KRLSRXSLWG LGGXFFGVSG LVWFSLGVSX
 51 SLGVSXGCAC FSGVSFRGSG RGTFVGSTGV SLSVFSACAP ASSGCLSVXA
101 VSAGCGLTRX FXGAAGDGSP LPLSSVPSGC AGADEEAXXC SGWAASCPTT
151 PFGSQNSVSR GLSVCCGSVW RVLSPFGXNV LTMPIANAPM AVIQMSNTAR
201 IRSLGVSLKG LFXFFAILIV LLGCRAMPSE GGSDGIAESA LDVVXVEGDD
251 FLYADGGADF LGNLRLFFGG EDAHNVGYVA VGNDFDARLC GGADAQQRGA
301 DFGCVPSVAG DVAGSARQGG DGNVXVHAFG GLFGTCNLTD ELFLAFGGDL
351 SEQQQVAVVA DNGDLGRVXF GLVVLAQIGA GGGFDTQRHY VVVGXRAGGS
401 AVDGGFRADR RAADDCADAA AEGKAEDGGS QGADGVRFGF HRVLPFLGVS
451 DGIALRHAV*
```

ORF34a and ORF34-1 show 91.3% identity in 459 aa overlap:

```
                    10        20        30        40        50        60
orf34a.pep  MMXPXIMLPWIAGVPAVPGQKRLSRXSLWGLGGXFFGVSGLVWFSLGVSXSLGVSXGCAC
            || | ||||||||||||||:|||| ||||||| ||||||||||||||       ||||
orf34-1     MMMPFIMLPWIAGVPAVPGQNRLSRISLWGLGGVFFGVSGLVWFSLGVSL------GCAC
                    10        20        30        40        50

                    70        80        90       100       110       120
orf34a.pep  FSGVSFRGSGRGTFVGSTGVSLSVFSACAPASSGCLSVXAVSAGCGLTRXFXGAAGDGSP
            ||||||||||||||||||||||||||||:||||||||||||||||||||| | |||||||
orf34-1     FSGVSFRGSGRGTFVGSTGVSLSVFSACVPASSGCLSVXAVSAGCGLTRFFLGAAGDGSP
              60        70        80        90       100       110

                   130       140       150       160       170       180
orf34a.pep  LPLSSVPSGCAGADEEAXXCSGWAASCPTTPFGSQNSVSRGLSVCCGSVWRVLSPFGXNV
            ||||||||||||:|| |  |||||||||||||||||||||||||||||: ||||||| ||
orf34-1     LPLSSVPSGCAGSDEAAWWCSGWAASCPTTPFGSQNSVSRGLSVCCGSAXRVLSPFGLNV
```

```
                        120       130       140       150       160       170

                            190       200       210       220       230       240
orf34a.pep          LTMPIANAPMAVIQMSNTARIRSLGVSLKGLFXFFAILIVLLGCRAMPSEGGSDGIAESA
                    |||||||||||:|||||||||||||||||||| |||||||||||||||||||||||||||
orf34-1             LTMPIANAPMAAIQMSNTARIRSLGVSLKGLFGFFAILIVLLGCRAMPSEGGSDGIAESA
                        180       190       200       210       220       230

                            250       260       270       280       290       300
orf34a.pep          LDVVXVEGDDFLYADGGADFLGNLRLFFGGEDAHNVGYVAVGNDFDARLCGGADAQQRGA
                    |||| |||||||||||||||||||||||||||||||||||||||||||||||||||||||
orf34-1             LDVVLVEGDDFLYADGGADFLGNLRLFFGGEDAHNVGYVAVGNDFDARLCGGADAQQRGA
                        240       250       260       270       280       290

                            310       320       330       340       350       360
orf34a.pep          DFGCVPSVAGDVAGSARQGGDGNVXVHAFGGLFGTCNLTDELFLAFGGDLSEQQQVAVVA
                    |||||||||||||||||||||||: ||||||||||||||||||:||||||||||||||||
orf34-1             DFGCVPSVAGDVAGSARQGGDGNIVVHAFGGLFGTCNLTDELFFAFGGDLSEQQQVAVVA
                        300       310       320       330       340       350

                            370       380       390       400       410       420
orf34a.pep          DNGDLGRVXFGLVVLAQIGAGGGFDTQRHYVVVGXRAGGSAVDGGFRADRRAADDCADAA
                    |:|||||| ||||||||||:||||||||| |||| ||||||||||||||  |:| |||||
orf34-1             DDGDLGRVAFGLVVLAQIGTGGGFDTQRHNVVVGLRAGGSAVDGGFRADGGASDYCADAA
                        360       370       380       390       400       410

                            430       440       450       460
orf34a.pep          AEGKAEDGGSQGADGVRFGFHRVLPFLGVSDGIALRHAVX
                    |:||||:||:||||||||||||||||||||||||||||||
orf34-1             AKGKAENGGNQGADGVRFGFHRVLPFLGVSDGIALRHAVX
                        420       430       440       450
```

<u>Homology with a predicted ORF from *N.gonorrhoeae*</u>

ORF34 shows 77.6% identity over a 161aa overlap with a predicted ORF (ORF34.ng) from *N. gonorrhoeae:*

```
orf34.pep                           QKSLSRISLWGLGGVFFGVSGLVWFSLGVSXE------CAC    35
                                    || |||||||||:|||||||||||||||||        |||
orf34ng        MMMPFIMLPWIAGVPAVPGQKRLSRISLWGLAGVFFGVSGLVWFSLGVSFSLGVSLGCAC    60

orf34.pep      FSGVSFRGSGRGTFVGSTGVSLSVFSACVXGVVRLPVGLSCV-----GRLXXLTRFFLGA    90
               |||||||||| |:|||||||||||||||     :||: | :      ||    ||||||||
orf34ng        FSGVSFRGSGWGAFVGSTGVSLSVFSACVP----VPVNESAARAASEGR--GLTRFFLGA   114

orf34.pep      AGDVILLPLSSVPSGCAGSDEAAWWCSGWAASCPTTPFGSQNSVSRGLSVCCGSAXRVLS   150
               |||   ||||||||||||||||||||||||||||||:|||||||||||||||||||: ||||
orf34ng        AGDGSPLPLSSVPSGCAGSDEAAWWCSGWAASCPTAPFGSQNSVSRGLSVCCGSVWRVLS   174

orf34.pep      S                                                              175

orf34ng        PFGLNVLTMPTANAPMAVIQMSNTARIRSLGVSLKGLFGFFAILIVLLGCRAMPSEGGSD   234
```

The complete length ORF34ng nucleotide sequence <SEQ ID 213> is:

```
   1  ATGATGATGC CGTTCATAAT GCTTCCTTGG ATTGCGGGTG TGCCTGCCGT
  51  GCCGGGTCAA AAGAGGTTGT CGAGAATCTC TTTATGGGGT TTGGCCGGCG
 101  TGTTTTTCGG GGTGTCCGGT TTGGTATGGT TTTCTTTGGG CGTTTCTTTT
 151  TCTTTGGGTG TTTCTTTGGG CTGCGCCTGT TTTTCGGGTG TTTCTTTTCG
 201  GGGTTCGGGA TGGGGGGCGT TTGTGGGCAG TACGGGGGTT TCTTTGAGTG
 251  TGTTTTCAGC TTGTGTTCCG GTGCCGGTTA ACGAATCGGC TGCCCGGGCC
 301  GCATCCGAAG GGCGCGGTTT gACCCGGTTT TTCTTGGGTG CGGCAGGGGA
 351  CGGCAGTCCG CTGCCGCTTT CTTCTGTGCC GTCCGGCTGT GCGGGTTCGG
 401  ATGAGGCGGC GTGGTGGTGT TCGGGTTGGG CGGCATCTTG TCCGACGGCG
 451  CCGTTTGGCA GCCAGAATTC GGTTTCGCGG GGGCTGTCGG TGTGTTGCGG
 501  TTCGGTTTGG AGGGTTTTGT CGCCGTTCGG GTTGAATGTG CTGACGATGC
 551  CTACTGCCAA TGCGCCGATG GCGGTGATAC AGATGAGCAA TACGGCGCGT
 601  ATCAGGAGTT TGGGGGTCAG CCTGAAGGGT TTGTTCGGTT TTTTTGCCAT
 651  TTTGATTGTG CTTTTGGGGT GTCGGGCAAT GCCGTCTGAA GGCGGTTCAG
 701  ACGGCATTGC CGAGTCAGCG TTGGACGTAG TTTTGGTAGA GGGTAATGAC
```

```
 751  TTTTTGTACG CCGAcggTGG TGCTGACTTT TTGGGTAATC TGCGCCTGTT
 801  CTTCGGGGGT GAGGATGCCC ATAACGTAGG TTACATTGCC GTAGGTAATG
 851  ATTTTGACGC GCGCCTGTGT AGCGGGGCTG ATGCCCAGCA GcgtgGCGCG
 901  GACTTTGGAC GTGTTCCAAG TGTCGCCGGC GATGTCGCCC GCAGTGCGCG
 951  GCAGGGAGGC GACGGTAATG TAGTTGTATA CGCCTTCGGC GGCCTGTTCG
1001  GAACGTGCAA TCTGACCGAC GAACTGTTTT TCGCCTTCGG TGGCGACTTG
1051  TCCGAGCAGC AGCAGGTGGC GGTTGTAGCC GACGACGGAG ATTTGGGGCG
1101  TGTAGCCTTT GGTTTGGTTG TTTTGGCGCA GGTAGGAACG GGCGGTGGTT
1151  TCGATACGCA ACGCCATAAC GTtgtCATCG GTTtgcgcgc CGGTGGTTcg
1201  gCGGTCGATG ACGGATTTTG CGCCGACGGC GGCCCCGCCG ACGACTGCGC
1251  TGAAGCAGCC GCCGAGGGCA AGGCTGAGGA CGGCGGCAAT CAGGGTGCGG
1301  ACGGTGTGTG GTTTGGGTTT CATCGGGGAC TTCCTTTCTT GGGCGTTTCA
1351  GACGGCATTG CTTTGCGCCA TGCCGTCTGA
```

This encodes a protein having amino acid sequence <SEQ ID 214>:

```
   1  MMMPFIMLPW IAGVPAVPGQ KRLSRISLWG LAGVFFGVSG LVWFSLGVSF
  51  SLGVSLGCAC FSGVSFRGSG WGAFVGSTGV SLSVFSACVP VPVNESAARA
 101  ASEGRGLTRF FLGAAGDGSP LPLSSVPSGC AGSDEAAWWC SGWAASCPTA
 151  PFGSQNSVSR GLSVCCGSVW RVLSPFGLNV LTMPTANAPM AVIQMSNTAR
 201  IRSLGVSLKG LFGFFAILIV LLGCRAMPSE GGSDGIAESA LDVVLVEGND
 251  FLYADGGADF LGNLRLFFGG EDAHNVGYIA VGNDFDARLC SGADAQQRGA
 301  DFGRVPSVAG DVARSARQGG DGNVVVYAFG GLFGTCNLTD ELFFAFGGDL
 351  SEQQQVAVVA DDGDLGRVAF GLVVLAQVGT GGGFDTQRHN VVIGLRAGGS
 401  AVDDGFCADG GPADDCAEAA AEGKAEDGGN QGADGVWFGF HRGLPFLGVS
 451  DGIALRHAV*
```

ORF34ng and ORF34-1 show 90.0% identity in 459 aa overlap:

```
                          10        20        30        40        4      50
orf34-1.pep     MMMPFIMLPWIAGVPAVPGQNRLSRISLWGLGGVFFGVSGLVWFSLGVS------LGCAC
                ||||||||||||||||||||:||||||||||:|||||||||||||||||      |||||
orf34ng         MMMPFIMLPWIAGVPAVPGQKRLSRISLWGLAGVFFGVSGLVWFSLGVSFSLGVSLGCAC
                          10        20        30        40        50        60

                  60        70        80        90       100       110
orf34-1.pep     FSGVSFRGSGRGTFVGSTGVSLSVFSACVPASSGCLSVXAVSAGCGLTRFFLGAAGDGSP
                |||||||||| |:|||||||||||||||||:  :  :: |:| | |||||||||||||||
orf34ng         FSGVSFRGSGWGAFVGSTGVSLSVFSACVPVPVNESAARAASEGRGLTRFFLGAAGDGSP
                          70        80        90       100       110       120

                 120       130       140       150       160       170
orf34-1.pep     LPLSSVPSGCAGSDEAAWWCSGWAASCPTTPFGSQNSVSRGLSVCCGSAXRVLSPFGLNV
                |||||||||||||||||||||||||||||:||||||||||||||||||: ||||||||||
orf34ng         LPLSSVPSGCAGSDEAAWWCSGWAASCPTAPFGSQNSVSRGLSVCCGSVWRVLSPFGLNV
                         130       140       150       160       170       180

                 180       190       200       210       220       230
orf34-1.pep     LTMPIANAPMAAIQMSNTARIRSLGVSLKGLFGFFAILIVLLGCRAMPSEGGSDGIAESA
                |||| ||||||:||||||||||||||||||||||||||||||||||||||||||||||||
orf34ng         LTMPTANAPMAVIQMSNTARIRSLGVSLKGLFGFFAILIVLLGCRAMPSEGGSDGIAESA
                         190       200       210       220       230       240

                 240       250       260       270       280       290
orf34-1.pep     LDVVLVEGDDFLYADGGADFLGNLRLFFGGEDAHNVGYVAVGNDFDARLCGGADAQQRGA
                ||||||||:|||||||||||||||||||||||||||||:|||||||||||:|||||||||
orf34ng         LDVVLVEGNDFLYADGGADFLGNLRLFFGGEDAHNVGYIAVGNDFDARLCSGADAQQRGA
                         250       260       270       280       290       300

                 300       310       320       330       340       350
orf34-1.pep     DFGCVPSVAGDVAGSARQGGDGNIVVHAFGGLFGTCNLTDELFFAFGGDLSEQQVAVVA
                ||| ||||||||| ||||||||:||:|||||||||||||||||||||||||||||||||
orf34ng         DFGRVPSVAGDVARSARQGGDGNVVVYAFGGLFGTCNLTDELFFAFGGDLSEQQVAVVA
                         310       320       330       340       350       360

                 360       370       380       390       400       410
orf34-1.pep     DDGDLGRVAFGLVVLAQIGTGGGFDTQRHNVVVGLRAGGSAVDGGFRADGGASDYCADAA
                |||||||||||||||||:|||||||||||||||:||||||||| || |||| :| ||:||
orf34ng         DDGDLGRVAFGLVVLAQVGTGGGFDTQRHNVVIGLRAGGSAVDDGFCADGGPADDCAEAA
                         370       380       390       400       410       420

                 420       430       440       450


orf34-1.pep     AKGKAENGGNQGADGVRFGFHRVLPFLGVSDGIALRHAVX
                |:||||:||||||||| ||||| |||||||||||||||||
orf34ng         AEGKAEDGGNQGADGVWFGFHRGLPFLGVSDGIALRHAVX
                       430       440       450       460
```

Based on this analysis, including the presence of a putative leader sequence (double-underlined) and several putative transmembrane domains (single-underlined) in the gonococcal protein, it is predicted that the proteins from *N.meningitidis* and *N.gonorrhoeae,* and their epitopes, could be useful antigens for vaccines or diagnostics, or for raising antibodies.

## Example 26

The following partial DNA sequence was identified in *N.meningitidis* <SEQ ID 215>:

```
  1  ATGAAAACCT TCTTCAAAAC CCTTTCCGCC GCCGCACTCG CGCTCATCCT
 51  CGCCGCCTGC GGATT.CAAA AAGACAGCGC GCCCGCCGCA TCCGCTTCTG
101  CCGCCGCCGA CAACGGCGCG GCGTAAAAAA GAAATCGTCT TCGGCACGAC
151  CGTCGGCGAC TTCGGCGATA TGGTCAAAGA ACAAATCCAA GCCGAGCTGG
201  AGAAAAAAGG CTACACCGTC AAACTGGTCG AGTTTACCGA CTATGTACGC
251  CCGAATCTGG CATTGGCTGA GGGCGAGTTG
```

This corresponds to the amino acid sequence <SEQ ID 216; ORF4>:

```
  1  MKTFFKTLSA AALALILAAC G.QKDSAPAA SASAAADNGA AKKEIVFGTT
 51  VGDFGDMVKE QIQAELEKKG YTVKLVEFTD YVRPNLALAE GEL
```

Further sequence analysis revealed the complete nucleotide sequence <SEQ ID 217>:

```
  1  ATGAAAACCT TCTTCAAAAC CCTTTCCGCC GCCGCACTCG CGCTCATCCT
 51  CGCCGCCTGC GGCGGTCAAA AAGACAGCGC GCCCGCCGCA TCCGCTTCTG
101  CCGCCGCCGA CAACGGCGCG GCGAAAAAAG AAATCGTCTT CGGCACGACC
151  GTCGGCGACT TCGGCGATAT GGTCAAAGAA CAAATCCAAG CCGAGCTGGA
201  GAAAAAAGGC TACACCGTCA AACTGGTCGA GTTTACCGAC TATGTACGCC
251  CGAATCTGGC ATTGGCTGAG GGCGAGTTGG ACATCAACGT CTTCCAACAC
301  AAACCCTATC TTGACGACTT CAAAAAAGAA CACAATCTGG ACATCACCGA
351  AGTCTTCCAA GTGCCGACCG CGCCTTTGGG ACTGTACCCG GGCAAGCTGA
401  AATCGCTGGA AGAAGTCAAA GACGGCAGCA CCGTATCCGC GCCCAACGAC
451  CCGTCCAACT TCGCCCGCGT CTTGGTGATG CTCGACGAAC TGGGTTGGAT
501  CAAACTCAAA GACGGCATCA ATCCGTTGAC CGCATCCAAA GCGGACATCG
551  CCGAGAACCT GAAAAACATC AAAATCGTCG AGCTTGAAGC CGCGCAACTG
601  CCGCGTAGCC GCGCCGACGT GGATTTTGCC GTCGTCAACG GCAACTACGC
651  CATAAGCAGC GGCATGAAGC TGACCGAAGC CCTGTTCCAA GAACCGAGCT
701  TTGCCTATGT CAACTGGTCT GCCGTCAAAA CCGCCGACAA AGACAGCCAA
751  TGGCTTAAAG ACGTAACCGA GGCCTATAAC TCCGACGCGT TCAAAGCCTA
801  CGCGCACAAA CGCTTCGAGG GCTACAAATC CCCTGCCGCA TGGAATGAAG
851  GCGCAGCCAA ATAA
```

This corresponds to the amino acid sequence <SEQ ID 218; ORF4-1>:

```
  1  MKTFFKTLSA AALALILAAC GGQKDSAPAA SASAAADNGA AKKEIVFGTT
 51  VGDFGDMVKE QIQAELEKKG YTVKLVEFTD YVRPNLALAE GELDINVFQH
101  KPYLDDFKKE HNLDITEVFQ VPTAPLGLYP GKLKSLEEVK DGSTVSAPND
151  PSNFARVLVM LDELGWIKLK DGINPLTASK ADIAENLKNI KIVELEAAQL
201  PRSRADVDFA VVNGNYAISS GMKLTEALFQ EPSFAYVNWS AVKTADKDSQ
251  WLKDVTEAYN SDAFKAYAHK RFEGYKSPAA WNEGAAK*
```

Computer analysis of this amino acid sequence gave the following results:

<u>Homology with a predicted ORF from *N.meningitidis* (strain A)</u>

ORF4 shows 93.5% identity over a 93aa overlap with an ORF (ORF4a) from strain A of *N. meningitidis:*

```
        10        20        30        40        50        59
```

```
orf4.pep    MKTFFKTLSAAALALILAACG-QKDSAPAASASAAADNGAAKKEIVFGTTVGDFGDMVKE
            ||||||||||||||||||||| ||||||||||||||||||| ||||||||||||||||||
orf4a       MKTFFKTLSAAALALILAACGGQKDSAPAASASAAADNGAAXKEIVFGTTVGDFGDMVKE
                      10        20        30        40        50        60

           60        70        80        90
orf4.pep    QIQAELEKKGYTVKLVEFTDYVRPNLALAEGEL
             || |||||||||||||| ||||| |||||||||
orf4a       XIQPELEKKGYTVKLVEXTDYVRXNLALAEGELDINVXQHXXYLDDXKKXHNLDITXVXQ
                      70        80        90       100       110       120

orf4a       VPTAPLGLYPGKLKSLXXVKXGSTVSAPNDPXXFXRVLVMLDELGXIKLKDXIXXXXXXX
                     130       140       150       160       170       180
```

The complete length ORF4a nucleotide sequence <SEQ ID 219> is:

```
  1 ATGAAAACCT TCTTCAAAAC CCTTTCCGCC GCCGCACTCG CGCTCATCCT
 51 CGCCGCCTGC GGCGGTCAAA AAGATAGCGC GCCCGCCGCA TCCGCTTCTG
101 CCGCCGCCGA CAACGGCGCG GCGAANAAAG AAATCGTCTT CGGCACGACC
151 GTCGGCGACT TCGGCGATAT GGTCAAAGAA CANATCCAAC CCGAGCTGGA
201 GAAAAAAGGC TACACCGTCA AACTGGTCGA GTNTACCGAC TATGTGCGCN
251 CGAATCTGGC ATTGGCTGAG GGCGAGTTGG ACATCAACGT CTTNCAACAC
301 ANACNCTATC TTGACGACTN CAAAAAANAA CACAATCTGG ACATCACCNN
351 AGTCTTNCAA GTGCCGACCG CGCCTTTGGG ACTGTACCCG GGCAAGCTGA
401 AATCGCTGGA NNAAGTCAAA GANGGCAGCA CCGTATCCGC GCCCAACGAC
451 CCGTNNNACT TCGNCCGCGT CTTGGTGATG CTCGACGAAC TGGGTTNGAT
501 CAAACTCAAA GACNGCATCA NNNNGNNGNN NNNANCNANA NNNGANANNN
551 NNNNANNNNT NNNNNNNNNN NNNNNCNNCG NNNNNNNANN NNNNNNNNNN
601 NCGNNTNNNN NNGCNNNNNT NNANNNTNNN NNCNNCNNNN NNNNNTNNNN
651 NANNANNAGC GGCATGAAGC TGACCGAAGC CCTGTTCCAA GAACCGAGCT
701 TTGCCTATGT CAACTGGTCT GCCGTCAAAA CCGCCGACAA AGACAGCCAA
751 TGGCTTAAAG ACGTAACCGA GGCCTATAAC TCCGACGCGT TCAAAGCCTA
801 CGCGCACAAA CGCTTCGAGG GCTACAAATC CCCTGCCGCA TGGAATGAAG
851 GCGCAGCCAA ATAA
```

This is predicted to encode a protein having amino acid sequence <SEQ ID 220>:

```
  1 MKTFFKTLSA AALALILAAC GGQKDSAPAA SASAAADNGA AXKEIVFGTT
 51 VGDFGDMVKE XIQPELEKKG YTVKLVEXTD YVRXNLALAE GELDINVXQH
101 XXYLDDXKKX HNLDITXVXQ VPTAPLGLYP GKLKSLXXVK XGSTVSAPND
151 PXXFXRVLVM LDELGXIKLK DXIXXXXXXX XXXXXXXXXX XXXXXXXXXX
201 XXXXAXXXXX XXXXXXXXXS GMKLTEALFQ EPSFAYVNWS AVKTADKDSQ
251 WLKDVTEAYN SDAFKAYAHK RFEGYKSPAA WNEGAAK*
```

A leader peptide is underlined.

Further analysis of these strain A sequences revealed the complete DNA sequence <SEQ ID 221 >:

```
  1  ATGAAAACCT TCTTCAAAAC CCTTTCCGCC GCCGCACTCG CGCTCATCCT
 51  CGCCGCCTGC GGCGGTCAAA AAGATAGCGC GCCCGCCGCA TCCGCTTCTG
101  CCGCCGCCGA CAACGGCGCG GCGAAAAAAG AAATCGTCTT CGGCACGACC
151  GTCGGCGACT TCGGCGATAT GGTCAAAGAA CAAATCCAAC CCGAGCTGGA
201  GAAAAAAGGC TACACCGTCA AACTGGTCGA GTTTACCGAC TATGTGCGCC
251  CGAATCTGGC ATTGGCTGAG GGCGAGTTGG ACATCAACGT CTTCCAACAC
301  AAACCCTATC TTGACGACTT CAAAAAAGAA CACAATCTGG ACATCACCGA
351  AGTCTTCCAA GTGCCGACCG CGCCTTTGGG ACTGTACCCG GGCAAGCTGA
401  AATCGCTGGA AGAAGTCAAA GACGGCAGCA CCGTATCCGC GCCCAACGAC
451  CCGTCCAACT TCGCCCGCGT CTTGGTGATG CTCGACGAAC TGGGTTGGAT
501  CAAACTCAAA GACGGCATCA ATCCGCTGAC CGCATCCAAA GCGGACATTG
551  CCGAAAACCT GAAAAACATC AAAATCGTCG AGCTTGAAGC CGCGCAACTG
601  CCGCGTAGCC GCGCCGACGT GGATTTTGCC GTCGTCAACG GCAACTACGC
651  CATAAGCAGC GGCATGAAGC TGACCGAAGC CCTGTTCCAA GAACCGAGCT
701  TTGCCTATGT CAACTGGTCT GCCGTCAAAA CCGCCGACAA AGACAGCCAA
751  TGGCTTAAAG ACGTAACCGA GGCCTATAAC TCCGACGCGT TCAAAGCCTA
801  CGCGCACAAA CGCTTCGAGG GCTACAAATC CCCTGCCGCA TGGAATGAAG
851  GCGCAGCCAA ATAA
```

This encodes a protein having amino acid sequence <SEQ ID 222; ORF4a-1>:

```
  1  MKTFFKTLSA AALALILAAC GGQKDSAPAA SASAAADNGA AKKEIVFGTT
 51  VGDFGDMVKE QIQPELEKKG YTVKLVEFTD YVRPNLALAE GELDINVFQH
101  KPYLDDFKKE HNLDITEVFQ VPTAPLGLYP GKLKSLEEVK DGSTVSAPND
151  PSNFARVLVM LDELGWIKLK DGINPLTASK ADIAENLKNI KIVELEAAQL
201  PRSRADVDFA VVNGNYAISS GMKLTEALFQ EPSFAYVNWS AVKTADKDSQ
251  WLKDVTEAYN SDAFKAYAHK RFEGYKSPAA WNEGAAK*
```

ORF4a-1 and ORF4-1 show 99.7% identity in 287 aa overlap:

```
                       10        20        30        40        50        60
orf4a-1        MKTFFKTLSAAALALILAACGGQKDSAPAASASAAADNGAAKKEIVFGTTVGDFGDMVKE
               ||||||||||||||||||||||||||||||||||||||||||||||||||||||||||||
orf4-1         MKTFFKTLSAAALALILAACGGQKDSAPAASASAAADNGAAKKEIVFGTTVGDFGDMVKE
                       10        20        30        40        50        60

                       70        80        90       100       110       120
orf4a-1        QIQPELEKKGYTVKLVEFTDYVRPNLALAEGELDINVFQHKPYLDDFKKEHNLDITEVFQ
               ||| ||||||||||||||||||||||||||||||||||||||||||||||||||||||||
orf4-1         QIQAELEKKGYTVKLVEFTDYVRPNLALAEGELDINVFQHKPYLDDFKKEHNLDITEVFQ
                       70        80        90       100       110       120

                      130       140       150       160       170       180
orf4a-1        VPTAPLGLYPGKLKSLEEVKDGSTVSAPNDPSNFARVLVMLDELGWIKLKDGINPLTASK
               ||||||||||||||||||||||||||||||||||||||||||||||||||||||||||||
orf4-1         VPTAPLGLYPGKLKSLEEVKDGSTVSAPNDPSNFARVLVMLDELGWIKLKDGINPLTASK
                      130       140       150       160       170       180

                      190       200       210       220       230       240
orf4a-1        ADIAENLKNIKIVELEAAQLPRSRADVDFAVVNGNYAISSGMKLTEALFQEPSFAYVNWS
               ||||||||||||||||||||||||||||||||||||||||||||||||||||||||||||
orf4-1         ADIAENLKNIKIVELEAAQLPRSRADVDFAVVNGNYAISSGMKLTEALFQEPSFAYVNWS
                      190       200       210       220       230       240

                      250       260       270       280
orf4a-1        AVKTADKDSQWLKDVTEAYNSDAFKAYAHKRFEGYKSPAAWNEGAAKX
               ||||||||||||||||||||||||||||||||||||||||||||||||
orf4-1         AVKTADKDSQWLKDVTEAYNSDAFKAYAHKRFEGYKSPAAWNEGAAKX
                      250       260       270       280
```

Homology with an outer membrane protein of *Pasteurella haemolitica* (accession q08869).

ORF4 and this outer membrane protein show 33% aa identity in 91aa overlap:

```
                                                    10        20
lip2.pasha                                  MNFKKLLGVALVSALALTACKDEKAQAP----
                                              || | ::||  || |:||  :|:  |
ORF4           VXTPNPDGRTPCPSFLFETATTSGENMKTFFKTLSAAAL--ALILAACGFKKTARPPHPL
                     110       120       130       140         150

                30        40        50        60        70        80
lip2.pasha     -ATTAKTENKAPLKVGVMTGPEAQMTEVAVKIAKEKYGLDVELVQFTEYTQPNAALHSKD
                   : :: |  :  |: :|  ::|::  ::   || |  |:||:||:|::|| ||   :
ORF4           LPPPTTARRKKEIVFGTTVGDFGDMVKEQIQAELEKKGYTVKLVEFTDYVRPNLALAEGE
             160       170       180       190       200       210

                90       100       110       120       130       140
lip2.pasha     LDANAFQTVPYLEQEVKDRGYKLAIIGNTLVWPIAAYSKKIKNISELKDGATVAIPNNAS
               |
ORF4           L.....
```

Homology with a predicted ORF from *N.gonorrhoeae*

ORF4 shows 93.6% identity over a 94aa overlap with a predicted ORF (ORF4.ng) from *N. gonorrhoeae:*

```
                                                      10        20        30
orf4nm.pep                                   MKTFFKTLSAAALALILAACGXQKDSAPAA
                                             |||||||||:|:||||||||| ||||||||
orf4ng         RANAVXTPNPDGRTPCLSFLFETATTSGENMKTFFKTLSTASLALILAACGGQKDSAPAA
                      200       210       220       230       240       250
```

```
                       40        50        60        70        80        89
orf4nm.pep    SASA-AADNGAAKKEIVFGTTVGDFGDMVKEQIQAELEKKGYTVKLVEFTDYVRPNLALA
              ||:| :|||||||||||||||||||||||||||||||||||||||||||||||||||||
orf4ng        SAAAPSADNGAAKKEIVFGTTVGDFGDMVKEQIQAELEKKGYTVKLVEFTDYVRPNLALA
                     260       270       280       290       300       310

             90
orf4nm.pep    EGEL
              ||||
orf4ng        EGELDINVFQHKPYLDDFKKEHNLDITEAFQVPTAPLGLYPGKLKSLEEVKDGSTVSAPN
                     320       330       340       350       360       370
```

The complete length ORF4ng nucleotide sequence <SEQ ID 223> was predicted to encode a protein having amino acid sequence <SEQ ID 224>:

```
  1 MKTFFKTLST ASLALILAAC GGQKDSAPAA SAAAPSADNG AAKKEIVFGT
 51 TVGDFGDMVK EQIQAELEKK GYTVKLVEFT DYVRPNLALA EGELDINVFQ
101 HKPYLDDFKK EHNLDITEAF QVPTAPLGLY PGKLKSLEEV KDGSTVSAPN
151 DPSNFARALV MLNELGWIKL KDGINPLTAS KADIAENLKN IKIVELEAAQ
201 LPRSRADVDF AVVNGNYAIS SGMKLTEALF QEPSFAYVNW SAVKTADKDS
251 QWLKDVTEAY NSDAFKAYAH KRFEGYKYPA AWNEGAAK*
```

Further analysis revealed the complete length ORF4ng DNA sequence <SEQ ID 225> to be:

```
  1 atgAAAACCT TCTTCAAAAC cctttccgcc gccgcaCTCG CGCTCATCCT
 51 CGCAGCCTGc ggCggtcaAA AAGACAGCGC GCCCgcagcc tctgcCGCCG
101 CCCCTTCTGC CGATAACGgc gCgGCGAAAA AAGAAAtcgt ctTCGGCACG
151 Accgtgggcg acttcggcgA TAtggTCAAA GAACAAATCC AagcCGAgct
201 gGAGAAAAAA GgctACACcg tcAAattggt cgaatttacc gactatgtGC
251 gCCCGAATCT GGCATTGGCG GAGGGCGAGT TGGACATCAA CGTCTTCCAA
301 CACAAACCCT ATCTTGACGA TTTCAAAAAA GAACACAACC TGGACATCAC
351 CGAAGCCTTC CAAGTGCCGA CCGCGCCTTT GGGACTGTAT CCGGGCAAAC
401 TGAAATCGCT GGAAGAAGTC AAAGACGGCA GCACCGTATC CGCGCCCAac
451 gACccgTCCA ACTTCGCACG CGCCTTGGTG ATGCTGAACG AACTGGGTTG
501 GATCAAACTC AAAGACGGCA TCAATCCGCT GACCGCATCC AAAGCCGACA
551 TCGCGGAAAA CCTGAAAAAC ATCAAAATCG TCGAGCTTGA AGCCGCACAA
601 CTGCCGCGCA GCCGCGCCGA CGTGGATTTT GCCGTCGTCA ACGGCAACTA
651 CGCCATAAGC AGCGGCATGA AGCTGACCGA AGCCCTGTTC CAAGAGCCGA
701 GCTTTGCCTA TGTCAACTGG TCTGCCgtcA AAACCGCCGA CAAAGACAGC
751 CAATGGCTTA AAGACGTAAC CGAGGCCTAT AACTCCGACG CGTTCAAAGC
801 CTACGCGCAC AAACGCTTCG AGGGCTACAA ATACCCTGCC GCATGGAATG
851 AAGGCGCAGC CAAATAA
```

This encodes a protein having amino acid sequence <SEQ ID 226; ORF4ng-1>:

```
  1 MKTFFKTLSA AALALILAAC GGQKDSAPAA SAAAPSADNG AAKKEIVFGT
 51 TVGDFGDMVK EQIQAELEKK GYTVKLVEFT DYVRPNLALA EGELDINVFQ
101 HKPYLDDFKK EHNLDITEAF QVPTAPLGLY PGKLKSLEEV KDGSTVSAPN
151 DPSNFARALV MLNELGWIKL KDGINPLTAS KADIAENLKN IKIVELEAAQ
201 LPRSRADVDF AVVNGNYAIS SGMKLTEALF QEPSFAYVNW SAVKTADKDS
251 QWLKDVTEAY NSDAFKAYAH KRFEGYKYPA AWNEGAAK*
```

This shows 97.6% identity in 288 aa overlap with ORF4-1:

```
                       10        20        30         40        50       59
orf4-1.pep     MKTFFKTLSAAALALILAACGGQKDSAPAASASA-AADNGAAKKEIVFGTTVGDFGDMVK
               ||||||||||||||||||||||||||||||||:| :||||||||||||||||||||||||
orf4ng-1       MKTFFKTLSAAALALILAACGGQKDSAPAASAAAPSADNGAAKKEIVFGTTVGDFGDMVK
                       10        20        30        40        50        60

               60        70        80        90       100       110      119
orf4-1.pep     EQIQAELEKKGYTVKLVEFTDYVRPNLALAEGELDINVFQHKPYLDDFKKEHNLDITEVF
               ||||||||||||||||||||||||||||||||||||||||||||||||||||||||||:|
orf4ng-1       EQIQAELEKKGYTVKLVEFTDYVRPNLALAEGELDINVFQHKPYLDDFKKEHNLDITEAF
                       70        80        90       100       110       120

              120       130       140       150       160       170      179
orf4-1.pep     QVPTAPLGLYPGKLKSLEEVKDGSTVSAPNDPSNFARVLVMLDELGWIKLKDGINPLTAS
               |||||||||||||||||||||||||||||||||||||:||||:|||||||||||||||||
orf4ng-1       QVPTAPLGLYPGKLKSLEEVKDGSTVSAPNDPSNFARALVMLNELGWIKLKDGINPLTAS
                       130       140       150       160       170       180

              180       190       200       210       220       230      239
orf4-1.pep     KADIAENLKNIKIVELEAAQLPRSRADVDFAVVNGNYAISSGMKLTEALFQEPSFAYVNW
               ||||||||||||||||||||||||||||||||||||||||||||||||||||||||||||
orf4ng-1       KADIAENLKNIKIVELEAAQLPRSRADVDFAVVNGNYAISSGMKLTEALFQEPSFAYVNW
                       190       200       210       220       230       240

              240       250       260       270       280
orf4-1.pep     SAVKTADKDSQWLKDVTEAYNSDAFKAYAHKRFEGYKSPAAWNEGAAKX
               |||||||||||||||||||||||||||||||||||||| |||||||||||
orf4ng-1       SAVKTADKDSQWLKDVTEAYNSDAFKAYAHKRFEGYKYPAAWNEGAAKX
                       250       260       270       280
```

In addition, ORF4ng-1 shows significant homology with an outer membrane protein from the database:

```
ID   LIP2_PASHA     STANDARD;      PRT;   276 AA.
AC   Q08869;
DT   01-NOV-1995 (REL. 32, CREATED)
DT   01-NOV-1995 (REL. 32, LAST SEQUENCE UPDATE)
DT   01-NOV-1995 (REL. 32, LAST ANNOTATION UPDATE)
DE   28.2 KD OUTER MEMBRANE PROTEIN PRECURSOR. . . .
SCORES      Init1:   279 Initn:   416 Opt:   494
Smith-Waterman score: 494;    36.0% identity in 275 aa overlap

                            10        20        30        40        50
orf4ng-1.pep    MKTFFKTLSAAAL--ALILAACGGQKDSAPAASAAAPSADNGAAKKEIVFGTTVGDFGDM
                    || | ::||  || |:||  :| :|||::|   :::| |  |     |: :|  ::|
lip2_pasha        MNFKKLLGVALVSALALTACKDEKAQAPATTA---KTENKAPLK---VGVMTGPEAQM
                          10        20        30           40          50

                60        70        80        90       100       110
orf4ng-1.pep    VKEQIQAELEKKGYTVKLVEFTDYVRPNLALAEGELDINVFQHKPYLDDFKKEHNLDITE
                ::  ::     || |  |:||:||:|::|| ||    :|| |:||  |||::  |:::  ::
lip2_pasha      TEVAVKIAKEKYGLDVELVQFTEYTQPNAALHSKDLDANAFQTVPYLEQEVKDRGYKLAI
                      60        70        80        90       100       110

               120       130       140       150       160       170
orf4ng-1.pep    AFQVPTAPLGLYPGKLKSLEEVKDGSTVSAPNDPSNFARALVMLNELGWIKLKDGINPLT
                  :: : |:: |  |:|:: |:|||:||: ||: || ||||::|:  | :||||  | :
lip2_pasha      IGNTLVWPIAAYSKKIKNISELKDGATVAIPNNASNTARALLLLQAHGLLKLKDPKN-VF
                      120       130       140       150       160       170

               180       190       200       210       220        230
orf4ng-1.pep    ASKADIAENLKNIKIVELEAAQLPRSRADVDFAVVNGNYAISSGMKLTE--ALFQEPSFA
                |:: || || ||||||: ::: | |    ||::||:|::|| ::|::  :    : :  :
lip2_pasha      ATENDIIENPKNIKIVQADTSLLTRMLDDVELAVINNTYAGQAGLSPDKDGIIVESKDSP
                      180       190       200       210       220       230

                 240       250       260       270       280       289
orf4ng-1.pep    YVNWSAVKTADKDSQWLKDVTEAYNSDAFKAYAHKRFEGYKYPAAWNEGAAKX
                |||  : :  :||:  |:  :::::::      | | |:|
lip2_pasha      YVNLVVSREDNKDDPRLQTFVKSFQTEEVFQEALKLFNGGVVKGW
                      240       250       260       270
```

Based on this analysis, including the homology with the outer membrane protein of *Pasteurella haemolitica,* and on the presence of a putative prokaryotic membrane lipoprotein lipid attachment site in the gonococcal protein, it was predicted that these proteins from *N.meningitidis* and *N.gonorrhoeae,* and their epitopes, could be useful antigens for vaccines or diagnostics, or for raising antibodies.

ORF4-1 (30kDa) was cloned in pET and pGex vectors and expressed in *E.coli,* as described above. The products of protein expression and purification were analyzed by SDS-PAGE. Figures 8A and 8B show, repsectively, the results of affinity purification of the His-fusion and GST-fusion proteins. Purified His-fusion protein was used to immunise mice, whose sera were used for ELISA (positive result), Western blot (Figure 8C), FACS analysis (Figure 8D), and a bactericidal assay (Figure 8E). These experiments confirm that ORF4-1 is a surface-exposed protein, and that it is a useful immunogen.

Figure 8F shows plots ofhydrophilicity, antigenic index, and AMPHI regions for ORF4-1.

### Example 27

The following partial DNA sequence was identified in *N.meningitidis* <SEQ ID 227>:

```
  1 CCTCGTCGTC CTCGGCATGC TCCAGTTTCA AGGGGCGATT TACTCCAAGG
 51 CGGTGGAACG TATGCTCGGC ACGGTCATCG GGCTGGGCGC GGGTTTGGGC
101 GTTTTATGGC TGAACCAGCA TTATTTCCAC GGCAACCTCC TCTTCTACCT
151 CACCGTCGGC ACGGCAAGCG CACTGGCCGG CTGGGCGGCG GTCGGCAAAA
201 ACGGCTACGT CCCTmTGCTG GCAGGGCTGA CGATGTGTAT GCTCATCGGC
251 GACAACGGCA GCGAATGGCT CGACAGCGGA CTCATGCGCG CCATGAACGT
301 CCTCATCGGC GyGGCCATCG CCATCGCCGC CGCCAAACTG CTGCCGCTGA
351 AATCCACACT GATGTGGCGT TTCATGCTTG CCGACAACCT GGCCGACTGC
401 AGCAAAATGA TTGCCGAAAT CAGCAACGGC AGGCGCATGA CCCGCGAACG
451 CCTCGAGGAG AACATGGCGA AAATGCGCCA AATCAACGCA CGCATGGTCA
501 AAAGCCGCAG CCATCTCGCC GCCACATCGG GCGAAAGCTG CATCAGCCCC
551 GCCATGATGG AAGCCATGCA GCACGCCCAC CGTAAAATCG TCAACACCAC
601 CGAGCTGCTC CTGACCACCG CCGCCAAGCT GCAATCTCCC AAACTCAACG
651 GCAGCGAAAT CCGGCTGCTT GACCGCCACT TCACACTGCT CCAAAC....
701 ............................ GC AGACACGCCC GCCGCATCCG
751 CATCGACACC GCCATCAACC CCGAACTGGA AGCCCTCGCC GAACACCTCC
801 ACTACCAATG GCAGGGCTTC CTCTGGCTCA GCACCGATAT GCGTCAGGAA
851 ATTTCCGCCC TCGTCATCCT GCTGCAACGC ACCCGCCGCA AATGGCTGGA
901 TGCCCACGAA CGCCAACACC TGCGCCAAAG CCTGCTTGA
```

This corresponds to the amino acid sequence <SEQ ID 228; ORF8>:

```
  1 ......PRRP RHAPVSRGDL LQGGGTYARH GHRAGRGFGR FMAEPALFPR
 51 QPPLLPHRRH GKRTGRLGGG RQKRLRPXAG RADDVYAHRR QRQRMARQRT
101 HARHERPHRR GHRHRRRQTA AAEIHTDVAF HACRQPGRLQ QNDCRNQQRQ
151 AHDPRTPRGE HGENAPNQRT HGQKPQPSRR HIGRKLHQPR HDGSHAARPP
201 XNRQHHRAAP DHRRQAAISQ TQRQRNPAAX PPLHTAPN.. .........Q
251 TRPPHPHRHR HQPRTGSPRR TPPLPMAGLP LAQHRYASGN FRPRHPAATH
301 PPQMAGCPRT PTPAPKPA*
```

Computer analysis of this amino acid sequence gave the following results:

Sequence motifs

ORF8 is proline-rich and has a distribution of proline residues consistent with a surface localization. Furthermore the presence of an RGD motif may indicate a possible role in bacterial adhesion events.

Homology with a predicted ORF from *N.gonorrhoeae*

ORF8 shows 86.5% identity over a 312aa overlap with a predicted ORF (ORF8.ng) from *N. gonorrhoeae:*

```
orf8ng         1 MDRDDRLRRPRHAPVPRRDLLQRGGTYARYGHRAGRGFGRFMAEPALFPR 50
                        |||||||| | |||| ||||||:||||||||||||||||||||
orf8.pep       1 ......PRRPRHAPVSRGDLLQGGGTYARHGHRAGRGFGRFMAEPALFPR 44

orf8ng        51 QPPLLPDHRHGKRTGRLGGGRQKRLRPYVGGADDVHAHRRQRQRMARQRP 100
                 ||||||  |||||||||||||||||||  | ||||:|||||||||||||
orf8.pep      45 QPPLLPHRRHGKRTGRLGGGRQKRLRPXAGRADDVYAHRRQRQRMARQRT 94

orf8ng       101 DARDERPHRRRHRHCRRQTAAAEIHTDVAFHACRQPGRLQQNDCRNQQRQ 150
                  || |||||| ||| ||||||||||||||||||||||| |||||||||||
orf8.pep      95 HARHERPHRRGHRHRRRQTAAAEIHTDVAFHACRQPGRMQQNDCRNQQRQ 144

orf8ng       151 AYDARTFGAEYGQNAPNQRTHGQKPQPPRRHIGRKPHQPLHDGSHAARPP 200
                 |:| ||   |:|:|||||||||||||| ||||||| ||| ||||||||||
orf8.pep     145 AHDPRTPRGEHGENAPNQRTHGQKPQPSRRHIGRKLHQPRHDGSHAARPP 194

orf8ng       201 QNRQHHRAAPDHRRQAAISQTQRQRNPAARPPLHTAPNRPATNRRPHQRQ 250
                  |||||||||||||||||||||||||||| ||||||||           |
orf8.pep     195 XNRQHHRAAPDHRRQAAISQTQRQRNPAAXPPLHTAPN...........Q 244

orf8ng       251 TRPPHPHRHRHQPRTGSPRRTPPLPMAGFPLAQHQYASGNFRPRHPPATH 300
                 |||||||||||||||||||||||||||| |||||.||||||||||| |||
orf8.pep     245 TRPPHPHRHRHQPRTGSPRRTPPLPMAGLPLAQHRYASGNFRPRHPAATH 294

orf8ng       301 PPQMAGCPRTPTPAPKPA* 319
                 |||||||||||||||||||
orf8.pep     295 PPQMAGCPRTPTPAPKPA* 313
```

The complete length ORF8ng nucleotide sequence <SEQ ID 229> is predicted to encode a protein having amino acid sequence <SEQ ID 230>:

```
  1  MDRDDRLRRP RHAPVPRRDL LQRGGTYARY GHRAGRGFGR FMAEPALFPR
 51  QPPLLPDHRH GKRTGRLGGG RQKRLRPYVG GADDVHAHRR QRQRMARQRP
101  DARDERPHRR RHRHCRRQTA AAEIHTDVAF HACRQPGRLQ QNDCRNQQRQ
151  AYDARTFGAE YGQNAPNQRT HGQKPQPPRR HIGRKPHQPL HDGSHAARPP
201  QNRQHHRAAP DHRRQAAISQ TQRQRNPAAR PPLHTAPNRP ATNRRPHQRQ
251  TRPPHPHRHR HQPRTGSPRR TPPLPMAGFP LAQHQYASGN FRPRHPPATH
301  PPQMAGCPRT PTPAPKPA*
```

Based on the sequence motifs in these proteins, it is predicted that the proteins from *N.meningitidis* and *N.gonorrhoeae,* and their epitopes, could be useful antigens for vaccines or diagnostics, or for raising antibodies.

## Example 28

The following partial DNA sequence was identified in *N.meningitidis* <SEQ ID 231>:

```
  1  ..GAAATCAGCC TGCGGTCCGA CNACAGGCCG GTTTCCGTGN CGAAGCGGCG
 51    GGATTCGGAA CGTTTTCTGC TGTTGGACGG CGGCAACAGC CGGCTCAAGT
101    GGGCGTGGGT GGAAAACGGC ACGTTCGCAA CCGTCGGTAG CGCGCCGTAC
151    CGCGATTTGT CGCCTTTGGG CGCGGAGTGG GCGGAAAAGG CGGATGGAAA
201    TGTCCGCATC GTCGGTTGCG CTGTGTGCGG AGAATTCAAA AAGGCACAAG
251    TGCAGGAACA GCTCGCCCGA AAAATCGAGT GGCTGCCGTC TTCCGCACAG
301    GCTTT.GGCA TACGCAACCA CTACCGCCAC CCCGAAGAAC ACGGTTCCGA
351    CCGCTGGTTC AACGCCTTGG GCAGCCGCCG CTTCAGCCGC AACGCCTGCG
401    TCGTCGTCAG TTGCGGCACG GCGGTAACGG TTGACGCGCT CACCGATGAC
451    GGACATTATC TCGGAGA.GG AACCATCATG CCCGGTTTCC ACCTGATGAA
501    AGAATCGCTC GCCGTCCGAA CCGCCAACCT CAACCGGCAC GCCGGTAAGC
551    GTTATCCTTT CCCGACCGG..
```

This corresponds to the amino acid sequence <SEQ ID 232; ORF61>:

```
  1 ..EISLRSDXRP VSVXKRRDSE RFLLLDGGNS RLKWAWVENG TFATVGSAPY
 51   RDLSPLGAEW AEKADGNVRI VGCAVCGEFK KAQVQEQLAR KIEWLPSSAQ
101   AXGIRNHYRH PEEHGSDRWF NALGSRRFSR NACVVVSCGT AVTVDALTDD
151   GHYLGXGTIM PGFHLMKESL AVRTANLNRH AGKRYPFPT..
```

Further work revealed the complete nucleotide sequence <SEQ ID 233>:

```
   1 ATGACGGTTT TGAAGCTTTC GCACTGGCGG GTGTTGGCGG AGCTTGCCGA
  51 CGGTTTGCCG CAACACGTCT CGCAACTGGC GCGTATGGCG GATATGAAGC
 101 CGCAGCAGCT CAACGGTTTT TGGCAGCAGA TGCCGGCGCA CATACGCGGG
 151 CTGTTGCGCC AACACGACGG CTATTGGCGG CTGGTGCGCC CATTGGCGGT
 201 TTTCGATGCC GAAGGTTTGC GCGAGCTGGG GGAAAGGTCG GGTTTTCAGA
 251 CGGCATTGAA GCACGAGTGC GCGTCCAGCA ACGACGAGAT ACTGGAATTG
 301 GCGCGGATTG CGCCGGACAA GGCGCACAAA ACCATATGCG TGACCCACCT
 351 GCAAAGTAAG GGCAGGGGGC GGCAGGGGCG GAAGTGGTCG CACCGTTTGG
 401 GCGAGTGTCT GATGTTCAGT TTTGGCTGGG TGTTTGACCG GCCGCAGTAT
 451 GAGTTGGGTT CGCTGTCGCC TGTTGCGGCA GTGGCGTGTC GGCGCGCCTT
 501 GTCGCGTTTA GGTTTGGATG TGCAGATTAA GTGGCCCAAT GATTTGGTTG
 551 TCGGACGCGA CAAATTGGGC GGCATTCTGA TTGAAACGGT CAGGACGGGC
 601 GGCAAAACGG TTGCCGTGGT CGGTATCGGC ATCAATTTTG TCCTGCCCAA
 651 GGAAGTAGAA AATGCCGCTT CCGTGCAATC GCTGTTTCAG ACGGCATCGC
 701 GGCGGGGCAA TGCCGATGCC GCCGTGCTGC TGGAAACGCT GTTGGTGGAA
 751 CTGGACGCGG TGTTGTTGCA ATATGCGCGG GACGGATTTG CGCCTTTTGT
 801 GGCGGAATAT CAGGCTGCCA ACCGCGACCA CGGCAAGGCG GTATTGCTGT
 851 TGCGCGACGG CGAAACCGTG TTCGAAGGCA CGGTTAAAGG CGTGGACGGA
 901 CAAGGCGTTT TGCACTTGGA AACGGCAGAG GGCAAACAGA CGGTCGTCAG
 951 CGGCGAAATC AGCCTGCGGT CCGACGACAG GCCGGTTTCC GTGCCGAAGC
1001 GGCGGGATTC GGAACGTTTT CTGCTGTTGG ACGGCGGCAA CAGCCGGCTC
1051 AAGTGGGCGT GGGTGGAAAA CGGCACGTTC GCAACCGTCG GTAGCGCGCC
1101 GTACCGCGAT TTGTCGCCTT TGGGCGCGGA GTGGGCGGAA AAGGCGGATG
1151 GAAATGTCCG CATCGTCGGT TGCGCTGTGT GCGGAGAATT CAAAAAGGCA
1201 CAAGTGCAGG AACAGCTCGC CCGAAAAATC GAGTGGCTGC CGTCTTCCGC
1251 ACAGGCTTTG GGCATACGCA ACCACTACCG CCACCCCGAA GAACACGGTT
1301 CCGACCGCTG GTTCAACGCC TTGGGCAGCC GCCGCTTCAG CCGCAACGCC
1351 TGCGTCGTCG TCAGTTGCGG CACGGCGGTA ACGGTTGACG CGCTCACCGA
1401 TGACGGACAT TATCTCGGGG GAACCATCAT GCCCGGTTTC CACCTGATGA
1451 AAGAATCGCT CGCCGTCCGA ACCGCCAACC TCAACCGGCA CGCCGGTAAG
1501 CGTTATCCTT TCCCGACCAC AACGGGCAAT GCCGTCGCCA GCGGCATGAT
1551 GGATGCGGTT TGCGGCTCGG TTATGATGAT GCACGGGCGT TTGAAAGAAA
1601 AAACCGGGGC GGGCAAGCCT GTCGATGTCA TCATTACCGG CGGCGGCGCG
1651 GCAAAAGTTG CCGAAGCCCT GCCGCCTGCA TTTTTGGCGG AAAATACCGT
1701 GCGCGTGGCG GACAACCTCG TCATTTACGG GTTGTTGAAC ATGATTGCCG
1751 CCGAAGGCAG GGAATATGAA CATATTTAA
```

This corresponds to the amino acid sequence <SEQ ID 234; ORF61-1>:

```
  1 MTVLKLSHWR VLAELADGLP QHVSQLARMA DMKPQQLNGF WQQMPAHIRG
 51 LLRQHDGYWR LVRPLAVFDA EGLRELGERS GFQTALKHEC ASSNDEILEL
101 ARIAPDKAHK TICVTHLQSK GRGRQGRKWS HRLGECLMFS FGWVFDRPQY
151 ELGSLSPVAA VACRRALSRL GLDVQIKWPN DLVVGRDKLG GILIETVRTG
201 GKTVAVVGIG INFVLPKEVE NAASVQSLFQ TASRRGNADA AVLLETLLVE
251 LDAVLLQYAR DGFAPFVAEY QAANRDHGKA VLLLRDGETV FEGTVKGVDG
301 QGVLHLETAE GKQTVVSGEI SLRSDDRPVS VPKRRDSERF LLLDGGNSRL
351 KWAWVENGTF ATVGSAPYRD LSPLGAEWAE KADGNVRIVG CAVCGEFKKA
401 QVQEQLARKI EWLPSSAQAL GIRNHYRHPE EHGSDRWFNA LGSRRFSRNA
451 CVVVSCGTAV TVDALTDDGH YLGGTIMPGF HLMKESLAVR TANLNRHAGK
501 RYPFPTTTGN AVASGMMDAV CGSVMMMHGR LKEKTGAGKP VDVIITGGGA
551 AKVAEALPPA FLAENTVRVA DNLVIYGLLN MIAAEGREYE HI*
```

Figure 9 shows plots of hydrophilicity, antigenic index, and AMPHI regions for ORF61-1. Further computer analysis of this amino acid sequence gave the following results:

Homology with the baf protein *of B. pertussis* accession number U12020).

ORF61 and baf protein show 33% aa identity in 166aa overlap:

```
orf61  23  LLLDGGNSRLKWAWVE-NGTFATVGSAPYR----DLSPLGAEWAEKADGNVRIVGCAVCG 77
           +L+D GNSRLK  W + +    A    AP       DL  LG    A         R +G  V G
baf    3   ILIDSGNSRLKVGWFDPDAPQAAREPAPVAFDNLDLDALGRWLATLPRRPQRALGVNVAG 62

orf61  78  EFKKAQVQEQLAR---KIEWLPSSAQAXGIRNHYRHPEEHGSDRW---FNALGSRRFSRN 131
            +   +   L       I WL +    A G+RN YR+P++ G+DRW       L  +
baf    63  LARGEAIAATLRAGGCDIRWLRAQPLAMGLRNGYRNPDQLGADRWACMVGVLARQPSVHP 122

orf61  132 ACVVVSCGTAVTVDALTDDGHYLGXGTIMPGFHLMKESLAVRTANL 177
             +V  S GTA  T+D +   D   + G G I+PG   +M+ +LA   TA+L
baf    123 PLLVASFGTATTLDTIGPDNVFPG-GLILPGPAMMRGALAYGTAHL 167
```

Homology with a predicted ORF from *N.meningitidis* (strain A)

ORF61 shows 97.4% identity over a 189aa overlap with an ORF (ORF61a) from strain A of *N. meningitidis:*

```
                                                         10        20        30
orf61.pep                                      EISLRSDXRPVSVXKRRDSERFLLLDGGNS
                                               ||||||| ||||| ||||||||||||||||
orf61a      TVFEGTVKGVDGQGVLHLETAEGKQTVVSGEISLRSDDRPVSVPKRRDSERFLLLDGGNS
           290       300       310       320       330       340

                    40        50        60        70        80        90
orf61.pep   RLKWAWVENGTFATVGSAPYRDLSPLGAEWAEKADGNVRIVGCAVCGEFKKAQVQEQLAR
            |||||||||||||||||||||||||||||||||:||||||||||||||||||||||||||
orf61a      RLKWAWVENGTFATVGSAPYRDLSPLGAEWAEKVDGNVRIVGCAVCGEFKKAQVQEQLAR
           350       360       370       380       390       400

                   100       110       120       130       140       150
orf61.pep   KIEWLPSSAQAXGIRNHYRHPEEHGSDRWFNALGSRRFSRNACVVVSCGTAVTVDALTDD
            ||||||||||| ||||||||||||||||||||||||||||||||||||||||||||||||
orf61a      KIEWLPSSAQALGIRNHYRHPEEHGSDRWFNALGSRRFSRNACVVVSCGTAVTVDALTDD
           410       420       430       440       450       460

                   160       170       180      189
orf61.pep   GHYLGXGTIMPGFHLMKESLAVRTANLNRHAGKRYPFPT
            ||||| |||||||||||||||||||||||||||||||||
orf61a      GHYLG-GTIMPGFHLMKESLAVRTANLNRHAGKRYPFPTTTGNAVASGMMDAVCGSVMMM
           470       480       490       500       510       520

orf61a      HGRLKEKTGAGKPVDVIITGGGAAKVAEALPPAFLAENTVRVADNLVIHGLLNLIAAEGG
           530       540       550       560       570       580
```

The complete length ORF61a nucleotide sequence <SEQ ID 235> is:

```
   1  ATGACGGTTT TGAAGCCTTC GCACTGGCGG GTGTTGGCGG AGCTTGCCGA
  51  CGGTTTGCCG CAACACGTCT CGCAACTGGC GCGTATGGCG GATATGAAGC
 101  CGCAGCAGCT CAACGGTTTT TGGCAGCAGA TGCCGGCGCA CATACGCGGG
 151  CTGTTGCGCC AACACGACGG CTATTGGCGG CTGGTGCGCC CATTGGCGGT
 201  TTTCGATGCC GAAGGTTTGC GCGAGCTGGG GGAAAGGTCG GGTTTTCAGA
 251  CGGCATTGAA GCACGAGTGC GCGTCCAGCA ACGACGAGAT ACTGGAATTG
 301  GCGCGGATTG CGCCGGACAA GGCGCACAAA ACCATATGTG TGACCCACCT
 351  GCAAAGTAAG GGCAGGGGGC GGCAGGGGCG GAAGTGGTCG CACCGTTTGG
 401  GCGAGTGTCT GATGTTCAGT TTTGGCTGGG TGTTTGACCG GCCGCAGTAT
 451  GAGTTGGGTT CGCTGTCGCC TGTTGCGGCA GTGGCGTGCC GGCGCGCCTT
 501  GTCGCGTTTG GGTTTGAAAA CGCAAATCAA GTGGCCAAAC GATTTGGTCG
 551  TCGGACGCGA CAAATTGGGC GGCATTCTGA TTGAAACGGT CAGGACGGGC
 601  GGCAAAACGG TTGCCGTGGT CGGTATCGGC ATCAATTTCG TGCTGCCCAA
 651  GGAAGTGGAA AACGCCGCTT CCGTGCAATC GCTGTTTCAG ACGGCATCGC
 701  GGCGGGGAAA TGCCGATGCC GCCGTGTTGC TGGAAACGCT GTTGGCGGAA
 751  CTTGATGCGG TGTTGTTGCA ATATGCGCGG GACGGATTTG CGCCTTTTGT
 801  GGCGGAATAT CAGGCTGCCA ACCGCGACCA CGGCAAGGCG GTATTGCTGT
 851  TGCGCGACGG CGAAACCGTG TTCGAAGGCA CGGTTAAAGG CGTGGACGGA
 901  CAAGGCGTTC TGCACTTGGA AACGGCAGAG GGCAAACAGA CGGTCGTCAG
 951  CGGCGAAATC AGCCTGCGGT CCGACGACAG GCCGGTTTCC GTGCCGAAGC
1001  GGCGGGATTC GGAACGTTTT CTGCTGTTGG ACGGCGGCAA CAGCCGGCTC
1051  AAGTGGGCGT GGGTGGAAAA CGGCACGTTC GCAACCGTCG GTAGCGCGCC
1101  GTACCGCGAT TTGTCGCCTT TGGGCGCGGA GTGGGCGGAA AAGGTGGATG
1151  GAAATGTCCG CATCGTCGGT TGCGCCGTGT GCGGAGAATT CAAAAAGGCA
1201  CAAGTGCAGG AACAGCTCGC CCGAAAAATC GAGTGGCTGC CGTCTTCCGC
1251  ACAGGCTTTG GGCATACGCA ACCACTACCG CCACCCCGAA GAACACGGTT
1301  CCGACCGCTG GTTCAACGCC TTGGGCAGCC GCCGCTTCAG CCGCAACGCC
1351  TGCGTCGTCG TCAGTTGCGG CACGGCGGTA ACGGTTGACG CGCTCACCGA
1401  TGACGGACAT TATCTCGGGG GAACCATCAT GCCCGGTTTC CACCTGATGA
1451  AAGAATCGCT CGCCGTCCGA ACCGCCAACC TCAACCGGCA CGCCGGTAAG
1501  CGTTATCCTT TCCCGACCAC AACGGGCAAT GCCGTCGCCA GCGGCATGAT
1551  GGATGCGGTT TGCGGCTCGG TTATGATGAT GCACGGGCGT TTGAAAGAAA
```

```
1601  AAACCGGGGC GGGCAAGCCT GTCGATGTCA TCATTACCGG CGGCGGCGCG
1651  GCAAAAGTTG CCGAAGCCCT GCCGCCTGCA TTTTTGGCGG AAAATACCGT
1701  GCGCGTGGCG GACAACCTCG TCATTCACGG GCTGCTGAAC CTGATTGCCG
1751  CCGAAGGCGG GGAATCGGAA CATACTTAA
```

This encodes a protein having amino acid sequence <SEQ ID 236>:

```
  1  MTVLKPSHWR VLAELADGLP QHVSQLARMA DMKPQQLNGF WQQMPAHIRG
 51  LLRQHDGYWR LVRPLAVFDA EGLRELGERS GFQTALKHEC ASSNDEILEL
101  ARIAPDKAHK TICVTHLQSK GRGRQGRKWS HRLGECLMFS FGWVFDRPQY
151  ELGSLSPVAA VACRRALSRL GLKTQIKWPN DLVVGRDKLG GILIETVRTG
201  GKTVAVVGIG INFVLPKEVE NAASVQSLFQ TASRRGNADA AVLLETLLAE
251  LDAVLLQYAR DGFAPFVAEY QAANRDHGKA VLLLRDGETV FEGTVKGVDG
301  QGVLHLETAE GKQTVVSGEI SLRSDDRPVS VPKRRDSERF LLLDGGNSRL
351  KWAWVENGTF ATVGSAPYRD LSPLGAEWAE KVDGNVRIVG CAVCGEFKKA
401  QVQEQLARKI EWLPSSAQAL GIRNHYRHPE EHGSDRWFNA LGSRRFSRNA
451  CVVVSCGTAV TVDALTDDGH YLGGTIMPGF HLMKESLAVR TANLNRHAGK
501  RYPFPTTTGN AVASGMMDAV CGSVMMMHGR LKEKTGAGKP VDVIITGGGA
551  AKVAEALPPA FLAENTVRVA DNLVIHGLLN LIAAEGGESE HT*
```

ORF61a and ORF61-1 show 98.5% identity in 591 aa overlap:

```
                    10        20        30        40        50        60
orf61a.pep  MTVLKPSHWRVLAELADGLPQHVSQLARMADMKPQQLNGFWQQMPAHIRGLLRQHDGYWR
            ||||| ||||||||||||||||||||||||||||||||||||||||||||||||||||||
orf61-1     MTVLKLSHWRVLAELADGLPQHVSQLARMADMKPQQLNGFWQQMPAHIRGLLRQHDGYWR
                    10        20        30        40        50        60

                    70        80        90       100       110       120
orf61a.pep  LVRPLAVFDAEGLRELGERSGFQTALKHECASSNDEILELARIAPDKAHKTICVTHLQSK
            ||||||||||||||||||||||||||||||||||||||||||||||||||||||||||||
orf61-1     LVRPLAVFDAEGLRELGERSGFQTALKHECASSNDEILELARIAPDKAHKTICVTHLQSK
                    70        80        90       100       110       120

                   130       140       150       160       170       180
orf61a.pep  GRGRQGRKWSHRLGECLMFSFGWVFDRPQYELGSLSPVAAVACRRALSRLGLKTQIKWPN
            |||||||||||||||||||||||||||||||||||||||||||||||||||| :||||||
orf61-1     GRGRQGRKWSHRLGECLMFSFGWVFDRPQYELGSLSPVAAVACRRALSRLGLDVQIKWPN
                   130       140       150       160       170       180

                   190       200       210       220       230       240
orf61a.pep  DLVVGRDKLGGILIETVRTGGKTVAVVGIGINFVLPKEVENAASVQSLFQTASRRGNADA
            ||||||||||||||||||||||||||||||||||||||||||||||||||||||||||||
orf61-1     DLVVGRDKLGGILIETVRTGGKTVAVVGIGINFVLPKEVENAASVQSLFQTASRRGNADA
                   190       200       210       220       230       240

                   250       260       270       280       290       300
orf61a.pep  AVLLETLLAELDAVLLQYARDGFAPFVAEYQAANRDHGKAVLLLRDGETVFEGTVKGVDG
            ||||||||:|||||||||||||||||||||||||||||||||||||||||||||||||||
orf61-1     AVLLETLLVELDAVLLQYARDGFAPFVAEYQAANRDHGKAVLLLRDGETVFEGTVKGVDG
                   250       260       270       280       290       300

                   310       320       330       340       350       360
orf61a.pep  QGVLHLETAEGKQTVVSGEISLRSDDRPVSVPKRRDSERFLLLDGGNSRLKWAWVENGTF
            ||||||||||||||||||||||||||||||||||||||||||||||||||||||||||||
orf61-1     QGVLHLETAEGKQTVVSGEISLRSDDRPVSVPKRRDSERFLLLDGGNSRLKWAWVENGTF
                   310       320       330       340       350       360

                   370       380       390       400       410       420
orf61a.pep  ATVGSAPYRDLSPLGAEWAEKVDGNVRIVGCAVCGEFKKAQVQEQLARKIEWLPSSAQAL
            |||||||||||||||||||||:||||||||||||||||||||||||||||||||||||||
orf61-1     ATVGSAPYRDLSPLGAEWAEKADGNVRIVGCAVCGEFKKAQVQEQLARKIEWLPSSAQAL
                   370       380       390       400       410       420

                   430       440       450       460       470       480
orf61a.pep  GIRNHYRHPEEHGSDRWFNALGSRRFSRNACVVVSCGTAVTVDALTDDGHYLGGTIMPGF
            ||||||||||||||||||||||||||||||||||||||||||||||||||||||||||||
orf61-1     GIRNHYRHPEEHGSDRWFNALGSRRFSRNACVVVSCGTAVTVDALTDDGHYLGGTIMPGF
                   430       440       450       460       470       480

                   490       500       510       520       530       540
orf61a.pep  HLMKESLAVRTANLNRHAGKRYPFPTTTGNAVASGMMDAVCGSVMMMHGRLKEKTGAGKP
            ||||||||||||||||||||||||||||||||||||||||||||||||||||||||||||
orf61-1     HLMKESLAVRTANLNRHAGKRYPFPTTTGNAVASGMMDAVCGSVMMMHGRLKEKTGAGKP
                   490       500       510       520       530       540

                   550       560       570       580       590
orf61a.pep  VDVIITGGGAAKVAEALPPAFLAENTVRVADNLVIHGLLNLIAAEGGESEHTX
            |||||||||||||||||||||||||||||||||||:||||:||||| | ||
orf61-1     VDVIITGGGAAKVAEALPPAFLAENTVRVADNLVIYGLLNMIAAEGREYEHIX
                   550       560       570       580       590
```

Homology with a predicted ORF from *N. gonorrhoeae*

ORF61 shows 94.2% identity over a 189aa overlap with a predicted ORF (ORF61.ng) from *N. gonorrhoeae:*

```
orf61.pep                                EISLRSDXRPVSVXKRRDSERFLLLDGGNS   30
                                         ||||| | | ||| || ||||||||:||||
orf61ng   TVCEGTVKGVDGRGVLHLETAEGEQTVVSGEISLRPDNRSVSVPKRPDSERFLLLEGGNS  211

orf61.pep RLKWAWVENGTFATVGSAPYRDLSPLGAEWAEKADGNVRIVGCAVCGEFKKAQVQEQLAR   90
          |||||||||||||||||||||||||||||||||||||||||||||||| |||||:|||||
orf61ng   RLKWAWVENGTFATVGSAPYRDLSPLGAEWAEKADGNVRIVGCAVCGESKKAQVKEQLAR  271

orf61.pep KIEWLPSSAQAXGIRNHYRHPEEHGSDRWFNALGSRRFSRNACVVVSCGTAVTVDALTDD  150
          ||||||||||| ||||||||||||||||||||||||||||||||||||||||||||||||
orf61ng   KIEWLPSSAQALGIRNHYRHPEEHGSDRWFNALGSRRFSRNACVVVSCGTAVTVDALTDD  331

orf61.pep GHYLGXGTIMPGFHLMKESLAVRTANLNRHAGKRYPFPT                      189
          ||||| ||||||||||||||||||||||| |||||||||
orf61ng   GHYLG-GTIMPGFHLMKESLAVRTANLNRPAGKRYPFPTTTGNAVASGMMDAVCGSIMMM  390
```

An ORF61ng nucleotide sequence <SEQ ID 237> was predicted to encode a protein having amino acid sequence <SEQ ID 238>:

```
  1 MFSFGWAFDR PQYELGSLSP VAALACRRAL GCLGLETQIK WPNDLVVGRD
 51 KLGGILIETV RAGGKTVAVV GIGINFVLPK EVENAASVQS LFQTASRRGN
101 ADAAVLLETL LAELGAVLEQ YAEEGFAPFL NEYETANRDH GKAVLLLRDG
151 ETVCEGTVKG VDGRGVLHLE TAEGEQTVVS GEISLRPDNR SVSVPKRPDS
201 ERFLLLEGGN SRLKWAWVEN GTFATVGSAP YRDLSPLGAE WAEKADGNVR
251 IVGCAVCGES KKAQVKEQLA RKIEWLPSSA QALGIRNHYR HPEEHGSDRW
301 FNALGSRRFS RNACVVVSCG TAVTVDALTD DGHYLGGTIM PGFHLMKESL
351 AVRTANLNRP AGKRYPFPTT TGNAVASGMM DAVCGSIMMM HGRLKEKNGA
401 GKPVDVIITG GGAAKVAEAL PPAFLAENTV RVADNLVIHG LLNLIAAEGG
451 ESEHA*
```

Further analysis revealed the complete gonococcal DNA sequence <SEQ ID 239> to be:

```
   1 ATGACGGTTT TGAAGCCTTC GCATTGGCGG GTGTTGGCGG AGCTTGCCGA
  51 CGGTTTGCCG CAACACGTAT CGCAATTGGC GCGTGAGGCG GACATGAAGC
 101 CGCAGCAGCT CAACGGTTTT TGGCAGCAGA TGCCGGCGCA TATACGCGGG
 151 CTGTTGCGCC AACACGACGG CTATTGGCGG CTGGTGCGCC CCTTGGCGGT
 201 TTTCGATGCC GAAGGTTTGC GCGATCTGGG GGAAAGGTCG GGTTTTCAGA
 251 CGGCATTGAA GCACGAGTGC GCGTCCAGCA ACGACGAGAT ACTGGAATTG
 301 GCGCGGATTG CGCCGGACAA GGCGCACAAA ACCATATGCG TGACCCACCT
 351 GCAAAGTAAG GGCAGGGGGC GGCAGGGGCG GAAGTGGTCG CACCGTTTGG
 401 GCGAGTGCCT GATGTTCAGT TTCGGCTGGG CGTTTGACCG GCCGCAGTAT
 451 GAGTTGGGTT CGCTGTCGCC TGTTGCGGCA CTTGCGTGCC GGCGCGCTTT
 501 GGGGTGTTTG GGTTTGGAAA CGCAAATCAA GTGGCCAAAC GATTTGGTCG
 551 TCGGACGCGA CAAATTGGGC GGCATTCTGA TTGAAACAGT CAGGGCGGGC
 601 GGTAAAACGG TTGCCGTGGT CGGTATCGGC ATCAATTTCG TGCTGCCCAA
 651 GGAAGTGGAA AACGCCGCTT CCGTGCAGTC GCTGTTTCAG ACGGCATCGC
 701 GGCGGGGCAA TGCCGATGCC GCCGTATTGC TGGAAACATT GCTTGCGGAA
 751 CTGGGCGCGG TGTTGGAACA ATATGCGGAA GAAGGGTTCG CGCCATTTTT
 801 AAATGAGTAT GAAACGGCCA ACCGCGACCA CGGCAAGGCG GTATTGCTGT
 851 TGCGCGACGG CGAAACCGTG TGCGAAGGCA CGGTTAAAGG CGTGGACGGA
 901 CGAGGCGTTC TGCACTTGGA AACGGCAgaa ggcgaACAGa cggtcgtcag
 951 cggcgaaaTC AGcctGCggc ccgacaacaG GTCGGtttcc gtgccgaagc
1001 ggccggatTC GgaacgtTTT tTGCtgttgg aaggcgggaa cagccgGCTC
```

```
1051  AAGTGGGCGT GggtggAAAa cggcacgttc gcaaccgtgg gcagcgcgCc
1101  gtaCCGCGAT TTGTCGCCTT TGGGCGCGGA GTGGGCGGAA AAGGCGGATG
1151  GAAATGTCCG CATCGTCGGT TGCGCCGTGT GCGGAGAATC CAAAAAGGCA
1201  CAAGTGAAGG AACAGCTCGC CCGAAAAATC GAGTGGCTGC CGTCTTCCGC
1251  ACAGGCTTTG GGCATACGCA ACCACTACCG CCACCCCGAA GAACACGGTT
1301  CCGACCGTTG GTTCAACGCC TTGGGCAGCC GCCGCTTCAG CCGCAACGCC
1351  TGCGTCGTCG TCAGTTGCGG CACGGCGGTA ACGGTTGACG CGCTCACCGA
1401  TGACGGACAT TATCTCGGCG GAACCATCAT GCCCGGCTTC CACCTGATGA
1451  AAGAATCGCT CGCCGTCCGA ACCGCCAACC TCAACCGCCC CGCCGGCAAA
1501  CGTTACCCTT TCCCGACCAC AACGGGCAAC GCCGTCGCAA GCGGCATGAT
1551  GGACGCGGTT TGCGGCTCGA TAATGATGAT GCACGGCCGT TTGAAAGAAA
1601  AAAACGGCGC GGGCAAGCCT GTCGATGTCA TCATTACCGG CGGCGGCGCG
1651  GCGAAAGTCG CCGAAGCCCT GCCGCCTGCA TTTTTGGCGG AAAATACCGT
1701  GCGCGTGGCG GACAACCTCG TCATCCACGG GCTGCTGAAC CTGATTGCCG
1751  CCGAAGGCGG GGAATCGGAA CACGCTTAA
```

This corresponds to the amino acid sequence <SEQ ID 240; ORF61ng-1>:

```
  1  MTVLKPSHWR VLAELADGLP QHVSQLAREA DMKPQQLNGF WQQMPAHIRG
 51  LLRQHDGYWR LVRPLAVFDA EGLRDLGERS GFQTALKHEC ASSNDEILEL
101  ARIAPDKAHK TICVTHLQSK GRGRQGRKWS HRLGECLMFS FGWAFDRPQY
151  ELGSLSPVAA LACRRALGCL GLETQIKWPN DLVVGRDKLG GILIETVRAG
201  GKTVAVVGIG INFVLPKEVE NAASVQSLFQ TASRRGNADA AVLLETLLAE
251  LGAVLEQYAE EGFAPFLNEY ETANRDHGKA VLLLRDGETV CEGTVKGVDG
301  RGVLHLETAE GEQTVVSGEI SLRPDNRSVS VPKRPDSERF LLLEGGNSRL
351  KWAWVENGTF ATVGSAPYRD LSPLGAEWAE KADGNVRIVG CAVCGESKKA
401  QVKEQLARKI EWLPSSAQAL GIRNHYRHPE EHGSDRWFNA LGSRRFSRNA
451  CVVVSCGTAV TVDALTDDGH YLGGTIMPGF HLMKESLAVR TANLNRPAGK
501  RYPFPTTTGN AVASGMMDAV CGSIMMMHGR LKEKNGAGKP VDVIITGGGA
551  AKVAEALPPA FLAENTVRVA DNLVIHGLLN LIAAEGGESE HA*
```

ORF61ng-1 and ORF61-1 show 93.9% identity in 591 aa overlap:

```
orf61ng-1.pep  MTVLKPSHWRVLAELADGLPQHVSQLAREADMKPQQLNGFWQQMPAHIRGLLRQHDGYWR  60
               ||||| |||||||||||||||||||||| |||||||||||||||||||||||||||||||
orf61-1        MTVLKLSHWRVLAELADGLPQHVSQLARMADMKPQQLNGFWQQMPAHIRGLLRQHDGYWR  60

orf61ng-1.pep  LVRPLAVFDAEGLRDLGERSGFQTALKHECASSNDEILELARIAPDKAHKTICVTHLQSK 120
               |||||||||||||:||||||||||||||||||||||||||||||||||||||||||||||
orf61-1        LVRPLAVFDAEGLRELGERSGFQTALKHECASSNDEILELARIAPDKAHKTICVTHLQSK 120

orf61ng-1.pep  GRGRQGRKWSHRLGECLMFSFGWAFDRPQYELGSLSPVAALACRRALGCLGLETQIKWPN 180
               |||||||||||||||||||||||:||||||||||||||||:||||||: |||::||||||
orf61-1        GRGRQGRKWSHRLGECLMFSFGWVFDRPQYELGSLSPVAAVACRRALSRLGLDVQIKWPN 180

orf61ng-1.pep  DLVVGRDKLGGILIETVRAGGKTVAVVGIGINFVLPKEVENAASVQSLFQTASRRGNADA 240
               ||||||||||||||||||:|||||||||||||||||||||||||||||||||||||||||
orf61-1        DLVVGRDKLGGILIETVRTGGKTVAVVGIGINFVLPKEVENAASVQSLFQTASRRGNADA 240

orf61ng-1.pep  AVLLETLLAELGAVLEQYAEEGFAPFLNEYETANRDHGKAVLLLRDGETVCEGTVKGVDG 300
               ||||||||:|| ||| |||::|||||: ||::|||||||||||||||||| |||||||||
orf61-1        AVLLETLLVELDAVLLQYARDGFAPFVAEYQAANRDHGKAVLLLRDGETVFEGTVKGVDG 300

orf61ng-1.pep  RGVLHLETAEGEQTVVSGEISLRPDNRSVSVPKRPDSERFLLLEGGNSRLKWAWVENGTF 360
               :||||||||||:||||||||||| |:| |||||| ||||||||:||||||||||||||||
orf61-1        QGVLHLETAEGKQTVVSGEISLRSDDRPVSVPKRRDSERFLLLDGGNSRLKWAWVENGTF 360

orf61ng-1.pep  ATVGSAPYRDLSPLGAEWAEKADGNVRIVGCAVCGESKKAQVKEQLARKIEWLPSSAQAL 420
               |||||||||||||||||||||||||||||||||||| |||||:|||||||||||||||||
orf61-1        ATVGSAPYRDLSPLGAEWAEKADGNVRIVGCAVCGEFKKAQVQEQLARKIEWLPSSAQAL 420

orf61ng-1.pep  GIRNHYRHPEEHGSDRWFNALGSRRFSRNACVVVSCGTAVTVDALTDDGHYLGGTIMPGF 480
               ||||||||||||||||||||||||||||||||||||||||||||||||||||||||||||
orf61-1        GIRNHYRHPEEHGSDRWFNALGSRRFSRNACVVVSCGTAVTVDALTDDGHYLGGTIMPGF 480

orf61ng-1.pep  HLMKESLAVRTANLNRPAGKRYPFPTTTGNAVASGMMDAVCGSIMMMHGRLKEKNGAGKP 540
               |||||||||||||||| ||||||||||||||||||||||||||:||||||||||:|||||
orf61-1        HLMKESLAVRTANLNRHAGKRYPFPTTTGNAVASGMMDAVCGSVMMMHGRLKEKTGAGKP 540

orf61ng-1.pep  VDVIITGGGAAKVAEALPPAFLAENTVRVADNLVIHGLLNLIAAEGGESEHAX  593
               |||||||||||||||||||||||||||||||||||:||||:||||| | ||

orf61-1        VDVIITGGGAAKVAEALPPAFLAENTVRVADNLVIYGLLNMIAAEGREYEHIX  593
```

Based on this analysis, including the homology with the baf protein of *B.pertussis* and the presence of a putative prokaryotic membrane lipoprotein lipid attachment site, it is predicted that these proteins from *N.meningitidis* and *N.gonorrhoeae,* and their epitopes, could be useful antigens for vaccines or diagnostics, or for raising antibodies.

**Example 29**

The following partial DNA sequence was identified in *N.meningitidis* <SEQ ID 241>:

```
  1 ATGTTTTACC AAATCCTTGC CCTGATTATC TGGAGCAGCT CGTTTATTGC
 51 CGCCAAATAT GTCTATGGCG GCATCGATCC CGCATTGATG GTCGGCGTGC
101 GCCTGCTAAT TGCCGCGCTG CCTGCACTGC CCGCCTGCCG CCGTCATGTC
151 GGCAAGATTC CGCGTGAGGA ATGGAAGCCG TTGCTGATTG TGTCGTTCGT
201 CAACTATGTG CTGACCCTGC TGCTTCAGTT TGTCGGGTTG AAATACACTT
251 CCGCCGCCAG CGCATCGGTC ATTGTCGGAC TCGAGCCGCT GCTGATGGTG
301 TTTGTCGGAC ACTTTTTCTT CAACGACAAA GCGCGTGCCT ACCACTGGAT
351 ATGCGGCGCG GCGGCATTTG CCGGTGTCGC GCTGCTGATG GCGGGCGGTG
401 CGGaAGAGGG CGGCGaAGTC GGCTGGTTCG GCTGCCTGCT GGTGTTGTTG
451 GCGGGCGCGG GCTTTTGTGC CGCTATGCGT CCGACGCAAA GGCTGATTGC
501 ACGCATCGGC GCACCGGCAT TCACATCTGT TTCCATTGCC GCCGCATCGT
551 TGATGTGCCT GCCGTTTTCG CTTGCTTTGG CGCAAAGTTA TACCGTGGAC
601 TGGAGCGTCG GGATGGTATT GTCGCTGCTG TATTTGGGTT TGGGGTGC..
```

This corresponds to the amino acid sequence <SEQ ID 242; ORF62>:

```
  1 MFYQILALII WSSSFIAAKY VYGGIDPALM VGVRLLIAAL PALPACRRHV
 51 GKIPREEWKP LLIVSFVNYV LTLLLQFVGL KYTSAASASV IVGLEPLLMV
101 FVGHFFFNDK ARAYHWICGA AAFAGVALLM AGGAEEGGEV GWFGCLLVLL
151 AGAGFCAAMR PTQRLIARIG APAFTSVSIA AASLMCLPFS LALAQSYTVD
201 WSVGMVLSLL YLGLGC..
```

Further work revealed the complete nucleotide sequence <SEQ ID 243>:

```
  1 ATGTTTTACC AAATCCTTGC CCTGATTATC TGGAGCAGCT CGTTTATTGC
 51 CGCCAAATAT GTCTATGGCG GCATCGATCC CGCATTGATG GTCGGCGTGC
101 GCCTGCTAAT TGCCGCGCTG CCTGCACTGC CCGCCTGCCG CCGTCATGTC
151 GGCAAGATTC CGCGTGAGGA ATGGAAGCCG TTGCTGATTG TGTCGTTCGT
201 CAACTATGTG CTGACCCTGC TGCTTCAGTT TGTCGGGTTG AAATACACTT
251 CCGCCGCCAG CGCATCGGTC ATTGTCGGAC TCGAGCCGCT GCTGATGGTG
301 TTTGTCGGAC ACTTTTTCTT CAACGACAAA GCGCGTGCCT ACCACTGGAT
351 ATGCGGCGCG GCGGCATTTG CCGGTGTCGC GCTGCTGATG GCGGGCGGTG
401 CGGAAGAGGG CGGCGAAGTC GGCTGGTTCG GCTGCCTGCT GGTGTTGTTG
451 GCGGGCGCGG GCTTTTGTGC CGCTATGCGT CCGACGCAAA GGCTGATTGC
501 ACGCATCGGC GCACCGGCAT TCACATCTGT TTCCATTGCC GCCGCATCGT
551 TGATGTGCCT GCCGTTTTCG CTTGCTTTGG CGCAAAGTTA TACCGTGGAC
601 TGGAGCGTCG GGATGGTATT GTCGCTGCTG TATTTGGGTT TGGGGTGCGG
651 CTGGTACGCC TATTGGCTGT GGAACAAGGG GATGAGCCGT GTTCCTGCCA
701 ATGTTTCGGG ACTGTTGATT TCGCTCGAAC CCGTCGTCGG CGTGCTGCTG
751 GCGGTTTTGA TTTTGGGCGA ACACCTGTCG CCCGTGTCCG CCTTGGGCGT
801 GTTTGTCGTC ATCGCCGCCA CCTTGGTTGC CGGCCGGCTG TCGCATCAAA
851 AATAA
```

This corresponds to the amino acid sequence <SEQ ID 244; ORF62-1>:

```
  1 MFYQILALII WSSSFIAAKY VYGGIDPALM VGVRLLIAAL PALPACRRHV
 51 GKIPREEWKP LLIVSFVNYV LTLLLQFVGL KYTSAASASV IVGLEPLLMV
101 FVGHFFFNDK ARAYHWICGA AAFAGVALLM AGGAEEGGEV GWFGCLLVLL
151 AGAGFCAAMR PTQRLIARIG APAFTSVSIA AASLMCLPFS LALAQSYTVD
201 WSVGMVLSLL YLGLGCGWYA YWLWNKGMSR VPANVSGLLI SLEPVVGVLL
251 AVLILGEHLS PVSALGVFVV IAATLVAGRL SHQK*
```

Computer analysis of this amino acid sequence gave the following results:

Homology with hypothetical transmembrane protein HI0976 *of H. influenzae* (accession number Q57147)

ORF62 and HI0976 show 50% aa identity in 114aa overlap:

```
Orf62    1   MFYQILALIIWSSSFIAAKYVYGGIDPALMVGVRXXXXXXXXXXXXCRRHVGKIPREEWKP 60
             M YQILAL+IWSSS I   K  Y  +DP L+V VR              R   KI +   K
HI0976   1   MLYQILALLIWSSSLIVGKLTYSMMDPVLVVQVRLIIAMIIVMPLFLRRWKKIDKPMRKQ 60

Orf62    61  LLIVSFVNYVLTLLLQFVGLKYTSAASASVIVGLEPLLMVFVGHFFFNDKARAY 114
             L   ++F NY    LLQF+GLKYTSA+SA   ++GLEPLL+VFVGHFFF  K    +
HI0976   61  LWWLAFFNYTAVFLLQFIGLKYTSASSAVTMIGLEPLLVVFVGHFFFKTKQNGF 114
```

Homology with a predicted ORF from *N.meningitidis* (strain A)

ORF62 shows 99.5% identity over a 216aa overlap with an ORF (ORF62a) from strain A of *N. meningitidis:*

```
                     10        20        30        40        50        60
orf62.pep    MFYQILALIIWSSSFIAAKYVYGGIDPALMVGVRLLIAALPALPACRRHVGKIPREEWKP
             ||||||||||||||||||||||||||||||||||||||||||||||||||||||||||||
orf62a       MFYQILALIIWSSSFIAAKYVYGGIDPALMVGVRLLIAALPALPACRRHVGKIPREEWKP
                     10        20        30        40        50        60

                     70        80        90       100       110       120
orf62.pep    LLIVSFVNYVLTLLLQFVGLKYTSAASASVIVGLEPLLMVFVGHFFFNDKARAYHWICGA
             ||||||||||||||||||||||||||||||||||||||||||||||||||||||||||||
orf62a       LLIVSFVNYVLTLLLQFVGLKYTSAASASVIVGLEPLLMVFVGHFFFNDKARAYHWICGA
                     70        80        90       100       110       120

                    130       140       150       160       170       180
orf62.pep    AAFAGVALLMAGGAEEGGEVGWFGCLLVLLAGAGFCAAMRPTQRLIARIGAPAFTSVSIA
             ||||||||||||||||||||||||||||||||||||||||||||||||||||||||||||
orf62a       AAFAGVALLMAGGAEEGGEVGWFGCLLVLLAGAGFCAAMRPTQRLIARIGAPAFTSVSIA
                    130       140       150       160       170       180

                    190       200       210
orf62.pep    AASLMCLPFSLALAQSYTVDWSVGMVLSLLYLGLGC
             |||||||||||||||||||||||||||||||||:||
orf62a       AASLMCLPFSLALAQSYTVDWSVGMVLSLLYLGVGCSWYAYWLWNKGMSRVPANVSGLLI
                    190       200       210       220       230       240

orf62a       SLEPVVGVLLAVLILGEHLSPVSVLGVFVVIAATLVAGRLSHQKX
                    250       260       270       280
```

The complete length ORF62a nucleotide sequence <SEQ ID 245> is:

```
  1  ATGTTTTACC AAATCCTTGC CCTGATTATC TGGAGCAGCT CGTTTATTGC
 51  CGCCAAATAT GTCTATGGCG GCATCGATCC CGCATTGATG GTCGGCGTGC
101  GCCTGCTGAT TGCTGCGCTG CCTGCACTGC CCGCCTGCCG CCGTCATGTC
151  GGCAAGATTC CGCGTGAGGA ATGGAAGCCG TTGCTGATTG TGTCGTTCGT
201  CAACTATGTG CTGACCCTGC TACTTCAGTT TGTCGGGTTG AAATACACTT
251  CCGCCGCCAG CGCATCGGTC ATTGTCGGAC TCGAGCCACT GCTGATGGTG
301  TTTGTCGGAC ACTTTTTCTT CAACGACAAA GCGCGTGCCT ACCACTGGAT
351  ATGCGGCGCG GCGGCATTTG CCGGTGTCGC GCTGCTGATG GCGGGCGGTG
401  CGGAAGAGGG CGGCGAAGTC GGCTGGTTCG GCTGCCTGCT GGTGTTGTTG
451  GCGGGCGCGG GCTTTTGTGC CGCTATGCGT CCGACGCAAA GGCTGATTGC
501  ACGCATCGGC GCACCGGCAT TCACATCTGT TTCCATTGCC GCCGCATCGT
551  TGATGTGCCT GCCGTTTTCG CTTGCTTTGG CGCAAAGTTA TACCGTGGAC
601  TGGAGCGTCG GAATGGTATT GTCGCTGCTG TATTTGGGCG TGGGGTGCAG
651  CTGGTACGCC TATTGGCTGT GGAACAAGGG GATGAGCCGT GTTCCTGCCA
701  ACGTTTCGGG ACTGTTGATT TCGCTCGAAC CCGTCGTCGG CGTGCTGCTG
751  GCGGTTTTGA TTTTGGGCGA ACACCTGTCG CCCGTGTCCG TCTTGGGCGT
801  GTTTGTCGTC ATCGCCGCCA CCTTGGTTGC CGGCCGGCTG TCGCATCAAA
851  AATAA
```

This encodes a protein having amino acid sequence <SEQ ID 246>:

```
  1  MFYQILALII WSSSFIAAKY VYGGIDPALM VGVRLLIAAL PALPACRRHV
 51  GKIPREEWKP LLIVSFVNYV LTLLLQFVGL KYTSAASASV IVGLEPLLMV
101  FVGHFFFNDK ARAYHWICGA AAFAGVALLM AGGAEEGGEV GWFGCLLVLL
151  AGAGFCAAMR PTQRLIARIG APAFTSVSIA AASLMCLPFS LALAQSYTVD
201  WSVGMVLSLL YLGVGCSWYA YWLWNKGMSR VPANVSGLLI SLEPVVGVLL
251  AVLILGEHLS PVSVLGVFVV IAATLVAGRL SHQK*
```

ORF62a and ORF62-1 show 98.9% identity in 284 aa overlap:

```
orf62a.pep   MFYQILALIIWSSSFIAAKYVYGGIDPALMVGVRLLIAALPALPACRRHVGKIPREEWKP  60
             ||||||||||||||||||||||||||||||||||||||||||||||||||||||||||||
orf62-1      MFYQILALIIWSSSFIAAKYVYGGIDPALMVGVRLLIAALPALPACRRHVGKIPREEWKP  60

orf62a.pep   LLIVSFVNYVLTLLLQFVGLKYTSAASASVIVGLEPLLMVFVGHFFFNDKARAYHWICGA 120
             ||||||||||||||||||||||||||||||||||||||||||||||||||||||||||||
orf62-1      LLIVSFVNYVLTLLLQFVGLKYTSAASASVIVGLEPLLMVFVGHFFFNDKARAYHWICGA 120

orf62a.pep   AAFAGVALLMAGGAEEGGEVGWFGCLLVLLAGAGFCAAMRPTQRLIARIGAPAFTSVSIA 180
             ||||||||||||||||||||||||||||||||||||||||||||||||||||||||||||
orf62-1      AAFAGVALLMAGGAEEGGEVGWFGCLLVLLAGAGFCAAMRPTQRLIARIGAPAFTSVSIA 180

orf62a.pep   AASLMCLPFSLALAQSYTVDWSVGMVLSLLYLGVGCSWYAYWLWNKGMSRVPANVSGLLI 240
             ||||||||||||||||||||||||||||||||:||:||||||||||||||||||||||||
orf62-1      AASLMCLPFSLALAQSYTVDWSVGMVLSLLYLGLGCGWYAYWLWNKGMSRVPANVSGLLI 240

orf62a.pep   SLEPVVGVLLAVLILGEHLSPVSVLGVFVVIAATLVAGRLSHQKX  285
             ||||||||||||||||||||||||:||||||||||||||||||||
orf62-1      SLEPVVGVLLAVLILGEHLSPVSALGVFVVIAATLVAGRLSHQKX  285
```

Homology with a predicted ORF from *N.gonorrhoeae*

ORF62 shows 99.5% identity over a 216aa overlap with a predicted ORF (ORF62.ng) from *N. gonorrhoeae:*

```
orf62.pep    MFYQILALIIWSSSFIAAKYVYGGIDPALMVGVRLLIAALPALPACRRHVGKIPREEWKP   60
             |||||||||||:||||||||||||||||||||||||||||||||||||||||||||||||
orf62ng      MFYQILALIIWGSSFIAAKYVYGGIDPALMVGVRLLIAALPALPACRRHVGKIPREEWKP   60

orf62.pep    LLIVSFVNYVLTLLLQFVGLKYTSAASASVIVGLEPLLMVFVGHFFFNDKARAYHWICGA  120
             ||||||||||||||||||||||||||||||||||||||||||||||||||||||||||||
orf62ng      LLIVSFVNYVLTLLLQFVGLKYTSAASASVIVGLEPLLMVFVGHFFFNDKARAYHWICGA  120

orf62.pep    AAFAGVALLMAGGAEEGGEVGWFGCLLVLLAGAGFCAAMRPTQRLIARIGAPAFTSVSIA  180
             ||||||||||||||||||||||||||||||||||||||||||||||||||||||||||||
orf62ng      AAFAGVALLMAGGAEEGGEVGWFGCLLVLLAGAGFCAAMRPTQRLIARIGAPAFTSVSIA  180

orf62.pep    AASLMCLPFSLALAQSYTVDWSVGMVLSLLYLGLGC                         216
             ||||||||||||||||||||||||||||||||||||
orf62ng      AASLMCLPFSLALAQSYTVDWSVGMVLSLLYLGLGCGWYAYWLWNKGMSRVPANASGLLI  240
```

The complete length ORF62ng nucleotide sequence <SEQ ID 247> is:

```
  1 ATGTTTTACC AAATCCTTGC CCTGATTATC TGGGGCAGCT CGTTTATTGC
 51 CGCCAAATAT GTCTATGGCG GCATCGATCC CGCATTGATG GTCGGCGTGC
101 GCCTGCTGAT TGCCGCGCTG CCTGCACTGC CCGCCTGCCG CCGTCATGTC
151 GGCAAGATTC CGCGTGAGGA ATGGAAGCCG TTGCTGATTG TGTCGTTCGT
201 CAACTATGTG CTGACCCTGC TGCTTCAGTT TGTCGGGTTG AAATACACTT
251 CCGCCGCCAG CGCATCGGTC ATTGTCGGAC TCGAGCCGCT GCTGATGGTG
301 TTTGTCGGAC ACTTTTTCTT CAACGACAAA GCGCGTGCCT ACCACTGGAT
351 ATGCGGCGCG GCGGCATTTG CCGGTGTCGC GCTGCTGATG GCGGGCGGTG
401 CGGAAGAGGG CGGCGAAGTC GGCTGGTTCG GCTGCCTGCT GGTGTTGTTG
451 GCGGGCGCGG GCTTTTGTGC CGCTATGCGT CCGACGCAAA GGCTGATTGC
501 CCGCATCGGC GCACCGGCAT TCACATCTGT TTCCATTGCC GCCGCATCGT
551 TGATGTGCCT GCCGTTTTCG CTTGCTTTGG CGCAAAGTTA TACCGTGGAC
601 TGGAGCGTCG GGATGGTATT GTCGCTGTTG TATTTGGGTT TGGGGTGCGG
651 CTGGTACGCC TATTGGCTGT GGAACAAGGG GATGAGCCGT GTTCCTGCCA
701 ACGCGTCGGG ACTGTTGATT TCGCTCGAAC CCGTCGTCGG CGTGCTGTTG
751 GCGGTTTTGA TTTTGGGCGA ACATTTATCG CCCGTGTCCG CCTTGGGCGT
801 GTTTGTCGTC ATCGCCGCCA CTTTCGCCGC CGGCCGGCTG TCGCGCAGGG
851 ACGCGCAAAA CGGCAATGCC GTCTGA
```

This encodes a protein having amino acid sequence <SEQ ID 248>:

```
  1 MFYQILALII WGSSFIAAKY VYGGIDPALM VGVRLLIAAL PALPACRRHV
 51 GKIPREEWKP LLIVSFVNYV LTLLLQFVGL KYTSAASASV IVGLEPLLMV
101 FVGHFFFNDK ARAYHWICGA AAFAGVALLM AGGAEEGGEV GWFGCLLVLL
151 AGAGFCAAMR PTQRLIARIG APAFTSVSIA AASLMCLPFS LALAQSYTVD
201 WSVGMVLSLL YLGLGCGWYA YWLWNKGMSR VPANASGLLI SLEPVVGVLL
251 AVLILGEHLS PVSALGVFVV IAATFAAGRL SRRDAQNGNA V*
```

ORF62ng and ORF62-1 show 97.9% identity in 283 aa overlap:

```
                        10        20        30        40        50        60
orf62ng.pep     MFYQILALIIWGSSFIAAKYVYGGIDPALMVGVRLLIAALPALPACRRHVGKIPREEWKP
                ||||||||||||:|||||||||||||||||||||||||||||||||||||||||||||||
orf62-1         MFYQILALIIWSSSFIAAKYVYGGIDPALMVGVRLLIAALPALPACRRHVGKIPREEWKP
                        10        20        30        40        50        60

                        70        80        90       100       110       120
orf62ng.pep     LLIVSFVNYVLTLLLQFVGLKYTSAASASVIVGLEPLLMVFVGHFFFNDKARAYHWICGA
                ||||||||||||||||||||||||||||||||||||||||||||||||||||||||||||
orf62-1         LLIVSFVNYVLTLLLQFVGLKYTSAASASVIVGLEPLLMVFVGHFFFNDKARAYHWICGA
                        70        80        90       100       110       120

                       130       140       150       160       170       180
orf62ng.pep     AAFAGVALLMAGGAEEGGEVGWFGCLLVLLAGAGFCAAMRPTQRLIARIGAPAFTSVSIA
                ||||||||||||||||||||||||||||||||||||||||||||||||||||||||||||
orf62-1         AAFAGVALLMAGGAEEGGEVGWFGCLLVLLAGAGFCAAMRPTQRLIARIGAPAFTSVSIA
                       130       140       150       160       170       180

                       190       200       210       220       230       240
orf62ng.pep     AASLMCLPFSLALAQSYTVDWSVGMVLSLLYLGLGCGWYAYWLWNKGMSRVPANASGLLI
                |||||||||||||||||||||||||||||||||||||||||||||||||||||||:||||
orf62-1         AASLMCLPFSLALAQSYTVDWSVGMVLSLLYLGLGCGWYAYWLWNKGMSRVPANVSGLLI
                       190       200       210       220       230       240

                       250       260       270       280       290
orf62ng.pep     SLEPVVGVLLAVLILGEHLSPVSALGVFVVIAATFAAGRLSRRDAQNGNAVX
                |||||||||||||||||||||||||||||||||::|||||::
orf62-1         SLEPVVGVLLAVLILGEHLSPVSALGVFVVIAATLVAGRLSHQKX
                       250       260       270       280
```

Furthermore, ORF62ng shows significant homology to a hypothetical *H.influenzae* protein:

```
sp|Q57147|Y976_HAEIN HYPOTHETICAL PROTEIN HI0976 >gi|1074589|pir||B64163
hypothetical protein HI0976 - Haemophilus influenzae (strain Rd KW20)
>gi|1574004 (U32778) hypothetical [Haemophilus influenzae] Length = 128
 Score =  106 bits (262), Expect = 2e-22
 Identities = 56/114 (49%), Positives = 68/114 (59%)

Query: 1    MFYQILALIIWGSSFIAAKYVYGGIDPALMVGVRXXXXXXXXXXXCRRHVGKIPREEWKP 60
             M YQILAL+IW SS I  K  Y  +DP L+V VR           R   KI +   K
Sbjct: 1    MLYQILALLIWSSSLIVGKLTYSMMDPVLVVQVRLIIAMIIVMPLFLRRWKKIDKPMRKQ 60

Query: 61   LLIVSFVNYVLTLLLQFVGLKYTSAASASVIVGLEPLLMVFVGHFFFNDKARAY 114
             L  ++F NY    LLQF+GLKYTSA+SA  ++GLEPLL+VFVGHFFF  K   +
Sbjct: 61   LWWLAFFNYTAVFLLQFIGLKYTSASSAVTMIGLEPLLVVFVGHFFFKTKQNGF 114
```

Based on this analysis, including the homology with the transmembrane protein of *H. influenzae* and the putative leader sequecne and several transmembrane domains in the gonococcal protein, it is predicted that these proteins from *N.meningitidis* and *N.gonorrhoeae,* and their epitopes, could be useful antigens for vaccines or diagnostics, or for raising antibodies.

**Example 30**

The following partial DNA sequence was identified in *N.meningitidis* <SEQ ID 249>:

```
   1  ATGCGCCGTT TTCTACCGAT CGCAGCCATA TGCGCmGwms TCCTGkkGTA
  51  sGGACTGACG GCGGCAACCG GCAGCACCAG TTCGCTGGCG GATTATTTCT
 101  GGTGGATTGT TGCGTTCAGC GCAATGCTGC TGCTGGTGTT GTCCGCCGTT
 151  TTGGCACGTT ATGTCATATT GCTGTTGAAA GACAGGCGCG ACGGCGTATT
 201  CGGTTCGCtA srTyGCCAAA gsGCCTgkks TGGG.ATGTT TACGCTGGTT
 251  GCCGkACTGC CCGGCGTGTT TCTGTTCGGC TTTCCCGCAC AGTTCATCAA
 301  CGGCACGATT AATTCGTGGT TCGGCAACGA TACCCACGAG GCGCTTGAAC
 351  GCAGCCTCAA TTTGAGCAAG TCCGCATTGA ATTTGGCGGC AGACAACGCC
 401  CTCGGCAACG CCGTCCCCGT GCAGATAGAC CTCATCGGCG CGGCTTCCCT
 451  GCCCGGGGAT ATGGGCAGGG TGCTGGAACA TTACGCCGGC AGCGGTTTTG
 501  CCCAGCTTGC CCTGTACAAy ksCGCAAGCG GCAAAATCGA AAAAAGCATC
 551  AACCCGCACA AGCTCGATCA GCCGTTTCCA GGTAAGGCGC GTTGGGAaAa
 601  AATCCaACGG GCGGGTTCGG TCAGGGATTT GGAAAGCATA GGCGGCGTAT
 651  TGTaCGCGCA GGGCTGGCTG TCGGCGGGTA CGCACwACGG GCGCGATTAC
 701  GCCTTGTTTT TCCGTCAGCC GGTTCCCAAA GGCGTGGCAG AGGATGCCGT
 751  yTTAATCGAA AAGGCAAGGG CGAAATATGC TGAGTTGAGT TACAGCAAAA
 801  AAGGTTTGCA GACCTTTTTC CTGGCAACCC TGCTGATTGC CTCGCTGCTG
 851  TCGATTTTTC TTGCACTGGT CATGGCACTG TATTTCGCCC GCCGTTTCGT
 901  CGAACCCGTC CTATCGCTTG CCGAGGGGGC GAAGGCGGTG GCGCAAGGCG
 951  ATTTCAGCCA GACGCGCCCC GTGTTGCGCA ACGACGAGTT CGGACGCTTG
1001  ACCArGTTGT TCAACCACAT GACCGAGCAG CTTTCCATCG CCAAAGATGC
1051  AGACGAGCGC AACCGCCGGC GCGAGGAAGC CGCCAGGCAT TATCTTGAAT
1101  GCGTGTTGGA GGGGCTGACC ACGGGCGTGG TGGTGTTTGA CGAACAAGGC
1151  TGTCTGAAAA CCTTCAACAA AGCGGCGGGT ACC..
```

This corresponds to the amino acid sequence <SEQ ID 250; ORF64>:

```
  1  MRRFLPIAAI CAXXLXXGLT AATGSTSSLA DYFWWIVAFS AMLLLVLSAV
 51  LARYVILLLK DRRDGVFGSX XAKXPXXXMF TLVAXLPGVF LFGFPAQFIN
101  GTINSWFGND THEALERSLN LSKSALNLAA DNALGNAVPV QIDLIGAASL
151  PGDMGRVLEH YAGSGFAQLA LYNXASGKIE KSINPHKLDQ PFPGKARWEK
201  IQRAGSVRDL ESIGGVLYAQ GWLSAGTHXG RDYALFFRQP VPKGVAEDAV
251  LIEKARAKYA ELSYSKKGLQ TFFLATLLIA SLLSIFLALV MALYFARRFV
301  EPVLSLAEGA KAVAQGDFSQ TRPVLRNDEF GRLTXLFNHM TEQLSIAKDA
351  DERNRRREEA ARHYLECVLE GLTTGVVVFD EQGCLKTFNK AAGT..
```

Further work revealed the complete nucleotide sequence <SEQ ID 251>:

```
   1 ATGCGCCGTT TTCTACCGAT CGCAGCCATA TGCGCCGTCG TCCTGTTGTA
  51 CGGACTGACG GCGGCAACCG GCAGCACCAG TTCGCTGGCG GATTATTTCT
 101 GGTGGATTGT TGCGTTCAGC GCAATGCTGC TGCTGGTGTT GTCCGCCGTT
 151 TTGGCACGTT ATGTCATATT GCTGTTGAAA GACAGGCGCG ACGGCGTATT
 201 CGGTTCGCAG ATTGCCAAAC GCCTTTCTGG GATGTTTACG CTGGTTGCCG
 251 TACTGCCCGG CGTGTTTCTG TTCGGCGTTT CCGCACAGTT CATCAACGGC
 301 ACGATTAATT CGTGGTTCGG CAACGATACC CACGAGGCGC TTGAACGCAG
 351 CCTCAATTTG AGCAAGTCCG CATTGAATTT GGCGGCAGAC AACGCCCTCG
 401 GCAACGCCGT CCCCGTGCAG ATAGACCTCA TCGGCGCGGC TTCCCTGCCC
 451 GGGGATATGG GCAGGGTGCT GGAACATTAC GCCGGCAGCG GTTTTGCCCA
 501 GCTTGCCCTG TACAATGCCG CAAGCGGCAA AATCGAAAAA AGCATCAACC
 551 CGCACAAGCT CGATCAGCCG TTTCCAGGTA AGGCGCGTTG GGAAAAAATC
 601 CAACGGGCGG GTTCGGTCAG GGATTTGGAA AGCATAGGCG GCGTATTGTA
 651 CGCGCAGGGC TGGCTGTCGG CGGGTACGCA CAACGGGCGC GATTACGCCT
 701 TGTTTTTCCG TCAGCCGGTT CCCAAAGGCG TGGCAGAGGA TGCCGTCTTA
 751 ATCGAAAAGG CAAGGGCGAA ATATGCTGAG TTGAGTTACA GCAAAAAAGG
 801 TTTGCAGACC TTTTTCCTGG CAACCCTGCT GATTGCCTCG CTGCTGTCGA
 851 TTTTTCTTGC ACTGGTCATG GCACTGTATT TCGCCCGCCG TTTCGTCGAA
 901 CCCGTCCTAT CGCTTGCCGA GGGGGCGAAG GCGGTGGCGC AAGGCGATTT
 951 CAGCCAGACG CGCCCCGTGT TGCGCAACGA CGAGTTCGGA CGCTTGACCA
1001 AGTTGTTCAA CCACATGACC GAGCAGCTTT CCATCGCCAA AGAAGCAGAC
1051 GAGCGCAACC GCCGGCGCGA GGAAGCCGCC AGGCATTATC TTGAATGCGT
1101 GTTGGAGGGG CTGACCACGG GCGTGGTGGT GTTTGACGAA CAAGGCTGTC
1151 TGAAAACCTT CAACAAAGCG GCGGAACAGA TTTTGGGGAT GCCGCTTACC
1201 CCCCTGTGGG GCAGCAGCCG GCACGGTTGG CACGGCGTTT CGGCGCAGCA
1251 GTCCCTGCTT GCCGAAGTGT TTGCCGCCAT CGGCGCGGCG GCAGGTACGG
1301 ACAAACCGGT CCATGTGAAA TATGCCGCGC CGGACGATGC CAAAATCCTG
1351 CTGGGCAAGG CAACCGTCCT GCCCGAAGAC AACGGCAACG GCGTGGTAAT

1401 GGTGATTGAC GACATCACCG TTTTGATACA CGCGCAAAAA GAAGCCGCGT
1451 GGGGCGAAGT GGCGAAGCGG CTGGCACACG AAATCCGCAA TCCGCTCACG
1501 CCCATCCAGC TTTCCGCCGA ACGGCTGGCG TGGAAATTGG GCGGGAAGCT
1551 GGATGAGCAG GATGCGCAAA TCCTGACGCG TTCGACCGAC ACCATCGTCA
1601 AACAGGTGGC GGCATTGAAG GAAATGGTCG AAGCATTCCG CAATTATGCG
1651 CGTTCCCCTT CGCTCAAATT GGAAAATCAG GATTTGAACG CCTTAATCGG
1701 CGATGTGTTG GCATTGTATG AAGCCGGTCC GTGCCGGTTT GCGGCGGAGC
1751 TTGCCGGCGA ACCGCTGACG GTGGCGGCGG ATACGACCGC CATGCGGCAG
1801 GTGCTGCACA ATATTTTCAA AAATGCCGCC GAAGCGGCGG AAGAAGCCGA
1851 TGTGCCCGAA GTCAGGGTAA AATCGGAAAC AGGGCAGGAC GGTCGGATTG
1901 TCCTGACGGT TTGCGACAAC GGCAAAGGGT TCGGCAGGGA AATGCTGCAC
1951 AACGCCTTCG AGCCGTATGT AACGGACAAA CCGGCGGGAA CGGGATTGGG
2001 TCTGCCTGTG GTGAAAAAAA TCATTGAAGA ACACGGCGGC CGCATCAGCC
2051 TGAGCAATCA GGATGCGGGT GGCGCGTGTG TCAGAATCAT CTTGCCAAAA
2101 ACGGTAAAAA CTTATGCGTA G
```

This corresponds to the amino acid sequence <SEQ ID 252; ORF64-1>:

```
  1  MRRFLPIAAI CAVVLLYGLT AATGSTSSLA DYFWWIVAFS AMLLLVLSAV
 51  LARYVILLLK DRRDGVFGSQ IAKRLSGMFT LVAVLPGVFL FGVSAQFING
101  TINSWFGNDT HEALERSLNL SKSALNLAAD NALGNAVPVQ IDLIGAASLP
151  GDMGRVLEHY AGSGFAQLAL YNAASGKIEK SINPHKLDQP FPGKARWEKI
201  QRAGSVRDLE SIGGVLYAQG WLSAGTHNGR DYALFFRQPV PKGVAEDAVL
251  IEKARAKYAE LSYSKKGLQT FFLATLLIAS LLSIFLALVM ALYFARRFVE
301  PVLSLAEGAK AVAQGDFSQT RPVLRNDEFG RLTKLFNHMT EQLSIAKEAD
351  ERNRRREEAA RHYLECVLEG LTTGVVVFDE QGCLKTFNKA AEQILGMPLT
401  PLWGSSRHGW HGVSAQQSLL AEVFAAIGAA AGTDKPVHVK YAAPDDAKIL
451  LGKATVLPED NGNGVVMVID DITVLIHAQK EAAWGEVAKR LAHEIRNPLT
501  PIQLSAERLA WKLGGKLDEQ DAQILTRSTD TIVKQVAALK EMVEAFRNYA
551  RSPSLKLENQ DLNALIGDVL ALYEAGPCRF AAELAGEPLT VAADTTAMRQ
601  VLHNIFKNAA EAAEEADVPE VRVKSETGQD GRIVLTVCDN GKGFGREMLH
651  NAFEPYVTDK PAGTGLGLPV VKKIIEEHGG RISLSNQDAG GACVRIILPK
701  TVKTYA*
```

Computer analysis of this amino acid sequence gave the following results:

Homology with a predicted ORF from *N. meningitidis* (strain A)

ORF64 shows 92.6% identity over a 392aa overlap with an ORF (ORF64a) from strain A of *N. meningitidis:*

```
                        10        20        30        40        50        60
orf64.pep       MRRFLPIAAICAXXLXXGLTAATGSTSSLADYFWWIVAFSAMLLLVLSAVLARYVILLLK
                ||||||||||||  |  |||||||||||||||||||||||||||||||||||||||||||
orf64a          MRRFLPIAAICAVVLLYGLTAATGSTSSLADYFWWIVAFSAMLLLVLSAVLARYVILLLK
                        10        20        30        40        50        60

                        70        80        90       100       110       120
orf64.pep       DRRDGVFGSXXAKXPXXXMFTLVAXLPGVFLFGFPAQFINGTINSWFGNDTHEALERSLN
                |||||||||  ||     |||||| ||||||||  |||||||||||||||||||||||||
orf64a          DRRDGVFGSQIAKR-LSGMFTLVAVLPGVFLFGVSAQFINGTINSWFGNDTHEALERSLN
                        70        80        90       100       110

                       130       140       150       160       170       180
orf64.pep       LSKSALNLAADNALGNAVPVQIDLIGAASLPGDMGRVLEHYAGSGFAQLALYNXASGKIE
                |||||||||||||||||:||||| ||||||| ||||||||||||||||||||| ||||||
orf64a          LSKSALNLAADNALGNAIPVQIDXIGAASLPXDMGRVLEHYAGSGFAQLALYNAASGKIE
             120       130       140       150       160       170

                       190       200       210       220       230       240
orf64.pep       KSINPHKLDQPFPGKARWEKIQRAGSVRDLESIGGVLYAQGWLSAGTHXGRDYALFFRQP
                ||||||||||||||||||||||:|||||| ||||||||| ||||| || |||||||||||
orf64a          KSINPHKLDQPFPGKARWEKIQQAGSVRDXESIGGVLYAXGWLSAXTHNGRDYALFFRQP
             180       190       200       210       220       230

                       250       260       270       280       290       300
orf64.pep       VPKGVAEDAVLIEKARAKYAELSYSKKGLQTFFLATLLIASLLSIFLALVMALYFARRFV
                |||||||||||||||||    |||||||||||||||||||||||||||||||||||||||
orf64a          VPKGVAEDAVLIEKARAXXXXLSYSKKGLQTFFLATLLIASLLSIFLALVMALYFARRFV
             240       250       260       270       280       290
```

```
                       310       320       330       340       350       360
orf64.pep    EPVLSLAEGAKAVAQGDFSQTRPVLRNDEFGRLTXLFNHMTEQLSIAKDADERNRRREEA
             |||||||||||||||||||||||||||||||||| |||||||||||||:|||||||||||
orf64a       EPVLSLAEGAKAVAQGDFSQTRPVLRNDEFGRLTKLFNHMTEQLSIAKEADERNRRREEA
           300       310       320       330       340       350

                       370       380       390
orf64.pep    ARHYLECVLEGLTTGVVVFDEQGCLKTFNKAAGT
             ||||||||||||||||||||||||||||||||
orf64a       ARHYLECVLEGLTTGVVVFDEQGCLKTFNKAAEQILGMPLTPLWGSSRHGWHGVSAQQSL
           360       370       380       390       400       410

orf64a       LAEVFAAIGAAAGTDKPVHVKYAAPDDAKILLGKATVLPEDNXNGVVMVIDDITVLIHAQ
           420       430       440       450       460       470
```

The complete length ORF64a nucleotide sequence <SEQ ID 253> is:

```
   1  ATGCGCCGTT TTCTACCGAT CGCAGCCATA TGCGCCGTCG TCCTGTTGTA
  51  CGGACTGACG GCGGCAACCG GCAGCACCAG TTCGCTGGCG GATTATTTCT
 101  GGTGGATTGT TGCGTTCAGC GCAATGCTGC TGCTGGTGTT GTCCGCCGTT
 151  TTGGCACGTT ATGTCATATT GCTGTTGAAA GACAGGCGCG ACGGCGTATT
 201  CGGTTCGCAG ATTGCCAAAC GCCTTTCCGG GATGTTTACG CTGGTTGCCG
 251  TACTGCCCGG CGTGTTTCTG TTCGGCGTTT CCGCACAGTT TATCAACGGC
 301  ACGATTAATT CGTGGTTCGG CAACGATACC CACGAGGCGC TTGAACGCAG
 351  CCTCAATTTG AGCAAGTCCG CATTGAATCT GGCGGCAGAC AACGCCCTTG
 401  GCAACGCCAT CCCCGTGCAG ATAGACNTCA TCGGCGCGGC TTCCCTGCCC
 451  NGGGATATGG GCAGGGTGCT GGAACATTAC GCCGGCAGCG GTTTTGCCCA
 501  GCTTGCCCTG TACAATGCCG CAAGCGGCAA AATCGAAAAA AGCATCAACC
 551  CGCACAAGCT CGATCAGCCG TTTCCAGGTA AGGCGCGTTG GGAAAAAATC
 601  CAACAGGCGG GTTCGGTCAG GGATNNGGAA AGCATAGGCG GCGTATTGTA
 651  CGCGCANGGC TGGCTGTCGG CAGNNACGCA CAACGGGCGC GATTACGCCT
 701  TGTTTTTCCG TCAGCCGGTT CCCAAAGGCG TGGCAGAGGA TGCCGTCTTA
 751  ATCGAAAAGG CAAGGGCGNA ANANNNTNAG TTGAGTTACA GCAAAAAAGG
 801  TTTGCAGACC TTTTTCCTNG CAACCCTGCT GATTGCCTCN CTGCTGTCGA
 851  TTTTTCTTGC ACTGGTCATG GCACTGTATT TCGCCCGCCG TTTCGTCGAA
 901  CCCGTCCTAT CGCTTGCCGA GGGGGCGAAG GCGGTGGCGC AAGGCGATTT
 951  CAGCCAGACG CGCCCCGTGT TGCGCAACGA CGAGTTCGGA CGCTTGACCA
1001  AGTTGTTCAA CCACATGACC GAGCAGCTTT CCATCGCCAA AGAAGCAGAC
1051  GAGCGCAACC GCCGGCGCGA GGAAGCCGCC AGACATTATC TCGAATGCGT
1101  GTTGGAGGGG CTGACCACGG GCGTGGTGGT GTTTGACGAA CAAGGCTGTC
1151  TGAAAACCTT CAACAAAGCG GCGGAACAGA TTTTGGGGAT GCCGCTTACC
1201  CCCCTGTGGG GCAGCAGCCG GCACGGTTGG CACGGCGTTT CGGCGCAGCA
1251  GTCCCTGCTT GCCGAAGTGT TTGCCGCCAT CGGCGCGGCG GCAGGTACGG
1301  ACAAACCGGT CCATGTGAAA TATGCCGCGC CGGACGATGC CAAAATCCTG
1351  CTGGGCAAGG CAACCGTCCT GCCCGAAGAC AACNGCAACG GCGTGGTAAT
1401  GGTGATTGAC GACATCACCG TTTTGATACA CGCGCAAAAA GAAGCCGCGT
1451  GGGGCGAAGT GGCAAAACGG CTGGCACACG AAATCCGCAA TCCGCTCACG
1501  CCCATCCAGC TTTCTGCCGA ACGGCTGGCG TGGAAATTGG GCGGGAAGCT
1551  GGACGAGCAN GACGCGCAAA TCCTGACACG TTCGACCGAC ACCATCATCA
1601  AACAAGTGGC GGCATTAAAA GAAATGGTCG AGGCATTCCG CAATTACNCG
1651  CGTTCCCCTT CGNCTCAATT GGAAAATCAG GATTTGAACG CCTTAATCGG
1701  CGATGTGTTG GCATTGTACG AAGCTGGTCC GTGCCGGTTT GCGGCGGAAC
1751  TTGCCGGCGA ACCGCTGATG ATGGCGGCGG ATACGACCGC CATGCGGCAG
1801  GTGCTGCACA ATATTTTCAA AAATGCCGCC GAAGCGGCGG AAGAAGCCGA
1851  TGTGCCCGAA GTCAGGGTAA AATCGGAAGC GGGGCAGGAC GGACGGATTG
1901  TCCTGACAGT TTGCGACAAC GGCAAGGGGT TCGGCAGGGA AATGCTGCAC
1951  AATGCCTTCG AGCCGTATGT AACGGACAAA CCGGCTGGAA CGGGATTGNG
2001  ACTGCCCGTG GTGAAAAAAA TCATTGAAGA ACACGGCGGC CNCATCAGCC
2051  TGAGCAATCA GGATGCGGGC GGCGCGTNTG TCAGAATCAT CTTGCCAAAA
2101  ACGGTAGAAA CTTATGCGTA G
```

This encodes a protein having amino acid sequence <SEQ ID 254>:

```
  1  MRRFLPIAAI CAVVLLYGLT AATGSTSSLA DYFWWIVAFS AMLLLVLSAV
 51  LARYVILLLK DRRDGVFGSQ IAKRLSGMFT LVAVLPGVFL FGVSAQFING
101  TINSWFGNDT HEALERSLNL SKSALNLAAD NALGNAIPVQ IDXIGAASLP
151  XDMGRVLEHY AGSGFAQLAL YNAASGKIEK SINPHKLDQP FPGKARWEKI
201  QQAGSVRDXE SIGGVLYAXG WLSAXTHNGR DYALFFRQPV PKGVAEDAVL
251  IEKARAXXXX LSYSKKGLQT FFLATLLIAS LLSIFLALVM ALYFARRFVE
301  PVLSLAEGAK AVAQGDFSQT RPVLRNDEFG RLTKLFNHMT EQLSIAKEAD
351  ERNRRREEAA RHYLECVLEG LTTGVVVFDE QGCLKTFNKA AEQILGMPLT
```

```
401  PLWGSSRHGW HGVSAQQSLL AEVFAAIGAA AGTDKPVHVK YAAPDDAKIL
451  LGKATVLPED NXNGVVMVID DITVLIHAQK EAAWGEVAKR LAHEIRNPLT
501  PIQLSAERLA WKLGGKLDEX DAQILTRSTD TIIKQVAALK EMVEAFRNYX
551  RSPSXQLENQ DLNALIGDVL ALYEAGPCRF AAELAGEPLM MAADTTAMRQ
601  VLHNIFKNAA EAAEEADVPE VRVKSEAGQD GRIVLTVCDN GKGFGREMLH
651  NAFEPYVTDK PAGTGLXLPV VKKIIEEHGG XISLSNQDAG GAXVRIILPK
701  TVETYA*
```

ORF64a and ORF64-1 show 96.6% identity in 706 aa overlap:

```
                       10        20        30        40        50        60
orf64a.pep  MRRFLPIAAICAVVLLYGLTAATGSTSSLADYFWWIVAFSAMLLLVLSAVLARYVILLLK
            ||||||||||||||||||||||||||||||||||||||||||||||||||||||||||||
orf64-1     MRRFLPIAAICAVVLLYGLTAATGSTSSLADYFWWIVAFSAMLLLVLSAVLARYVILLLK
                       10        20        30        40        50        60

                       70        80        90       100       110       120
orf64a.pep  DRRDGVFGSQIAKRLSGMFTLVAVLPGVFLFGVSAQFINGTINSWFGNDTHEALERSLNL
            ||||||||||||||||||||||||||||||||||||||||||||||||||||||||||||
orf64-1     DRRDGVFGSQIAKRLSGMFTLVAVLPGVFLFGVSAQFINGTINSWFGNDTHEALERSLNL
                       70        80        90       100       110       120

                      130       140       150       160       170       180
orf64a.pep  SKSALNLAADNALGNAIPVQIDXIGAASLPXDMGRVLEHYAGSGFAQLALYNAASGKIEK
            ||||||||||||||||:||||| ||||||| |||||||||||||||||||||||||||||
orf64-1     SKSALNLAADNALGNAVPVQIDLIGAASLPGDMGRVLEHYAGSGFAQLALYNAASGKIEK
                      130       140       150       160       170       180

                      190       200       210       220       230       240
orf64a.pep  SINPHKLDQPFPGKARWEKIQQAGSVRDXESIGGVLYAXGWLSAXTHNGRDYALFFRQPV
            |||||||||||||||||||||:|||||| |||||||||| ||||| ||||||||||||||
orf64-1     SINPHKLDQPFPGKARWEKIQRAGSVRDLESIGGVLYAQGWLSAGTHNGRDYALFFRQPV
                      190       200       210       220       230       240

                      250       260       270       280       290       300
orf64a.pep  PKGVAEDAVLIEKARAXXXXLSYSKKGLQTFFLATLLIASLLSIFLALVMALYFARRFVE
            ||||||||||||||||    ||||||||||||||||||||||||||||||||||||||||
orf64-1     PKGVAEDAVLIEKARAKYAELSYSKKGLQTFFLATLLIASLLSIFLALVMALYFARRFVE
                      250       260       270       280       290       300

                      310       320       330       340       350       360
orf64a.pep  PVLSLAEGAKAVAQGDFSQTRPVLRNDEFGRLTKLFNHMTEQLSIAKEADERNRRREEAA
            ||||||||||||||||||||||||||||||||||||||||||||||||||||||||||||
orf64-1     PVLSLAEGAKAVAQGDFSQTRPVLRNDEFGRLTKLFNHMTEQLSIAKEADERNRRREEAA
                      310       320       330       340       350       360

                      370       380       390       400       410       420
orf64a.pep  RHYLECVLEGLTTGVVVFDEQGCLKTFNKAAEQILGMPLTPLWGSSRHGWHGVSAQQSLL
            ||||||||||||||||||||||||||||||||||||||||||||||||||||||||||||
orf64-1     RHYLECVLEGLTTGVVVFDEQGCLKTFNKAAEQILGMPLTPLWGSSRHGWHGVSAQQSLL
                      370       380       390       400       410       420

                      430       440       450       460       470       480
orf64a.pep  AEVFAAIGAAAGTDKPVHVKYAAPDDAKILLGKATVLPEDNXNGVVMVIDDITVLIHAQK
            ||||||||||||||||||||||||||||||||||||||||| ||||||||||||||||||
orf64-1     AEVFAAIGAAAGTDKPVHVKYAAPDDAKILLGKATVLPEDNGNGVVMVIDDITVLIHAQK
                      430       440       450       460       470       480

                      490       500       510       520       530       540
orf64a.pep  EAAWGEVAKRLAHEIRNPLTPIQLSAERLAWKLGGKLDEXDAQILTRSTDTIIKQVAALK
            ||||||||||||||||||||||||||||||||||||||| ||||||||||||:|||||||
orf64-1     EAAWGEVAKRLAHEIRNPLTPIQLSAERLAWKLGGKLDEQDAQILTRSTDTIVKQVAALK
                      490       500       510       520       530       540

                      550       560       570       580       590       600
orf64a.pep  EMVEAFRNYXRSPSXQLENQDLNALIGDVLALYEAGPCRFAAELAGEPLMMAADTTAMRQ
            ||||||||| |||| :|||||||||||||||||||||||||||||||||| :||||||||
orf64-1     EMVEAFRNYARSPSLKLENQDLNALIGDVLALYEAGPCRFAAELAGEPLTVAADTTAMRQ
                      550       560       570       580       590       600

                      610       620       630       640       650       660
orf64a.pep  VLHNIFKNAAEAAEEADVPEVRVKSEAGQDGRIVLTVCDNGKGFGREMLHNAFEPYVTDK
```

```
              ||||||||||||||||||||||||||:|||||||||||||||||||||||||||||||||
orf64-1       VLHNIFKNAAEAAEEADVPEVRVKSETGQDGRIVLTVCDNGKGFGREMLHNAFEPYVTDK
                     610       620       630       640       650       660

                     670       680       690       700
orf64a.pep    PAGTGLXLPVVKKIIEEHGGXISLSNQDAGGAXVRIILPKTVETYAX
              |||||| ||||||||||||| ||||||||||| |||||||||:||||
orf64-1       PAGTGLGLPVVKKIIEEHGGRISLSNQDAGGACVRIILPKTVKTYAX
                     670       680       690       700
```

Homology with a predicted ORF from *N.gonorrhoeae*

ORF64 shows 86.6% identity over a 387aa overlap with a predicted ORF (ORF64.ng) from *N. gonorrhoeae:*

```
orf64.pep    MRRFLPIAAICAXXLXXGLTAATGSTSSLADYFWWIVAFSAMLLLVLSAVLARYVILLLK  60
             |||||||||||   |   |||||||||||||||||||:|||||||||||||||||||||||
orf64ng      MRRFLPIAAICAVVLLYGLTAATGSTSSLADYFWWIVSFSAMLLLVLSAVLARYVILLLK  60

orf64.pep    DRRDGVFGSXXAKXPXXXMFTLVAXLPGVFLFGFPAQFINGTINSWFGNDTHEALERSLN 120
             |||:|||||  ||     |||||| |||:||||: ||||||||||||||||||||||||||
orf64ng      DRRNGVFGSQIAKR-LSGMFTLVAVLPGLFLFGISAQFINGTINSWFGNDTHEALERSLN 119

orf64.pep    LSKSALNLAADNALGNAVPVQIDLIGAASLPGDMGRVLEHYAGSGFAQLALYNXASGKIE 180
             ||||||:||||||::|||||||||||||:||| |:|| |||||||||||||||| ||||||
orf64ng      LSKSALDLAADNAVSNAVPVQIDLIGTASLSGNMGSVLEHYAGSGFAQLALYNAASGKIE 179

orf64.pep    KSINPHKLDQPFPGKARWEKIQRAGSVRDLESIGGVLYAQGWLSAGTHXGRDYALFFRQP 240
             ||||||::|||:| | :||:||::||||:||||||||||||||||||||| ||||||||||
orf64ng      KSINPHQFDQPLPDKEHWEQIQQTGSVRSLESIGGVLYAQGWLSAGTHNGRDYALFFRQP 239

orf64.pep    VPKGVAEDAVLIEKARAKYAELSYSKKGLQTFFLATLLIASLLSIFLALVMALYFARRFV 300
             :|::||:||||||||||||||||||||||||||:||||||||||||||||||||||||||
orf64ng      IPENVAQDAVLIEKARAKYAELSYSKKGLQTFFLVTLLIASLLSIFLALVMALYFARRFV 299

orf64.pep    EPVLSLAEGAKAVAQGDFSQTRPVLRNDEFGRLTXLFNHMTEQLSIAKDADERNRRREEA 360
             ||:|||||||||||||||||||||||||||||||| |||||||||||||:||||||||||
orf64ng      EPILSLAEGAKAVAQGDFSQTRPVLRNDEFGRLTKLFNHMTEQLSIAKEADERNRRREEA 359

orf64.pep    ARHYLECVLEGLTTGVVVFDEQGCLKTFNKAAGT            394
             ||||||||:||||||||     :| :|
orf64ng      ARHYLECVLDGLTTGVVVSYPLSCCRTAVFSTCHSSPLSYF     400
```

An ORF64ng nucleotide sequence <SEQ ID 255> was predicted to encode a protein having amino acid sequence <SEQ ID 256>:

```
  1  MRRFLPIAAI CAVVLLYGLT AATGSTSSLA DYFWWIVSFS AMLLLVLSAV
 51  LARYVILLLK DRRNGVFGSQ IAKRLSGMFT LVAVLPGLFL FGISAQFING
101  TINSWFGNDT HEALERSLNL SKSALDLAAD NAVSNAVPVQ IDLIGTASLS
151  GNMGSVLEHY AGSGFAQLAL YNAASGKIEK SINPHQFDQP LPDKEHWEQI
201  QQTGSVRSLE SIGGVLYAQG WLSAGTHNGR DYALFFRQPI PENVAQDAVL
251  IEKARAKYAE LSYSKKGLQT FFLVTLLIAS LLSIFLALVM ALYFARRFVE
301  PILSLAEGAK AVAQGDFSQT RPVLRNDEFG RLTKLFNHMT EQLSIAKEAD
351  ERNRRREEAA RHYLECVLDG LTTGVVVSYP LSCCRTAVFS TCHSSPLSYF*
```

Further work revealed the complete gonococcal DNA sequence <SEQ ID 257>:

```
   1 ATGCGCCGCT TCCTACCGAT CGCAGCCATA TGCGCCGTCG TCCTGCTGTA
  51 CGGATTGACG GCGGCGACCG GCAGCACCAG TTCGCTGGCG GATTATTTCT
 101 GGTGGATAGT CTCGTTCAGC GCAATGCTGC TGCTGGTGTT GTCCGCCGTT
 151 TTGGCACGTT ATGTCATATT GCTGTTGAAA GACAGGCGCA ACGGCGTGTT
 201 CGGTTCGCAG ATTGCCAAAC GCCTTTCCGG GATGTTCACG CTGGTCGCCG
 251 TACTGCCCGG CTTGTTCCTG TTCGGCATTT CCGCGCAGTT TATCAACGGC
 301 ACGATTAATT CGTGGTTCGG CAACGACACC CACGAAGCCC TCGAACGCAG
 351 CCTTAATTTG AGCAAGTCCG CACTGGATTT GGCGGCAGAC AATGCCGTCA
 401 GCAACGCCGT TCCCGTACAG ATAGACCTCA TCGGCACCGC CTCCCTGTCG
 451 GGCAATATGG GCAGTGTGCT GGAACACTAC GCCGGCAGCG GTTTTGCCCA
 501 GCTTGCCCTG TACAATGCCG CAAGCGGGAA AATCGAAAAA AGCATCAATC
```

```
 551 CGCACCAATT CGACCAGCCG CTTCCCGACA AAGAACATTG GGAACAGATT
 601 CAGCAGACCG GTTCGGTTCG GAGTTTGGAA AGCATAGGCG GCGTATTGTA
 651 CGCGCAGGGA TGGTTGTCGG CAGGTACGCA CAACGGGCGC GATTACGCGC
 701 TGTTCTTCCG CCAGCCGATT CCCGAAAATG TGGCACAGGA TGCCGTTCTG
 751 ATTGAAAAGG CGCGGGCGAA ATATGCCGAA TTGAGTTACA GCAAAAAAGG
 801 TTTGCAGACC TTTTTTCTGG TAACCCTGCT GATTGCCTCG CTGCTGTCGA
 851 TTTTTCTTGC GCTGGTAATG GCACTGTATT TTGCCCGCCG TTTCGTCGAA
 901 CCCATTCTGT CGCTTGCCGA GGGCGCAAAG GCGGTGGCGC AGGGTGATTT
 951 CAGCCAGACG CGCCCCGTAT TGCGCAACGA CGAGTTCGGA CGTTTGACCA
1001 AGCTGTTCAA CCATATGACC GAGCAGCTTT CCATCGCCAA AGAAGCAGAC
1051 GAACGCAACC GCCGGCGCGA GGAAGCCGCC CGTCACTACC TCGAGTGCGT
1101 GTTGGATGGG TTGACTACCG GTGTGGTGGT GTTTGACGAA AAAGGCCGTT
1151 TGAAAACCTT CAACAAGGCG GCGGAACAGA TTTTGGGGAT GCCGCTCGCC
1201 CCCCTGTGGG GCAGCAGCCG GCACGGTTGG CACGGCGTTT CGGCGCAGCA
1251 GTCCCTGCTT GCCGAAGTGT TtgccgccAT CGGTGCGGCG GCAGGTACGG
1301 ACAAACCGGT CCAGGTGGAA TATGCCGCGC CGGACGATGC CAAAATCCTG
1351 CTGGGCAAGG CGACGGTATT GCCCGAAGAC AACGGCAACG GCGTGGTGAT
1401 GGTGATTGAC GACATCACCG TGCTGATACG CGCGCAAAAA GAAGCCGCGT
1451 GGGGTGAAGT GGCGAAGCGG CTGGCACACG AAATCCGCAA TCCGCTCACG
1501 CCCATCCAGC TTTCCGCCGA ACGGCTGGCG TGGAAATTGG GCGGGAAGCT
1551 GGACGATCAG GACGCGCAAA TCCTGACGCG TtcgACCGAC ACCATCATCA
1601 AACAGgtggc gGCGTTAAAA GAAATGGTCG AGGCATTCCG CAATTACGCG
1651 CGCGCCCCTT CGCTCAAACT GGAAAATCAG GATTTGAACG CCTTAATCGG
1701 CGATGTTTTG GCCCTGTACG AAGCCGGCCC GTGCCGGTTT GAGGCGGAAC
1751 TTGCCGGCGA ACCGCTGATG ATGGCGGCGG ATACGACCGC CATGCGGCAG
1801 GTGCTGCACA ATATTTTCAA AAATGCCGCC GAAGCGGCGG AAGAAGCCGA
1851 TATGCCCGAA GTCAGGGTAA AATCGGAAAC GGGGCAGGAC GGACGGATTG
1901 TCCTGACGGT TTGCGACAAC GGCAAGGGAT TCGGCAAGGA AATGCTGCAC
1951 AATGCTTTCG AGCCGTATGT GACGGATAAG CCGGCGGGAA CGGGACTGGG
2001 TCTGCCTGTA GTGAAAAAAA TCATTGGAGA ACACGGCGGC CGCATCAGCC
2051 TGAGCAATCA GGATGCGGGT GGGGCGTGTG TCAGAATCAT CTTGCCAAAA
2101 ACGGTAGAAA CTTATGCGTA G
```

This corresponds to the amino acid sequence <SEQ ID 258; ORF64ng-1>:

```
  1  MRRFLPIAAI CAVVLLYGLT AATGSTSSLA DYFWWIVSFS AMLLLVLSAV
 51  LARYVILLLK DRRNGVFGSQ IAKRLSGMFT LVAVLPGLFL FGISAQFING
101  TINSWFGNDT HEALERSLNL SKSALDLAAD NAVSNAVPVQ IDLIGTASLS
151  GNMGSVLEHY AGSGFAQLAL YNAASGKIEK SINPHQFDQP LPDKEHWEQI
201  QQTGSVRSLE SIGGVLYAQG WLSAGTHNGR DYALFFRQPI PENVAQDAVL
251  IEKARAKYAE LSYSKKGLQT FFLVTLLIAS LLSIFLALVM ALYFARRFVE
301  PILSLAEGAK AVAQGDFSQT RPVLRNDEFG RLTKLFNHMT EQLSIAKEAD
351  ERNRRREEAA RHYLECVLDG LTTGVVVFDE KGRLKTFNKA AEQILGMPLA
401  PLWGSSRHGW HGVSAQQSLL AEVFAAIGAA AGTDKPVQVE YAAPDDAKIL
451  LGKATVLPED NGNGVVMVID DITVLIRAQK EAAWGEVAKR LAHEIRNPLT
501  PIQLSAERLA WKLGGKLDDQ DAQILTRSTD TIIKQVAALK EMVEAFRNYA
551  RAPSLKLENQ DLNALIGDVL ALYEAGPCRF EAELAGEPLM MAADTTAMRQ
601  VLHNIFKNAA EAAEEADMPE VRVKSETGQD GRIVLTVCDN GKGFGKEMLH
651  NAFEPYVTDK PAGTGLGLPV VKKIIGEHGG RISLSNQDAG GACVRIILPK
701  TVETYA*
```

ORF64ng-1 and ORF64-1 show 93.8% identity in 706 aa overlap:

```
                          10        20        30        40        50        60
orf64ng-1.pep    MRRFLPIAAICAVVLLYGLTAATGSTSSLADYFWWIVSFSAMLLLVLSAVLARYVILLLK
                 |||||||||||||||||||||||||||||||||||||:||||||||||||||||||||||
orf64-1          MRRFLPIAAICAVVLLYGLTAATGSTSSLADYFWWIVAFSAMLLLVLSAVLARYVILLLK
                          10        20        30        40        50        60

                          70        80        90       100       110       120
orf64ng-1.pep    DRRNGVFGSQIAKRLSGMFTLVAVLPGLFLFGISAQFINGTINSWFGNDTHEALERSLNL
                 |||:|||||||||||||||||||||||:||||:|||||||||||||||||||||||||||
orf64-1          DRRDGVFGSQIAKRLSGMFTLVAVLPGVFLFGVSAQFINGTINSWFGNDTHEALERSLNL
                          70        80        90       100       110       120

                         130       140       150       160       170       180
orf64ng-1.pep    SKSALDLAADNAVSNAVPVQIDLIGTASLSGNMGSVLEHYAGSGFAQLALYNAASGKIEK
                 |||||:|||||::||||||||||||:||| |:|| |||||||||||||||||||||||||
orf64-1          SKSALNLAADNALGNAVPVQIDLIGAASLPGDMGRVLEHYAGSGFAQLALYNAASGKIEK
                         130       140       150       160       170       180

                         190       200       210       220       230       240
```

```
orf64ng-1.pep  SINPHQFDQPLPDKEHWEQIQQTGSVRSLESIGGVLYAQGWLSAGTHNGRDYALFFRQPI
               |||||::|||:| | :||:||::||||:|||||||||||||||||||||||||||||||:
orf64-1        SINPHKLDQPFPGKARWEKIQRAGSVRDLESIGGVLYAQGWLSAGTHNGRDYALFFRQPV
                     190       200       210       220       230       240

                     250       260       270       280       290       300
orf64ng-1.pep  PENVAQDAVLIEKARAKYAELSYSKKGLQTFFLVTLLIASLLSIFLALVMALYFARRFVE
               |::||:||||||||||||||||||||||||||:|||||||||||||||||||||||||||
orf64-1        PKGVAEDAVLIEKARAKYAELSYSKKGLQTFFLATLLIASLLSIFLALVMALYFARRFVE
                     250       260       270       280       290       300

                     310       320       330       340       350       360
orf64ng-1.pep  PILSLAEGAKAVAQGDFSQTRPVLRNDEFGRLTKLFNHMTEQLSIAKEADERNRRREEAA
               |:||||||||||||||||||||||||||||||||||||||||||||||||||||||||||
orf64-1        PVLSLAEGAKAVAQGDFSQTRPVLRNDEFGRLTKLFNHMTEQLSIAKEADERNRRREEAA
                     310       320       330       340       350       360

                     370       380       390       400       410       420
orf64ng-1.pep  RHYLECVLDGLTTGVVVFDEKGRLKTFNKAAEQILGMPLAPLWGSSRHGWHGVSAQQSLL
               ||||||||:|||||||||||:| |||||||||||||||||:|||||||||||||||||||
orf64-1        RHYLECVLEGLTTGVVVFDEQGCLKTFNKAAEQILGMPLTPLWGSSRHGWHGVSAQQSLL
                     370       380       390       400       410       420

                     430       440       450       460       470       480
orf64ng-1.pep  AEVFAAIGAAAGTDKPVQVEYAAPDDAKILLGKATVLPEDNGNGVVMVIDDITVLIRAQK
               ||||||||||||||||:|:|||||||||||||||||||||||||||||||||||||:|||
orf64-1        AEVFAAIGAAAGTDKPVHVKYAAPDDAKILLGKATVLPEDNGNGVVMVIDDITVLIHAQK
                     430       440       450       460       470       480

                     490       500       510       520       530       540
orf64ng-1.pep  EAAWGEVAKRLAHEIRNPLTPIQLSAERLAWKLGGKLDDQDAQILTRSTDTIIKQVAALK
               |||||||||||||||||||||||||||||||||||||:|||||||||||||:||||||||
orf64-1        EAAWGEVAKRLAHEIRNPLTPIQLSAERLAWKLGGKLDEQDAQILTRSTDTIVKQVAALK
                     490       500       510       520       530       540

                     550       560       570       580       590       600
orf64ng-1.pep  EMVEAFRNYARAPSLKLENQDLNALIGDVLALYEAGPCRFEAELAGEPLMMAADTTAMRQ
               |||||||||||:||||||||||||||||||||||||||||| ||||||||  :|||||||||
orf64-1        EMVEAFRNYARSPSLKLENQDLNALIGDVLALYEAGPCRFAAELAGEPLTVAADTTAMRQ
                     550       560       570       580       590       600

                     610       620       630       640       650       660
orf64ng-1.pep  VLHNIFKNAAEAAEEADMPEVRVKSETGQDGRIVLTVCDNGKGFGKEMLHNAFEPYVTDK
               ||||||||||||||||:||||||||||||||||||||||||||||:||||||||||||||
orf64-1        VLHNIFKNAAEAAEEADVPEVRVKSETGQDGRIVLTVCDNGKGFGREMLHNAFEPYVTDK
                     610       620       630       640       650       660

                     670       680       690       700
orf64ng-1.pep  PAGTGLGLPVVKKIIGEHGGRISLSNQDAGGACVRIILPKTVETYAX
               |||||||||||||| ||||||||||||||||||||||||||||:||||
orf64-1        PAGTGLGLPVVKKIIEEHGGRISLSNQDAGGACVRIILPKTVKTYAX
                     670       680       690       700
```

Furthermore, ORF64ng-1 shows significant homology to a protein from *A. caulinodans:*

```
sp|Q04850|NTRY_AZOCA NITROGEN REGULATION PROTEIN NTRY >gi|77479|pir||S18624 ntrY
protein - Azorhizobium caulinodans >gi|38737 (X63841) NtrY gene product
[Azorhizobium caulinodans] Length = 771
 Score =  218 bits (550), Expect = 7e-56
 Identities = 195/720 (27%), Positives = 320/720 (44%), Gaps = 58/720 (8%)

Query: 7   IAAICAVVLLYGLTAATGSTSSLADYFWWIXXXXXXXXXXXXXXXXRYVILLLKDRRNGV 66
            I+A+   ++L GLT    +   +     +                R +  + K R  G
Sbjct: 35  ISALATFLILMGLTPVVPTHQVVIS----VLLVNAAAVLILSAMVGREIWRIAKARARGR 90

Query: 67  FGSQIAKRLSGMFTLVAVLPGLFLFGISAQFINGTINSWFGNDTHEALERSLNLSKSALD 126
             +++  R+ G+F +V+V+P + +  +++  ++  ++ WF   T E +  S++++++ +
Sbjct: 91  AAARLHIRIVGLFAVVSVVPAILVAVVASLTLDRGLDRWFSMRTQEIVASSVSVAQTYVR 150

Query: 127 LAADNAVSNAVPVQIDLIGTASLSGNMGSVLEHYAG--SGFAQLALYNAASGKIEKSINP 184
             A N   + + +  DL    S+            Y G  S F Q+   AA   +  ++
Sbjct: 151 EHALNIRGDILAMSADLTRLKSV----------YEGDRSRFNQILTAQAALRNLPGAMLI 200

Query: 185 HQFDQPLPDKEHWEQIQQTGSVRSLESIGGVLYAQGWLSAGTHNGRDYA----------- 233
             + D  + ++ +   I +   V + +IG     Q  +     N  DY
Sbjct: 201 RR-DLSVVERAN-VNIGREFIVPANLAIGDATPDQPVIYLP--NDADYVAAVVPLKDYDD 256

Query: 234 --LFFRQPIPENVAQDAVLIEKARAKYAELSYSKKGLQTFFLVTXXXXXXXXXXXXXVMA 291
              L+  + I   V        ++  A Y  L   + G+Q  F +                +
Sbjct: 257 LYLYVARLIDPRVIGYLKTTQETLADYRSLEERRFGVQVAFALMYAVITLIVLLSAVWLG 316

Query: 292 LYFARRFVEPILSLAEGAKAVAQGDFSQTRPVLRND-EFGRLTKLFNHMTEQLSIXXXXX 350
            L F++  V PI  L  A  VA+G+     P+ R + +  L + FN MT +L
Sbjct: 317 LNFSKWLVAPIRRLMSAADHVAEGNLDVRVPIYRAEGDLASLAETFNKMTHELRSQREAI 376

Query: 351 XXXXXXXXXXXHYLECVLDGLTTGVVVFDEKGRLKTFNKAAEQILGMPLAPLWGSSRHGW 410
                        + E VL G+  GV+  D + R+  N++AE++LG  L+ +    RH
Sbjct: 377 LTARDQIDSRRRFTEAVLSGVGAGVIGLDSQERITILNRSAERLLG--LSEVEALHRHLA 434

Query: 411 HGVSAQQSLLAEVFXXXXXXXXXTDKPVQVEYAAPDDAKILLGKATVLPEDNG---NGVVM 467
             V        LL E          + VQ     D +    +   V  E +   +G V+
Sbjct: 435 EVVPETAGLLEEA------EHARQRSVQGNITLTRDGRERVFAVRVTTEQSPEAEHGWVV 488

Query: 468 VIDDITVLIRAQKEAAWGEVAKRLAHEIRNPLTPIQLSAERLAWKLGGKLDDQDAQILTR 527
            +DDIT LI AQ+ +AW +VA+R+AHEI+NPLTPIQLSAERL  K G +  QD +I  +
Sbjct: 489 TLDDITELISAQRTSAWADVARRIAHEIKNPLTPIQLSAERLKRKFGRHV-TQDREIFDQ 547

Query: 528 STDTIIKQVAALKEMVEAFRNYARAPSLKLENQDLNALIGDVLALYEAGPCRFEAELAGE 587
             TDTII+QV   +  MV+ F ++AR P  +++QD++ +I  + L  G       +
Sbjct: 548 CTDTIIRQVGDIGRMVDEFSSFARMPKPVVDSQDMSEIIRQTVFLMRVGHPEVVFDSEVP 607

Query: 588 PLMMAA-DTTAMRQVLHNIFKNXXXXXXXXXDMPEVRVK-------SETGQDGRIVLTVCD 639
            P M A  D   + Q L NI KN          P+VR +       +  G+D  +V+ + D
Sbjct: 608 PAMPARFDRRLVSQALTNILKNAAEAIEAVP-PDVRGQGRIRVSANRVGED--LVIDIID 664

Query: 640 NGKGFGKEMLHNAFEPYVTDKPAGTGLGLPVVKKIIGEHGGRISLSNQDAG-GACVRIIL 698
            NG G  +E +  EPYVT +  GTGLGL +V KI+ EHGG I L++  G GA +R+ L
Sbjct: 665 NGTGLPQESRNRLLEPYVTTREKGTGLGLAIVGKIMEEHGGGIELNDAPEGRGAWIRLTL 724
```

Based on this analysis, including the presence of a putative leader sequence (double-underlined) and several putative transmembrane domains (single-underlined) in the gonococcal protein, it is predicted that the proteins from *N.meningitidis* and *N.gonorrhoeae,* and their epitopes, could be useful antigens for vaccines or diagnostics, or for raising antibodies.

**Example 31**

The following partial DNA sequence was identified in *N.meningitidis* <SEQ ID 259>:

```
  1  ATGTACGCAT TTACCGCCGC ACAGCAACAG AAGGCACTCT TCCGGCTGGT
 51  GCTTTTTCAT ATCCTCATCA TCGCCGCCAG CAACTATCTG GTGCAGTTCC
101  CTTTCCAAAT TTTCGGCATC CACACCACTT GGGGCGCATT TTCCTTTCCC
151  TTCATCTTCC TTGCCACCGA CCTGACCGTC CGCATTTTCG GTTCTCACTT
201  GGCACGGCGG ATTATCTTTT GGGTGATGTT CCCCGCCCTT TTGCTTTCCT
251  ACGTCTTTTC CGTTTTGTTC CACAACGGCA GTTGGACAGG CTTGGGCGCG
301  CTGTCCGAAT TCAACACCTT TGTCGGACGC ATCGCCTTAG CCAGCTTTGC
351  CGCCTACGCG ATCGGACAAA TCCTTGATAT TTTTGTATTC AACAAATTAC
401  GCCGTCTGAA AGCGTGGTGG ATTGCACCGA ACGCATCAAC CGTCATCGGG
451  CACGCGTTGG ATACG...
```

This corresponds to the amino acid sequence <SEQ ID 260; ORF66>:

```
  1  MYAFTAAQQQ KALFRLVLFH ILIIAASNYL VQFPFQIFGI HTTWGAFSFP
 51  FIFLATDLTV RIFGSHLARR IIFWVMFPAL LLSYVFSVLF HNGSWTGLGA
101  LSEFNTFVGR IALASFAAYA IGQILDIFVF NKLRRLKAWW IAPNASTVIG
151  HALDT...
```

Further work revealed the complete nucleotide sequence <SEQ ID 261>:

```
  1  ATGTACGCAT TTACCGCCGC ACAGCAACAG AAGGCACTCT TCCGGCTGGT
 51  GCTTTTTCAT ATCCTCATCA TCGCCGCCAG CAACTATCTG GTGCAGTTCC
101  CTTTCCAAAT TTTCGGCATC CACACCACTT GGGGCGCATT TTCCTTTCCC
151  TTCATCTTCC TTGCCACCGA CCTGACCGTC CGCATTTTCG GTTCTCACTT
201  GGCACGGCGG ATTATCTTTT GGGTGATGTT CCCCGCCCTT TTGCTTTCCT
251  ACGTCTTTTC CGTTTTGTTC CACAACGGCA GTTGGACAGG CTTGGGCGCG
301  CTGTCCGAAT TCAACACCTT TGTCGGACGC ATCGCCTTAG CCAGCTTTGC
351  CGCCTACGCG ATCGGACAAA TCCTTGATAT TTTTGTATTC AACAAATTAC
401  GCCGTCTGAA AGCGTGGTGG ATTGCACCGA CCGCATCAAC CGTCATCGGC
451  AACGCCTTGG ATACGCTGGT ATTTTTCGCC GTTGCCTTCT ACGCAAGCAG
501  CGATGGATTT ATGGCGGCAA ACTGGCAGGG CATCGCTTTT GTCGATTACC
551  TGTTCAAACT TACCGTCTGC ACCCTCTTCT TCCTGCCCGC CTACGGCGTG
601  ATACTGAATC TGCTGACGAA AAAACTGACA ACCCTGCAAA CCAAACAGGC
651  GCAAGACCGC CCCGCGCCCT CGCTGCAAAA TCCGTAA
```

This corresponds to the amino acid sequence <SEQ ID 262; ORF66-1>:

```
  1  MYAFTAAQQQ KALFRLVLFH ILIIAASNYL VQFPFQIFGI HTTWGAFSFP
 51  FIFLATDLTV RIFGSHLARR IIFWVMFPAL LLSYVFSVLF HNGSWTGLGA
101  LSEFNTFVGR IALASFAAYA IGQILDIFVF NKLRRLKAWW IAPTASTVIG
151  NALDTLVFFA VAFYASSDGF MAANWQGIAF VDYLFKLTVC TLFFLPAYGV
201  ILNLLTKKLT TLQTKQAQDR PAPSLQNP*
```

Computer analysis of this amino acid sequence gave the following results:

Homology with the hypothetical protein o221 of *E. coli* (accession number P37619)

ORF66 and o221 protein show 67% aa identity in 155aa overlap:

```
orf66  1   MYAFTAAQQQKALFRLVLFHILIIAASNYLVQFPFQIFGIHTTWGAFSFPFIFLATDLTV 60
           M  F+  Q+ KALF L LFH+L+I +SNYLVQ P  I G HTTWGAFSFPFIFLATDLTV
o221   1   MNVFSQTQRYKALFWLSLFHLLVITSSNYLVQLPVSILGFHTTWGAFSFPFIFLATDLTV 60

orf66  61  RIFGSHLARRIIFWVMFPALLLSYVFSVLFHNGSWTGLGALSEFNTFVGRIALASFAAYA 120
           RIFG+ LARRIIF VM PALL+SYV S LF+ GSW G GAL+ FN FV RIA ASF AYA
o221   61  RIFGAPLARRIIFAVMIPALLISYVISSLFYMGSWQGFGALAHFNLFVARIATASFMAYA 120

orf66  121 IGQILDIFVFNKLRRLKAWWIAPNASTVIGHALDT 155
           +GQILD+ VFN+LR+ + WW+AP AST+ G+  DT
o221   121 LGQILDVHVFNRLRQSRRWWLAPTASTLFGNVSDT 155
```

Homology with a predicted ORF from *N.meningitidis* (strain A)

ORF66 shows 96.1% identity over a 155aa overlap with an ORF (ORF66a) from strain A of *N. meningitidis:*

```
                    10        20        30        40        50        60
orf66.pep   MYAFTAAQQQKALFRLVLFHILIIAASNYLVQFPFQIFGIHTTWGAFSFPFIFLATDLTV
            |||||||||||||| ||||||||||||||||||||||| |||||||||||||||||||||
orf66a      MYAFTAAQQQKALFWLVLFHILIIAASNYLVQFPFQISGIHTTWGAFSFPFIFLATDLTV
                    10        20        30        40        50        60

                    70        80        90       100       110       120
orf66.pep   RIFGSHLARRIIFWVMFPALLLSYVFSVLFHNGSWTGLGALSEFNTFVGRIALASFAAYA
            ||||||||||||||||||||||||||||||||||||||||||||||||||||||||||||
orf66a      RIFGSHLARRIIFWVMFPALLLSYVFSVLFHNGSWTGLGALSEFNTFVGRIALASFAAYA
                    70        80        90       100       110       120

                   130       140       150
orf66.pep   IGQILDIFVFNKLRRLKAWWIAPNASTVIGHALDT
            :||||||||||||||||||||:||:||||||:||||
orf66a      LGQILDIFVFNKLRRLKAWWVAPTASTVIGNALDTLVFFAVAFYASSDGFMAANWQGIAF
                   130       140       150       160       170       180

orf66a      VDYLFKLTVCGLFFLPAYGVILNLLTKKLTTLQTKQAQDRPAPSLQNPX
                   190       200       210       220
```

The complete length ORF66a nucleotide sequence <SEQ ID 263> is:

```
  1  ATGTACGCAT TTACCGCCGC ACAGCAACAG AAGGCACTCT TCTGGCTGGT
 51  GCTTTTTCAT ATCCTCATCA TCGCCGCCAG CAACTATCTG GTGCAGTTCC
101  CCTTCCAAAT TTCCGGCATC CACACCACTT GGGGCGCGTT TTCCTTTCCC
151  TTCATCTTCC TCGCCACCGA CCTGACCGTC CGCATTTTCG GTTCGCACTT
201  GGCACGGCGG ATTATCTTTT GGGTCATGTT CCCCGCCCTT TTGCTTTCCT
251  ACGTCTTTTC CGTTTTGTTC CACAACGGCA GTTGGACGGG CTTGGGCGCG
301  CTGTCCGAAT TCAACACCTT TGTCGGACGC ATCGCGCTGG CAAGTTTTGC
351  CGCCTACGCG CTCGGACAAA TCCTTGATAT TTTTGTGTTC AACAAATTAC
401  GCCGTCTGAA AGCGTGGTGG GTTGCCCCGA CTGCATCAAC CGTCATCGGC
451  AACGCCTTAG ATACGTTGGT ATTTTTCGCC GTTGCCTTCT ACGCAAGCAG
501  CGATGGATTT ATGGCGGCAA ACTGGCAGGG CATCGCTTTT GTCGATTACC
551  TGTTCAAACT CACCGTCTGC GGTCTGTTTT TCCTGCCCGC CTACGGCGTG
601  ATTCTGAATC TGCTGACGAA AAAACTGACG ACCCTGCAAA CCAAACAGGC
651  GCAAGACCGC CCCGCGCCCT CGCTGCAAAA TCCGTAA
```

This encodes a protein having amino acid sequence <SEQ ID 264>:

```
  1  MYAFTAAQQQ KALFWLVLFH ILIIAASNYL VQFPFQISGI HTTWGAFSFP
 51  FIFLATDLTV RIFGSHLARR IIFWVMFPAL LLSYVFSVLF HNGSWTGLGA
101  LSEFNTFVGR IALASFAAYA LGQILDIFVF NKLRRLKAWW VAPTASTVIG
151  NALDTLVFFA VAFYASSDGF MAANWQGIAF VDYLFKLTVC GLFFLPAYGV
201  ILNLLTKKLT TLQTKQAQDR PAPSLQNP*
```

ORF66a and ORF66-1 show 97.8% identity in 228 aa overlap:

```
                       10        20        30        40        50        60
orf66a.pep      MYAFTAAQQQKALFWLVLFHILIIAASNYLVQFPFQISGIHTTWGAFSFPFIFLATDLTV
                |||||||||||||||| |||||||||||||||||| ||||||||||||||||||||||||
orf66-1         MYAFTAAQQQKALFRLVLFHILIIAASNYLVQFPFQIFGIHTTWGAFSFPFIFLATDLTV
                       10        20        30        40        50        60

                       70        80        90       100       110       120
orf66a.pep      RIFGSHLARRIIFWVMFPALLLSYVFSVLFHNGSWTGLGALSEFNTFVGRIALASFAAYA
                ||||||||||||||||||||||||||||||||||||||||||||||||||||||||||||
orf66-1         RIFGSHLARRIIFWVMFPALLLSYVFSVLFHNGSWTGLGALSEFNTFVGRIALASFAAYA
                       70        80        90       100       110       120

                      130       140       150       160       170       180
orf66a.pep      LGQILDIFVFNKLRRLKAWWVAPTASTVIGNALDTLVFFAVAFYASSDGFMAANWQGIAF
                :|||||||||||||||||||:|||||||||||||||||||||||||||||||||||||||
orf66-1         IGQILDIFVFNKLRRLKAWWIAPTASTVIGNALDTLVFFAVAFYASSDGFMAANWQGIAF
                      130       140       150       160       170       180

                      190       200       210       220      229
orf66a.pep      VDYLFKLTVCGLFFLPAYGVILNLLTKKLTTLQTKQAQDRPAPSLQNPX
                |||||||||| ||||||||||||||||||||||||||||||||||||||
orf66-1         VDYLFKLTVCTLFFLPAYGVILNLLTKKLTTLQTKQAQDRPAPSLQNPX
                      190       200       210       220
```

<u>Homology with a predicted ORF from *N.gonorrhoeae*</u>

ORF66shows 94.2% identity over a 155aa overlap with a predicted ORF (ORF66.ng) from *N. gonorrhoeae:*

```
orf66.pep    MYAFTAAQQQKALFRLVLFHILIIAASNYLVQFPFQIFGIHTTWGAFSFPFIFLATDLTV   60
             |||:||||||||||||||||||||||||||||||:|||||||||||||||||||||||||
orf66ng      MYALTAAQQQKALFRLVLFHILIIAASNYLVQFPFRIFGIHTTWGAFSFPFIFLATDLTV   60

orf66.pep    RIFGSHLARRIIFWVMFPALLLSYVFSVLFHNGSWTGLGALSEFNTFVGRIALASFAAYA  120
             ||||||||||||||||||||| |||||||||||||||||| |:|||||||||||||||||
orf66ng      RIFGSHLARRIIFWVMFPALSLSYVFSVLFHNGSWTGLGAPSQFNTFVGRIALASFAAYA  120

orf66.pep    IGQILDIFVFNKLRRLKAWWIAPNASTVIGHALDT                          155
             :|||||||||:|||||||||||| ||||||:||||
orf66ng      LGQILDIFVFDKLRRLKAWWIAPAASTVIGNALDTLVFFAVAFYASSDEFMAANWQGIAF  180
```

The complete length ORF66ng nucleotide sequence <SEQ ID 265> is:

```
 1  ATGTACGCAT TGACCGCCGC ACAGCAACAG AAGGCACTCT TCCGGCTGGT
51  GCTTTTCCAT ATCCTCATCA TCGCCGCCAG CAACTATCTG GTGCAGTTCC
```

```
101  CCTTCCGGAT TTTCGGCATC CACACCACTT GGGGCGCGTT TTCCTTTCCC
151  TTCATCTTCC TCGCCACCGA CCTGACCGTC CGCATTTTCG GTTCGCACTT
201  GGCGCGGCGG ATTATCTTTT GGGTGATGTT CCCCGCCCTT ttgCTTTcat
251  aCGTCTTTTC CGTTTTGTTC CACAACGGCA GTTGGACGGG CTTGGGCGCG
301  ctgTCCCAAT TCAACACCTT TGTCGGACGC ATCGCGCTGG CAAGTTTTGC
351  CGCCTACGCG CTCGGACAAA TCCTTGATAT TTTCGTATTC GACAAATTAC
401  GCCGTCTGAA AGCGTGGTGG ATTGCCCCGG CCGCATCAAC CGTCATCGGC
451  AATGCACTGG ACACGTTAGT ATTTTTTGCC GTTGCCTTTT ACGCAAGCAG
501  CGATGAATTT ATGGCGGCAA ACTGGCAGGG CATCGCTTTT GTCGATTACC
551  TGTTCAAACT TACCGTCTGC ACCCTCTTCT TCCTGCCCGC CTACGGCGTG
601  ATACTGAATC TGCTGACGAA AAAACTGACG GCCCTGCAAA CCAAACAGGC
651  GCAAGACCGC CCCGTGCCCT CGCTGCAAAA TCCGTAA
```

This encodes a protein having amino acid sequence <SEQ ID 266>:

```
  1  MYALTAAQQQ KALFRLVLFH ILIIAASNYL VQFPFRIFGI HTTWGAFSFP
 51  FIFLATDLTV RIFGSHLARR IIFWVMFPAL SLSYVFSVLF HNGSWTGLGA
101  PSQFNTFVGR IALASFAAYA LGQILDIFVF DKLRRLKAWW IAPAASTVIG
151  NALDTLVFFA VAFYASSDEF MAANWQGIAF VDYLFKLTVC TLFFLPAYGV
201  ILNLLTKKLT ALQTKQAQDR PVPSLQNP*
```

An alternative annotated sequence is:

```
  1  MYALTAAQQQ KALFRLVLFH ILIIAASNYL VQFPFRIFGI HTTWGAFSFP
 51  FIFLATDLTV RIFGSHLARR IIFWVMFPAL LLSYVFSVLF HNGSWTGLGA
101  LSQFNTFVGR IALASFAAYA LGQILDIFVF DKLRRLKAWW IAPAASTVIG
151  NALDTLVFFA VAFYASSDEF MAANWQGIAF VDYLFKLTVC TLFFLPAYGV
201  ILNLLTKKLT ALQTKQAQDR PVPSLQNP*
```

ORF66ng and ORF66-1 show 96.1% identity in 228 aa overlap:

```
orf66-1.pep  MYAFTAAQQQKALFRLVLFHILIIAASNYLVQFPFQIFGIHTTWGAFSFPFIFLATDLTV   60
             |||:|||||||||||||||||||||||||||||||:||||||||||||||||||||||||
orf66ng      MYALTAAQQQKALFRLVLFHILIIAASNYLVQFPFRIFGIHTTWGAFSFPFIFLATDLTV   60

orf66-1.pep  RIFGSHLARRIIFWVMFPALLLSYVFSVLFHNGSWTGLGALSEFNTFVGRIALASFAAYA  120
             ||||||||||||||||||||||||||||||||||||||||||:|||||||||||||||||
orf66ng      RIFGSHLARRIIFWVMFPALLLSYVFSVLFHNGSWTGLGALSQFNTFVGRIALASFAAYA  120

orf66-1.pep  IGQILDIFVFNKLRRLKAWWIAPTASTVIGNALDTLVFFAVAFYASSDGFMAANWQGIAF  180
             :|||||||||:||||||||||||:|||||||||||||||||||||||| |||||||||||
orf66ng      LGQILDIFVFDKLRRLKAWWIAPAASTVIGNALDTLVFFAVAFYASSDEFMAANWQGIAF  180

orf66-1.pep  VDYLFKLTVCTLFFLPAYGVILNLLTKKLTTLQTKQAQDRPAPSLQNPX    229
             ||||||||||||||||||||||||||||||:||||||||||:|||||||
orf66ng      VDYLFKLTVCTLFFLPAYGVILNLLTKKLTALQTKQAQDRPVPSLQNPX    229
```

Furthermore, ORF66ng shows significant homology with an *E.coli* ORF:

```
 sp|P37619|YHHQ_ECOLI HYPOTHETICAL 25.3 KD PROTEIN IN FTSY-NIKA INTERGENIC
REGION (O221)
>gi|1073495|pir||S47690 hypothetical protein o221 - Escherichia coli >gi|466607
(U00039) No definition line found [Escherichia coli] >gi|1789882 (AE000423)
hypothetical 25.3 kD protein in ftsY-nikA intergenic region [Escherichia coli]
Length = 221
 Score =  273 bits (692), Expect = 5e-73
 Identities = 132/203 (65%), Positives = 155/203 (76%)

Query: 1   MYALTAAQQQKALFRLVLFHILIIAASNYLVQFPFRIFGIHTTWGAFSFPFIFLATDLTV 60
            M   +  Q+ KALF L LFH+L+I +SNYLVQ P  I G HTTWGAFSFPFIFLATDLTV
Sbjct: 1   MNVFSQTQRYKALFWLSLFHLLVITSSNYLVQLPVSILGFHTTWGAFSFPFIFLATDLTV 60

Query: 61  RIFGSHLARRIIFWVMFPALLLSYVFSVLFHNGSWTGLGALSQFNTFVGRIALASFAAYA 120
            RIFG+ LARRIIF VM PALL+SYV S LF+ GSW G GAL+ FN FV RIA ASF AYA
Sbjct: 61  RIFGAPLARRIIFAVMIPALLISYVISSLFYMGSWQGFGALAHFNLFVARIATASFMAYA 120

Query: 121 LGQILDIFVFDKLRRLKAWWIAPAASTVIGNALDTLVFFAVAFYASSDEFMAANWQGIAF 180
            LGQILD+ VF++LR+ + WW+AP AST+ GN  DTL FF +AF+ S D FMA +W  IA
Sbjct: 121 LGQILDVHVFNRLRQSRRWWLAPTASTLFGNVSDTLAFFFIAFWRSPDAFMAEHWMEIAL 180

Query: 181 VDYLFKLTVCTLFFLPAYGVILN 203

                       VDY FK+ +  +FFLP YGV+LN
            Sbjct: 181 VDYCFKVLISIVFFLPMYGVLLN 203
```

Based on this analysis, including the homology with the *E.coli* protein and the presence of several putative transmembrane domains in the gonococcal protein, it is predicted that these proteins from

*N.meningitidis* and *N.gonorrhoeae,* and their epitopes, could be useful antigens for vaccines or diagnostics, or for raising antibodies.

**Example 32**

The following partial DNA sequence was identified in *N.meningitidis* <SEQ ID 267>:

```
  1 ATGGTCATAA AATATACAAA TTTGAATTTT GCGAAATTGT CGATAATTGC
 51 AATTTTGATG ATGTATTCGT TTGAAGCGAA TGCAAAyGCA GTmwrAATAT
101 CTGAAACTGT TTCAGTTGAT ACCGGACAAG GTGCGAAAAT TCATAAGTTT
151 GTACCTAAAA ATAGTAAAAC TTATTCATCT GATTTAATAA AAACGGTAGA
201 TTTAACACAC AyyCCTACGG GCGCAAAAGC CCGAATCAAC GCCAAAATAA
251 CCGCCAGCGT ATCCCGCGCC GGCGTATTGG CGGGGGTCGG CAAACTTGCC
301 CGCTTAGgCG CGAAATTCAG CACAAGGGCG GTtCCCTATG TCGGAACAGC
351 CcTTTTAGCC CACGACGTAT ACGAAAcTTT CAAAGAAGAC ATACAGGCAC
401 GAGGCTACCA ATACGACCCC GAAACCGACA AATTTGTAAA AGGCTACGAA
451 TATAGTAATT GCCTTTGGTA CGAAGACAAA AGACGTATTA ATAGAACCTA
501 TGGCTGCTAC GGCGTTGAT..
```

This corresponds to the amino acid sequence <SEQ ID 268; ORF72>:

```
  1 MVIKYTNLNF AKLSIIAILM MYSFEANANA VXISETVSVD TGQGAKIHKF
 51 VPKNSKTYSS DLIKTVDLTH XPTGAKARIN AKITASVSRA GVLAGVGKLA
101 RLGAKFSTRA VPYVGTALLA HDVYETFKED IQARGYQYDP ETDKFVKGYE
151 YSNCLWYEDK RRINRTYGCY GVD..
```

Further work revealed the complete nucleotide sequence <SEQ ID 269>:

```
  1  ATGGTCATAA AATATACAAA TTTGAATTTT GCGAAATTGT CGATAATTGC
 51  AATTTTGATG ATGTATTCGT TTGAAGCGAA TGCAAATGCA GTAAAAATAT
101  CTGAAACTGT TTCAGTTGAT ACCGGACAAG GTGCGAAAAT TCATAAGTTT
151  GTACCTAAAA ATAGTAAAAC TTATTCATCT GATTTAATAA AAACGGTAGA
201  TTTAACACAC ATCCCTACGG GCGCAAAAGC CCGAATCAAC GCCAAAATAA
251  CCGCCAGCGT ATCCCGCGCC GGCGTATTGG CGGGGGTCGG CAAACTTGCC
301  CGCTTAGGCG CGAAATTCAG CACAAGGGCG GTTCCCTATG TCGGAACAGC
351  CCTTTTAGCC CACGACGTAT ACGAAACTTT CAAAGAAGAC ATACAGGCAC
401  GAGGCTACCA ATACGACCCC GAAACCGACA AATTTGCAAA GGTCTCAGGC
451  TAA
```

This corresponds to the amino acid sequence <SEQ ID 270; ORF72-1>:

```
  1  MVIKYTNLNF AKLSIIAILM MYSFEANANA VKISETVSVD TGQGAKIHKF
 51  VPKNSKTYSS DLIKTVDLTH IPTGAKARIN AKITASVSRA GVLAGVGKLA
101  RLGAKFSTRA VPYVGTALLA HDVYETFKED IQARGYQYDP ETDKFAKVSG
151  *
```

Computer analysis of this amino acid sequence gave the following results:

<u>Homology with a predicted ORF from *N.meningitidis* (strain A)</u>

ORF72 shows 98.0% identity over a 147aa overlap with an ORF (ORF72a) from strain A of *N. meningitidis:*

```
                     10        20        30        40        50        60
orf72.pep    MVIKYTNLNFAKLSIIAILMMYSFEANANAVXISETVSVDTGQGAKIHKFVPKNSKTYSS
             |||||||||||||||||||||||||||||| |||||||||||||||||||||||||||||
orf72a       MVIKYTNLNFAKLSIIAILMMYSFEANANAVKISETVSVDTGQGAKIHKFVPKNSKTYSS
                     10        20        30        40        50        60

                     70        80        90       100       110       120
orf72.pep    DLIKTVDLTHXPTGAKARINAKITASVSRAGVLAGVGKLARLGAKFSTRAVPYVGTALLA
             |||||||||| |||||||||||||||||||||||||||||||||||||||||||||||||
orf72a       DLIKTVDLTHIPTGAKARINAKITASVSRAGVLAGVGKLARLGAKFSTRAVPYVGTALLA
                     70        80        90       100       110       120

                    130       140       150       160       170
orf72.pep    HDVYETFKEDIQARGYQYDPETDKFVKGYEYSNCLWYEDKRRINRTYGCYGVD
             |||||||||||||||||||||||||:|
orf72a       HDVYETFKEDIQARGYQYDPETDKFAKVSGX
                    130       140       150
```

The complete length ORF72a nucleotide sequence <SEQ ID 271> is:

```
  1  ATGGTCATAA AATATACAAA TTTGAATTTT GCGAAATTGT CGATAATTGC
 51  AATTTTGATG ATGTATTCGT TTGAAGCGAA TGCAAATGCA GTAAAAATAT
101  CTGAAACTGT TTCAGTTGAT ACCGGACAAG GTGCGAAAAT TCATAAGTTT
151  GTACCTAAAA ATAGTAAAAC TTATTCATCT GATTTAATAA AAACGGTAGA
201  TTTAACACAC ATCCCTACGG GCGCAAAAGC CCGAATCAAC GCCAAAATAA
251  CCGCCAGCGT ATCCCGCGCC GGCGTATTGG CGGGGGTCGG CAAACTTGCC
301  CGCTTAGGCG CGAAATTCAG CACAAGGGCG GTTCCCTATG TCGGAACAGC
351  CCTTTTAGCC CACGACGTAT ACGAAACTTT CAAAGAAGAC ATACAGGCAC
401  GAGGCTACCA ATACGACCCC GAAACCGACA AATTTGCAAA GGTCTCAGGC
451  TAA
```

This encodes a protein having amino acid sequence <SEQ ID 272>:

```
  1  MVIKYTNLNF AKLSIIAILM MYSFEANANA VKISETVSVD TGQGAKIHKF
 51  VPKNSKTYSS DLIKTVDLTH IPTGAKARIN AKITASVSRA GVLAGVGKLA
101  RLGAKFSTRA VPYVGTALLA HDVYETFKED IQARGYQYDP ETDKFAKVSG
151  *
```

ORF72a and ORF72-1 show 100.0% identity in 150 aa overlap:

```
                      10        20        30        40        50        60
orf72a.pep   MVIKYTNLNFAKLSIIAILMMYSFEANANAVKISETVSVDTGQGAKIHKFVPKNSKTYSS
             ||||||||||||||||||||||||||||||||||||||||||||||||||||||||||||
orf72-1      MVIKYTNLNFAKLSIIAILMMYSFEANANAVKISETVSVDTGQGAKIHKFVPKNSKTYSS
                      10        20        30        40        50        60

                      70        80        90       100       110       120
orf72a.pep   DLIKTVDLTHIPTGAKARINAKITASVSRAGVLAGVGKLARLGAKFSTRAVPYVGTALLA
             ||||||||||||||||||||||||||||||||||||||||||||||||||||||||||||
orf72-1      DLIKTVDLTHIPTGAKARINAKITASVSRAGVLAGVGKLARLGAKFSTRAVPYVGTALLA
                      70        80        90       100       110       120

                     130       140       150
orf72a.pep   HDVYETFKEDIQARGYQYDPETDKFAKVSGX
             |||||||||||||||||||||||||||||||
orf72-1      HDVYETFKEDIQARGYQYDPETDKFAKVSGX
                     130       140       150
```

Homology with a predicted ORF from *N.gonorrhoeae*

ORF72 shows 89% identity over a 173aa overlap with a predicted ORF (ORF72.ng) from *N. gonorrhoeae:*

```
orf72.pep   MVIKYTNLNFAKLSIIAILMMYSFEANANAVXISETVSVDTGQGAKIHKFVPKNSKTYSS   60
            || |:|||||||||||||||||||||||||| ||||:|||||||||:||||||:|: |||
orf72ng     MVTKHTNLNFAKLSIIAILMMYSFEANANAVKISETLSVDTGQGAKVHKFVPKSSNIYSS   60
orf72.pep   DLIKTVDLTHXPTGAKARINAKITASVSRAGVLAGVGKLARLGAKFSTRAVPYVGTALLA  120
            || |:||||| ||||||||||||||||||||||:|||||:| ||||:|||||||||||||
orf72ng     DLTKAVDLTHIPTGAKARINAKITASVSRAGVLSGVGKLVRQGAKFGTRAVPYVGTALLA  120
orf72.pep   HDVYETFKEDIQARGYQYDPETDKFVKGYEYSNCLWYEDKRRINRTYGCYGVD        173
            ||||||||||||||| :||||||||||||||:|||||||:|||||||||||||
orf72ng     HDVYETFKEDIQARGCRYDPETDKFVKGYEYANCLWYEDERRINRTYGCYGVDSSIMRLM  180
```

An ORF72ng nucleotide sequence <SEQ ID 273> was predicted to encode a protein having amino acid sequence <SEQ ID 274>:

```
  1  MVTKHTNLNF AKLSIIAILM MYSFEANANA VKISETLSVD TGQGAKVHKF
 51  VPKSSNIYSS DLTKAVDLTH IPTGAKARIN AKITASVSRA GVLSGVGKLV
101  RQGAKFGTRA VPYVGTALLA HDVYETFKED IQARGCRYDP ETDKFVKGYE
151  YANCLWYEDE RRINRTYGCY GVDSSIMRLM PDRSRFPEVK QLMESQMYRL
201  ARPFWNWRKE ELNKLSSLDW NNFVLNRCTF DWNGGGCAVN KGDDFRAGAS
251  FSLGRNPKYK EEMDAKKPEE ILSLKVDADP DKYIEATGYP GYSEKVEVAP
301  GTKVNMGPVT DRNGNPVQVA ATFGRDAQGN TTADVQVIPR PDLTPASAEA
351  PHAQPLPEVS PAENPANNPD PDENPGTRPN PEPDPDLNPD ANPDTDGQPG
401  TSPDSPAVPD RPNGRHRKER KEGEDGGLSC DYFPEILACQ EMGKPSDRMF
451  HDISIPQVTD DKTWSSHNFL PSNGVCPQPK TFHVFGRQYR ASYEPLCVFA
501  EKIRFAVLLA FIIMSAFVVF GSLGGE*
```

After further analysis, the following gonococcal DNA sequence <SEQ ID 275> was identified:

```
  1  ATGGTCACAA AACATACAAA TTTGAATTTT GCGAAATTGT CGATAATTGC
 51  AATTTTGATG ATGTATTCGT TTGAAGCGAA TGCAAATGCA GTAAAAATAT
101  CTGAAACTCT TTCGGTTGAT ACCGGACAAG GCGCGAAAGT TCATAAGTTC
151  GTTCCTAAAT CAAGTAATAT TTATTCATCT GATTTAACAA AAGCGGTAGA
201  TTTAACGCAT ATCCCCACGG GCGCAAAAGC CCGAATCAAC GCCAAAATAA
251  CCGCCAGCGT ATCCCGCGCC GGCGTATTGT CGGGGGTCGG CAAACTTGTC
301  CGCCAAGGCG CGAAATTCGG CACAAGGGCG GTTCCCTATG TCGGAACAGC
351  CCTTTTAGCC CACGACGTAT ACGAAACTTT CAAAGAAGAC ATACAGGCAC
401  GAGGCTGCCG ATACGATCCC GAAACCGACA AATTT
```

This corresponds to the amino acid sequence <SEQ ID 276; ORF72ng-1>:

```
  1  MVTKHTNLNF AKLSIIAILM MYSFEANANA VKISETLSVD TGQGAKVHKF
 51  VPKSSNIYSS DLTKAVDLTH IPTGAKARIN AKITASVSRA GVLSGVGKLV
101  RQGAKFGTRA VPYVGTALLA HDVYETFKED IQARGCRYDP ETDKF
```

ORF72ng-1 and ORF721-1 show 89.7% identity in 145 aa overlap:

```
                       10        20        30        40        50        60
orf72ng-1.pe   MVTKHTNLNFAKLSIIAILMMYSFEANANAVKISETLSVDTGQGAKVHKFVPKSSNIYSS
               || |:|||||||||||||||||||||||||||||||:||||||||:||||||:|: |||
orf72-1        MVIKYTNLNFAKLSIIAILMMYSFEANANAVKISETVSVDTGQGAKIHKFVPKNSKTYSS
                       10        20        30        40        50        60

                       70        80        90       100       110       120
orf72ng-1.pe   DLTKAVDLTHIPTGAKARINAKITASVSRAGVLSGVGKLVRQGAKFGTRAVPYVGTALLA
               || |:|||||||||||||||||||||||||||||:|||||:| ||||:|||||||||||||
orf72-1        DLIKTVDLTHIPTGAKARINAKITASVSRAGVLAGVGKLARLGAKFSTRAVPYVGTALLA
                       70        80        90       100       110       120

                      130       140
orf72ng-1.pe   HDVYETFKEDIQARGCRYDPETDKF
               ||||||||||||||| :||||||||
orf72-1        HDVYETFKEDIQARGYQYDPETDKFAKVSGX
                      130       140       150
```

Based on this analysis, including the presence of a putative leader sequence and transmembrane domains in the gonococcal protein, it is predicted that the proteins from *N.meningitidis* and *N.gonorrhoeae,* and their epitopes, could be useful antigens for vaccines or diagnostics, or for raising antibodies.

**Example 33**

The following partial DNA sequence was identified in *N.meningitidis* <SEQ ID 277>:

```
  1  ATGAGATTTT TCGGTATCGG TTTTTTGGTG CTGCTGTTTT TGGAGATTAT

 51  GTCGATTGTG TGGGTTGCCG ATTGGCTGGG CGGCGGCTGG ACGTTGTTTT
101  TGATGGCGGC AGGTTTTGCC GCCGGCGTGC TGATGCTCAG GCAAACCGGG
151  GCTGACCGGT CTTTTATTGG CGGGCGCGGC AATGAGAAGC GGCGGGAAGG
201  TATCCGTTTA TCAGATGTTG TGGCCTATC..
```

This corresponds to the amino acid sequence <SEQ ID 278; ORF73>:

```
  1  MRFFGIGFLV LLFLEIMSIV WVADWLGGGW TLFLMAAGFA AGVLMLRQTG
 51  LTGLLLAGAA MRSGGKVSVY QMLWPI..
```

Further work revealed the complete nucleotide sequence <SEQ ID 279>:

```
  1  ATGAGATTTT TCGGTATCGG TTTTTTGGTG CTGCTGTTTT TGGAGATTAT
 51  GTCGATTGTG TGGGTTGCCG ATTGGCTGGG CGGCGGCTGG ACGTTGTTTT
101  TGATGGCGGC AGGTTTTGCC GCCGGCGTGC TGATGCTCAG GCATACGGGG
151  CTGTCCGGTC TTTTATTGGC GGGCGCGGCA ATGAGAAGCG GCGGGAGGGT
201  ATCCGTTTAT CAGATGTTGT GGCCTATCCG TTATACGGTG GCGGCTGTGT
251  GTCTGATGAG TCCGGGATTC GTATCCTCGG TGTTGGCGGT ATTGCTGCTG
301  CTGCCGTTTA AGGGAGGGGC AGTGTTGCAG GCAGGAGGTG CGGAAAATTT
351  TTTCAACATG AACCAATCGG GCAGAAAAGA GGGCTTTTCC CGCGATGACG
401  ATATTATCGA GGGAGAATAT ACGGTTGAAG AGCCTTACGG CGGCAATCGT
451  TCCCGAAACG CCATCGAACA CAAAAAAGAC GAATAA
```

This corresponds to the amino acid sequence <SEQ ID 280; ORF73-1>:

```
  1  MRFFGIGFLV LLFLEIMSIV WVADWLGGGW TLFLMAAGFA AGVLMLRHTG
 51  LSGLLLAGAA MRSGGRVSVY QMLWPIRYTV AAVCLMSPGF VSSVLAVLLL
101  LPFKGGAVLQ AGGAENFFNM NQSGRKEGFS RDDDIIEGEY TVEEPYGGNR
151  SRNAIEHKKD E*
```

Computer analysis of this amino acid sequence gave the following results:

Homology with a predicted ORF from *N.meningitidis* (strain A)

ORF73 shows 90.8% identity over a 76aa overlap with an ORF (ORF73a) from strain A of *N. meningitidis:*

```
                    10        20        30        40        50        60
orf73.pep   MRFFGIGFLVLLFLEIMSIVWVADWLGGGWTLFLMAAGFAAGVLMLRQTGLTGLLLAGAA
            |||||||||||||||||||||||||||||||||||||| |||||:|||:|||:|||||||
orf73a      MRFFGIGFLVLLFLEIMSIVWVADWLGGGWTLFLMAATFAAGVVMLRHTGLSGLLLAGAA
                    10        20        30        40        50        60

                    70
orf73.pep   MRSGGKVSVYQMLWPI
            |||||:|||| ||| |
orf73a      MRSGGRVSVYXMLWXIRYTVAAVCXMSPGFVSSVXAVLLXLPFKGGAVLQAGGAENFFNM
```

The complete length ORF73a nucleotide sequence <SEQ ID 281> is:

```
  1  ATGAGATTTT TCGGTATCGG TTTTTTGGTG CTGCTGTTTT TGGAGATTAT
 51  GTCGATTGTG TGGGTTGCCG ATTGGTTGGG CGGCGGTTGG ACGCTGTTTC
101  TAATGGCGGC AACCTTTGCC GCCGGCGTGG TGATGCTCAG GCATACGGGG
151  CTGTCCGGTC TTTTATTGGC GGGCGCGGCA ATGAGAAGCG GCGGGAGGGT
201  ATCCGTTTAT CANATGTTGT GGCNTATCCG TTATACGGTG GCGGCGGTGT
251  GTCNGATGAG TCCGGGATTC GTATCCTCGG TGTNGGCGGT ATTGCTGNTG
301  CTNCCGTTTA AGGGAGGTGC AGTGTTGCAG GCAGGAGGTG CGGAAAATTT
351  TTTCAACATG AACCANTCGG GCAGAAAAGA NGGCNTTTCC CGCGATGACG
401  ATATTATCGA GGGGGAATAT ACGGTTGAAG ANCCTTACGG CGGCANTCGT
451  TTCCGAAACG CCNTNGAACA CAAAAAAGAC GAATAA
```

This encodes a protein having amino acid sequence <SEQ ID 282>:

```
  1   MRFFGIGFLV LLFLEIMSIV WVADWLGGGW TLFLMAATFA AGVVMLRHTG
 51   LSGLLLAGAA MRSGGRVSVY XMLWXIRYTV AAVCXMSPGF VSSVXAVLLX
101   LPFKGGAVLQ AGGAENFFNM NXSGRKXGXS RDDDIIEGEY TVEXPYGGXR
151   FRNAXEHKKD E*
```

ORF73a and ORF73-1 show 91.3% identity in 161 aa overlap

```
                     10        20        30        40        50        60
orf73a.pep   MRFFGIGFLVLLFLEIMSIVWVADWLGGGWTLFLMAATFAAGVVMLRHTGLSGLLLAGAA
             ||||||||||||||||||||||||||||||||||||| |||||:||||||||||||||||
orf73-1      MRFFGIGFLVLLFLEIMSIVWVADWLGGGWTLFLMAAGFAAGVLMLRHTGLSGLLLAGAA
                     10        20        30        40        50        60

                     70        80        90       100       110       120
orf73a.pep   MRSGGRVSVYXMLWXIRYTVAAVCXMSPGFVSSVXAVLLXLPFKGGAVLQAGGAENFFNM
             |||||||||| ||| ||||||||| ||||||||| |||| ||||||||||||||||||||
orf73-1      MRSGGRVSVYQMLWPIRYTVAAVCLMSPGFVSSVLAVLLLLPFKGGAVLQAGGAENFFNM
                     70        80        90       100       110       120

                    130       140       150       160
orf73a.pep   NXSGRKXGXSRDDDIIEGEYTVEXPYGGXRFRNAXEHKKDEX
             | |||| | |||||||||||||| |||| | ||| |||||||
orf73-1      NQSGRKEGFSRDDDIIEGEYTVEEPYGGNRSRNAIEHKKDEX
                    130       140       150       160
```

## Homology with a predicted ORF from *N.gonorrhoeae*

ORF73 shows 92.1 % identity over a 76aa overlap with a predicted ORF (ORF73.ng) from *N. gonorrhoeae:*

```
orf73.pep    MRFFGIGFLVLLFLEIMSIVWVADWLGGGWTLFLMAAGFAAGVLMLRQTGLTGLLLAGAA    60
             ||||||||||||||||||||||||||||||||||||| |||||||||:|||:||||||||
orf73ng      MRFFGIGFLVLLFLEIMSIVWVADWLGGGWTLFLMAATFAAGVLMLRHTGLSGLLLAGAA    60

orf73.pep    MRSGGKVSVYQMLWPI                                                76
             ::|:||||||||||||
orf73ng      VKSSGKVSVYQMLWPIRYTVAAVCLMSPGFVSSVLAVLLLLPFKGGAVLQAGGAENFFNM   120
```

The complete length ORF73ng nucleotide sequence <SEQ ID 283> is:

```
  1   ATGAGATTTT TCGGTATCGG TTTTTTGGTG CTGCTGTTTT TGGAAATTAT
 51   GTCGATTGTG TGGGTTGCCG ATTGGCTGGG CGGCGGTTGG AcgcTGTTTC
101   TAATGGCGGC AACCTTTGCC GCCGGTGTGC TGATGCTCAG GCATAcggGG
151   CTGTCCGGTC TTTTATTGGC TGGCGCGGCG GTAAAAagta gtgGGAAGGT
201   ATCTGTTTAT CagatgtTGT GGCCTATCCG TTATAcggtg gcggcggtgT
251   GTCTGatgag tCcggGATTC GTATCCTccg tgttggCGGT ATTGCTGCTG
301   CTGCcgttta aggGaggGgc agtgttgcag gcaggaggtg cggaaaATTT
351   TTTCAACATg aaCcaatcgg gcagaaAaga gggatttttc cacgatgacg
401   atattatcga gggagaatat acggttgaaa aacctgacgg cggcaatcgt
451   tcccgaAAcg ccatcgaaca cgaaaAagac gaataA
```

This encodes a protein having amino acid sequence <SEQ ID 284>:

```
  1   MRFFGIGFLV LLFLEIMSIV WVADWLGGGW TLFLMAATFA AGVLMLRHTG
 51   LSGLLLAGAA VKSSGKVSVY QMLWPIRYTV AAVCLMSPGF VSSVLAVLLL
101   LPFKGGAVLQ AGGAENFFNM NQSGRKEGFF HDDDIIEGEY TVEKPDGGNR
151   SRNAIEHEKD E*
```

ORF73ng and ORG73-1 show 93.8% identity in 161 aa overlap

```
                      10        20        30        40        50        60
orf73-1.pep    MRFFGIGFLVLLFLEIMSIVWVADWLGGGWTLFLMAAGFAAGVLMLRHTGLSGLLLAGAA
               |||||||||||||||||||||||||||||||||||| |||||||||||||||||||||||
orf73ng        MRFFGIGFLVLLFLEIMSIVWVADWLGGGWTLFLMAATFAAGVLMLRHTGLSGLLLAGAA
                      10        20        30        40        50        60

                      70        80        90       100       110       120
orf73-1.pep    MRSGGRVSVYQMLWPIRYTVAAVCLMSPGFVSSVLAVLLLPFKGGAVLQAGGAENFFNM
               ::|:|:||||||||||||||||||||||||||||||||||||||||||||||||||||||
orf73ng        VKSSGKVSVYQMLWPIRYTVAAVCLMSPGFVSSVLAVLLLPFKGGAVLQAGGAENFFNM
                      70        80        90       100       110       120

                     130       140       150       160
orf73-1.pep    NQSGRKEGFSRDDDIIEGEYTVEEPYGGNRSRNAIEHKKDEX
               ||||||||| :||||||||||||:| |||||||||||:||||
orf73ng        NQSGRKEGFFHDDDIIEGEYTVEKPDGGNRSRNAIEHEKDEX
                     130       140       150       160
```

Based on this analysis, including the presence of a putative leader sequence and putative transmembrane domain in the gonococcal protein, it is predicted that the proteins from

*N.meningitidis* and *N.gonorrhoeae,* and their epitopes, could be useful antigens for vaccines or diagnostics, or for raising antibodies.

**Example 34**

The following partial DNA sequence was identified in *N.meningitidis* <SEQ ID 285>:

```
  1 ATGTTTGTTT TTCAGACGGC ATTCTT.ATG TTTCAGAAAC ATTTGCAGAA
 51 AGCCTCCGAC AGCGTCGTCG GAGGGACATT ATACGTGGTT GCCACGCCCA
101 TCGGCAATTT GGCGGACATT ACCCTGCGCG CTTTGGCGGT ATTGCAAAAG
151 GCG....... .....GCCGA AGACACGCGC GTTACCGCAC AGCTTTTGAG
201 CGCGTACGGC ATTCAGGGCA AACTCGTCAG TGTGCGCGAA CACAACGAAC
251 GGCAGATGGC GGACAAGATT GTCGGCTATC TTTCAGACGG CATGGTTGTG
301 GCACAGGTTT CCGATGCGGG TACGCCGGCC GTGTGCGACC CGGGCGCGAA
351 ACTCGCCCGC CGCGTGCGTG AGGCCGGGTT TAAAGTCGTT CCCGTCGTGG
401 GCGCAAC.GC GGTGATGGCG GCTTTGAGCG TGGCCGGTGT GGAAGGATCC
451 GATTTTTATT TCAACGGTTT TGTACCGCCG AAATCGGGAG AACGCAGGAA
501 ACTGTTTGCC AAATGGGTGC GGGCGGCGTT TCCTATCGTC ATGTTTGAAA
551 CGCCGCACCG CATCGGTGCA GCGCTTGCCG ATATGGCGGA ACTGTTCCCC
601 GAACGCCGAT TAATGCTGGC GCGCGAAATT ACGAAAACGT TTGAAACGTT
651 CTTAAGCGGC ACGGTTGGGG AAATTCAGAC GGCATTGTCT GCCGACGGCG
701 ACCAATCGCG CGGCGAGATG GTGTTGGTGC TTTATCCGGC GCAGGATGAA
751 AAACACGAAG GCTTGTCCGA GTCCGCGCAA AACATCATGA AAATCCTCAC
801 AGCCGAGCTG CCGACCAAAC AGGCGGCGGA GCTTGCTGCC AAAATCACGG
851 GCGAGGGAAA GAAAGCTTTG TACGAT..
```

This corresponds to the amino acid sequence <SEQ ID 286; ORF75>:

```
  1 MFVFQTAFXM FQKHLQKASD SVVGGTLYVV ATPIGNLADI TLRALAVLQK
 51 A....AEDTR VTAQLLSAYG IQGKLVSVRE HNERQMADKI VGYLSDGMVV
101 AQVSDAGTPA VCDPGAKLAR RVREAGFKVV PVVGAXAVMA ALSVAGVEGS
151 DFYFNGFVPP KSGERRKLFA KWVRAAFPIV MFETPHRIGA ALADMAELFP
201 ERRLMLAREI TKTFETFLSG TVGEIQTALS ADGDQSRGEM VLVLYPAQDE
251 KHEGLSESAQ NIMKILTAEL PTKQAAELAA KITGEGKKAL YD..
```

Further work revealed the complete nucleotide sequence <SEQ ID 287>:

```
  1  ATGTTTCAGA AACATTTGCA GAAAGCCTCC GACAGCGTCG TCGGAGGGAC
 51  ATTATACGTG GTTGCCACGC CCATCGGCAA TTTGGCGGAC ATTACCCTGC
101  GCGCTTTGGC GGTATTGCAA AAGGCGGACA TCATCTGTGC CGAAGACACG
151  CGCGTTACCG CACAGCTTTT GAGCGCGTAC GGCATTCAGG GCAAACTCGT
201  CAGTGTGCGC GAACACAACG AACGGCAGAT GGCGGACAAG ATTGTCGGCT
251  ATCTTTCAGA CGGCATGGTT GTGGCACAGG TTTCCGATGC GGGTACGCCG
301  GCCGTGTGCG ACCCGGGCGC GAAACTCGCC CGCCGCGTGC GTGAGGCCGG
351  GTTTAAAGTC GTTCCCGTCG TGGGCGCAAG CGCGGTGATG GCGGCTTTGA
401  GCGTGGCCGG TGTGGAAGGA TCCGATTTTT ATTTCAACGG TTTTGTACCG
451  CCGAAATCGG GAGAACGCAG GAAACTGTTT GCCAAATGGG TGCGGGCGGC
501  GTTTCCTATC GTCATGTTTG AAACGCCGCA CCGCATCGGT GCGACGCTTG
551  CCGATATGGC GGAACTGTTC CCCGAACGCC GATTAATGCT GGCGCGCGAA
601  ATTACGAAAA CGTTTGAAAC GTTCTTAAGC GGCACGGTTG GGGAAATTCA
651  GACGGCATTG TCTGCCGACG GCAACCAATC GCGCGGCGAG ATGGTGTTGG
701  TGCTTTATCC GGCGCAGGAT GAAAAACACG AAGGCTTGTC CGAGTCCGCG
751  CAAAACATCA TGAAAATCCT CACAGCCGAG CTGCCGACCA AACAGGCGGC
801  GGAGCTTGCT GCCAAAATCA CGGGCGAGGG AAAGAAAGCT TTGTACGATC
851  TGGCTCTGTC TTGGAAAAAC AAATAG
```

This corresponds to the amino acid sequence <SEQ ID 288; ORF75-1>:

```
  1  MFQKHLQKAS DSVVGGTLYV VATPIGNLAD ITLRALAVLQ KADIICAEDT
 51  RVTAQLLSAY GIQGKLVSVR EHNERQMADK IVGYLSDGMV VAQVSDAGTP
101  AVCDPGAKLA RRVREAGFKV VPVVGASAVM AALSVAGVEG SDFYFNGFVP
151  PKSGERRKLF AKWVRAAFPI VMFETPHRIG ATLADMAELF PERRLMLARE
201  ITKTFETFLS GTVGEIQTAL SADGNQSRGE MVLVLYPAQD EKHEGLSESA
251  QNIMKILTAE LPTKQAAELA AKITGEGKKA LYDLALSWKN K*
```

Computer analysis of this amino acid sequence gave the following results:

Homology with a predicted ORF from *N.meningitidis* (strain A)

ORF75 shows 95.8% identity over a 283aa overlap with an ORF (ORF75a) from strain A of *N. meningitidis:*

```
                        10        20        30        40        50        60
orf75.pep     MFVFQTAFXMFQKHLQKASDSVVGGTLYVVATPIGNLADITLRALAVLQKAXXXXAEDTR
                       ||||||||||||||||||||||||||||||||||||||||||||||    |||||
orf75a                 MFQKHLQKASDSVVGGTLYVVATPIGNLADITLRALAVLQKADIICAEDTR
                               10        20        30        40        50

                        70        80        90       100       110       120
orf75.pep     VTAQLLSAYGIQGKLVSVREHNERQMADKIVGYLSDGMVVAQVSDAGTPAVCDPGAKLAR
              ||||||||||||||||||||||||||||||||||||||||||||||||||||||||||||
orf75a        VTAQLLSAYGIQGKLVSVREHNERQMADKIVGYLSDGMVVAQVSDAGTPAVCDPGAKLAR
                      60        70        80        90       100       110

                       130       140       150       160       170       180
orf75.pep     RVREAGFKVVPVVGAXAVMAALSVAGVEGSDFYFNGFVPPKSGERRKLFAKWVRAAFPIV
              ||||:||||||||| |||||||||| ||||||||||||||||||||||||||||:|||:|
orf75a        RVREVGFKVVPVVGASAVMAALSVAGVAGSDFYFNGFVPPKSGERRKLFAKWVRVAFPVV
                     120       130       140       150       160       170

                       190       200       210       220       230       240
orf75.pep     MFETPHRIGAALADMAELFPERRLMLAREITKTFETFLSGTVGEIQTALSADGDQSRGEM
              |||||||||:||||||||||||||||||||||||||||||||||||||:|||:|||||||
orf75a        MFETPHRIGATLADMAELFPERRLMLAREITKTFETFLSGTVGEIQTALAADGNQSRGEM
                     180       190       200       210       220       230

                       250       260       270       280       290
orf75.pep     VLVLYPAQDEKHEGLSESAQNIMKILTAELPTKQAAELAAKITGEGKKALYD
              ||||||||||||||||||||||||||||||||||||||||||||||||||||
orf75a        VLVLYPAQDEKHEGLSESAQNIMKILTAELPTKQAAELAAKITGEGKKALYDLALSWKNK
                     240       250       260       270       280       290

orf75a        X
```

The complete length ORF75a nucleotide sequence <SEQ ID 289> is:

```
  1  ATGTTTCAGA AACATTTGCA GAAAGCCTCC GACAGCGTCG TCGGAGGGAC
 51  ATTATACGTG GTTGCCACGC CCATCGGCAA TTTGGCGGAC ATTACCCTGC
101  GCGCTTTGGC GGTATTGCAA AAGGCGGACA TCATCTGTGC CGAAGACACG
151  CGCGTTACCG CGCAGCTTTT GAGCGCGTAC GGCATTCAGG GCAAACTCGT
201  CAGCGTGCGC GAACACAACG AACGGCAGAT GGCGGACAAG ATTGTCGGCT
251  ATCTTTCAGA CGGCATGGTT GTGGCACAGG TTTCCGATGC GGGTACGCCG
301  GCCGTGTGCG ACCCGGGCGC GAAACTCGCC CGCCGCGTGC GTGAGGTCGG
351  GTTTAAAGTT GTCCCTGTTG TCGGCGCAAG CGCGGTGATG GCGGCTTTGA
401  GTGTGGCTGG TGTGGCGGGA TCCGATTTTT ATTTCAACGG TTTTGTACCG
451  CCGAAATCGG GCGAACGTAG GAAATTGTTT GCCAAATGGG TGCGGGTGGC
501  GTTTCCCGTC GTGATGTTTG AAACGCCGCA CCGCATCGGG GCGACGCTTG
551  CCGATATGGC GGAACTGTTC CCCGAACGCC GATTAATGCT GGCGCGCGAA
601  ATCACGAAAA CGTTTGAAAC GTTCTTAAGC GGCACGGTTG GGGAAATTCA
651  GACGGCATTG GCGGCGGACG GCAACCAATC GCGCGGCGAG ATGGTGTTGG
701  TGCTTTATCC GGCGCAGGAT GAAAAACACG AAGGCTTGTC CGAGTCCGCG
751  CAAAACATCA TGAAAATCCT CACAGCCGAG CTGCCGACCA AACAGGCGGC
801  GGAGCTTGCC GCCAAAATCA CGGGCGAGGG AAAAAAAGCT TTGTACGATC
851  TGGCACTGTC TTGGAAAAAC AAATGA
```

This encodes a protein having amino acid sequence <SEQ ID 290>:

```
  1  MFQKHLQKAS DSVVGGTLYV VATPIGNLAD ITLRALAVLQ KADIICAEDT
 51  RVTAQLLSAY GIQGKLVSVR EHNERQMADK IVGYLSDGMV VAQVSDAGTP
101  AVCDPGAKLA RRVREVGFKV VPVVGASAVM AALSVAGVAG SDFYFNGFVP
151  PKSGERRKLF AKWVRVAFPV VMFETPHRIG ATLADMAELF PERRLMLARE
201  ITKTFETFLS GTVGEIQTAL AADGNQSRGE MVLVLYPAQD EKHEGLSESA
251  QNIMKILTAE LPTKQAAELA AKITGEGKKA LYDLALSWKN K*
```

ORF75a and ORF75-1 show 98.3% identity in 291 aa overlap:

```
                    10        20        30        40        50        60
orf75a.pep  MFQKHLQKASDSVVGGTLYVVATPIGNLADITLRALAVLQKADIICAEDTRVTAQLLSAY
            ||||||||||||||||||||||||||||||||||||||||||||||||||||||||||||
orf75-1     MFQKHLQKASDSVVGGTLYVVATPIGNLADITLRALAVLQKADIICAEDTRVTAQLLSAY
                    10        20        30        40        50        60

                    70        80        90       100       110       120
orf75a.pep  GIQGKLVSVREHNERQMADKIVGYLSDGMVVAQVSDAGTPAVCDPGAKLARRVREVGFKV
            ||||||||||||||||||||||||||||||||||||||||||||||||||||||||:|||
orf75-1     GIQGKLVSVREHNERQMADKIVGYLSDGMVVAQVSDAGTPAVCDPGAKLARRVREAGFKV
                    70        80        90       100       110       120

                   130       140       150       160       170       180
orf75a.pep  VPVVGASAVMAALSVAGVAGSDFYFNGFVPPKSGERRKLFAKWVRVAFPVVMFETPHRIG
            |||||||||||||||||| ||||||||||||||||||||||||||:|||:||||||||||
orf75-1     VPVVGASAVMAALSVAGVEGSDFYFNGFVPPKSGERRKLFAKWVRAAFPIVMFETPHRIG
                   130       140       150       160       170       180

                   190       200       210       220       230       240
orf75a.pep m ATLADMAELFPERRLMLAREITKTFETFLSGTVGEIQTALAADGNQSRGEMVLVLYPAQD
            ||||||||||||||||||||||||||||||||||||||||:|||||||||||||||||||
orf75-1     ATLADMAELFPERRLMLAREITKTFETFLSGTVGEIQTALSADGNQSRGEMVLVLYPAQD
                   190       200       210       220       230       240

                   250       260       270       280       290
orf75a.pep  EKHEGLSESAQNIMKILTAELPTKQAAELAAKITGEGKKALYDLALSWKNKX
            ||||||||||||||||||||||||||||||||||||||||||||||||||||
orf75-1     EKHEGLSESAQNIMKILTAELPTKQAAELAAKITGEGKKALYDLALSWKNKX
                   250       260       270       280       290
```

Homology with a predicted ORF from *N.gonorrhoeae*

ORF75 shows 93.2% identity over a 292aa overlap with a predicted ORF (ORF75.ng) from *N. gonorrhoeae:*

```
orf75.pep   MFVFQTAFXMFQKHLQKASDSVVGGTLYVVATPIGNLADITLRALAVLQKA----AEDTR   56
            | |||||| ||||||||||||||||||||||||||||||||||||||||||    |||||
orf75ng     MSVFQTAFFMFQKHLQKASDSVVGGTLYVVATPIGNLADITLRALAVLQKADIICAEDTR   60

orf75.pep   VTAQLLSAYGIQGKLVSVREHNERQMADKIVGYLSDGMVVAQVSDAGTPAVCDPGAKLAR  116
            ||||||||||||:||||||||||||||||::|:||||:||||||||||||||||||||||
orf75ng     VTAQLLSAYGIQGRLVSVREHNERQMADKVIGFLSDGLVVAQVSDAGTPAVCDPGAKLAR  120

orf75.pep   RVREAGFKVVPVVGAXAVMAALSVAGVEGSDFYFNGFVPPKSGERRKLFAKWVRAAFPIV  176
            |||||||||||||| |||||||||||  ||||||||||||||||||||||||||||||:|
orf75ng     RVREAGFKVVPVVGASAVMAALSVAGVAESDFYFNGFVPPKSGERRKLFAKWVRAAFPVV  180

orf75.pep   MFETPHRIGAALADMAELFPERRLMLAREITKTFETFLSGTVGEIQTALSADGDQSRGEM  236
            |||||||||:||||||||||||||||||||||||||||||||||||||||:|||:|||||
orf75ng     MFETPHRIGATLADMAELFPERRLMLAREITKTFETFLSGTVGEIQTALAADGNQSRGEM  240

orf75.pep   VLVLYPAQDEKHEGLSESAQNIMKILTAELPTKQAAELAAKITGEGKKALYD          288
            |||||||||||||||||||| ||||:||||||||||||||||||||||||||
orf75ng     VLVLYPAQDEKHEGLSESAQNAMKILAAELPTKQAAELAAKITGEGKKALYDLALSWKNK  300
```

An ORF75ng nucleotide sequence <SEQ ID 291 > was predicted to encode a protein having amino acid sequence <SEQ ID 292>:

```
  1 MSVFQTAFFM FQKHLQKASD SVVGGTLYVV ATPIGNLADI TLRALAVLQK
 51 ADIICAEDTR VTAQLLSAYG IQGRLVSVRE HNERQMADKV IGFLSDGLVV
101 AQVSDAGTPA VCDPGAKLAR RVREAGFKVV PVVGASAVMA ALSVAGVAES
151 DFYFNGFVPP KSGERRKLFA KWVRAAFPVV MFETPHRIGA TLADMAELFP
201 ERRLMLAREI TKTFETFLSG TVGEIQTALA ADGNQSRGEM VLVLYPAQDE
251 KHEGLSESAQ NAMKILAAEL PTKQAAELAA KITGEGKKAL YDLALSWKNK
301 *
```

After further analysis, the following gonococcal DNA sequence <SEQ ID 293> was identified:

```
  1 ATGTTTCAGA AACACTTGCA GAAAGCCTCC GACAGCGTCG TCGGAGGGAC
 51 ATTATACGTG GTTGCCACGC CCATCGGCAA TTTGGCAGAC ATTACCCTGC
101 GCGCTTTGGC GGTATTGCAA AAGGCGGACA TCATTTGTGC CGAAGACACG
151 CGCGTTACTG CGCAGCTTTT GAGCGCGTAC GGCATTCAGG GCAGGTTGGT
201 CAGTGTGCGC GAACACAACG AGCGGCAGAT GGCGGACAAG GTAATCGGTT
251 TCCTTTCAGA CGGCCTGGTT GTGGCGCAGG TTTCCGATGC GGGTACGCCG
301 GCCGTGTGCG ACCCGGGCGC GAAACTCGCC CGCCGCGTGC GCGAAGCAGG
351 GTTCAAAGTC GTTCCCGTCG TGGGCGCAAG CGCGGTAATG GCGGCGTTGA
401 GTGTGGCCGG TGTGGCGGAA TCCGATTTTT ATTTCAACGG TTTTGTACCG
451 CCGAAATCGG GCGAACGTAG GAAATTGTTT GCCAAATGGG TGCGGGCGGC
501 ATTTCCTGTC GTCATGTTTG AAACGCCGCA CCGAATCGGG GCAACGCTTG
551 CCGATATGGC GGAATTGTTC CCCGAACGCC GTCTGATGCT GGCGCGCGAA
601 ATCACGAAAA CGTTTGAAAC GTTCTTAAGC GGCACGGTTG GGGAAATTCA
651 GACGGCATTG GCGGCGGACG GCAACCAATC GCGCGGCGAG ATGGTGTTGG
701 TGCTTTATCC GGCGCAGGAT GAAAAACACG AAGGCTTGTC CGAGTCTGCG
751 CAAAATGCGA TGAAAATCCT TGCGGCCGAG CTGCCGACCA AGCAGGCGGC
801 GGAGCTTGCC GCCAAGATTA CAGGTGAGGG CAAAAAGGCT TTGTACGATT
851 TGGCACTGTC GTGGAAAAAC AAATGA
```

This corresponds to the amino acid sequence <SEQ ID 294; ORF75ng-1>:

```
  1 MFQKHLQKAS DSVVGGTLYV VATPIGNLAD ITLRALAVLQ KADIICAEDT
 51 RVTAQLLSAY GIQGRLVSVR EHNERQMADK VIGFLSDGLV VAQVSDAGTP
101 AVCDPGAKLA RRVREAGFKV VPVVGASAVM AALSVAGVAE SDFYFNGFVP
151 PKSGERRKLF AKWVRAAFPV VMFETPHRIG ATLADMAELF PERRLMLARE
201 ITKTFETFLS GTVGEIQTAL AADGNQSRGE MVLVLYPAQD EKHEGLSESA
251 QNAMKILAAE LPTKQAAELA AKITGEGKKA LYDLALSWKN K*
```

ORF75ng-1 and ORF75-1 show 96.2% identity in 291 aa overlap:

```
                    10        20        30        40        50        60
orf75-1.pep  MFQKHLQKASDSVVGGTLYVVATPIGNLADITLRALAVLQKADIICAEDTRVTAQLLSAY
             ||||||||||||||||||||||||||||||||||||||||||||||||||||||||||||
orf75ng-1    MFQKHLQKASDSVVGGTLYVVATPIGNLADITLRALAVLQKADIICAEDTRVTAQLLSAY
                    10        20        30        40        50        60

                    70        80        90       100       110       120
orf75-1.pep  GIQGKLVSVREHNERQMADKIVGYLSDGMVVAQVSDAGTPAVCDPGAKLARRVREAGFKV
             ||||:|||||||||||||||::|:||||:|||||||||||||||||||||||||||||||
orf75ng-1    GIQGRLVSVREHNERQMADKVIGFLSDGLVVAQVSDAGTPAVCDPGAKLARRVREAGFKV
                    70        80        90       100       110       120

                   130       140       150       160       170       180
orf75-1.pep  VPVVGASAVMAALSVAGVEGSDFYFNGFVPPKSGERRKLFAKWVRAAFPIVMFETPHRIG
             ||||||||||||||||| ||||||||||||||||||||||||||||||||:|||||||||
orf75ng-1    VPVVGASAVMAALSVAGVAESDFYFNGFVPPKSGERRKLFAKWVRAAFPVVMFETPHRIG
                   130       140       150       160       170       180

                   190       200       210       220       230       240
orf75-1.pep  ATLADMAELFPERRLMLAREITKTFETFLSGTVGEIQTALSADGNQSRGEMVLVLYPAQD
             |||||||||||||||||||||||||||||||||||||||:||||||||||||||||||||
orf75ng-1    ATLADMAELFPERRLMLAREITKTFETFLSGTVGEIQTALAADGNQSRGEMVLVLYPAQD
                   190       200       210       220       230       240

                   250       260       270       280       290
orf75-1.pep  EKHEGLSESAQNIMKILTAELPTKQAAELAAKITGEGKKALYDLALSWKNKX
             |||||||||||| ||||:||||||||||||||||||||||||||||||||||
orf75ng-1    EKHEGLSESAQNAMKILAAELPTKQAAELAAKITGEGKKALYDLALSWKNKX
                   250       260       270       280       290
```

Furthermore, ORG75ng-1 shows significant homology to a hypothetical *E.coli* protein:

```
sp|P45528|YRAL_ECOLI HYPOTHETICAL 31.3 KD PROTEIN IN AGAI-MTR INTERGENIC REGION
(F286)
>gi|606086 (U18997) ORF_f286 [Escherichia coli]
>gi|1789535 (AE000395) hypothetical 31.3 kD protein in agai-mtr intergenic
region [Escherichia coli] Length = 286
 Score =  218 bits (550), Expect = 3e-56
 Identities = 128/284 (45%), Positives = 171/284 (60%), Gaps = 4/284 (1%)


Query: 4   KHLQKASDSVVGGTLYVVATPIGNLADITLRALAVLQKADIICAEDTRVTAQLLSAYGIQ 63
            K  Q A +S   G LY+V TPIGNLADIT RAL VLQ  D+I AEDTR T  LL  +GI
Sbjct: 2   KQHQSADNSQ--GQLYIVPTPIGNLADITQRALEVLQAVDLIAAEDTRHTGLLLQHFGIN 59

Query: 64  GRLVSVREHNERQMADKVIGFLSDGLVVAQVSDAGTPAVCDPGAKLARRVREAGFKVVPV 123
             RL ++ +HNE+Q A+ ++  L +G  +A VSDAGTP + DPG  L R  REAG +VVP+
Sbjct: 60  ARLFALHDHNEQQKAETLLAKLQEGQNIALVSDAGTPLINDPGYHLVRTCREAGIRVVPL 119

Query: 124 VGASAVMAALSVAGVAESDFYFNGFVPPKSGERRKLFAKWVRAAFPVVMFETPHRIGATL 183
             G  A + ALS AG+    F + GF+P KS  RR             ++ +E+ HR+  +L
Sbjct: 120 PGPCAAITALSAAGLPSDRFCYEGFLPAKSKGRRDALKAIEAEPRTLIFYESTHRLLDSL 179

Query: 184 ADMAELFPERR-LMLAREITKTFETFLSGTVGEIQTALAADGNQSRGEMVLVLYPAQDEK 242
             D+  +  E R ++LARE+TKT+ET      VGE+   +  D N+ +GEMVL++      +
Sbjct: 180 EDIVAVLGESRYVVLARELTKTWETIHGAPVGELLAWVKEDENRRKGEMVLIV-EGHKAQ 238

Query: 243 HEGLSESAQNAMKILAAELPTKQAAELAAKITGEGKKALYDLAL 286
             E L   A   + +L AELP K+AA LAA+I G  K ALY  AL
Sbjct: 239 EEDLPADALRTLALLQAELPLKKAAALAAEIHGVKKNALYKYAL 282
```

Based on this analysis, including the presence of a putative transmembrane domain in the gonococcal protein, it is predicted that the proteins from *N.meningitidis* and *N.gonorrhoeae,* and their epitopes, could be useful antigens for vaccines or diagnostics, or for raising antibodies.

**Example 35**

The following partial DNA sequence was identified in *N.meningitidis* <SEQ ID 295>:

```
  1  ATGAAACAGA AAAAAACCGC TGCCGCAGTT ATTGCTGCAA TGTTGGCAGG
 51  TTTTGCGGCA GC.AAAGCAC CCGAAATCGA CCCGGCTTTG ..........
                                      //
651  .......... ...GAGTTGG TCAGAAACCA GTTGGAGCAG GGTTTGAGAC
701  AGGAAAAAGC CCGCTTGAAA ATCGATGCCC TTTTGGAAGA AAACGGTGTC
751  AAACCGTAA
```

This corresponds to the amino acid sequence <SEQ ID 296; ORF76>:

```
  1  MKQKKTAAAV IAAMLAGFAA XKAPEIDPAL .......... ..........
                                  //
201  .......... .......... ELVRNQLEQG LRQEKARLKI DALLEENGVK
251  P*
```

Further work revealed the complete nucleotide sequence <SEQ ID 297>:

```
  1  ATGAAACAGA AAAAAACCGC TGCCGCAGTT ATTGCTGCAA TGTTGGCAGG
 51  TTTTGCGGCA GCCAAAGCAC CCGAAATCGA CCCGGCTTTG GTGGATACGC
101  TGGTGGCGCA GATCATGCAG CAGGCAGACC GGCATGCGGA GCAGTCCCAA
151  AAACCGGACG GGCAGGCAAT CCGAAACGAT GCCGTCCGCC GGCTACAAAC
201  TTTGGAAGTT TTGAAAAACA GGGCATTGAA GGAAGGTTTG GATAAGGATA
251  AGGATGTCCA AAACCGCTTT AAAATCGCCG AAGCGTCTTT TTATGCCGAG
301  GAGTACGTCC GTTTTCTGGA ACGTTCGGAA ACGGTTTCCG AAGACGAGCT
351  GCACAAGTTT TACGAACAGC AAATCCGCAT GATCAAATTG CAGCAGGTCA
401  GCTTCGCAAC CGAAGAGGAG GCGCGTCAGG CGCAGCAGCT CCTGCTCAAA
451  GGGCTGTCTT TTGAAGGGCT GATGAAGCGT TATCCGAACG ACGAGCAGGC
501  TTTTGACGGT TTCATTATGG CGCAGCAGCT TCCCGAGCCG CTGGCTTCGC
551  AGTTTGCCGC GATGAATCGG GGCGACGTTA CCCGCGATCC GGTCAAATTG
601  GGCGAACGCT ATTATCTGTT CAAACTCAGC GAGGTCGGGA AAAACCCCGA
651  CGCGCAGCCT TTCGAGTTGG TCAGAAACCA GTTGGAGCAG GGTTTGAGAC
701  AGGAAAAAGC CCGCTTGAAA ATCGATGCCC TTTTGGAAGA AAACGGTGTC
751  AAACCGTAA
```

This corresponds to the amino acid sequence <SEQ ID 298; ORF76-1>:

```
  1  MKQKKTAAAV IAAMLAGFAA AKAPEIDPAL VDTLVAQIMQ QADRHAEQSQ
 51  KPDGQAIRND AVRRLQTLEV LKNRALKEGL DKDKDVQNRF KIAEASFYAE
101  EYVRFLERSE TVSEDELHKF YEQQIRMIKL QQVSFATEEE ARQAQQLLLK
151  GLSFEGLMKR YPNDEQAFDG FIMAQQLPEP LASQFAAMNR GDVTRDPVKL
201  GERYYLFKLS EVGKNPDAQP FELVRNQLEQ GLRQEKARLK IDALLEENGV
251  KP*
```

Computer analysis of this amino acid sequence gave the following results:

Homology with a predicted ORF from *N.meningitidis* (strain A)

ORF76 shows 96.7% identity over a 30aa overlap and 96.8% identity over a 31 aa overlap with an ORF (ORF76a) from strain A of *N. meningitidis:*

```
                         10        20        30
orf76.pep      MKQKKTAAAVIAAMLAGFAAXKAPEIDPAL
               |||||||||||||||||||| |||||||||
orf76a         MKQKKTAAAVIAAMLAGFAAAKAPEIDPALVDTLVAQIMQQADRHAEQSQKPDGQAIRND
                         10        20        30        40        50        60
                              //
                                                70        80        90
orf76.pep                                    XELVRNQLEQGLRQEKARLKIDALLEENGVKPX
                                             |||||||||||||||||||||||||:|||||||
orf76a         DVTRDPVKLGERYYLFKLSEVGKNPDAQPFELVRNQLEQGLRQEKARLKIDAILEENGVKPX
                       200       210       220       230       240       250
```

The complete length ORF76a nucleotide sequence <SEQ ID 299> is:

```
  1  ATGAAACAGA AAAAAACCGC TGCCGCAGTT ATTGCTGCAA TGTTGGCAGG
 51  TTTTGCGGCA GCCAAAGCAC CCGAAATCGA CCCGGCTTTG GTGGATACGC
101  TGGTGGCGCA GATCATGCAG CAGGCAGACC GGCATGCGGA GCAGTCCCAA
151  AAACCGGACG GGCAGGCAAT CCGAAACGAT GCCGTCCGTC GGCTGCAAAC
201  TTTGGAAGTT TTGAAAAACA GGGCATTGAA GGAAGGTTTG GATAAGGATA
251  AGGATGTCCA AAACCGCTTT AAAATCGCCG AAGCGTCTTT TTATGCCGAG
301  GAGTACGTCC GTTTTCTGGA ACGTTCGGAA ACGGTTTCCG AAAGCGCACT
351  GCGTCAGTTT TATGAGCGGC AAATCCGCAT GATCAAATTG CAGCAGGTCA
401  GCTTCGCAAC CGAAGAGGAG GCGCGTCAGG CGCAGCAGCT CCTGCTCAAA
451  GGGCTGTCTT TTGAAGGGCT GATGAAGCGT TATCCGAACG ACGAGCAGGC
501  TTTTGACGGT TTCATTATGG CGCAGCAGCT TCCCGAGCCG CTGGCTTCGC
551  AGTTTGCAGC GATGAATCGG GGCGACGTTA CCCGCGATCC GGTCAAATTG
601  GGCGAACGCT ATTATCTGTT CAAACTCAGC GAGGTCGGGA AAAACCCCGA
651  CGCGCAGCCT TTCGAGTTGG TCAGAAACCA GTTGGAACAA GGTTTGAGAC
701  AGGAAAAAGC CCGCTTGAAA ATCGATGCCA TTTTGGAAGA AAACGGTGTC
751  AAACCGTAA
```

This encodes a protein having amino acid sequence <SEQ ID 300>:

```
  1  MKQKKTAAAV IAAMLAGFAA AKAPEIDPAL VDTLVAQIMQ QADRHAEQSQ
 51  KPDGQAIRND AVRRLQTLEV LKNRALKEGL DKDKDVQNRF KIAEASFYAE
101  EYVRFLERSE TVSESALRQF YERQIRMIKL QQVSFATEEE ARQAQQLLLK
151  GLSFEGLMKR YPNDEQAFDG FIMAQQLPEP LASQFAAMNR GDVTRDPVKL
201  GERYYLFKLS EVGKNPDAQP FELVRNQLEQ GLRQEKARLK IDAILEENGV
251  KP*
```

ORF76a and ORF76-1 show 97.6% identity in 252 aa overlap:

```
                         10        20        30        40        50        60
orf76a.pep     MKQKKTAAAVIAAMLAGFAAAKAPEIDPALVDTLVAQIMQQADRHAEQSQKPDGQAIRND
               ||||||||||||||||||||||||||||||||||||||||||||||||||||||||||||
orf76-1        MKQKKTAAAVIAAMLAGFAAAKAPEIDPALVDTLVAQIMQQADRHAEQSQKPDGQAIRND
                         10        20        30        40        50        60

                         70        80        90       100       110       120
orf76a.pep     AVRRLQTLEVLKNRALKEGLDKDKDVQNRFKIAEASFYAEEYVRFLERSETVSESALRQF
               |||||||||||||||||||||||||||||||||||||||||||||||||||||: |::|
orf76-1        AVRRLQTLEVLKNRALKEGLDKDKDVQNRFKIAEASFYAEEYVRFLERSETVSEDELHKF
                         70        80        90       100       110       120

                        130       140       150       160       170       180
orf76a.pep     YERQIRMIKLQQVSFATEEEARQAQQLLLKGLSFEGLMKRYPNDEQAFDGFIMAQQLPEP
               ||:|||||||||||||||||||||||||||||||||||||||||||||||||||||||||
```

```
orf76-1      YEQQIRMIKLQQVSFATEEEARQAQQLLLKGLSFEGLMKRYPNDEQAFDGFIMAQQLPEP
                     130       140       150       160       170       180

                     190       200       210       220       230       240
orf76a.pep   LASQFAAMNRGDVTRDPVKLGERYYLFKLSEVGKNPDAQPFELVRNQLEQGLRQEKARLK
             ||||||||||||||||||||||||||||||||||||||||||||||||||||||||||||
orf76-1      LASQFAAMNRGDVTRDPVKLGERYYLFKLSEVGKNPDAQPFELVRNQLEQGLRQEKARLK
                     190       200       210       220       230       240

                     250
orf76a.pep   IDAILEENGVKPX
             |||:|||||||||
orf76-1      IDALLEENGVKPX
                     250
```

Homology with a predicted ORF from *N.gonorrhoeae*

The aligned aa sequences of ORF76 and a predicted ORF (ORF76.ng) from *N. gonorrhoeae* of the N- and C-termini show 96.7 % and 100% identity in 30 and 31 overlap, respectively:

```
orf76.pep   MKQKKTAAAVIAAMLAGFAAXKAPEIDPAL                                 30
            |||||||||||||||||||| |||||||||
orf76ng     MKQKKTAAAVIAAMLAGFAAAKAPEIDPALVDTLVAQIMQQADRHAEQSQRPDGQAIRND   60
                                      //
orf76.pep                                ELVRNQLEQGLRQEKARLKIDALLEENGVKP  251
                                         |||||||||||||||||||||||||||||||
orf76ng     VTRNPVKLGERYYLFKLGAVGKNPDAQPFELVRNQLEQGLRQEKARLKIDALLEENGVKP  251
```

The complete length ORF76ng nucleotide sequence <SEQ ID 301> is:

```
  1 ATGAAACAGA AAAAGACCGC TGCCGCAGTT ATTGCTGCAA TGTTGGCAGG
 51 TTTTGCGGCA GCCAAAGCAC CCGAAATCGA CCCGGCTTTG GTGGATACGC
101 TGGTGGCGCA GATCATGCAG CAGGCAGACC GGCATGCGGA GCAGTCCCAA
151 AGACCGGACG GGCAGGCAAT CCGAAACGAT GCCGTCCGCC GGCTGCAAAC
201 TTTGGAAGTT TTGAAAAACA GGGCATTGAA GGAAGGTTTG GATAAGGATA
251 AGGATGTCCA AAACCGCTTT AAAATCGCCG AAGCGTCTTT TTATGCCGAG
301 GAGTACGTCC GTTTTCTGGA ACGTTCGGAA ACGGTTTCCG AAAGCGCACT
351 GCGTCAGTTT TATGAGCGGC AAATCCGCAT GATCAAATTG CAGCAGGTCA
401 GCTTCGCAAC CGAAGAGGAG GCGCGTCAGG CGCAGCAGCT CCTGCTCAAA
451 GGGCTGTCTT TTGAAGGGCT GATGAAGCGT TATCCGAACG ACGAGCAGGC
501 GTTCGACGGT TTCATTATGG CGCAGCAGCT TCCCGAGCCG CTGGCTTcgc
551 agtttgCCGG TATGAACCGT GGCGACGTTA CCCGCAATCC GGTCAAATTG
601 GGCGAACGCT ATTACCTGTT CAAACTCGGC GCGGTCGGGA AAAACCCCGA
651 CGCGCAGCCT TTCGAGTTGG TCAGAAACCA GTTGGAACAA GGTTTGAGGC
701 AGGAAAAAGC CCGCTTGAAA ATCGATGCCC TTTTGGAaga Aaacggtgtc
751 AaacCGTAA
```

This encodes a protein having amino acid sequence <SEQ ID 302>:

```
  1 MKQKKTAAAV IAAMLAGFAA AKAPEIDPAL VDTLVAQIMQ QADRHAEQSQ
 51 RPDGQAIRND AVRRLQTLEV LKNRALKEGL DKDKDVQNRF KIAEASFYAE
101 EYVRFLERSE TVSESALRQF YERQIRMIKL QQVSFATEEE ARQAQQLLLK
151 GLSFEGLMKR YPNDEQAFDG FIMAQQLPEP LASQFAGMNR GDVTRNPVKL
201 GERYYLFKLG AVGKNPDAQP FELVRNQLEQ GLRQEKARLK IDALLEENGV
251 KP*
```

ORF76ng and ORF76-1 show 96.0% identity in 252 aa overlap

```
                        10        20        30        40        50        60
orf76-1.pep   MKQKKTAAAVIAAMLAGFAAAKAPEIDPALVDTLVAQIMQQADRHAEQSQKPDGQAIRND
              ||||||||||||||||||||||||||||||||||||||||||||||||||:|||||||||
orf76ng       MKQKKTAAAVIAAMLAGFAAAKAPEIDPALVDTLVAQIMQQADRHAEQSQRPDGQAIRND
                        10        20        30        40        50        60

                        70        80        90       100       110       120
orf76-1.pep   AVRRLQTLEVLKNRALKEGLDKDKDVQNRFKIAEASFYAEEYVRFLERSETVSEDELHKF
              ||||||||||||||||||||||||||||||||||||||||||||||||||||||: |::|
orf76ng       AVRRLQTLEVLKNRALKEGLDKDKDVQNRFKIAEASFYAEEYVRFLERSETVSESALRQF
                        70        80        90       100       110       120

                       130       140       150       160       170       180
orf76-1.pep   YEQQIRMIKLQQVSFATEEEARQAQQLLLKGLSFEGLMKRYPNDEQAFDGFIMAQQLPEP
              ||:|||||||||||||||||||||||||||||||||||||||||||||||||||||||||
orf76ng       YERQIRMIKLQQVSFATEEEARQAQQLLLKGLSFEGLMKRYPNDEQAFDGFIMAQQLPEP
                       130       140       150       160       170       180

                       190       200       210       220       230       240
orf76-1.pep   LASQFAAMNRGDVTRDPVKLGERYYLFKLSEVGKNPDAQPFELVRNQLEQGLRQEKARLK
              ||||||:||||||||:|||||||||||||: |||||||||||||||||||||||||||||
orf76ng       LASQFAGMNRGDVTRNPVKLGERYYLFKLGAVGKNPDAQPFELVRNQLEQGLRQEKARLK
                       190       200       210       220       230       240

                       250
orf76-1.pep   IDALLEENGVKPX
              |||||||||||||
orf76ng       IDALLEENGVKPX
                       250
```

Furthermore, ORF76ng shows significant homology to a *B.subtilis* export protein precursor:

```
sp|P24327|PRSA_BACSU PROTEIN EXPORT PROTEIN PRSA PRECURSOR >gi|98227|pir||S15269
33K lipoprotein - Bacillus subtilis >gi|39782 (X57271) 33kDa lipoprotein
[Bacillus subtilis]
>gi|2226124|gnl|PID|e325181 (Y14077) 33kDa lipoprotein [Bacillus subtilis]
>gi|2633331|gnl|PID|e1182997 (Z99109) molecular chaperonin [Bacillus subtilis]
Length = 292
 Score = 50.4 bits (118), Expect = 1e-05
 Identities = 48/199 (24%), Positives = 82/199 (41%), Gaps = 32/199 (16%)

Query: 70  VLKNRALKEGLDK-----DKDVQNRFKIAEASF----------YAEEYVRFLERSETVSE 114
            VL     ++ LDK     DK++ N+ K  +             Y ++Y++    + E +++
Sbjct: 53  VLTQLVQEKVLDKKYKVSDKEIDNKLKEYKTQLGDQYTALEKQYGKDYLKEQVKYELLTQ 112

Query: 115 SA-----------LRQFYERQIRMIKLQQVSFATEEEARQAQQLLLKGLSFEGLMKRYPN 163
             A            +++++E     I+    +  A ++ A + ++ L KG  FE L K Y
Sbjct: 113 KAAKDNIKVTDADIKEYWEGLKGKIRASHILVADKKTAEEVEKKLKKGEKFEDLAKEYST 172

Query: 164 DEQAFDG-----FIMAQQLPEPLASQFAAMNRGDVTRDPVKLGERYYLFKLSEVGKNPDA 218
            D  A  G     F    Q+ E  +      +  G+V+ DPVK    Y++ K +E     D
Sbjct: 173 DSSASKGGDLGWFAKEGQMDETFSKAAFKLKTGEVS-DPVKTQYGYHIIKKTEERGKYDD 231

Query: 219 QPFELVRNQLEQGLRQEKA 237
               EL    LEQ L    A
Sbjct: 232 MKKELKSEVLEQKLNDNAA 250
```

Based on this analysis, including the presence of a putative leader sequence and a RGD motif in the gonococcal protein, it was predicted that the proteins from *N.meningitidis* and *N.gonorrhoeae,* and their epitopes, could be useful antigens for vaccines or diagnostics, or for raising antibodies.

ORF76-1 (27.8kDa) was cloned in the pET vector and expressed in *E.coli,* as described above. The products of protein expression and purification were analyzed by SDS-PAGE. Figure 10A shows the results of affinity purification of the His-fusion protein, Purified His-fusion protein was used to immunise mice, whose sera were used for Western blot (Figure 10B), ELISA (positive result), and FACS analysis (Figure 10C). These experiments confirm that ORF76-1 is a surface-exposed protein, and that it is a useful immunogen.

**Example 36**

The following partial DNA sequence was identified in *N.meningitidis* <SEQ ID 303>:

```
   1  ATGAAAAAAT CTTTCCTTAC GCTTGTTCTG TATTCGTCTT TACTTACCGC
  51  CAGCGAAATT GCCTTACCCC TTGGAATTGG GGATTGAAAC CTTACCGGCG
 101  GCAAAAATTG CGGAAACGTT TGCGCTGACA TTTGTGATTG CTGCGCTGTA
 151  TCTGTTTGCG CGTAATAAGG TGACGCGTTT GTTGATTGCG GTGTTTTTTG
 201  CGTTCAGCAT TATTGCCAAC AATGTGCATT ACGCGGATTA TCAAAGCTGG
 251  ATGACG.... .......... .......... .......... ..........
                                    //
1201  .......... CAAACCGTAT TCGAGCAGCT GCAAAAGACT CCTGACGGCA
1251  ACTGGCTGTT TGCCTATACC TCCGATCATG GCCAGTATGT TCGCCAAGAT
1301  ATCTACAATC AAGGCACGGT GCAGCCCGAC AGCTATCTCG TGCCGCTAGT
1351  GTTGTACAGC CCGGATAAGG CCGTGCAACA GGCTGCCAAC CAGGCTTTTG
1401  CGCCTTGCGA GATTGCCTTC CATCAGCAGC TTTCAACGTT CCTGATTCAC
1451  ACGTTGGGCT ACGATATGCC GGTTTCAGGT TGTCGCGAAG GCTCGGTAAC
1501  GGGCAACCTG ATTACGGGTG ATGCAGGCAG CTTGAACATT CGCGACGGCA
1551  AGGCGGAATA TGTTTATCCG CAATGA
```

This corresponds to the amino acid sequence <SEQ ID 304; ORF81>:

```
   1  MKKSFLTLVL YSSLLTASEI AYPLELGIET LPAAKIAETF ALTFVIAALY
  51  LFARNKVTRL LIAVFFAFSI IANNVHYADY QSWMT..... ..........
                                    //
 401  ...QTVFEQL QKTPDGNWLF AYTSDHGQYV RQDIYNQGTV QPDSYLVPLV
 451  LYSPDKAVQQ AANQAFAPCE IAFHQQLSTF LIHTLGYDMP VSGCREGSVT
 501  GNLITGDAGS LNIRDGKAEY VYPQ*
```

Further work revealed the complete nucleotide sequence <SEQ ID 305>:

```
   1  ATGAAAAAAT CTTTCCTTAC GCTTGTTCTG TATTCGTCTT TACTTACCGC
  51  CAGCGAAATT GCCTATCGCT TTGTATTTGG GATTGAAACC TTACCGGCGG
 101  CAAAAATTGC GGAAACGTTT GCGCTGACAT TTGTGATTGC TGCGCTGTAT
 151  CTGTTTGCGC GTTATAAGGT GACGCGTTTG TTGATTGCGG TGTTTTTTGC
 201  GTTCAGCATT ATTGCCAACA ATGTGCATTA CGCGGTTTAT CAAAGCTGGA
 251  TGACGGGCAT CAATTATTGG CTGATGCTGA AAGAGGTTAC CGAAGTCGGC
 301  AGCGCGGGTG CGTCGATGTT GGATAAGTTG TGGCTGCCTG TGTTGTGGGG
 351  CGTGTTGGAA GTCATGTTGT TTTGCAGCCT TGCCAAGTTC CGCCGTAAGA
 401  CGCATTTTTC TGCCGATATA CTGTTTGCCT TCCTAATGCT GATGATTTTC
 451  GTGCGTTCGT TCGACACGAA ACAAGAGCAC GGTATTTCGC CCAAACCGAC
 501  ATACAGCCGC ATCAAAGCCA ATTATTTCAG CTTCGGTTAT TTTGTCGGAC
 551  GCGTGTTGCC GTATCAGTTG TTTGATTTAA GCAGGATTCC CGCCTTTAAG
 601  CAGCCTGCTC CAAGCAAAAT CGGGCAGGGC AGTGTTCAAA ATATCGTCCT
 651  GATTATGGGC GAAAGCGAAA GCGCGGCGCA TTTGAAGCTG TTTGGCTACG
 701  GACGCGAAAC TTCGCCGTTT TTAACCCGGC TGTCGCAAGC CGATTTTAAG
 751  CCGATTGTGA AACAAAGTTA TTCCGCAGGC TTTATGACTG CAGTGTCCCT
 801  GCCCAGTTTT TTCAATGCGA TACCGCACGC CAACGGCTTG GAACAAATCA
 851  GCGGCGGCGA TACCAATATG TTCCGCCTCG CCAAAGAGCA GGGCTATGAA
 901  ACGTATTTTT ACAGCGCGCA GGCGGAAAAC GAGATGGCGA TTTTGAACTT
 951  AATCGGTAAG AAATGGATAG ACCATCTGAT TCAGCCGACG CAACTTGGCT
1001  ACGGCAACGG CGACAATATG CCCGATGAGA AGCTGCTGCC GTTGTTCGAC
1051  AAAATCAATT TGCAGCAGGG CAAGCATTTT ATCGTGTTGC ACCAACGCGG
1101  TTCGCACGCC CCATACGGCG CATTGTTGCA GCCTCAAGAT AAAGTATTCG
1151  GCGAAGCCGA TATTGTGGAT AAGTACGACA ACACCATCCA CAAAACCGAC
1201  CAAATGATTC AAACCGTATT CGAGCAGCTG CAAAAGCAGC CTGACGGCAA
1251  CTGGCTGTTT GCCTATACCT CCGATCATGG CCAGTATGTT CGCCAAGATA
1301  TCTACAATCA AGGCACGGTG CAGCCCGACA GCTATCTCGT GCCGCTAGTG
1351  TTGTACAGCC CGGATAAGGC CGTGCAACAG GCTGCCAACC AGGCTTTTGC
1401  GCCTTGCGAG ATTGCCTTCC ATCAGCAGCT TTCAACGTTC CTGATTCACA
1451  CGTTGGGCTA CGATATGCCG GTTTCAGGTT GTCGCGAAGG CTCGGTAACG
1501  GGCAACCTGA TTACGGGTGA TGCAGGCAGC TTGAACATTC GCGACGGCAA
1551  GGCGGAATAT GTTTATCCGC AATGA
```

This corresponds to the amino acid sequence <SEQ ID 306; ORF81-1>:

```
   1  MKKSFLTLVL YSSLLTASEI AYRFVFGIET LPAAKIAETF ALTFVIAALY
  51  LFARYKVTRL LIAVFFAFSI IANNVHYAVY QSWMTGINYW LMLKEVTEVG
 101  SAGASMLDKL WLPVLWGVLE VMLFCSLAKF RRKTHFSADI LFAFLMLMIF
 151  VRSFDTKQEH GISPKPTYSR IKANYFSFGY FVGRVLPYQL FDLSRIPAFK

 201  QPAPSKIGQG SVQNIVLIMG ESESAAHLKL FGYGRETSPF LTRLSQADFK
 251  PIVKQSYSAG FMTAVSLPSF FNAIPHANGL EQISGGDTNM FRLAKEQGYE
 301  TYFYSAQAEN EMAILNLIGK KWIDHLIQPT QLGYGNGDNM PDEKLLPLFD
 351  KINLQQGKHF IVLHQRGSHA PYGALLQPQD KVFGEADIVD KYDNTIHKTD
 401  QMIQTVFEQL QKQPDGNWLF AYTSDHGQYV RQDIYNQGTV QPDSYLVPLV
 451  LYSPDKAVQQ AANQAFAPCE IAFHQQLSTF LIHTLGYDMP VSGCREGSVT
 501  GNLITGDAGS LNIRDGKAEY VYPQ*
```

Computer analysis of this amino acid sequence gave the following results:

<u>Homology with a predicted ORF from *N.meningitidis* (strain A)</u>

ORF81 shows 84.7% identity over a 85aa overlap and 99.2% identity over a 121aa overlap with an ORF (ORF81a) from strain A of *N. meningitidis:*

```
                       10        20        30        40        50        60
orf81.pep     MKKSFLTLVLYSSLLTASEIAYPLELGIETLPAAKIAETFALTFVIAALYLFARNKVTRL
              ||||:::| ||||||||||||| : :|||||||||:|||||||||||||||||| |:|||
orf81a        MKKSLFVLFLYSSLLTASEIAYRFVFGIETLPAAKMAETFALTFVIAALYLFARYKATRL
                       10        20        30        40        50        60

                       70        80
orf81.pep     LIAVFFAFSIIANNVHYADYQSWMT
              |||||||||||||||||| ||||:|
orf81a        LIAVFFAFSIIANNVHYAVYQSWITGINYWLMLKEITEVGGAGASMLDKLWLPALWGVLE
                       70        80        90       100       110       120
                                 //
                                                     120       130       140
orf81.pep                                   QTVFEQLQKTPDGNWLFAYTSDHGQYVRQD
                                            ||||||||| ||||||||||||||||||||
orf81a        IPHANGLEQISGGDIVDKYDNTIHKTDQMIQTVFEQLQKQPDGNWLFAYTSDHGQYVRQD
                   280       290       300       310       320       330

                      150       160       170       180       190       200
orf81.pep     IYNQGTVQPDSYLVPLVLYSPDKAVQQAANQAFAPCEIAFHQQLSTFLIHTLGYDMPVSG
              ||||||||||||||||||||||||||||||||||||||||||||||||||||||||||||
orf81a        IYNQGTVQPDSYLVPLVLYSPDKAVQQAANQAFAPCEIAFHQQLSTFLIHTLGYDMPVSG
                   340       350       360       370       380       390

                      210       220       230
orf81.pep     CREGSVTGNLITGDAGSLNIRDGKAEYVVYPQX
              ||||||||||||||||||||||||||||||||
orf81a        CREGSVTGNLITGDAGSLNIRDGKAEYVVYPQX
                   400       410       420
```

The complete length ORF81a nucleotide sequence <SEQ ID 307> is:

```
   1  ATGAAAAAAT CCCTTTTCGT TCTCTTTCTG TATTCGTCCC TACTTACTGC
  51  CAGCGAAATT GCTTATCGCT TTGTATTCGG AATTGAAACC TTACCGGCTG
 101  CAAAAATGGC AGAAACGTTT GCGCTGACAT TTGTGATTGC TGCGCTGTAT
 151  CTGTTTGCGC GTTATAAGGC AACGCGTTTG TTGATTGCGG TGTTTTTCGC
 201  GTTCAGCATT ATTGCCAACA ATGTGCATTA CGCGGTTTAT CAAAGCTGGA
 251  TAACGGGCAT TAATTATTGG CTGATGCTGA AAGAGATTAC CGAAGTTGGC
 301  GGCGCAGGGG CGTCGATGTT GGATAAGTTG TGGCTGCCTG CGTTGTGGGG
 351  CGTGTTGGAA GTCATGTTGT TTTGCAGCCT TGCCAAGTTC CGCCGTAAGA
 401  CGCATTTTTC TGCCGATATA CTGTTTGCCT TCCTAATGCT GATGATTTTC
 451  GTGCGTTCGT TCGACACGAA ACAAGAACAC GGTATTTCGC CCAAACCGAC
 501  ATACAGCCGC ATCAAAGCCA ATTATTTCAG CTTCGGTTAT TTTGTCGGAC
 551  GCGTGTTGCC GTATCAGTTG TTTGATTTAA GCAAGATTCC TGTGTTCAAA
 601  CAGCCTGCTC CAAGCAGAAT CGGGCAAGGC AGTATTCAAA ATATCGTCCT
 651  GATTATGGGC GAAAGCGAAA GCGCGGCGCA TTTGAAATTG TTTGGCTACG
 701  GGCGCGAAAC TTCGCCGTTT TTGACCCAGC TTTCGCAAGC CGATTTTAAG
 751  CCGATTGTGA AACAAAGTTA TTCCGCAGGC TTTATGACGG CAGTATCCCT
 801  GCCCAGTTTC TTTAACGTCA TACCGCATGC CAACGGCTTG GAACAAATCA
 851  GCGGCGGCGA TATTGTGGAT AAGTACGACA ACACCATCCA CAAAACCGAC
 901  CAAATGATTC AAACCGTATT CGAGCAGCTG CAAAAGCAGC CTGACGGCAA
 951  CTGGCTGTTT GCCTATACCT CCGATCATGG CCAGTATGTT CGCCAAGATA
1001  TCTACAATCA AGGCACGGTG CAGCCCGACA GCTATCTCGT GCCGCTGGTG
1051  TTGTACAGCC CGGATAAGGC CGTGCAACAG GCTGCCAACC AGGCTTTTGC
1101  GCCTTGCGAG ATTGCCTTCC ATCAGCAGCT TTCAACGTTC CTGATTCACA
1151  CGTTGGGCTA CGATATGCCG GTTTCAGGTT GTCGCGAAGG CTCGGTAACG
1201  GGCAACCTGA TTACGGGTGA TGCAGGCAGC TTGAACATTC GCGACGGCAA
1251  GGCGGAATAT GTTTATCCGC AATGA
```

This encodes a protein having amino acid sequence <SEQ ID 308>:

```
  1  MKKSLFVLFL YSSLLTASEI AYRFVFGIET LPAAKMAETF ALTFVIAALY
 51  LFARYKATRL LIAVFFAFSI IANNVHYAVY QSWITGINYW LMLKEITEVG
101  GAGASMLDKL WLPALWGVLE VMLFCSLAKF RRKTHFSADI LFAFLMLMIF
151  VRSFDTKQEH GISPKPTYSR IKANYFSFGY FVGRVLPYQL FDLSKIPVFK
201  QPAPSRIGQG SIQNIVLIMG ESESAAHLKL FGYGRETSPF LTQLSQADFK
251  PIVKQSYSAG FMTAVSLPSF FNVIPHANGL EQISGGDIVD KYDNTIHKTD
301  QMIQTVFEQL QKQPDGNWLF AYTSDHGQYV RQDIYNQGTV QPDSYLVPLV
351  LYSPDKAVQQ AANQAFAPCE IAFHQQLSTF LIHTLGYDMP VSGCREGSVT
401  GNLITGDAGS LNIRDGKAEY VYPQ*
```

ORF81a and ORF81-1 show 77.9% identity in 524 aa overlap:

```
                      10        20        30        40        50        60
orf81a.pep    MKKSLFVLFLYSSLLTASEIAYRFVFGIETLPAAKMAETFALTFVIAALYLFARYKATRL
              ||||:::| |||||||||||||||||||||||||||:|||||||||||||||||||:|||
orf81-1       MKKSFLTLVLYSSLLTASEIAYRFVFGIETLPAAKIAETFALTFVIAALYLFARYKVTRL
                      10        20        30        40        50        60

                      70        80        90       100       110       120
orf81a.pep    LIAVFFAFSIIANNVHYAVYQSWITGINYWLMLKEITEVGGAGASMLDKLWLPALWGVLE
              |||||||||||||||||||||||:|||||||||||:||||:||||||||||||:||||||
orf81-1       LIAVFFAFSIIANNVHYAVYQSWMTGINYWLMLKEVTEVGSAGASMLDKLWLPVLWGVLE
                      70        80        90       100       110       120

                     130       140       150       160       170       180
orf81a.pep    VMLFCSLAKFRRKTHFSADILFAFLMLMIFVRSFDTKQEHGISPKPTYSRIKANYFSFGY
              ||||||||||||||||||||||||||||||||||||||||||||||||||||||||||||
orf81-1       VMLFCSLAKFRRKTHFSADILFAFLMLMIFVRSFDTKQEHGISPKPTYSRIKANYFSFGY
                     130       140       150       160       170       180

                     190       200       210       220       230       240
orf81a.pep    FVGRVLPYQLFDLSKIPVFKQPAPSRIGQGSIQNIVLIMGESESAAHLKLFGYGRETSPF
              ||||||||||||||:||:|||||||:|||||:||||||||||||||||||||||||||||
orf81-1       FVGRVLPYQLFDLSRIPAFKQPAPSKIGQGSVQNIVLIMGESESAAHLKLFGYGRETSPF
                     190       200       210       220       230       240

                     250       260       270       280
orf81a.pep    LTQLSQADFKPIVKQSYSAGFMTAVSLPSFFNVIPHANGLEQISGGD-------------
              ||:|||||||||||||||||||||||||||||:||||||||||||||
orf81-1       LTRLSQADFKPIVKQSYSAGFMTAVSLPSFFNAIPHANGLEQISGGDTNMFRLAKEQGYE
                     250       260       270       280       290       300

orf81a.pep    ------------------------------------------------------------

orf81-1       TYFYSAQAENEMAILNLIGKKWIDHLIQPTQLGYGNGDNMPDEKLLPLFDKINLQQGKHF
                     310       320       330       340       350       360

                                           290       300       310       320
orf81a.pep    ---------------------------IVDKYDNTIHKTDQMIQTVFEQLQKQPDGNWLF
                                         |||||||||||||||||||||||||||||||||
orf81-1       IVLHQRGSHAPYGALLQPQDKVFGEADIVDKYDNTIHKTDQMIQTVFEQLQKQPDGNWLF
                     370       380       390       400       410       420

                     330       340       350       360       370       380
orf81a.pep    AYTSDHGQYVRQDIYNQGTVQPDSYLVPLVLYSPDKAVQQAANQAFAPCEIAFHQQLSTF
              ||||||||||||||||||||||||||||||||||||||||||||||||||||||||||||
orf81-1       AYTSDHGQYVRQDIYNQGTVQPDSYLVPLVLYSPDKAVQQAANQAFAPCEIAFHQQLSTF
                     430       440       450       460       470       480

                     390       400       410       420
orf81a.pep    LIHTLGYDMPVSGCREGSVTGNLITGDAGSLNIRDGKAEYVYPQX
              ||||||||||||||||||||||||||||||||||||||||||||
orf81-1       LIHTLGYDMPVSGCREGSVTGNLITGDAGSLNIRDGKAEYVYPQX
                     490       500       510       520
```

Homology with a predicted ORF from *N.gonorrhoeae*

The aligned aa sequences of ORF81 and a predicted ORF (ORF81.ng) from *N. gonorrhoeae* of the N- and C-termini show 82.4 % and 97.5% identity in 85 and 121 overlap, respectively:

```
orf81.pep   MKKSFLTLVLYSSLLTASEIAYPLELGIETLPAAKIAETFALTFVIAALYLFARNKVTRL   60
            ||||:::| ||||||||||||| : :|||||||||:||||||||:||||||||| |::||
orf81ng     MKKSLFVLFLYSSLLTASEIAYRFVFGIETLPAAKMAETFALTFMIAALYLFARYKASRL   60

orf81.pep   LIAVFFAFSIIANNVHYADYQSWMT                                      85
            |||||||||:|||||||| ||||||
orf81ng     LIAVFFAFSMIANNVHYAVYQSWMTGINYWLMLKEVTEVGSAGASMLDKLWLPALWGVAE  120
                                    //
orf81.pep                                 QTVFEQLQKTPDGNWLFAYTSDHGQYVRQD  433
                                          ||||||||| ||||||||||||||||||||
orf81ng     ALLQPQDKVFGEADIVDKYDNTIHKTDQMIQTVFEQLQKQPDGNWLFAYTSDHGQYVRQD  433

orf81.pep   IYNQGTVQPDSYLVPLVLYSPDKAVQQAANQAFAPCEIAFHQQLSTFLIHTLGYDMPVSG  493
            ||||||||||||:|||||||||||||||||||||||||||||||||||||||||||||||
orf81ng     IYNQGTVQPDSYIVPLVLYSPDKAVQQAANQAFAPCEIAFHQQLSTFLIHTLGYDMPVSG  493

orf81.pep   CREGSVTGNLITGDAGSLNIRDGKAEYVYPQ  524
            |||||||||||||||||||||:|||||||||
orf81ng     CREGSVTGNLITGDAGSLNIRNGKAEYVYPQ  524
```

The complete length ORF81ng nucleotide sequence <SEQ ID 309> is:

```
   1  ATGAAAAAAT CCCTTTTCGT TCTCTTTCTG TATTCATCCC TACTTACCGC
  51  CAGCGAAATC GCCTATCGCT TTGTATTCGG AATTGAAACC TTACCGGCTG
 101  CAAAAATGGC GGAAACGTTT GCGCTGACAT TTATGATTGC TGCGCTGTAT
 151  CTGTTTGCGC GTTATAAGGC TTCGCGGCTG CTGATTGCGG TGTTTTTCGC
 201  GTTCAGCATG ATTGCCAACA ATGTGCATTA CGCGGTTTAT CAAAGCTGGA
 251  TGACGGGTAT TAACTATTGG CTGATGCTGA AAGAGGTTAC CGAAGTCGGC
 301  AGCGCGGGCG CGTCGATGTT GGATAAGTTG TGGCTGCCTG CTTTGTGGGG
 351  CGTGGCGGAA GTCATGTTGT TTTGCAGCCT TGCCAAGTTC CGCCGTAAGA
 401  CGCATTTTTC TGCCGATATA CTGTTTGCCT TCCTAATGCT GATGATTTTC
 451  GTGCGTTCGT TCGACACGAA ACAAGAGCAC GGTATTTCGC CCAAACCGAC
 501  ATACAGCCGC ATCAAAGCCA ATTATTTCAG CTTCGGTTAT TTTGTCGGGC
 551  GCGTGTTGCC GTATCAGTTG TTTGATTTAA GCAAGATCCC TGTGTTCAAA
 601  CAGCCTGCTC CAAGCAAAAT CGGGCAAGGC AGTATTCAAA ATATCGTCCT
 651  GATTATGGGC GAAAGCGAAA GCGCGGCGCA TTTGAAATTG TTTGGTTACG
 701  GGCGCGAAAC TTCGCCGTTT TTAACCCGGC TGTCGCAAGC CGATTTTAAG
 751  CCGATTGTGA AACAAAGTTA TTCCGCAGGC TTTATGACGG CAGTATCCCT
 801  GCCCAGTTTC TTTAACGTCA TACCGCACGC CAACGGCTTG GAACAAATCA
 851  GCGGCGGCGA TACCAATATG TTCCGCCTCG CCAAAGAGCA GGGCTATGAA
 901  ACGTATTTTT ACAGTGCCCA GGCTGAAAAC CAAATGGCAA TTTTGAACTT
 951  AATCGGTAAG AAATGGATAG ACCATCTGAT TCAGCCGACG CAACTTGGCT
1001  ACGGCAACGG CGACAATATG CCCGATGAGA AGCTGCTGCC GTTGTTCGAC
1051  AAAATCAATT TGCAGCAGGG CAGGCATTTT ATCGTGTTGC ACCAACGCGG
1101  TTCGCACGCC CCATACGGCG CATTGTTGCA GCCTCAAGAT AAAGTATTCG
1151  GCGAAGCCGA TATTGTGGAT AAGTACGACA ACACCATCCA CAAAACCGAC
1201  CAAATGATTC AAACCGTATT CGAGCAGCTG CAAAAGCAGC CTGACGGCAA
1251  CTGGCTGTTT GCCTATACCT CCGATCATGG CCAGTATGTG CGCCAAGATA
1301  TCTACAATCA AGGCACGGTG CAGCCCGACA GCTATATTGT GCCTCTGGTT
1351  TTGTACAGCC CGGATAAGGC CGTGCAACAG GCTGCCAACC AGGCTTTTGC
1401  GCCTTGCGAG ATTGCCTTCC ATCAGCAGCT TTCAACGTTC CTGATTCACA
1451  CGTTGGGCTA CGATATGCCG GTTTCAGGTT GTCGCGAAGG CTCGGTAACA
1501  GGCAACCTGA TTACGGGCGA TGCAGGCAGC TTGAACATTC GCAACGGCAA
1551  GGCGGAATAT GTTTATCCGC AATAA
```

This encodes a protein having amino acid sequence <SEQ ID 310>:

```
  1 MKKSLFVLFL YSSLLTASEI AYRFVFGIET LPAAKMAETF ALTFMIAALY
 51 LFARYKASRL LIAVFFAFSM IANNVHYAVY QSWMTGINYW LMLKEVTEVG
101 SAGASMLDKL WLPALWGVAE VMLFCSLAKF RRKTHFSADI LFAFLMLMIF
151 VRSFDTKQEH GISPKPTYSR IKANYFSFGY FVGRVLPYQL FDLSKIPVFK
201 QPAPSKIGQG SIQNIVLIMG ESESAAHLKL FGYGRETSPF LTRLSQADFK
251 PIVKQSYSAG FMTAVSLPSF FNVIPHANGL EQISGGDTNM FRLAKEQGYE
301 TYFYSAQAEN QMAILNLIGK KWIDHLIQPT QLGYGNGDNM PDEKLLPLFD
351 KINLQQGRHF IVLHQRGSHA PYGALLQPQD KVFGEADIVD KYDNTIHKTD

401 QMIQTVFEQL QKQPDGNWLF AYTSDHGQYV RQDIYNQGTV QPDSYIVPLV
451 LYSPDKAVQQ AANQAFAPCE IAFHQQLSTF LIHTLGYDMP VSGCREGSVT
501 GNLITGDAGS LNIRNGKAEY VYPQ*
```

ORF81ng and ORF81-1 show 96.4% identity in 524 aa overlap:

```
                        10        20        30        40        50        60
orf81ng-1.pep   MKKSLFVLFLYSSLLTASEIAYRFVFGIETLPAAKMAETFALTFMIAALYLFARYKASRL
                ||||:::| ||||||||||||||||||||||||||:||||||||:|||||||||||::||
orf81-1         MKKSFLTLVLYSSLLTASEIAYRFVFGIETLPAAKIAETFALTFVIAALYLFARYKVTRL
                        10        20        30        40        50        60

                        70        80        90       100       110       120
orf81ng-1.pep   LIAVFFAFSMIANNVHYAVYQSWMTGINYWLMLKEVTEVGSAGASMLDKLWLPALWGVAE
                |||||||||:|||||||||||||||||||||||||||||||||||||||||||:|||| |
orf81-1         LIAVFFAFSIIANNVHYAVYQSWMTGINYWLMLKEVTEVGSAGASMLDKLWLPVLWGVLE
                        70        80        90       100       110       120

                       130       140       150       160       170       180
orf81ng-1.pep   VMLFCSLAKFRRKTHFSADILFAFLMLMIFVRSFDTKQEHGISPKPTYSRIKANYFSFGY
                ||||||||||||||||||||||||||||||||||||||||||||||||||||||||||||
orf81-1         VMLFCSLAKFRRKTHFSADILFAFLMLMIFVRSFDTKQEHGISPKPTYSRIKANYFSFGY
                       130       140       150       160       170       180

                       190       200       210       220       230       240
orf81ng-1.pep   FVGRVLPYQLFDLSKIPVFKQPAPSKIGQGSIQNIVLIMGESESAAHLKLFGYGRETSPF
                ||||||||||||||:||:|||||||||||||:||||||||||||||||||||||||||||
orf81-1         FVGRVLPYQLFDLSRIPAFKQPAPSKIGQGSVQNIVLIMGESESAAHLKLFGYGRETSPF
                       190       200       210       220       230       240

                       250       260       270       280       290       300
orf81ng-1.pep   LTRLSQADFKPIVKQSYSAGFMTAVSLPSFFNVIPHANGLEQISGGDTNMFRLAKEQGYE
                ||||||||||||||||||||||||||||||||:|||||||||||||||||||||||||||
orf81-1         LTRLSQADFKPIVKQSYSAGFMTAVSLPSFFNAIPHANGLEQISGGDTNMFRLAKEQGYE
                       250       260       270       280       290       300

                       310       320       330       340       350       360
orf81ng-1.pep   TYFYSAQAENQMAILNLIGKKWIDHLIQPTQLGYGNGDNMPDEKLLPLFDKINLQQGRHF
                ||||||||||:||||||||||||||||||||||||||||||||||||||||||||||:||
orf81-1         TYFYSAQAENEMAILNLIGKKWIDHLIQPTQLGYGNGDNMPDEKLLPLFDKINLQQGKHF
                       310       320       330       340       350       360

                       370       380       390       400       410       420
orf81ng-1.pep   IVLHQRGSHAPYGALLQPQDKVFGEADIVDKYDNTIHKTDQMIQTVFEQLQKQPDGNWLF
                ||||||||||||||||||||||||||||||||||||||||||||||||||||||||||||
orf81-1         IVLHQRGSHAPYGALLQPQDKVFGEADIVDKYDNTIHKTDQMIQTVFEQLQKQPDGNWLF
                       370       380       390       400       410       420

                       430       440       450       460       470       480
orf81ng-1.pep   AYTSDHGQYVRQDIYNQGTVQPDSYIVPLVLYSPDKAVQQAANQAFAPCEIAFHQQLSTF
                |||||||||||||||||||||||||:||||||||||||||||||||||||||||||||||
orf81-1         AYTSDHGQYVRQDIYNQGTVQPDSYLVPLVLYSPDKAVQQAANQAFAPCEIAFHQQLSTF
                       430       440       450       460       470       480

                       490       500       510       520
orf81ng-1.pep   LIHTLGYDMPVSGCREGSVTGNLITGDAGSLNIRNGKAEYVYPQX
                ||||||||||||||||||||||||||||||||||:||||||||||
orf81-1         LIHTLGYDMPVSGCREGSVTGNLITGDAGSLNIRDGKAEYVYPQX
                       490       500       510       520
```

Furthermore, ORF81ng shows significant homology to an *E.coli* OMP:

```
gi|1256380 (U50906) outer membrane adherence protein-associated protein [E.
coli] Length = 547
 Score = 87.4 bits (213), Expect = 2e-16
 Identities = 122/468 (26%), Positives = 198/468 (42%), Gaps = 70/468 (14%)

Query: 25  VFGIETLPAAKMAETFA-LTFMIAALYLFARYKAS--RLLIAVFFAFSMIANNVHYAVYQ 81
            VFGI  L A+  A      L F +  + +  R  +    RLL+A  F    + A ++   ++Y
Sbjct: 29  VFGITNLVASSGAHMVQRLLFFVLTILVVKRISSLPLRLLVAAPFVL-LTAADMSISLY- 86

Query: 82  SWMT-------GINYWLMLKEVTEVGSAGASMLDKLWLPALWGVAEVMLFCSLAKFRRKT 134
            SW T        G     ++  +  EV     A ML  ++ P L    A + L        +
Sbjct: 87  SWCTFGTTFNDGFAISVLQSDPDEV----AKMLG-MYSPYLCAFAFLSLLFLAVIIKYDV 141

Query: 135 HFSADILFAFLMLMIFVRSF---------DTKQEHGISPKPTYSRIKAN--YFSFGYFVG 183
                 +     L+L++    S              D K ++   SP      SR        +F+  YF
Sbjct: 142 SLPTKKVTGILLLIVISGSLFSACQFAYKDAKNKNAFSPYILASRFATYTPFFNLNYFAL 201

Query: 184 RVLPYQ--LFDLSKIPVFKQPAPSKIGQGSIQNIVLIMGESESAAHLKLFGYGRETSPFL 241
            +Q  L    + +P F+        +    I      VLI+GES       ++ L+GY  R T+P +
Sbjct: 202 AAKEHQRLLSIANTVPYFQL----SVRDTGIDTYVLIVGESVRVDNMSLYGYTRSTTPQV 257

Query: 242 TRLSQADFKPIVKQSYSAGFMTAVSLP---SFFNVIPHANGLEQISGGDTNMFRLAKEQG 298
              +Q      +  Q+ S     TA+S+P    +   +V+ H        I         N+   +A + G
Sbjct: 258 E--AQRKQIKLFNQAISGAPYTALSVPLSLTADSVLSH-----DIHNYPDNIINMANQAG 310

Query: 299 YETYFYSAQA---ENQMAILNLIGKKWIDHLIQPTQLGYGNGDNMPDEKLLPLFDKINLQ 355
            ++T++  S+Q+     +N   A++  ++                ++   +   Y   G      DE LLP       +     Q
Sbjct: 311 FQTFWLSSQSAFRQNGTAVTSI--------AMRAMETVYVRGF---DELLLPHLSQALQQ 359

Query: 356 --QGRHFIVLHQRGSHAPYGALLQPQDKVFGEADIVDK-YDNTIHKTDQMIQTVFEQLQK 412
              Q +   IVLH   GSH  P   +             VF    D    D   YDN+IH  TD ++     VFE  L+
Sbjct: 360 NTQQKKLIVLHLNGSHEPACSAYPQSSAVFQPQDDQDACYDNSIHYTDSLLGQVFELLK- 418

Query: 413 QPDGNWLFAYTSDHG---QYVRQDIYNQG--TVQPDSYIVPL-VLYSP 454
              D        Y +DHG        ++++Y   G          +Y VP+ + YSP
Sbjct: 419 --DRRASVMYFADHGLERDPTKKNVYFHGGREASQQAYHVPMFIWYSP 464
```

Based on this analysis, including the presence of a putative leader sequence (double-underlined) and several putative transmembrane domains (single-underlined) in the gonococcal protein, it is predicted that the proteins from *N.meningitidis* and *N.gonorrhoeae,* and their epitopes, could be useful antigens for vaccines or diagnostics, or for raising antibodies.

**Example 37**

The following partial DNA sequence was identified in *N.meningitidis* <SEQ ID 311>:

```
  1  ...ACCCTGCTCC TCTTCATCCC CCTCGTCCTC ACAC.GTGCG GCACACTGAC
 51     CGGCATACTC GCCCaCGGCG GCGGCAAACG CTTTGCCGTC GAACAAGAAC
101     TCGTCGCCGC ATCGTCCCGC GCCGCCGTCA AAGAAATGGA TTTGTCCGCC
151     yTAAAAGGAC GCAAAGCCGC CyTTTACGTC TCCGTTATGG GCGACCAAGG
201     TTCGGGCAAC ATAAGCGGCG GACGCTACTC TATCGACGCA CTGATACGCG
251     GCGGCTACCA CAACAACCCC GAAAGTGCCA CCCAATACAG CTACCCCGCC
301     TACGACACTA CCGCCACCAC CAAATCCGAC GCGCTCTCCA GCGTAACCAC
351     TTCCACATCG CTTTTGAACG CCCCCGCCGC CGyCyTGACG AAAAACAGCG
401     GACGCAAAGG CGAACGcTCC GCCGGACTGT CCGTCAACGG CACGGGCGAC
451     TACCGCAACG AAACCCTGCT CGCCAACCCC CGCGACGTTT CCTTCCTGAC
501     CAACCTCATC CAAACCGTCT TCTACCTGCG CGGCATCGAA GTCgTACCGC
551     CCGrATACGC CGACACCGAC GTATTCGTAA CCGTCGACGT A...
```

This corresponds to the amino acid sequence <SEQ ID 312; ORF83>:

```
  1 ..TLLLFIPLVL TXCGTLTGIL AHGGGKRFAV EQELVAASSR AAVKEMDLSA
 51   LKGRKAAXYV SVMGDQGSGN ISGGRYSIDA LIRGGYHNNP ESATQYSYPA
101   YDTTATTKSD ALSSVTTSTS LLNAPAAXLT KNSGRKGERS AGLSVNGTGD
151   YRNETLLANP RDVSFLTNLI QTVFYLRGIE VVPPXYADTD VFVTVDV..
```

Further work revealed the complete nucleotide sequence <SEQ ID 313>:

```
  1  ATGAAAACCC TGCTCCTCCT CATCCCCCTC GTCCTCACAG CCTGCGGCAC
 51  ACTGACCGGC ATACCCGCCC ACGGCGGCGG CAAACGCTTT GCCGTCGAAC
101  AAGAACTCGT CGCCGCATCG TCCCGCGCCG CCGTCAAAGA AATGGATTTG
151  TCCGCCCTAA AAGGACGCAA AGCCGCCCTT TACGTCTCCG TTATGGGCGA
201  CCAAGGTTCG GGCAACATAA GCGGCGGACG CTACTCTATC GACGCACTGA
251  TACGCGGCGG CTACCACAAC AACCCCGAAA GTGCCACCCA ATACAGCTAC
301  CCCGCCTACG ACACTACCGC CACCACCAAA TCCGACGCGC TCTCCAGCGT
```

```
351  AACCACTTCC ACATCGCTTT TGAACGCCCC CGCCGCCGCC CTGACGAAAA
401  ACAGCGGACG CAAAGGCGAA CGCTCCGCCG GACTGTCCGT CAACGGCACG
451  GGCGACTACC GCAACGAAAC CCTGCTCGCC AACCCCCGCG ACGTTTCCTT
501  CCTGACCAAC CTCATCCAAA CCGTCTTCTA CCTGCGCGGC ATCGAAGTCG
551  TACCGCCCGA ATACGCCGAC ACCGACGTAT TCGTAACCGT CGACGTATTC
601  GGCACCGTCC GCAGCCGTAC CGAACTGCAC CTCTACAACG CCGAAACCCT
651  TAAAGCCCAA ACCAAGCTCG AATATTTCGC CGTTGACCGC GACAGCCGGA
701  AACTGCTGAT TACCCCTAAA ACCGCCGCCT ACGAATCCCA ATACCAAGAA
751  CAATACGCCC TTTGGACCGG CCCTTACAAA GTCAGCAAAA CCGTCAAAGC
801  CTCAGACCGC CTGATGGTCG ATTTCTCCGA CATTACCCCC TACGGCGACA
851  CAACCGCCCA AAACCGTCCC GACTTCAAAC AAAACAACGG TAAAAAACCC
901  GATGTCGGCA ACGAAGTCAT CCGCCGCCGC AAAGGAGGAT AA
```

This corresponds to the amino acid sequence <SEQ ID 314; ORF83-1>:

```
  1  MKTLLLLIPL VLTACGTLTG IPAHGGGKRF AVEQELVAAS SRAAVKEMDL
 51  SALKGRKAAL YVSVMGDQGS GNISGGRYSI DALIRGGYHN NPESATQYSY
101  PAYDTTATTK SDALSSVTTS TSLLNAPAAA LTKNSGRKGE RSAGLSVNGT
151  GDYRNETLLA NPRDVSFLTN LIQTVFYLRG IEVVPPEYAD TDVFVTVDVF
201  GTVRSRTELH LYNAETLKAQ TKLEYFAVDR DSRKLLITPK TAAYESQYQE
251  QYALWTGPYK VSKTVKASDR LMVDFSDITP YGDTTAQNRP DFKQNNGKKP
301  DVGNEVIRRR KGG*
```

Computer analysis of this amino acid sequence gave the following results:

Homology with a predicted ORF from *N.meningitidis* (strain A)

ORF83 shows 96.4% identity over a 197aa overlap with an ORF (ORF83a) from strain A of *N. meningitidis:*

```
                   10        20        30        40        50
orf83.pep   TLLLFIPLVLTXCGTLTGILAHGGGKRFAVEQELVAASSRAAVKEMDLSALKGRKAAX
             ||| :|||||| ||||||| ||||||||||||||||||||||||||||||||||||
orf83a    MKTLLXLIPLVLTACGTLTGIPAHGGGKRFAVEQELVAASSRAAVKEMDLSALKGRKAAL
                  10        20        30        40        50        60

          60        70        80        90       100       110
orf83.pep   YVSVMGDQGSGNISGGRYSIDALIRGGYHNNPESATQYSYPAYDTTATTKSDALSSVTTS
            ||||||||||||||||||||||||||||||||||||||||||||||||||||||||||||
orf83a      YVSVMGDQGSGNISGGRYSIDALIRGGYHNNPESATQYSYPAYDTTATTKSDALSSVTTS
                  70        80        90       100       110       120

         120       130       140       150       160       170
orf83.pep   TSLLNAPAAXLTKNSGRKGERSAGLSVNGTGDYRNETLLANPRDVSFLTNLIQTVFYLRG
            ||||||||| ||||||||||||||||||||||||||||||||||||||||||||||||||
orf83a      TSLLNAPAAALTKNSGRKGERSAGLSVNGTGDYRNETLLANPRDVSFLTNLIQTVFYLRG
                 130       140       150       160       170       180

         180       190
orf83.pep   IEVVPPXYADTDVFVTVDV
            |||||| ||||||||||||
orf83a      IEVVPPEYADTDVFVTVDVFGTVRSRTELHLYNAETLKAQTKLEYFAVDRDSRKLLIAPK
                 190       200       210       220       230       240
```

The complete length ORF83a nucleotide sequence <SEQ ID 315> is:

```
  1  ATGAAAACCC TGCTCNTCCT CATCCCCCTC GTCCTCACAG CCTGCGGCAC
 51  ACTGACCGGC ATACCCGCCC ACGGCGGCGG CAAACGCTTT GCCGTCGAAC
101  AAGAACTCGT CGCCGCATCG TCCCGCGCCG CCGTCAAAGA AATGGACTTG
151  TCCGCCCTGA AAGGACGCAA AGCCGCCCTT TACGTCTCCG TTATGGGCGA
201  CCAAGGTTCG GGCAACATAA GCGGCGGACG CTACTCTATC GACGCACTGA
251  TACGCGGCGG CTACCACAAC AACCCCGAAA GTGCCACCCA ATACAGCTAC
301  CCCGCCTACG ACACTACCGC CACCACCAAA TCCGACGCGC TCTCCAGCGT
351  AACCACTTCC ACATCGCTTT TGAACGCCCC CGCCGCCGCC CTGACGAAAA
401  ACAGCGGACG CAAAGGCGAA CGCTCCGCCG GACTGTCCGT CAACGGCACG
451  GGCGACTACC GCAACGAAAC CCTGCTCGCC AACCCCCGCG ACGTTTCCTT
501  CCTGACCAAC CTCATCCAAA CCGTCTTCTA CCTGCGCGGC ATCGAAGTCG
551  TACCGCCCGA ATACGCCGAC ACCGACGTAT TCGTAACCGT CGACGTATTC
601  GGCACCGTCC GCAGCCGCAC CGAACTGCAC CTCTACAACG CCGAAACCCT
651  TAAAGCCCAA ACCAAGCTCG AATATTTCGC CGTTGACCGC GACAGCCGGA

701  AACTGCTGAT TGCCCCTAAA ACCGCCGCCT ACGAATCCCA ATACCAAGAA
751  CAATACGCCC TCTGGATGGG ACCTTACAGC GTCGGCAAAA CCGTCAAAGC
801  CTCAGACCGC CTGATGGTCG ATTTCTCCGA CATCACCCCC TACGGCGACA
851  CAACCGCCCA AAACCGTCCC GACTTCAAAC AAAACAACGG TAAAAAACCC
901  GATGTCGGCA ACGAAGTCAT CCGCCGCCGC AAAGGAGGAT AA
```

This encodes a protein having amino acid sequence <SEQ ID 316>:

```
  1  MKTLLXLIPL VLTACGTLTG IPAHGGGKRF AVEQELVAAS SRAAVKEMDL
 51  SALKGRKAAL YVSVMGDQGS GNISGGRYSI DALIRGGYHN NPESATQYSY
101  PAYDTTATTK SDALSSVTTS TSLLNAPAAA LTKNSGRKGE RSAGLSVNGT
151  GDYRNETLLA NPRDVSFLTN LIQTVFYLRG IEVVPPEYAD TDVFVTVDVF
201  GTVRSRTELH LYNAETLKAQ TKLEYFAVDR DSRKLLIAPK TAAYESQYQE
251  QYALWMGPYS VGKTVKASDR LMVDFSDITP YGDTTAQNRP DFKQNNGKKP
301  DVGNEVIRRR KGG*
```

ORF83a and ORF83-1 show 98.4% identity in 313 aa overlap:

```
                    10        20        30        40        50        60
orf83a.pep  MKTLLXLIPLVLTACGTLTGIPAHGGGKRFAVEQELVAASSRAAVKEMDLSALKGRKAAL
            ||||| ||||||||||||||||||||||||||||||||||||||||||||||||||||||
orf83-1     MKTLLLLIPLVLTACGTLTGIPAHGGGKRFAVEQELVAASSRAAVKEMDLSALKGRKAAL
                    10        20        30        40        50        60

                    70        80        90       100       110       120
orf83a.pep  YVSVMGDQGSGNISGGRYSIDALIRGGYHNNPESATQYSYPAYDTTATTKSDALSSVTTS
            ||||||||||||||||||||||||||||||||||||||||||||||||||||||||||||
orf83-1     YVSVMGDQGSGNISGGRYSIDALIRGGYHNNPESATQYSYPAYDTTATTKSDALSSVTTS
                    70        80        90       100       110       120

                   130       140       150       160       170       180
orf83a.pep  TSLLNAPAAALTKNSGRKGERSAGLSVNGTGDYRNETLLANPRDVSFLTNLIQTVFYLRG
            ||||||||||||||||||||||||||||||||||||||||||||||||||||||||||||
orf83-1     TSLLNAPAAALTKNSGRKGERSAGLSVNGTGDYRNETLLANPRDVSFLTNLIQTVFYLRG
                   130       140       150       160       170       180

                   190       200       210       220       230       240
orf83a.pep  IEVVPPEYADTDVFVTVDVFGTVRSRTELHLYNAETLKAQTKLEYFAVDRDSRKLLIAPK
            |||||||||||||||||||||||||||||||||||||||||||||||||||||||||:||
orf83-1     IEVVPPEYADTDVFVTVDVFGTVRSRTELHLYNAETLKAQTKLEYFAVDRDSRKLLITPK
                   190       200       210       220       230       240

                   250       260       270       280       290       300
orf83a.pep  TAAYESQYQEQYALWMGPYSVGKTVKASDRLMVDFSDITPYGDTTAQNRPDFKQNNGKKP
            |||||||||||||| |||:|:|||||||||||||||||||||||||||||||||||||||
orf83-1     TAAYESQYQEQYALWTGPYKVSKTVKASDRLMVDFSDITPYGDTTAQNRPDFKQNNGKKP
                   250       260       270       280       290       300

                   310
orf83a.pep  DVGNEVIRRRKGGX
            ||||||||||||||
orf83-1     DVGNEVIRRRKGGX
                   310
```

Homology with a predicted ORF from *N.gonorrhoeae*

ORF83 shows 94.9% identity over a 197aa overlap with a predicted ORF (ORF83.ng) from *N. gonorrhoeae:*

```
orf83.pep     TLLLFIPLVLTXCGTLTGILAHGGGKRFAVEQELVAASSRAAVKEMDLSALKGRKAAX   58
              ||||:||||||| ||||||| ||||||||||||||||||||||||||||||||||||||
orf83ng     MKTLLLLIPLVLTACGTLTGIPAHGGGKRFAVEQELVAASSRAAVKEMDLSALKGRKAAL   60

orf83.pep   YVSVMGDQGSGNISGGRYSIDALIRGGYHNNPESATQYSYPAYDTTATTKSDALSSVTTS  118
            |||||||||||||||||||||||||||||||:|||:||||||||||||||||||:||||
orf83ng     YVSVMGDQGSGNISGGRYSIDALIRGGYHNNPDSATRYSYPAYDTTATTKSDALSGVTTS  120

orf83.pep   TSLLNAPAAXLTKNSGRKGERSAGLSVNGTGDYRNETLLANPRDVSFLTNLIQTVFYLRG  178
            ||||||||| ||||:|||||||||||||||||||||||||||||||||||||||||||||
orf83ng     TSLLNAPAAALTKNNGRKGERSAGLSVNGTGDYRNETLLANPRDVSFLTNLIQTVFYLRG  180

orf83.pep   IEVVPPXYADTDVFVTVDV                                          197
            |||||| ||||||||||||
orf83ng     IEVVPPEYADTDVFVTVDVFGTVRSRTELHLYNAETLKAQTKLEYFAVDRDSRKLLIAPK  240
```

The complete length ORF83ng nucleotide sequence <SEQ ID 317> is:

```
  1 ATGAAAACCC TGCTCCTCCT CATCCCCCTC GTACTCACCG CCTGCGGCAC
 51 ACTGACCGGC ATACCCGCCC ACGGCGGCGG CAAACGCTTT GCCGTCGAAC
101 AGGAACTCGT CGCCGCATCG TCCCGCGCCG CCGTCAAAGA AATGGACTTG
151 TCCGCCCTGA AAGGACGCAA AGCCGCCCTT TACGTCTCCG TTATGGGCGA
201 CCAAGGTTCG GGCAACATAA GCGGCGGACG CTACTCCATC GACGCACTGA
251 TACGCGGCGG CTACCACAAC AACCCCGACA GCGCCACCCG ATACAGCTAC
301 CCCGCCTATG ACACTACCGC CACCACCAAA TCCGACGCGC TCTCCGGCGT
351 AACCACTTCC ACATCGCTTT TGAACGCCCC CGCCGCCGCC CTGACGAAAA
401 ACAACGGACG CAAAGGCGAA CGCTCCGCCG GACTGTCCGT CAACGGCACG
451 GGCGACTACC GCAACGAAAC CCTGCTCGCC AACCCCCGCG ACGTTTCCTT
501 CCTGACCAAC CTCATCCAAA CCGTCTTCTA CCTGCGCGGC ATCGAAGTCG
551 TACCGCCCGA ATACGCCGAC ACCGACGTAT TCGTAACCGT CGACGTATTC
601 GGCACCGTCC GCAGCCGTAC CGAACTGCAC CTCTACAACG CCGAAACCCT
651 TAAAGCCCAA ACCAAGCTCG AATATTTCGC CGTCGACCGC GACAGCCGGA
701 AACTGCTGAT TGCCCCTAAA ACCGCCGCCT ACGAATCCCA ATACCAAGAA
751 CAATACGCCC TCTGGATGGG ACCTTACAGC GTCGGCAAAA CCGTCAAAGC
801 CTCAGACCGC CTGATGGTCG ATTTCTCCGA CATCACCCCC TACGGCGACA
851 CAACCGCCCA AAACCGTCCC GACTTCAAAC AAAACAACGG TAAAAACCCC
901 GATGTCGGCA ACGAAGTCAT CCGCCGCCGC AAAGGAGGAT AA
```

This encodes a protein having amino acid sequence <SEQ ID 318>:

```
  1 MKTLLLLIPL VLTACGTLTG IPAHGGGKRF AVEQELVAAS SRAAVKEMDL
 51 SALKGRKAAL YVSVMGDQGS GNISGGRYSI DALIRGGYHN NPDSATRYSY
101 PAYDTTATTK SDALSGVTTS TSLLNAPAAA LTKNNGRKGE RSAGLSVNGT
151 GDYRNETLLA NPRDVSFLTN LIQTVFYLRG IEVVPPEYAD TDVFVTVDVF
201 GTVRSRTELH LYNAETLKAQ TKLEYFAVDR DSRKLLIAPK TAAYESQYQE
251 QYALWMGPYS VGKTVKASDR LMVDFSDITP YGDTTAQNRP DFKQNNGKNP
301 DVGNEVIRRR KGG*
```

ORF83ng and ORF83-1 show 97.1% identity in 313 aa overlap

```
                      10        20        30        40        50        60
orf83-1.pep  MKTLLLLIPLVLTACGTLTGIPAHGGGKRFAVEQELVAASSRAAVKEMDLSALKGRKAAL
             ||||||||||||||||||||||||||||||||||||||||||||||||||||||||||||
orf83ng      MKTLLLLIPLVLTACGTLTGIPAHGGGKRFAVEQELVAASSRAAVKEMDLSALKGRKAAL
                      10        20        30        40        50        60

                      70        80        90       100       110       120
orf83-1.pep  YVSVMGDQGSGNISGGRYSIDALIRGGYHNNPESATQYSYPAYDTTATTKSDALSSVTTS
             |||||||||||||||||||||||||||||||:|||:|||||||||||||||||||:||||
orf83ng      YVSVMGDQGSGNISGGRYSIDALIRGGYHNNPDSATRYSYPAYDTTATTKSDALSGVTTS
                      70        80        90       100       110       120

                     130       140       150       160       170       180
orf83-1.pep  TSLLNAPAAALTKNSGRKGERSAGLSVNGTGDYRNETLLANPRDVSFLTNLIQTVFYLRG
             ||||||||||||||:|||||||||||||||||||||||||||||||||||||||||||||
orf83ng      TSLLNAPAAALTKNNGRKGERSAGLSVNGTGDYRNETLLANPRDVSFLTNLIQTVFYLRG
                     130       140       150       160       170       180

                     190       200       210       220       230       240
orf83-1.pep  IEVVPPEYADTDVFVTVDVFGTVRSRTELHLYNAETLKAQTKLEYFAVDRDSRKLLITPK
             |||||||||||||||||||||||||||||||||||||||||||||||||||||||||:||
orf83ng      IEVVPPEYADTDVFVTVDVFGTVRSRTELHLYNAETLKAQTKLEYFAVDRDSRKLLIAPK
                     190       200       210       220       230       240

                     250       260       270       280       290       300
orf83-1.pep  TAAYESQYQEQYALWTGPYKVSKTVKASDRLMVDFSDITPYGDTTAQNRPDFKQNNGKKP
             ||||||||||||||| |||:|:||||||||||||||||||||||||||||||||||||:|
orf83ng      TAAYESQYQEQYALWMGPYSVGKTVKASDRLMVDFSDITPYGDTTAQNRPDFKQNNGKNP
                     250       260       270       280       290       300

                     310

orf83-1.pep  DVGNEVIRRRKGGX
             ||||||||||||||
orf83ng      DVGNEVIRRRKGGX
                     310
```

Based on this analysis, including the presence of a putative ATP/GTP-binding site motif A (P-loop) in the gonococcal protein (double-underlined) and a putative prokaryotic membrane lipoprotein lipid attachment site (single-underlined), it is predicted that the proteins from *N.meningitidis* and *N.gonorrhoeae,* and their epitopes, could be useful antigens for vaccines or diagnostics, or for raising antibodies.

**Example 38**

The following DNA sequence, believed to be complete, was identified in *N.meningitidis* <SEQ ID 319>:

```
   1  ATGGCAGAGA TCTGTTTGAT AACCGGCACG CCCGGTTCAG GGAAAACATT
  51  AAAAATGGTT TCCATGATGG CGAATGATGA AATGTTTAAG CCTGATGAAA
 101  AAGCCATACG CCGTAAAGTA TTTACGAACA TAAAAGGCTT GAAAATACCG
 151  CACACCTACA TAGAAACGGA CGCAAAAAAG CTGCCGAAAT CGACAGATGA
 201  GCAGCTTTCG GCGCATGATA TGTACGAATG GATAAAGAAG CCCGAAAATA
 251  TCGGGTCTAT TGTCATTGTA GATGAAGCTC AAGACGTATG GCCGGCACGC
 301  TCGGCAGGTT CAAAAATCCC TGAAAATGTC CAATGGCTGA ATACGCACAG
 351  ACATCAGGGC ATTGATATAT TTGTTTTGAC TCAAGGTCCT AAGCTTCTAG
 401  ATCAAAATCT TAGAACGCTT GTACGGAAAC ATTACCACAT CGCTTCAAAC
 451  AAGATGGGTA TGCGTACGCT TTTAGAATGG AAAATATGCG CGGACGATCC
 501  CGTAAAAATG GCATCAAGCG CATTCTCCAG TATCTATACA CTGGATAAAA
 551  AAGTTTATGA CTTGTAysrr TmmGCGGAAG TTCATACCGT AAATAAGGTC
 601  AAGCGGTCAA AGTGGTTTTA CACTCTGCCa GTAATAGTAT TGCTGATTCC
 651  CGTGTTTGTC GGCCTGTCCT ATAAAATGTT GagCaGTTAC GGAAAAAAAC
 701  aGGAAGAACC CGCAGCACAA GAATCGGCGG CAACAGAACA GCAGGCAGTA
 751  CTTCCGGATA AAACAGAAGG CGAGCCGGTA AATAACGGCA ACCTTACCGC
 801  AGATATGTTT GTTCCGACAT TGTCCGAaAA ACCCGrAAGC AAGCcgaTTT
 851  ATAACGGTGT AAGGCAGGTA AGAACCTTTG AATATATAGC AGGCTGTATA
 901  GAAGGCGGAA GAACCGGATG CGCCTGCTAT TCGCaTCAAG GGACGGCATt
 951  gaAAGAAGTG ACGGaGTTGA TGTGccaAgG aCTATGTaAA AAacGGCTTG
1001  CCGTTTAACC CaTACAAAGA AGAAAGCCAA GGGCAGGAAG TTCAGCAAAG
1051  CGCGCAgCAA CATTCGGACA GGGCGcCAAG TTGCCACATT GGGCGGAAAA
1101  CCGTAGCAGA ACCTAATGTA CGATAATTGG GAAGAACGCG GGAAACCGTT
1151  TGAAGGAATC GGaCGGGGGC GTGGTCGGAT CGGCAAACTG A
```

This corresponds to the amino acid sequence <SEQ ID 320; ORF84>:

```
  1  MAEICLITGT PGSGKTLKMV SMMANDEMFK PDEKAIRRKV FTNIKGLKIP
 51  HTYIETDAKK LPKSTDEQLS AHDMYEWIKK PENIGSIVIV DEAQDVWPAR
101  SAGSKIPENV QWLNTHRHQG IDIFVLTQGP KLLDQNLRTL VRKHYHIASN
151  KMGMRTLLEW KICADDPVKM ASSAFSSIYT LDKKVYDLYX XAEVHTVNKV
201  KRSKWFYTLP VIVLLIPVFV GLSYKMLSSY GKKQEEPAAQ ESAATEQQAV
251  LPDKTEGEPV NNGNLTADMF VPTLSEKPXS KPIYNGVRQV RTFEYIAGCI
301  EGGRTGCACY SHQGTALKEV TELMCKDYVK NGLPFNPYKE ESQGQEVQQS
351  AQQHSDRAQV ATLGGKPXQN LMYDNWEERG KPFEGIGGGV VGSAN*
```

Further work revealed the complete nucleotide sequence <SEQ ID 321>:

```
  1  ATGGCAGAGA TCTGTTTGAT AACCGGCACG CCCGGTTCAG GGAAAACATT
 51  AAAAATGGTT TCCATGATGG CGAATGATGA AATGTTTAAG CCTGATGAAA
101  ACGGCATACG CCGTAAAGTA TTTACGAACA TAAAAGGCTT GAAAATACCG
151  CACACCTACA TAGAAACGGA CGCAAAAAAG CTGCCGAAAT CGACAGATGA
201  GCAGCTTTCG GCGCATGATA TGTACGAATG GATAAAGAAG CCCGAAAATA
251  TCGGGTCTAT TGTCATTGTA GATGAAGCTC AAGACGTATG GCCGGCACGC
301  TCGGCAGGTT CAAAAATCCC TGAAAATGTC CAATGGCTGA ATACGCACAG
351  ACATCAGGGC ATTGATATAT TTGTTTTGAC TCAAGGTCCT AAGCTTCTAG
401  ATCAAAATCT TAGAACGCTT GTACGGAAAC ATTACCACAT CGCTTCAAAC
```

```
 451  AAGATGGGTA TGCGTACGCT TTTAGAATGG AAAATATGCG CGGACGATCC
 501  CGTAAAAATG GCATCAAGCG CATTCTCCAG TATCTATACA CTGGATAAAA
 551  AAGTTTATGA CTTGTACGAA TCAGCGGAAG TTCATACCGT AAATAAGGTC
 601  AAGCGGTCAA AGTGGTTTTA CACTCTGCCA GTAATAGTAT TGCTGATTCC
 651  CGTGTTTGTC GGCCTGTCCT ATAAAATGTT GAGCAGTTAC GGAAAAAAAC
 701  AGGAAGAACC CGCAGCACAA GAATCGGCGG CAACAGAACA GCAGGCAGTA
 751  CTTCCGGATA AAACAGAAGG CGAGCCGGTA AATAACGGCA ACCTTACCGC
 801  AGATATGTTT GTTCCGACAT TGTCCGAAAA ACCCGAAAGC AAGCCGATTT
 851  ATAACGGTGT AAGGCAGGTA AGAACCTTTG AATATATAGC AGGCTGTATA
 901  GAAGGCGGAA GAACCGGATG CGCCTGCTAT TCGCATCAAG GGACGGCATT
 951  GAAAGAAGTG ACGGAGTTGA TGTGCAAGGA CTATGTAAAA AACGGCTTGC
1001  CGTTTAACCC ATACAAAGAA GAAAGCCAAG GGCAGGAAGT TCAGCAAAGC
1051  GCGCAGCAAC ATTCGGACAG GGCGCAAGTT GCCACATTGG GCGGAAAACC
1101  GTAGCAGAAC CTAATGTACG ATAATTGGGA AGAACGCGGG AAACCGTTTG
1151  AAGGAATCGG CGGGGGCGTG GTCGGATCGG CAAACTGA
```

This corresponds to the amino acid sequence <SEQ ID 322; ORF84-1>:

```
  1  MAEICLITGT PGSGKTLKMV SMMANDEMFK PDENGIRRKV FTNIKGLKIP
 51  HTYIETDAKK LPKSTDEQLS AHDMYEWIKK PENIGSIVIV DEAQDVWPAR
101  SAGSKIPENV QWLNTHRHQG IDIFVLTQGP KLLDQNLRTL VRKHYHIASN
151  KMGMRTLLEW KICADDPVKM ASSAFSSIYT LDKKVYDLYE SAEVHTVNKV
201  KRSKWFYTLP VIVLLIPVFV GLSYKMLSSY GKKQEEPAAQ ESAATEQQAV
251  LPDKTEGEPV NNGNLTADMF VPTLSEKPES KPIYNGVRQV RTFEYIAGCI
301  EGGRTGCACY SHQGTALKEV TELMCKDYVK NGLPFNPYKE ESQGQEVQQS
351  AQQHSDRAQV ATLGGKP*QN LMYDNWEERG KPFEGIGGGV VGSAN*
```

Computer analysis of this amino acid sequence gave the following results:

<u>Homology with a predicted ORF from *N.meningitidis* (strain A)</u>

ORF84 shows 93.9% identity over a 395aa overlap with an ORF (ORF84a) from strain A of *N. meningitidis:*

```
                    10        20        30        40        50        60
orf84.pep   MAEICLITGTPGSGKTLKMVSMMANDEMFKPDEKAIRRKVFTNIKGLKIPHTYIETDAKK
            |||||||||||||||||||||||||||||||||||::|||||||||||||||||||||||
orf84a      MAEICLITGTPGSGKTLKMVSMMANDEMFKPDENGIRRKVFTNIKGLKIPHTYIETDAKK
                    10        20        30        40        50        60

                    70        80        90       100       110       120
orf84.pep   LPKSTDEQLSAHDMYEWIKKPENIGSIVIVDEAQDVWPARSAGSKIPENVQWLNTHRHQG
            ||||||||||||||||||||||||||||||||||||||||||||||||||||||||||||
orf84a      LPKSTDEQLSAHDMYEWIKKPENIGSIVIVDEAQDVWPARSAGSKIPENVQWLNTHRHQG
                    70        80        90       100       110       120

                   130       140       150       160       170       180
orf84.pep   IDIFVLTQGPKLLDQNLRTLVRKHYHIASNKMGMRTLLEWKICADDPVKMASSAFSSIYT
            ||||||||| ||||||||||||||||||||||||||||||||||||||||||||||||||
orf84a      IDIFVLTQGSKLLDQNLRTLVRKHYHIASNKMGMRTLLEWKICADDPVKMASSAFSSIYT
                   130       140       150       160       170       180

                   190       200       210       220       230       240
orf84.pep   LDKKVYDLYXXAEVHTVNKVKRSKWFYTLPVIVLLIPVFVGLSYKMLSSYGKKQEEPAAQ
            |||||||||  ||||||||||||||||||||:||||||||||||||||||||||||||||
orf84a      LDKKVYDLYESAEVHTVNKVKRSKWFYTLPVIILLIPVFVGLSYKMLSSYGKKQEEPAAQ
                   190       200       210       220       230       240

                   250       260       270       280       290       300
orf84.pep   ESAATEQQAVLPDKTEGEPVNNGNLTADMFVPTLSEKPXSKPIYNGVRQVRTFEYIAGCI
            ||||||:|||: |||||||||||||||||||||||||| ||||||||||||||||||||:
orf84a      ESAATEHQAVFQDKTEGEPVNNGNLTADMFVPTLSEKPESKPIYNGVRQVRTFEYIAGCV
                   250       260       270       280       290       300

                   310       320       330       340       350       360
orf84.pep   EGGRTGCACYSHQGTALKEVTELMCKDYVKNGLPFNPYKEESQGQEVQQSAQQHSDRAQV
            |||||||:|||||||||||:|: |||||::|||||||||||||||::|||| |:|||| ||
orf84a      EGGRTGCTCYSHQGTALKEITKEMCKDYARNGLPFNPYKEESQGRDVQQSEQHHSDRPQV
                   310       320       330       340       350       360

                   370       380       390
orf84.pep   ATLGGKPXQNLMYDNWEERGKPFEGIGGVVGSANX
            ||||||| |||||||||:|||||||||||||||||
orf84a      ATLGGKPWQNLMYDNWQERGKPFEGIGGVVGSANX
                   370       380       390
```

In the alignment, FYTLPVIVLLIPVFVGL (orf84.pep) and FYTLPVIILLIPVFVGL (orf84a), positions 206–222, are underlined.

The complete length ORF84a nucleotide sequence <SEQ ID 323> is:

```
   1  ATGGCAGAGA TCTGTTTGAT AACCGGCACG CCCGGTTCAG GGAAAACATT
  51  AAAAATGGTT TCCATGATGG CAAACGATGA AATGTTTAAG CCGGATGAAA
 101  ACGGCATACG CCGTAAAGTA TTTACGAACA TCAAAGGCTT GAAGATACCG
 151  CACACCTACA TAGAAACGGA CGCGAAAAAG CTGCCGAAAT CGACAGATGA
 201  GCAGCTTTCG GCGCATGATA TGTACGAATG GATAAAGAAG CCCGAAAATA
 251  TCGGGTCTAT TGTCATTGTA GATGAAGCTC AAGACGTATG GCCGGCACGC
 301  TCGGCAGGTT CAAAAATCCC TGAAAATGTC CAATGGCTGA ATACGCACAG
 351  ACATCAGGGC ATTGATATAT TTGTTTTGAC TCAAGGCTCT AAGCTTCTAG
 401  ATCAAAATCT TAGAACGCTT GTACGGAAAC ATTACCACAT CGCTTCAAAC
 451  AAGATGGGTA TGCGTACGCT TTTAGAATGG AAAATATGCG CGGACGATCC
 501  CGTAAAAATG GCATCAAGCG CATTCTCCAG TATCTATACA CTGGATAAAA
 551  AAGTTTATGA CTTGTACGAA TCAGCGGAAG TTCATACCGT AAATAAGGTC
 601  AAGCGGTCAA AATGGTTTTA TACTCTGCCA GTAATAATAT TGCTGATTCC
 651  CGTTTTTGTC GGCCTGTCCT ATAAAATGTT AAGTAGTTAT GGAAAAAAAC
 701  AGGAAGAACC CGCAGCACAA GAATCGGCGG CAACAGAACA TCAGGCAGTA
 751  TTTCAGGATA AAACAGAAGG CGAGCCGGTA AACAACGGTA ACCTTACCGC
 801  AGATATGTTT GTTCCGACAT TGTCCGAAAA ACCCGAAAGC AAGCCGATTT
 851  ATAACGGTGT AAGGCAGGTA AGAACCTTTG AATATATAGC AGGCTGTGTA
 901  GAAGGCGGAA GAACCGGATG CACATGCTAT TCGCATCAAG GGACGGCATT
 951  GAAAGAAATT ACAAAGGAAA TGTGCAAGGA TTACGCAAGA AACGGATTGC
1001  CGTTTAACCC ATATAAAGAA GAAAGCCAAG GGCGGGATGT CCAGCAAAGT
1051  GAGCAGCACC ATTCGGACAG ACCGCAAGTT GCCACGTTGG GCGGAAAGCC
1101  GTGGCAAAAT CTTATGTATG ATAATTGGCA GGAGCGCGGA AAACCGTTTG
1151  AAGGAATCGG CGGGGGCGTG GTCGGATCGG CAAACTGA
```

This encodes a protein having amino acid sequence <SEQ ID 324>:

```
  1  MAEICLITGT PGSGKTLKMV SMMANDEMFK PDENGIRRKV FTNIKGLKIP
 51  HTYIETDAKK LPKSTDEQLS AHDMYEWIKK PENIGSIVIV DEAQDVWPAR
101  SAGSKIPENV QWLNTHRHQG IDIFVLTQGS KLLDQNLRTL VRKHYHIASN
151  KMGMRTLLEW KICADDPVKM ASSAFSSIYT LDKKVYDLYE SAEVHTVNKV
201  KRSKWFYTLP VIILLIPVFV GLSYKMLSSY GKKQEEPAAQ ESAATEHQAV
251  FQDKTEGEPV NNGNLTADMF VPTLSEKPES KPIYNGVRQV RTFEYIAGCV
301  EGGRTGCTCY SHQGTALKEI TKEMCKDYAR NGLPFNPYKE ESQGRDVQQS
351  EQHHSDRPQV ATLGGKPWQN LMYDNWQERG KPFEGIGGGV VGSAN*
```

ORF84a and ORF84-1 show 95.2% identity in 395 aa overlap:

```
                     10        20        30        40        50        60
orf84a.pep  MAEICLITGTPGSGKTLKMVSMMANDEMFKPDENGIRRKVFTNIKGLKIPHTYIETDAKK
            ||||||||||||||||||||||||||||||||||||||||||||||||||||||||||||
orf84-1     MAEICLITGTPGSGKTLKMVSMMANDEMFKPDENGIRRKVFTNIKGLKIPHTYIETDAKK
                     10        20        30        40        50        60

                     70        80        90       100       110       120
orf84a.pep  LPKSTDEQLSAHDMYEWIKKPENIGSIVIVDEAQDVWPARSAGSKIPENVQWLNTHRHQG
            ||||||||||||||||||||||||||||||||||||||||||||||||||||||||||||
orf84-1     LPKSTDEQLSAHDMYEWIKKPENIGSIVIVDEAQDVWPARSAGSKIPENVQWLNTHRHQG
                     70        80        90       100       110       120

                    130       140       150       160       170       180
orf84a.pep  IDIFVLTQGSKLLDQNLRTLVRKHYHIASNKMGMRTLLEWKICADDPVKMASSAFSSIYT
            ||||||||| ||||||||||||||||||||||||||||||||||||||||||||||||||
orf84-1     IDIFVLTQGPKLLDQNLRTLVRKHYHIASNKMGMRTLLEWKICADDPVKMASSAFSSIYT
                    130       140       150       160       170       180

                    190       200       210       220       230       240
orf84a.pep  LDKKVYDLYESAEVHTVNKVKRSKWFYTLPVIILLIPVFVGLSYKMLSSYGKKQEEPAAQ
            ||||||||||||||||||||||||||||||||:|||||||||||||||||||||||||||
orf84-1     LDKKVYDLYESAEVHTVNKVKRSKWFYTLPVIVLLIPVFVGLSYKMLSSYGKKQEEPAAQ
                    190       200       210       220       230       240

                    250       260       270       280       290       300

orf84a.pep  ESAATEHQAVFQDKTEGEPVNNGNLTADMFVPTLSEKPESKPIYNGVRQVRTFEYIAGCV
            ||||||:|||: |||||||||||||||||||||||||||||||||||||||||||||||:
orf84-1     ESAATEQQAVLPDKTEGEPVNNGNLTADMFVPTLSEKPESKPIYNGVRQVRTFEYIAGCI
                    250       260       270       280       290       300

                    310       320       330       340       350       360
orf84a.pep  EGGRTGCTCYSHQGTALKEITKEMCKDYARNGLPFNPYKEESQGRDVQQSEQHHSDRPQV
            |||||||:|||||||||||:|: |||||::||||||||||||||::|||| |:|||| ||
orf84-1     EGGRTGCACYSHQGTALKEVTELMCKDYVKNGLPFNPYKEESQGQEVQQSAQQHSDRAQV
                    310       320       330       340       350       360

                    370       380       390
orf84a.pep  ATLGGKPWQNLMYDNWQERGKPFEGIGGGVVGSANX
            ||||||| ||||||||:|||||||||||||||||||
orf84-1     ATLGGKPXQNLMYDNWEERGKPFEGIGGGVVGSANX
                    370       380       390
```

<u>Homology with a predicted ORF from *N.gonorrhoeae*</u>

ORF84 shows 94.2% identity over a 395aa overlap with a predicted ORF (ORF84.ng) from *N. gonorrhoeae:*

```
orf84.pep    MAEICLITGTPGSGKTLKMVSMMANDEMFKPDEKAIRRKVFTNIKGLKIPHTYIETDAKK   60
             |||||||||||||||||||||||||||||||||:::||||||||||||||||:|||||||
orf84ng      MAEICLITGTPGSGKTLKMVSMMANDEMFKPDENGVRRKVFTNIKGLKIPHTHIETDAKK   60

orf84.pep    LPKSTDEQLSAHDMYEWIKKPENIGSIVIVDEAQDVWPARSAGSKIPENVQWLNTHRHQG  120
             |||||||||||||||||||||||:|:||||||||||||||||||||||||||||||||||
orf84ng      LPKSTDEQLSAHDMYEWIKKPENVGAIVIVDEAQDVWPARSAGSKIPENVQWLNTHRHQG  120

orf84.pep    IDIFVLTQGPKLLDQNLRTLVRKHYHIASNKMGMRTLLEWKICADDPVKMASSAFSSIYT  180
             |||||||||||||||||||||::|||||:||||:|||||||:||||||||||||||||||
orf84ng      IDIFVLTQGPKLLDQNLRTLVKRHYHIAANKMGLRTLLEWKVCADDPVKMASSAFSSIYT  180

orf84.pep    LDKKVYDLYXXAEVHTVNKVKRSKWFYTLPVIVLLIPVFVGLSYKMLSSYGKKQEEPAAQ  240
             |||||||||  ||:|||||||||||||:||||:||||:|||||||||:||||||||||||
orf84ng      LDKKVYDLYESAEIHTVNKVKRSKWFYALPVIILLIPLFVGLSYKMLGSYGKKQEEPAAQ  240

orf84.pep    ESAATEQQAVLPDKTEGEPVNNGNLTADMFVPTLSEKPXSKPIYNGVRQVRTFEYIAGCI  300
             |||||||||||||||||| ||||||||||||||| ||| |||||||||||||||||||||
orf84ng      ESAATEQQAVLPDKTEGESVNNGNLTADMFVPTLPEKPESKPIYNGVRQVRTFEYIAGCI  300

orf84.pep    EGGRTGCACYSHQGTALKEVTELMCKDYVKNGLPFNPYKEESQGQEVQQSAQQHSDRAQV  360
             |||||||:||||||||||||||||||||||||||||||||||||||||||||||||||||
orf84ng      EGGRTGCTCYSHQGTALKEVTELMCKDYVKNGLPFNPYKEESQGQEVQQSAQQHSDRAQV  360

orf84.pep    ATLGGKPXQNLMYDNWEERGKPFEGIGGGVVGSAN  395
             ||||||| |||||||||||||||||||||||||||
orf84ng      ATLGGKPQQNLMYDNWEERGKPFEGIGGGVVGSAN  395
```

The complete length ORF84ng nucleotide sequence <SEQ ID 325> is:

```
   1  ATGGCAGAAA TCTGTTTGAT AACCGGCACG CCCGGTTCAG GGAAAACATT
  51  AAAAATGGTT TCCATGATGG CAAACGATGA AATGTTTAAG CCAGATGAAA
 101  ACGGCGTACG CCGTAAAGTA TTTACGAACA TCAAAGGTTT GAAGATACCG
 151  CACACCCACA TAGAAACAGA CGCAAAGAAG CTGCCGAAAT CAACCGATGA
 201  ACAGCTTTCG GCGCATGATA TGTATGAATG GATCAAGAAG CCTGAAAacg
 251  tcggcgCAAT CGTTATTGTC GATGAGGCGC AAGACGTATG GCCCGCACGC
 301  TccgCAGGTT CGAAAATCCC CGAAAACGTC CAATGGCTGA ACACACACAG
 351  GCATCAGGGC ATAGATATAT TTGTATTGAC ACAAGGTCCT AAACTCTTAG
 401  ATCAGAACTT GCGAACATTG GTTAAAAGAC ATTACCACAT TGCGGCCAAC
 451  AAAATGGGTT TGCGTACCCT GCTTGAATGG AAAGTATGCG CGGATGACCC
 501  GGTAAAAATG GCATCAAGTG CATTTTCCAG TATCTACACA CTGGATAAAA
 551  AAGTTTATGA CTTGTACGAA TCCGCAGAAA TTCACACGGT AAACAAAGTC
 601  AAGCGTTCAA AATGGTTTTA TGCATTGCCC GTCATCATAT TATTGATTCC
 651  GCTATTTGTC GGTTTGTCTT ACAAAATGTT GGGCAGTTAC GGAAAAAAAC
 701  AGGAAGAACC CGCAGCACAA GAATCGGCGG CAACAGAACA GCAGGCAGTA
 751  CTTCCGGATA AAACAGAAGG AGAATCGGTG AATAACGGAA ACCTTACGGC
 801  AGATATGTTT GTTCCGACAT TGCCCGAAAA ACCCGAAAGC AAGCCGATTT
 851  ATAACGGTGT AAGGCAGGTA AGGACCTTTG AATATATAGC AGGCTGTATA

 901  GAAGGCGGAA GAACCGGATG CACCTGCTAT TCGCATCAAG GGACGGCATT
 951  GAAAGAAGTG ACGGAGTTGA TGTGCAAGGA CTATGTAAAA AACGGCTTGC
1001  CGTTTAACCC ATACAAAGAA GAAAGCCAAG GGCAGGAAGT TCAGCAAAGC
1051  GCGCAGCAAC ATTCGGACAG GGCGCAAGTT GCCACCTTGG GCGGAAAACC
1101  GCAGCAGAAC CTAATGTACG ACAATTGGGA AGAACGCGGG AAACCGTTTG
1151  AAGGAATCGG CGGGGGCGTG GTCGGATCGG CAAACTGA
```

This encodes a protein having amino acid sequence <SEQ ID 326>:

```
  1 MAEICLITGT PGSGKTLKMV SMMANDEMFK PDENGVRRKV FTNIKGLKIP
 51 HTHIETDAKK LPKSTDEQLS AHDMYEWIKK PENVGAIVIV DEAQDVWPAR
101 SAGSKIPENV QWLNTHRHQG IDIFVLTQGP KLLDQNLRTL VKRHYHIAAN
151 KMGLRTLLEW KVCADDPVKM ASSAFSSIYT LDKKVYDLYE SAEIHTVNKV
201 KRSKWFYALP VIILLIPLFV GLSYKMLGSY GKKQEEPAAQ ESAATEQQAV
251 LPDKTEGESV NNGNLTADMF VPTLPEKPES KPIYNGVRQV RTFEYIAGCI
301 EGGRTGCTCY SHQGTALKEV TELMCKDYVK NGLPFNPYKE ESQGQEVQQS
351 AQQHSDRAQV ATLGGKPQQN LMYDNWEERG KPFEGIGGGV VGSAN*
```

ORF84ng and ORF84-1 show 95.4% identity in 395 aa overlap:

```
                     10        20        30        40        50        60
orf84-1.pep  MAEICLITGTPGSGKTLKMVSMMANDEMFKPDENGIRRKVFTNIKGLKIPHTYIETDAKK
             |||||||||||||||||||||||||||||||||||||||:||||||||||||:|||||||
orf84ng      MAEICLITGTPGSGKTLKMVSMMANDEMFKPDENGVRRKVFTNIKGLKIPHTHIETDAKK
                     10        20        30        40        50        60

                     70        80        90       100       110       120
orf84-1.pep  LPKSTDEQLSAHDMYEWIKKPENIGSIVIVDEAQDVWPARSAGSKIPENVQWLNTHRHQG
             |||||||||||||||||||||||:|:||||||||||||||||||||||||||||||||||
orf84ng      LPKSTDEQLSAHDMYEWIKKPENVGAIVIVDEAQDVWPARSAGSKIPENVQWLNTHRHQG
                     70        80        90       100       110       120

                    130       140       150       160       170       180
orf84-1.pep  IDIFVLTQGPKLLDQNLRTLVRKHYHIASNKMGMRTLLEWKICADDPVKMASSAFSSIYT
             |||||||||||||||||||||::|||||:||||:|||||||:||||||||||||||||||
orf84ng      IDIFVLTQGPKLLDQNLRTLVKRHYHIAANKMGLRTLLEWKVCADDPVKMASSAFSSIYT
                    130       140       150       160       170       180

                    190       200       210       220       230       240
orf84-1.pep  LDKKVYDLYESAEVHTVNKVKRSKWFYTLPVIVLLIPVFVGLSYKMLSSYGKKQEEPAAQ
             |||||||||||||:|||||||||||||:||||:||||:|||||||||:||||||||||||
orf84ng      LDKKVYDLYESAEIHTVNKVKRSKWFYALPVIILLIPLFVGLSYKMLGSYGKKQEEPAAQ
                    190       200       210       220       230       240

                    250       260       270       280       290       300
orf84-1.pep  ESAATEQQAVLPDKTEGEPVNNGNLTADMFVPTLSEKPESKPIYNGVRQVRTFEYIAGCI
             |||||||||||||||||| ||||||||||||||| |||||||||||||||||||||||||
orf84ng      ESAATEQQAVLPDKTEGESVNNGNLTADMFVPTLPEKPESKPIYNGVRQVRTFEYIAGCI
                    250       260       270       280       290       300

                    310       320       330       340       350       360
orf84-1.pep  EGGRTGCACYSHQGTALKEVTELMCKDYVKNGLPFNPYKEESQGQEVQQSAQQHSDRAQV
             |||||||:||||||||||||||||||||||||||||||||||||||||||||||||||||
orf84ng      EGGRTGCTCYSHQGTALKEVTELMCKDYVKNGLPFNPYKEESQGQEVQQSAQQHSDRAQV
                    310       320       330       340       350       360

                    370       380       390
orf84-1.pep  ATLGGKPXQNLMYDNWEERGKPFEGIGGGVVGSANX
             ||||||| ||||||||||||||||||||||||||||
orf84ng      ATLGGKPQQNLMYDNWEERGKPFEGIGGGVVGSANX
                    370       380       390
```

Based on this analysis, includng the presence of a putative transmembrane domain (single-underlined) in the gonococcal protein, and a putative ATP/GTP-binding site motif A (P-loop, double-underlined), it is predicted that the proteins from *N.meningitidis* and *N.gonorrhoeae,* and their epitopes, could be useful antigens for vaccines or diagnostics, or for raising antibodies.

### Example 39

The following partial DNA sequence was identified in *N.meningitidis* <SEQ ID 327>:

```
   1 GTGGTTTTCC TGAATGCCGA CAACGGGATA TTGGTTCAGG ACTTGCCTTT
  51 TGAAGTCAAA CTGAAAAAAT TCCATATCGA TTTTTACAAT ACGGGTATGC
 101 CGCGTGATTT CGCCAGCGAT ATTGAAGTGA CGGACAAGGC AACCGGTGAG
 151 AAACTCGAGC GCACCATCCG CGTGAACCAT CCTTTGACCT TGCACGGCAT
 201 CACGATTTAT CAGGCGAGTT TTGCCGACGG CGGTTCGGAT TTGACATTCA
 251 AGGCGTGGAA TTTGGGTGAT GCTTCGCGCG AGCCTGTCGT GTTGAAGGCA
 301 ACATCCATAC ACCAGTTTCC GTTGGAAATT GGCAAACACA AATATCGTCT
 351 TGAGTTCGAT CAGTTCACTT CTATGAATGT GGAGGACATG AGCGAGGGCG
 401 CGGAACGGGA AAAAAGCCTG AAATCCACGC TGCCCGATGT CCGCGCCGTT
 451 ACTCAGGAAG GTCACAAATA CACCAAT... .......... .....TACCG
 501 TATCCGTGAT GCGCCAGGCC AGGCGGTCGA ATATAAAAAC TATATGCTGC
 551 CGGTTTTGCA GGAACAGGAT TATTTTTGGA TTACCGGCAC GCGCAGCGC.
 601 TTGCAGCAGC AATACCGCTG GCTGCGTATC CCCTTGGACA AGCAGTTGAA
 651 AGCGGACACC TTTATGGCAT TGCGTGAGTT TTTGAAAGAT GGGGAAGGGC
 701 GCAAACGTCT .GTTGCCGAC GCAACCAAAG GCGCACCTGC CGAAATCCGC
 751 GAACAATTCA TGCTGGCTGC GGAAAACACG CTGAACATCT TTGCACAAAA
 801 AGGCTATTTG GGATTGGACG AATTTATTAC GTCCAATATC CCGAAAGAGC
 851 AGCAGGATAA GATGCAGGGC TATTTCTACG AAATGCTTTA CGGCGTGATG
 901 AACGCTGCTT TGGATGAAAC CAT.ACCCGG TACGGCTTGC CCGAATGGCA
 951 GCAGGATGAA GCGCGGAATC GTTTCCTGCT GCACAGTATG GATGCGTACA
1001 CGGGTTTGAC CGAATATCCC GCGCCTATGC TGCTGCAACT TGATGGGTTT
1051 TCCGAGGTGC GTTCGTCGGG TTTGCAGATG ACCCGTTCCC C.GGTCCGCT
1101 TTTGGTCTAT CTC...
```

This corresponds to the amino acid sequence <SEQ ID 328; ORF88>:

```
  1 MVFLNADNGI LVQDLPFEVK LKKFHIDFYN TGMPRDFASD IEVTDKATGE
 51 KLERTIRVNH PLTLHGITIY QASFADGGSD LTFKAWNLGD ASREPVVLKA
101 TSIHQFPLEI GKHKYRLEFD QFTSMNVEDM SEGAEREKSL KSTLPDVRAV
151 TQEGHKYTNX XXXXXYRIRD APGQAVEYKN YMLPVLQEQD YFWITGTRSX
201 LQQQYRWLRI PLDKQLKADT FMALREFLKD GEGRKRXVAD ATKGAPAEIR
251 EQFMLAAENT LNIFAQKGYL GLDEFITSNI PKEQQDKMQG YFYEMLYGVM
301 NAALDETXTR YGLPEWQQDE ARNRFLLHSM DAYTGLTEYP APMLLQLDGF
351 SEVRSSGLQM TRSXGPLLVY L...
```

Further work revealed the complete nucleotide sequence <SEQ ID 329>:

```
   1 ATGAGTAAAT CCCGTAGATC TCCCCCACTT CTTTCCCGTC CGTGGTTCGC
  51 TTTTTTCAGC TCCATGCGCT TTGCAGTCGC TTTGCTCAGT CTGCTGGGTA
 101 TTGCATCGGT TATCGGTACG GTGTTGCAGC AAAACCAGCC GCAGACGGAT
 151 TATTTGGTCA AATTCGGATC GTTTTGGGCG CAGATTTTTG GTTTTCTGGG
 201 ACTGTATGAC GTCTATGCTT CGGCATGGTT TGTCGTTATC ATGATGTTTT
 251 TGGTGGTTTC TACCAGTTTG TGCCTGATTC GCAATGTGCC GCCGTTCTGG
 301 CGCGAAATGA AGTCTTTTCG GGAAAAGGTT AAAGAAAAAT CTCTGGCGGC
 351 GATGCGCCAT TCTTCGCTGT TGGATGTAAA AATTGCGCCC GAGGTTGCCA
 401 AACGTTATCT GGAAGTACAA GGTTTTCAGG GAAAAACCAT TAACCGTGAA
 451 GACGGGTCGG TTCTGATTGC CGCCAAAAAA GGCACAATGA ACAAATGGGG
 501 CTATATCTTT GCCCATGTTG CTTTGATTGT CATTTGCCTG GGCGGGTTGA
 551 TAGACAGTAA CCTGCTGTTG AAACTGGGTA TGCTGACCGG TCGGATTGTT
 601 CCGGACAATC AGGCGGTTTA TGCCAAGGAT TTCAAGCCCG AAAGTATTTT
 651 GGGTGCGTCC AATCTCTCAT TTAGGGGCAA CGTCAATATT TCCGAGGGGC
 701 AGAGTGCGGA TGTGGTTTTC CTGAATGCCG ACAACGGGAT ATTGGTTCAG
 751 GACTTGCCTT TTGAAGTCAA ACTGAAAAAA TTCCATATCG ATTTTTACAA
 801 TACGGGTATG CCGCGTGATT TCGCCAGCGA TATTGAAGTG ACGGACAAGG
 851 CAACCGGTGA GAAACTCGAG CGCACCATCC GCGTGAACCA TCCTTTGACC
 901 TTGCACGGCA TCACGATTTA TCAGGCGAGT TTTGCCGACG GCGGTTCGGA
 951 TTTGACATTC AAGGCGTGGA ATTTGGGTGA TGCTTCGCGC GAGCCTGTCG
1001 TGTTGAAGGC AACATCCATA CACCAGTTTC CGTTGGAAAT TGGCAAACAC
1051 AAATATCGTC TTGAGTTCGA TCAGTTCACT TCTATGAATG TGGAGGACAT
1101 GAGCGAGGGC GCGGAACGGG AAAAAAGCCT GAAATCCACG CTGAACGATG
1151 TCCGCGCCGT TACTCAGGAA GGTAAAAAAT ACACCAATAT CGGCCCTTCC
1201 ATTGTTTACC GTATCCGTGA TGCGGCAGGG CAGGCGGTCG AATATAAAAA
1251 CTATATGCTG CCGGTTTTGC AGGAACAGGA TTATTTTTGG ATTACCGGCA
1301 CGCGCAGCGG CTTGCAGCAG CAATACCGCT GGCTGCGTAT CCCCTTGGAC
1351 AAGCAGTTGA AAGCGGACAC CTTTATGGCA TTGCGTGAGT TTTTGAAAGA
1401 TGGGGAAGGG CGCAAACGTC TGGTTGCCGA CGCAACCAAA GGCGCACCTG
1451 CCGAAATCCG CGAACAATTC ATGCTGGCTG CGGAAAACAC GCTGAACATC
1501 TTTGCACAAA AAGGCTATTT GGGATTGGAC GAATTTATTA CGTCCAATAT
1551 CCCGAAAGAG CAGCAGGATA AGATGCAGGG CTATTTCTAC GAAATGCTTT
1601 ACGGCGTGAT GAACGCTGCT TTGGATGAAA CCATACGCCG GTACGGCTTG
1651 CCCGAATGGC AGCAGGATGA AGCGCGGAAT CGTTTCCTGC TGCACAGTAT
1701 GGATGCGTAC ACGGGTTTGA CCGAATATCC CGCGCCTATG CTGCTGCAAC
1751 TTGATGGGTT TTCCGAGGTG CGTTCGTCGG GTTTGCAGAT GACCCGTTCC
1801 CCGGGTGCGC TTTTGGTCTA TCTCGGCTCG GTGCTGTTGG TATTGGGTAC
1851 GGTATTGATG TTTTATGTGC GCGAAAAACG GGCGTGGGTA TTGTTTTCAG
1901 ACGGCAAAAT CCGTTTTGCC ATGTCTTCGG CCCGCAGCGA ACGGGATTTG
1951 CAGAAGGAAT TTCCAAAACA CGTCGAGAGT CTGCAACGGC TCGGCAAGGA
2001 CTTGAATCAT GACTGA
```

This corresponds to the amino acid sequence <SEQ ID 330; ORF88-1>:

```
  1 MSKSRRSPPL LSRPWFAFFS SMRFAVALLS LLGIASVIGT VLQQNQPQTD
 51 YLVKFGSFWA QIFGFLGLYD VYASAWFVVI MMFLVVSTSL CLIRNVPPFW
101 REMKSFREKV KEKSLAAMRH SSLLDVKIAP EVAKRYLEVQ GFQGKTINRE
151 DGSVLIAAKK GTMNKWGYIF AHVALIVICL GGLIDSNLLL KLGMLTGRIV
201 PDNQAVYAKD FKPESILGAS NLSFRGNVNI SEGQSADVVF LNADNGILVQ
251 DLPFEVKLKK FHIDFYNTGM PRDFASDIEV TDKATGEKLE RTIRVNHPLT
301 LHGITIYQAS FADGGSDLTF KAWNLGDASR EPVVLKATSI HQFPLEIGKH
351 KYRLEFDQFT SMNVEDMSEG AEREKSLKST LNDVRAVTQE GKKYTNIGPS
401 IVYRIRDAAG QAVEYKNYML PVLQEQDYFW ITGTRSGLQQ QYRWLRIPLD
451 KQLKADTFMA LREFLKDGEG RKRLVADATK GAPAEIREQF MLAAENTLNI
501 FAQKGYLGLD EFITSNIPKE QQDKMQGYFY EMLYGVMNAA LDETIRRYGL
551 PEWQQDEARN RFLLHSMDAY TGLTEYPAPM LLQLDGFSEV RSSGLQMTRS
601 PGALLVYLGS VLLVLGTVLM FYVREKRAWV LFSDGKIRFA MSSARSERDL
651 QKEFPKHVES LQRLGKDLNH D*
```

Computer analysis of this amino acid sequence gave the following results:

Homology with a predicted ORF from *N.meningitidis* (strain A)

ORF88 shows 95.7% identity over a 371aa overlap with an ORF (ORF88a) from strain A of *N. meningitidis:*

```
                                                 10        20        30
orf88.pep                                  MVFLNADNGILVQDLPFEVKLKKFHIDFYN
                                           :|||||||||||||||||||||||||||||
orf88a     AKDFKPESILGASNLSFRGNVNISEGQSADVVFLNADNGILVQDLPFEVKLKKFHIDFYN
           210       220       230       240       250       260

                   40        50        60        70        80        90
orf88.pep  TGMPRDFASDIEVTDKATGEKLERTIRVNHPLTLHGITIYQASFADGGSDLTFKAWNLGD
           ||||||||||||||||||||||||||||||||||||||||||||||||||||||||||||
orf88a     TGMPRDFASDIEVTDKATGEKLERTIRVNHPLTLHGITIYQASFADGGSDLTFKAWNLGD
           270       280       290       300       310       320

                  100       110       120       130       140       150
orf88.pep  ASREPVVLKATSIHQFPLEIGKHKYRLEFDQFTSMNVEDMSEGAEREKSLKSTLPDVRAV
           |||||||||||||||||||||||||||||||||||||||||||||||||||||| |||||
orf88a     ASREPVVLKATSIHQFPLEIGKHKYRLEFDQFTSMNVEDMSEGAEREKSLKSTLNDVRAV
           330       340       350       360       370       380

                  160       170       180       190       200       210
orf88.pep  TQEGHKYTNXXXXXXYRIRDAPGQAVEYKNYMLPVLQEQDYFWITGTRSXLQQQYRWLRI
           ||||:||||      |||||| ||||||||||||||||||||||||||| ||||||||||
orf88a     TQEGKKYTNIGPSIVYRIRDAAGQAVEYKNYMLPVLQEQDYFWITGTRSGLQQQYRWLRI
           390       400       410       420       430       440

                  220       230       240       250       260       270
orf88.pep  PLDKQLKADTFMALREFLKDGEGRKRXVADATKGAPAEIREQFMLAAENTLNIFAQKGYL
           |||||||||||||||||||||||||| |||||||||||||||||||||||||||||||||
orf88a     PLDKQLKADTFMALREFLKDGEGRKRLVADATKGAPAEIREQFMLAAENTLNIFAQKGYL
           450       460       470       480       490       500

                  280       290       300       310       320       330
orf88.pep  GLDEFITSNIPKEQQDKMQGYFYEMLYGVMNAALDETXTRYGLPEWQQDEARNRFLLHSM
           |||||||||||||||||||||||||||||||||||||  |||||||||||||||||||||
orf88a     GLDEFITSNIPKEQQDKMQGYFYEMLYGVMNAALDETIRRYGLPEWQQDEARNRFLLHSM
           510       520       530       540       550       560

                  340       350       360       370
orf88.pep  DAYTGLTEYPAPMLLQLDGFSEVRSSGLQMTRSXGPLLVYL
           ||||||||||||||||||||||||||||||||| | |||||
orf88a     DAYTGLTEYPAPMLLQLDGFSEVRSSGLQMTRSPGALLVYLGSVLLVLGTVLMFYVREKR
           570       580       590       600       610       620

orf88a     AWVLFSDGKIRFAMSSARSERDLQKEFPKHVESLQRLGKDLNHDX
           630       640       650       660       670
```

The complete length ORF88a nucleotide sequence <SEQ ID 331> is:

```
   1 ATGAGTAAAT CCCGTAGATC TCCCCCACTT CTTTCCCGTC CGTGGTTCGC
  51 TTTTTTCAGC TCCATGCGCT TTGCGGTCGC TTTGCTCAGT CTGCTGGGTA
 101 TTGCATCGGT TATCGGTACG GTGTTGCAGC AAAACCAGCC GCAGACGGAT
 151 TATTTGGTCA AATTCGGATC GTTTTGGGCG CAGATTTTTG GTTTTCTGGG
 201 ACTGTATGAC GTCTATGCTT CGGCATGGTT TGTCGTTATC ATGATGTTTT
 251 TGGTGGTTTC TACCAGTTTG TGCCTGATTC GCAATGTGCC GCCGTTCTGG
 301 CGCGAAATGA AGTCTTTTCG GGAAAAGGTT AAAGAAAAAT CTCTGGCGGC
 351 GATGCGCCAT TCTTCGCTGT TGGATGTAAA AATTGCGCCC GAGGTTGCCA
 401 AACGTTATCT GGAAGTACAA GGTTTTCAGG GAAAAACCAT TAACCGTGAA
 451 GACGGGTCGG TTCTGATTGC CGCCAAAAAA GGCACAATGA ACAAATGGGG
 501 CTATATCTTT GCCCATGTTG CTTTGATTGT CATTTGCCTG GGCGGGTTGA
 551 TAGACAGTAA CCTGCTGTTG AAACTGGGTA TGCTGACCGG TCGGATTGTT
 601 CCGGACAATC AGGCGGTTTA TGCCAAGGAT TTCAAGCCCG AAAGTATTTT
 651 GGGTGCGTCC AATCTCTCAT TTAGGGGCAA CGTCAATATT TCCGAGGGGC
 701 AGAGTGCGGA TGTGGTTTTC CTGAATGCCG ACAACGGGAT ATTGGTTCAG
 751 GACTTGCCTT TTGAAGTCAA ACTGAAAAAA TTCCATATCG ATTTTTACAA
 801 TACGGGTATG CCGCGCGATT TTGCCAGTGA TATTGAAGTA ACGGATAAGG
 851 CAACCGGTGA GAAACTCGAG CGCACCATCC GCGTGAACCA TCCTTTGACC
 901 TTGCACGGCA TCACGATTTA TCAGGCGAGT TTTGCCGACG GCGGTTCGGA
 951 TTTGACATTC AAGGCGTGGA ATTTGGGTGA TGCTTCGCGC GAGCCTGTCG
1001 TGTTGAAGGC AACATCCATA CACCAGTTTC CGTTGGAAAT TGGCAAACAC
1051 AAATATCGTC TTGAGTTCGA TCAGTTTACT TCTATGAATG TGGAGGACAT
1101 GAGCGAGGGC GCGGAACGGG AAAAAAGCCT GAAATCCACG CTGAACGATG
1151 TCCGCGCCGT TACTCAGGAA GGTAAAAAAT ACACCAATAT CGGCCCTTCC
1201 ATTGTTTACC GTATCCGTGA TGCGGCAGGG CAGGCGGTCG AATATAAAAA
1251 CTATATGCTG CCGGTTTTGC AGGAACAGGA TTATTTTTGG ATTACCGGCA
1301 CGCGCAGCGG CTTGCAGCAG CAATACCGCT GGCTGCGTAT CCCCTTGGAC
1351 AAGCAGTTGA AAGCGGACAC CTTTATGGCA TTGCGTGAGT TTTTGAAAGA
1401 TGGGGAAGGG CGCAAACGTC TGGTTGCCGA CGCAACCAAA GGCGCACCTG
1451 CCGAAATCCG CGAACAATTC ATGCTGGCTG CGGAAAACAC GCTGAACATC
1501 TTTGCACAAA AAGGCTATTT GGGATTGGAC GAATTTATTA CGTCCAATAT
1551 CCCGAAAGAG CAGCAGGATA AGATGCAGGG CTATTTCTAC GAAATGCTTT
1601 ACGGCGTGAT GAACGCTGCT TTGGATGAAA CCATACGCCG GTACGGCTTG
1651 CCCGAATGGC AGCAGGATGA AGCGCGGAAT CGTTTCCTGC TGCACAGTAT
1701 GGATGCGTAC ACGGGTTTGA CCGAATATCC CGCGCCTATG CTGCTGCAAC
1751 TTGATGGGTT TTCCGAGGTG CGTTCGTCGG GTTTGCAGAT GACCCGTTCC
1801 CCGGGTGCGC TTTTGGTCTA TCTCGGCTCG GTGCTGTTGG TATTGGGTAC
1851 GGTATTGATG TTTTATGTGC GCGAAAAACG GGCGTGGGTA TTGTTTTCAG
1901 ACGGCAAAAT CCGTTTTGCC ATGTCTTCGG CCCGCAGCGA ACGGGATTTG
1951 CAGAAGGAAT TTCCAAAACA CGTCGAGAGT CTGCAACGGC TCGGCAAGGA
2001 CTTGAATCAT GACTGA
```

This encodes a protein having amino acid sequence <SEQ ID 332>:

```
  1 MSKSRRSPPL LSRPWFAFFS SMRFAVALLS LLGIASVIGT VLQQNQPQTD
 51 YLVKFGSFWA QIFGFLGLYD VYASAWFVVI MMFLVVSTSL CLIRNVPPFW
101 REMKSFREKV KEKSLAAMRH SSLLDVKIAP EVAKRYLEVQ GFQGKTINRE
151 DGSVLIAAKK GTMNKWGYIF AHVALIVICL GGLIDSNLLL KLGMLTGRIV
201 PDNQAVYAKD FKPESILGAS NLSFRGNVNI SEGQSADVVF LNADNGILVQ
251 DLPFEVKLKK FHIDFYNTGM PRDFASDIEV TDKATGEKLE RTIRVNHPLT
301 LHGITIYQAS FADGGSDLTF KAWNLGDASR EPVVLKATSI HQFPLEIGKH
351 KYRLEFDQFT SMNVEDMSEG AEREKSLKST LNDVRAVTQE GKKYTNIGPS
401 IVYRIRDAAG QAVEYKNYML PVLQEQDYFW ITGTRSGLQQ QYRWLRIPLD
451 KQLKADTFMA LREFLKDGEG RKRLVADATK GAPAEIREQF MLAAENTLNI
501 FAQKGYLGLD EFITSNIPKE QQDKMQGYFY EMLYGVMNAA LDETIRRYGL

551 PEWQQDEARN RFLLHSMDAY TGLTEYPAPM LLQLDGFSEV RSSGLQMTRS
601 PGALLVYLGS VLLVLGTVLM FYVREKRAWV LFSDGKIRFA MSSARSERDL
651 QKEFPKHVES LQRLGKDLNH D*
```

ORF88a and ORF88-1 100.0% identity in 671 aa overlap:

```
orf88a.pep   MSKSRRSPPLLSRPWFAFFSSMRFAVALLSLLGIASVIGTVLQQNQPQTDYLVKFGSFWA   60
             ||||||||||||||||||||||||||||||||||||||||||||||||||||||||||||
orf88-1      MSKSRRSPPLLSRPWFAFFSSMRFAVALLSLLGIASVIGTVLQQNQPQTDYLVKFGSFWA   60

orf88a.pep   QIFGFLGLYDVYASAWFVVIMMFLVVSTSLCLIRNVPPFWREMKSFREKVKEKSLAAMRH  120
             ||||||||||||||||||||||||||||||||||||||||||||||||||||||||||||
orf88-1      QIFGFLGLYDVYASAWFVVIMMFLVVSTSLCLIRNVPPFWREMKSFREKVKEKSLAAMRH  120

orf88a.pep   SSLLDVKIAPEVAKRYLEVQGFQGKTINREDGSVLIAAKKGTMNKWGYIFAHVALIVICL  180
             ||||||||||||||||||||||||||||||||||||||||||||||||||||||||||||
orf88-1      SSLLDVKIAPEVAKRYLEVQGFQGKTINREDGSVLIAAKKGTMNKWGYIFAHVALIVICL  180

orf88a.pep   GGLIDSNLLLKLGMLTGRIVPDNQAVYAKDFKPESILGASNLSFRGNVNISEGQSADVVF  240
             ||||||||||||||||||||||||||||||||||||||||||||||||||||||||||||
orf88-1      GGLIDSNLLLKLGMLTGRIVPDNQAVYAKDFKPESILGASNLSFRGNVNISEGQSADVVF  240

orf88a.pep   LNADNGILVQDLPFEVKLKKFHIDFYNTGMPRDFASDIEVTDKATGEKLERTIRVNHPLT  300
             ||||||||||||||||||||||||||||||||||||||||||||||||||||||||||||
orf88-1      LNADNGILVQDLPFEVKLKKFHIDFYNTGMPRDFASDIEVTDKATGEKLERTIRVNHPLT  300

orf88a.pep   LHGITIYQASFADGGSDLTFKAWNLGDASREPVVLKATSIHQFPLEIGKHKYRLEFDQFT  360
             ||||||||||||||||||||||||||||||||||||||||||||||||||||||||||||
orf88-1      LHGITIYQASFADGGSDLTFKAWNLGDASREPVVLKATSIHQFPLEIGKHKYRLEFDQFT  360

orf88a.pep   SMNVEDMSEGAEREKSLKSTLNDVRAVTQEGKKYTNIGPSIVYRIRDAAGQAVEYKNYML  420
             ||||||||||||||||||||||||||||||||||||||||||||||||||||||||||||
orf88-1      SMNVEDMSEGAEREKSLKSTLNDVRAVTQEGKKYTNIGPSIVYRIRDAAGQAVEYKNYML  420

orf88a.pep   PVLQEQDYFWITGTRSGLQQQYRWLRIPLDKQLKADTFMALREFLKDGEGRKRLVADATK  480
             ||||||||||||||||||||||||||||||||||||||||||||||||||||||||||||
orf88-1      PVLQEQDYFWITGTRSGLQQQYRWLRIPLDKQLKADTFMALREFLKDGEGRKRLVADATK  480

orf88a.pep   GAPAEIREQFMLAAENTLNIFAQKGYLGLDEFITSNIPKEQQDKMQGYFYEMLYGVMNAA  540
             ||||||||||||||||||||||||||||||||||||||||||||||||||||||||||||
orf88-1      GAPAEIREQFMLAAENTLNIFAQKGYLGLDEFITSNIPKEQQDKMQGYFYEMLYGVMNAA  540

orf88a.pep   LDETIRRYGLPEWQQDEARNRFLLHSMDAYTGLTEYPAPMLLQLDGFSEVRSSGLQMTRS  600
             ||||||||||||||||||||||||||||||||||||||||||||||||||||||||||||
orf88-1      LDETIRRYGLPEWQQDEARNRFLLHSMDAYTGLTEYPAPMLLQLDGFSEVRSSGLQMTRS  600

orf88a.pep   PGALLVYLGSVLLVLGTVLMFYVREKRAWVLFSDGKIRFAMSSARSERDLQKEFPKHVES  660
             ||||||||||||||||||||||||||||||||||||||||||||||||||||||||||||
orf88-1      PGALLVYLGSVLLVLGTVLMFYVREKRAWVLFSDGKIRFAMSSARSERDLQKEFPKHVES  660

orf88a.pep   LQRLGKDLNHD    672
             |||||||||||
orf88-1      LQRLGKDLNHD    672
```

<u>Homolog with a predicted ORF from *N. gonorrhoeae*</u>

ORF88 shows 93.8% identity over a 371aa overlap with a predicted ORF (ORF88.ng) from *N. gonorrhoeae:*

```
orf88.pep   MVFLNADNGILVQDLPFEVKLKKFHIDFYNTGMPRDFASDIEVTDKATGEKLERTIRVNH   60
            |||||||||:||||||||||||||||||||||||||||||||||||||||||||||||||
orf88ng     MVFLNADNGMLVQDLPFEVKLKKFHIDFYNTGMPRDFASDIEVTDKATGEKLERTIRVNH   60

orf88.pep   PLTLHGITIYQASFADGGSDLTFKAWNLGDASREPVVLKATSIHQFPLEIGKHKYRLEFD  120
            |||||||||||||||||||||||||||| |||||||||||||||||||||||||||||||
orf88ng     PLTLHGITIYQASFADGGSDLTFKAWNLRDASREPVVLKATSIHQFPLEIGKHKYRLEFD  120

orf88.pep   QFTSMNVEDMSEGAEREKSLKSTLPDVRAVTQEGHKYTNXXXXXXYRIRDAPGQAVEYKN  180
            |||||||||||||||||||||||| |||||||||:||||      |||||| ||||||||
orf88ng     QFTSMNVEDMSEGAEREKSLKSTLNDVRAVTQEGKKYTNIGPSIVYRIRDAAGQAVEYKN  180
```

```
orf88.pep   YMLPVLQEQDYFWITGTRSXLQQQYRWLRIPLDKQLKADTFMALREFLKDGEGRKRXVAD  240
            ||||:||::||||:||||| |||||||||||||||||||||||||||||||||||| |||
orf88ng     YMLPILQDKDYFWLTGTRSGLQQQYRWLRIPLDKQLKADTFMALREFLKDGEGRKRLVAD  240

orf88.pep   ATKGAPAEIREQFMLAAENTLNIFAQKGYLGLDEFITSNIPKEQQDKMQGYFYEMLYGVM  300
            ||| |||||||||||||||||||||||||||||||||||||| |||||||||||||||||
orf88ng     ATKDAPAEIREQFMLAAENTLNIFAQKGYLGLDEFITSNIPKGQQDKMQGYFYEMLYGVM  300

orf88.pep   NAALDETXTRYGLPEWQQDEARNRFLLHSMDAYTGLTEYPAPMLLQLDGFSEVRSSGLQM  360
            |||||||  |||||||||||||||||||||||||||||||||||||||||||||||||||
orf88ng     NAALDETIRRYGLPEWQQDEARNRFLLHSMDAYTGLTEYPAPMLLQLDGFSEVRSSGLQM  360

orf88.pep   TRSXGPLLVYL                                                  371
            ||| | |||||
orf88ng     TRSPGALLVYLGSVLLVLGTVFMFYVPKKRAWVLFSNXKIRFAMSSARSERDLQKEFPKH  420
```

An ORF88ng nucleotide sequence <SEQ ID 333> was predicted to encode a protein having amino acid sequence <SEQ ID 334>:

```
  1  MVFLNADNGM LVQDLPFEVK LKKFHIDFYN TGMPRDFASD IEVTDKATGE
 51  KLERTIRVNH PLTLHGITIY QASFADGGSD LTFKAWNLRD ASREPVVLKA
101  TSIHQFPLEI GKHKYRLEFD QFTSMNVEDM SEGAEREKSL KSTLNDVRAV
151  TQEGKKYTNI GPSIVYRIRD AAGQAVEYKN YMLPILQDKD YFWLTGTRSG
201  LQQQYRWLRI PLDKQLKADT FMALREFLKD GEGRKRLVAD ATKDAPAEIR
251  EQFMLAAENT LNIFAQKGYL GLDEFITSNI PKGQQDKMQG YFYEMLYGVM
301  NAALDETIRR YGLPEWQQDE ARNRFLLHSM DAYTGLTEYP APMLLQLDGF
351  SEVRSSGLQM TRSPGALLVY LGSVLLVLGT VFMFYVPKKR AWVLFSNXKI
401  RFAMSSARSE RDLQKEFPKH VESLQRLGKD LNHD*
```

Further work revealed the complete gonococcal DNA sequence <SEQ ID 335>:

1 ATGAGTAAAT CCCGTATATC TCCCACACTT CTTTCCCGTC CGTGGTTCGC
51 TTTTTTCAGC TCCATGCGCT TTGCGGTCGC TTTGCTCAGT CTGCTGGGTA
101 TTGCATCGGT TATCGGCACG GTGTTACAGC AAAACCAGCC GCAGACGGAT
151 TATTTGGTCA AATTCGGACC GTTTTGGACT CGGATTTTTG ATTTTTTGGG
201 TTTGTATGAT GTCTATGCTT CGGCATGGTT TGTCGTTATC ATGATGTTTC
251 TGGTGGTTTC TACCAGTTTG TGTTTAATCC GTAACGTTCC GCCGTTTTGG
301 CGCGAAATGA AGTCTTTCCG GGAAAAGGTT AAAGAAAAAT CTCTGGCGGC
351 GATGCGCCAT TCTTCGCTGT TGGATGTAAA AATTGCCCCC GAAGTTGCCA
401 AACGTTATCT GGAGGTGCGG GGTTTTCAGG GAAAAACCGT CAGCCGTGAG
451 GACGGGTCGG TTCTGATTGC CGCCAAAAAA GGCAcaatga acaaATGGGG
501 CTATATCTTT GCccaagtag ctTTGATTGT CATTTGCCTG GGCGGGTTGA
551 TAGACAGTAA CCTGCTGCTG AAGCTGGGTA TGCTGGCCGG TCGGATTGTT
601 CCGGACAATC AGGCGGTTTA TGCCAAGGAT TTCAAGCCCG AAAGTATTTT
651 GGGTGCGTCC AATCTCTCAT TTAGGGGCAA CGTCAATATT TCCGAGGGGC
701 AAAGTGCGGA TGTGGTTTTC CTGAATGCCG ACAACGGGAT GTTGGTTCAG
751 GACTTGCCTT TTGAAGTCAA ACTGAAAAAA TTCCATATCG ATTTTTACAA
801 TACGGGTATG CCGCGCGATT TTGCCAGCGA TATTGAAGTA ACGGACAAGG
851 CAACCGGTGA GAAACTCGAG CGCACCATCC GCGTGAACCA TCCTTTGACC
901 TTGCACGGCA TCACGATTTA TCAGGCGAGT TTTGCCGACG GCGGTTCGGA
951 TTTGACATTC AAGGCGTGGA ATTTGAGGGA TGCTTCGCGC GAACCTGTCG
1001 TGTTGAAGGC AACCTCCATA CACCAGTTTC CGTTGGAAAT CGGCAAACAC
1051 AAATATCGTC TTGAGTTCGA TCAGTTCACT TCTATGAATG TGGAGGACAT
1101 GAGCGAGGGT GCGGAACGGG AAAAAAGCCT GAAATCCACT CTGAACGATG
1151 TCCGCGCCGT TACTCAGGAA GGTAAAAAAT ACACCAATAT CGGCCCTTCC
1201 ATCGTGTACC GCATCCGTGA TGcggCAGGG CAGGCGGTCG AATATAAAAA
1251 CTATATGCTG CCGATTTTGC AGGACAAAGA TTATTTTTGG CTGACCGGCA
1301 CGCGCAGCGG CTTGCAGCAG CAATACCGCT GGCTGCGTAT CCCCTTGGAC
1351 AAGCAGTTGA AAGCGGACAC CTTTATGGCA TTGCGTGAGT TTTTGAAAGA
1401 TGGGGAAGGG CGCAAACGTC TGGTTGCCGA CGCAACCAAA GACGCACCTG
1451 CCGAAATCCG CGAACAATTC ATGCTGGCTG CGGAAAACAC GCTGAATATC
1501 TTTGCGCAAA AAGGCTATTT GGGATTGGAC GAATTTATTA CGTCCAATAT
1551 CCCGAAAGGG CAGCAGGATA AGATGCAGGG CTATTTCTAC GAAATGCTTT
1601 ACGGCGTGAT GAACGCTGCT TTGGATGAAA CCATACGCCG GTACGGCTTG
1651 CCCGAATGGC AGCAGGATGA AGCGCGGAAC CGTTTCCTGC TGCACAGTAT
1701 GGATGCCTAT ACGGGGCTGA CGGAATATCC CGCGCCTATG CTGCTCCAGC
1751 TTGACGGGTT TTCCGAGGTG CGTTCCTCAG GTTTGCAGAT GACCCGTTCG
1801 CCGGGTGCGC TTTTGGTCTA TCtcggctcg gtattgttgg TTTTGGtac
1851 ggtaTttatg tTTTATGTGC GCGAAAAACG GGCGTGGgta tTGTTTTCag

1901 aCGGCAAAAT CCGTTTTGCT ATGtCTTcgg CCcgcagcga ACGGGATTTG
1951 cAGAaggaaT TTCCAAAACA CGtcgAGAGC CTGCAACggc tcggcaaggA
2001 CttgaaTCAT GACTga

This corresponds to the amino acid sequence <SEQ ID 336; ORF88ng-1>:

1 MSKSRISPTL LSRPWFAFFS SMRFAVALLS LLGIASVIGT VLQQNQPQTD
51 YLVKFGPFWT RIFDFLGLYD VYASAWFVVI MMFLVVSTSL CLIRNVPPFW
101 REMKSFREKV KEKSLAAMRH SSLLDVKIAP EVAKRYLEVR GFQGKTVSRE
151 DGSVLIAAKK GTMNKWGYIF AQVALIVICL GGLIDSNLLL KLGMLAGRIV
201 PDNQAVYAKD FKPESILGAS NLSFRGNVNI SEGQSADVVF LNADNGMLVQ
251 DLPFEVKLKK FHIDFYNTGM PRDFASDIEV TDKATGEKLE RTIRVNHPLT
301 LHGITIYQAS FADGGSDLTF KAWNLRDASR EPVVLKATSI HQFPLEIGKH
351 KYRLEFDQFT SMNVEDMSEG AEREKSLKST LNDVRAVTQE GKKYTNIGPS
401 IVYRIRDAAG QAVEYKNYML PILQDKDYFW LTGTRSGLQQ QYRWLRIPLD
451 KQLKADTFMA LREFLKDGEG RKRLVADATK DAPAEIREQF MLAAENTLNI
501 FAQKGYLGLD EFITSNIPKG QQDKMQGYFY EMLYGVMNAA LDETIRRYGL
551 PEWQQDEARN RFLLHSMDAY TGLTEYPAPM LLQLDGFSEV RSSGLQMTRS
601 PGALLVYLGS VLLVLGTVFM FYVREKRAWV LFSDGKIRFA MSSARSERDL
651 QKEFPKHVES LQRLGKDLNH D*

ORF88ng-1 and ORF88-1 show 97.0% identity in 671 aa overlap:

```
orf88-1.pep  MSKSRRSPPLLSRPWFAFFSSMRFAVALLSLLGIASVIGTVLQQNQPQTDYLVKFGSFWA   60
             ||||| || ||||||||||||||||||||||||||||||||||||||||||||||| ||:
orf88ng-1    MSKSRISPTLLSRPWFAFFSSMRFAVALLSLLGIASVIGTVLQQNQPQTDYLVKFGPFWT   60

orf88-1.pep  QIFGFLGLYDVYASAWFVVIMMFLVVSTSLCLIRNVPPFWREMKSFREKVKEKSLAAMRH  120
             :|| ||||||||||||||||||||||||||||||||||||||||||||||||||||||||
orf88ng-1    RIFDFLGLYDVYASAWFVVIMMFLVVSTSLCLIRNVPPFWREMKSFREKVKEKSLAAMRH  120

orf88-1.pep  SSLLDVKIAPEVAKRYLEVQGFQGKTINREDGSVLIAAKKGTMNKWGYIFAHVALIVICL  180
             |||||||||||||||||||:||||||::|||||||||||||||||||||||:||||||||
orf88ng-1    SSLLDVKIAPEVAKRYLEVRGFQGKTVSREDGSVLIAAKKGTMNKWGYIFAQVALIVICL  180

orf88-1.pep  GGLIDSNLLLKLGMLTGRIVPDNQAVYAKDFKPESILGASNLSFRGNVNISEGQSADVVF  240
             |||||||||||||||:||||||||||||||||||||||||||||||||||||||||||||
orf88ng-1    GGLIDSNLLLKLGMLAGRIVPDNQAVYAKDFKPESILGASNLSFRGNVNISEGQSADVVF  240

orf88-1.pep  LNADNGILVQDLPFEVKLKKFHIDFYNTGMPRDFASDIEVTDKATGEKLERTIRVNHPLT  300
             ||||||:|||||||||||||||||||||||||||||||||||||||||||||||||||||
orf88ng-1    LNADNGMLVQDLPFEVKLKKFHIDFYNTGMPRDFASDIEVTDKATGEKLERTIRVNHPLT  300

orf88-1.pep  LHGITIYQASFADGGSDLTFKAWNLGDASREPVVLKATSIHQFPLEIGKHKYRLEFDQFT  360
             ||||||||||||||||||||||||| ||||||||||||||||||||||||||||||||||
orf88ng-1    LHGITIYQASFADGGSDLTFKAWNLRDASREPVVLKATSIHQFPLEIGKHKYRLEFDQFT  360

orf88-1.pep  SMNVEDMSEGAEREKSLKSTLNDVRAVTQEGKKYTNIGPSIVYRIRDAAGQAVEYKNYML  420
             ||||||||||||||||||||||||||||||||||||||||||||||||||||||||||||
orf88ng-1    SMNVEDMSEGAEREKSLKSTLNDVRAVTQEGKKYTNIGPSIVYRIRDAAGQAVEYKNYML  420

orf88-1.pep  PVLQEQDYFWITGTRSGLQQQYRWLRIPLDKQLKADTFMALREFLKDGEGRKRLVADATK  480
             |:||::||||:|||||||||||||||||||||||||||||||||||||||||||||||||
orf88ng-1    PILQDKDYFWLTGTRSGLQQQYRWLRIPLDKQLKADTFMALREFLKDGEGRKRLVADATK  480

orf88-1.pep  GAPAEIREQFMLAAENTLNIFAQKGYLGLDEFITSNIPKEQQDKMQGYFYEMLYGVMNAA  540
              |||||||||||||||||||||||||||||||||||||| ||||||||||||||||||||
orf88ng-1    DAPAEIREQFMLAAENTLNIFAQKGYLGLDEFITSNIPKGQQDKMQGYFYEMLYGVMNAA  540

orf88-1.pep  LDETIRRYGLPEWQQDEARNRFLLHSMDAYTGLTEYPAPMLLQLDGFSEVRSSGLQMTRS  600
             ||||||||||||||||||||||||||||||||||||||||||||||||||||||||||||
orf88ng-1    LDETIRRYGLPEWQQDEARNRFLLHSMDAYTGLTEYPAPMLLQLDGFSEVRSSGLQMTRS  600

orf88-1.pep  PGALLVYLGSVLLVLGTVLMFYVREKRAWVLFSDGKIRFAMSSARSERDLQKEFPKHVES  660
             ||||||||||||||||||:|||||||||||||||||||||||||||||||||||||||||
orf88ng-1    PGALLVYLGSVLLVLGTVFMFYVREKRAWVLFSDGKIRFAMSSARSERDLQKEFPKHVES  660

orf88-1.pep  LQRLGKDLNHD   671
             |||||||||||
orf88ng-1    LQRLGKDLNHD   671
```

Furthermore, ORG88ng-1 shows homology with a hypothetical protein from *Aquifex aeolicus:*

```
gi|2984296 (AE000771) hypothetical protein [Aquifex aeolicus] Length = 537
 Score = 94.4 bits (231), Expect = 2e-18
 Identities = 91/334 (27%), Positives = 159/334 (47%), Gaps = 59/334 (17%)

Query: 16  FAFFSSMRFAVALLSLLGIASVIG-TVLQQNQPQTDYLVKFGPFWTRIFDFLGLYDVYAS 74
            + F +S++ A+ ++ +LGI S++G T ++QNQ    YL +FG         L L DV+ S
Sbjct: 80  YDFLASLKLAIFIMLVLGILSMLGSTYIKQNQSFEWYLDQFGYDVGIWIWKLWLNDVFHS 139

Query: 75  AWFVVIMMFLVVSTSLCLIRNVPPFWREMKSFREKVKEKSLAAMRHSSLLDVKIAPEVAK 134
             ++++ ++ L V+    C I+ +P  W++  S +E++ +    A +H   + VKI P+  K
Sbjct: 140 WYYILFIVLLAVNLIFCSIKRLPRVWKQAFS-KERILKLDEHAEKHLKPITVKI-PDKDK 197

Query: 135 --RYLEVRGFQGKTVSREDGSVLIAAKKGTMNKWGYIFAQVALIVICLGGLIDSNLLLKL 192
      ++L  +GF+   V  E   + + A+KG  ++ G     +AL+VI  G LID
Sbjct: 198 VLKFLLKKGFK-VFVEEEGNKLYVFAEKGRFSRLGVYITHIALLVIMAGALID------- 249

Query: 193 GMLAGRIVPDNQAVYAKDFKPESILGASNLSFRGNVNISEGQSADVVFLNADNGMLVQDL 252
                                 +I+G      RG++ ++EG + DV+ + A+       L
Sbjct: 250 ---------------------AIVGV-----RGSLIVAEGDTNDVMLVGAE--QKPYKL 280

Query: 253 PFEVKLKKFHIDFY---NTGMPRDFA-------SDIEVTDKATGEKLER--TIRVNHPLT 300
            PF V L  F I  Y   N  + + FA       SDIE+ +   G K+E   T++VN P
Sbjct: 281 PFAVHLIDFRIKTYAEENPNVDKRFAQAVSSYESDIEIIN---GGKVEAKGTVKVNEPFD 337

Query: 301 LHGITIYQASFA--DGGSDLTFKAWNLRDASREP 332
                ++QA++   DG S +     + + A  +P
Sbjct: 338 FGRYRLFQATYGILDGTSGMGVIVVDRKKAHEDP 371
```

Based on this analysis, including the putative transmembrane domain in the gonococcal protein, it is predicted that the proteins from *N.meningitidis* and *N.gonorrhoeae,* and their epitopes, could be useful antigens for vaccines or diagnostics, or for raising antibodies.

**Example 40**

The following DNA sequence, believed to be complete, was identified in *N.meningitidis* <SEQ ID 337>:

```
  1 ATGATGAGTA ATAmAATGGm ACAAAAAGGG TTTACATTGA TTGmGmTGAT
 51 GATAGTCGTC GCGATACTCG GCATTATCAG CGTCATTGCC ATACCTTCTT
101 ATCmAAGTTA TATTGAAAAA GGCTATCAGT CCCAGCTTTA TACGGAGATG
151 GyCGGTATCA ACAATATTTC CAAACAGTTT ATTTTGAAAA ATCCCCTGGA
201 CGATAATCAG ACCATCGAGA ACAAACTGGA AATATTTGTC TCAGGCTATA
251 AGATGAATCC GAAAATTGCC AAAAAaTATA GTGTTTCGGT AAAGTTTGTC
301 GATAAGGAAA AATCAAGGGC ATACAGGTTG GTCGGCGTTC CGAAGGCGGG
351 GACGGGTTAT ACTTTGTCGG TATGGATGAA CAGCGTGGGC GACGGATACA
401 AATGCCGTGA TGCCGCTTCT GCCCAAGCCC ATTTGGAGAC CTTGTCCTCA
451 GATGTCGGCT GTGAAGCCTT CTCTAATCGT AAAAAATAA
```

This corresponds to the amino acid sequence <SEQ ID 338; ORF89>:

```
  1 MMSNXMXQKG FTLIXXMIVV AILGIISVIA IPSYXSYIEK GYQSQLYTEM
 51 XGINNISKQF ILKNPLDDNQ TIENKLEIFV SGYKMNPKIA KKYSVSVKFV
101 DKEKSRAYRL VGVPKAGTGY TLSVWMNSVG DGYKCRDAAS AQAHLETLSS
151 DVGCEAFSNR KK*
```

Further work revealed the complete nucleotide sequence <SEQ ID 339>:

```
  1  ATGATGAGTA ATAAAATGGA ACAAAAAGGG TTTACATTGA TTGAGATGAT
 51  GATAGTCGTC GCGATACTCG GCATTATCAG CGTCATTGCC ATACCTTCTT
101  ATCAAAGTTA TATTGAAAAA GGCTATCAGT CCCAGCTTTA TACGGAGATG
151  GTCGGTATCA ACAATATTTC CAAACAGTTT ATTTTGAAAA ATCCCCTGGA
201  CGATAATCAG ACCATCGAGA ACAAACTGGA AATATTTGTC TCAGGCTATA
251  AGATGAATCC GAAAATTGCC AAAAAATATA GTGTTTCGGT AAAGTTTGTC

301  GATAAGGAAA AATCAAGGGC ATACAGGTTG GTCGGCGTTC CGAAGGCGGG
351  GACGGGTTAT ACTTTGTCGG TATGGATGAA CAGCGTGGGC GACGGATACA
401  AATGCCGTGA TGCCGCTTCT GCCCAAGCCC ATTTGGAGAC CTTGTCCTCA
451  GATGTCGGCT GTGAAGCCTT CTCTAATCGT AAAAAATAA
```

This corresponds to the amino acid sequence <SEQ ID 340; ORF89-1>:

```
  1  MMSNKMEQKG FTLIEMMIVV AILGIISVIA IPSYQSYIEK GYQSQLYTEM
 51  VGINNISKQF ILKNPLDDNQ TIENKLEIFV SGYKMNPKIA KKYSVSVKFV
101  DKEKSRAYRL VGVPKAGTGY TLSVWMNSVG DGYKCRDAAS AQAHLETLSS
151  DVGCEAFSNR KK*
```

Computer analysis of this amino acid sequence gave the following results:

<u>Homology with PilE of *N. gonorrhoeae* (accession number Z69260).</u>

ORF89 and PilE protein show 30% aa identity in 120a overlap:

```
orf89  8   QKGFTLIXXMIVVAILGIISVIAIPSYXSYIEKGYQSQLYTEMXGINNISKQFILKNPL- 66
           QKGFTLI  MIV+AI+GI++ +A+P+Y  Y  +   S+      G  +    ++ L + +
PilE   5   QKGFTLIELMIVIAIVGILAAVALPAYQDYTARAQVSEAILLAEGQKSAVTEYYLNHGIW 64

orf89  67  -DDNQTIENKLEIFVSGYKMNPKIAKKYSVSVKFVDKEKSRAYRLVGVPKAGTGYTLSVW 125
             DN +         +G   + KI  KY  SV       +      GV K   G  LS+W
PilE   65  PKDNTS---------AGVASSDKIKGKYVQSVTVAKGVVTAEMASTGVNKEIQGKKLSLW 115
```

<u>Homology with a predicted ORF from *N. meningitidis* (strain A)</u>

ORF89 shows 83.3% identity over a 162aa overlap with an ORF (ORF89a) from strain A of *N. meningitidis:*

```
                      10        20        30        40        50        60
orf89.pep   MMSNXMXQKGFTLIXXMIVVAILGIISVIAIPSYXSYIEKGYQSQLYTEMXGINNISKQF
            |||| | |||||||||    ||    |||    ||||||||||||||||| ||||||||
orf89a      MMSNKMEQKGFTLIXXXXXXAIXXXXSVIXXXXYXSYIEKGYQSQLYTEMVGINNISKQX
                      10        20        30        40        50        60

                      70        80        90       100       110       120
orf89.pep   ILKNPLDDNQTIENKLEIFVSGYKMNPKIAKKYSVSVKFVDKEKSRAYRLVGVPKAGTGY
            ||||||||||||::||||||||||||||||:||:|||:||::|| ||| ||||||:||||
orf89a      ILKNPLDDNQTIKSKLEIFVSGYKMNPKIAEKYNVSVHFVNEEKPRAYSLVGVPKTGTGY
                      70        80        90       100       110       120

                     130       140       150       160
orf89.pep   TLSVWMNSVGDGYKCRDAASAQAHLETLSSDVGCEAFSNRKKX
            |||||||||||||||||||||:||||||||||||||||||||
orf89a      TLSVWMNSVGDGYKCRDAASARAHLETLSSDVGCEAFSNRKKX
                     130       140       150       160
```

The complete length ORF89a nucleotide sequence <SEQ ID 341> is:

```
  1 ATGATGAGTA ATAAAATGGA ACAAAAAGGG TTTACATTGA TTGNGANGNT
 51 NATNGNCNTC GCGATACNCN GCNTTANCAG CGTCATTNCN ATNNNTNCNT
101 ATCNNAGTTA TATTGAAAAA GGCTATCAGT CCCAGCTTTA TACGGAGATG
151 GTCGGTATCA ACAATATTTC CAAACAGTNT ATTTTGAAAA ATCCCCTGGA
201 CGATAATCAG ACCATCAAGA GCAAACTGGA AATATTTGTC TCAGGCTATA
251 AGATGAATCC GAAAATTGCC GAAAAATATA ATGTTTCGGT GCATTTTGTC
301 AATGAGGAAA AACCNAGGGC ATACAGCTTG GTCGGCGTTC CAAAGACGGG
351 GACGGGTTAT ACTTTGTCGG TATGGATGAA CAGCGTGGGC GACGGATACA
401 AATGCCGTGA TGCCGCTTCT GCCCGAGCCC ATTTGGAGAC CTTGTCCTCA
451 GATGTCGGCT GTGAAGCCTT CTCTAATCGT AAAAAATAG
```

This encodes a protein having amino acid sequence <SEQ ID 342>:

```
  1 MMSNKMEQKG FTLIXXXXXX AIXXXXSVIX XXXYXSYIEK GYQSQLYTEM
 51 VGINNISKQX ILKNPLDDNQ TIKSKLEIFV SGYKMNPKIA EKYNVSVHFV
101 NEEKPRAYSL VGVPKTGTGY TLSVWMNSVG DGYKCRDAAS ARAHLETLSS
151 DVGCEAFSNR KK*
```

ORF89a and ORF89-1 show 83.3% identity in 162 aa overlap:

```
                        10        20        30        40        50        60
orf89a.pep     MMSNKMEQKGFTLIXXXXXXAIXXXXSVIXXXXYXSYIEKGYQSQLYTEMVGINNISKQX
               ||||||||||||||      ||    |||    | |||||||||||||||||||||||| 
orf89-1        MMSNKMEQKGFTLIEMMIVVAILGIISVIAIPSYQSYIEKGYQSQLYTEMVGINNISKQF
                        10        20        30        40        50        60

                        70        80        90       100       110       120
orf89a.pep     ILKNPLDDNQTIKSKLEIFVSGYKMNPKIAEKYNVSVHFVNEEKPRAYSLVGVPKTGTGY
               ||||||||||||::||||||||||||||||:||:|||:||::|| ||| ||||||:||||
orf89-1        ILKNPLDDNQTIENKLEIFVSGYKMNPKIAKKYSVSVKFVDKEKSRAYRLVGVPKAGTGY
                        70        80        90       100       110       120

                       130       140       150       160
orf89a.pep     TLSVWMNSVGDGYKCRDAASARAHLETLSSDVGCEAFSNRKKX
               |||||||||||||||||||||:|||||||||||||||||||||
orf89-1        TLSVWMNSVGDGYKCRDAASAQAHLETLSSDVGCEAFSNRKKX
                       130       140       150       160
```

## Homology with a predicted ORF from *N.gonorrhoeae*

ORF89 shows 84.6% identity over a 162aa overlap with a predicted ORF (ORF89.ng) from *N. gonorrhoeae:*

```
orf89      MMSNXMXQKGFTLIXXMIVVAILGIISVIAIPSYXSYIEKGYQSQLYTEMXGINNISKQF   60
           |||| | |||||||  ||||:||||||||||||| ||||||||||||||| ||||: |||
orf89ng    MMSNKMEQKGFTLIEMMIVVTILGIISVIAIPSYQSYIEKGYQSQLYTEMVGINNVLKQF   60

orf89      ILKNPLDDNQTIENKLEIFVSGYKMNPKIAKKYSVSVKFVDKEKSRAYRLVGVPKAGTGY  120
           ||||| |||:|:::||:||||||||||||||||||||:||| || |||||||||:|||||
orf89ng    ILKNPQDDNDTLKSKLKIFVSGYKMNPKIAKKYSVSVRFVDAEKPRAYRLVGVPNAGTGY  120

orf89      TLSVWMNSVGDGYKCRDAASAQAHLETLSSDVGCEAFSNRKK  162
           ||||||||||||||||||:||||: :|||:| ||||||||||
orf89ng    TLSVWMNSVGDGYKCRDATSAQAYSDTLSADSGCEAFSNRKK  162
```

The complete length ORF89ng nucleotide sequence <SEQ ID 343> is:

```
  1 aTGATGAGCA ATAAAATGGA ACAAAAAGGG TTTACATTGA TTGAGATGAT
 51 GATAGTTGTC ACGATACTCG GCATCATCAG CGTCATTGCC ATACCTTCTT
101 ATCAGAGTTA TATTGAAAAA GGCTATCAGT CCCAGCTTTA TACGGAGATG
151 GTCGGTATCA ACAATGTTCT CAAACAGTTT ATTTTGAAAA ATCCCCAGGA
201 CGATAATGAT ACCCTCAAGA GCAAACTGAA AATATTTGTC TCAGGCTATA
251 AGATGAATCC GAAAAttgCC AAAAAATATA GTGTTTCGGt aaggtttGTC
301 gatGCGGAAA AACCAAGGGC ATACAGGTTG GTCGGCGTTC CGAACGCGGG
351 GACGGGTTAT ACTTTGTCGG TATGGATGAA CAGCGTGGGC GACGGATACA
401 AATGCCGTGA TGCCACTTCT GCCCAGGCCT ATTCGGACAC CTTGTCCGCA
451 GATAGCGGCT GTGAAGCTTT CTCTAATCGT AAAAAATAG
```

This encodes a protein having amino acid sequence <SEQ ID 344>:

```
  1 MMSNKMEQKG FTLIEMMIVV TILGIISVIA IPSYQSYIEK GYQSQLYTEM
 51 VGINNVLKQF ILKNPQDDND TLKSKLKIFV SGYKMNPKIA KKYSVSVRFV
101 DAEKPRAYRL VGVPNAGTGY TLSVWMNSVG DGYKCRDATS AQAYSDTLSA
151 DSGCEAFSNR KK*
```

This gonococcal protein has a putative leader peptide (underlined) and N-terminal methylation site (NMePhe or type-4 pili, double-underlined). In addition, ORF89ng and ORF89-1 show 88.3% identity in 162 aa overlap:

```
                    10        20        30        40        50        60
orf89-1.pep  MMSNKMEQKGFTLIEMMIVVAILGIISVIAIPSYQSYIEKGYQSQLYTEMVGINNISKQF
             |||||||||||||||||||:||||||||||||||||||||||||||||||||||: |||
orf89ng      MMSNKMEQKGFTLIEMMIVVTILGIISVIAIPSYQSYIEKGYQSQLYTEMVGINNVLKQF
                    10        20        30        40        50        60


                    70        80        90       100       110       120
orf89-1.pep  ILKNPLDDNQTIENKLEIFVSGYKMNPKIAKKYSVSVKFVDKEKSRAYRLVGVPKAGTGY
             ||||| |||:|:::||:||||||||||||||||||||:||| || ||||||||:|||||
orf89ng      ILKNPQDDNDTLKSKLKIFVSGYKMNPKIAKKYSVSVRFVDAEKPRAYRLVGVPNAGTGY
                    70        80        90       100       110       120

                   130       140       150       160
orf89-1.pep  TLSVWMNSVGDGYKCRDAASAQAHLETLSSDVGCEAFSNRKKX
             ||||||||||||||||||:||||: :|||:| |||||||||||
orf89ng      TLSVWMNSVGDGYKCRDATSAQAYSDTLSADSGCEAFSNRKKX
                   130       140       150       160·
```

Based on this analysis, including the gonococcal motifs and the homology with the known PilE protein, it was predicted that these proteins from *N.meningitidis* and *N.gonorrhoeae,* and their epitopes, could be useful antigens for vaccines or diagnostics, or for raising antibodies.

ORF89-1 (13.6kDa) was cloned in the pGex vector and expressed in *E.coli,* as described above. The products of protein expression and purification were analyzed by SDS-PAGE. Figure 11A shows the results of affinity purification of the GST-fusion protein. Purified GST-fusion protein was used to immunise mice, whose sera gave a positive result in the ELISA test., confirming that ORF89-1 is a surface-exposed protein, and that it is a useful immunogen.

## Example 41

The following partial DNA sequence was identified in *N.meningitidis* <SEQ ID 345>:

```
  1  ATGAAAAAAT CCTCCCTCAT CAGCGCATTG GGCATCGGTA TTTTGAGCAT
 51  CGGCATGGCA TTTGCCGCCC CTGCCGACGC GGTAAGCCAA ATCCGTCAAA
101  ACGCCACTCA AGTATTGAGC ATCTTAAAAA ACGGCGATGC CAACACCGCT
151  CGCCAAAAAG CCGAAGCCTA TGCGATTCCC TATTTCGATT TCCAACGTAT
201  GACCGCATTG GCGGTCGGCA ACCCTTGGsG CACCG.GTCC GACG.GCAAA
251  AACAAGCGTT GGCCn.AGAA TTTCAACCC...
```

This corresponds to the amino acid sequence <SEQ ID 346; ORF91>:

```
  1  MKKSSLISAL GIGILSIGMA FAAPADAVSQ IRQNATQVLS ILKNGDANTA
 51  RQKAEAYAIP YFDFQRMTAL AVGNPWXTXS DXQKQALAXE FQP...
```

Further work revealed the complete nucleotide sequence <SEQ ID 347>:

```
  1  ATGAAAAAAT CCTCCCTCAT CAGCGCATTG GGCATCGGTA TTTTGAGCAT
 51  CGGCATGGCA TTTGCCGCCC CTGCCGACGC GGTAAGCCAA ATCCGTCAAA
101  ACGCCACTCA AGTATTGAGC ATCTTAAAAA ACGGCGATGC CAACACCGCT
151  CGCCAAAAAG CCGAAGCCTA TGCGATTCCC TATTTCGATT TCCAACGTAT
201  GACCGCATTG GCGGTCGGCA ACCCTTGGCG CACCGCGTCC GACGCGCAAA
251  AACAAGCGTT GGCCAAAGAA TTTCAAACCC TGCTGATCCG CACCTATTCC
301  GGCACGATGC TGAAATTAAA AAACGCCAAC GTCAACGTCA AAGACAATCC
351  CATCGTCAAT AAAGGCGGCA AAGAAATCAT CGTCCGCGCC GAAGTCGGCG
401  TACCCGGGCA AAAACCCGTC AACATGGACT TCACCACCTA CCAAAGCGGC
451  GGTAAATACC GTACCTACAA CGTCGCCATC GAAGGCGCGA GCCTGGTTAC
501  CGTGTACCGC AACCAATTCG GCGAAATTAT CAAAGCGAAA GGCGTGGACG
551  GACTGATTGC CGAGTTGAAA GCCAAAAACG GCGGCAAATA A
```

This corresponds to the amino acid sequence <SEQ ID 348; ORF91-1>:

```
  1  MKKSSLISAL GIGILSIGMA FAAPADAVSQ IRQNATQVLS ILKNGDANTA
 51  RQKAEAYAIP YFDFQRMTAL AVGNPWRTAS DAQKQALAKE FQTLLIRTYS
101  GTMLKLKNAN VNVKDNPIVN KGGKEIIVRA EVGVPGQKPV NMDFTTYQSG
151  GKYRTYNVAI EGASLVTVYR NQFGEIIKAK GVDGLIAELK AKNGGK*
```

Computer analysis of this amino acid sequence gave the following results:

Homology with a predicted ORF from *N.meningitidis* strain A)

ORF91 shows 92.4% identity over a 92aa overlap with an ORF (ORF91 a) from strain A of *N. meningitidis:*

```
                    10        20        30        40        50        60
orf91.pep   MKKSSLISALGIGILSIGMAFAAPADAVSQIRQNATQVLSILKNGDANTARQKAEAYAIP
            |||||:||||||||||||||||||||||||:||||||||||||||:|||||||||||||||
orf91a      MKKSSFISALGIGILSIGMAFAAPADAVNQIRQNATQVLSILKSGDANTARQKAEAYAIP
                    10        20        30        40        50        60

                    70        80        90
orf91.pep   YFDFQRMTALAVGNPWXTXSDXQKQALAXEFQP
            ||||||||||||||||  | || |||||| |||
orf91a      YFDFQRMTALAVGNPWRTASDAQKQALAKEFQTLLIRTYSGTMLKLKNANVNVKDNPIVN
                    70        80        90       100       110       120

orf91a      KGGKEIIVRAEVGVPGQKPVNMDFTTYQSGGKYRTYNVAIEGASLVTVYRNQFGEIIKAK
                   130       140       150       160       170       180
```

The complete length ORF91 a nucleotide sequence <SEQ ID 349> is:

```
  1 ATGAAAAAAT CCTCCTTCAT CAGCGCATTG GGCATCGGTA TTTTGAGCAT
 51 CGGCATGGCA TTTGCCGCCC CTGCCGACGC GGTAAACCAA ATCCGTCAAA
101 ACGCCACTCA AGTATTGAGC ATCTTAAAAA GCGGTGATGC CAACACCGCC
151 CGCCAAAAAG CCGAAGCCTA TGCGATTCCC TATTTCGATT TCCAACGTAT
201 GACCGCATTG GCGGTCGGCA ACCCTTGGCG CACCGCGTCC GACGCGCAAA
251 AACAAGCGTT GGCCAAAGAA TTTCAAACCC TGCTGATCCG CACCTATTCC
301 GGCACGATGC TGAAATTAAA AAACGCCAAC GTCAACGTCA AAGACAATCC
351 CATCGTCAAT AAAGGCGGCA AAGAAATCAT CGTCCGCGCC GAAGTCGGCG
401 TACCCGGGCA AAAACCCGTC AACATGGACT TCACCACCTA CCAAAGCGGC
451 GGTAAATACC GTACCTACAA CGTCGCCATC GAAGGCGCGA GCCTGGTTAC
501 CGTGTACCGC AACCAATTCG GCGAAATTAT CAAAGCGAAA GGCGTGGACG
551 GACTGATTGC CGAGTTGAAG GCTAAAAACG GCAGCAAGTA A
```

This encodes a protein having amino acid sequence <SEQ ID 350>:

```
  1 MKKSSFISAL GIGILSIGMA FAAPADAVNQ IRQNATQVLS ILKSGDANTA
 51 RQKAEAYAIP YFDFQRMTAL AVGNPWRTAS DAQKQALAKE FQTLLIRTYS
101 GTMLKLKNAN VNVKDNPIVN KGGKEIIVRA EVGVPGQKPV NMDFTTYQSG
151 GKYRTYNVAI EGASLVTVYR NQFGEIIKAK GVDGLIAELK AKNGSK*
```

ORF91a and ORF91-1 show 98.0% identity in 196 aa overlap:

```
                    10        20        30        40        50        60
orf91a.pep  MKKSSFISALGIGILSIGMAFAAPADAVNQIRQNATQVLSILKSGDANTARQKAEAYAIP
            |||||:|||||||||||||||||||||||:||||||||||||||:|||||||||||||||
orf91-1     MKKSSLISALGIGILSIGMAFAAPADAVSQIRQNATQVLSILKNGDANTARQKAEAYAIP
                    10        20        30        40        50        60

                    70        80        90       100       110       120
orf91a.pep  YFDFQRMTALAVGNPWRTASDAQKQALAKEFQTLLIRTYSGTMLKLKNANVNVKDNPIVN
            ||||||||||||||||||||||||||||||||||||||||||||||||||||||||||||
orf91-1     YFDFQRMTALAVGNPWRTASDAQKQALAKEFQTLLIRTYSGTMLKLKNANVNVKDNPIVN
                    70        80        90       100       110       120

                   130       140       150       160       170       180
orf91a.pep  KGGKEIIVRAEVGVPGQKPVNMDFTTYQSGGKYRTYNVAIEGASLVTVYRNQFGEIIKAK
            ||||||||||||||||||||||||||||||||||||||||||||||||||||||||||||
orf91-1     KGGKEIIVRAEVGVPGQKPVNMDFTTYQSGGKYRTYNVAIEGASLVTVYRNQFGEIIKAK
                   130       140       150       160       170       180

                   190
orf91a.pep  GVDGLIAELKAKNGSKX
            ||||||||||||||:||
orf91-1     GVDGLIAELKAKNGGKX
                   190
```

Homology with a predicted ORF from *N.gonorrhoeae*

ORF91 shows 84.8% identity over a 92aa overlap with a predicted ORF (ORF91.ng) from *N. gonorrhoeae:*

```
orf91.pep   MKKSSLISALGIGILSIGMAFAAPADAVSQIRQNATQVLSILKNGDANTARQKAEAYAIP   60
            :||||:|||||||||||||||||:|||||:||||||||||:|||:||| :|| ||||||:|
orf91ng     VKKSSFISALGIGILSIGMAFASPADAVGQIRQNATQVLTILKSGDAASARPKAEAYAVP   60

orf91.pep   YFDFQRMTALAVGNPWXTXSDXQKQALAXEFQP                              93
            |||||||||||||||| | || |||||| |||
orf91ng     YFDFQRMTALAVGNPWRTASDAQKQALAKEFQTLLIRTYSGTMLKFKNATVNVKDNPIVN  120
```

The complete length ORF91ng nucleotide sequence <SEQ ID 351> is predicted to encode a protein having amino acid sequence <SEQ ID 352>:

```
  1   VKKSSFISAL GIGILSIGMA FASPADAVGQ IRQNATQVLT ILKSGDAASA
 51   RPKAEAYAVP YFDFQRMTAL AVGNPWRTAS DAQKQALAKE FQTLLIRTYS
101   GTMLKFKNAT VNVKDNPIVN KGGKEIVVRA EVGIPGQKPV NMDFTTYQSG
151   GKYRTYNVAI EGTSLVTVYR NQFGEIIKAK GIDGLIAELK AKNGGK*
```

Further work revealed the complete nucleotide sequence <SEQ ID 353>:

```
  1   ATGAAAAAAT CCTCCTTCAT CAGCGCATTG GGCATCGGTA TTTTGAGCAT
 51   CGGCATGGCA TTTGCCTCCC CGGCCGACGC AGTGGGACAA ATCCGCCAAA
101   ACGCCACACA GGTTTTGACC ATCCTCAAAA GCGGCGACGC GGCTTCTGCA
151   CGCCCAAAAG CCGAAGCCTA TGCGGTTCCC TATTTCGATT TCCAACGTAT
201   GACCGCATTG GCGGTCGGCA ACCCTTGGCG TACCGCGTCC GACGCGCAAA
251   AACAAGCGTT GGCCAAAGAA TTTCAAACCC TGCTGATCCG CACCTATTCC
301   GGCACGATGC TGAAATTCAA AAACGCGACC GTCAACGTCA AAGACAATCC
351   CATCGTCAAT AAGGGCGGCA AGGAAATCGT CGTCCGTGCC GAAGTCGGCA
401   TCCCCGGTCA GAAGCCCGTC AATATGGACT TTACCACCTA CCAAAGCGGC
451   GGCAAATACC GTACCTACAA CGTCGCCATC GAAGGCACGA GCCTGGTTAC
501   CGTGTACCGC AACCAATTCG GCGAAATCAT CAAAGCCAAA GGCATCGACG
551   GGCTGATTGC CGAGTTGAAA GCCAAAAACG GCGGCAAATA A
```

This corresponds to the amino acid sequence <SEQ ID 354; ORF91ng-1>:

```
  1   MKKSSFISAL GIGILSIGMA FASPADAVGQ IRQNATQVLT ILKSGDAASA
 51   RPKAEAYAVP YFDFQRMTAL AVGNPWRTAS DAQKQALAKE FQTLLIRTYS
101   GTMLKFKNAT VNVKDNPIVN KGGKEIVVRA EVGIPGQKPV NMDFTTYQSG
151   GKYRTYNVAI EGTSLVTVYR NQFGEIIKAK GIDGLIAELK AKNGGK*
```

ORF91ng-1 and ORF91-1 show 92.3% identity in 196 aa overlap:

```
                      10        20        30        40        50        60
orf91-1.pep   MKKSSLISALGIGILSIGMAFAAPADAVSQIRQNATQVLSILKNGDANTARQKAEAYAIP
              |||||:||||||||||||||||:|||||:||||||||||:|||:||| :|| ||||||:|
orf91ng-1     MKKSSFISALGIGILSIGMAFASPADAVGQIRQNATQVLTILKSGDAASARPKAEAYAVP
                      10        20        30        40        50        60

                      70        80        90       100       110       120
orf91-1.pep   YFDFQRMTALAVGNPWRTASDAQKQALAKEFQTLLIRTYSGTMLKLKNANVNVKDNPIVN
              |||||||||||||||||||||||||||||||||||||||||||||:|||:||||||||||
orf91ng-1     YFDFQRMTALAVGNPWRTASDAQKQALAKEFQTLLIRTYSGTMLKFKNATVNVKDNPIVN
                      70        80        90       100       110       120

                     130       140       150       160       170       180
orf91-1.pep   KGGKEIIVRAEVGVPGQKPVNMDFTTYQSGGKYRTYNVAIEGASLVTVYRNQFGEIIKAK
              ||||||:||||||:||||||||||||||||||||||||||||:|||||||||||||||||
orf91ng-1     KGGKEIVVRAEVGIPGQKPVNMDFTTYQSGGKYRTYNVAIEGTSLVTVYRNQFGEIIKAK
                     130       140       150       160       170       180

                     190
orf91-1.pep   GVDGLIAELKAKNGGKX
              |:|||||||||||||||
orf91ng-1     GIDGLIAELKAKNGGKX
                     190
```

In addition, ORF91ng-1 shows homology to a hypothetical *E. coli* protein:

```
 sp|P45390|YRBC_ECOLI HYPOTHETICAL 24.0 KD PROTEIN IN MURA-RPON INTERGENIC
REGION PRECURSOR (F211) >gi|606130 (U18997) ORF_f211 [Escherichia coli]
>gi|1789583 (AE000399) hypothetical 24.0 kD protein in murZ-rpoN intergenic
region [Escherichia coli]Length = 211

 Score = 70.6 bits (170), Expect = 6e-12
 Identities = 42/137 (30%), Positives = 76/137 (54%), Gaps = 6/137 (4%)

Query: 59  VPYFDFQRMTALAVGNPWRTASDAQKQALAKEFQTLLIRTYSGTMLKFKNATVNVKDNPI 118
            +PY    +    AL +G  +++A+ AQ++A     F+  L + Y    +  +    T  +    P
Sbjct: 65  LPYVQVKYAGALVLGQYYKSATPAQREAYFAAFREYLKQAYGQALAMYHGQTYQIA--PE 122

Query: 119 VNKGGKEIV-VRAEVGIP-GQKPVNMDFTTYQSG--GKYRTYNVAIEGTSLVTVYRNQFG 174
               G K IV +R  +  P G+ PV +DF    ++    G ++ Y++   EG S++T   +N++G
Sbjct: 123 QPLGDKTIVPIRVTIIDPNGRPPVRLDFQWRKNSQTGNWQAYDMIAEGVSMITTKQNEWG 182

Query: 175 EIIKAKGIDGLIAELKA 191
            +++ KGIDGL A+LK+
Sbjct: 183 TLLRTKGIDGLTAQLKS 199
```

Based on this analysis, including the presence of a putative leader sequence in the gonococcal protein, it is predicted that the proteins from *N.meningitidis* and *N.gonorrhoeae,* and their epitopes, could be useful antigens for vaccines or diagnostics, or for raising antibodies.

**Example 42**

The following DNA sequence was identified in *N.meningitidis* <SEQ ID 355>:

```
  1  ATGAAACACA TACTCCCCCT GATTGCCGCA TCCGCACTCT GCATTTCAAC
 51  CGCTTCGGCA CATCCTGCCA GCGAACCGTC CACTCAAAAC GAAACCGCTA
101  TGATCACGCA TACCCTCATC TCAAAATACA GTTTTGGnnn nnnnnnnnnn
151  nnnnnnnnnn nnGCCATAAA AAGCAAAGGG ATGGACATTT TTGCCGTCAT
201  CGACCATCAG GAAGCCGCAC GCCGAAACGG CTTAACGATG CAGCCGGCAA
251  AAGTCATCGT CTTCGGCACG CCCAAAGCCG GCACGCCGCT GATGGTCAAA
301  GACCCCGCCT TCGCCCTGCA ACTGCCCCTA CGCGTCCTCG TTACCGAAAC
351  GGACGGCAAA GTACGCGCCG CCTATACCGA TACGCGCGCC CTCATCGCCG
401  GCAGCCGCAT CGGTTTCGAC GAAGTGGCAA ACACTTTGGC AAACGCCGAA
451  AAACTGATAC AAAAAACCGT AGGCGAATAA
```

This corresponds to the amino acid sequence <SEQ ID 356; ORF97>:

```
  1  MKHILPLIAA SALCISTASA HPASEPSTQN ETAMITHTLI SKYSFGXXXX
 51  XXXXAIKSKG MDIFAVIDHQ EAARRNGLTM QPAKVIVFGT PKAGTPLMVK
101  DPAFALQLPL RVLVTETDGK VRAAYTDTRA LIAGSRIGFD EVANTLANAE
151  KLIQKTVGE*
```

Further work revealed the complete nucleotide sequence <SEQ ID 357>:

```
  1  ATGAAACACA TACTCCCCCT GATTGCCGCA TCCGCACTCT GCATTTCAAC
 51  CGCTTCGGCA CATCCTGCCA GCGAACCGTC CACCCAAAAC GAAACCGCTA
101  TGACCACGCA TACCCTCACC TCAAAATACA GTTTTGACGA AACCGTCAGC
151  CGCCTTGAAA CCGCCATAAA AAGCAAAGGG ATGGACATTT TTGCCGTCAT
201  CGACCATCAG GAAGCCGCCC GCCGAAACGG CTTAACGATG CAGCCGGCAA
251  AAGTCATCGT CTTCGGCACG CCCAAAGCCG GCACGCCGCT GATGGTCAAA
301  GACCCCGCCT TCGCCCTGCA ACTGCCCCTA CGCGTCCTCG TTACCGAAAC
351  GGACGGCAAA GTACGCGCCG CCTATACCGA TACGCGCGCC CTCATCGCCG
401  GCAGCCGCAT CGGTTTCGAC GAAGTGGCAA ACACTTTGGC AAACGCCGAA
451  AAACTGATAC AAAAAACCGT AGGCGAATAA
```

This corresponds to the amino acid sequence <SEQ ID 358; ORF97-1>:

```
  1  MKHILPLIAA SALCISTASA HPASEPSTQN ETAMTTHTLT SKYSFDETVS
 51  RLETAIKSKG MDIFAVIDHQ EAARRNGLTM QPAKVIVFGT PKAGTPLMVK
101  DPAFALQLPL RVLVTETDGK VRAAYTDTRA LIAGSRIGFD EVANTLANAE
151  KLIQKTVGE*
```

Computer analysis of this amino acid sequence gave the following results:

Homology with a predicted ORF from *N.meningitidis* (strain A)

ORF97 shows 88.7% identity over a 159aa overlap with an ORF (ORF97a) from strain A of *N. meningitidis:*

```
                        10        20        30        40        50        60
orf97.pep     MKHILPLIAASALCISTASAHPASEPSTQNETAMITHTLISKYSFGXXXXXXXXAIKSKG
              | |||||  |||||||||| ||||||:||||||| |||| |||||  :       :||||||
orf97a        MXHILPLXXASALCISTASXHPASEPQTQNETAMTTHTLTSKYSFDETVSRLETAIKSKG
                        10        20        30        40        50        60

                        70        80        90       100       110       120
orf97.pep     MDIFAVIDHQEAARRNGLTMQPAKVIVFGTPKAGTPLMVKDPAFALQLPLRVLVTETDGK
              |||||||||||||||||||||||||||||||||||||||||||||||||||| |||||||
orf97a        MDIFAVIDHQEAARRNGLTMQPAKVIVFGTPKAGTPLMVKDPAFALQLPLRVXVTETDGK
                        70        80        90       100       110       120

                       130       140       150       160
orf97.pep     VRAAYTDTRALIAGSRIGFDEVANTLANAEKLIQKTVGEX
              |||||||||||||||||||||||||||||||||||:|||
orf97a        VRAAYTDTRALIAGSRIGFDEVANTLANAEKLIQKTIGEX
                       130       140       150       160
```

The complete length ORF97a nucleotide sequence <SEQ ID 359> is:

```
  1  ATGANACACA TACTCCCCCT GANTGNCGCA TCCGCACTCT GCATTTCAAC
 51  CGCTTCGGNN CATCCTGCCA GCGAACCGCA AACCCAAAAC GAAACCGCTA
101  TGACCACGCA TACCCTCACC TCAAAATACA GTTTTGACGA AACCGTCAGC
151  CGCCTTGAAA CCGCCATAAA AAGCAAAGGG ATGGACATTT TTGCCGTCAT
201  CGACCATCAG GAAGCCGCCC GCCGAAACGG CTTAACGATG CAGCCGGCAA
251  AAGTCATCGT CTTCGGCACG CCCAAAGCCG GTACGCCGCT GATGGTCAAA
301  GACCCCGCCT TCGCCCTGCA ACTGCCCCTG CGCGTCNTCG TTACCGAAAC
351  GGACGGCAAA GTACGCGCCG CCTATACCGA TACGCGCGCC CTCATCGCCG
401  GCAGCCGCAT CGGTTTCGAC GAAGTGGCAA ACACTTTGGC AAACGCCGAA
451  AAACTGATAC AAAAAACCAT AGGCGAATAA
```

This encodes a protein having amino acid sequence <SEQ ID 360>:

```
  1  MXHILPLXXA SALCISTASX HPASEPQTQN ETAMTTHTLT SKYSFDETVS
 51  RLETAIKSKG MDIFAVIDHQ EAARRNGLTM QPAKVIVFGT PKAGTPLMVK
101  DPAFALQLPL RVXVTETDGK VRAAYTDTRA LIAGSRIGFD EVANTLANAE
151  KLIQKTIGE*
```

ORF97a and ORF97-1 show 95.6% identity in 159 aa overlap:

```
                     10        20        30        40        50        60
orf97a.pep   MXHILPLXXASALCISTASXHPASEPQTQNETAMTTHTLTSKYSFDETVSRLETAIKSKG
             | |||||  ||||||||| ||||||:||||||||||||||||||||||||||||||||||
orf97-1      MKHILPLIAASALCISTASAHPASEPSTQNETAMTTHTLTSKYSFDETVSRLETAIKSKG
                     10        20        30        40        50        60

                     70        80        90       100       110       120
orf97a.pep   MDIFAVIDHQEAARRNGLTMQPAKVIVFGTPKAGTPLMVKDPAFALQLPLRVXVTETDGK
             |||||||||||||||||||||||||||||||||||||||||||||||||||| |||||||
orf97-1      MDIFAVIDHQEAARRNGLTMQPAKVIVFGTPKAGTPLMVKDPAFALQLPLRVLVTETDGK
                     70        80        90       100       110       120

                    130       140       150       160
orf97a.pep   VRAAYTDTRALIAGSRIGFDEVANTLANAEKLIQKTIGEX
             |||||||||||||||||||||||||||||||||||:|||
orf97-1      VRAAYTDTRALIAGSRIGFDEVANTLANAEKLIQKTVGEX
                    130       140       150       160
```

Homology with a predicted ORF from *N.gonorrhoeae*

ORF97 shows 88.1% identity over a 159aa overlap with a predicted ORF (ORF97.ng) from *N. gonorrhoeae:*

```
orf97.pep    MKHILPLIAASALCISTASAHPASEPSTQNETAMITHTLISKYSFGXXXXXXXXAIKSKG   60
             |||||| |||||:||||||||||::| ||||||| |||| |||||  :      :||||||
orf97ng      MKHILPPIAASAFCISTASAHPAGKPPTQNETAMTTHTLTSKYSFDETVSRLETAIKSKG   60

orf97.pep    MDIFAVIDHQEAARRNGLTMQPAKVIVFGTPKAGTPLMVKDPAFALQLPLRVLVTETDGK  120
             ||||||||||||||||||||||||||||||||||||||||||||||||||||||||||||
orf97ng      MDIFAVIDHQEAARRNGLTMQPAKVIVFGTPKAGTPLMVKDPAFALQLPLRVLVTETDGK  120

orf97.pep    VRAAYTDTRALIAGSRIGFDEVANTLANAEKLIQKTVGE  159
             ||:|||||||||:||||:|||||||||||||||||||||
orf97ng      VRTAYTDTRALIVGSRISFDEVANTLANAEKLIQKTVGE  159
```

The complete length ORF97ng nucleotide sequence <SEQ ID 361> is predicted to encode a protein having amino acid sequence <SEQ ID 362>:

```
  1  MKHILPPIAA SAFCISTASA HPAGKPPTQN ETAMTTHTLT SKYSFDETVS
 51  RLETAIKSKG MDIFAVIDHQ EAARRNGLTM QPAKVIVFGT PKAGTPLMVK
101  DPAFALQLPL RVLVTETDGK VRTAYTDTRA LIVGSRISFD EVANTLANAE
151  KLIQKTVGE*
```

Further work revealed the complete nucleotide sequence <SEQ ID 363>:

```
  1  ATGAAACACA TACTCCCcct gatcgccgca TccgcactCT GCATTTCAAC
 51  CGCTTCGGCA CACCCTGCCG GCAAACCGCC CACCCAAAAC GAAACCGCTA
101  TGACCACGCA CACCCTCACC TCGAAATACA GTTTTGACGA AACCGTCAGC
151  CGCCTTGAAA CCGCCATAAA AAGCAAAGGG ATGGACATTT TTGCCGTCAT
201  CGACCATCAG GAAGCGGCAC GCCGAAACGG CCTGACCATG CAGCCGGCAA
251  AAGTCATCGT CTTCGGCACG CCCAAGGCCG GTACGCCgct GATGGTCAAA
301  GACCCCGCCT TCGCCCTGCA ACTGCCCCTG CGCGTCCTCG TTACCGAAAC
351  GGACGGCAAA GTACGCACCG CCTATACCGA TACGCGCGCC CTCATCGTCG
401  GCAGCCGCAT CAGTTTCGAC GAAGTGGCAA ACACTTTGGC AAACGCCGAA
451  AAACTGATAC AAAAAACCGT AGGCGAATAA
```

This corresponds to the amino acid sequence <SEQ ID 364; ORF97ng-1>:

```
  1  MKHILPLIAA SALCISTASA HPAGKPPTQN ETAMTTHTLT SKYSFDETVS
 51  RLETAIKSKG MDIFAVIDHQ EAARRNGLTM QPAKVIVFGT PKAGTPLMVK
101  DPAFALQLPL RVLVTETDGK VRTAYTDTRA LIVGSRISFD EVANTLANAE
151  KLIQKTVGE*
```

ORF97ng-1 and ORF97-1 show 96.2% identity in 159 aa overlap:

```
                       10        20        30        40        50        60
orf97-1.pep    MKHILPLIAASALCISTASAHPASEPSTQNETAMTTHTLTSKYSFDETVSRLETAIKSKG
               |||||||||||||||||||||||::| ||||||||||||||||||||||||||||||||
orf97ng-1      MKHILPLIAASALCISTASAHPAGKPPTQNETAMTTHTLTSKYSFDETVSRLETAIKSKG
                       10        20        30        40        50        60

                       70        80        90       100       110       120
orf97-1.pep    MDIFAVIDHQEAARRNGLTMQPAKVIVFGTPKAGTPLMVKDPAFALQLPLRVLVTETDGK
               ||||||||||||||||||||||||||||||||||||||||||||||||||||||||||||
orf97ng-1      MDIFAVIDHQEAARRNGLTMQPAKVIVFGTPKAGTPLMVKDPAFALQLPLRVLVTETDGK
                       70        80        90       100       110       120

                      130       140       150       160
orf97-1.pep    VRAAYTDTRALIAGSRIGFDEVANTLANAEKLIQKTVGEX
               ||:|||||||||:||||:|||||||||||||||||||||
orf97ng-1      VRTAYTDTRALIVGSRISFDEVANTLANAEKLIQKTVGEX
                      130       140       150       160
```

Based on this analysis, including the presence of a putative leader sequence in the gonococcal protein, it was predicted that the proteins from *N.meningitidis* and *N.gonorrhoeae,* and their epitopes, could be useful antigens for vaccines or diagnostics, or for raising antibodies.

ORF97-1 (15.3kDa) was cloned in pET and pGex vectors and expressed in *E.coli,* as described above. The products of protein expression and purification were analyzed by SDS-PAGE. Figures 12A & 12B show, repsectively, the results of affinity purification of the GST-fusion and His-fusion proteins. Purified GST-fusion protein was used to immunise mice, whose sera were used for Western Blot (Figure 12C), ELISA (positive result), and FACS analysis (Figure 12D). These experiments confirm that ORF97-1 is a surface-exposed protein, and that it is a useful immunogen.

Figure 12E shows plots of hydrophilicity, antigenic index, and AMPHI regions for ORF97-1.

**Example 43**

The following DNA, believed to be complete, sequence was identified in *N. meningitidis* <SEQ ID 365>:

```
  1  ATGGCTTTTA TTACGCGCTT ATTCAAAAGC AGTAAATGGC TGATTGTGCC
 51  GCTGATGCTC CCCGCCTTTC AGAATGTGGC GGCGGAGGGG ATAGATGTGA
101  GCCGTGCCGA AGCGAGGATA ACCGACGGCG GGCAGCTTTC CATCAGCAGC
151  CGCTTCCAAA CCGAGCTGCC CGACCAGCTC CAACAGGCGT TGCGCCGGGg
201  CGTGCCGCTC AACTTTACCT TAAGCTGGCA GCTTTCCGCC CCGATAATCG
251  CTTCTTATCG GTTTAAATTG GGGCAACTGA TTGGCGATGA CGACaATATT
301  GACTACAAAC TGAGTTTCCA TCCGCTGACc AaACGCTACC GCGTTACCgT
351  CGgCGCGTTT TCGACAGACT ACGACACCTT GGATGCGGCA TTGCGCGCGA
401  CCGGCGCGGT TGCCAACTGG AAAGTCCTGA ACAAAGGCGC GCTGTCCGGT
451  GCGGAAGCAG GGGAAACCAA GGCGGAAATC CGCCTGACGC TGTCCACTTC
501  AAAACTGCCC AAGCCTTTTC AAATCAATGC ATTGACTTCT CAAAACTGGC
551  ATTTGGATTC GGGTTGGAAA CCTCTAAACA TCATCGGGAA CAAATAA
```

This corresponds to the amino acid sequence <SEQ ID 366; ORF106>:

```
  1  MAFITRLFKS SKWLIVPLML PAFQNVAAEG IDVSRAEARI TDGGQLSISS
 51  RFQTELPDQL QQALRRGVPL NFTLSWQLSA PIIASYRFKL GQLIGDDDNI
101  DYKLSFHPLT KRYRVTVGAF STDYDTLDAA LRATGAVANW KVLNKGALSG
151  AEAGETKAEI RLTLSTSKLP KPFQINALTS QNWHLDSGWK PLNIIGNK*
```

Further work revealed the following DNA sequence <SEQ ID 367>:

```
  1  ATGGCTTTTA TTACGCGCTT ATTCAAAAGC AGTAAATGGC TGATTGTGCC
 51  GCTGATGCTC CCCGCCTTTC AGAATGTGGC GGCGGAGGGG ATAGATGTGA
101  GCCGTGCCGA AGCGAGGATA ACCGACGGCG GGCAGCTTTC CATCAGCAGC
151  CGCTTCCAAA CCGAGCTGCC CGACCAGCTC CAACAGGCGT TGCGCCGGGG
201  CGTGCCGCTC AACTTTACCT TAAGCTGGCA GCTTTCCGCC CCGATAATCG
251  CTTCTTATCG GTTTAAATTG GGGCAACTGA TTGGCGATGA CGACAATATT
301  GACTACAAAC TGAGTTTCCA TCCGCTGACC AACCGCTACC GCGTTACCGT
351  CGGCGCGTTT TCGACAGACT ACGACACCTT GGATGCGGCA TTGCGCGCGA
401  CCGGCGCGGT TGCCAACTGG AAAGTCCTGA ACAAAGGCGC GCTGTCCGGT
451  GCGGAAGCAG GGGAAACCAA GGCGGAAATC CGCCTGACGC TGTCCACTTC
501  AAAACTGCCC AAGCCTTTTC AAATCAATGC ATTGACTTCT CAAAACTGGC
551  ATTTGGATTC GGGTTGGAAA CCTCTAAACA TCATCGGGAA CAAATAA
```

This corresponds to the amino acid sequence <SEQ ID 368; ORF106-1>:

```
  1  MAFITRLFKS SKWLIVPLML PAFQNVAAEG IDVSRAEARI TDGGQLSISS
 51  RFQTELPDQL QQALRRGVPL NFTLSWQLSA PIIASYRFKL GQLIGDDDNI
101  DYKLSFHPLT NRYRVTVGAF STDYDTLDAA LRATGAVANW KVLNKGALSG
151  AEAGETKAEI RLTLSTSKLP KPFQINALTS QNWHLDSGWK PLNIIGNK*
```

Computer analysis of this amino acid sequence gave the following results:

Homology with a predicted ORF from *N. meningitidis* (strain A)

ORF106 shows 87.4% identity over a 199aa overlap with an ORF (ORF106a) from strain A of *N. meningitidis:*

```
                        10        20        30        40        50        59
orf106.pep    MAFITRLFKSSK-WLIVPLMLPAFQNVAAEGIDVSRAEARITDGGQLSISSRFQTELPDQ
              |||||||||| | ||::  || :: ::|||||||||||||:||||||  ||||||||||
orf106a       MAFITRLFKSIKQWLVLLPMLSVLPDAAAEGIDVSRAEARIXDGGQLSXXSRFQTELPDQ
                      10        20        30        40        50        60

             60        70        80        90       100       110       119
orf106.pep    LQQALRRGVPLNFTLSWQLSAPIIASYRFKLGQLIGDDDNIDYKLSFHPLTKRYRVTVGA
              || |  ||| || || |||||||||||| ||||||||| |||||||||||:|||||||
orf106a       LQXAXXRGVXLNXTLXWQLSAPIIASYRFXLGQLIGDDDXIDYKLSFHPLTNRYRVTVGA
                      70        80        90       100       110       120

            120       130       140       150       160       170       179
orf106.pep    FSTDYDTLDAALRATGAVANWKVLNKGALSGAEAGETKAEIRLTLSTSKLPKPFQINALT
              ||| ||||||||||||||||||||||||||||||||||||||||||||||||||||||||
orf106a       FSTXYDTLDAALRATGAVANWKVLNKGALSGAEAGETKAEIRLTLSTSKLPKPFQINALT
                     130       140       150       160       170       180

            180       190       199
orf106.pep    SQNWHLDSGWKPLNIIGNKX
              ||||||||||||||||||||
orf106a       SQNWHLDSGWKPLNIIGNKX
                     190       200
```

Due to the K→N substitution at residue 111, the homology between ORF106a and ORF106-1 is 87.9% over the same 199 aa overlap.

The complete length ORF106a nucleotide sequence <SEQ ID 369> is:

```
  1  ATGGCTTTTA TTACGCGCTT ATTCAAAAGC ATTAAACAAT GGCTTGTGCT
 51  GCTGCCGATG CTTTCCGTTT TGCCGGACGC GGCGGCGGAG GGGATAGATG
101  TGAGCCGCGC CGAAGCGAGG ATAANCGACG GCGGGCAGCT TTCCATNAGN
151  AGCCGCTTCC AAACCGAGCT GCCCGACCAG CTCCAANNNG CGNNGNGCCG
201  GGGCGTGNCG CTCAACTNTA CCTTAAGNTG GCAGCTTTCC GCCCCGATAA
251  TCGCTTCTTA TCGGTTTNAA TTGGGGCAAC TGATTGGCGA TGACGACNAT
301  ATTGACTACA AACTGAGTTT CCATCCGCTG ACCAACCGCT ACCGCGTTAC
351  CGTCGGCGCG TTTTCGACAG ANTACGACAC CTTGGATGCG GCATTGCGCG
401  CGACCGGCGC GGTTGCCAAC TGGAAAGTCC TGAACAAAGG CGCGCTGTCC
451  GGTGCGGAAG CAGGGGAAAC CAAGGCGGAA ATCCGCCTGA CGCTGTCCAC
501  TTCAAAACTG CCCAAGCCTT TTCAAATCAA TGCATTGACT TCTCAAAACT
551  GGCATTTGGA TTCGGGTTGG AAACCTCTAA ACATCATCGG GAACAAATAA
```

This encodes a protein having amino acid sequence <SEQ ID 370>:

```
  1  MAFITRLFKS IKQWLVLLPM LSVLPDAAAE GIDVSRAEAR IXDGGQLSXX
 51  SRFQTELPDQ LQXAXXRGVX LNXTLXWQLS APIIASYRFX LGQLIGDDDX
101  IDYKLSFHPL TNRYRVTVGA FSTXYDTLDA ALRATGAVAN WKVLNKGALS
151  GAEAGETKAE IRLTLSTSKL PKPFQINALT SQNWHLDSGW KPLNIIGNK*
```

Homology with a predicted ORF from *N. gonorrhoeae*

ORF106 shows 90.5% identity over a 199aa overlap with a predicted ORF (ORF I 06.ng) from *N. gonorrhoeae:*

```
orf106.pep   MAFITRLFKSSK-WLIVPLMLPAFQNVAAEGIDVSRAEARITDGGQLSISSRFQTELPDQ   59
             |||||||||| | ||::  :| :: ::||||| ::||||||||||:|||||||||||||
orf106ng     MAFITRLFKSIKQWLVLLPILSVLPDAAAEGIAATRAEARITDGGRLSISSRFQTELPDQ   60

orf106.pep   LQQALRRGVPLNFTLSWQLSAPIIASYRFKLGQLIGDDDNIDYKLSFHPLTKRYRVTVGA  119
             |||||||||||||||||||||| ||||||||||||||||||||||||||||:||||||||
orf106ng     LQQALRRGVPLNFTLSWQLSAPTIASYRFKLGQLIGDDDNIDYKLSFHPLTNRYRVTVGA  120

orf106.pep   FSTDYDTLDAALRATGAVANWKVLNKGALSGAEAGETKAEIRLTLSTSKLPKPFQINALT  179
             ||||||||||||||||||||||||||||||||||||||||||||||||||||||||||||
orf106ng     FSTDYDTLDAALRATGAVANWKVLNKGALSGAEAGETKAEIRLTLSTSKLPKPFQINALT  180

orf106.pep   SQNWHLDSGWKPLNIIGNK  198
             |||||||||||||||||||
orf106ng     SQNWHLDSGWKPLNIIGNK  199
```

Due to the K→N substitution at residue 111, the homology between ORF106ng and ORF106-1 is 91.0% over the same 199 aa overlap.

The complete length ORF106ng nucleotide sequence <SEQ ID 371> is:

```
  1  ATGGCTTTTA TTACGCGCTT ATTCAAAAGC ATTAAACAAT GGCTTGTGCT
 51  GTTGCCGATA CTCTCCGTTT TGCCGGACGC GGCGGCGGAG GGCATTGCCG
101  CGACCCGCGC CGAAGCGAGG ATAACCGACG GCGGGCGGCT TTCCATCAGC
151  AGCCGCTTCC AAACCGAGCT GCCCGACCAG CTCCAACAGG CGTTGCGCCG
201  GGGCGTACCG CTCAACTTTA CCTTAAGCTG GCAGCTTTCC GCCCCGACAA
251  TCGCTTCTTA TCGGTTTAAA TTGGGGCAAC TGATTGGCGA TGACGACAAT
301  ATTGACTACA AACTAAGTTT CCATCCGCTG ACCAACCGCT ACCGCGTTAC
351  CGTCGGCGCA TTTTCCACCG ATTACGACAC TTTGGATGCG GCATTGCGCG
401  CGACCGGCGC GGTTGCCAAC TGGAAAGTCC TGAACAAAGG CGCGTTGTCC
451  GGTGCGGAAG CAGGGGAAAC CAAGGCGGAA ATCCGCCTGA CGCTGTCCAC
501  TTCAAAACTG CCCAAGCCTT TCCAAATCAA CGCATTGACT TCTCAAAACT
551  GGCATTTGGA TTCGGGTTGG AAACCTCTAA ACATCATCGG GAACAAATAA
```

This encodes a protein having amino acid sequence <SEQ ID 372>:

```
  1  MAFITRLFKS IKQWLVLLPI LSVLPDAAAE GIAATRAEAR ITDGGRLSIS
 51  SRFQTELPDQ LQQALRRGVP LNFTLSWQLS APTIASYRFK LGQLIGDDDN
101  IDYKLSFHPL TNRYRVTVGA FSTDYDTLDA ALRATGAVAN WKVLNKGALS
151  GAEAGETKAE IRLTLSTSKL PKPFQINALT SQNWHLDSGW KPLNIIGNK*
```

Based on this analysis, including the presence of a putative leader sequence in the gonococcal protein, it was predicted that the proteins from *N.meningitidis* and *N.gonorrhoeae,* and their epitopes, could be useful antigens for vaccines or diagnostics, or for raising antibodies.

ORF106-1 (18kDa) was cloned in pET and pGex vectors and expressed in *E.coli,* as described above. The products of protein expression and purification were analyzed by SDS-PAGE. Figure 13A shows the results of affinity purification of the His-fusion protein, and Figure 13B shows the results of expression of the GST-fusion in *E.coli.* Purified His-fusion protein was used to immunise mice, whose sera were used for FACS analysis (Figure 13C) These experiments confirm that ORF106-1 is a surface-exposed protein, and that it is a useful immunogen.

**Example 44**

The following DNA sequence, believed to be complete, was identified in *N.meningitidis* <SEQ ID 373>:

```
   1 ATGGACACAA AAGAAATCCT CGG.TACGCG GcAGGcTCGA TCGGCAGCGC
  51 GGTTTTAGCC GTCATCATCc TGCCGCTGCT GTCGTGGTAT TTCCCCGCCG
 101 ACGACATCGG GCGCATCGTG CTGATGCAGA CGGCGGCGGG GCTgACGGTG
 151 TCGGTGTTGT GCCTCGGGCT GGATCAGGCA TACGTCCGCG AATACTATGC
 201 CACCGCCGAC AAAGACAcCT TGTTCAAAAC CCTGTTCCTG CCGCCGCTGC
 251 TGTCTGCCGC CGCGATAGCC GCCCTGCTGC TTTCCCGCCC GTCCCTGCCG
 301 TCTGAAATCC TGTTTTCACT CGACGATGCC gCCGCCGGCa TCGGGCTGGT
 351 GCTGTTTGAA CtGAGCTTCC TGCCCATCCG cTTTCTCTTA CTGGTTTTGC
 401 GTATGGAAGG ACGCGCCcTT GCCTTTTCGT CCGCGCAACT CGTGCcCAAG
 451 CTCGCCATCC TGCTGCTG.T GCCGCTGACG GTCGGGCTGC TGCACTTTCC
 501 AGCGAACACC GCCGTCCTGA CCGCCGTTTA CGCGCTGGCA AACCTTGCCG
 551 CCGCCGCCTT TTTGCTGTTT CAAAACCGAT GCCGTCTGAA GGCCGTCCGG
 601 CACGCACCGT TTTCGCCCGC CGTCCTGCAC CGGGGG.TGC GCTACGGCAT
 651 ACCGATCGCA CTGAGCAGCA TCGCCTATTG GGGGCTGGCA TCCGCCGACC
 701 GTTTGTTCCT GAAAAAATAT GCCGGCCTGG AACAGCTCGG CGTTTATTCG
 751 ATGGGTATTT CGTTCGGCGG GGCGGCATTA TTGTTCCAAA GCATCTTTTC
 801 AACGGTCTGG ACACCGTATA TTTTCCGCGC AATCGAAGAA AACGCCCCGC
 851 CCGCTCGCCT CTCGGCAACG GCAGAATCCG CCGCCGCCCT GCTTGCCTCC
 901 GCCCTCTGC. TGACCGGCAT TTTCTCGCCC CTTGCCTCCC TCCTGCTGCC
 951 GGAAAACTAC GCCGCCGTCC GGTTTATCGT CGTATCGTGT ATG.TGCCGC
1001 CGCTGTTTTG CACGCTGGCG GAAATCAGCG GCATCGGTTT GAACGTCGTT
1051 CGCAAAACGC GCCCGATCGC GCTCGCCACC TTGGGCGCGC TGGCGGCAAA
1101 CCTGCTGCTG CTGGGGCTTG ACCGTGCCGT ACCGGCGAGG CCGCC.GGCG
1151 CGGCGGTTGC CTGTGCCGCC TCATTCTGGC TGTTTTTTGC CTTCAAGACC
1201 GAAAGCTCyT GCCGCCTGTG GCAGCCGCTC AAACGCCTGC CGCTTTATCT
1251 GCACACATTG TTCTGCCTGA CCTCCTCGGC GGCCTACACC TGCTTCGGCA
1301 CGCCGGCAAA CTATCCCCTG TTTGCCGGCG TATGGGCGGC ATATCTGGCA
1351 GGCTGCATCC TGCGCCACCG GAAAGATTTG CACAAACTGT TTCATTATTT
1401 GAAAAAACAA GGTTTCCCAT TATGA
```

This corresponds to the amino acid sequence <SEQ ID 374; ORF10>:

```
  1 MDTKEILXYA AGSIGSAVLA VIILPLLSWY FPADDIGRIV LMQTAAGLTV
 51 SVLCLGLDQA YVREYYATAD KDTLFKTLFL PPLLSAAAIA ALLLSRPSLP
101 SEILFSLDDA AAGIGLVLFE LSFLPIRFLL LVLRMEGRAL AFSSAQLVPK
151 LAILLLXPLT VGLLHFPANT AVLTAVYALA NLAAAAFLLF QNRCRLKAVR
201 HAPFSPAVLH RGXRYGIPIA LSSIAYWGLA SADRLFLKKY AGLEQLGVYS
251 MGISFGGAAL LFQSIFSTVW TPYIFRAIEE NAPPARLSAT AESAAALLAS
301 ALCXTGIFSP LASLLLPENY AAVRFIVVSC MXPPLFCTLA EISGIGLNVV
351 RKTRPIALAT LGALAANLLL LGLDRAVPAR PXGAAVACAA SFWLFFAFKT
401 ESSCRLWQPL KRLPLYLHTL FCLTSSAAYT CFGTPANYPL FAGVWAAYLA
451 GCILRHRKDL HKLFHYLKKQ GFPL*
```

Further sequence analysis revealed the complete DNA sequence<SEQ ID 375> to be:

```
   1  ATGGACACAA AAGAAATCCT CGGCTACGCG GCAGGCTCGA TCGGCAGCGC
  51  GGTTTTAGCC GTCATCATCC TGCCGCTGCT GTCGTGGTAT TTCCCCGCCG
 101  ACGACATCGG GCGCATCGTG CTGATGCAGA CGGCGGCGGG GCTGACGGTG
 151  TCGGTGTTGT GCCTCGGGCT GGATCAGGCA TACGTCCGCG AATACTATGC
 201  CACCGCCGAC AAAGACACCT TGTTCAAAAC CCTGTTCCTG CCGCCGCTGC
 251  TGTCTGCCGC CGCGATAGCC GCCCTGCTGC TTTCCCGCCC GTCCCTGCCG
 301  TCTGAAATCC TGTTTTCACT CGACGATGCC GCCGCCGGCA TCGGGCTGGT
 351  GCTGTTTGAA CTGAGCTTCC TGCCCATCCG CTTTCTCTTA CTGGTTTTGC
 401  GTATGGAAGG ACGCGCCCTT GCCTTTTCGT CCGCGCAACT CGTGCCCAAG
 451  CTCGCCATCC TGCTGCTGCT GCCGCTGACG GTCGGGCTGC TGCACTTTCC
 501  AGCGAACACC GCCGTCCTGA CCGCCGTTTA CGCGCTGGCA AACCTTGCCG
 551  CCGCCGCCTT TTTGCTGTTT CAAAACCGAT GCCGTCTGAA GGCCGTCCGG
 601  CACGCACCGT TTTCGCCCGC CGTCCTGCAC CGGGGGCTGC GCTACGGCAT
 651  ACCGATCGCA CTGAGCAGCA TCGCCTATTG GGGGCTGGCA TCCGCCGACC
 701  GTTTGTTCCT GAAAAAATAT GCCGGCCTGG AACAGCTCGG CGTTTATTCG
 751  ATGGGTATTT CGTTCGGCGG GGCGGCATTA TTGTTCCAAA GCATCTTTTC
 801  AACGGTCTGG ACACCGTATA TTTTCCGCGC AATCGAAGAA AACGCCCCGC
 851  CCGCCCGCCT CTCGGCAACG GCAGAATCCG CCGCCGCCCT GCTTGCCTCC
 901  GCCCTCTGCC TGACCGGCAT TTTCTCGCCC CTTGCCTCCC TCCTGCTGCC
 951  GGAAAACTAC GCCGCCGTCC GGTTTATCGT CGTATCGTGT ATGCTGCCGC
1001  CGCTGTTTTG CACGCTGGCG GAAATCAGCG GCATCGGTTT GAACGTCGTC
1051  CGCAAAACGC GCCCGATCGC GCTCGCCACC TTGGGCGCGC TGGCGGCAAA
1101  CCTGCTGCTG CTGGGGCTTG CCGTGCCGTC CGGCGGCGCG CGCGGCGCGG
1151  CGGTTGCCTG TGCCGCCTCA TTCTGGCTGT TTTTTGCCTT CAAGACCGAA
1201  AGCTCCTGCC GCCTGTGGCA GCCGCTCAAA CGCCTGCCGC TTTATCTGCA
1251  CACATTGTTC TGCCTGACCT CCTCGGCGGC CTACACCTGC TTCGGCACGC
1301  CGGCAAACTA TCCCCTGTTT GCCGGCGTAT GGGCGGCATA TCTGGCAGGC

1351  TGCATCCTGC GCCACCGGAA AGATTTGCAC AAACTGTTTC ATTATTTGAA
1401  AAAACAAGGT TTCCCATTAT GA
```

This corresponds to the amino acid sequence <SEQ ID 376; ORF10-1>:

```
  1  MDTKEILGYA AGSIGSAVLA VIILPLLSWY FPADDIGRIV LMQTAAGLTV
 51  SVLCLGLDQA YVREYYATAD KDTLFKTLFL PPLLSAAAIA ALLLSRPSLP
101  SEILFSLDDA AAGIGLVLFE LSFLPIRFLL LVLRMEGRAL AFSSAQLVPK
151  LAILLLLPLT VGLLHFPANT AVLTAVYALA NLAAAAFLLF QNRCRLKAVR
201  HAPFSPAVLH RGLRYGIPIA LSSIAYWGLA SADRLFLKKY AGLEQLGVYS
251  MGISFGGAAL LFQSIFSTVW TPYIFRAIEE NAPPARLSAT AESAAALLAS
301  ALCLTGIFSP LASLLLPENY AAVRFIVVSC MLPPLFCTLA EISGIGLNVV
351  RKTRPIALAT LGALAANLLL LGLAVPSGGA RGAAVACAAS FWLFFAFKTE
401  SSCRLWQPLK RLPLYLHTLF CLTSSAAYTC FGTPANYPLF AGVWAAYLAG
451  CILRHRKDLH KLFHYLKKQG FPL*
```

Computer analysis of this amino acid sequence gave the following results:

Prediction

ORF10-1 is predicted to be the precursor of an integral membrane protein, since it comprises several (12-13) potential transmembrane segments, and a probable cleavable signal peptide

Homology with EpsM from *Streptococcus thermophilus* (accession number U40830).

ORF10 shows homology with the epsM gene of *S. thermophilus,* which encodes a protein of a size similar to ORF10 and is involved in expolysaccharide synthesis. Other homologies are with prokaryotic membrane proteins:

```
Identities = (25%)

Query:   213 LRYGIPLALSSLAYWGLASADRLFLKKYAGLEQLGVYSMGISFGGAALLLQSIFSTVW 270
             L Y +PL   SS+ +W L ++ R F+  + G    G+ ++            +  +IF+  W
Sbjct:   210 LYYALPLIPSSILWWLLNASSRYFVLFFLGAGANGLLAVATKIPSIISIFNTIFTQAW 267


Identities = 15/57 (26%), Positives = 31/57 (54%)

Query:     7 LGYAAGSIGSAVLAVIILPLLSWYFPADDIGRIVLMQTAAGLTVSVLCLGLDQAYVR 63
             L +   G++GS +L   +++PL ++       + G    L QT A L + ++ + +   A +R
Sbjct:    12 LVFTIGNLGSKLLVFLLVPLYTYAMTPQEYGMADLYQTTANLLLPLITMNVFDATLR 68

 Identities = 16/96 (16%), Positives = 36/96 (37%)

Query: 307 IFSPLASLLLPENYAAVRFTVVSCMLPPLFYTLTEISGIGLNVVRKTRPIXXXXXXXXXX 366
           +   P+    ++   +YA++     V     ML   LF + ++  G         ++T+ +
Sbjct: 305 VLKPIVEKVVSSDYASSWQYVPFFMLSMLFSSFSDFFGTNYIAAKQTKGVFMTSIYGTIV 364
```

Homology with a predicted ORF from *N.meningitidis (*strain A)

ORF10 shows 95.4% identity over a 475aa overlap with an ORF (ORF10a) from strain A of *N. meningitidis:*

```
                        10        20        30        40        50        60
orf10.pep   MDTKEILXYAAGSIGSAVLAVIILPLLSWYFPADDIGRIVLMQTAAGLTVSVLCLGLDQA
            ||||||| ||||||||||||||||||||||||||||||||||||||||||||||||||||
orf10a      MDTKEILGYAAGSIGSAVLAVIILPLLSWYFPADDIGRIVLMQTAAGLTVSVLCLGLDQA
                        10        20        30        40        50        60

                        70        80        90       100       110       120
orf10.pep   YVREYYATADKDTLFKTLFLPPLLSAAAIAALLLSRPSLPSEILFSLDDAAAGIGLVLFE
            |||||||:||||||||||||||||||||||||||||||||||||||||||||||||||||
orf10a      YVREYYAAADKDTLFKTLFLPPLLSAAAIAALLLSRPSLPSEILFSLDDAAAGIGLVLFE
                        70        80        90       100       110       120
```

```
                    130       140       150       160       170       180
orf10.pep    LSFLPIRFLLLVLRMEGRALAFSSAQLVPKLAILLLXPLTVGLLHFPANTAVLTAVYALA
             |||||||||||||||||||||||||||| ||||||| |||||||||||||||||||||||
orf10a       LSFLPIRFLLLVLRMEGRALAFSSAQLVSKLAILLLLPLTVGLLHFPANTAVLTAVYALA
                    130       140       150       160       170       180

                    190       200       210       220       230       240
orf10.pep    NLAAAAFLLFQNRCRLKAVRHAPFSPAVLHRGXRYGIPIALSSIAYWGLASADRLFLKKY
             ||||||||||||||||||||:|||| |||||| |||||||||||||||||||||||||||
orf10a       NLAAAAFLLFQNRCRLKAVRRAPFSSAVLHRGLRYGIPIALSSIAYWGLASADRLFLKKY
                    190       200       210       220       230       240

                    250       260       270       280       290       300
orf10.pep    AGLEQLGVYSMGISFGGAALLFQSIFSTVWTPYIFRAIEENAPPARLSATAESAAALLAS
             |||||||||||||||||||||||||||||||||||||||| |||||||||||||||||||
orf10a       AGLEQLGVYSMGISFGGAALLFQSIFSTVWTPYIFRAIEANAPPARLSATAESAAALLAS
                    250       260       270       280       290       300

                    310       320       330       340       350       360
orf10.pep    ALCXTGIFSPLASLLLPENYAAVRFIVVSCMXPPLFCTLAEISGIGLNVVRKTRPIALAT
             ||| |||||||||||||||||||||||||||| |||||||:|||||||||||||||||||
orf10a       ALCLTGIFSPLASLLLPENYAAVRFIVVSCMLPPLFCTLVEISGIGLNVVRKTRPIALAT
                    310       320       330       340       350       360

                    370       380       390       400       410      419
orf10.pep    LGALAANLLLLGLDRAVPAR-PXGAAVACAASFWLFFAFKTESSCRLWQPLKRLPLYLHT
             ||||||||||||  |||:     ||||||||||||||:||||||||||||||||||||:||
orf10a       LGALAANLLLLGL--AVPSGGARGAAVACAASFWLFFVFKTESSCRLWQPLKRLPLYMHT
                    370       380       390       400       410

                  420       430       440       450       460       470
orf10.pep    LFCLTSSAAYTCFGTPANYPLFAGVWAAYLAGCILRHRKDLHKLFHYLKKQGFPLX
             ||||:|||||||||||||||||||||||:|||||||||||||||||||||||||||
orf10a       LFCLASSAAYTCFGTPANYPLFAGVWAVYLAGCILRHRKDLHKLFHYLKKQGFPLX
                  420       430       440       450       460       470
```

The complete length ORF10a nucleotide sequence <SEQ ID 377> is:

```
   1  ATGGACACAA AAGAAATCCT CGGCTACGCG GCAGGCTCGA TCGGCAGCGC
  51  GGTTTTAGCC GTCATCATCC TGCCGCTGCT GTCGTGGTAT TTCCCTGCCG
 101  ACGACATCGG ACGCATCGTG CTGATGCAGA CGGCGGCGGG GCTGACGGTG
 151  TCGGTGTTGT GCCTCGGGCT GGATCAGGCA TACGTCCGCG AATACTATGC
 201  CGCCGCCGAC AAAGACACTT TGTTCAAAAC CCTGTTCCTG CCGCCGCTGC
 251  TGTCTGCCGC CGCGATAGCC GCCCTGCTGC TTTCCCGCCC ATCCCTGCCG
 301  TCTGAAATCC TGTTTTCGCT CGACGATGCC GCCGCCGGCA TCGGGCTGGT
 351  GCTGTTTGAA CTGAGCTTCC TGCCCATCCG CTTTCTCTTA CTGGTTTTGC
 401  GTATGGAAGG ACGCGCCCTT GCCTTTTCGT CCGCGCAACT CGTGTCCAAG
 451  CTCGCCATCC TGCTGCTGCT GCCGCTGACG GTCGGGCTGC TGCACTTTCC
 501  GGCGAACACC GCCGTCCTGA CCGCCGTTTA CGCGCTGGCA AACCTTGCCG
 551  CCGCCGCCTT TTTGCTGTTT CAAAACCGAT GCCGTCTGAA GGCCGTCCGG
 601  CGCGCACCGT TTTCATCCGC CGTCCTGCAT CGCGGCCTGC GCTACGGCAT
 651  ACCGATCGCA CTAAGCAGCA TCGCCTATTG GGGGCTGGCA TCCGCCGACC
 701  GTTTGTTCCT GAAAAAATAT GCCGGCCTAG AACAGCTCGG CGTTTATTCG
 751  ATGGGTATTT CGTTCGGCGG AGCGGCATTA TTGTTCCAAA GCATCTTTTC
 801  AACGGTCTGG ACACCGTATA TTTTCCGCGC AATCGAAGCA AACGCCCCGC
 851  CCGCCCGCCT CTCGGCAACG GCAGAATCCG CCGCCGCCCT GCTTGCCTCC
 901  GCCCTCTGCC TGACCGGCAT TTTCTCGCCC CTCGCCTCCC TCCTGCTGCC
 951  GGAAAACTAC GCCGCCGTCC GGTTTATCGT CGTATCGTGT ATGCTGCCTC
1001  CGCTGTTTTG CACGCTGGTA GAAATCAGCG GCATCGGTTT GAACGTCGTC
1051  CGAAAAACAC GCCCGATCGC GCTCGCCACC TTGGGCGCGC TGGCGGCAAA
1101  CCTGCTGCTG CTGGGGCTTG CCGTACCGTC CGGCGGCGCG CGCGGCGCGG
1151  CGGTTGCCTG TGCCGCCTCA TTTTGGCTGT TTTTTGTTTT CAAGACCGAA
1201  AGCTCCTGCC GCCTGTGGCA GCCGCTCAAA CGCCTGCCGC TTTATATGCA
1251  CACATTGTTC TGCCTGGCCT CCTCGGCGGC CTACACCTGC TTCGGCACTC
1301  CGGCAAACTA CCCCCTGTTT GCCGGCGTAT GGGCGGTATA TCTGGCAGGC
1351  TGCATCCTGC GCCACCGGAA AGATTTGCAC AAACTGTTTC ATTATTTGAA
1401  AAAACAAGGT TTCCCATTAT GA
```

This encodes a protein having amino acid sequence <SEQ ID 378>:

```
  1  MDTKEILGYA AGSIGSAVLA VIILPLLSWY FPADDIGRIV LMQTAAGLTV
 51  SVLCLGLDQA YVREYYAAAD KDTLFKTLFL PPLLSAAAIA ALLLSRPSLP
101  SEILFSLDDA AAGIGLVLFE LSFLPIRFLL LVLRMEGRAL AFSSAQLVSK
151  LAILLLLPLT VGLLHFPANT AVLTAVYALA NLAAAAFLLF QNRCRLKAVR
201  RAPFSSAVLH RGLRYGIPIA LSSIAYWGLA SADRLFLKKY AGLEQLGVYS
251  MGISFGGAAL LFQSIFSTVW TPYIFRAIEA NAPPARLSAT AESAAALLAS
301  ALCLTGIFSP LASLLLPENY AAVRFIVVSC MLPPLFCTLV EISGIGLNVV
351  RKTRPIALAT LGALAANLLL LGLAVPSGGA RGAAVACAAS FWLFFVFKTE
401  SSCRLWQPLK RLPLYMHTLF CLASSAAYTC FGTPANYPLF AGVWAVYLAG
451  CILRHRKDLH KLFHYLKKQG FPL*
```

ORF10a and ORF10-1 show 95.4% identity in 475 aa overlap:

```
                             10        20        30        40        50        60
orf10-1.pep         MDTKEILXYAAGSIGSAVLAVIILPLLSWYFPADDIGRIVLMQTAAGLTVSVLCLGLDQA
                    ||||||| ||||||||||||||||||||||||||||||||||||||||||||||||||||
orf10a              MDTKEILGYAAGSIGSAVLAVIILPLLSWYFPADDIGRIVLMQTAAGLTVSVLCLGLDQA
                             10        20        30        40        50        60

                             70        80        90       100       110       120
orf10-1.pep         YVREYYATADKDTLFKTLFLPPLLSAAAIAALLLSRPSLPSEILFSLDDAAAGIGLVLFE
                    |||||||:||||||||||||||||||||||||||||||||||||||||||||||||||||
orf10a              YVREYYAAADKDTLFKTLFLPPLLSAAAIAALLLSRPSLPSEILFSLDDAAAGIGLVLFE
                             70        80        90       100       110       120

                            130       140       150       160       170       180
orf10-1.pep         LSFLPIRFLLLVLRMEGRALAFSSAQLVPKLAILLLXPLTVGLLHFPANTAVLTAVYALA
                    |||||||||||||||||||||||||||| ||||||| |||||||||||||||||||||||
orf10a              LSFLPIRFLLLVLRMEGRALAFSSAQLVSKLAILLLLPLTVGLLHFPANTAVLTAVYALA
                            130       140       150       160       170       180

                            190       200       210       220       230       240
orf10-1.pep         NLAAAAFLLFQNRCRLKAVRHAPFSPAVLHRGXRYGIPIALSSIAYWGLASADRLFLKKY
                    ||||||||||||||||||||:|||| |||||| |||||||||||||||||||||||||||
orf10a              NLAAAAFLLFQNRCRLKAVRRAPFSSAVLHRGLRYGIPIALSSIAYWGLASADRLFLKKY
                            190       200       210       220       230       240

                            250       260       270       280       290       300
orf10-1.pep         AGLEQLGVYSMGISFGGAALLFQSIFSTVWTPYIFRAIEENAPPARLSATAESAAALLAS
                    ||||||||||||||||||||||||||||||||||||||| ||||||||||||||||||||
orf10a              AGLEQLGVYSMGISFGGAALLFQSIFSTVWTPYIFRAIEANAPPARLSATAESAAALLAS
                            250       260       270       280       290       300

                            310       320       330       340       350       360
orf10-1.pep         ALCXTGIFSPLASLLLPENYAAVRFIVVSCMXPPLFCTLAEISGIGLNVVRKTRPIALAT
                    ||| ||||||||||||||||||||||||||| |||||||:||||||||||||||||||||
orf10a              ALCLTGIFSPLASLLLPENYAAVRFIVVSCMLPPLFCTLVEISGIGLNVVRKTRPIALAT
                            310       320       330       340       350       360

                            370       380       390       400       410      419
orf10-1.pep         LGALAANLLLLGLDRAVPAR-PXGAAVACAASFWLFFAFKTESSCRLWQPLKRLPLYLHT
                    |||||||||||||  |||:    ||||||||||||||:|||||||||||||||||||:||
orf10a              LGALAANLLLLGL--AVPSGGARGAAVACAASFWLFFVFKTESSCRLWQPLKRLPLYMHT
                            370         380       390       400       410

                  420       430       440       450       460       470
orf10-1.pep         LFCLTSSAAYTCFGTPANYPLFAGVWAAYLAGCILRHRKDLHKLFHYLKKQGFPLX
                    ||||:||||||||||||||||||||||:||||||||||||||||||||||||||||
orf10a              LFCLASSAAYTCFGTPANYPLFAGVWAVYLAGCILRHRKDLHKLFHYLKKQGFPLX
                    420       430       440       450       460       470
```

Homology with a predicted ORF from *N.gonorrhoeae*

ORF10 shows 94.1 % identity over a 475aa overlap with a predicted ORF (ORF10.ng) from *N. gonorrhoeae:*

```
orf10ng.pep  MDTKEILGYAAGSIGSAVLAVIILPLLSWYFPADDIGRIVLMQTAAGLTVSVLCLGLDQA  60
             ||||||| ||||||||||||||||||||||||||||||||||||||||||||||||||||
orf10nm      MDTKEILXYAAGSIGSAVLAVIILPLLSWYFPADDIGRIVLMQTAAGLTVSVLCLGLDQA  60

orf10ng.pep  YVREYYAAADKDTLFKTLFLPPLLFSAAIAALLLSRPSLPSEILFSLDDAAAGIGLVLFE 120
             |||||||:||||||||||||||||| :|||||||||||||||||||||||||||||||||
orf10nm      YVREYYATADKDTLFKTLFLPPLLSAAAIAALLLSRPSLPSEILFSLDDAAAGIGLVLFE 120

orf10ng.pep  LSFLPIRFLLLVLRMEGRALAFSSAQLVPKLAILLLLPLTVGLLHFPANTSVLTAVYALA 180
             |||||||||||||||||||||||||||||||||||| ||||||||||||||:|||||||||
orf10nm      LSFLPIRFLLLVLRMEGRALAFSSAQLVPKLAILLLXPLTVGLLHFPANTAVLTAVYALA 180

orf10ng.pep  NLAAAAFLLFQNRCRLKAVRRAPFSPAVLHRGLRYGIPLALSSLAYWGLASADRLFLKKY 240
             ||||||||||||||||||||:||||||||||| |||||:||||:||||||||||||||||
orf10nm      NLAAAAFLLFQNRCRLKAVRHAPFSPAVLHRGXRYGIPIALSSIAYWGLASADRLFLKKY 240

orf10ng.pep  AGLEQLGVYSMGISFGGAALLLQSIFSTVWTPYIFRAIEENATPARLSATAESAAALLAS 300
             |||||||||||||||||||||:|||||||||||||||||||| |||||||||||||||||
orf10nm      AGLEQLGVYSMGISFGGAALLFQSIFSTVWTPYIFRAIEENAPPARLSATAESAAALLAS 300

orf10ng.pep  ALCLTGIFSPLASLLLPENYAAVRFTVVSCMLPPLFYTLTEISGIGLNVVRKTRPIALAT 360
             ||| ||||||||||||||||||||| ||||| |||| || :||||||||||||||||||||
orf10nm      ALCXTGIFSPLASLLLPENYAAVRFIVVSCMXPPLFCTLAEISGIGLNVVRKTRPIALAT 360

                     370       380       390       400       410
orf10ng.pep  LGALAANLLLLGL--AVPSGGTRGAAVACAASFWLFFVFKTESSCRLWQPLKRLPLYMHT
             |||||||||||||  |||:    ||||||||||||||:|||||||||||||||||||:||
orf10nm      LGALAANLLLLGLDRAVPAR-PXGAAVACAASFWLFFAFKTESSCRLWQPLKRLPLYLHT
                     370       380       390       400       410

           420       430       440       450       460       470
orf10ng.pep  LFCLASSAAYTCFGTPANYPLFAGVWAAYLAGCILRHRKNLHKLFHYLKKQGFPLX
             ||||:||||||||||||||||||||||||||||||||||:||||||||||||||||
orf10nm      LFCLTSSAAYTCFGTPANYPLFAGVWAAYLAGCILRHRKDLHKLFHYLKKQGFPLX
          420       430       440       450       460       470
```

The complete length ORF10ng nucleotide sequence <SEQ ID 379> is:

```
   1 ATGGACACAA AAGAAATCCT CGGCTACGCG GCAGGCTCGA TCGGCAGCGC
  51 GGTTTTAGCC GTCATCATCC TGCCGCTGCT GTCGTGGTAT TTCcccgCCG
 101 ACGACATCGG GCGCATCGTG CTGATGCAGA CGGCGGCGGG ACTGACGGTG
 151 TCGGTATTGT GCCTCGGGCT GGATCAGGCA TACGTCCGCG AATACTATGC
 201 CGCCGCCGAC AAAGACACTT TGTTCAAAAC CCTGTTCCTG CCGCCGCTGC
 251 TGTTTTCCGC CGCGATAGCC GCCCTGCTGC TTTCCCGCCC GTCCCTGCCG
 301 TCTGAAATCC TGTTTTCGCT CGACGATGCC GCCGCCGGCA TCGGGCTGGT
 351 GCTGTTTGAA CTGAGCTTCC TGCCCATCCG CTTTCTCTTA CTGGTTTTGC
 401 GTATGGAAGG GCGCGCCCTT GCCTTTTCGT CCGCGCAACT CGTGCCCAAA
 451 CTCGCCATTC TGCTGCTGTT GCCGCTGACG GTCGGGCTGC TGCACTTTCC
 501 GGCGAACACC TCCGTCCTGA CCGCCGTTTA CGCGCTGGCA AACCTTGCCG
 551 CCGCCGCCTT TTTGCTGTTT CAAAACCGAT GCCGTCTGAA GGCCGTCCGG
 601 CGCGCGCCGT TTTCGCCCGC CGTCCTGCAC CGGGGGCTGC GCTACGGCAT
 651 ACCGCTCGCA CTGAGCAGCC TTGCCTATTG GGGGCTGGCA TCCGCCGACC
 701 GTTTGTTCCT GAAAAAATAT GCGGGCCTGG AACAGCTCGG CGTTTATTCG
 751 ATGGGTATTT CGTTCGGCGG GGCGGCATTA TTGCTCCAAA GCATCTTTTC
 801 AACGGTCTGG ACACCGTATA TTTTCCGTGC AATCGAAGAA AACGCCACGC
 851 CCGCCCGCCT CTCGGCAACG GCAGAATCCG CCGCCGCCCT GCTTGCCTCC
 901 GCCCTCTGCC TGACCGGAAT TTTCTCGCCC CTCGCCTCCC TCCTGCTGCC
 951 GGAAAACTAC GCCGCCGTCC GGTTTACCGT CGTATCGTGT ATGCTGccgc
1001 cgctGTTTTA CACGCTGACC GAAATCAGCG GCATCGGTTT GAACGTCGTC
1051 CGCAAAACGC GTCCGATCGC GCTTGCCACC TTGGGCGCGC TGGCGGCAAA
1101 CCTGCTGCTG CTGGGGCTTG CCGTACCGTC CGGCGGCACG CGCGGCGCGG
1151 CGGTTGCCTG TGCCGCCTCA TTCTGGTTGT TTTTTGTTTT CAAGACAGAA
1201 AGCTCCTGCC GCCTGTGGCA GCCGCTCAAA CGCCTGCCGC TTTATATGCA
1251 CACATTGTTC TGCCTgGCCT CCTCGGCGGC CTACACCTGC TTCGGCACAC
1301 CGGCAAACTA CCCcctgttt gccggcgtAT GGGCGGCATA TCTGGCAGGC
1351 TGCATCCTGC GCCACCGGAA AAATTTGCAC AAACTGTTTC ATTATTTGAA
1401 AAAACAAGGT TTCCCATTAT GA
```

This encodes a protein having amino acid sequence <SEQ ID 380>:

```
  1 MDTKEILGYA AGSIGSAVLA VIILPLLSWY FPADDIGRIV LMQTAAGLTV
 51 SVLCLGLDQA YVREYYAAAD KDTLFKTLFL PPLLFSAAIA ALLLSRPSLP
101 SEILFSLDDA AAGIGLVLFE LSFLPIRFLL LVLRMEGRAL AFSSAQLVPK
151 LAILLLLPLT VGLLHFPANT SVLTAVYALA NLAAAAFLLF QNRCRLKAVR
201 RAPFSPAVLH RGLRYGIPLA LSSLAYWGLA SADRLFLKKY AGLEQLGVYS
```

```
251 MGISFGGAAL LLQSIFSTVW TPYIFRAIEE NATPARLSAT AESAAALLAS
301 ALCLTGIFSP LASLLLPENY AAVRFTVVSC MLPPLFYTLT EISGIGLNVV
351 RKTRPIALAT LGALAANLLL LGLAVPSGGT RGAAVACAAS FWLFFVFKTE
401 SSCRLWQPLK RLPLYMHTLF CLASSAAYTC FGTPANYPLF AGVWAAYLAG
451 CILRHRKNLH KLFHYLKKQG FPL*
```

ORF10ng and ORF10-1 show 96.4% identity in 473 aa overlap:

```
                      10        20        30        40        50        60
orf10-1.pep   MDTKEILGYAAGSIGSAVLAVIILPLLSWYFPADDIGRIVLMQTAAGLTVSVLCLGLDQA
              ||||||||||||||||||||||||||||||||||||||||||||||||||||||||||||
orf10ng-1     MDTKEILGYAAGSIGSAVLAVIILPLLSWYFPADDIGRIVLMQTAAGLTVSVLCLGLDQA
                      10        20        30        40        50        60

                      70        80        90       100       110       120
orf10-1.pep   YVREYYATADKDTLFKTLFLPPLLSAAAIAALLLSRPSLPSEILFSLDDAAAGIGLVLFE
              ||||||:||||||||||||||||| :||||||||||||||||||||||||||||||||||
orf10ng-1     YVREYYAAADKDTLFKTLFLPPLLFSAAIAALLLSRPSLPSEILFSLDDAAAGIGLVLFE
                      70        80        90       100       110       120

                     130       140       150       160       170       180
orf10-1.pep   LSFLPIRFLLLVLRMEGRALAFSSAQLVPKLAILLLLPLTVGLLHFPANTAVLTAVYALA
              |||||||||||||||||||||||||||||||||||||||||||||||||||:||||||||
orf10ng-1     LSFLPIRFLLLVLRMEGRALAFSSAQLVPKLAILLLLPLTVGLLHFPANTSVLTAVYALA
                     130       140       150       160       170       180

                     190       200       210       220       230       240
orf10-1.pep   NLAAAAFLLFQNRCRLKAVRHAPFSPAVLHRGLRYGIPIALSSIAYWGLASADRLFLKKY
              ||||||||||||||||||||:|||||||||||||||||:||||:||||||||||||||||
orf10ng-1     NLAAAAFLLFQNRCRLKAVRRAPFSPAVLHRGLRYGIPLALSSLAYWGLASADRLFLKKY
                     190       200       210       220       230       240

                     250       260       270       280       290       300
orf10-1.pep   AGLEQLGVYSMGISFGGAALLFQSIFSTVWTPYIFRAIEENAPPARLSATAESAAALLAS
              |||||||||||||||||||||:|||||||||||||||||||| |||||||||||||||||
orf10ng-1     AGLEQLGVYSMGISFGGAALLLQSIFSTVWTPYIFRAIEENATPARLSATAESAAALLAS
                     250       260       270       280       290       300

                     310       320       330       340       350       360
orf10-1.pep   ALCLTGIFSPLASLLLPENYAAVRFIVVSCMLPPLFCTLAEISGIGLNVVRKTRPIALAT
              ||||||||||||||||||||||||| |||||||||| ||:||||||||||||||||||||
orf10ng-1     ALCLTGIFSPLASLLLPENYAAVRFTVVSCMLPPLFYTLTEISGIGLNVVRKTRPIALAT
                     310       320       330       340       350       360

                     370       380       390       400       410       420
orf10-1.pep   LGALAANLLLLGLAVPSGGARGAAVACAASFWLFFAFKTESSCRLWQPLKRLPLYLHTLF
              |||||||||||||||||||:|||||||||||||||:|||||||||||||||||||:||||
orf10ng-1     LGALAANLLLLGLAVPSGGTRGAAVACAASFWLFFVFKTESSCRLWQPLKRLPLYMHTLF
                     370       380       390       400       410       420

                     430       440       450       460       470
orf10-1.pep   CLTSSAAYTCFGTPANYPLFAGVWAAYLAGCILRHRKDLHKLFHYLKKQGFPLX
              ||:||||||||||||||||||||||||||||||||||:||||||||||||||||
orf10ng-1     CLASSAAYTCFGTPANYPLFAGVWAAYLAGCILRHRKNLHKLFHYLKKQGFPLX
                     430       440       450       460       470
```

Based on this analysis, including the presence of a putative leader peptide and several transmembrane segments and the presence of a leucine-zipper motif (4 Leu residues spaced by 6 aa, shown in bold), it is predicted that these proteins from *N.meningitidis* and *N.gonorrhoeae,* and their epitopes, could be useful antigens for vaccines or diagnostics, or for raising antibodies.

**Example 45**

The following partial DNA sequence was identified in *N.meningitidis* <SEQ ID 381>:

```
1..ATCCTGAAAC CGCATAACCA GCTTAAGGAA GACATCCAAC CTGATCCGGC
 51  CGATCAAAAC GCCTTGTCCG AACCGGATGC TGCGACAGAG GCAGAGCAGT
101  CGGATGCGGA AAATGCTGCC GACAAGCAGC CCGTTGCCGA TAAAGCCGAC
151  GAGGTTGAAG AAAAGGCGGG CGAGCCGGAA CGGGAAGAGC CGGACGGACA
201  GGCAGTGCGT AAGAAAGCGC TGACGGAAGA GCGTGAACAA ACCGTCAGGG
251  AAAAAGCGCA GAAGAAAGAT GCCGAAACGG TTAAAATACA AGCGGTAAAA
301  CCGTCTAAAG AAACAGAGAA AAAAGCTTCA AAAGAAGAGA AAAAGGCGGC
351  GAAGGAAAAA GTTGCACCCA AACCAACCCC GGAACAAATC CTCAACAGCG
401  GCAgCATCGA AAAmGCGCGC AgTGCCGCCG CCAAAGAAGT GCAGAAAATG
451  AA.AACGTCC GACAAGGCGG AAGC.AACGC ATTATCTGCA AATGGGCGCG
501  TATGCCGACC GTCAGAGCGC GGAAGGGCAG CGTGCCAAAC TGGCAATCTT
551  GGGCATATCT TCCAAGGTGG TCGGTTATCA GGCGGGACAT AAAACGCTTT
601  ACCGGGTGCA AAGCGGCAAT ATGTCTGCCG ATGCGGTGA
```

This corresponds to the amino acid sequence <SEQ ID 382; ORF65>:

```
1..ILKPHNQLKE DIQPDPADQN ALSEPDAATE AEQSDAENAA DKQPVADKAD
 51  EVEEKAGEPE REEPDGQAVR KKALTEEREQ TVREKAQKKD AETVKIQAVK
101  PSKETEKKAS KEEKKAAKEK VAPKPTPEQI LNSGSIEXAR SAAAKEVQKM
151  XNVRQGGSXR IICKWARMPT VRARKGSVPN WQSWAYLPRW SVIRRDIKRF
201  TGCKAAICLP MR*
```

Further work revealed the complete nucleotide sequence <SEQ ID 383>:

```
  1  ATGTTTATGA ACAAATTTTC CCAATCCGGA AAAGGTCTGT CCGGTTTTTT
 51  CTTCGGTTTG ATACTGGCGA CGGTCATTAT TGCCGGTATT TTGTTTTATC
101  TGAACCAGAG CGGTCAAAAT GCGTTCAAAA TCCCGGCTTC GTCGAAGCAG
151  CCTGCAGAAA CGGAAATCCT GAAACCGAAA AACCAGCCTA AGGAAGACAT
201  CCAACCTGAA CCGGCCGATC AAAACGCCTT GTCCGAACCG GATGCTGCGA
251  CAGAGGCAGA GCAGTCGGAT GCGGAAAAAG CTGCCGACAA GCAGCCCGTT
301  GCCGATAAAG CCGACGAGGT TGAAGAAAAG GCGGGCGAGC CGGAACGGGA
351  AGAGCCGGAC GGACAGGCAG TGCGTAAGAA AGCGCTGACG GAAGAGCGTG
401  AACAAACCGT CAGGGAAAAA GCGCAGAAGA AAGATGCCGA AACGGTTAAA
451  AAACAAGCGG TAAAACCGTC TAAAGAAACA GAGAAAAAAG CTTCAAAAGA
501  AGAGAAAAAG GCGGCGAAGG AAAAAGTTGC ACCCAAACCA ACCCCGGAAC
551  AAATCCTCAA CAGCGGCAGC ATCGAAAAAG CGCGCAGTGC CGCCGCCAAA
601  GAAGTGCAGA AAATGAAAAC GTCCGACAAG GCGGAAGCAA CGCATTATCT
651  GCAAATGGGC GCGTATGCCG ACCGTCAGAG CGCGGAAGGG CAGCGTGCCA
701  AACTGGCAAT CTTGGGCATA TCTTCCAAGG TGGTCGGTTA TCAGGCGGGA
751  CATAAAACGC TTTACCGGGT GCAAAGCGGC AATATGTCTG CCGATGCGGT
801  GAAAAAAATG CAGGACGAGT TGAAAAAACA TGAAGTCGCC AGCCTGATCC
851  GTTCTATCGA AAGCAAATAA
```

This corresponds to the amino acid sequence <SEQ ID 384; ORF65-1>:

```
  1  MFMNKFSQSG KGLSGFFFGL ILATVIIAGI LFYLNQSGQN AFKIPASSKQ
 51  PAETEILKPK NQPKEDIQPE PADQNALSEP DAATEAEQSD AEKAADKQPV
101  ADKADEVEEK AGEPEREEPD GQAVRKKALT EEREQTVREK AQKKDAETVK
151  KQAVKPSKET EKKASKEEKK AAKEKVAPKP TPEQILNSGS IEKARSAAAK
201  EVQKMKTSDK AEATHYLQMG AYADRQSAEG QRAKLAILGI SSKVVGYQAG
251  HKTLYRVQSG NMSADAVKKM QDELKKHEVA SLIRSIESK*
```

Computer analysis of this amino acid sequence gave the following results:

<u>Homology with a predicted ORF from *N.meningitidis* (strain A)</u>

ORF65 shows 92.0% identity over a 150aa overlap with an ORF (ORF65a) from strain A of *N. meningitidis:*

```
                                                    10        20        30
orf65.pep                                   ILKPHNQLKEDIQPDPADQNALSEPDAATE
                                            ||||:|| ||||||:||||||||||||| |
orf65a        IIAGILFYLNQSGQNAFKIPVPSKQPAETEILKPKNQPKEDIQPEPADQNALSEPDAAKE
                 30        40        50        60        70        80

                      40        50        60        70        80        90
orf65.pep     AEQSDAENAADKQPVADKADEVEEKAGEPEREEPDGQAVRKKALTEEREQTVREKAQKKD
              |||||||:|||||||||||||||||| |||||: |||||||||||||||||| |||||||
orf65a        AEQSDAEKAADKQPVADKADEVEEKADEPEREKSDGQAVRKKALTEEREQTVGEKAQKKD

                 90       100       110       120       130       140

                     100       110       120       130       140       150
orf65.pep     AETVKIQAVKPSKETEKKASKEEKKAAKEKVAPKPTPEQILNSGSIEXARSAAAKEVQKM
              ||||| |||||||||||||||||||| |||||||||||||||||||| ||||||||||||
orf65a        AETVKKQAVKPSKETEKKASKEEKKAEKEKVAPKPTPEQILNSGSIEKARSAAAKEVQKM
                150       160       170       180       190       200

                     160       170       180       190       200       210
orf65.pep     XNVRQGGSXRIICKWARMPTVRARKGSVPNWQSWAYLPRWSVIRRDIKRFTGCKAAICLP

orf65a        KTPDKAEATHYLQMGAYADRRSAEGQRAKLAILGISSKVVGYQAGHKTLYRVQSGNMSAD
                210       220       230       240       250       260
```

The complete length ORF65a nucleotide sequence <SEQ ID 385> is:

```
  1 ATGTTTATGA ACAAATTTTC CCAATCCGGA AAAGGTCTGT CCGGTTTTTT
 51 CTTCGGTTTG ATACTGGCGA CGGTCATTAT TGCCGGTATT TTGTTTTATC
101 TGAACCAGAG CGGTCAAAAT GCGTTCAAAA TCCCGGTTCC GTCGAAGCAG
151 CCTGCAGAAA CGGAAATCCT GAAACCGAAA AACCAGCCTA AGGAAGACAT
201 CCAACCTGAA CCGGCCGATC AAAACGCCTT GTCCGAACCG GATGCTGCGA
251 AAGAGGCAGA GCAGTCGGAT GCGGAAAAAG CTGCCGACAA GCAGCCCGTT
301 GCCGACAAAG CCGACGAGGT TGAGGAAAAG GCGGACGAGC CGGAGCGGGA
351 AAAGTCGGAC GGACAGGCAG TGCGCAAGAA AGCACTGACG GAAGAGCGTG
401 AACAAACCGT CGGGGAAAAA GCGCAGAAGA AAGATGCCGA AACGGTTAAA
451 AAACAAGCGG TAAAACCATC TAAAGAAACA GAGAAAAAAG CTTCAAAAGA
501 AGAGAAAAAG GCGGAGAAGG AAAAAGTTGC ACCCAAACCG ACCCCGGAAC
551 AAATCCTCAA CAGCGGCAGC ATCGAAAAAG CGCGCAGTGC CGCTGCCAAA
601 GAAGTGCAGA AAATGAAAAC GCCCGACAAG GCGGAAGCAA CGCATTATCT
651 GCAAATGGGC GCGTATGCCG ACCGCCGGAG CGCGGAAGGG CAGCGTGCCA
701 AACTGGCAAT CTTGGGCATA TCTTCCAAGG TGGTCGGTTA TCAGGCGGGA
751 CATAAAACGC TTTACCGGGT GCAAAGCGGC AATATGTCTG CCGATGCGGT
801 GAAAAAAATG CAGGACGAGT TGAAAAAACA TGAAGTCGCC AGCCTGATCC
851 GTTCTATCGA AAGCAAATAA
```

This encodes a protein having amino acid sequence <SEQ ID 386>:

```
  1 MFMNKFSQSG KGLSGFFFGL ILATVIIAGI LFYLNQSGQN AFKIPVPSKQ
 51 PAETEILKPK NQPKEDIQPE PADQNALSEP DAAKEAEQSD AEKAADKQPV
101 ADKADEVEEK ADEPEREKSD GQAVRKKALT EEREQTVGEK AQKKDAETVK
151 KQAVKPSKET EKKASKEEKK AEKEKVAPKP TPEQILNSGS IEKARSAAAK
201 EVQKMKTPDK AEATHYLQMG AYADRRSAEG QRAKLAILGI SSKVVGYQAG
251 HKTLYRVQSG NMSADAVKKM QDELKKHEVA SLIRSIESK*
```

ORF65a and ORF65-1 show 96.5% identity in 289 aa overlap:

```
                    10        20        30        40        50        60
orf65a.pep  MFMNKFSQSGKGLSGFFFGLILATVIIAGILFYLNQSGQNAFKIPVPSKQPAETEILKPK
            ||||||||||||||||||||||||||||||||||||||||||||:  ||||||||||||
orf65-1     MFMNKFSQSGKGLSGFFFGLILATVIIAGILFYLNQSGQNAFKIPASSKQPAETEILKPK
                    10        20        30        40        50        60

                    70        80        90       100       110       120
orf65a.pep  NQPKEDIQPEPADQNALSEPDAAKEAEQSDAEKAADKQPVADKADEVEEKADEPEREKSD
            ||||||||||||||||||||||| |||||||||||||||||||||||||||| |||||: |
orf65-1     NQPKEDIQPEPADQNALSEPDAATEAEQSDAEKAADKQPVADKADEVEEKAGEPEREEPD
                    70        80        90       100       110       120

                   130       140       150       160       170       180
orf65a.pep  GQAVRKKALTEEREQTVGEKAQKKDAETVKKQAVKPSKETEKKASKEEKKAEKEKVAPKP
            |||||||||||||||| |||||||||||||||||||||||||||||||||| ||||||||
orf65-1     GQAVRKKALTEEREQTVREKAQKKDAETVKKQAVKPSKETEKKASKEEKKAAKEKVAPKP
                   130       140       150       160       170       180

                   190       200       210       220       230       240
orf65a.pep  TPEQILNSGSIEKARSAAAKEVQKMKTPDKAEATHYLQMGAYADRRSAEGQRAKLAILGI
            |||||||||||||||||||||||||| ||||||||||||||||||:||||||||||||||
orf65-1     TPEQILNSGSIEKARSAAAKEVQKMKTSDKAEATHYLQMGAYADRQSAEGQRAKLAILGI
                   190       200       210       220       230       240

                   250       260       270       280       290

orf65a.pep  SSKVVGYQAGHKTLYRVQSGNMSADAVKKMQDELKKHEVASLIRSIESKX
            ||||||||||||||||||||||||||||||||||||||||||||||||||
orf65-1     SSKVVGYQAGHKTLYRVQSGNMSADAVKKMQDELKKHEVASLIRSIESKX
                     250       260       270       280       290
```

Homology with a predicted ORF from *N.gonorrhoeae*

ORF65 shows 89.6% identity over a 212aa overlap with a predicted ORF (ORF65.ng) from *N. gonorrhoeae:*

```
                30        40        50        60        70        80
ORF65ng  IIAGILLYLNQGGQNAFKIPAPSKQPAETEILKLKNQPKEDIQPEPADQNALSEPDVAKE
                                             ||| :|| ||||||:||||||||||||:| |
ORF65                                        ILKPHNQLKEDIQPDPADQNALSEPDAATE
                                                     10        20        30

                90       100       110       120       130       140
ORF65ng  AEQSDAEKAADKQPVADKADEVEEKAGEPEREEPDGQAVRKKALTEEREQTVREKAQKKD
            ||||||:|||||||||||||||||||||||||||||||||||||||||||||||||||||
ORF65       AEQSDAENAADKQPVADKADEVEEKAGEPEREEPDGQAVRKKALTEEREQTVREKAQKKD
                    40        50        60        70        80        90

               150       160       170       180       190       200
ORF65ng  AETVKKKAVKPSKETEKKASKEEKKAAKEKVAPKPTPEQILNSRSIEKARSAAAKEVQKM
            ||||| :|||||||||||||||||||||||||||||||||||| ||| ||||||||||||
ORF65       AETVKIQAVKPSKETEKKASKEEKKAAKEKVAPKPTPEQILNSGSIEXARSAAAKEVQKM
                   100       110       120       130       140       150

               210       220       230       240       250       260
ORF65ng  KNFGQGGSQRIICKWARMPNPGARKGSVPNWQSWAYLPKWSAIRRDIKRFTACKAAICPP
            |   |||| ||||||||||:  |||||||||||||||||:||:||||||||||:|||||| |
ORF65       XNVRQGGSXRIICKWARMPTVRARKGSVPNWQSWAYLPRWSVIRRDIKRFTGCKAAICLP
                   160       170       180       190       200       210

ORF65ng     MR
            ||
ORF65       MR
```

An ORF65ng nucleotide sequence <SEQ ID 387> was predicted to encode a protein having amino acid sequence <SEQ ID 388>:

```
  1  MFMNKFSQSG KGLSGFFFGL ILATVIIAGI LLYLNQGGQN AFKIPAPSKQ
 51  PAETEILKLK NQPKEDIQPE PADQNALSEP DVAKEAEQSD AEKAADKQPV
101  ADKADEVEEK AGEPEREEPD GQAVRKKALT EEREQTVREK AQKKDAETVK
151  KKAVKPSKET EKKASKEEKK AAKEKVAPKP TPEQILNSRS IEKARSAAAK
201  EVQKMKNFGQ GGSQRIICKW ARMPNPGARK GSVPNWQSWA YLPKWSAIRR
251  DIKRFTACKA AICPPMR*
```

After further analysis, the complete gonococcal DNA sequence <SEQ ID 389> was found to be:

```
  1  ATGTTTATGA ACAAATTTTC CCAATCCGGA AAAGGTCTGT CCGGTTTCTT
 51  CTTCGGTTTG ATACTGGCAA CGGTCATTAT TGCCGGTATT TTGCTTTATC
101  TGAACCAGGG CGGTCAAAAT GCGTTCAAAA TCCCGGCTCC GTCGAAGCAG
151  CCTGCAGAAA CGGAAATCCT GAAACTGAAA AACCAGCCTA AGGAAGACAT
201  CCAACCTGAA CCGGCCGATC AAAACGCCTT GTCCGAACCG GATGTTGCGA
251  AAGAGGCAGA GCAGTCGGAT GCGGAAAAAG CTGCCGACAA GCAGCCCGTT
301  GCCGACAAag ccgacgAGGT TGAAGAAAag GcGGgcgAgc cggaACGGga
351  aGAGCCGGAC ggACAGGCAG TGCGCAAGAA AGCACTGAcg gAAGAgcGTG
401  AACAAACcgt cagggAAAAA GCGCagaaga AAGATGCCGA AACGgTTAAA
451  AAacaaGCgg tAaaaccgtc tAAAGAAACa gagaaaaaag cTtcaaaaga
501  agagaaaaag gcggcgaaag aaaAAGttgc acccaaaccg accccggaaC
551  aaatcctcaa cagccgCagc atcgaaaaag cgcgtagtgc cgctgccaaa
601  gaAgtgcaGA AAatgaaaaa ctTtgggcaa ggcgGaagcc aacgcattaT
651  CTGcaaatgg gcgcgtatgc cgaccgtccg gagcgcggaA gggcagcgtg
701  ccaaACtggc aAtcttgGgc atatctTccg aagtggtcgG CTATCAGGCG
751  GGACATAAAA CGCTTTACCG CGTGCAAagc GGCAatatgt ccgccgatgc
801  gGTGAAAAAA ATGCAGGACG AGTTGAAAAA GCATGGGGtt gcCAGCCTGA
851  TCCGTGcgAT TGAAGGCAAA TAA
```

This encodes the following amino acid sequence <SEQ ID 390>:

```
  1  MFMNKFSQSG KGLSGFFFGL ILATVIIAGI LLYLNQGGQN AFKIPAPSKQ
 51  PAETEILKLK NQPKEDIQPE PADQNALSEP DVAKEAEQSD AEKAADKQPV
101  ADKADEVEEK AGEPEREEPD GQAVRKKALT EEREQTVREK AQKKDAETVK
151  KQAVKPSKET EKKASKEEKK AAKEKVAPKP TPEQILNSRS IEKARSAAAK
201  EVQKMKNFGQ GGSQRIICKW ARMPTVRSAE GQRAKLAILG ISSEVVGYQA
251  GHKTLYRVQS GNMSADAVKK MQDELKKHGV ASLIRAIEGK *
```

ORF65ng-1 and ORF65-1 show 89.0% identity in 290 aa overlap:

```
                    10        20        30        40        50        60
orf65-1.pep  MFMNKFSQSGKGLSGFFFGLILATVIIAGILFYLNQSGQNAFKIPASSKQPAETEILKPK
             ||||||||||||||||||||||||||||||:||||:||||||||| |||||||||| |
orf65ng-1    MFMNKFSQSGKGLSGFFFGLILATVIIAGILLYLNQGGQNAFKIPAPSKQPAETEILKLK
                    10        20        30        40        50        60

                    70        80        90       100       110       120
orf65-1.pep  NQPKEDIQPEPADQNALSEPDAATEAEQSDAEKAADKQPVADKADEVEEKAGEPEREEPD
             |||||||||||||||||||||:| ||||||||||||||||||||||||||||||||||||
orf65ng-1    NQPKEDIQPEPADQNALSEPDVAKEAEQSDAEKAADKQPVADKADEVEEKAGEPEREEPD
                    70        80        90       100       110       120

                   130       140       150       160       170       180
orf65-1.pep  GQAVRKKALTEEREQTVREKAQKKDAETVKKQAVKPSKETEKKASKEEKKAAKEKVAPKP
             ||||||||||||||||||||||||||||||||||||||||||||||||||||||||||||
orf65ng-1    GQAVRKKALTEEREQTVREKAQKKDAETVKKQAVKPSKETEKKASKEEKKAAKEKVAPKP
                   130       140       150       160       170       180

                   190       200       210       220       230       239
orf65-1.pep  TPEQILNSGSIEKARSAAAKEVQKMKTSDKAEATHYL-QMGAYADRQSAEGQRAKLAILG
             |||||||| ||||||||||||||||:  :: : : : : :   :||||||||||||
orf65ng-1    TPEQILNSRSIEKARSAAAKEVQKMKNFGQGGSQRIICKWARMPTVRSAEGQRAKLAILG
                   190       200       210       220       230       240

               240       250       260       270       280       290
orf65-1.pep  ISSKVVGYQAGHKTLYRVQSGNMSADAVKKMQDELKKHEVASLIRSIESKX
             |||:|||||||||||||||||||||||||||||||||| ||||||:||:||
orf65ng-1    ISSEVVGYQAGHKTLYRVQSGNMSADAVKKMQDELKKHGVASLIRAIEGKX
                   250       260       270       280       290
```

On this basis, including the presence of a putative transmembrane domain in the gonococcal protein, it is predicted that the proteins from *N.meningitidis* and *N.gonorrhoeae,* and their epitopes, could be useful antigens for vaccines or diagnostics, or for raising antibodies.

**Example 46**

The following DNA sequence, believed to be complete, was identified in *N.meningitidis* <SEQ ID 391>:

```
  1  ATGAACCACG ACATCACTTT CCTCACCCTG TTCCTACTCG GTkTCTTCGG
 51  CGGAAcGCAC TGCATCGGTA TGTGCGGCGG ATTAAGCAGC GcGTTTGs.s
101  TCCAACTCCC CCCGCATATC AACCGCTTTT GGCTGATCCT GCTGCTTAAC
151  ACAGGACGGG TAAGCAGCTA TACGGCAAtC GGCCTGATAC TCGGATTAAT
201  CGGACAGGTC GGCGTTTCAC TCGAcCAaAC CCGCGTCCTG CAGAATATTT
251  TATACACGGC CGCCAACCTC CTGCTGCTCT TTTTAGGCTT ATACTTGAGC
301  GGTATTTCTT CCTTGGCGGC AAAAATCGAG AAaATCGGCA AACCGATATG
351  GCGGAACCTG AACCCGATAC TCAACCGGCT GTTACCCATA AAATCCATAC
401  CCGCCTGCCT tGCGgTCGGA ATATTATGGG GCTGGCTGCC GTGCGGACTG
451  GTTTACAGCG CGTCGCTTTA CGCGCTGGGA AgCGGTAGTG CGGCAACGGG
501  CGGGTTATAT ATGCTTGCCT TTGCACTGGG TACGCTGCCC AATCTTtTAG
551  CAATCGGCAT TTTtTCCCTG CAACTGAAwA AAATCATGCA AAACCGATAT
601  ATCCGCCTGT GTACGGGATT ATCCGTATCA TTATGGGCAT TATGGAAACT
651  TGCCGTCCTG TGGCTGTAA
```

This corresponds to the amino acid sequence <SEQ ID 392; ORF103>:

```
  1  MNHDITFLTL FLLGXFGGTH CIGMCGGLSS AFXXQLPPHI NRFWLILLLN
 51  TGRVSSYTAI GLILGLIGQV GVSLDQTRVL QNILYTAANL LLLFLGLYLS
101  GISSLAAKIE KIGKPIWRNL NPILNRLLPI KSIPACLAVG ILWGWLPCGL
151  VYSASLYALG SGSAATGGLY MLAFALGTLP NLLAIGIFSL QLXKIMQNRY
201  IRLCTGLSVS LWALWKLAVL WL*
```

Further work elaborated the DNA sequence <SEQ ID 393> as:

```
  1  ATGAACCACG ACATCACTTT CCTCACCCTG TTCCTACTCG GTTTCTTCGG
 51  CGGAACGCAC TGCATCGGTA TGTGCGGCGG ATTAAGCAGC GCGTTTGCGC
101  TCCAACTCCC CCCGCATATC AACCGCTTTT GGCTGATCCT GCTGCTTAAC
151  ACAGGACGGG TAAGCAGCTA TACGGCAATC GGCCTGATAC TCGGATTAAT
201  CGGACAGGTC GGCGTTTCAC TCGACCAAAC CCGCGTCCTG CAGAATATTT
251  TATACACGGC CGCCAACCTC CTGCTGCTCT TTTTAGGCTT ATACTTGAGC
301  GGTATTTCTT CCTTGGCGGC AAAAATCGAG AAAATCGGCA AACCGATATG
351  GCGGAACCTG AACCCGATAC TCAACCGGCT GTTACCCATA AAATCCATAC
401  CCGCCTGCCT TGCGGTCGGA ATATTATGGG GCTGGCTGCC GTGCGGACTG
451  GTTTACAGCG CGTCGCTTTA CGCGCTGGGA AGCGGTAGTG CGGCAACGGG
501  CGGGTTATAT ATGCTTGCCT TTGCACTGGG TACGCTGCCC AATCTTTTAG
551  CAATCGGCAT TTTTTCCCTG CAACTGAAAA AAATCATGCA AAACCGATAT
601  ATCCGCCTGT GTACGGGATT ATCCGTATCA TTATGGGCAT TATGGAAACT
651  TGCCGTCCTG TGGCTGTAA
```

This corresponds to the amino acid sequence <SEQ ID 394; ORF103-1>:

```
  1  MNHDITFLTL FLLGFFGGTH CIGMCGGLSS AFALQLPPHI NRFWLILLLN
 51  TGRVSSYTAI GLILGLIGQV GVSLDQTRVL QNILYTAANL LLLFLGLYLS
101  GISSLAAKIE KIGKPIWRNL NPILNRLLPI KSIPACLAVG ILWGWLPCGL
151  VYSASLYALG SGSAATGGLY MLAFALGTLP NLLAIGIFSL QLKKIMQNRY
201  IRLCTGLSVS LWALWKLAVL WL*
```

Computer analysis of this amino acid sequence gave the following results:

Homology with a predicted ORF from *N.meningitidis* (strain A)

ORF103 shows 93.8% identity over a 222aa overlap with an ORF (ORF103a) from strain A of *N. meningitidis:*

```
                        10        20        30        40        50        60
orf103.pep    MNHDITFLTLFLLGXFGGTHCIGMCGGLSSAFXXQLPPHINRFWLILLLNTGRVSSYTAI
              || ||||||||||| |||||||||||||||||  |||||||| |||||||||||||||||
orf103a       MNXDITFLTLFLLGFFGGTHCIGMCGGLSSAFALQLPPHINRXWLILLLNTGRVSSYTAI
                        10        20        30        40        50        60

                        70        80        90       100       110       120
orf103.pep    GLILGLIGQVGVSLDQTRVLQNILYTAANLLLLFLGLYLSGISSLAAKIEKIGKPIWRNL
              ||||||||||||||||||| ||||||||||||||||||||||||||||||||||||||||
orf103a       GLILGLIGQVGVSLDQTRVXQNILYTAANLLLLFLGLYLSGISSLAAKIEKIGKPIWRNL
                        70        80        90       100       110       120

                       130       140       150       160       170       180
orf103.pep    NPILNRLLPIKSIPACLAVGILWGWLPCGLVYSASLYALGSGSAATGGLYMLAFALGTLP
              ||||||||||||||||||||||||||||||||||||||||||||||||||||||||||||
orf103a       NPILNRLLPIKSIPACLAVGILWGWLPCGLVYSASLYALGSGSAATGGLYMLAFALGTLP
                       130       140       150       160       170       180

                       190       200       210       220
orf103.pep    NLLAIGIFSLQLXKIMQNRYIRLCTGLSVSLWALWKLAVLWLX
              || |||||||||||||||||||||||||||||||||||||||
orf103a       NLXAIGIFSLQLXKIMQNRYIRLCTGLSVSLWALWKLAVLWLX
                       190       200       210       220
```

The complete length ORF103a nucleotide sequence <SEQ ID 395> is:

```
  1  ATGAACCANG ACATCACTTT CCTCACCCTG TTCCTACTCG GTTTCTTCGG
 51  CGGAACGCAC TGCATCGGTA TGTGCGGCGG ATTAAGCAGC GCGTTTGCGC
101  TCCAACTCCC CCCGCATATC AACCGCTTNT GGCTGATCCT GCTGCTTAAC
151  ACAGGACGGG TAAGCAGCTA TACGGCAATC GGCCTGATAC TCGGATTAAT
201  CGGACAGGTC GGCGTTTCAC TCGACCAAAC CCGCGTCNTG CAGAATATTT
251  TATACACGGC CGCCAACCTC CTGCTGCTCT TTTTAGGCTT ATACTTGAGC
301  GGTATTTCTT CCTTGGCGGC AAAAATCGAG AAAATCGGCA AACCGATATG
351  GCGGAACCTG AACCCGATAC TCAACCGGCT GTTACCCATA AAATCCATAC
401  CCGCCTGCCT TGCGGTCGGA ATATTATGGG GCTGGCTGCC GTGCGGACTA
451  GTTTACAGCG CGTCGCTTTA CGCGCTGGGA AGCGGTAGTG CGGCAACGGG
501  CGGGTTATAT ATGCTTGCCT TTGCACTGGG TACGCTGCCC AATCTTTNGG
551  CAATCGGCAT TTTTTCCCTG CAACTGNAAA AAATCATGCA AAACCGATAT
601  ATCCGCCTGT GTACGGGATT ATCCGTATCA TTATGGGCAT TATGGAAACT
651  TGCCGTCCTG TGGCTGTAA
```

This encodes a protein having amino acid sequence <SEQ ID 396>:

```
  1  MNXDITFLTL FLLGFFGGTH CIGMCGGLSS AFALQLPPHI NRXWLILLLN
 51  TGRVSSYTAI GLILGLIGQV GVSLDQTRVX QNILYTAANL LLLFLGLYLS
101  GISSLAAKIE KIGKPIWRNL NPILNRLLPI KSIPACLAVG ILWGWLPCGL
151  VYSASLYALG SGSAATGGLY MLAFALGTLP NLXAIGIFSL QLXKIMQNRY
201  IRLCTGLSVS LWALWKLAVL WL*
```

ORF103a and ORF103-1 show 97.7% identity in 222 aa overlap:

```
                  10        20        30        40        50        60
orf103a.pep    MNXDITFLTLFLLGFFGGTHCIGMCGGLSSAFALQLPPHINRXWLILLLNTGRVSSYTAI
               || ||||||||||||||||||||||||||||||||||||||||| ||||||||||||||||
orf103-1       MNHDITFLTLFLLGFFGGTHCIGMCGGLSSAFALQLPPHINRFWLILLLNTGRVSSYTAI
                  10        20        30        40        50        60

                  70        80        90       100       110       120
orf103a.pep    GLILGLIGQVGVSLDQTRVXQNILYTAANLLLLFLGLYLSGISSLAAKIEKIGKPIWRNL
               ||||||||||||||||||| ||||||||||||||||||||||||||||||||||||||||
orf103-1       GLILGLIGQVGVSLDQTRVLQNILYTAANLLLLFLGLYLSGISSLAAKIEKIGKPIWRNL
                  70        80        90       100       110       120

                 130       140       150       160       170       180
orf103a.pep    NPILNRLLPIKSIPACLAVGILWGWLPCGLVYSASLYALGSGSAATGGLYMLAFALGTLP
               ||||||||||||||||||||||||||||||||||||||||||||||||||||||||||||
orf103-1       NPILNRLLPIKSIPACLAVGILWGWLPCGLVYSASLYALGSGSAATGGLYMLAFALGTLP
                 130       140       150       160       170       180

                 190       200       210       220
orf103a.pep    NLXAIGIFSLQLXKIMQNRYIRLCTGLSVSLWALWKLAVLWLX
               || ||||||||| |||||||||||||||||||||||||||||
orf103-1       NLLAIGIFSLQLKKIMQNRYIRLCTGLSVSLWALWKLAVLWLX
                 190       200       210       220
```

<u>Homology with a predicted ORF from *N.gonorrhoeae*</u>

ORF103 shows 95.5% identity over a 222aa overlap with a predicted ORF (ORF103.ng) from *N. gonorrhoeae:*

```
orf103.pep   MNHDITFLTLFLLGXFGGTHCIGMCGGLSSAFXXQLPPHINRFWLILLLNTGRVSSYTAI   60
             |||||||||||||| |||||||||||||||||  ||||||||||||||||||||:||||||
orf103ng     MNHDITFLTLFLLGFFGGTHCIGMCGGLSSAFALQLPPHINRFWLILLLNTGRISSYTAI   60

orf103.pep   GLILGLIGQVGVSLDQTRVLQNILYTAANLLLLFLGLYLSGISSLAAKIEKIGKPIWRNL  120
             ||:|||||:|:||||||||||||||||:||||||||||||||||||||||||||||||||
orf103ng     GLMLGLIGQLGISLDQTRVLQNILYTASNLLLLFLGLYLSGISSLAAKIEKIGKPIWRNL  120

orf103.pep   NPILNRLLPIKSIPACLAVGILWGWLPCGLVYSASLYALGSGSAATGGLYMLAFALGTLP  180
             |||||||||||||||||||||||||||||||||||||||||||||:||||||||||||||
orf103ng     NPILNRLLPIKSIPACLAVGILWGWLPCGLVYSASLYALGSGSATTGGLYMLAFALGTLP  180

orf103.pep   NLLAIGIFSLQLXKIMQNRYIRLCTGLSVSLWALWKLAVLWL  222
             |||||||||||| |||||||||||||||||||||||||||||
orf103ng     NLLAIGIFSLQLKKIMQNRYIRLCTGLSVSLWALWKLAVLWL  222
```

The complete length ORF103ng nucleotide sequence <SEQ ID 397> is:

```
1  ATGAACCACG ACATCACTTT CCTCACCCTG TTCCTGCTCG GTTTCTTCGG
```

```
 51  CGGAACTCAC TGCATCGGTA TGTGCGGCGG ATTAAGCAGC GCGTTTGCGC
101  TCCAACTCCC CCCGCATATC AACCGCTTTT GGCTGATTCT GCTGCTTAAC
151  ACAGGACGGA TAAGCAGCTA TACGGCAATC GGCCTGATGC TCGGATTAAT
201  CGGACAACTC GGCATTTCAC TCGACCAAAc ccgcgTCCTG CAAAATATTT
251  tatacacagc ctccaaCCTC CTGCTGCTCT TTTTAGGCTT ATACTTGAGC
301  GGTATTTCTT CCTTGGCGGC AAAAATCGAG AAAATCGGCA AACCGATATG
351  GCGCAACCTG AACCCGATAC TCAACCGGCT GCTGCCCATA AAATCCATAC
401  CCGCCTGCCT TGCTGTCGGA ATATTATGGG GCTGGCTGCC GTGCGGACTG
451  GTTTACAGCG CATCACTTTA CGCGCTGGGA AGCGGTAGTG CGACAACCGG
501  CGGACTGTAT ATGCTTGCCT TTGCACTGGG TACGCTGCCC AATCTTTTGG
551  CAATCGGCAT TTTTTCCCTG CAACTGAAAA AAATCATGCA AAACCGATAT
601  ATCCGCCTGT GTACAGGATT ATCCGTATCA TTATGGGCAT TATGGAAGCT
651  TGCCGTCCTG TGGCTGTAA
```

This encodes a protein having amino acid sequence <SEQ ID 398>:

```
  1  MNHDITFLTL FLLGFFGGTH CIGMCGGLSS AFALQLPPHI NRFWLILLLN
 51  TGRISSYTAI GLMLGLIGQL GISLDQTRVL QNILYTASNL LLLFLGLYLS
101  GISSLAAKIE KIGKPIWRNL NPILNRLLPI KSIPACLAVG ILWGWLPCGL
151  VYSASLYALG SGSATTGGLY MLAFALGTLP NLLAIGIFSL QLKKIMQNRY
201  IRLCTGLSVS LWALWKLAVL WL*
```

In addition, ORF103ng and ORF103-1 show 97.3% identity in 222 aa overlap:

```
                       10        20        30        40        50        60
orf103-1.pep   MNHDITFLTLFLLGFFGGTHCIGMCGGLSSAFALQLPPHINRFWLILLLNTGRVSSYTAI
               ||||||||||||||||||||||||||||||||||||||||||||||||||||||:|||||
orf103ng       MNHDITFLTLFLLGFFGGTHCIGMCGGLSSAFALQLPPHINRFWLILLLNTGRISSYTAI
                       10        20        30        40        50        60

                       70        80        90       100       110       120
orf103-1.pep   GLILGLIGQVGVSLDQTRVLQNILYTAANLLLLFLGLYLSGISSLAAKIEKIGKPIWRNL
               ||:|||||:|:||||||||||||||||:||||||||||||||||||||||||||||||||
orf103ng       GLMLGLIGQLGISLDQTRVLQNILYTASNLLLLFLGLYLSGISSLAAKIEKIGKPIWRNL
                       70        80        90       100       110       120

                      130       140       150       160       170       180
orf103-1.pep   NPILNRLLPIKSIPACLAVGILWGWLPCGLVYSASLYALGSGSAATGGLYMLAFALGTLP
               ||||||||||||||||||||||||||||||||||||||||||||:|||||||||||||||
orf103ng       NPILNRLLPIKSIPACLAVGILWGWLPCGLVYSASLYALGSGSATTGGLYMLAFALGTLP
                      130       140       150       160       170       180

                      190       200       210       220
orf103-1.pep   NLLAIGIFSLQLKKIMQNRYIRLCTGLSVSLWALWKLAVLWLX
               |||||||||||||||||||||||||||||||||||||||||||
orf103ng       NLLAIGIFSLQLKKIMQNRYIRLCTGLSVSLWALWKLAVLWLX
                      190       200       210       220
```

Based on this analysis, including the presence of a putative leader sequence (double-underlined) and several putative transmembrane domains (single-underlined) in the gonococcal protein, it is predicted that the proteins from *N.meningitidis* and *N.gonorrhoeae,* and their epitopes, could be useful antigens for vaccines or diagnostics, or for raising antibodies.

**Example 47**

The following partial DNA sequence was identified in *N.meningitidis* <SEQ ID 399>:

```
  1 ATGGAAAACC AAAGGCCGCT CCTAGGCTTT CGCTTGGCAC TTTTGGCGGC
 51 GATGACGTGG GGAACGCTGC CGAT.TCCGT GCGGCAGGTA TTGAAGTTTG
101 TCGATGCGCC GACGCTGGTG TGGGTGCGTT TTACCGTGGC GGCGGCGGTA
151 TTGTTTGTTT TGCTGGCACT GGGCGGGCGG CTGCcGAAGC GGCGaGGATT
201 TTTCTTGGTG CTCATTCAGG CTGCTGCTGC TCGGCGTGGC GGGCATTTCG
251 GCAAACTTTG TGCTGATTGC CCAAGGGCTG CATTATATTT CGCCGACCAC
301 GACGCAGGTT TTGTGGCAGA TTTCGCCGTT TACGATGATT GTwGTCGGTG
351 TGTTGGTGTT TAAAGACCGG ATGACTGCCG CTCAGAAAAT CGGCTTGGTT
401 TTGCTGCTTG CCGGTTTGCT TATGTATTTT AACGATAAAT TCGGCGAGTT

451 GTCGGGTTTG GGCGCGTATG C.AAGGGCGT GTTGCTGTGT GCGGCAGGCA
501 GTATGGCATG GGTGTGTAAT GCCGTGGCGC AAAAGCTGCT GTCGGCGCAA
551 TTCGGGCCGC AACAGATTCT GCTGTTGATT TATGCGGCAA GTGCCGCCGT
601 GTTCCTGCCG TTTGCCGAAC CGGCACACAT CGGAAGTATG GACGGTACGT
651 TGGCGTGGGT ATGTATTGCG TATTGCTGCT TGAATACGTT AATCGGTTAC
701 GGCTCGTTCG GCGAGGCGTT GAAACATTGG GAGGCTTCCA AAGTCAGCGC
751 GGTAACAACC TTGCTCCCCG TGTTTACCGT AATAAATACT TTGCTCGGGC
801 ATTATGTGAT GCCTGAAACT TTTGCCGCGC CGGA..
```

This corresponds to the amino acid sequence <SEQ ID 400; ORF104>:

```
  1 MENQRPLLGF RLALLAAMTW GTLPXSVRQV LKFVDAPTLV WVRFTVAAAV
 51 LFVLLALGGR LPKRRDFSWC SFRLLLLGVA GISANFVLIA QGLHYISPTT
101 TQVLWQISPF TMIVVGVLVF KDRMTAAQKI GLVLLLAGLL MYFNDKFGEL
151 SGLGAYXKGV LLCAAGSMAW VCNAVAQKLL SAQFGPQQIL LLIYAASAAV
201 FLPFAEPAHI GSMDGTLAWV CIAYCCLNTL IGYGSFGEAL KHWEASKVSA
251 VTTLLPVFTV INTLLGHYVM PETFAAP...
```

Further work revealed further partial DNA sequence <SEQ ID 401>:

```
  1 ATGGAAAACC AAAGGCCGCT CCTAGGCTTC GCGTTGGCAC TTTTGGCGGC
 51 GATGACGTGG GGAACGCTGC CGATTGCCGT GCGGCAGGTA TTGAAGTTTG
101 TCGATGCGCC GACGCTGGTG TGGGTGCGTT TTACCGTGGC GGCGGCGGTA
151 TTGTTTGTTT TGCTGGCACT GGGCGGGCGG CTGCCGAAGC GGCGGGATTT
201 TTCTTGGTGC TCATTCAGGC TGCTGCTGCT CGGCGTGGCG GGCATTTCGG
251 CAAACTTTGT GCTGATTGCC CAAGGGCTGC ATTATATTTC GCCGACCACG
301 ACGCAGGTTT TGTGGCAGAT TTCGCCGTTT ACGATGATTG TTGTCGGTGT
351 GTTGGTGTTT AAAGACCGGA TGACTGCCGC TCAGAAAATC GGCTTGGTTT
401 TGCTGCTTGC CGGTTTGCTT ATGTTTTTTA ACGATAAATT CGGCGAGTTG
451 TCGGGTTTGG GCGCGTATGC GAAGGGCGTG TTGCTGTGTG CGGCAGGCAG
501 TATGGCATGG GTGTGTTATG CCGTGGCGCA AAAGCTGCTG TCGGCGCAAT
551 TCGGGCCGCA ACAGATTCTG CTGTTGATTT ATGCGGCAAG TGCCGCCGTG
601 TTCCTGCCGT TTGCCGAACC GGCACACATC GGAAGTTTGG ACGGTACGTT
651 GGCGTGGGTT TGTTTTGCGT ATTGCTGCTT GAATACGTTA ATCGGTTACG
701 GCTCGTTCGG CGAGGCGTTG AAACATTGGG AGGCTTCCAA AGTCAGCGCG
751 GTAACAACCT TGCTCCCCGT GTTTACCGTA ATAwTwwCTT TGCTCGGGCA
801 TTATGTGATG CCTGAAACTT TTGCCGCGCC GGA...
```

This corresponds to the amino acid sequence <SEQ ID 402; ORF104-1>:

```
  1 MENQRPLLGF ALALLAAMTW GTLPIAVRQV LKFVDAPTLV WVRFTVAAAV
 51 LFVLLALGGR LPKRRDFSWC SFRLLLLGVA GISANFVLIA QGLHYISPTT
101 TQVLWQISPF TMIVVGVLVF KDRMTAAQKI GLVLLLAGLL MFFNDKFGEL
151 SGLGAYAKGV LLCAAGSMAW VCYAVAQKLL SAQFGPQQIL LLIYAASAAV
201 FLPFAEPAHI GSLDGTLAWV CFAYCCLNTL IGYGSFGEAL KHWEASKVSA
251 VTTLLPVFTV IXXLLGHYVM PETFAAP...
```

Computer analysis of this amino acid sequence gave the following results:

Homology with hypothetical HI0878 protein of *H. influenzae* (accession number U32769)

ORF104 and HI0878 show 40% aa identity in 277aa overlap:

```
orf104    4   QRPLLGFRLALLAAMTWGTLPXSVRQVLKFVDAPTLVWXXXXXXXXXXXXXXXXXXXXP-  62
              Q+PLLGF  AL+ AM WG+LP +++QVL  ++A T+VW                    P
HI0878    3   QQPLLGFTFALITAMAWGSLPIALKQVLSVMNAQTIVWYRFIIAAVSLLALLAYKKQLPE  62

orf104    63  --KRRDFSWCSFRLLLLGVAGISANFVLIAQGLHYISPTTTQVLWQISPFTMIVVGVLVF 120
                K R ++W    ++L+GV G+++NF+L +  L+YI P+  Q+    +S F M++ GVL+F
HI0878    63  LMKVRQYAW----IMLIGVIGLTSNFLLFSSSLNYIEPSVAQIFIHLSSFGMLICGVLIF 118

orf104    121 KDRMTAAQKIXXXXXXXXXXMYFNDKFGELSGLGAYXKGVLLCAAGSMAWVCNAVAQKLL 180
              K+++    QKI          ++FND+F    +GL  Y  GV+L    G++ WV   +AQKL+
HI0878    119 KEKLGLHQKIGLFLLLIGLGLFFNDRFDAFAGLNQYSTGVILGVGGALIWVAYGMAQKLM 178

orf104    181 SAQFGPQQILLLIYAASAAVFLPPFAEPAHIGSMDGTLAWVCIAYCCLNTLIGYGSFGEAL 240
                +F  QQILL++Y    A  F+P A+ + +   +    LA +C  YCCLNTLIGYGS+ EAL
HI0878    179 LRKFNSQQILLMMYLGCAIAFMPMADFSQVQELT-PLALICFIYCCLNTLIGYGSYAEAL 237

orf104    241 KHWEASKVSAVTTLLPVFTVINTLLGHYVMPETFAAP 277
                W+ SKVS V TL+P+FT++ + + HY   P  FAAP

HI0878    238 NRWDVSKVSVVITLVPLFTILFSHIAHYFSPADFAAP 274
```

Homology with a predicted ORF from *N.meningitidis* (strain A)

ORF104 shows 95.3% identity over a 277aa overlap with an ORF (ORF104a) from strain A of *N. meningitidis:*

```
                        10        20        30        40        50        60
orf104.pep  MENQRPLLGFRLALLAAMTWGTLPXSVRQVLKFVDAPTLVWVRFTVAAAVLFVLLALGGR
            |||||||||| ||||||||||||| :||||||||||||||||||||||||||||||||||
orf104a     MENQRPLLGFALALLAAMTWGTLPIAVRQVLKFVDAPTLVWVRFTVAAAVLFVLLALGGR
                        10        20        30        40        50        60

                        70        80        90       100       110       120
orf104.pep  LPKRRDFSWCSFRLLLLGVAGISANFVLIAQGLHYISPTTTQVLWQISPFTMIVVGVLVF
            ||| ||||||||||||||||||||||||||||||||||||||||||||||||||||||||
orf104a     LPKWRDFSWCSFRLLLLGVAGISANFVLIAQGLHYISPTTTQVLWQISPFTMIVVGVLVF
                        70        80        90       100       110       120

                       130       140       150       160       170       180
orf104.pep  KDRMTAAQKIGLVLLLAGLLMYFNDKFGELSGLGAYXKGVLLCAAGSMAWVCNAVAQKLL
            ||||||||||||||||||||:||||||||||||||| ||||||||||||||| |||||||
orf104a     KDRMTAAQKIGLVLLLAGLLMFFNDKFGELSGLGAYAKGVLLCAAGSMAWVCYAVAQKLL
                       130       140       150       160       170       180

                       190       200       210       220       230       240
orf104.pep  SAQFGPQQILLLIYAASAAVFLPPFAEPAHIGSMDGTLAWVCIAYCCLNTLIGYGSFGEAL
            ||||||||||||||||||||||||||| |||||:||||||||:||||||||||||||||
orf104a     SAQFGPQQILLLIYAASAAVFLPPFAELAHIGSLDGTLAWVCFAYCCLNTLIGYGSFGEAL
                       190       200       210       220       230       240

                       250       260       270
orf104.pep  KHWEASKVSAVTTLLPVFTVINTLLGHYVMPETFAAP
            |||||||||||||||||||||| :||||||||:|||||
orf104a     KHWEASKVSAVTTLLPVFTVIFSLLGHYVMPDTFAAPDMNGLGYAGALVVVGGAVTAAVG
                       250       260       270       280       290       300
```

The complete length ORF104a nucleotide sequence <SEQ ID 403> is:

```
  1 ATGGAAAACC AAAGGCCGCT CCTAGGCTTC GCGTTGGCAC TTTTGGCGGC
 51 GATGACGTGG GGAACGCTGC CGATTGCCGT GCGGCAGGTA TTGAAGTTTG
101 TCGATGCGCC GACGCTGGTG TGGGTGCGTT TTACCGTGGC GGCGGCGGTA
151 TTGTTTGTTT TGCTGGCATT GGGCGGGCGG CTGCCGAAGT GGCGGGATTT
201 TTCTTGGTGC TCATTCAGGC TGCTGCTGCT CGGCGTGGCG GGCATTTCGG
251 CAAACTTTGT GCTGATTGCC CAAGGGCTGC ATTATATTTC GCCGACCACG
301 ACGCAGGTTT TGTGGCAGAT TTCGCCGTTT ACGATGATTG TTGTCGGTGT
351 GTTGGTGTTT AAAGACCGGA TGACTGCCGC TCAGAAAATC GGCTTGGTTT
401 TGCTGCTTGC CGGTTTGCTT ATGTTTTTTA ACGATAAATT CGGCGAGTTG
451 TCGGGTTTGG GCGCGTATGC GAAGGGCGTG TTGCTGTGTG CGGCAGGCAG
501 TATGGCATGG GTGTGTTATG CCGTGGCGCA AAAGCTGCTG TCGGCGCAAT
551 TCGGGCCGCA ACAGATTCTG CTGTTGATTT ATGCGGCAAG TGCCGCCGTG
601 TTCCTGCCGT TTGCCGAACT GGCACACATC GGAAGTTTGG ACGGTACGTT
651 GGCGTGGGTT TGTTTTGCGT ATTGCTGCTT GAATACGTTA ATCGGTTACG
701 GCTCGTTCGG CGAGGCGTTG AAACATTGGG AGGCTTCCAA AGTCAGCGCG
751 GTAACAACCT TGCTCCCCGT GTTTACCGTA ATATTTTCTT TGCTCGGGCA
801 TTATGTGATG CCTGATACTT TTGCCGCGCC GGATATGAAC GGTTTGGGTT
851 ATGCCGGCGC ACTGGTCGTG GTCGGGGGTG CGGTTACGGC GGCGGTGGGG
901 GACAGGCTGT TCAAACGCCG CTAG
```

This encodes a protein having amino acid sequence <SEQ ID 404>:

```
  1 MENQRPLLGF ALALLAAMTW GTLPIAVRQV LKFVDAPTLV WVRFTVAAAV
 51 LFVLLALGGR LPKWRDFSWC SFRLLLLGVA GISANFVLIA QGLHYISPTT
101 TQVLWQISPF TMIVVGVLVF KDRMTAAQKI GLVLLLAGLL MFFNDKFGEL
151 SGLGAYAKGV LLCAAGSMAW VCYAVAQKLL SAQFGPQQIL LLIYAASAAV
201 FLPFAELAHI GSLDGTLAWV CFAYCCLNTL IGYGSFGEAL KHWEASKVSA
251 VTTLLPVFTV IFSLLGHYVM PDTFAAPDMN GLGYAGALVV VGGAVTAAVG
301 DRLFKRR*
```

ORF104a and ORF104-1 show 98.2% identity in 277 aa overlap:

```
                     10        20        30        40        50        60
orf104a.pep  MENQRPLLGFALALLAAMTWGTLPIAVRQVLKFVDAPTLVWVRFTVAAAVLFVLLALGGR
             ||||||||||||||||||||||||||||||||||||||||||||||||||||||||||||
orf104-1     MENQRPLLGFALALLAAMTWGTLPIAVRQVLKFVDAPTLVWVRFTVAAAVLFVLLALGGR
                     10        20        30        40        50        60

                     70        80        90       100       110       120
orf104a.pep  LPKWRDFSWCSFRLLLLGVAGISANFVLIAQGLHYISPTTTQVLWQISPFTMIVVGVLVF
             ||| ||||||||||||||||||||||||||||||||||||||||||||||||||||||||
orf104-1     LPKRRDFSWCSFRLLLLGVAGISANFVLIAQGLHYISPTTTQVLWQISPFTMIVVGVLVF
                     70        80        90       100       110       120

                    130       140       150       160       170       180
orf104a.pep  KDRMTAAQKIGLVLLLAGLLMFFNDKFGELSGLGAYAKGVLLCAAGSMAWVCYAVAQKLL
             ||||||||||||||||||||||||||||||||||||||||||||||||||||||||||||
orf104-1     KDRMTAAQKIGLVLLLAGLLMFFNDKFGELSGLGAYAKGVLLCAAGSMAWVCYAVAQKLL
                    130       140       150       160       170       180

                    190       200       210       220       230       240
orf104a.pep  SAQFGPQQILLLIYAASAAVFLPFAELAHIGSLDGTLAWVCFAYCCLNTLIGYGSFGEAL
             ||||||||||||||||||||||||||| ||||||||||||||||||||||||||||||||
orf104-1     SAQFGPQQILLLIYAASAAVFLPFAEPAHIGSLDGTLAWVCFAYCCLNTLIGYGSFGEAL
                    190       200       210       220       230       240

                    250       260       270       280       290       300
orf104a.pep  KHWEASKVSAVTTLLPVFTVIFSLLGHYVMPDTFAAPDMNGLGYAGALVVVGGAVTAAVG
             ||||||||||||||||||||||  ||||||||:||||
orf104-1     KHWEASKVSAVTTLLPVFTVIXXLLGHYVMPETFAAP
                    250       260       270
```

Homology with a predicted ORF from *N.gonorrhoeae*

ORF104 shows 93.9% identity over a 277aa overlap with a predicted ORF (ORF104.ng) from *N. gonorrhoeae:*

```
orf104.pep   MENQRPLLGFRLALLAAMTWGTLPXSVRQVLKFVDAPTLVWVRFTVAAAVLFVLLALGGR   60
             |||||||||| ||||||||||||| :||||||||||||||||||||||||||||||||||
orf104ng     MENQRPLLGFALALLAAMTWGTLPIAVRQVLKFVDAPTLVWVRFTVAAAVLFVLLALGGR   60

orf104.pep   LPKRRDFSWCSFRLLLLGVAGISANFVLIAQGLHYISPTTTQVLWQISPFTMIVVGVLVF  120
             ||||||||| |||||||||:||||||||||||||||||||||||||||||||||||||||
orf104ng     LPKRRDFSWHSFRLLLLGVTGISANFVLIAQGLHYISPTTTQVLWQISPFTMIVVGVLVF  120

orf104.pep   KDRMTAAQKIGLVLLLAGLLMYFNDKFGELSGLGAYXKGVLLCAAGSMAWVCNAVAQKLL  180
             ||||||||||||||||:||||:|||||||||||||| ||||||||||||||| |||||||
orf104ng     KDRMTAAQKIGLVLLLVGLLMFFNDKFGELSGLGAYAKGVLLCAAGSMAWVCYAVAQKLL  180

orf104.pep   SAQFGPQQILLLIYAASAAVFLPFAEPAHIGSMDGTLAWVCIAYCCLNTLIGYGSFGEAL  240
             ||||||||||||||||||||||  ||||||||:||||||||::|||||||||||||||||
orf104ng     SAQFGPQQILLLIYAASAAVFLLXAEPAHIGSLDGTLAWVCFVYCCLNTLIGYGSFGEAL  240

orf104.pep   KHWEASKVSAVTTLLPVFTVINTLLGHYVMPETFAAP                        277
             ||||||||||||||||||||| :||||||||:|||||
orf104ng     KHWEASKVSAVTTLLPVFTVIFSLLGHYVMPDTFAAPDMNGLGYVGALVVVGGAVTAAVG  300
```

The complete length ORF104ng nucleotide sequence <SEQ ID 405> is predicted to encode a protein having amino acid sequence <SEQ ID 406>:

```
  1 MENQRPLLGF ALALLAAMTW GTLPIAVRQV LKFVDAPTLV WVRFTVAAAV
 51 LFVLLALGGR LPKRRDFSWH SFRLLLLGVT GISANFVLIA QGLHYISPTT
101 TQVLWQISPF TMIVVGVLVF KDRMTAAQKI GLVLLLVGLL MFFNDKFGEL
151 SGLGAYAKGV LLCAAGSMAW VCYAVAQKLL SAQFGPQQIL LLIYAASAAV
201 FLLXAEPAHI GSLDGTLAWV CFVYCCLNTL IGYGSFGEAL KHWEASKVSA
251 VTTLLPVFTV IFSLLGHYVM PDTFAAPDMN GLGYVGALVV VGGAVTAAVG
301 DRPFKRR*
```

Further work revealed the complete gonococcal nucleotide sequence <SEQ ID 407>:

```
  1 ATGGAAAACC AAAGGCCGCT CCTAGGCTTC GCGTTGGCAC TTTTGGCGGC
 51 GATGACGTGG GGGACGCTGC CGATTGCCGT GCGGCAGGTA TTGAAGTTTG
101 TCGATGCGCC GACGCTGGTG TGGGTGCGTT TTACCGTGGC GGCGGCGGTA
151 TTGTTTGTTT TGCTGGCATT GGGCGGGCGG CTGCCGAAGC GGCGGGATTT
201 TTCTTGGCAT TCATTCAGGC TGCTGCTGCT CGGCGTGACG GGCATTTCGG
251 CAAACTTTGT GCTGATTGCC CAAGGGCTGC ATTATATTTC GCCGACCACG
301 ACGCAGGTTT TGTGGCAGAT TTCGCCGTTT ACGATGATTG TTGTCGGCGT
351 GTTGGTGTTT AAAGACCGGA tgaCTGCCGC GCAGAAAATC GGTTTGGTTT
401 TGCTGCttgT CGGTttgCTT ATGTTTTtta ACGACAAATT CGGCGAGTTG
451 TCGGGTTTGG GCGCGTATGC GAAGGGCGTG TTGCTGTGTG CGGCAGGCAG
501 TATGGCCTGG GTGTGTTATG CCGTGGCGCA AAAGCTGCTG TCGGCGCAAT
551 TCGGGCCGCA ACAGATTCTG CTGTTGATTT ATGCGGcaag tgccgccGTG
601 TTCCtgccgT TTGccgaaCC GGCACACATC GGAAGTTTgg aCGGTACGtt
651 GGCGTGGGTT TGTTTTGTGT ATTGCTGCTT GAATACGTTA ATCGGTTACG
701 GCTCGTTCGG CGAGGCGTTG AAACATTGGG AGGCTTCCAA AGTCAGCGCG
751 GTAACAACCT TGCTCCCCGT GTTTACCGTA ATATTTTCTT TGCTCGGGCA
801 TTATGTGATG CCTGATACTT TTGCCGCGCC GGATATGAAC GGTTTGGGTT
851 ATGTCGGCGC ACTGGTCGTG GTCGGGGGTG CGGTTACGGC GGCGGTGGGG
901 GACAGGCCGT TCAAACGCCG CTAG
```

This corresponds to the amino acid sequence <SEQ ID 408; ORF104ng-1>:

```
  1  MENQRPLLGF ALALLAAMTW GTLPIAVRQV LKFVDAPTLV WVRFTVAAAV
 51  LFVLLALGGR LPKRRDFSWH SFRLLLLGVT GISANFVLIA QGLHYISPTT
101  TQVLWQISPF TMIVVGVLVF KDRMTAAQKI GLVLLLVGLL MFFNDKFGEL
151  SGLGAYAKGV LLCAAGSMAW VCYAVAQKLL SAQFGPQQIL LLIYAASAAV
201  FLPFAEPAHI GSLDGTLAWV CFVYCCLNTL IGYGSFGEAL KHWEASKVSA
251  VTTLLPVFTV IFSLLGHYVM PDTFAAPDMN GLGYVGALVV VGGAVTAAVG
301  DRPFKRR*
```

ORF104ng-1 and ORF104-1 show 97.5% identity in 277 aa overlap:

```
                        10        20        30        40        50        60
orf104-1.pep    MENQRPLLGFALALLAAMTWGTLPIAVRQVLKFVDAPTLVWVRFTVAAAVLFVLLALGGR
                ||||||||||||||||||||||||||||||||||||||||||||||||||||||||||||
orf104ng-1      MENQRPLLGFALALLAAMTWGTLPIAVRQVLKFVDAPTLVWVRFTVAAAVLFVLLALGGR
                        10        20        30        40        50        60

                        70        80        90       100       110       120
orf104-1.pep    LPKRRDFSWCSFRLLLLGVAGISANFVLIAQGLHYISPTTTQVLWQISPFTMIVVGVLVF
                ||||||||| ||||||||:|||||||||||||||||||||||||||||||||||||||||
orf104ng-1      LPKRRDFSWHSFRLLLLGVTGISANFVLIAQGLHYISPTTTQVLWQISPFTMIVVGVLVF
                        70        80        90       100       110       120

                       130       140       150       160       170       180
orf104-1.pep    KDRMTAAQKIGLVLLLAGLLMFFNDKFGELSGLGAYAKGVLLCAAGSMAWVCYAVAQKLL
                ||||||||||||||||:|||||||||||||||||||||||||||||||||||||||||||
orf104ng-1      KDRMTAAQKIGLVLLLVGLLMFFNDKFGELSGLGAYAKGVLLCAAGSMAWVCYAVAQKLL
                       130       140       150       160       170       180

                       190       200       210       220       230       240
orf104-1.pep    SAQFGPQQILLLIYAASAAVFLPFAEPAHIGSLDGTLAWVCFAYCCLNTLIGYGSFGEAL
                ||||||||||||||||||||||||||||||||||||||||||:|||||||||||||||||
orf104ng-1      SAQFGPQQILLLIYAASAAVFLPFAEPAHIGSLDGTLAWVCFVYCCLNTLIGYGSFGEAL
                     190       200       210       220       230       240

                       250       260       270
orf104-1.pep    KHWEASKVSAVTTLLPVFTVIXXLLGHYVMPETFAAP
                |||||||||||||||||||||  ||||||||:|||||
orf104ng-1      KHWEASKVSAVTTLLPVFTVIFSLLGHYVMPDTFAAPDMNGLGYVGALVVVGGAVTAAVG
                       250       260       270       280       290       300
```

In addition, ORF104ng-1 shows significant homology with a hypothetical *H.influenzae* protein:

```
gi|1573895 (U32769) hypothetical [Haemophilus influenzae] Length = 306
 Score =  237 bits (598), Expect = 8e-62
 Identities = 114/280 (40%), Positives = 168/280 (59%), Gaps = 8/280 (2%)

Query: 30  QRPXXXXXXXXXXXMTWGTLPIAVRQVLKFVDAPTLVWXXXXXXXXXXXXXXXXXXXXP- 88
            Q+P           M WG+LPIA++QVL   ++A T+VW                    P
Sbjct: 3   QQPLLGFTFALITAMAWGSLPIALKQVLSVMNAQTIVWYRFIIAAVSLLALLAYKKQLPE 62
```

```
Query: 89  --KRRDFSWHSFRLLLLGVTGISANFVLIAQGLHYISPTTTQVLWQISPFTMIVVGVLVF 146
             K R ++W    ++L+GV G+++NF+L +   L+YI P+  Q+   +S F M++ GVL+F
Sbjct: 63  LMKVRQYAW----IMLIGVIGLTSNFLLFSSSLNYIEPSVAQIFIHLSSFGMLICGVLIF 118

Query: 147 KDRMTAAQKIXXXXXXXXXXMFFNDKFGELSGLGAYAKGVLLCAAGSMAWVCYAVAQKLL 206
            K+++   QKI          +FFND+F    +GL  Y+ GV+L   G++ WV Y +AQKL+
Sbjct: 119 KEKLGLHQKIGLFLLLIGLGLFFNDRFDAFAGLNQYSTGVILGVGGALIWVAYGMAQKLM 178

Query: 207 SAQFGPQQILLLIYAASAAVFLPPFAEPAHIGSLDGTLAWVCFVYCCLNTLIGYGSFGEAL 266
             +F  QQILL++Y   A  F+P A+ + +  L    LA +CF+YCCLNTLIGYGS+ EAL
Sbjct: 179 LRKFNSQQILLMMYLGCAIAFMPMADFSQVQELT-PLALICFIYCCLNTLIGYGSYAEAL 237

Query: 267 KHWEASKVSAVTTLLPVFTVIFSLLGHYVMPDTFAAPDMN 306
             W+ SKVS V TL+P+FT++FS + HY  P  FAAP++N
Sbjct: 238 NRWDVSKVSVVITLVPLFTILFSHIAHYFSPADFAAPELN 277
```

Based on this analysis, including the presence of a putative leader sequence and several putative transmembrane domains in the gonococcal protein, it is predicted that the proteins from *N.meningitidis* and *N.gonorrhoeae,* and their epitopes, could be useful antigens for vaccines or diagnostics, or for raising antibodies.

**Example 48**

The following partial DNA sequence was identified in *N.meningitidis* <SEQ ID 409>:

```
  1 ATGGTAGCTC GTCGGGCTCA TAACCCGAAG GTCGTAGGTT CGAATCCTGT
 51 .CCCGCAACC TAATTTCAAA CCCCTCGGTT CAATGCCGAG GG.GTTTTGT
101 T.TTGCCTGT TTCCTGTTTC CTGTTTCCTG CCGCCTCCGT TTTTTGCCGG
151 ATTTTCCTTC CGGCCGCAAT ATCGGAACGG CAGACCGCCG TCTGTTTGCG
201 GTTGCAAATT CAGGCAGTTT GGCTACAATC TTCCGCATTG TCTTCAAGAA
251 AGCCAACCAT GCCGACCGTC CGTTTTACCG AATCCGTCAG CAAACAAGAC
301 CTTGATGCTC TGTTCGAGTG GGCAAAAGCA AGTTACGGTG CAGAAAGTTG
351 CTGGAAAACG CTGTATCTGA ACGGTCysCC TTTGGGCAAC CTGTCGCCGG
401 AATGGGTGGA ACGCGTsmmA AAAGACTGGG AGGCAGGCTG CyCGGAGTCT
451 TCAGACGGCA TTTTTCTGAA TgCGGACGGc TGgCctGATA TGGgCGGAcg
501 cTTACAGCAC CTCGCCCTCG GTTGGCACTG TGCGGGGCTG TTGGACGgsT
551 GGCGCAACGA GTGTTTCGAC CTGACCGACG GCGGCGGCAA CCCCTTGTTC
601 ACGCTCGaAc GCGCCGyTTT mCGTCCTkTC GGACTGCTCA GCCGCGCCGT
651 CCATCTCAAC GGTCTGACCG AATCGGACGG CCGATGGCAT TTCTGGATAG
701 GCAGGCGCAG TCCGCACAAA GCAGTCGATC CCAACAAACT CGACAATACT
751 rCCGCCGGCG GTGTTTCCGG CGGCGAAATG CCGTCTGAAG CCGTGTGTCG
801 CGAAAGCAGC GAAGAAGCCG GTTTGGATAA AACGCTGcTT CCGCTCATCC
851 GCCCGGTATC GCAGCTGCAC AGCCTGCGCT CCGTCAGCCG GGGTGTACAC
901 AATGAAATCC TGTATGTATT CGATGCCGTC CTGCCG...
```

This corresponds to the amino acid sequence <SEQ ID 410; ORF105>:

```
  1 MVARRAHNPK VVGSNPXPAT XFQTPRFNAE XVLXLPVSCF LFPAASVFCR
 51 IFLPAAISER QTAVCLRLQI QAVWLQSSAL SSRKPTMPTV RFTESVSKQD
101 LDALFEWAKA SYGAESCWKT LYLNGXPLGN LSPEWVERVX KDWEAGCXES
151 SDGIFLNADG WPDMGGRLQH LALGWHCAGL LDGWRNECFD LTDGGGNPLF
201 TLERAXXRPX GLLSRAVHLN GLTESDGRWH FWIGRRSPHK AVDPNKLDNT
251 XAGGVSGGEM PSEAVCRESS EEAGLDKTLL PLIRPVSQLH SLRSVSRGVH
301 NEILYVFDAV LP...
```

Further work revealed the complete nucleotide sequence <SEQ ID 411>:

```
  1 ATGCCGACCG TCCGTTTTAC CGAATCCGTC AGCAAACAAG ACCTTGATGC
 51 TCTGTTCGAG TGGGCAAAAG CAAGTTACGG TGCAGAAAGT TGCTGGAAAA
101 CGCTGTATCT GAACGGTCTG CCTTTGGGCA ACCTGTCGCC GGAATGGGTG
151 GAACGCGTCA AAAAAGACTG GGAGGCAGGC TGCTCGGAGT CTTCAGACGG
201 CATTTTTCTG AATGCGGACG GCTGGCCTGA TATGGGCGGA CGCTTACAGC
251 ACCTCGCCCT CGGTTGGCAC TGTGCGGGGC TGTTGGACGG CTGGCGCAAC
301 GAGTGTTTCG ACCTGACCGA CGGCGGCGGC AACCCCTTGT TCACGCTCGA
351 ACGCGCCGCT TTCCGTCCTT TCGGACTGCT CAGCCGCGCC GTCCATCTCA
```

```
401 ACGGTCTGAC CGAATCGGAC GGCCGATGGC ATTTCTGGAT AGGCAGGCGC
451 AGTCCGCACA AAGCAGTCGA TCCCAACAAA CTCGACAATA CTGCCGCCGG
501 CGGTGTTTCC GGCGGCGAAA TGCCGTCTGA AGCCGTGTGT CGCGAAAGCA
551 GCGAAGAAGC CGGTTTGGAT AAAACGCTGC TTCCGCTCAT CCGCCCGGTA
601 TCGCAGCTGC ACAGCCTGCG CTCCGTCAGC CGGGGTGTAC ACAATGAAAT
651 CCTGTATGTA TTCGATGCCG TCCTGCCCGA AACCTTCCTG CCTGAAAATC
701 AGGATGGCGA AGTGGCGGGT TTTGAGAAAA TGGACATCGG CGGTCTGTTG
751 GATGCCATGT TGTCGGGAAA CATGATGCAC GACGCGCAAC TGGTTACGCT
801 GGACGCGTTT TGCCGTTACG GTCTGATTGA TGCCGCCCAT CCGCTGTCCG
851 AGTGGCTGGA CGGCATACGT TTATAG
```

This corresponds to the amino acid sequence <SEQ ID 412; ORF105-1>:

```
  1 MPTVRFTESV SKQDLDALFE WAKASYGAES CWKTLYLNGL PLGNLSPEWV
 51 ERVKKDWEAG CSESSDGIFL NADGWPDMGG RLQHLALGWH CAGLLDGWRN
101 ECFDLTDGGG NPLFTLERAA FRPFGLLSRA VHLNGLTESD GRWHFWIGRR
151 SPHKAVDPNK LDNTAAGGVS GGEMPSEAVC RESSEEAGLD KTLLPLIRPV
201 SQLHSLRSVS RGVHNEILYV FDAVLPETFL PENQDGEVAG FEKMDIGGLL
251 DAMLSGNMMH DAQLVTLDAF CRYGLIDAAH PLSEWLDGIR L*
```

Computer analysis of this amino acid sequence gave the following results:

Homology with a predicted ORF from *N.meningitidis* (strain A)

ORF105 shows 89.4% identity over a 226aa overlap with an ORF (ORF105a) from strain A of *N. meningitidis:*

```
                60        70        80        90       100       110
orf105.pep    ISERQTAVCLRLQIQAVWLQSSALSSRKPTMPTVRFTESVSKQDLDALFEWAKASYGAES
                                              |||||||||||:|||||||||||||||||
orf105a                                       MPTVRFTESVSKHDLDALFEWAKASYGAES
                                                      10        20        30

               120       130       140       150       160       170
orf105.pep    CWKTLYLNGXPLGNLSPEWVERVXKDWEAGCXESSDGIFLNADGWPDMGGRLQHLALGWH
              ||||||||| |||||||||:||| ||||||| |||||||||||||||| ||||||  |:
orf105a       CWKTLYLNGLPLGNLSPEWAERVKKDWEAGCSESSDGIFLNADGWPDMGRRLQHLARIWK
                      40        50        60        70        80        90

               180       190       200       210       220       230
orf105.pep    CAGLLDGWRNECFDLTDGGGNPLFTLERAXXRPXGLLSRAVHLNGLTESDGRWHFWIGRR
              |||| |||:|||||||||:||||:||||  || |||||||||||:|||||||||||||
orf105a       EAGLLHGWRDECFDLTDGGSNPLFALERAAFRPFGLLSRAVHLNGLVESDGRWHFWIGRR
                     100       110       120       130       140       150

               240       250       260       270       280       290
orf105.pep    SPHKAVDPNKLDNTXAGGVSGGEMPSEAVCRESSEEAGLDKTLLPLIRPVSQLHSLRSVS
              |||||||:||||| |||||:||:|||:||||||||||||||||||||||||||||| ||
orf105a       SPHKAVDPDKLDNTAAGGVSSGELPSETVCRESSEEAGLDKTLLPLIRPVSQLHSLRPVS
                     160       170       180       190       200       210

               300       310
orf105.pep    RGVHNEILYVFDAVLP
              ||||||||||||||||
orf105a       RGVHNEILYVFDAVLPETFLPENQDGEVAGFEKMDIGGLLAAMLSGNMMHDAQLVTLDAF
                     220       230       240       250       260       270
```

The complete length ORF105a nucleotide sequence <SEQ ID 413> is:

```
  1  ATGCCGACCG TCCGTTTTAC CGAATCCGTC AGCAAACACG ACCTTGATGC
 51  CCTATTCGAG TGGGCAAAGG CAAGTTACGG TGCGGAAAGT TGCTGGAAAA
101  CGCTGTATCT GAACGGTCTG CCTTTGGGCA ATCTGTCGCC GGAATGGGCG
151  GAGCGCGTCA AAAAAGACTG GGAGGCAGGC TGCTCGGAGT CTTCAGACGG
201  CATTTTCCTG AATGCGGACG GCTGGCCAGA TATGGGCAGA CGCTTGCAGC
251  ACCTCGCCCG AATATGGAAA GAAGCGGGAC TGCTTCACGG CTGGCGCGAC
301  GAGTGTTTCG ACCTGACCGA CGGCGGCAGC AATCCCTTGT TCGCGCTCGA
351  ACGCGCCGCT TTCCGTCCGT TCGGACTGCT CAGCCGCGCC GTCCATCTCA
401  ACGGTTTGGT CGAATCGGAC GGCCGATGGC ATTTCTGGAT AGGCAGGCGC
451  AGTCCGCACA AAGCAGTCGA TCCCGACAAA CTCGACAATA CTGCCGCCGG
501  CGGTGTTTCC AGCGGTGAAT TGCCGTCTGA AACCGTGTGT CGCGAAAGCA

551  GCGAAGAAGC CGGTTTGGAT AAAACGCTGC TTCCGCTCAT CCGCCCGGTA
601  TCGCAGCTGC ACAGCCTGCG CCCCGTCAGC CGGGGTGTGC ACAATGAAAT
651  CCTGTATGTA TTCGATGCCG TCCTGCCCGA AACCTTCCTG CCTGAAAATC
701  AGGATGGCGA AGTGGCGGGT TTTGAGAAAA TGGACATCGG CGGTCTGTTG
751  GCTGCCATGT TGTCGGGAAA CATGATGCAC GACGCGCAAC TGGTTACGCT
801  GGACGCGTTT TGCCGTTACG GTCTGATTGA TGCCGCCCAT CCGCTGTCCG
851  AGTGGCTGGA CGGCATACGT TTATAG
```

This encodes a protein having amino acid sequence <SEQ ID 414>:

```
  1  MPTVRFTESV SKHDLDALFE WAKASYGAES CWKTLYLNGL PLGNLSPEWA
 51  ERVKKDWEAG CSESSDGIFL NADGWPDMGR RLQHLARIWK EAGLLHGWRD
101  ECFDLTDGGS NPLFALERAA FRPFGLLSRA VHLNGLVESD GRWHFWIGRR
151  SPHKAVDPDK LDNTAAGGVS SGELPSETVC RESSEEAGLD KTLLPLIRPV
201  SQLHSLRPVS RGVHNEILYV FDAVLPETFL PENQDGEVAG FEKMDIGGLL
251  AAMLSGNMMH DAQLVTLDAF CRYGLIDAAH PLSEWLDGIR L*
```

ORF105a and ORF105-1 show 93.8% identity in 291 aa overlap:

```
                          10        20        30        40        50        60
orf105a.pep       MPTVRFTESVSKHDLDALFEWAKASYGAESCWKTLYLNGLPLGNLSPEWAERVKKDWEAG
                  ||||||||||||:||||||||||||||||||||||||||||||||||||:||||||||||
orf105-1          MPTVRFTESVSKQDLDALFEWAKASYGAESCWKTLYLNGLPLGNLSPEWVERVKKDWEAG
                          10        20        30        40        50        60

                          70        80        90       100       110       120
orf105a.pep       CSESSDGIFLNADGWPDMGRRLQHLARIWKEAGLLHGWRDECFDLTDGGSNPLFALERAA
                  ||||||||||||||||||| ||||||  |: |||| |||:|||||||||:||||:|||||
orf105-1          CSESSDGIFLNADGWPDMGGRLQHLALGWHCAGLLDGWRNECFDLTDGGGNPLFTLERAA
                          70        80        90       100       110       120

                         130       140       150       160       170       180
orf105a.pep       FRPFGLLSRAVHLNGLVESDGRWHFWIGRRSPHKAVDPDKLDNTAAGGVSSGELPSETVC
                  ||||||||||||||||:|||||||||||||||||||||:|||||||||||:||:|||:||
orf105-1          FRPFGLLSRAVHLNGLTESDGRWHFWIGRRSPHKAVDPNKLDNTAAGGVSGGEMPSEAVC
                         130       140       150       160       170       180

                         190       200       210       220       230       240
orf105a.pep       RESSEEAGLDKTLLPLIRPVSQLHSLRPVSRGVHNEILYVFDAVLPETFLPENQDGEVAG
                  ||||||||||||||||||||||||||| ||||||||||||||||||||||||||||||||
orf105-1          RESSEEAGLDKTLLPLIRPVSQLHSLRSVSRGVHNEILYVFDAVLPETFLPENQDGEVAG
                         190       200       210       220       230       240

                         250       260       270       280       290
orf105a.pep       FEKMDIGGLLAAMLSGNMMHDAQLVTLDAFCRYGLIDAAHPLSEWLDGIRLX
                  |||||||||| |||||||||||||||||||||||||||||||||||||||||
orf105-1          FEKMDIGGLLDAMLSGNMMHDAQLVTLDAFCRYGLIDAAHPLSEWLDGIRLX
                         250       260       270       280       290
```

.

<u>Homology with a predicted ORF from *N.gonorrhoeae*</u>

ORF105 shows 87.5% identity over a 312aa overlap with a predicted ORF (ORF105.ng) from *N. gonorrhoeae:*

```
orf105.pep    MVARRAHNPKVVGSNPXPATXFQTPRFNAEXVLXLPVSCFLFPAASVFCRIFLPAAISER   60
              |||||||||||||||| ||| :|||||||| ||      |||||||||||||||||||||
orf105ng      MVARRAHNPKVVGSNPAPATKYQTPRFNAEGVLF-----FLFPAASVFCRIFLPAAISER   55

orf105.pep    QTAVCLRLQIQAVWLQSSALSSRKPTMPTVRFTESVSKQDLDALFEWAKASYGAESCWKT  120
              |:|||||||||||||||||| ||||:|||||||||||||||||||| |||||||||||||
orf105ng      QAAVCLRLQIQAVWLQSSALCSRKPAMPTVRFTESVSKQDLDALFERAKASYGAESCWKT  115

orf105.pep    LYLNGXPLGNLSPEWVERVXKDWEAGCXESSDGIFLNADGWPDMGGRLQHLALGWHCAGL  180
              ||||  |||||||||:||: ||||||| |||:||||||||||||||||||||  |: |||
orf105ng      LYLNRLPLGNLSPEWAERIKKDWEAGCSESSNGIFLNADGWPDMGGRLQHLARTWNKAGL  175

orf105.pep    LDGWRNECFDLTDGGGNPLFTLERAXXRPXGLLSRAVHLNGLTESDGRWHFWIGRRSPHK  240
              | |||||||||||||||||||||||  || ||| ||||||||:||:||||||||||||||
orf105ng      LHGWRNECFDLTDGGGNPLFTLERAAFRPFGLLIRAVHLNGLVESNGRWHFWIGRRSPHK  235
```

```
orf105.pep   AVDPNKLDNTXAGGVSGGEMPSEAVCRESSEEAGLDKTLLPLIRPVSQLHSLRSVSRGVH  300
             ||||:||||  :|||||||||||||||||||||||||:|||||||:||||| ||||||
orf105ng     AVDPGKLDNIAGGGVSGGEMPSEAVCRESSEEAGLDKTLFPLIRPVSRLHSLRPVSRGVH  295

orf105.pep   NEILYVFDAVLP                                                 312
             ||||||||||||
orf105ng     NEILYVFDAVLPETFLPENQDGEVAGFEKMDIGGLLDAMLSKNMMHDAQLVTLDAFYRYG  355
```

A complete length ORF105ng nucleotide sequence <SEQ ID 415> was predicted to encode a protein having amino acid sequence <SEQ ID 416>:

```
  1  MVARRAHNPK VVGSNPAPAT KYQTPRFNAE GVLFFLFPAA SVFCRIFLPA
 51  AISERQAAVC LRLQIQAVWL QSSALCSRKP AMPTVRFTES VSKQDLDALF
101  ERAKASYGAE SCWKTLYLNR LPLGNLSPEW AERIKKDWEA GCSESSNGIF
151  LNADGWPDMG GRLQHLARTW NKAGLLHGWR NECFDLTDGG GNPLFTLERA
201  AFRPFGLLIR AVHLNGLVES NGRWHFWIGR RSPHKAVDPG KLDNIAGGGV
251  SGGEMPSEAV CRESSEEAGL DKTLFPLIRP VSRLHSLRPV SRGVHNEILY
301  VFDAVLPETF LPENQDGEVA GFEKMDIGGL LDAMLSKNMM HDAQLVTLDA
351  FYRYGLIDAA HPLSEWLDGI RL*
```

Further work revealed the complete nucleotide sequence <SEQ ID 417>:

```
  1  ATGCCGACCG TCCGTTTTAC CGAATCCGTC AGCAAACAAG ACCTTGATGC
 51  CCTGTTCGAG CGGGCAAAAG CAAGTTACGG TGCCGAAAGT TGCTGGAAAA
101  CGCTGTATCT GAACCGTCTT CCTTTGGGCA ATCTGTCGCC GGAATGGGCT
151  GAGCGCATCA AAAAAGACTG GGAGGCAGGC TGCTCCGAGT CTTCAGACGG
201  CATTTTTCTG AATGCGGACG GCTGGCCGGA TATGGGCGGA CGCTTGCAGC
251  ACCTCGCCCG CACATGGAAC AAGGCGGGGC TGCTTCACGG ATGGCGCAAC
301  GAGTGTTTCG ACCTGACCGA CGGCGGCGGC AACCCCTTGT TCACGCTCGA
351  ACGCGCCGCT TTCCGTCCGT TCGGACTACT CAGCCGCGCC GTCCATCTCA
401  ACGGTTTGGT CGAATCGAAC GGCAGATGGC ATTTTTGGAT AGGCAGGCGC
451  AGTCCGCACA AAGCAGTCGa tcCCGGCAAG CTCGACAATA TTGCCGGCGG
501  CGGTGTTTCC GGCGGCGAAA TGCCGTCTGA AGCCGTGTGC CGCGAAAGCA
551  GCGAAGAAGC CGGTTTGGAT AAAACGCTGT TTCCGCTCAT CCGCCCAGTA
601  TCGCGGCTGC ACAGCCTTCG CCCCGTCAGC CGAGGTGTGC ACAATGAAAT
651  CCTGTATGTG TTCGATGCCG TCCTGCCCGA AACCTTCCTG CCTGAAAATC
701  AGGATGGCGA GGTAGCGGGT TTTGAAAAGA TGGACATTGG CGGCCTATTG
751  GATGCCATGT TGTCGAAAAA CATGATGCAC GACGCGCAAC TGGTTACGCT
801  GGACGCGTTT TACCGTTACG GTCTGATTGA TGCCGCCCAT CCGCTGTCCG
851  AGTGGCTGGA CGGCATACGT TTATAG
```

This corresponds to the amino acid sequence <SEQ ID 418; ORF105ng-1>:

```
  1  MPTVRFTESV SKQDLDALFE RAKASYGAES CWKTLYLNRL PLGNLSPEWA
 51  ERIKKDWEAG CSESSDGIFL NADGWPDMGG RLQHLARTWN KAGLLHGWRN
101  ECFDLTDGGG NPLFTLERAA FRPFGLLSRA VHLNGLVESN GRWHFWIGRR
151  SPHKAVDPGK LDNIAGGGVS GGEMPSEAVC RESSEEAGLD KTLFPLIRPV
201  SRLHSLRPVS RGVHNEILYV FDAVLPETFL PENQDGEVAG FEKMDIGGLL
251  DAMLSKNMMH DAQLVTLDAF YRYGLIDAAH PLSEWLDGIR L*
```

ORG105ng-1 and ORF105-1 show 93.5% identity in 291 aa overlap:

```
                         10        20        30        40        50        60
orf105-1.pep     MPTVRFTESVSKQDLDALFEWAKASYGAESCWKTLYLNGLPLGNLSPEWVERVKKDWEAG
                 |||||||||||||||||||| |||||||||||||||||| ||||||||||:||:||||||
orf105ng-1       MPTVRFTESVSKQDLDALFERAKASYGAESCWKTLYLNRLPLGNLSPEWAERIKKDWEAG
                         10        20        30        40        50        60

                         70        80        90       100       110       120
orf105-1.pep     CSESSDGIFLNADGWPDMGGRLQHLALGWHCAGLLDGWRNECFDLTDGGGNPLFTLERAA
                 ||||||||||||||||||||||||||  |: |||| ||||||||||||||||||||||||
orf105ng-1       CSESSDGIFLNADGWPDMGGRLQHLARTWNKAGLLHGWRNECFDLTDGGGNPLFTLERAA
                         70        80        90       100       110       120

                        130       140       150       160       170       180
orf105-1.pep     FRPFGLLSRAVHLNGLTESDGRWHFWIGRRSPHKAVDPNKLDNTAAGGVSGGEMPSEAVC
                 ||||||||||||||||:||:|||||||||||||||||||:|||| |:|||||||||||||
orf105ng-1       FRPFGLLSRAVHLNGLVESNGRWHFWIGRRSPHKAVDPGKLDNIAGGGVSGGEMPSEAVC
                        130       140       150       160       170       180
```

```
                        190       200       210       220       230       240
orf105-1.pep     RESSEEAGLDKTLLPLIRPVSQLHSLRSVSRGVHNEILYVFDAVLPETFLPENQDGEVAG
                 |||||||||||||:|||||||:||||| ||||||||||||||||||||||||||||||||
orf105ng-1       RESSEEAGLDKTLFPLIRPVSRLHSLRPVSRGVHNEILYVFDAVLPETFLPENQDGEVAG
                        190       200       210       220       230       240

                        250       260       270       280       290
orf105-1.pep     FEKMDIGGLLDAMLSGNMMHDAQLVTLDAFCRYGLIDAAHPLSEWLDGIRLX
                 ||||||||||||||| |||||||||||||| |||||||||||||||||||||
orf105ng-1       FEKMDIGGLLDAMLSKNMMHDAQLVTLDAFYRYGLIDAAHPLSEWLDGIRLX
                        250       260       270       280       290
```

Furthermore, ORF105ng-1 shows homology with a yeast enzyme:

```
sp|P41888|TNR3_SCHPO THIAMIN PYROPHOSPHOKINASE (TPK) (THIAMIN KINASE)
>gi|1076928|pir||S52350 thiamin pyrophosphokinase (EC 2.7.6.2) - fission yeast
(Schizosaccharomyces pombe) >gi|666111 (X84417) thiamin pyrophosphokinase
[Schizosaccharomyces pombe] >gi|2330852|gnl|PID|e334056  (Z98533) thiamin
pyrophosphokinase [Schizosaccharomyces pombe] Length = 569
 Score =  105 bits (259), Expect = 4e-22
 Identities = 64/192 (33%), Positives = 94/192 (48%), Gaps = 3/192 (1%)

Query: 268 NKAGLLHGWRNECFDLTDGGGNPLFTLERAAFRPFGLLSRAVHLNGLVESNGRW--HFWI 441
          N  G+    WRNE + +        P+  +ER   F  FG LS   VH     + +       W+
Sbjct: 96  NTFGIADQWRNELYTVYGKSKKPVLAVERGGFWLFGFLSTGVHCTMYIPATKEHPLRIWV 155

Query: 442 GRRSPHKAVDPGKLDNIAGGGVSGGEMPSEAVCRESSEEAGLDKTLFPLIRPVSRLHSLR 621
           RRSP K    P  LDN    GG++ G+        + +E  SEEA LD +     LI P    +  ++
Sbjct: 156 PRRSPTKQTWPNYLDNSVAGGIAHGDSVIGTMIKEFSEEANLDVSSMNLI-PCGTVSYIK 214

Query: 622 PVSRG-VHNEILYVFDAVLPETFLPENQDGEVAGFEKMDIGGLLDAMLSKNMMHDAQLVT 798
              R   +  E+ YVFD  + +  +P    DGEVAGF  + +  +L  +  K+   +  LV
Sbjct: 215 MEKRHWIQPELQYVFDLPVDDLVIPRINDGEVAGFSLLPLNQVLHELELKSFKPNCALVL 274

Query: 799 LDAFYRYGLIDAAHP 843
          LD   R+G+I    HP
Sbjct: 275 LDFLIRHGIITPQHP 289
```

Based on this analysis, including the presence of a putative transmembrane domain in the gonococcal protein, it is predicted that the proteins from *N.meningitidis* and *N.gonorrhoeae,* and their epitopes, could be useful antigens for vaccines or diagnostics, or for raising antibodies.

### Example 49

The following DNA sequence, believed to be complete, was identified in *N. meningitidis* <SEQ ID 419>:

```
  1  ATGAATAGAC CCAAGCAACC CTTCTTCCGT CCCGAAGTCG CCGTTGCCCG
 51  CCAAACCAGC CTGACGGGTA AAGTGATTCT GACACGACCG TTGTCATTTT
101  CCCTATGGAC GACATTTGCA TCGATATCTG CGTTATTGAT TATCCTGTTT
151  TTGATATTTG GTAACTATAC GCGAAAGACA ACAGTGGAGG GACAAATTTT
201  ACCTGCATCG GGCGTAATCA GGGTGTATGC ACCGgATACG rGkACAATTA
251  CAGCGAAATT CGTGGAAGAT GGmsAAAAGG TTAAGGCTGG CGACAAGCTA
301  TTTGCGCTTT CGACCTCACG TTTCGGCGCA GGAGGTAGCG TGCAGCAGCA
351  GTTGAAAACG GAGGCAGTTT TGAAGAAAAC GTTGGCAGAA CAGGAACTGG
401  GTCGTCTGAA GCTGATACAC GGGAATGAAA CGCGCAgCcT TAAAGCAACT
451  GTCGAACGTT TGGAAAACCA GGAACTCCAT ATTTCGCAAC AGATAGACGG
501  TCAGAAAAGG CGCATTAGAC TTGCGGAAGA AATGTTGCAG AAATATCGTT
551  TCCTATCCGC .CAATGA
```

This corresponds to the amino acid sequence <SEQ ID 420; ORF107>:

```
  1  MNRPKQPFFR PEVAVARQTS LTGKVILTRP LSFSLWTTFA SISALLIILF
 51  LIFGNYTRKT TVEGQILPAS GVIRVYAPDT XTITAKFVED GXKVKAGDKL
101  FALSTSRFGA GGSVQQQLKT EAVLKKTLAE QELGRLKLIH GNETRSLKAT
151  VERLENQELH ISQQIDGQKR RIRLAEEMLQ KYRFLSXQ*
```

Computer analysis of this amino acid sequence gave the following results:

<u>Homology with a predicted ORF from *N.meningitidis* (strain A)</u>

ORF107 shows 97.8% identity over a 186aa overlap with an ORF (ORF107a) from strain A of *N. meningitidis:*

```
                      10        20        30        40        50        60
orf107.pep    MNRPKQPFFRPEVAVARQTSLTGKVILTRPLSFSLWTTFASISALLIILFLIFGNYTRKT
              ||||||||||||||||||||||||||||||||||||||||||||||||||||||||||||
orf107a       MNRPKQPFFRPEVAVARQTSLTGKVILTRPLSFSLWTTFASISALLIILFLIFGNYTRKT
                      10        20        30        40        50        60

                      70        80        90       100       110       120
orf107.pep    TVEGQILPASGVIRVYAPDTXTITAKFVEDGXKVKAGDKLFALSTSRFGAGGSVQQQLKT
              |||||||||||||||||||| |||||| ||| |||||||||||||||||| ||||||||
orf107a       TVEGQILPASGVIRVYAPDTGTITAKFXEDGEKVKAGDKLFALSTSRFGAGDSVQQQLKT
                      70        80        90       100       110       120

                     130       140       150       160       170       180
orf107.pep    EAVLKKTLAEQELGRLKLIHGNETRSLKATVERLENQELHISQQIDGQKRRIRLAEEMLQ
              ||||||||||||||||||||||||||||||||||||||||||||||||||||||||||||
orf107a       EAVLKKTLAEQELGRLKLIHGNETRSLKATVERLENQELHISQQIDGQKRRIRLAEEMLQ
                     130       140       150       160       170       180

                     189
orf107.pep    KYRFLSXQX
              ||||||
orf107a       KYRFLSANDAVPKQEMMNVKAELLEQKAKLDAYRREEVGLLQEIRTQNLTLXSLPQAAX
                     190       200       210       220       230
```

The complete length ORF107a nucleotide sequence <SEQ ID 421> is:

```
  1  ATGAATAGAC CCAAGCAACC NTTCTTCCGT CCCGAAGTCG CCGTTGCCCG
 51  CCAAACCAGC CTGACGGGTA AAGTGATTCT GACACGACCG TTGTCATTTT
101  CCCTATGGAC GACATTTGCA TCGATATCTG CGTTATTGAT TATCCTGTTT
151  TTGATATTTG GTAACTATAC GCGAAAGACA ACAGTGGAGG GACAAATTTT
201  ACCTGCATCG GGCGTAATCA GGGTGTATGC ACCGGATACG GGGACAATTA
251  CNGCGAAATT CNTGGAAGAT GGAGAAAAGG TTAAGGCTGG CGACAAGCTA
301  TTTGCGCTTT CGACCTCACG TTTCGGCGCA GGAGATAGCG TGCAGCAGCA
351  GTTGAAAACG GAGGCAGTTT TGAAGAAAAC GTTGGCAGAA CAGGAACTGG
401  GTCGTCTGAA GCTGATACAC GGGAATGAAA CGCGCAGCCT TAAAGCAACT
451  GTCGAACGTT TGGAAAACCA GGAACTCCAT ATTTCGCAAC AGATAGACGG
501  TCAGAAAAGG CGCATTAGAC TTGCGGAAGA AATGTTGCAG AAATATCGTT
551  TCCTATCCGC CAATGATGCA GTGCCAAAAC AAGAAATGAT GAATGTCAAG
601  GCAGAGCTTT TAGAGCAGAA AGCCAAACTT GATGCCTACC GCCGAGAAGA
651  AGTCGGGCTG CTTCAGGAAA TCCGCACGCA GAATCTGACA TTGGNNAGCC
701  TCCCCCAAGC GGCATGA
```

This encodes a protein having amino acid sequence <SEQ ID 422>:

```
  1  MNRPKQPFFR PEVAVARQTS LTGKVILTRP LSFSLWTTFA SISALLIILF
 51  LIFGNYTRKT TVEGQILPAS GVIRVYAPDT GTITAKFXED GEKVKAGDKL
101  FALSTSRFGA GDSVQQQLKT EAVLKKTLAE QELGRLKLIH GNETRSLKAT
151  VERLENQELH ISQQIDGQKR RIRLAEEMLQ KYRFLSANDA VPKQEMMNVK
201  AELLEQKAKL DAYRREEVGL LQEIRTQNLT LXSLPQAA*
```

<u>Homology with a predicted ORF from *N.gonorrhoeae*</u>

ORF107 shows 95.7% identity over a 188aa overlap with a predicted ORF (ORF107.ng) from *N. gonorrhoeae:*

```
orf107.pep   MNRPKQPFFRPEVAVARQTSLTGKVILTRPLSFSLWTTFASISALLIILFLIFGNYTRKT   60
             |||||||||||||:||||||||||||||||||||||||||||||||||||||||||||||
orf107ng     MNRPKQPFFRPEVAIARQTSLTGKVILTRPLSFSLWTTFASISALLIILFLIFGNYTRKT   60

orf107.pep   TVEGQILPASGVIRVYAPDTXTITAKFVEDGXKVKAGDKLFALSTSRFGAGGSVQQQLKT  120
             |:|||||||||||||||||| |||||||||| ||||||||||||||||||||||||||||
orf107ng     TMEGQILPASGVIRVYAPDTGTITAKFVEDGEKVKAGDKLFALSTSRFGAGGSVQQQLKT  120

orf107.pep   EAVLKKTLAEQELGRLKLIHGNETRSLKATVERLENQELHISQQIDGQKRRIRLAEEMLQ  180
             ||||||||||||||||||| ||||||||||||||||:|||||||||||||||||||||:
orf107ng     EAVLKKTLAEQELGRLKLIHENETRSLKATVERLENQKLHISQQIDGQKRRIRLAEEMLR  180

orf107.pep   KYRFLSXQ  188
             |||||| |
orf107ng     KYRFLSAQ  188
```

The complete length ORF107ng nucleotide sequence <SEQ ID 423> is predicted to encode a protein having amino acid sequence <SEQ ID 424>:

```
  1  MNRPKQPFFR PEVAIARQTS LTGKVILTRP LSFSLWTTFA SISALLIILF
 51  LIFGNYTRKT TMEGQILPAS GVIRVYAPDT GTITAKFVED GEKVKAGDKL
101  FALSTSRFGA GGSVQQQLKT EAVLKKTLAE QELGRLKLIH ENETRSLKAT
151  VERLENQKLH ISQQIDGQKR RIRLAEEMLR KYRFLSAQ*
```

Based on the presence of a putative ransmembrane domain in the gonococcal protein, it is predicted that the proteins from *N.meningitidis* and *N.gonorrhoeae,* and their epitopes, could be useful antigens for vaccines or diagnostics, or for raising antibodies.

**Example 50**

The following DNA sequence, believed to be complete, was identified in *N.meningitidis* <SEQ ID 425>:

```
  1  ATGCTGAATA CTTTTTTTGC CGTATTGGGC GGCTGCCTGC TGCT.TTGCC
 51  GTGCGGCAAA TCCGTAAATA CGGCGGTACA GCCGCAAAAC GCGGTACAAA
101  GCGCGCCGAA ACCGGTTTTC AAAGTCATAT ATATCGACAA TACGGCGATT
151  GCCGGTTTGG ATTTGGGACA AAGCAGCGAA GGCAAAACCA ACGACGGCAA
201  AAAACAAATC AGTTATCCGA TTAAAGGCTT GCCGGAACAA AATGTTATCC
251  GACTGATCGG CAAGCATCCC GGCGACTTGG AAGCCGTCAG CGGCAAATGT
301  ATGGAAACCG ATGATAAGGA CAGTCCGGCA GGTTGGGCAG AAAACGGCGT
351  GTGCCATACC TTGTTTGCCA AACTGGTGGG CAATATCGCC GAAGACGGCG
401  GCAAACTGAC GGATTACCTA GTTTCGCATG CCGCCCTGCA ACCCTATCAG
451  GCAGGCAAAA GCGGCTATGC CGCCGTGCAG AACGGACGCT ATGTGCTGGA
501  AATCGACAGC GAAGGGGCGT TTTATTTCCG CCGCCGCCAT TATTGA
```

This corresponds to the amino acid sequence <SEQ ID 426; ORF108>:

```
  1  MLNTFFAVLG GCLLXLPCGK SVNTAVQPQN AVQSAPKPVF KVIYIDNTAI
 51  AGLDLGQSSE GKTNDGKKQI SYPIKGLPEQ NVIRLIGKHP GDLEAVSGKC
101  METDDKDSPA GWAENGVCHT LFAKLVGNIA EDGGKLTDYL VSHAALQPYQ
151  AGKSGYAAVQ NGRYVLEIDS EGAFYFRRRH Y*
```

Further work revealed the following DNA sequence <SEQ ID 427>:

```
  1  ATGCTGAAAA CATCTTTTGC CGTATTGGGC GGCTGCCTGC TGCTTGCCGC
 51  CTGCGGCAAA TCCGAAAATA CGGCGGAACA GCCGCAAAAC GCGGTACAAA
101  GCGCGCCGAA ACCGGTTTTC AAAGTCAAAT ATATCGACAA TACGGCGATT
151  GCCGGTTTGG ATTTGGGACA AAGCAGCGAA GGCAAAACCA ACGACGGCAA
201  AAAACAAATC AGTTATCCGA TTAAAGGCTT GCCGGAACAA AATGTTATCC
251  GACTGATCGG CAAGCATCCC GGCGACTTGG AAGCCGTCAG CGGCAAATGT
301  ATGGAAACCG ATGATAAGGA CAGTCCGGCA GGTTGGGCAG AAAACGGCGT
351  GTGCCATACC TTGTTTGCCA AACTGGTGGG CAATATCGCC GAAGACGGCG
401  GCAAACTGAC GGATTACCTA GTTTCGCATG CCGCCCTGCA ACCCTATCAG
451  GCAGGCAAAA GCGGCTATGC CGCCGTGCAG AACGGACGCT ATGTGCTGGA
501  AATCGACAGC GAAGGGGCGT TTTATTTCCG CCGCCGCCAT TATTGA
```

This corresponds to the amino acid sequence <SEQ ID 428; ORF108-1>:

```
  1  MLKTSFAVLG GCLLLAACGK SENTAEQPQN AVQSAPKPVF KVKYIDNTAI
 51  AGLDLGQSSE GKTNDGKKQI SYPIKGLPEQ NVIRLIGKHP GDLEAVSGKC
101  METDDKDSPA GWAENGVCHT LFAKLVGNIA EDGGKLTDYL VSHAALQPYQ
151  AGKSGYAAVQ NGRYVLEIDS EGAFYFRRRH Y*
```

Computer analysis of this amino acid sequence gave the following results:

Homology with a predicted ORF from *N.gonorrhoeae*

ORF108 shows 88.4% identity over a 181aa overlap with a predicted ORF (ORF108.ng) from *N. gonorrhoeae:*

```
orf108.pep    MLNTFFAVLGGCLLXLPCGKSVNTAVQPQNAVQSAPKPVFKVIYIDNTAIAGLDLGQSSE   60
              ||:  ||||||||    |||| ||| |||||:|||||||||| |||||||||| ||||||
orf108ng      MLKIPFAVLGGCLLLAACGKSENTAEQPQNAAQSAPKPVFKVKYIDNTAIAGLALGQSSE   60

orf108.pep    GKTNDGKKQISYPIKGLPEQNVIRLIGKHPGDLEAVSGKCMETDDKDSPAGWAENGVCHT  120
              |||||||||||||||||||||::|| ||||:||||| ||||||| ||:|:||||||||||
orf108ng      GKTNDGKKQISYPIKGLPEQNAVRLTGKHPNDLEAVVGKCMETDGKDAPSGWAENGVCHT  120

orf108.pep    LFAKLVGNIAEDGGKLTDYLVSHAALQPYQAGKSGYAAVQNGRYVLEIDSEGAFYFRRRHY  181
              ||||||||||||||||||||:||:|||||||||||||||||||||||||||||||||||||
orf108ng      LFAKLVGNIAEDGGKLTDYLISHSALQPYQAGKSGYAAVQNGRYVLEIDSEGAFYFRRRHY  181
```

ORF108-1 shows 92.3% identity with ORF108ng over the same 181 aa overlap:

```
orf108-1.pep  MLKTSFAVLGGCLLLAACGKSENTAEQPQNAVQSAPKPVFKVKYIDNTAIAGLDLGQSSE   60
              |||  |||||||||||||||||||||||||||:|||||||||||||||||||||| ||||||
orf108ng-1    MLKIPFAVLGGCLLLAACGKSENTAEQPQNAAQSAPKPVFKVKYIDNTAIAGLALGQSSE   60

orf108-1.pep  GKTNDGKKQISYPIKGLPEQNVIRLIGKHPGDLEAVSGKCMETDDKDSPAGWAENGVCHT  120
              |||||||||||||||||||||::|| ||||:||||| ||||||| ||:|:||||||||||
orf108ng-1    GKTNDGKKQISYPIKGLPEQNAVRLTGKHPNDLEAVVGKCMETDGKDAPSGWAENGVCHT  120

orf108-1.pep  LFAKLVGNIAEDGGKLTDYLVSHAALQPYQAGKSGYAAVQNGRYVLEIDSEGAFYFRRRHY  181
              ||||||||||||||||||||:||:|||||||||||||||||||||||||||||||||||||
orf108ng-1    LFAKLVGNIAEDGGKLTDYLISHSALQPYQAGKSGYAAVQNGRYVLEIDSEGAFYFRRRHY  181
```

The complete length ORF108ng nucleotide sequence <SEQ ID 429> is:

```
  1  ATGCTGAAAa tacctTTTGC CGTGTtgggc ggCtgcctGC TGCTTGCCGC
 51  CTGCGGCAAA TCCGAAAATa cggcggaACA GCCGCAAAAT gcggCACAAA
101  GCGCGCCGAA ACCGGTTTTC AAAGTCAAAT ACATCGACAA TACGGCGATT
151  GCCGGTTTGG CTTTGGGACA AAGTAGCGAA GGCAAAACCA acgacgGCAA
201  AAAACAAATC AGTTATccgA TTAAAGGCTT GCCGGAACAA Aacgccgtcc
251  gGCTGACCGG AAAGCATCCC AACGACTTGG AagccgtcgT CGGCAAATGT
301  ATGGAAACCG ACGGAAAGGA CGCGCCTTCG GGCTGGGCGG AAAACGGCGT
351  GTGCCATACC TTGTTTGCCA AACTGGTGGG CAATATCGCC GAAGACGGCG
401  GCAAACTGAC TGATTACCTG ATTTCGCATT CCGCCCTGCA ACCCTATCAG
451  GCAGGCAAAA GCGGCTATGC CGCCGTGCAG AACGGACGCT ATGTGCTGGA
501  AATCGACAGC GagggGGCGT TTTATttccg ccgccgccat tattgA
```

This encodes a protein having amino acid sequence <SEQ ID 430>:

```
  1  MLKIPFAVLG GCLLLAACGK SENTAEQPQN AAQSAPKPVF KVKYIDNTAI
 51  AGLALGQSSE GKTNDGKKQI SYPIKGLPEQ NAVRLTGKHP NDLEAVVGKC
101  METDGKDAPS GWAENGVCHT LFAKLVGNIA EDGGKLTDYL ISHSALQPYQ
151  AGKSGYAAVQ NGRYVLEIDS EGAFYFRRRH Y*
```

Based on this analysis, including the presence of a predicted prokaryotic membrane lipoprotein lipid attachment site (underlined) and a putative ATP/GTP-binding site motif A (P-loop, double-underlined) in the gonococcal protein, it is predicted that the proteins from *N.meningitidis* and *N.gonorrhoeae,* and their epitopes, could be useful antigens for vaccines or diagnostics, or for raising antibodies.

**Example 51**

The following DNA sequence was identified in *N.meningitidis* <SEQ ID 431>:

```
  1 ATGGAAGATT TATATATAAT ACTCGCTTTG GGTTTGGTTG CGATGATTGC
 51 CGgATTTATC GATgcgatTg cGggcGGGGG TGGTTTGATT ACGCTGCCCG
101 CACTCTTGTT GGCAGGTATT CCTCCCGTGT CGGCAATTGC CACCAACAAG
151 CTGCAAgCAG CCGCTGCTAC GTTTTCAGCT ACGGTTTCTT TTGCACGCAA
201 AGGTTTGATT GATTGGAAGA AAGGTCTCCC GATTGCCGCA GCATCGTTTG
251 TAGGCGGCGT GGcCGGTGCA TTATCGGTCA GCTTGGTTTC CAAAGATATT
301 CTgCTgGCGG TCGTGCCGGT TTTGTTGATA TTTGTCGCAC TGTATTTTGT
351 GTTTTCGCCC AAGCTCGACG GCAGTAAGGA AGGCAAAGCC AGAATGTCTT
401 TTTTTCTGTT cGGGCTGACG GTCGC.ACCG CTTTTGGGTT TTTACGACGG
451 TGTGTTCGGA CCGGGTGTCG GCTCGTTTTT TCTGATTGCC TTTATTGTTT
501 TGCTCGGCTG CAAgCTGTTG AACGCGATGT CTTACACCAA ATTGGCGAAC
551 GTTGCCTGCA ATCTTGGTTC GCTATCGGTA TTCCTGCTGC ACGGTTCGAT
601 TATTTTCCCG ATTGCGGCAA CGaTGGCGGT CGGTGCGTTT GTCGGtGCGA
651 ATTTAgGTGC GAGATTTGCC GTaCgctTCG GTTCGAAGCT GATTAA
```

This corresponds to the amino acid sequence <SEQ ID 432; ORF109>:

```
  1 MEDLYIILAL GLVAMIAGFI DAIAGGGGLI TLPALLLAGI PPVSAIATNK
 51 LQAAAATFSA TVSFARKGLI DWKKGLPIAA ASFVGGVAGA LSVSLVSKDI
101 LLAVVPVLLI FVALYFVFSP KLDGSKEGKA RMSFFLFGLT VXTAFGFLRR
151 CVRTGCRLVF SDCLYCFARL QAVERDVLHQ IGERCLQSWF AIGIPAARFD
201 YFPDCGNDGG RCVCRCEFRC EICRTLRFEA D*
```

Further work revealed the following DNA sequence <SEQ ID 433>:

```
  1 ATGGAAGATT TATATATAAT ACTCGCTTTG GGTTTGGTTG CGATGATTGC
 51 CGGATTTATC GATGCGATTG CGGGCGGGGG TGGTTTGATT ACGCTGCCCG
101 CACTCTTGTT GGCAGGTATT CCTCCCGTGT CGGCAATTGC CACCAACAAG
151 CTGCAAGCAG CCGCTGCTAC GTTTTCAGCT ACGGTTTCTT TTGCACGCAA
201 AGGTTTGATT GATTGGAAGA AAGGTCTCCC GATTGCCGCA GCATCGTTTG
251 TAGGCGGCGT GGCCGGTGCA TTATCGGTCA GCTTGGTTTC CAAAGATATT
301 CTGCTGGCGG TCGTGCCGGT TTTGTTGATA TTTGTCGCAC TGTATTTTGT
351 GTTTTCGCCC AAGCTCGACG GCAGTAAGGA AGGCAAAGCC AGAATGTCTT
401 TTTTTCTGTT CGGGCTGACG GTCGCACCGC TTTTGGGTTT TTACGACGGT
451 GTGTTCGGAC CGGGTGTCGG CTCGTTTTTT CTGATTGCCT TTATTGTTTT
501 GCTCGGCTGC AAGCTGTTGA ACGCGATGTC TTACACCAAA TTGGCGAACG
551 TTGCCTGCAA TCTTGGTTCG CTATCGGTAT TCCTGCTGCA CGGTTCGATT
601 ATTTTCCCGA TTGCGGCAAC GATGGCGGTC GGTGCGTTTG TCGGTGCGAA
651 TTTAGGTGCG AGATTTGCCG TCCGCTTCGG TTCGAAGCTG ATTAAGCCGC
701 TGCTGATTGT CATCAGCATT TCGATGGCTG TGAAATTGTT GATAGACGAG
751 AGAAATCCGC TGTATCAGAT GATTGTTTCG ATGTTTTAA
```

This corresponds to the amino acid sequence <SEQ ID 434; ORF109-1>:

```
  1 MEDLYIILAL GLVAMIAGFI DAIAGGGGLI TLPALLLAGI PPVSAIATNK
 51 LQAAAATFSA TVSFARKGLI DWKKGLPIAA ASFVGGVAGA LSVSLVSKDI
101 LLAVVPVLLI FVALYFVFSP KLDGSKEGKA RMSFFLFGLT VAPLLGFYDG
151 VFGPGVGSFF LIAFIVLLGC KLLNAMSYTK LANVACNLGS LSVFLLHGSI
201 IFPIAATMAV GAFVGANLGA RFAVRFGSKL IKPLLIVISI SMAVKLLIDE
251 RNPLYQMIVS MF*
```

Computer analysis of this amino acid sequence gave the following results:

Homology with a predicted ORF from *N.meningitidis* (strain A)

ORF 109 shows 95.9% identity over a 147aa overlap with an ORF (ORF109a) from strain A of *N. meningitidis:*

```
                      10        20        30        40        50        60
orf109.pep  MEDLYIILALGLVAMIAGFIDAIAGGGGLITLPALLLAGIPPVSAIATNKLQAAAATFSA
            ||||||||||||||||||||||||||||||||||||||||||||||||||||||||||||
orf109a     MEDLYIILALGLVAMIAGFIDAIAGGGGLITLPALLLAGIPPVSAIATNKLQAAAATFSA
                      10        20        30        40        50        60
```

```
                      70        80        90       100       110       120
orf109.pep  TVSFARKGLIDWKKGLPIAAASFVGGVAGALSVSLVSKDILLAVVPVLLIFVALYFVFSP
            |||||||||||||||||||||||:|||:||||||||||||||||||||||||||||||||
orf109a     TVSFARKGLIDWKKGLPIAAASFAGGVVGALSVSLVSKDILLAVVPVLLIFVALYFVFSP
                      70        80        90       100       110       120

                     130       140       150       160       170       180
orf109.pep  KLDGSKEGKARMSFFLFGLTVXTAFGFLRRCVRTGCRLVFSDCLYCFARLQAVERDVLHQ
            |||||||||||||||||||||   :||
orf109a     KLDGSKEGKARMSFFLFGLTVAPLLGFYDGVFGPGVGSFFLIAFIVLLGCKLLNAMSYTK
                     130       140       150       160       170       180
```

The complete length ORF109a nucleotide sequence <SEQ ID 435> is:

```
  1 ATGGAAGATT TATACATAAT ACTCGCTTTG GGTTTGGTTG CGATGATTGC
 51 CGGATTTATC GATGCGATTG CGGGTGGGGG TGGTTTGATT ACGCTGCCTG
101 CACTCTTGTT GGCAGGTATT CCTCCCGTGT CGGCAATTGC CACCAACAAG
151 CTGCAAGCAG CCGCTGCTAC GTTTTCGGCT ACGGTTTCTT TTGCACGCAA
201 AGGTTTGATT GATTGGAAGA AAGGTCTCCC GATTGCGGCA GCATCGTTTG
251 CAGGCGGCGT GGTCGGTGCA TTATCGGTCA GCTTGGTTTC CAAAGATATT
301 CTGCTGGCGG TCGTGCCGGT TTTGTTGATA TTTGTCGCGC TGTATTTTGT
351 GTTTTCGCCC AAGCTCGACG GCAGTAAGGA AGGCAAAGCC AGAATGTCTT
401 TTTTTCTGTT CGGTCTGACG GTTGCACCAC TTTTGGGTTT TTACGACGGT
451 GTGTTCGGAC CGGGTGTCGG CTCGTTTTTT CTGATTGCCT TTATTGTTTT
501 GCTCGGCTGC AAGCTGTTGA ACGCGATGTC TTACACCAAA TTGGCGAACG
551 TTGCCTGCAA TCTTGGTTCG CTATCGGTAT TCCTGCTGCA CGGTTCGATT
601 ATTTTCCCGA TTGCGGCAAC GATGGCGGTC GGTGCGTTTG TCGGTGCGAA
651 TTTAGGTGCG AGATTTGCCG TCCGCTTCGG TTCGAAGCTG ATTAAGCCGC
701 TGCTGATTGT CATCAGCATT TCGATGGCTG TGAAATTGTT GATAGACGAG
751 AGAAATCCGC TGTATCAGAT GATTGTTTCG ATGTTTTAA
```

This encodes a protein having amino acid sequence <SEQ ID 436>:

```
  1 MEDLYIILAL GLVAMIAGFI DAIAGGGGLI TLPALLLAGI PPVSAIATNK
 51 LQAAAATFSA TVSFARKGLI DWKKGLPIAA ASFAGGVVGA LSVSLVSKDI
101 LLAVVPVLLI FVALYFVFSP KLDGSKEGKA RMSFFLFGLT VAPLLGFYDG
151 VFGPGVGSFF LIAFIVLLGC KLLNAMSYTK LANVACNLGS LSVFLLHGSI
201 IFPIAATMAV GAFVGANLGA RFAVRFGSKL IKPLLIVISI SMAVKLLIDE
251 RNPLYQMIVS MF*
```

ORF109a and ORF109-1 show 99.2% identity in 262 aa overlap:

```
                     10        20        30        40        50        60
orf109a.pep   MEDLYIILALGLVAMIAGFIDAIAGGGGLITLPALLLAGIPPVSAIATNKLQAAAATFSA
              ||||||||||||||||||||||||||||||||||||||||||||||||||||||||||||
orf109-1      MEDLYIILALGLVAMIAGFIDAIAGGGGLITLPALLLAGIPPVSAIATNKLQAAAATFSA
                     10        20        30        40        50        60

                     70        80        90       100       110       120
orf109a.pep   TVSFARKGLIDWKKGLPIAAASFAGGVVGALSVSLVSKDILLAVVPVLLIFVALYFVFSP
              ||||||||||||||||||||||:|||:|||||||||||||||||||||||||||||||||
orf109-1      TVSFARKGLIDWKKGLPIAAASFVGGVAGALSVSLVSKDILLAVVPVLLIFVALYFVFSP
                     70        80        90       100       110       120

                    130       140       150       160       170       180
orf109a.pep   KLDGSKEGKARMSFFLFGLTVAPLLGFYDGVFGPGVGSFFLIAFIVLLGCKLLNAMSYTK
              ||||||||||||||||||||||||||||||||||||||||||||||||||||||||||||
orf109-1      KLDGSKEGKARMSFFLFGLTVAPLLGFYDGVFGPGVGSFFLIAFIVLLGCKLLNAMSYTK
                    130       140       150       160       170       180

                    190       200       210       220       230       240
orf109a.pep   LANVACNLGSLSVFLLHGSIIFPIAATMAVGAFVGANLGARFAVRFGSKLIKPLLIVISI
              ||||||||||||||||||||||||||||||||||||||||||||||||||||||||||||
orf109-1      LANVACNLGSLSVFLLHGSIIFPIAATMAVGAFVGANLGARFAVRFGSKLIKPLLIVISI
                    190       200       210       220       230       240

                    250       260
orf109a.pep   SMAVKLLIDERNPLYQMIVSMFX
              |||||||||||||||||||||||
orf109-1      SMAVKLLIDERNPLYQMIVSMFX
                    250       260
```

Homology with a predicted ORF from *N.gonorrhoeae*

ORF109 shows 98.3% identity over a 231 aa overlap with a predicted ORF (ORF109.ng) from *N. gonorrhoeae:*

```
orf109.pep   MEDLYIILALGLVAMIAGFIDAIAGGGGLITLPALLLAGIPPVSAIATNKLQAAAATFSA   60
             ||||||||||||||||||||||||||||||||||||||||||||||||||||||||||||
orf109ng     MEDLYIILALGLVAMIAGFIDAIAGGGGLITLPALLLAGIPPVSAIATNKLQAAAATFSA   60

orf109.pep   TVSFARKGLIDWKKGLPIAAASFVGGVAGALSVSLVSKDILLAVVPVLLIFVALYFVFSP  120
             ||||||||||||||||||||||:|||:|||||||||||||||||||||||||||||||||
orf109ng     TVSFARKGLIDWKKGLPIAAASFAGGVVGALSVSLVSKDILLAVVPVLLIFVALYFVFSP  120

orf109.pep   KLDGSKEGKARMSFFLFGLTVXTAFGFLRRCVRTGCRLVFSDCLYCFARLQAVERDVLHQ  180
             |||||||||||||||||||| |||||||||||||||||||||||||||||||||||||||
orf109ng     KLDGSKEGKARMSFFLFGLTVATAFGFLRRCVRTGCRLVFSDCLYCFARLQAVERDVLHQ  180

orf109.pep   IGERCLQSWFAIGIPAARFDYFPDCGNDGGRCVCRCEFRCEICRTLRFEAD  231
             |||||||||||||||||||||||||||||||||||||||||||| ||||||
orf109ng     IGERCLQSWFAIGIPAARFDYFPDCGNDGGRCVCRCEFRCEICRPLRFEAD  231
```

An ORF109ng nucleotide sequence <SEQ ID 437> was predicted to encode a protein having amino acid sequence <SEQ ID 438>:

```
  1  MEDLYIILAL GLVAMIAGFI DAIAGGGGLI TLPALLLAGI PPVSAIATNK
 51  LQAAAATFSA TVSFARKGLI DWKKGLPIAA ASFAGGVVGA LSVSLVSKDI
101  LLAVVPVLLI FVALYFVFSP KLDGSKEGKA RMSFFLFGLT VATAFGFLRR
151  CVRTGCRLVF SDCLYCFARL QAVERDVLHQ IGERCLQSWF AIGIPAARFD
201  YFPDCGNDGG RCVCRCEFRC EICRPLRFEA D*
```

Further work revealed the following gonococcal DNA sequence <SEQ ID 439>:

```
  1 ATGGAAGATT TATACATAAT ACTCGCTTTG GGTTTGGTTG CGATGATCGC
 51 CGGATTTATC GATGCGATTG CGGGCGGGGG TGGTTTGATT ACGCTGCCTG
101 CACTCTTGTT GGCAGGTATT CCTCCCGTGT CGGCAATTGC CACCAACAAG
151 CTGCAAGCAG CCGCTGCTAC GTTTTCGGCT ACGGTTTCTT TTGCACGCAA
201 AGGTTTGATT GATTGGAAGA AAGGTCTCCC GATTGCCGCA GCATCGTTTG
251 CAGGCGGCGT GGTCGGTGCA TTATCGGTCA GCTTGGTTTC CAAAGATATT
301 TTGCTGGCGG TCGTGCCGGT TTTGTTGATA TTTGTCGCGC TGTATTTTGT
351 GTTTTCGCCC AAGCTCGACG GCAGTAAGGA AGGCAAAGCC AGAATGTCTT
401 TTTTCTATT CGGGCTGACG GTTGCACCGC TTTTGGGTTT TTACGACGGT
451 GTGTTCGGAC CGGGTGTCGG CTCGTTTTTT CTGATTGCCT TTATTGTTTT
501 GCTCGGCTGC AAGCTGTTGA ACGCGATGTC TTACACCAAA TTGGCGAACG
551 TTGCTTGCAA TCTTGGTTCG CTATCGGTAT TCCTGCTGCA CGGTTCGATT
601 ATTTTCCCGA TTGTGGCAAC GATGGCGGTC GGTGCGTTTG TCGGTGCGAA
651 TTTAGGTGCG AGATTTGCCG TCCGCTTCGG TTCGAAGCTG ATTAAGCCGC
701 TGCTGATTGT CATCAGCATT TCGATGGCTG TGAAATTGTT GATAGACGAG
751 AGAAATCCGC TGTATCAGAT GATTGTTTCG ATGTTTTAA
```

This corresponds to the amino acid sequence <SEQ ID 440; ORF109ng-1>:

```
  1 MEDLYIILAL GLVAMIAGFI DAIAGGGGLI TLPALLLAGI PPVSAIATNK
 51 LQAAAATFSA TVSFARKGLI DWKKGLPIAA ASFAGGVVGA LSVSLVSKDI
101 LLAVVPVLLI FVALYFVFSP KLDGSKEGKA RMSFFLFGLT VAPLLGFYDG
151 VFGPGVGSFF LIAFIVLLGC KLLNAMSYTK LANVACNLGS LSVFLLHGSI
201 IFPIVATMAV GAFVGANLGA RFAVRFGSKL IKPLLIVISI SMAVKLLIDE
251 RNPLYQMIVS MF*
```

ORF109ng-1 and ORF109-1 show 98.9% identity in 262 aa overlap:

```
                           10        20        30        40        50        60
orf109ng-1.pep MEDLYIILALGLVAMIAGFIDAIAGGGGLITLPALLLAGIPPVSAIATNKLQAAAATFSA
               ||||||||||||||||||||||||||||||||||||||||||||||||||||||||||||
orf109-1       MEDLYIILALGLVAMIAGFIDAIAGGGGLITLPALLLAGIPPVSAIATNKLQAAAATFSA
                           10        20        30        40        50        60

                           70        80        90       100       110       120


orf109ng-1.pep TVSFARKGLIDWKKGLPIAAASFAGGVVGALSVSLVSKDILLAVVPVLLIFVALYFVFSP
               |||||||||||||||||||||:|||:||||||||||||||||||||||||||||||||||
orf109-1       TVSFARKGLIDWKKGLPIAAASFVGGVAGALSVSLVSKDILLAVVPVLLIFVALYFVFSP
                           70        80        90       100       110       120

                          130       140       150       160       170       180
orf109ng-1.pep KLDGSKEGKARMSFFLFGLTVAPLLGFYDGVFGPGVGSFFLIAFIVLLGCKLLNAMSYTK
               ||||||||||||||||||||||||||||||||||||||||||||||||||||||||||||
orf109-1       KLDGSKEGKARMSFFLFGLTVAPLLGFYDGVFGPGVGSFFLIAFIVLLGCKLLNAMSYTK
                          130       140       150       160       170       180

                          190       200       210       220       230       240
orf109ng-1.pep LANVACNLGSLSVFLLHGSIIFPIVATMAVGAFVGANLGARFAVRFGSKLIKPLLIVISI
               ||||||||||||||||||||||||:|||||||||||||||||||||||||||||||||||
orf109-1       LANVACNLGSLSVFLLHGSIIFPIAATMAVGAFVGANLGARFAVRFGSKLIKPLLIVISI
                          190       200       210       220       230       240

                          250       260
orf109ng-1.pep SMAVKLLIDERNPLYQMIVSMFX
               |||||||||||||||||||||||
orf109-1       SMAVKLLIDERNPLYQMIVSMFX
                          250       260
```

In addition, ORF109ng-1 shows homology to a hypothetical *Pseudomonas* protein:

```
sp|P29942|YCB9_PSEDE HYPOTHETICAL 27.4 KD PROTEIN IN COBO 3'REGION (ORF9)
>gi|94984|pir||I38164 hypothetical protein 9 - Pseudomonas sp >gi|551929
(M62866) ORF9 [Pseudomonas denitrificans] Length = 261
 Score =  175 bits (439), Expect = 3e-43
 Identities = 83/214 (38%), Positives = 131/214 (60%), Gaps = 1/214 (0%)

Query: 41  PPVSAIATNKLQXXXXXXXXXXXXXRKGLIDWKKGLPIXXXXXXXXXXXXXXXXXXXKDI 100
            PP+  + TNKLQ             R+G ++ K+ LP+                   D+
Sbjct: 43  PPLQTLGTNKLQGLFGSGSATLSYARRGHVNLKEQLPMALMSAAGAVLGALLATIVPGDV 102

Query: 101 LLAVVPVLLIFVALYFVFSPKLDGSKEGKARMSFFLFGLTVAPLLGFYDGVFGPGVGSFF 160
            L A++P LLI +ALYF   P + G  +  +R++ F+F LT+ PL+GFYDGVFGPG GSFF
Sbjct: 103 LKAILPFLLIAIALYFGLKPNM-GDVDQHSRVTPFVFTLTLVPLIGFYDGVFGPGTGSFF 161

Query: 161 LIAFIVLLGCKLLNAMSYTKLANVACNLGSLSVFLLHGSIIFPIVATMAVGAFVGANLGA 220
            ++ F+ L G  +L A ++TK  N   N+G+  VFL  G++++ +   M +G F+GA +G+
Sbjct: 162 MLGFVTLAGFGVLKATAHTKFLNFGSNVGAFGVFLFFGAVLWKVGLLMGLGQFLGAQVGS 221

Query: 221 RFAVRFGSKLIKPLLIVISISMAVKLLIDERNPL 254
            R+A+  G+K+IKPLL+++SI++A++LL D  +PL
Sbjct: 222 RYAMAKGAKIIKPLLVIVSIALAIRLLADPTHPL 255
```

Based on this analysis, including the presence of a putative leader sequence (double-underlined) and several putative transmembrane domains (single-underlined) in the gonococcal protein, it is predicted that the proteins from *N.meningitidis* and *N.gonorrhoeae,* and their epitopes, could be useful antigens for vaccines or diagnostics, or for raising antibodies.

**Example 52**

The following partial DNA sequence was identified in *N.meningitidis* <SEQ ID 441>:

```
  1 ..CTGCTAGGGT ATTGCATCGG TTATCGGTAC GGCTGTTGCA GCAAAACCAG
 51   CCGCAGACGG ATTATTTGGT CAAATTCGGA TCGTTTTGGG CGAG.ATTTT
101   TGGTTTTCTG GGACTGTATG ACGTCTATGC TTCGGCATGG TTTGTCGTTA
151   TCATGATGTT TTTGGTGGTT TCTACCAGTT TGTGCCTGAT TCGCAATGTG
201   CCGCCGTTCT GGCGCGAAAT GAAGTCTTTT CGGGAAAAGG TTAAAGAAAA
251   ATCTCTGGCG GCGATGCGCC ATTCTTCGCT GTTGGATGTA AAAATTGCGC
301   CCGAGGTTGC CAAACGTTAT CTGGAAGTAC AAGGTTTTCA GGGGAAAACC
351   ATTAACCGTG AAGACGGGTC GGTTCTGATT GCCGCCAAAA AAGGCACAAT
401   GAACAAATGG GGCTATATCT TTGCCCATGT TGCTTTGATT GTCATTTGCC
451   TGGGCGGGTT GATAGACAGT AACCTGCTGT TGAAACTGGG TATGCTGACC

501   GGTCGGATTG TTCCGGACAA TCAGGCGGTT TATGCCAAGG ATTTC.AAGC
551   CCGAAAGTAT .TTTGGGTGC gTCCAATCTC TCATTTAGGG GCAACGTCAA
601   TATTTCCG.A GGGGCAGAgT GCGGATGTGG TTTTCCTGA
```

This corresponds to the amino acid sequence <SEQ ID 442; ORF110>:

```
  1 ..LLGIASVIGT LLQQNQPQTD YLVKFGSFWA XIFGFLGLYD VYASAWFVVI
 51   MMFLVVSTSL CLIRNVPPFW REMKSFREKV KEKSLAAMRH SSLLDVKIAP
101   EVAKRYLEVQ GFQGKTINRE DGSVLIAAKK GTMNKWGYIF AHVALIVICL
151   GGLIDSNLLL KLGMLTGRIF RTIRRFMPRI XKPESXFGCV QSLI*GQRQY
201   FXRGRVRMWF S*
```

Computer analysis of this amino acid sequence gave the following results:

Homology with ORF88a from *N.meningitidis* (strain A)

ORF110 shows 91.5% identity over a 188aa overlap with ORF88a from strain A of *N. meningitidis:*

```
                      10        20        30        40        50        60
orf88a.pep   MSKSRRSPPLLSRPWFAFFSSMRFAVALLSLLGIASVIGTVLQQNQPQTDYLVKFGSFWA
                                           |||||||||:||||||||||||||||||||
orf110                                     LLGIASVIGTLLQQNQPQTDYLVKFGSFWA
                                                   10        20        30

                      70        80        90       100       110       120
orf88a.pep   QIFGFLGLYDVYASAWFVVIMMFLVVSTSLCLIRNVPPFWREMKSFREKVKEKSLAAMRH
             ||||||||||||||||||||||||||||||||||||||||||||||||||||||||||||
orf110       XIFGFLGLYDVYASAWFVVIMMFLVVSTSLCLIRNVPPFWREMKSFREKVKEKSLAAMRH
                      40        50        60        70        80        90

                     130       140       150       160       170       180
orf88a.pep   SSLLDVKIAPEVAKRYLEVQGFQGKTINREDGSVLIAAKKGTMNKWGYIFAHVALIVICL
             ||||||||||||||||||||||||||||||||||||||||||||||||||||||||||||
orf110       SSLLDVKIAPEVAKRYLEVQGFQGKTINREDGSVLIAAKKGTMNKWGYIFAHVALIVICL
                     100       110       120       130       140       150

                     190       200       210       220       230       240
orf88a.pep   GGLIDSNLLLKLGMLTGRIVPDNQAVYAKDFKPESILGASNLSFRGNVNISEGQSADVVF
             |||||||||||||||||||    : :: :   |||| :|
orf110       GGLIDSNLLLKLGMLTGRIFRTIRRFMPRIXKPESXFGCVQSLIXGQRQYFXRGRVRMWF
                     160       170       180       190       200       210

                     250       260       270       280       290       300
orf88a.pep   LNADNGILVQDLPFEVKLKKFHIDFYNTGMPRDFASDIEVTDKATGEKLERTIRVNHPLT

orf110       SX
```

However, ORF88 and ORF110 do not align, because they represent two different fragments of the same protein.

Homology with a predicted ORF from *N.gonorrhoeae*

ORF110 shows 88.6% identity over a 211 aa overlap with a predicted ORF (ORF110.ng) from *N. gonorrhoeae:*

```
orf110.pep                                 LLGIASVIGTLLQQNQPQTDYLVKFGSFWA   30
                                           ||||||||||:||||||||||||||| ||:
orf110ng     MSKSRISPTLLSRPWFAFFSSMRFAVALLSLLGIASVIGTVLQQNQPQTDYLVKFGPFWT   60

orf110.pep   XIFGFLGLYDVYASAWFVVIMMFLVVSTSLCLIRNVPPFWREMKSFREKVKEKSLAAMRH   90
              || |||||||||||||||||||||||||||||||||||||||||||||||||||||||||
orf110ng     RIFDFLGLYDVYASAWFVVIMMFLVVSTSLCLIRNVPPFWREMKSFREKVKEKSLAAMRH  120

orf110.pep   SSLLDVKIAPEVAKRYLEVQGFQGKTINREDGSVLIAAKKGTMNKWGYIFAHVALIVICL  150
             |||||||||||||||||||:||||||::||||||||||||||||||||| ||||||||||
orf110ng     SSLLDVKIAPEVAKRYLEVRGFQGKTVSREDGSVLIAAKKGTMNKWGYIXAHVALIVICL  180

orf110.pep   GGLIDSNLLLKLGMLTGRIFRTIRRFMPRIXKPESXFGCVQSLIXGQRQYFXRGRVRMWF  210
             | ||: |||||||||:| |||: || |||| |||| :| ||||| |||||| || :|||||
orf110ng     GRLINXNLLLKLGMLAGSIFRNNRRVMPRISKPESIWGGVQSLIKGQRQYFQRGKVRMWF  240

orf110.pep   S  211
             |
orf110ng     S  241
```

The complete length ORF1 110ng nucleotide sequence <SEQ ID 443> is predicted to encode a protein having amino acid sequence <SEQ ID 444>:

```
  1  MSKSRISPTL LSRPWFAFFS SMRFAVALLS LLGIASVIGT VLQQNQPQTD
 51  YLVKFGPFWT RIFDFLGLYD VYASAWFVVI MMFLVVSTSL CLIRNVPPFW
101  REMKSFREKV KEKSLAAMRH SSLLDVKIAP EVAKRYLEVR GFQGKTVSRE
151  DGSVLIAAKK GTMNKWGYIX AHVALIVICL GRLINXNLLL KLGMLAGSIF
201  RNNRRVMPRI SKPESIWGGV QSLIKGQRQY FQRGKVRMWF S*
```

Based on the putative transmembrane domains in the gonococcal protein, it is predicted that the proteins from *N.meningitidis* and *N.gonorrhoeae,* and their epitopes, could be useful antigens for vaccines or diagnostics, or for raising antibodies.

**Example 53**

The following DNA sequence was identified in *N.meningitidis* <SEQ ID 445>:

```
   1  ATGCCGTCTG AAACACGCCT GCCGAACTTT ATCCGCGTCT TGATATTTGC
  51  CCTGGGTTTC ATCTTCCTGA ACGCCTGTTC GGAACAAACC GCGCAAACCG
 101  TTACCCTGCA AGGCGAAACG ATGGGCACGA CCTATACCGT CAAATACCTT
 151  TCAAATAATC GGGACAAACT CCCCTCACCT GCCGAAATAC AAAAACGCAT
 201  CGATGACGCG CTTAAAGAAG TCAACCGGCA GATGTCCACC TATCAGCCCG
 251  ACTCCGAAAT CAGCCGGTTC AACCAACACA CAGCCGGCAA GCCCCTCCGC
 301  ATTTCAAGCG ACTTCGCACA CGTTACTGCC GAAGCCGTCC GCCTGAACCG
 351  CCTGACACAC GGCGCGCTGG ACGTAACCGT CGGCCCCTTG GTCAACCTTT
 401  GGGGATTCGG CCCCGACAAA TCCGTTACCC GTGAACCGTC GCCGGAACAA
 451  ATCAAACAGG CGGCATCTTA TACGGGCATA GACAAAATCA TTTTGAAACA
 501  AGGCAAAGAT TACGCTTCCT TGAGCAAAAC CCACCCCAAG GCCTATTTGG
 551  ATTTATCTTC GATTGCCAAA GGCTTCGGCG TTGATAAAGT TGCGGGCGAA
 601  CTGGAAAAAT ACGGCATTCA AAATTATCTG GTCGAAATCG GCGGCGAGTT
 651  GCACGGCAAA GGCAAAAACG CGCGCGGCGA ACCGTGGCGC ATCGGTATCG
 701  AGCAGCCCAA TATCGTCCAA GGCGGCAATA CGCAGATTAT CGTCCCGCTG
 751  AACAACCGTT CGCTTGCCAC TTCCGGCGAT TACCGTATTT TCCACGTCGA
 801  TAAAAACGGC AAACGCCTCT CCCATATCAT CAACCCGAAC AACAAACGAC
 851  CCATCAGCCA CAACCTCGCC TCCATCAGCG TGGTCGCAGA CAGTGCGATG
 901  ACGGCGGACG GCTTGTCCAC AGGATTATTC GTATTGGGCG AAACCGAAGC
 951  CTTAAAGCTG GCAGAGCGCG AAAAACTCGC TGTTTTCCTG ATTGTCAGGG
1001  ATAAAGGCGG CTACCGCACC GCCATGTCTT CCGAATTTGA AAAACTGCTC
1051  CGCTAA
```

This corresponds to the amino acid sequence <SEQ ID 446; ORF111>:

```
  1  MPSETRLPNF IRVLIFALGF IFLNACSEQT AQTVTLQGET MGTTYTVKYL
 51  SNNRDKLPSP AEIQKRIDDA LKEVNRQMST YQPDSEISRF NQHTAGKPLR
101  ISSDFAHVTA EAVRLNRLTH GALDVTVGPL VNLWGFGPDK SVTREPSPEQ
151  IKQAASYTGI DKIILKQGKD YASLSKTHPK AYLDLSSIAK GFGVDKVAGE
201  LEKYGIQNYL VEIGGELHGK GKNARGEPWR IGIEQPNIVQ GGNTQIIVPL
251  NNRSLATSGD YRIFHVDKNG KRLSHIINPN NKRPISHNLA SISVVADSAM
301  TADGLSTGLF VLGETEALKL AEREKLAVFL IVRDKGGYRT AMSSEFEKLL
351  R*
```

Computer analysis of this amino acid sequence gave the following results:

Homology with a predicted ORF from *N.meningitidis* (strain A)

ORF111 shows 96.9% identity over a 351aa overlap with an ORF (ORF 111a) from strain A of *N. meningitidis:*

```
                        10        20        30        40        50        60
orf111a.pep   MPSETRLPNFIRTLIFALSFIFLNACSEQTAQTVTLQGETMGTTYTVKYLSNNRDXLPSP
              ||||||||||||:|||||:|||||||||||||||||||||||||||||||||||| ||||
orf111        MPSETRLPNFIRVLIFALGFIFLNACSEQTAQTVTLQGETMGTTYTVKYLSNNRDKLPSP
                        10        20        30        40        50        60

                        70        80        90       100       110       120
orf111a.pep   AEIQXRIDDALKEVNRQMSTYQPDSEISRFNQHTAGKPLRISSDFAHVTAEAVHLNRLTH
              |||| ||||||||||||||||||||||||||||||||||||||||||||||||:||||||
orf111        AEIQKRIDDALKEVNRQMSTYQPDSEISRFNQHTAGKPLRISSDFAHVTAEAVRLNRLTH
                        70        80        90       100       110       120

                       130       140       150       160       170       180
orf111a.pep   GALDVTVGPLVNLWGFGPDKSVTREPSPEQIKQAASYTGIDKIILKQGKDYASLSKTHPK
              ||||||||||||||||||||||||||||||||||||||||||||||||||||||||||||
orf111        GALDVTVGPLVNLWGFGPDKSVTREPSPEQIKQAASYTGIDKIILKQGKDYASLSKTHPK
                       130       140       150       160       170       180

                       190       200       210       220       230       240
orf111a.pep   AYLDLSSIAKGFGVDXVAGELEKYGIQNYLVEIGGELHGKXKNARGEPWRIGIEQPNIVQ
              ||||||||||||||| |||||||||||||||||||||||| |||||||||||||||||||
orf111        AYLDLSSIAKGFGVDKVAGELEKYGIQNYLVEIGGELHGKGKNARGEPWRIGIEQPNIVQ
                       190       200       210       220       230       240

                       250       260       270       280       290       300
orf111a.pep   GGNTQIIVPLNNRSXATSGDYRIFHVDKSGKRLSHIINPNNKRPISHNLASISVXADSAM
              |||||||||||||| |||||||||||||:||||||||||||||||||||||||| |||||
orf111        GGNTQIIVPLNNRSLATSGDYRIFHVDKNGKRLSHIINPNNKRPISHNLASISVVADSAM
                       250       260       270       280       290       300

                       310       320       330       340       350
orf111a.pep   TADGXSTGLFVLGETEALKLAEREKLAVFLIVRDKGGYRTAMSSEFEKLLRX
              |||| |||||||||||||||||||||||||||||||||||||||||||
orf111        TADGLSTGLFVLGETEALKLAEREKLAVFLIVRDKGGYRTAMSSEFEKLLRX
                       310       320       330       340       350
```

The complete length ORF111a nucleotide sequence <SEQ ID 447> is:

```
   1  ATGCCGTCTG AAACACGCCT GCCGAACTTT ATCCGCACCT TGATATTTGC
  51  CCTGAGTTTT ATCTTCCTGA ACGCCTGTTC GGAACAAACC GCGCAAACCG
 101  TTACCCTGCA AGGTGAAACG ATGGGCACGA CCTATACCGT CAAATACCTT
 151  TCAAATAATC GGGACNAACT CCCNTCACCT GCCGAAATAC AAAANCGCAT
 201  CGATGACGCG CTTAAAGAAG TCAACCGGCA GATGTCCACC TATCAGCCCG
 251  ACTCCGAAAT CAGCCGGTTC AACCAACACA CAGCCGGCAA GCCCCTCCGC
 301  ATTTCAAGCG ACTTCGCACA CGTTACTGCC GAAGCCGTCC ACCTGAACCG
 351  CCTGACACAC GGCGCGCTGG ACGTAACCGT CGGCCCCTTG GTCAACCTTT
 401  GGGGATTCGG CCCCGACAAA TCCGTTACCC GTGAACCGTC GCCGGAACAA
 451  ATCAAACAAG CAGCATCTTA TACGGGCATA GACAAAATCA TTTTGAAACA
 501  AGGCAAAGAT TACGCTTCCT TGAGCAAAAC CCACCCCAAG GCCTATTTGG
 551  ATTTATCTTC GATTGCCAAA GGCTTCGGCG TTGATNANGT TGCGGGCGAA
 601  CTGGAAAAAT ACGGCATTCA AAATTATCTG GTCGAAATCG GCGGNGAGTT
 651  GCACGGCAAA GNCAAAAACG CGCGCGGCGA ACCTTGGCGC ATCGGCATCG
 701  AACAGCCCAA CATCGTCCAA GGCGGCAATA CGCAGATTAT CGTCCCGCTG
 751  AACAACCGTT CGNTTGCCAC TTCCGGCGAT TACCGTATTT TCCACGTCGA
 801  TAAAAGCGGC AAACGCCTCT CCCATATCAT TAATCCGAAC AACAAACGAC
 851  CCATCAGCCA CAACCTCGCC TCCATCAGCG TGNTCGCAGA CAGTGCGATG
 901  ACGGCGGACG GCTTNTCCAC AGGATTATTC GTATTGGGCG AAACCGAAGC
 951  CTTAAAGCTG GCAGAGCGCG AAAAACTCGC TGTTTTCCTG ATTGTCAGGG
1001  ATAAAGGCGG CTACCGCACC GCCATGTCTT CCGAATTTGA AAAACTGCTC
1051  CGCTAA
```

This encodes a protein having amino acid sequence <SEQ ID 448>:

```
  1  MPSETRLPNF IRTLIFALSF IFLNACSEQT AQTVTLQGET MGTTYTVKYL
 51  SNNRDXLPSP AEIQXRIDDA LKEVNRQMST YQPDSEISRF NQHTAGKPLR
101  ISSDFAHVTA EAVHLNRLTH GALDVTVGPL VNLWGFGPDK SVTREPSPEQ
151  IKQAASYTGI DKIILKQGKD YASLSKTHPK AYLDLSSIAK GFGVDXVAGE
201  LEKYGIQNYL VEIGGELHGK XKNARGEPWR IGIEQPNIVQ GGNTQIIVPL
251  NNRSXATSGD YRIFHVDKSG KRLSHIINPN NKRPISHNLA SISVXADSAM
301  TADGXSTGLF VLGETEALKL AEREKLAVFL IVRDKGGYRT AMSSEFEKLL
351  R*
```

Homology with a predicted ORF from *N.gonorrhoeae*

ORF11 I shows 96.6% identity over a 351aa overlap with a predicted ORF (ORF111.ng) from *N. gonorrhoeae:*

```
                   10        20        30        40        50        60
orf111ng    MPSETRLPNLIRALIFALGFIFLNACSEQTAQTVTLQGETMGTTYTVKYLSNNRDKLPSP
            |||||||||:||:||||||||||||||||||||||||||||||||||||||||||||||||
orf111      MPSETRLPNFIRVLIFALGFIFLNACSEQTAQTVTLQGETMGTTYTVKYLSNNRDKLPSP
                   10        20        30        40        50        60

                   70        80        90       100       110       120
orf111      AKIQKRIDDALKEVNRQMSTYQTDSEISRFNQHTAGKPLRISSDFAHVTAEAVRLNRLTH
            |:|||||||||||||||||| ||||||||||||||||||||||||||||||||||||||||
orf111      AEIQKRIDDALKEVNRQMSTYQPDSEISRFNQHTAGKPLRISSDFAHVTAEAVRLNRLTH
                   70        80        90       100       110       120

                  130       140       150       160       170       180
orf111ng    GALDVTVGPLVNLWGFGPDKSVTREPSPEQIKQAASYTGIDKIILQQGKDYASLSKTHPK
            |||||||||||||||||||||||||||||||||||||||||||||:||||||||||||||
orf111      GALDVTVGPLVNLWGFGPDKSVTREPSPEQIKQAASYTGIDKIILKQGKDYASLSKTHPK
                  130       140       150       160       170       180

                  190       200       210       220       230       240
orf111ng    AYLDLSSIAKGFGVDKVAGELEKYGIQNYLVEIGGELHGKGKNAHGEPWRIGIEQPNIIQ
            ||||||||||||||||||||||||||||||||||||||||||||:|||||||||||||:|
orf111      AYLDLSSIAKGFGVDKVAGELEKYGIQNYLVEIGGELHGKGKNARGEPWRIGIEQPNIVQ
                  190       200       210       220       230       240

                  250       260       270       280       290       300
orf111ng    GGNTQIIVPLNNRSLATSGDYRIFHVDKNGKRLSHIINPNNKRPISHNLASISVVSDSAM
            |||||||||||||||||||||||||||||||||||||||||||||||||||||||:||||
orf111      GGNTQIIVPLNNRSLATSGDYRIFHVDKNGKRLSHIINPNNKRPISHNLASISVVADSAM
                  250       260       270       280       290       300

                  310       320       330       340       350
orf111ng    TADGLSTGLFVLGETEALRLAEQEKLAVFLIVRDKDGYRTAMSSEFAKLLRX
            ||||||||||||||||||:|||:||||||||||||| |||||||||| |||||
orf111      TADGLSTGLFVLGETEALKLAEREKLAVFLIVRDKGGYRTAMSSEFEKLLRX
                  310       320       330       340       350
```

The complete length ORF111ng nucleotide sequence <SEQ ID 449> is:

```
   1  ATGCCGTCTG AAACACGCCT GCCGAACCTT ATCCGCGCCT TGATATTTGC
  51  CCTGGGTTTC ATCTTCCTGA ACGCCTGTTC GGaacaaacC GCGCAaaccg
 101  TTACCCTGCA AGGCGAAAcg aTGGGTACGA CCTATACCGT CAAATACCTT
 151  TCAAATAATC GGGACAAACT CCCCTCCCCT GCCAAAATAC AAAAGCGCAT
 201  TGATGATGCG CTTAAAGAAG TCAACCGGCA GATGTCCACC TACCAGACCG
 251  ATTCCGAAAT CAGCCGGTTC AACCAACACA CAGCCGGCAA GCCCCTCCGC
 301  ATTTCAAGCG ATTTCGCACA CGTTACCGCC GAAGCCGTCC GCCTGAACCG
 351  CCTGACTCAC GGCGCACTGG ACGTAACCGT CGGCCCTTTG GTCAACCTTT
 401  GGGGGTTCGG CCCCGACAAA TCCGTTACCC GTGAACCGTC GCCGGAACAA
 451  ATCAAACAGG CGGCATCTTA TACGGGCATA GACAAAATCA TTTTGCAACA
 501  AGGCAAAGAT TACGCTTCCT TGAGCAAAAC CCACCCCAAA GCCTATTTGG
 551  ATTTATCTTC GATTGCCAAA GGCTTCGGCG TTGATAAAGT TGCGGGCGAA
 601  CTGGAAAAAT ACGGCATTCA AAATTATCTG GTCGAAAtcg gcggcGAGTT
 651  GCACGGCAAA GGCAAAAATG CGCACGGCGA ACCGTGGCGC ATCGGTATAG
 701  AGCAACCCAA TATCATCCAA GgcgGCAata CGCAGATTAt cgtcccgctg
 751  aaCaaccgtt cgctTGCCAC TTCCGGCGAT TAccgtaTTT tccacgtcgA
 801  TAAAAAcggc aaacgccttt cccacaTCAT CAATCCCaAC aacAAACgac
 851  ccATCAGcca caacctcgcc tccatcagcg tggtctcAGA CAGTGCAATG
 901  ACGGCGGACG GTTtatCCAC AGGATTATTT GTTTTAGGCG AAACCGAAGC
 951  CTTAAGGCTG GCAGAACAAG AAAAACTCGC TGTTTTCCTA ATTGTCCGGG

1001  ATAAGGACGG CTACCGCACC GCCATGTCTT CCGAATTTGC CAAGCTGCTC
1051  CGCTAA
```

This encodes a protein having amino acid sequence <SEQ ID 450>:

```
   1  MPSETRLPNL IRALIFALGF IFLNACSEQT AQTVTLQGET MGTTYTVKYL
  51  SNNRDKLPSP AKIQKRIDDA LKEVNRQMST YQTDSEISRF NQHTAGKPLR
 101  ISSDFAHVTA EAVRLNRLTH GALDVTVGPL VNLWGFGPDK SVTREPSPEQ
 151  IKQAASYTGI DKIILQQGKD YASLSKTHPK AYLDLSSIAK GFGVDKVAGE
 201  LEKYGIQNYL VEIGGELHGK GKNAHGEPWR IGIEQPNIIQ GGNTQIIVPL
 251  NNRSLATSGD YRIFHVDKNG KRLSHIINPN NKRPISHNLA SISVVSDSAM
 301  TADGLSTGLF VLGETEALRL AEQEKLAVFL IVRDKDGYRT AMSSEFAKLL
 351  R*
```

This protein shosw homology with a hypothetical lipoprotein precursor from *H. influenzae:*

```
sp|P44550|YOJL_HAEIN HYPOTHETICAL LIPOPROTEIN HI0172 PRECURSOR >gi|1074292|pir|4
hypothetical protein HI0172 - Haemophilus influenzae (strain Rd KW20)
>gi|1573128 (U32702) hypothetical  [Haemophilus influenzae] Length = 346
 Score =  353 bits (896), Expect = 9e-97
 Identities = 181/344 (52%), Positives = 247/344 (71%), Gaps = 4/344 (1%)

Query: 7   LPNLIRALIFALGFIFLNACSEQTAQTVTLQGETMGTTYTVKYLSNNRDKLPSPAKIQKR 66
            +  LI  +I     + L AC ++T + ++L G+TMGTTY VKYL +      S  K  +
Sbjct: 1   MKKLISGIIAVAMALSLAACQKET-KVISLSGKTMGTTYHVKYLDDGSITATSE-KTHEE 58

Query: 67  IDDALKEVNRQMSTYQTDSEISRFNQHT-AGKPLRISSDFAHVTAEAVRLNRLTHGALDV 125
            I+  LK+VN +MSTY+ DSE+SRFNQ+T    P+ IS+DFA V AEA+RLN++T GALDV
Sbjct: 59  IEAILKDVNAKMSTYKKDSELSRFNQNTQVNTPIEISADFAKVLAEAIRLNKVTEGALDV 118

Query: 126 TVGPLVNLWGFGPDKSVTREPSPEQIKQAASYTGIDKIILQQGKDYASLSKTHPKAYLDL 185
            TVGP+VNLWGFGP+K    ++P+P+PEQ+ +  ++ GIDKI L    K+ A+LSK  P+ Y+DL
Sbjct: 119 TVGPVVNLWGFGPEKRPEKQPTPEQLAERQAWVGIDKITLDTNKEKATLSKALPQVYVDL 178

Query: 186 SSIAKGFGVDKVAGELEKYGIQNYLVEIGGELHGKGKNAHGEPWRIGIEQPNIIQGGNTQ 245
            SSIAKGFGVD+VA +LE+   QNY+VEIGGE+  KGKN  G+PW+I IE+P        +

Sbjct: 179 SSIAKGFGVDQVAEKLEQLNAQNYMVEIGGEIRAKGKNIEGKPWQIAIEKPTTTGERAVE 238
Query: 246 IIVPLNNRSLATSGDYRIFHVDKNGKRLSHIINPNNKRPISHNLASISVVSDSAMTADGL 305
             ++ LNN  +A+SGDYRI+  ++NGKR +H I+P    PI H+LASI+V++ ++MTADGL
Sbjct: 239 AVIGLNNMGMASSGDYRIY-FEENGKRFAHEIDPKTGYPIQHHLASITVLAPTSMTADGL 297

Query: 306 STGLFVLGETEALRLAEQEKLAVFLIVRDKDGYRTAMSSEFAKL 349
            STGLFVLGE +AL +AE+  LAV+LI+R  +G+ T  SS F KL
Sbjct: 298 STGLFVLGEDKALEVAEKNNLAVYLIIRTDNGFVTKSSSAFKKL 341
```

Based on this analysis, it is predicted that the proteins from *N.meningitidis* and *N.gonorrhoeae,* and their epitopes, could be useful antigens for vaccines or diagnostics, or for raising antibodies.

## Example 54

The following partial DNA sequence was identified in *N.meningitidis* <SEQ ID 451>:

```
  1 ..CCGTGCCGCC GACAGGGCGA CGACGTGTAT GCGGCGCACG CGTCCCGTCA
 51   AAAATTGTGG CTGCGCTTCA TCGGCGGCCG GTCGCATCAA AATATACGGG
101   GCGGCGCGGC TGCGGACGGG TGGCGCAAAG GCGTGCAAAT CGGCGGCGAG
151   GTGTTTGTAC GGCAAAATGA AGGCAGCCkA yTGGCAATCG GCGTGATGGG
201   CGGCAGGGCC GGCCAGCACG CwTCAGTCAA CGGCAAAGGC GGTGCGGCAG
251   gCAGTGATTT GTATGGTTAT GgCGGGGgTG TTTATGCTgC GTGGCATCAG
301   TTGCGCGATA AACAAACGGG TgCGTATTTG GACGGCTGGT TGCAATACCA
351   ACGTTTCAAA CACCGCATCA ATGATGAAAA CCGTGCGGAA CgCTACAAAA
401   CCAAAGGTTG GACGGCTTCT GTCGAAGGCG GCTACAACGC GCTTGTGGCG
451   GAAGGCATTG TCGGAAAAGG CAATAATGTG CGGTTTTACC TACAACCGCA
501   GgCGCAGTTT ACCTACTTGG GCGTAAACGG CGGCTTTACC GACAGCGAGG
551   GGACGGCGGT CGGACTGCTC GGCAGCGGTC AGTGGCAAAG CCGCGCCGGC
601   AtTCGGGCAA AAACCCGTTT TGCTTTGCGT AACGGTGTCA ATCTTCAGCC
651   TTTTGCCGCT TTTAATGTtt TGCACAGGTC AAAATCTTTC GGCGTGGAAA
701   TGGACGGCGA AAAACAGACG CTGGCAGGCA GGACGGCACT CGAAGGGCGG

751   TTCGGTATTG AAGCCGGTTG GAAAGGCCAT ATGTCCGCA..
```

This corresponds to the amino acid sequence <SEQ ID 452; ORF35>:

```
  1   ..PCRRQGDDVY AAHASRQKLW LRFIGGRSHQ NIRGGAAADG WRKGVQIGGE
 51     VFVRQNEGSX LAIGVMGGRA GQHASVNGKG GAAGSDLYGY GGGVYAAWHQ
101     LRDKQTGAYL DGWLQYQRFK HRINDENRAE RYKTKGWTAS VEGGYNALVA
151     EGIVGKGNNV RFYLQPQAQF TYLGVNGGFT DSEGTAVGLL GSGQWQSRAG
201     IRAKTRFALR NGVNLQPFAA FNVLHRSKSF GVEMDGEKQT LAGRTALEGR
251     FGIEAGWKGH MSA..
```

Computer analysis of this amino acid sequence gave the following results:

Homology with putative secreted VirG-homolgue of *N. meningitidis* (accession number A32247)

ORF and virg-h protein show 51% aa identity in 261 aa overlap:

```
Orf35  5   QGDDVYAAHASRQKLWLRFIGGRSHQNIRGGAA-ADGWRKGVQIGGEVFVRQNEGSXLAI 63
           +  D++     R+ LWLR I G S+Q ++G  A  +G+RKGVQ+GGEVF  QNE + L+I
virg-h 396 KNSDIFDRTLPRKGLWLRVIDGHSNQWVQGKTAPVEGYRKGVQLGGEVFTWQNESNQLSI 455

Orf35  64  GVMGGRAGQHASVNGKG--GAAGSDLYGYGGGVYAAWHQLRDKQTGAYLDGWLQYQRFKH 121
           G+MGG+A Q ++ +          ++ G+G GVYA WHQL+DKQTGAY D W+QYQRF+H
virg-h 456 GLMGGQAEQRSTFHNPDTDNLTTGNVKGFGAGVYATWHQLQDKQTGAYADSWMQYQRFRH 515

Orf35  122 RINDENRAERYKTKGWTASVEGGYNALVAEGIVGKGNNVRFYLQPQAQFTYLGVNGGFTD 181
           RIN E+  ER+ +KG TAS+E GYNAL+AE    KGN++R YLQPQAQ TYLGVNG F+D
virg-h 516 RINTEDGTERFTSKGITASIEAGYNALLAEHFTKKGNSLRVYLQPQAQLTYLGVNGKFSD 575

Orf35  182 SEGTAVGLLGSGQWQSRAGIRAKTRFALRNGVNLQPFAAFNVLHRSKSFGVEMDGEKQTL 241
           SE   V LLGS Q Q+R G++AK +F+L   + ++PFAA N L+ +K FGVEMDGE++ +
virg-h 576 SENAHVNLLGSRQLQTRVGVQAKAQFSLYKNIAIEPFAAVNALYHNKPFGVEMDGERRVI 635

Orf35  242 AGRTALEGRFGIEAGWKGHMS 262
             +TA+E + G+    K H++
virg-h 636 NNKTAIESQLGVAVKIKSHLT 656
```

Homology with a predicted ORF from *N.meningitidis* (strain A)

ORF35 shows 96.9% identity over a 259aa overlap with an ORF (ORF35a) from strain A of *N. meningitidis:*

```
                                                    10        20        30
orf35.pep                                 PCRRQGDDVYAAHASRQKLWLRFIGGRSHQNIRG
                                            :|||||||  ||||||||||||||||||||
orf35a     QRLAIPEAEAVLYAQQAYAANTLFGLRAADRGDDVYAADPSRQKLWLRFIGGRSHQNIRG
          310       320       330       340       350       360

              40        50        60        70        80        90
orf35.pep  GAAADGWRKGVQIGGEVFVRQNEGSXLAIGVMGGRAGQHASVNGKGGAAGSDLYGYGGGV
           |||||| |||||||||||||||||| ||||||||||||||||||||||||| |:||||||
orf35a     GAAADGRRKGVQIGGEVFVRQNEGSRLAIGVMGGRAGQHASVNGKGGAAGSYLHGYGGGV
          370       380       390       400       410       420

             100       110       120       130       140       150
orf35.pep  YAAWHQLRDKQTGAYLDGWLQYQRFKHRINDENRAERYKTKGWTASVEGGYNALVAEGIV
           ||||||||||||||||||||||||||||||||||||||||||||||||||||||||||:|
orf35a     YAAWHQLRDKQTGAYLDGWLQYQRFKHRINDENRAERYKTKGWTASVEGGYNALVAEGVV
          430       440       450       460       470       480

             160       170       180       190       200       210
orf35.pep  GKGNNVRFYLQPQAQFTYLGVNGGFTDSEGTAVGLLGSGQWQSRAGIRAKTRFALRNGVN
           ||||||||||||||||||||||||||||||||||||||||||||||||||||||||||||
orf35a     GKGNNVRFYLQPQAQFTYLGVNGGFTDSEGTAVGLLGSGQWQSRAGIRAKTRFALRNGVN
          490       500       510       520       530       540

             220       230       240       250       260
orf35.pep  LQPFAAFNVLHRSKSFGVEMDGEKQTLAGRTALEGRFGIEAGWKGHMSA
           |||||||||||||||||||||||||||||||||||||||||||||||||


orf35a     LQPFAAFNVLHRSKSFGVEMDGEKQTLAGRTALEGRFGIEAGWKGHMSARIGYGKRTDGD
          550       560       570       580       590       600

orf35a     KEAALSLKWLFX
          610       620
```

The complete length ORF35a nucleotide sequence <SEQ ID 453> is:

```
   1 ATGTTCAGAG CTCAGCTTGG TTCAAATACT CGTTCTACCA AAATCGGCGA
  51 CGATGCCGAT TTTTCATTTT CAGACAAGCC GAAACCCGGC ACTTCCCATT
 101 ATTTTTCCAG CGGTAAAACC GATCAAAATT CATCCGAATA TGGGTATGAC
 151 GAAATCAATA TCCAAGGTAA AAACTACAAT AGCGGCATAC TCGCCGTCGA
 201 TAATATGCCC GTTGTTAAGA AATATATTAC AGATACTTAC GGGGATAATT
 251 TAAAGGATGC GGTTAAGAAG CAATTACAGG ATTTATACAA AACAAGACCC
 301 GAAGCTTGGG AAGAAAATAA AAAACGGACT GAGGAGGCGT ATATAGAACA
 351 GCTTGGACCA AAATTTAGTA TACTCAAACA GAAAAACCCC GATTTAATTA
 401 ATAAATTGGT AGAAGATTCC GTACTCACTC CTCATAGTAA TACATCACAG
 451 ACTAGTCTCA ACAACATCTT CAATAAAAAA TTACACGTCA AAATCGAAAA
 501 CAAATCCCAC GTCGCCGGAC AGGTGTTGGA ACTGACCAAG ATGACGCTGA
 551 AAGATTCCCT TTGGGAACCG CGCCGCCATT CCGACATCCA TATGCTGGAA
 601 ACTTCCGATA ATGCCCGCAT CCGCCTGAAC ACGAAAGATG AAAAACTGAC
 651 CGTCCATAAA GCGTATCAGG GCGGTGCGGA TTTCCTGTTC GGCTACGACG
 701 TGCGGGAGTC GGACAAACCC GCCCTGACCT TTGAAGAAAA AGTCAGCGGA
 751 CAATCCGGCG TGGTTTTGGA ACGCCGGCCG GAAAATCTGA AAACGCTCGA
 801 CGGGCGCAAA CTGATTGCGG CGGAAAAGGC AGACTCTAAT TCGTTTGCGT
 851 TTAAACAAAA TTACCGGCAG GGACTGTACG AATTATTGCT CAAGCAATGC
 901 GAAGGCGGAT TTTGCTTGGG CGTGCAGCGT TTGGCTATCC CCGAGGCGGA
 951 AGCGGTTTTA TATGCCCAAC AGGCTTATGC GGCAAATACT TTGTTCGGGC
1001 TGCGTGCCGC CGACAGGGGC GACGACGTGT ATGCCGCCGA TCCGTCCCGT
1051 CAAAAATTGT GGCTGCGCTT CATCGGCGGC CGGTCGCATC AAAATATACG
1101 GGGCGGCGCG GCTGCGGACG GGCGGCGCAA AGGCGTGCAA ATCGGCGGCG
1151 AGGTGTTTGT ACGGCAAAAT GAAGGCAGCC GGCTGGCAAT CGGCGTGATG
1201 GGCGGCAGGG CTGGCCAGCA CGCATCAGTC AACGGCAAAG GCGGTGCGGC
1251 AGGCAGTTAT TTGCATGGTT ATGGCGGGGG TGTTTATGCT GCGTGGCATC
1301 AGTTGCGCGA TAAACAAACG GGTGCGTATT TGGACGGCTG GTTGCAATAC
1351 CAACGTTTCA AACACCGCAT CAATGATGAA AACCGTGCGG AACGCTACAA
1401 AACCAAAGGT TGGACGGCTT CTGTCGAAGG CGGCTACAAC GCGCTTGTGG
1451 CGGAAGGCGT TGTCGGAAAA GGCAATAATG TGCGGTTTTA CCTGCAACCG
1501 CAGGCGCAGT TTACCTACTT GGGCGTAAAC GGCGGCTTTA CCGACAGCGA
1551 GGGGACGGCG GTCGGACTGC TCGGCAGCGG TCAGTGGCAA AGCCGCGCCG
1601 GCATTCGGGC AAAAACCCGT TTTGCTTTGC GTAACGGTGT CAATCTTCAG
1651 CCTTTTGCCG CTTTTAATGT TTTGCACAGG TCAAAATCTT TCGGCGTGGA
1701 AATGGACGGC GAAAAACAGA CGCTGGCAGG CAGGACGGCG CTCGAAGGGC
1751 GGTTCGGCAT TGAAGCCGGT TGGAAAGGCC ATATGTCCGC ACGCATCGGA
1801 TACGGCAAAA GGACGGACGG CGACAAAGAA GCCGCATTGT CGCTCAAATG
1851 GCTGTTTTGA
```

This encodes a protein having amino acid sequence <SEQ ID 454>:

```
  1 MFRAQLGSNT RSTKIGDDAD FSFSDKPKPG TSHYFSSGKT DQNSSEYGYD
 51 EINIQGKNYN SGILAVDNMP VVKKYITDTY GDNLKDAVKK QLQDLYKTRP
101 EAWEENKKRT EEAYIEQLGP KFSILKQKNP DLINKLVEDS VLTPHSNTSQ
151 TSLNNIFNKK LHVKIENKSH VAGQVLELTK MTLKDSLWEP RRHSDIHMLE
201 TSDNARIRLN TKDEKLTVHK AYQGGADFLF GYDVRESDKP ALTFEEKVSG
251 QSGVVLERRP ENLKTLDGRK LIAAEKADSN SFAFKQNYRQ GLYELLLKQC
301 EGGFCLGVQR LAIPEAEAVL YAQQAYAANT LFGLRAADRG DDVYAADPSR
351 QKLWLRFIGG RSHQNIRGGA AADGRRKGVQ IGGEVFVRQN EGSRLAIGVM
401 GGRAGQHASV NGKGGAAGSY LHGYGGGVYA AWHQLRDKQT GAYLDGWLQY
451 QRFKHRINDE NRAERYKTKG WTASVEGGYN ALVAEGVVGK GNNVRFYLQP
501 QAQFTYLGVN GGFTDSEGTA VGLLGSGQWQ SRAGIRAKTR FALRNGVNLQ
551 PFAAFNVLHR SKSFGVEMDG EKQTLAGRTA LEGRFGIEAG WKGHMSARIG
601 YGKRTDGDKE AALSLKWLF*
```

Homology with a predicted ORF from *N.gonorrhoeae*

ORF35 shows 51.7% identity over a 261aa overlap with a predicted ORF (ORF35ngh) from *N. gonorrhoeae:*

```
orf35.pep                                PCRRQGDDVYAAHASRQKLWLRFIGGRSHQNIRG   34
                                         :::|::      |: |||| | |:|:| ::|
orf35ngh    FTKVQERDDIAIYAQQAQAANTLFALRLNDKNSDIFDRTLPRKGLWLRVIDGHSNQWVQG  370
```

```
orf35.pep      GAA-ADGWRKGVQIGGEVFVRQNEGSXLAIGVMGGRAGQHASVNGKG--GAAGSDLYGYG   91
                :| ::|:||||||:|||||: |||:: |:|||:|||:| |:::  :    : : ::: |:|
orf35ngh       KTAPVEGYRKGVQLGGEVFTWQNESNQLSIGLMGGQAEQRSTFRNPDTDNLTTGNVKGFG  430

orf35.pep      GGVYAAWHQLRDKQTGAYLDGWLQYQRFKHRINDENRAERYKTKGWTASVEGGYNALVAE  151
               :||||:||||:||||||||::|:|||||:|||| |  :||: :|| |||:|:|||||:||
orf35ngh       AGVYATWHQLQDKQTGAYVDSWMQYQRFRHRINTEYATERFTSKGITASIEAGYNALLAE  490

orf35.pep      GIVGKGNNVRFYLQPQAQFTYLGVNGGFTDSEGTAVGLLGSGQWQSRAGIRAKTRFALRN  211
                :: |||::| |||||||:||||||| |:|||:: |:|||| | |||:|::||::||: |
orf35ngh       HFTKKGNSLRVYLQPQAQLTYLGVNGKFSDSENAQVNLLGSRQLQSRVGVQAKAQFAFTN  550

orf35.pep      GVNLQPFAAFNVLHRSKSFGVEMDGEKQTLAGRTALEGRFGIEAGWKGHMSA          263
               ||::|||:| | ::::| ||||:||::::: ::|::| ::|: |  |:|::
orf35ngh       GVTFQPFVAVNSIYQQKPFGVEIDGDRRVINNKTVIETQLGVAAKIKSHLTLQASFNRQT  610
```

A partial ORF35ngh nucleotide sequence <SEQ ID 455> is predicted to encode a protein having partial amino acid sequence <SEQ ID 456>:

```
  1 ..KKLRDRNSEY WKEETYHIKS NGRTYPNIPA LFPKHPFDPF ENINNSKKIS
 51   FYDKEYTEDY LVGFARGFGV EKRNGEEEKP LRQYFKDCVN TENSNNDNCK
101   ISSFGNYGPI LIKSDIFALA SQIKNSHINS EILSVGNYIE WLRPTLNKLT
151   GWQEHLYAGL DPFHYIEVTD NSHVIGQTID LGALELTNSL WKPRWNSNID
201   YLITKNAEIR FNTKNESLLV KEDYAGGARF RFAYDLKDKV PEIPVLTFEK
251   NITGTSDIIF EGKALDNLKH LDGHQIVKVN DTADKDAFRL SSKYRKGIYT
301   LSLQQRPEGF FTKVQERDDI AIYAQQAQAA NTLFALRLND KNSDIFDRTL
351   PRKGLWLRVI DGHSNQWVQG KTAPVEGYRK GVQLGGEVFT WQNESNQLSI
401   GLMGGQAEQR STFRNPDTDN LTTGNVKGFG AGVYATWHQL QDKQTGAYVD
451   SWMQYQRFRH RINTEYATER FTSKGITASI EAGYNALLAE HFTKKGNSLR
501   VYLQPQAQLT YLGVNGKFSD SENAQVNLLG SRQLQSRVGV QAKAQFAFTN
551   GVTFQPFVAV NSIYQQKPFG VEIDGDRRVI NNKTVIETQL GVAAKIKSHL
601   TLQASFNRQT SKHHHAKQGA LNLQWTF*
```

Based on this prediction, these proteins from *N.meningitidis* and *N.gonorrhoeae,* and their epitopes, could be useful antigens for vaccines or diagnostics, or for raising antibodies.

**Example 55**

The following partial DNA sequence was identified in *N.meningitidis* <SEQ ID 457>:

```
  1 ..GCGGAATATG TTCAGTTCTC TATAGATTTG TTCAGTGTGG GTAAATCGGG
 51   GGGCGGTATA CCTAAGGCTA AGCCTGTGTT TGATGCGAAA CCGAGATGGG
101   AGGTTGATAG GAAGCTTAAT AAATTGACAA CTCGTGAGCA GGTGGAGAAA
151   AATGTTCAGG AAACGAGAAG AAGGAGTCAG AGTAGTCAGT TTAAAGCCCA
201   TGCGCAACGA GAATGGGAAA ATAAAACAGG GTTAGATTTT AATCATTTTA
251   TAGGTGGTGA TATCAATAAA AAAGGCACAG TAACAGGAGG GCATAGTCTA
301   ACCCGTGGTG ATGTACGGGT GATACAACAA ACCTCGGCAC CTGATAAACA
351   TGGGGT.TTA TCAAGCGACA GTGGAAATTN A
```

This corresponds to the amino acid sequence <SEQ ID 458; ORF46>:

```
  1 ..AEYVQFSIDL FSVGKSGGGI PKAKPVFDAK PRWEVDRKLN KLTTREQVEK
 51   NVQETRRRSQ SSQFKAHAQR EWENKTGLDF NHFIGGDINK KGTVTGGHSL
101   TRGDVRVIQQ TSAPDKHGXL SSDSGNX
```

Further work revealed further partial nucleotide sequence <SEQ ID 459>:

```
  1   ..GCAGTGTGCC TnCCGATGCA TGCACACGCC TCAnATTTGG CAAACGATTC
 51     TTTTATCCGG CAGGTTCTCG ACCGTCAGCA TTTCGAACCC GACGGGAAAT
101     ACCACCTATT CGGCAGCAGG GGGGAACTTG CCGAGCGCCA GTCTCATATC
151     GGATTGGGAA AAATACAAAG CCATCAGTTG GGCAACCTGA TGATTCAACA
201     GGCGGCCATT AAAGGAAATA TCGGCTACAT TGTCCGCTTT TCCGATCACG
251     GGCACGAAGT CCATTCCCCs TTCGACAACC ATGCCTCACA TTCCGATTCT
301     GATGAAGCCG GTAGTCCCGT TGACGGATTT AGCCTTTACC GCATCCATTG
351     GGACGGATAC GAACACCATC CCGCCGACGG CTATGACGGG CCACAGGGCG
401     GCGGCTATCC CGCTCCCAAA GGCGCGAGGG ATATATACAG TTACGACATA

451     AAAGGCGTTG CCCAAAATAT CCGCCTCAAC CTGACCGACA ACCGCAGCAC
501     CGGACAACGG CTTGCCGACC GTTTCCACAA TGCCGGTAGT ATGCTGACGC
551     AAGGAGTAGG CGACGGATTC AAACGCGCCA CCCGATACAG CCCCGAGCTG
601     GACAGATCGG GCAATGCCGC CGAAGCCTTC AACGGCACTG CAGATATCGT
651     TAAAAACATC ATCGGCGCTG CAGGAGAAAT TGT
```

This corresponds to the amino acid sequence <SEQ ID 460; ORF46-1>:

```
  1   ..AVCLPMHAHA SXLANDSFIR QVLDRQHFEP DGKYHLFGSR GELAERQSHI
 51     GLGKIQSHQL GNLMIQQAAI KGNIGYIVRF SDHGHEVHSP FDNHASHSDS
101     DEAGSPVDGF SLYRIHWDGY EHHPADGYDG PQGGGYPAPK GARDIYSYDI
151     KGVAQNIRLN LTDNRSTGQR LADRFHNAGS MLTQGVGDGF KRATRYSPEL
201     DRSGNAAEAF NGTADIVKNI IGAAGEI
```

Computer analysis of this amino acid sequence gave the following results:

Homology with a predicted ORF from *N. gonorrhoeae*

ORF46 shows 98.2% identity over a 111aa overlap with a predicted ORF (ORF46ng) from *N. gonorrhoeae:*

```
orf46.pep                  AEYVQFSIDLFSVGKSGGGIPKAKPVFDAKPRWEVDRKLNKLTTR   45
                                               |||||||||||||||||||||||||||||
orf46ng    PKTGVPFDGKGFPNFEKHVKYDTKLDIQELSGGGIPKAKPVFDAKPRWEVDRKLNKLTTR  217

orf46.pep  EQVEKNVQETRRRSQSSQFKAHAQREWENKTGLDFNHFIGGDINKKGTVTGGHSLTRGDV  105
           |||||||||||||||||||||||||||||||||||||||||||||||:||||||||||||
orf46ng    EQVEKNVQETRRRSQSSQFKAHAQREWENKTGLDFNHFIGGDINKKGAVTGGHSLTRGDV  277

orf46.pep  RVIQQTSAPDKHGXLSSDSGN  126
           ||||||||||||| |||||||
orf46ng    RVIQQTSAPDKHGVLSSDSGN  298
```

A partial ORF46ng nucleotide sequence <SEQ ID 461> is predicted to encode a protein having partial amino acid sequence <SEQ ID 462>:

```
  1   ..RRLKHCCHAR LGSAFHRKQD GAHQRFGRYG ATQRLCRSSH PRLGSPKPQC
 51     RTRHRSRQQY LYGSHPHQRD WSCPGKIQLG RHHGTSCRAV ADXRDRICER
101     EIRRQRQXCR CRLGKIPSLS IPKYPLKLEQ RYGKENITSS TVPPSNGKNV
151     KLADQRHPKT GVPFDGKGFP NFEKHVKYDT KLDIQELSGG GIPKAKPVFD
201     AKPRWEVDRK LNKLTTREQV EKNVQETRRR SQSSQFKAHA QREWENKTGL
251     DFNHFIGGDI NKKGAVTGGH SLTRGDVRVI QQTSAPDKHG VLSSDSGN*
```

Further work revealed the complete gonococcal DNA sequence <SEQ ID 463>:

```
   1  TTGGGCATTT CCCGCAAAAT ATCCCTTATT CTGTCCATAC TGGCAGTGTG
  51  CCTGCCGATG CATGCACACG CCTCAGATTT GGcaAACGAT CCCTTTATCC
 101  GgCaggttcT CGaccGTCAG CATTTCGaac ccgacggGAa ATACCaCCTA
 151  TTcggCaGCA GGGGGGAGCT TgccnagcGC aacggccATa tcggattggG
 201  aaacaTAcaa Agccatcagt tGggccacct gatgattcaa caggcggccg
 251  ttgaaggaaA TAtcgGctac attgtccgct tttccgatca cggcacaaa
 301  ttccattcgc ccttcGAcaa ccaTGCCTCA CATTCCGATT CTGACGAAGC
 351  CGGTAGTCCC GTTGACGGAT TCAGCCTTTA CCGCATCCAT TGGGACGGAT
 401  ACGAACACCA TCCCGCCGAC GGCTATGACG GGCCACAGGG CGGCGGCTAT
 451  CCCGCTCCCA AAGGCGCGAG GGATATATAC AGCTACGACA TAAAAGGCGT
 501  TGCCCAAAAT ATCCGCCTCA ACCTGACCGA CAACCGCAGC ACCGGACAAC
 551  GGCTTGCCGA CCGTTTCCAC AATGCCGGCG CTATGCTGAC GCAAGGAGTA
 601  GGCGACGGAT TCAAACGCGC CACCCGATAC AGCCCCGAGC TGGACAGATC
 651  GGGCAATGCc gccGAAGCCT TCAACGGCAC TGCAGATATC GTCAAAAACA
 701  TCATCGGCGC GGCAGGAGAA ATTGTCGGCG CAGGCGATGC CGTGCagGGT
 751  ATAAGCGAAG GCTCAAACAT TGCTGTCATG CACGGCTTGG GTCTGCTTTC
 801  CACCGAAAAC AAGATGGCGC GCATCAACGA TTTGGCAGAT ATGGCGCAAC
 851  TCAAAGACTA TGCCGCAGCA GCCATCCGCG ATTGGGCAGT CCAAAACCCC
 901  AATGCCGCAC AAGGCATAGA AGCCGTCAGC AATATCTTTA TGGCAGCCAT
 951  CCCCATCAAA GGGATTGGAG CTGTCCGGGG AAAATACGGC TTGGGCGGCA
1001  TCACGGCACA TCCTGTCAAG CGGTCGCAGA TGGGCGCGAT CGCATTGCCG
1051  AAAGGGAAAT CCGCCGTCAG CGACAATTTT GCCGATGCGG CATACGCCAA
1101  ATACCCGTCC CCTTACCATT CCCGAAATAT CCGTTCAAAC TTGGAGCAGC
```

```
1151  GTTACGGCAA AGAAAACATC ACCTCCTCAA CCGTGCCGCC GTCAAACGGC
1201  AAAAATGTCA AACTGGCAGA CCAACGCCAC CCGAAGACAG GCGTACCGTT
1251  TGACGGTAAA GGGTTTCCGA ATTTTGAGAA GCACGTGAAA TATGATACGA
1301  AGCTCGATAT TCAAGAATTA TCGGGGGGCG GTATACCTAA GGCTAAGCCT
1351  GTGTTTGATG CGAAACCGAG ATGGGAGGTT GATAGGAAGC TTAATAAATT
1401  GACAACTCGT GAGCAGGTGG AGAAAAATGT TCAGGAAACG AGAAGAAGGA
1451  GTCAGAGTAG TCAGTTTAAA GCCCATGCGC AACGAGAATG GGAAAATAAA
1501  ACAGGGTTAG ATTTTAATCA TTTTATAGGT GGTGATATCA ATAAGAAAGG
1551  CACAGTAACA GGAGGGCATA GTCTAACCCG TGGTGATGTA CGGGTGATAC
1601  AACAAACCTC GGCACCTGAT AAACATGGGG TTTATCAAGC GACAGTGGAA
1651  ATTAAAAAGC CTGATGGAAG TTGGGAGGTG AAAACGAAAA AAGGTGGGAA
1701  AGTGATGACC AAGCACACCA TGTTCCCAAA AGATTGGGAT GAGGCTAGAA
1751  TTAGGGCTGA AGTTACTTCG GCTTGGGAAA GTAGAATAAT GCTTAAGGAT
1801  AATAAATGGC AGGGTACAAG TAAATCGGGT ATTAAAATAG AAGGATTTAC
1851  CGAACCTAAT AGAACAGCAT ATCCCATTTA TGAATAG
```

This corresponds to the amino acid sequence <SEQ ID 464; ORF46ng-1>:

```
  1  LGISRKISLI LSILAVCLPM HAHASDLAND PFIRQVLDRQ HFEPDGKYHL
 51  FGSRGELAXR NGHIGLGNIQ SHQLGHLMIQ QAAVEGNIGY IVRFSDHGHK
101  FHSPFDNHAS HSDSDEAGSP VDGFSLYRIH WDGYEHHPAD GYDGPQGGGY
151  PAPKGARDIY SYDIKGVAQN IRLNLTDNRS TGQRLADRFH NAGAMLTQGV
201  GDGFKRATRY SPELDRSGNA AEAFNGTADI VKNIIGAAGE IVGAGDAVQG
251  ISEGSNIAVM HGLGLLSTEN KMARINDLAD MAQLKDYAAA AIRDWAVQNP
301  NAAQGIEAVS NIFMAAIPIK GIGAVRGKYG LGGITAHPVK RSQMGAIALP
351  KGKSAVSDNF ADAAYAKYPS PYHSRNIRSN LEQRYGKENI TSSTVPPSNG
401  KNVKLADQRH PKTGVPFDGK GFPNFEKHVK YDTKLDIQEL SGGGIPKAKP
451  VFDAKPRWEV DRKLNKLTTR EQVEKNVQET RRRSQSSQFK AHAQREWENK
501  TGLDFNHFIG GDINKKGTVT GGHSLTRGDV RVIQQTSAPD KHGVYQATVE
551  IKKPDGSWEV KTKKGGKVMT KHTMFPKDWD EARIRAEVTS AWESRIMLKD
601  NKWQGTSKSG IKIEGFTEPN RTAYPIYE*
```

ORF46ng-1 and ORF46-1 show 94.7% identity in 227 aa overlap:

```
                                       10        20        30        40
orf46-1.pep                   AVCLPMHAHASXLANDSFIRQVLDRQHFEPDGKYHLFGSRGELAER
                              |||||||||| |||| ||||||||||||||||||||||||||| |
orf46ng-1        LGISRKISLILSILAVCLPMHAHASDLANDPFIRQVLDRQHFEPDGKYHLFGSRGELAXR
                         10        20        30        40        50        60

                  50        60        70        80        90       100
orf46-1.pep      QSHIGLGKIQSHQLGNLMIQQAAIKGNIGYIVRFSDHGHEVHSPFDNHASHSDSDEAGSP
                 ::|||||:|||||||:|||||||::|||||||||||||||: ||||||||||||||||||
orf46ng-1        NGHIGLGNIQSHQLGHLMIQQAAVEGNIGYIVRFSDHGHKFHSPFDNHASHSDSDEAGSP
                         70        80        90       100       110       120

                 110       120       130       140       150       160
orf46-1.pep      VDGFSLYRIHWDGYEHHPADGYDGPQGGGYPAPKGARDIYSYDIKGVAQNIRLNLTDNRS
                 ||||||||||||||||||||||||||||||||||||||||||||||||||||||||||||
orf46ng-1        VDGFSLYRIHWDGYEHHPADGYDGPQGGGYPAPKGARDIYSYDIKGVAQNIRLNLTDNRS
                        130       140       150       160       170       180

                 170       180       190       200       210       220
orf46-1.pep      TGQRLADRFHNAGSMLTQGVGDGFKRATRYSPELDRSGNAAEAFNGTADIVKNIIGAAGE
                 ||||||||||||:|||||||||||||||||||||||||||||||||||||||||||||||
orf46ng-1        TGQRLADRFHNAGAMLTQGVGDGFKRATRYSPELDRSGNAAEAFNGTADIVKNIIGAAGE
                        190       200       210       220       230       240

orf46-1.pep      I
                 |
orf46ng-1        IVGAGDAVQGISEGSNIAVMHGLGLLSTENKMARINDLADMAQLKDYAAAAIRDWAVQNP
                        250       260       270       280       290       300
```

Homology with a predicted ORF from *N.meningitidis* strain A)

ORF46ng-1 shows 87.4% identity over a 486aa overlap with an ORF (ORF46a) from strain A of *N. meningitidis:*

```
                    10        20        30        40        50        60
```

```
orf46a.pep        LGISRKISLILSILAVCLPMHAHASDLANDSFIRQVLDRQHFEPDGKYHLFGSRGELAER
                  |||||||||||||||||||||||||||| ||||||||||||||||||||||||||||| |
orf46ng-1         LGISRKISLILSILAVCLPMHAHASDLANDPFIRQVLDRQHFEPDGKYHLFGSRGELAXR
                          10        20        30        40        50        60

                          70        80        90       100       110       120
orf46a.pep        SGHIGLGNIQSHQLGNLFIQQAAIKGNIGYIVRFSDHGHEVHSPFDNHASHSDSDEAGSP
                  :||||||||||||||:|:|||||::|||||||||||||||: ||||||||||||||||||
orf46ng-1         NGHIGLGNIQSHQLGHLMIQQAAVEGNIGYIVRFSDHGHKFHSPFDNHASHSDSDEAGSP
                          70        80        90       100       110       120

                         130       140       150       160       170       180
orf46a.pep        VDGFSLYRIHWDGYEHHPADGYDGPQGGGYPAPKGARDIYSYDIKGVAQNIRLNLTDNRS
                  ||||||||||||||||||||||||||||||||||||||||||||||||||||||||||||
orf46ng-1         VDGFSLYRIHWDGYEHHPADGYDGPQGGGYPAPKGARDIYSYDIKGVAQNIRLNLTDNRS
                         130       140       150       160       170       180

                         190       200       210       220       230       240
orf46a.pep        TGQRLVDRFHNTGSMLTQGVGDGFKRATRYSPELDRSGNAAEAFNGTADIVKNIIGAAGE
                  |||||:|||||:|:||||||||||||||||||||||||||||||||||||||||||||||
orf46ng-1         TGQRLADRFHNAGAMLTQGVGDGFKRATRYSPELDRSGNAAEAFNGTADIVKNIIGAAGE
                         190       200       210       220       230       240

                         250       260       270       280       290       300
orf46a.pep        IVGAGDAVQGISEGSNIAVMHGLGLLSTENKMARINDLADMAQLKDYAAAAIRDWAVQNP
                  ||||||||||||||||||||||||||||||||||||||||||||||||||||||||||||
orf46ng-1         IVGAGDAVQGISEGSNIAVMHGLGLLSTENKMARINDLADMAQLKDYAAAAIRDWAVQNP
                         250       260       270       280       290       300

                         310       320       330       340       350       360
orf46a.pep        NAAQGIEAVSNIFTAVIPVKGIGAVRGKYGLGGITAHPVKRSQMGEIALPKGKSAVSDNF
                  ||||||||||||| |:||:|||||||||||||||||||||||||| ||||||||||||||
orf46ng-1         NAAQGIEAVSNIFMAAIPIKGIGAVRGKYGLGGITAHPVKRSQMGAIALPKGKSAVSDNF
                         310       320       330       340       350       360

                         370       380       390       400       410       420
orf46a.pep        ADAAYAKYPSPYHSRNIRSNLEQRYGKENITSSTVPPSNGKNVKLANKRHPKTKVPFDGK
                  ||||||||||||||||||||||||||||||||||||||||||||||::|||||| |||||
orf46ng-1         ADAAYAKYPSPYHSRNIRSNLEQRYGKENITSSTVPPSNGKNVKLADQRHPKTGVPFDGK
                         370       380       390       400       410       420

                         430       440           450        460       470
orf46a.pep        GFPNFEKDVKYDTRINTAVPQVN----PIDEPVFN--PKGSVGSAHSWSITARIQYAKLP
                  ||||||| |||||:::  : :::    |  :|||:  |:  |    : ::|:| |  |
orf46ng-1         GFPNFEKHVKYDTKLD--IQELSGGGIPKAKPVFDAKPRWEVDRKLN-KLTTREQVEKNV
                         430         440       450       460        470

                     480       490       500       510       520       530
orf46a.pep        RQGRIRYIPPKNYSPSAPLPKGPNNGYLDKFGNEWTKGPSRTKGQEFEWDVQLSKTGREQ
                  :: | |
orf46ng-1         QETRRRSQSSQFKAHAQREWENKTGLDFNHFIGGDINKKGTVTGGHSLTRGDVRVIQQTS
                  480       490       500       510       520       530
```

The complete length ORF46a DNA sequence <SEQ ID 465> is:

```
   1  TTGGGCATTT CCCGCAAAAT ATCCCTTATT CTGTCCATAC TGGCAGTGTG
  51  CCTGCCGATG CATGCACACG CCTCAGATTT GGCAAACGAT TCTTTTATCC
 101  GGCAGGTTCT CGACCGTCAG CATTTCGAAC CCGACGGGAA ATACCACCTA
 151  TTCGGCAGCA GGGGGGAACT TGCCGAGCGC AGCGGTCATA TCGGATTGGG
 201  AAACATACAA AGCCATCAGT TGGGCAACCT GTTCATCCAG CAGGCGGCCA
 251  TTAAAGGAAA TATCGGCTAC ATTGTCCGCT TTTCCGATCA CGGGCACGAA
 301  GTCCATTCCC CCTTCGACAA CCATGCCTCA CATTCCGATT CTGATGAAGC
 351  CGGTAGTCCC GTTGACGGAT TCAGCCTTTA CCGCATCCAT TGGGACGGAT
 401  ACGAACACCA TCCCGCCGAC GGCTATGACG GGCCACAGGG CGGCGGCTAT
 451  CCCGCTCCCA AAGGCGCGAG GGATATATAC AGCTACGACA TAAAAGGCGT
 501  TGCCCAAAAT ATCCGCCTCA ACCTGACCGA CAACCGCAGC ACCGGACAAC
 551  GGCTTGTCGA CCGTTTCCAC AATACCGGTA GTATGCTGAC GCAAGGAGTA
 601  GGCGACGGAT TCAAACGCGC CACCCGATAC AGCCCCGAGC TGGACAGATC
 651  GGGCAATGCC GCCGAAGCTT TCAACGGCAC TGCAGATATC GTCAAAAACA
 701  TCATCGGCGC GGCAGGAGAA ATTGTCGGCG CAGGCGATGC CGTGCAGGGT
 751  ATAAGCGAAG GCTCAAACAT TGCTGTTATG CACGGCTTGG GTCTGCTTTC
 801  CACCGAAAAC AAGATGGCGC GCATCAACGA TTTGGCAGAT ATGGCGCAAC
```

```
 851  TCAAAGACTA TGCCGCAGCA GCCATCCGCG ATTGGGCAGT CCAAAACCCC
 901  AATGCCGCAC AAGGCATAGA AGCCGTCAGC AATATCTTTA CGGCAGTCAT
 951  CCCCGTCAAA GGGATTGGAG CTGTTCGGGG AAAATACGGC TTGGGCGGCA
1001  TCACGGCACA TCCTGTCAAG CGGTCGCAGA TGGGCGAGAT CGCATTGCCG
1051  AAAGGGAAAT CCGCCGTCAG CGACAATTTT GCCGATGCGG CATACGCCAA
1101  ATACCCGTCC CCTTACCATT CCCGAAATAT CCGTTCAAAC TTGGAGCAGC
1151  GTTACGGCAA AGAAAACATC ACCTCCTCAA CCGTGCCGCC GTCAAACGGA
1201  AAGAATGTGA AACTGGCAAA CAAACGCCAC CCGAAGACCA AAGTGCCGTT
1251  TGACGGTAAA GGGTTTCCGA ATTTTGAAAA AGACGTAAAA TACGATACGA
1301  GAATTAATAC CGCTGTACCA CAAGTGAATC CTATAGATGA ACCCGTCTTT
1351  AATCCTAAAG GTTCTGTCGG ATCGGCTCAT TCTTGGTCTA TAACTGCCAG
1401  AATTCAATAC GCAAAATTAC CAAGGCAAGG TAGAATCAGA TATATCCCAC
1451  CTAAAAATTA CTCTCCTTCA GCACCGCTAC CAAAAGGACC TAATAATGGA
1501  TATTTGGATA AATTTGGTAA TGAATGGACT AAAGGTCCAT CAAGAACTAA
1551  AGGTCAAGAA TTTGAATGGG ATGTTCAATT GTCTAAAACA GGAAGAGAGC
1601  AACTTGGATG GGCTAGTAGG GATGGTAAGC ATTTAAATAT ATCAATTGAT
1651  GGAAAGATTA CACACAAATG A
```

This corresponds to the amino acid sequence <SEQ ID 466>:

```
   1  LGISRKISLI LSILAVCLPM HAHASDLAND SFIRQVLDRQ HFEPDGKYHL
  51  FGSRGELAER SGHIGLGNIQ SHQLGNLFIQ QAAIKGNIGY IVRFSDHGHE
 101  VHSPFDNHAS HSDSDEAGSP VDGFSLYRIH WDGYEHHPAD GYDGPQGGGY
 151  PAPKGARDIY SYDIKGVAQN IRLNLTDNRS TGQRLVDRFH NTGSMLTQGV
 201  GDGFKRATRY SPELDRSGNA AEAFNGTADI VKNIIGAAGE IVGAGDAVQG
 251  ISEGSNIAVM HGLGLLSTEN KMARINDLAD MAQLKDYAAA AIRDWAVQNP
 301  NAAQGIEAVS NIFTAVIPVK GIGAVRGKYG LGGITAHPVK RSQMGEIALP
 351  KGKSAVSDNF ADAAYAKYPS PYHSRNIRSN LEQRYGKENI TSSTVPPSNG
 401  KNVKLANKRH PKTKVPFDGK GFPNFEKDVK YDTRINTAVP QVNPIDEPVF
 451  NPKGSVGSAH SWSITARIQY AKLPRQGRIR YIPPKNYSPS APLPKGPNNG
 501  YLDKFGNEWT KGPSRTKGQE FEWDVQLSKT GREQLGWASR DGKHLNISID
 551  GKITHK*
```

Based on this analysis, including the presence of a RGD sequence in the gonococcal protein, typical of adhesins, it is predicted that the proteins from *N.meningitidis* and *N.gonorrhoeae,* and their epitopes, could be useful antigens for vaccines or diagnostics, or for raising antibodies.

**Example 56**

The following partial DNA sequence was identified in *N.meningitidis* <SEQ ID 467>:

```
  1 ATGAATATTC ACACCCTGCT CTCCAAACAA TGGACGCTGC CGCCATTCCT
 51 GCCGAAACGG CTGCTGCTGT CCCTGCTGAT ACTGCTTGCC CCCAATGCGG
101 TGTTTTGGGT TTTGGCACTG CTGACCGCCA CCGCCCGCCC GATTGTCAAT
151 TTGGACTATC TTCCCGCCGC GCTGCTGATC GCCCTGCCTT GGCGTTTCGT
201 CAAAATTGCC GGCGTATTGG CGTTTTGGCT GGCGGTTTTG TTTGACGGGC
251 TGATGATGGT GATCCAACTC TTCCCTTTTA TGGATCTCAT CGGCGCCATC
301 AACCTCGTCC CCTTCATCCT GACCGCCCCC GCCCCTTATC AGATAATGAC
351 CGGGCTG...
```

This corresponds to the amino acid sequence <SEQ ID 468; ORF48>:

```
  1 MNIHTLLSKQ WTLPPFLPKR LLLSLLILLA PNAVFWVLAL LTATARPIVN
 51 LDYLPAALLI ALPWRFVKIA GVLAFWLAVL FDGLMMVIQL FPFMDLIGAI
101 NLVPFILTAP APYQIMTGL...
```

Further work revealed the complete nucleotide sequence <SEQ ID 469>:

```
   1 ATGAATATTC ACACCCTGCT CTCCAAACAA TGGACGCTGC CGCCATTCCT
  51 GCCGAAACGG CTGCTGCTGT CCCTGCTGAT ACTGCTTGCC CCCAATGCGG
 101 TGTTTTGGGT TTTGGCACTG CTGACCGCCA CCGCCCGCCC GATTGTCAAT
 151 TTGGACTATC TTCCCGCCGC GCTGCTGATC GCCCTGCCTT GGCGTTTCGT
 201 CAAAATTGCC GGCGTATTGG CGTTTTGGCT GGCGGTTTTG TTTGACGGGC
 251 TGATGATGGT GATCCAACTC TTCCCTTTTA TGGATCTCAT CGGCGCCATC
 301 AACCTCGTCC CCTTCATCCT GACCGCCCCC GCCCCTTATC AGATAATGAC
 351 CGGGCTGTTG CTGCTGTATA TGCTGGCGAT GCCGTTTGTG TTGCAGAAAG
 401 CCGCCGCCAA AACCGACTTC CGGCACATTG CCGTCTGCGC CGCCGTTGTG
 451 GCGGCAGCCG GCTATTTCAC CGGCCATTTG AGTTACTACG ACCGGGGTCG
 501 GATGGCCAAT ATCTTCGGCG CAAACAACTT CTACTACGCC AAAAGTCAGG
 551 CGATGCTCTA CACCGTCAGC CAGAATGCCG ACTTTATTAC CGCCGGCCTG
 601 GTCGATCCCG TCTTCCTCCC CTTGGGCAAT CAACAGCGTG CCGCCACGCA
 651 TCTGAACGAG CCGAAATCTC AAAAAATCCT CTTTATCGTC GCCGAATCTT
 701 GGGGGCTGCC GGCCAATCCC GAACTTCAAA ACGCCACTTT TGCCAAACTG
 751 CTGGCGCAAA AAGACCGTTT TTCGGTTTGG GAAAGCGGCA GTTTTCCCTT
 801 CATCGGCGCG ACGGTCGAAG GCGAAATGCG CGAACTGTGT GCCTACGGCG
 851 GTTTGCGCGG GTTCGCACTG CGCCGCGCGC CCGACGAAAA ATTTGCCCGC
 901 TGCCTCCCCA ACCGTTTGAA ACAAGAAGGT TACGCCACCT TTGCGATGCA
 951 CGGCGCGGGC AGTTCGCTTT ACGACCGCTT CAGCTGGTAT CCGAGGGCGG
1001 GCTTTCAAGA AATCAAAACC GCCGAAAACC TGATCGGTAA AAAAACCTGC
1051 GCCATTTTCG GCGGCGTGTG CGACAGCGAG CTGTTCGGCG AAGTGTCGGC
1101 ATTTTTCAAA AAACACGACA AGGGACTGTT TTACTGGATG ACGCTGACCA
1151 GCCACGCCGA CTATCCCGAA TCCGACATTT TCAACCACAG GCTCAAATGC
1201 ACCGAATATG GCCTGCCCGC CGAAACCGAC CTCTGCCGCA ATTTCAGCCT
1251 GCACACCCAA TTCTTCGACC AACTGGCGGA TTTGATCCAA CGCCCCGAAA
1301 TGAAAGGCAC GGAAGTCATC ATCGTCGGCG ACCATCCGCC GCCCGTCGGC
1351 AACCTCAATG AAACCTTCCG CTACCTCAAA CAGGGGCACG TCGCCTGGCT
1401 GAACTTCAAA ATCAAATAA
```

This corresponds to the amino acid sequence <SEQ ID 470; ORF48-1>:

```
  1  MNIHTLLSKQ WTLPPFLPKR LLLSLLILLA PNAVFWVLAL LTATARPIVN
 51  LDYLPAALLI ALPWRFVKIA GVLAFWLAVL FDGLMMVIQL FPFMDLIGAI
101  NLVPFILTAP APYQIMTGLL LLYMLAMPFV LQKAAAKTDF RHIAVCAAVV
151  AAAGYFTGHL SYYDRGRMAN IFGANNFYYA KSQAMLYTVS QNADFITAGL
201  VDPVFLPLGN QQRAATHLNE PKSQKILFIV AESWGLPANP ELQNATFAKL
251  LAQKDRFSVW ESGSFPFIGA TVEGEMRELC AYGGLRGFAL RRAPDEKFAR
301  CLPNRLKQEG YATFAMHGAG SSLYDRFSWY PRAGFQEIKT AENLIGKKTC
351  AIFGGVCDSE LFGEVSAFFK KHDKGLFYWM TLTSHADYPE SDIFNHRLKC
401  TEYGLPAETD LCRNFSLHTQ FFDQLADLIQ RPEMKGTEVI IVGDHPPPVG
451  NLNETFRYLK QGHVAWLNFK IK*
```

Computer analysis of this amino acid sequence gave the following results:

Homology with a predicted ORF from *N.meningitidis* (strain A)

ORF48 shows 94.1 % identity over a 119aa overlap with an ORF (ORF48a) from strain A of *N. meningitidis:*

```
                    10        20        30        40        50        60
orf48.pep   MNIHTLLSKQWTLPPFLPKRLLLSLLILLAPNAVFWVLALLTATARPIVNLDYLPAALLI
            |||||||||||||||||||||||||||||| |||||||||||||||||||| ||||||||
orf48a      MNIHTLLSKQWTLPPFLPKRLLLSLLILLXPNAVFWVLALLTATARPIVNLXYLPAALLI
                    10        20        30        40        50        60

                    70        80        90       100       110       119
orf48.pep   ALPWRFVKIAGVLAFWLAVLFDGLMMVIQLFPFMDLIGAINLVPFILTAPAPYQIMTGL
            ||||| ||| |||| |||||||||||||||||||||||||||||||| |||| |||||||
orf48a      ALPWRXVKIXGVLAXWLAVLFDGLMMVIQLFPFMDLIGAINLVPFIXTAPALYQIMTGLL
                    70        80        90       100       110       120

orf48a      LLYMLAMPFVLQKAAAKTDFRHIAACAAVVVAAGYFTGHLSXYDRGRMANIFGANNFYYA
                   130       140       150       160       170       180
```

The complete length ORF48a nucleotide sequence <SEQ ID 471> is:

```
  1  ATGAATATTC ACACCCTGCT CTCCAAACAA TGGACGCTGC CGCCATTCCT
 51  GCCGAAACGG CTGCTGCTGT CCCTGCTGAT ACTGCTNNCC CCCAATGCGG
101  TGTTTTGGGT TTTGGCACTG CTGACCGCCA CCGCCCGCCC GATTGTCAAT
151  TTGGANTACC TTCCCGCCGC GCTGCTGATC GCCCTGCCTT GGCGTNTCGT
201  CAAAATTGNC GGCGTATTGG CGTNTTGGCT GGCGGTTTTG TTTGACGGGC
251  TGATGATGGT GATCCAACTC TTCCCTTTTA TGGATCTCAT CGGCGCCATC
301  AACCTCGTCC CCTTCATCNT GACCGCCCCC GCCCTTTATC AGATAATGAC
351  CGGGCTGTTA CTGCTGTATA TGCTGGCGAT GCCGTTTGTG TTGCAGAAAG
401  CCGCCGCCAA AACCGACTTC CGACACATTG CCGCCTGTGC CGCCGTTGTG
451  GTGGCAGCCG GCTATTTTAC CGGCCATTTG AGTTANTACG ACCGGGGGCG
501  GATGGCCAAT ATCTTCGGCG CAAACAACTT CTATTACGCC AAAAGTCAGG
```

```
 551  CGATGCTCTA CACCGTCAGC CAGAATGCCG ACTTTATTAC CGCCGGCCTG
 601  GTCGATCCCG TCTTCCTCCC CTTGGGCAAT CAACAGCGTG CCGCCACGCA
 651  TCTGAACGAG CCGAAATCTC AAAAAATCCT CTTTATCGTC GCCGAATCTT
 701  GGGGGCTGCC GGCCAATCCC GAACTTCAAA ACGCCACTTT TGCCAAACTG
 751  CTGGCGCAAA AAGANCGTTT TTCGGTTTGG GAAAGCGGCA GTTTTCCCTT
 801  CATCGGCGCG ACGATCGAAG GCGAAATGCG CGAACTGTGT GCCTACGGCG
 851  GTTTGCGCGG GTTCGCACTG CGCCGCGCGC CCGACGAAAA ATTTGCCCGC
 901  TGCCTCCCCA ACCGTTTGAA ACAAGAAGGT TACGCCACCT TTGCGATGCA
 951  CGGCGCGGGC AGTTCGCTTT ACGACCGCTT CAGCTGGTAT CCGAGGGCGG
1001  GCTTTCAAGA AATCAAAACC GCCGAAAACC TGATCGGTAA AAAAACCTGC
1051  GCCATTTTCG GCGGCGTGTG CGACAGCGAG CTGTTCGGCG AAGTGTCGGC
1101  ANTTTTCAAA AAACACGACA AGGGACTGTT TTACTGGATG ACGCTGACCA
1151  GCCACGCCGA CTATCCCGAA TCNGACATTT TCAACCACAG GCTCAAATGC
1201  ACCGAATATG GCCTGCCCGC CGAAACCGAC NTCTGCCGCA ATTTCAGCCT
1251  GCACACCCAA TTCTTCGACC AACTGGCGGA TTTGATCCAA CGCCCCGAAA
1301  TGAAAGGCAC GGAAGTCATC ATCGTCGGCG ACCATCCGCC GCCCGTCGGC
1351  AACCTCAATG AAACCTTCCG CTACCTCAAA CAGGGGCACG TCGNCTGGCT
1401  GAACTTCAAA ATCAAATAA
```

This encodes a protein having amino acid sequence <SEQ ID 472>:

```
  1  MNIHTLLSKQ WTLPPFLPKR LLLSLLILLX PNAVFWVLAL LTATARPIVN
 51  LXYLPAALLI ALPWRXVKIX GVLAXWLAVL FDGLMMVIQL FPFMDLIGAI
101  NLVPFIXTAP ALYQIMTGLL LLYMLAMPFV LQKAAAKTDF RHIAACAAVV
151  VAAGYFTGHL SXYDRGRMAN IFGANNFYYA KSQAMLYTVS QNADFITAGL
201  VDPVFLPLGN QQRAATHLNE PKSQKILFIV AESWGLPANP ELQNATFAKL
251  LAQKXRFSVW ESGSFPFIGA TIEGEMRELC AYGGLRGFAL RRAPDEKFAR
301  CLPNRLKQEG YATFAMHGAG SSLYDRFSWY PRAGFQEIKT AENLIGKKTC
351  AIFGGVCDSE LFGEVSAXFK KHDKGLFYWM TLTSHADYPE SDIFNHRLKC
401  TEYGLPAETD XCRNFSLHTQ FFDQLADLIQ RPEMKGTEVI IVGDHPPPVG
451  NLNETFRYLK QGHVXWLNFK IK*
```

ORF48a and ORF48-1 show 96.8% identity in 472 aa overlap:

```
                        10        20        30        40        50        60
orf48a.pep  MNIHTLLSKQWTLPPFLPKRLLLSLLILLXPNAVFWVLALLTATARPIVNLXYLPAALLI
            ||||||||||||||||||||||||||||||| ||||||||||||||||||| ||||||||
orf48-1     MNIHTLLSKQWTLPPFLPKRLLLSLLILLAPNAVFWVLALLTATARPIVNLDYLPAALLI
                    10        20        30        40        50        60

                        70        80        90       100       110       120
orf48a.pep  ALPWRXVKIXGVLAXWLAVLFDGLMMVIQLFPFMDLIGAINLVPFIXTAPALYQIMTGLL
            ||||| ||| ||| |||||||||||||||||||||||||||||| |||| ||||||||||
orf48-1     ALPWRFVKIAGVLAFWLAVLFDGLMMVIQLFPFMDLIGAINLVPFILTAPAPYQIMTGLL
                    70        80        90       100       110       120

                       130       140       150       160       170       180
orf48a.pep  LLYMLAMPFVLQKAAAKTDFRHIAACAAVVVAAGYFTGHLSXYDRGRMANIFGANNFYYA
            |||||||||||||||||||||||:|||||:||||||||||| ||||||||||||||||||
orf48-1     LLYMLAMPFVLQKAAAKTDFRHIAVCAAVVAAAGYFTGHLSYYDRGRMANIFGANNFYYA
                   130       140       150       160       170       180

                       190       200       210       220       230       240
orf48a.pep  KSQAMLYTVSQNADFITAGLVDPVFLPLGNQQRAATHLNEPKSQKILFIVAESWGLPANP
            ||||||||||||||||||||||||||||||||||||||||||||||||||||||||||||
orf48-1     KSQAMLYTVSQNADFITAGLVDPVFLPLGNQQRAATHLNEPKSQKILFIVAESWGLPANP
                   190       200       210       220       230       240

                       250       260       270       280       290       300
orf48a.pep  ELQNATFAKLLAQKXRFSVWESGSFPFIGATIEGEMRELCAYGGLRGFALRRAPDEKFAR
            ||||||||||||| ||||||||||||||:|||||||||||||||||||||||||||||||
orf48-1     ELQNATFAKLLAQKDRFSVWESGSFPFIGATVEGEMRELCAYGGLRGFALRRAPDEKFAR
                   250       260       270       280       290       300

                       310       320       330       340       350       360
orf48a.pep  CLPNRLKQEGYATFAMHGAGSSLYDRFSWYPRAGFQEIKTAENLIGKKTCAIFGGVCDSE
            ||||||||||||||||||||||||||||||||||||||||||||||||||||||||||||
orf48-1     CLPNRLKQEGYATFAMHGAGSSLYDRFSWYPRAGFQEIKTAENLIGKKTCAIFGGVCDSE
                   310       320       330       340       350       360

                       370       380       390       400       410       420
orf48a.pep  LFGEVSAXFKKHDKGLFYWMTLTSHADYPESDIFNHRLKCTEYGLPAETDXCRNFSLHTQ
            ||||||| |||||||||||||||||||||||||||||||||||||||||||| |||||||
orf48-1     LFGEVSAFFKKHDKGLFYWMTLTSHADYPESDIFNHRLKCTEYGLPAETDLCRNFSLHTQ
                   370       380       390       400       410       420

                       430       440       450       460       470
orf48a.pep  FFDQLADLIQRPEMKGTEVIIVGDHPPPVGNLNETFRYLKQGHVXWLNFKIKX
            |||||||||||||||||||||||||||||||||||||||||||| ||||||||
orf48-1     FFDQLADLIQRPEMKGTEVIIVGDHPPPVGNLNETFRYLKQGHVAWLNFKIKX
                   430       440       450       460       470
```

Homology with a predicted ORF from *N.gonorrhoeae*

ORF48 shows 97.5% identity over a 119aa overlap with a predicted ORF (ORF48ng) from *N. gonorrhoeae:*

```
orf48.pep     MNIHTLLSKQWTLPPFLPKRLLLSLLILLAPNAVFWVLALLTATARPIVNLDYLPAALLI   60
              ||||:|||:|||||||||||||||||||||||||||||||||||||||||||||||||||
orf48ng       MNIHALLSEQWTLPPFLPKRLLLSLLILLAPNAVFWVLALLTATARPIVNLDYLPAALLI   60

orf48.pep     ALPWRFVKIAGVLAFWLAVLFDGLMMVIQLFPFMDLIGAINLVPFILTAPAPYQIMTGL   119
              ||||||||||||||| |||||||||||||||||||||||||||||||||||||||||||
orf48ng       ALPWRFVKIAGVLAFWPAVLFDGLMMVIQLFPFMDLIGAINLVPFILTAPAPYQIMTGLL  120
```

The ORF48ng nucleotide sequence <SEQ ID 473> was predicted to encode a protein having amino acid sequence <SEQ ID 474>:

```
  1  MNIHALLSEQ WTLPPFLPKR LLLSLLILLA PNAVFWVLAL LTATARPIVN
 51  LDYLPAALLI ALPWRFVKIA GVLAFWPAVL FDGLMMVIQL FPFMDLIGAI
101  NLVPFILTAP APYQIMTGLL LLYMLAMPFV LQKAAVKTDF RHIAVCAAVV
151  AAARYFTGPF ELLRTGGRWQ YVQHRRLLLS GSRASFRRRQ KADVLRRLGN
201  PYASMGNGG..
```

Further work identified the complete gonococcal DNA sequence <SEQ ID 475>:

```
   1  ATGAATATTC ACGCCCTGCT CTCCGAACAA TGGACGCTGC CGCCATTCCT
  51  GCCGAAACGG CTGCTGCTGT CCCTGCTGAT ACTGCTGGCC CCCAATGCGG
 101  TGTTTTGGGT TTTGGCACTG CTGACCGCCA CCGCCCGCCC GATTGTCAAT
 151  TTGGACTACC TTCCCGCCGC GCTGCTGATC GCCCTGCCTT GGCGTTTCGT
 201  CAAAATTGCC GGCGTATTGG CGTTTTGGCC GGCGGTTTTG TTTGACGGGC
 251  TGATGATGGT GATCCAACTC TTCCCTTTTA TGGACCTCAT CGGCGCCATC
 301  AACCTCGTCC CCTTCATCCT GACCGCCCCC GCCCCTTATC AGATAATGAC
 351  CGGGCTGTTG CTGCTGTATA TGCTGGCGAT GCCGTTTGTG TTGCAAAAAG
 401  CCGCCGTCAA AACCGACTTC CGACACATTG CCGTCTGTGC CGCCGTTGTG
 451  GCGGCAGCCG GCTATTTCAC CGGCCATTTG AGTTACTACG ACCGGGGGCG
 501  GATGGCCAAT ATCTTCGGCG CAAACAACTT CTATTACGCc aAAAGTCAGG
 551  CGATGCTCTA CACCGTCAGC CAGAATGCCG ACTTTATTAC CGCCGgcctG
 601  GTCGACCCCG TCTTCCTCCC CTTGGGCAAT CAGCAGCGTG CCGCCACGCG
 651  GCTGAGTGAG CCGAAATCTC AAAAAATCCT CTTTATCGTC GCCGAATCTT
 701  GGGGGCTGCC GGGCAATCCC GAGCTTCAAA ACGCCACTTT TGCCAAACTG
 751  CTGGCGCAAA AAGACCGTTT TTCGGTTTGG GAAAGCGGCA GTTTTCCCTT
 801  CATCGGCGCG ACGGTCGAAG GCGAAATGCG CGAATTGTGC GCCTACGGCG
 851  GTTTGCGCGG GTTCGCACTG CGCCGCGCGC CCGACGAAAA ATTTGCCCGC
 901  TGCCTCCCCA ACCGTTTGAA ACAAGAAGGT TACGCCACCT TTGCGATGCA
 951  CGGCGCGGGT AGTTCGCTTT ACGACCGCTT CAGCTGGTAT CCGAGGGCGG
1001  GCTTTCAAAA AATCAAAACC GCCGAAAACC TGATCGGTAA AAAAACCTGC
1051  GCCATTTTCG GCGGCGTGTG CGACAGCGAG CTGTTCGGCG AAGTGTCGGC
1101  ATTTTTCAAA AAACACGACA AGGGACTGTT TTACTGGATG ACGCTGACCA
1151  GCCACGCCGA CTATCCCGAA TCCGACATTT TCAACCACAG GCTCAAATGC
1201  ACCGAATACG GCCTGCCCGC CGAAACCGAC CTCTGCCGCA ATTTCAGCCT
1251  GCACACCCAA TtcttcgACC AACTGGCGGA TTTGATCCGA CGCCCCGAAA
1301  TGAAAGGCAC GGAAGTCATC ATCGTCGGCG ACCATCCGCC GCCCGTCGGC
1351  AACCTCAATG AAACCTTCCG CTACCTCAAA CAGGGACACG TCGCCTGGCT
1401  GCACTTCAAA ATCAAATAA
```

This encodes a protein having amino acid sequence <SEQ ID 476; ORF48ng-1>:

```
  1  MNIHALLSEQ WTLPPFLPKR LLLSLLILLA PNAVFWVLAL LTATARPIVN
 51  LDYLPAALLI ALPWRFVKIA GVLAFWPAVL FDGLMMVIQL FPFMDLIGAI
```

```
101  NLVPFILTAP APYQIMTGLL LLYMLAMPFV LQKAAVKTDF RHIAVCAAVV
151  AAAGYFTGHL SYYDRGRMAN IFGANNFYYA KSQAMLYTVS QNADFITAGL
201  VDPVFLPLGN QQRAATRLSE PKSQKILFIV AESWGLPGNP ELQNATFAKL
251  LAQKDRFSVW ESGSFPFIGA TVEGEMRELC AYGGLRGFAL RRAPDEKFAR
301  CLPNRLKQEG YATFAMHGAG SSLYDRFSWY PRAGFQKIKT AENLIGKKTC
351  AIFGGVCDSE LFGEVSAFFK KHDKGLFYWM TLTSHADYPE SDIFNHRLKC
401  TEYGLPAETD LCRNFSLHTQ FFDQLADLIR RPEMKGTEVI IVGDHPPPVG
451  NLNETFRYLK QGHVAWLHFK IK*
```

ORG48ng-1 and ORF48-1 show 97.9% identity in 472 aa overlap:

```
                        10        20        30        40        50        60
orf48-1.pep     MNIHTLLSKQWTLPPFLPKRLLLSLLILLAPNAVFWVLALLTATARPIVNLDYLPAALLI
                ||||:|||:|||||||||||||||||||||||||||||||||||||||||||||||||||
orf48ng-1       MNIHALLSEQWTLPPFLPKRLLLSLLILLAPNAVFWVLALLTATARPIVNLDYLPAALLI
                        10        20        30        40        50        60

                        70        80        90       100       110       120
orf48-1.pep     ALPWRFVKIAGVLAFWLAVLFDGLMMVIQLFPFMDLIGAINLVPFILTAPAPYQIMTGLL
                |||||||||||||||||| |||||||||||||||||||||||||||||||||||||||||
orf48ng-1       ALPWRFVKIAGVLAFWPAVLFDGLMMVIQLFPFMDLIGAINLVPFILTAPAPYQIMTGLL
                        70        80        90       100       110       120

                       130       140       150       160       170       180
orf48-1.pep     LLYMLAMPFVLQKAAAKTDFRHIAVCAAVVAAAGYFTGHLSYYDRGRMANIFGANNFYYA
                |||||||||||||||:||||||||||||||||||||||||||||||||||||||||||||
orf48ng-1       LLYMLAMPFVLQKAAVKTDFRHIAVCAAVVAAAGYFTGHLSYYDRGRMANIFGANNFYYA
                       130       140       150       160       170       180

                       190       200       210       220       230       240
orf48-1.pep     KSQAMLYTVSQNADFITAGLVDPVFLPLGNQQRAATHLNEPKSQKILFIVAESWGLPANP
                ||||||||||||||||||||||||||||||||||||:|:|||||||||||||||||:||
orf48ng-1       KSQAMLYTVSQNADFITAGLVDPVFLPLGNQQRAATRLSEPKSQKILFIVAESWGLPGNP
                       190       200       210       220       230       240

                       250       260       270       280       290       300
orf48-1.pep     ELQNATFAKLLAQKDRFSVWESGSFPFIGATVEGEMRELCAYGGLRGFALRRAPDEKFAR
                ||||||||||||||||||||||||||||||||||||||||||||||||||||||||||||
orf48ng-1       ELQNATFAKLLAQKDRFSVWESGSFPFIGATVEGEMRELCAYGGLRGFALRRAPDEKFAR
                       250       260       270       280       290       300

                       310       320       330       340       350       360
orf48-1.pep     CLPNRLKQEGYATFAMHGAGSSLYDRFSWYPRAGFQEIKTAENLIGKKTCAIFGGVCDSE
                ||||||||||||||||||||||||||||||||||||:|||||||||||||||||||||||
orf48ng-1       CLPNRLKQEGYATFAMHGAGSSLYDRFSWYPRAGFQKIKTAENLIGKKTCAIFGGVCDSE
                       310       320       330       340       350       360

                       370       380       390       400       410       420
orf48-1.pep     LFGEVSAFFKKHDKGLFYWMTLTSHADYPESDIFNHRLKCTEYGLPAETDLCRNFSLHTQ
                ||||||||||||||||||||||||||||||||||||||||||||||||||||||||||||
orf48ng-1       LFGEVSAFFKKHDKGLFYWMTLTSHADYPESDIFNHRLKCTEYGLPAETDLCRNFSLHTQ
                       370       380       390       400       410       420

                       430       440       450       460       470
orf48-1.pep     FFDQLADLIQRPEMKGTEVIIVGDHPPPVGNLNETFRYLKQGHVAWLNFKIKX
                |||||||||:|||||||||||||||||||||||||||||||||||||:|||||
orf48ng-1       FFDQLADLIRRPEMKGTEVIIVGDHPPPVGNLNETFRYLKQGHVAWLHFKIKX
                       430       440       450       460       470
```

Based on this analysis, including the presence of a putative leader sequence (double-underlined) and two putative transmembrane domains (single-underlined) in the gonococcal protein, it is predicted that the proteins from *N.meningitidis* and *N.gonorrhoeae,* and their epitopes, could be useful antigens for vaccines or diagnostics, or for raising antibodies.

**Example 57**

The following partial DNA sequence was identified in *N.meningitidis* <SEQ ID 477>:

```
  1  ..GTGAGCGGAC GTTACCGCGC TTTGGATCGC GTTTCCAAAA TCATCATCGT
 51    TACTTTGAGT ATCGCCACGC TTGCCGCCGC CGGCATCGCT ATGTCGCGCG
101    GTATGCAGAT GCAGTCCGAT TTTATCGAGC CGACACCGTG GACGCTTGCC
151    GGTTTGGGCT TCCTGATCGC GCTGATGGGC TGGATGCCCG CGCCGATTGA
201    AATTTCCGCC ATCAATTCTT TGTGGGTAAC CGAAAAACAA CGCATCAATC
251    CTTCCGAATA CCGCGACGGG ATTTTTGAAT TCAACGTCGG TTATATCGCC
301    AGTGCGGTTT TGGCTTTGGT TTTCCTTGCA CTGGGCGC.G TAGCGCCGAA
351    CGGCAACGGC GA.ACAGTGC AGATGGCGGG CGGCAAATAT AACGGGCAAT
401    TGATCAATAT GTACGCC..
```

This corresponds to the amino acid sequence <SEQ ID 478; ORF53>:

```
  1  ..VSGRYRALDR VSKIIIVTLS IATLAAAGIA MSRGMQMQSD FIEPTPWTLA
 51    GLGFLIALMG WMPAPIEISA INSLWVTEKQ RINPSEYRDG IFEFNVGYIA
101    SAVLALVFLA LGXVAPNGNG XTVQMAGGKY NGQLINMYA..
```

Further work revealed the complete nucleotide sequence <SEQ ID 479>:

```
   1  ATGTCCGAAC AACATATTTC GACTTGGAAA AGTAAAATCA ACGCATTGGG
  51  TCCGGGGATC ATGATGGCTT CGGCGGCGGT CGGCGGTTCG CACCTGATTG
 101  CCTCGACGCA GGCGGGCGCG CTTTACGGCT GGCAGATCGC GCTCATCATC
 151  ATCCTGACCA ACCTCTTCAA ATACCCGTTT TTCCGCTTCA GCGCGCATTA
 201  CACGCTGGAC ACGGGCAAGA GCCTGATTGA AGGTTATGCC GAGAAAAGCC
 251  GCGTTTATTT GTGGGTATTC CTGATTTTGT GCATCCTCTC CGCCACGATT
 301  AACGCGGGCG CGGTCGCCAT TGTAACCGCC GCCATCGTCA AAATGGCGAT
 351  TCCCTCGCTG ATGTTTGATG CCGGCACGGT TGCCGCCTTG ATTATGGCAT
 401  CCTGCCTGAT TATTTTGGTG AGCGGACGTT ACCGCGCTTT GGATCGCGTT
 451  TCCAAAATCA TCATCGTTAC TTTGAGTATC GCCACGCTTG CCGCCGCCGG
 501  CATCGCTATG TCGCGCGGTA TGCAGATGCA GTCCGATTTT ATCGAGCCGA
 551  CACCGTGGAC GCTTGCCGGT TTGGGCTTCC TGATCGCGCT GATGGGCTGG
 601  ATGCCCGCGC CGATTGAAAT TTCCGCCATC AATTCTTTGT GGGTAACCGA
 651  AAAACAACGC ATCAATCCTT CCGAATACCG CGACGGGATT TTTGATTTCA
 701  ACGTCGGTTA TATCGCCAGT GCGGTTTTGG CTTTGGTTTT CCTTGCACTG
 751  GGCGCGTTTG TGCAATACGG CAACGGCGAA GCAGTGCAGA TGGCGGGCGG
 801  CAAATATATC GGGCAATTGA TCAATATGTA CGCCGTTACC ATCGGCGGCT
 851  GGTCGCGCCC GCTGGTGGCG TTTATCGCGT TTGCCTGTAT GTACGGCACG
 901  ACGATTACCG TCGTGGACGG CTATGCCCGT GCCATTGCCG AACCCGTGCG
 951  CCTGCTGCGC GGAAAAGACA AAACGGGCAA CGCCGAATTC TTTGCCTGGA
1001  ATATTTGGGT GGCGGGCAGC GGTTTGGCGG TGATTTTCTG GTTTGACGGC
1051  GTAATGGCGA ATCTGCTCAA ATTTGCGATG ATTGCCGCTT TTGTGTCCGC
1101  CCCTGTGTTT GCCTGGCTGA ATTACCGTTT GGTTAAAGGT GATGAAAAAC
1151  ACAAACTCAC ATCAGGTATG AATGCCCTTG CATTGGCAGG CTTGATTTAT
1201  CTGACCGGTT TTACCGTTTT GTTCTTATTG AATTTGGCGG GAATGTTCAA
1251  ATGA
```

This corresponds to the amino acid sequence <SEQ ID 480; ORF53-1>:

```
  1 MSEQHISTWK SKINALGPGI MMASAAVGGS HLIASTQAGA LYGWQIALII
 51 ILTNLFKYPF FRFSAHYTLD TGKSLIEGYA EKSRVYLWVF LILCILSATI
101 NAGAVAIVTA AIVKMAIPSL MFDAGTVAAL IMASCLIILV SGRYRALDRV
151 SKIIIVTLSI ATLAAAGIAM SRGMQMQSDF IEPTPWTLAG LGFLIALMGW
201 MPAPIEISAI NSLWVTEKQR INPSEYRDGI FDFNVGYIAS AVLALVFLAL
251 GAFVQYGNGE AVQMAGGKYI GQLINMYAVT IGGWSRPLVA FIAFACMYGT
301 TITVVDGYAR AIAEPVRLLR GKDKTGNAEF FAWNIWVAGS GLAVIFWFDG
351 VMANLLKFAM IAAFVSAPVF AWLNYRLVKG DEKHKLTSGM NALALAGLIY
401 LTGFTVLFLL NLAGMFK*
```

Computer analysis of this amino acid sequence gave the following results:

Homology with a predicted ORF from *N.meningitidis* (strain A)

ORF53 shows 93.5% identity over a 139aa overlap with an ORF (ORF53a) from strain A of *N. meningitidis:*

```
                                                            10        20        30
orf53.pep                                           VSGRYRALDRVSKIIIVTLSIATLAAAGIA
                                                    ||||||||||||||||||||||||||||||
orf53a          AAIVKMAIPSLMFDAGTVAALIMASCLIILVSGRYRALDRVSKIIIVTLSIATLAAAGIA
              110       120       130       140       150       160

                          40        50        60        70        80        90
orf53.pep       MSRGMQMQSDFIEPTPWTLAGLGFLIALMGWMPAPIEISAINSLWVTEKQRINPSEYRDG
                ||||||||||||||||||||||||||||||||||||||||||||||||||||||||||||
orf53a          MSRGMQMQSDFIEPTPWTLAGLGFLIALMGWMPAPIEISAINSLWVTEKQRINPSEYRDG
              170       180       190       200       210       220

                        100       110       120       130      139
orf53.pep       IFEFNVGYIASAVLALVFLALGXVAPNGNGXTVQMAGGKYNGQLINMYA
                ||:|||||||||||||||||||  :  ||| :||||||| ||||||||
orf53a          IFDFNVGYIASAVLALVFLALGAFVQYGNGEAVQMAGGKYIGQLINMYAVTIGGWSRPLV
              230       240       250       260       270       280

orf53a          AFIAFACMYGTTITVVDGYARAIAEPVRLLRGKDKTGNAEFFAWNIWVAGSGLAVIFWFD
              290       300       310       320       330       340
```

The complete length ORF53a nucleotide sequence <SEQ ID 481> is:

```
   1  ATGTCCGAAC AACATATTTC GACTTGGAAA AGTAAAATCA ACGCATTGGG
  51  ACCGGGGATT ATGATGGCTT CGGCGGCGGT CGGCGGTTCG CACCTGATTG
 101  CCTCGACGCA GGCGGGCGCG CTTTACGGCT GGCAGATCGC GCTCATCATC
 151  ATCCTGACCA ACCTCTTCAA ATACCCGTTT TTCCGCTTCA GCGCGCATTA
 201  CACGCTGGAC ACGGGCAAGA GCCTGATTGA AGGTTATGCC GAGAAAAGCC
 251  GCGTTTATTT GTGGGTATTC CTGATTTTGT GCATCCTCTC CGCCACGATT
 301  AACGCGGGCG CGGTCGCCAT TGTAACCGCC GCCATCGTCA AAATGGCGAT
 351  TCCCTCGCTG ATGTTTGATG CCGGCACGGT TGCCGCCTTG ATTATGGCAT
 401  CCTGCCTGAT TATTTTGGTG AGCGGACGTT ACCGCGCTTT GGATCGCGTT
 451  TCCAAAATCA TCATCGTTAC TTTGAGTATC GCCACGCTTG CCGCCGCCGG
 501  CATCGCTATG TCGCGCGGTA TGCAGATGCA GTCCGATTTT ATCGAGCCGA
 551  CACCGTGGAC GCTTGCCGGT TTGGGCTTCC TGATCGCGCT GATGGGCTGG
 601  ATGCCCGCGC CGATTGAAAT TTCCGCCATC AATTCTTTGT GGGTAACCGA
 651  AAAACAACGC ATCAATCCTT CCGAATACCG CGACGGGATT TTTGATTTCA
 701  ACGTCGGTTA TATCGCCAGT GCGGTTTTGG CTTTGGTTTT CCTTGCACTG
 751  GGCGCGTTTG TGCAATACGG CAACGGCGAA GCAGTGCAGA TGGCGGGCGG
 801  CAAATATATC GGGCAATTGA TCAATATGTA CGCCGTTACC ATCGGCGGCT
 851  GGTCGCGCCC GCTGGTGGCG TTTATCGCGT TTGCCTGTAT GTACGGCACG
 901  ACGATTACCG TTGTGGACGG CTATGCCCGT GCCATTGCCG AACCCGTGCG
 951  CCTGCTGCGC GGAAAAGACA AAACGGGCAA CGCCGAATTC TTTGCCTGGA
1001  ATATTTGGGT GGCGGGCAGC GGTTTGGCGG TGATTTTCTG GTTTGACGGC
1051  GTAATGGCGA ATCTGCTCAA ATTTGCGATG ATTGCCGCTT TTGTGTCCGC
1101  CCCTGTGTTT GCCTGGCTGA ATTACCGTTT GGTCAAAGGT GATGAAAAAC
1151  ACAAACTCAC ATCAGGTATG AATGCCCTTG CATTGGCAGG CTTGATTTAT
1201  CTGACCGGTT TTACCGTTTT GTTCTTATTG AATTTGGCGG GAATGTTCAA
1251  ATGA
```

This encodes a protein having amino acid sequence <SEQ ID 482>:

```
  1  MSEQHISTWK SKINALGPGI MMASAAVGGS HLIASTQAGA LYGWQIALII
 51  ILTNLFKYPF FRFSAHYTLD TGKSLIEGYA EKSRVYLWVF LILCILSATI
101  NAGAVAIVTA AIVKMAIPSL MFDAGTVAAL IMASCLIILV SGRYRALDRV
151  SKIIIVTLSI ATLAAAGIAM SRGMQMQSDF IEPTPWTLAG LGFLIALMGW
201  MPAPIEISAI NSLWVTEKQR INPSEYRDGI FDFNVGYIAS AVLALVFLAL
251  GAFVQYGNGE AVQMAGGKYI GQLINMYAVT IGGWSRPLVA FIAFACMYGT
301  TITVVDGYAR AIAEPVRLLR GKDKTGNAEF FAWNIWVAGS GLAVIFWFDG
351  VMANLLKFAM IAAFVSAPVF AWLNYRLVKG DEKHKLTSGM NALALAGLIY
401  LTGFTVLFLL NLAGMFK*
```

ORF 53a shows 100.0% identity in 417 aa overlap with ORF53-1:

```
                      10        20        30        40        50        60
orf53a.pep  MSEQHISTWKSKINALGPGIMMASAAVGGSHLIASTQAGALYGWQIALIIILTNLFKYPF
            ||||||||||||||||||||||||||||||||||||||||||||||||||||||||||||
orf53-1     MSEQHISTWKSKINALGPGIMMASAAVGGSHLIASTQAGALYGWQIALIIILTNLFKYPF
                      10        20        30        40        50        60

                      70        80        90       100       110       120
```

```
orf53a.pep     FRFSAHYTLDTGKSLIEGYAEKSRVYLWVFLILCILSATINAGAVAIVTAAIVKMAIPSL
               ||||||||||||||||||||||||||||||||||||||||||||||||||||||||||||
orf53-1        FRFSAHYTLDTGKSLIEGYAEKSRVYLWVFLILCILSATINAGAVAIVTAAIVKMAIPSL
                          70        80        90       100       110       120

                         130       140       150       160       170       180
orf53a.pep     MFDAGTVAALIMASCLIILVSGRYRALDRVSKIIIVTLSIATLAAAGIAMSRGMQMQSDF
               ||||||||||||||||||||||||||||||||||||||||||||||||||||||||||||
orf53-1        MFDAGTVAALIMASCLIILVSGRYRALDRVSKIIIVTLSIATLAAAGIAMSRGMQMQSDF
                         130       140       150       160       170       180

                         190       200       210       220       230       240
orf53a.pep     IEPTPWTLAGLGFLIALMGWMPAPIEISAINSLWVTEKQRINPSEYRDGIFDFNVGYIAS
               ||||||||||||||||||||||||||||||||||||||||||||||||||||||||||||
orf53-1        IEPTPWTLAGLGFLIALMGWMPAPIEISAINSLWVTEKQRINPSEYRDGIFDFNVGYIAS
                         190       200       210       220       230       240

                         250       260       270       280       290       300
orf53a.pep     AVLALVFLALGAFVQYGNGEAVQMAGGKYIGQLINMYAVTIGGWSRPLVAFIAFACMYGT
               ||||||||||||||||||||||||||||||||||||||||||||||||||||||||||||
orf53-1        AVLALVFLALGAFVQYGNGEAVQMAGGKYIGQLINMYAVTIGGWSRPLVAFIAFACMYGT
                         250       260       270       280       290       300

                         310       320       330       340       350       360
orf53a.pep     TITVVDGYARAIAEPVRLLRGKDKTGNAEFFAWNIWVAGSGLAVIFWFDGVMANLLKFAM
               ||||||||||||||||||||||||||||||||||||||||||||||||||||||||||||
orf53-1        TITVVDGYARAIAEPVRLLRGKDKTGNAEFFAWNIWVAGSGLAVIFWFDGVMANLLKFAM
                         310       320       330       340       350       360

                         370       380       390       400       410
orf53a.pep     IAAFVSAPVFAWLNYRLVKGDEKHKLTSGMNALALAGLIYLTGFTVLFLLNLAGMFKX
               ||||||||||||||||||||||||||||||||||||||||||||||||||||||||||
orf53-1        IAAFVSAPVFAWLNYRLVKGDEKHKLTSGMNALALAGLIYLTGFTVLFLLNLAGMFKX
                         370       380       390       400       410
```

Homology with a predicted ORF from *N.gonorrhoeae*

ORF53 shows 92.1 % identity over a 139aa overlap with a predicted ORF (ORF53ng) from *N. gonorrhoeae:*

```
orf53.pep                                 VSGRYRALDRVSKIIIVTLSIATLAAAGIA   30
                                          ||||||||||||||||||||||||||||||
orf53ng    AAIVKMAIPSLMFDAGTVAALIMASCLIILVSGRYRALDRVSKIIIVTLSIATLAAAGIA   91

orf53.pep  MSRGMQMQSDFIEPTPWTLAGLGFLIALMGWMPAPIEISAINSLWVTEKQRINPSEYRDG   90
           |||||||| |||||||||||||||||||||||||||||||||||||||||||||||||||
orf53ng    MSRGMQMQPDFIEPTPWTLAGLGFLIALMGWMPAPIEISAINSLWVTEKQRINPSEYRDG  151

orf53.pep  IFEFNVGYIASAVLALVFLALGXVAPNGNGXTVQMAGGKYNGQLINMYA             139
           ||:||||||||||||||||||| :   ||| :|||:|||| ||||||||
orf53ng    IFDFNVGYIASAVLALVFLALGAFVQYGNGEAVQMGGGKYIGQLINMYAVTIGGGSRPLV  211
```

An ORF53ng nucleotide sequence <SEQ ID 483> was predicted to encode a protein having amino acid sequence <SEQ ID 484>:

```
  1  MPKKSCVYLW VFLILCIASA TINAGAVAIV TAAIVKMAIP SLMFDAGTVA
 51  ALIMASCLII LVSGRYRALD RVSKIIIVTL SIATLAAAGI AMSRGMQMQP
101  DFIEPTPWTL AGLGFLIALM GWMPAPIEIS AINSLWVTEK QRINPSEYRD
151  GIFDFNVGYI ASAVLALVFL ALGAFVQYGN GEAVQMGGGK YIGQLINMYA
201  VTIGGGSRPL VAFIAFACMY GAASTVVDGY ARAIAEPVRL LRGKDKTARP
251  IVLLEKLGGR HRFGRDFLV*
```

Further analysis revealed further partial DNA gonococcal sequence <SEQ ID 485>:

```
   1  ..aagaAAAGCT GCGTTTATTT GTGGGTTTTT TTGATTTTGT GTATCGCCTC
  51    CGCCACGATT AACGCGGGCG CGGTCGCCAT TGTAACCGCC GCCATCGTCA
 101    AAATGGCGAT TCCCTCGCTG ATGTTTGATG CCGGCACGGT TGCCGCCTTG

 151    ATTATGGCAT CCTGCCTGAT TATTTTGGTG AGCGGACGTT ACCGCGCTTT
 201    GGATCGTGTT TCCAAAATCA TCATTGTTAC TTTGAGCATC GCCACGCTTG
 251    CCGCCGCCGG CATCGCTATG TCGCGCGGTA TGCAGATGCA GCCCGATTTT
 301    ATCGAGCCGA CACCGTGGAC GCTTGCCGGT TTGGGCTTCC TGATCGCGCT
 351    GATGGGCTGG ATGCCCGCGC CGATCGAAAT TTCCGCCATC AATTCTTTGT
 401    GGGTAACCGA AAAACAACGC ATCAATCCTT CTGAATACCG CGACGGGATT
 451    TTCGATTTCA ACGTCGGTTA TATCGCcagT GCGGTTTTGG CTTTGGTTTT
 501    CCTTGCACTG GGCGCGTTTG TGCAATACGG CAACGGCGAA GCAGTGCAGA
 551    TGGCGGGCGG CAAATATATC GGGCAATTGA TTAATATGTA TGCCGTAACC
 601    ATCGGCGGCT GGTCTCGTCC GCTGGTGGCG TTTATCGCGT TTGCCTGTAT
 651    GTACGGCACG ACGATTACCG TTGTGGACGG TTATGCGCGT GCCATTGCCG
 701    AACCCGTGCG CCTGCTGCGC GGCAGGGATA AAACCGGCAA CGCCGAGTTG
 751    TTtgccTGGA ATATTTGGGT GGCGGGCAGC GGTTTGGCGG TGATTTTCTG
 801    GTTTGACggc gcaaTGGCgG AACtgcTCAA ATTTGCGATG ATtgccgcCT
 851    TTGTGTCCGC CCCTGTGTTC GCCTGGCTCA ACTACCGCCT CGTCAAAGGG
 901    GACAAACGCC ACAGGCTTAC CGCCGGTATG AACGCCCTTG CCATTGTCGG
 951    CCTGCTCTAC CTGGCCGGGT TTGCCGTTTT GTTCCTGTTG AACCTTACCG
1001    GACTTTTGGC ATAG
```

This corresponds to the amino acid sequence <SEQ ID 486; ORF53ng-1>:

```
   1  ..KKSCVYLWVF LILCIASATI NAGAVAIVTA AIVKMAIPSL MFDAGTVAAL
  51    IMASCLIILV SGRYRALDRV SKIIIVTLSI ATLAAAGIAM SRGMQMQPDF
 101    IEPTPWTLAG LGFLIALMGW MPAPIEISAI NSLWVTEKQR INPSEYRDGI
 151    FDFNVGYIAS AVLALVFLAL GAFVQYGNGE AVQMAGGKYI GQLINMYAVT
 201    IGGWSRPLVA FIAFACMYGT TITVVDGYAR AIAEPVRLLR GRDKTGNAEL
 251    FAWNIWVAGS GLAVIFWFDG AMAELLKFAM IAAFVSAPVF AWLNYRLVKG
 301    DKRHRLTAGM NALAIVGLLY LAGFAVLFLL NLTGLLA*
```

ORF53ng-1 and ORF53-1 show 94.0% identity in 336 aa overlap:

```
                      60        70        80        90       100       110
orf53-1.pep       ILTNLFKYPFFRFSAHYTLDTGKSLIEGYAEKSRVYLWVFLILCILSATINAGAVAIVTA
                                                ::|| ||||||||||| ||||||||||||||
orf53ng-1                                       KKSCVYLWVFLILCIASATINAGAVAIVTA
                                                        10        20        30

                     120       130       140       150       160       170
orf53-1.pep       AIVKMAIPSLMFDAGTVAALIMASCLIILVSGRYRALDRVSKIIIVTLSIATLAAAGIAM
                  ||||||||||||||||||||||||||||||||||||||||||||||||||||||||||||
orf53ng-1         AIVKMAIPSLMFDAGTVAALIMASCLIILVSGRYRALDRVSKIIIVTLSIATLAAAGIAM
                      40        50        60        70        80        90

                     180       190       200       210       220       230
orf53-1.pep       SRGMQMQSDFIEPTPWTLAGLGFLIALMGWMPAPIEISAINSLWVTEKQRINPSEYRDGI
                  ||||||| ||||||||||||||||||||||||||||||||||||||||||||||||||||
orf53ng-1         SRGMQMQPDFIEPTPWTLAGLGFLIALMGWMPAPIEISAINSLWVTEKQRINPSEYRDGI
                     100       110       120       130       140       150

                     240       250       260       270       280       290
orf53-1.pep       FDFNVGYIASAVLALVFLALGAFVQYGNGEAVQMAGGKYIGQLINMYAVTIGGWSRPLVA
                  ||||||||||||||||||||||||||||||||||||||||||||||||||||||||||||
orf53ng-1         FDFNVGYIASAVLALVFLALGAFVQYGNGEAVQMAGGKYIGQLINMYAVTIGGWSRPLVA
                     160       170       180       190       200       210

                     300       310       320       330       340       350
orf53-1.pep       FIAFACMYGTTITVVDGYARAIAEPVRLLRGKDKTGNAEFFAWNIWVAGSGLAVIFWFDG
                  |||||||||||||||||||||||||||||||:|||||||:||||||||||||||||||||
orf53ng-1         FIAFACMYGTTITVVDGYARAIAEPVRLLRGRDKTGNAELFAWNIWVAGSGLAVIFWFDG
                     220       230       240       250       260       270

                     360       370       380       390       400       410
orf53-1.pep       VMANLLKFAMIAAFVSAPVFAWLNYRLVKGDEKHKLTSGMNALALAGLIYLTGFTVLFLL
                  :||:|||||||||||||||||||||||||||::|:||:||||||::||:||:||:|||||
orf53ng-1         AMAELLKFAMIAAFVSAPVFAWLNYRLVKGDKRHRLTAGMNALAIVGLLYLAGFAVLFLL
                     280       290       300       310       320       330

orf53-1.pep       NLAGMFKX
                  ||:|::
orf53ng-1         NLTGLLAX
```

Based on this analysis, including the presence of a putative leader sequence (double-underlined) and several putative transmembrane domains (single-underlined) in the gonococcal protein, it is predicted that the proteins from *N.meningitidis* and *N.gonorrhoeae,* and their epitopes, could be useful antigens for vaccines or diagnostics, or for raising antibodies.

**Example 58**

The following partial DNA sequence was identified in *N.meningitidis* <SEQ ID 487>:

```
  1  ..TTGCGGGAAA CGGCATATGT TTTGGATAGT TTTGATCGTT ATTTTGTTGT
 51    TGCGCTTGCC GGCTTGTTTT TTGTCCGCGC ACAATCCGAA CGCGAGTGGA
101    TGCGCGAGGT TTCTGCGTGG CAGGAAAAGA AAGGGGAAAA ACAGGCGGAG
151    CTGCCTGAAA TCAAAGACGG TATGCCCGAT TTTCCCGAAC TTGCCCTGAT
201    GCTTTTCCAC GCCGTCAAAA CGGCAGTGTA TTGGCTGTTT GTCGGTGTCG
251    TCCGTTTCTG CCGAAACTAT CTGGCGCACG AATCCGAACC GGACAGGCCC
301    GTTCCGCCT..
```

This corresponds to the amino acid sequence <SEQ ID 488; ORF58>:

1 ..LRETAYVLDS FDRYFVV*ALA GL*FFVRAQSE REWMREVSAW QEKKGEKQAE
51 LPEIKDGMPD FPELALMLFH AVKTAVYWLF VGVVRFCRNY LAHESEPDRP
101 VPP..

Further work revealed the complete nucleotide sequence <SEQ ID 489>:

1 ATGTTTTGGA TAGTTTTGAT CGTTATTTTG TTGCTTGCGC TTGCCGGCTT
51 GTTTTTTGTC CGCGCACAAT CCGAACGCGA GTGGATGCGC GAGGTTTCTG
101 CGTGGCAGGA AAAGAAAGGG GAAAAACAGG CGGAGCTGCC TGAAATCAAA
151 GACGGTATGC CCGATTTTCC CGAACTTGCC CTGATGCTTT TCCATGCCGT
201 CAAAACGGCA GTGTATTGGC TGTTTGTCGG TGTCGTCCGT TTCTGCCGAA
251 ACTATCTGGC GCACGAATCC GAACCGGACA GGCCCGTTCC GCCTGCTTCT
301 GCAAACCGTG CGGATGTTCC GACCGCATCC GACGGATATT CAGACAGTGG
351 AAACGGGACG GAAGAAGCGG AAACGGAAGA AGCAGAAGCT GCGGAGGAAG
401 AGGCTGCCGA TACGGAAGAC ATTGCAACTG CCGTAATCGA CAACCGCCGC
451 ATCCCATTCG ACCGGAGTAT TGCTGAAGGG TTGATGCCGT CTGAAAGCGA
501 AATTTCGCCC GTCCGTCCGG TTTTTAAAGA AATCACTTTG GAAGAAGCAA
551 CGCGTGCTTT AAACAGCGCG GCTTTAAGGG AAACGAAAAA ACGCTATATC
601 GATGCATTTG AGAAAAACGA AACAGCGGTC CCCAAAGTCC GCGTGTCCGA
651 TACCCCGATG GAAGGGCTGC AGATTATCGG TTTGGACGAC CCTGTGCTTC
701 AACGCACGTA TTCCCATATG TTCGATGCGG ACAAAGAAGC GTTTTCCGAG
751 TCTGCGGATT ACGGATTTGA GCCGTATTTT GAGAAGCAGC ATCCGTCTGC
801 CTTTTCTGCA GTCAAAGCCG AAAATGCACG GAATGCGCCG TTCCACCGTC
851 ATGCAGGGCA GGGGAAAGGG CAGGCGGAGG CAAAATCCCC GGATGTTTCC
901 CAAGGGCAGT CCGTTTCAGA CGGCACGGCC GTCCGCGATG CCCGCCGCCG
951 CGTTTCCGTC AATTTGAAAG AACCGAACAA GGCAACGGTT TCTGCGGAGG
1001 CGCGAATTTC TCGCCTGATT CCGGAAAGTC AGACGGTTGT CGGGAAACGG
1051 GATGTCGAAA TGCCGTCTGA AACCGAAAAT GTTTTCACGG AAACCGTTTC
1101 GTCTGTGGGA TACGGCGGTC CGGTTTATGA TGAAACTGCC GATATCCATA
1151 TTGAAGAACC TGCCGCGCCC GATGCTTGGG TGGTCGAACC ACCCGAAGTG
1201 CCGAAAGTTC CCATGACCGC AATCGATATT CAGCCGCCGC CTCCCGTATC
1251 GGAAATCTAC AACCGTACCT ATGAACCGCC GTCAGGATTC GAGCAGGTGC
1301 AACGCAGCCG CATTGCCGAG ACCGACCATC TTGCCGATGA TGTTTTGAAT
1351 GGAGGTTGGC AGGAGGAAAC CGCCGCTATT GCGGATGACG GCAGTGAAGG
1401 TGCGGCAGAG CGGTCAAGCG GGCAATATCT GTCGGAAACC GAAGCGTTCG
1451 GGCATGACAG TCAGGCGGTT TGTCCGTTTG AAAATGTGCC GTCTGAACGC
1501 CCGTCCTGCC GGGTATCGGA TACGGAAGCG GATGAAGGGG CGTTCCCATC
1551 TGAAGAAACC GGTGCGGTAT CCGAACACCT GCCGACAACC GACCTGCTTC
1601 TGCCTCCGCT GTTCAATCCC GAGGCGACGC AAACCGAAGA AGAACTGTTG
1651 GAAAACAGCA TCACCATCGA AGAAAAATTG GCGGAGTTCA AAGTCAAGGT
1701 CAAGGTTGTC GATTCTTATT CCGGCCCCGT AATTACGCGT TATGAAATCG
1751 AACCCGATGT CGGCGTGCGC GGCAATTCCG TTCTGAATCT GGAAAAAGAT
1801 TTGGCGCGTT CGCTCGGCGT GGCTTCCATC CGCGTTGTCG AAACCATCCC
1851 CGGCAAAACC TGCATGGGTT TGGAACTTCC GAACCCGAAA CGCCAAATGA
1901 TACGCCTGAG CGAAATCTTC AATTCGCCCG AGTTTGCCGA ATCCAAATCC

```
1951  AAGCTGACGC TCGCGCTCGG TCAGGACATC ACCGGACAGC CCGTCGTAAC
2001  CGACTTGGGA AAAGCACCGC ATTTGTTGGT TGCCGGCACG ACCGGTTCGG
2051  GCAAATCGGT GGGTGTCAAC GCGATGATTC TGTCTATGCT TTTCAAAGCC
2101  GCGCCGGAAG ACGTGCGTAT GATTATGATC GATCCGAAAA TGCTGGAATT
2151  GAGCATTTAC GAAGGCATCC CGCACCTGCT CGCCCCTGTC GTTACCGATA
2201  TGAAGCTGGC GGCAAACGCG CTGAACTGGT GTGTTAACGA AATGGAAAAA
2251  CGCTACCGCC TGATGAGCTT TATGGGCGTG CGTAATCTTG CGGGCTTCAA
2301  TCAAAAAATC GCCGAAGCCG CAGCAAGGGG AGAAAAAATC GGCAATCCGT
2351  TCAGCCTCAC GCCCGACGAT CCCGAACCTT TGGAAAAACT GCCGTTTATC
2401  GTGGTCGTGG TCGATGAGTT TGCCGACCTG ATGATGACGG CAGGCAAGAA
2451  AATCGAAGAA CTGATTGCCC GCCTCGCCCA AAAAGCCCGC GCGGCAGGCA
2501  TCCATTTGAT TCTTGCCACA CAACGCCCCA GCGTCGATGT CATCACGGGT
2551  CTGATTAAGG CGAACATCCC GACGCGTATC GCGTTCCAAG TGTCCAGCAA
2601  AATCGACAGC CGCACGATTC TCGACCAAAT GGGCGCGGAA AACCTGCTCG
2651  GTCAGGGCGA TATGCTGTTC CTGCTGCCGG GTACTGCCTA TCCGCAGCGC
2701  GTTCACGGCG CGTTTGCCTC GGATGAAGAG GTGCACCGCG TGGTCGAATA
2751  TTTGAAACAG TTTGGCGAAC CGGACTATGT TGACGATATT TTGAGCGGCG
2801  GCGGCAGCGA AGAGCTGCCC GGCATCGGGC GCAGCGGCGA CGACGAAACC
2851  GATCCGATGT ACGACGAGGC CGTATCCGTT GTCCTGAAAA CGCGCAAAGC
2901  CAGCATTTCG GGCGTACAGC GCGCCTTGCG TATCGGCTAC AACCGCGCCG
2951  CGCGTCTGAT TGACCAGATG GAGGCGGAAG GCATTGTGTC CGCACCGGAA
3001  CACAACGGCA ACCGTACGAT TCTCGTCCCC TTGGACAATG CTTGA
```

This corresponds to the amino acid sequence <SEQ ID 490; ORF58-1>:

```
   1  MFWIVLIVIL LLALAGLFFV RAQSEREWMR EVSAWQEKKG EKQAELPEIK
  51  DGMPDFPELA LMLFHAVKTA VYWLFVGVVR FCRNYLAHES EPDRPVPPAS
 101  ANRADVPTAS DGYSDSGNGT EEAETEEAEA AEEEAADTED IATAVIDNRR
 151  IPFDRSIAEG LMPSESEISP VRPVFKEITL EEATRALNSA ALRETKKRYI
 201  DAFEKNETAV PKVRVSDTPM EGLQIIGLDD PVLQRTYSHM FDADKEAFSE
 251  SADYGFEPYF EKQHPSAFSA VKAENARNAP FHRHAGQGKG QAEAKSPDVS
 301  QGQSVSDGTA VRDARRRVSV NLKEPNKATV SAEARISRLI PESQTVVGKR
 351  DVEMPSETEN VFTETVSSVG YGGPVYDETA DIHIEEPAAP DAWVVEPPEV
 401  PKVPMTAIDI QPPPPVSEIY NRTYEPPSGF EQVQRSRIAE TDHLADDVLN
 451  GGWQEETAAI ADDGSEGAAE RSSGQYLSET EAFGHDSQAV CPFENVPSER
 501  PSCRVSDTEA DEGAFPSEET GAVSEHLPTT DLLLPPLFNP EATQTEEELL
 551  ENSITIEEKL AEFKVKVKVV DSYSGPVITR YEIEPDVGVR GNSVLNLEKD
 601  LARSLGVASI RVVETIPGKT CMGLELPNPK RQMIRLSEIF NSPEFAESKS
 651  KLTLALGQDI TGQPVVTDLG KAPHLLVAGT TGSGKSVGVN AMILSMLFKA
 701  APEDVRMIMI DPKMLELSIY EGIPHLLAPV VTDMKLAANA LNWCVNEMEK
 751  RYRLMSFMGV RNLAGFNQKI AEAAARGEKI GNPFSLTPDD PEPLEKLPFI
 801  VVVVDEFADL MMTAGKKIEE LIARLAQKAR AAGIHLILAT QRPSVDVITG
 851  LIKANIPTRI AFQVSSKIDS RTILDQMGAE NLLGQGDMLF LLPGTAYPQR
 901  VHGAFASDEE VHRVVEYLKQ FGEPDYVDDI LSGGGSEELP GIGRSGDDET
 951  DPMYDEAVSV VLKTRKASIS GVQRALRIGY NRAARLIDQM EAEGIVSAPE
1001  HNGNRTILVP LDNA*
```

Computer analysis of this amino acid sequence predicts the indicated transmembrane region, and also gave the following results:

Homology with a predicted ORF from *N.meningitidis* (strain A)

ORF58 shows 96.6% identity over a 89aa overlap with an ORF (ORF58a) from strain A of *N. meningitidis:*

```
                   10        20        30        40        50        60
orf58.pep  LRETAYVLDSFDRYFVVALAGLFFVRAQSEREWMREVSAWQEKKGEKQAELPEIKDGMPD
                          :::||||||||||||||||||||||||||||||||||||||||||||
orf58a        MFWIVLIVILLLALAGLFFVRAQSEREWMREVSAWQEKKGEKQAELPEIKDGMPD
                      10        20        30        40        50

                   70        80        90       100
orf58.pep  FPELALMLFHAVKTAVYWLFVGVVRFCRNYLAHESEPDRPVPP
           |||||||||||||||||||||||||||||||||||||||||||
orf58a     FPELALMLFHAVKTAVYWLFVGVVRFCRNYLAHESEPDRPVPPASANRADVPTASDGYSD
             60        70        80        90       100       110
```

The complete length ORF58a nucleotide sequence <SEQ ID 491> is:

```
   1 ATGTTTTGGA TAGTTTTGAT CGTTATTTTG TTGCTTGCGC TTGCCGGCTT
  51 GTTTTTTGTC CGCGCACAAT CCGAACGCGA GTGGATGCGC GAGGTTTCTG
 101 CGTGGCAGGA AAAGAAAGGG GAAAAACAGG CGGAGCTGCC TGAAATCAAA
 151 GACGGTATGC CCGATTTTCC CGAACTTGCC CTGATGCTTT TCCATGCCGT
 201 CAAAACGGCA GTGTATTGGC TGTTTGTCGG TGTCGTCCGT TTCTGCCGAA
 251 ACTATCTGGC GCACGAATCC GAACCGGACA GGCCCGTTCC GCCTGCTTCT
 301 GCAAATCGTG CGGATGTTCC GACCGCATCC GACGGATATT CAGACAGTGG
 351 AAACGGGACG GAAGAAGCGG AAACGGAAGA AGCAGAAGCT GCGGAGGAAG
 401 AGGCTGCCGA TACGGAAGAC ATTGCAACTG CCGTAATCGA CAACCGCCGC
 451 ATCCCATTCG ACCGGAGTAT TGCTGAAGGG TTGATGCCGT CTGAAAGCGA
 501 AATTTCGCCC GTCCGTCCGG TTTTTAAGGA AATCACTTTG GAAGAAGCAA
 551 CGCGTGCTTT AAACAGCGCG GCTTTAAGGG AAACGAAAAA ACGCTATATC
 601 GATGCATTTG AGAAAAACGA AACAGCGGTC CCCAAAGTCC GCGTGTCCGA
 651 TACCCCGATG GAAGGGCTGC AGATTATCGG TTTGGACGAC CCTGTGCTTC
 701 AACGCACGTA TTCCCGTATG TTCGATGCGG ACAAAGAAGC GTTTTCCGAG
 751 TCTGCGGATT ACGGATTTGA GCCGTATTTT GAGAAGCAGC ATCCGTCTGC
 801 CTTTTCTGCA GTCAAAGCCG AAAATGCACG GAATGCGCCG TTCCGCCGTC
 851 ATGCAGGGCA GGGNAAAGGG CAGGCGGAGG CNAAATCCCC GGATGTTTCC
 901 CAAGGGCAGT CCGTTTCAGA CGGCACAGCC GTCCGCGATG CCNGCCGCCG
 951 CGTTTCCGTC AATTTGAAAG AACCGAACAA GGCAACGGTT TCTGCGGAGG
1001 CGCGGATTTC GCGCCTGATT CCGGAAAGTC GGACGGTTGT CGGGAAACGG
1051 GATGTCGAAA TGCCGTCTGA AACCGAAAAT GTTTTCACGG AAANTGTTTC
1101 GTCTGTGGGA TACGGCGNTC CGGTTTATGA TGAAACTGCC GATATCCATA
1151 TTGAAGAACC TGCCGCGCCC GATGCTTGGG TGGTCGAACC ACCCGAAGTG
1201 CCGAAAGTTC CCATGCCCGC AATNGATATT CCGCCGCCGC CTCCCGTATC
1251 GGAAATCTAC AACCGTACCT ATGAACCGCC GGCAGGATTC GAGCAGGTGC
1301 AACGCAGCCG CATTGCCGAA ACCGATCATC TTGCCGATGA TGTTTTGAAT
1351 GGAGGTTGGC AGGAGGAAAC CGCCGCTATT GCGAATGACG GCAGTGAGGG
1401 TGTGGCAGAG CGGTCAAGCG GGCAATATTT GTCGGAAACC GAAGCGTTCG
1451 GGCATGACAG TCAGGCGGTT TGTCCGTTTG AAAATGTGCC GTCTGAACGC
1501 CCGTCCCGCC GGGCATNGGA TACGGAAGCG GATGAAGGGG CGTTCCAATC
1551 TGAAGAAACC GGTGCGGTAT CCGAACACCT GCCGACAACC GACCTGCTTC
1601 TGCCGCCGCT GTTCAATCCC GGGGCGACGC AAACCGAAGA AGANCTGTTG
1651 GANAACAGCA TCACCATCGA AGAAAAATNG GCGGAGTTCA AAGTCAAGGT
1701 CAAGGTTGTC GATTCTTATT CCGGCCCCGT GATTACGCGT TATGAAATCG
1751 AACCCGATGT CGGCGTGCGC GGCAATTCCG TTCTAAATCT GGAAAAAGAN
1801 TTGGCGCGTT CGCTCGGCGT GGCTTCCATC CGCGTTGTCG AAACCATCCT
1851 CGGCAAAACC TGTATGGGTT TGGAACTTCC GAACCCGAAA CGCCAAATGA
1901 TACGCCTGAG CGAAATCTTC AATTCGCCCG AGTTTGCCGA ATCCAAATCC
1951 AAGCTGACGC TCGCGCTCGG TCAGGACATC ACCGGACAGC CCGTCGTAAC
2001 CGACTTGGGC AAAGCACCGC ATTTGTTGGT TGCCGGCACG ACCGGTTCGG
2051 GCAAATCGGT GGGTGTCAAC GCGATGATTC TGTCTATGCT TTTCAAAGCC
2101 GCGCCGGAAG ACGTGCGTAT GATTATGATC GATCCGAAAA TGCTGGAATT
2151 GAGCATTTAC GAAGGCATCC CGCACCTGCT CGCCCCTGTC GTTACCGATA
2201 TGAAGCTGGC GGCAAACGCG CTGAACTGGT GTGTTAACGA AATGGAAAAA
2251 CGCTACCGCC TGATGAGCTT TATGGGCGTG CGCAATCTTG CGGGTNTCAA
2301 TCAAAAAATC GCCGAAGCCG CAGCAAGGGG GGAGAAAATC GGCAACCCGT
2351 TCAGCCTCAC GCCCGACAAT CCCGAACCTT TGGANAAATT GCCGTTTATC
2401 GTGGTCGTGG TTGATGAGTT TGCCGACCTG ATGATGACGG CAGGCAAGAA
2451 AATCGAAGAA CTGATTGCCC GCCTCGCCCA AAAAGCCCGC GCGGCAGGCA
2501 TCCATCTTAT CCTTGCCACA CAACGCCCCA GTGTCGATGT CATCACGGGT
2551 CTGATTAAGG CGAACATCCC GACGCGTATC GCGTTCCAAG TGTCCAGCAA
2601 AATCGACAGC CGCACGATTC TTGACCAAAT GGGTGCGGAA AACCTGCTCG
2651 GGCAGGGCGA TATGCTGTTC CTGCCGCCGG GTACGGCCTA TCCGCAGCGC
2701 GTTCACGGCG CGTTTGCCTC GGATGAAGAG GTGCACCGCG TGGTCGAATA
2751 TCTGAAACAG TTTGGCGAAC CGGACTATGT TGACGATATN TTGAGCGGCG
2801 GTATGTCCGA CGATTTGCTG GGAATCAGCC GGAGCGGCGA CGGCGAAACC
2851 GATCCGATGT ACGACGAGGC CGTGTCNGTT GTTTTGAAAA CGCGCAAAGC
2901 CAGCATTTCT GGCGTGCAGC GCGCATTGCG TATCGGCTAT AATCGCGCCG
2951 CGCGTCTGAT TGACCAGATG GAGGCGGAAG GCATTGTGTC CGCACCGGAA
3001 CACAACGGCA ACCGTACGAT TCTCGTCCCC TTNGACAATG CTTGA
```

This encodes a protein having amino acid sequence <SEQ ID 492>:

```
   1  MFWIVLIVIL LLALAGLFFV RAQSEREWMR EVSAWQEKKG EKQAELPEIK
  51  DGMPDFPELA LMLFHAVKTA VYWLFVGVVR FCRNYLAHES EPDRPVPPAS
 101  ANRADVPTAS DGYSDSGNGT EEAETEEAEA AEEEAADTED IATAVIDNRR
 151  IPFDRSIAEG LMPSESEISP VRPVFKEITL EEATRALNSA ALRETKKRYI
 201  DAFEKNETAV PKVRVSDTPM EGLQIIGLDD PVLQRTYSRM FDADKEAFSE
 251  SADYGFEPYF EKQHPSAFSA VKAENARNAP FRRHAGQGKG QAEAKSPDVS
 301  QGQSVSDGTA VRDAXRRVSV NLKEPNKATV SAEARISRLI PESRTVVGKR
 351  DVEMPSETEN VFTEXVSSVG YGXPVYDETA DIHIEEPAAP wDAWVVEPPEV
```

```
 401  PKVPMPAXDI PPPPPVSEIY NRTYEPPAGF EQVQRSRIAE TDHLADDVLN
 451  GGWQEETAAI ANDGSEGVAE RSSGQYLSET EAFGHDSQAV CPFENVPSER
 501  PSRRAXDTEA DEGAFQSEET GAVSEHLPTT DLLLPPLFNP GATQTEEXLL
 551  XNSITIEEKX AEFKVKVKVV DSYSGPVITR YEIEPDVGVR GNSVLNLEKX
 601  LARSLGVASI RVVETILGKT CMGLELPNPK RQMIRLSEIF NSPEFAESKS
 651  KLTLALGQDI TGQPVVTDLG KAPHLLVAGT TGSGKSVGVN AMILSMLFKA
 701  APEDVRMIMI DPKMLELSIY EGIPHLLAPV VTDMKLAANA LNWCVNEMEK
 751  RYRLMSFMGV RNLAGXNQKI AEAAARGEKI GNPFSLTPDN PEPLXKLPFI
 801  VVVVDEFADL MMTAGKKIEE LIARLAQKAR AAGIHLILAT QRPSVDVITG
 851  LIKANIPTRI AFQVSSKIDS RTILDQMGAE NLLGQGDMLF LPPGTAYPQR
 901  VHGAFASDEE VHRVVEYLKQ FGEPDYVDDX LSGGMSDDLL GISRSGDGET
 951  DPMYDEAVSV VLKTRKASIS GVQRALRIGY NRAARLIDQM EAEGIVSAPE
1001  HNGNRTILVP XDNA*
```

ORF58a and ORF58-1 show 96.6% identity in 1014 aa overlap:

```
                        10        20        30        40        50        60
orf58a.pep   MFWIVLIVILLLALAGLFFVRAQSEREWMREVSAWQEKKGEKQAELPEIKDGMPDFPELA
             ||||||||||||||||||||||||||||||||||||||||||||||||||||||||||||
orf58-1      MFWIVLIVILLLALAGLFFVRAQSEREWMREVSAWQEKKGEKQAELPEIKDGMPDFPELA
                        10        20        30        40        50        60

                        70        80        90       100       110       120
orf58a.pep   LMLFHAVKTAVYWLFVGVVRFCRNYLAHESEPDRPVPPASANRADVPTASDGYSDSGNGT
             ||||||||||||||||||||||||||||||||||||||||||||||||||||||||||||
orf58-1      LMLFHAVKTAVYWLFVGVVRFCRNYLAHESEPDRPVPPASANRADVPTASDGYSDSGNGT
                        70        80        90       100       110       120

                       130       140       150       160       170       180
orf58a.pep   EEAETEEAEAAEEEAADTEDIATAVIDNRRIPFDRSIAEGLMPSESEISPVRPVFKEITL
             ||||||||||||||||||||||||||||||||||||||||||||||||||||||||||||
orf58-1      EEAETEEAEAAEEEAADTEDIATAVIDNRRIPFDRSIAEGLMPSESEISPVRPVFKEITL
                       130       140       150       160       170       180

                       190       200       210       220       230       240
orf58a.pep   EEATRALNSAALRETKKRYIDAFEKNETAVPKVRVSDTPMEGLQIIGLDDPVLQRTYSRM
             ||||||||||||||||||||||||||||||||||||||||||||||||||||||||||:|
orf58-1      EEATRALNSAALRETKKRYIDAFEKNETAVPKVRVSDTPMEGLQIIGLDDPVLQRTYSHM
                       190       200       210       220       230       240

                       250       260       270       280       290       300
orf58a.pep   FDADKEAFSESADYGFEPYFEKQHPSAFSAVKAENARNAPFRRHAGQGKGQAEAKSPDVS
             |||||||||||||||||||||||||||||||||||||||||:||||||||||||||||||
orf58-1      FDADKEAFSESADYGFEPYFEKQHPSAFSAVKAENARNAPFHRHAGQGKGQAEAKSPDVS
                       250       260       270       280       290       300

                       310       320       330       340       350       360
orf58a.pep   QGQSVSDGTAVRDAXRRVSVNLKEPNKATVSAEARISRLIPESRTVVGKRDVEMPSETEN
             |||||||||||||| ||||||||||||||||||||||||||||:||||||||||||||||
orf58-1      QGQSVSDGTAVRDARRRVSVNLKEPNKATVSAEARISRLIPESQTVVGKRDVEMPSETEN
                       310       320       330       340       350       360

                       370       380       390       400       410       420
orf58a.pep   VFTEXVSSVGYGXPVYDETADIHIEEPAAPDAWVVEPPEVPKVPMPAXDIPPPPPVSEIY
             ||||:||||||| |||||||||||||||||||||||||||||||| | || |||||||||
orf58-1      VFTETVSSVGYGGPVYDETADIHIEEPAAPDAWVVEPPEVPKVPMTAIDIQPPPPVSEIY
                       370       380       390       400       410       420

                       430       440       450       460       470       480
orf58a.pep   NRTYEPPAGFEQVQRSRIAETDHLADDVLNGGWQEETAAIANDGSEGVAERSSGQYLSET
             |||||||:|||||||||||||||||||||||||||||||||:|||||:||||||||||||
orf58-1      NRTYEPPSGFEQVQRSRIAETDHLADDVLNGGWQEETAAIADDGSEGAAERSSGQYLSET
                       430       440       450       460       470       480

                       490       500       510       520       530       540
orf58a.pep   EAFGHDSQAVCPFENVPSERPSRRAXDTEADEGAFQSEETGAVSEHLPTTDLLLPPLFNP
             |||||||||||||||||||||| |: ||||||||| ||||||||||||||||||||||||
orf58-1      EAFGHDSQAVCPFENVPSERPSCRVSDTEADEGAFPSEETGAVSEHLPTTDLLLPPLFNP
                       490       500       510       520       530       540

                       550       560       570       580       590       600
orf58a.pep   GATQTEEXLLXNSITIEEKXAEFKVKVKVVDSYSGPVITRYEIEPDVGVRGNSVLNLEKX
```

```
                 |||||| || |||||||| ||||||||||||||||||||||||||||||||||||||||
orf58-1          EATQTEEELLENSITIEEKLAEFKVKVKVVDSYSGPVITRYEIEPDVGVRGNSVLNLEKD
                        550       560       570       580       590       600

                        610       620       630       640       650       660
orf58a.pep       LARSLGVASIRVVETILGKTCMGLELPNPKRQMIRLSEIFNSPEFAESKSKLTLALGQDI
                 |||||||||||||||| |||||||||||||||||||||||||||||||||||||||||||
orf58-1          LARSLGVASIRVVETIPGKTCMGLELPNPKRQMIRLSEIFNSPEFAESKSKLTLALGQDI
                        610       620       630       640       650       660

                        670       680       690       700       710       720
orf58a.pep       TGQPVVTDLGKAPHLLVAGTTGSGKSVGVNAMILSMLFKAAPEDVRMIMIDPKMLELSIY
                 ||||||||||||||||||||||||||||||||||||||||||||||||||||||||||||
orf58-1          TGQPVVTDLGKAPHLLVAGTTGSGKSVGVNAMILSMLFKAAPEDVRMIMIDPKMLELSIY
                        670       680       690       700       710       720

                        730       740       750       760       770       780
orf58a.pep       EGIPHLLAPVVTDMKLAANALNWCVNEMEKRYRLMSFMGVRNLAGXNQKIAEAAARGEKI
                 |||||||||||||||||||||||||||||||||||||||||||||| |||||||||||||
orf58-1          EGIPHLLAPVVTDMKLAANALNWCVNEMEKRYRLMSFMGVRNLAGFNQKIAEAAARGEKI
                        730       740       750       760       770       780

                        790       800       810       820       830       840
orf58a.pep       GNPFSLTPDNPEPLXKLPFIVVVVDEFADLMMTAGKKIEELIARLAQKARAAGIHLILAT
                 |||||||||:|||| |||||||||||||||||||||||||||||||||||||||||||||
orf58-1          GNPFSLTPDDPEPLEKLPFIVVVVDEFADLMMTAGKKIEELIARLAQKARAAGIHLILAT
                        790       800       810       820       830       840

                        850       860       870       880       890       900
orf58a.pep       QRPSVDVITGLIKANIPTRIAFQVSSKIDSRTILDQMGAENLLGQGDMLFLPPGTAYPQR
                 |||||||||||||||||||||||||||||||||||||||||||||||||||| |||||||
orf58-1          QRPSVDVITGLIKANIPTRIAFQVSSKIDSRTILDQMGAENLLGQGDMLFLLPGTAYPQR
                        850       860       870       880       890       900

                        910       920       930       940       950       960
orf58a.pep       VHGAFASDEEVHRVVEYLKQFGEPDYVDDXLSGGMSDDLLGISRSGDGETDPMYDEAVSV
                 ||||||||||||||||||||||||||||| |||| |::| ||:|||| ||||||||||||
orf58-1          VHGAFASDEEVHRVVEYLKQFGEPDYVDDILSGGGSEELPGIGRSGDDETDPMYDEAVSV
                        910       920       930       940       950       960

                        970       980       990      1000      1010
orf58a.pep       VLKTRKASISGVQRALRIGYNRAARLIDQMEAEGIVSAPEHNGNRTILVPXDNAX
                 ||||||||||||||||||||||||||||||||||||||||||||||||||| ||||
orf58-1          VLKTRKASISGVQRALRIGYNRAARLIDQMEAEGIVSAPEHNGNRTILVPLDNAX
                        970       980       990      1000      1010
```

Homology with a predicted ORF from *N.gonorrhoeae*

ORF58 shows complete identity over a 9aa overlap with a predicted ORF (ORF58ng) from *N. gonorrhoeae:*

```
orf58.pep    ALMLFHAVKTAVYWLFVGVVRFCRNYLAHESEPDRPVPP                    103
                                            |||||||||
orf58ng                                     SEPDRPVPPASANRADVPTASDGYSDSGNG   30
```

The ORF58ng nucleotide sequence <SEQ ID 493> is predicted to encode a protein having partial amino acid sequence <SEQ ID 494>:

```
  1  ..SEPDRPVPPA SANRADVPTA SDGYSDSGNG TEEAETEAAE AAEEEAADTE
 51    DIATAVIDNR RIPFDRSIAE GLMQSESKTS PVRPVFKEIT LEEATRALSS
101    AALRETKKRY IDAFEKNGTA VPKVRVSDTP MEGLQIIGLD DPVLQRTYSR
151    MFDADKEAFS ESADYGFEPY FEKQHPSAFS AVKAENARNA PFRRHAGQEK
201    GQAEAKSPDV SQGQSVSDGT AVRDARRRVS VNLKEPNKAT VSAEARISRL
251    IPESRTVVGK RDVEMPSETE NVFTETVSSV GYGGPVYDEA ADIHIEEPAA
301    PDAWVVEPPE VPEVAVPEID ILPPPPVSEI YNRTYEPPAG FEQAQRSRIA
351    ETDHLAADVL NGGWQEETAA IADDGSEGAA ERSSGQYLSE TEAFGHDSQA
401    VCPFEDVPSE RPSCRVSDTE ADEGAFQSEE TGAVSEHLPT TDLLLPPLFN
451    PEATQTEEEL LENSITIEEK LAEFKVKVKV VDSYSGPVIT RYEIEPDVGV
501    RGNSVLNLEK DLARSLGVAS IRVVETIPGK TCMGLELPNP KRQMIRLSEI
```

```
551  FNSPEFAESK SKLTLALGQD ITGQPVVTDL GKAPHLLVAG TTGSGKSVGV
601  NAMILSMLFK AAPEDVRMIM IDPKMLELSI YEGITHLLAP VVTDMKLAAN
651  ALNWCVNEME KRYRLMSFMG VRNLAGFNQK IAEAAARGEK IGNPFSLTPD
701  DPEPLEKLPF IVVVVDEFAD LMMTAGKKIE ELIARLAQKA RAAGIHLILA
751  TQRPSVDVIT GLIKANIPTR IAFQVSSKID SRTILDQMGA ENLLGQGDML
801  FLPPGTAYPQ RVHGAFASDE EVHRVVEYLK QFGEPDYVDD ILSGGGSEEL
851  PGIGRSGDGE TDPMYDEAVS VVLKTRKASI SGVQRALRIG YNRAARLIDQ
901  MEAEGIVSAP EHNGNRTILV PLDNA*
```

This partial gonococcal sequence contains a predicted transmembrane region and a predicted ATP/GTP-binding site motif A (P-loop; double underlined). Furthermore, it has a domain homologous to the FTSK cell division protein of *E. coli.* Alignment of ORF58ng and FtsK (accession number p46889) show a 65 % amino acid identity in 459 overlap:

```
ORF58ng:  467  IEEKLAEFKVKVKVVDSYSGPVIVTRYEIEPDVGVRGNSVLNLEKDLARSLGVASIRVVET 526
               +E +LA+F++K  VV+   GPVITR+E+    GV+   + NL +DLARSL    ++RVVE
FtsK:     868  VEARLADFRIKADVVNYSPGPVITRFELNLAPGVKAARISNLSRDLARSLSTVAVRVVEV 927

ORF58ng:  527  IPGKTCMGLELPNPKRQMIRLSEIFNSPEFAESKSKLTLALGQDITGQPVVTDLGKAPHL 586
               IPGK  +GLELPN KRQ + L E+ ++ +F ++ S LT+ LG+DI G+PVV DL K PHL
FtsK:     928  IPGKPYVGLELPNKKRQTVYLREVLDNAKFRDNPSPLTVVLGKDIAGEPVVADLAKMPHL 987

ORF58ng:  587  LVAGTTGSGKSVGVNAMILSMLFKAAPEDVRMIMIDPKMLELSIYEGITHLLAPVVTDMK 646
               LVAGTTGSGKSVGVNAMILSML+KA PEDVR IMIDPKMLELS+YEGI HLL  VVTDMK
FtsK:     988  LVAGTTGSGKSVGVNAMILSMLYKAQPEDVRFIMIDPKMLELSVYEGIPHLLTEVVTDMK 1047

ORF58ng:  647  LAANALNWCVNEMEKRYRLMSFMGVRNLAGFNQKIAEAAARGEKIGNPFSLTPDDPEP-- 704
                AANAL WCVNEME+RY+LMS +GVRNLAG+N+KIAEA      I +P+    D  +
FtsK:     1048 DAANALRWCVNEMERRYKLMSALGVRNLAGYNEKIAEADRMMRPIPDPYWKPGDSMDAQH 1107

ORF58ng:  705  --LEKLPFIVVVVDEFADLMMTAGKKIEELIARLAQKARAAGIHLILATQRPSVDVITGL 762
                 L+K P+IVV+VDEFADLMMT GKK+EELIARLAQKARAAGIHL+LATQRPSVDVITGL
FtsK:     1108 PVLKKEPYIVVLVDEFADLMMTVGKKVEELIARLAQKARAAGIHLVLATQRPSVDVITGL 1167

ORF58ng:  763  IKANIPTRIAFQVSSKIDSRTILDQMGAENLLGQGDMLFLPPGTAYPQRVHGAFASDEEV 822
               IKANIPTRIAF VSSKIDSRTILDQ GAE+LLG GDML+  P +  P RVHGAF  D+EV
FtsK:     1168 IKANIPTRIAFTVSSKIDSRTILDQAGAESLLGMGDMLYSGPNSTLPVRVHGAFVRDQEV 1227

ORF58ng:  823  HRVVEYLKQFGEPDYVDDILSGGGSEELPGIGRSGDGETDPMYDEAVSVVLKTRKASISG 882
               H VV+  K  G P YVD I S   SE   G G  G  E DP++D+AV  V + RKASISG
FtsK:     1228 HAVVQDWKARGRPQYVDGITSDSESEGGAG-GFDGAEELDPLFDQAVQFVTEKRKASISG 1286

ORF58ng:  883  VQRALRIGYNRAARLIDQMEAEGIVSAPEHNGNRTILVP 921
               VQR  RIGYNRAAR+I+QMEA+GIVS   HNGNR +L P
FtsK:     1287 VQRQFRIGYNRAARIIEQMEAQGIVSEQGHNGNREVLAP 1325
```

Further work on ORF58ng revealed the complete gonococcal DNA sequence to be <SEQ ID 495>:

```
   1 ATGTTTTGGA TAGTTTTGAT CGTTATtgtg TTGCTTGCGC TTGCCGGCCT
  51 GTTTTTTGTC CGCGCACAAT CCGAACGCGA GTGGATGCGC GAGGTTTCTG
 101 CGTGGCAGGA AAAGAAAGGG GAAAAACAGG CGGAGCTGCC TGAAATCAAA
 151 GACGGTATGC CCGATTTTCC CGAGTTTTCC CTGATGCTTT TCCATGCCGT
 201 CAAAACGGCA GTGTATTGGC TGTTTGTCGG TGTCGTCCGT TTCTGCCGAA
 251 ACTATCTGGC GCACGAATCC GAACCGGACA GGCCCGTTCC GCCTGCTTCT
 301 GCAAACCGTG CGGATGTTCC GACCGCATCC GACGGGTATT CAGACAGTGG
 351 AAACGGGACG GAAGAAGCGG AAACGGAAGC AGCAGAAGCT GCGGAGGAAG
 401 AGGCTGCCgA TACgGAAGAC ATTGCAACTG CCGTAATCGA CAACCGCCGC
 451 ATCCcatTCG ACCGGAGTAT TGCTGAAGGG TTGATGCAGT CTGAAAGCAA
 501 AACTTCGCCC GTCCGTCCGG TTTTTAAGGA AATCACTTTG GAAGAAGCAA
 551 CGCGTGCTTT AAGCAGCGCG GCTTTAAGGG AAACGAAAAA ACGCTATATC
 601 GATGCATTTG AGAAAAAACGG AACAGCCGTC CCCAAAGTAC GCGTGTCCGA
 651 TACCCCGATG GAAGGGCTGC AGATTATCGG TTTGGACGAC CCTGTGCTTC
 701 AACGCACGTA TTCCCGTATG TTTGATGCGG ACAAAGAAGC GTTTTCCGAG
 751 TCTGCGGATT ACGGATTTGA GCCGTATTTT GAGAAGCAGC ATCCGTCTGC
 801 CTTTTCTGCA GTCAAAGCCG AAAATGCACG GAATGCGCCG TTCCGCCGTC
 851 ATGCAGGGCA GGAGAAAGGG CAGGCGGAGG CAAAATCCCC GGATGTTTCC
 901 CAAGGGCAGT CCGTTTCAGA CGGCACAGCC GTCCGCGATG CCCGCCGCCG
 951 CGTTTCCGTC AATTTGAAAG AACCGAACAA GGCAACGGTT TCTGCGGAGG
```

```
1001 CGCGGATTTC GCGCCTGATT CCGGAAAGTC GGACGGTTGT CGGGAAACGG
1051 GATGTCGAAA TGCCGTCTGA AACCGAAAAT GTTTTCACGG AAACCGTTTC
1101 GTCTGTGGGA TACGGCGGTC CGGTTTATGA TGAAGCTGCC GATATCCATA
1151 TTGAAGAGCC TGCCGCGCCC GATGCTTGGG TGGTCGAACC ACCCGAAGTG
1201 CCGGAGGTAG CCGTACCCGA AATCGATATT CTGCCGCCGC CTCCCGTATC
1251 GGAAATCTAC AACCGTACCT ATGAGCCGCC GGCAGGATTC GAGCAGGCGC
1301 AACGCAGCCG CATTGCCGAA ACCGACCATC TTGCCGCTGA TGTTTTGAAT
1351 GGAGGTTGGC AGGAGGAAAC CGCCGCTATT GCAGATGACG GCAGTGAGGG
1401 TGCGGCAGAG CGGTCAAGCG GGCAATATCT GTCGGAAACC GAAGCGTTCG
1451 GGCATGACAG TCAGGCGGTT TGTCCGTTTG AAGATGTGCC GTCTGAACGC
1501 CCGTCCTGCC GGGTATCGGA TACGGAAGCG GATGAAGGGG CGTTCCAATC
1551 GGAAGAGACC GGTGCGGTAT CCGAACACCT GCCGACAACC GACCTGCTTC
1601 TGCCTCCGCT GTTCAATCCC GAGGCGACGC AAACCGAAGA AGAACTGTTG
1651 GAAAACAGCA TCACCATCGA AGAAAAATTG GCGGAGTTCA AAGTCAAGGT
1701 CAAGGTTGTC GATTCTTATT CCGGCCCCGT GATTACGCGT TATGAAATCG
1751 AACCCGATGT CGGCGTGCGC GGCAATTCCG TTCTGAATTT GGAAAAAGAC
1801 TTGGCGCGTT CGCTCGGCGT GGCTTCCATC CGCGTTGTCG AAACCATCCC
1851 CGGCAAAACC TGCATGGGTT TGGAACTTCC GAACCCGAAA CGCCAAATGA
1901 TACGCCTGAG CGAAATTTTC AATTCGCCCG AGTTTGCCGA ATCCAAATCC
1951 AAGCTGACGC TCGCGCTCGG TCAGGACATT ACCGGACAGC CCGTCGTAAC
2001 CGACTTGGGC AAAGCACCGC ATTTGCTGGT TGCCGGCACG ACCGGTTCGG
2051 GCAAATCGGT GGGTGTCAAC GCGATGATTC TGTCTATGCT TTTCAAAGCC
2101 GCGCCGGAAG ACGTGCGTAT GATTATGATC GATCCGAAAA TGCTGGAATT
2151 GAGCATTTAC GAAGGCATCA CGCACCTGCT CGCCCCTGTC GTTACCGATA
2201 TGAAGCTGGC GGCAAACGCG CTGAACTGGT GTGTTAACGA AATGGAAAAA
2251 CGCTACCGCC TGATGAGCTT TATGGGCGTG CGCAATCTTG CGGGCTTCAA
2301 CCAAAAAATC GCCGAAGCCG CAGCAAGGGG AGAAAAAATC GGCAATCCGT
2351 TCAGCCTCAC GCCCGACGAT CCCGAACCTT TGGAAAAACT GCCGTTTATC
2401 GTGGTCGTGG TCGATGAGTT TGCCGATTTG ATGATGACGG CAGGCAAGAA
2451 AATCGAAGAA CTGATTGCGC GCCTCGCCCA AAAAGCCCGC GCGGCAGGCA
2501 TCCACCTTAT CCTTGCCACA CAACGCCCCA GCGTCGATGT CATCACGGGT
2551 CTGATTAAGG CGAACATCCC GACGCGTATC GCGTTCCAAG TGTCCAGCAA
2601 AATCGACAGC CGCACGATTC TCGACCAAAT GGGCGCGGAA AACCTGCTCG
2651 GTCAGGGCGA TATGCTGTTC CTGCCGCCGG GTACTGCCTA TCCGCAGCGC
2701 GTTCACGGCG CGTTTGCCTC GGATGAAGAG GTGCACCGCG TGGTCGAATA
2751 TCTGAAGCAG TTTGGCGAGC CGGACTATGT TGACGATATT TTGAGCGGCG
2801 GCGGCAGCGA AGAGCTGCCC GGCATCGGGC GCAGCGGCGA CGGCGAAACC
2851 GATCCGATGT ACGACGAGGC CGTATCCGTT GTCCTGAAAA CGCGCAAAGC
2901 CAGCATTTCG GGCGTACAGC GCGCCTTGCG CATCGGCTAC AACCGCGCCG
2951 CGCGTCTGAT TGACCAAATG GAAGCGGAAG GCATTGTGTC CGCACCGGAA
3001 CACAACGGCA ACCGTACGAT TCTCGTCCCC TTGGACAATG CTTGA
```

This corresponds to the amino acid sequence <SEQ ID 496; ORF58ng-1>:

```
   1  MFWIVLIVIV LLALAGLFFV RAQSEREWMR EVSAWQEKKG EKQAELPEIK
  51  DGMPDFPEFS LMLFHAVKTA VYWLFVGVVR FCRNYLAHES EPDRPVPPAS
 101  ANRADVPTAS DGYSDSGNGT EEAETEAAEA AEEEAADTED IATAVIDNRR
 151  IPFDRSIAEG LMQSESKTSP VRPVFKEITL EEATRALSSA ALRETKKRYI
 201  DAFEKNGTAV PKVRVSDTPM EGLQIIGLDD PVLQRTYSRM FDADKEAFSE
 251  SADYGFEPYF EKQHPSAFSA VKAENARNAP FRRHAGQEKG QAEAKSPDVS
 301  QGQSVSDGTA VRDARRRVSV NLKEPNKATV SAEARISRLI PESRTVVGKR
 351  DVEMPSETEN VFTETVSSVG YGGPVYDEAA DIHIEEPAAP DAWVVEPPEV
 401  PEVAVPEIDI LPPPPVSEIY NRTYEPPAGF EQAQRSRIAE TDHLAADVLN
 451  GGWQEETAAI ADDGSEGAAE RSSGQYLSET EAFGHDSQAV CPFEDVPSER
 501  PSCRVSDTEA DEGAFQSEET GAVSEHLPTT DLLLPPLFNP EATQTEEELL
 551  ENSITIEEKL AEFKVKVKVV DSYSGPVITR YEIEPDVGVR GNSVLNLEKD
 601  LARSLGVASI RVVETIPGKT CMGLELPNPK RQMIRLSEIF NSPEFAESKS
 651  KLTLALGQDI TGQPVVTDLG KAPHLLVAGT TGSGKSVGVN AMILSMLFKA
 701  APEDVRMIMI DPKMLELSIY EGITHLLAPV VTDMKLAANA LNWCVNEMEK
 751  RYRLMSFMGV RNLAGFNQKI AEAAARGEKI GNPFSLTPDD PEPLEKLPFI
 801  VVVVDEFADL MMTAGKKIEE LIARLAQKAR AAGIHLILAT QRPSVDVITG
 851  LIKANIPTRI AFQVSSKIDS RTILDQMGAE NLLGQGDMLF LPPGTAYPQR
 901  VHGAFASDEE VHRVVEYLKQ FGEPDYVDDI LSGGGSEELP GIGRSGDGET
 951  DPMYDEAVSV VLKTRKASIS GVQRALRIGY NRAARLIDQM EAEGIVSAPE
1001  HNGNRTILVP LDNA*
```

ORF58ng-1 and ORF58-1 show 97.2% identity in 1014 aa overlap:

```
                        10        20        30        40        50        60
orf58-1.pep  MFWIVLIVILLLALAGLFFVRAQSEREWMREVSAWQEKKGEKQAELPEIKDGMPDFPELA
             |||||||||:||||||||||||||||||||||||||||||||||||||||||||||||::
```

```
orf58ng-1    MFWIVLIVIVLLALAGLFFVRAQSEREWMREVSAWQEKKGEKQAELPEIKDGMPDFPEFS
                     10        20        30        40        50        60

                     70        80        90       100       110       120
orf58-1.pep  LMLFHAVKTAVYWLFVGVVRFCRNYLAHESEPDRPVPPASANRADVPTASDGYSDSGNGT
             ||||||||||||||||||||||||||||||||||||||||||||||||||||||||||||
orf58ng-1    LMLFHAVKTAVYWLFVGVVRFCRNYLAHESEPDRPVPPASANRADVPTASDGYSDSGNGT
                     70        80        90       100       110       120

                    130       140       150       160       170       180
orf58-1.pep  EEAETEEAEAAEEEEAADTEDIATAVIDNRRIPFDRSIAEGLMPSESEISPVRPVFKEITL
             |||||| |||||||||||||||||||||||||||||||||||| |||: |||||||||||
orf58ng-1    EEAETEAAEAAEEEEAADTEDIATAVIDNRRIPFDRSIAEGLMQSESKTSPVRPVFKEITL
                    130       140       150       160       170       180

                    190       200       210       220       230       240
orf58-1.pep  EEATRALNSAALRETKKRYIDAFEKNETAVPKVRVSDTPMEGLQIIGLDDPVLQRTYSHM
             |||||||:|||||||||||||||||| |||||||||||||||||||||||||||||||:|
orf58ng-1    EEATRALSSAALRETKKRYIDAFEKNGTAVPKVRVSDTPMEGLQIIGLDDPVLQRTYSRM
                    190       200       210       220       230       240

                    250       260       270       280       290       300
orf58-1.pep  FDADKEAFSESADYGFEPYFEKQHPSAFSAVKAENARNAPFHRHAGQGKGQAEAKSPDVS
             |||||||||||||||||||||||||||||||||||||||||:||||| ||||||||||||
orf58ng-1    FDADKEAFSESADYGFEPYFEKQHPSAFSAVKAENARNAPFRRHAGQEKGQAEAKSPDVS
                    250       260       270       280       290       300

                    310       320       330       340       350       360
orf58-1.pep  QGQSVSDGTAVRDARRRVSVNLKEPNKATVSAEARISRLIPESQTVVGKRDVEMPSETEN
             |||||||||||||||||||||||||||||||||||||||||||:||||||||||||||||
orf58ng-1    QGQSVSDGTAVRDARRRVSVNLKEPNKATVSAEARISRLIPESRTVVGKRDVEMPSETEN
                    310       320       330       340       350       360

                    370       380       390       400       410       420
orf58-1.pep  VFTETVSSVGYGGPVYDETADIHIEEPAAPDAWVVEPPEVPKVPMTAIDIQPPPPVSEIY
             ||||||||||||||||||:||||||||||||||||||||||:| :  ||| |||||||||
orf58ng-1    VFTETVSSVGYGGPVYDEAADIHIEEPAAPDAWVVEPPEVPEVAVPEIDILPPPPVSEIY
                    370       380       390       400       410       420

                    430       440       450       460       470       480
orf58-1.pep  NRTYEPPSGFEQVQRSRIAETDHLADDVLNGGWQEETAAIADDGSEGAAERSSGQYLSET
             |||||||:||||:|||||||||||| ||||||||||||||||||||||||||||||||||
orf58ng-1    NRTYEPPAGFEQAQRSRIAETDHLAADVLNGGWQEETAAIADDGSEGAAERSSGQYLSET
                    430       440       450       460       470       480

                    490       500       510       520       530       540
orf58-1.pep  EAFGHDSQAVCPFENVPSERPSCRVSDTEADEGAFPSEETGAVSEHLPTTDLLLPPLFNP
             ||||||||||||||:|||||||||||||||||||| ||||||||||||||||||||||||
orf58ng-1    EAFGHDSQAVCPFEDVPSERPSCRVSDTEADEGAFQSEETGAVSEHLPTTDLLLPPLFNP
                    490       500       510       520       530       540

                    550       560       570       580       590       600
orf58-1.pep  EATQTEEELLENSITIEEKLAEFKVKVKVVDSYSGPVITRYEIEPDVGVRGNSVLNLEKD
             ||||||||||||||||||||||||||||||||||||||||||||||||||||||||||||
orf58ng-1    EATQTEEELLENSITIEEKLAEFKVKVKVVDSYSGPVITRYEIEPDVGVRGNSVLNLEKD
                    550       560       570       580       590       600

                    610       620       630       640       650       660
orf58-1.pep  LARSLGVASIRVVETIPGKTCMGLELPNPKRQMIRLSEIFNSPEFAESKSKLTLALGQDI
             ||||||||||||||||||||||||||||||||||||||||||||||||||||||||||||
orf58ng-1    LARSLGVASIRVVETIPGKTCMGLELPNPKRQMIRLSEIFNSPEFAESKSKLTLALGQDI
                    610       620       630       640       650       660

                    670       680       690       700       710       720
orf58-1.pep  TGQPVVTDLGKAPHLLVAGTTGSGKSVGVNAMILSMLFKAAPEDVRMIMIDPKMLELSIY
             ||||||||||||||||||||||||||||||||||||||||||||||||||||||||||||
orf58ng-1    TGQPVVTDLGKAPHLLVAGTTGSGKSVGVNAMILSMLFKAAPEDVRMIMIDPKMLELSIY
                    670       680       690       700       710       720

                    730       740       750       760       770       780
orf58-1.pep  EGIPHLLAPVVTDMKLAANALNWCVNEMEKRYRLMSFMGVRNLAGFNQKIAEAAARGEKI
             ||| ||||||||||||||||||||||||||||||||||||||||||||||||||||||||
```

```
orf58ng-1    EGITHLLAPVVTDMKLAANALNWCVNEMEKRYRLMSFMGVRNLAGFNQKIAEAAARGEKI
                     730       740       750       760       770       780

                     790       800       810       820       830       840
orf58-1.pep  GNPFSLTPDDPEPLEKLPFIVVVVDEFADLMMTAGKKIEELIARLAQKARAAGIHLILAT
             ||||||||||||||||||||||||||||||||||||||||||||||||||||||||||||
orf58ng-1    GNPFSLTPDDPEPLEKLPFIVVVVDEFADLMMTAGKKIEELIARLAQKARAAGIHLILAT
                     790       800       810       820       830       840

                     850       860       870       880       890       900
orf58-1.pep  QRPSVDVITGLIKANIPTRIAFQVSSKIDSRTILDQMGAENLLGQGDMLFLLPGTAYPQR
             |||||||||||||||||||||||||||||||||||||||||||||||||||| |||||||
orf58ng-1    QRPSVDVITGLIKANIPTRIAFQVSSKIDSRTILDQMGAENLLGQGDMLFLPPGTAYPQR
                     850       860       870       880       890       900

                     910       920       930       940       950       960
orf58-1.pep  VHGAFASDEEVHRVVEYLKQFGEPDYVDDILSGGGSEELPGIGRSGDDETDPMYDEAVSV
             ||||||||||||||||||||||||||||||||||||||||||||||| ||||||||||||
orf58ng-1    VHGAFASDEEVHRVVEYLKQFGEPDYVDDILSGGGSEELPGIGRSGDGETDPMYDEAVSV
                     910       920       930       940       950       960

                     970       980       990      1000      1010
orf58-1.pep  VLKTRKASISGVQRALRIGYNRAARLIDQMEAEGIVSAPEHNGNRTILVPLDNAX
             |||||||||||||||||||||||||||||||||||||||||||||||||||||||
orf58ng-1    VLKTRKASISGVQRALRIGYNRAARLIDQMEAEGIVSAPEHNGNRTILVPLDNAX
                     970       980       990      1000      1010
```

Furthermore, ORF58ng-1 shows significant homology to the *E.coli* protein FtsK:

```
sp|P46889|FTSK_ECOLI CELL DIVISION PROTEIN FTSK >gi|1651412|gnl|PID|d1015290 (D1
division protein FtsK [Escherichia coli] >gi|1651418|gnl|PID|d1015296 (D90727) Cell
division protein FtsK [Escherichia coli] >gi|1787117 (AE000191) cell division
protein FtsK [Escherichia coli] Length = 1329
 Score =  576 bits (1469), Expect = e-163
 Identities = 301/459 (65%), Positives = 353/459 (76%), Gaps = 5/459 (1%)

Query: 556  IEEKLAEFKVKVKVVDSYSGPVITRYEIEPDVGVRGNSVLNLEKDLARSLGVASIRVVET 615
             +E +LA+F++K  VV+   GPVITR+E+    GV+   + NL +DLARSL   ++RVVE
Sbjct: 868  VEARLADFRIKADVVNYSPGPVITRFELNLAPGVKAARISNLSRDLARSLSTVAVRVVEV 927

Query: 616  IPGKTCMGLELPNPKRQMIRLSEIFNSPEFAESKSKLTLALGQDITGQPVVTDLGKAPHL 675
             IPGK  +GLELPN KRQ + L E+ ++ +F ++ S LT+ LG+DI G+PVV DL K PHL
Sbjct: 928  IPGKPYVGLELPNKKRQTVYLREVLDNAKFRDNPSPLTVVLGKDIAGEPVVADLAKMPHL 987

Query: 676  LVAGTTGSGKSVGVNAMILSMLFKAAPEDVRMIMIDPKMLELSIYEGITHLLAPVVTDMK 735
             LVAGTTGSGKSVGVNAMILSML+KA PEDVR IMIDPKMLELS+YEGI HLL  VVTDMK
Sbjct: 988  LVAGTTGSGKSVGVNAMILSMLYKAQPEDVRFIMIDPKMLELSVYEGIPHLLTEVVTDMK 1047

Query: 736  LAANALNWCVNEMEKRYRLMSFMGVRNLAGFNQKIAEAAARGEKIGNPFSLTPDDPEP-- 793
              AANAL WCVNEME+RY+LMS +GVRNLAG+N+KIAEA      I +P+    D +
Sbjct: 1048 DAANALRWCVNEMERRYKLMSALGVRNLAGYNEKIAEADRMMRPIPDPYWKPGDSMDAQH 1107

Query: 794  --LEKLPFIVVVVDEFADLMMTAGKKIEELIARLAQKARAAGIHLILATQRPSVDVITGL 851
               L+K P+IVV+VDEFADLMMT GKK+EELIARLAQKARAAGIHL+LATQRPSVDVITGL
Sbjct: 1108 PVLKKEPYIVVLVDEFADLMMTVGKKVEELIARLAQKARAAGIHLVLATQRPSVDVITGL 1167

Query: 852  IKANIPTRIAFQVSSKIDSRTILDQMGAENLLGQGDMLFLPPGTAYPQRVHGAFASDEEV 911
             IKANIPTRIAF VSSKIDSRTILDQ GAE+LLG GDML+  P +  P RVHGAF  D+EV
Sbjct: 1168 IKANIPTRIAFTVSSKIDSRTILDQAGAESLLGMGDMLYSGPNSTLPVRVHGAFVRDQEV 1227

Query: 912  HRVVEYLKQFGEPDYVDDILSGGGSEELPGIGRSGDGETDPMYDEAVSVVLKTRKASISG 971
             H VV+  K  G P YVD I S  SE   G G  G  E DP++D+AV  V + RKASISG
Sbjct: 1228 HAVVQDWKARGRPQYVDGITSDSESEGGAG-GFDGAEELDPLFDQAVQFVTEKRKASISG 1286

Query: 972  VQRALRIGYNRAARLIDQMEAEGIVSAPEHNGNRTILVP 1010
             VQR  RIGYNRAAR+I+QMEA+GIVS   HNGNR +L P
Sbjct: 1287 VQRQFRIGYNRAARIIEQMEAQGIVSEQGHNGNREVLAP 1325
```

Based on this analysis, it is predicted that the proteins from *N.meningitidis* and *N.gonorrhoeae,* and their epitopes, could be useful antigens for vaccines or diagnostics, or for raising antibodies.

## Example 59

The following partial DNA sequence was identified in *N. meningitidis* <SEQ ID 497>:

```
   1  ATGATTTATC AAAGAAACCT CATCAAAGAA CTCTCTTTTA CCGCCGTCGG
  51  CATTTTCGTC GTCCTCTTGG CGGTATTGGT CTCCACGCAG GCAATCAACC
 101  TGCTCGGCCG TGCCGCCGAC GGGC..GTGA TCGCCATCGA TGCCGTGTTG
 151  GCATTGGTCG GCTTCTGGGT C......... .......... ..........
                                //
 901  .........A TTGCCATCGG TTTGTTTTTA ATTTACCAAA ACGGGCTGAC
 951  CCTGCTTTTT GAAGCCGTGG AAGACGGCAA AATCCATTTT TGGCTCGGAC
1001  TGCTGCCTAT GCACATTATC ATGTTTGTCC TTGCACTCAT CCTGTTGCGC
1051  GTCCGCAGTA TGCCCAGCCA GCCCTTCTGG CAGGCGGTTG GCAAAAGTCT
1101  GACATTGAAA GGCGGAAAAT GA
```

This corresponds to the amino acid sequence <SEQ ID 498; ORF101>:

```
  1  MIYQRNLIKE LSFTAVGIFV VLLAVLVSTQ AINLLGRAAD GXVIAIDAVL
 51  ALVGFWV... .......... .......... .......... ..........
                          //
301  ...IAIGLFL IYQNGLTLLF EAVEDGKIHF WLGLLPMHII MFVLALILLR
351  VRSMPSQPFW QAVGKSLTLK GGK*
```

Further work revealed the complete nucleotide sequence <SEQ ID 499>:

```
   1  ATGATTTATC AAAGAAACCT CATCAAAGAA CTCTCTTTTA CCGCCGTCGG
  51  CATTTTCGTC GTCCTCTTGG CGGTATTGGT CTCCACGCAG GCAATCAACC
 101  TGCTCGGCCG TGCCGCCGAC GGGCGTGTCG CCATCGATGC CGTGTTGGCA
 151  TTGGTCGGCT TCTGGGTCAT CGGTATGACG CCGCTTTTGC TGGTGTTGAC
 201  CGCATTTATC AGTACGTTGA CCGTGTTGAC CCGCTACTGG CGCGACAGCG
 251  AAATGTCGGT CTGGCTATCC TGCGGATTGG CATTGAAACA ATGGATACGC
 301  CCGGTGATGC AGTTTGCCGT GCCGTTTGCC GTTTTGGTTG CCGTCATGCA
 351  GCTTTGGGTG ATACCGTGGG CAGAGCTACG CAGCCGCGAA TACGCTGAAA
 401  TCCTGAAGCA GAAGCAGGAA TTGTCTTTGG TGGAGGCAGG CGAGTTCAAC
 451  AGTTTGGGCA AGCGCAACGG CAGGGTTTAT TTTGTCGAAA CCTTCGATAC
 501  CGAATCCGGC ATCATGAAAA ACCTGTTCCT GCGCGAACAG GACAAAAACG
 551  GCGGCGACAA CATCATCTTC GCCAAAGAAG GTAACTTCTC GCTGAACGAC
 601  AACAAACGCA CGCTCGAATT GCGCCACGGC TACCGTTACA GCGGCACGCC
 651  CGGACGCGCC GACTACAATC AGGTTTCCTT CCAAAAACTC AACCTGATTA
 701  TCAGCACCAC GCCCAAACTC ATCGACCCCG TTTCCCACCG CCGTACCATT
 751  CCGACCGCCC AACTGATTGG CAGCAGCAAC CCGCAACATC AGGCGGAATT
 801  GATGTGGCGC ATCTCGCTGA CCGTCAGCGT CCTCCTACTC TGCCTGCTTG
 851  CCGTGCCGCT TTCCTATTTC AACCCGCGCA GCGGACATAC CTACAATATC
 901  TTGATTGCCA TCGGTTTGTT TTTAATTTAC CAAAACGGGC TGACCCTGCT
 951  TTTTGAAGCC GTGGAAGACG GCAAAATCCA TTTTTGGCTC GGACTGCTGC
1001  CTATGCACAT TATCATGTTT GCCGTTGCAC TCATCCTGTT GCGCGTCCGC
1051  AGTATGCCCA GCCAGCCCTT CTGGCAGGCG GTTGGCAAAA GTCTGACATT
1101  GAAAGGCGGA AAATGA
```

This corresponds to the amino acid sequence <SEQ ID 500; ORF101-1>:

```
  1  MIYQRNLIKE LSFTAVGIFV VLLAVLVSTQ AINLLGRAAD GRVAIDAVLA
 51  LVGFWVIGMT PLLLVLTAFI STLTVLTRYW RDSEMSVWLS CGLALKQWIR
101  PVMQFAVPFA VLVAVMQLWV IPWAELRSRE YAEILKQKQE LSLVEAGEFN
151  SLGKRNGRVY FVETFDTESG IMKNLFLREQ DKNGGDNIIF AKEGNFSLND
201  NKRTLELRHG YRYSGTPGRA DYNQVSFQKL NLIISTTPKL IDPVSHRRTI
251  PTAQLIGSSN PQHQAELMWR ISLTVSVLLL CLLAVPLSYF NPRSGHTYNI
301  LIAIGLFLIY QNGLTLLFEA VEDGKIHFWL GLLPMHIIMF AVALILLRVR
351  SMPSQPFWQA VGKSLTLKGG K*
```

Computer analysis of this amino acid sequence gave the following results:

Homology with a predicted ORF from *N.meningitidis* (strain A)

ORF101 shows 91.2% identity over a 57aa overlap and 95.7% identity over a 69aa overlap with an ORF (ORF101a) from strain A of *N. meningitidis:*

```
                       10        20        30        40        50
orf101.pep   MIYQRNLIKELSFTAVGIFVVLLAVLVSTQAINLLGRAADGXVIAIDAVLALVGFWVX
             |||||||||||||||||||||||||||||||||||| |||   ||||||||||||||
orf101a      MIYQRNLIKELSFTAVGIFVVLLAVLVSTQAINLLGXAADXRX-AIDAVLALVGFWVXXM
                       10        20        30        40        50
                                         //
                                           90       100       110
orf101.pep   ............................IAIGLFLIYQNGLTLLFEAVEDGKIHFWLGL
                                         ||||||||||||||||||||||||||||||
orf101a      LTVSVLLLCLLAVPLSYFNPRSGHTYNILXAIGLFLIYQNGLTLLFEAVEDGKIHFWLGL
                  280       290       300       310       320       330

               120       130       140       150
orf101.pep   LPMHIIMFVLALILLRVRSMPSQPFWQAVGKSLTLKGGKX
             ||||||||:|::||||||||||||||||||||||||||||
orf101a      LPMHIIMFVIAIVLLRVRSMPSQPFWQAVGKSLTLKGGKX
                  340       350       360       370
```

The complete length ORF101a nucleotide sequence <SEQ ID 501> is:

```
   1 ATGATTTATC AAAGAAACCT CATCAAAGAA CTCTCTTTTA CCGCCGTCGG
  51 CATTTTCGTC GTCCTCTTGG CGGTATTGGT CTCCACGCAG GCAATCAACC
 101 TGCTCGGCCN TGCCGCCGAC NGGCGTNTCG CCATCGATGC CGTGTTGGCA
 151 TTGGTCGGCT TCTGGGTCNN NNGNATGACG CCGCTTTTGC TNGTGTTGAC
 201 CGCATTTATC AGTACGTTGA CCGTGTTGAC CCGCTACTGG CGNGACAGCG
 251 AAATGTCGGT CTGGNTATCC TGCGGATTGG CATTGAAACA ATGGATACGC
 301 CCGGTGATGC AGTTTGCCGT GCCGTTTGCC GTTTTGGTTG CCGTCATGCA
 351 GCTTTGGGTG ATACCGTGGG CAGAGCTACG CAGCCGCGAA TACGCTGAAA
 401 TCCTGAAGCA GAAGCAGGAA TTGTCTTTGG TGGAGGCAGG CGGGTTCAAC
 451 AGTTTGGGCA AGCGCAACGG CAGGGTTTAT TTTGTCGAAA CCTTCGATAC
 501 CGAATCCGGC ATCATGAAAA ACCTGTTCCT GCGCGAACAG GACAAAAACG
 551 GCGGCGACAA CATCATCTTC NCCAAAGAAA GTAACTTCTC GCTGAACGAC
 601 AACAAACGCA CGCTCGAATT GCGCCACGGC TACCGTTACA GCGGCACGCC
 651 CGGACGCGCC GACTACAATC AGGTTTCCTT CCNAAAACTC AACCTGATTA
 701 TCAGCACCAC GCCCAAACTC ATCGACCCCG TTTCCCACCG CCGTACNATN
 751 CCNACNGCCC AACTGATTGG CAGCAGCAAC CCGCAACATC ANGCGGAATT
 801 GATGTGGCGC ATCTCGCTGA CCGTCAGCGT CCTCCTACTC TGCCTGCTTG
 851 CCGTGCCGCT TTCCTATTTC AACCCGCGCA GCGGACATAC CTACAATATC
 901 TTGANTGCCA TCGGTTTGTT TTTAATTTAC CAAAACGGGC TGACCCTGCT
 951 TTTTGAAGCC GTGGAAGACG GCAAAATCCA TTTTTGGCTC GGACTGCTGC
1001 CTATGCACAT CATCATGTTC GTCATCGCAA TCGTACTTCT GCGCGTCCGC
1051 AGCATGCCCA GCCAGCCCTT CTGGCAGGCG GTTGGCAAAA GTCTGACATT
1101 GAAAGGCGGA AAATGA
```

This encodes a protein having amino acid sequence <SEQ ID 502>:

```
  1 MIYQRNLIKE LSFTAVGIFV VLLAVLVSTQ AINLLGXAAD XRXAIDAVLA
 51 LVGFWVXXMT PLLLVLTAFI STLTVLTRYW RDSEMSVWXS CGLALKQWIR
101 PVMQFAVPFA VLVAVMQLWV IPWAELRSRE YAEILKQKQE LSLVEAGGFN
151 SLGKRNGRVY FVETFDTESG IMKNLFLREQ DKNGGDNIIF XKESNFSLND
201 NKRTLELRHG YRYSGTPGRA DYNQVSFXKL NLIISTTPKL IDPVSHRRTX
251 PTAQLIGSSN PQHXAELMWR ISLTVSVLLL CLLAVPLSYF NPRSGHTYNI
301 LXAIGLFLIY QNGLTLLFEA VEDGKIHFWL GLLPMHIIMF VIAIVLLRVR
351 SMPSQPFWQA VGKSLTLKGG K*
```

ORF101a and ORF101-1 show 95.4% identity in 371 aa overlap:

```
orf101a.pep   MIYQRNLIKELSFTAVGIFVVLLAVLVSTQAINLLGXAADXRXAIDAVLALVGFWVXXMT  60
              |||||||||||||||||||||||||||||||||||| ||| | |||||||||||||  ||
orf101-1      MIYQRNLIKELSFTAVGIFVVLLAVLVSTQAINLLGRAADGRVAIDAVLALVGFWVIGMT  60

orf101a.pep   PLLLVLTAFISTLTVLTRYWRDSEMSVWXSCGLALKQWIRPVMQFAVPFAVLVAVMQLWV 120
              |||||||||||||||||||||||||||| |||||||||||||||||||||||||||||||
orf101-1      PLLLVLTAFISTLTVLTRYWRDSEMSVWLSCGLALKQWIRPVMQFAVPFAVLVAVMQLWV 120
```

```
orf101a.pep   IPWAELRSREYAEILKQKQELSLVEAGGFNSLGKRNGRVYFVETFDTESGIMKNLFLREQ 180
              ||||||||||||||||||||||||||| ||||||||||||||||||||||||||||||||
orf101-1      IPWAELRSREYAEILKQKQELSLVEAGEFNSLGKRNGRVYFVETFDTESGIMKNLFLREQ 180

orf101a.pep   DKNGGDNIIFXKESNFSLNDNKRTLELRHGYRYSGTPGRADYNQVSFXKLNLIISTTPKL 240
              |||||||||| ||:||||||||||||||||||||||||||||||||| ||||||||||||
orf101-1      DKNGGDNIIFAKEGNFSLNDNKRTLELRHGYRYSGTPGRADYNQVSFQKLNLIISTTPKL 240

orf101a.pep   IDPVSHRRTXPTAQLIGSSNPQHXAELMWRISLTVSVLLLCLLAVPLSYFNPRSGHTYNI 300
              ||||||||| ||||||||||||| ||||||||||||||||||||||||||||||||||||
orf101-1      IDPVSHRRTIPTAQLIGSSNPQHQAELMWRISLTVSVLLLCLLAVPLSYFNPRSGHTYNI 300

orf101a.pep   LXAIGLFLIYQNGLTLLFEAVEDGKIHFWLGLLPMHIIMFVIAIVLLRVRSMPSQPFWQA 360
              | ||||||||||||||||||||||||||||||||||||||::|::|||||||||||||||
orf101-1      LIAIGLFLIYQNGLTLLFEAVEDGKIHFWLGLLPMHIIMFAVALILLRVRSMPSQPFWQA 360

orf101a.pep   VGKSLTLKGGK  371
              |||||||||||
orf101-1      VGKSLTLKGGK  371
```

<u>Homology with a predicted ORF from *N.gonorrhoeae*</u>

ORF101 shows 96.5 % identity in 57aa overlap at the N-terminal domain and 95.1% identity in 61aa overlap at the C-terminal domain, respectively, with a predicted ORF (ORF101ng) from *N. gonorrhoeae:*

```
orf101.pep   MIYQRNLIKELSFTAVGIFVVLLAVLVSTQAINLLGRAADGXVIAIDAVLALVGFWV       57
             |||||||||||||||||||||||||||||||||||||||| | |||||||||||||
orf101ng     MIYQRNLIKELSFTAVGIFVVLLAVLVSTQAINLLGRAADGRV-AIDAVLALVGFWVIGM    59

                                          //

orf101.pep                                 IAIGLFLIYQNGLTLLFEAVEDGKIHFWLG   333
                                           ||||||||||||||||||||||||||||||
orf101ng     SLTVSVLLLCLLAVPLSYFNPRSGHTYNILIAIGLFLIYQNGLTLLFEAVEDGKIHFWLG   331

orf101.pep   LLPMHIIMFVLALILLRVRSMPSQPFWQAVGKSLTLKGGK  373
             ||||||||||:|::||||||||||||||||
orf101ng     LLPMHIIMFVIAIVLLRVRSMPSQPFWQAVG           362
```

The ORF101ng nucleotide sequence <SEQ ID 503> is predicted to encode a protein having partial amino acid sequence <SEQ ID 504>:

```
  1  MIYQRNLIKE LSFTAVGIFV VLLAVLVSTQ AINLLGRAAD GRVAIDAVLA
 51  LVGFWVIGMT PLLLVLTAFI STLTVLTRYW RDSEMSVWLS CGLALKQWIR
101  PVMQFAVPFA ILIAVMQLWV IPWAELRSRE YAEILKQKQE LSLVEAGEFN
151  NLGKRNGRVY FVETFDTESG IMKNLFLREQ DKNGGDNIIF AKEGNFSLKD
201  NKRTLELRHG YRYSGTPGRA DYNQVSFQKL NLIISTTPKL IDPVSHRRTI
251  STAQLIGSSN PQHQAELMWR ISLTVSVLLL CLLAVPLSYF NPRSGHTYNI
301  LIAIGLFLIY QNGLTLLFEA VEDGKIHFWL GLLPMHIIMF VIAIVLLRVR
351  SMPSQPFWQA VG...
```

Further work revealed the complete nucleotide sequence <SEQ ID 505>:

```
   1  ATGATTTATC AAAGAAACCT CATCAAAGAA CTCTCTTTTA CCGCCGTCGG
  51  CATTTTCGTC GTCCTCTTGG CGGTGTTGGT GTCCACGCAG GCGATCAACC
 101  TGCTTGGCCG CGCAGCTGAC GGGCGTGTCG CCATCGATGC CGTGTTGGCC
 151  TTAGTCGGCT TCTGGGTCAT CGGTATGACC CCGCTTTTGC TGGTGTTGAC
 201  CGCATTCATC AGCACGCTGA CCGTATTGAC CCGCTACTGG CGCGACAGCG
 251  AAATGTCGGT CTGGCTATCC TGCGGATTGG CGTTGAAACA GTGGATACGC
 301  CCCGTCATGC AGTTTGCCGT GCCGTTTGCC ATCCTGATTG CCGTCATGCA
 351  GCTTTGGGTG ATACCGTGGG CAGAGCTGCG CAGCCGCGAA TATGCCGAAA
 401  TTTTGAAGCA GAAGCAGGAA TTGTCTTTGG TGGAAGCCGG CGAGTTCAAT
 451  AACTTGGGCA AGCGCAACGG CAgggtttaT TtcgtcgaaA CCTTTGACAC
 501  CGaatccgGC ATCATGAAAA ACCTGTtcct GcGCGAACAG GACAAAAACG
 551  gcggcgacaA CATCATCTTC GCcaaaGAag gtaactTctc gctgaaggaC
 601  AACAAAcgca cgctcgaATT GCGCCACGGC TACCGTTACA GCGGcacgcC

 651  CGGacGCGCc gactaCAATC AGGTTtcctt cCAAAAacTc aacctgATta
 701  TCAGCACCAC GCCCAAacTT ATCGacccCG TTTCCCACCG CCGCACCATT
 751  tcgacCGCCC AAcTGATTGG CAGCAGCAAT CCGCAACATC AGGCAGAATT
 801  GATGTGGCGC ATCTCGCTGA CCGTCAGCGT CCTCCTGCTC TGCCTACTCG
 851  CCGTGCCGCT TTCCTATTTC AACCCGCGCA GCGGACATAC CTACAATATC
 901  TTGATTGCCA TCGGTTTGTT TTTAATTTAC CAAAACGGGC TGACCCTGCT
 951  TTTTGAAGCC GTGGAAGACG GCAAAATCCA TTTTTGGCTC GGACTGCTGC
1001  CTATGCACAT CATCATGTTC GTCATCGCAA TCGTACTTCT GCGCGTCCGC
1051  AGTATGCCCA GCCAGCCCTT CTGGCAGGCG GTTGGCAAAA GTCTGACATT
1101  GAAAGgcgGA AAATGA
```

This corresponds to the amino acid sequence <SEQ ID 506; ORF101ng-1>:

```
  1  MIYQRNLIKE LSFTAVGIFV VLLAVLVSTQ AINLLGRAAD GRVAIDAVLA
 51  LVGFWVIGMT PLLLVLTAFI STLTVLTRYW RDSEMSVWLS CGLALKQWIR
101  PVMQFAVPFA ILIAVMQLWV IPWAELRSRE YAEILKQKQE LSLVEAGEFN
151  NLGKRNGRVY FVETFDTESG IMKNLFLREQ DKNGGDNIIF AKEGNFSLKD
201  NKRTLELRHG YRYSGTPGRA DYNQVSFQKL NLIISTTPKL IDPVSHRRTI
251  STAQLIGSSN PQHQAELMWR ISLTVSVLLL CLLAVPLSYF NPRSGHTYNI
301  LIAIGLFLIY QNGLTLLFEA VEDGKIHFWL GLLPMHIIMF VIAIVLLRVR
351  SMPSQPFWQA VGKSLTLKGG K*
```

ORF101ng-1 and ORF101-1 show 97.6% identity in 371 aa overlap:

```
                          10        20        30        40        50        60
orf101-1.pep   MIYQRNLIKELSFTAVGIFVVLLAVLVSTQAINLLGRAADGRVAIDAVLALVGFWVIGMT
               ||||||||||||||||||||||||||||||||||||||||||||||||||||||||||||
orf101ng-1     MIYQRNLIKELSFTAVGIFVVLLAVLVSTQAINLLGRAADGRVAIDAVLALVGFWVIGMT
                          10        20        30        40        50        60

                          70        80        90       100       110       120
orf101-1.pep   PLLLVLTAFISTLTVLTRYWRDSEMSVWLSCGLALKQWIRPVMQFAVPFAVLVAVMQLWV
               ||||||||||||||||||||||||||||||||||||||||||||||||||:|:|||||||
orf101ng-1     PLLLVLTAFISTLTVLTRYWRDSEMSVWLSCGLALKQWIRPVMQFAVPFAILIAVMQLWV
                          70        80        90       100       110       120

                         130       140       150       160       170       180
orf101-1.pep   IPWAELRSREYAEILKQKQELSLVEAGEFNSLGKRNGRVYFVETFDTESGIMKNLFLREQ
               ||||||||||||||||||||||||||||||:|||||||||||||||||||||||||||||
orf101ng-1     IPWAELRSREYAEILKQKQELSLVEAGEFNNLGKRNGRVYFVETFDTESGIMKNLFLREQ
                         130       140       150       160       170       180

                         190       200       210       220       230       240
orf101-1.pep   DKNGGDNIIFAKEGNFSLNDNKRTLELRHGYRYSGTPGRADYNQVSFQKLNLIISTTPKL
               ||||||||||||||||||||||:|||||||||||||||||||||||||||||||||||||||||||||
orf101ng-1     DKNGGDNIIFAKEGNFSLKDNKRTLELRHGYRYSGTPGRADYNQVSFQKLNLIISTTPKL
                         190       200       210       220       230       240

                         250       260       270       280       290       300
orf101-1.pep   IDPVSHRRTIPTAQLIGSSNPQHQAELMWRISLTVSVLLLCLLAVPLSYFNPRSGHTYNI
               |||||||||| |||||||||||||||||||||||||||||||||||||||||||||||||
orf101ng-1     IDPVSHRRTISTAQLIGSSNPQHQAELMWRISLTVSVLLLCLLAVPLSYFNPRSGHTYNI
                         250       260       270       280       290       300

                         310       320       330       340       350       360
orf101-1.pep   LIAIGLFLIYQNGLTLLFEAVEDGKIHFWLGLLPMHIIMFAVALILLRVRSMPSQPFWQA
               ||||||||||||||||||||||||||||||||||||||||::|::|||||||||||||||
orf101ng-1     LIAIGLFLIYQNGLTLLFEAVEDGKIHFWLGLLPMHIIMFVIAIVLLRVRSMPSQPFWQA
                         310       320       330       340       350       360

                         370
orf101-1.pep   VGKSLTLKGGKX
               ||||||||||||
orf101ng-1     VGKSLTLKGGKX
                         370
```

Based on this analysis, including the presence of a putative leader sequence (double-underlined) and several putative transmembrane domains (single-underlined) in the gonococcal protein, it is predicted that the proteins from *N.meningitidis* and *N.gonorrhoeae,* and their epitopes, could be useful antigens for vaccines or diagnostics, or for raising antibodies.

**Example 60**

The following partial DNA sequence was identified in *N.meningitidis* <SEQ ID 507>:

```
  1  ..GGTGGTGGTT TTATCAATGC TTCCTGTGCC ACTTTGACGA CAGCCAAACC
 51    GCAATATCAA GCAGGAGACC TTAGCGCTTT TAAGATAAGG CAAGGCAATG
101    TTGTAATCGC CGGACACGGT TTGGATGCAC GTGATACCGA TTACACACGT
151    ATTCTCAGTT ATCATTCCAA AATCGATGCA CCCGTATGGG GACAAGATGT
201    TCGTGTCGTC GCGGGACAAA ACGATGTGGC CGCAACAGGT GATGCACATT
251    CGCCTATTCT CAATAATGCT GCTGCCAATA CGTCAAACAA TACAGCCAAC
301    AACGGCACAC ATATCCCTTT ATTTGCGATT GATACAGGCA AATTAGGAGG
351    TAT.GTATGC CAACAAAATC ACCTTGATCA GTACGGTCGA GCAAGCAGGC
401    ATTCGTAA
```

This corresponds to the amino acid sequence <SEQ ID 508; ORF113>:

```
  1  ..GGGFINASCA TLTTAKPQYQ AGDLSAFKIR QGNVVIAGHG LDARDTDYTR
 51    ILSYHSKIDA PVWGQDVRVV AGQNDVAATG DAHSPILNNA AANTSNNTAN
101    NGTHIPLFAI DTGKLGGXVC QQNHLDQYGR ASRHS*
```

Computer analysis of this amino acid sequence gave the following results:

Homology with with pspA putative secreted protein of *N.meningitidis* (accession AF030941)

ORF and pspA show 44% aa identity in 179aa overlap:

```
orf113   GGGFINASCATLTTAKPQYQAGDLSAFKIRQGNVVIAGHGLDARDTDYTRILSYHSKIDA 60
         GGG INA+  TLT+  P    G+L+ F +  G VVI G GLD  D DYTRILS  ++I+A
pspa     GGGLINAASVTLTSGVPVLNNGNLTGFDVSSGKVVIGGKGLDTSDADYTRILSRAAEINA 256

orf113   PVWGQDVRVVAGQNDVAATGDAHSPILXXXXXXXXXXXXXXGTHIPLFAIDTGKLGGMYA 120
          VWG+DV+VV+G+N +   G                     +  P  AIDT  LGGMYA
pspa     GVWGKDVKVVSGKNKLDFDG---------SLAKTASAPSSSDSVTPTVAIDTATLGGMYA 307

orf113   NKITLISTVEQAGIRNQGQWFASAGNVAVNAEGKLVNTGMIAATGENHAVSLHARNVHN 179
         +KITLIST   A IRN+G+ FA+ G V ++A+GKL N+G I A      +++ A+ V N
pspa     DKITLISTDNGAVIRNKGRIFAATGGVTLSADGKLSNSGSIDAA----EITISAQTVDN 362
```

Homology with a predicted ORF from *N.gonorrhoeae*

ORF113 shows 86.5% identity in 52aa overlap at the N- terminal part and 94.1% identity in l7aa overlap at the C-terminal part with a predicted ORF (ORF113ng) from *N. gonorrhoeae:*

```
orf113                                   GGGFINASCATLTTAKPQYQAGDLSAFKIR   30
                                         |||||||| |||||::|||||||:|:||||
orf113ng   SHPSQLNGYIEVGGRRAEVVIANPAGIAVNGGGFINASRATLTTGQPQYQAGDFSGFKIR  224

orf113     QGNVVIAGHGLDARDTDYTRILSYHSKIDAPVWGQDVRVVAGQNDVAATGDAHSPILNNA   90
           |||:|||||||||||||:||||
orf113ng   QGNAVIAGHGLDARDTDFTRILVCQQNHLDQYGRTSRHS  263

orf113                         IDTGKLGGXVCQQNHLDQYGRASRHS  135
                                         ||||||||||||:||||
orf113ng   DFSGFKIRQGNAVIAGHGLDARDTDFTRILVCQQNHLDQYGRTSRHS  263
```

The complete length ORF113ng nucleotide sequence <SEQ ID 509> is predicted to encode a protein having amino acid sequence <SEQ ID 510>:

```
  1  MNKTLYRVIF NRKRGAVVAV AETTKREGKS CADSGSGSVY VKSVSFIPTH
 51  SKAFCFSALG FSLCLALGTV NIAFADGIIT DKAAPKTQQA TILQTGNGIP

101  QVNIQTPTSA GVSVNQYAQF DVGNRGAILN NSRSNTQTQL GGWIQGNPWL
151  TRGEARVVVN QINSSHPSQL NGYIEVGGRR AEVVIANPAG IAVNGGGFIN
201  ASRATLTTGQ PQYQAGDFSG FKIRQGNAVI AGHGLDARDT DFTRILVCQQ
251  NHLDQYGRTS RHS*
```

Based on this analysis, it is predicted that these proteins from *N.meningitidis* and *N.gonorrhoeae,* and their epitopes, could be useful antigens for vaccines or diagnostics, or for raising antibodies.

**Example 61**

The following partial DNA sequence was identified in *N.meningitidis* <SEQ ID 511>:

```
  1 ..TCAACGGGAC ATAGCGAACA AAATTACACT TTGCCGCGAG AAATCACACG
 51   CAACATTTCA CTGGGTTCAT TTGCCTATGA ATCGCATCGC AAAGCATTAA
101   GCCATCATGC GCCCAGCCAA GGCACTGAGT TGCCGCAAAG CAACGGTATT
151   TCGCTACCCT ATACGTCCAA TTCTTTTACC CCATTACCCA GCAGCAGCTT
201   ATACATTATC AATCCTGTCA ATAAAGGCTA TCTTGTTGAA ACCGATCCAC
251   GCTTTGCCAA CTACCGTCAA TGGTTGGGTA GTGACTATAT GCtGGACAGC
301   CTCAAACTAG ACCCAAACAA TTTACATAAA CGTTTGGGTG ATGGTTATTA
351   CGAGCAACGT TTAATCAATG AACAAATCGC AGAGCTGACA GGGCATCGTC
401   GTTTAGAcGG TTATCAAAAC GACGAAGAAC AATTTAAAGC CTTAATGGAT
451   AATGGCGCGA CTGCGGCACG TTcGATGAAT CTCAGCGTTG GCATTGCATT
501   AAGTGCCGAG CAAGTAGCGC AACTGACCAG CGATATTGTT TGGTTGGTAC
551   AAAAAGAAGT TAAGCTTCCT GATGGCGGCA CACAAACCGT ATTGGTGCCA
601   CAGGTTTATG TACGCGTTAA AAATGGCGAC ATAGACGGTA AAGGTGCATT
651   GTTGTCAGGC AGCAATACAC AAATCAATGT TTCAGGCAGC CTGAAAAACT
701   CAGGCACGAT TGCAGGgCGC AATGCGCTTA TTATCAATAC CGATACGCTA
751   GACAATATCG GTGGGCGTAT TCATGCGCAA AAATCAGCGG TTACGGCCAC
801   ACAAGACATC AATAATATTG GCGGCATGCT TTCTGCCGAA CAGACATTAT
851   TGCTCAACGC AGGCAACAAC ATCAACAGCC AAAGCACCAC CGCCAGCAGT
901   CAAAATACAC AAGGCAGCAG CACCTACCTA GACCGAATGG CAGGTATTTA
951   TATCACAGGC AAAGAAAAAG GTGTTT..
```

This corresponds to the amino acid sequence <SEQ ID 512; ORF115>:

```
  1 ..STGHSEQNYT LPREITRNIS LGSFAYESHR KALSHHAPSQ GTELPQSNGI
 51   SLPYTSNSFT PLPSSSLYII NPVNKGYLVE TDPRFANYRQ WLGSDYMLDS
101   LKLDPNNLHK RLGDGYYEQR LINEQIAELT GHRRLDGYQN DEEQFKALMD
151   NGATAARSMN LSVGIALSAE QVAQLTSDIV WLVQKEVKLP DGGTQTVLVP
201   QVYVRVKNGD IDGKGALLSG SNTQINVSGS LKNSGTIAGR NALIINTDTL
251   DNIGGRIHAQ KSAVTATQDI NNIGGMLSAE QTLLLNAGNN INSQSTTASS
301   QNTQGSSTYL DRMAGIYITG KEKGV..
```

Computer analysis of this amino acid sequence gave the following results:

Homology with the pspA putative secreted protein of *N.meningitidis* (accession number AF030941)

ORF115 and pspA protein show 50% aa identity in 325aa overlap:

```
Orf115: 1    STGHSEQNYTLPREITRNISLGSFAYESHRKALSHHAPSQGTELPQSNGISLPYTSNSFT 60
             STG+S   Y    E++ +I +G  AY+ +    +    P     +   NGI   +T
pspA:   778  STGYSRSPYEPAPEVS-SIRMGISAYKGYAPQQASDIPGTVVPVVAENGIHPTFT----- 831

Orf115: 61   PLPSSSLYIINPVNKGYLVETDPRFANYRQWLGSDYMLDSLKLDPNNLHKRLGDGYYEQR 120
              LP+SSL+ I P NKGYL+ETDP F +YR+WLGS YML +L+ DPN++HKRLGDGYYEQ+
pspA:   832  -LPNSSLFAIAPNNKGYLIETDPAFTDYRKWLGSGYMLAALQQDPNHIHKRLGDGYYEQK 890

Orf115: 121  LINEQIAELTGHRRLDGYQNDEEQFKALMDNGATAARSMNLSVGIALSAEQVAQLTSDIV 180
             L+NEQIA+LTG+RRLDGY NDEEQFKALMDNG T A+ + L+ GIALSAEQVA+LTSDIV
pspA:   891  LVNEQIAKLTGYRRLDGYTNDEEQFKALMDNGITIAKELQLTPGIALSAEQVARLTSDIV 950

Orf115: 181  WLVQKEVKLPDGGTQTVLVPQVYVRVKNGDIDGKGALLSGSNTQINVSGSLKN-SGTIAG 239
             WL  + V LPDG TQTVL P+VYVR +  D++G+GALLSGS   I  SG+++N  G IAG
pspA:   951  WLENETVTLPDGTTQTVLKPKVYVRARPKDMNGQGALLSGSVVDIG-SGAIENRGGLIAG 1009

Orf115: 240  RNALIINTDTLDNIGGRIHAQKSAVTATQDINNIGGMLSAEQTLLLNAGXXXXXXXXXXX 299
             R ALI+N   + N+ G +  +     A  DI N G  + AE  LLL A
pspA:   1010 REALILNAQNIKNLQGDLQGKNIFAAAGSDITNTGS-IGAENALLLKASNNIESRSETRS 1068
```

```
Orf115: 300  XXXXXXXXXYLDRMAGIYITGKEKG 324
                       + R+AGIY+TG++ G
pspA:   1069 NQNEQGSVRNIGRVAGIYLTGRQNG 1093
```

Homology with a predicted ORF from *N.gonorrhoeae*

ORF115 shows 91.9% identity over a 334aa overlap with a predicted ORF (ORF115ng) from *N.gonorrhoeae:*

```
orf115.pep                                        STGHSEQNYTLPREITRNISLGSFAYESHRK   31
                                                  ||| |||||||:||||:||||||||||| |
orf115ng    NEQTFGEKKVFSENGKLHNYWRARRKGHDETGHREQNYTLPEEITRDISLGSFAYESHSK   71

orf115.pep  ALSHHAPSQGTELPQSN----------GISLPYTSNSFTPLPSSSLYIINPVNKGYLVET   81
            |||:|||||||||||||          ||||||| |||||||:||||||||:||||||||
orf115ng    ALSRHAPSQGTELPQSNRDNIRTAKSNGISLPYTPNSFTPLPGSSLYIINPANKGYLVET  131

orf115.pep  DPRFANYRQWLGSDYMLDSLKLDPNNLHKRLGDGYYEQRLINEQIAELTGHRRLDGYQND  141
            ||||||||||||||||| ||||||||||||||||||||||||||||||||||||||||||
orf115ng    DPRFANYRQWLGSDYMLGSLKLDPNNLHKRLGDGYYEQRLINEQIAELTGHRRLDGYQND  191

orf115.pep  EEQFKALMDNGATAARSMNLSVGIALSAEQVAQLTSDIVWLVQKEVKLPDGGTQTVLVPQ  201
            ||||||||||||||||||||||||||||||:||||||||||||||||||||||||||:||
orf115ng    EEQFKALMDNGATAARSMNLSVGIALSAEQAAQLTSDIVWLVQKEVKLPDGGTQTVLMPQ  251

orf115.pep  VYVRVKNGDIDGKGALLSGSNTQINVSGSLKNSGTIAGRNALIINTDTLDNIGGRIHAQK  261
            |||||||| |||||||||||||||||||||||||||||||||||||||||||||||||||
orf115ng    VYVRVKNGGIDGKGALLSGSNTQINVSGSLKNSGTIAGRNALIINTDTLDNIGGRIHAQK  311

orf115.pep  SAVTATQDINNIGGMLSAEQTLLLNAGNNINSQSTTASSQNTQGSSTYLDRMAGIYITGK  321
            ||||||||||||||:||||||||||||||||:|||: ||||:||||||||||||||||||
orf115ng    SAVTATQDINNIGGILSAEQTLLLNAGNNINNQSTAKSSQNAQGSSTYLDRMAGIYITGK  371

orf115.pep  EKGV                                                         325
            ||||
orf115ng    EKGVLAAQAGKDINIIAGQISNQSDQGQTRLQAGRDINLDTVQTGKYQEIHFDADNHTIR  431
```

An ORF115ng nucleotide sequence <SEQ ID 513> was predicted to encode a protein having amino acid sequence <SEQ ID 514>:

```
  1  MLVQTEKDGL HNEQTFGEKK VFSENGKLHN YWRARRKGHD ETGHREQNYT
 51  LPEEITRDIS LGSFAYESHS KALSRHAPSQ GTELPQSNRD NIRTAKSNGI
101  SLPYTPNSFT PLPGSSLYII NPANKGYLVE TDPRFANYRQ WLGSDYMLGS
151  LKLDPNNLHK RLGDGYYEQR LINEQIAELT GHRRLDGYQN DEEQFKALMD
201  NGATAARSMN LSVGIALSAE QAAQLTSDIV WLVQKEVKLP DGGTQTVLMP
251  QVYVRVKNGG IDGKGALLSG SNTQINVSGS LKNSGTIAGR NALIINTDTL
301  DNIGGRIHAQ KSAVTATQDI NNIGGILSAE QTLLLNAGNN INNQSTAKSS
351  QNAQGSSTYL DRMAGIYITG KEKGVLAAQA GKDINIIAGQ ISNQSDQGQT
401  RLQAGRDINL DTVQTGKYQE IHFDADNHTI RGSTNEVGSS IQTKGDVTLL
451  SGNNLNAKAA EVGSAKGTLA VYAKNDITIS SGIHAGQVDD ASKHTGRSGG
501  GNKLVITDKA QSHHETAQSS TFEGKQVVLQ AGNDANILGS NVISDNGTRI
551  QAGNHVRIGT TQTQSQSETY HQTQKSGLMS AGIGFTIGSK TNTQENQSQS
601  NEHTGSTVGS LKGDTTIVAS KHYEQTGSNV SSPEGNNLIS TQSMDIGAAQ
651  NQLNSKTTQT YEQKGLTVAF SSPVTDLAQQ AIAVAHKAAK QFDKAKTTAL
701  MPWRLPMQVG RLFKQAKAPK K*
```

Further work revealed the following partial gonococcal DNA sequence <SEQ ID 515>:

```
   1  TTGCTTGTGC AAACAGAAAA AGACGGTTTG CATAACGAGC AAACCTTTGG
  51  CGAGAAGAAA GTCTTCAGCG AAAATGGTAA GTTGCACAAC TACTGGCGTG
 101  CGCGTCGTAA AGGACATGAT GAAACAGGGC ATCGTGAACA AAATTATACT
 151  TTGCCGGAGG AAATCACACG CGACATTTCA CTGGGTTCAT TTGCCTATGA
 201  ATCGCATAGC AAAGCATTAA GCCGTCATGC GCCCAGCCAA GGCACTGAGT
 251  TGCCACAAAG TAACCGGGAT AATATCCGTA CTGCGAAAAG CAACGGTATT
 301  TCGCTACCCT ATACGCCCAA TTCTTTTACC CCATTACCCG GCAGCAGCTT
 351  ATACATTATC AATCCTGCCA ATAAAGGCTA TCTTGTTGAA ACCGATCCAC
 401  GCTTTGCCAA CTACCGTCAA TGGTTGGGTA GTGACTATAT GCTGGGCAGC
```

```
 451  CTCAAACTAG ACCCAAACAA TTTACATAAA CGTTTGGGTG ATGGTTATTA
 501  CGAGCAACGT TTAATCAATG AACAAATCGC AGAGCTGACA GGGCATCGTC
 551  GTTTAGACGG TTATCAAAAC GACGAAGAAC AATTTAAAGC CTTAATGGAT
 601  AATGGCGCGA CTGCGGCACG TTCGATGAAT CTCAGCGTTG GCATTGCATT
 651  AAGTGCCGAG CAAGCAGCGC AACTGACCAG CGATATTGTT TGGTTGGTAC
 701  AAAAAGAAGT TAAACTTCCT GATGGCGGCA CACAAACCGT ATTGATGCCA
 751  CAGGTTTATG TACGCGTTAA AAATGGCGGC ATAGACGGTA AAGGTGCATT
 801  GTTGTCAGGC AGCAATACAC AAATCAATGT TTCAGGCAGC CTGAAAAACT
 851  CAGGCACGAT TGCAGGGCGC AATGCGCTTA TTATCAATAC CGATACGCTA
 901  GACAATATCG GTGGGCGTAT TCATGCGCAA AAATCAGCGG TTACGGCCAC
 951  ACAAGACATC AATAATATTG GCGGCATTCT TTCTGCCGAA CAGACATTAT
1001  TGCTCAATGC GGGTAACAAC ATCAACAACC AAAGCACGGC CAAGAGCAGT
1051  CAAAATGCAC AAGGTAGCAG CACCTACCTA GACCGAATGG CAGGTATTTA
1101  TATCACAGGC AAAGAAAAAG GTGTTTTAGC AGCGCAGGCA GGCAAAGACA
1151  TCAACATCAT TGCCGGTCAA ATCAGCAATC AATCAGATCA AGGGCAAACC
1201  CGGCTGCAGG CAGGACGCGA CATTAACCTG GATACGGTAC AAACCGGCAA
1251  ATATCAAGAA ATCCATTTTG ATGCCGATAA CCATACCATC CGAGGTTCAA
1301  CGAACGAAGT CGGCAGCAGC ATTCAAACAA AAGGCGATGT TACCCtatTG
1351  TCAGGGAATA ATCTCAATGC CAAAGCTGCC GAAGTCGGCA GCGCAAAAGG
1401  CACACTTGCC GTGTATGCTA AAAATGACAT TACTATCAGC TCAGGCATCC
1451  ATGCCGGCCA AGTTGATGAT GCGTCCAAAC ATACAGGCAG AAGCGGCGGC
1501  GGTAATAAAT TAGTCATTAC CGATAAAGCC CAAAGTCATC ACGAAACTGC
1551  TCAAAGCAGC ACCTTTGAAG GCAAGCAAGT TGTATTGCAG GCAGGAAACG
1601  ATGCCAACAT CCTTGGCAGT AATGTTATTT CCGATAATGG CACCCGGATT
1651  CAAGCAGGCA ATCATGTTCG CATTGGTACA ACCCAAACTC AAAGCCAAAG
1701  CGAAACCTAT CATCAAACCC AAAAATCAGG ATTGATGAGT GCAGGTATCG
1751  GCTTCACTAT TGGCAGCAAG ACAAACACAC AAGAAAACCA ATCCCAAAGC
1801  AACGAACATA CAGGCAGTAC CGTAGGCAGC CTGAAAGGCG ATACCACCAT
1851  TGTTGCAAGC AAACACTACG AACAAACCGG CAGCAACGTT TCCAGCCCTG
1901  AGGGCAACAA CCTTATCAGC ACGCAAAGTA TGGATATTGG CGCAGCACAA
1951  AACCAATTAA ACAGCAAAAC CACCCAAACC TACGAACAAA AAGGCTTAAC
2001  GGTGGCATTC AGTTCGCCCG TTACCGATTT GGCACAACAA GCGATTGCCG
2051  TAGCACACAA AGCAGCAAAC AAGTCGGACA AAGCAAAAAC GACCGCGTTA
2101  ATGCCATGGC GGCTGCCAAT GCAGGTTGGC AGGCCTATCA AACAGGCAAA
2151  GGCGCACAAA ACTTAG
```

This corresponds to the amino acid sequence <SEQ ID 516; ORF115ng-1>:

```
  1 LLVQTEKDGL HNEQTFGEKK VFSENGKLHN YWRARRKGHD ETGHREQNYT
 51 LPEEITRDIS LGSFAYESHS KALSRHAPSQ GTELPQSNRD NIRTAKSNGI
101 SLPYTPNSFT PLPGSSLYII NPANKGYLVE TDPRFANYRQ WLGSDYMLGS
151 LKLDPNNLHK RLGDGYYEQR LINEQIAELT GHRRLDGYQN DEEQFKALMD
201 NGATAARSMN LSVGIALSAE QAAQLTSDIV WLVQKEVKLP DGGTQTVLMP
251 QVYVRVKNGG IDGKGALLSG SNTQINVSGS LKNSGTIAGR NALIINTDTL
301 DNIGGRIHAQ KSAVTATQDI NNIGGILSAE QTLLLNAGNN INNQSTAKSS
351 QNAQGSSTYL DRMAGIYITG KEKGVLAAQA GKDINIIAGQ ISNQSDQGQT
401 RLQAGRDINL DTVQTGKYQE IHFDADNHTI RGSTNEVGSS IQTKGDVTLL
451 SGNNLNAKAA EVGSAKGTLA VYAKNDITIS SGIHAGQVDD ASKHTGRSGG
501 GNKLVITDKA QSHHETAQSS TFEGKQVVLQ AGNDANILGS NVISDNGTRI
551 QAGNHVRIGT TQTQSQSETY HQTQKSGLMS AGIGFTIGSK TNTQENQSQS
601 NEHTGSTVGS LKGDTTIVAS KHYEQTGSNV SSPEGNNLIS TQSMDIGAAQ
651 NQLNSKTTQT YEQKGLTVAF SSPVTDLAQQ AIAVAHKAAN KSDKAKTTAL
701 MPWRLPMQVG RPIKQAKAHK T*
```

This gonococcal protein (ORF115ng-1) shows 91.9% identity with ORF115 over 334aa:

```
                     20        30        40        50        60        70
orf115ng-1.p NEQTFGEKKVFSENGKLHNYWRARRKGHDETGHREQNYTLPEEITRDISLGSFAYESHSK
                                           ||| |||||||:||||:||||||||||| |
orf115                                    STGHSEQNYTLPREITRNISLGSFAYESHRK
                                                  10        20        30

                     80        90       100       110       120       130
orf115ng-1.p ALSRHAPSQGTELPQSNRDNIRTAKSNGISLPYTPNSFTPLPGSSLYIINPANKGYLVET
             |||:|||||||||||||          ||||||| |||||||:||||||||:||||||||
orf115       ALSHHAPSQGTELPQSN----------GISLPYTSNSFTPLPSSSLYIINPVNKGYLVET
                     40                  50        60        70        80

                    140       150       160       170       180       190
orf115ng-1.p DPRFANYRQWLGSDYMLGSLKLDPNNLHKRLGDGYYEQRLINEQIAELTGHRRLDGYQND
             ||||||||||||||||| ||||||||||||||||||||||||||||||||||||||||||
orf115       DPRFANYRQWLGSDYMLDSLKLDPNNLHKRLGDGYYEQRLINEQIAELTGHRRLDGYQND



                     90       100       110       120       130       140

                    200       210       220       230       240       250
orf115ng-1.p EEQFKALMDNGATAARSMNLSVGIALSAEQAAQLTSDIVWLVQKEVKLPDGGTQTVLMPQ
             ||||||||||||||||||||||||||||||:||||||||||||||||||||||||||:||
orf115       EEQFKALMDNGATAARSMNLSVGIALSAEQVAQLTSDIVWLVQKEVKLPDGGTQTVLVPQ
                    150       160       170       180       190       200

                    260       270       280       290       300       310
orf115ng-1.p VYVRVKNGGIDGKGALLSGSNTQINVSGSLKNSGTIAGRNALIINTDTLDNIGGRIHAQK
             |||||||| |||||||||||||||||||||||||||||||||||||||||||||||||||
orf115       VYVRVKNGDIDGKGALLSGSNTQINVSGSLKNSGTIAGRNALIINTDTLDNIGGRIHAQK
                    210       220       230       240       250       260

                    320       330       340       350       360       370
orf115ng-1.p SAVTATQDINNIGGILSAEQTLLLNAGNNINNQSTAKSSQNAQGSSTYLDRMAGIYITGK
             |||||||||||||||:|||||||||||||||:|||: ||||:||||||||||||||||||
orf115       SAVTATQDINNIGGMLSAEQTLLLNAGNNINSQSTTASSQNTQGSSTYLDRMAGIYITGK
                    270       280       290       300       310       320

                    380       390       400       410       420       430
orf115ng-1.p EKGVLAAQAGKDINIIAGQISNQSDQGQTRLQAGRDINLDTVQTGKYQEIHFDADNHTIR
             ||||
orf115       EKGV
```

In addition, it shows homology with a secreted *N.meningitidis* protein in the database:

```
gi|2623258 (AF030941) putative secreted protein [Neisseria meningitidis] Length
= 2273
 Score =  604 bits (1541), Expect = e-172
 Identities = 325/678 (47%), Positives = 449/678 (65%), Gaps = 22/678 (3%)

Query: 1     LLVQTEKDGLHNEQTFGEKKVFSENGKLHNYWRARRKGHDETGHREQNYTLPEEITRDIS 60
              L+V T +   L N++T G K + ++ G LH Y R  +KG D TG+    Y    E++  I
Sbjct: 739   LIVGTPESALDNDETLGTKTI-TDKGDLHRYHRHHKKGRDSTGYSRSPYEPAPEVS-SIR 796

Query: 61    LGSFAYESHSKALSRHAPSQGTELPQSNRDNIRTAKSNGISLPYTPNSFTPLPGSSLYII 120
              +G  AY+ +       AP Q +++P +     +     NGI   +T      LP SSL+ I
Sbjct: 797   MGISAYKGY-------APQQASDIPGTV---VPVVAENGIHPTFT------LPNSSLFAI 840

Query: 121   NPANKGYLVETDPRFANYRQWLGSDYMLGSLKLDPNNLHKRLGDGYYEQRLINEQIAELT 180
               P NKGYL+ETDP F +YR+WLGS YML +L+ DPN++HKRLGDGYYEQ+L+NEQIA+LT
Sbjct: 841   APNNKGYLIETDPAFTDYRKWLGSGYMLAALQQDPNHIHKRLGDGYYEQKLVNEQIAKLT 900

Query: 181   GHRRLDGYQNDEEQFKALMDNGATAARSMNLSVGIALSAEQAAQLTSDIVWLVQKEVKLP 240
              G+RRLDGY NDEEQFKALMDNG T A+ + L+ GIALSAEQ A+LTSDIVWL  + V LP
Sbjct: 901   GYRRLDGYTNDEEQFKALMDNGITIAKELQLTPGIALSAEQVARLTSDIVWLENETVTLP 960

Query: 241   DGGTQTVLMPQVYVRVKNGGIDGKGALLSGSNTQINVSGSLKN-SGTIAGRNALIINTDT 299
              DG TQTVL P+VYVR +    ++G+GALLSGS    I  SG+++N  G IAGR ALI+N
Sbjct: 961   DGTTQTVLKPKVYVRARPKDMNGQGALLSGSVVDIG-SGAIENRGGLIAGREALILNAQN 1019

Query: 300   LDNIGGRIHAQKSAVTATQDINNIGGILSAEQTLLLNAGNNINNQSTAKSSQNAQGSSTY 359
              + N+ G +  +     A  DI N G I  AE  LLL A NNI ++S  +S+QN QGS
Sbjct: 1020  IKNLQGDLQGKNIFAAAGSDITNTGSI-GAENALLLKASNNIESRSETRSNQNEQGSVRN 1078

Query: 360   LDRMAGIYITGKEKGVLAAQAGKDINIIAGQISNQSDQGQTRLQAGRDINLDTVQTGKYQ 419
              + R+AGIY+TG++ G +   AG +I + A +++NQS+ GQT L AG DI  DT    + Q
Sbjct: 1079  IGRVAGIYLTGRQNGSVLLDAGNNIVLTASELTNQSEDGQTVLNAGGDIRSDTTGISRNQ 1138

Query: 420   EIHFDADNHTIRGSTNEVGSSIQTKGDVTLLSGNNLNAKAAEVGSAKGTLAVYAKNDITI 479
                 FD+DN+ IR   NEVGS+I+T+G+++L +  ++  +AAEVGS +G L + A  DI +
Sbjct: 1139  NTIFDSDNYVIRKEQNEVGSTIRTRGNLSLNAKGDIRIRAAEVGSEQGRLKLAAGRDIKV 1198

Query: 480   SSGIHAGQVDDASKHTGRSGGGNKLVITDKAQSHHETAQSSTFEGKQVVLQAGNDANILG 539
               +G    + +DA K+TGRSGGG K  +T    ++ +  A S T +GK+++L +G D  + G
Sbjct: 1199  EAGKAHTETEDALKYTGRSGGGIKQKMTRHLKNQNGQAVSGTLDGKEIILVSGRDITVTG 1258

Query: 540   SNVISDNGTRIQAGNHVRIGTTQTQSQSETYHQTQKSGLM-SAGIGFTIGSKTNTQENQS 598
              SN+I+DN T + A N++ +   +T+S+S   ++ +KSGLM S GIGFT GSK +TQ N+S
Sbjct: 1259  SNIIADNHTILSAKNNIVLKAAETRSRSAEMNKKEKSGLMGSGGIGFTAGSKKDTQTNRS 1318

Query: 599   QSNEHTGSTVGSLKGDTTIVASKHYEQTGSNVSSPEGNNLISTQSMDIGAAQNQLNSKTT 658
              ++  HT S VGSL G+T I A KHY QTGS +SSP+G+  IS+  + I AAQN+ + ++
Sbjct: 1319  ETVSHTESVVGSLNGNTLISAGKHYTQTGSTISSPQGDVGISSGKISIDAAQNRYSQESK 1378

Query: 659   QTYEQKGLTVAFSSPVTD 676
              Q YEQKG+TVA S PV +
Sbjct: 1379  QVYEQKGVTVAISVPVVN 1396
```

Based on this analysis, it is predicted that the proteins from *N.meningitidis* and *N.gonorrhoeae,* and their epitopes, could be useful antigens for vaccines or diagnostics, or for raising antibodies.

**Example 62**

The following partial DNA sequence was identified in *N.meningitidis* <SEQ ID 517>:

```
  1  ..TCAGGGAATA ACCTCAATGC CAAAGCTGCC GAAGTCAGCA GCGCAAACGG
 51    TACACTCGCT GTGTCTGCCA ATAATGACAT CAACATCAGC GCAGGCATCA
101    ACACGACCCA TGTTGATGAT GCGTCCAAAC ACACAGGCAG AAGCGGTGGT
151    GGCAATAAAT TAGTCATTAC CGATAAAGCC CAAAGTCATC ACGAAACCGC
201    CCAAAGCAGC ACCTTTGAAG GCAAGCAAGT TGTATTGCAG GCAGGAAACG
251    ATGCCAACAT CCTTGGCAGC AATGTTATTT CCGATAATGG CACCCAGATT
301    CAAGCAGGCA ATCATGTTCG CATTGGTACA ACCCAAACTC AAAGCCAAAG
351    CGAAACCTAT CATCAAACCC AGAAATCAGG ATTGATGAGT GCAGGTATCG
401    GCTTCACTAT TGGCAGCAAG ACAAACACAC AAGAAAACCA ATCCCAAAGC
451    AACGAACATA CAGGCAGTAC CGTAGGCAGC TTGAAAGGCG ATACCACCAT
501    TGTTGCAGGC AAACACTACG AACAAATCGG CAGTACCGTT TCCAGCCCGG
551    AAGGCAACAA TACCATCTAT GCCCAAAGCA TAGACATTCA AGCGGCACAC
601    AACAAATTAA ACAGTAATAC CACCCAAACC TATGAACAAA AAGG.CTAAC
651    GGTGGCATTC AGTTCGCCCG TTACCGATTT GGCACAACAA ...
```

This corresponds to the amino acid sequence <SEQ ID 518; ORF117>:

```
  1  ..SGNNLNAKAA EVSSANGTLA VSANNDINIS AGINTTHVDD ASKHTGRSGG
 51    GNKLVITDKA QSHHETAQSS TFEGKQVVLQ AGNDANILGS NVISDNGTQI
101    QAGNHVRIGT TQTQSQSETY HQTQKSGLMS AGIGFTIGSK TNTQENQSQS
151    NEHTGSTVGS LKGDTTIVAG KHYEQIGSTV SSPEGNNTIY AQSIDIQAAH
201    NKLNSNTTQT YEQKXLTVAF SSPVTDLAQQ ...
```

Computer analysis of this amino acid sequence gave the following results:

Homology with the pspA putative secreted protein of *N.meningitidis* (accession number AF030941)

ORF117 and pspA protein show 45% aa identity in 224aa overlap:

```
Orf117: 4    NLNAKAAEVSSANGTLAVSANNDINISAGINTTHVDDASKHTGRSGGGNKLVITDKAQSH 63
             ++  +AAEV S  G L ++A  DI + AG   T  +DA K+TGRSGGG K  +T   ++
pspA:   1173 DIRIRAAEVGSEQGRLKLAAGRDIKVEAGKAHTETEDALKYTGRSGGGIKQKMTRHLKNQ 1232

Orf117: 64   HETAQSSTFEGKQVVLQAGNDANILGSNVISDNGTQIQAGNHVRIGTTQTQSQSETYHQT 123
             +  A S T +GK+++L +G D  + GSN+I+DN T + A N++ +   +T+S+S   ++
pspA:   1233 NGQAVSGTLDGKEIILVSGRDITVTGSNIIADNHTILSAKNNIVLKAAETRSRSAEMNKK 1292

Orf117: 124  QKSGLM-SAGIGFTIGSKTNTQENQSQSNEHTGSTVGSLKGDTTIVAGKHYEQIGSTVSS 182
             +KSGLM S GIGFT GSK +TQ N+S++  HT S VGSL G+T I AGKHY Q GST+SS
pspA:   1293 EKSGLMGSGGIGFTAGSKKDTQTNRSETVSHTESVVGSLNGNTLISAGKHYTQTGSTISS 1352

Orf117: 183  PEGNNTIYAQSIDIQAAHNKLNSNTTQTYEQKXLTVAFSSPVTD 226
             P+G+  I +  I I AA N+ +  + Q YEQK +TVA S PV +
pspA:   1353 PQGDVGISSGKISIDAAQNRYSQESKQVYEQKGVTVAISVPVVN 1396
```

Homology with a predicted ORF from *N.gonorrhoeae*

ORF117 shows 90% identity over a 230aa overlap with a predicted ORF (ORF117ng) from *N.gonorrhoeae:*

```
orf117.pep                                   SGNNLNAKAAEVSSANGTLAVSANNDINIS   30
                                             ||||||||||||:||:||||| |:|||:||
orf117ng     IHFDADNHTIRGSTNEVGSSIQTKGDVTLLSGNNLNAKAAEVGSAKGTLAVYAKNDITIS  480

orf117.pep   AGINTTHVDDASKHTGRSGGGNKLVITDKAQSHHETAQSSTFEGKQVVLQAGNDANILGS   90
             :||:: :|||||||||||||||||||||||||||||||||||||||||||||||||||||
orf117ng     SGIHAGQVDDASKHTGRSGGGNKLVITDKAQSHHETAQSSTFEGKQVVLQAGNDANILGS  540

orf117.pep   NVISDNGTQIQAGNHVRIGTTQTQSQSETYHQTQKSGLMSAGIGFTIGSKTNTQENQSQS  150
             ||||||||:|||||||||||||||||||||||||||||||||||||||||||||||||||
orf117ng     NVISDNGTRIQAGNHVRIGTTQTQSQSETYHQTQKSGLMSAGIGFTIGSKTNTQENQSQS  600

orf117.pep   NEHTGSTVGSLKGDTTIVAGKHYEQIGSTVSSPEGNNTIYAQSIDIQAAHNKLNSNTTQT  210
             |||||||||||||||||||:||||| ||:|||||||| | :||:|| ||:|:|||:||||
orf117ng     NEHTGSTVGSLKGDTTIVASKHYEQTGSNVSSPEGNNLISTQSMDIGAAQNQLNSKTTQT  660

orf117.pep   YEQKXLTVAFSSPVTDLAQQ                                         230
             |||| |||||||||||||||
orf117ng     YEQKGLTVAFSSPVTDLAQQAIAVAHKAAKQFDKAKTTALMPWRLPMQVGRLFKQAKAPK  720
```

An ORF117ng nucleotide sequence <SEQ ID 519> was predicted to encode a protein having amino acid sequence <SEQ ID 520>:

```
  1  ..LLVQTEKDGL HNEQTFGEKK VFSENGKLHN YWRARRKGHD ETGHREQNYT
 51    LPEEITRDIS LGSFAYESHS KALSRHAPSQ GTELPQSNRD NIRTAKSNGI
101    SLPYTPNSFT PLPGSSLYII NPANKGYLVE TDPRFANYRQ WLGSDYMLGS
151    LKLDPNNLHK RLGDGYYEQR LINEQIAELT GHRRLDGYQN DEEQFKALMD
201    NGATAARSMN LSVGIALSAE QAAQLTSDIV WLVQKEVKLP DGGTQTVLMP
251    QVYVRVKNGG IDGKGALLSG SNTQINVSGS LKNSGTIAGR NALIINTDTL
301    DNIGGRIHAQ KSAVTATQDI NNIGGILSAE QTLLLNAGNN INNQSTAKSS
351    QNAQGSSTYL DRMAGIYITG KEKGVLAAQA GKDINIIAGQ ISNQSDQGQT
401    RLQAGRDINL DTVQTGKYQE IHFDADNHTI RGSTNEVGSS IQTKGDVTLL
451    SGNNLNAKAA EVGSAKGTLA VYAKNDITIS SGIHAGQVDD ASKHTGRSGG
501    GNKLVITDKA QSHHETAQSS TFEGKQVVLQ AGNDANILGS NVISDNGTRI
551    QAGNHVRIGT TQTQSQSETY HQTQKSGLMS AGIGFTIGSK TNTQENQSQS
601    NEHTGSTVGS LKGDTTIVAS KHYEQTGSNV SSPEGNNLIS TQSMDIGAAQ
651    NQLNSKTTQT YEQKGLTVAF SSPVTDLAQQ AIAVAHKAAK QFDKAKTTAL
701    MPWRLPMQVG RLFKQAKAPK K*
```

Further work revealed the following gonococcal partial DNA sequence <SEQ ID 521>:

1 TTGCTTGTGC AAACAGAAAA AGACGGTTTG CATAACGAGC AAACCTTTGG
51 CGAGAAGAAA GTCTTCAGCG AAAATGGTAA GTTGCACAAC TACTGGCGTG
101 CGCGTCGTAA AGGACATGAT GAAACAGGGC ATCGTGAACA AAATTATACT
151 TTGCCGGAGG AAATCACACG CGACATTTCA CTGGGTTCAT TTGCCTATGA
201 ATCGCATAGC AAAGCATTAA GCCGTCATGC GCCCAGCCAA GGCACTGAGT
251 TGCCACAAAG TAACCGGGAT AATATCCGTA CTGCGAAAAG CAACGGTATT
301 TCGCTACCCT ATACGCCCAA TTCTTTTACC CCATTACCCG GCAGCAGCTT
351 ATACATTATC AATCCTGCCA ATAAAGGCTA TCTTGTTGAA ACCGATCCAC
401 GCTTTGCCAA CTACCGTCAA TGGTTGGGTA GTGACTATAT GCTGGGCAGC
451 CTCAAACTAG ACCCAAACAA TTTACATAAA CGTTTGGGTG ATGGTTATTA
501 CGAGCAACGT TTAATCAATG AACAAATCGC AGAGCTGACA GGGCATCGTC
551 GTTTAGACGG TTATCAAAAC GACGAAGAAC AATTTAAAGC CTTAATGGAT
601 AATGGCGCGA CTGCGGCACG TTCGATGAAT CTCAGCGTTG GCATTGCATT
651 AAGTGCCGAG CAAGCAGCGC AACTGACCAG CGATATTGTT TGGTTGGTAC
701 AAAAAGAAGT TAAACTTCCT GATGGCGGCA CACAAACCGT ATTGATGCCA
751 CAGGTTTATG TACGCGTTAA AAATGGCGGC ATAGACGGTA AAGGTGCATT
801 GTTGTCAGGC AGCAATACAC AAATCAATGT TTCAGGCAGC CTGAAAAACT
851 CAGGCACGAT TGCAGGGCGC AATGCGCTTA TTATCAATAC CGATACGCTA
901 GACAATATCG GTGGGCGTAT TCATGCGCAA AAATCAGCGG TTACGGCCAC
951 ACAAGACATC AATAATATTG GCGGCATTCT TTCTGCCGAA CAGACATTAT
1001 TGCTCAATGC GGGTAACAAC ATCAACAACC AAAGCACGGC CAAGAGCAGT
1051 CAAAATGCAC AAGGTAGCAG CACCTACCTA GACCGAATGG CAGGTATTTA
1101 TATCACAGGC AAAGAAAAAG GTGTTTTAGC AGCGCAGGCA GGCAAAGACA
1151 TCAACATCAT TGCCGGTCAA ATCAGCAATC AATCAGATCA AGGGCAAACC
1201 CGGCTGCAGG CAGGACGCGA CATTAACCTG GATACGGTAC AAACCGGCAA
1251 ATATCAAGAA ATCCATTTTG ATGCCGATAA CCATACCATC CGAGGTTCAA
1301 CGAACGAAGT CGGCAGCAGC ATTCAAACAA AAGGCGATGT TACCCtatTG
1351 TCAGGGAATA ATCTCAATGC CAAAGCTGCC GAAGTCGGCA GCGCAAAAGG
1401 CACACTTGCC GTGTATGCTA AAAATGACAT TACTATCAGC TCAGGCATCC

1451 ATGCCGGCCA AGTTGATGAT GCGTCCAAAC ATACAGGCAG AAGCGGCGGC
1501 GGTAATAAAT TAGTCATTAC CGATAAAGCC CAAAGTCATC ACGAAACTGC
1551 TCAAAGCAGC ACCTTTGAAG GCAAGCAAGT TGTATTGCAG GCAGGAAACG
1601 ATGCCAACAT CCTTGGCAGT AATGTTATTT CCGATAATGG CACCCGGATT
1651 CAAGCAGGCA ATCATGTTCG CATTGGTACA ACCCAAACTC AAAGCCAAAG
1701 CGAAACCTAT CATCAAACCC AAAAATCAGG ATTGATGAGT GCAGGTATCG
1751 GCTTCACTAT TGGCAGCAAG ACAAACACAC AAGAAAACCA ATCCCAAAGC
1801 AACGAACATA CAGGCAGTAC CGTAGGCAGC CTGAAAGGCG ATACCACCAT
1851 TGTTGCAAGC AAACACTACG AACAAACCGG CAGCAACGTT TCCAGCCCTG
1901 AGGGCAACAA CCTTATCAGC ACGCAAAGTA TGGATATTGG CGCAGCACAA
1951 AACCAATTAA ACAGCAAAAC CACCCAAACC TACGAACAAA AAGGCTTAAC
2001 GGTGGCATTC AGTTCGCCCG TTACCGATTT GGCACAACAA GCGATTGCCG
2051 TAGCACACAA AGCAGCAAAC AAGTCGGACA AAGCAAAAAC GACCGCGTTA
2101 ATGCCATGGC GGCTGCCAAT GCAGGTTGGC AGGCCTATCA AACAGGCAAA
2151 GGCGCACAAA ACTTAG

This corresponds to the amino acid sequence <SEQ ID 522; ORF117ng-1>:

1 LLVQTEKDGL HNEQTFGEKK VFSENGKLHN YWRARRKGHD ETGHREQNYT
51 LPEEITRDIS LGSFAYESHS KALSRHAPSQ GTELPQSNRD NIRTAKSNGI
101 SLPYTPNSFT PLPGSSLYII NPANKGYLVE TDPRFANYRQ WLGSDYMLGS
151 LKLDPNNLHK RLGDGYYEQR LINEQIAELT GHRRLDGYQN DEEQFKALMD
201 NGATAARSMN LSVGIALSAE QAAQLTSDIV WLVQKEVKLP DGGTQTVLMP
251 QVYVRVKNGG IDGKGALLSG SNTQINVSGS LKNSGTIAGR NALIINTDTL
301 DNIGGRIHAQ KSAVTATQDI NNIGGILSAE QTLLLNAGNN INNQSTAKSS
351 QNAQGSSTYL DRMAGIYITG KEKGVLAAQA GKDINIIAGQ ISNQSDQGQT
401 RLQAGRDINL DTVQTGKYQE IHFDADNHTI RGSTNEVGSS IQTKGDVTLL
451 SGNNLNAKAA EVGSAKGTLA VYAKNDITIS SGIHAGQVDD ASKHTGRSGG
501 GNKLVITDKA QSHHETAQSS TFEGKQVVLQ AGNDANILGS NVISDNGTRI
551 QAGNHVRIGT TQTQSQSETY HQTQKSGLMS AGIGFTIGSK TNTQENQSQS
601 NEHTGSTVGS LKGDTTIVAS KHYEQTGSNV SSPEGNNLIS TQSMDIGAAQ
651 NQLNSKTTQT YEQKGLTVAF SSPVTDLAQQ AIAVAHKAAN KSDKAKTTAL
701 MPWRLPMQVG RPIKQAKAHK T*

ORF117ng-1 shows the same 90% identity over a 230aa overlap with ORF117. In addition, it shows homology with a secreted *N.meningitidis* protein in the database:

```
gi|2623258 (AF030941) putative secreted protein [Neisseria meningitidis]Length =
2273
 Score =  604 bits (1541), Expect = e-172
 Identities = 325/678 (47%), Positives = 449/678 (65%), Gaps = 22/678 (3%)

Query: 1    LLVQTEKDGLHNEQTFGEKKVFSENGKLHNYWRARRKGHDETGHREQNYTLPEEITRDIS 60
            L+V T +  L N++T G K + ++ G LH Y R  +KG D TG+    Y    E++  I
Sbjct: 739  LIVGTPESALDNDETLGTKTI-TDKGDLHRYHRHHKKGRDSTGYSRSPYEPAPEVS-SIR 796

Query: 61   LGSFAYESHSKALSRHAPSQGTELPQSNRDNIRTAKSNGISLPYTPNSFTPLPGSSLYII 120
            +G  AY+ +        AP Q +++P +     +      NGI    +T      LP SSL+ I
Sbjct: 797  MGISAYKGY-------APQQASDIPGTV---VPVVAENGIHPTFT------LPNSSLFAI 840

Query: 121  NPANKGYLVETDPRFANYRQWLGSDYMLGSLKLDPNNLHKRLGDGYYEQRLINEQIAELT 180
             P NKGYL+ETDP F +YR+WLGS YML +L+ DPN++HKRLGDGYYEQ+L+NEQIA+LT
Sbjct: 841  APNNKGYLIETDPAFTDYRKWLGSGYMLAALQQDPNHIHKRLGDGYYEQKLVNEQIAKLT 900

Query: 181  GHRRLDGYQNDEEQFKALMDNGATAARSMNLSVGIALSAEQAAQLTSDIVWLVQKEVKLP 240
            G+RRLDGY NDEEQFKALMDNG T A+ + L+ GIALSAEQ A+LTSDIVWL  + V LP
Sbjct: 901  GYRRLDGYTNDEEQFKALMDNGITIAKELQLTPGIALSAEQVARLTSDIVWLENETVTLP 960

Query: 241  DGGTQTVLMPQVYVRVKNGGIDGKGALLSGSNTQINVSGSLKN-SGTIAGRNALIINTDT 299
            DG TQTVL P+VYVR +    ++G+GALLSGS   I  SG+++N  G IAGR ALI+N
Sbjct: 961  DGTTQTVLKPKVYVRARPKDMNGQGALLSGSVVDIG-SGAIENRGGLIAGREALILNAQN 1019

Query: 300  LDNIGGRIHAQKSAVTATQDINNIGGILSAEQTLLLNAGNNINNQSTAKSSQNAQGSSTY 359
            + N+ G +  +    A  DI N G I  AE  LLL A NNI ++S  +S+QN QGS
Sbjct: 1020 IKNLQGDLQGKNIFAAAGSDITNTGSI-GAENALLLKASNNIESRSETRSNQNEQGSVRN 1078

Query: 360  LDRMAGIYITGKEKGVLAAQAGKDINIIAGQISNQSDQGQTRLQAGRDINLDTVQTGKYQ 419
            + R+AGIY+TG++ G +  AG +I + A +++NQS+ GQT L AG DI  DT    + Q
Sbjct: 1079 IGRVAGIYLTGRQNGSVLLDAGNNIVLTASELTNQSEDGQTVLNAGGDIRSDTTGISRNQ 1138

Query: 420  EIHFDADNHTIRGSTNEVGSSIQTKGDVTLLSGNNLNAKAAEVGSAKGTLAVYAKNDITI 479
               FD+DN+ IR    NEVGS+I+T+G+++L +  ++  +AAEVGS +G L + A  DI +
Sbjct: 1139 NTIFDSDNYVIRKEQNEVGSTIRTRGNLSLNAKGDIRIRAAEVGSEQGRLKLAAGRDIKV 1198

Query: 480  SSGIHAGQVDDASKHTGRSGGGNKLVITDKAQSHHETAQSSTFEGKQVVLQAGNDANILG 539
     +G    + +DA K+TGRSGGG K  +T   ++ +  A S T +GK++L +G D  + G
Sbjct: 1199 EAGKAHTETEDALKYTGRSGGGIKQKMTRHLKNQNGQAVSGTLDGKEIILVSGRDITVTG 1258

Query: 540  SNVISDNGTRIQAGNHVRIGTTQTQSQSETYHQTQKSGLM-SAGIGFTIGSKTNTQENQS 598
     SN+I+DN T + A N++ +   +T+S+S  ++ +KSGLM S GIGFT GSK +TQ N+S
Sbjct: 1259 SNIIADNHTILSAKNNIVLKAAETRSRSAEMNKKEKSGLMGSGGIGFTAGSKKDTQTNRS 1318

Query: 599  QSNEHTGSTVGSLKGDTTIVASKHYEQTGSNVSSPEGNNLISTQSMDIGAAQNQLNSKTT 658
     ++  HT S VGSL G+T I A KHY QTGS +SSP+G+  IS+  + I AAQN+ + ++
Sbjct: 1319 ETVSHTESVVGSLNGNTLISAGKHYTQTGSTISSPQGDVGISSGKISIDAAQNRYSQESK 1378

Query: 659  QTYEQKGLTVAFSSPVTD 676
     Q YEQKG+TVA S PV +
Sbjct: 1379 QVYEQKGVTVAISVPVVN 1396
```

Based on this analysis, it is predicted that the proteins from *N.meningitidis* and *N.gonorrhoeae,* and their epitopes, could be useful antigens for vaccines or diagnostics, or for raising antibodies.

**Example 63**

The following partial DNA sequence was identified in *N.meningitidis* <SEQ ID 523>:

```
  1 ATGATTTACA TCGTACTGTT TCTAGCTGTC GTCCTCGCCG TTGTCGCCTA
 51 CAACATGTAT CAGGAAAACC AATACCGCAA AAAAGTGCGC GACCAGTTCG
101 GACACTCCGA CAAAGATGCC CTGCTCAACA GCAwAACCAG CCATGTCCGC
151 GACGGCAAAC CGTCCGGCGG GTCAGTCATG ATGCCGAAAC CCCAACCGGC
201 GGTCAAAAAA ACGGCAAAAC CCCAAGACCC CGyCATGCGC AACCTGCAAG
251 AACAGGATGC CGTCTACATC GCCAAGCAGA AACAGGCAAA AGCCTCCCCG
301 TTCAAAACCG AAATCGAAAC CGCCTTGGAA GAAAGCGGCA TTATCGGCAA
351 CTCCGCCCAC ACCGTTTCCG AACCCCAAAC CGGACATTCC GCAACGAAAC
401 CTGCCGACGC GTCGGCAAAA CCTGCACCCG TTCCGCAAAC ACCTGCAAAA
451 CCGCTGATTA CGCTCAAAGA ACTGTCAAAA GTCGAATTAT CCTGGTTTGA
501 CGTGCGCATC GACTTCATCT CCTAT...
```

This corresponds to the amino acid sequence <SEQ ID 524; ORF119>:

```
  1 MIYIVLFLAV VLAVVAYNMY QENQYRKKVR DQFGHSDKDA LLNSXTSHVR
 51 DGKPSGGSVM MPKPQPAVKK TAKPQDPXMR NLQEQDAVYI AKQKQAKASP
101 FKTEIETALE ESGIIGNSAH TVSEPQTGHS ATKPADASAK PAPVPQTPAK
151 PLITLKELSK VELSWFDVRI DFISY...
```

Further work revealed the complete nucleotide sequence <SEQ ID 525>:

```
   1 ATGATTTACA TCGTACTGTT TCTAGCTGTC GTCCTCGCCG TTGTCGCCTA
  51 CAACATGTAT CAGGAAAACC AATACCGCAA AAAAGTGCGC GACCAGTTCG
 101 GACACTCCGA CAAAGATGCC CTGCTCAACA GCAAAACCAG CCATGTCCGC
 151 GACGGCAAAC CGTCCGGCGG GTCAGTCATG ATGCCGAAAC CCCAACCGGC
 201 GGTCAAAAAA ACGGCAAAAC CCCAAGACCC CGCCATGCGC AACCTGCAAG
 251 AACAGGATGC CGTCTACATC GCCAAGCAGA AACAGGCAAA AGCCTCCCCG
 301 TTCAAAACCG AAATCGAAAC CGCCTTGGAA GAAAGCGGCA TTATCGGCAA
 351 CTCCGCCCAC ACCGTTTCCG AACCCCAAAC CGGACATTCC GCACCGAAAC
 401 CTGCCGACGC GCCGGCAAAA CCTGCACCCG TTCCGCAAAC ACCTGCAAAA
 451 CCGCTGATTA CGCTCAAAGA ACTGTCAAAA GTCGAATTAC CCTGGTTTGA
 501 CGTGCGCTTC GACTTCATCT CCTATATCGC GCTGACCGAA GCCAAAGAAC
 551 TGCACGCACT GCCGCGCCTT TCCAACCGCT GCCGCTACCA GATTGTCGGC
 601 TGCACCATGG ACGACCATTT CCAGATTGCC GAACCCATCC CGGGCATCCG
 651 CTATCAGGCA TTTATCGTGG GTATTCAGGC AGTCAGCCGC AACGGACTTG
 701 CCTCGCAGGA AGAACTCTCC GCATTCAACC GCCAGGTGGA CGCATTCGCA
 751 CAAAGCATGG GCGGTCAGAC GCTGCACACC GACCTTGCCG CCTTTATCGA
 801 AGTGGCTTCC GCACTGGACG CATTCTGCGC GCGCGTCGAC CAGACCATCG
 851 CCATCCATTT GGTTTCCCCG ACCAGCATCA GCGGCGTAGA ACTGCGTTCC
 901 GCCGTAACGG GCGTGGGTTT CGTTTTGGAA GACGACGGCG CGTTCCACTA
 951 TACCGACACG TCGGGCTCGA CCATGTTCTC CATCTGCTCG CTCAACAACG
1001 AGCCGTTTAC CAACGCCCTT TTGGACAACC AGTCCTACAA AGGCTTCAGT
1051 ATGCTGCTCG ACATCCCGCA CTCTCCGGCA GGCGAAAAAA CCTTCGACGA
1101 TTTGTTTATG GATTTGGCGG TACGCCTGTC CGGCCAGTTG AACCTGAATC
1151 TGGTCAACGA CAAAATGGAA GAAGTTTCGA CCCAATGGCT CAAAGACGTG
1201 CGCACTTATG TATTGGCGCG TCAGTCCGAG ATGCTCAAAG TCGGTATCGA
1251 ACCGGGCGGC AAAACCGCAT TGCGCCTGTT CTCCTAA
```

This corresponds to the amino acid sequence <SEQ ID 526; ORF119-1>:

```
  1  MIYIVLFLAV VLAVVAYNMY QENQYRKKVR DQFGHSDKDA LLNSKTSHVR
 51  DGKPSGGSVM MPKPQPAVKK TAKPQDPAMR NLQEQDAVYI AKQKQAKASP
101  FKTEIETALE ESGIIGNSAH TVSEPQTGHS APKPADAPAK PAPVPQTPAK
151  PLITLKELSK VELPWFDVRF DFISYIALTE AKELHALPRL SNRCRYQIVG
201  CTMDDHFQIA EPIPGIRYQA FIVGIQAVSR NGLASQEELS AFNRQVDAFA
251  QSMGGQTLHT DLAAFIEVAS ALDAFCARVD QTIAIHLVSP TSISGVELRS
301  AVTGVGFVLE DDGAFHYTDT SGSTMFSICS LNNEPFTNAL LDNQSYKGFS
351  MLLDIPHSPA GEKTFDDLFM DLAVRLSGQL NLNLVNDKME EVSTQWLKDV
401  RTYVLARQSE MLKVGIEPGG KTALRLFS*
```

Computer analysis of this amino acid sequence gave the following results:

Homology with a predicted ORF from *N.meningitidis* (strain A)

ORF119 shows 93.7% identity over a 175aa overlap with an ORF (ORF119a) from strain A of *N. meningitidis:*

```
                      10        20        30        40        50        60
orf119.pep    MIYIVLFLAVVLAVVAYNMYQENQYRKKVRDQFGHSDKDALLNSXTSHVRDGKPSGGSVM
              ||||||||:||||||||||||||||||||||||||||||||||| ||||||||||||| ||
orf119a       MIYIVLFLAAVLAVVAYNMYQENQYRKKVRDQFGHSDKDALLNSKTSHVRDGKPSGGPVM
                      10        20        30        40        50        60

                      70        80        90       100       110       120
orf119.pep    MPKPQPAVKKTAKPQDPXMRNLQEQDAVYIAKQKQAKASPFKTEIETALEESGIIGNSAH
              ||||||||||||| ||| ||||||||||||||||||||||||||||||||||||||||||
orf119a       MPKPQPAVKKTAKSQDPAMRNLQEQDAVYIAKQKQAKASPFKTEIETALEESGIIGNSAH
                      70        80        90       100       110       120

                     130       140       150       160       170
orf119.pep    TVSEPQTGHSATKPADASAKPAPVPQTPAKPLITLKELSKVELSWFDVRIDFISY
              || |||||||| ||||| |||:||||||||||||||||||||| |||||:|||||
orf119a       TVPEPQTGHSAPKPADAPAKPVPVPQTPAKPLITLKELSKVELPWFDVRFDFISYIALTE
                     130       140       150       160       170       180

orf119a       AKELHALPRLSNRCRYQIVGCTMDDHFQIAEPIPGIRYQAFIVGIQAVSRNGLASQEELS
                     190       200       210       220       230       240
```

The complete length ORF119a nucleotide sequence <SEQ ID 527> is:

```
   1  ATGATTTACA TCGTACTGTT CCTCGCCGCC GTCCTCGCCG TTGTCGCCTA
  51  CAATATGTAT CAGGAAAACC AATACCGCAA AAAAGTGCGC GACCAGTTCG
 101  GGCACTCCGA CAAAGATGCC CTGCTCAACA GCAAAACCAG CCATGTCCGC
 151  GACGGCAAAC CGTCCGGCGG GCCAGTCATG ATGCCGAAAC CCCAACCGGC
 201  GGTCAAAAAA ACGGCAAAAT CCCAAGACCC CGCCATGCGC AACCTGCAAG
 251  AGCAGGATGC CGTCTACATC GCCAAGCAGA AACAGGCAAA AGCCTCCCCG
 301  TTCAAAACCG AAATCGAAAC CGCCTTGGAA GAAAGCGGCA TTATCGGCAA
 351  CTCCGCCCAC ACCGTTCCCG AACCCCAAAC CGGACATTCC GCACCAAAAC
 401  CTGCCGACGC GCCGGCAAAA CCTGTTCCCG TTCCGCAAAC GCCGGCAAAA
 451  CCGCTGATTA CGCTCAAAGA GCTGTCGAAG GTCGAGCTGC CCTGGTTTGA
 501  CGTGCGCTTC GACTTCATCT CTTATATCGC GCTGACCGAA GCCAAAGAAC
 551  TGCACGCACT GCCGCGCCTT TCCAACCGCT GCCGCTACCA GATTGTCGGC
 601  TGCACCATGG ACGACCATTT CCAGATTGCC GAACCCATCC CGGGCATCCG
 651  CTATCAGGCA TTTATCGTGG GTATTCAGGC AGTCAGCCGC AACGGACTTG
 701  CCTCGCAGGA AGAACTCTCC GCATTCAACC GCCAGGTGGA TGCATTCGCA
 751  CACAGCATGG GCGGTCAGAC GCTGCACACC GACCTTGCCG CCTTTATCGA
 801  AGTGGCTTCC GCACTGGACG CATTCTGCGC GCGCGTCGAC CAGACTATCG
 851  CCATCCATTT GGTTTCCCCG ACCAGCATCA GCGGCGTAGA ACTGCGTTCC
 901  GCCGTAACGG GCGTGGGTTT CGTTTTGGAA GACGACGGCG CGTTCCACTA
 951  TACCGACACG TCGGGCTCGA CCATGTTCTC CATCTGCTCG CTCAACAACG
1001  AGCCGTTTAC CAATGCCCTT TTGGACAACC AGTCCTATAA AGGCTTCAGT
```

```
1051  ATGCTGCTCG ACATCCCGCA CTCTCCGGCA GGCGAAAAAA CCTTCGACGA
1101  TTTGTTTATG GATTTGGCGG TACGCCTGTC CGGCCAGTTG AACCTGAATC
1151  TGGTCAACGA CAAAATGGAA GAAGTTTCGA CCCAATGGCT CAAAGACGTG
1201  CGCACTTATG TATTGGCTCG TCAGTCCGAG ATGCTCAAAG TCGGTATCGA
1251  ACCGGGCGGC AAAACCGCAT TGCGCCTGTT CTCCTAA
```

This encodes a protein having amino acid sequence <SEQ ID 528>:

```
  1  MIYIVLFLAA VLAVVAYNMY QENQYRKKVR DQFGHSDKDA LLNSKTSHVR
 51  DGKPSGGPVM MPKPQPAVKK TAKSQDPAMR NLQEQDAVYI AKQKQAKASP
101  FKTEIETALE ESGIIGNSAH TVPEPQTGHS APKPADAPAK PVPVPQTPAK
151  PLITLKELSK VELPWFDVRF DFISYIALTE AKELHALPRL SNRCRYQIVG
201  CTMDDHFQIA EPIPGIRYQA FIVGIQAVSR NGLASQEELS AFNRQVDAFA
251  HSMGGQTLHT DLAAFIEVAS ALDAFCARVD QTIAIHLVSP TSISGVELRS
301  AVTGVGFVLE DDGAFHYTDT SGSTMFSICS LNNEPFTNAL LDNQSYKGFS
351  MLLDIPHSPA GEKTFDDLFM DLAVRLSGQL NLNLVNDKME EVSTQWLKDV
401  RTYVLARQSE MLKVGIEPGG KTALRLFS*
```

ORF119a and ORF119-1 show 98.6% identity in 428 aa overlap:

```
                    10        20        30        40        50        60
orf119a.pep  MIYIVLFLAAVLAVVAYNMYQENQYRKKVRDQFGHSDKDALLNSKTSHVRDGKPSGGPVM
             ||||||||:|||||||||||||||||||||||||||||||||||||||||||||||| ||
orf119-1     MIYIVLFLAVVLAVVAYNMYQENQYRKKVRDQFGHSDKDALLNSKTSHVRDGKPSGGSVM
                    10        20        30        40        50        60

                    70        80        90       100       110       120
orf119a.pep  MPKPQPAVKKTAKSQDPAMRNLQEQDAVYIAKQKQAKASPFKTEIETALEESGIIGNSAH
             ||||||||||||| ||||||||||||||||||||||||||||||||||||||||||||||
orf119-1     MPKPQPAVKKTAKPQDPAMRNLQEQDAVYIAKQKQAKASPFKTEIETALEESGIIGNSAH
                    70        80        90       100       110       120

                   130       140       150       160       170       180
orf119a.pep  TVPEPQTGHSAPKPADAPAKPVPVPQTPAKPLITLKELSKVELPWFDVRFDFISYIALTE
             || ||||||||||||||||||:||||||||||||||||||||||||||||||||||||||
orf119-1     TVSEPQTGHSAPKPADAPAKPAPVPQTPAKPLITLKELSKVELPWFDVRFDFISYIALTE
                   130       140       150       160       170       180

                   190       200       210       220       230       240
orf119a.pep  AKELHALPRLSNRCRYQIVGCTMDDHFQIAEPIPGIRYQAFIVGIQAVSRNGLASQEELS
             ||||||||||||||||||||||||||||||||||||||||||||||||||||||||||||
orf119-1     AKELHALPRLSNRCRYQIVGCTMDDHFQIAEPIPGIRYQAFIVGIQAVSRNGLASQEELS
                   190       200       210       220       230       240

                   250       260       270       280       290       300
orf119a.pep  AFNRQVDAFAHSMGGQTLHTDLAAFIEVASALDAFCARVDQTIAIHLVSPTSISGVELRS
             ||||||||||:|||||||||||||||||||||||||||||||||||||||||||||||||
orf119-1     AFNRQVDAFAQSMGGQTLHTDLAAFIEVASALDAFCARVDQTIAIHLVSPTSISGVELRS
                   250       260       270       280       290       300

                   310       320       330       340       350       360
orf119a.pep  AVTGVGFVLEDDGAFHYTDTSGSTMFSICSLNNEPFTNALLDNQSYKGFSMLLDIPHSPA
             ||||||||||||||||||||||||||||||||||||||||||||||||||||||||||||
orf119-1     AVTGVGFVLEDDGAFHYTDTSGSTMFSICSLNNEPFTNALLDNQSYKGFSMLLDIPHSPA
                   310       320       330       340       350       360

                   370       380       390       400       410       420
orf119a.pep  GEKTFDDLFMDLAVRLSGQLNLNLVNDKMEEVSTQWLKDVRTYVLARQSEMLKVGIEPGG
             ||||||||||||||||||||||||||||||||||||||||||||||||||||||||||||
orf119-1     GEKTFDDLFMDLAVRLSGQLNLNLVNDKMEEVSTQWLKDVRTYVLARQSEMLKVGIEPGG
                   370       380       390       400       410       420

                  429
orf119a.pep  KTALRLFSX
             |||||||||
orf119-1     KTALRLFSX
```

Homology with a predicted ORF from *N.gonorrhoeae*

ORF119 shows 93.1 % identity over a 175aa overlap with a predicted ORF (ORF 119ng) from *N.gonorrhoeae:*

```
orf119.pep  MIYIVLFLAVVLAVVAYNMYQENQYRKKVRDQFGHSDKDALLNSXTSHVRDGKPSGGSVM   60
            ||||||||:|||||||||||||||||||||||||||||||||||| |||||||||||| ||
orf119ng    MIYIVLFLAAVLAVVAYNMYQENQYRKKVRDQFGHSDKDALLNSKTSHVRDGKPSGGPVM   60

orf119.pep  MPKPQPAVKKTAKPQDPXMRNLQEQDAVYIAKQKQAKASPFKTEIETALEESGIIGNSAH  120
            |||||||||| |||||  ||||||||||||||||||||||||||||||||| ||||||||
orf119ng    MPKPQPAVKKPAKPQDSAMRNLQEQDAVYIAKQKQAKASPFKTEIETALEEIGIIGNSAH  120

orf119.pep  TVSEPQTGHSATKPADASAKPAPVPQTPAKPLITLKELSKVELSWFDVRIDFISY       175
            ||||||||||| ||||| |||:||||||||||||||||||||| |||||:|||||
orf119ng    TVSEPQTGHSAPKPADAPAKPVPVPQTPAKPLITLKELSKVELPWFDVRFDFISYIALTE  180
```

The complete length ORF119ng nucleotide sequence <SEQ ID 529> is:

```
   1  ATGATTTACA TCGTACTGTT CCTCGCCGCC GTCCTCGCCG TTGTCGCCTA
  51  CAATATGTAT CAGGAAAACC AATACCGCAA AAAAGTGCGC GACCAGTTCG
 101  GACACTCCGA CAAAGATGCC CTGCTCAACA GCAAAACCAG CCATGTCCGC
 151  GACGGCAAAC CGTCCGGCGG GCCAGTCATG ATGCCGAAAC CCCAACCGGC
 201  GGTCAAAAAA CCGGCCAAAC CCCAAGACTC CGCCATGCGC AACCTGCAAG
 251  AACAGGATGC CGTCTACATC GCCAAGCAGA AACAGGCAAA AGCCTCCCCG
 301  TTCAAAACCG AAATCGAAAC CGCCTTGGAA GAAATCGGCA TTATCGGCAA
 351  CTCCGCCCAC ACCGTTTCCG AACCCCAAAC CGGACATTCC GCACCGAAAC
 401  CTGCCGACGC GCCGGCAAAA CCCGTTCCCG TTCCGCAAAC GCCGGCAAAA
 451  CCGCTGATTA CGCTCAAAGA GCTGTCGAAG GTCGAGCTGC CCTGGTTTGA
 501  CGTGCGCTtc gACTTCATCT CCTATATCGC GCTGACCGAA GCCAAAGAAC
 551  TGCACGCACT GCCGCGCCTT tccAACCGCT GCCGCTACCA GATTGTCGGC
 601  TGCACCATGG ACGACCATTT CCAGATTGCC GAACCCATCC CGGGCATCCG
 651  CTATCAGGCA TTTATCGTGG GTATCCAGGC AGTCAGCCGC AACGGACTTG
 701  CCTCGCAGGA AGAACTCTCC GCATTCAACC GCCAGGCGGA CGCATTCGCA
 751  CAAAGCATGG GCGGTCAGAC GCTGCACACC GACCTTGCCG CCTTTATCGA
 801  AGTGGCTTCC GCACTGGACG CATTCTGCGC GCGCGTCGAC CAGACCATCG
 851  CCATCCATTT GGTTTCGCCG ACCAGCATCA GCGGCGTAGA ACTGCGTTCC
 901  GCCGTAACGG GCGTGGGTTT CGTTTTGGAA GACGACGGCG CGTTCCACTA
 951  TACCGACACG TCGGGCTCGA CCATGTTCTC CATCTGCTCG CTCAACAACG
1001  AGCCGTTTAC CAATGCCCTT TTGGACAACC AGTCCTACAA AGGCTTCAGT
1051  ATGCTGCTCG ACATCCCGCA CTCTCCGGCA GGCGAAAAAA CCTTCGACGA
1101  TTTGTTTATG GATTTGGCGG TACGCCTGTC CGGTCAGTTG AACCTGAATC
1151  TGGTCAACGA CAAAATGGAA GAAGTTTCGA CCCAATGGCT CAAAGACGTA
1201  CGCACTTATG TATTGGCGCG TCAGTCCGAG ATGCTCAAAG TCGGTATCGA
1251  ACCGGGCGGC AAAACCGCCC TGCGCCTGTT TTCATAA
```

This encodes a protein having amino acid sequence <SEQ ID 530>:

```
  1  MIYIVLFLAA VLAVVAYNMY QENQYRKKVR DQFGHSDKDA LLNSKTSHVR
 51  DGKPSGGPVM MPKPQPAVKK PAKPQDSAMR NLQEQDAVYI AKQKQAKASP
101  FKTEIETALE EIGIIGNSAH TVSEPQTGHS APKPADAPAK PVPVPQTPAK
151  PLITLKELSK VELPWFDVRF DFISYIALTE AKELHALPRL SNRCRYQIVG
201  CTMDDHFQIA EPIPGIRYQA FIVGIQAVSR NGLASQEELS AFNRQADAFA
251  QSMGGQTLHT DLAAFIEVAS ALDAFCARVD QTIAIHLVSP TSISGVELRS
301  AVTGVGFVLE DDGAFHYTDT SGSTMFSICS LNNEPFTNAL LDNQSYKGFS
351  MLLDIPHSPA GEKTFDDLFM DLAVRLSGQL NLNLVNDKME EVSTQWLKDV
401  RTYVLARQSE MLKVGIEPGG KTALRLFS*
```

ORF119ng and ORF119-1 show 98.4% identity over 428 aa overlap:

```
                     10        20        30        40        50        60
orf119ng     MIYIVLFLAAVLAVVAYNMYQENQYRKKVRDQFGHSDKDALLNSKTSHVRDGKPSGGPVM
             |||||||||:||||||||||||||||||||||||||||||||||||||||||||||| ||
orf119-1     MIYIVLFLAVVLAVVAYNMYQENQYRKKVRDQFGHSDKDALLNSKTSHVRDGKPSGGSVM
                     10        20        30        40        50        60

                     70        80        90       100       110       120
orf119ng     MPKPQPAVKKPAKPQDSAMRNLQEQDAVYIAKQKQAKASPFKTEIETALEEIGIIGNSAH
             |||||||||| ||||| |||||||||||||||||||||||||||||||||| ||||||||
orf119-1     MPKPQPAVKKTAKPQDPAMRNLQEQDAVYIAKQKQAKASPFKTEIETALEESGIIGNSAH
```

```
                    70        80        90       100       110       120

                   130       140       150       160       170       180
orf119ng     TVSEPQTGHSAPKPADAPAKPVPVPQTPAKPLITLKELSKVELPWFDVRFDFISYIALTE
             ||||||||||||||||||||:|||||||||||||||||||||||||||||||||||||||
orf119-1     TVSEPQTGHSAPKPADAPAKPAPVPQTPAKPLITLKELSKVELPWFDVRFDFISYIALTE
                   130       140       150       160       170       180

                   190       200       210       220       230       240
orf119ng     AKELHALPRLSNRCRYQIVGCTMDDHFQIAEPIPGIRYQAFIVGIQAVSRNGLASQEELS
             ||||||||||||||||||||||||||||||||||||||||||||||||||||||||||||
orf119-1     AKELHALPRLSNRCRYQIVGCTMDDHFQIAEPIPGIRYQAFIVGIQAVSRNGLASQEELS
                   190       200       210       220       230       240

                   250       260       270       280       290       300
orf119ng     AFNRQADAFAQSMGGQTLHTDLAAFIEVASALDAFCARVDQTIAIHLVSPTSISGVELRS
             |||||:||||||||||||||||||||||||||||||||||||||||||||||||||||||
orf119-1     AFNRQVDAFAQSMGGQTLHTDLAAFIEVASALDAFCARVDQTIAIHLVSPTSISGVELRS
                   250       260       270       280       290       300

                   310       320       330       340       350       360
orf119ng     AVTGVGFVLEDDGAFHYTDTSGSTMFSICSLNNEPFTNALLDNQSYKGFSMLLDIPHSPA
             ||||||||||||||||||||||||||||||||||||||||||||||||||||||||||||
orf119-1     AVTGVGFVLEDDGAFHYTDTSGSTMFSICSLNNEPFTNALLDNQSYKGFSMLLDIPHSPA
                   310       320       330       340       350       360

                   370       380       390       400       410       420
orf119ng     GEKTFDDLFMDLAVRLSGQLNLNLVNDKMEEVSTQWLKDVRTYVLARQSEMLKVGIEPGG
             ||||||||||||||||||||||||||||||||||||||||||||||||||||||||||||
orf119-1     GEKTFDDLFMDLAVRLSGQLNLNLVNDKMEEVSTQWLKDVRTYVLARQSEMLKVGIEPGG
                   370       380       390       400       410       420

                  429
orf119ng     KTALRLFSX
             |||||||||
orf119-1     KTALRLFSX
```

Based on this analysis, including the presence of a putative leader sequence in the gonococcal protein, it is predicted that the proteins from *N.meningitidis* and *N.gonorrhoeae,* and their epitopes, could be useful antigens for vaccines or diagnostics, or for raising antibodies.

**Example 64**

The following partial DNA sequence was identified in *N.meningitidis* <SEQ ID 531>

```
  1  ..GCGCGGCACG GCACGGAAGA TTTCTTCATG AACAACAGCG ACAC.ATCAG
 51    GCAGATAGTC GAAAGCACCA CCGGTACGAT GAAGCTGCTG ATTTCCTCCA
101    TCGCCCTGAT TTCATTGGTA GTCGGCGGCA TCGGCGTGAT GAACATCATG
151    CTGGTGTCCG TTACCGAGCG CACCAAAGAA ATCGGCATAC GGATGGCAAT
201    CGGCGCGCGG CGCGGCAATA TTTyGCAGCA GTTTTTGATT GAGGCGGTGT
251    TAATCTGCGT CATCGGCGGT TTGGTCGGCG TGGGTTTGTC CGCCGCCGTC
301    AGCCTCGTGT TCAATCATTT TGTAACCGAC TTCCCGATGG ACATTTCCGC
351    CATGTCCGTC ATCGGCGCGG TCGCCTGTTC GACCGGAATC GGCATCGCGT
401    TCGGCTTTAT GCCTGCCAAT AAAGCAGCCA AACTCAATCC GATAGACGCA
451    TTGGCACAGG ATTGA
```

This corresponds to the amino acid sequence <SEQ ID 532; ORF134>:

```
  1  ..ARHGTEDFFM NNSDXIRQIV ESTTGTMKLL ISSIALISLV VGGIGVMNIM
 51    LVSVTERTKE IGIRMAIGAR RGNIXQQFLI EAVLICVIGG LVGVGLSAAV
101    SLVFNHFVTD FPMDISAMSV IGAVACSTGI GIAFGFMPAN KAAKLNPIDA
151    LAQD*
```

Further work revealed the complete nucleotide sequence <SEQ ID 533>:

```
   1  ATGTCGGTGC AAGCAGTATT GGCGCACAAA ATGCGTTCGC TTCTGACGAT
  51  GCTCGGCATC ATCATCGGTA TCGCGTCGGT GGTTTCCGTC GTCGCATTGG
 101  GCAATGGTTC GCAGAAAAAA ATCCTTGAAG ACATCAGTTC GATAGGGACG
 151  AACACCATCA GCATCTTCCC GGGGCGCGGC TTCGGCGACA GGCGCAGCGG
 201  CAGGATTAAA ACCCTGACCA TAGACGACGC AAAAATCATC GCCAAACAAA
 251  GCTACGTTGC TTCCGCCACG CCCATGACTT CGAGCGGCGG CACGCTGACT
 301  TACCGCAACA CCGACCTGAC CGCCTCGCTT TACGGCGTGG GCGAACAATA
 351  TTTCGACGTG CGCGGACTGA AGCTGGAAAC GGGGCGGCTG TTTGACGAAA
 401  ACGATGTGAA AGAAGACGCG CAGGTCGTCG TCATCGACCA AAATGTCAAA
 451  GACAAACTCT TTGCGGACTC GGATCCGTTG GGTAAAACCA TTTTGTTCAG
 501  GAAACGCCCC TTGACCGTCA TCGGCGTGAT GAAAAAAGAC GAAAACGCTT
 551  TCGGCAATTC CGACGTGCTG ATGCTTTGGT CGCCCTATAC GACGGTGATG
 601  CACCAAATCA CAGGCGAGAG CCACACCAAC TCCATCACCG TCAAAATCAA
 651  AGACAATGCC AATACCCAGG TTGCCGAAAA AGGGCTGACC GATCTGCTCA
 701  AAGCGCGGCA CGGCACGGAA GATTTCTTCA TGAACAACAG CGACAGCATC
 751  AGGCAGATAG TCGAAAGCAC CACCGGTACG ATGAAGCTGC TGATTTCCTC
 801  CATCGCCCTG ATTTCATTGG TAGTCGGCGG CATCGGCGTG ATGAACATCA
 851  TGCTGGTGTC CGTTACCGAG CGCACCAAAG AAATCGGCAT ACGGATGGCA
 901  ATCGGCGCGC GGCGCGGCAA TATTTTGCAG CAGTTTTTGA TTGAGGCGGT
 951  GTTAATCTGC GTCATCGGCG GTTTGGTCGG CGTGGGTTTG TCCGCCGCCG
1001  TCAGCCTCGT GTTCAATCAT TTTGTAACCG ACTTCCCGAT GGACATTTCC
1051  GCCATGTCCG TCATCGGCGC GGTCGCCTGT TCGACCGGAA TCGGCATCGC
1101  GTTCGGCTTT ATGCCTGCCA ATAAAGCAGC CAAACTCAAT CCGATAGACG
1151  CATTGGCACA GGATTGA
```

This corresponds to the amino acid sequence <SEQ ID 534; ORF134-1>:

```
  1  MSVQAVLAHK MRSLLTMLGI IIGIASVVSV VALGNGSQKK ILEDISSIGT
 51  NTISIFPGRG FGDRRSGRIK TLTIDDAKII AKQSYVASAT PMTSSGGTLT
101  YRNTDLTASL YGVGEQYFDV RGLKLETGRL FDENDVKEDA QVVVIDQNVK
151  DKLFADSDPL GKTILFRKRP LTVIGVMKKD ENAFGNSDVL MLWSPYTTVM
201  HQITGESHTN SITVKIKDNA NTQVAEKGLT DLLKARHGTE DFFMNNSDSI
251  RQIVESTTGT MKLLISSIAL ISLVVGGIGV MNIMLVSVTE RTKEIGIRMA
301  IGARRGNILQ QFLIEAVLIC VIGGLVGVGL SAAVSLVFNH FVTDFPMDIS
351  AMSVIGAVAC STGIGIAFGF MPANKAAKLN PIDALAQD*
```

Computer analysis of this amino acid sequence gave the following results:

Homology with the hypothetical protein o648 of *E.coli* (accession number AE000189)

ORF134 and o648 protein show 45% aa identity in 153aa overlap:

```
Orf134: 2   RHGTEDFFMNNSDXIRQIVESTTGTMKXXXXXXXXXXXVVGGIGVMNIMLVSVTERTKEI 61
            RHG +DFF  N D + + VE TT T++           VVGGIGVMNIMLVSVTERT+EI
o648:   496 RHGKKDFFTWNMDGVLKTVEKTTRTLQLFLTLVAVISLVVGGIGVMNIMLVSVTERTREI 555

Orf134: 62  GIRMAIGARRGNIXQQFLIEAXXXXXXXXXXXXXXXXXXXXFNHFVTDFPMDISAMSVI 121
            GIRMA+GAR  ++ QQFLIEA                    F+  + +  S ++++
o648:   556 GIRMAVGARASDVLQQFLIEAVLVCLVGGALGITLSLLIAFTLQLFLPGWEIGFSPLALL 615

Orf134: 122 GAVACSTGIGIAFGFMPANKAAKLNPIDALAQD 154
             A  CST  GI FG++PA  AA+L+P+DALA++
o648:   616 LAFLCSTVTGILFGWLPARNAARLDPVDALARE 648
```

Homology with a predicted ORF from *N. meningitidis* (strain A)

ORF134 shows 98.7% identity over a 154aa overlap with an ORF (ORF134a) from strain A of *N. meningitidis:*

```
                                                   10        20        30
orf134.pep                                 ARHGTEDFFMNNSDXIRQIVESTTGTMKLL
                                           ||||||||||||| ||||||||||||||||
orf134a       GESHTNSITVKIKDNANTQVAEKGLTDLLKARHGTEDFFMNNSDSIRQIVESTTGTMKLL
                  210       220       230       240       250       260

                       40        50        60        70        80        90
orf134.pep    ISSIALISLVVGGIGVMNIMLVSVTERTKEIGIRMAIGARRGNIXQQFLIEAVLICVIGG
              ||||||||||||||||||||||||||||||||||||||||||||| ||||||||||||||
orf134a       ISSIALISLVVGGIGVMNIMLVSVTERTKEIGIRMAIGARRGNILQQFLIEAVLICVIGG
                  270       280       290       300       310       320

                      100       110       120       130       140       150
orf134.pep    LVGVGLSAAVSLVFNHFVTDFPMDISAMSVIGAVACSTGIGIAFGFMPANKAAKLNPIDA
              ||||||||||||||||||||||||||||||||||||||||||||||||||||||||||||
orf134a       LVGVGLSAAVSLVFNHFVTDFPMDISAMSVIGAVACSTGIGIAFGFMPANKAAKLNPIDA
                  330       340       350       360       370       380

orf134.pep    LAQDX
              |||||
orf134a       LAQDX
```

The complete length ORF134a nucleotide sequence <SEQ ID 535> is:

```
   1  ATGTCGGTGC AAGCAGTATT GGCGCACAAA ATGCGTTCGC TTCTGACGAT
  51  GCTCGGCATC ATCATCGGTA TCGCTTCGGT TGTCTCCGTC GTCGCATTGG
 101  GCAACGGTTC GCAGAAAAAA ATCCTTGAAG ACATCAGTTC GATAGGGACG
 151  AACACCATCA GCATCTTCCC AGGGCGCGGC TTCGGCGACA GGCGCAGCGG
 201  CAGGATTAAA ACCCTGACCA TAGACGACGC AAAAATCATC GCCAAACAAA
 251  GCTACGTTGC TTCCGCCACG CCCATGACTT CGAGCGGCGG CACGCTGACT
 301  TACCGCAATA CCGACCTGAC CGCTTCTTTG TACGGTGTGG GCGAACAATA
 351  TTTCGACGTG CGCGGGCTGA AGCTGGAAAC GGGGCGGCTG TTTGACGAAA
 401  ACGATGTGAA AGAAGACGCG CAGGTCGTCG TCATCGACCA AAATGTCAAA
 451  GACAAACTCT TTGCGGACTC GGATCCGTTG GGTAAAACCA TTTTGTTCAG
 501  GAAACGCCCC TTGACCGTCA TCGGCGTGAT GAAAAAAGAC GAAAACGCTT
 551  TCGGCAATTC CGACGTGCTG ATGCTTTGGT CGCCCTATAC GACGGTGATG
 601  CACCAAATCA CAGGCGAGAG CCACACCAAC TCCATCACCG TCAAAATCAA
 651  AGACAATGCC AATACCCAGG TTGCCGAAAA AGGGCTGACC GATCTGCTCA
 701  AAGCGCGGCA CGGCACGGAA GATTTCTTCA TGAACAACAG CGACAGCATC
 751  AGGCAGATAG TCGAAAGCAC CACCGGTACG ATGAAGCTGC TGATTTCCTC
 801  CATCGCCCTG ATTTCATTGG TAGTCGGCGG CATCGGCGTG ATGAACATCA
 851  TGCTGGTGTC CGTTACCGAG CGCACCAAAG AAATCGGCAT ACGGATGGCA
 901  ATCGGCGCGC GGCGCGGCAA TATTTTGCAG CAGTTTTTGA TTGAGGCGGT
 951  GTTAATCTGC GTCATCGGCG GTTTGGTCGG CGTGGGTTTG TCCGCCGCCG
1001  TCAGCCTCGT GTTCAATCAT TTTGTAACCG ACTTCCCGAT GGACATTTCC
1051  GCCATGTCCG TCATCGGCGC GGTCGCCTGT TCGACCGGAA TCGGCATCGC
1101  GTTCGGCTTT ATGCCTGCCA ATAAAGCAGC CAAACTCAAT CCGATAGATG
1151  CATTGGCGCA GGATTGA
```

This encodes a protein having amino acid sequence <SEQ ID 536>:

```
  1  MSVQAVLAHK MRSLLTMLGI IIGIASVVSV VALGNGSQKK ILEDISSIGT
 51  NTISIFPGRG FGDRRSGRIK TLTIDDAKII AKQSYVASAT PMTSSGGTLT
101  YRNTDLTASL YGVGEQYFDV RGLKLETGRL FDENDVKEDA QVVVIDQNVK
151  DKLFADSDPL GKTILFRKRP LTVIGVMKKD ENAFGNSDVL MLWSPYTTVM
201  HQITGESHTN SITVKIKDNA NTQVAEKGLT DLLKARHGTE DFFMNNSDSI
251  RQIVESTTGT MKLLISSIAL ISLVVGGIGV MNIMLVSVTE RTKEIGIRMA
301  IGARRGNILQ QFLIEAVLIC VIGGLVGVGL SAAVSLVFNH FVTDFPMDIS
351  AMSVIGAVAC STGIGIAFGF MPANKAAKLN PIDALAQD*
```

ORF134a and ORF134-1 show 100.0% identity in 388 aa overlap:

```
orf134a.pep    MSVQAVLAHKMRSLLTMLGIIIGIASVVSVVALGNGSQKKILEDISSIGTNTISIFPGRG
               ||||||||||||||||||||||||||||||||||||||||||||||||||||||||||||
orf134-1       MSVQAVLAHKMRSLLTMLGIIIGIASVVSVVALGNGSQKKILEDISSIGTNTISIFPGRG

orf134a.pep    FGDRRSGRIKTLTIDDAKIIAKQSYVASATPMTSSGGTLTYRNTDLTASLYGVGEQYFDV
               ||||||||||||||||||||||||||||||||||||||||||||||||||||||||||||
orf134-1       FGDRRSGRIKTLTIDDAKIIAKQSYVASATPMTSSGGTLTYRNTDLTASLYGVGEQYFDV

orf134a.pep    RGLKLETGRLFDENDVKEDAQVVVIDQNVKDKLFADSDPLGKTILFRKRPLTVIGVMKKD
               ||||||||||||||||||||||||||||||||||||||||||||||||||||||||||||
orf134-1       RGLKLETGRLFDENDVKEDAQVVVIDQNVKDKLFADSDPLGKTILFRKRPLTVIGVMKKD

orf134a.pep    ENAFGNSDVLMLWSPYTTVMHQITGESHTNSITVKIKDNANTQVAEKGLTDLLKARHGTE
               ||||||||||||||||||||||||||||||||||||||||||||||||||||||||||||
orf134-1       ENAFGNSDVLMLWSPYTTVMHQITGESHTNSITVKIKDNANTQVAEKGLTDLLKARHGTE

orf134a.pep    DFFMNNSDSIRQIVESTTGTMKLLISSIALISLVVGGIGVMNIMLVSVTERTKEIGIRMA
               ||||||||||||||||||||||||||||||||||||||||||||||||||||||||||||
orf134-1       DFFMNNSDSIRQIVESTTGTMKLLISSIALISLVVGGIGVMNIMLVSVTERTKEIGIRMA

orf134a.pep    IGARRGNILQQFLIEAVLICVIGGLVGVGLSAAVSLVFNHFVTDFPMDISAMSVIGAVAC
               ||||||||||||||||||||||||||||||||||||||||||||||||||||||||||||
orf134-1       IGARRGNILQQFLIEAVLICVIGGLVGVGLSAAVSLVFNHFVTDFPMDISAMSVIGAVAC

orf134a.pep    STGIGIAFGFMPANKAAKLNPIDALAQDX
               |||||||||||||||||||||||||||||
orf134-1       STGIGIAFGFMPANKAAKLNPIDALAQDX
```

Homology with a predicted ORF from *N.gonorrhoeae*

ORF134 shows 96.8% identity over a 154aa overlap with a predicted ORF (ORF134.ng) from *N. gonorrhoeae:*

```
orf134.pep                                ARHGTEDFFMNNSDXIRQIVESTTGTMKLL   30
                                          |||||||||||||| |||:|||||||||||
orf134ng     GESHTNSITVKIKDNANTRVAEKGLAELLKARHGTEDFFMNNSDSIRQMVESTTGTMKLL  264

orf134.pep   ISSIALISLVVGGIGVMNIMLVSVTERTKEIGIRMAIGARRGNIXQQFLIEAVLICVIGG   90
             |||||||||||||||||||||||||||||||||||||||||||| |||||||||||:|||
orf134ng     ISSIALISLVVGGIGVMNIMLVSVTERTKEIGIRMAIGARRGNILQQFLIEAVLICIIGG  324

orf134.pep   LVGVGLSAAVSLVFNHFVTDFPMDISAMSVIGAVACSTGIGIAFGFMPANKAAKLNPIDA  150
             ||||||||||||||||||||||||||| ||||||||||||||||||||||||||||||||
orf134ng     LVGVGLSAAVSLVFNHFVTDFPMDISAASVIGAVACSTGIGIAFGFMPANKAAKLNPIDA  384

orf134.pep   LAQD  154
             ||||
orf134ng     LAQD  388
```

The complete length ORF134ng nucleotide sequence <SEQ ID 537> is:

```
   1  ATGTCGGTGC AAGCAGTATT GGCGCACAAA ATGCGTTCGC TTCTGACCAT
  51  GCTCGGCATC ATCATCGGTA TCGCTTCGGT TGTCTCCGTC GTCGCGCTGG
 101  GCAACGGTTC GCAGAAAAAA ATCCTCGAAG ACATCAGTTC GATGGGGACG
 151  AACACCATCA GCATCTTCCC CGGGCGCGGC TTCGGCGACA GGCGCAGCGG
 201  CAAAATCAAA ACCCTGACCA TAGACGACGC AAAAATCATC GCCAAACAAA
 251  GCTACGTTGC CTCCGCCACG CCCATGACTT CGAGCGGCGG CACGCTGACC
 301  TACCGCAATA CCGACCTGAC CGCTTCTTTG TACGGTGTGG GCGAACAATA
 351  TTTCGACGTG CGCGGGCTGA AGCTGGAAAC GGGGCGGCTG TTTGATGAGA
 401  ACGATGTGAA AGAAGACGCG CAAGTCGTCG TCATCGACCA AAATGTCAAA
 451  GACAAACTCT TTGCGGACTC GGATCCGTTG GGTAAAACCA TTTTGTTCAG
 501  GAAACGCCCC TTGACCGTCA TCGGCGTGAT GAAAAAAGAC GAAAACGCTT
 551  TCGGCAATTC CGACGTGCTG ATGCTTTGGT CGCCCTATAC GACGGTGATG
 601  CACCAAATCA CAGGCGAGAG CCACACCAAC TCCATCACCG TCAAAATCAA
 651  AGACAATGCC AATACCCGGG TTGCCGAAAA AGGGCTGGCC GAGCTGCTCA
 701  AAGCACGGCA CGGCACGGAA GACTTCTTTA TGAACAACAG CGACAGCATC
 751  AGGCAGATGG TCGAAAGCAC CACCGGTACG ATGAAGCTGC TGATTTCCTC
 801  CATCGCCCTG ATTTCATTGG TAGTCGGCGG CATCGGTGTG ATGAACATTA
 851  TGCTGGTGTC CGTTACCGAG CGCACCAAAG AAATCGGCAT ACGGATGGCA
 901  ATCGGCGCGC GGCGCGGCAA TATTTTGCAG CAGTTTTTGA TTGAGGCGGT
 951  GTTAATCTGC ATCATCGGAG GCTTGGTCGG CGTAGGTTTG TCCGCCGCCG
1001  TCAGCCTCGT GTTCAATCAT TTTGTAACCG ATTTCCCGAT GGACATTTCG
1051  GCGGCATCCG TTATCGGGGC GGTCGCCTGT TCGACCGGAA TCGGCATCGC
1101  GTTCGGCTTT ATGCCTGCCA ATAAGGCAGC CAAACTCAAT CCGATAGATG
1151  CATTGGCGCA GGATTGA
```

This encodes a protein having amino acid sequence <SEQ ID 538>:

```
  1  MSVQAVLAHK MRSLLTMLGI IIGIASVVSV VALGNGSQKK ILEDISSMGT
 51  NTISIFPGRG FGDRRSGKIK TLTIDDAKII AKQSYVASAT PMTSSGGTLT
101  YRNTDLTASL YGVGEQYFDV RGLKLETGRL FDENDVKEDA QVVVIDQNVK
151  DKLFADSDPL GKTILFRKRP LTVIGVMKKD ENAFGNSDVL MLWSPYTTVM
201  HQITGESHTN SITVKIKDNA NTRVAEKGLA ELLKARHGTE DFFMNNSDSI
251  RQMVESTTGT MKLLISSIAL ISLVVGGIGV MNIMLVSVTE RTKEIGIRMA
301  IGARRGNILQ QFLIEAVLIC IIGGLVGVGL SAAVSLVFNH FVTDFPMDIS
351  AASVIGAVAC STGIGIAFGF MPANKAAKLN PIDALAQD*
```

ORF134ng and ORF134-1 show 97.9% identity in 388 aa overlap:

```
orf134ng        MSVQAVLAHKMRSLLTMLGIIIGIASVVSVVALGNGSQKKILEDISSMGTNTISIFPGRG
```

```
              |||||||||||||||||||||||||||||||||||||||||||||||:||||||||||||
orf134-1      MSVQAVLAHKMRSLLTMLGIIIGIASVVSVVALGNGSQKKILEDISSIGTNTISIFPGRG

orf134ng      FGDRRSGKIKTLTIDDAKIIAKQSYVASATPMTSSGGTLTYRNTDLTASLYGVGEQYFDV
              |||||||:||||||||||||||||||||||||||||||||||||||||||||||||||||
orf134-1      FGDRRSGRIKTLTIDDAKIIAKQSYVASATPMTSSGGTLTYRNTDLTASLYGVGEQYFDV

orf134ng      RGLKLETGRLFDENDVKEDAQVVVIDQNVKDKLFADSDPLGKTILFRKRPLTVIGVMKKD
              ||||||||||||||||||||||||||||||||||||||||||||||||||||||||||||
orf134-1      RGLKLETGRLFDENDVKEDAQVVVIDQNVKDKLFADSDPLGKTILFRKRPLTVIGVMKKD

orf134ng      ENAFGNSDVLMLWSPYTTVMHQITGESHTNSITVKIKDNANTRVAEKGLAELLKARHGTE
              ||||||||||||||||||||||||||||||||||||||||||:||||||::|||||||||
orf134-1      ENAFGNSDVLMLWSPYTTVMHQITGESHTNSITVKIKDNANTQVAEKGLTDLLKARHGTE

orf134ng      DFFMNNSDSIRQMVESTTGTMKLLISSIALISLVVGGIGVMNIMLVSVTERTKEIGIRMA
              ||||||||||||:|||||||||||||||||||||||||||||||||||||||||||||||
orf134-1      DFFMNNSDSIRQIVESTTGTMKLLISSIALISLVVGGIGVMNIMLVSVTERTKEIGIRMA

orf134ng      IGARRGNILQQFLIEAVLICIIGGLVGVGLSAAVSLVFNHFVTDFPMDISAASVIGAVAC
              ||||||||||||||||||||:|||||||||||||||||||||||||||||||| ||||||||
orf134-1      IGARRGNILQQFLIEAVLICVIGGLVGVGLSAAVSLVFNHFVTDFPMDISAMSVIGAVAC

orf134ng      STGIGIAFGFMPANKAAKLNPIDALAQDX
              ||||||||||||||||||||||||||||
orf134-1      STGIGIAFGFMPANKAAKLNPIDALAQDX
```

ORF134ng also shows homology to an *E.coli* ABC transporter:

```
sp|P75831|YBJZ_ECOLI HYPOTHETICAL ABC TRANSPORTER ATP-BINDING PROTEIN YBJZ >gi5
(AE000189) o648; similar to YBBA_HAEIN SW: P45247 [Escherichia coli] Length =
648
 Score =  297 bits (753), Expect = 6e-80
 Identities = 162/389 (41%), Positives = 230/389 (58%), Gaps = 1/389 (0%)

Query: 1   MSVQAVLAHKMRSLLTMLXXXXXXXXXXXXXXLGNGSQKKILEDISSMGTNTISIFPGRG 60
            M+ +A+ A+KMR+LLTML              +G+ +++ +L DI S+GTNTI ++PG+
Sbjct: 260 MAWRALAANKMRTLLTMLGIIIGIASVVSIVVVGDAAKQMVLADIRSIGTNTIDVYPGKD 319

Query: 61  FGDRRSGKIKTLTIDDAKIIAKQSYVASATPMTSSGGTLTYRNTDLTASLYGVGEQYFDV 120
            FGD      + L  DD   I KQ +VASATP  S    L Y N D+ AS  GV   YF+V
Sbjct: 320 FGDDDPQYQQALKYDDLIAIQKQPWVASATPAVSQNLRLRYNNVDVAASANGVSGDYFNV 379

Query: 121 RGLKLETGRLFDENDVKEDAQVVVIDQNVKDKLFAD-SDPLGKTILFRKRPLTVIGVMKK 179
             G+    G  F++ +    AQVVV+D N + +LF   +D +G+ IL    P  VIGV ++
Sbjct: 380 YGMTFSEGNTFNQEQLNGRAQVVVLDSNTRRQLFPHKADVVGEVILVGNMPARVIGVAEE 439

Query: 180 DENAFGNSDVLMLWSPYTTVMHQITGESHTNSITVKIKDNANTRVAEKGLAELLKARHGT 239
             ++ FG+S VL +W PY+T+  ++ G+S  NSITV++K+  ++  AE+ L  LL  RHG
Sbjct: 440 KQSMFGSSKVLRVWLPYSTMSGRVMGQSWLNSITVRVKEGFDSAEAEQQLTRLLSLRHGK 499

Query: 240 EDFFMNNSDSIRQMVESTTGTMKXXXXXXXXXXXVVGGIGVMNIMLVSVTERTKEIGIRM 299
            +DFF  N D + + VE TT T++           VVGGIGVMNIMLVSVTERT+EIGIRM
Sbjct: 500 KDFFTWNMDGVLKTVEKTTRTLQLFLTLVAVISLVVGGIGVMNIMLVSVTERTREIGIRM 559

Query: 300 AIGARRGNILQQFLIEXXXXXXXXXXXXXXXXXXXXXXXFNHFVTDFPMDISAASVIGAVA 359
            A+GAR  ++LQQFLIE                        F+  + +  S  +++ A
Sbjct: 560 AVGARASDVLQQFLIEAVLVCLVGGALGITLSLLIAFTLQLFLPGWEIGFSPLALLLAFL 619

Query: 360 CSTGIGIAFGFMPANKAAKLNPIDALAQD 388
            CST  GI FG++PA  AA+L+P+DALA++
Sbjct: 620 CSTVTGILFGWLPARNAARLDPVDALARE 648
```

Based on this analysis, including the presence of the leader peptide and transmembrane regions in the gonococcal protein, it is prediceted that these proteins from *N.meningitidis* and *N.gonorrhoeae,* and their epitopes, could be useful antigens for vaccines or diagnostics, or for raising antibodies.

**Example 65**

The following partial DNA sequence was identified in *N.meningitidis* <SEQ ID 539>:

```
  1 ..GGGACGGGAG CGATGCTGCT GCTGTTTTAC GCGGTAACGA T.CTGCCTTT
 51   GGCCACTGGC GTTACCCTGA GTTACACCTC GTCGATTTTT TTGGCGGTAT
101   TTTCCTTCCT GATTTTGAAA GAACGGATTT CCGTTTACAC GCAGGCGGTG
151   CTGCTCCTTG GTTTTGCCGG CGTGGTATTG CTGCTTAATC CCTCGTTCCG
201   CAGCGGTCAG GAAACGGCGG CACTCGCCGG GCTGGCGGGC GGCGCGATGT
251   CCGGCTGGGC GTATTTGAAA GTGCGCGAAC TGTCTTTGGC GGGCGAACCC
301   GGCTGGCGCG TCGTGTTTTA CCTTTCCGTG ACAGGTGTGG CGATGTCGTC
351   GGTTTGGGCG ACGCTGACCG GCTGGCACAC CCTGTCCTTT CCATCGGCAG
401   TTTATCTGTC GTGCATCGGC GTGTCCGCGC TGATTGCCCA ACTGTCGATG
451   ACGCGCGCCT ACAAAGTCGG CGACAAATTC ACGGTTGCCT CGCTTTCCTA
501   TATGACCGTC GTTTTTTCCG CTCTGTCTGC CGCATTTTTT CTGGGCGAAG
551   AGCTTTTCTG GCAGGAAATA CTCGGTATGT GCATCATCAT CCTCAGCGGT
601   ATTTTGA
```

This corresponds to the amino acid sequence <SEQ ID 540; ORF135>:

```
  1 ..GTGAMLLLFY AVTILPLATG VTLSYTSSIF LAVFSFLILK ERISVYTQAV
 51   LLLGFAGVVL LLNPSFRSGQ ETAALAGLAG GAMSGWAYLK VRELSLAGEP
101   GWRVVFYLSV TGVAMSSVWA TLTGWHTLSF PSAVYLSCIG VSALIAQLSM
151   TRAYKVGDKF TVASLSYMTV VFSALSAAFF LGEELFWQEI LGMCIIISAV
201   F*
```

Further work revealed the complete nucleotide sequence <SEQ ID 541>:

```
  1 ATGGATACCG CAAAAAAAGA CATTTTAGGA TCGGGCTGGA TGCTGGTGGC
 51 GGCGGCCTGC TTTACCATTA TGAACGTATT GATTAAAGAG GCATCGGCAA
101 AATTTGCCCT CGGCAGCGGC GAATTGGTCT TTTGGCGCAT GCTGTTTTCA
151 ACCGTTGCGC TCGGGGCTGC CGCCGTATTG CGTCGGGACA mCTTCCGCAC
201 GCCCCATTGG AAAAACCACT TAAACCGCAG TATGGTCGGG ACGGGGGCGA
251 TGCTGCTGCT GTTTTACGCG GTAACGCATC TGCCTTTGGC CACTGGCGTT
301 ACCCTGAGTT ACACCTCGTC GATTTTTTTG GCGGTATTTT CCTTCCTGAT
351 TTTGAAAGAA CGGATTTCCG TTTACACGCA GGCGGTGCTG CTCCTTGGTT
401 TTGCCGGCGT GGTATTGCTG CTTAATCCCT CGTTCCGCAG CGGTCAGGAA
451 ACGGCGGCAC TCGCCGGGCT GGCGGGCGGC GCGATGTCCG GCTGGGCGTA
501 TTTGAAAGTG CGCGAACTGT CTTTGGCGGG CGAACCCGGC TGGCGCGTCG
551 TGTTTTACCT TTCCGTGACA GGTGTGGCGA TGTCGTCGGT TTGGGCGACG
601 CTGACCGGCT GGCACACCCT GTCCTTTCCA TCGGCAGTTT ATCTGTCGTG
651 CATCGGCGTG TCCGCGCTGA TTGCCCAACT GTCGATGACG CGCGCCTACA
701 AAGTCGGCGA CAAATTCACG GTTGCCTCGC TTTCCTATAT GACCGTCGTT
751 TTTTCCGCTC TGTCTGCCGC ATTTTTTCTG GGCGAAGAGC TTTTCTGGCA
801 GGAAATACTC GGTATGTGCA TCATCATCCT CAGCGGTATT TTGAGCAGCA
851 TCCGCCCCAC TGCCTTCAAA CAGCGGCTGC AATCCCTGTT CCGCCAAAGA
901 TAA
```

This corresponds to the amino acid sequence <SEQ ID 542; ORF135-1>:

```
  1 MDTAKKDILG SGWMLVAAAC FTIMNVLIKE ASAKFALGSG ELVFWRMLFS
 51 TVALGAAAVL RRDXFRTPHW KNHLNRSMVG TGAMLLLFYA VTHLPLATGV
101 TLSYTSSIFL AVFSFLILKE RISVYTQAVL LLGFAGVVLL LNPSFRSGQE
151 TAALAGLAGG AMSGWAYLKV RELSLAGEPG WRVVFYLSVT GVAMSSVWAT
201 LTGWHTLSFP SAVYLSCIGV SALIAQLSMT RAYKVGDKFT VASLSYMTVV
251 FSALSAAFFL GEELFWQEIL GMCIIILSGI LSSIRPTAFK QRLQSLFRQR
301 *
```

Computer analysis of this amino acid sequence gave the following results:

Homology with a predicted ORF from *N.meningitidis* (strain A)

ORF135 shows 99.0% identity over a 197aa overlap with an ORF (ORF135a) from strain A of *N. meningitidis:*

```
                                                10        20        30
orf135.pep                              GTGAMLLLFYAVTILPLATGVTLSYTSSIF
                                        ||||||||||||| ||||||||||||||||
orf135a     STVALGAAAVLRRDTFRTPHWKNHLNRSMVGTGAMLLLFYAVTHLPLATGVTLSYTSSIF
           50        60        70        80        90       100

                    40        50        60        70        80        90
orf135.pep  LAVFSFLILKERISVYTQAVLLLGFAGVVLLLNPSFRSGQETAALAGLAGGAMSGWAYLK
            ||||||||||||||||||||||||||||||||||||||||||||||||||||||||||||
orf135a     LAVFSFLILKERISVYTQAVLLLGFAGVVLLLNPSFRSGQETAALAGLAGGAMSGWAYLK
          110       120       130       140       150       160

                   100       110       120       130       140       150
orf135.pep  VRELSLAGEPGWRVVFYLSVTGVAMSSVWATLTGWHTLSFPSAVYLSCIGVSALIAQLSM
            ||||||||||||||||||||||||||||||||||||||||||||||||||||||||||||
orf135a     VRELSLAGEPGWRVVFYLSVTGVAMSSVWATLTGWHTLSFPSAVYLSCIGVSALIAQLSM
          170       180       190       200       210       220

                   160       170       180       190       200
orf135.pep  TRAYKVGDKFTVASLSYMTVVFSALSAAFFLGEELFWQEILGMCIIISAVFX
            ||||||||||||||||||||||||||||||:|||||||||||||||
orf135a     TRAYKVGDKFTVASLSYMTVVFSALSAAFFLAEELFWQEILGMCIIILSGILSSIRPTAF
          230       240       250       260       270       280

orf135a     KQRLQSLFRQRX
          290       300
```

The complete length ORF135a nucleotide sequence <SEQ ID 543> is:

```
  1  ATGGATACCG CAAAAAAAGA CATTTTAGGA TCGGGCTGGA TGCTGGTGGC
 51  GGCGGCCTGC TTTACCATTA TGAACGTATT GATTAAAGAG GCATCGGCAA
101  AATTTGCCCT CGGCAGCGGC GAATTGGTCT TTGGCGCAT GCTGTTTTCA
151  ACCGTTGCGC TCGGGGCTGC CGCCGTATTG CGTCGGGACA CCTTCCGCAC
201  GCCCCATTGG AAAAACCACT TAAACCGCAG TATGGTCGGG ACGGGGGCGA
251  TGCTGCTGCT GTTTTACGCG GTAACGCATC TGCCTTTGGC CACCGGCGTT
301  ACCCTGAGTT ACACCTCGTC GATTTTTTTG GCGGTATTTT CCTTCCTGAT
351  TTTGAAAGAA CGGATTTCCG TTTACACGCA GGCGGTGCTG CTCCTTGGTT
401  TTGCCGGCGT GGTATTGCTG CTTAATCCCT CGTTCCGCAG CGGTCAGGAA
451  ACGGCGGCAC TCGCCGGGCT GGCGGGCGGC GCGATGTCCG GCTGGGCGTA
501  TTTGAAAGTG CGCGAACTGT CTTTGGCGGG CGAACCCGGC TGGCGCGTCG
551  TGTTTTACCT TTCCGTGACA GGTGTGGCGA TGTCATCGGT TTGGGCGACG
601  CTGACCGGCT GGCACACCCT GTCCTTTCCA TCGGCAGTTT ATCTGTCGTG
651  CATCGGCGTG TCCGCGCTGA TTGCCCAACT GTCGATGACG CGCGCCTACA
701  AAGTCGGCGA CAAATTCACG GTTGCCTCGC TTTCCTATAT GACCGTCGTT
751  TTTTCCGCTC TGTCTGCCGC ATTTTTTCTG GCCGAAGAGC TTTTCTGGCA
801  GGAAATACTC GGTATGTGCA TCATCATCCT CAGCGGTATT TTGAGCAGCA
851  TCCGCCCCAC TGCCTTCAAA CAGCGGCTGC AATCCCTGTT CCGCCAAAGA
901  TAA
```

This encodes a protein having amino acid sequence <SEQ ID 544>:

```
  1  MDTAKKDILG SGWMLVAAAC FTIMNVLIKE ASAKFALGSG ELVFWRMLFS
 51  TVALGAAAVL RRDTFRTPHW KNHLNRSMVG TGAMLLLFYA VTHLPLATGV
101  TLSYTSSIFL AVFSFLILKE RISVYTQAVL LLGFAGVVLL LNPSFRSGQE
151  TAALAGLAGG AMSGWAYLKV RELSLAGEPG WRVVFYLSVT GVAMSSVWAT
201  LTGWHTLSFP SAVYLSCIGV SALIAQLSMT RAYKVGDKFT VASLSYMTVV
251  FSALSAAFFL AEELFWQEIL GMCIIILSGI LSSIRPTAFK QRLQSLFRQR
301  *
```

ORF135a and ORF135-1 show 99.3% identity in 300 aa overlap:

```
orf135a.pep   MDTAKKDILGSGWMLVAAACFTIMNVLIKEASAKFALGSGELVFWRMLFSTVALGAAAVL
              ||||||||||||||||||||||||||||||||||||||||||||||||||||||||||||
orf135-1      MDTAKKDILGSGWMLVAAACFTIMNVLIKEASAKFALGSGELVFWRMLFSTVALGAAAVL

orf135a.pep   RRDTFRTPHWKNHLNRSMVGTGAMLLLFYAVTHLPLATGVTLSYTSSIFLAVFSFLILKE
              |||:||||||||||||||||||||||||||||||||||||||||||||||||||||||||
orf135-1      RRDXFRTPHWKNHLNRSMVGTGAMLLLFYAVTHLPLATGVTLSYTSSIFLAVFSFLILKE

orf135a.pep   RISVYTQAVLLLGFAGVVLLLNPSFRSGQETAALAGLAGGAMSGWAYLKVRELSLAGEPG
              ||||||||||||||||||||||||||||||||||||||||||||||||||||||||||||
orf135-1      RISVYTQAVLLLGFAGVVLLLNPSFRSGQETAALAGLAGGAMSGWAYLKVRELSLAGEPG

orf135a.pep   WRVVFYLSVTGVAMSSVWATLTGWHTLSFPSAVYLSCIGVSALIAQLSMTRAYKVGDKFT
              ||||||||||||||||||||||||||||||||||||||||||||||||||||||||||||
orf135-1      WRVVFYLSVTGVAMSSVWATLTGWHTLSFPSAVYLSCIGVSALIAQLSMTRAYKVGDKFT

orf135a.pep   VASLSYMTVVFSALSAAFFLAEELFWQEILGMCIIILSGILSSIRPTAFKQRLQSLFRQR
              ||||||||||||||||||||:|||||||||||||||||||||||||||||||||||||||
orf135-1      VASLSYMTVVFSALSAAFFLGEELFWQEILGMCIIILSGILSSIRPTAFKQRLQSLFRQR
```

Homology with a predicted ORF from *N.gonorrhoeae*

ORF135 shows 97% identity over a 201aa overlap with a predicted ORF (ORF135ng) from *N.gonorrhoeae:*

```
orf135.pep                                GTGAMLLLFYAVTXLPLATGVTLSYTSSIF   30
                                          |||||||||||| |||:||||||||||||
orf135ng    STVTLGAAAVLRRDTFRTPHWKNHLNRSMVGTGAMLLLFYAVTHLPLTTGVTLSYTSSIF  335

orf135.pep  LAVFSFLILKERISVYTQAVLLLGFAGVVLLLNPSFRSGQETAALAGLAGGAMSGWAYLK   90
            |||||||||||||||||||||||||||||||||||||||||| |||||||||||||||||
orf135ng    LAVFSFLILKERISVYTQAVLLLGFAGVVLLLNPSFRSGQEPAALAGLAGGAMSGWAYLK  395

orf135.pep  VRELSLAGEPGWRVVFYLSVTGVAMSSVWATLTGWHTLSFPSAVYLSCIGVSALIAQLSM  150
            ||||||||||||||||||:||||||||||||||||||||||||||||| ||||||||||||
orf135ng    VRELSLAGEPGWRVVFYLSATGVAMSSVWATLTGWHTLSFPSAVYLSGIGVSALIAQLSM  455

orf135.pep  TRAYKVGDKFTVASLSYMTVVFSALSAAFFLGEELFWQEILGMCIIISAVF  201
            ||||||||||||||||||||||||||||||||||||||||||||||||:|
orf135ng    TRAYKVGDKFTVASLSYMTVVFSALSAAFFLGEELFWQEILGMCIIISAAF  506
```

An ORF135ng nucleotide sequence <SEQ ID 545> was predicted to encode a protein having amino acid sequence <SEQ ID 546>:

```
  1  MPSEKAFRRH LRTASFQGLH LHHFHQKVGK CGIIGFGIHI FPTLLPAAQG
 51  ILDIQLGLFR IDFAALAVYR RTQVDFIHTV IDGIASDQAF SEVVQILRRL
101  NLGHFTDTHL IAQARRFIAD FGNIRPMRRG EAKTFCRCFR FDGIDGIHGD
151  FRQCGHINRL APGKDCRNGK RDKVFFHTRH YNQVCLEKTN CSARKIKFRH
201  QKQAKTHSTS LAARFTIRPS LSQRPFMDTA KKDILGSGWM LVAAACFTVM
251  NVLIKEASAK FALGSGELVF WRMLFSTVTL GAAAVLRRDT FRTPHWKNHL
301  NRSMVGTGAM LLLFYAVTHL PLTTGVTLSY TSSIFLAVFS FLILKERISV
351  YTQAVLLLGF AGVVLLLNPS FRSGQEPAAL AGLAGGAMSG WAYLKVRELS
401  LAGEPGWRVV FYLSATGVAM SSVWATLTGW HTLSFPSAVY LSGIGVSALI
451  AQLSMTRAYK VGDKFTVASL SYMTVVFSAL SAAFFLGEEL FWQEILGMCI
501  IISAAF*
```

Further work revealed the following gonococcal sequence <SEQ ID 547>:

```
  1  ATGGATACCG CAAAAAAAGA CATTTTAGGA TCGGGCTGGA TGCTGGTGGC
 51  GGCGGCCTGC TTCACCGTTA TGAACGTATT GATTAAAGAG GCATCGGCAA
101  AATTTGCCCT CGGCAGCGGC GAATTGGTCT TTTGGCGCAT GCTGTTTTCA
151  ACCGTTACGC TCGGTGCTGC CGCCGTATTG CGGCGCGACA CCTTCCGCAC
201  GCCCCATTGG AAAAACCACT TAAACCGCAG TATGGTCGGG ACGGGGGCGA
251  TGCTGCTGCT GTTTTACGCG GTAACGCATC TGCCTTTGAC AACCGGCGTT
301  ACCCTGAGTT ACACCTCGTC GATTTTTttg GCGGTATTTT CCTTCCTGAT
351  TTTGAAAGAA CGGATTTCCG TTTACACGCA GGCGGTGCTG CTCCTTGGTT
401  TTGCCGGCGT GGTATTGCTG CTTAATCCCT CGTTCCGCAG CGGTCAGGAA
451  CCGGCGGCAC TCGCCGGGCT GGCGGGCGGC GCGATGTCCG GCTGGGCGTA
501  TTTGAAAGTG CGCGAACTGT CTTTGGCGGG CGAACCCGGC TGGCGCGTCG
551  TGTTTTACCT TTCCGCAACC GGCGTGGCGA TGTCGTCggt ttgggcgacg
601  Ctgaccggct ggCACAcccT GTCCTTTcca tcggcagttt ATCtgtCGGG
651  CATCGGCGTG tccgcgCtgA TTGCCCAaCT GtcgatgAcg cGCGcctaca
701  aaGTCGGCGA CAAATTCACG GTTGCCTCGC tttcctaTAt gaccgtcGTC
751  TTTTCCGCCC TGTCTGCCGC ATTTTTTCTg ggcgaagagc tttTCtggCA
801  GGAAATACTC GGTATGTGCA TCATTAtccT CAGCGGCATT TTGAGCAGCA
851  TCCGCCCCAT TGCCTTCAAA CAGCGGCTGC AAGCCCTCTT CCGCCAAAGA
901  TAA
```

This corresponds to the amino acid sequence <SEQ ID 548; ORF135ng-1>:

```
  1  MDTAKKDILG SGWMLVAAAC FTVMNVLIKE ASAKFALGSG ELVFWRMLFS

 51  TVTLGAAAVL RRDTFRTPHW KNHLNRSMVG TGAMLLLFYA VTHLPLTTGV
101  TLSYTSSIFL AVFSFLILKE RISVYTQAVL LLGFAGVVLL LNPSFRSGQE
151  PAALAGLAGG AMSGWAYLKV RELSLAGEPG WRVVFYLSAT GVAMSSVWAT
201  LTGWHTLSFP SAVYLSGIGV SALIAQLSMT RAYKVGDKFT VASLSYMTVV
251  FSALSAAFFL GEELFWQEIL GMCIIILSGI LSSIRPIAFK QRLQALFRQR
301  *
```

ORF135ng-1 and ORF135-1 show 97.0% identity in 300 aa overlap:

```
orf135ng-1.pep MDTAKKDILGSGWMLVAAACFTVMNVLIKEASAKFALGSGELVFWRMLFSTVTLGAAAVL
               ||||||||||||||||||||||:|||||||||||||||||||||||||||||:|||||||
orf135-1       MDTAKKDILGSGWMLVAAACFTIMNVLIKEASAKFALGSGELVFWRMLFSTVALGAAAVL

orf135ng-1.pep RRDTFRTPHWKNHLNRSMVGTGAMLLLFYAVTHLPLTTGVTLSYTSSIFLAVFSFLILKE
               |||:||||||||||||||||||||||||||||||||:|||||||||||||||||||||||
orf135-1       RRDXFRTPHWKNHLNRSMVGTGAMLLLFYAVTHLPLATGVTLSYTSSIFLAVFSFLILKE

orf135ng-1.pep RISVYTQAVLLLGFAGVVLLLNPSFRSGQEPAALAGLAGGAMSGWAYLKVRELSLAGEPG
               |||||||||||||||||||||||||||||| |||||||||||||||||||||||||||||
orf135-1       RISVYTQAVLLLGFAGVVLLLNPSFRSGQETAALAGLAGGAMSGWAYLKVRELSLAGEPG

orf135ng-1.pep WRVVFYLSATGVAMSSVWATLTGWHTLSFPSAVYLSGIGVSALIAQLSMTRAYKVGDKFT
               ||||||||:||||||||||||||||||||||||||| |||||||||||||||||||||||
orf135-1       WRVVFYLSVTGVAMSSVWATLTGWHTLSFPSAVYLSCIGVSALIAQLSMTRAYKVGDKFT

orf135ng-1.pep VASLSYMTVVFSALSAAFFLGEELFWQEILGMCIIILSGILSSIRPIAFKQRLQALFRQR
               |||||||||||||||||||||||||||||||||||||||||||||| |||||||:|||||
orf135-1       VASLSYMTVVFSALSAAFFLGEELFWQEILGMCIIILSGILSSIRPTAFKQRLQSLFRQR
```

Based on this analysis, including the presence of several putative transmembrane domains in the gonococcal protein, it is predicted that the proteins from *N.meningitidis* and *N.gonorrhoeae,* and their epitopes, could be useful antigens for vaccines or diagnostics, or for raising antibodies.

**Example 66**

The following DNA sequence was identified in *N.meningitidis* <SEQ ID 549>:

```
  1 ATGAAGCGGC GTATAGCCGT CTTCGTCCTG TTCCCGCAGA TAATCCGAGT
 51 TTTGGGACAA CTGTTGCCGA AAATCGTCAA TACAGTTCCG GCACATCGGA
101 TGCTCTTCCA GATTTTCGGG ATGTTCTTTT TCTTCATACA CCAGCAATAT
151 CTGCCCGGGA TCGCCGAAAT CGATTCCCCA TGCGGCATCG TGTTCGGTGC
201 GCTCCTCTTC CGTCATCTGC CCGCGCATTG CCTGTATGGT AAAGCCGCCG
251 TAGGGGATGC CgTTGCACAC GAACATCCAG TCGCTGATGT CGTCAACCGG
301 AACGCAAACG cTTTCGCCTT GTTCGACATT GGTCAGTTCG CCsGGTTCAT
351 TGTTCAGCAC ACCGTAAATA TAAAGACCGT CAAAATAAAT ATCGTCGATC
401 CACATATGTT CGCAAATTTC GCCGTCTTCG CCGTCTTGGA AAAAAGGGAC
451 TTTGACCATG GCAAAATCCA AGGCGGAAAT AATGCGGCGG CGTTCCCAAA
501 AAAGcTCGCG CCAAAAATAT TTGAATGTTT TACGGGCGCG TTCGTCGGCA
551 CGGTTTACCG GTTCGTCTGC CTGTTCTACA TAATAAATGA CGGAATCGCC
601 CATCATATCT GCTCCTCAAC GTGTACGGTA TCTGTTTGCA CCTTACTGCG
651 GCTTTCTgcC kTCGGCATCC GATTCGGATT TGAAAAGTTC mmrwyATTCG
701 GAATAG
```

This corresponds to the amino acid sequence <SEQ ID 550; ORF136>:

```
  1 MKRRIAVFVL FPQIIRVLGQ LLPKIVNTVP AHRMLFQIFG MFFFFIHQQY
 51 LPGIAEIDSP CGIVFGALLF RHLPAHCLYG KAAVGDAVAH EHPVADVVNR
101 NANAFALFDI GQFAXFIVQH TVNIKTVKIN IVDPHMFANF AVFAVLEKRD
151 FDHGKIQGGN NAAAFPKKLA PKIFECFTGA FVGTVYRFVC LFYIINDGIA
201 HHSAPQRVRY LFAPYCGFLP SASDSDLKSS XXSE*
```

Further work revealed the complete nucleotide sequence <SEQ ID 551>:

```
  1 ATGATGAAGC GGCGTATAGC CGTCTTCGTC CTGTTCCCGC AGATAATCCG
 51 AGTTTTGGGA CAACTGTTGC CGAAAATCGT CAATACAGTT CCGGCACATC
101 GGATGCTCTT CCAGATTTTC GGGATGTTCT TTTTCTTCAT ACACCAGCAA
151 TATCTGCCCG GGATCGCCGA AATCGATTCC CCATGCGGCA TCGTGTTCGG
```

```
201  TGCGCTCCTC TTCCGTCATC TGCCCGCGCA TTGCCTGTAT GGTAAAGCCG
251  CCGTAGGGGA TGCCGTTGCA CACGAACATC CAGTCGCTGA TGTCGTCAAC
301  CGGAACGCAA ACGCTTTCGC CTTGTTCGAC ATTGGTCAGT TCGCCGGGTT
351  CATTGTTCAG CACACCGTAA ATATAAAGAC CGTCAAAATA AATATCGTCG
401  ATCCACATAT GTTCGCAAAT TTCGCCGTCT TCGCCGTCTT GGAAAAAAGG
451  GACTTTGACC ATGGCAAAAT CCAAGGCGGA AATAATGCGG CGGCGTTCCC
501  AAAAAAGCTC GCGCCAAAAA TATTTGAATG TTTTACGGGC GCGTTCGTCG
551  GCACGGTTTA CCGGTTCGTC TGCCTGTTCT ACATAATAAA TGACGGAATC
601  GCCCATCATT CTGCTCCTCA ACGTGTACGG TATCTGTTTG CACCTTACTG
651  CGGCTTTCTG CCTTCGGCAT CCGATTCGGA TTTGAAAAGT TCCAAATATT
701  CGGAATAG
```

This corresponds to the amino acid sequence <SEQ ID 552; ORF136-1>:

```
  1  MMKRRIAVFV LFPQIIRVLG QLLPKIVNTV PAHRMLFQIF GMFFFFIHQQ
 51  YLPGIAEIDS PCGIVFGALL FRHLPAHCLY GKAAVGDAVA HEHPVADVVN
101  RNANAFALFD IGQFAGFIVQ HTVNIKTVKI NIVDPHMFAN FAVFAVLEKR
151  DFDHGKIQGG NNAAAFPKKL APKIFECFTG AFVGTVYRFV CLFYIINDGI
201  AHHSAPQRVR YLFAPYCGFL PSASDSDLKS SKYSE*
```

Computer analysis of this amino acid sequence gave the following results:

Homology with a predicted ORF from *N.meningitidis* (strain A)

ORF136 shows 71.7% identity over a 237aa overlap with an ORF (ORF136a) from strain A of *N. meningitidis:*

```
                       10        20        30        40        50       59
orf136.pep     MKRRIAVFVLFPQIIRVLGQLLPKIVNTVPAHRMLFQIFGMFFFFIHQQYLPGIAEIDS
               ||||||||||: | ||:|||||||||||||||||||| |||||||||||||||||||||
orf136a       MMKRRIAVFVLLMQKIRILGQLLPKIVNTVPAHRMLFQXFGMFFFFIHQQYLPGIAEIDS
                      10        20        30        40        50        60

             60        70        80        90       100       110      119
orf136.pep    PCGIVFGALLFRHLPAHCLYGKAAVGDAVAHEHPVADVVNRNANAFALFDIGQFAXFIVQ
              |||||||:|||||  :||||||||||:|||||||||||||||||||||||||||| ||||
orf136a       PCGIVFGTLLFRHXSTHCLYGKAAVGNAVAHEHPVADVVNRNANAFALFDIGQFAGFIVQ
                      70        80        90       100       110       120

            120       130       140       150       160       170      179
orf136.pep    HTVNIKTVKINIVDPHMFANFAVFAVLEKRDFDHGKIQGGNNAAAFPKKLAPKIFECFTG
              |::|:||||||||||||||||| ||||||| :  :| :         |:   | :: : :
orf136a       HAINVKTVKINIVDPHMFANFAXFAVLEKRALTMAKSKXXXMRRRSQKSSRQKYLNVLRA
                     130       140       150       160       170       180

            180       190       200          210       220       230
orf136.pep    AFVGTVYRFVCLFYIINDGIAHH---SAPQRVRYLFAPYCGFLPSASDSDLKSSXXSEX
                  :  ||: |    :  :::    ||||||||||||||||||||||||||||  |||
orf136a       R---SPARFTGLSACSTXXMTESPIISAPQRVRYLFAPYCGFLPSASDSDLKSSKYSEX
                        190       200       210       220       230
```

The complete length ORF136a nucleotide sequence <SEQ ID 553> is:

```
  1 ATGATGAAGC GGCGTATAGC CGTCTTCGTC CTGCTCATGC AGAAAATCCG
 51 GATTTTGGGA CAACTGTTGC CGAAAATCGT CAATACAGTT CCGGCACATC
101 GGATGCTCTT CCAGATNTTC GGGATGTTCT TTTTCTTCAT ACACCAGCAA
151 TACCTGCCCG GGATCGCCGA AATCGATTCC CCATGCGGCA TCGTGTTCGG
201 TACGCTCCTC TTCCGTCATC NGTCCACGCA TTGCCTGTAT GGTAAAGCCG
251 CCGTAGGGAA TGCCGTTGCA CACGAACATC CAGTCGCTGA TGTCGTCAAC
301 CGGAACGCAA ACGCTTTCGC CTTGTTCGAC ATTGGTCAGT TCGCCGGGTT
351 CATTGTTCAG CACGCCATAA ATGTAAAGAC CGTCAAAATA AATATCGTCG
401 ATCCACATAT GTTCGCAAAT TTCGCCNTCT TCGCCGTCTT GGAAAAAAGG
451 GCTTTGACCA TGGCAAAATC TAAGGNGNNA NNGATGCGGC GGCGTTCCCA
501 AAAAAGCTCG CGCCAAAAAT ATTTGAATGT TTTGCGGGCG CGTTCGCCGG
551 CACGGTTTAC CGGTTTGTCT GCCTGTTCTA CATAATAAAT GACGGAATCG
601 CCCATCATAT CTGCTCCTCA ACGTGTACGG TATCTGTTTG CACCTTACTG
651 CGGCTTTCTG CCTTCGGCAT CCGATTCGGA TTTGAAAAGT TCCAAATATT
701 CGGAATAG
```

This encodes a protein having amino acid sequence <SEQ ID 554>:

```
  1 MMKRRIAVFV LLMQKIRILG QLLPKIVNTV PAHRMLFQXF GMFFFFIHQQ
 51 YLPGIAEIDS PCGIVFGTLL FRHXSTHCLY GKAAVGNAVA HEHPVADVVN
101 RNANAFALFD IGQFAGFIVQ HAINVKTVKI NIVDPHMFAN FAXFAVLEKR
151 ALTMAKSKXX XMRRRSQKSS RQKYLNVLRA RSPARFTGLS ACST**MTES
201 PIISAPQRVR YLFAPYCGFL PSASDSDLKS SKYSE*
```

ORF136a and ORF136-1 show 73.1% identity in 238 aa overlap:

```
                        10        20        30        40        50        60
orf136a.pep    MMKRRIAVFVLLMQKIRILGQLLPKIVNTVPAHRMLFQXFGMFFFFIHQQYLPGIAEIDS
               |||||||||||: | ||:|||||||||||||||||||| |||||||||||||||||||||
orf136-1       MMKRRIAVFVLFPQIIRVLGQLLPKIVNTVPAHRMLFQIFGMFFFFIHQQYLPGIAEIDS
                        10        20        30        40        50        60

                        70        80        90       100       110       120
orf136a.pep    PCGIVFGTLLFRHXSTHCLYGKAAVGNAVAHEHPVADVVNRNANAFALFDIGQFAGFIVQ
               |||||||:|||||  :||||||||||:|||||||||||||||||||||||||||||||||
orf136-1       PCGIVFGALLFRHLPAHCLYGKAAVGDAVAHEHPVADVVNRNANAFALFDIGQFAGFIVQ
                        70        80        90       100       110       120

                       130       140       150       160       170       180
orf136a.pep    HAINVKTVKINIVDPHMFANFAXFAVLEKRALTMAKSKXXXMRRRSQKSSRQKYLNVLRA
               |::|:||||||||||||||||| ||||||| :  :| :         |:   | :: : :
orf136-1       HTVNIKTVKINIVDPHMFANFAVFAVLEKRDFDHGKIQGGNNAAAFPKKLAPKIFECFTG
                       130       140       150       160       170       180

                           190       200       210       220       230
orf136a.pep    R---SPARFTGLSACSTXXMTESPIISAPQRVRYLFAPYCGFLPSASDSDLKSSKYSEX
                   :  ||: |    :  :::    |||||||||||||||||||||||||||||||||
orf136-1       AFVGTVYRFVCLFYIINDGIAHH---SAPQRVRYLFAPYCGFLPSASDSDLKSSKYSEX
                        190       200          210       220       230
```

<u>Homology with a predicted ORF from *N.gonorrhoeae*</u>

ORF136 shows 92.3% identity over a 234aa overlap with a predicted ORF (ORF136ng) from *N.gonorrhoeae:*

```
orf136.pep     MKRRIAVFVLFPQIIRVLGQLLPKIVNTVPAHRMLFQIFGMFFFFIHQQYLPGIAEIDS   59
               ||||||||||: | || :||||||||||||||||||||||||||||||:|||||||||||
orf136ng      MMKRRIAVFVLLMQKIRILGQLLPKIVNTVPAHRMLFQIFGMFFFFIHRQYLPGIAEIDS   60

orf136.pep    PCGIVFGALLFRHLPAHCLYGKAAVGDAVAHEHPVADVVNRNANAFALFDIGQFAXFIVQ  119
              | |||||:|||||| |||||||||||||||||||||||:|||||||||||||| | ||||
orf136ng      PGGIVFGTLLFRHLSAHCLYGKAAVGDAVAHEHPVADVANRNANAFALFDIGQSAGFIVQ  120

orf136.pep    HTVNIKTVKINIVDPHMFANFAVFAVLEKRDFDHGKIQGGNNAAAFPKKLAPKIFECFTG  179
              |||||||||||||||||||||||||||||||||||||||||||||||||||||:||||||
orf136ng      HTVNIKTVKINIVDPHMFANFAVFAVLEKRDFDHGKIQGGNNAAAFPKKLAPKVFECFTG  180

orf136.pep    AFVGTVYRFVCLFYIINDGIAHHSAPQRVRYLFAPYCGFLPSASDSDLKSSXXSE  234
              ||:||||||||||||||||||||:|||||||||||| |||| |||||||||  ||
orf136ng      AFAGTVYRFVCLFYIINDGIAHHTAPQRVRYLFAPYRGFLPPASDSDLKSSKYSE  235
```

The complete length ORF136ng nucleotide sequence <SEQ ID 555> is:

```
  1  ATGATGAAGC GGCGTATAGC CGTCTTCGTC CTGCTCATGC AGAAAATCCG
 51  GATTTTGGGA CAACTGTTGC CGAAAATCGT CAATACAGTT CCGGCACATC
101  GGATGCTCTT CCAAATTTTC GGGATGTTCT TTTTCTTCAT ACACCGGCAA
151  TACCTGCCCG GGATCGCCGA AATCGATTCC CCAGGCGGTA TCGTGTTCGG
201  TACGCTCCTC TTCCGTCATC TGTCCGCGCA TTGCCTGTAC GGTAAAGCCG
251  CCGTAGGGGA TGCCGTTGCA CACGAACATC CAGTCGCTGA TGTCGCCAAC
301  CGGAACGCAA ACGCTTTCGC CTTGTTCGAC ATTGGTCAGT CCGCCGGGTT
351  CATTGTTCAG CACACCGTAA ATATAAAGAC CGTCAAAATA AATATCGTCG
401  ATCCACATAT GTTCGCAAAT TTCGCCGTCT TCGCCGTCTT GGAAAAAAGG
451  GACTTTGACC ATGGCAAAAT CCAAGGCGGA AATAATGCGG CGGCGTTCCC
501  AAAAAAGCTC GCGCCAAAAG TATTTGAATG TTTTACGGGC GCGTTCGCCG
551  GCACGGTTTA CCGGTTCGTC TGCCTGTTCT ACATAATAAA TGACGGAATC

601  GCCCATCATA CTGCTCCTCA ACGTGTACGG TATCTGTTTG CACCTTACCG
651  CGGTTTTCTA CCTCCGGCAT CCGATTCGGA TTTGAAAAGT TCCAAATATT
701  CGGAATAG
```

This encodes a protein having amino acid sequence <SEQ ID 556>:

```
  1  MMKRRIAVFV LLMQKIRILG QLLPKIVNTV PAHRMLFQIF GMFFFFIHRQ
 51  YLPGIAEIDS PGGIVFGTLL FRHLSAHCLY GKAAVGDAVA HEHPVADVAN
101  RNANAFALFD IGQSAGFIVQ HTVNIKTVKI NIVDPHMFAN FAVFAVLEKR
151  DFDHGKIQGG NNAAAFPKKL APKVFECFTG AFAGTVYRFV CLFYIINDGI
201  AHHTAPQRVR YLFAPYRGFL PPASDSDLKS SKYSE*
```

ORF136ng and ORF136-1 show 93.6% identity in 235 aa overlap:

```
orf136ng      MMKRRIAVFVLLMQKIRILGQLLPKIVNTVPAHRMLFQIFGMFFFFIHRQYLPGIAEIDS
              |||||||||||: | ||:||||||||||||||||||||||||||||||:|||||||||||
orf136-1      MMKRRIAVFVLFPQIIRVLGQLLPKIVNTVPAHRMLFQIFGMFFFFIHQQYLPGIAEIDS

orf136ng      PGGIVFGTLLFRHLSAHCLYGKAAVGDAVAHEHPVADVANRNANAFALFDIGQSAGFIVQ
              | |||||:|||||| |||||||||||||||||||||||:|||||||||||||| ||||||
orf136-1      PCGIVFGALLFRHLPAHCLYGKAAVGDAVAHEHPVADVVNRNANAFALFDIGQFAGFIVQ

orf136ng      HTVNIKTVKINIVDPHMFANFAVFAVLEKRDFDHGKIQGGNNAAAFPKKLAPKVFECFTG
              |||||||||||||||||||||||||||||||||||||||||||||||||||||:||||||
orf136-1      HTVNIKTVKINIVDPHMFANFAVFAVLEKRDFDHGKIQGGNNAAAFPKKLAPKIFECFTG

orf136ng      AFAGTVYRFVCLFYIINDGIAHHTAPQRVRYLFAPYRGFLPPASDSDLKSSKYSEX
              ||:||||||||||||||||||||:|||||||||||| |||| ||||||||||||||
orf136-1      AFVGTVYRFVCLFYIINDGIAHHSAPQRVRYLFAPYCGFLPSASDSDLKSSKYSEX
```

Based on the presence of the putative transmembrane domains in the gonococcal protein, it is predicted that the proteins from *N.meningitidis* and *N.gonorrhoeae,* and their epitopes, could be useful antigens for vaccines or diagnostics, or for raising antibodies.

**Example 67**

The following partial DNA sequence was identified in *N.meningitidis* <SEQ ID 557>:

```
  1  ATGGAAAATA TGGTAACGTT TTCAAAAATC AGACCGCTTT TGGCAATCGC
 51  CGCCGCCGCG TTGCTTGCCG CC.TGCGGAC GGCGGGAAAT AATGCTGTCC
101  GCAAGCCGGT GCAAACCGCC AAACCCGCCG CAGTGGTCGG TTTGGCACTC
151  GGTGGCGGCG CATCTAAAGG ATTTGCCCAT GTAGGTATTA TTAAGGTTTT
201  GAAAGAAAAC GGTATTCCTG TGAAGGTGGT TACCGGCACC TCCGCAGGTT
251  CGATTGTCGG CAACCTTTTT GCATCGGGTA TGTCGCCCGA CCGCCTCGAA
301  TTGGAAGCCG AAATTTTAGG CAAAACCGAT TTGGTCGATT TAACCTTGTC
351  CACCAATGGG TTTATCAAAG GCGCAAAGCT GCAAAATTAC ATCAACCGAA
401  AACTCCGCGG CATGCAGATT CAGCAGTTTC CCATCAAATT TGCCGCC..
```

This corresponds to the amino acid sequence <SEQ ID 558; ORF137>:

```
  1  MENMVTFSKI RPLLAIAAAA LLAAXRTAGN NAVRKPVQTA KPAAVVGLAL
 51  GGGASKGFAH VGIIKVLKEN GIPVKVVTGT SAGSIVGNLF ASGMSPDRLE
101  LEAEILGKTD LVDLTLSTNG FIKGAKLQNY INRKLRGMQI QQFPIKFAA..
```

Further work revealed the complete nucleotide sequence <SEQ ID 559>:

```
  1  ATGGAAAATA TGGTAACGTT TTCAAAAATC AGACCGCTTT TGGCAATCGC
 51  CGCCGCCGCG TTGCTTGCCG CCTGCGGCAC GGCGGGAAAT AATGCTGTCC
101  GCAAGCCGGT GCAAACCGCC AAACCCGCCG CAGTGGTCGG TTTGGCACTC
151  GGTGGCGGCG CATCTAAAGG ATTTGCCCAT GTAGGTATTA TTAAGGTTTT
201  GAAAGAAAAC GGTATTCCTG TGAAGGTGGT TACCGGCACA TCGGCAGGTT
251  CGATTGTCGG CAGCCTTTTT GCATCGGGTA TGTCGCCCGA CCGCCTCGAA
301  TTGGAAGCCG AAATTTTAGG CAAAACCGAT TTGGTCGATT TAACCTTGTC
351  CACCAGTGGT TTTATCAAAG GCGAAAAGCT GCAAAATTAC ATCAACCGAA
401  AAGTCGGCGG CAGGCAGATT CAGCAGTTTC CCATCAAATT TGCCGCCGTT
451  GCTACTGATT TTGAAACCGG CAAGGCCGTC GCTTTCAATC AGGGGAATGC
501  CGGGCAGGCT GTGCGCGCTT CCGCCGCCAT TCCCAATGTG TTCCAACCCG
```

```
551  TTATCATCGG CAGGCATACA TATGTTGACG GCGGTCTGTC GCAGCCCGTG
601  CCCGTCAGTG CCGCCCGGCG GCAGGGGGCG AATTTCGTGA TTGCCGTCGA
651  TATTTCCGCC CGTCCGGGCA AAAACATCAG CCAAGGTTTC TTCTCTTATC
701  TCGATCAGAC GCTGAACGTA ATGAGCGTTT CTGCGTTGCA AAATGAGTTG
751  GGGCAGGCGG ATGTGGTTAT CAAACCGCAG GTTTTGGATT TGGGTGCAGT
801  CGGCGGATTC GATCAGAAAA AACGCGCCAT CCGGTTGGGT GAGGAGGCAG
851  CACGTGCCGC ATTGCCTGAA ATCAAACGCA AACTGGCGGC ATACCGTTAT
901  TGA
```

This corresponds to the amino acid sequence <SEQ ID 560; ORF137-1>:

```
  1  MENMVTFSKI RPLLAIAAAA LLAACGTAGN NAVRKPVQTA KPAAVVGLAL
 51  GGGASKGFAH VGIIKVLKEN GIPVKVVTGT SAGSIVGSLF ASGMSPDRLE
101  LEAEILGKTD LVDLTLSTSG FIKGEKLQNY INRKVGGRQI QQFPIKFAAV
151  ATDFETGKAV AFNQGNAGQA VRASAAIPNV FQPVIIGRHT YVDGGLSQPV
201  PVSAARRQGA NFVIAVDISA RPGKNISQGF FSYLDQTLNV MSVSALQNEL
251  GQADVVIKPQ VLDLGAVGGF DQKKRAIRLG EEAARAALPE IKRKLAAYRY
301  *
```

Computer analysis of this amino acid sequence gave the following results:

Homology with a predicted ORF from *N.meningitidis* strain A)

ORF137 shows 93.3% identity over a 149aa overlap with an ORF (ORF137a) from strain A of *N. meningitidis:*

```
                    10        20        30        40        50        60
orf137.pep   MENMVTFSKIRPLLAIAAAALLAAXRTAGNNAVRKPVQTAKPAAVVGLALGGGASKGFAH
             |||||||||||||||||||||||| ||||||:||||||||||||||||||||||||||||
orf137a      MENMVTFSKIRPLLAIAAAALLAACGTAGNNAARKPVQTAKPAAVVGLALGGGASKGFAH
                    10        20        30        40        50        60

                    70        80        90       100       110       120
orf137.pep   VGIIKVLKENGIPVKVVTGTSAGSIVGNLFASGMSPDRLELEAEILGKTDLVDLTLSTNG
             |||||||||||||||||||||||||||:||||||||||||||||||||||||||||||:|
orf137a      VGIIKVLKENGIPVKVVTGTSAGSIVGSLFASGMSPDRLELEAEILGKTDLVDLTLSTSG
                    70        80        90       100       110       120

                   130       140      149
orf137.pep   FIKGAKLQNYINRKLRGMQIQQFPIKFAA
             |||| |||||||||: | :||||||||||
orf137a      FIKGEKLQNYINRKVGGRRIQQFPIKFAAVATDFETGKAVAFNQGNAGQAVRASAAIPNV
                   130       140       150       160       170       180
```

The complete length ORF137a nucleotide sequence <SEQ ID 561> is:

```
  1 ATGGAAAATA TGGTAACGTT TTCAAAAATC AGACCGCTTT TGGCAATCGC
 51 CGCCGCCGCG TTGCTTGCCG CCTGCGGCAC GGCGGGAAAT AATGCTGCCC
101 GCAAGCCGGT GCAAACCGCC AAACCCGCCG CAGTGGTCGG TTTGGCACTC
151 GGTGGCGGCG CATCTAAAGG ATTTGCCCAT GTAGGTATTA TTAAGGTTTT
201 GAAAGAAAAC GGTATTCCTG TGAAGGTGGT TACCGGCACA TCGGCAGGTT
251 CGATAGTCGG CAGCCTTTTT GCATCGGGTA TGTCGCCCGA CCGCCTCGAA
301 TTGGAAGCCG AAATTTTAGG TAAAACCGAT TTGGTCGATT TAACCTTGTC
351 CACCAGTGGT TTTATCAAAG GCGAAAAGCT GCAAAATTAC ATCAACCGAA
401 AAGTCGGCGG CAGGCGGATT CAGCAGTTTC CCATCAAATT TGCCGCCGTT
451 GCTACTGATT TTGAAACCGG CAAGGCCGTC GCTTTCAATC AAGGGAATGC
501 CGGGCAGGCT GTGCGCGCTT CCGCCGCCAT TCCCAATGTG TTCCAACCCG
551 TTATCATCGG CAGGCATACA TATGTTGACG GCGGTCTGTC GCAGCCCGTG
601 CCCGTCAGTG CCGCCCGGCG GCANGNNNNG NATNTCGTGA TTGCCGTCGA
651 TATTTCCGCC CGTCCGAGCA AAAACATCAG CCAAGGCTTC TTCTCTTATC
701 TCGATCAGAC GCTGAACGTA ATGAGCGTTT CCGCGTTGCA AAATGAGTTG
751 GGGCAGGCGG ATGTGGTTAT CAAACCGCAG GTTTTGGATT TGGGTGCAGT
801 CGGCGGATTC GATCAGAAAA AACGCGCCAT CCGGTTGGGT GAGGAGGCAG
851 CACGTGCCGC ATTGCCTGAA ATCAAACGCA AACTGGCGGC ATACCGTTAT
901 TGA
```

This encodes a protein having amino acid sequence <SEQ ID 562>:

```
  1 MENMVTFSKI RPLLAIAAAA LLAACGTAGN NAARKPVQTA KPAAVVGLAL
 51 GGGASKGFAH VGIIKVLKEN GIPVKVVTGT SAGSIVGSLF ASGMSPDRLE
101 LEAEILGKTD LVDLTLSTSG FIKGEKLQNY INRKVGGRRI QQFPIKFAAV
151 ATDFETGKAV AFNQGNAGQA VRASAAIPNV FQPVIIGRHT YVDGGLSQPV
201 PVSAARRXXX XXVIAVDISA RPSKNISQGF FSYLDQTLNV MSVSALQNEL
251 GQADVVIKPQ VLDLGAVGGF DQKKRAIRLG EEAARAALPE IKRKLAAYRY
301 *
```

ORF137a and ORF137-1 show 97.3% identity in 300 aa overlap:

```
orf137a.pep   MENMVTFSKIRPLLAIAAAALLAACGTAGNNAARKPVQTAKPAAVVGLALGGGASKGFAH
              ||||||||||||||||||||||||||||||||:|||||||||||||||||||||||||||
orf137-1      MENMVTFSKIRPLLAIAAAALLAACGTAGNNAVRKPVQTAKPAAVVGLALGGGASKGFAH

orf137a.pep   VGIIKVLKENGIPVKVVTGTSAGSIVGSLFASGMSPDRLELEAEILGKTDLVDLTLSTSG
              ||||||||||||||||||||||||||||||||||||||||||||||||||||||||||||
orf137-1      VGIIKVLKENGIPVKVVTGTSAGSIVGSLFASGMSPDRLELEAEILGKTDLVDLTLSTSG

orf137a.pep   FIKGEKLQNYINRKVGGRRIQQFPIKFAAVATDFETGKAVAFNQGNAGQAVRASAAIPNV
              |||||||||||||||||||:||||||||||||||||||||||||||||||||||||||||
orf137-1      FIKGEKLQNYINRKVGGRQIQQFPIKFAAVATDFETGKAVAFNQGNAGQAVRASAAIPNV

orf137a.pep   FQPVIIGRHTYVDGGLSQPVPVSAARRXXXXXVIAVDISARPSKNISQGFFSYLDQTLNV
              |||||||||||||||||||||||||||     ||||||||||:|||||||||||||||||
orf137-1      FQPVIIGRHTYVDGGLSQPVPVSAARRQGANFVIAVDISARPGKNISQGFFSYLDQTLNV

orf137a.pep   MSVSALQNELGQADVVIKPQVLDLGAVGGFDQKKRAIRLGEEAARAALPEIKRKLAAYRY
              ||||||||||||||||||||||||||||||||||||||||||||||||||||||||||||
orf137-1      MSVSALQNELGQADVVIKPQVLDLGAVGGFDQKKRAIRLGEEAARAALPEIKRKLAAYRY
```

Homology with a predicted ORF from *N.gonorrhoeae*

ORF137 shows 89.9% identity over a 149aa overlap with a predicted ORF (ORF137ng) from *N.gonorrhoeae:*

```
orf137.pep   MENMVTFSKIRPLLAIAAAALLAAXRTAGNNAVRKPVQTAKPAAVVGLALGGGASKGFAH   60
             |||||||||||  :||||||||||||  |||||:||||||||||||||:||||||||||||
orf137ng     MENMVTFSKIRSFLAIAAAALLAACGTAGNNAARKPVQTAKPAAVVALALGGGASKGFAH   60

orf137.pep   VGIIKVLKENGIPVKVVTGTSAGSIVGNLFASGMSPDRLELEAEILGKTDLVDLTLSTNG  120
             :||:||||||||||||||||||||||||:|:|||||||||||||||||||||||||||||:|
orf137ng     IGIVKVLKENGIPVKVVTGTSAGSIVGSLLASGMSPDRLELEAEILGKTDLVDLTLSTSG  120

orf137.pep   FIKGAKLQNYINRKLRGMQIQQFPIKFAA                                149
             |||| |||||||||: | ||||||||||
orf137ng     FIKGEKLQNYINRKVGGRQIQQFPIKFAAVATDFETGKAVAFNQGNAGQAVRASAAIPNV  180
```

The complete length ORF137ng nucleotide sequence <SEQ ID 563> is:

```
  1  ATGGAAAATA TGGTAACGTT TTCAAAAATC AGATCATTTT TGGCAATCGC
 51  CGCCGCCGCG TTGCTTGCCG CCTGCGGTAC GGCGGGAAAC AATGCCGCCC
101  GCAAGCCGGT GCAAACCGCC AAACCCGCCG CAGTGGTCGC TTTGGCACTC
151  GGTGGCGGCG CATCTAAAGG ATTTGCCCAT ATAGGAATTG TTAAGGTTTT
201  GAAAGAAAAC GGTATTCCTG TGAAGGTGGT TACCGGCACA TCGGCAGGTT
251  CGATAGTCGG CAGCCTTTTG GCATCGGGTA TGTCGCCCGA CCGCCTCGAA
301  TTGGAAGCCG AGATTTTAGG TAAAACCGAT TTAGTCGATT TAACCTTGTC
351  CACCAGTGGT TTTATCAAAG GCGAAAAGCT GCAAAATTAC ATCAACCGAA
401  AAGTCGGCGG CAGGCAGATT CAGCAGTTTC CCATCAAATT TGCCGCCGTT
451  GCCACTGATT TTGAAACCGG CAAGGCCGTC GCTTTCAATC AAGGGAATGC
501  CGGGCAGGCG GTTCGTGCTT CCGCCGCCAT TCCCAATGTG TTCCAGCCAG
551  TCATCATCGG CAGGCACAAA TATGTTGACG GCGGTCTGTC GCAGCCCGTG
601  CCCGTCAGTG CCGCTCGGCG GCAGGGGGCG AATTTCGTGA TTGCCGTCGA
651  TATTTCCGCA CGTCCGAGCA AAAATGTCGG TCAAGGTTTC TTCTCTTATC
701  TCGATCAGAC GCTGAACGTG ATGAGCGTTT CCGTGTTGCA AAACGAGTTG
751  gggcAGGCGG ATGTGGTTAT CAAACCGCag gtTTTGGATT TGGGTGCAGT
801  CGGCGGATTC GATCAGAAAA AGCGCGCCAT CCGGTTGGGC GAGGAGGCAG
851  CACGTGCCGC ATTGCCTGAA ATCAAACGCA AACTGGCGGC ATACCGTTAT
901  TGA
```

This encodes a protein having amino acid sequence <SEQ ID 564>:

```
  1  MENMVTFSKI RSFLAIAAAA LLAACGTAGN NAARKPVQTA KPAAVVALAL
 51  GGGASKGFAH IGIVKVLKEN GIPVKVVTGT SAGSIVGSLL ASGMSPDRLE
101  LEAEILGKTD LVDLTLSTSG FIKGEKLQNY INRKVGGRQI QQFPIKFAAV
151  ATDFETGKAV AFNQGNAGQA VRASAAIPNV FQPVIIGRHK YVDGGLSQPV
201  PVSAARRQGA NFVIAVDISA RPSKNVGQGF FSYLDQTLNV MSVSVLQNEL
251  GQADVVIKPQ VLDLGAVGGF DQKKRAIRLG EEAARAALPE IKRKLAAYRY
301  *
```

ORF137ng and ORF137-1 show 96.0% identity in 300 aa overlap:

```
orf137ng    MENMVTFSKIRSFLAIAAAALLAACGTAGNNAARKPVQTAKPAAVVALALGGGASKGFAH
            ||||||||||| :|||||||||||||||||||:|||||||||||||:|||||||||||||
orf137-1    MENMVTFSKIRPLLAIAAAALLAACGTAGNNAVRKPVQTAKPAAVVGLALGGGASKGFAH

orf137ng    IGIVKVLKENGIPVKVVTGTSAGSIVGSLLASGMSPDRLELEAEILGKTDLVDLTLSTSG
            :||:|||||||||||||||||||||||||:||||||||||||||||||||||||||||||
orf137-1    VGIIKVLKENGIPVKVVTGTSAGSIVGSLFASGMSPDRLELEAEILGKTDLVDLTLSTSG

orf137ng    FIKGEKLQNYINRKVGGRQIQQFPIKFAAVATDFETGKAVAFNQGNAGQAVRASAAIPNV
            ||||||||||||||||||||||||||||||||||||||||||||||||||||||||||||
orf137-1    FIKGEKLQNYINRKVGGRQIQQFPIKFAAVATDFETGKAVAFNQGNAGQAVRASAAIPNV

orf137ng    FQPVIIGRHKYVDGGLSQPVPVSAARRQGANFVIAVDISARPSKNVGQGFFSYLDQTLNV
            ||||||||| ||||||||||||||||||||||||||||||||:||::|||||||||||||
orf137-1    FQPVIIGRHTYVDGGLSQPVPVSAARRQGANFVIAVDISARPGKNISQGFFSYLDQTLNV

orf137ng    MSVSVLQNELGQADVVIKPQVLDLGAVGGFDQKKRAIRLGEEAARAALPEIKRKLAAYRY
            ||||:|||||||||||||||||||||||||||||||||||||||||||||||||||||||
orf137      MSVSALQNELGQADVVIKPQVLDLGAVGGFDQKKRAIRLGEEAARAALPEIKRKLAAYRY
```

Based on the presence of a predicted prokaryotic membrane lipoprotein lipid attachment site (underlined) in the gonococcal protein, it is predicted that the proteins from *N.meningitidis* and *N.gonorrhoeae,* and their epitopes, could be useful antigens for vaccines or diagnostics, or for raising antibodies.

**Example 68**

The following partial DNA sequence was identified in *N.meningitidis* <SEQ ID 565>:

```
  1  ATGTTTCGTT TACAATTCAG GCTGTTTCCC CCTTTGCGAA CCGCCATGCA
 51  CATCCTGTTG ACCGCCCTGC TCAAATGCCT CTCCCTGcTG CCGCTTTCCT
101  GTCTGCACAC GCTGGGAAAC CGGCTCGGAC ATCTGGCGTT TTACCTTTTA
151  AAGGAAGACC GCGCGCGCAT CGTCGCCmAT ATGCGGCAGG CGGGTTTGAA
201  CCCCGACCCC AAAACGGTCA AAGCCGTTTT TGCGGAAACG GCAAAAGGCG
251  GTTTGGAACT TGCCCCCGCG TTTTTCAGAA AACCGGAAGA CATAGAAACA
301  ATGTTCAAAG CGGTACACGG CTGGGAACAT GTGCAGCAGG CTTTGGACAA
351  ACACGAAGGG CTGCTATTC..
```

This corresponds to the amino acid sequence <SEQ ID 566; ORF138>:

```
  1  MFRLQFRLFP PLRTAMHILL TALLKCLSLL PLSCLHTLGN RLGHLAFYLL
 51  KEDRARIVAX MRQAGLNPDP KTVKAVFAET AKGGLELAPA FFRKPEDIET
101  MFKAVHGWEH VQQALDKHEG LLF
```

Further work revealed the complete nucleotide sequence <SEQ ID 567>:

```
  1  ATGTTTCGTT TACAATTCAG GCTGTTTCCC CCTTTGCGAA CCGCCATGCA
 51  CATCCTGTTG ACCGCCCTGC TCAAATGCCT CTCCCTGCTG CCGCTTTCCT
101  GTCTGCACAC GCTGGGAAAC CGGCTCGGAC ATCTGGCGTT TTACCTTTTA
151  AAGGAAGACC GCGCGCGCAT CGTCGCCAAT ATGCGGCAGG CGGGTTTGAA
201  CCCCGACCCC AAAACGGTCA AAGCCGTTTT TGCGGAAACG GCAAAAGGCG
251  GTTTGGAACT TGCCCCCGCG TTTTTCAGAA AACCGGAAGA CATAGAAACA
301  ATGTTCAAAG CGGTACACGG CTGGGAACAT GTGCAGCAGG CTTTGGACAA
351  ACACGAAGGG CTGCTATTCA TCACGCCGCA CATCGGCAGC TACGATTTGG
401  GCGGACGCTA CATCAGCCAG CAGCTTCCGT TCCCGCTGAC CGCCATGTAC
451  AAACCGCCGA AAATCAAAGC GATAGACAAA ATCATGCAGG CGGGCAGGGT
```

```
501  TCGCGGCAAA GGAAAAACCG CGCCTACCAG CATACAAGGG GTCAAACAAA
551  TCATCAAAGC CCTGCGTTCG GGCGAAGCAA CCATCGTCCT GCCCGACCAC
601  GTCCCCTCCC CTCAAGAAGG CGGGGAAGGC GTATGGGTGG ATTTCTTCGG
651  CAAACCTGCC TATACCATGA CGCTGGCGGC AAAATTGGCA CACGTCAAAG
701  GCGTGAAAAC CCTGTTTTTC TGCTGCGAAC GCCTGCCTGG CGGACAAGGT
751  TTCGATTTGC ACATCCGCCC CGTCCAAGGG GAATTGAACG GCGACAAAGC
801  CCATGATGCC GCCGTGTTCA ACCGCAATGC CGAATATTGG ATACGCCGTT
851  TTCCGACGCA GTATCTGTTT ATGTACAACC GCTACAAAAT GCCGTAA
```

This corresponds to the amino acid sequence <SEQ ID 568; ORF138-1>:

```
  1  MFRLQFRLFP PLRTAMHILL TALLKCLSLL PLSCLHTLGN RLGHLAFYLL
 51  KEDRARIVAN MRQAGLNPDP KTVKAVFAET AKGGLELAPA FFRKPEDIET
101  MFKAVHGWEH VQQALDKHEG LLFITPHIGS YDLGGRYISQ QLPFPLTAMY
151  KPPKIKAIDK IMQAGRVRGK GKTAPTSIQG VKQIIKALRS GEATIVLPDH
201  VPSPQEGGEG VWVDFFGKPA YTMTLAAKLA HVKGVKTLFF CCERLPGGQG
251  FDLHIRPVQG ELNGDKAHDA AVFNRNAEYW IRRFPTQYLF MYNRYKMP*
```

Computer analysis of this amino acid sequence gave the following results:

Homology with a predicted ORF from *N.meningitidis* (strain A)

ORF138 shows 99.2% identity over a 123aa overlap with an ORF (ORF138a) from strain A of *N. meningitidis:*

```
                    10        20        30        40        50        60
orf138.pep  MFRLQFRLFPPLRTAMHILLTALLKCLSLLPLSCLHTLGNRLGHLAFYLLKEDRARIVAX
            |||||||||||||||||||||||||||||||||||||||||||||||||||||||||||
orf138a     MFRLQFRLFPPLRTAMHILLTALLKCLSLLPLSCLHTLGNRLGHLAFYLLKEDRARIVAN
                    10        20        30        40        50        60

                    70        80        90       100       110       120
orf138.pep  MRQAGLNPDPKTVKAVFAETAKGGLELAPAFFRKPEDIETMFKAVHGWEHVQQALDKHEG
            ||||||||||||||||||||||||||||||||||||||||||||||||||||||||||||
orf138a     MRQAGLNPDPKTVKAVFAETAKGGLELAPAFFRKPEDIETMFKAVHGWEHVQQALDKHEG
                    70        80        90       100       110       120

orf138.pep  LLF
            |||
orf138a     LLFITPHIGSYDLGGRYISQQLPFPLTAMYKPPKIKAIDKIMQAGRVRGKGKTAPTSIQG
                   130       140       150       160       170       180
```

The complete length ORF138a nucleotide sequence <SEQ ID 569> is:

```
  1 ATGTTTCGTT TACAATTCAG GCTGTTTCCC CCTTTGCGAA CCGCCATGCA
 51 CATCCTGTTG ACCGCCCTGC TCAAATGCCT CTCCCTGCTG CCGCTTTCCT
101 GTCTGCACAC GCTGGGAAAC CGGCTCGGAC ATCTGGCGTT TTACCTTTTA
151 AAGGAAGACC GCGCGCGCAT CGTCGCCAAT ATGCGTCAGG CAGGCATGAA
201 TCCCGACCCC AAAACGGTCA AAGCCGTTTT TGCGGAAACG GCAAAAGGCG
251 GTTTGGAACT TGCCCCCGCG TTTTTCAGAA AACCGGAAGA CATAGAAACA
301 ATGTTCAAAG CGGTACACGG CTGGGAACAT GTGCAGCAGG CTTTGGACAA
351 ACACGAAGGG CTGCTATTCA TCACGCCGCA CATCGGCAGC TACGATTTGG
401 GCGGACGCTA CATCAGCCAG CAGCTTCCGT TCCCGCTGAC CGCCATGTAC
451 AAACCGCCGA AAATCAAAGC GATAGACAAA ATCATGCAGG CGGGCAGGGT
501 TCGCGGCAAA GGAAAAACCG CGCCTACCAG CATACAAGGG GTCAAACAAA
551 TCATCAAAGC CCTGCGTTCG GGCGAAGCAA CCATCGTCCT GCCCGACCAC
601 GTCCCCTCCC CTCAAGAAGG CGGGGAAGGC GTATGGGTGG ATTTCTTCGG
651 CAAACCTGCC TATACCATGA CGCTGGCGGC AAAATTGGCA CACGTCAAAG
701 GCGTGAAAAC CCTGTTTTTC TGCTGCGAAC GCCTGCCTGG CGGACAAGGT
751 TTCGATTTGC ACATCCGCCC CGTCCAAGGG GAATTGAACG GCGACAAAGC
801 CCATGATGCC GCCGTGTTCA ACCGCAATGC CGAATATTGG ATACGCCGTT
851 TTCCGACGCA GTATCTGTTT ATGTACAACC GCTACAAAAT GCCGTAA
```

This encodes a protein having amino acid sequence <SEQ ID 570>:

```
  1 MFRLQFRLFP PLRTAMHILL TALLKCLSLL PLSCLHTLGN RLGHLAFYLL
 51 KEDRARIVAN MRQAGLNPDP KTVKAVFAET AKGGLELAPA FFRKPEDIET
101 MFKAVHGWEH VQQALDKHEG LLFITPHIGS YDLGGRYISQ QLPFPLTAMY
151 KPPKIKAIDK IMQAGRVRGK GKTAPTSIQG VKQIIKALRS GEATIVLPDH
201 VPSPQEGGEG VWVDFFGKPA YTMTLAAKLA HVKGVKTLFF CCERLPGGQG
251 FDLHIRPVQG ELNGDKAHDA AVFNRNAEYW IRRFPTQYLF MYNRYKMP*
```

ORF138a and ORF138-1 show 99.7% identity over a 298aa overlap:

```
orf138a.pep   MFRLQFRLFPPLRTAMHILLTALLKCLSLLPLSCLHTLGNRLGHLAFYLLKEDRARIVAN
              ||||||||||||||||||||||||||||||||||||||||||||||||||||||||||||
orf138-1      MFRLQFRLFPPLRTAMHILLTALLKCLSLLPLSCLHTLGNRLGHLAFYLLKEDRARIVAN

orf138a.pep   MRQAGMNPDPKTVKAVFAETAKGGLELAPAFFRKPEDIETMFKAVHGWEHVQQALDKHEG
              |||||:||||||||||||||||||||||||||||||||||||||||||||||||||||||
orf138-1      MRQAGLNPDPKTVKAVFAETAKGGLELAPAFFRKPEDIETMFKAVHGWEHVQQALDKHEG

orf138a.pep   LLFITPHIGSYDLGGRYISQQLPFPLTAMYKPPKIKAIDKIMQAGRVRGKGKTAPTSIQG
              ||||||||||||||||||||||||||||||||||||||||||||||||||||||||||||
orf138-1      LLFITPHIGSYDLGGRYISQQLPFPLTAMYKPPKIKAIDKIMQAGRVRGKGKTAPTSIQG

orf138a.pep   VKQIIKALRSGEATIVLPDHVPSPQEGGEGVWVDFFGKPAYTMTLAAKLAHVKGVKTLFF
              ||||||||||||||||||||||||||||||||||||||||||||||||||||||||||||
orf138-1      VKQIIKALRSGEATIVLPDHVPSPQEGGEGVWVDFFGKPAYTMTLAAKLAHVKGVKTLFF

orf138a.pep   CCERLPGGQGFDLHIRPVQGELNGDKAHDAAVFNRNAEYWIRRFPTQYLFMYNRYKMP
              ||||||||||||||||||||||||||||||||||||||||||||||||||||||||||
orf138-1      CCERLPGGQGFDLHIRPVQGELNGDKAHDAAVFNRNAEYWIRRFPTQYLFMYNRYKMP
```

Homology with a predicted ORF from *N.gonorrhoeae*

ORF138 shows 94.3% identity over a 123aa overlap with a predicted ORF (ORF138ng) from *N. gonorrhoeae:*

```
orf138.pep   MFRLQFRLFPPLRTAMHILLTALLKCLSLLPLSCLHTLGNRLGHLAFYLLKEDRARIVAX   60
             |||||||||||||||||||||||||||||| |||||||||||||||||||||||||||| 
orf138ng     MFRLQFRLFPPLRTAMHILLTALLKCLSLLSLSCLHTLGNRLGHLAFYLLKEDRARIVAN   60

orf138.pep   MRQAGLNPDPKTVKAVFAETAKGGLELAPAFFRKPEDIETMFKAVHGWEHVQQALDKHEG  120
             ||||||||| :||||||||||| |||||||||:|||||||||||||||||||||||| ||
orf138ng     MRQAGLNPDTQTVKAVFAETAKCGLELAPAFFKKPEDIETMFKAVHGWEHVQQALDKGEG  120

orf138.pep   LLF                                                          123
             |||
orf138ng     LLFITPHIGSYDLGGRYISQQLPFHLTAMYKPPKIKAIDKIMQAGRVRGKGKTAPTGIQG  180
```

The complete length ORF138ng nucleotide sequence <SEQ ID 571> is:

```
  1  ATGTTTCGTT TACAATTCAG GCTGTTTCCC CCTTTGCGAA CCGCCATGCA
 51  CATCCTGTTG ACCGCCCTGC TCAAATGCCT CTCCCTGCTG TCGCTTTCCT
101  GTCTGCACAC GCTGGGAAAC CGGCTCGGAC ATCTGGCGTT TTACCTTTTA
151  AAGGAAGACC GCGCGCGCAT CGTCGCCAAT ATGCGGCAGG CGGGTTTGAA
201  CCCCGACACG CAGACGGTCA AAGCCGTTTT TGCGGAAACG GCAAAATGCG
251  GTTTGGAACT TGCCCCCGCG TTTTTCAAAA AACCGGAAGA CATCGAAACA
301  ATGTTCAAAG CGGTACACGG CTGGGAACAC GTGCAGCAGG CTTTGGACAA
351  GGGCGAAGGG CTGCTGTTCA TCACGCCGCA CATCGGCAGC TACGATTTGG
401  GCGGACGCTA CATCAGCCAG CAGCTTCCGT TCCACCTGAC CGCCATGTAC
451  AAGCCGCCGA AAATCAAAGC GATAGACAAA ATCATGCAGG CGGGCAGGGT
501  GCGCGGCAAA GGCAAAACcg cgcccaccgg catACAAGGG GTCAAACAAA
551  tcatcaAGGC CCTGCGCGCG GGCGAGGCAA CCAtcATCCT GCCCGACCAC
601  GTCCCTTCTC CGCAGGAagg cggCGGCGTG TGGGCGGATT TTTCGGCAA
651  ACCTGCATAc acCATGACAC TGGCGGCAAA ATTGGCACAC GTCAAAGGCG
701  TGAAAACCCT GTTTTTCTGC TGCGAACGCC TGCCCGACGG ACAAGGCTTC
751  GTGTTGCACA TCCGCCCCGT CCAAGGGGAA TTGAACGGCA ACAAAGCCCA
801  CGATGCCGCC GTGTTCAACC GCAATACCGA ATATTGGATA CGCCGTTTTC
851  CGACGCAGTA TCTGTTTATG TACAACCGCT ATAAAACGCC GTAA
```

This encodes a protein having amino acid sequence <SEQ ID 572>:

```
  1  MFRLQFRLFP PLRTAMHILL TALLKCLSLL SLSCLHTLGN RLGHLAFYLL
 51  KEDRARIVAN MRQAGLNPDT QTVKAVFAET AKCGLELAPA FFKKPEDIET
101  MFKAVHGWEH VQQALDKGEG LLFITPHIGS YDLGGRYISQ QLPFHLTAMY
151  KPPKIKAIDK IMQAGRVRGK GKTAPTGIQG VKQIIKALRA GEATIILPDH
201  VPSPQEGGGV WADFFGKPAY TMTLAAKLAH VKGVKTLFFC CERLPDGQGF
251  VLHIRPVQGE LNGNKAHDAA VFNRNTEYWI RRFPTQYLFM YNRYKTP*
```

ORF138ng and ORF138-1 show 94.3% identity over 299aa overlap:

```
orf138-1.pep  MFRLQFRLFPPLRTAMHILLTALLKCLSLLPLSCLHTLGNRLGHLAFYLLKEDRARIVAN
              |||||||||||||||||||||||||||||| |||||||||||||||||||||||||||||
orf138ng      MFRLQFRLFPPLRTAMHILLTALLKCLSLLSLSCLHTLGNRLGHLAFYLLKEDRARIVAN

orf138-1.pep  MRQAGLNPDPKTVKAVFAETAKGGLELAPAFFRKPEDIETMFKAVHGWEHVQQALDKHEG
              ||||||||| :||||||||||| |||||||||:|||||||||||||||||||||||| ||
orf138ng      MRQAGLNPDTQTVKAVFAETAKCGLELAPAFFKKPEDIETMFKAVHGWEHVQQALDKGEG

orf138-1.pep  LLFITPHIGSYDLGGRYISQQLPFPLTAMYKPPKIKAIDKIMQAGRVRGKGKTAPTSIQG
              |||||||||||||||||||||||| |||||||||||||||||||||||||||||||:|||
orf138ng      LLFITPHIGSYDLGGRYISQQLPFHLTAMYKPPKIKAIDKIMQAGRVRGKGKTAPTGIQG

orf138-1.pep  VKQIIKALRSGEATIVLPDHVPSPQEGGEGVWVDFFGKPAYTMTLAAKLAHVKGVKTLFF
              |||||||||:|||||:|||||||||||| |||:|||||||||||||||||||||||||||
orf138ng      VKQIIKALRAGEATIILPDHVPSPQEGG-GVWADFFGKPAYTMTLAAKLAHVKGVKTLFF

orf138-1.pep   CCERLPGGQGFDLHIRPVQGELNGDKAHDAAVFNRNAEYWIRRFPTQYLFMYNRYKMP
               |||||| |||| ||||||||||||:|||||||||||:||||||||||||||||||| |
orf138ng       CCERLPDGQGFVLHIRPVQGELNGNKAHDAAVFNRNTEYWIRRFPTQYLFMYNRYKTP
```

In addition, ORF138ng is homologous to htrB protein from *Pseudomonas fluorescens:*

```
gnl|PID|e334283 (Y14568) htrB [Pseudomonas fluorescens] Length = 253
 Score = 80.8 bits (196), Expect = 9e-15
 Identities = 49/151 (32%), Positives = 79/151 (51%), Gaps = 6/151 (3%)

Query: 101 MFKAVHGWEHVQQALDKGEGLLFITPHIGSYD-LGGRYISQQLPFHLTAMYKPPKIKAID 159
           + + V G E +++AL  G+G++ IT H+G+++ L   Y SQ  P      Y+PPK+KA+D
Sbjct: 94  LVREVEGLEVLKEALASGKGVVGITSHLGNWEVLNHFYCSQCKPI---IFYRPPKLKAVD 150

Query: 160 KIMQAGRVRGKGKTAPTGIQGVKQIIKALRAGEATIILPDHVPSPQEGGGVWADFFGKPA 219
           ++++  RV+   K A +  +G+  +IK +R G    I  D  P P E  G++  FF   A
Sbjct: 151 ELLRKQRVQLGNKVAASTKEGILSVIKEVRKGGQVGIPAD--PEPAESAGIFVPFFATQA 208

Query: 220 YTMTLAAKLAHVKGVKTLFFCCERLPDGQGF 250
            T     +        +F    RLPDG G+
Sbjct: 209 LTSKFVPNMLAGGKAVGVFLHALRLPDGSGY 239
```

Based on this analysis, including the presence of a putative transmembrane domain in the gonococcal protein, it was predicted that the proteins from *N.meningitidis* and *N.gonorrhoeae,* and their epitopes, could be useful antigens for vaccines or diagnostics, or for raising antibodies.

ORF138-1 (57kDa) was cloned in the pGex vectors and expressed in *E.coli,* as described above. The products of protein expression and purification were analyzed by SDS-PAGE. Figure 14A shows the results of affinity purification of the GST-fusion protein. Purified GST-fusion protein was used to immunise mice, whose sera were used for ELISA (positive result) and FACS analysis (Figure 14B). These experiments confirm that ORF138-1 is a surface-exposed protein, and that it is a useful immunogen.

### Example 69

The following partial DNA sequence was identified in *N.meningitidis* <SEQ ID 573>:

```
 1  ..GCGTGGTCGG CCGGCGAATC GTGGCGTGTG TTAATGGAAA GTGAAACGTG
51    GCATGCGGTG TGGAATACTT TGCGCTTCTC GGCGGCGGCG GTGTATGCGG
```

```
 101  CAGCGGTTTT GGGTGTGGTG TATGCGGCGC CGGCGCGGCG GTCGGCGTGG
 151  ATGCGCGGGC TGATGTTTTA GCCGTTTATG GTGTCGCCGG TTTGTGTTTC
 201  GGCGGGCGTG CTGCTGCTTT ATCCGCAGTG GACGGCTTCG TTGCCGTTGC
 251  TGCTGGCGAT GTATGCGCTG CTGGCGTATC CGTTTGTGGC AAAAGATGTT
 301  TTATCAGCCT GGGATGCACT GCCGCCGGAT TACGGCAGGG CGGCGGCGGG
 351  TTTGGGTGCA AACGGCTTTC AGACGGCATG CCGCATCACG TTCCCCCTCT
 401  TGAAACCGGC GTTGCGGCGC GGTCTGACTT TGGCGGCGGC AACCTGCGTG
 451  GGCGAATTTG CGGCGACATT GTTTCTGTCG CGTCCGGAAT GGCAGACGCT
 501  GACGACTTTG ATTTATGCCT ATTTGGGACG CGCGGGTGAG GATAATTACG
 551  CGCGGGCGAT GGTGCTG..
```

This corresponds to the amino acid sequence <SEQ ID 574; ORF139>:

```
   1  ..AWSAGESWRV LMESETWHAV WNTLRFSAAA VYAAAVLGVV YAAPARRSAW
  51    MRGLMFXPFM VSPVCVSAGV LLLYPQWTAS LPLLLAMYAL LAYPFVAKDV
 101    LSAWDALPPD YGRAAAGLGA NGFQTACRIT FPLLKPALRR GLTLAAATCV
 151    GEFAATLFLS RPEWQTLTTL IYAYLGRAGE DNYARAMVL..
```

Further work revealed the complete nucleotide sequence <SEQ ID 575>:

```
   1  ATGGATGGAC GGCGTTGGGT GGTATGGGGT GCTTTTGCCC TGCTGCCTTC
  51  GGCTTTTTTG GCGGTAATGG TCGTTGCGCC TTTGTGGGCG GTGGCGGCGT
 101  ATGACGGTTT GGCGTGGCGC GCGGTGCTGT CGGATGCCTA TATGCTCAAA
 151  CGTTTGGCGT GGACGGTATT TCAGGCAGCG GCAACCTGTG TGCTGGTGCT
 201  GCCTTTGGGC GTGCCTGTCG CGTGGGTGCT GGCGCGGCTG GCGTTTCCGG
 251  GGCGGGCTTT GGTGCTGCGC CTGCTGATGC TGCCTTTTGT GATGCCCACG
 301  TTGGTGGCGG GCGTGGGCGT GCTGGCCCTG TTCGGGGCGG ACGGGCTGTT
 351  GTGGCGCGGC AGGCAGGATA CGCCGTATCT GTTGTTGTAC GGCAATGTGT
 401  TTTTCAACCT TCCTGTGTTG GTCAGGGCGG CGTATCAGGG GTTTGTGCAA
 451  GTGCCTGCGG CACGGCTTCA GACGGCACGG ACGTTGGGCG CGGGGGCGTG
 501  GCGGCGGTTT TGGGACATTG AAATGCCCGT TTTGCGCCCG TGGCTTGCCG
 551  GCGGCGTGTG CCTTGTCTTT CTGTATTGTT TTTCCGGGTT CGGGCTGGCG
 601  CTGCTGCTGG GCGGCAGCCG TTATGCCACG GTCGAAGTGG AAATTTACCA
 651  GTTGGTCATG TTCGAACTCG ATATGGCGGT TGCTTCGGTG CTGGTGTGGC
 701  TGGTGTTGGG GGTAACGGCG GCGGCAGGGT TGCTGTATGC GTGGTTCGGC
 751  AGGCGCGCGG TTTCGGATAA GGCGGTTTCC CCTGTGATGC CGTCGCCGCC
 801  GCAGTCGGTC GGGGAATATG TGCTGCTGGC GTTTGCGGCG GCGGTGTTGT
 851  CTGTGTGCTG CCTGTTTCCT TTGTTGGCAA TTGTTGTGAA AGCGTGGTCG
 901  GCCGGCGAAT CGTGGCGTGT GTTAATGGAA AGTGAAACGT GGCAGGCGGT
 951  GTGGAATACT TTGCGCTTCT CGGCGGCGGC GGTGTATGCG GCGGCGGTTT
1001  TGGGTGTGGT GTATGCGGCG GCGGCGCGGC GGTCGGCGTG GATGCGCGGG
1051  CTGATGTTTT TGCCGTTTAT GGTGTCGCCG GTTTGTGTTT CGGCGGGCGT
1101  GCTGCTGCTT TATCCGCAGT GGACGGCTTC GTTGCCGTTG CTGCTGGCGA
1151  TGTATGCGCT GCTGGCGTAT CCGTTTGTGG CAAAAGATGT TTTATCAGCC
1201  TGGGATGCAC TGCCGCCGGA TTACGGCAGG GCGGCGGCGG GTTTGGGTGC
1251  AAACGGCTTT CAGACGGCAT GCCGCATCAC GTTCCCCCTC TTGAAACCGG
1301  CGTTGCGGCG CGGTCTGACT TTGGCGGCGG CAACCTGCGT GGGCGAATTT
1351  GCGGCGACAT TGTTTCTGTC GCGTCCGGAA TGGCAGACGC TGACGACTTT
1401  GATTTATGCC TATTTGGGAC GCGCGGGTGA GGATAATTAC GCGCGGGCGA
1451  TGGTGCTGAC ATTGCTGTTG GCGGCGTTCG CGCTGGGTAT TTTCCTGCTG
1501  TTGGACGGCG GCGAAGGCGG AAAACAGACG GAAACGTTAT AA
```

This corresponds to the amino acid sequence <SEQ ID 576; ORF139-1>:

```
  1  MDGRRWVVWG AFALLPSAFL AVMVVAPLWA VAAYDGLAWR AVLSDAYMLK
 51  RLAWTVFQAA ATCVLVLPLG VPVAWVLARL AFPGRALVLR LLMLPFVMPT
101  LVAGVGVLAL FGADGLLWRG RQDTPYLLLY GNVFFNLPVL VRAAYQGFVQ
151  VPAARLQTAR TLGAGAWRRF WDIEMPVLRP WLAGGVCLVF LYCFSGFGLA
201  LLLGGSRYAT VEVEIYQLVM FELDMAVASV LVWLVLGVTA AAGLLYAWFG
251  RRAVSDKAVS PVMPSPPQSV GEYVLLAFAA AVLSVCCLFP LLAIVVKAWS
301  AGESWRVLME SETWQAVWNT LRFSAAAVYA AAVLGVVYAA AARRSAWMRG
351  LMFLPFMVSP VCVSAGVLLL YPQWTASLPL LLAMYALLAY PFVAKDVLSA
401  WDALPPDYGR AAAGLGANGF QTACRITFPL LKPALRRGLT LAAATCVGEF
451  AATLFLSRPE WQTLTTLIYA YLGRAGEDNY ARAMVLTLLL AAFALGIFLL
501  LDGGEGGKQT ETL*
```

Computer analysis of this amino acid sequence gave the following results:

Homology with a predicted ORF from *N.meningitidis* (strain A)

ORF139 shows 94.7% identity over a 189aa overlap with an ORF (ORF139a) from strain A of *N. meningitidis:*

```
                                                    10        20        30
orf139.pep                              AWSAGESWRVLMESETWHAVWNTLRFSAAA
                                        ||||||||||||||||:||||| ||||||
orf139a     QSVGEYVLLAFAAAVXSVCCLFXLLAIVVKAWSAGESWRVLMESETWQAVWNTXRFSAAA
            270       280       290       300       310       320

                     40        50        60        70        80        90
orf139.pep  VYAAAVLGVVYAAPARRSAWMRGLMFXPFMVSPVCVSAGVLLLYPQWTASLPLLLAMYAL
            ||||||||||||| ||||||||||| ||||||||||||||| |||||||||||||||
orf139a     VYAAAVLGVVYAAAARRSAWMRGLMFLPFMVSPVCVSAGVLLLXPQWTASLPLLLAMYAL
            330       340       350       360       370       380

                    100       110       120       130       140       150
orf139.pep  LAYPFVAKDVLSAWDALPPDYGRAAAGLGANGFQTACRITFPLLKPALRRGLTLAAATCV
            |||||||||||| |||||||||||||||||||||||||||||||||||||||||||||||
orf139a     LAYPFVAKDVLSAXDALPPDYGRAAAGLGANGFQTACRITFPLLKPALRRGLTLAAATCV
            390       400       410       420       430       440

                    160       170       180       189
orf139.pep  GEFAATLFLSRPEWQTLTTLIYAYLGRAGEDNYARAMVL
            |||||||| || |||||||||||| |||| |||||||||
orf139a     GEFAATLFXSRXEWQTLTTLIYAYXGRAGXDNYARAMVLTLLLAAFALGXFLLLDGGEGG
            450       460       470       480       490       500
```

The complete length ORF139a nucleotide sequence <SEQ ID 577> is:

```
   1  ATGGATGGAC GGCGTTGGGC GGTATGGGGT GCTTTTGCCC TGCTGCCTTC
  51  GGCTTTTTTG GCGGCAATGG TCGTTGCGCC TTTGTGGGCG GTGGCGGCGT
 101  ATGACGGTTT GGCGTGGCGC GCGGTGCTGT CGGATGCCTA TATGCTCAAA
 151  CGTTTGGCGT GGACGGTATT TCAGGCAGCG GCAACCTGTG TGCTGGTGCT
 201  GCCTTTGGGC GTGCCTGTCG CGTGGGTGCT GGCGCGGCTG GCGTTTCCGG
 251  GGCGGGCTTT GGTGCTGCGC CTGCTGATGC TGCCTTTTGT GATGCCCACG
 301  TTGGTGGCGG GCGTGGGCGT GCTGGCTCTG TTCGGGGCGG ACGGCCTGTN
 351  GTGGCGCGGC TGGCAGGATA CGCCGTATCT GTTGTTGTAC GGCAATGTGT
 401  TTTTTNACCT TCCTGTGTTG GTCAGGGCGG CATATCAGGG GTTTGTGCAA
 451  GTGCCTGCGG CACGGCTTCA GACGGCACNG ACATTGGGCG CGGGGGCGTG
 501  GCGGCGGTTT TGGGACATTG AAATGCCCGT TTTGCGCCCG TGGCTTGCCG
 551  GCGGCGTGTG CCTTGTCTTC CTGTATTGTT TTTCGGGGTT CGGGCTGGCA
 601  TTGCTGCTGG GCGGCAGCCG TTATGCCACG GTCGAAGTGG AAATTTACCA
 651  GTTGGTCATG TTCGAACTCG ATATGGCGGT TGCTTCGGTG CTNGTGTGGC
 701  TGGTGTNGGG GGTAACNGCG GCGGCAGGGT TGCTGTATGC GTGGTTCGGC
 751  AGGCGCGCGG TTTCGGATAA GGCNGTTTCC CCTGTGATGC CGTCGCCGCC
 801  GCAGTCGGTC GGGGAATATG TGCTNCTGGC GTTTGCGGCG GCGGTGTNGT
 851  CTGTGTGCTG CCTGTTTCNT TTGTTGGCAA TTGTTGTGAA AGCGTGGTCG
 901  GCCGGCGAAT CGTGGCGTGT GTTAATGGAA AGTGAAACGT GGCAGGCGGT
 951  GTGGAATACT NTGCGCTTCT CGGCGGCGGC GGTGTATGCG GCGGCGGTTT
1001  TGGGTGTGGT GTATGCGGCG GCGGCGCGGC GGTCGGCGTG GATGCGCGGG
1051  CTGATGTTTT TGCCGTTTAT GGTGTCGCCG GTTTGTGTTT CGGCGGGCGT
1101  GCTGCTGCTT NATCCGCAGT GGACGGCTTC GTTGCCGCTG CTGCTGGCGA
1151  TGTATGCGCT GCTGGCGTAT CCGTTTGTGG CAAAAGATGT TTTATCAGCC
1201  TGNGATGCAC TGCCGCCGGA TTACGGCAGG GCGGCGGCGG GTTTGGGTGC
1251  AAACGGCTTT CAGACGGCAT GCCGCATCAC GTTCCCCCTC TTGAAACCGG
1301  CGTTGCGGCG CGGTCTGACT TTGGCGGCGG CAACCTGCGT GGGCGAATTT
1351  GCGGCAACCT TGTTCNTGTC GCGTCNCGAG TGGCAGACGC TGACGACTTT
1401  GATTTATGCC TATNTGGGAC GCGCGGGTGA NGATAATTAC GCGCGGGCGA
1451  TGGTGCTGAC ATTGCTGTTG GCGGCGTTCG CGCTGGGTAT NTTCCTGCTG
1501  TTGGACGGCG GCGAAGGCGG AAAACGGACG GAAACGTTAT AA
```

This encodes a protein having amino acid sequence <SEQ ID 578>:

```
   1  MDGRRWAVWG AFALLPSAFL AAMVVAPLWA VAAYDGLAWR AVLSDAYMLK
  51  RLAWTVFQAA ATCVLVLPLG VPVAWVLARL AFPGRALVLR LLMLPFVMPT
 101  LVAGVGVLAL FGADGLXWRG WQDTPYLLLY GNVFFXLPVL VRAAYQGFVQ
 151  VPAARLQTAX TLGAGAWRRF WDIEMPVLRP WLAGGVCLVF LYCFSGFGLA
 201  LLLGGSRYAT VEVEIYQLVM FELDMAVASV LVWLVXGVTA AAGLLYAWFG
```

```
 251  RRAVSDKAVS PVMPSPPQSV GEYVLLAFAA AVXSVCCLFX LLAIVVKAWS
 301  AGESWRVLME SETWQAVWNT XRFSAAAVYA AAVLGVVYAA AARRSAWMRG
 351  LMFLPFMVSP VCVSAGVLLL XPQWTASLPL LLAMYALLAY PFVAKDVLSA
 401  XDALPPDYGR AAAGLGANGF QTACRITFPL LKPALRRGLT LAAATCVGEF
 451  AATLFXSRXE WQTLTTLIYA YXGRAGXDNY ARAMVLTLLL AAFALGXFLL
 501  LDGGEGGKRT ETL*
```

ORF139a and ORF139-1 show 96.5% homology over a 514aa overlap:

```
orf139a.pep   MDGRRWAVWGAFALLPSAFLAAMVVAPLWAVAAYDGLAWRAVLSDAYMLKRLAWTVFQAA
              ||||||:||||||||||||||:||||||||||||||||||||||||||||||||||||||
orf139-1      MDGRRWVVWGAFALLPSAFLAVMVVAPLWAVAAYDGLAWRAVLSDAYMLKRLAWTVFQAA

orf139a.pep   ATCVLVLPLGVPVAWVLARLAFPGRALVLRLLMLPFVMPTLVAGVGVLALFGADGLXWRG
              |||||||||||||||||||||||||||||||||||||||||||||||||||||||| |||
orf139-1      ATCVLVLPLGVPVAWVLARLAFPGRALVLRLLMLPFVMPTLVAGVGVLALFGADGLLWRG

orf139a.pep   WQDTPYLLLYGNVFFXLPVLVRAAYQGFVQVPAARLQTAXTLGAGAWRRFWDIEMPVLRP
               ||||||||||||| ||||||||||||||||||||||| |||||||||||||||||||
orf139-1      RQDTPYLLLYGNVFFNLPVLVRAAYQGFVQVPAARLQTARTLGAGAWRRFWDIEMPVLRP

orf139a.pep   WLAGGVCLVFLYCFSGFGLALLLGGSRYATVEVEIYQLVMFELDMAVASVLVWLVXGVTA
              ||||||||||||||||||||||||||||||||||||||||||||||||||||||| ||||
orf139-1      WLAGGVCLVFLYCFSGFGLALLLGGSRYATVEVEIYQLVMFELDMAVASVLVWLVLGVTA

orf139a.pep   AAGLLYAWFGRRAVSDKAVSPVMPSPPQSVGEYVLLAFAAAVXSVCCLFXLLAIVVKAWS
              |||||||||||||||||||||||||||||||||||||||||| |||||| ||||||||||
orf139-1      AAGLLYAWFGRRAVSDKAVSPVMPSPPQSVGEYVLLAFAAAVLSVCCLFPLLAIVVKAWS

orf139a.pep   AGESWRVLMESETWQAVWNTXRFSAAAVYAAAVLGVVYAAAARRSAWMRGLMFLPFMVSP
              |||||||||||||||||||| |||||||||||||||||||||||||||||||||||||||
orf139-1      AGESWRVLMESETWQAVWNTLRFSAAAVYAAAVLGVVYAAAARRSAWMRGLMFLPFMVSP

orf139a.pep   VCVSAGVLLLXPQWTASLPLLLAMYALLAYPFVAKDVLSAXDALPPDYGRAAAGLGANGF
              |||||||||| ||||||||||||||||||||||||||||| ||||||||||||||||||
orf139-1      VCVSAGVLLLYPQWTASLPLLLAMYALLAYPFVAKDVLSAWDALPPDYGRAAAGLGANGF

orf139a.pep   QTACRITFPLLKPALRRGLTLAAATCVGEFAATLFXSRXEWQTLTTLIYAYXGRAGXDNY
              |||||||||||||||||||||||||||||||||||| || |||||||||||| |||| |||
orf139-1      QTACRITFPLLKPALRRGLTLAAATCVGEFAATLFLSRPEWQTLTTLIYAYLGRAGEDNY

orf139a.pep   ARAMVLTLLLAAFALGXFLLLDGGEGGKRTETLX
              |||||||||||||||| |||||||||||:|||||
orf139-1      ARAMVLTLLLAAFALGIFLLLDGGEGGKQTETLX
```

Homology with a predicted ORF from *N.gonorrhoeae*

ORF139 shows 95.2% identity over a 189aa overlap with a predicted ORF (ORF139ng) from *N.gonorrhoeae:*

```
orf139.pep                                 AWSAGESWRVLMESETWHAVWNTLRFSAAA   30
                                           ||||||| |||||||||:||||||||||||
orf139ng    QSVGEYVLLAFSVAVLSVCCLFPLSAIVVKAWSAGESRRVLMESETWQAVWNTLRFSAAA   327

orf139.pep  VYAAAVLGVVYAAPARRSAWMRGLMFXPFMVSPVCVSAGVLLLYPQWTASLPLLLAMYAL   90
            |:|||||||||| ||| :|||||:| ||||||||||||||||||| ||||||||||||||
orf139ng    VFAAAVLGVVYAAAARRLVWMRGLVFLPFMVSPVCVSAGVLLLYPGWTASLPLLLAMYAL   387

orf139.pep  LAYPFVAKDVLSAWDALPPDYGRAAAGLGANGFQTACRITFPLLKPALRRGLTLAAATCV   150
            ||||||||||||||||||||||||||||||||||||||||||||||||||||||||||||
orf139ng    LAYPFVAKDVLSAWDALPPDYGRAAAGLGANGFQTACRITFPLLKPALRRGLTLAAATCV   447

orf139.pep  GEFAATLFLSRPEWQTLTTLIYAYLGRAGEDNYARAMVL                        189
            |||||||||||||||||||||||||||||||||||||||
orf139ng    GEFAATLFLSRPEWQTLTTLIYAYLGRAGEDNYARAMVLTLLLSAFAVCIFLLLDNGEGG   507
```

The complete length ORF139ng nucleotide sequence <SEQ ID 579> is predicted to encode a protein having amino acid sequence <SEQ ID 580>:

```
  1  MDGRCWAVRG AFSLLPSAFL AVMVVAPLWA VAAYDGLAWR AVLSDAYMLK
 51  RLAWTVFQAA ATCVLVLPLG VPVAWVLARL AFPGRALVLR LLMLPFVMPT
101  LVAGVGVLAL FGADGLLWRG RQDTPYLLLY GNVFFNLPVL VRAAYQGFAQ
151  VPAARLQTAR TLGAGAWRPF WDIEMPVLRP WLAGGVCLVF LYCFSGFGLA
201  LLLGGSRYAT VEVEIYQLVM FELDMAGASA LVWLVLGVTA AAGLLYAWFG
251  RRAVSDKAVS PVMPSPPQSV GEYVLLAFSV AVLSVCCLFP LSAIVVKAWS
301  AGESRRVLME SETWQAVWNT LRFSAAAVFA AAVLGVVYAA AARRLVWMRG
351  LVFLPFMVSP VCVSAGVLLL YPGWTASLPL LLAMYALLAY PFVAKDVLSA
401  WDALPPDYGR AAAGLGANGF QTACRITFPL LKPALRRGLT LAAATCVGEF
451  AATLFLSRPE WQTLTTLIYA YLGRAGEDNY ARAMVLTLLL SAFAVCIFLL
501  LDNGEGGKRT ETL*
```

Further work revealed a variant gonococcal DNA sequence <SEQ ID 581>:

```
   1  ATGGATGGAC GGTGTTGGGC GGTACGGGGT GCTTTTTCCC TGCTGCCTTC
  51  GGCTTTTTTG GCGGTAATGG TCGTTGCGCC TTTGTGGGCG GTGGCGGCGT
 101  ATGACGGTTT GGCGTGGCGC GCGGTGCTGT CGGATGCCTA TATGCTCAAA
 151  CGTTTGGCGT GGACGGTGTT TCAGGCGGCG GCAACCTGTG TGCTGGTGCT
 201  GCCTTTGGGC GTGCCTGTCG CGTGGGTGCT GGCGCGGCTG GCGTTCCCGG
 251  GGCGGGCTTT GGTGCTGCGC CTGCTGATGC TGCCGTTTGT GATGCCCACG
 301  CTGGTGGCGG GCGTGGGCGT GCTGGCTCTG TTCGGGGCGG ACGGGCTGTT
 351  GTGGCGCGGC CGGCAGGATA CGCCGTATCT GTTGTTGTAC GGCAATGTGT
 401  TTTTCAACCT GCCCGTGTTG GTCAGGGCGG CGTATCAGGG GTTTGCTCAA
 451  GTGCCTGCGG CACGGCTTCA GACGGCACGG ACGTTGGGCG CGGGGGCGTG
 501  GCGGCGGTTT TGGGACATTG AAATGCCCGT TTTGCGCCCG TGGCTTGCCG
 551  GCGGCGTGTG CCTTGTCTTC CTGTATTGTT TTTCGGGGTT CGGGCTGGCA
 601  TTGCTGTTGG GCGGCAGCCG TTATGCCACG GTCGAAGTGG AAATTTACCA
 651  GTTGGTTATG TTCGAACTCG ATATGGCGGG GGCTTCGGCG CTGGTGTGGC
 701  TGGTGTTGGG GGTAACGGCG GCGGCAGGGT TGCTGTATGC GTGGTTCGGC
 751  AGGCGCGCGG TTTCGGATAA GGCGGTTTCC CCCGTGATGC CGTCGCCGCC
 801  GCAATCGGTG GGGGAATATG TATTGCTGGC ATTTTCGGTG GCGGTGTTGT
 851  CCGTGTGCTG CCTGTTTCCT TTGTCGGCAA TTGTTGTGAA AGCGTGGTCG
 901  GCCGGCGAAT CGCGGCGTGT GTTAATGGAA AGTGAAACGT GGCAGGCAGT
 951  GTGGAATACt ttGCGCTTTT CGGCGGCGGC GGTGTTTGCG GCGGCGGTTT
1001  TGGGTGTGGT GTATGCGGCG GCGGCGCGGC GGCTGGTGTG GATGCGCGGA
1051  CTGGTGTTTT TACCGTTTAT GGTGTCGCCG GTTTGTGTTT CGGCGGGCGT
1101  GCTGCTGCTT TATCCGGGGT GGACGGCTTC GTTACCGCTG CTGCTGGCGA
1151  TGTATGCGCT GCTGGCGTAT CCGTTTGTGG CAAAAGATGT TTTATCGGCC
1201  TGGGATGCAC TGCCGCCGGA TTACGGCAGG GCGGCGGCAG GTTTGGGCGC
1251  AAACGGCTTT CAGACGGCAT GCCGTATCAC GTTCCCCCTC TTGAAACCGG
1301  CGTTGCGGCG CGGTCTGACT TTGGCGGCGG CGACGTGTGT GGGCGAATTT
1351  GCGGCAACCT TGTTCCTGTC GCGTCCGGAA TGGCAGACGT TGACGACTTT
1401  GATTTATGCC TATTTGGGGC GTGCGGGTGA GGACAATTAT GCGCGGGCAA
1451  TGGTGTTGAC ATTGCTGTTG TCGGCATTTG CGGTGTGCAT TTTCCTGCTG
1501  TTGGACAACG GCGAAGGCGg aaaACGGACG GAAACGTTAT AA
```

This corresponds to the amino acid sequence <SEQ ID 582; ORF139ng-1>:

```
  1  MDGRCWAVRG AFSLLPSAFL AVMVVAPLWA VAAYDGLAWR AVLSDAYMLK
 51  RLAWTVFQAA ATCVLVLPLG VPVAWVLARL AFPGRALVLR LLMLPFVMPT
101  LVAGVGVLAL FGADGLLWRG RQDTPYLLLY GNVFFNLPVL VRAAYQGFAQ
151  VPAARLQTAR TLGAGAWRRF WDIEMPVLRP WLAGGVCLVF LYCFSGFGLA
201  LLLGGSRYAT VEVEIYQLVM FELDMAGASA LVWLVLGVTA AAGLLYAWFG
251  RRAVSDKAVS PVMPSPPQSV GEYVLLAFSV AVLSVCCLFP LSAIVVKAWS
301  AGESRRVLME SETWQAVWNT LRFSAAAVFA AAVLGVVYAA AARRLVWMRG
351  LVFLPFMVSP VCVSAGVLLL YPGWTASLPL LLAMYALLAY PFVAKDVLSA
401  WDALPPDYGR AAAGLGANGF QTACRITFPL LKPALRRGLT LAAATCVGEF
451  AATLFLSRPE WQTLTTLIYA YLGRAGEDNY ARAMVLTLLL SAFAVCIFLL
501  LDNGEGGKRT ETL*
```

ORF139ng-1 and ORF139-1 show 95.9% identity over 513aa overlap:

```
orf139ng    MDGRCWAVRGAFSLLPSAFLAVMVVAPLWAVAAYDGLAWRAVLSDAYMLKRLAWTVFQAA
            |||| |:| |||:|||||||||||||||||||||||||||||||||||||||||||||||
orf139-1    MDGRRWVVWGAFALLPSAFLAVMVVAPLWAVAAYDGLAWRAVLSDAYMLKRLAWTVFQAA

orf139ng    ATCVLVLPLGVPVAWVLARLAFPGRALVLRLLMLPFVMPTLVAGVGVLALFGADGLLWRG
            ||||||||||||||||||||||||||||||||||||||||||||||||||||||||||||
orf139-1    ATCVLVLPLGVPVAWVLARLAFPGRALVLRLLMLPFVMPTLVAGVGVLALFGADGLLWRG

orf139ng    RQDTPYLLLYGNVFFNLPVLVRAAYQGFAQVPAARLQTARTLGAGAWRRFWDIEMPVLRP
            ||||||||||||||||||||||||||||:|||||||||||||||||||||||||||||||
orf139-1    RQDTPYLLLYGNVFFNLPVLVRAAYQGFVQVPAARLQTARTLGAGAWRRFWDIEMPVLRP

orf139ng    WLAGGVCLVFLYCFSGFGLALLLGGSRYATVEVEIYQLVMFELDMAGASALVWLVLGVTA
            |||||||||||||||||||||||||||||||||||||||||||||| ||:||||||||||
orf139-1    WLAGGVCLVFLYCFSGFGLALLLGGSRYATVEVEIYQLVMFELDMAVASVLVWLVLGVTA

orf139ng    AAGLLYAWFGRRAVSDKAVSPVMPSPPQSVGEYVLLAFSVAVLSVCCLFPLSAIVVKAWS
            ||||||||||||||||||||||||||||||||||||||::||||||||||| ||||||||
orf139-1    AAGLLYAWFGRRAVSDKAVSPVMPSPPQSVGEYVLLAFAAAVLSVCCLFPLLAIVVKAWS

orf139ng    AGESRRVLMESETWQAVWNTLRFSAAAVFAAAVLGVVYAAAARRLVWMRGLVFLPFMVSP
            |||| |||||||||||||||||||||||:||||||||||||||| :|||||:||||||||
orf139      AGESWRVLMESETWQAVWNTLRFSAAAVYAAAVLGVVYAAAARRSAWMRGLMFLPFMVSP

orf139ng    VCVSAGVLLLYPGWTASLPLLLAMYALLAYPFVAKDVLSAWDALPPDYGRAAAGLGANGF
            |||||||||||| |||||||||||||||||||||||||||||||||||||||||||||||
orf139-1    VCVSAGVLLLYPQWTASLPLLLAMYALLAYPFVAKDVLSAWDALPPDYGRAAAGLGANGF

orf139ng    QTACRITFPLLKPALRRGLTLAAATCVGEFAATLFLSRPEWQTLTTLIYAYLGRAGEDNY
            ||||||||||||||||||||||||||||||||||||||||||||||||||||||||||||
orf139-1    QTACRITFPLLKPALRRGLTLAAATCVGEFAATLFLSRPEWQTLTTLIYAYLGRAGEDNY

orf139ng    ARAMVLTLLLSAFAVCIFLLLDNGEGGKRTETL
            ||||||||||:|||: ||||||:|||||:||||
orf139-1    ARAMVLTLLLAAFALGIFLLLDGGEGGKQTETL
```

Based on the presence of a predicted binding-protein-dependent transport systems inner membrane component signature (underlined) in the gonococcal protein, it is predicted that the proteins from *N.meningitidis* and *N.gonorrhoeae,* and their epitopes, could be useful antigens for vaccines or diagnostics, or for raising antibodies.

**Example 70**

The following partial DNA sequence was identified in *N.meningitidis* <SEQ ID 583>:

```
  1  ATGGACGGCT GGACACAGAC GCTGTCCGCG CAAACCCTGT TGGGCATTTC
 51  GGCGGCGGCA ATCATCCTCA TTCTGATTTT AATCGTCAGA TTCCGCATCC
101  ACGCGCTGCT GACACTGGTC ATCGTCAGCC TGCTGACGGC TTTGGCAACC
151  GGTTTGCCCA CAGGCAGCAT TGTCAAAGAC ATACTGGTCA AAAACTTCGG
201  CGGCACGCTC GGCGGCGTGG CGCTTCTGGT CGGCCTGGGC GCGATGCTCG
251  AACGTTTGGT C...
```

This corresponds to the amino acid sequence <SEQ ID 584; ORF140>:

```
 1  MDGWTQTLSA QTLLGISAAA IILILILIVR FRIHALLTLV IVSLLTALAT
51  GLPTGSIVKD ILVKNFGGTL GGVALLVGLG AMLERLV..
```

Further work revealed the complete nucleotide sequence <SEQ ID 585>:

```
   1  ATGGACGGCT GGACACAGAC GCTGTCCGCG CAAACCCTGT TGGGCATTTC
  51  GGCGGCGGCA ATCATCCTCA TTCTGATTTT AATCGTCAAA TTCCGCATCC
 101  ACGCGCTGCT GACACTGGTC ATCGTCAGCC TGCTGACGGC TTTGGCAACC
 151  GGTTTGCCCA CAGGCAGCAT TGTCAACGAC ATACTGGTCA AAAACTTCGG
 201  CGGCACGCTC GGCGGCGTGG CGCTTCTGGT CGGCCTGGGC GCGATGCTCG
 251  GACGTTTGGT CGAAACATCC GGCGGCGCAC AGTCGCTGGC GGACGCGCTG
 301  ATCCGGATGT TCGGCGAAAA ACGCGCACCG TTCGCGCTGG GCGTTGCCTC
 351  GCTGATTTTC GGCTTCCCGA TTTTCTTCGA TGCCGGACTA ATCGTCATGC
 401  TGCCCATCGT GTTCGCCACC GCACGGCGCA TGAAACAGGA CGTACTGCCC
 451  TTCGCGCTTG CCTCCATCGG CGCATTTTCC GTCATGCACG TCTTCCTGCC
 501  GCCCCATCCG GGCCCGATTG CCGCTTCCGA ATTTTACGGC GCGAACATCG
 551  GCCAAGTTTT GATTTTGGGT CTGCCGACCG CCTTCATCAC ATGGTATTTC
 601  AGCGGCTATA TGCTCGGCAA AGTGTTGGGG CGCACCATCC ATGTTCCCGT
 651  TCCCGAACTG CTCAGCGGCG GCACGCAAGA CAACGACCTG CCGAAAGAAC
 701  CTGCCAAAGC AGGAACGGTC GTCGCCATCA TGCTGATTCC CATGCTGCTG
 751  ATTTTCCTGA ATACCGGCGT ATCGGCCCTC ATCAGCGAAA AACTCGTAAG
 801  TGCGGACGAA ACCTGGGTTC AGACGGCAAA AATAATCGGT TCGACACCGA
 851  TCGCCCTTCT GATTTCCGTA TTGGTCGCAC TGTTTGTCTT GGGACGCAAA
 901  CGCGGCGAAA GCGGCAGCGC GTTGGAAAAA ACCGTGGACG GCGCACTCGC
 951  CCCCGTCTGT TCCGTGATTC TGATTACCGG CGCGGGCGGT ATGTTCGGCG
1001  GCGTTTTGCG CGCTTCCGGC ATCGGCAAGG CACTCGCCGA CAGCATGGCG
1051  GATTTGGGCA TTCCCGTCCT TTTGGGCTGT TTCCTTGTCG CCTTGGCACT
1101  GCGTATCGCG CAAGGTTCGG CAACCGTCGC CCTGACCACC GCCGCCGCGC
1151  TGATGGCTCC TGCCGTTGCC GCCGCCGGCT TTACCGACTG GCAGCTCGCC
1201  TGTATCGTAT TGGCAACGGC GGCAGGTTCG GTCGGTTGCA GCCACTTCAA
1251  CGACTCCGGC TTCTGGCTGG TCGGCCGTCT CTTGGACATG GACGTACCGA
1301  CCACGCTGAA AACCTGGACG GTCAACCAAA CCCTCATCGC ACTCATCGGC
1351  TTTGCCTTGT CCGCACTGCT GTTCGCCATC GTCTGA
```

This corresponds to the amino acid sequence <SEQ ID 586; ORF140-1>:

```
  1  MDGWTQTLSA QTLLGISAAA IILILILIVK FRIHALLTLV IVSLLTALAT
 51  GLPTGSIVND ILVKNFGGTL GGVALLVGLG AMLGRLVETS GGAQSLADAL
101  IRMFGEKRAP FALGVASLIF GFPIFFDAGL IVMLPIVFAT ARRMKQDVLP
151  FALASIGAFS VMHVFLPPHP GPIAASEFYG ANIGQVLILG LPTAFITWYF
201  SGYMLGKVLG RTIHVPVPEL LSGGTQDNDL PKEPAKAGTV VAIMLIPMLL
251  IFLNTGVSAL ISEKLVSADE TWVQTAKIIG STPIALLISV LVALFVLGRK
301  RGESGSALEK TVDGALAPVC SVILITGAGG MFGGVLRASG IGKALADSMA
351  DLGIPVLLGC FLVALALRIA QGSATVALTT AAALMAPAVA AAGFTDWQLA
401  CIVLATAAGS VGCSHFNDSG FWLVGRLLDM DVPTTLKTWT VNQTLIALIG
451  FALSALLFAI V*
```

Computer analysis of this amino acid sequence gave the following results:

Homology with a predicted ORF from *N.meningitidis* strain A)

ORF140 shows 95.4% identity over a 87aa overlap with an ORF (ORF140a) from strain A of *N. meningitidis:*

```
                    10        20        30        40        50        60
orf140.pep  MDGWTQTLSAQTLLGISAAAIILILILIVRFRIHALLTLVIVSLLTALATGLPTGSIVKD
            ||||||||||||||||||||||||||||:|||||||||||||||||||||||||||||:|
orf140a     MDGWTQTLSAQTLLGISAAAIILILILIVKFRIHALLTLVIVSLLTALATGLPTGSIVND
                    10        20        30        40        50        60

                    70        80
orf140.pep  ILVKNFGGTLGGVALLVGLGAMLERLV
            :||||||||||||||||||||||| |||
orf140a     VLVKNFGGTLGGVALLVGLGAMLGRLVETSGGAQSLADALIRMFGEKRAPFALGVASLIF
                    70        80        90       100       110       120
```

The complete length ORF140a nucleotide sequence <SEQ ID 587> is:

```
   1 ATGGACGGCT GGACACAGAC GCTGTCCGCG CAAACCCTGT TGGGCATTTC
  51 GGCGGCGGCA ATCATCCTCA TTCTGATTTT AATCGTCAAA TTCCGCATCC
 101 ACGCGCTGCT GACACTGGTC ATCGTCAGCC TGCTGACGGC TTTGGCAACC
 151 GGTTTGCCCA CAGGCAGCAT TGTCAACGAC GTACTGGTCA AAAACTTCGG
 201 CGGCACGCTC GGCGGCGTGG CGCTTCTGGT CGGCCTGGGC GCGATGCTCG
 251 GACGTTTGGT CGAAACATCC GGCGGCGCAC AGTCGCTGGC GGACGCGCTG
 301 ATCCGGATGT TCGGCGAAAA ACGCGCACCG TTCGCGCTGG GCGTTGCCTC
 351 GCTGATTTTC GGCTTCCCGA TTTTCTTCGA TGCCGGACTA ATCGTCATGC
 401 TGCCCATCGT GTTCGCCACC GCACGGCGCA TGAAACAGGA CGTACTGCCC
 451 TTCGCGCTTG CCTCCATCGG CGCATTTTCC GTCATGCACG TCTTCCTGCC
 501 GCCCCATCCG GGCCCGATTG CCGCTTCCGA ATTTTACGGC GCGAACATCG
 551 GCCAAGTTTT GATTTTGGGT CTGCCGACCG CCTTCATCAC ATGGTATTTC
 601 AGCGGCTATA TGCTCGGCAA AGTGTTGGGG CGCACCATCC ATGTTCCCGT
 651 TCCCGAACTG CTCAGCGGCG GCACGCAAGA CAACGACCTG CCGAAAGAAC
 701 CTGCCAAAGC AGGAACGGTC GTCGCCATCA TGCTGATTCC CATGCTGCTG
 751 ATTTTCCTGA ATACCGGCGT ATCGGCCCTC ATCAGCGAAA AACTCGTAAG
 801 TGCGGACGAA ACCTGGGTTC AGACGGCAAA AATAATCGGT TCGACACCGA
 851 TCGCCCTTCT GATTTCCGTA TTGGTCGCAC TGTTTGTCTT GGGACGCAAA
 901 CGCGGCGAAA GCGGCAGCGC GTTGGAAAAA ACCGTGGACG GCGCACTCGC
 951 CCCCGTCTGT TCCGTGATTC TGATTACCGG CGCGGGCGGT ATGTTCGGCG
1001 GCGTTTTGCG CGCTTCCGGC ATCGGCAAGG CACTCGCCGA CAGCATGGCG
1051 GATTTGGGCA TTCCCGTCCT TTTGGGCTGT TTCCTTGTCG CCTTGGCACT
1101 GCGTATCGCG CAAGGTTCGG CAACCGTCGC CCTGACCACC GCCGCCGCGC

1151 TGATGGCTCC TGCCGTTGCC GCCGCCGGCT TTACCGACTG GCAGCTCGCC
1201 TGTATCGTAT TGGCAACGGC GGCAGGTTCG GTCGGTTGCA GCCACTTCAA
1251 CGACTCCGGC TTCTGGCTGG TCGGCCGCCT CTTGGACATG GACGTACCGA
1301 CCACGCTGAA AACCTGGACG GTCAACCAAA CCCTCATCGC ACTCATCGGC
1351 TTTGCCTTGT CCGCACTGCT GTTCGCCATC GTCTGA
```

This encodes a protein having amino acid sequence <SEQ ID 588>:

```
  1 MDGWTQTLSA QTLLGISAAA IILILILIVK FRIHALLTLV IVSLLTALAT
 51 GLPTGSIVND VLVKNFGGTL GGVALLVGLG AMLGRLVETS GGAQSLADAL
101 IRMFGEKRAP FALGVASLIF GFPIFFDAGL IVMLPIVFAT ARRMKQDVLP
151 FALASIGAFS VMHVFLPPHP GPIAASEFYG ANIGQVLILG LPTAFITWYF
201 SGYMLGKVLG RTIHVPVPEL LSGGTQDNDL PKEPAKAGTV VAIMLIPMLL
251 IFLNTGVSAL ISEKLVSADE TWVQTAKIIG STPIALLISV LVALFVLGRK
301 RGESGSALEK TVDGALAPVC SVILITGAGG MFGGVLRASG IGKALADSMA
351 DLGIPVLLGC FLVALALRIA QGSATVALTT AAALMAPAVA AAGFTDWQLA
401 CIVLATAAGS VGCSHFNDSG FWLVGRLLDM DVPTTLKTWT VNQTLIALIG
451 FALSALLFAI V*
```

ORF140a and ORF140-1 show 99.8% identity over a 461aa overlap:

```
orf140-1.pep   MDGWTQTLSAQTLLGISAAAIILILILIVKFRIHALLTLVIVSLLTALATGLPTGSIVND  60
               ||||||||||||||||||||||||||||||||||||||||||||||||||||||||||||
orf140a        MDGWTQTLSAQTLLGISAAAIILILILIVKFRIHALLTLVIVSLLTALATGLPTGSIVND  60

orf140-1.pep   ILVKNFGGTLGGVALLVGLGAMLGRLVETSGGAQSLADALIRMFGEKRAPFALGVASLIF 120
               :|||||||||||||||||||||||||||||||||||||||||||||||||||||||||||
orf140a        VLVKNFGGTLGGVALLVGLGAMLGRLVETSGGAQSLADALIRMFGEKRAPFALGVASLIF 120

orf140-1.pep   GFPIFFDAGLIVMLPIVFATARRMKQDVLPFALASIGAFSVMHVFLPPHPGPIAASEFYG 180
               ||||||||||||||||||||||||||||||||||||||||||||||||||||||||||||
orf140a        GFPIFFDAGLIVMLPIVFATARRMKQDVLPFALASIGAFSVMHVFLPPHPGPIAASEFYG 810

orf140-1.pep   ANIGQVLILGLPTAFITWYFSGYMLGKVLGRTIHVPVPELLSGGTQDNDLPKEPAKAGTV 240
               ||||||||||||||||||||||||||||||||||||||||||||||||||||||||||||
orf140a        ANIGQVLILGLPTAFITWYFSGYMLGKVLGRTIHVPVPELLSGGTQDNDLPKEPAKAGTV 240

orf140-1.pep   VAIMLIPMLLIFLNTGVSALISEKLVSADETWVQTAKIIGSTPIALLISVLVALFVLGRK 300
               ||||||||||||||||||||||||||||||||||||||||||||||||||||||||||||
orf140a        VAIMLIPMLLIFLNTGVSALISEKLVSADETWVQTAKIIGSTPIALLISVLVALFVLGRK 300

orf140-1.pep   RGESGSALEKTVDGALAPVCSVILITGAGGMFGGVLRASGIGKALADSMADLGIPVLLGC 360
               ||||||||||||||||||||||||||||||||||||||||||||||||||||||||||||
orf140a        RGESGSALEKTVDGALAPVCSVILITGAGGMFGGVLRASGIGKALADSMADLGIPVLLGC 360

orf140-1.pep   FLVALALRIAQGSATVALTTAAALMAPAVAAAGFTDWQLACIVLATAAGSVGCSHFNDSG 420
               ||||||||||||||||||||||||||||||||||||||||||||||||||||||||||||
orf140a        FLVALALRIAQGSATVALTTAAALMAPAVAAAGFTDWQLACIVLATAAGSVGCSHFNDSG 420

orf140-1.pep   FWLVGRLLDMDVPTTLKTWTVNQTLIALIGFALSALLFAIV  461
               |||||||||||||||||||||||||||||||||||||||||
orf140a        FWLVGRLLDMDVPTTLKTWTVNQTLIALIGFALSALLFAIV  461
```

Homology with a predicted ORF from *N.gonorrhoeae*

ORF140 shows 92% identity over a 87aa overlap with a predicted ORF (ORF140ng) from *N.gonorrhoeae:*

```
orf140.pep   MDGWTQTLSAQTLLGISAAAIILILILIVRFRIHALLTLVIVSLLTALATGLPTGSIVKD   60
             ||| |||||||||||||||||||||||||:|||:|||||||:||||||||||||||||:|
orf140ng     MDGRTQTLSAQTLLGISAAAIILILILIVKFRIRALLTLVIASLLTALATGLPTGSIVND   60

orf140.pep   ILVKNFGGTLGGVALLVGLGAMLERLV                                    87
             :|||||||||||||||||||||| |||
orf140ng     VLVKNFGGTLGGVALLVGLGAMLGRLVETSGGAQSLADALIRMFGEKRAPFAPGVASLIF  120
```

The complete length ORF140ng nucleotide sequence <SEQ ID 589> was predicted to encode a protein having amino acid sequence <SEQ ID 590>:

```
  1  MDGRTQTLSA QTLLGISAAA IILILILIVK FRIRALLTLV IASLLTALAT
 51  GLPTGSIVND VLVKNFGGTL GGVALLVGLG AMLGRLVETS GGAQSLADAL
101  IRMFGEKRAP FAPGVASLIF GFPIFFDAGL IVMLPIVFAT ARRMKQDVLP
151  FALASVGAFS VMHVFLPPHP GPIAASEFYG ANIGQVLILG LPTAFITWYF
201  SGYMLGKVLG RAIHVPVPEL LSGGTQDSDP PKEPAKAGTV VAVMLIPMLL
251  IFLNTGVSAL ISEKLVSADE TWVQTAKMIG STPVALLISV LAALLVLGRK
301  RGESGSTLEK TVDGALAPAC SVILITGAGG MFGGVLRASG IGKALADSMA
351  DLGIPVLLGC FLVALALRIA QGSATVALTT AAALMAPAVA AAGFTDWQLA
401  CIVLATAAGS VGCSHFNDSG FWLVGRLSDM DVPTTLKTWT VNQTLIAFIG
451  FALSALLFAI V*
```

Further work revealed a variant gonococcal DNA sequence <SEQ ID 591>:

```
   1 ATGGACGGCC GGACACAGAC GCTGTCCGCG CAAACCTTGT TGGGCATTTC
  51 GGCGGCGGCA ATCATCCTCA TTCTGATTTT AATCGTCAAA TTCCGCATCC
 101 GCGCGCTGCT GACACTGGTC ATCGCCAGCC TGCTGACGGC TTTGGCAACC
 151 GGTTTGCCCA CAGGCAGCAT CGTCAACGAC GTACTGGTCA AAAACTTCGG
 201 CGGCACGCTC GGCGGCGTGG CGCTTCTGGT CGGTCTGGGC GCAATGCTCG
 251 GACGTTTGGT AGAAACATCC GGCGGCGCAC AGTCGCTGGC GGACGCGCTG
 301 ATCCGGATGT TCGGCGAAAA ACGCGCACCG TTCGCTCCGG GCGTTGCCTC
 351 GCTGATTTTC GGCTTCCCGA TTTTCTTCGA TGCCGGACTA ATCGTCATGC
 401 TGCCCATCGT ATTCGCCACC GCACGGCGCA TGAAACAGGA CGTACTGCCC
 451 TTCGCGCTTG CCTCCGTCGG CGCATTTTCC GTCATGCACG TCTTCCTGCC
 501 GCCCCATCCG GGCCCGATTG CCGCTTCCGA ATTTTACGGC GCGAACATCG
 551 GCCAGGTTTT GATTTTGGGT CTGCCGACCG CCTTCATCAC ATGGTATTTC
 601 AGCGGCTATA TGCTCGGCAA AGTGTTGGGG CGCGCCATCC ATGTTCCCGT
 651 TCCCGAACTG CTCAGCGGCG GCACGCAAGA CAGCGACCCG CCGAAAGAAC
 701 CTGCCAAAGC AGGAACGGTC GTCGCCGTCA TGCTGATTCC CATGCTGCTG
 751 ATTTTCCTGA ATACCGGCGT ATCAGCCCTC ATCAGCGAAA AACTCGTAAG
 801 TGCGGACGAA ACTTGGGTTC AGACGGCAAA AATGATCGGT TCGACACCTG
 851 TCGCCCTTCT GATTTCCGTA TTGGCCGCAC TGTTGGTCTT GGGACGCAAA
 901 CGCGGCGAAA GCGGCAGCAC GTTGGAAAAA ACCGTGGACG GCGCACTCGC
 951 CCCCGCCTGT TCCGTGATTC TGATTACCGG CGCGGGCGGT ATGTTCGGCG
1001 GCGTTTTGCG CGCTTCCGGC ATCGGCAAGG CACTCGCCGA CAGCATGGCG
1051 GATTTGGGCA TTCCCGTCCT TTTGGGCTGC TTCCTTGTCG CCTTGGCACT
1101 GCGTATCGCG CAAGGTTCGG CAACCGTCGC CCTGACCACA GCCGCCGCGC
1151 TGATGGCTCC TGCCGTTGCC GCCGCCGGCT TTACCGACTG GCAGCTCGCC
1201 TGTATCGTAT TGGCAACGGC GGCAGGTTCG GTCGGTTGCA GCCACTTCAA
1251 CGACTCCGGC TTCTGGCTGG TCGGCCGCCT CTTGGATATG GACGTACCGA
1301 CCACGCTGAA AACCTGGACG GTCAACCAAA CCCTCATCGC ATTCATCGGC
1351 TTTGCCTTGT CCGCACTGCT GTTTGCCATC GTCTGA
```

This corresponds to the amino acid sequence <SEQ ID 592; ORF140ng-1>:

```
  1 MDGRTQTLSA QTLLGISAAA IILILILIVK FRIRALLTLV IASLLTALAT
 51 GLPTGSIVND VLVKNFGGTL GGVALLVGLG AMLGRLVETS GGAQSLADAL
101 IRMFGEKRAP FAPGVASLIF GFPIFFDAGL IVMLPIVFAT ARRMKQDVLP
151 FALASVGAFS VMHVFLPPHP GPIAASEFYG ANIGQVLILG LPTAFITWYF
201 SGYMLGKVLG RAIHVPVPEL LSGGTQDSDP PKEPAKAGTV VAVMLIPMLL
251 IFLNTGVSAL ISEKLVSADE TWVQTAKMIG STPVALLISV LAALLVLGRK
301 RGESGSTLEK TVDGALAPAC SVILITGAGG MFGGVLRASG IGKALADSMA
351 DLGIPVLLGC FLVALALRIA QGSATVALTT AAALMAPAVA AAGFTDWQLA
401 CIVLATAAGS VGCSHFNDSG FWLVGRLLDM DVPTTLKTWT VNQTLIAFIG
451 FALSALLFAI V*
```

ORF140ng-1 and ORF140-1 show 96.3% identity over 461 aa overlap:

```
orf140ng-1.pep MDGRTQTLSAQTLLGISAAAIILILILIVKFRIRALLTLVIASLLTALATGLPTGSIVND
               ||| |||||||||||||||||||||||||||||:|||||||:||||||||||||||||||
orf140-1       MDGWTQTLSAQTLLGISAAAIILILILIVKFRIHALLTLVIVSLLTALATGLPTGSIVND

orf140ng-1.pep VLVKNFGGTLGGVALLVGLGAMLGRLVETSGGAQSLADALIRMFGEKRAPFAPGVASLIF
               :|||||||||||||||||||||||||||||||||||||||||||||||||||| ||||||
orf140-1       ILVKNFGGTLGGVALLVGLGAMLGRLVETSGGAQSLADALIRMFGEKRAPFALGVASLIF

orf140ng-1.pep GFPIFFDAGLIVMLPIVFATARRMKQDVLPFALASVGAFSVMHVFLPPHPGPIAASEFYG
               |||||||||||||||||||||||||||||||||||:||||||||||||||||||||||||
orf140-1       GFPIFFDAGLIVMLPIVFATARRMKQDVLPFALASIGAFSVMHVFLPPHPGPIAASEFYG

orf140ng-1.pep ANIGQVLILGLPTAFITWYFSGYMLGKVLGRAIHVPVPELLSGGTQDSDPPKEPAKAGTV
               ||||||||||||||||||||||||||||||:|||||||||||||||:| |||||||||||
```

```
orf140-1         ANIGQVLILGLPTAFITWYFSGYMLGKVLGRTIHVPVPELLSGGTQDNDLPKEPAKAGTV

orf140ng-1.pep VAVMLIPMLLIFLNTGVSALISEKLVSADETWVQTAKMIGSTPVALLISVLAALLVLGRK
               ||:||||||||||||||||||||||||||||||||||||:|||||:||||||:||:|||||
orf140-1       VAIMLIPMLLIFLNTGVSALISEKLVSADETWVQTAKIIGSTPIALLISVLVALFVLGRK

orf140ng-1.pep RGESGSTLEKTVDGALAPACSVILITGAGGMFGGVLRASGIGKALADSMADLGIPVLLGC
               ||||||:|||||||||||:||||||||||||||||||||||||||||||||||||||||||
orf140-1       RGESGSALEKTVDGALAPVCSVILITGAGGMFGGVLRASGIGKALADSMADLGIPVLLGC

orf140ng-1.pep FLVALALRIAQGSATVALTTAAALMAPAVAAAGFTDWQLACIVLATAAGSVGCSHFNDSG
               ||||||||||||||||||||||||||||||||||||||||||||||||||||||||||||
orf140-1       FLVALALRIAQGSATVALTTAAALMAPAVAAAGFTDWQLACIVLATAAGSVGCSHFNDSG

orf140ng-1.pep FWLVGRLLDMDVPTTLKTWTVNQTLIAFIGFALSALLFAIV
               |||||||||||||||||||||||||||:|||||||||||||
orf140-1       FWLVGRLLDMDVPTTLKTWTVNQTLIALIGFALSALLFAIV
```

Furthermore, ORF140ng-1 is homologous to an *E.coli* protein:

```
gi|882633 (U29579) ORF_o454 [Escherichia coli] >gi|1789097 (AE000358) o454;
This 454 aa ORF is 34% identical (9 gaps) to 444 residues of an approx. 456 aa
protein GNTP_BACLI SW: P46832 [Escherichia coli] Length = 454
 Score = 210 bits (529), Expect = 1e-53
 Identities = 130/384 (33%), Positives = 194/384 (49%), Gaps = 19/384 (4%)

Query: 88  ETSGGAQSLADALIRMFGEKRAPFAPGVASLIFGFPIFFDAGLIVMLPIVFATARRMKQD 147
            E SGGA+SLA+   R  G+KR   A  +A+   G P+FFD G I++  PI++   A+  K
Sbjct: 80  EHSGGAESLANYFSRKLGDKRTIAALTLAAFFLGIPVFFDVGFIILAPIIYGFAKVAKIS 139

Query: 148 VLPFALASVGAFSVMHVFLPPHPGPIAASEFYGANIGQVLILGLPTAFITWYFSGYMLGK 207
              L F L   G    +HV +PPHPGP+AA+    A+IG + I+G+  + I     GY   K
Sbjct: 140 PLKFGLPVAGIMLTVHVAVPPHPGPVAAAGLLHADIGWLTIIGIAIS-IPVGVVGYFAAK 198

Query: 208 VLGRAIHVPVPELL----------SGGTQDSDPPKEPAKAGTVVAVMLIPMLLIFLNTGV 257
            ++ +  +     E+L            G T+ SD    P  A  V ++++IP+ +I    T
Sbjct: 199 IINKRQYAMSVEVLEQMQLAPASEEGATKLSDKINPPGVA-LVTSLIVIPIAIIMAGT-- 255

Query: 258 SALISEKLVSADETWVQTAKMIGSTPXXXXXXXXXXXXXXGRKRGESGSTLEKTVDGALA 317
               +S  L+       + T ++IGS                  +RG  S         +  AL
Sbjct: 256 ---VSATLMPPSHPLLGTLQLIGSPMVALMIALVLAFWLLALRRGWSLQHTSDIMGSALP 312

Query: 318 PACSVILITGAGGMFGGVLRASGIGKALADSMADLGIPVLLGCFLVALALRIAQGSXXXX 377
            A  VIL+TGAGG+FG VL  SG+GKALA+ +  + +P+L  F+++LALR +QGS
Sbjct: 313 TAAVVILVTGAGGVFGKVLVESGVGKALANMLQMIDLPLLPAAFIISLALRASQGS--AT 370

Query: 378 XXXXXXXXXXXXXXXGFTDWQLACIVLATAAGSVGCSHFNDSGFWLVGRLLDMDVPTTLK 437
                           G    Q    + LA   G +G SH NDSGFW+V + L + V   LK
Sbjct: 371 VAILTTGGLLSEAVMGLNPIQCVLVTLAACFGGLGASHINDSGFWIVTKYLGLSVADGLK 430

Query: 438 TWTVNQTLIAFIGFALSALLFAIV 461
            TWTV  T++ F GF ++   ++A++
Sbjct: 431 TWTVLTTILGFTGFLITWCVWAVI 454
```

Based on this analysis, including the identification of the presence of a putative leader sequence (double-underlined) and several putative transmembrane domains (single-underlined) in the gonococcal protein, it is predicted that the proteins from *N.meningitidis* and *N.gonorrhoeae,* and their epitopes, could be useful antigens for vaccines or diagnostics, or for raising antibodies.

## Example 71

The following partial DNA sequence was identified in *N.meningitidis* <SEQ ID 593>:

```
  1  ..GATTTCGGCA TATCGCCCGT GTATCTTTGG GTTGCCGCCG CGTTCAAACA
 51    TTTGCTGTCG CCGTGGGCTG CCGACTCATA CGATGTCGCA CGCTTTGCAG
101    GCGTATTTTT TGCCGTTATC GGACTGACTT CCTGCGGCTT TGCCGGTTTC
```

```
151    AACTTTTTGG GCAGACACCA CGGGCGCAC. GTCGTCCTGA TTCTCATCGG
201    CTGTATCGGG CTGATTCCAG TTGCCCATTT CCTCAACCCC GCTGCCGCCG
251    CCTTTGCCGC CGCCGGACTG GTGCTGCACG GTTATTCTTT GGCTCGCCGG
301    CGCGTGATTG CCGCCTCTTT TCTGCTCGGT ACGGGCTGGA CGCTGATGTC
351    GTTGGCAGCA GCTTATCCGG CAGCATTTGC CCTGATGCTG CCCTTGCCCG
401    TACTGATGTT TTTCCGTCCG ..
```

This corresponds to the amino acid sequence <SEQ ID 594; ORF141>:

```
  1  ..DFGISPVYLW VAAAFKHLLS PWAADSYDVA RFAGVFFAVI GLTSCGFAGF
 51    NFLGRHHGRX VVLILIGCIG LIPVAHFLNP AAAAFAAAGL VLHGYSLARR
101    RVIAASFLLG TGWTLMSLAA AYPAAFALML PLPVLMFFRP ..
```

Further work revealed the complete nucleotide sequence <SEQ ID 595>:

```
   1  ATGCTGACCT ATACCCCGCC CGATGCCCGC CCGCCCGCCA AAACCCACGA
  51  AAAGCCGTGG CTGCTGCTGT TGATGGCGTT TGCCTGGTTG TGGCCCGGCG
 101  TGTTTTCCCA CGATTTGTGG AATCCTGACG AACCTGCCGT CTATACCGCC
 151  GTCGAAGCAC TGGCAGGCAG CCCCACCCCC TTGGTTGCCC ATCTGTTCGG
 201  TCAAACCGAT TTCGGCATAC CGCCCGTGTA TCTTTGGGTT GCCGCCGCGT
 251  TCAAACATTT GCTGTCGCCG TGGGCTGCCG ACTCATACGA TGCCGCACGC
 301  TTTGCAGGCG TATTTTTTGC CGTTATCGGA CTGACTTCCT GCGGCTTTGC
 351  CGGTTTCAAC TTTTTGGGCA GACACCACGG GCGCAgCGTC GTCCTGATTC
 401  TCATCGGCTG TATCGGGCTG ATTCCAGTTG CCCATTTCCT CAACCCCGCT
 451  GCCGCCGCCT TTGCCGCCGC CGGACTGGTG CTGCACGGTT ATTCTTTGGC
 501  TCGCCGGCGC GTGATTGCCG CCTCTTTTCT GCTCGGTACG GGCTGGACGC
 551  TGATGTCGTT GGCAGCAGCT TATCCGGCAG CATTTGCCCT GATGCTGCCC
 601  TTGCCCGTAC TGATGTTTTT CCGTCCGTGG CAAAGCAGGC GTTTGATGTT
 651  GACGGCAGTC GCCTCACTTG CCTTTGCCCT GCCGCTTATG ACCGTTTACC
 701  CGCTGCTCTT GGCAAAAACG CAGCCCGCGC TGTTCGCGCA ATGGCTCGAC
 751  TATCACGTTT TCGGTACGTT CGGCGGCGTG CGGCACGTTC AGACGGCATT
 801  CAGTTTGTTT TACTATCTGA AAAACCTGCT TTGGTTTGCA TTGCCCGCGC
 851  TGCCGCTGGC GGTTTGGACG GTTTGCCGCA CGCGCCTGTT TTCGACCGAC
 901  TGGGGGATTT TGGGCGTCGT CTGGATGCTT GCCGTTTTGG TGCTGCTTGC
 951  CGTCAATCCG CAGCGTTTTC AGGATAACCT CGTCTGGCTG CTTCCGCCGC
1001  TTGCCCTGTT CGGCGCGGCG CAACTGGACA GCCTGAGGCG CGGCGCGGCG
1051  GCGTTTGTCA ACTGGTTCGG CATTATGGCG TTCGGACTGT TTGCCGTGTT
1101  CCTGTGGACG GGCTTTTTCG CCATGAATTA CGGCTGGCCC GCCAAGCTTG
1151  CCGAACGCGC CGCCTATTTC AGCCCGTATT ATGTTCCTGA TATCGATCCC
1201  ATTCCGATGG CGGTTGCCGT ACTGTTCACA CCCTTGTGGC TGTGGGCGAT
1251  TACCCGGAAA AACATACGCG GCAGGCAGGC GGTTACCAAC TGGGCGGCAG
1301  GCGTTACCCT GACCTGGGCT TTGCTGATGA CGCTGTTCCT GCCGTGGCTG
1351  GACGCGGCGA AAAGCCACGC GCCGGTCGTC CGGAGTATGG AGGCATCGCT
1401  TTCCCCGGAA TTGAAACGGG AGCTTTCAGA CGGCATCGAG TGTATCGGCA
1451  TAGGCGGCGG CGACCTGCAC ACGCGGATTG TTTGGACGCA GTACGGCACA
1501  TTGCCGCACC GCGTCGGCGA TGTACAATGC CGCTACCGCA TCGTCCTCCT
1551  GCCCCAAAAT GCGGATGCGC CGCAAGGCTG GCAGACGGTT TGGCAGGGTG
1601  CGCGTCCGCG CAACAAAGAC AGTAAGTTCG CACTGATACG GAAAATCGGG
1651  GAAAATATAT AA
```

This corresponds to the amino acid sequence <SEQ ID 596; ORF141-1>:

```
  1  MLTYTPPDAR PPAKTHEKPW LLLLMAFAWL WPGVFSHDLW NPDEPAVYTA
 51  VEALAGSPTP LVAHLFGQTD FGIPPVYLWV AAAFKHLLSP WAADSYDAAR
101  FAGVFFAVIG LTSCGFAGFN FLGRHHGRSV VLILIGCIGL IPVAHFLNPA
151  AAAFAAAGLV LHGYSLARRR VIAASFLLGT GWTLMSLAAA YPAAFALMLP
201  LPVLMFFRPW QSRRLMLTAV ASLAFALPLM TVYPLLLAKT QPALFAQWLD
251  YHVFGTFGGV RHVQTAFSLF YYLKNLLWFA LPALPLAVWT VCRTRLFSTD
301  WGILGVVWML AVLVLLAVNP QRFQDNLVWL LPPLALFGAA QLDSLRRGAA
351  AFVNWFGIMA FGLFAVFLWT GFFAMNYGWP AKLAERAAYF SPYYVPDIDP
401  IPMAVAVLFT PLWLWAITRK NIRGRQAVTN WAAGVTLTWA LLMTLFLPWL
451  DAAKSHAPVV RSMEASLSPE LKRELSDGIE CIGIGGGDLH TRIVWTQYGT
501  LPHRVGDVQC RYRIVLLPQN ADAPQGWQTV WQGARPRNKD SKFALIRKIG
551  ENI*
```

Computer analysis of this amino acid sequence gave the following results:

Homology with a predicted ORF from *N.meningitidis* (strain A)

ORF141 shows 95.0% identity over a 140aa overlap with an ORF (ORF141a) from strain A of *N. meningitidis:*

```
                                                 10        20        30
orf141.pep                               DFGISPVYLWVAAAFKHLLSPWAADSYDVA
                                         |||| |||||||||||||||||||| ||:|
orf141a      WNPDEPAVYTAVEALAGSPTPLVAHLFGQIDFGIPPVYLWVAAAFKHLLSPWAADPYDAA
              40        50        60        70        80        90

                     40        50        60        70        80        90
orf141.pep   RFAGVFFAVIGLTSCGFAGFNFLGRHHGRXVVLILIGCIGLIPVAHFLNPAAAAFAAAGL
             ||||||||:||||||||||||||||||||| ||||||||||||::||||||||||||||||
orf141a      RFAGVFFAVVGLTSCGFAGFNFLGRHHGRSVVLILIGCIGLIPTVHFLNPAAAAFAAAGL
            100       110       120       130       140       150

                    100       110       120       130       140
orf141.pep   VLHGYSLARRRVIAASFLLGTGWTLMSLAAAYPAAFALMLPLPVLMFFRP
             ||||||||||||||||||||||||||||||||||||||||||||||||||
orf141a      VLHGYSLARRRVIAASFLLGTGWTLMSLAAAYPAAFALMLPLPVLMFFRPWQSRRLMLTA
            160       170       180       190       200       210

orf141a      VASLAFALPLMTVYPLLLAKTQPALFAQWLDDHVFGTFGGVRHIQTAFSLFYYLKNLLWF
            220       230       240       250       260       270
```

The complete length ORF141a nucleotide sequence <SEQ ID 597> is:

```
   1 ATGCTGACCT ATACCCCGCC CGATGCCCGC CCGCCCGCCA AAACCCACGA
  51 AAAGCCGTGG CTGTTGCTGT TGATGGCGTT TGCCTGGTTG TGGCCCGGCG
 101 TGTTTTCCCA CGATTTGTGG AATCCTGACG AACCTGCCGT CTATACCGCC
 151 GTCGAAGCAC TGGCAGGCAG CCCCACCCCT TTGGTTGCCC ATCTGTTCGG
 201 TCAAATCGAT TTCGGCATAC CGCCCGTGTA TCTTTGGGTT GCCGCCGCGT
 251 TCAAACATTT GCTGTCGCCG TGGGCTGCCG ACCCGTATGA TGCCGCACGC
 301 TTTGCCGGCG TGTTTTTCGC CGTTGTCGGA CTGACTTCCT GCGGCTTTGC
 351 CGGTTTCAAC TTTTTGGGCA GACACCACGG GCGCAGCGTC GTCCTGATTC
 401 TCATCGGCTG TATCGGGCTG ATTCCGACCG TACACTTTCT CAACCCCGCT
 451 GCCGCCGCCT TTGCCGCCGC CGGACTGGTG CTGCACGGTT ATTCTTTGGC
 501 TCGCCGGCGC GTGATTGCCG CCTCTTTTCT GCTCGGTACG GGTTGGACGC
 551 TGATGTCGTT GGCAGCAGCT TATCCGGCGG CATTTGCCCT GATGCTGCCC
 601 CTGCCCGTGC TGATGTTTTT CCGTCCGTGG CAAAGCAGGC GTTTGATGTT
 651 GACGGCAGTC GCCTCGCTTG CCTTTGCCCT GCCGCTTATG ACCGTTTACC
 701 CGCTGCTCTT GGCAAAAACG CAGCCCGCGC TGTTCGCGCA ATGGCTCGAC
 751 GATCACGTTT TCGGTACGTT CGGCGGCGTG CGGCACATTC AGACGGCATT
 801 CAGTTTGTTT TACTATCTGA AAAACCTGCT TTGGTTTGCA TTGCCTGCGC
 851 TGCCGCTGGC GGTTTGGACG GTTTGCCGCA CGCGCCTGTT TTCGACCGAC
 901 TGGGGGATTT TGGGCGTCGT CTGGATGCTT GCCGTTTTGG TGCTGCTTGC
 951 CGTCAATCCG CAGCGTTTTC AGGATAACCT CGTCTGGCTG CTTCCGCCGC
1001 TTGCCCTGTT CGGCGCGGCG CAACTGGACA GCCTGAGACG CGGCGCGGCG
1051 GCGTTTGTCA ACTGGTTCGG CATTATGGCG TTCGGACTGT TTGCCGTGTT
1101 CCTGTGGACG GGCTTTTTCG CCATGAATTA CGGCTGGCCC GCCAAGCTTG
1151 CCGAACGCGC CGCCTATTTC AGCCCGTATT ATGTTCCTGA TATCGATCCC
1201 ATTCCGATGG CGGTTGCCGT ACTGTTCACA CCCTTGTGGC TGTGGGCGAT
1251 TACCCGCAAA AACATACGCG GCAGGCAGGC GGTTACCAAC TGGGCGGCAG
1301 GCGTTACCCT GACCTGGGCT TTGCTGATGA CGCTGTTCCT GCCGTGGCTG
1351 GACGCGGCGA AAAGCCACGC GCCCGTCGTC CGGAGTATGG AGGCATCGCT
1401 TTCCCCGGAA TTAAAACGGG AGCTTTCAGA CGGCATCGAG TGTATCGACA
1451 TAGGCGGCGG CGACCTACAC ACGCGGATTG TTTGGACGCA GTACGGCACA
1501 TTGCCGCACC GCGTCGGCGA TGTACAATGC CGCTACCGCA TCGTCCGCTT
1551 GCCCCAAAAC GCGGATGCGC CGCAAGGCTG GCAGACGGTC TGGCAGGGTG
1601 CGCGCCCGCG CAACAAAGAC AGTAAGTTCG CACTGATACG GAAAACCGGG
1651 GAAAATATAT TAAAAACAAC AGATTGA
```

This encodes a protein having amino acid sequence <SEQ ID 598>:

```
  1 MLTYTPPDAR PPAKTHEKPW LLLLMAFAWL WPGVFSHDLW NPDEPAVYTA
 51 VEALAGSPTP LVAHLFGQID FGIPPVYLWV AAAFKHLLSP WAADPYDAAR
101 FAGVFFAVVG LTSCGFAGFN FLGRHHGRSV VLILIGCIGL IPTVHFLNPA
151 AAAFAAAGLV LHGYSLARRR VIAASFLLGT GWTLMSLAAA YPAAFALMLP
201 LPVLMFFRPW QSRRLMLTAV ASLAFALPLM TVYPLLLAKT QPALFAQWLD

251 DHVFGTFGGV RHIQTAFSLF YYLKNLLWFA LPALPLAVWT VCRTRLFSTD
301 WGILGVVWML AVLVLLAVNP QRFQDNLVWL LPPLALFGAA QLDSLRRGAA
351 AFVNWFGIMA FGLFAVFLWT GFFAMNYGWP AKLAERAAYF SPYYVPDIDP
401 IPMAVAVLFT PLWLWAITRK NIRGRQAVTN WAAGVTLTWA LLMTLFLPWL
451 DAAKSHAPVV RSMEASLSPE LKRELSDGIE CIDIGGGDLH TRIVWTQYGT
501 LPHRVGDVQC RYRIVRLPQN ADAPQGWQTV WQGARPRNKD SKFALIRKTG
551 ENILKTTD*
```

ORF141a and ORF141-1 show 98.2% identity in 553 aa overlap:

```
orf141a.pep    MLTYTPPDARPPAKTHEKPWLLLLMAFAWLWPGVFSHDLWNPDEPAVYTAVEALAGSPTP
               ||||||||||||||||||||||||||||||||||||||||||||||||||||||||||||
orf141-1       MLTYTPPDARPPAKTHEKPWLLLLMAFAWLWPGVFSHDLWNPDEPAVYTAVEALAGSPTP

orf141a.pep    LVAHLFGQIDFGIPPVYLWVAAAFKHLLSPWAADPYDAARFAGVFFAVVGLTSCGFAGFN
               |||||||| ||||||||||||||||||||||||| |||||||||||||:|||||||||||
orf141-1       LVAHLFGQTDFGIPPVYLWVAAAFKHLLSPWAADSYDAARFAGVFFAVIGLTSCGFAGFN

orf141a.pep    FLGRHHGRSVVLILIGCIGLIPTVHFLNPAAAAFAAAGLVLHGYSLARRRVIAASFLLGT
               ||||||||||||||||||||||::||||||||||||||||||||||||||||||||||||
orf141-1       FLGRHHGRSVVLILIGCIGLIPVAHFLNPAAAAFAAAGLVLHGYSLARRRVIAASFLLGT

orf141a.pep    GWTLMSLAAAYPAAFALMLPLPVLMFFRPWQSRRLMLTAVASLAFALPLMTVYPLLLAKT
               ||||||||||||||||||||||||||||||||||||||||||||||||||||||||||||
orf141-1       GWTLMSLAAAYPAAFALMLPLPVLMFFRPWQSRRLMLTAVASLAFALPLMTVYPLLLAKT

orf141a.pep    QPALFAQWLDDHVFGTFGGVRHIQTAFSLFYYLKNLLWFALPALPLAVWTVCRTRLFSTD
               |||||||||| ||||||||||:||||||||||||||||||||||||||||||||||||||
orf141-1       QPALFAQWLDYHVFGTFGGVRHVQTAFSLFYYLKNLLWFALPALPLAVWTVCRTRLFSTD

orf141a.pep    WGILGVVWMLAVLVLLAVNPQRFQDNLVWLLPPLALFGAAQLDSLRRGAAAFVNWFGIMA
               ||||||||||||||||||||||||||||||||||||||||||||||||||||||||||||
orf141-1       WGILGVVWMLAVLVLLAVNPQRFQDNLVWLLPPLALFGAAQLDSLRRGAAAFVNWFGIMA

orf141a.pep    FGLFAVFLWTGFFAMNYGWPAKLAERAAYFSPYYVPDIDPIPMAVAVLFTPLWLWAITRK
               ||||||||||||||||||||||||||||||||||||||||||||||||||||||||||||
orf141-1       FGLFAVFLWTGFFAMNYGWPAKLAERAAYFSPYYVPDIDPIPMAVAVLFTPLWLWAITRK

orf141a.pep    NIRGRQAVTNWAAGVTLTWALLMTLFLPWLDAAKSHAPVVRSMEASLSPELKRELSDGIE
               ||||||||||||||||||||||||||||||||||||||||||||||||||||||||||||
orf141-1       NIRGRQAVTNWAAGVTLTWALLMTLFLPWLDAAKSHAPVVRSMEASLSPELKRELSDGIE

orf141a.pep    CIDIGGGDLHTRIVWTQYGTLPHRVGDVQCRYRIVRLPQNADAPQGWQTVWQGARPRNKD
               || |||||||||||||||||||||||||||||||| ||||||||||||||||||||||||
orf141-1       CIGIGGGDLHTRIVWTQYGTLPHRVGDVQCRYRIVLLPQNADAPQGWQTVWQGARPRNKD

orf141a.pep    SKFALIRKTGENI
               |||||||| ||||
orf141-1       SKFALIRKIGENI
```

Homology with a predicted ORF from *N.gonorrhoeae*

ORF141 shows 95% identity over a 140aa overlap with a predicted ORF (ORF141ng) from *N.gonorrhoeae:*

```
orf141.pep                                 DFGISPVYLWVAAAFKHLLSPWAADSYDVA   30
                                           |||| ||||||||||||||||||   ||:|
orf141ng    WNPAEPAVYTAVEALAGSPTPLVAHLFGQTDFGIPPVYLWVAAAFKHLLSPWAAHPYDAA   126

orf141.pep  RFAGVFFAVIGLTSCGFAGFNFLGRHHGRXVVLILIGCIGLIPVAHFLNPAAAAFAAAGL   90
            ||||||||||||||||||||||||||||| |||| ||||||||||||:|||||||||||
orf141ng    RFAGVFFAVIGLTSCGFAGFNFLGRHHGRSVVLIHIGCIGLIPVAHFFNPAAAAFAAAGL   186

orf141.pep  VLHGYSLARRRVIAASFLLGTGWTLMSLAAAYPAAFALMLPLPVLMFFRP           140
            ||||||||||||||||||||||||||||||||||||||||||||||||||
orf141ng    VLHGYSLARRRVIAASFLLGTGWTLMSLAAAYPAAFALMLPLPVLMFFRPWQSRRLMLTA   246
```

An ORF141ng nucleotide sequence <SEQ ID 599> was predicted to encode a protein having amino acid sequence <SEQ ID 600>:

```
  1 MPSEAVSARP LCEYLLHLAI RPFLLTLMLT YTPPDARPPA KTHEKPWLLL
 51 LMAFAWLWPG VFSHDLWNPA EPAVYTAVEA LAGSPTPLVA HLFGQTDFGI
101 PPVYLWVAAA FKHLLSPWAA HPYDAARFAG VFFAVIGLTS CGFAGFNFLG
151 RHHGRSVVLI HIGCIGLIPV AHFFNPAAAA FAAAGLVLHG YSLARRRVIA
201 ASFLLGTGWT LMSLAAAYPA AFALMLPLPV LMFFRPWQSR RLMLTAVASL
251 AFALPLMTVY PLLLAKTQPA LFAQWLNYHV FGTFGGVRHI QRAFSLFHYL
301 KNLLWFAPPG LPLAVWTVCR TRLFSTDWGI LGIVWMLAVL VLLAFNPQRF
351 QDNLVWLLPP LALFGAAQLD SLRRGAAAFV NWFGIMAFGL FAVFLWTGFF
401 AMNYGWPAKL AERAAYFSPY YVPDIDPIPM AVAVLFTPLW LWAITRKNIR
451 GRQAVTNWAA GVTLTWALLM TLFLPWLDAA KSHAPVVRSM EASFSPELKR
501 ELSDGIECIG IGGGDLHTRI VWTQYGTLPH RVGDVRCRYR IVRLPQNADA
551 PQGWQTVWQG ARPRNKDSKF ALIRKIGENI LKTTD*
```

Further work revealed the following gonococcal DNA sequence <SEQ ID 601>:

```
   1 ATGCTGACCT ATACCCCGCC CGATGCCCGC CCGCCCGCCA AAACCCACGA
  51 AAAACCGTGG CTGCTGCTGT TGATGGCGTT TGCCTGGCTG TGGCCCGGCG
 101 TGTTTTCCCA CGATTTGTGG AATCCTGCCG AACCTGCCGT CTATACCGCC
 151 GTCGAAGCAC TGGCAGGCAG CCCCACCCCC TTGGTTGCCC ATCTGTTCGG
 201 TCAAACCGAT TTCGGCATAC CGCCCGTGTA TCTTTGGGTT GCCGCCGCAT
 251 TCAAACATTT GCTGTCGCCG TGGGCAGCCG ACCCGTATGA TGCCGCACGC
 301 TTTGCAGGCG TATTTTTTGC CGTTATCGGA CTGACTTCTT GCGGCTTTGC
 351 CGGTTTCAAC TTTTTGGGCA GACACCACGG GCGCAGCGTT GTTTTAATCC
 401 ATATCGGCTG TATCGGGCTG ATTCCGGTTG CCCATTTCCT CAATCCcgcc
 451 gccgccgcct tTGCCGCCGC CGGACTGGTG CTGCacggct actcgctgGC
 501 ACGCCGGCGC GTGATtgccg cctctTtccT GCTCGGTACG GGTTGGACGT
 551 TGATGTCGCT GGCGGCAGCT TATCCGGCGG CGTTTGCGCT GATGCTGCCC
 601 CTGCCCGTGC TGATGTTTTT CCGTCCGTGG CAAAGCAGGC GTTTGATGTT
 651 GACGGCAGTC GCCTCGCTTG CCTTTGCCCT GCCGCTTATG ACCGTTTACC
 701 CGCTGCTCTt gGCAAAAACG CAGCCCGCGC TGTTTGCGCA ATGGCTCAAC
 751 TATCACGTTT TCGGTACGTt cggcgGCGTG CGGCAcaTTC AGAggGCatT
 801 Cagtttgttt cactatctgA AAatctgct ttggttcgca ccgcccgggC
 851 TGCCGCTGGC GGTTTGGACG GTTTGCCGCA CACGCCTGTT TTCGACCGAC
 901 TGGGGGATTT TGGGCATTGT CTGGATGCTT GCCGTTTTGG TGCTGCTCGC
 951 CTTTAATCCG CAGCGTTTTC AAGACAACCT CGTCTGGCTG CTGCCGCCGC
1001 TTGCCCTGTT CGGCGCGGCG CAACTGGACA GCCTGAGGCG CGGCGCGGCG
1051 GCTTTTGTCA ACTGGTTCGG CATTATGGCG TTCGGGCTGT TTGCCGTGTT
1101 CCTGTGGACG GGCTTTTTCG CCATGAATTA CGGCTGGCCC GCCAAGCTTG
1151 CCGAACGCGC CGCCTACTTC AGCCCGTATT ACGTTCCCGA CATCGATCCC
1201 ATTCCGATGG CGGTTGCCGT ACTGTTCACA CCCTTGTGGC TGTGGGCGAT
1251 TACCCGGAAA AACATACGCG GCAGGCAGGC GGTTACCAAC TGGGCGGCAG
1301 GCGTTACCCT GACCTGGGCT TTGCTGATGA CGCTGTTCCT GCCGTGGCTG
1351 GACGCGGCGA AAGCCACGC GCCCGTCGTC CGGAGTATGG AGGCATCGTT
1401 TTCCCCGGAA TTAAAACGGG AGCTTTCAGA CGGCATCGAG TGTATCGGCA
1451 TAGGCGGCGG CGACCTGCAC ACGCGGATTG TTTGGACGCA GTACGGCACA
1501 TTGCCGCACC GCGTCGGCGA TGTCCGTTGC CGCTACCGTA TCGTCCGCCT
1551 GCCCCAAAAC GCGGATGCGC CGCAAGGCTG GCAGACGGTC TGGCAGGGTG
1601 CGCGCCCGCG CAACAAAGAC AGTAAGTTTG CACTGATACG GAAAATCGGG
1651 GAAAATATAT TAAAAACAAC AGATTGA
```

This corresponds to the amino acid sequence <SEQ ID 602; ORF141ng-1>:

```
  1 MLTYTPPDAR PPAKTHEKPW LLLLMAFAWL WPGVFSHDLW NPAEPAVYTA
 51 VEALAGSPTP LVAHLFGQTD FGIPPVYLWV AAAFKHLLSP WAADPYDAAR
101 FAGVFFAVIG LTSCGFAGFN FLGRHHGRSV VLIHIGCIGL IPVAHFLNPA
151 AAAFAAAGLV LHGYSLARRR VIAASFLLGT GWTLMSLAAA YPAAFALMLP
201 LPVLMFFRPW QSRRLMLTAV ASLAFALPLM TVYPLLLAKT QPALFAQWLN
251 YHVFGTFGGV RHIQRAFSLF HYLKNLLWFA PPGLPLAVWT VCRTRLFSTD
301 WGILGIVWML AVLVLLAFNP QRFQDNLVWL LPPLALFGAA QLDSLRRGAA
351 AFVNWFGIMA FGLFAVFLWT GFFAMNYGWP AKLAERAAYF SPYYVPDIDP
401 IPMAVAVLFT PLWLWAITRK NIRGRQAVTN WAAGVTLTWA LLMTLFLPWL
451 DAAKSHAPVV RSMEASFSPE LKRELSDGIE CIGIGGGDLH TRIVWTQYGT
501 LPHRVGDVRC RYRIVRLPQN ADAPQGWQTV WQGARPRNKD SKFALIRKIG
551 ENILKTTD*
```

ORF141ng-l and ORF141-1 show 97.5% identity in 553 aa overlap:

```
orf141ng-1.pep MLTYTPPDARPPAKTHEKPWLLLLMAFAWLWPGVFSHDLWNPAEPAVYTAVEALAGSPTP
               |||||||||||||||||||||||||||||||||||||||||| ||||||||||||||||
orf141-1       MLTYTPPDARPPAKTHEKPWLLLLMAFAWLWPGVFSHDLWNPDEPAVYTAVEALAGSPTP

orf141ng-1.pep LVAHLFGQTDFGIPPVYLWVAAAFKHLLSPWAADPYDAARFAGVFFAVIGLTSCGFAGFN
               |||||||||||||||||||||||||||||||||| |||||||||||||||||||||||||
orf141-1       LVAHLFGQTDFGIPPVYLWVAAAFKHLLSPWAADSYDAARFAGVFFAVIGLTSCGFAGFN

orf141ng-1.pep FLGRHHGRSVVLIHIGCIGLIPVAHFLNPAAAAFAAAGLVLHGYSLARRRVIAASFLLGT
               ||||||||||||| ||||||||||||||||||||||||||||||||||||||||||||||
orf141-1       FLGRHHGRSVVLILIGCIGLIPVAHFLNPAAAAFAAAGLVLHGYSLARRRVIAASFLLGT

orf141ng-1.pep GWTLMSLAAAYPAAFALMLPLPVLMFFRPWQSRRLMLTAVASLAFALPLMTVYPLLLAKT
               ||||||||||||||||||||||||||||||||||||||||||||||||||||||||||||
orf141-1       GWTLMSLAAAYPAAFALMLPLPVLMFFRPWQSRRLMLTAVASLAFALPLMTVYPLLLAKT

orf141ng-1.pep QPALFAQWLNYHVFGTFGGVRHIQRAFSLFHYLKNLLWFAPPGLPLAVWTVCRTRLFSTD
               |||||||||:||||||||||||:| |||||:||||||||| |:||||||||||||||||
orf141-1       QPALFAQWLDYHVFGTFGGVRHVQTAFSLFYYLKNLLWFALPALPLAVWTVCRTRLFSTD

orf141ng-1.pep WGILGIVWMLAVLVLLAFNPQRFQDNLVWLLPPLALFGAAQLDSLRRGAAAFVNWFGIMA
               |||||:||||||||||| ||||||||||||||||||||||||||||||||||||||||||
orf141-1       WGILGVVWMLAVLVLLAVNPQRFQDNLVWLLPPLALFGAAQLDSLRRGAAAFVNWFGIMA

orf141ng-1.pep FGLFAVFLWTGFFAMNYGWPAKLAERAAYFSPYYVPDIDPIPMAVAVLFTPLWLWAITRK
               ||||||||||||||||||||||||||||||||||||||||||||||||||||||||||||
orf141-1       FGLFAVFLWTGFFAMNYGWPAKLAERAAYFSPYYVPDIDPIPMAVAVLFTPLWLWAITRK

orf141ng-1.pep NIRGRQAVTNWAAGVTLTWALLMTLFLPWLDAAKSHAPVVRSMEASFSPELKRELSDGIE
               ||||||||||||||||||||||||||||||||||||||||||||||:|||||||||||||
orf141-1       NIRGRQAVTNWAAGVTLTWALLMTLFLPWLDAAKSHAPVVRSMEASLSPELKRELSDGIE

orf141ng-1.pep CIGIGGGDLHTRIVWTQYGTLPHRVGDVRCRYRIVRLPQNADAPQGWQTVWQGARPRNKD
               |||||||||||||||||||||||||||:|||||| |||||||||||||||||||||||||
orf141-1       CIGIGGGDLHTRIVWTQYGTLPHRVGDVQCRYRIVLLPQNADAPQGWQTVWQGARPRNKD

orf141ng-1.pep SKFALIRKIGENILKTTDX
               |||||||||||||
orf141-1       SKFALIRKIGENIX
```

Based on the presence of several putative transmembrane domains in the gonococcal protein, it is predicted that the proteins from *N.meningitidis* and *N.gonorrhoeae,* and their epitopes, could be useful antigens for vaccines or diagnostics, or for raising antibodies.

**Example 72**

The following partial DNA sequence was identified in *N.meningitidis* <SEQ ID 603>:

```
  1 ..CAATCCGCCA AATGGTTATC GGGCCAAACT CTAGTCGGCA CAGCAATTGG
 51   GATACGCGGG CAGATAAAGC TTGGCGGCAA CCTGCATTAC GATATATTTA
101   CCGGCCGCGC ATTGAAAAAG CCCGAATTTT TCCAATCAAG GAAATGGGCA
151   AGCGGTTTTC AGGTAGGCTA TACGTTTTAA
```

This corresponds to the amino acid sequence <SEQ ID 604; ORF142>:

```
  1 ..QSAKWLSGQT LVGTAIGIRG QIKLGGNLHY DIFTGRALKK PEFFQSRKWA
 51   SGFQVGYTF*
```

Further work revealed the complete nucleotide sequence <SEQ ID 605>:

```
   1 ATGGATAATT CGGGTAGTGA GGCGACAGGA AAATACCAAG GAAATATCAC
  51 TTTCTCTGCC GACAATCCTT TGGGACTGAG TGATATGTTC TATGTAAATT
 101 ATGGACGTTC GATTGGCGGT ACGCCCGATG AGGAAAGTTT TGACGGCCAT
 151 CGCAAAGAAG GCGGATCAAA CAATTACGCC GTACATTATT CAGCCCCTTT
 201 CGGTAAATGG ACATGGGCAT TCAATCACAA TGGCTACCGT TACCATCAGG
 251 CAGTTTCCGG ATTATCGGAA GTCTATGACT ATAATGGAAA AAGTTACAAT
 301 ACTGATTTCG GCTTCAACCG CCTGTTGTAT CGTGATGCCA AACGCAAAAC
 351 CTATCTCGGT GTAAAACTGT GGATGAGGGA AACAAAAAGT TACATTGATG
 401 ATGCCGAACT GACTGTACAA CGGCGTAAAA CTGCGGGTTG GTTGGCAGAA
 451 CTTTCCCACA AAGAATATAT CGGTCGCAGT ACGGCAGATT TTAAGTTGAA
 501 ATATAAACGC GGCACCGGCA TGAAAGATGC TCTGCGCGCG CCTGAAGAAG
 551 CCTTTGGCGA AGGCACGTCA CGTATGAAAA TTTGGACGGC ATCGGCTGAT
 601 GTAAATACTC CTTTTCAAAT CGGTAAACAG CTATTTGCCT ATGACACATC
 651 CGTTCATGCA CAATGGAACA AAACCCCGCT AACATCGCAA GACAAACTGG
 701 CTATCGGCGG ACACCACACC GTACGTGGCT TCGACGGTGA AATGAGTTTG
 751 TCTGCCGAGC GGGGATGGTA TTGGCGCAAC GATTTGAGCT GGCAATTTAA
 801 ACCAGGCCAT CAGCTTTATC TTGGGGCTGA TGTAGGACAT GTTTCAGGAC
 851 AATCCGCCAA ATGGTTATCG GGCCAAACTC TAGTCGGCAC AGCAATTGGG
 901 ATACGCGGGC AGATAAAGCT TGGCGGCAAC CTGCATTACG ATATATTTAC
 951 CGGCCGCGCA TTGAAAAAGC CCGAATTTTT CCAATCAAGG AAATGGGCAA
1001 GCGGTTTTCA GGTAGGCTAT ACGTTTTAA
```

This corresponds to the amino acid sequence <SEQ ID 606; ORF142-1>:

```
  1 MDNSGSEATG KYQGNITFSA DNPLGLSDMF YVNYGRSIGG TPDEESFDGH
 51 RKEGGSNNYA VHYSAPFGKW TWAFNHNGYR YHQAVSGLSE VYDYNGKSYN
101 TDFGFNRLLY RDAKRKTYLG VKLWMRETKS YIDDAELTVQ RRKTAGWLAE
151 LSHKEYIGRS TADFKLKYKR GTGMKDALRA PEEAFGEGTS RMKIWTASAD
201 VNTPFQIGKQ LFAYDTSVHA QWNKTPLTSQ DKLAIGGHHT VRGFDGEMSL
251 SAERGWYWRN DLSWQFKPGH QLYLGADVGH VSGQSAKWLS GQTLVGTAIG
301 IRGQIKLGGN LHYDIFTGRA LKKPEFFQSR KWASGFQVGY TF*
```

Computer analysis of this amino acid sequence gave the following results:

Homology with a predicted ORF from *N.gonorrhoeae*

ORF142 shows 88.1% identity over a 59aa overlap with a predicted ORF (ORF142ng) from *N.gonorrhoeae:*

```
orf142.pep                                        QSAKWLSGQTLVGTAIGIRGQIKLGGNLHY    30
                                                  ||||||||||:|||||||||||||||||||
orf142ng      RGWYWRNDLSWQFKPGHQLYLGADVGHVSGQSAKWLSGQTLAGTAIGIRGQIKLGGNLHY   313

orf142.pep    DIFTGRALKKPEFFQSRKWASGFQVGYTF    59
              ||||||||||||:||::||::||||||:|
orf142ng      DIFTGRALKKPEYFQTKKWVTGFQVGYSF   342
```

The complete length ORF142ng nucleotide sequence <SEQ ID 607> is:

```
   1  ATGGATAATT CGGGTAGTGA GGCGACAGGA AAATACCAAG GAAATATCAC
  51  TTTCTCTGCC GACAATCCTT TTGGACTGAG TGATATGTTC TATGTAAATT
 101  ATGGACGTTC AATTGGCGGT ACGCCCGATG AGGAAAATTT TGACGGCCAT
 151  CGCAAAGAAG GCGGATCAAA CAATTACGCC GTACATTATT CAGCCCCTTT
 201  CGGTAAATGG ACATGGGCAT TCAATCACAA TGGCTACCGT TACCATCAGG
 251  CGGTTTCCGG ATTATCGGAA GTCTATGACT ATAATGGAAA AAGTTACAAC
 301  ACTGATTTCG GCTTCAACCG CCTGTTGTAT CGTGATGCCA AACGCAAAAC
 351  CTATCTCAGT GTAAAACTGT GGACGAGGGA AACAAAAAGT TACATTGATG
 401  ATGCCGAACT GACTGTACAA CGGCGTAAAA CCACAGGTTG GTTGGCAGAA
 451  CTTTCCCACA AAGGATATAT CGGTCGCAGT ACGGCAGATT TTAAGTTGAA
 501  ATATAAACAC GGCACCGGCA TGAAAGATGC TCTGCGCGCG CCTGAAGAAG
 551  CCTTTGGCGA AGGCACGTCA CGTATGAAAA TTTGGACGGC ATCGGCTGAT
 601  GTAAATACTC CTTTTCAAAT CGGTAAACAG CTATTTGCCT ATGACACATC
 651  CGTTCATGCA CAATGGAACA AAACCCCGCT AACATCGCAA GACAAACTGG
 701  CTATCGGCGG ACACCACACC GTACGTGGCT TCGACGGTGA AATGAGTTTG
 751  CCTGCCGAGC GGGGATGGTA TTGGCGCAAC GATTTGAGCT GGCAATTTAA
 801  ACCAGGCCAT CAGCTTTATC TTGGGGCTGA TGTAGGACAT GTTTCAGGAC
 851  AATCCGCCAA ATGGTTATCG GGCCAAACTC TAGCCGGCAC AGCAATTGGG
 901  ATACGCGGGC AGATAAAGCT TGGCGGCAAC CTGCATTACG ATATATTTAC
 951  CGGCCGTGCA TTGAAAAAGC CCGAATATTT TCAGACGAAG AAATGGGTAA
1001  CGGGGTTTCA GGTGGGTTAT TCGTTTTGA
```

This encodes a protein having amino acid sequence <SEQ ID 608>:

```
  1  MDNSGSEATG KYQGNITFSA DNPFGLSDMF YVNYGRSIGG TPDEENFDGH
 51  RKEGGSNNYA VHYSAPFGKW TWAFNHNGYR YHQAVSGLSE VYDYNGKSYN
101  TDFGFNRLLY RDAKRKTYLS VKLWTRETKS YIDDAELTVQ RRKTTGWLAE
151  LSHKGYIGRS TADFKLKYKH GTGMKDALRA PEEAFGEGTS RMKIWTASAD
201  VNTPFQIGKQ LFAYDTSVHA QWNKTPLTSQ DKLAIGGHHT VRGFDGEMSL
251  PAERGWYWRN DLSWQFKPGH QLYLGADVGH VSGQSAKWLS GQTLAGTAIG
301  IRGQIKLGGN LHYDIFTGRA LKKPEYFQTK KWVTGFQVGY SF*
```

The underlined sequence (aromatic-Xaa-aromatic amino acid motif) is usually found at the C-terminal end of outer membrane proteins.

ORF142ng and ORF142-1 show 95.6% identity over 342aa overlap:

```
orf142-1.pep MDNSGSEATGKYQGNITFSADNPLGLSDMFYVNYGRSIGGTPDEESFDGHRKEGGSNNYA
             ||||||||||||||||||||||||:||||||||||||||||||||||:||||||||||||||
orf142ng-1   MDNSGSEATGKYQGNITFSADNPFGLSDMFYVNYGRSIGGTPDEENFDGHRKEGGSNNYA

orf142-1.pep VHYSAPFGKWTWAFNHNGYRYHQAVSGLSEVYDYNGKSYNTDFGFNRLLYRDAKRKTYLG
             |||||||||||||||||||||||||||||||||||||||||||||||||||||||||||:
orf142ng-1   VHYSAPFGKWTWAFNHNGYRYHQAVSGLSEVYDYNGKSYNTDFGFNRLLYRDAKRKTYLS

orf142-1.pep VKLWMRETKSYIDDAELTVQRRKTAGWLAELSHKEYIGRSTADFKLKYKRGTGMKDALRA
             |||| |||||||||||||||||||:||||||||| |||||||||||||:||||||||||
orf142ng-1   VKLWTRETKSYIDDAELTVQRRKTTGWLAELSHKGYIGRSTADFKLKYKHGTGMKDALRA

orf142-1.pep PEEAFGEGTSRMKIWTASADVNTPFQIGKQLFAYDTSVHAQWNKTPLTSQDKLAIGGHHT
             ||||||||||||||||||||||||||||||||||||||||||||||||||||||||||||
orf142ng-1   PEEAFGEGTSRMKIWTASADVNTPFQIGKQLFAYDTSVHAQWNKTPLTSQDKLAIGGHHT

orf142-1.pep VRGFDGEMSLSAERGWYWRNDLSWQFKPGHQLYLGADVGHVSGQSAKWLSGQTLVGTAIG
             |||||||||| ||||||||||||||||||||||||||||||||||||||||||||:|||||
orf142ng-1   VRGFDGEMSLPAERGWYWRNDLSWQFKPGHQLYLGADVGHVSGQSAKWLSGQTLAGTAIG

orf142-1.pep IRGQIKLGGNLHYDIFTGRALKKPEFFQSRKWASGFQVGYTF
             |||||||||||||||||||||||||:||::||::||||||:|
orf142ng-1   IRGQIKLGGNLHYDIFTGRALKKPEYFQTKKWVTGFQVGYSF
```

In addition, ORF142ng is homologous to the HecB protein of *E.chrysanthemi:*

```
gi|1772622 (L39897) HecB [Erwinia chrysanthemi] Length = 558
Score =  119 bits (295), Expect = 3e-26
Identities = 88/346 (25%), Positives = 151/346 (43%), Gaps = 22/346 (6%)

Query: 2   DNSGSEATGKYQGNITFSADNPFGLSDMFYVNYGRSIGGTPDEENFDGHRKEGGSNNYAV 61
           DNSG ++TG+ Q N + + DN FGL+D ++++ G S    +    + D    + G
Sbjct: 230 DNSGQKSTGEEQLNGSLALDNVFGLADQWFISAGHS---SRFATSHDAESLQAG------ 280

Query: 62  HYSAPFGKWTWAFNHNGYRYHQAVSGLSEVYDYNGKSYNTDFGFNRLLYRDAKRKTYLSV 121
            +S P+G W   +N++   RY            +   G S     F  +R+++RD   KT ++
Sbjct: 281 -FSMPYGYWNLGYNYSQSRYRNTFINRDFPWHSTGDSDTHRFSLSRVVFRDGTMKTAIAG 339

Query: 122 KLWTRETKSYIDDAELTVQRRKTTGWLAELSHKGYIGRSTADFKLKYKHGTGMKDALRAP 181
               R    +Y++ + L      RK +     ++H     +      A F    Y  G         +
Sbjct: 340 TFSQRTGNNYLNGSLLPSSSRKLSSVSLGVNHSQKLWGGLATFNPTYNRGVRWLGSETDT 399

Query: 182 EEAFGEGTSRMKIWTASADVNTPFQIGKQLFAYDTSVHAQWNKTPLTSQDKLAIGGHHTV 241
           +++   E   +    WT SA    P           Y  S++ Q++    L    ++L +GG  ++
Sbjct: 400 DKSADEPRAEFNKWTLSASYYHPV---TDSITYLGSLYGQYSARALYGSEQLTLGGESSI 456

Query: 242 RGFDGEMSLPAERGWYWRNDLSWQFKP----GHQLYLGA-DVGHVSGQSAKWLSGQTLAG 296
           RGF   E       RG YWRN+L+WQ          G+  ++ A D GH+          +  +L G
Sbjct: 457 RGF-REQYTSGNRGAYWRNELNWQAWQLPVLGNVTFMAAVDGGHLYNHKQDNSTAASLWG 515

Query: 297 TAIGIRGQIKLGGNLHYDIFTGRALKKPEYFQTKKWVTGFQVGYSF 342
            A+G+     +     L    +  G   +  P + Q     V G++VG SF
Sbjct: 516 GAVGMTVASRW---LSQQVTVGWPISYPAWLQPDTMVVGYRVGLSF 558
```

On the basis of this analysis, it is predicted that the proteins from *N.meningitidis* and *N.gonorrhoeae,* and their epitopes, could be useful antigens for vaccines or diagnostics, or for raising antibodies.

**Example 73**

The following partial DNA sequence was identified in *N.meningitidis* <SEQ ID 609>:

```
  1  ATGCGGACGA AATGGTCAGC AGTGAGAAGC TGCTTACTTG GgCGGACACC
 51  GCCGACATCG ATACCGCTTT GAACCTGTTG TACCGTTTGC AAAAACTCGA
101  ATTCCTCTAT GGCGATGAAA ACGGTCATTC AGACGGCATC AATTTGwCGG
151  ACGAGCAATT GCCGTTGCTG ATGGAACAAT TGTCCGGCAG CGGTAAGGCG
201  TTATTGGTCG ATCGGAACGG TCTGTATCTT GCCAACGCCA ATTTCCATCA
251  TGAGGCGGCG GAAGAGTTGG GGTTGTTGGC GGCAGAAGTC GCACAGATGG
301  AAAAGAAATA CCGGCTGCTG ATTAAGAACA AC..
```

This corresponds to the amino acid sequence <SEQ ID 610; ORF143>:

```
  1  MRTKWSAVRS CTWADTADID TALNLLYRLQ KLEFLYGDEN GHSDGINLXD
 51  EQLPLLMEQL SGSGKALLVD RNGLYLANAN FHHEAAEELG LLAAEVAQME
101  KKYRLLIKNN ..
```

Further work revealed the complete nucleotide sequence <SEQ ID 611>:

```
  1  ATGGAATCAA CACTTTCACT ACAAGCAAAT TTATATCCCC GCCTGACTCC
 51  TGCCGGTGCA TTTTATGCCG TATCCAGCGA TGCCCCCAGT GCCGGTAAAA
101  CTTTGTTGCA CAGCCTGTTG AAAGCAGATG CGGACGAAAT GGTCAGCAGT
151  GAGAAGCTGC TTACTTGGGC GGACACCGCC GACATCGATA CCGCTTTGAA
201  CCTGTTGTAC CGTTTGCAAA AACTCGAATT CCTCTATGGC GATGAAAACG
251  GTCATTCAGA CGGCATCAAT TTGTCGGACG AGCAATTGCC GTTGCTGATG
301  GAACAATTGT CCGGCAGCGG TAAGGCGTTA TTGGTCGATC GGAACGGTCT
351  GTATCTTGCC AACGCCAATT TCCATCATGA GGCGGCGGAA GAGTTGGGGT
401  TGTTGGCGGC AGAAGTCGCA CAGATGGAAA AGAAATACCG GCTGCTGATT
451  AAGAACAACC TGTATATCAA CAATAACGCT TGGGGCGTTT GCGATCCTTC
501  CGGTCAGAGC GAATTGACAT TTTTCCCATT GTATATCGGT TCAACCAAAT
551  TTATTTTGGT TATCGGCGGC ATTCCCGATT TGGGCAAAGA GGCATTTGTT
601  ACTTTGGTAA GGATTTTATA CCGCCGTTAC AGCAACCGCG TGTAA
```

This corresponds to the amino acid sequence <SEQ ID 612; ORF143-1>:

```
  1  MESTLSLQAN LYPRLTPAGA FYAVSSDAPS AGKTLLHSLL KADADEMVSS
 51  EKLLTWADTA DIDTALNLLY RLQKLEFLYG DENGHSDGIN LSDEQLPLLM
101  EQLSGSGKAL LVDRNGLYLA NANFHHEAAE ELGLLAAEVA QMEKKYRLLI
151  KNNLYINNNA WGVCDPSGQS ELTFFPLYIG STKFILVIGG IPDLGKEAFV
201  TLVRILYRRY SNRV*
```

Computer analysis of this amino acid sequence gave the following results:

Homology with a predicted ORF from *N.meningitidis* (strain A)

ORF143 shows 92.4% identity over a 105aa overlap with an ORF (ORF143a) from strain A of *N. meningitidis:*

```
                                                   10        20        30
orf143.pep                                MRTKWSAVRSCTWADTADIDTALNLLYRLQKLEFL
                                                 |: :  ||| ||||||||||||||||||||
orf143a     GAFYAVSSDXPSAGKTLLHSLLKADADEMVSSEKLLTWAXTADIDTALNLLYRLQKLEFL
            20        30        40        50        60        70

              40        50        60        70        80        90
orf143.pep  YGDENGHSDGINLXDEQLPLLMEQLSGSGKALLVDRNGLYLANANFHHEAAEELGLLAAE
            ||||||||||||| ||||||||||||||||||||||||||||||||||||||||||||||
orf143a     YGDENGHSDGINLSDEQLPLLMEQLSGSGKALLVDRNGLYLANANFHHEAAEELGLLAAE
            80        90        100       110       120       130

              100       110
orf143.pep  VAQMEKKYRLLIKNN
            |||||||||| ||||
orf143a     VAQMEKKYRLXIKNNLYINNNAWGVCDPSGQSELTFFPLYIGSTKFILVIGGIPDLGKEA
           140       150       160       170       180       190
```

The complete length ORF143a nucleotide sequence <SEQ ID 613> is:

```
  1  ATGGAATCAA CANTTTCACT ACAAGCAAAT TTATATCNCC GCCTGACTCC
 51  TGCCGGTGCA TTTTATGCCG TATCCAGCGA TGNCCCCAGT GCCGGTAAAA
101  CTTTGTTGCA CAGCCTGTTG AAAGCGGATG CGGACGAAAT GGTNAGCAGT
151  GAGAAGCTGC TTACCTGGGC GGANACCGCC GACATCGATA CCGCTTTGAA
201  CCTGTTGTAC CGTTTGCAAA AACTCGAATT CCTCTATGGC GATGAAAACG
251  GTCATTCAGA CGGCATCAAT TTGTCGGACG AGCAATTGCC GTTGCTGATG
301  GAACAATTGT CCGGCAGCGG TAAGGCGTTA TTGGTCGATC GGAACGGTCT
351  GTATCTTGCC AACGCCAATT TCCATCATGA GGCGGCGGAA GAGTTGGGGT
401  TGTTGGCGGC AGAAGTCGCA CAGATGGAAA AGAAATACCG GCTGCNNATT
451  AAGAACAACC TGTATATCAA CAATAACGCT TGGGGCGTTT GCGATCCTTC
501  CGGTCAGAGC GAATTGACAT TTTTCCCATT GTATATCGGT TCAACCAAAT
551  TTATTTTGGT TATCGGCGGC ATTCCCGATT TGGGCAAAGA GGCATTTGTT
601  ACTTTGGTAA GGATNTTATA CCNCCNGTTA CAGCAACCGC GTGTAAAACT
651  TGGGAGAGAG GANGGGTTAT GCAGCAATTA TTGA
```

This encodes a protein having amino acid sequence <SEQ ID 614>:

```
  1  MESTXSLQAN LYXRLTPAGA FYAVSSDXPS AGKTLLHSLL KADADEMVSS
 51  EKLLTWAXTA DIDTALNLLY RLQKLEFLYG DENGHSDGIN LSDEQLPLLM
101  EQLSGSGKAL LVDRNGLYLA NANFHHEAAE ELGLLAAEVA QMEKKYRLXI
151  KNNLYINNNA WGVCDPSGQS ELTFFPLYIG STKFILVIGG IPDLGKEAFV
201  TLVRXLYXXL QQPRVKLGRE XGLCSNY*
```

ORF143a and ORF143-1 show 97.1% identity in 207 aa overlap:

```
orf143a.pep   MESTXSLQANLYXRLTPAGAFYAVSSDXPSAGKTLLHSLLKADADEMVSSEKLLTWAXTA
              |||| ||||||| |||||||||||||| |||||||||||||||||||||||||||||| ||
orf143-1      MESTLSLQANLYPRLTPAGAFYAVSSDAPSAGKTLLHSLLKADADEMVSSEKLLTWADTA

orf143a.pep   DIDTALNLLYRLQKLEFLYGDENGHSDGINLSDEQLPLLMEQLSGSGKALLVDRNGLYLA
              ||||||||||||||||||||||||||||||||||||||||||||||||||||||||||||
orf143-1      DIDTALNLLYRLQKLEFLYGDENGHSDGINLSDEQLPLLMEQLSGSGKALLVDRNGLYLA

orf143a.pep   NANFHHEAAEELGLLAAEVAQMEKKYRLXIKNNLYINNNAWGVCDPSGQSELTFFPLYIG
              |||||||||||||||||||||||||||| |||||||||||||||||||||||||||||||
orf143-1      NANFHHEAAEELGLLAAEVAQMEKKYRLLIKNNLYINNNAWGVCDPSGQSELTFFPLYIG

orf143a.pep   STKFILVIGGIPDLGKEAFVTLVRXLY
              |||||||||||||||||||||||| ||
orf143-1      STKFILVIGGIPDLGKEAFVTLVRILY
```

Homology with a predicted ORF from *N.gonorrhoeae*

ORF143 shows 95.5% identity over a 110aa overlap with a predicted ORF (ORF143ng) from *N.gonorrhoeae:*

```
orf143.pep   MRTKWSAVRSCTWADTADIDTALNLLYRLQKLEFLYGDENGHSDGINLXDEQLPLLMEQL   60
             |||||||||||: |||||||||||||||||||||||||||||||||||| |||||||||||
orf143ng     MRTKWSAVRSCSRADTADIDTALNLLYRLQKLEFLYGDENGHSDGINLSDEQLPLLMEQL   60

orf143.pep   SGSGKALLVDRNGLYLANANFHHEAAEELGLLAAEVAQMEKKYRLLIKNN            110
             ||||||||||||||||||||||||:||||||||||||||||||||||||:||
orf143ng     SGSGKALLVDRNGLYLANANFHHESAEELGLLAAEVAQMEKKYRLLIRNNLYINNNAWGV  120
```

An ORF143ng nucleotide sequence <SEQ ID 615> was predicted to encode a protein having amino acid sequence <SEQ ID 616>:

```
  1  MRTKWSAVRS CSRADTADID TALNLLYRLQ KLEFLYGDEN GHSDGINLSD
 51  EQLPLLMEQL SGSGKALLVD RNGLYLANAN FHHESAEELG LLAAEVAQME
101  KKYRLLIRNN LYINNNAWGV CDPSGQSELT FFPLYIGSTK FILVIAGIPD
151  LSKGGICYFG KDFIPPLQQP RVKLGTGGIM RQLLISILED LNNTSTDIIA
201  SAVISTDGLP MATMLPSHLN SDRVGAISAT LLALGSRSVQ ELACGELEQV
251  MIKGKSGYIL LSQAGKDAVL VLVAKETGRL GLILLDAKRA ARHIAEAI*
```

Further work revealed the following gonococcal DNA sequence <SEQ ID 617>:

```
  1  ATGGAATCAA CACTTTCACT ACAAGCGAAT TTATATCCCT GCCTGACTCC
 51  TGCCGGTGCA TTTTATGCCG TATCCAGCGA TGCCCCCAGT GCCGGTAAAA
101  CTTTGTTGCG CAGCCTGTTG AAAGCGGATG CGGACGAAGT GGTCAGCAGT
151  GAGAAGCTGC TCGCGGCGGA CACCGCCGAC ATCGATACCG CTTTGAACCT
201  GTTGTACCGT TTGCAAAAAC TCGAATTCCT CTATGGCGAT GAAAACGGTC
251  ATTCAGACGG CATCAATTTG TCGGACGAGC AATTGCCGTT GCTGATGGAA
301  CAATTGTCCG GCAGCGGTAA GGCATTATTG GTCGATCGGA ACGGTCTGTA
351  TCTTGCCAAC GCCAATTTCC ATCATGAGTC GGCGGAAGAG TTGGGGTTGT
401  TGGCGGCAGA AGTCGCACAG ATGGAAAAGA AATACCGGCT GCTGATTAGG
451  AACAACCTGT ATATCAACAA TAACGCTTGG GGCGTTTGCG ATCCTTCCGG
501  TCAGAGCGAA TTGACATTTT TCCCATTGTA TATCGGTTCA ACCAAATTTA
551  TTTTGGTTAT CGCCGGCATT CCCGATTTGA GCAAAGAGGC ATTTGTTACT
601  TTGGTAAGGA TTTTATACCG CCGTTACAGC AACCGCGTGT AA
```

This corresponds to the amino acid sequence <SEQ ID 618; ORF143ng-1>:

```
  1  MESTLSLQAN LYPCLTPAGA FYAVSSDAPS AGKTLLRSLL KADADEVVSS
 51  EKLLAADTAD IDTALNLLYR LQKLEFLYGD ENGHSDGINL SDEQLPLLME
101  QLSGSGKALL VDRNGLYLAN ANFHHESAEE LGLLAAEVAQ MEKKYRLLIR
151  NNLYINNNAW GVCDPSGQSE LTFFPLYIGS TKFILVIAGI PDLSKEAFVT
201  LVRILYRRYS NRV*
```

ORF143ng-1 and ORF143-1 show 95.8% identity in 214 aa overlap:

```
orf143ng-1.pep MESTLSLQANLYPCLTPAGAFYAVSSDAPSAGKTLLRSLLKADADEVVSSEKLLA-ADTA  59
               ||||||||||||| ||||||||||||||||||||||:|||||||||:||||||||: ||||
orf143-1       MESTLSLQANLYPRLTPAGAFYAVSSDAPSAGKTLLHSLLKADADEMVSSEKLLTWADTA  60

orf143ng-1.pep DIDTALNLLYRLQKLEFLYGDENGHSDGINLSDEQLPLLMEQLSGSGKALLVDRNGLYLA 119
               ||||||||||||||||||||||||||||||||||||||||||||||||||||||||||||
orf143-1       DIDTALNLLYRLQKLEFLYGDENGHSDGINLSDEQLPLLMEQLSGSGKALLVDRNGLYLA 120

orf143ng-1.pep NANFHHESAEELGLLAAEVAQMEKKYRLLIRNNLYINNNAWGVCDPSGQSELTFFPLYIG 179
               |||||||:||||||||||||||||||||||:|||||||||||||||||||||||||||||
orf143-1       NANFHHEAAEELGLLAAEVAQMEKKYRLLIKNNLYINNNAWGVCDPSGQSELTFFPLYIG 180

orf143ng-1.pep STKFILVIAGIPDLSKEAFVTLVRILYRRYSNRV  213
               ||||||||:|||||:|||||||||||||||||||
orf143-1       STKFILVIGGIPDLGKEAFVTLVRILYRRYSNRV  214
```

Based on the presence of the putative transmembrane domains in the gonococcal protein, it is predicted that the proteins from *N.meningitidis* and *N.gonorrhoeae,* and their epitopes, could be useful antigens for vaccines or diagnostics, or for raising antibodies.

**Example 74**

The following partial DNA sequence was identified in *N.meningitidis* <SEQ ID 619>:

```
  1  ATGACCTTTT TACAACGTTT GCAAGGTTTG GCAGACAATA AAATCTGTGC
 51  GTTTGCATGG TTCGTCGTCC GCCGCTTTGA TGAAGAACGC GTACCGCAGr
101  CGGCGGCAAG CATGACGTTT ACGACGCTGC TGGCACTCGT CCCCGTGCTG
151  ACCGTGATGG TGGCGGTCGC TTCGATTTTC CCCGTGTTCG ACCGCTGGTC
201  GGATTCGTTC GTCTCCTTCG TCAACCAAAC CATTGTGCCG CA.GGCGCGG
251  ACATGGTGTT CGACTATATC AATGCGTTCC GCGAGCAGGC GAACCGGCTG
301  ACGGCAATCG GCAGCGTGAT GCTGGTCGTT ACCTCGCTGA TGCTGATTCG
351  GACGATAGAC AATACGTTCA ACCGCATCTG GaCGGTCAA wTyCCAGCGT
401  CCGTGGATG..
```

This corresponds to the amino acid sequence <SEQ ID 620; ORF144>:

```
  1  MTFLQRLQGL ADNKICAFAW FVVRRFDEER VPQXAASMTF TTLLALVPVL
 51  TVMVAVASIF PVFDRWSDSF VSFVNQTIVP XGADMVFDYI NAFREQANRL
101  TAIGSVMLVV TSLMLIRTID NTFNRIWRVX XQRPWM...
```

Further work revealed the complete nucleotide sequence <SEQ ID 621>:

```
  1  ATGACCTTTT TACAACGTTT GCAAGGTTTG GCAGACAATA AAATCTGTGC
 51  GTTTGCATGG TTCGTCGTCC GCCGCTTTGA TGAAGAACGC GTACCGCAGG
101  CGGCGGCAAG CATGACGTTT ACGACGCTGC TGGCACTCGT CCCCGTGCTG
```

```
 151  ACCGTGATGG TGGCGGTCGC TTCGATTTTC CCCGTGTTCG ACCGCTGGTC
 201  GGATTCGTTC GTCTCCTTCG TCAACCAAAC CATTGTGCCG CAGGGCGCGG
 251  ACATGGTGTT CGACTATATC AATGCGTTCC GCGAGCAGGC GAACCGGCTG
 301  ACGGCAATCG GCAGCGTGAT GCTGGTCGTT ACCTCGCTGA TGCTGATTCG
 351  GACGATAGAC AATACGTTCA ACCGCATCTG GCGGGTCAAT TCCCAGCGTC
 401  CGTGGATGAT GCAGTTTCTC GTCTATTGGG CTTTACTGAC GTTCGGGCCG
 451  CTGTCTTTGG GCGTGGGCAT TTCCTTTATG GTCGGCTCGG TACAGGATGC
 501  CGCGCTTGCC TCAGGTGCGC CGCAGTGGTC GGGCGCGTTG CGAACGGCGG
 551  CGACGCTGAC CTTCATGACG CTTTTGCTGT GGGGGCTGTA CCGCTTCGTG
 601  CCAAACCGCT TCGTTCCCGC GCGGCAGGCG TTTGTCGGGG CTTTGGCAAC
 651  AGCGTTTTGT CTGGAAACCG CGCGCTCCCT CTTCACTTGG TATATGGGCA
 701  ATTTCGACGG CTACCGCTCG ATTTACGGCG CGTTTGCCGC CGTGCCGTTT
 751  TTTCTGTTGT GGCTGAACCT GTTGTGGACG CTGGTCTTGG GCGGCGCGGT
 801  GCTGACTTCT TCACTCTCCT ACTGGCAGGG AGAAGCGTTC CGCAGGGGCT
 851  TCGACTCGCG CGGACGGTTT GACGACGTGT TGAAAATCCT GCTGCTTCTG
 901  GATGCGGCGC AAAAAGAAGG CAAAGCCTTG CCTGTTCAGG AGTTCAGACG
 951  GCATATCAAT ATGGGCTACG ACGAGTTGGG CGAGCTTTTG GAAAAGCTGG
1001  CGCGGCACGG CTACATCTAT TCCGGCAGAC AGGGTTGGGT GTTGAAAACG
1051  GGGGCGGATT CGATTGAGTT GAACGAACTC TTCAAGCTCT TCGTTTACCG
1101  TCCGTTGCCT GTGGAAAGGG ATCATGTGAA CCAAGCTGTC GATGCGGTAA
1151  TGACACCGTG TTTGCAGACT TTGAACATGA CGCTGGCAGA GTTTGACGCT
1201  CAGGCGAAAA AACGGCAGTA G
```

This corresponds to the amino acid sequence <SEQ ID 622; ORF144-1>:

```
  1  MTFLQRLQGL ADNKICAFAW FVVRRFDEER VPQAAASMTF TTLLALVPVL
 51  TVMVAVASIF PVFDRWSDSF VSFVNQTIVP QGADMVFDYI NAFREQANRL
101  TAIGSVMLVV TSLMLIRTID NTFNRIWRVN SQRPWMMQFL VYWALLTFGP
151  LSLGVGISFM VGSVQDAALA SGAPQWSGAL RTAATLTFMT LLLWGLYRFV
201  PNRFVPARQA FVGALATAFC LETARSLFTW YMGNFDGYRS IYGAFAAVPF
251  FLLWLNLLWT LVLGGAVLTS SLSYWQGEAF RRGFDSRGRF DDVLKILLLL
301  DAAQKEGKAL PVQEFRRHIN MGYDELGELL EKLARHGYIY SGRQGWVLKT
351  GADSIELNEL FKLFVYRPLP VERDHVNQAV DAVMTPCLQT LNMTLAEFDA
401  QAKKRQ*
```

Computer analysis of this amino acid sequence gave the following results:

Homology with a predicted ORF from *N.meningitidis* (strain A)

ORF144 shows 96.3% identity over a 136aa overlap with an ORF (ORF144a) from strain A of *N. meningitidis:*

```
                    10        20        30        40        50        60
orf144.pep  MTFLQRLQGLADNKICAFAWFVVRRFDEERVPQXAASMTFTTLLALVPVLTVMVAVASIF
            |||||||||||||||||||||||||||||||||| |||||||||||||||||||||||||
orf144a     MTFLQRLQGLADNKICAFAWFVVRRFDEERVPQAAASMTFTTLLALVPVLTVMVAVASIF
                    10        20        30        40        50        60

                    70        80        90       100       110       120
orf144.pep  PVFDRWSDSFVSFVNQTIVPXGADMVFDYINAFREQANRLTAIGSVMLVVTSLMLIRTID
            |||||||||||||||||||| ||||||||||||||||||||||||||||||| |||||||
orf144a     PVFDRWSDSFVSFVNQTIVPQGADMVFDYINAFREQANRLTAIGSVMLVVTSXMLIRTID
                    70        80        90       100       110       120

                   130
orf144.pep  NTFNRIWRVXXQRPWM
            |||||||||  |||||
orf144a     NTFNRIWRVNSQRPWMMQFLVYWALLTFGPLSLGVGISFXVGSVQDAALASGAPQWSGAL
                   130       140       150       160       170       180
```

The complete length ORF144a nucleotide sequence <SEQ ID 623> is:

```
   1  ATGACCTTTT TACAACGTTT GCAAGGTTTG GCAGACAATA AAATCTGTGC
  51  GTTTGCATGG TTCGTCGTCC GCCGCTTTGA TGAAGAACGC GTACCGCAGG
 101  CGGCGGCAAG CATGACGTTT ACGACACTGC TGGCACTCGT CCCCGTGCTG
 151  ACCGTGATGG TGGCGGTCGC TTCGATTTTC CCCGTGTTCG ACCGNTGGTC
 201  GGATTCGTTC GTCTCCTTCG TCAACCAAAC CATTGTGCCG CAGGGCGCGG
 251  ACATGGTNTT CGACTATATC AATGCGTTCC GCGAGCAGGC GAACCGGCTG
 301  ACGGCAATCG GCAGCGTGAT GCTGGTCGTT ACCTCGCNGA TGCTGATTCG
 351  GACGATAGAC AATACGTTCA ACCGCATCTG GCGGGTCAAT TCCCAGCGTC
```

```
 401  CGTGGATGAT GCAGTTTCTC GTCTATTGGG CTTTACTGAC GTTCGGGCCG
 451  CTGTCTTTGG GCGTGGGCAT TTCCTTTATN GTCGGCTCGG TACAGGATGC
 501  CGCGCTTGCC TCAGGTGCGC CGCAGTGGTC GGGCGCGTTG CGAACGGCGG
 551  CGACGCTGAN CTTCATGACG CTTTTGCTGT GGGGGCTGTA CCGCTNCGTG
 601  CCAAACCGCT TCGTTCCCGC GCGGCANGCG TTTGTCGGGG CTTTGGCAAC
 651  AGCGTTCTGT CTGGAAACCG CGCGTTCCCT CTTTACTTGG TATATGGGCA
 701  ATTTCGACGG CTACCGCTCG ATTTACGGNG CGTTTGCCGC CGTGCCGTTT
 751  TTTCTGTTGT GGCTGAACCT GTTGTGGACG CTGGTCTTGG GCGGCGCGGT
 801  GCTGACTTCT TCACTCTCCT ACTGGCAGGG AGAAGCGTTC CGCAGGGNCT
 851  TCGACTCGCG CGGACGGTTT GACGACGTGT TGAAAATCCT GCTGCTTCTG
 901  GATGCGGCGC AAAAAGAAGG CNAAGCCTTG CCTGTTCAGG AGTTCAGACG
 951  GCATATCAAT ATGGGCTACG ACGAGTTGGG CGAGCTTTTG GAAAAGCTGG
1001  CGCGGCACGG CTACATCTAT TCCGGCAGAC AGGGTTGGGT GTTGAAAACG
1051  GGGGCGGATT CGATTGAGTT GAACGAACTC TTCAAGCTCT TCGTTTACCG
1101  TCCGTTGCCT GTGGAAAGGG ATCATGTGAA CCAAGCTGTC GATGCGGTAA
1151  TGATGCCGTG TTTGCAGACT TTGAACATGA CGCTGGCAGA GTTTGACGCT
1201  CAGGCGAAAA AACAGCAGCA ATCTTGA
```

This encodes a protein having amino acid sequence <SEQ ID 624>:

```
  1  MTFLQRLQGL ADNKICAFAW FVVRRFDEER VPQAAASMTF TTLLALVPVL
 51  TVMVAVASIF PVFDRWSDSF VSFVNQTIVP QGADMVFDYI NAFREQANRL
101  TAIGSVMLVV TSXMLIRTID NTFNRIWRVN SQRPWMMQFL VYWALLTFGP
151  LSLGVGISFX VGSVQDAALA SGAPQWSGAL RTAATLXFMT LLLWGLYRXV
201  PNRFVPARXA FVGALATAFC LETARSLFTW YMGNFDGYRS IYGAFAAVPF
251  FLLWLNLLWT LVLGGAVLTS SLSYWQGEAF RRXFDSRGRF DDVLKILLLL
301  DAAQKEGXAL PVQEFRRHIN MGYDELGELL EKLARHGYIY SGRQGWVLKT
351  GADSIELNEL FKLFVYRPLP VERDHVNQAV DAVMMPCLQT LNMTLAEFDA
401  QAKKQQQS*
```

ORF144a and ORF144-1 show 97.8% identity in 406 aa overlap:

```
orf144a.pep   MTFLQRLQGLADNKICAFAWFVVRRFDEERVPQAAASMTFTTLLALVPVLTVMVAVASIF
              ||||||||||||||||||||||||||||||||||||||||||||||||||||||||||||
orf144-1      MTFLQRLQGLADNKICAFAWFVVRRFDEERVPQAAASMTFTTLLALVPVLTVMVAVASIF

orf144a.pep   PVFDRWSDSFVSFVNQTIVPQGADMVFDYINAFREQANRLTAIGSVMLVVTSXMLIRTID
              |||||||||||||||||||||||||||||||||||||||||||||||||||| |||||||
orf144-1      PVFDRWSDSFVSFVNQTIVPQGADMVFDYINAFREQANRLTAIGSVMLVVTSLMLIRTID

orf144a.pep   NTFNRIWRVNSQRPWMMQFLVYWALLTFGPLSLGVGISFXVGSVQDAALASGAPQWSGAL
              ||||||||||||||||||||||||||||||||||||||| ||||||||||||||||||||
orf144-1      NTFNRIWRVNSQRPWMMQFLVYWALLTFGPLSLGVGISFMVGSVQDAALASGAPQWSGAL

orf144a.pep   RTAATLXFMTLLLWGLYRXVPNRFVPARXAFVGALATAFCLETARSLFTWYMGNFDGYRS
              ||||||:||||||||||| ||||||||| |||||||||||||||||||||||||||||||
orf144-1      RTAATLTFMTLLLWGLYRFVPNRFVPARQAFVGALATAFCLETARSLFTWYMGNFDGYRS

orf144a.pep   IYGAFAAVPFFLLWLNLLWTLVLGGAVLTSSLSYWQGEAFRRXFDSRGRFDDVLKILLLL
              |||||||||||||||||||||||||||||||||||||||||| |||||||||||||||||
orf144-1      IYGAFAAVPFFLLWLNLLWTLVLGGAVLTSSLSYWQGEAFRRGFDSRGRFDDVLKILLLL

orf144a.pep   DAAQKEGXALPVQEFRRHINMGYDELGELLEKLARHGYIYSGRQGWVLKTGADSIELNEL
              ||||||| ||||||||||||||||||||||||||||||||||||||||||||||||||||
orf144-1      DAAQKEGKALPVQEFRRHINMGYDELGELLEKLARHGYIYSGRQGWVLKTGADSIELNEL

orf144a.pep   FKLFVYRPLPVERDHVNQAVDAVMMPCLQTLNMTLAEFDAQAKKQQQS  408
              |||||||||||||||||||||||| ||||||||||||||||||:|
orf144-1      FKLFVYRPLPVERDHVNQAVDAVMTPCLQTLNMTLAEFDAQAKKRQ    406
```

Homology with a predicted ORF from *N.gonorrhoeae*

ORF144 shows 91.2% identity over a 136aa overlap with a predicted ORF (ORF144ng) from *N.gonorrhoeae:*

```
orf144.pep  MTFLQRLQGLADNKICAFAWFVVRRFDEERVPQXAASMTFTTLLALVPVLTVMVAVASIF   60
            |||||  || ||||||||||||:|||:|||||| |||||||||||||||||||||||||||
orf144ng    MTFLQCWQGSADNKICAFAWFVIRRFSEERVPQAAASMTFTTLLALVPVLTVMVAVASIF   60

orf144.pep  PVFDRWSDSFVSFVNQTIVPXGADMVFDYINAFREQANRLTAIGSVMLVVTSLMLIRTID  120
            |||||||||||||||||||| |||||||||:|||:|||||||||||||||||||||||||
orf144ng    PVFDRWSDSFVSFVNQTIVPQGADMVFDYIDAFRDQANRLTAIGSVMLVVTSLMLIRTID  120

orf144.pep  NTFNRIWRVXXQRPWM                                              136
            |:||||||| :|||||
orf144ng    NAFNRIWRVNTQRPWMMQFLVYWALLTFGPLSLGVGISFMVGSVQDSVLSSGAQQWADAL  180
```

The complete length ORF144ng nucleotide sequence <SEQ ID 625> is predicted to encode a protein having amino acid sequence <SEQ ID 626>:

```
  1  MTFLQCWQGS ADNKICAFAW FVIRRFSEER VPQAAASMTF TTLLALVPVL
 51  TVMVAVASIF PVFDRWSDSF VSFVNQTIVP QGADMVFDYI DAFRDQANRL
101  TAIGSVMLVV TSLMLIRTID NAFNRIWRVN TQRPWMMQFL VYWALLTFGP
151  LSLGVGISFM VGSVQDSVLS SGAQQWADAL KTAARLAFMT LLLWGLYRFV
201  PNRFVPARQA FVGALITAFC LETARFLFTW YMGNFDGYRS IYGAFAAVPF
251  FLLWLNLLWT LVLGGAVLTS SLSYWQGEAF RRGFDSRGRF DDVLKILLLL
301  DAAQKEGRTL SVQEFRRHIN MGYDELGELL EKLARYGYIY SGRQGWVLKT
351  GADSIELSEL FKLFVYRPLP VERDHVNQAV DAVMTPCLQT LNMTLAEFDA
401  QAKKQQQS*
```

Further work revealed the following gonococcal DNA sequence <SEQ ID 627>:

```
   1  ATGACCTTTT TACAACGTTG GCAAGGTTTG GCGGACAATA AAATCTGTGC
  51  ATTTGCATGG TTCGTCATCC GCCGTTTCAG TGAAGAGCGC GTACCGCAGG
 101  CAGCGGCGAG CATGACGTTT ACGACACTGC TGGCACTCGT CCCCGTACTG
 151  ACCGTAATGG TCGCGGTCGC TTCGATTTTC CCCGTGTTCG ACCGCTGGTC
 201  GGATTCGTTC GTCTCCTTCG TCAACCAAAC CATTGTGCCG CAGGGCGCGG
 251  ATATGGTGTT CGACTATATC GACGCATTCC GCGATCAGGC AAACCGGCTG
 301  ACCGCCATCG GCAGCGTGAT GCTGGTCGTA ACCTCGCTGA TGCTGATTCG
 351  GACGATAGAC AATGCGTTCA ACCGCATCTG GCGGGTTAAC ACGCAACGCC
 401  CCTGGATGAT GCAGTTCCTC GTTTATTGGG CGTTGCTGAC TTTCGGGCCT
 451  TTGTCTTTGG GTGTGGGCAT TTCCTTTATG GTCGGGTCGG TTCAAGACTC
 501  CGTACTCTCC TCCGGAGCGC AACAATGGGC GGACGCGTTG AAGACGGCGG
 551  CAAGGCTGGC TTTCATGACG CTTTTGCTGT GGGGGCTGTA CCGCTTCGTG
 601  CCCAACCGCT TCGTGCCCGC CCGGCAGGCG TTTGTCGGAG CTTTGATTAC
 651  GGCATTCTGC CTGGAGACGG CACGTTTCCT GTTCACCTGG TATATGGGCA
 701  ATTTCGACGG CTACCGCTCG ATTTACGGCG CATTTGCCGC CGTGCCGTTT
 751  TTCCTGCTGT GGTTAAACCT GCTGTGGACG CTGGTCTTGG GCGGGGCGGT
 801  GCTGACTTCG TCGCTGTCTT ATTGGCAGGG CGAGGCCTTC CGCAGGGGAT
 851  TCGACTCGCG CGGACGGTTT GACGACGTGT TGAAAATCCT GCTGCTTCTG
 901  GATGCGGCGC AAAAAGAAGG CCGAACCCTG TCCGTTCAGG AGTTCAGACG
 951  GCATATCAAT ATGGGTTACG ATGAATTGGG CGAGCTTTTG GAAAAGCTGG
1001  CGCGGTACGG CTATATCTAT TCCGGCAGAC AGGGCTGGGT TTTGAAAACG
1051  GGGGCGGATT CGATTGAGTT GAGCGAACTC TTCAAGCTCT TCGTGTACCG
1101  CCCGTTGCct gtggaAAGGG ATCATGTGAA CCAAGCTGtc gaTGCGGTAA
1151  TGAcgccgtG TTTGCAGACT TTGAACATGA CGCTGGCGGA GTTTGACGCT
1201  CAGgcgAAAA AACAGCAGCA GTCTTGA
```

This encodes a variant ofORF144ng, having the amino acid sequence <SEQ ID 628; ORF144ng-1>:

```
  1  MTFLQRWQGL ADNKICAFAW FVIRRFSEER VPQAAASMTF TTLLALVPVL
 51  TVMVAVASIF PVFDRWSDSF VSFVNQTIVP QGADMVFDYI DAFRDQANRL
101  TAIGSVMLVV TSLMLIRTID NAFNRIWRVN TQRPWMMQFL VYWALLTFGP
151  LSLGVGISFM VGSVQDSVLS SGAQQWADAL KTAARLAFMT LLLWGLYRFV
201  PNRFVPARQA FVGALITAFC LETARFLFTW YMGNFDGYRS IYGAFAAVPF
251  FLLWLNLLWT LVLGGAVLTS SLSYWQGEAF RRGFDSRGRF DDVLKILLLL
301  DAAQKEGRTL SVQEFRRHIN MGYDELGELL EKLARYGYIY SGRQGWVLKT
351  GADSIELSEL FKLFVYRPLP VERDHVNQAV DAVMTPCLQT LNMTLAEFDA
401  QAKKQQQS*
```

ORF144ng-1 and ORF144-1 show 94.1% identity in 406 aa overlap:

```
orf144ng-1.pep MTFLQRWQGLADNKICAFAWFVIRRFSEERVPQAAASMTFTTLLALVPVLTVMVAVASIF
               |||||| ||||||||||||||:|||:||||||||||||||||||||||||||||||||||
orf144-1       MTFLQRLQGLADNKICAFAWFVVRRFDEERVPQAAASMTFTTLLALVPVLTVMVAVASIF

orf144ng-1.pep PVFDRWSDSFVSFVNQTIVPQGADMVFDYIDAFRDQANRLTAIGSVMLVVTSLMLIRTID
               ||||||||||||||||||||||||||||||:|||:|||||||||||||||||||||||||
orf144-1       PVFDRWSDSFVSFVNQTIVPQGADMVFDYINAFREQANRLTAIGSVMLVVTSLMLIRTID
```

```
orf144ng-1.pep NAFNRIWRVNTQRPWMMQFLVYWALLTFGPLSLGVGISFMVGSVQDSVLSSGAQQWADAL
               |:||||||||:|||||||||||||||||||||||||||||||||||::|:||| ||: ||
orf144-1       NTFNRIWRVNSQRPWMMQFLVYWALLTFGPLSLGVGISFMVGSVQDAALASGAPQWSGAL

orf144ng-1.pep KTAARLAFMTLLLWGLYRFVPNRFVPARQAFVGALITAFCLETARFLFTWYMGNFDGYRS
               :||| |:|||||||||||||||||||||||||||| ||||||||| ||||||||||||||
orf144-1       RTAATLTFMTLLLWGLYRFVPNRFVPARQAFVGALATAFCLETARSLFTWYMGNFDGYRS

orf144ng-1.pep IYGAFAAVPFFLLWLNLLWTLVLGGAVLTSSLSYWQGEAFRRGFDSRGRFDDVLKILLLL
               ||||||||||||||||||||||||||||||||||||||||||||||||||||||||||||
orf144-1       IYGAFAAVPFFLLWLNLLWTLVLGGAVLTSSLSYWQGEAFRRGFDSRGRFDDVLKILLLL

orf144ng-1.pep DAAQKEGRTLSVQEFRRHINMGYDELGELLEKLARYGYIYSGRQGWVLKTGADSIELSEL
               |||||||::| ||||||||||||||||||||||||:|||||||||||||||||||||:||
orf144-1       DAAQKEGKALPVQEFRRHINMGYDELGELLEKLARHGYIYSGRQGWVLKTGADSIELNEL

orf144ng-1.pep FKLFVYRPLPVERDHVNQAVDAVMTPCLQTLNMTLAEFDAQAKKQQQS
               ||||||||||||||||||||||||||||||||||||||||||||:|
orf144-1       FKLFVYRPLPVERDHVNQAVDAVMTPCLQTLNMTLAEFDAQAKKRQ
```

On this basis of this analysis, including the identification of several putative transmembrane domains in the gonococcal protein, it is predicted that the proteins from *N.meningitidis* and *N.gonorrhoeae,* and their epitopes, could be useful antigens for vaccines or diagnostics, or for raising antibodies.

**Example 75**

The following partial DNA sequence was identified in *N.meningitidis* <SEQ ID 629>:

```
  1  ..AGACACGCCC GCCGCATCCG CATCGACACC GCCATCAACC CCGAACTGGA
 51    AGCCCTCGCC GAACACCTCC ACTACCAATG GCAGGGCTTC CTCTGGCTCA
101    GCACCGATAT GCGTCAGGAA ATTTCCGCCC TCGTCATCCT GCTGCAACGC
151    ACCCGCCGCA AATGGCTGGA TGCCCACGAA CGCCAACACC TGCGCCAAAG
201    CCTGCTTGAA ACACGGGAAC ACGGCTGA
```

This corresponds to the amino acid sequence <SEQ ID 630; ORF146>:

```
 1  ..RHARRIRIDT AINPELEALA EHLHYQWQGF LWLSTDMRQE ISALVILLQR
51    TRRKWLDAHE RQHLRQSLLE TREHG*
```

Further work revealed the complete nucleotide sequence <SEQ ID 631>:

```
   1  ATGAACACCT CGCAACGCAA CCGCCTCGTC AGCCGCTGGC TCAACTCCTA
  51  CGAACGCTAC CGCTACCGCC GCCTCATCCA CGCCGTCCGG CTCGGCGGGG
 101  CCGTCCTGTT CGCCACCGCC TCCGCCCGGC TGCTCCACCT CCAACACGGC
 151  GAGTGGATAG GGATGACCGT CTTCGTCGTC CTCGGCATGC TCCAGTTTCA
 201  AGGGGCGATT TACTCCAAGG CGGTGGAACG TATGCTCGGC ACGGTCATCG
 251  GGCTGGGCGC GGGTTTGGGC GTTTTATGGC TGAACCAGCA TTATTTCCAC
 301  GGCAACCTCC TCTTCTACCT CACCGTCGGC ACGGCAAGCG CACTGGCCGG
 351  CTGGGCGGCG GTCGGCAAAA ACGGCTACGT CCCTATGCTG GCAGGGCTGA
 401  CGATGTGTAT GCTCATCGGC GACAACGGCA GCGAATGGCT CGACAGCGGA
 451  CTCATGCGCG CCATGAACGT CCTCATCGGC GCGGCCATCG CCATCGCCGC
 501  CGCCAAACTG CTGCCGCTGA AATCCACACT GATGTGGCGT TTCATGCTTG
 551  CCGACAACCT GGCCGACTGC AGCAAAATGA TTGCCGAAAT CAGCAACGGC
 601  AGGCGCATGA CCCGCGAACG CCTCGAGGAG AACATGGCGA AAATGCGCCA
 651  AATCAACGCA CGCATGGTCA AAAGCCGCAG CCATCTCGCC GCCACATCGG
 701  GCGAAAGCCG CATCAGCCCC GCCATGATGG AAGCCATGCA GCACGCCCAC
 751  CGTAAAATCG TCAACACCAC CGAGCTGCTC CTGACCACCG CCGCCAAGCT
 801  GCAATCTCCC AAACTCAACG GCAGCGAAAT CCGGCTGCTT GACCGCCACT
 851  TCACACTGCT CCAAACCGAC CTGCAACAAA CCGTCGCCCT TATCAACGGC
 901  AGACACGCCC GCCGCATCCG CATCGACACC GCCATCAACC CCGAACTGGA
 951  AGCCCTCGCC GAACACCTCC ACTACCAATG GCAGGGCTTC CTCTGGCTCA
1001  GCACCAATAT GCGTCAGGAA ATTTCCGCCC TCGTCATCCT GCTGCAACGC
1051  ACCCGCCGCA AATGGCTGGA TGCCCACGAA CGCCAACACC TGCGCCAAAG
1101  CCTGCTTGAA ACACGGGAAC ACGGCTGA
```

This corresponds to the amino acid sequence <SEQ ID 632; ORF146-1>:

```
  1  MNTSQRNRLV SRWLNSYERY RYRRLIHAVR LGGAVLFATA SARLLHLQHG
 51  EWIGMTVFVV LGMLQFQGAI YSKAVERMLG TVIGLGAGLG VLWLNQHYFH
101  GNLLFYLTVG TASALAGWAA VGKNGYVPML AGLTMCMLIG DNGSEWLDSG
151  LMRAMNVLIG AAIAIAAAKL LPLKSTLMWR FMLADNLADC SKMIAEISNG
201  RRMTRERLEE NMAKMRQINA RMVKSRSHLA ATSGESRISP AMMEAMQHAH
251  RKIVNTTELL LTTAAKLQSP KLNGSEIRLL DRHFTLLQTD LQQTVALING
301  RHARRIRIDT AINPELEALA EHLHYQWQGF LWLSTNMRQE ISALVILLQR
351  TRRKWLDAHE RQHLRQSLLE TREHG*
```

Computer analysis of this amino acid sequence gave the following results:

Homology with a predicted ORF from *N.meningitidis* (strain A)

ORF146 shows 98.6% identity over a 74aa overlap with an ORF (ORF146a) from strain A of *N. meningitidis:*

```
                                                      10        20        30
orf146.pep                                  RHARRIRIDTAINPELEALAEHLHYQWQGF
                                            ||||||||||||||||||||||||||||||
orf146a     KLNGSEIRLLDRHFTLLQTDLQQTVALINGRHARRIRIDTAINPELEALAEHLHYQWQGF
                   280       290       300       310       320       330

                   40        50        60        70
orf146.pep  LWLSTDMRQEISALVILLQRTRRKWLDAHERQHLRQSLLETREHGX
            |||||:||||||||||||||||||||||||||||||||||||||:
orf146a     LWLSTNMRQEISALVILLQRTRRKWLDAHERQHLRQSLLETREHSX
                   340       350       360       370
```

The complete length ORF146a nucleotide sequence <SEQ ID 633> is:

```
   1  ATGAACACCT CGCAACGCAA CCGCCTCGTC AGCCGCTGGC TCAACTCCTA
  51  CGAACGCTAC CGCTACCGCC GCCTCATCCA CGCCGTCCGG CTCGGCGGGG
 101  CCGTCCTGTT CGCCACCGCC TCCGCCCGGC TGCTCCACCT CCAACACGGC
 151  GAGTGGATAG GGATGACCGT CTTCGTCGTC CTCGGCATGC TCCAGTTTCA
 201  AGGGGCGATT TACTCCAAGG CGGTGGAACG TATGCTCGGC ACGGTCATCG
 251  GGCTGGGCGC GGGTTTGGGC GTTTTATGGC TGAACCAGCA TTATTTCCAC
 301  GGCAACCTCC TCTTCTACCT CACCGTCGGC ACGGCAAGCG CACTGGCCGG
 351  CTGGGCGGCG GTCGGCAAAA ACGGCTACGT CCCTATGCTG GCGGGGCTGA
 401  CGATGTGCAT GCTCATCGGC GACAACGGCA GCGAATGGTT CGACAGCGGC
 451  CTGATGCGCG CGATGAACGT CCTCATCGGC GCGGCCATCG CCATCGCCGC
 501  CGCCAAACTG CTGCCGCTGA AATCCACACT GATGTGGCGT TTCATGCTTG
 551  CCGACAACCT GACCGACTGC AGCAAAATGA TTGCCGAAAT CAGCAACGGC
 601  AGGCGCATGA CCCGCGAACG CCTCGAAGAG AACATGGCGA AAATGCGCCA
 651  AATCAACGCA CGCATGGTCA AAAGCCGCAG CCACCTCGCC GCCACATCGG
 701  GCGAAAGCCG CATCAGCCCC GCCATGATGG AAGCCATGCA GCACGCCCAC
 751  CGTAAAATTG TCAACACCAC CGAGCTGCTC CTGACCACCG CCGCCAAGCT
 801  GCAATCTCCC AAACTCAACG GCAGCGAAAT CCGGCTGCTT GACCGCCACT
 851  TCACACTGCT CCAAACCGAC CTGCAACAAA CCGTCGCCCT TATCAACGGC
 901  AGACACGCCC GCCGCATCCG CATCGACACC GCCATCAACC CCGAACTGGA
 951  AGCCCTCGCC GAACACCTCC ACTACCAATG GCAGGGCTTC CTCTGGCTCA
1001  GCACCAATAT GCGTCAGGAA ATTTCCGCCC TCGTCATCCT GCTGCAACGC
1051  ACCCGCCGCA AATGGCTGGA TGCCCACGAA CGCCAACACC TGCGCCAAAG
1101  CCTGCTTGAA ACACGGGAAC ACAGTTGA
```

This encodes a protein having amino acid sequence <SEQ ID 634>:

```
  1  MNTSQRNRLV SRWLNSYERY RYRRLIHAVR LGGAVLFATA SARLLHLQHG
 51  EWIGMTVFVV LGMLQFQGAI YSKAVERMLG TVIGLGAGLG VLWLNQHYFH
101  GNLLFYLTVG TASALAGWAA VGKNGYVPML AGLTMCMLIG DNGSEWFDSG
151  LMRAMNVLIG AAIAIAAAKL LPLKSTLMWR FMLADNLTDC SKMIAEISNG
201  RRMTRERLEE NMAKMRQINA RMVKSRSHLA ATSGESRISP AMMEAMQHAH
251  RKIVNTTELL LTTAAKLQSP KLNGSEIRLL DRHFTLLQTD LQQTVALING
301  RHARRIRIDT AINPELEALA EHLHYQWQGF LWLSTNMRQE ISALVILLQR
351  TRRKWLDAHE RQHLRQSLLE TREHS*
```

ORF146a and ORF146-1 show 99.5% identity in 374 aa overlap:

```
orf146a.pep   MNTSQRNRLVSRWLNSYERYRYRRLIHAVRLGGAVLFATASARLLHLQHGEWIGMTVFVV
              ||||||||||||||||||||||||||||||||||||||||||||||||||||||||||||
orf146-1      MNTSQRNRLVSRWLNSYERYRYRRLIHAVRLGGAVLFATASARLLHLQHGEWIGMTVFVV

orf146a.pep   LGMLQFQGAIYSKAVERMLGTVIGLGAGLGVLWLNQHYFHGNLLFYLTVGTASALAGWAA
              ||||||||||||||||||||||||||||||||||||||||||||||||||||||||||||
orf146-1      LGMLQFQGAIYSKAVERMLGTVIGLGAGLGVLWLNQHYFHGNLLFYLTVGTASALAGWAA

orf146a.pep   VGKNGYVPMLAGLTMCMLIGDNGSEWFDSGLMRAMNVLIGAAIAIAAAKLLPLKSTLMWR
              ||||||||||||||||||||||||||:|||||||||||||||||||||||||||||||||
orf146-1      VGKNGYVPMLAGLTMCMLIGDNGSEWLDSGLMRAMNVLIGAAIAIAAAKLLPLKSTLMWR

orf146a.pep   FMLADNLTDCSKMIAEISNGRRMTRERLEENMAKMRQINARMVKSRSHLAATSGESRISP
              |||||||:||||||||||||||||||||||||||||||||||||||||||||||||||||
orf146-1      FMLADNLADCSKMIAEISNGRRMTRERLEENMAKMRQINARMVKSRSHLAATSGESRISP

orf146a.pep   AMMEAMQHAHRKIVNTTELLLTTAAKLQSPKLNGSEIRLLDRHFTLLQTDLQQTVALING
              ||||||||||||||||||||||||||||||||||||||||||||||||||||||||||||
orf146-1      AMMEAMQHAHRKIVNTTELLLTTAAKLQSPKLNGSEIRLLDRHFTLLQTDLQQTVALING

orf146a.pep   RHARRIRIDTAINPELEALAEHLHYQWQGFLWLSTNMRQEISALVILLQRTRRKWLDAHE
              ||||||||||||||||||||||||||||||||||||||||||||||||||||||||||||
orf146-1      RHARRIRIDTAINPELEALAEHLHYQWQGFLWLSTNMRQEISALVILLQRTRRKWLDAHE

orf146a.pep   RQHLRQSLLETREHSX
              ||||||||||||||:
orf146-1      RQHLRQSLLETREHGX
```

Homology with a predicted ORF from *N.gonorrhoeae*

ORF146 shows 97.3% identity over a 75aa overlap with a predicted ORF (ORF146ng) from *N.gonorrhoeae:*

```
orf146.pep                                  RHARRIRIDTAINPELEALAEHLHYQWQGF   30
                                            ||||||||||||||||||||||||||||||
orf146ng     KLNGSEIRLLDRHFTLLQTDLQQTAALINGRHARRIRIDTAINPELEALAEHLHYQWQGF   364

orf146.pep   LWLSTDMRQEISALVILLQRTRRKWLDAHERQHLRQSLLETREHG   75
             |||||:|||||||||| |||||||||||||||||||||||||||||
orf146ng     LWLSTNMRQEISALVIPLQRTRRKWLDAHERQHLRQSLLETREHG   409
```

An ORF146ng nucleotide sequence <SEQ ID 635> was predicted to encode a protein having amino acid sequence <SEQ ID 636>:

```
  1  MSGVRFPSPA PIPSTDPPSG SLCFFTFPLQ TASDMNSSQR KRLSGRWLNS
 51  YERYRHRRLI HAVRLGGTVL FATALARLLH LQHGEWIGMT VFVVLGMLQF
101  QGAIYSNAVE RMLGTVIGLG AGLGVLWLNQ HYFHGNLLFY LTIGTASALA
151  GWAAVGKNGY VPMLAGLTMC MLIGDNGSEW LDSGLMRAMN VLIGAAIAIA
201  AAKLLPLKST LMWRFMLADN LADCSKMIAE ISNGRRMTRE RLEQNMVKMR
251  QINARMVKSR SHLAATSGES RISPSMMEAM QHAHRKIVNT TELLLTTAAK
301  LQSPKLNGSE IRLLDRHFTL LQTDLQQTAA LINGRHARRI RIDTAINPEL
351  EALAEHLHYQ WQGFLWLSTN MRQEISALVI PLQRTRRKWL DAHERQHLRQ
401  SLLETREHG*
```

Further work revealed the following gonococcal DNA sequence <SEQ ID 637>:

```
   1  ATGAACTCCT CGCAACGCAA ACGCCTTTCC GgccGCTGGC TCAACTCCTA
  51  CGAACGCTac cGCCaccGCC GCCTCATACA TGCCGTGCGG CTCGGCggaa
 101  ccgtCCTGTT CGCCACCGCA CTCGCCCGgc tACTCCACCT CCAacacggc
 151  gAATGGATAG GGAtgaCCGT CTTCGTCGTC CTCGGCATGC TCCAGTTCCA
 201  AGGCgcgatt tActccaacg cggtgGAacg taTGctcggt acggtcatcg
 251  ggctgGGCGC GGGTTTGGgc gTTTTATGGC TGAACCAGCA TTAtttccac
 301  ggcaacCTcc tcttctacct gaccatcggc acggcaagcg cactggccgg
 351  ctGGGCGGCG GTCGGCAAAA acggctacgt ccctatgctg GCGGGGctgA
 401  CGATGTGCAT gctcatcggc gACAACGGCA GCGAATGGCT CGACAGCGGC
 451  CTGATGCGCG CGATGAACGT CCTCATCGGC GCCGCCATCG CCATTGCCGC
 501  CGCCAAACTG CTGCCGCTGA AATCCACACT GATGTGGCGT TTCATGCTTG
 551  CCGACAACCT GGCCGACTGC AGCAAAATGA TTGCCGAAAT CAGCAACGGC
```

```
 601  AGGCGTATGA CGCGCGAACG TTTGGAGCAG AATATGGTCA AAATGCGCCA
 651  AATCAACGCA CGCATGGTCA AAAGCCGCAG CCACCTCGCC GCCACATCGG
 701  GCGAAAGCCG CATCAGCCCC TCCATGATGG AAGCCATGCA GCACGCCCAC
 751  CGCAAAATCG TCAACACCAC CGAGCTGCTC CTGACCACCG CCGCCAAGCT
 801  GCAATCTCCC AAACTCAACG GCAGCGAAAT CCGGCTGCTC GACCGCCACT
 851  TCACACTGCT CCAAACCGAC CTGCAACAAA CCGCCGCCCT CATCAACGGC
 901  AGACACGCCC GCCGCATCCG CATCGACACC GCCATCAACC CCGAACTGGA
 951  AGCCCTCGCC GAACACCTCC ACTACCAATG GCAGGGCTTC CTCTGGCTCA
1001  GCACCAATAT GCGTCAGGAA ATTTCCGCCC TCGTCATCCT GCTGCAACGC
1051  ACCCGCCGCA AATGGCTGGA TGCCCACGAA CGCCAACACC TGCGCCAAAG
1101  CCTGCTTGAA ACACGGGAAC ACGGCTGA
```

This corresponds to the amino acid sequence <SEQ ID 638; ORF146ng-1>:

```
   1  MNSSQRKRLS GRWLNSYERY RHRRLIHAVR LGGTVLFATA LARLLHLQHG
  51  EWIGMTVFVV LGMLQFQGAI YSNAVERMLG TVIGLGAGLG VLWLNQHYFH
 101  GNLLFYLTIG TASALAGWAA VGKNGYVPML AGLTMCMLIG DNGSEWLDSG
 151  LMRAMNVLIG AAIAIAAAKL LPLKSTLMWR FMLADNLADC SKMIAEISNG
 201  RRMTRERLEQ NMVKMRQINA RMVKSRSHLA ATSGESRISP SMMEAMQHAH
 251  RKIVNTTELL LTTAAKLQSP KLNGSEIRLL DRHFTLLQTD LQQTAALING
 301  RHARRIRIDT AINPELEALA EHLHYQWQGF LWLSTNMRQE ISALVILLQR
 351  TRRKWLDAHE RQHLRQSLLE TREHG*
```

ORF146ng-1 and ORF146-1 show 96.5% identity in 375 aa overlap

```
orf146-1.pep   MNTSQRNRLVSRWLNSYERYRYRRLIHAVRLGGAVLFATASARLLHLQHGEWIGMTVFVV
               ||:|||:|| :||||||||||:|||||||||||:|||||| |||||||||||||||||||
orf146ng-1     MNSSQRKRLSGRWLNSYERYRHRRLIHAVRLGGTVLFATALARLLHLQHGEWIGMTVFVV

orf146-1.pep   LGMLQFQGAIYSKAVERMLGTVIGLGAGLGVLWLNQHYFHGNLLFYLTVGTASALAGWAA
               ||||||||||||:|||||||||||||||||||||||||||||||||||:|||||||||||
orf146ng-1     LGMLQFQGAIYSNAVERMLGTVIGLGAGLGVLWLNQHYFHGNLLFYLTIGTASALAGWAA

orf146-1.pep   VGKNGYVPMLAGLTMCMLIGDNGSEWLDSGLMRAMNVLIGAAIAIAAAKLLPLKSTLMWR
               ||||||||||||||||||||||||||||||||||||||||||||||||||||||||||||
orf146ng-1     VGKNGYVPMLAGLTMCMLIGDNGSEWLDSGLMRAMNVLIGAAIAIAAAKLLPLKSTLMWR

orf146-1.pep   FMLADNLADCSKMIAEISNGRRMTRERLEENMAKMRQINARMVKSRSHLAATSGESRISP
               |||||||||||||||||||||||||||||:||:|||||||||||||||||||||||||||
orf146ng-1     FMLADNLADCSKMIAEISNGRRMTRERLEQNMVKMRQINARMVKSRSHLAATSGESRISP

orf146-1.pep   AMMEAMQHAHRKIVNTTELLLTTAAKLQSPKLNGSEIRLLDRHFTLLQTDLQQTVALING
               :|||||||||||||||||||||||||||||||||||||||||||||||||||||:|||||
orf146ng-1     SMMEAMQHAHRKIVNTTELLLTTAAKLQSPKLNGSEIRLLDRHFTLLQTDLQQTAALING

orf146-1.pep   RHARRIRIDTAINPELEALAEHLHYQWQGFLWLSTNMRQEISALVILLQRTRRKWLDAHE
               ||||||||||||||||||||||||||||||||||||||||||||||||||||||||||||
orf146ng-1     RHARRIRIDTAINPELEALAEHLHYQWQGFLWLSTNMRQEISALVILLQRTRRKWLDAHE

orf146-1.pep   RQHLRQSLLETREHGX
               ||||||||||||||||
orf146ng-1     RQHLRQSLLETREHGX
```

Furthermore, ORF146ng-1 shows homology with a hypothetical *E.coli* protein:

```
sp|P33011|YEEA_ECOLI HYPOTHETICAL 40.0 KD PROTEIN IN COBU-SBMC INTERGENIC REGION
>gi|1736674|gnl|PID|d1016553  (D90838)  ORF_ID:o348#20;  similar  to  [SwissProt
Accession Number P33011]  [Escherichia coli] >gi|1736682|gnl|PID|d1016560 (D90839)
ORF_ID:o348#20; similar to [SwissProt Accession Number P33011] [Escherichia coli]
>gi|1788318 (AE000292) f352; 100% identical to fragment YEEA_ECOLI SW: P33011 but
has 203 additional C-terminal residues [Escherichia coli] Length = 352
 Score =  109 bits (271), Expect = 2e-23
 Identities = 89/347 (25%), Positives = 150/347 (42%), Gaps = 21/347 (6%)

Query: 20  YRHRRLIHAVRLGGTVLFATALARLLHLQHGEWIGMTVFVVLGMLQFQGAIYSNAVERML 79
            YRH R++H  R+    L    + RL  +    W  +T+ V++G + F G +   A ER+
Sbjct: 15  YRHYRIVHGTRVALAFLLTFLIIRLFTIPESTWPLVTMVVIMGPISFWGNVVPRAFERIG 74

Query: 80  GTVIGLGAGLGVLWLNQHYFHGNLLFYLTIGTASALAGWAAVGKNGYVPMLAGLTMCMLI 139
            GTV+G   GL  L L         L  +    A  L GW A+GK  Y  +L G+T+ +++
Sbjct: 75  GTVLGSILGLIALQLE---LISLPLMLVWCAAAMFLCGWLALGKKPYQGLLIGVTLAIVV 131
```

```
Query: 140 GDNGSEWLDSGLMRAMNVLIGXXXXXXXXKLLPLKSTLMWRFMLADNLADCSKMIAEISN 199
           G    E +D+ L R+ +V++G         + P ++ + WR  LA +L + +++     +
Sbjct: 132 GSPTGE-IDTALWRSGDVILGSLLAMLFTGIWPQRAFIHWRIQLAKSLTEYNRVYQSAFS 190

Query: 200 GRRMTRERLEQNMVKMRQINARMVKSRSHLAATSGESRISPSMMEAMQHAHRKIVNXXXX 259
              + R RLE ++ K+       VK R  +A  S E+RI  S+ E +Q  +R +V
Sbjct: 191 PNLLERPRLESHLQKLL---TDAVKMRGLIAPASKETRIPKSIYEGIQTINRNLVCMLEL 247

Query: 260 XXXXXXXXQSPK---LNGSEIRLLDRHFXXXXXXXXXXAALINGRHARRIRIDTAINPEL 316
                   +      LN  ++R  D               AL  G            +N  +
Sbjct: 248 QINAYWATRPSHFVLLNAQKLR--DTQHMMQQILLSLVHALYEGNPQPVFANTEKLNDAV 305

Query: 317 EALAEHL--HYQWQ-------GFLWLSTNMRQEISALVILLQRTRRK 354
           E L + L   H+  +       G++WL+     ++  L  L+ R  RK
Sbjct: 306 EELRQLLNNHHDLKVVETPIYGYVWLNMETAHQLELLSNLICRALRK 352
```

On the basis of this analysis, including the identification of several transmembrane domains in the gonococcal protein, it is predicted that the proteins from *N.meningitidis* and *N.gonorrhoeae,* and their epitopes, could be useful antigens for vaccines or diagnostics, or for raising antibodies.

**Example 76**

The following partial DNA sequence was identified in *N.meningitidis* <SEQ ID 639>

```
  1  ..GCCGAAGACA CGCGCGTTAC CGCACAGCTT TTGAGCGCGT ACGGCATTCA
 51    GGGCAAACTC GTCAGTGTGC GCGAACACAA CGAACGGCAG ATGGCGGACA
101    AGATTGTCGG CTATCTTTCA GACGGCATGG TTGTGGCACA GGTTTCCGAT
151    GCGGGTACGC CGGCCGTGTG CGACCCGGGC GCGAAACTCG CCCGCCGCGT
201    GCGTGAGGCC GGGTTTAAAG TCGTTCCCGT CGTGGGCGCA AC.GCGGTGA
251    TGGCGGCTTT GAGCGTGGCC GGTGTGGAAG GATCCGATTT TTATTTCAAC
301    GGTTTTGTAC CGCCGAAATC GGGAGAACGC AGGAAACTGT TTGCCAAATG
351    GGTGCGGGCG GCGTTTCCTA TCGTCATGTT TGAAACGCCG CACCGCATCG
401    GTGCAGCGCT TGCCGATATG GCGGAACTGT TCCCCGAACG CCGATTAATG
451    CTGGCGCGCG AAATTACGAA AACGTTTGAA ACGTTCTTAA GCGGCACGGT
501    TGGGGAAATT CAGACGGCAT TGTCTGCCGA CGGCGACCAA TCGCGCGGCG
551    AGATGGTGTT GGTGCTTTAT CCGGCGCAGG ATGAAAAACA CGAAGGCTTG
601    TCCGAGTCCG CGCAAAACAT CATGAAAATC CTCACAGCCG AGCTGCCGAC
651    CAAACAGGCG GCGGAGCTTG CTGCCAAAAT CACGGGCGAG GGAAAGAAAG
701    CTTTGTACGA T..
```

This corresponds to the amino acid sequence <SEQ ID 640; ORF147>:

```
  1  ..AEDTRVTAQL LSAYGIQGKL VSVREHNERQ MADKIVGYLS DGMVVAQVSD
 51    AGTPAVCDPG AKLARRVREA GFKVVPVVGA XAVMAALSVA GVEGSDFYFN
101    GFVPPKSGER RKLFAKWVRA AFPIVMFETP HRIGAALADM AELFPERRLM
151    LAREITKTFE TFLSGTVGEI QTALSADGDQ SRGEMVLVLY PAQDEKHEGL
201    SESAQNIMKI LTAELPTKQA AELAAKITGE GKKALYD..
```

Further work revealed the complete nucleotide sequence <SEQ ID 641>:

```
  1  ATGTTTCAGA AACATTTGCA GAAAGCCTCC GACAGCGTCG TCGGAGGGAC
 51  ATTATACGTG GTTGCCACGC CCATCGGCAA TTTGGCGGAC ATTACCCTGC
101  GCGCTTTGGC GGTATTGCAA AAGGCGGACA TCATCTGTGC CGAAGACACG
151  CGCGTTACCG CACAGCTTTT GAGCGCGTAC GGCATTCAGG GCAAACTCGT
201  CAGTGTGCGC GAACACAACG AACGGCAGAT GGCGGACAAG ATTGTCGGCT
251  ATCTTTCAGA CGGCATGGTT GTGGCACAGG TTTCCGATGC GGGTACGCCG
301  GCCGTGTGCG ACCCGGGCGC GAAACTCGCC CGCCGCGTGC GTGAGGCCGG
351  GTTTAAAGTC GTTCCCGTCG TGGGCGCAAG CGCGGTGATG GCGGCTTTGA
401  GCGTGGCCGG TGTGGAAGGA TCCGATTTTT ATTTCAACGG TTTTGTACCG
451  CCGAAATCGG GAGAACGCAG GAAACTGTTT GCCAAATGGG TGCGGGCGGC
501  GTTTCCTATC GTCATGTTTG AAACGCCGCA CCGCATCGGT GCGACGCTTG
551  CCGATATGGC GGAACTGTTC CCCGAACGCC GATTAATGCT GGCGCGCGAA
601  ATTACGAAAA CGTTTGAAAC GTTCTTAAGC GGCACGGTTG GGGAAATTCA
651  GACGGCATTG TCTGCCGACG GCAACCAATC GCGCGGCGAG ATGGTGTTGG
701  TGCTTTATCC GGCGCAGGAT GAAAAACACG AAGGCTTGTC CGAGTCCGCG
751  CAAAACATCA TGAAAATCCT CACAGCCGAG CTGCCGACCA AACAGGCGGC

801  GGAGCTTGCT GCCAAAATCA CGGGCGAGGG AAAGAAAGCT TTGTACGATC
851  TGGCTCTGTC TTGGAAAAAC AAATAG
```

This corresponds to the amino acid sequence <SEQ ID 642; ORF147-1>:

```
  1  MFQKHLQKAS DSVVGGTLYV VATPIGNLAD ITLRALAVLQ KADIICAEDT
 51  RVTAQLLSAY GIQGKLVSVR EHNERQMADK IVGYLSDGMV VAQVSDAGTP
101  AVCDPGAKLA RRVREAGFKV VPVVGASAVM AALSVAGVEG SDFYFNGFVP
151  PKSGERRKLF AKWVRAAFPI VMFETPHRIG ATLADMAELF PERRLMLARE
201  ITKTFETFLS GTVGEIQTAL SADGNQSRGE MVLVLYPAQD EKHEGLSESA
251  QNIMKILTAE LPTKQAAELA AKITGEGKKA LYDLALSWKN K*
```

Computer analysis of this amino acid sequence gave the following results:

Homology with hypothetical protein ORF286 of *E. coli* (accession number U18997)

ORF147 and *E.coli* ORF286 protein show 36% aa identity in 237aa overlap:

```
Orf147: 1   AEDTRVTAQLLSAYGIQGKLVSVREHNERQMADKIVGYLSDGMVVAQVSDAGTPAVCDPG 60
            AEDTR T  LL  +GI  +L ++ +HNE+Q A+ ++  L +G  +A VSDAGTP + DPG
Orf286: 43  AEDTRHTGLLLQHFGINARLFALHDHNEQQKAETLLAKLQEGQNIALVSDAGTPLINDPG 102

Orf147: 61  AKLARRVREXXXXXXXXXXXXXXXXXXXXXXXXXEGSDFYFNGFVPPKSGERRKLFAKWVRA 120
              L R  RE                         F + GF+P KS  RR
Orf286: 103 YHLVRTCREAGIRVVPLPGPCAAITALSAAGLPSDRFCYEGFLPAKSKGRRDALKAIEAE 162

Orf147: 121 AFPIVMFETPHRIGAALADMAELFPERR-LMLAREITKTFETFLSGTVGEIQTALSADGD 179
              ++ +E+ HR+  +L D+  +  E R ++LARE+TKT+ET     VGE+   +  D +
Orf286: 163 PRTLIFYESTHRLLDSLEDIVAVLGESRYVVLARELTKTWETIHGAPVGELLAWVKEDEN 222

Orf147: 180 QSRGEMVLVLYPAQDEKHEGLSESAQNIMKILTAELPTKQAAELAAKITGEGKKALY 236
            + +GEMVL++      + E L   A   + +L AELP K+AA LAA+I G  K ALY
Orf286: 223 RRKGEMVLIV-EGHKAQEEDLPADALRTLALLQAELPLKKAAALAAEIHGVKKNALY 278
```

Homology with a predicted ORF from *N.meningitidis* strain A)

ORF147 shows 96.6% identity over a 237aa overlap with ORF75a from strain A of *N. meningitidis:*

```
                                                   10        20        30
orf147.pep                               AEDTRVTAQLLSAYGIQGKLVSVREHNERQ
                                         ||||||||||||||||||||||||||||||
orf75a     TLYVVATPIGNLADITLRALAVLQKADIICAEDTRVTAQLLSAYGIQGKLVSVREHNERQ
             20        30        40        50        60        70

                   40        50        60        70        80        90
orf147.pep MADKIVGYLSDGMVVAQVSDAGTPAVCDPGAKLARRVREAGFKVVPVVGAXAVMAALSVA
           |||||||||||||||||||||||||||||||||||||||:||||||||| ||||||||
orf75a     MADKIVGYLSDGMVVAQVSDAGTPAVCDPGAKLARRVREVGFKVVPVVGASAVMAALSVA
             80        90       100       110       120       130

                  100       110       120       130       140       150
orf147.pep GVEGSDFYFNGFVPPKSGERRKLFAKWVRAAFPIVMFETPHRIGAALADMAELFPERRLM
           || |||||||||||||||||||||||||:|||:||||||||||||:||||||||||||||
orf75a     GVAGSDFYFNGFVPPKSGERRKLFAKWVRVAFPVVMFETPHRIGATLADMAELFPERRLM
             140       150       160       170       180       190

                  160       170       180       190       200       210
orf147.pep LAREITKTFETFLSGTVGEIQTALSADGDQSRGEMVLVLYPAQDEKHEGLSESAQNIMKI
           ||||||||||||||||||||||||:|||:|||||||||||||||||||||||||||||||
orf75a     LAREITKTFETFLSGTVGEIQTALAADGNQSRGEMVLVLYPAQDEKHEGLSESAQNIMKI
             200       210       220       230       240       250

                  220       230
orf147.pep LTAELPTKQAAELAAKITGEGKKALYD
           |||||||||||||||||||||||||||
orf75a     LTAELPTKQAAELAAKITGEGKKALYDLALSWKNKX
             260       270       280       290
```

ORF147a is identical to ORF75a, which includes aa 56-292 of ORF75.

Homology with a predicted ORF from *N.gonorrhoeae*

ORF147 shows 94.1% identity over a 237aa overlap with a predicted ORF (ORF147ng) from *N. gonorrhoeae:*

```
orf147.pep                               AEDTRVTAQLLSAYGIQGKLVSVREHNERQ   30
                                         ||||||||||||||||||:|||||||||||
orf147ng   TLYVVATPIGNLADITLRALAVLQKADIICAEDTRVTAQLLSAYGIQGRLVSVREHNERQ   85

orf147.pep MADKIVGYLSDGMVVAQVSDAGTPAVCDPGAKLARRVREAGFKVVPVVGAXAVMAALSVA   90
           ||||::|:||||:|||||||||||||||||||||||||||||||||||||| ||||||||
orf147ng   MADKVIGFLSDGLVVAQVSDAGTPAVCDPGAKLARRVREAGFKVVPVVGASAVMAALSVA  145

orf147.pep GVEGSDFYFNGFVPPKSGERRKLFAKWVRAAFPIVMFETPHRIGAALADMAELFPERRLM  150
           || |||||||||||||||||||||||||||||||:||||||||||:||||||||||||||
orf147ng   GVAESDFYFNGFVPPKSGERRKLFAKWVRAAFPVVMFETPHRIGATLADMAELFPERRLM  205

orf147.pep LAREITKTFETFLSGTVGEIQTALSADGDQSRGEMVLVLYPAQDEKHEGLSESAQNIMKI  210
           ||||||||||||||||||||||||:|||:||||||||||||||||||||||||||| |||
orf147ng   LAREITKTFETFLSGTVGEIQTALAADGNQSRGEMVLVLYPAQDEKHEGLSESAQNAMKI  265

orf147.pep LTAELPTKQAAELAAKITGEGKKALYD         237
           |:|||||||||||||||||||||||||
orf147ng   LAAELPTKQAAELAAKITGEGKKALYDLALSWKNK  300
```

An ORF147ng nucleotide sequence <SEQ ID 643> was predicted to encode a protein having amino acid sequence <SEQ ID 644>:

```
  1 MSVFQTAFFM FQKHLQKASD SVVGGTLYVV ATPIGNLADI TLRALAVLQK
 51 ADIICAEDTR VTAQLLSAYG IQGRLVSVRE HNERQMADKV IGFLSDGLVV
101 AQVSDAGTPA VCDPGAKLAR RVREAGFKVV PVVGASAVMA ALSVAGVAES
151 DFYFNGFVPP KSGERRKLFA KWVRAAFPVV MFETPHRIGA TLADMAELFP
201 ERRLMLAREI TKTFETFLSG TVGEIQTALA ADGNQSRGEM VLVLYPAQDE
251 KHEGLSESAQ NAMKILAAEL PTKQAAELAA KITGEGKKAL YDLALSWKNK
301 *
```

Further work revealed the following gonococcal DNA sequence <SEQ ID 645>:

```
  1 ATGTTTCAGA AACACTTGCA GAAAGCCTCC GACAGCGTCG TCGGAGGGAC
 51 ATTATACGTG GTTGCCACGC CCATCGGCAA TTTGGCAGAC ATTACCCTGC
101 GCGCTTTGGC GGTATTGCAA AAGGCGGACA TCATTTGTGC CGAAGACACG
151 CGCGTTACTG CGCAGCTTTT GAGCGCGTAC GGCATTCAGG GCAGGTTGGT
201 CAGTGTGCGC GAACACAACG AGCGGCAGAT GGCGGACAAG GTAATCGGTT
251 TCCTTTCAGA CGGCCTGGTT GTGGCGCAGG TTTCCGATGC GGGTACGCCG
301 GCCGTGTGCG ACCCGGGCGC GAAACTCGCC CGCCGCGTGC GCGAAGCAGG
351 GTTCAAAGTC GTTCCCGTCG TGGGCGCAAG CGCGGTAATG GCGGCGTTGA
401 GTGTGGCCGG TGTGGCGGAA TCCGATTTTT ATTTCAACGG TTTTGTACCG
451 CCGAAATCGG GCGAACGTAG GAAATTGTTT GCCAAATGGG TGCGGGCGGC
501 ATTTCCTGTC GTCATGTTTG AAACGCCGCA CCGAATCGGG GCAACGCTTG
551 CCGATATGGC GGAATTGTTC CCCGAACGCC GTCTGATGCT GGCGCGCGAA
601 ATCACGAAAA CGTTTGAAAC GTTCTTAAGC GGCACGGTTG GGAAATTCA
651 GACGGCATTG GCGGCGGACG GCAACCAATC GCGCGGCGAG ATGGTGTTGG
701 TGCTTTATCC GGCGCAGGAT GAAAAACACG AAGGCTTGTC CGAGTCTGCG
751 CAAAATGCGA TGAAAATCCT TGCGGCCGAG CTGCCGACCA AGCAGGCGGC
801 GGAGCTTGCC GCCAAGATTA CAGGTGAGGG CAAAAAGGCT TTGTACGATT
851 TGGCACTGTC GTGGAAAAAC AAATGA
```

This corresponds to the amino acid sequence <SEQ ID 646; ORF147ng-1>:

```
  1 MFQKHLQKAS DSVVGGTLYV VATPIGNLAD ITLRALAVLQ KADIICAEDT
 51 RVTAQLLSAY GIQGRLVSVR EHNERQMADK VIGFLSDGLV VAQVSDAGTP
101 AVCDPGAKLA RRVREAGFKV VPVVGASAVM AALSVAGVAE SDFYFNGFVP
151 PKSGERRKLF AKWVRAAFPV VMFETPHRIG ATLADMAELF PERRLMLARE
201 ITKTFETFLS GTVGEIQTAL AADGNQSRGE MVLVLYPAQD EKHEGLSESA
251 QNAMKILAAE LPTKQAAELA AKITGEGKKA LYDLALSWKN K*
```

ORF147ng shows homology to a hypothetical *E.coli* protein:

```
sp|P45528|YRAL_ECOLI HYPOTHETICAL 31.3 KD PROTEIN IN AGAI-MTR INTERGENIC REGION
(F286)
>gi|606086 (U18997) ORF_f286 [Escherichia coli]
>gi|1789535 (AE000395) hypothetical 31.3 kD protein in agai-mtr intergenic region
[Escherichia coli] Length = 286
 Score =  218 bits (550), Expect = 3e-56
 Identities = 128/284 (45%), Positives = 171/284 (60%), Gaps = 4/284 (1%)

Query: 4   KHLQKASDSVVGGTLYVVATPIGNLADITLRALAVLQKADIICAEDTRVTAQLLSAYGIQ 63
            K  Q A +S   G LY+V TPIGNLADIT RAL VLQ  D+I AEDTR T  LL  +GI
Sbjct: 2   KQHQSADNSQ--GQLYIVPTPIGNLADITQRALEVLQAVDLIAAEDTRHTGLLLQHFGIN 59

Query: 64  GRLVSVREHNERQMADKVIGFLSDGLVVAQVSDAGTPAVCDPGAKLARRVREAGFKVVPV 123
             RL ++ +HNE+Q A+ ++  L +G  +A VSDAGTP + DPG  L R  REAG +VVP+
Sbjct: 60  ARLFALHDHNEQQKAETLLAKLQEGQNIALVSDAGTPLINDPGYHLVRTCREAGIRVVPL 119

Query: 124 VGASAVMAALSVAGVAESDFYFNGFVPPKSGERRKLFAKWVRAAFPVVMFETPHRIGATL 183
            G  A + ALS AG+     F + GF+P KS  RR            ++ +E+ HR+  +L
Sbjct: 120 PGPCAAITALSAAGLPSDRFCYEGFLPAKSKGRRDALKAIEAEPRTLIFYESTHRLLDSL 179

Query: 184 ADMAELFPERR-LMLAREITKTFETFLSGTVGEIQTALAADGNQSRGEMVLVLYPAQDEK 242
            D+  +  E R ++LARE+TKT+ET     VGE+   +  D N+ +GEMVL++      +
Sbjct: 180 EDIVAVLGESRYVVLARELTKTWETIHGAPVGELLAWVKEDENRRKGEMVLIV-EGHKAQ 238

Query: 243 HEGLSESAQNAMKILAAELPTKQAAELAAKITGEGKKALYDLAL 286
             E L   A   + +L AELP K+AA LAA+I G  K ALY  AL
Sbjct: 239 EEDLPADALRTLALLQAELPLKKAAALAAEIHGVKKNALYKYAL 282
```

Based on the computer analysis and the presence of a putative transmembrane domain in the gonococcal protein, it is predicted that these proteins from *N.meningitidis* and *N.gonorrhoeae,* and their epitopes, could be useful antigens for vaccines or diagnostics, or for raising antibodies.

**Example 77**

The following partial DNA sequence was identified in *N.meningitidis* <SEQ ID 647>

```
   1  ATGAAAACAA CCGACAAACG GACAACCGAA ACACACCGCA AAGCCCCGAA
  51  AACCGGTCGC ATCCGCTTCT C.GCTGCTTA CTTAGCCATA TGCCTGTCGT
 101  TCGGCATTCT TCCCCAAGCC TGGGCGGGAC ACACTTATTT CGGCATCAAC
 151  TACCAATACT ATCGCGACTT TGCCGAAAAT AAAGGCAAGT TTGCAGTCGG
 201  GGCGAAAGAT ATTGAGGTTT ACAACAAAAA AGGGGAGTTG GTCGGCAAAT
 251  CAATGACAAA AGCCCCGATG ATTGATTTTT CTGTGGTGTC GCGTAACGGC
 301  GTGGCGGcAT TGGTGGGCGt ATCAATATAT TGTGAGCGTG GCACATAACG
 351  GCGGCTATAA CAACGTTGAT TTTGGTGCGG AAGGAAk.AA tATCCC.GAT
 401  CAACAwCGww TTACTTATAA AATTGTGAAA CGGAATAATT ATAAAGCAGG
 451  GACTAAAGGC CATCCTTATG GCGGCGATTA TCATATGCCG CGTTTGCATA
 501  AATwTGTCAC AGATGCAGAA CCTGTTGAAA TGACCAGTTA TATGGATGGG
 551  CGGAAATATA TCGATCAAAA TAATTACCCT GACCGTGTTC GTATTGGGGC
 601  AGGCAGGCAA TATTGGCGAT CTGATGAAGA TGAGCCCAAT AACCGCGAAA
 651  GTTCATATCA TATTGCAAGT .......... .......... ..........
 701  .......... .....GGCTC ACCAATGTTT ATCTATGATG CCCAAAAGCA
 751  AAAGTGGTTA ATTAATGGGG TATTGCAAAC GGGCAACCCC TATATAGGAA
 801  AAAGCAATGG CTTCCAGCTG GTTCGTAAAG ATTGGTTCTA TGATGAAATC
 851  TTTGCTGGAG ATACCCATTC AGTATTCTAC GAACCACGTC AAAATGGGAA
 901  ATACTCTTTT AACGACGATA ATAATGGCAC AGGAAAAATC AATGCCAAAC
 951  ATGAACACAA TTCTCTGCCT AATAGATTAA AAACACGAAC CGTTCAATTG
1001  TTTAATGTTT CTTTATCCGA GACAGCAAGA GAACCTGTTT ATCATGCTGC
1051  AGGTGGTGTC AACAGTTATC GACCCAGACT GAATAATGGA GAAAATATTT
1101  CCTTTATTGA CGAAGGAAAA GGCGAATTGA TACTTACCAG CAACATCAAT
1151  CAAGGTGCTG GAGGATTATA TTTCCAAGGA GATTTTACGG TCTCGCCTGA
1201  AAATAACGAA ACTTGGCAAG GCGCGGGCGT TCATATCAGT GAAGACAGTA
1251  CCGTTACTTG GAAAGTAAAC GGCGTGGCAA ACGACCGCCT GTCCAAAATC
1301  GGCAAAGGCA CGCTG..... .......... .......... ..........
                                   //
2101  .......... .......... .......... .......... ...GATAAAG
2151  TGACTGCTTC ATTGACTAAG ACCGACATCA GCGGCAATGT CGATCTTGCC
2201  GATCACGCTC ATTTAAATCT CACAGGGCTT GCCACACTCA ACGGCAATCT
```

```
2251  TAGTGCAAAT GGCGATACAC GTTATACAGT CAGCCACAAC GCCACCCAAA
2301  ACGGCAACCk TAgCCtCGtG G.sAATGcCC AAGCAACATT TAATCAAGCC
2351  ACATTAAACG GCAACACATC GGCTTCgGGC AATGCTTCAT TTAATCTAAG
2401  CGACCACGCC GTACAAAACG GCAGTCTGAC GCTTTCCGGC AACGCTAAGG
2451  CAAACGTAAG CCATTCCGCA CTCAACGGTA ATGTCTCCCT AGCCGATAAG
2501  GCAGTATTCC ATTTTGAAAG CAGCCGCTTT ACCGGACAAA TCAGCGGCGG
2551  CAagGATACG GCATTACACT TAAAAGACAG CGAATGGACG CTGCCGTCAg
2601  GarCGGAATT AGGCAATTTA AACCTTGACA ACGCCACCAT TACaCTCAAT
2651  TCCGCCTATC GCCACGATGC GGCAGGGGCG CAAACCGGCA GTGCGACAGA
2701  TGCGCCGCGC CGCCGTTCGC GCCGTTCGCG CCGTTCCCTA TTATmCGTTA
2751  CACCGCCAAC TTCGGTAGAA TCCCGTTTCA ACACGCTGAC GGTAAACGGC
2801  AAATTGAACG GTCAGGGAAC ATTCCGCTTT ATGTCGGAAC TCTTCGGCTA
2851  CCGCAGCGAC AAATTGAAGC TGGCGGAAAG TTCCGAAGGC ACTTACACCT
2901  TGGCGGTCAA CAATACCGGC AACGAACCTG CAAGCCTCGA ACAATTGACG
2951  GTAGTGGAAG GAAAAGACAA CAAACCGCTG TCCGAAAACC TTAATTTCAC
3001  CCTGCAAAAC GAACACGTCG ATGCAGGCGC GTGG...... ..........
                                      //
3551  .......... .......... ....TTAGAC CGCGTATTTG CCGAAGACCG
3601  CCGCAACGCC GTTTGGACAA GCGGCATCCG GGACACCAAA CACTACCGTT
3651  CGCAAGATTT CCGCGCCTAC CGCCAACAAA CCGACCTGCG CCAAATCGGT
3701  ATGCAGAAAA ACCTCGGCAG CGGGCGCGTC GGCATCCTGT TTTCGCACAA
3751  CCGGACCGAA AACACCTTCG ACGACGGCAT CGGCAACTCG GCACGGCTTG
3801  CCCACGGCGC CGTTTTCGGG CAATACGGCA TCGACAGGTT CTACATCGGC
3851  ATCAGnCGCG GGCGCGGGTT TTAGCAGCGG CAGCCTTTcA GACGGCATCG
3901  GAGsmAAAwT CCGCCGCCGC GTGCtGCATT ACGGCATTCA GGCACGAtAC
3951  CGCGCCGgtt tCggCGgATt CGGCATCGAA CCGCACATCG GCGCAACGCg
4001  ctATTTCGTC CAAAAAGCGG ATTACCGCTA CGAAAACGTC AATATCGCCA
4051  CCCCCGGCCT TGCATTCAAC CGcTACCGCG CGGGCATTAa GGCAGATTAT
4101  TCATTCAAAC CGGCGCAACA CATTTCCATC ACGCCTTATT TGAGCCTGTC
4151  CTATACCGAT GCCGCTTCGG GCAAAGTCCG AACACGCGTC AATACCGCCG
4201  TATTGGCTCA GGATTTCGGC AAAACCCGCA GTGCGGAATG GGgCGTAAAC
4251  GCCGAAATCA AAGGTTTCAC GCTGTCCCTC CACGCTGCCG CCGCCAAAGG
4301  CCCGCAACTG GAAGCGCAAC ACAGCGCGGG CATCAAATTA GGCTACCGCT
4351  GGTAA...
```

This corresponds to the amino acid sequence <SEQ ID 648; ORF1>:

```
   1  MKTTDKRTTE THRKAPKTGR IRFXAAYLAI CLSFGILPQA WAGHTYFGIN
  51  YQYYRDFAEN KGKFAVGAKD IEVYNKKGEL VGKSMTKAPM IDFSVVSRNG
 101  VAALVGVQYI VSVAHNGGYN NVDFGAEGXN IXDQXRXTYK IVKRNNYKAG
 151  TKGHPYGGDY HMPRLHKXVT DAEPVEMTSY MDGRKYIDQN NYPDRVRIGA
 201  GRQYWRSDED EPNNRESSYH IAS....... ........GS PMFIYDAQKQ
 251  KWLINGVLQT GNPYIGKSNG FQLVRKDWFY DEIFAGDTHS VFYEPRQNGK
 301  YSFNDDNNGT GKINAKHEHN SLPNRLKTRT VQLFNVSLSE TAREPVYHAA
 351  GGVNSYRPRL NNGENISFID EGKGELILTS NINQGAGGLY FQGDFTVSPE
 401  NNETWQGAGV HISEDSTVTW KVNGVANDRL SKIGKGTL.. ..........
                                    //
 701  .......... ....DKVTAS LTKTDISGNV DLADHAHLNL TGLATLNGNL
 751  SANGDTRYTV SHNATQNGNX SLVXNAQATF NQATLNGNTS ASGNASFNLS
 801  DHAVQNGSLT LSGNAKANVS HSALNGNVSL ADKAVFHFES SRFTGQISGG
 851  KDTALHLKDS EWTLPSGXEL GNLNLDNATI TLNSAYRHDA AGAQTGSATD
 901  APRRRSRRSR RSLLXVTPPT SVESRFNTLT VNGKLNGQGT FRFMSELFGY
 951  RSDKLKLAES SEGTYTLAVN NTGNEPASLE QLTVVEGKDN KPLSENLNFT
1001  LQNEHVDAGA W......... .......... .......... ..........
                                    //
1151  .......... .......... .......... .......... .LDRVFAEDR
1201  RNAVWTSGIR DTKHYRSQDF RAYRQQTDLR QIGMQKNLGS GRVGILFSHN
1251  RTENTFDDGI GNSARLAHGA VFGQYGIDRF YIGISAGAGF SSGSLSDGIG
1301  XKXRRRVLHY GIQARYRAGF GGFGIEPHIG ATRYFVQKAD YRYENVNIAT
1351  PGLAFNRYRA GIKADYSFKP AQHISITPYL SLSYTDAASG KVRTRVNTAV
1401  LAQDFGKTRS AEWGVNAEIK GFTLSLHAAA AKGPQLEAQH SAGIKLGYRW
1451  *
```

Further sequencing analysis revealed the complete nucleotide sequence <SEQ ID 649>:

```
  1  ATGAAAACAA CCGACAAACG GACAACCGAA ACACACCGCA AAGCCCCGAA
 51  AACCGGCCGC ATCCGCTTCT CGCCTGCTTA CTTAGCCATA TGCCTGTCGT
101  TCGGCATTCT TCCCCAAGCC TGGGCGGGAC ACACTTATTT CGGCATCAAC
151  TACCAATACT ATCGCGACTT TGCCGAAAAT AAAGGCAAGT TTGCAGTCGG
201  GGCGAAAGAT ATTGAGGTTT ACAACAAAAA AGGGGAGTTG GTCGGCAAAT
251  CAATGACAAA AGCCCCGATG ATTGATTTTT CTGTGGTGTC GCGTAACGGC
301  GTGGCGGCAT TGGTGGGCGA TCAATATATT GTGAGCGTGG CACATAACGG
```

```
 351 CGGCTATAAC AACGTTGATT TTGGTGCGGA AGGAAGAAAT CCCGATCAAC
 401 ATCGTTTTAC TTATAAAATT GTGAAACGGA ATAATTATAA AGCAGGGACT
 451 AAAGGCCATC CTTATGGCGG CGATTATCAT ATGCCGCGTT TGCATAAATT
 501 TGTCACAGAT GCAGAACCTG TTGAAATGAC CAGTTATATG GATGGGCGGA
 551 AATATATCGA TCAAAATAAT TACCCTGACC GTGTTCGTAT TGGGGCAGGC
 601 AGGCAATATT GGCGATCTGA TGAAGATGAG CCCAATAACC GCGAAAGTTC
 651 ATATCATATT GCAAGTGCGT ATTCTTGGCT CGTTGGTGGC AATACCTTTG
 701 CACAAAATGG ATCAGGTGGT GGCACAGTCA ACTTAGGTAG TGAAAAAATT
 751 AAACATAGCC CATATGGTTT TTTACCAACA GGAGGCTCAT TTGGCGACAG
 801 TGGCTCACCA ATGTTTATCT ATGATGCCCA AAAGCAAAAG TGGTTAATTA
 851 ATGGGGTATT GCAAACGGGC AACCCCTATA TAGGAAAAAG CAATGGCTTC
 901 CAGCTGGTTC GTAAAGATTG GTTCTATGAT GAAATCTTTG CTGGAGATAC
 951 CCATTCAGTA TTCTACGAAC CACGTCAAAA TGGGAAATAC TCTTTTAACG
1001 ACGATAATAA TGGCACAGGA AAAATCAATG CCAAACATGA ACACAATTCT
1051 CTGCCTAATA GATTAAAAAC ACGAACCGTT CAATTGTTTA ATGTTTCTTT
1101 ATCCGAGACA GCAAGAGAAC CTGTTTATCA TGCTGCAGGT GGTGTCAACA
1151 GTTATCGACC CAGACTGAAT AATGGAGAAA ATATTTCCTT TATTGACGAA
1201 GGAAAAGGCG AATTGATACT TACCAGCAAC ATCAATCAAG GTGCTGGAGG
1251 ATTATATTTC CAAGGAGATT TTACGGTCTC GCCTGAAAAT AACGAAACTT
1301 GGCAAGGCGC GGGCGTTCAT ATCAGTGAAG ACAGTACCGT TACTTGGAAA
1351 GTAAACGGCG TGGCAAACGA CCGCCTGTCC AAAATCGGCA AAGGCACGCT
1401 GCACGTTCAA GCCAAAGGGG AAAACCAAGG CTCGATCAGC GTGGGCGACG
1451 GTACAGTCAT TTTGGATCAG CAGGCAGACG ATAAAGGCAA AAAACAAGCC
1501 TTTAGTGAAA TCGGCTTGGT CAGCGGCAGG GGTACGGTGC AACTGAATGC
1551 CGATAATCAG TTCAACCCCG ACAAACTCTA TTTCGGCTTT CGCGGCGGAC
1601 GTTTGGATTT AAACGGGCAT TCGCTTTCGT TCCACCGTAT TCAAAATACC
1651 GATGAAGGGG CGATGATTGT CAACCACAAT CAAGACAAAG AATCCACCGT
1701 TACCATTACA GGCAATAAAG ATATTGCTAC AACCGGCAAT AACAACAGCT
1751 TGGATAGCAA AAAAGAAATT GCCTACAACG GTTGGTTTGG CGAGAAAGAT
1801 ACGACCAAAA CGAACGGGCG GCTCAACCTT GTTTACCAGC CCGCCGCAGA
1851 AGACCGCACC CTGCTGCTTT CCGGCGGAAC AAATTTAAAC GGCAACATCA
1901 CGCAAACAAA CGGCAAACTG TTTTTCAGCG GCAGACCAAC ACCGCACGCC
1951 TACAATCATT TAAACGACCA TTGGTCGCAA AAAGAGGGCA TTCCTCGCGG
2001 GGAAATCGTG TGGGACAACG ACTGGATCAA CCGCACATTT AAAGCGGAAA
2051 ACTTCCAAAT TAAAGGCGGA CAGGCGGTGG TTTCCCGCAA TGTTGCCAAA
2101 GTGAAAGGCG ATTGGCATTT GAGCAATCAC GCCCAAGCAG TTTTTGGTGT
2151 CGCACCGCAT CAAAGCCACA CAATCTGTAC ACGTTCGGAC TGGACGGGTC
2201 TGACAAATTG TGTCGAAAAA ACCATTACCG ACGATAAAGT GATTGCTTCA
2251 TTGACTAAGA CCGACATCAG CGGCAATGTC GATCTTGCCG ATCACGCTCA
2301 TTTAAATCTC ACAGGGCTTG CCACACTCAA CGGCAATCTT AGTGCAAATG
2351 GCGATACACG TTATACAGTC AGCCACAACG CCACCCAAAA CGGCAACCTT
2401 AGCCTCGTGG GCAATGCCCA AGCAACATTT AATCAAGCCA CATTAAACGG
2451 CAACACATCG GCTTCGGGCA ATGCTTCATT TAATCTAAGC GACCACGCCG
2501 TACAAAACGG CAGTCTGACG CTTTCCGGCA ACGCTAAGGC AAACGTAAGC
2551 CATTCCGCAC TCAACGGTAA TGTCTCCCTA GCCGATAAGG CAGTATTCCA
2601 TTTTGAAAGC AGCCGCTTTA CCGGACAAAT CAGCGGCGGC AAGGATACGG
2651 CATTACACTT AAAAGACAGC GAATGGACGC TGCCGTCAGG CACGGAATTA
2701 GGCAATTTAA ACCTTGACAA CGCCACCATT ACACTCAATT CCGCCTATCG
2751 CCACGATGCG GCAGGGGCGC AAACCGGCAG TGCGACAGAT GCGCCGCGCC
2801 GCCGTTCGCG CCGTTCGCGC CGTTCCCTAT TATCCGTTAC ACCGCCAACT
2851 TCGGTAGAAT CCCGTTTCAA CACGCTGACG GTAAACGGCA AATTGAACGG
2901 TCAGGGAACA TTCCGCTTTA TGTCGGAACT CTTCGGCTAC CGCAGCGACA
2951 AATTGAAGCT GGCGGAAAGT TCCGAAGGCA CTTACACCTT GGCGGTCAAC
3001 AATACCGGCA ACGAACCTGC AAGCCTCGAA CAATTGACGG TAGTGGAAGG
3051 AAAAGACAAC AAACCGCTGT CCGAAAACCT TAATTTCACC CTGCAAAACG
3101 AACACGTCGA TGCCGGCGCG TGGCGTTACC AACTCATCCG CAAAGACGGC
3151 GAGTTCCGCC TGCATAATCC GGTCAAAGAA CAAGAGCTTT CCGACAAACT
3201 CGGCAAGGCA GAAGCCAAAA AACAGGCGGA AAAAGACAAC GCGCAAAGCC
3251 TTGACGCGCT GATTGCGGCC GGGCGCGATG CCGTCGAAAA GACAGAAAGC
3301 GTTGCCGAAC CGGCCCGGCA GGCAGGCGGG GAAAATGTCG GCATTATGCA
3351 GGCGGAGGAA GAGAAAAAAC GGGTGCAGGC GGATAAAGAC ACCGCCTTGG
3401 CGAAACAGCG CGAAGCGGAA ACCCGGCCGG CTACCACCGC CTTCCCCCGC
3451 GCCCGCCGCG CCCGCCGGGA TTTGCCGCAA CTGCAACCCC AACCGCAGCC
3501 CCAACCGCAG CGCGACCTGA TCAGCCGTTA TGCCAATAGC GGTTTGAGTG
3551 AATTTTCCGC CACGCTCAAC AGCGTTTTCG CCGTACAGGA CGAATTAGAC
3601 CGCGTATTTG CCGAAGACCG CCGCAACGCC GTTTGGACAA GCGGCATCCG
3651 GGACACCAAA CACTACCGTT CGCAAGATTT CCGCGCCTAC CGCCAACAAA
3701 CCGACCTGCG CCAAATCGGT ATGCAGAAAA ACCTCGGCAG CGGGCGCGTC
3751 GGCATCCTGT TTTCGCACAA CCGGACCGAA AACACCTTCG ACGACGGCAT
3801 CGGCAACTCG GCACGGCTTG CCCACGGCGC CGTTTTCGGG CAATACGGCA
3851 TCGACAGGTT CTACATCGGC ATCAGCGCGG GCGCGGGTTT TAGCAGCGGC
3901 AGCCTTTCAG ACGGCATCGG AGGCAAAATC CGCCGCCGCG TGCTGCATTA
```

```
3951  CGGCATTCAG GCACGATACC GCGCCGGTTT CGGCGGATTC GGCATCGAAC
4001  CGCACATCGG CGCAACGCGC TATTTCGTCC AAAAAGCGGA TTACCGCTAC
4051  GAAAACGTCA ATATCGCCAC CCCCGGCCTT GCATTCAACC GCTACCGCGC
4101  GGGCATTAAG GCAGATTATT CATTCAAACC GGCGCAACAC ATTTCCATCA
4151  CGCCTTATTT GAGCCTGTCC TATACCGATG CCGCTTCGGG CAAAGTCCGA
4201  ACACGCGTCA ATACCGCCGT ATTGGCTCAG GATTTCGGCA AAACCCGCAG
4251  TGCGGAATGG GGCGTAAACG CCGAAATCAA AGGTTTCACG CTGTCCCTCC
4301  ACGCTGCCGC CGCCAAAGGC CCGCAACTGG AAGCGCAACA CAGCGCGGGC
4351  ATCAAATTAG GCTACCGCTG GTAA
```

This corresponds to the amino acid sequence <SEQ ID 650; ORF1-1>:

```
   1  MKTTDKRTTE THRKAPKTGR IRFSPAYLAI CLSFGILPQA WAGHTYFGIN
  51  YQYYRDFAEN KGKFAVGAKD IEVYNKKGEL VGKSMTKAPM IDFSVVSRNG
 101  VAALVGDQYI VSVAHNGGYN NVDFGAEGRN PDQHRFTYKI VKRNNYKAGT
 151  KGHPYGGDYH MPRLHKFVTD AEPVEMTSYM DGRKYIDQNN YPDRVRIGAG
 201  RQYWRSDEDE PNNRESSYHI ASAYSWLVGG NTFAQNGSGG GTVNLGSEKI
 251  KHSPYGFLPT GGSFGDSGSP MFIYDAQKQK WLINGVLQTG NPYIGKSNGF
 301  QLVRKDWFYD EIFAGDTHSV FYEPRQNGKY SFNDDNNGTG KINAKHEHNS
 351  LPNRLKTRTV QLFNVSLSET AREPVYHAAG GVNSYRPRLN NGENISFIDE
 401  GKGELILTSN INQGAGGLYF QGDFTVSPEN NETWQGAGVH ISEDSTVTWK
 451  VNGVANDRLS KIGKGTLHVQ AKGENQGSIS VGDGTVILDQ QADDKGKKQA
 501  FSEIGLVSGR GTVQLNADNQ FNPDKLYFGF RGGRLDLNGH SLSFHRIQNT
 551  DEGAMIVNHN QDKESTVTIT GNKDIATTGN NNSLDSKKEI AYNGWFGEKD
 601  TTKTNGRLNL VYQPAAEDRT LLLSGGTNLN GNITQTNGKL FFSGRPTPHA
 651  YNHLNDHWSQ KEGIPRGEIV WDNDWINRTF KAENFQIKGG QAVVSRNVAK
 701  VKGDWHLSNH AQAVFGVAPH QSHTICTRSD WTGLTNCVEK TITDDKVIAS
 751  LTKTDISGNV DLADHAHLNL TGLATLNGNL SANGDTRYTV SHNATQNGNL
 801  SLVGNAQATF NQATLNGNTS ASGNASFNLS DHAVQNGSLT LSGNAKANVS
 851  HSALNGNVSL ADKAVFHFES SRFTGQISGG KDTALHLKDS EWTLPSGTEL
 901  GNLNLDNATI TLNSAYRHDA AGAQTGSATD APRRRSRRSR RSLLSVTPPT
 951  SVESRFNTLT VNGKLNGQGT FRFMSELFGY RSDKLKLAES SEGTYTLAVN
1001  NTGNEPASLE QLTVVEGKDN KPLSENLNFT LQNEHVDAGA WRYQLIRKDG
1051  EFRLHNPVKE QELSDKLGKA EAKKQAEKDN AQSLDALIAA GRDAVEKTES
1101  VAEPARQAGG ENVGIMQAEE EKKRVQADKD TALAKQREAE TRPATTAFPR
1151  ARRARRDLPQ LQPQPQPQPQ RDLISRYANS GLSEFSATLN SVFAVQDELD
1201  RVFAEDRRNA VWTSGIRDTK HYRSQDFRAY RQQTDLRQIG MQKNLGSGRV
1251  GILFSHNRTE NTFDDGIGNS ARLAHGAVFG QYGIDRFYIG ISAGAGFSSG
1301  SLSDGIGGKI RRRVLHYGIQ ARYRAGFGGF GIEPHIGATR YFVQKADYRY
1351  ENVNIATPGL AFNRYRAGIK ADYSFKPAQH ISITPYLSLS YTDAASGKVR
1401  TRVNTAVLAQ DFGKTRSAEW GVNAEIKGFT LSLHAAAAKG PQLEAQHSAG
1451  IKLGYRW*
```

Computer analysis of these sequences gave the following results:

<u>Homology with a predicted ORF from *N.meningitidis* (strain A)</u>

ORF1 shows 57.8% identity over a 1456aa overlap with an ORF (ORF1a) from strain A of *N. meningitidis:*

```
                    10        20        30        40        50        60
orf1.pep    MKTTDKRTTETHRKAPKTGRIRFXAAYLAICLSFGILPQAWAGHTYFGINYQYYRDFAEN
            |||||||||||||||||||||||| |||||||||||||||||||||||||||||||||||
orf1a       MKTTDKRTTETHRKAPKTGRIRFSPAYLAICLSFGILPQAWAGHTYFGINYQYYRDFAEN
                    10        20        30        40        50        60

                    70        80        90       100       110       120
orf1.pep    KGKFAVGAKDIEVYNKKGELVGKSMTKAPMIDFSVVSRNGVAALVGVQYIVSVAHNGGYN
            ||||||||||||||||||||||||||||||||||||||||||||| ||||||||||||||
orf1a       KGKFAVGAKDIEVYNKKGELVGKSMTKAPMIDFSVVSRNGVAALVGDQYIVSVAHNGGYN
                    70        80        90       100       110       120

                   130       140       150       160       170       180
orf1.pep    NVDFGAEGXNIXDQXRXTYKIVKRNNYKAGTKGHPYGGDYHMPRLHKXVTDAEPVEMTSY
            ||||||||||  || | :|:||||||||  ::  |||:|| ||||||| |||||||||||
orf1a       NVDFGAEGXN-PDQHRFSYQIVKRNNYKPDNS-HPYNGDXHMPRLHKFVTDAEPVEMTSD
                   130       140       150         160       170

                   190       200       210
orf1.pep    MDGRKYIDQNNYPDRVRIGAGRQYWRSDEDEP---------------------NN-----
```

```
              |  |    |  |:::||:|||||:|::|||  |:|:                          ||
orf1a         MRGNTYSDKEKYPERVRIGSGHHYWRYDDDKHGDLSYSGAWLIGGNTHMQGWGNNGVXSL
             180       190       200       210       220       230

                     220                 230       240       250       260
orf1.pep      ----RESSYH----IA-----SGSPMFIYDAQKQKWLINGVLQTGNPYIGKSNGFQLVRK
                  |::: :    ||     |||||||||  ::|||:|||||||  || |:  |||||:||
orf1a         SGDVRHANDYGPMPIAGAAGDSGSPMFIYDKTNNKWLLNGVLQTGYPYSGRENGFQLIRK
             240       250       260       270       280       290

                    270       280       290       300       310       320
orf1.pep      DWFYDEIFAGDTHSVFYEPRQNGKYSFNDDNNGTGKINAKHEHNSLPNRLKTRTVQLFNV
              |||||:|:  ||||:|  :|||:||::||:::|||||  ::   :|:  |  |  :||::||:||:
orf1a         DWFYDDIYRGDTHTVXFEPRSNGHFSFTSNNNGTGTVTETNEKVSNP-KLKVQTVRLFDE
             300       310       320       330       340       350

                    330       340       350       360       370       380
orf1.pep      SLSETAREPVYHAAGGVNSYRPRLNNGENISFIDEGKGELILTSNINQGAGGLYFQGDFT
              ||:||  :||||  ||||||:|||||||||||:||||  |:|:|||::|||||||||||:||||
orf1a         SLNETDKEPVY-AAGGVNQYRPRLNNGENLSFIDYGNGKLILSNNINQGAGGLYFEGDFT
             360       370       380       390       400       410

                    390       400       410       420       430
orf1.pep      VSPENNETWQGAGVHISEDSTVTWKVNGVANDRLSKIGKGTL------------------
              ||||||||||||||||||||||||||||||||||||||||||
orf1a         VSPENNETWQGAGVHISEDSTVTWKVNGVANDRLSKIGKGTLHVQAKGENQGSISVGDGT
               420       430       440       450       460       470

orf1.pep      ------------------------------------------------------------

orf1a         VILDQQADDKGKKQAFSEIGLXSGRGTVQLNADNQFNPDKLYFGFRGGRLDLNGHSLSFH
               480       490       500       510       520       530

orf1.pep      ------------------------------------------------------------

orf1a         RIQNTDEGAMIXXHNATTTSTVTITGNESITQPSGKNINRLNYSKEIAYNGWFGEKDTTK
               540       550       560       570       580       590

orf1.pep      ------------------------------------------------------------

orf1a         TNGRLNLVYQPAAEDRTXLLSGGTNLNGNITQTNGKLFFSGRPTPHAYNHLGSGWSKMEG
               600       610       620       630       640       650

orf1.pep      ------------------------------------------------------------

orf1a         IPQGEIVWDNDWIXRTFKAENFHIQGGQAVISRNVAKVEGDXHLSNHAQAVFGVAPHQSH
               660       670       680       690       700       710

                            440       450       460       470       480
orf1.pep      ---------------XXXXXDKVTASLTKTDISGNVDLADHAHLNLTGLATLNGNLSAN
                             :    || :  |||  ||||||| || | |           |:| |:| ||||||
orf1a         TICTRSDWTGLTNCVEXXITDDKVIASLTKTDXSGXVXLXXXXXXXLXGXAXLXGNLSAN
               720       730       740       750       760       770

                    490       500       510       520       530       540
orf1.pep      GDTRYTVSHNATQNGNXSLVXNAQATFNQATLNGNTSASGNASFNLSDHAVQNGSLTLSG
              |||||||||||||||  |||  |||||||||||||||:|  ||||||||::|:||||||||
orf1a         GDTRYTVSHNATQNGNLSLVGNAQATFNQATLNGNXSXSGNASFNLSNNAAQNGSLTLSD
               780       790       800       810       820       830

                    550       560       570       580       590       600
orf1.pep      NAKANVSHSALNGNVSLADKAVFHFESSRFTGQISGGKDTALHLKDSEWTLPSGXELGNL
              |||||||||||||||||||||||||||:||||||:||:|  |||||||||||||||:|||||
orf1a         NAKANVSHSALNGNVSLADKAVFHFENSRFTGQLSGSKXTALHLKDSEWTLPSGTELGNL
               840       850       860       870       880       890

                    610       620       630       640       650       660
orf1.pep      NLDNATITLNSAYRHDAAGAQTGSATDAPRRRSRRSRRSLLXVTPPTSVESRFNTLTVNG
```

```
            ||||||||||||||||||||||| ::|:||||||||   || ||||||||||||||||||
orf1a       NLDNATITLNSAYRHDAAGAQTGXVSDTPRRRSRRS---LLSVTPPTSVESRFNTLTVNG
             900       910       920       930          940       950

                  670       680       690       700       710       720
orf1.pep    KLNGQGTFRFMSELFGYRSDKLKLAESSEGTYTLAVNNTGNEPASLEQLTVVEGKDNKPL
            ||| |||||||||||||||||||||||||||||||||||||||:||:|||||||||||||
orf1a       KLNXQGTFRFMSELFGYRSDKLKLAESSEGTYTLAVNNTGNEPVSLDQLTVVEGKDNKPL
                960       970       980       990      1000      1010

                  730       740       750
orf1.pep    SENLNFTLQNEHVDAGAW------------------------------------------
            ||||||||||||||||||
orf1a       SENLNFTLQNEHVDAGAWRYQLIRKDGEFRLHNPVKEQELSDKLGKAEAKKQAEKDNAQS
               1020      1030      1040      1050      1060      1070

orf1.pep    ------------------------------------------------------------

orf1a       LDALIAAGRDAAEKTESVAEPARXAGGENVGIMQAEEEKKRVQADKDSALAKQREAETRP
               1080      1090      1100      1110      1120      1130

                                                                760
orf1.pep    ---------------------------------------------------------LDR
                                                                     |||
orf1a       XTTAFPRARXARRDLPQPQPQPQPQPQPQRDLXSRYANSGLSEFSATLNSVFAVQDELDR
               1140      1150      1160      1170      1180      1190

              770       780       790       800       810       820
orf1.pep    VFAEDRRNAVWTSGIRDTKHYRSQDFRAYRQQTDLRQIGMQKNLGSGRVGILFSHNRTEN
            ||||||||||||| || |||||||||||||||||||||||||||||||||||||||||||
orf1a       VFAEDRRNAVWTSXIRXTKHYRSQDFRAYRQQTDLRQIGMQKNLGSGRVGILFSHNRTEN
               1200      1210      1220      1230      1240      1250

              830       840       850       860       870       880
orf1.pep    TFDDGIGNSARLAHGAVFGQYGIDRFYIGISAGAGFSSGSLSDGIGXKXRRRVLHYGIQA
            :|||||||||||||||||||||| || ||||:||||||| |||||| | |||||||||||
orf1a       XFDDGIGNSARLAHGAVFGQYGIGRFDIGISTGAGFSSGXLSDGIGGKIRRRVLHYGIQA
               1260      1270      1280      1290      1300      1310

              890       900       910       920       930       940
orf1.pep    RYRAGFGGFGIEPHIGATRYFVQKADYRYENVNIATPGLAFNRYRAGIKADYSFKPAQHI
            |||||||||||||:|||||||||||||||||||||||||||||||||||||||||||||
orf1a       RYRAGFGGFGIEPYIGATRYFVQKADYRYENVNIATPGLAFNRYRAGIKADYSFKPAQHX
               1320      1330      1340      1350      1360      1370

              950       960       970       980       990      1000
orf1.pep    SITPYLSLSYTDAASGKVRTRVNTAVLAQDFGKTRSAEWGVNAEIKGFTLSLHAAAAKGP
            ||||| ||||||||||||||||||||||||||||||||||||||||||||| ||||||||
orf1a       SITPYXSLSYTDAASGKVRTRVNTAVLAQDFGKTRSAEWGVNAEIKGFTLSXHAAAAKGP
               1380      1390      1400      1410      1420      1430

             1010      1020
orf1.pep    QLEAQHSAGIKLGYRWX
            |||||||||||||||||
orf1a       QLEAQHSAGIKLGYRWX
               1440      1450
```

The complete length ORF1a nucleotide sequence <SEQ ID 651> is:

```
  1 ATGAAAACAA CCGACAAACG GACAACCGAA ACACACCGCA AAGCCCCGAA
 51 AACCGGCCGC ATCCGCTTCT CGCCTGCTTA CTTAGCCATA TGCCTGTCGT
101 TCGGCATTCT TCCCCAAGCT TGGGCGGGAC ACACTTATTT CGGCATCAAC
151 TACCAATACT ATCGCGACTT TGCCGAAAAT AAAGGCAAGT TTGCAGTCGG
201 GGCGAAAGAT ATTGAGGTNT ACAACAAAAA AGGGGAGTTG GTCGGCAAAT
251 CAATGACAAA AGCCCCGATG ATTGATTTTT CTGTGGTGTC GCGTAACGGC
301 GTGGCGGCAT TGGTGGGCGA TCAATATATT GTGAGCGTGG CACATAACGG
351 CGGCTATAAC AACGTTGATT TTGGTGCGGA AGGAAGNAAT CCCGATCAGC
401 ACCGTTTTTC TTACCAAATT GTGAAAAGAA ATAATTATAA GCCTGACAAT
451 TCACACCCTT ACAACGGCGA TTANCATATG CCGCGTTTGC ATAAATTTGT
501 CACAGATGCA GAACCTGTCG AAATGACGAG TGACATGAGG GGGAATACCT
551 ATTCCGATAA AGAAAAATAT CCCGAGCGTG TCCGCATCGG CTCAGGACAC
```

601 CACTATTGGC GTTATGATGA TGACAAACAC GGCGATTTAT CCTACTCCGG
651 CGCATGGTTA ATTGGCGGCA ATACACATAT GCAGGGTTGG GGAAATAATG
701 GCGTANTTAG TTTGAGCGGC GATGTGCGCC ATGCCAACGA CTATGGCCCT
751 ATGCCGATTG CAGGTGCGGC AGGCGACAGC GGTTCGCCAA TGTTTATTTA
801 TGACAAAACA AACAATAAAT GGCTGCTCAA CGGAGTTTTA CAAACCGGCT
851 ACCCTTATTC CGGCAGGGAA AACGGTTTCC AGCTGATACG CAAAGATTGG
901 TTCTACGATG ACATTTACAG AGGCGATACA CATACCGTCT NTTTTGAACC
951 GCGCAGTAAC GGACATTTTT CCTTTACATC CAACAACAAC GGTACGGGTA
1001 CGGTAACAGA AACCAACGAA AAGGTNTCCA ATCCAAAGCT TAAAGTACAG
1051 ACAGTCCGAC TGTTTGACGA ATCTTTGAAT GAAACTGATA AAGAACCAGT
1101 TTACGCGGCA GGGGGTGTTA ATCAGTACCG TCCAAGGTTA AACAACGGTG
1151 AAAACCTTTC TTTTATCGAT TACGGCAACG GCAAACTCAT CTTATCAAAC
1201 AACATCAACC AAGGCGCGGG CGGTTTGTAT TTTGAAGGTG ATTTTACGGT
1251 CTCGCCTGAA AACAACGAAA CGTGGCAAGG CGCGGGCGTT CATATCAGTG
1301 AAGACAGTAC CGTTACTTGG AAAGTAAACG GCGTGGCAAA CGACCGCCTG
1351 TCCAAAATCG GCAAAGGCAC GCTGCACGTT CAAGCCAAAG GGGAAAACCA
1401 AGGCTCGATC AGCGTGGGCG ACGGTACAGT CATTTTGGAT CAGCAGGCAG
1451 ACGATAAAGG CAAAAAACAA GCCTTTAGTG AAATCGGCTT GNTCAGCGGC
1501 AGGGGTACGG TGCAACTGAA TGCCGATAAT CAGTTCAACC CCGACAAACT
1551 CTATTTCGGC TTTCGCGGCG GACGTTTGGA TTTAAACGGG CATTCGCTTT
1601 CGTTCCACCG TATTCAAAAT ACCGATGAAG GGGCGATGAT TGNCNATCAT
1651 AATGCCACAA CAACATCCAC CGTTACCATT ACAGGGAATG AAAGTATTAC
1701 ACAACCGAGT GGTAAGAATA TCAATAGACT TAATTACAGC AAAGAAATTG
1751 CCTACAACGG TTGGTTTGGC GAGAAAGATA CGACCAAAAC GAACGGGCGG
1801 CTCAACCTTG TTTACCAGCC CGCCGCAGAA GACCGCACCC NGCTGCTTTC
1851 CGGCGGAACA AATTTAAACG GCAACATCAC GCAAACAAAC GGCAAACTGT
1901 TTTTCAGCGG CAGACCGACA CCGCACGCCT ACAATCATTT AGGAAGCGGG
1951 TGGTCAAAAA TGGAAGGTAT CCCACAAGGA GAAATCGTGT GGGACAACGA
2001 CTGGATCNAC CGCACGTTTA AAGCGGAAAA TTTCCATATT CAGGGCGGGC
2051 AGGCGGTGAT TTCCCGCAAT GTTGCCAAAG TGGAAGGCGA TTGNCATTTG
2101 AGCAATCACG CCCAAGCAGT TTTTGGTGTC GCACCGCATC AAAGCCATAC
2151 AATCTGTACA CGTTCGGACT GGACNGGTCT GACAAATTGT GTCGAANAAA
2201 NCATTACCGA CGATAAAGTG ATTGCTTCAT TGACTAAGAC NGACNTNAGC
2251 GGCANTGTNA GNCTNNCCNA TNACGNTNNT TNAAANCTCN CNGGGCNTGC
2301 NNCACTNAAN GGCAATCTTA GTGCAAATGG CGATACACGT TATACAGTCA
2351 GCCACAACGC CACCCAAAAC GGCAACCTTA GCCTCGTGGG CAATGCCCAA
2401 GCAACATTTA ATCAAGCCAC ATTAAACGGC AACNCATCGG NTTCGGGCAA
2451 TGCTTCATTT AATCTAAGCA ACAACGCCGC ACAAAACGGC AGTCTGACGC
2501 TTTCCGACAA CGCTAAGGCA AACGTAAGCC ATTCCGCACT CAACGGCAAT
2551 GTCTCCCTAG CCGATAAGGC AGTATTCCAT TTTGAAAACA GCCGCTTTAC
2601 CGGACAACTC AGCGGCAGCA AGGANACAGC ATTACACTTA AAAGACAGCG
2651 AATGGACGCT GCCGTCAGGC ACGGAATTAG GCAATTTAAA CCTTGACAAC
2701 GCCACCATTA CACTCAATTC CGCCTATCGC CACGATGCTG CAGGCGCGCA
2751 AACCGGCAGN GTGTCAGACA CGCCGCGCCG CCGTTCGCGC CGTTCCCTAT
2801 TATCCGTTAC ACCGCCAACT TCGGTAGAAT CCCGTTTCAA CACGCTGACG
2851 GTAAACGGCA AATTGAACNG TCAAGGAACA TTCCGCTTTA TGTCGGAACT
2901 CTTCGGCTAC CGAAGCGACA AATTGAAGCT GGCGGAAAGT TCCGAAGGNA
2951 CTTACACCTT GGCGGTCAAC AATACCGGCA ACGAACCCGT AAGCCTCGAT
3001 CAATTGACGG TAGTGGAAGG GAAAGACAAC AAACCGCTGT CCGAAAACCT
3051 TAATTTCACC CTGCAAAACG AACACGTCGA TGCCGGCGCG TGGCGTTACC
3101 AACTCATCCG CAAAGACGGC GAGTTCCGCC TGCATAATCC GGTCAAAGAA
3151 CAAGAGCTTT CCGACAAACT CGGCAAGGCA GAAGCCAAAA AACAGGCGGA
3201 AAAAGACAAC GCGCAAAGCC TTGACGCGCT GATTGCGGCC GGGCGCGATG
3251 CCGCCGAAAA GACAGAAAGC GTTGCCGAAC CGGCCCGGCN GGCAGGCGGG
3301 GAAAATGTCG GCATTATGCA GGCGGAGGAA GAGAAAAAAC GGGTGCAGGC
3351 GGATAAAGAC AGCGCNTTGG CGAAACAGCG CGAAGCGGAA ACCCGGCCGG
3401 NTACCACCGC CTTCCCCCGC GCCCGCNGCG CCCGCCGGGA TTTGCCGCAA
3451 CCGCAGCCCC AACCGCAACC TCAACCCCAA CCGCAGCGCG ACCTGATNAG
3501 CCGTTATGCC AATAGCGGTT TGAGTGAATT TTCCGCCACG CTCAACAGCG
3551 TTTTCGCCGT ACAGGACGAA TTGGACCGCG TGTTTGCCGA AGACCGCCGC
3601 AACGCNGTTT GGACAAGCNG CATCCGGNAC ACCAAACACT ACCGTTCGCA
3651 AGATTTCCGC GCCTACCGCC AACAAACCGA CCTGCGCCAA ATCGGTATGC
3701 AGAAAAACCT CGGCAGCGGG CGCGTCGGCA TCCTGTTTTC GCACAACCGG
3751 ACCGAAAACA NCTTCGACGA CGGCATCGGC AACTCGGCAC GGCTTGCCCA
3801 CGGCGCCGTT TTCGGGCAAT ACGGCATCGG CAGGTTCGAC ATCGGCATCA
3851 GCACGGGCGC GGGTTTTAGC AGCGGCANTC TNTCAGACGG CATCGGAGGC
3901 AAAATCCGCC GCCGCGTGCT GCATTACGGC ATTCAGGCAC GATACCGCGC
3951 CGGTTTCGGC GGATTCGGCA TCGAACCGTA CATCGGCGCA ACGCGCTATT
4001 TCGTCCAAAA AGCGGATTAC CGCTACGAAA ACGTCAATAT CGCCACCCCC
4051 GGTCTTGCGT TCAACCGNTA CCGNGCGGGC ATTAAGGCAG ATTATTCATT
4101 CAAACCGGCG CAACACATNT CCATCACNCC TTATTTNAGC CTGTCCTATA
4151 CCGATGCCGC TTCGGGCAAA GTCCGAACAC GCGTCAATAC CGCNGTATTG

```
4201  GCTCAGGATT TCGGCAAAAC CCGCAGTGCG GAATGGGGCG TAAACGCCGA
4251  AATCAAAGGT TTCACGCTGT CCNTCCACGC TGCCGCCGCC AAAGGNCCGC
4301  AACTGGAAGC GCAACACAGC GCGGGCATCA AATTAGGCTA CCGCTGGTAA
```

This encodes a protein having amino acid sequence <SEQ ID 652>:

```
   1  MKTTDKRTTE THRKAPKTGR IRFSPAYLAI CLSFGILPQA WAGHTYFGIN
  51  YQYYRDFAEN KGKFAVGAKD IEVYNKKGEL VGKSMTKAPM IDFSVVSRNG
 101  VAALVGDQYI VSVAHNGGYN NVDFGAEGXN PDQHRFSYQI VKRNNYKPDN
 151  SHPYNGDXHM PRLHKFVTDA EPVEMTSDMR GNTYSDKEKY PERVRIGSGH
 201  HYWRYDDDKH GDLSYSGAWL IGGNTHMQGW GNNGVXSLSG DVRHANDYGP
 251  MPIAGAAGDS GSPMFIYDKT NNKWLLNGVL QTGYPYSGRE NGFQLIRKDW
 301  FYDDIYRGDT HTVXFEPRSN GHFSFTSNNN GTGTVTETNE KVSNPKLKVQ
 351  TVRLFDESLN ETDKEPVYAA GGVNQYRPRL NNGENLSFID YGNGKLILSN
 401  NINQGAGGLY FEGDFTVSPE NNETWQGAGV HISEDSTVTW KVNGVANDRL
 451  SKIGKGTLHV QAKGENQGSI SVGDGTVILD QQADDKGKKQ AFSEIGLXSG
 501  RGTVQLNADN QFNPDKLYFG FRGGRLDLNG HSLSFHRIQN TDEGAMIXXH
 551  NATTTSTVTI TGNESITQPS GKNINRLNYS KEIAYNGWFG EKDTTKTNGR
 601  LNLVYQPAAE DRTXLLSGGT NLNGNITQTN GKLFFSGRPT PHAYNHLGSG
 651  WSKMEGIPQG EIVWDNDWIX RTFKAENFHI QGGQAVISRN VAKVEGDXHL
 701  SNHAQAVFGV APHQSHTICT RSDWTGLTNC VEXXITDDKV IASLTKTDXS
 751  GXVXLXXXXX XXLXGXAXLX GNLSANGDTR YTVSHNATQN GNLSLVGNAQ
 801  ATFNQATLNG NXSXSGNASF NLSNNAAQNG SLTLSDNAKA NVSHSALNGN
 851  VSLADKAVFH FENSRFTGQL SGSKXTALHL KDSEWTLPSG TELGNLNLDN
 901  ATITLNSAYR HDAAGAQTGX VSDTPRRRSR RSLLSVTPPT SVESRFNTLT
 951  VNGKLNXQGT FRFMSELFGY RSDKLKLAES SEGTYTLAVN NTGNEPVSLD
1001  QLTVVEGKDN KPLSENLNFT LQNEHVDAGA WRYQLIRKDG EFRLHNPVKE
1051  QELSDKLGKA EAKKQAEKDN AQSLDALIAA GRDAAEKTES VAEPARXAGG
1101  ENVGIMQAEE EKKRVQADKD SALAKQREAE TRPXTTAFPR ARXARRDLPQ
1151  PQPQPQPQPQ PQRDLXSRYA NSGLSEFSAT LNSVFAVQDE LDRVFAEDRR
1201  NAVWTSXIRX TKHYRSQDFR AYRQQTDLRQ IGMQKNLGSG RVGILFSHNR
1251  TENXFDDGIG NSARLAHGAV FGQYGIGRFD IGISTGAGFS SGXLSDGIGG
1301  KIRRRVLHYG IQARYRAGFG GFGIEPYIGA TRYFVQKADY RYENVNIATP
1351  GLAFNRYRAG IKADYSFKPA QHXSITPYXS LSYTDAASGK VRTRVNTAVL
1401  AQDFGKTRSA EWGVNAEIKG FTLSXHAAAA KGPQLEAQHS AGIKLGYRW*
```

A transmembrane region is underlined.

ORF1-1 shows 86.3% identity over a 1462aa overlap with ORF1a:

```
                       10        20        30        40        50        60
orf1a.pep      MKTTDKRTTETHRKAPKTGRIRFSPAYLAICLSFGILPQAWAGHTYFGINYQYYRDFAEN
               ||||||||||||||||||||||||||||||||||||||||||||||||||||||||||||
orf1-1         MKTTDKRTTETHRKAPKTGRIRFSPAYLAICLSFGILPQAWAGHTYFGINYQYYRDFAEN
                       10        20        30        40        50        60

                       70        80        90       100       110       120
orf1a.pep      KGKFAVGAKDIEVYNKKGELVGKSMTKAPMIDFSVVSRNGVAALVGDQYIVSVAHNGGYN
               ||||||||||||||||||||||||||||||||||||||||||||||||||||||||||||
orf1-1         KGKFAVGAKDIEVYNKKGELVGKSMTKAPMIDFSVVSRNGVAALVGDQYIVSVAHNGGYN
                       70        80        90       100       110       120

                      130       140       150        160       170      179
orf1a.pep      NVDFGAEGXNPDQHRFSYQIVKRNNYKPDNS-HPYNGDXHMPRLHKFVTDAEPVEMTSDM
               |||||||| |||||||:|:||||||||  ::  |||:|| ||||||||||||||||||| |
orf1-1         NVDFGAEGRNPDQHRFTYKIVKRNNYKAGTKGHPYGGDYHMPRLHKFVTDAEPVEMTSYM
                      130       140       150       160       170       180

            180        190       200       210            220       230
orf1a.pep      RGNTYSDKEKYPERVRIGSGHHYWRYDDDKHGDL--SYSGA----WLIGGNTHMQGWGNN
                |  | |:::||:|||||:|::||| |:|: ::   ||  |    ||:||||  |: :::
orf1-1         DGRKYIDQNNYPDRVRIGAGRQYWRSDEDEPNNRESSYHIASAYSWLVGGNTFAQNGSGG
                      190       200       210       220       230       240

                   240        250       260       270        280       290
orf1a.pep      GVXSLSGD-VRHANDYGPMPIAGAAGDSGSPMFIYDKTNNKWLLNGVLQTGYPYSGRENG
               |: :|::: ::|:  || :| :|: |||||||||||  ::|||:||||||| || |: ||
orf1-1         GTVNLGSEKIKHS-PYGFLPTGGSFGDSGSPMFIYDAQKQKWLINGVLQTGNPYIGKSNG
                      250        260       270       280       290
```

```
              300       310       320       330       340       350
orf1a.pep   FQLIRKDWFYDDIYRGDTHTVXFEPRSNGHFSFTSNNNGTGTVTETNEKVSNP-KLKVQT
            |||:|||||||:|:  ||||:| :|||:||::||:::|||||  ::   :|:  | |  :||::|
orf1-1      FQLVRKDWFYDEIFAGDTHSVFYEPRQNGKYSFNDDNNGTGKINAKHEHNSLPNRLKTRT
           300       310       320       330       340       350

              360       370       380       390       400       410
orf1a.pep   VRLFDESLNETDKEPVY-AAGGVNQYRPRLNNGENLSFIDYGNGKLILSNNINQGAGGLY
            |:||: ||:|| :|||| |||||:|||||||||||:|||| |:|:|||::||||||||||
orf1-1      VQLFNVSLSETAREPVYHAAGGVNSYRPRLNNGENISFIDEGKGELILTSNINQGAGGLY
           360       370       380       390       400       410

              420       430       440       450       460       470
orf1a.pep   FEGDFTVSPENNETWQGAGVHISEDSTVTWKVNGVANDRLSKIGKGTLHVQAKGENQGSI
            |:||||||||||||||||||||||||||||||||||||||||||||||||||||||||||
orf1-1      FQGDFTVSPENNETWQGAGVHISEDSTVTWKVNGVANDRLSKIGKGTLHVQAKGENQGSI
           420       430       440       450       460       470

              480       490       500       510       520       530
orf1a.pep   SVGDGTVILDQQADDKGKKQAFSEIGLXSGRGTVQLNADNQFNPDKLYFGFRGGRLDLNG
            |||||||||||||||||||||||||||| |||||||||||||||||||||||||||||||
orf1-1      SVGDGTVILDQQADDKGKKQAFSEIGLVSGRGTVQLNADNQFNPDKLYFGFRGGRLDLNG
           480       490       500       510       520       530

              540       550       560       570       580       590
orf1a.pep   HSLSFHRIQNTDEGAMIXXHNATTTSTVTITGNESITQPSGKNINRLNYSKEIAYNGWFG
            ||||||||||||||||  ||    ||||||||::|:  :|:| | |: :|||||||||
orf1-1      HSLSFHRIQNTDEGAMIVNHNQDKESTVTITGNKDIAT-TGNN-NSLDSKKEIAYNGWFG
           540       550       560       570       580       590

              600       610       620       630       640       650
orf1a.pep   EKDTTKTNGRLNLVYQPAAEDRTXLLSGGTNLNGNITQTNGKLFFSGRPTPHAYNHLGSG
            ||||||||||||||||||||||| ||||||||||||||||||||||||||||||||||::
orf1-1      EKDTTKTNGRLNLVYQPAAEDRTLLLSGGTNLNGNITQTNGKLFFSGRPTPHAYNHLNDH
           600       610       620       630       640       650

              660       670       680       690       700       710
orf1a.pep   WSKMEGIPQGEIVWDNDWIXRTFKAENFHIQGGQAVISRNVAKVEGDXHLSNHAQAVFGV
            ||: ||||:|||||||||| |||||||:|:|||||:|||||||:|| |||||||||||
orf1-1      WSQKEGIPRGEIVWDNDWINRTFKAENFQIKGGQAVVSRNVAKVKGDWHLSNHAQAVFGV
           660       670       680       690       700       710

              720       730       740       750       760       770
orf1a.pep   APHQSHTICTRSDWTGLTNCVEXXITDDKVIASLTKTDXSGXVXLXXXXXXXLXGXAXLX
            ||||||||||||||||||||||  :||||||||||||| || | |       |:| |:|
orf1-1      APHQSHTICTRSDWTGLTNCVEKTITDDKVIASLTKTDISGNVDLADHAHLNLTGLATLN
           720       730       740       750       760       770

              780       790       800       810       820       830
orf1a.pep   GNLSANGDTRYTVSHNATQNGNLSLVGNAQATFNQATLNGNXSXSGNASFNLSNNAAQNG
            ||||||||||||||||||||||||||||||||||||||||||:| |||||||||::|:|||
orf1-1      GNLSANGDTRYTVSHNATQNGNLSLVGNAQATFNQATLNGNTSASGNASFNLSDHAVQNG
           780       790       800       810       820       830

              840       850       860       870       880       890
orf1a.pep   SLTLSDNAKANVSHSALNGNVSLADKAVFHFENSRFTGQLSGSKXTALHLKDSEWTLPSG
            ||||| ||||||||||||||||||||||||:||||||:||:| ||||||||||||||
orf1-1      SLTLSGNAKANVSHSALNGNVSLADKAVFHFESSRFTGQISGGKDTALHLKDSEWTLPSG
           840       850       860       870       880       890

              900       910       920       930       940
orf1a.pep   TELGNLNLDNATITLNSAYRHDAAGAQTGXVSDTPRRRSRRS---LLSVTPPTSVESRFN
            ||||||||||||||||||||||||||||| ::|:|||||||||   ||||||||||||||
orf1-1      TELGNLNLDNATITLNSAYRHDAAGAQTGSATDAPRRRSRRSRRSLLSVTPPTSVESRFN
           900       910       920       930       940       950

             950       960       970       980       990      1000
orf1a.pep   TLTVNGKLNXQGTFRFMSELFGYRSDKLKLAESSEGTYTLAVNNTGNEPVSLDQLTVVEG
            ||||||||| ||||||||||||||||||||||||||||||||||||||:||:|||||||
orf1-1      TLTVNGKLNGQGTFRFMSELFGYRSDKLKLAESSEGTYTLAVNNTGNEPASLEQLTVVEG
            960       970       980       990      1000      1010
```

```
                  1010      1020      1030      1040      1050      1060
orf1a.pep         KDNKPLSENLNFTLQNEHVDAGAWRYQLIRKDGEFRLHNPVKEQELSDKLGKAEAKKQAE
                  ||||||||||||||||||||||||||||||||||||||||||||||||||||||||||||
orf1-1            KDNKPLSENLNFTLQNEHVDAGAWRYQLIRKDGEFRLHNPVKEQELSDKLGKAEAKKQAE
                  1020      1030      1040      1050      1060      1070

                  1070      1080      1090      1100      1110      1120
orf1a.pep         KDNAQSLDALIAAGRDAAEKTESVAEPARXAGGENVGIMQAEEEKKRVQADKDSALAKQR
                  ||||||||||||||||:|||||||||||| ||||||||||||||||||||||||:|||||
orf1-1            KDNAQSLDALIAAGRDAVEKTESVAEPARQAGGENVGIMQAEEEKKRVQADKDTALAKQR
                  1080      1090      1100      1110      1120      1130

                  1130      1140      1150      1160      1170      1180
orf1a.pep         EAETRPXTTAFPRARXARRDLPQPQPQPQPQPQPQRDLXSRYANSGLSEFSATLNSVFAV
                  |||||| |||||||| ||||||| ||||||||  |||| ||||||||||||||||||||
orf1-1            EAETRPATTAFPRARRARRDLPQLQPQPQPQP--QRDLISRYANSGLSEFSATLNSVFAV
                  1140      1150      1160        1170      1180      1190

                  1190      1200      1210      1220      1230      1240
orf1a.pep         QDELDRVFAEDRRNAVWTSXIRXTKHYRSQDFRAYRQQTDLRQIGMQKNLGSGRVGILFS
                  ||||||||||||||||||| || |||||||||||||||||||||||||||||||||||||
orf1-1            QDELDRVFAEDRRNAVWTSGIRDTKHYRSQDFRAYRQQTDLRQIGMQKNLGSGRVGILFS
                    1200      1210      1220      1230      1240      1250

                  1250      1260      1270      1280      1290      1300
orf1a.pep         HNRTENXFDDGIGNSARLAHGAVFGQYGIGRFDIGISTGAGFSSGXLSDGIGGKIRRRVL
                  ||||||:|||||||||||||||||||||| || ||||:||||||| ||||||||||||||
orf1-1            HNRTENTFDDGIGNSARLAHGAVFGQYGIDRFYIGISAGAGFSSGSLSDGIGGKIRRRVL
                    1260      1270      1280      1290      1300      1310

                  1310      1320      1330      1340      1350      1360
orf1a.pep         HYGIQARYRAGFGGFGIEPYIGATRYFVQKADYRYENVNIATPGLAFNRYRAGIKADYSF
                  |||||||||||||||||||:||||||||||||||||||||||||||||||||||||||||
orf1-1            HYGIQARYRAGFGGFGIEPHIGATRYFVQKADYRYENVNIATPGLAFNRYRAGIKADYSF
                    1320      1330      1340      1350      1360      1370

                  1370      1380      1390      1400      1410      1420
orf1a.pep         KPAQHXSITPYXSLSYTDAASGKVRTRVNTAVLAQDFGKTRSAEWGVNAEIKGFTLSXHA
                  ||||| |||||| |||||||||||||||||||||||||||||||||||||||||||| ||
orf1-1            KPAQHISITPYLSLSYTDAASGKVRTRVNTAVLAQDFGKTRSAEWGVNAEIKGFTLSLHA
                    1380      1390      1400      1410      1420      1430

                  1430      1440      1450
orf1a.pep         AAAKGPQLEAQHSAGIKLGYRWX
                  ||||||||||||||||||||||
orf1-1            AAAKGPQLEAQHSAGIKLGYRWX
                    1440      1450
```

Homology with adhesion and penetration protein hap precursor of *H.influenzae* (accession number P45387)

Amino acids 23-423 of ORF1 show 59% aa identity with hap protein in 450aa overlap:

```
orf1   23  FXAAYLAICLSFGILPQAWAGHTYFGINYQYYRDFAENKGKFAVGAKDIEVYNKKGELVG 82
           F   +L  C+S GI  QAWAGHTYFGI+YQYYRDFAENKGKF VGAK+IEVYNK+G+LVG
hap    6   FRLNFLTACVSLGIASQAWAGHTYFGIDYQYYRDFAENKGKFTVGAKNIEVYNKEGQLVG 65

orf1   83  KSMTKAPMIDFSVVSRNGVAALVGVQYIVSVAHNGGYNNVDFGAEGXNIXDQXRXTYKIV 142
            SMTKAPMIDFSVVSRNGVAALVG QYIVSVAHNGGYN+VDFGAEG N   DQ R TY+IV
hap    66  TSMTKAPMIDFSVVSRNGVAALVGDQYIVSVAHNGGYNDVDFGAEGRN-PDQHRFTYQIV 124

orf1   143 KRNNYKAGTKGHPYGGDYHMPRLHKXVTDAEPVEMTSYMDGRKYIDQNNYPDRVRIGAGR 202
           KRNNY+A   + HPY GDYHMPRLHK VT+AEPV MT+ MDG+ Y D+ NYP+RVRIG+GR
hap    125 KRNNYQAWERKHPYDGDYHMPRLHKFVTEAEPVGMTTNMDGKVYADRENYPERVRIGSGR 184

orf1   203 QYWRSDEDEPNNRESSYHIA---------------------------------------- 222
           QYWR+D+DE  N  SSY+++
hap    185 QYWRTDKDEETNVHSSYYVSGAYRYLTAGNTHTQSGNGNGTVNLSGNVVSPNHYGPLPTG 244

orf1   223 -----SGSPMFIYDAQKQKWLINGVLQTGNPYIGKSNGFQLVRKDWFYDEIFAGDTHSVF 277
                SGSPMFIYDA+K++WLIN VLQTG+P+ G+ NGFQL+R++WFY+E+ A DT SVF
hap    245 GSKGDSGSPMFIYDAKKKQWLINAVLQTGHPFFGRGNGFQLIREEWFYNEVLAVDTPSVF 304
```

```
orf1   278 --YEPRQNGKYSFNDDNNGTGKIN-AKHEHNSLPNRLKTRTVQLFNVSLSETAREPVYHA 334
             Y P  NG YSF  +N+GTGK+   +   +    + +  TV+LFN SL++TA+E V  A
hap    305 QRYIPPINGHYSFVSNNDGTGKLTLTRPSKDGSKAKSEVGTVKLFNPSLNQTAKEHV-KA 363

orf1   335 AGGVNSYRPRLNNGENISFIDEGKGELILTSNINQGAGGLYFQGDFTV-SPENNETWQGA 393
           A G N Y+PR+  G+NI   D+GKG L + +NINQGAGGLYF+G+F V   +NN TWQGA
hap    364 AAGYNIYQPRMEYGKNIYLGDQGKGTLTIENNINQGAGGLYFEGNFVVKGKQNNITWQGA 423

orf1   394 GVHISEDSTVTWKVNGVANDRLSKIGKGTL 423
           GV I +D+TV WKV+   NDRLSKIG GTL
hap    424 GVSIGQDATVEWKVHNPENDRLSKIGIGTL 453
```

Amino acids 715-1011 of ORF1 show 50% aa identity with hap protein in 258aa overlap:

```
Orf1   41  DTRYTVSHNATQ-NGNXSLVXNAQATFNQ-ATLNGNTSASGNASFNLSDHAVQNGSLTLS 98
           DT+   S   TQ NG+ +L  NA     + A LNGN +    ++ F LS++A Q G++ LS
hap    733 DTKVINSIPITQINGSINLTNNATVNIHGLAKLNGNVTLIDHSQFTLSNNATQTGNIKLS 792

orf1   99  GNAKANVSHSALNGNVSLADKAVFHFESSRFTGQISGGKDTALHLKDSEWTLPSGXELGN 158
            +A A V+++ LNGNV  L D A F  ++S F  QI G KDT + L+++ WT+PS   L N
hap    793 NHANATVNNATLNGNVHLTDSAQFSLKNSHFWHQIQGDKDTTVTLENATWTMPSDTTLQN 852

orf1   159 LNLDNATITLNSAYRHDAAGAQTGSATDAPXXXXXXXXXXLLXVTPPTSVESRFNTLTVN 218
           L L+N+T+TLNSAY        + S+ +AP          L   T PTS E RFNTLTVN
hap    853 LTLNNSTVTLNSAY--------SASSNNAPRHRRS-----LETETTPTSAEHRFNTLTVN 899

orf1   219 GKLNGQGTFRFMSELFGYRSDKLKLAESSEGTYTLAVNNTGNEPASLEQLTVVEGKDNKP 278
           GKL+GQGTF+F S LFGY+SDKLKL+   +EG YTL+V NTG EP +LEQLT++E  DNKP
hap    900 GKLSGQGTFQFTSSLFGYKSDKLKLSNDAEGDYTLSVRNTGKEPVTLEQLTLIESLDNKP 959

orf1   279 LSENLNFTLQNEHVDAGA 296
           LS+ L FTL+N+HVDAGA
hap    960 LSDKLKFTLENDHVDAGA 977
```

Amino acids 1192-1450 of ORF1 show 41% aa identity with hap protein in 259aa overlap:

```
Orf1    1    LDRVFAEDRRNAVWTSGIRDTKHYRSQDFRAYRQQTDLRQIGMQKNLGSGRVGILFSHNR 60
             LDR+F +   ++AVWT+   +D + Y S  FRAY+Q+T+LRQIG+QK L +GR+G +FSH+R
hap     1135 LDRLFVDQAQSAVWTNIAQDKRRYDSDAFRAYQQKTNLRQIGVQKALANGRIGAVFSHSR 1194

orf1    61   TENTFDDGIGNSARLAHGAVFGQYGIDRFYXXXXXXXXXXXXXXXXXIGXKXRRRVLHYG 120
             ++NTFD+ + N A L    + F QY                            K  R+ ++YG
hap     1195 SDNTFDEQVKNHATLTMMSGFAQYQWGDLQFGVNVGTGISASKMAEEQSRKIHRKAINYG 1254

orf1    121  IQARYRAGFGGFGIEPHIGATRYFVQKADYRYENVNIATPGLAFNRYRAGIKADYSFKPA 180
             + A Y+   G  GI+P+ G   RYF+++ +Y+ E V + TP LAFNRY AGI+ DY+F P
hap     1255 VNASYQFRLGQLGIQPYFGVNRYFIERENYQSEEVRVKTPSLAFNRYNAGIRVDYTFTPT 1314

orf1    181  QHISITPYLSLSYTDAASGKVRTRVNTAVLAQDFGKTRSAEWGVNAEIKGFTLSLHAAAA 240
              +IS+ PY  ++Y D ++   V+T VN  VL Q FG+    E G+ AEI  F +S   + +
hap     1315 DNISVKPYFFVNYVDVSNANVQTTVNLTVLQQPFGRYWQKEVGLKAEILHFQISAFISKS 1374

orf1    241  KGPQLEAQHSAGIKLGYRW 259
             +G QL  Q + G+KLGYRW
hap     1375 QGSQLGKQQNVGVKLGYRW 1393
```

Homology with a predicted ORF from *N. gonorrhoeae*

The blocks of ORF1 show 83.5%, 88.3%, and 97.7% identities in 467, 298, and 259 aa overlap, respectively with a predicted ORF (ORF1ng) from *N.gonorrhoeae*:

```
orf1.pep   MKTTDKRTTETHRKAPKTGRIRFXAAYLAICLSFGILPQAWAGHTYFGINYQYYRDFAEN   60
           |||||||||||||||||||||||  ||||||||||||||| ||||||||||||||||||||
orf1ng     MKTTDKRTTETHRKAPKTGRIRFSPAYLAICLSFGILPQARAGHTYFGINYQYYRDFAEN   60

orf1.pep   KGKFAVGAKDIEVYNKKGELVGKSMTKAPMIDFSVVSRNGVAALVGVQYIVSVAHNGGYN  120
           ||||||||||||||||||||||||||||||||||||||||||||||:| |||||||||||||
orf1ng     KGKFAVGAKDIEVYNKKGELVGKSMTKAPMIDFSVVSRNGVAALAGDQYIVSVAHNGGYN  120
```

```
orf1.pep   NVDFGAEGXNIXDQXRXTYKIVKRNNYKAGTKGHPYGGDYHMPRLHKXVTDAEPVEMTSY  180
           |||||||| |   || |  :|:||||||||||:|||||||||||||| ||||||||||||
orf1ng     NVDFGAEGSN-PDQHRFSYQIVKRNNYKAGTNGHPYGGDYHMPRLHKFVTDAEPVEMTSY  179

orf1.pep   MDGRKYIDQNNYPDRVRIGAGRQYWRSDEDEPNNRESSYHIAS-----------------  223
           ||| || | |:|||||||||||||||||||||||||||||||
orf1ng     MDGWKYADLNKYPDRVRIGAGRQYWRSDEDEPNNRESSYHIASAYSWLVGGNTFAQNGSG  239

orf1.pep   ---------------------------GSPMFIYDAQKQKWLINGVLQTGNPYIGKSNG  255
                                      |||||||||||||||||||||||||||||||||
orf1ng     GGTVNLGSEKIKHSPYGFLPTGGSFGDSGSPMFIYDAQKQKWLINGVLQTGNPYIGKSNG  289

orf1.pep   FQLVRKDWFYDEIFAGDTHSVFYEPRQNGKYSFNDDNNGTGKINAKHEHNSLPNRLKTRT  315
           |||||||||||||||||||||||||:||||| |||:|||:|||:|||:| ||| ||||||
orf1ng     FQLVRKDWFYDEIFAGDTHSVFYEPHQNGKYFFNDNNNGAGKIDAKHKHYSLPYRLKTRT  359

orf1.pep   VQLFNVSLSETAREPVYHAAGGVNSYRPRLNNGENISFIDEGKGELILTSNINQGAGGLY  375
           ||||||||||||||||||||||||||||||||||||||||:|||||||||||||||||||
orf1ng     VQLFNVSLSETAREPVYHAAGGVNSYRPRLNNGENISFIDKGKGELILTSNINQGAGGLY

orf1.pep   FQGDFTVSPENNETWQGAGVHISEDSTVTWKVNGVANDRLSKIGKGT              422
           |:|:|||||:||||||||||||||: ||||||||||||||||||||||
orf1ng     FEGNFTVSPKNNETWQGAGVHISDGSTVTWKVNGVANDRLSKIGKGTLLVQAKGENQGSV  479
                                           //
orf1.pep                                   DKVTASLTKTDISGNVDLADHAHLNLTGLA  744
                                           ||| |||:|||: |||:|||||||||||||
orf1ng     FGVAPHQSHTICTRSDWTGLTSCTEKTITDDKVIASLSKTDVRGNVSLADHAHLNLTGLA  774

orf1.pep   TLNGNLSANGDTR-YTVSHNATQNGNXSLVXNAQATFNQATLNGNTSASGNASFNLSDHA  803
           |:|||| ::::||   :  ||||||| ||| ||||||||||||||||||| |||||||::|
orf1ng     TFNGNL-VQAETRTIRLRANATQNGNLSLVGNAQATFNQATLNGNTSASDNASFNLSNNA  833

orf1.pep   VQNGSLTLSGNAKANVSHSALNGNVSLADKAVFHFESSRFTGQISGGKDTALHLKDSEWT  863
           ||||||||| ||||||||||||||||||||||||||||:|||||:|||||||||||||||
orf1ng     VQNGSLTLSDNAKANVSHSALNGNVSLADKAVFHFENSRFTGKISGGKDTALHLKDSEWT  893

orf1.pep   LPSGXELGNLNLDNATITLNSAYRHDAAGAQTGSATDAPRRRSRRSRRSLLXVTPPTSVE  923
           ||||:||||||||||||||||||||||||||||||:||||||||||    || ||||||:|
orf1ng     LPSGTELGNLNLDNATITLNSAYRHDAAGAQTGSAADAPRRRSRRS---LLSVTPPTSAE  950

orf1.pep   SRFNTLTVNGKLNGQGTFRFMSELFGYRSDKLKLAESSEGTYTLAVNNTGNEPASLEQLT  983
           |||||||||||||||||||||||||||||| ||||||||||||||||||||||||:||||||
orf1ng     SRFNTLTVNGKLNGQGTFRFMSELFGYRSGKLKLAESSEGTYTLAVNNTGNEPVSLEQLT 1010

orf1.pep   VVEGKDNKPLSENLNFTLQNEHVDAGAW                                 1011
           ||||||| ||||||||||||||||||||
orf1ng     VVEGKDNTPLSENLNFTLQNEHVDAGAWRYQLIRKDGEFRLHNPVKEQELSDKLGKAGET 1070
                                       //
orf1.pep                                    LDRVFAEDRRNAVWTSGIRDTKHYRSQDFR 1211
                                            ||||||||||||||||||||||||||||||
orf1ng     PQRDLISRYANSGLSEFSATLNSVFAVQDELDRVFAEDRRNAVWTSGIRDTKHYRSQDFR 1239

orf1.pep   AYRQQTDLRQIGMQKNLGSGRVGILFSHNRTENTFDDGIGNSARLAHGAVFGQYGIDRFY 1271
           |||||||||||||||||||||||||||||||| ||||||||||||||||||||||||| ||
orf1ng     AYRQQTDLRQIGMQKNLGSGRVGILFSHNRTGNTFDDGIGNSARLAHGAVFGQYGIGRFD 1299

orf1.pep   IGISAGAGFSSGSLSDGIGXKXRRRVLHYGIQARYRAGFGGFGIEPHIGATRYFVQKADY 1331
           ||||||||||||||||||| | ||||||||||||||||||||||||||||||||||||||
orf1ng     IGISAGAGFSSGSLSDGIRGKIRRRVLHYGIQARYRAGFGGFGIEPHIGATRYFVQKADY 1359

orf1.pep   RYENVNIATPGLAFNRYRAGIKADYSFKPAQHISITPYLSLSYTDAASGKVRTRVNTAVL 1391
           ||||||||||||||||||||||||||||||||||||||||||||||||||||||||||||
orf1ng     RYENVNIATPGLAFNRYRAGIKADYSFKPAQHISITPYLSLSYTDAASGKVRTRVNTAVL 1419

orf1.pep   AQDFGKTRSAEWGVNAEIKGFTLSLHAAAAKGPQLEAQHSAGIKLGYRW 1440
           |||||||||||||||||||||||||||||||||||||||||||||||||
orf1ng     AQDFGKTRSAEWGVNAEIKGFTLSLHAAAAKGPQLEAQHSAGIKLGYRW 1468
```

The complete length ORF1ng nucleotide sequence was identified <SEQ ID 653>:

1 ATGAAAACAA CCGACAAACG GACAACCGAA ACACACCGCA AAGCCCCTAA
51 AACCGGCCGC ATCCGCTTCT CGCCCGCTTA CTTAGCCATA TGCCTGTCGT

```
 101  TCGGCATTCT GCCCCAAGCC CGGGCGGGAC ACACTTATTT CGGCATCAAC
 151  TACCAATACT ATCGCGACTT TGCCGAAAAT AAAGGCAAGT TTGCAGTCGG
 201  GGCGAAAGAT ATTGAGGTTT ACAACAAAAA AGGGGAGTTG GTCGGCAAAT
 251  CGATGACGAA AGCCCCGATG ATTGATTTTT CTGTGGTATC GCGTAACGGC
 301  GTGGCGGCAT TGGCGGGCGA TCAATATATT GTGAGCGTGG CACATAACGG
 351  CGGCTATAAC AATGTTGATT TTGGTGCGGA GGGAAGCAAT CCCGATCAGC
 401  ACCGCTTTTC TTACCAAATT GTGAAAAGAA ATAATTATAA AGCAGGGACT
 451  AACGGCCATC CTTATGGCGG CGATTATCAT ATGCCGCGTT TGCACAAATT
 501  TGTCACAGAT GCAGAACCTG TTGAGATGAC CAGTTATATG GATGGGTGGA
 551  AATACGCTGA TTTAAATAAA TACCCTGATC GTGTTCGAAT CGGAGCAGGC
 601  AGACAATATT GGCGGTCTGA TGAAGACGAA CCCAATAACC GCGAAAGTTC
 651  ATATCATATT GCAAGCGCAT ATTCTTGGCT CGTCGGTGGC AATACCTTTG
 701  CACAAAATGG ATCAGGTGGT GGCACAGTCA ACTTAGGTAG CGAAAAAATT
 751  AAACATAGCC CATATGGTTT TTTACCAACA GGAGGCTCAT TTGGCGACAG
 801  TGGCTCACCA ATGTTTATCT ATGATGCCCA AAAGCAAAAG TGGTTAATTA
 851  ATGGGGTATT GCAAACAGGC AACCCCTATA TAGGAAAAAG CAATGGCTTC
 901  CAGCTAGTTC GTAAAGATTG GTTCTATGAT GAAATCTTTG CTGGAGATAC
 951  CCATTCAGTA TTCTACGAAC CACATCAAAA TGGGAAATAC TTTTTTAACG
1001  ACAATAATAA TGGCGCAGGA AAAATCGATG CCAAACATAA ACACTATTCT
1051  CTACCTTATA GATTAAAAAC ACGAACCGTT CAATTGTTTA ATGTTTCTTT
1101  ATCCGAGACA GCAAGAGAAC CTGTTTATCA TGCTGCAGGT GGGGTCAACA
1151  GTTATCGACC CAGACTGAAT AATGGAGAAA ATATTTCCTT TATTGACAAA
1201  GGAAAAGGTG AATTGATACT TACCAGCAAC ATCAACCAAG GCGCGGGCGG
1251  TTTGTATTTT GAGGGTAATT TTACGGTCTC GCCTAAAAAC AACGAAACGT
1301  GGCAAGGCGC GGGCGTTCAT ATCAGTGATG GCAGTACCGT TACTTGGAAA
1351  GTAAACGGCG TGGCAAACGA CCGCCTGTCC AAAATCGGCA AAGGCACGCT
1401  GCTGGTTCAA GCCAAAGGGG AAAACCAAGG CTCGGTCAGC GTGGGCGACG
1451  GTAAAGTCAT CTTAGATCAG CAGGCGGACG ATCAAGGCAA AAAACAAGCC
1501  TTTAGTGAAA TCGGCTTGGT CAGCGGCAGG GGGACGGTGC AACTGAATGC
1551  CGATAATCAG TTCAACCCCG ACAAACTCTA TTTCGGCTTT CGCGGCGGAC
1601  GTTTGGATTT GAACGGGCAT TCGCTTTCGT TCCACCGCAT TCAAAATACC
1651  GATGAAGGGG CGATGATTGT CAACCACAAT CAAGACAAAG AATCCACCGT
1701  TACCATTACA GGCAATAAAG ATATTACTAC AACCGGCAAT AACAACAACT
1751  TGGATAGCAA AAAAGAAATT GCCTACAACG GTTGGTTTGG CGAGAAAGAT
1801  GCAACCAAAA CGAACGGGCG GCTCAATCTG AATTACCAAC CGGAAGAAGC
1851  GGATCGCACT TTACTGCTTT CCGGCGGAAC AAATTTAAAC GGCAATATCA
1901  CGCAAACAAA CGGCAAACTG TTTTTCAGCG GCAGACCGAC ACCGCACGCC
1951  TACAATCATT TAGGAAGCGG GTGGTCAAAA ATGGAAGGTA TCCCACAAGG
2001  AGAAATCGTG TGGGACAACG ATTGGATCGA CCGCACATTT AAAGCGGAAA
2051  ACTTCCATAT TCAGGGCGGA CAAGCGGTGG TTTCCCGCAA TGTTGCCAAA
2101  GTGGAAGGCG ATTGGCATTT AAGCAATCAC GCCCAAGCAG TTTTCGGTGT
2151  CGCACCGCAT CAAAGCCACA CAATCTGTAC ACGTTCGGAC TGGACGGGTC
2201  TGACAAGTTG TACCGAAAAA ACCATTACCG ACGATAAAGT GATTGCTTCA
2251  TTGAGCAAGA CCGACATCAG AGGCAATGTC AGCCTTGCCG ATCACGCTCA
2301  TTTAAATCTC ACAGGACTTG CCACACTCAA CGGCAATCTT AGTGCAGGCG
2351  GAGACACGCA CTATACGGTT ACGCGCAACG CCACCCAAAA CGGCAACCTC
2401  AGCCTCGTGG GCAATGCCCA AGCAACATTT AATCAAGCCA CATTAAACGG
2451  CAACACATCG GCTTCGGACA ATGCTTCATT TAATCTAAGC AACAACGCCG
2501  TACAAAACGG CAGTCTGACG CTTTCCGACA ACGCTAAGGC AAACGTAAGC
2551  CATTCCGCAC TCAACGGCAA TGTCTCCCTA GCCGATAAGG CAGTATTCCA
2601  TTTTGAAAAC AGCCGCTTTA CCGGAAAAAT CAGCGGCGGC AAGGATACGG
2651  CATTACACTT AAAAGACAGC GAATGGACGC TGCCGTCGGG CACGGAATTA
2701  GGCAATTTAA ACCTTGACAA CGCCACCATT ACACTCAATT CCGCCTATCG
2751  ACACGATGCG GCAGGCGCGC AAACCGGCAG TGCGGCAGAT GCGCCGCGCC
2801  GCCGTTCGCG CCGTTCCCTA TTATCCGTTA CGCCGCCAAC TTCGGCAGAA
2851  TCCCGTTTCA ACACGCTGAC GGTAAACGGC AAATTGAACG GTCAGGGAAC
2901  ATTCCGCTTT ATGTCGGAAC TCTTCGGCTA CCGCAGCGGC AAATTGAAGC
2951  TGGCGGAAAG TTCCGAAGGC ACTTACACCT TGGCTGTCAA CAATACCGGC
3001  AACGAACCCG TAAGTCTCGA GCAATTGACG GTAGTGGAAG GAAAAGACAA
3051  CACACCGCTG TCCGAAAATC TTAATTTCAC CCTGCaaaAc gaacacgtcg
3101  atgccggcgc atggCGTTAT CAGCTTATCC gcaaagacgG CGAGTTCCgc
3151  CTGCATAATC CGGTCAAAGA ACAAGAGCTT TCCGACAAAC TCGGCAAGgc
3201  gggagaaACA GAggccgccT TGACGGCAAA ACAGGCacaA CTTGCCGCCA
3251  AAcaacaggc ggaaaAAGAC AACgcgcaaa gccttgAcgc gctgattgcg
3301  gCcgggcgca atgccaccga AAAGGCAgaa agtgttgccg aaccgGCCCG
3351  GCAGGCAGGC GGGGAAAAtg ccgGCATTAT GCAGGCGGAG GAAGAGAAAA
3401  AACGGGTGCA GGCGGATAAA GACACCGCCT TGGCGAAACA GCGCGAAGCG
3451  GAAACCCGGC CGGCTACCAC CGCCTTCCCC CGCGCCCGCC GCGCCCGCCG
3501  GGATTTGCCG CAACCGCAGC CCCAACCGCA ACCCCAACCG CAGCGCGACC
3551  TGATCAGCCG TTATGCCAAT AGCGGTTTGA GTGAATTTTC CGCCACGCTC
3601  AACAGCGTTT TCGCCGTACA GGACGAATTG GACCGCGTGT TTGCCGAAGA
3651  CCGCCGCAAC GCCGTTTGGA CAAGCGGCAT CCGGGACACC AAACACTACC
```

```
3701  GTTCGCAAGA TTTCCGCGCC TACCGCCAAC AAACCGACCT GCGCCAAATC
3751  GGTATGCAGA AAAACCTCGG CAGCGGGCGC GTCGGCATCC TGTTTTCGCA
3801  CAACCGGACC GGAAACACCT TCGACGACGG CATCGGCAAC TCGGCACGGC
3851  TTGCCCACGG TGCCGTTTTC GGGCAATACG GCATCGGCAG GTTCGACATC
3901  GGCATCAGCG CGGGCGCGGG TTTTAGTAGC GGCAGCCTTT CAGACGGCAT
3951  CAGAGGCAAA ATCCGCCGCC GCGTGCTGCA TTACGGCATT CAGGCAAGAT
4001  ACCGCGCAGG TTTCGGCGGA TTCGGCATCG AACCGCACAT CGGCGCAACG
4051  CGCTATTTCG TCCAAAAAGC GGATTACCGA TACGAAAACG TCAATATCGC
4101  CACCCCGGGC CTTGCATTCA ACCGCTACCG CGCGGGCATT AAGGCAGATT
4151  ATTCATTCAA ACCGGCGCAA CACATTTCCA TCACGCCTTA TTTGAGCCTG
4201  TCCTATACCG ATGCCGCTTC CGGCAAAGTC CGAACGCGCG TCAATACCGC
4251  CGTATTGGCG CAGGATTTCG GCAAAACCCG CAGTGCGGAA TGGGGCGTAA
4301  ACGCCGAAAT CAAAGGTTTC ACGCTGTCCC TCCACGCTGC CGCCGCCAAG
4351  GGGCCGCAAT TGGAAGCGCA GCACAGCGCG GGCATCAAAT TAGGCTACCG
4401  CTGGTAA
```

This is predicted to encode a protein having amino acid sequence <SEQ ID 654>:

```
   1  MKTTDKRTTE THRKAPKTGR IRFSPAYLAI CLSFGILPQA RAGHTYFGIN
  51  YQYYRDFAEN KGKFAVGAKD IEVYNKKGEL VGKSMTKAPM IDFSVVSRNG
 101  VAALAGDQYI VSVAHNGGYN NVDFGAEGSN PDQHRFSYQI VKRNNYKAGT
 151  NGHPYGGDYH MPRLHKFVTD AEPVEMTSYM DGWKYADLNK YPDRVRIGAG
 201  RQYWRSDEDE PNNRESSYHI ASAYSWLVGG NTFAQNGSGG GTVNLGSEKI
 251  KHSPYGFLPT GGSFGDSGSP MFIYDAQKQK WLINGVLQTG NPYIGKSNGF
 301  QLVRKDWFYD EIFAGDTHSV FYEPHQNGKY FFNDNNNGAG KIDAKHKHYS
 351  LPYRLKTRTV QLFNVSLSET AREPVYHAAG GVNSYRPRLN NGENISFIDK
 401  GKGELILTSN INQGAGGLYF EGNFTVSPKN NETWQGAGVH ISDGSTVTWK
 451  VNGVANDRLS KIGKGTLLVQ AKGENQGSVS VGDGKVILDQ QADDQGKKQA
 501  FSEIGLVSGR GTVQLNADNQ FNPDKLYFGF RGGRLDLNGH SLSFHRIQNT
 551  DEGAMIVNHN QDKESTVTIT GNKDITTTGN NNNLDSKKEI AYNGWFGEKD
 601  ATKTNGGLNL NYPPEEADRT LLLSGGTNLN GNITQTNGKL FFSGRPTPHA
 651  YNHLGSGWSK MEGIPQGEIV WDNDWIDRTF KAENFHIQGG QAVVSRNVAK
 701  VEGDWHLSNH AQAVFGVAPH QSHTICTRSD WTGLTSCTEK TITDDKVIAS
 751  LSKTDVRGNV SLADHAHLNL TGLATFNGNL VQAETRTIRL RANATQNGNL
 801  SLVGNAQATF NQATLNGNTS ASDNASFNLS NNAVQNGSLT LSDNAKANVS
 851  HSALNGNVSL ADKAVFHFEN SRFTGKISGG KDTALHLKDS EWTLPSGTEL
 901  GNLNLDNATI TLNSAYRHDA AGAQTGSAAD APRRRSRRSL LSVTPPTSAE
 951  SRFNTLTVNG KLNGQGTFRF MSELFGYRSG KLKLAESSEG TYTLAVNNTG
1001  NEPVSLEQLT VVEGKDNTPL SENLNFTLQN EHVDAGAWRY QLIRKDGEFR
1051  LHNPVKEQEL SDKLGKAGET EAALTAKQAQ LAAKQQAEKD NAQSLDALIA
1101  AGRNATEKAE SVAEPARQAG GENAGIMQAE EEKKRVQADK DTALAKQREA
1151  ETRPATTAFP RARRARRDLP QPQPQPQPQP QRDLISRYAN SGLSEFSATL
1201  NSVFAVQDEL DRVFAEDRRN AVWTSGIRDT KHYRSQDFRA YRQQTDLRQI
1251  GMQKNLGSGR VGILFSHNRT GNTFDDGIGN SARLAHGAVF GQYGIGRFDI
1301  GISAGAGFSS GSLSDGIRGK IRRRVLHYGI QARYRAGFGG FGIEPHIGAT
1351  RYFVQKADYR YENVNIATPG LAFNRYRAGI KADYSFKPAQ HISITPYLSL
1401  SYTDAASGKV RTRVNTAVLA QDFGKTRSAE WGVNAEIKGF TLSLHAAAAK
1451  GPQLEAQHSA GIKLGYRW*
```

Underlined and double-underlined sequences represent the active site of a serine protease (trypsin family) and an ATP/GTP-binding site motif A (P-loop).

ORF1-1 and ORF1ng show 93.7% identity in 1471 aa overlap:

```
                        10        20        30        40        50        60
orf1-1.pep      MKTTDKRTTETHRKAPKTGRIRFSPAYLAICLSFGILPQAWAGHTYFGINYQYYRDFAEN
                |||||||||||||||||||||||||||||||||||||||| |||||||||||||||||||
orf1ng-1        MKTTDKRTTETHRKAPKTGRIRFSPAYLAICLSFGILPQARAGHTYFGINYQYYRDFAEN
                        10        20        30        40        50        60

                        70        80        90       100       110       120
orf1-1.pep      KGKFAVGAKDIEVYNKKGELVGKSMTKAPMIDFSVVSRNGVAALVGDQYIVSVAHNGGYN
                ||||||||||||||||||||||||||||||||||||||||||||:|||||||||||||||
orf1ng-1        KGKFAVGAKDIEVYNKKGELVGKSMTKAPMIDFSVVSRNGVAALAGDQYIVSVAHNGGYN
                        70        80        90       100       110       120

                       130       140       150       160       170       180
orf1-1.pep      NVDFGAEGRNPDQHRFTYKIVKRNNYKAGTKGHPYGGDYHMPRLHKFVTDAEPVEMTSYM
                |||||||| |||||||:|:|||||||||||:|||||||||||||||||||||||||||||
```

```
orf1ng-1      NVDFGAEGSNPDQHRFSYQIVKRNNYKAGTNGHPYGGDYHMPRLHKFVTDAEPVEMTSYM
                      130       140       150       160       170       180

                      190       200       210       220       230       240
orf1-1.pep    DGRKYIDQNNYPDRVRIGAGRQYWRSDEDEPNNRESSYHIASAYSWLVGGNTFAQNGSGG
              || || | |:||||||||||||||||||||||||||||||||||||||||||||||||||
orf1ng-1      DGWKYADLNKYPDRVRIGAGRQYWRSDEDEPNNRESSYHIASAYSWLVGGNTFAQNGSGG
                      190       200       210       220       230       240

                      250       260       270       280       290       300
orf1-1.pep    GTVNLGSEKIKHSPYGFLPTGGSFGDSGSPMFIYDAQKQKWLINGVLQTGNPYIGKSNGF
              ||||||||||||||||||||||||||||||||||||||||||||||||||||||||||||
orf1ng-1      GTVNLGSEKIKHSPYGFLPTGGSFGDSGSPMFIYDAQKQKWLINGVLQTGNPYIGKSNGF
                      250       260       270       280       290       300

                      310       320       330       340       350       360
orf1-1.pep    QLVRKDWFYDEIFAGDTHSVFYEPRQNGKYSFNDDNNGTGKINAKHEHNSLPNRLKTRTV
              ||||||||||||||||||||||||:||||| |||:|||:|||:|||:| ||| |||||||
orf1ng-1      QLVRKDWFYDEIFAGDTHSVFYEPHQNGKYFFNDNNNGAGKIDAKHKHYSLPYRLKTRTV
                      310       320       330       340       350       360

                      370       380       390       400       410       420
orf1-1.pep    QLFNVSLSETAREPVYHAAGGVNSYRPRLNNGENISFIDEGKGELILTSNINQGAGGLYF
              |||||||||||||||||||||||||||||||||||||||:||||||||||||||||||||
orf1ng-1      QLFNVSLSETAREPVYHAAGGVNSYRPRLNNGENISFIDKGKGELILTSNINQGAGGLYF
                      370       380       390       400       410       420

                      430       440       450       460       470       480
orf1-1.pep    QGDFTVSPENNETWQGAGVHISEDSTVTWKVNGVANDRLSKIGKGTLHVQAKGENQGSIS
              :|:|||||:|||||||||||||: ||||||||||||||||||||||| ||||||||||:|
orf1ng-1      EGNFTVSPKNNETWQGAGVHISDGSTVTWKVNGVANDRLSKIGKGTLLVQAKGENQGSVS
                      430       440       450       460       470       480

                      490       500       510       520       530       540
orf1-1.pep    VGDGTVILDQQADDKGKKQAFSEIGLVSGRGTVQLNADNQFNPDKLYFGFRGGRLDLNGH
              |||| ||||||||:||||||||||||||||||||||||||||||||||||||||||||||
orf1ng-1      VGDGKVILDQQADDQGKKQAFSEIGLVSGRGTVQLNADNQFNPDKLYFGFRGGRLDLNGH
                      490       500       510       520       530       540

                      550       560       570       580       590       600
orf1-1.pep    SLSFHRIQNTDEGAMIVNHNQDKESTVTITGNKDIATTGNNNSLDSKKEIAYNGWFGEKD
              |||||||||||||||||||||||||||||||||||:||||||:|||||||||||||||||
orf1ng-1      SLSFHRIQNTDEGAMIVNHNQDKESTVTITGNKDITTTGNNNNLDSKKEIAYNGWFGEKD
                      550       560       570       580       590       600

                      610       620       630       640       650       660
orf1-1.pep    TTKTNGRLNLVYQPAAEDRTLLLSGGTNLNGNITQTNGKLFFSGRPTPHAYNHLNDHWSQ
              :||||||||| |||   |||||||||||||||||||||||||||||||||||||:: ||:
orf1ng-1      ATKTNGRLNLNYQPEEADRTLLLSGGTNLNGNITQTNGKLFFSGRPTPHAYNHLGSGWSK
                      610       620       630       640       650       660

                      670       680       690       700       710       720
orf1-1.pep    KEGIPRGEIVWDNDWINRTFKAENFQIKGGQAVVSRNVAKVKGDWHLSNHAQAVFGVAPH
               ||||:||||||||||:||||||||:|:|||||||||||||:||||||||||||||||||
orf1ng-1      MEGIPQGEIVWDNDWIDRTFKAENFHIQGGQAVVSRNVAKVEGDWHLSNHAQAVFGVAPH
                      670       680       690       700       710       720

                      730       740       750       760       770       780
orf1-1.pep    QSHTICTRSDWTGLTNCVEKTITDDKVIASLTKTDISGNVDLADHAHLNLTGLATLNGNL
              |||||||||||||||:|:|||||||||||||:|||| |||:|||||||||||||||||||
orf1ng-1      QSHTICTRSDWTGLTSCTEKTITDDKVIASLSKTDIRGNVSLADHAHLNLTGLATLNGNL
                      730       740       750       760       770       780

                      790       800       810       820       830       840
orf1-1.pep    SANGDTRYTVSHNATQNGNLSLVGNAQATFNQATLNGNTSASGNASFNLSDHAVQNGSLT
              ||:|||:|||::|||||||||||||||||||||||||||||||| |||||||::||||||||
orf1ng-1      SAGGDTHYTVTRNATQNGNLSLVGNAQATFNQATLNGNTSASDNASFNLSNNAVQNGSLT
                      790       800       810       820       830       840

                      850       860       870       880       890       900
orf1-1.pep    LSGNAKANVSHSALNGNVSLADKAVFHFESSRFTGQISGGKDTALHLKDSEWTLPSGTEL
              || ||||||||||||||||||||||||||:|||||:||||||||||||||||||||||||
```

```
orf1ng-1       LSDNAKANVSHSALNGNVSLADKAVFHFENSRFTGKISGGKDTALHLKDSEWTLPSGTEL
                      850       860       870       880       890       900

                      910       920       930       940       950       960
orf1-1.pep     GNLNLDNATITLNSAYRHDAAGAQTGSATDAPRRRSRRSRRSLLSVTPPTSVESRFNTLT
               ||||||||||||||||||||||||||||:||||||||   ||||||||||:||||||||
orf1ng-1       GNLNLDNATITLNSAYRHDAAGAQTGSAADAPRRRSR---RSLLSVTPPTSAESRFNTLT
                      910       920       930          940       950

                      970       980       990      1000      1010      1020
orf1-1.pep     VNGKLNGQGTFRFMSELFGYRSDKLKLAESSEGTYTLAVNNTGNEPASLEQLTVVEGKDN
               |||||||||||||||||||||| |||||||||||||||||||||||:|||||||||||||
orf1ng-1       VNGKLNGQGTFRFMSELFGYRSGKLKLAESSEGTYTLAVNNTGNEPVSLEQLTVVEGKDN
               960       970       980       990      1000      1010

                     1030      1040      1050      1060      1070
orf1-1.pep     KPLSENLNFTLQNEHVDAGAWRYQLIRKDGEFRLHNPVKEQELSDKLGKA----------
                |||||||||||||||||||||||||||||||||||||||||||||||||
orf1ng-1       TPLSENLNFTLQNEHVDAGAWRYQLIRKDGEFRLHNPVKEQELSDKLGKAGETEAALTAK
              1020      1030      1040      1050      1060      1070

                          1080      1090      1100      1110      1120
orf1-1.pep     ----EAKKQAEKDNAQSLDALIAAGRDAVEKTESVAEPARQAGGENVGIMQAEEEKKRVQ
                    ||:||||||||||||||||||:|:||:|||||||||||||:||||||||||||
orf1ng-1       QAQLAAKQQAEKDNAQSLDALIAAGRNATEKAESVAEPARQAGGENAGIMQAEEEKKRVQ
              1080      1090      1100      1110      1120      1130

               1130      1140      1150      1160      1170      1180
orf1-1.pep     ADKDTALAKQREAETRPATTAFPRARRARRDLPQLQPQPQPQPQRDLISRYANSGLSEFS
               |||||||||||||||||||||||||||||||||| |||||||||||||||||||||||||
orf1ng-1       ADKDTALAKQREAETRPATTAFPRARRARRDLPQPQPQPQPQPQRDLISRYANSGLSEFS
              1140      1150      1160      1170      1180      1190

               1190      1200      1210      1220      1230      1240
orf1-1.pep     ATLNSVFAVQDELDRVFAEDRRNAVWTSGIRDTKHYRSQDFRAYRQQTDLRQIGMQKNLG
               ||||||||||||||||||||||||||||||||||||||||||||||||||||||||||||
orf1ng-1       ATLNSVFAVQDELDRVFAEDRRNAVWTSGIRDTKHYRSQDFRAYRQQTDLRQIGMQKNLG
              1200      1210      1220      1230      1240      1250

               1250      1260      1270      1280      1290      1300
orf1-1.pep     SGRVGILFSHNRTENTFDDGIGNSARLAHGAVFGQYGIDRFYIGISAGAGFSSGSLSDGI
               ||||||||||||| |||||||||||||||||||||||||| || |||||||||||||||||
orf1ng-1       SGRVGILFSHNRTGNTFDDGIGNSARLAHGAVFGQYGIGRFDIGISAGAGFSSGSLSDGI
              1260      1270      1280      1290      1300      1310

               1310      1320      1330      1340      1350      1360
orf1-1.pep     GGKIRRRVLHYGIQARYRAGFGGFGIEPHIGATRYFVQKADYRYENVNIATPGLAFNRYR
                |||||||||||||||||||||||||||||||||||||||||||||||||||||||||||
orf1ng-1       RGKIRRRVLHYGIQARYRAGFGGFGIEPHIGATRYFVQKADYRYENVNIATPGLAFNRYR
              1320      1330      1340      1350      1360      1370

               1370      1380      1390      1400      1410      1420
orf1-1.pep     AGIKADYSFKPAQHISITPYLSLSYTDAASGKVRTRVNTAVLAQDFGKTRSAEWGVNAEI
               ||||||||||||||||||||||||||||||||||||||||||||||||||||||||||||
orf1ng-1       AGIKADYSFKPAQHISITPYLSLSYTDAASGKVRTRVNTAVLAQDFGKTRSAEWGVNAEI
              1380      1390      1400      1410      1420      1430

               1430      1440      1450
orf1-1.pep     KGFTLSLHAAAAKGPQLEAQHSAGIKLGYRWX
               ||||||||||||||||||||||||||||||||
orf1ng-1       KGFTLSLHAAAAKGPQLEAQHSAGIKLGYRWX
              1440      1450      1460
```

In addition, ORF1ng shows 55.7% identity with hap protein (P45387) over a 1455aa overlap:

```
SCORES      Initl:  1104 Initn:  4632 Opt:  2680
Smith-Waterman score: 5165;    55.7% identity in 1455 aa overlap

                          10        20        30        40        50        60
orf1ng-1.pep    MKTTDKRTTETHRKAPKTGRIRFSPAYLAICLSFGILPQARAGHTYFGINYQYYRDFAEN
                                      |   :|: |:|:||: || ||||||||:||||||||||
```

```
p45387                             MKKTVFRLNFLTACISLGIVSQAWAGHTYFGIDYQYYRDFAEN
                                           10        20        30        40

                   70        80        90       100       110       120
orflng-1.pep  KGKFAVGAKDIEVYNKKGELVGKSMTKAPMIDFSVVSRNGVAALAGDQYIVSVAHNGGYN
              ||||:|||::|:||||:|:||| ||||||||||||||||||||: :||||||||| ||:
p45387        KGKFTVGAQNIKVYNKQGQLVGTSMTKAPMIDFSVVSRNGVAALVENQYIVSVAHNVGYT
                  50        60        70        80        90       100

                  130       140       150       160       170       180
orflng-1.pep  NVDFGAEGSNPDQHRFSYQIVKRNNYKAGTNGHPYGGDYHMPRLHKFVTDAEPVEMTSYM
              :|||||||:||||||||:|:||||||||     | |||  ||| ||||||||:| |::||| |
p45387        DVDFGAEGNNPDQHRFTYKIVKRNNYKKD-NLHPYEDDYHNPRLHKFVTEAAPIDMTSNM
                 110       120       130       140       150       160

                  190       200       210       220       230       240
orflng-1.pep  DGWKYADLNKYPDRVRIGAGRQYWRSDEDEPNNRESSYHIASAYSWLVGGNTFAQNGSGG
              :|  |:| :|||:|||||:|||:||:|:|: :        ::|:|| :|::|||  | |:|:
p45387        NGSTYSDRTKYPERVRIGSGRQFWRNDQDKGD------QVAGAYHYLTAGNTHNQRGAGN
                  170       180       190            200       210

                  250       260       270       280       290       300
orflng-1.pep  GTVNLGSEKIKHSPYGFLPTGGSFGDSGSPMFIYDAQKQKWLINGVLQTGNPYIGKSNGF
              |   ||::  |  : || || :|| ||||||||||||:||||||||:|: |||: || |||
p45387        GYSYLGGDVRKAGEYGPLPIAGSKGDSGSPMFIYDAEKQKWLINGILREGNPFEGKENGF
               220       230       240       250       260       270

                  310       320       330       340       350       360
orflng-1.pep  QLVRKDWFYDEIFAGDTHSVFYEPHQNGKYFFNDNNNGAGKIDAKHKHYSLPYRLKTRTV
              |||||::| ||||  | |: :|      || | :: |:|| |:|  |       ::| ::|   :
p45387        QLVRKSYF-DEIFERDLHTSLYTRAGNGVYTISGNDNGQGSITQKS---GIPSEIK---I
               280       290       300       310       320

                  370       380       390       400       410      419
orflng-1.pep  QLFNVSLSETAREPVYHAA-GGVNSYRPRLNNGENISFIDKGKGELILTSNINQGAGGLY
               | |:||      :: |::      | | | |||||||:: |:|: :| ||::|:||||||||
p45387        TLANMSLPLKEKDKVHNPRYDGPNIYSPRLNNGETLYFMDQKQGSLIFASDINQGAGGLY
            330       340       350       360       370       380

            420       430       440       450       460       470      479
orflng-1.pep  FEGNFTVSPKNNETWQGAGVHISDGSTVTWKVNGVANDRLSKIGKGTLLVQAKGENQGSV
              |||||||||::|:||||||:|:|::|||||||||| :||||||||||| |||||||:||:
p45387        FEGNFTVSPNSNQTWQGAGIHVSENSTVTWKVNGVEHDRLSKIGKGTLHVQAKGENKGSI
            390       400       410       420       430       440

            480       490       500       510       520       530      539
orflng-1.pep  SVGDGKVILDQQADDQGKKQAFSEIGLVSGRGTVQLNADNQFNPDKLYFGFRGGRLDLNG
              ||||||||:||||||:||||||||||||||||||| |:||: ||:|||||||||||||
p45387        SVGDGKVILEQQADDQGNKQAFSEIGLVSGRGTVQLNDDKQFDTDKFYFGFRGGRLDLNG
            450       460       470       480       490       500

            540       550       560       570       580       590
orflng-1.pep  HSLSFHRIQNTDEGAMIVNHNQDKESTVTITGNKDITT-TGNN-NNLDSKKEIAYNGWFG
              |||:|:|||||||||||||||  : ::||||||::|:  :|||  |:|| :||||||||||
p45387        HSLTFKRIQNTDEGAMIVNHNTTQAANVTITGNESIVLPNGNNINKLDYRKEIAYNGWFG
            510       520       530       540       550       560

            600       610       620       630       640       650
orflng-1.pep  EKDATKTNGRLNLNYQPEEADRTLLLSGGTNLNGNITQTNGKLFFSGRPTPHAYNHLGSG
              | | :| ||||||  |:|     ||||||||||||:|:||||:||||||||||||||||||::
p45387        ETDKNKHNGRLNLIYKPTTEDRTLLLSGGTNLKGDITQTKGKLFFSGRPTPHAYNHLNKR
            570       580       590       600       610       620

            660       670       680       690       700       710
orflng-1.pep  WSKMEGIPQGEIVWDNDWIDRTFKAENFHIQGGQAVVSRNVAKVEGDWHLSNHAQAVFGV
              ||:||||||||||||:||:||||||||:|:|:|||||||:::||:| :||:|:|:|||
p45387        WSEMEGIPQGEIVWDHDWINRTFKAENFQIKGGSAVVSRNVSSIEGNWTVSNNANATFGV
            630       640       650       660       670       680

            720       730       740       750       760       770
orflng-1.pep  APHQSHTICTRSDWTGLTSCTEKTITDDKVIASLSKTDIRGNVSLADHAHLNLTGLATLN
              :|:|::|||||||||||||:| :  :|| ||| |: ||:| |:::|:|:|  |: ||| ||
```

```
p45387            VPNQQNTICTRSDWTGLTTCQKVDLTDTKVINSIPKTQINGSINLTDNATANVKGLAKLN
                690       700       710       720       730       740

                  780       790       800       810       820       830
orflng-1.pep      GNLSAGGDTHYTVTRNATQNGNLSLVGNAQATFNQATLNGNTSASDNASFNLSNNAVQNG
                  ||::                                        :::::|:|||||:| |
p45387            GNVTL---------------------------------------TNHSQFTLSNNATQIG
                750                                                 760       770

                  840       850       860       870       880       890
orflng-1.pep      SLTLSDNAKANVSHSALNGNVSLADKAVFHFENSRFTGKISGGKDTALHLKDSEWTLPSG
                  :: ||||: |:|::: ||||| |:|:| | ::||:|: :|:| | |:: |::: ||:||
p45387            NIRLSDNSTATVDNANLNGNVHLTDSAQFSLKNSHFSHQIQGDKGTTVTLENATWTMPSD
                        780       790       800       810       820       830

                  900       910       920       930       940       950
orflng-1.pep      TELGNLNLDNATITLNSAYRHDAAGAQTGSAADAPRRRSRRSLLSVTPPTSAESRFNTLT
                  | | ||:|:|:||||||||        ::|: ::|||||   | : | ||||| ||||||
p45387            TTLQNLTLNNSTITLNSAY--------SASSNNTPRRRS---LETETTPTSAEHRFNTLT
                        840                 850       860         870

                  960       970       980       990       1000      1010
orflng-1.pep      VNGKLNGQGTFRFMSELFGYRSGKLKLAESSEGTYTLAVNNTGNEPVSLEQLTVVEGKDN
                  |||||:|||||:| | ||||:| ||||::::|| | |:| |||:|| :|||||:||:|||
p45387            VNGKLSGQGTFQFTSSLFGYKSDKLKLSNDAEGDYILSVRNTGKEPETLEQLTLVESKDN
                880       890       900       910       920       930

                 1020      1030      1040      1050      1060      1070
orflng-1.pep      TPLSENLNFTLQNEHVDAGAWRYQLIRKDGEFRLHNPVKEQELSDKLGKAGETEAALTAK
                   |||::|:|||:|:|||||| ||:|:::|||||||||:||||| : | :| ::| :| ||
p45387            QPLSDKLKFTLENDHVDAGALRYKLVKNDGEFRLHNPIKEQELHNDLVRAEQAERTLEAK
                940       950       960       970       980       990

                 1080      1090      1100      1110      1120      1130
orflng-1.pep      QAQLAAKQQAEKDNAQSLDALIAAGRNAT-EKAESVAEPARQAGGENAGIMQAEEEKKRV
                  |:: :|| |: : :::|  |  ||  ::  :::   |  |:||   :|  :::: : |:|
p45387            QVEPTAKTQTGEPKVRSRRAARAAFPDTLPDQSLLNALEAKQAE-LTAETQKSKAKTKKV
               1000      1010      1020      1030      1040      1050

                  1140          1150      1160      1170      1180      1190
orflng-1.pep      QADK---DTALAKQREAETRPATTAFPRARRARRD-LPQPQPQPQPQPQRDLISRYANSG
                  :: :   :  |  |      :|  ::   :::::| | | : : | : |:||||||:||:
p45387            RSKRAVFSDPLLDQSLFALEAALEVIDAPQQSEKDRLAQEEAEKQ-RKQKDLISRYSNSA
                1060      1070      1080      1090      1100      1110

                      1200      1210      1220      1230      1240      1250
orflng-1.pep      LSEFSATLNSVFAVQDELDRVFAEDRRNAVWTSGIRDTKHYRSQDFRAYRQQ-TDLRQIG
                  |||:|||:||:::|||||||:|::: ::||||:  :| ::| |: ||||:|| |:|||||
p45387            LSELSATVNSMLSVQDELDRLFVDQAQSAVWTNIAQDKRRYDSDAFRAYQQQKTNLRQIG
                 1120      1130      1140      1150      1160      1170

                      1260      1270      1280      1290      1300      1310
orflng-1.pep      MQKNLGSGRVGILFSHNRTGNTFDDGIGNSARLAHGAVFGQYGIGRFDIGISAGAGFSSG
                  :|| |::||:| :|||:|: ||||: : | | |:  : |:||  | :::|:::|:|:|::
p45387            VQKALANGRIGAVFSHSRSDNTFDEQVKNHATLTMMSGFAQYQWGDLQFGVNVGTGISAS
                 1180      1190      1200      1210      1220      1230

                      1320      1330      1340      1350      1360      1370
orflng-1.pep      SLSDGIRGKIRRRVLHYGIQARYRAGFGGFGIEPHIGATRYFVQKADYRYENVNIATPGL
                  ::::    ||:|::::||::| |:  :| :||:|::|::|||::: :|: |:| : ||:|
p45387            KMAEEQSRKIHRKAINYGVNASYQFRLGQLGIQPYFGVNRYFIERENYQSEEVRVKTPSL
                 1240      1250      1260      1270      1280      1290

                      1380      1390      1400      1410      1420      1430
orflng-1.pep      AFNRYRAGIKADYSFKPAQHISITPYLSLSYTDAASGKVRTRVNTAVLAQDFGKTRSAEW
                  ||||| |||::||:| |:::||:||: ::|:|:::::|:| || :|| | ||:  : |
p45387            AFNRYNAGIRVDYTFTPTDNISVKPYFFVNYVDVSNANVQTTVNLTVLQQPFGRYWQKEV
                 1300      1310      1320      1330      1340      1350

                      1440      1450      1460      1469
orflng-1.pep      GVNAEIKGFTLSLHAAAAKGPQLEAQHSAGIKLGYRWX
                  |::|||   | :|    : ::| ||   |:::|:||||||
```

```
p45387          GLKAEILHFQISAFISKSQGSQLGKQQNVGVKLGYRW
               1360      1370      1380      1390
```

Based on this analysis, it is predicted that these proteins from *N.meningitidis* and *N.gonorrhoeae,* and their epitopes, could be useful antigens for vaccines or diagnostics, or for raising antibodies.

**Example 78**

The following partial DNA sequence was identified in *N.meningitidis* <SEQ ID 655>:

```
  1  ..AAGGTGTGGC AATTTGTCGA AGA.CCGCTG CGTGCCGTCG TGCCTGCCGA
 51    CAGTTTTGAA CCGACCGCGC AAAAATTGAA CCTGTTTAAG GCGGGTGCGG
101    CAACCATTTT GTTTTATGAA GATCAAAATG TCGTCAAAGG TTTGCAGGAG
151    CAGTTCCCTG CTTATGCCGC TAACTTCCCC GTTTGGGCGg ATCAGGCAAA
201    CGCGATGGTG CAGTATGCCG TTTGGACGAC ACTTGCCGCG GTCGGCGTAG
251    GTGCAAACCT GCAACATTAC AATCCCTTGC CCGATGCGGC GATTGCCAAA
301    GCGTGGAATA TCCCCGAAAA CTGGTTGTTG CGCGCACAAA TGGTTATCGG
351    CGGTATTGAA GGGGCGGCAG GTGAAAAGAC CTTTGAACCC GTTGCAGAAC
401    GTTTGAAAGT GTTCGGCGCA TAA
```

This corresponds to the amino acid sequence <SEQ ID 656; ORF6>:

```
  1  ..KVWQFVEXPL RAVVPADSFE PTAQKLNLFK AGAATILFYE DQNVVKGLQE
 51    QFPAYAANFP VWADQANAMV QYAVWTTLAA VGVGANLQHY NPLPDAAIAK
101    AWNIPENWLL RAQMVIGGIE GAAGEKTFEP VAERLKVFGA *
```

Further sequence analysis revealed a further partial DNA sequence <SEQ ID 657>:

```
  1  ..CTGCGTGCCG TCGTGCCTGC CGACAGTTTT GAACCGACCG CGCAAAAATT
 51    GAACCTGTTT AAGGCGGGTG CGGCAACCAT TTTGTTTTAT GAAGATCAAA
101    ATGTCGTCAA AGGTTTGCAG GAGCAGTTCC CTGCTTATGC CGCTAACTTC
151    CCCGTTTGGG CGGATCAGGC AAACGCGATG GTGCAGTATG CCGTTTGGAC
201    GACACTTGCC GCGGTCGGCG TAGGTGCAAA CCTGCAACAT TACAATCCCT
251    TGCCCGATGC GGCGATTGCC AAAGCGTGGA ATATCCCCGA AAACTGGTTG
301    TTGCGCGCAC AAATGGTTAT CGGCGGTATT GAAGGGGCGG CAGGTGAAAA
351    GACCTTTGAA CCCGTTGCAG AACGTTTGAA AGTGTTCGGC GCATAA
```

This corresponds to the amino acid sequence <SEQ ID 658; ORF6-1>:

```
  1  ..LRAVVPADSF EPTAQKLNLF KAGAATILFY EDQNVVKGLQ EQFPAYAANF
 51    PVWADQANAM VQYAVWTTLA AVGVGANLQH YNPLPDAAIA KAWNIPENWL
101    LRAQMVIGGI EGAAGEKTFE PVAERLKVFG A*
```

Computer analysis of this amino acid sequence gave the following results:

Homology with a predicted ORF from *N. meningitidis* (strain A)

ORF6 shows 98.6% identity over a 140aa overlap with an ORF (ORF6a) from strain A of *N. meningitidis:*

```
                                                      10        20        30
orf6.pep                                    KVWQFVEXPLRAVVPADSFEPTAQKLNLFK
                                            |||||||  |||||||||||||||||||||
orf6a         QIVEHAVLHTPSSFNSQSARVVVLFGEEHDKVWQFVEDALRAVVPADSFEPTAQKLNLFK
                      40        50        60        70        80        90

                      40        50        60        70        80        90
orf6.pep      AGAATILFYEDQNVVKGLQEQFPAYAANFPVWADQANAMVQYAVWTTLAAVGVGANLQHY
              ||||||||||||||||||||||||||||||||||||||||||||||||||||||||||||
orf6a         AGAATILFYEDQNVVKGLQEQFPAYAANFPVWADQANAMVQYAVWTTLAAVGVGANLQHY
                     100       110       120       130       140       150

                     100       110       120       130       140
orf6.pep      NPLPDAAIAKAWNIPENWLLRAQMVIGGIEGAAGEKTFEPVAERLKVFGAX
              ||||||||||||||||||||||||||||||||||||||||||||||||||
orf6a         NPLPDAAIAKAWNIPENWLLRAQMVIGGIEGAAGEKTFEPVAERLKVFGAX
```

```
                     160       170       180       190       200
```

The complete length ORF6a nucleotide sequence <SEQ ID 659> is:

```
  1  ATGACCCGTC AATCTCTGCA ACAGGCTGCC GAAAGCCGCC GTTCCATTTA
 51  TTCGTTAAAT AAAAATCTGC CCGTCGGCAA AGATGAAATC GTCCAAATCG
101  TCGAACACGC CGTTTTGCAC ACACCTTCTT CGTTCAATTC CCAATCTGCC
151  CGTGTGGTCG TGCTGTTTGG CGAAGAGCAT GATAAGGTGT GGCAATTTGT
201  CGAAGACGCG CTGCGTGCCG TCGTGCCTGC CGACAGTTTT GAACCGACCG
251  CGCAAAAATT GAACCTGTTT AAGGCGGGTG CGGCAACTAT TTTGTTTTAT
301  GAAGATCAAA ATGTCGTCAA AGGTTTGCAG GAGCAGTTCC CTGCTTATGC
351  CGCCAACTTT CCCGTTTGGG CGGACCAGGC GAACGCGATG GTGCAGTATG
401  CCGTTTGGAC GACACTTGCC GCGGTCGGCG TAGGTGCAAA CCTGCAACAT
451  TACAATCCCT TGCCCGATGC GGCGATTGCC AAAGCGTGGA ATATCCCCGA
501  AAACTGGTTG TTGCGCGCAC AAATGGTTAT CGGCGGTATT GAAGGGGCGG
551  CAGGTGAAAA GACCTTTGAA CCAGTTGCAG AACGTTTGAA AGTGTTCGGC
601  GCATAA
```

This is predicted to encode a protein having amino acid sequence <SEQ ID 660>:

```
  1  MTRQSLQQAA ESRRSIYSLN KNLPVGKDEI VQIVEHAVLH TPSSFNSQSA
 51  RVVVLFGEEH DKVWQFVEDA LRAVVPADSF EPTAQKLNLF KAGAATILFY
101  EDQNVVKGLQ EQFPAYAANF PVWADQANAM VQYAVWTTLA AVGVGANLQH
151  YNPLPDAAIA KAWNIPENWL LRAQMVIGGI EGAAGEKTFE PVAERLKVFG
201  A*
```

ORF6a and ORF6-1 show 100.0% identity in 131 aa overlap:

```
                    50        60        70        80        90        100
orf6a.pep    TPSSFNSQSARVVVLFGEEHDKVWQFVEDALRAVVPADSFEPTAQKLNLFKAGAATILFY
                                                ||||||||||||||||||||||||||||||
orf6-1                                          LRAVVPADSFEPTAQKLNLFKAGAATILFY
                                                        10        20        30

                   110       120       130       140       150       160
orf6a.pep    EDQNVVKGLQEQFPAYAANFPVWADQANAMVQYAVWTTLAAVGVGANLQHYNPLPDAAIA
             ||||||||||||||||||||||||||||||||||||||||||||||||||||||||||||
orf6-1       EDQNVVKGLQEQFPAYAANFPVWADQANAMVQYAVWTTLAAVGVGANLQHYNPLPDAAIA
                    40        50        60        70        80        90

                   170       180       190       200
orf6a.pep    KAWNIPENWLLRAQMVIGGIEGAAGEKTFEPVAERLKVFGAX
             ||||||||||||||||||||||||||||||||||||||||||
orf6-1       KAWNIPENWLLRAQMVIGGIEGAAGEKTFEPVAERLKVFGAX
                   100       110       120       130
```

Homology with a predicted ORF from *N.gonorrhoeae*

ORF6 shows 95.7% identity over a 140aa overlap with a predicted ORF (ORF6ng) from *N.gonorrhoeae:*

```
orf6.pep                                 KVWQFVEXPLRAVVPADSFEPTAQKLNLFK   30
                                         |||||||  ||||||||||||||||||:|||
orf6ng     SNVSLDMSNPTVLRMGLPLYIASLRRGAIYKVWQFVEDALRAVVPADSFEPTAQKLKLFK   64

orf6.pep   AGAATILFYEDQNVVKGLQEQFPAYAANFPVWADQANAMVQYAVWTTLAAVGVGANLQHY   90
           |||||||||||||||||||||||||||||||||||||||||||||||||||||:|||||||
orf6ng     AGAATILFYEDQNVVKGLQEQFPAYAANFPVWADQANAMVQYAVWTTLAAVGAGANLQHY  124

orf6.pep   NPLPDAAIAKAWNIPENWLLRAQMVIGGIEGAAGEKTFEPVAERLKVFGA  140
           |||||:||||||||||||||||||||||||||||||||:||||||||||||
orf6ng     NPLPDVAIAKAWNIPENWLLRAQMVIGGIEGAAGEKVFEPVAERLKVFGA  174
```

The complete length ORF6ng nucleotide sequence <SEQ ID 661> was identified as:

```
  1  ATGGCCGTTG CGTCAAATGT CAGCTTGGAT ATGTCCAATC CTACGGTGTT
 51  ACGCATGGGA TTACCCTTAT ATATTGCGTC CCTAAGAAGG GGCGCAATAT
101  ATAAGGTGTG GCAATTTGTC GAAGACGCGC TGCGTGCCGT CGTGCCTGCC
151  GACAGTTTTG AACCGACCGC GCAAAAATTG AAGCTGTTTA AGGCGGGCGC
201  GGCAACCATT TTGTTTTATG AAGATCAAAA TGTCGTCAAA GGTTTGCAGG
251  AGCAGTTCCC TGCTTATGCC GCCAACTTTC CCGTTTGGGC GGACCAGGCG
301  AACGCTATGG TACAGTATGC CGTCTGGACG ACACTTGCCG CGGTCGGTGC
351  AGGTGCAAAT CTGCAACATT ACAACCCCTT GCCCGATGTG GCGATTGCTA
401  AAGCGTGGAA TATTCCCGAA AACTGGCTGT TGCGCGCGCA AATGGTTATC
451  GGTGGTATTG AAGGGGcggc aggtgaaaaa gtctttgaac CCGTTGCgga
501  acgtttgAAA GTGTTCGGCG CATAA
```

This encodes a protein having amino acid sequence <SEQ ID 662>:

```
  1  MAVASNVSLD MSNPTVLRMG LPLYIASLRR GAIYKVWQFV EDALRAVVPA
 51  DSFEPTAQKL KLFKAGAATI LFYEDQNVVK GLQEQFPAYA ANFPVWADQA
101  NAMVQYAVWT TLAAVGAGAN LQHYNPLPDV AIAKAWNIPE NWLLRAQMVI
151  GGIEGAAGEK VFEPVAERLK VFGA*
```

ORF6ng and ORF6-1 show 96.9% identity in 131 aa overlap:

```
                                                      10        20        30
orf6-1.pep                                  LRAVVPADSFEPTAQKLNLFKAGAATILFY
                                            ||||||||||||||||:|||||||||||||
orf6ng        PTVLRMGLPLYIASLRRGAIYKVWQFVEDALRAVVPADSFEPTAQKLKLFKAGAATILFY
                  20        30        40        50        60        70

                40        50        60        70        80        90
orf6-1.pep    EDQNVVKGLQEQFPAYAANFPVWADQANAMVQYAVWTTLAAVGVGANLQHYNPLPDAAIA
              |||||||||||||||||||||||||||||||||||||||||:|||||||||||||:|||
orf6ng        EDQNVVKGLQEQFPAYAANFPVWADQANAMVQYAVWTTLAAVGAGANLQHYNPLPDVAIA
                  80        90       100       110       120       130

                100       110       120       130
orf6-1.pep    KAWNIPENWLLRAQMVIGGIEGAAGEKTFEPVAERLKVFGAX
              ||||||||||||||||||||||||||:||||||||||||||
orf6ng        KAWNIPENWLLRAQMVIGGIEGAAGEKVFEPVAERLKVFGAX
                 140       150       160       170
```

It is predicted that the proteins from *N.meningitidis* and *N.gonorrhoeae,* and their epitopes, could be useful antigens for vaccines or diagnostics, or for raising antibodies.

**Example 79**

The following partial DNA sequence was identified in *N.meningitidis* <SEQ ID 663>

```
  1  ..GGCTACAACT ACCTGTTCGC GCGCGGCAGC CGCATCGCCA ACTACCAAAT
 51    CAACGGCATC CCCGTTGCCG ACGCGCTGGC CGATACGGGt CAATGCCAAC
101    ACCGCCGCCT ATGAGCGCGT AGAAGTCGTG CGCGGCGTGG CGGGGCTGCT
151    GGACGGCACG GGCGAGCCTT CCGCCACCGT CAATCTGGTG CGCAAACGCC
201    TGACCCGCAA GCCATTGTTT GAAGTCCGCG CCGAAGCgGG CAACCGcAAA
251    CATTTCGGGC TGGACGCGGA CGTATCGGGC AGCCTGAACA CCGAAG.crC
301    rCTGCGCgGC CGCCTGGTTT CCAcCTTCGG ACGCGGCGAC TCGTGGCGGC
351    GGCGCGAACG CAGCCGskAT GCCGAACTCT ACGGCATTTT GGAATACGAC
401    ATCGCACCGC AAACCCGCGT CCACGCArGC ATGGACTACC AGCAGGCGAA
451    AGAAACCGCC GACGCGCCGC TCAGcTACGC CGTGTACGAC AGCCAAGGTT
501    ATGCCACCGC CTTCGGCCCG AAAGACAACC CCGCCACAAA TTGGGCGAAC
551    AGCCACCACC GTGCGCTCAA CCTGTTCGCC GGCATCGAAC ACCGCTTCAA
601    CCAAGACTGG AAACTCAAAG CCGAATACGA CTAC..
```

This corresponds to the amino acid sequence <SEQ ID 664; ORF23>:

```
  1  ..GYNYLFARGS RIANYQINGI PVADALADTG NANTAAYERV EVVRGVAGLL
 51    DGTGEPSATV NLVRKRLTRK PLFEVRAEAG NRKHFGLDAD VSGSLNTEXX
101    LRGRLVSTFG RGDSWRRRER SRXAELYGIL EYDIAPQTRV HAXMDYQQAK
151    ETADAPLSYA VYDSQGYATA FGPKDNPATN WANSHHRALN LFAGIEHRFN
201    QDWKLKAEYD Y..
```

Further work revealed the complete nucleotide sequence <SEQ ID 665>:

```
1    ATGACACGCT TCAAATATTC CCTGCTGTTT GCCGCCCTGT TGCCCGTGTA
51   CGCGCAGGCC GATGTTTCTG TTTCAGACGA CCCCAAACCG CAGGAAAGCA
101  CTGAATTGCC GACCATCACC GTTACCGCCG ACCGCACCGC GAGTTCCAAC
151  GACGGCTACA CTGTTTCCGG CACGCACACC CCGCTCGGGC TGCCCATGAC
201  CCTGCGCGAA ATCCCGCAGA GCGTCAGCGT CATCACATCG CAACAAATGC
251  GCGACCAAAA CATCAAAACG CTCGACCGCG CCCTGTTGCA GGCGACCGGC
301  ACCAGCCGCC AGATTTACGG CTCCGACCGC GCGGGCTACA ACTACCTGTT
351  CGCGCGCGGC AGCCGCATCG CCAACTACCA AATCAACGGC ATCCCCGTTG
401  CCGACGCGCT GGCCGATACG GGCAATGCCA ACACCGCCGC CTATGAGCGC
451  GTAGAAGTCG TGCGCGGCGT GGCGGGGCTG CTGGACGGCA CGGGCGAGCC
501  TTCCGCCACC GTCAATCTGG TGCGCAAACG CCTGACCCGC AAGCCATTGT
551  TTGAAGTCCG CGCCGAAGCG GGCAACCGCA AACATTTCGG GCTGGACGCG
601  GACGTATCGG GCAGCCTGAA CACCGAAGGC ACGCTGCGCG GCCGCCTGGT
651  TTCCACCTTC GGACGCGGCG ACTCGTGGCG GCGGCGCGAA CGCAGCCGCG
701  ATGCCGAACT CTACGGCATT TTGGAATACG ACATCGCACC GCAAACCCGC
751  GTCCACGCAG GCATGGACTA CCAGCAGGCG AAAGAAACCG CCGACGCGCC
801  GCTCAGCTAC GCCGTGTACG ACAGCCAAGG TTATGCCACC GCCTTCGGCC
851  CGAAAGACAA CCCCGCCACA AATTGGGCGA ACAGCCGCCA CCGTGCGCTC
901  AACCTGTTCG CCGGCATCGA ACACCGCTTC AACCAAGACT GGAAACTCAA
951  AGCCGAATAC GACTACACCC GCAGCCGCTT CCGCCAGCCC TACGGCGTAG
1001 CAGGCGTGCT TTCCATCGAC CACAACACCG CCGCCACCGA CCTGATTCCC
1051 GGTTATTGGC ACGCCGACCC GCGCACCCAC AGCGCCAGCG TGTCATTGAT
1101 CGGCAAATAC CGCCTGTTCG GCCGCGAACA CGATTTAATC GCGGGTATCA
1151 ACGGTTACAA ATACGCCAGC AACAAATACG GCGAACGCAG CATCATCCCC
1201 AACGCCATTC CCAACGCCTA CGAATTTTCC CGCACGGGTG CCTACCCGCA
1251 GCCTGCATCG TTTGCCCAAA CCATCCCGCA ATACGGCACC AGGCGGCAAA
1301 TCGGCGGCTA TCTCGCCACC CGTTTCCGCG CCGCCGACAA CCTTTCGCTG
1351 ATTTTGGGCG GACGATACAC CCGTTACCGC ACCGGCAGCT ACGACAGCCG
1401 CACACAAGGC ATGACCTATG TGTCCGCCAA CCGTTTCACC CCCTACACAG
1451 GCATCGTGTT CGACCTGACC GGCAACCTGT CTCTTTACGG CTCGTACAGC
1501 AGCCTGTTCG TCCCGCAATC GCAAAAAGAC GAACACGGCA GCTACCTGAA
1551 ACCCGTAACC GGCAACAATC TGGAAGCCGG CATCAAAGGC GAATGGCTTG
1601 AAGGCCGTCT GAACGCATCC GCCGCCGTGT ACCGCGCCCG TAAAAACAAC
1651 CTCGCCACCG CAGCAGGACG CGACCCGAGC GGCAACACCT ACTACCGCGC
1701 CGCCAACCAA GCCAAAACCC ACGGCTGGGA AATCGAAGTC GGCGGCCGCA
1751 TCACGCCCGA ATGGCAGATA CAGGCAGGTT ACAGCCAAAG CAAAACCCGC
1801 GACCAAGACG GCAGCCGCCT GAACCCCGAC AGCGTACCCG AACGCAGCTT
1851 CAAACTCTTC ACTGCCTACC ACTTTGCCCC CGAAGCCCCC AGCGGCTGGA
1901 CCATCGGCGC AGGCGTGCGC TGGCAGAGCG AAACCCACAC CGACCCTGCC
1951 ACGCTCCGCA TCCCCAACCC CGCCGCCAAA GCCCGCGCCG CCGACAACAG
2001 CCGCCAAAAA GCCTACGCCG TCGCCGACAT CATGGCGCGT TACCGCTTCA
2051 ATCCGCGCGC CGAACTGTCG CTGAACGTGG ACAATCTGTT CAACAAACAC
2101 TACCGCACCC AGCCCGACCG CCACAGCTAC GGCGCACTGC GGACAGTGAA
2151 CGCGGCGTTT ACCTATCGGT TTAAATAA
```

This corresponds to the amino acid sequence <SEQ ID 666; ORF23-1>:

```
1    MTRFKYSLLF AALLPVYAQA DVSVSDDPKP QESTELPTIT VTADRTASSN
51   DGYTVSGTHT PLGLPMTLRE IPQSVSVITS QQMRDQNIKT LDRALLQATG
101  TSRQIYGSDR AGYNYLFARG SRIANYQING IPVADALADT GNANTAAYER
151  VEVVRGVAGL LDGTGEPSAT VNLVRKRLTR KPLFEVRAEA GNRKHFGLDA
201  DVSGSLNTEG TLRGRLVSTF GRGDSWRRRE RSRDAELYGI LEYDIAPQTR
251  VHAGMDYQQA KETADAPLSY AVYDSQGYAT AFGPKDNPAT NWANSRHRAL
301  NLFAGIEHRF NQDWKLKAEY DYTRSRFRQP YGVAGVLSID HNTAATDLIP
351  GYWHADPRTH SASVSLIGKY RLFGREHDLI AGINGYKYAS NKYGERSIIP
401  NAIPNAYEFS RTGAYPQPAS FAQTIPQYGT RRQIGGYLAT RFRAADNLSL
451  ILGGRYTRYR TGSYDSRTQG MTYVSANRFT PYTGIVFDLT GNLSLYGSYS
501  SLFVPQSQKD EHGSYLKPVT GNNLEAGIKG EWLEGRLNAS AAVYRARKNN
551  LATAAGRDPS GNTYYRAANQ AKTHGWEIEV GGRITPEWQI QAGYSQSKTR
601  DQDGSRLNPD SVPERSFKLF TAYHFAPEAP SGWTIGAGVR WQSETHTDPA
651  TLRIPNPAAK ARAADNSRQK AYAVADIMAR YRFNPRAELS LNVDNLFNKH
701  YRTQPDRHSY GALRTVNAAF TYRFK*
```

Computer analysis of this amino acid sequence gave the following results:

Homology with the ferric-pseudobactin receptor PupB of *Pseudomonas putida* (accession number P38047)

ORF23 and PupB protein show 32% aa identity in 205aa overlap:

```
Orf23   6   FARGSRIANYQINGIPVADALADTGNANTAAYERVEVVRGVAGLLDGTGEPSATVNLVRK 65
            ++RG  I NY+++G+P +  L D  + + A ++RVE+VRG  GL+ G G PSAT+NL+RK
PupB   215  WSRGFAIQNYEVDGVPTSTRL-DNYSQSMAMFDRVEIVRGATGLISGMGNPSATINLIRK 273

Orf23   66  RLTRKPLFEVRAEAGNRKHFGLDADVSGSLNTEXXLRGRLVSTFXXXXXXXXXXXXXXAE 125
            R T +    +  EAGN   +G   DVSG L     +RGR V+ +
PupB   274  RPTAEAQASITGEAGNWDRYGTGFDVSGPLTETGNIRGRFVADYKTEKAWIDRYNQQSQL 333

Orf23  126  LYGILEYDIAPQTRVHAXMDYQQAKETADAPLSYAVYD--SQGYATAFGPKDNPATNWAN 183
            +YGI E+D++  T +      Y   +   D+PL   +     S G  T      N A +W+
PupB   334  MYGITEFDLSEDTLLTVGFSY--LRSDIDSPLRSGLPTRFSTGERTNLKRSLNAAPDWSY 391

Orf23  184  SHHRALNLFAGIEHRFNQDWKLKAE 208
            + H   + F  IE +    W  K E
PupB   392  NDHEQTSFFTSIEQQLGNGWSGKIE 416
```

Homology with a predicted ORF from *N.meningitidis* (strain A)

ORF23 shows 95.7% identity over a 211 aa overlap with an ORF (ORF23a) from strain A of *N. meningitidis:*

```
                                                 10        20        30
orf23.pep                              GYNYLFARGSRIANYQINGIPVADALADTG
                                       ||||||||||||||||||||||||||||||
orf23a      QMRDQNIKALDRALLQATGTSRQIYGSDRAGYNYLFARGSRIANYQINGIPVADALADTG
                   90       100       110       120       130       140

                    40        50        60        70        80        90
orf23.pep   NANTAAYERVEVVRGVAGLLDGTGEPSATVNLVRKRLTRKPLFEVRAEAGNRKHFGLDAD
            ||||||||||||||||||||||||||||||||||| ||||||||||||||||||| ||
orf23a      NANTAAYERVEVVRGVAGLLDGTGEPSATVNLVRKRPTRKPLFEVRAEAGNRKHFGLGAD
                  150       160       170       180       190       200

                   100       110       120       130       140       150
orf23.pep   VSGSLNTEXXLRGRLVSTFGRGDSWRRRERSRXAELYGILEYDIAPQTRVHAXMDYQQAK
            ||||||:| :||||||||||||||||:||||| |||||||||||||||||||| ||||||
orf23a      VSGSLNAEGTLRGRLVSTFGRGDSWRQRERSRDAELYGILEYDIAPQTRVHAGMDYQQAK
                  210       220       230       240       250       260

                   160       170       180       190       200       210
orf23.pep   ETADAPLSYAVYDSQGYATAFGPKDNPATNWANSHHRALNLFAGIEHRFNQDWKLKAEYD
            ||||||||||||||||||||||||||||||||||:|||||||||||||||||||||||||
orf23a      ETADAPLSYAVYDSQGYATAFGPKDNPATNWANSRHRALNLFAGIEHRFNQDWKLKAEYD
                  270       280       290       300       310       320

orf23.pep   Y
            |
orf23a      YTRSRFRQPYGVAGVLSIDHNTAATDLIPGYWHADPRTHSASVSLIGKYRLFGREHDLIA
                  330       340       350       360       370       380
```

The complete length ORF23a nucleotide sequence <SEQ ID 667> is:

```
   1 ATGACACGCT TCAAATATTC CCTGCTGTTT GCCGCCCTGT TGCCCGTGTA
  51 CGCGCAGGCC GATGTTTCTG TTTCAGACGA CCCAAAACCG CAGGAAAGCA
 101 CTGAATTGCC GACCATCACC GTTACCGCCG ACCGCACCGC GAGTTCCAAC
 151 GACGGCTACA CTGTTTCCGG CACGCACACC CCGCTCGGGC TGCCCATGAC
 201 CCTGCGCGAA ATCCCGCAGA GCGTCAGCGT CATCACATCG CAACAAATGC
 251 GCGACCAAAA CATCAAAGCG CTCGACCGCG CCCTGTTGCA GGCGACCGGC
 301 ACCAGCCGCC AGATTTACGG CTCCGACCGC GCGGGCTACA ACTACCTGTT
 351 CGCGCGCGGC AGCCGCATCG CCAACTACCA AATCAACGGC ATCCCCGTTG
 401 CCGACGCGCT GGCCGATACG GGCAATGCCA ACACCGCCGC CTATGAGCGC
 451 GTAGAAGTCG TGCGCGGCGT GGCGGGGCTG CTGGACGGCA CGGGCGAGCC
 501 TTCCGCCACC GTCAATCTGG TGCGCAAACG CCCGACCCGC AAGCCATTGT
 551 TTGAAGTCCG CGCCGAAGCG GGCAACCGCA AACATTTCGG GCTGGGCGCG
 601 GACGTATCGG GCAGCCTGAA TGCCGAAGGC ACGCTGCGCG GCCGCCTGGT
 651 TTCCACCTTC GGACGCGGCG ACTCGTGGCG GCAGCGCGAA CGCAGCCGCG
 701 ATGCCGAACT CTACGGCATT TTGGAATACG ACATCGCACC GCAAACCCGC
 751 GTCCACGCAG GCATGGACTA CCAGCAGGCG AAAGAAACCG CCGACGCGCC
 801 GCTCAGCTAC GCCGTGTACG ACAGCCAAGG TTATGCCACC GCCTTCGGCC
```

```
 851 CGAAAGACAA CCCCGCCACA AATTGGGCGA ACAGCCGCCA CCGTGCGCTC
 901 AACCTGTTCG CCGGCATCGA ACACCGCTTC AACCAAGACT GGAAACTCAA
 951 AGCCGAATAC GACTACACCC GCAGCCGCTT CCGCCAGCCC TACGGCGTAG
1001 CAGGCGTGCT TTCCATCGAC CACAACACCG CCGCCACCGA CCTGATTCCC
1051 GGTTATTGGC ACGCCGACCC GCGCACCCAC AGCGCCAGCG TGTCATTAAT
1101 CGGCAAATAC CGCCTGTTCG GCCGCGAACA CGATTTAATC GCGGGTATCA
1151 ACGGTTACAA ATACGCCAGC AACAAATACG GCGAACGCAG CATCATCCCC
1201 AACGCCATTC CCAACGCCTA CGAATTTTCC CGCACGGGTG CCTACCCGCA
1251 GCCTGCATCG TTTGCCCAAA CCATCCCGCA ATACGGCACC AGGCGGCAAA
1301 TCGGCGGCTA TCTCGCCACC CGTTTCCGCG CCGCCGACAA CCTTTCGCTG
1351 ATACTCGGCG GCAGATACAG CCGTTACCGC ACCGGCAGCT ACGACAGCCG
1401 CACACAAGGC ATGACCTATG TGTCCGCCAA CCGTTTCACC CCCTACACAG
1451 GCATCGTGTT CGACCTGACC GGCAACCTGT CGCTTTACGG CTCGTACAGC
1501 AGCCTGTTCG TCCCGCAATC GCAAAAAGAC GAACACGGCA GCTACCTGAA
1551 ACCCGTAACC GGCAACAATC TGGAAGCCGG CATCAAAGGC GAATGGCTTG
1601 AAGGCCGTCT GAACGCATCC GCCGCCGTGT ACCGCGCCCG TAAAAACAAC
1651 CTCGCCACCG CAGCAGGACG CGACCCGAGC GGCAACACCT ACTACCGCGC
1701 CGCCAACCAA GCCAAAACCC ACGGCTGGGA AATCGAAGTC GGCGGCCGCA
1751 TCACGCCCGA ATGGCAGATA CAGGCAGGTT ACAGCCAAAG CAAAACCCGC
1801 GACCAAGACG GCAGCCGCCT GAACCCCGAC AGCGTACCCG AACGCAGCTT
1851 CAAACTCTTC ACTGCCTACC ACTTTGCCCC CGAAGCCCCC AGCGGCTGGA
1901 CCATCGGCGC AGGCGTGCGC TGGCAGAGCG AAACCCACAC CGACCCTGCC
1951 ACGCTCCGCA TCCCCAACCC CGCCGCCAAA GCCCGCGCCG CCGACAACAG
2001 CCGCCAAAAA GCCTACGCCG TCGCCGACAT CATGGCGCGT TACCGCTTCA
2051 ATCCGCGCGC CGAACTGTCG CTGAACGTGG ACAATCTGTT CAACAAACAC
2101 TACCGCACCC AGCCCGACCG CCACAGCTAC GGCGCACTGC GGACAGTGAA
2151 CGCGGCGTTT ACCTATCGGT TTAAATAA
```

This encodes a protein having amino acid sequence <SEQ ID 668>:

```
  1 MTRFKYSLLF AALLPVYAQA DVSVSDDPKP QESTELPTIT VTADRTASSN
 51 DGYTVSGTHT PLGLPMTLRE IPQSVSVITS QQMRDQNIKA LDRALLQATG
101 TSRQIYGSDR AGYNYLFARG SRIANYQING IPVADALADT GNANTAAYER
151 VEVVRGVAGL LDGTGEPSAT VNLVRKRPTR KPLFEVRAEA GNRKHFGLGA
201 DVSGSLNAEG TLRGRLVSTF GRGDSWRQRE RSRDAELYGI LEYDIAPQTR
251 VHAGMDYQQA KETADAPLSY AVYDSQGYAT AFGPKDNPAT NWANSRHRAL
301 NLFAGIEHRF NQDWKLKAEY DYTRSRFRQP YGVAGVLSID HNTAATDLIP
351 GYWHADPRTH SASVSLIGKY RLFGREHDLI AGINGYKYAS NKYGERSIIP
401 NAIPNAYEFS RTGAYPQPAS FAQTIPQYGT RRQIGGYLAT RFRAADNLSL
451 ILGGRYSRYR TGSYDSRTQG MTYVSANRFT PYTGIVFDLT GNLSLYGSYS
501 SLFVPQSQKD EHGSYLKPVT GNNLEAGIKG EWLEGRLNAS AAVYRARKNN
551 LATAAGRDPS GNTYYRAANQ AKTHGWEIEV GGRITPEWQI QAGYSQSKTR
601 DQDGSRLNPD SVPERSFKLF TAYHFAPEAP SGWTIGAGVR WQSETHTDPA
651 TLRIPNPAAK ARAADNSRQK AYAVADIMAR YRFNPRAELS LNVDNLFNKH
701 YRTQPDRHSY GALRTVNAAF TYRFK*
```

ORF23a and ORF23-1 show 99.2% identity in 725 aa overlap:

```
                     10        20        30        40        50        60
orf23a.pep  MTRFKYSLLFAALLPVYAQADVSVSDDPKPQESTELPTITVTADRTASSNDGYTVSGTHT
            ||||||||||||||||||||||||||||||||||||||||||||||||||||||||||||
orf23-1     MTRFKYSLLFAALLPVYAQADVSVSDDPKPQESTELPTITVTADRTASSNDGYTVSGTHT
                     10        20        30        40        50        60

                     70        80        90       100       110       120
orf23a.pep  PLGLPMTLREIPQSVSVITSQQMRDQNIKALDRALLQATGTSRQIYGSDRAGYNYLFARG
            |||||||||||||||||||||||||||||:||||||||||||||||||||||||||||||
orf23-1     PLGLPMTLREIPQSVSVITSQQMRDQNIKTLDRALLQATGTSRQIYGSDRAGYNYLFARG
                     70        80        90       100       110       120

                    130       140       150       160       170       180
orf23a.pep  SRIANYQINGIPVADALADTGNANTAAYERVEVVRGVAGLLDGTGEPSATVNLVRKRPTR
            ||||||||||||||||||||||||||||||||||||||||||||||||||||||||| ||
orf23-1     SRIANYQINGIPVADALADTGNANTAAYERVEVVRGVAGLLDGTGEPSATVNLVRKRLTR
                    130       140       150       160       170       180

                    190       200       210       220       230       240
orf23a.pep  KPLFEVRAEAGNRKHFGLGADVSGSLNAEGTLRGRLVSTFGRGDSWRQRERSRDAELYGI
            |||||||||||||||||| ||||||||:|||||||||||||||||||:||||||||||||
orf23-1     KPLFEVRAEAGNRKHFGLDADVSGSLNTEGTLRGRLVSTFGRGDSWRRRERSRDAELYGI
                    190       200       210       220       230       240
```

```
                      250       260       270       280       290       300
orf23a.pep     LEYDIAPQTRVHAGMDYQQAKETADAPLSYAVYDSQGYATAFGPKDNPATNWANSRHRAL
               ||||||||||||||||||||||||||||||||||||||||||||||||||||||||||||
orf23-1        LEYDIAPQTRVHAGMDYQQAKETADAPLSYAVYDSQGYATAFGPKDNPATNWANSRHRAL
                      250       260       270       280       290       300

                      310       320       330       340       350       360
orf23a.pep     NLFAGIEHRFNQDWKLKAEYDYTRSRFRQPYGVAGVLSIDHNTAATDLIPGYWHADPRTH
               ||||||||||||||||||||||||||||||||||||||||||||||||||||||||||||
orf23-1        NLFAGIEHRFNQDWKLKAEYDYTRSRFRQPYGVAGVLSIDHNTAATDLIPGYWHADPRTH
                      310       320       330       340       350       360

                      370       380       390       400       410       420
orf23a.pep     SASVSLIGKYRLFGREHDLIAGINGYKYASNKYGERSIIPNAIPNAYEFSRTGAYPQPAS
               ||||||||||||||||||||||||||||||||||||||||||||||||||||||||||||
orf23-1        SASVSLIGKYRLFGREHDLIAGINGYKYASNKYGERSIIPNAIPNAYEFSRTGAYPQPAS
                      370       380       390       400       410       420

                      430       440       450       460       470       480
orf23a.pep     FAQTIPQYGTRRQIGGYLATRFRAADNLSLILGGRYSRYRTGSYDSRTQGMTYVSANRFT
               ||||||||||||||||||||||||||||||||||||:|||||||||||||||||||||||
orf23-1        FAQTIPQYGTRRQIGGYLATRFRAADNLSLILGGRYTRYRTGSYDSRTQGMTYVSANRFT
                      430       440       450       460       470       480

                      490       500       510       520       530       540
orf23a.pep     PYTGIVFDLTGNLSLYGSYSSLFVPQSQKDEHGSYLKPVTGNNLEAGIKGEWLEGRLNAS
               ||||||||||||||||||||||||||||||||||||||||||||||||||||||||||||
orf23-1        PYTGIVFDLTGNLSLYGSYSSLFVPQSQKDEHGSYLKPVTGNNLEAGIKGEWLEGRLNAS
                      490       500       510       520       530       540

                      550       560       570       580       590       600
orf23a.pep     AAVYRARKNNLATAAGRDPSGNTYYRAANQAKTHGWEIEVGGRITPEWQIQAGYSQSKTR
               ||||||||||||||||||||||||||||||||||||||||||||||||||||||||||||
orf23-1        AAVYRARKNNLATAAGRDPSGNTYYRAANQAKTHGWEIEVGGRITPEWQIQAGYSQSKTR
                      550       560       570       580       590       600

                      610       620       630       640       650       660
orf23a.pep     DQDGSRLNPDSVPERSFKLFTAYHFAPEAPSGWTIGAGVRWQSETHTDPATLRIPNPAAK
               ||||||||||||||||||||||||||||||||||||||||||||||||||||||||||||
orf23-1        DQDGSRLNPDSVPERSFKLFTAYHFAPEAPSGWTIGAGVRWQSETHTDPATLRIPNPAAK
                      610       620       630       640       650       660

                      670       680       690       700       710       720
orf23a.pep     ARAADNSRQKAYAVADIMARYRFNPRAELSLNVDNLFNKHYRTQPDRHSYGALRTVNAAF
               ||||||||||||||||||||||||||||||||||||||||||||||||||||||||||||
orf23-1        ARAADNSRQKAYAVADIMARYRFNPRAELSLNVDNLFNKHYRTQPDRHSYGALRTVNAAF
                      670       680       690       700       710       720

orf23a.pep     TYRFKX
               ||||||
orf23-1        TYRFKX
```

Homology with a predicted ORF from *N.gonorrhoeae*

ORF23 shows 93.4% identity over a 211aa overlap with a predicted ORF (ORF23.ng) from *N. gonorrhoeae:*

```
orf23.pep             GYNYLFARGSRIANYQINGIPVADALADTGNANTAAYERVEVVRGVAGLLD   51
                      |||||||||||||||||||||||||||||||||||||||||||||||| |
orf23ng     SAVDACRIPGYNYLFARGSRIANYQINGIPVADALADTGNANTAAYERVEVVRGVAGLPD   60

orf23.pep   GTGEPSATVNLVRKRLTRKPLFEVRAEAGNRKHFGLDADVSGSLNTEXXLRGRLVSTFGR  111
            ||||||||||||||: |||||||||||||||||||| ||||||||:| :|||||||||||
orf23ng     GTGEPSATVNLVRKHPTRKPLFEVRAEAGNRKHFGLGADVSGSLNAEGTLRGRLVSTFGR  120

orf23.pep   GDSWRRRERSRXAELYGILEYDIAPQTRVHAXMDYQQAKETADAPLSYAVYDSQGYATAF  171
            |||||: |||| ||||||||||||||||||| ||||||||||||||||||||||||||||
orf23ng     GDSWRQLERSRDAELYGILEYDIAPQTRVHAGMDYQQAKETADAPLSYAVYDSQGYATAF  180
```

```
orf23.pep   GPKDNPATNWANSHHRALNLFAGIEHRFNQDWKLKAEYDY                      211
            ||||||||||:||::|||||||||||||||||||||||||
orf23ng     GPKDNPATNWSNSRNRALNLFAGIEHRFNQDWKLKAEYDYTRSRFRQPYGVAGVLSIDHS  240
```

The ORF23ng nucleotide sequence <SEQ ID 669> is predicted to encode a protein comprising amino acid sequence <SEQ ID 670>:

```
  1  SAVDACRIPG YNYLFARGSR IANYQINGIP VADALADTGN ANTAAYERVE
 51  VVRGVAGLPD GTGEPSATVN LVRKHPTRKP LFEVRAEAGN RKHFGLGADV
101  SGSLNAEGTL RGRLVSTFGR GDSWRQLERS RDAELYGILE YDIAPQTRVH
151  AGMDYQQAKE TADAPLSYAV YDSQGYATAF GPKDNPATNW SNSRNRALNL
201  FAGIEHRFNQ DWKLKAEYDY TRSRFRQPYG VAGVLSIDHS TAATDLIPGY
251  WHADPRTHSA SMSLTGKYRL FGREHDLIAG INGYKYASNK YGERSIIPNA
301  IPNAYEFSRT GAYPQPSSFA QTIPQYDTRR QIGGYLATRF RAADNLSLIL
351  GGRYSRYRAG SYNSRTQGMT YVSANRFTPY TGIVFDLTGN LSLYGSYSSL
401  FVPQLQKDEH GSYLKPVTGN NLEADIKGEW LEGRLNASAA VYRARKNNLA
451  TAAGRDQSGN TYYRAANQAK THGWEIEVGG RITPEWQIQA GYSQSKPRDQ
501  DGSRLNPDSV PERSFKLFTA YHLAPEAPSG RTIGAGVRRQ GETHTDPAAL
551  RIPNPAAKAR AVANSRQKAY AVADIMARYR FNPRTELSLN VDNLFNKHYR
601  TQPDRHSYGA LRTVNAAFTY RFK*
```

Further work revealed the complete nucleotide sequence <SEQ ID 671>:

```
   1  ATGACACGCT TCAAATACTC CCTGCTTTTT GCCGCCCTGC TACCCGTGTA
  51  CGCGCAGGCC GATGTTTCTG TTTCAGACGA CCCCAAACCG CAGGAAAGCA
 101  CCGAATTGCC GACCATCACC GTTACCGCCG ACCGCACCGC GAGTTCCAAC
 151  GACGGCTACA CCGTTTCCGG CACGCACACC CCGTTCGGGC TGCCCATGAC
 201  CCTGCGCGAA ATCCCGCAGA GCGTCAGCGT CATCACATCG CAACAAATGC
 251  GCGACCAAAA CATCAAAACG CTCGACCGCG CCCTGTTGCA GGCGACCGGC
 301  ACCAGCCGCC AGATTTACGG CTCCGACCGC GCGGGCTACA ACTACCTGTT
 351  CGCGCGCGGC AGCCGCATCG CCAACTACCA AATCAACGGC ATCCCCGTTG
 401  CCGACGCGCT GGCCGATACG GGCAATGCCA ACACCGCCGC CTATGAGCGC
 451  GTAGAAGTCG TGCGCGGCGT GGCGGGGCTG CCGGACGGCA CGGGCGAGCC
 501  TTCTGCCACC GTCAATCTGG TACGCAAACA CCCGACCCGC AAGCCATTGT
 551  TTGAAGTCCG CGCCGAAGCC GGCAACCGCA AACATTTCGG GCTGGGCGCG
 601  GACGTATCGG GCAGCCTGAA CGCCGAAGGC ACGCTGCGCG GCCGCCTGGT
 651  TTCCACCTTC GGACGCGGCG ACTCGTGGCG GCAGCTCGAA CGCAGCCGCG
 701  ATGCCGAACT CTACGGCATT TTGGAATACG ACATCGCACC GCAAACCCGC
 751  GTCCACGCAG GCATGGACTA CCAGCAGGCG AAAGAAACCG CAGACGCGCC
 801  GCTCAGCTAC GCCGTGTACG ACAGCCAAGG TTATGCCACC GCCTTCGGCC
 851  CAAAAGACAA CCCCGCCACA AATTGGTCGA ACAGCCGCAA CCGTGCGCTC
 901  AACCTGTTCG CCGGCATAGA ACACCGCTTC AACCAAGACT GGAAACTCAA
 951  AGCCGAATAC GACTACACCC GTAGCCGCTT CCGCCAGCCC TACGGTGTGG
1001  CAGGCGTACT TTCCATCGAC CACAGCACTG CCGCCACCGA CCTGATTCCC
1051  GGTTATTGGC ACGCcgatcc GCGCACCCAC AGCGCCAGCA TGTCATTGAC
1101  CGGCAAATAC CgcctGTTCG GCCGCGAGCA CGATTTAATC GCGGGTATCA
1151  ACGGCTACAA ATACGCCAGC AACAAATACG GCGAACGCAG CATCATTCCC
1201  AACGCCATTC CCAACGCCTA CGAATTTTCC CGCACGGGCG CCTATCCGCA
1251  GCCATCATCG TTTGCCCAAA CCATCCCGCA ATACGACACC AGGCGGCAAA
1301  TCGGCGGCTA TCTCGCCACC CGTTTCCGCG CCGCCGACAA CCTTTCGCTG
1351  ATACTCGGCG GCAGATACAG CCGCTACCGC GCAGGCAGCT ACAACAGCCG
1401  CACACAAGGC ATGACCTATG TGTCCGCCAA CCGTTTCACC CCCTACACAG
1451  GCATCGTGTT CGATCTGACC GGCAACCTGT CGCTTTACGG CTCGTACAGC
1501  AGCCTGTTCG TCCCGCAATT GCAAAAAGAC GAACACGGCA GCTACCTGAA
1551  ACCCGTAACC GGCAACAATC TGGAAGCCGA CATCAAAGGC GAATGGCTTG
1601  AAGGGCGTCT GAACGCATCC GCCGCCGTGT ACCGCGCCCG TAAAAACAAC
1651  CTCGCCACCG CAGCAGGACG CGACCAGAGC GGCAACACCT ACTATCGCGC
1701  CGCCAACCAA GCCAAAACCC ACGGCTGGGA AATCGAAGTC GGCGGCCGCA
1751  TCACGCCCGA ATGGCAGATA CAGGCAGGCT ACAGCCAAAG CAAACCCCGC
1801  GACCAAGACG GCAGCCGCCT GAACCCCGAC AGCGTAcCCG AACGCAGCTT
1851  CAAACTCTTC ACCGCCTACC ACTTAGCCCC CGAAGCCCCC AGCGGCCGGA
1901  CCATcggTGC GGGTGTGCGC CGGCAGGGCG AAACCCACAC CGACCCAGCC
1951  GCGCTCCGCA TCCCCAACCC CGCCGCCAAA GCCCGCGCCG TCGCCAACAG
2001  CCGCCAGAAA GCCTACGCCG TCGCCGACAT CATGGCGCGT TACCGCTTCA
2051  ATCCGCGCAC CGAACTGTCG CTGAACGTGG ACAACCTGTT CAACAAACAC
2101  TACCGCACCC AGCCCGACCG CCACAGCTAC GGCGCACTGC GGACAGTGAA
2151  CGCGGCGTTT ACCTATCGGT TTAAATAA
```

This corresponds to the amino acid sequence <SEQ ID 672; ORF23ng-1>:

```
   1  MTRFKYSLLF AALLPVYAQA DVSVSDDPKP QESTELPTIT VTADRTASSN
  51  DGYTVSGTHT PFGLPMTLRE IPQSVSVITS QQMRDQNIKT LDRALLQATG
 101  TSRQIYGSDR AGYNYLFARG SRIANYQING IPVADALADT GNANTAAYER
 151  VEVVRGVAGL PDGTGEPSAT VNLVRKHPTR KPLFEVRAEA GNRKHFGLGA
 201  DVSGSLNAEG TLRGRLVSTF GRGDSWRQLE RSRDAELYGI LEYDIAPQTR
 251  VHAGMDYQQA KETADAPLSY AVYDSQGYAT AFGPKDNPAT NWSNSRNRAL
 301  NLFAGIEHRF NQDWKLKAEY DYTRSRFRQP YGVAGVLSID HSTAATDLIP
 351  GYWHADPRTH SASMSLTGKY RLFGREHDLI AGINGYKYAS NKYGERSIIP
 401  NAIPNAYEFS RTGAYPQPSS FAQTIPQYDT RRQIGGYLAT RFRAADNLSL
 451  ILGGRYSRYR AGSYNSRTQG MTYVSANRFT PYTGIVFDLT GNLSLYGSYS
 501  SLFVPQLQKD EHGSYLKPVT GNNLEADIKG EWLEGRLNAS AAVYRARKNN
 551  LATAAGRDQS GNTYYRAANQ AKTHGWEIEV GGRITPEWQI QAGYSQSKPR
 601  DQDGSRLNPD SVPERSFKLF TAYHLAPEAP SGRTIGAGVR RQGETHTDPA
 651  ALRIPNPAAK ARAVANSRQK AYAVADIMAR YRFNPRTELS LNVDNLFNKH
 701  YRTQPDRHSY GALRTVNAAF TYRFK*
```

ORF23ng-1 and ORF23-1 show 95.9% identity in 725 aa overlap:

```
                        10        20        30        40        50        60
orf23-1.pep    MTRFKYSLLFAALLPVYAQADVSVSDDPKPQESTELPTITVTADRTASSNDGYTVSGTHT
               ||||||||||||||||||||||||||||||||||||||||||||||||||||||||||||
orf23ng-1      MTRFKYSLLFAALLPVYAQADVSVSDDPKPQESTELPTITVTADRTASSNDGYTVSGTHT
                        10        20        30        40        50        60

                        70        80        90       100       110       120
orf23-1.pep    PLGLPMTLREIPQSVSVITSQQMRDQNIKTLDRALLQATGTSRQIYGSDRAGYNYLFARG
               |:||||||||||||||||||||||||||||||||||||||||||||||||||||||||||
orf23ng-1      PFGLPMTLREIPQSVSVITSQQMRDQNIKTLDRALLQATGTSRQIYGSDRAGYNYLFARG
                        70        80        90       100       110       120

                       130       140       150       160       170       180
orf23-1.pep    SRIANYQINGIPVADALADTGNANTAAYERVEVVRGVAGLLDGTGEPSATVNLVRKRLTR
               |||||||||||||||||||||||||||||||||||||||| |||||||||||||||: ||
orf23ng-1      SRIANYQINGIPVADALADTGNANTAAYERVEVVRGVAGLPDGTGEPSATVNLVRKHPTR
                       130       140       150       160       170       180

                       190       200       210       220       230       240
orf23-1.pep    KPLFEVRAEAGNRKHFGLDADVSGSLNTEGTLRGRLVSTFGRGDSWRRRERSRDAELYGI
               |||||||||||||||||| ||||||||:|||||||||||||||||||: |||||||||||
orf23ng-1      KPLFEVRAEAGNRKHFGLGADVSGSLNAEGTLRGRLVSTFGRGDSWRQLERSRDAELYGI
                       190       200       210       220       230       240

                       250       260       270       280       290       300
orf23-1.pep    LEYDIAPQTRVHAGMDYQQAKETADAPLSYAVYDSQGYATAFGPKDNPATNWANSRHRAL
               ||||||||||||||||||||||||||||||||||||||||||||||||||||:|||:|||
orf23ng-1      LEYDIAPQTRVHAGMDYQQAKETADAPLSYAVYDSQGYATAFGPKDNPATNWSNSRNRAL
                       250       260       270       280       290       300

                       310       320       330       340       350       360
orf23-1.pep    NLFAGIEHRFNQDWKLKAEYDYTRSRFRQPYGVAGVLSIDHNTAATDLIPGYWHADPRTH
               |||||||||||||||||||||||||||||||||||||||||:||||||||||||||||||
orf23ng-1      NLFAGIEHRFNQDWKLKAEYDYTRSRFRQPYGVAGVLSIDHSTAATDLIPGYWHADPRTH
                       310       320       330       340       350       360

                       370       380       390       400       410       420
orf23-1.pep    SASVSLIGKYRLFGREHDLIAGINGYKYASNKYGERSIIPNAIPNAYEFSRTGAYPQPAS
               |||:|| |||||||||||||||||||||||||||||||||||||||||||||||||||:|
orf23ng-1      SASMSLTGKYRLFGREHDLIAGINGYKYASNKYGERSIIPNAIPNAYEFSRTGAYPQPSS
                       370       380       390       400       410       420

                       430       440       450       460       470       480
orf23-1.pep    FAQTIPQYGTRRQIGGYLATRFRAADNLSLILGGRYTRYRTGSYDSRTQGMTYVSANRFT
               |||||||| |||||||||||||||||||||||||||:|||:|||:|||||||||||||||
orf23ng-1      FAQTIPQYDTRRQIGGYLATRFRAADNLSLILGGRYSRYRAGSYNSRTQGMTYVSANRFT
                       430       440       450       460       470       480

                       490       500       510       520       530       540
orf23-1.pep    PYTGIVFDLTGNLSLYGSYSSLFVPQSQKDEHGSYLKPVTGNNLEAGIKGEWLEGRLNAS
               |||||||||||||||||||||||||| |||||||||||||||||| ||||||||||||||
orf23ng-1      PYTGIVFDLTGNLSLYGSYSSLFVPQLQKDEHGSYLKPVTGNNLEADIKGEWLEGRLNAS
                       490       500       510       520       530       540
```

```
                       550       560       570       580       590       600
orf23-1.pep   AAVYRARKNNLATAAGRDPSGNTYYRAANQAKTHGWEIEVGGRITPEWQIQAGYSQSKTR
              ||||||||||||||||| |||||||||||||||||||||||||||||||||||||||| |
orf23ng-1     AAVYRARKNNLATAAGRDQSGNTYYRAANQAKTHGWEIEVGGRITPEWQIQAGYSQSKPR
                       550       560       570       580       590       600

                       610       620       630       640       650       660
orf23-1.pep   DQDGSRLNPDSVPERSFKLFTAYHFAPEAPSGWTIGAGVRWQSETHTDPATLRIPNPAAK
              ||||||||||||||||||||||||:||||||| ||||||| |:||||||:||||||||||
orf23ng-1     DQDGSRLNPDSVPERSFKLFTAYHLAPEAPSGRTIGAGVRRQGETHTDPAALRIPNPAAK
                       610       620       630       640       650       660

                       670       680       690       700       710       720
orf23-1.pep   ARAADNSRQKAYAVADIMARYRFNPRAELSLNVDNLFNKHYRTQPDRHSYGALRTVNAAF
              |||: ||||||||||||||||||||||:||||||||||||||||||||||||||||||||||
orf23ng-1     ARAVANSRQKAYAVADIMARYRFNPRTELSLNVDNLFNKHYRTQPDRHSYGALRTVNAAF
                       670       680       690       700       710       720

orf23-1.pep   TYRFKX
              ||||||
orf23ng-1     TYRFKX
```

In addition, ORF23ng-1 shows significant homology with an OMP from *E.coli:*

```
sp|P16869|FHUE_ECOLI OUTER-MEMBRANE RECEPTOR FOR FE(III)-COPROGEN, FE(III)-
FERRIOXAMINE B AND FE(III)-RHODOTRULIC ACID PRECURSOR >gi|1651542|gnl|PID|d1015403
(D90745) Outer membrane protein FhuE precursor [Escherichia coli]
>gi|1651545|gnl|PID|d1015405 (D90746) Outer membrane protein FhuE precursor
[Escherichia coli] >gi|1787344 (AE000210) outer-membrane receptor for Fe(III)-
coprogen, Fe(III)-ferrioxamine B and Fe(III)-rhodotrulic acid precursor
[Escherichia coli] Length = 729
 Score = 332 bits (843), Expect = 3e-90
 Identities = 228/717 (31%), Positives = 350/717 (48%), Gaps = 60/717 (8%)

Query: 38  TITVTADRTASSN--DGYTVSGTHTPFGLPMTLREIPQSVSVITSQQMRDQNIKTLDRAL 95
            T+ V    TA  +  + Y+V+ T     + MT R+IPQSV++++ Q+M DQ ++TL   +
Sbjct: 43  TVIVEGSATAPDDGENDYSVTSTSAGTKMQMTQRDIPQSVTIVSQQRMEDQQLQTLGEVM 102

Query: 96  LQATGTSRQIYGSDRAGYNYLFARGSRIANYQINGIP--------VADALADTGNANTAA 147
              G S+    SDRA Y   ++RG +I NY ++GIP         + DAL+D      A
Sbjct: 103 ENTLGISKSQADSDRALY---YSRGFQIDNYMVDGIPTYFESRWNLGDALSDM-----AL 154

Query: 148 YERVEVVRGVAGLPDGTGEPSATVNLVRKHPTRKPLF-EVRAEAGNRKHFGLGADVSGSL 206
            +ERVEVVRG  GL  GTG PSA +N+VRKH T +   +V AE G+      AD+   L
Sbjct: 155 FERVEVVRGATGLMTGTGNPSAAINMVRKHATSREFKGDVSAEYGSWNKERYVADLQSPL 214

Query: 207 NAEGTLRGRLVSTFGRGDSWRQLERSRDAELYGILEYDIAPQTRVHAGMDYQQAKETADA 266
              +G +R R+V  +   DSW    S     GI++ D+   T + AG +YQ+    +
Sbjct: 215 TEDGKIRARIVGGYQNNDSWLDRYNSEKTFFSGIVDADLGDLTTLSAGYEYQRIDVNSPT 274

Query: 267 PLSYAVYDSQGYATAFGPKDNPATNWSNSRNRALNLFAGIEHRFNQDWKLKAEYDYTRSR 326
                 +++ G + ++     + A +W+ +       +F  ++ +F   W+       ++
Sbjct: 275 WGGLPRWNTDGSSNSYDRARSTAPDWAYNDKEINKVFMTLKQQFADTWQATLNATHSEVE 334

Query: 327 F--RQPYGVAGVLSIDHSTAA--TDLIPGY-------WHADPRTHSA-SMSLTGKYRLFG 374
            F  + Y  A V   D    ++  PG+       W++  R   A  +   G Y LFG
Sbjct: 335 FDSKMMYVDAYVNKADGMLVGPYSNYGPGFDYVGGTGWNSGKRKVDALDLFADGSYELFG 394

Query: 375 REHDLIAGINGYKYASNKYGER--SIIPNAIPNAYEFSRTGAYPQPSSFAQTIPQYDTRR 432
            R+H+L+ G   Y   +N+Y     +I P+ I + Y F+  G +PQ     Q++ Q DT
Sbjct: 395 RQHNLMFG-GSYSKQNNRYFSSWANIFPDEIGSFYNFN--GNFPQTDWSPQSLAQDDTTH 451

Query: 433 QIGGYLATRFRAADNLSLILGGRYSRYRAGSYNSRTQGMTY-VSANRFTPYTGIVFDXXX 491
                Y ATR   AD L LILG RY+ +R   +        +TY +  N  TPY G+VFD
Sbjct: 452 MKSLYAATRVTLADPLHLILGARYTNWRVDT-------LTYSMEKNHTTPYAGLVFDIND 504

Query: 492 XXXXXXXXXXXFVPQLQKDEHGSYLKPVTGNNLEADIKGEWLEGRLNASAAVYRARKNNL 551
                       F PQ  +D  G YL P+TGNN E  +K +W+  RL  + A++R  ++N+
Sbjct: 505 NWSTYASYTSIFQPQNDRDSSGKYLAPITGNNYELGLKSDWMNSRLTTTLAIFRIEQDNV 564

Query: 552 ATAAGR---DQSGNTYYRAANQAKTHGWEIEVGGRITPEWQIQAGYSQSKPRDQDGSRLN 608
            A + G      +G T Y+A +    + G E E+ G IT  WQ+  G ++    D +G+ +N
Sbjct: 565 AQSTGTPIPGSNGETAYKAVDGTVSKGVEFELNGAITDNWQLTFGATRYIAEDNEGNAVN 624

Query: 609 PDSVPERSFKLFTAYHLAPEAPSGRTIGAGVRRQGETHTDPAALRIPNPAAKARAVANSR 668
            P ++P   K+FT+Y L P  P   T+G GV  Q   +TD        P      RA
Sbjct: 625 P-NLPRTTVKMFTSYRL-PVMPE-LTVGGGVNWQNRVYTDTV-----TPYGTFRA----E 672

Query: 669 QKAYAVADIMARYRFNPRTELSLNVDNLFNKHYRTQPDRH-SYGALRTVNAAFTYRF 724
            Q +YA+ D+  RY+       L  NV+NLF+K Y T   +    YG  R  +   TY+F
Sbjct: 673 QGSYALVDLFTRYQVTKNFSLQGNVNNLFDKTYDTNVEGSIVYGTPRNFSITGTYQF 729
```

Based on this analysis, it was predicted that these proteins from *N.meningitidis* and *N.gonorrhoeae,* and their epitopes, could be useful antigens for vaccines or diagnostics, or for raising antibodies. ORF23-1 (77.5kDa) was cloned in pET and pGex vectors and expressed in *E. coli,* as described above. The products of protein expression and purification were analyzed by SDS-PAGE. Figure 15A shows the results of affinity purification of the His-fusion protein, and Figure 15B shows the results of expression of the GST-fusion in *E.coli.* Purified His-fusion protein was used to immunise mice, whose sera were used for Western blot (Figure 15C) and for ELISA (positive result). These experiments confirm that ORF23-1 is a surface-exposed protein, and that it is a useful immunogen.

**Example 80**

The following partial DNA sequence was identified in *N.meningitidis* <SEQ ID 673>:

```
  1  ATGCGCACGG CAGTGGTTTT GCTGTTGATC ATGCCGATGG CGGCTTCGTC
 51  GGCAATGATG CCGGAAATGG TGTGCGCGGG CGTGTCGCCG GGAACGGCAA
101  TCATATCCAA GCCGACCGAA CAAACGGCGG TCATGGCTTC GAGTTTGTCC
151  AGCGTCAgcA CGCCTGCTTC GGCGgcGgCa ATCATACCTT CGTCTTCGGA
201  AACGGGGATA AACGcGCCAC TCAAACCCCC GACCGCGCTG GAAGCCATCA
251  TGCCGCCTTT TTTCACGGCA TCGTTCAGCA ATGCCAAAGC TGCTGTTGTG
301  CCGTGCGTAC CGCAGACGCT CAAGCCCATT TnTTCAAGAA TGCGTGCCAC
351  TnAGTCGCCG ACGGGG..
```

This corresponds to the amino acid sequence <SEQ ID 674; ORF24>:

```
  1  MRTAVVLLLI MPMAASSAMM PEMVCAGVSP GTAIISKPTE QTAVMASSLS
 51  SVSTPASAAA IIPSSSETGI NAPLKPPTAL EAIMPPFFTA SFSNAKAAVV
101  PCVPQTLKPI XSRMRATXSP TG..
```

Further work revealed the complete nucleotide sequence <SEQ ID 675>:

```
  1  ATGCGCACGG CAGTGGTTTT GCTGTTGATC ATGCCGATGG CGGCTTCGTC
 51  GGCAATGATG CCGGAAATGG TGTGCGCGGG CGTGTCGCCG GGAACGGCAA
101  TCATATCCAA GCCGACCGAA CAAACGGCGG TCATGGCTTC GAGTTTGTCC
151  AGCGTCAGCA CGCCTGCTTC GGCGGCGGCA ATCATACCTT CGTCTTCGGA
201  AACGGGGATA AACGCGCCAC TCAAACCCCC GACCGCGCTG GAAGCCATCA
251  TGCCGCCTTT TTTCACGGCA TCGTTCAGCA ATGCCAAAGC TGCTGTTGTG
301  CCGTGCGTAC CGCAGACGCT CAAGCCCATT TCTTCAAGAA TGCGTGCCAC
351  TGAGTCGCCG ACGGCGGGGG TCGGCGCCAG CGACAAGTCG AGAATACCAA
401  ACGGGATATT CAGCATTTTT GAGGCTTCGC GGCCGATGAG TTCGCCCACG
451  CGGGTAATTT TGAAAGCAGT TTTCTTCACT ACTTCCGCAA CTTCGGTCAA
501  TGTCGTTGCA TCTGAATTTT CCAACGCGGC TTTTACGACA CCTGGGCCGG
551  ATACGCCGAC ATTGATAACG GCATCCGCTT CGCCCGAACC ATGAAACGCG
601  CCCGCCATAA ACGGGTTGTC TTCCACCGCG TTGCAGAACA CGACAATTTT
651  AGCGCAGCCG AAACCTTCGG GCGTGATTTC CGCCGTGCGT TTGACGGTTT
701  CGCCCGCCAG CTTGACCGCA TCCATATTGA TACCGGCACG CGTACTGCCG
751  ATATTGATGG AGCTGCACAC AATATCGGTA GTCTTCATCG CTTCGGGAAT
801  GGAGCGGATT AACACCTCAT CCGAAGGCGA CATCCCTTTT TGCACCAACG
851  CGGAAAAACC GCCGATAAAA GACACACCGA TGGCTTTGGC AGCTTTATCC

901  AAAGTTTGCG CCACGCTGAC GTAA
```

This corresponds to the amino acid sequence <SEQ ID 676; ORF24-1>:

```
  1  MRTAVVLLLI MPMAASSAMM PEMVCAGVSP GTAIISKPTE QTAVMASSLS
 51  SVSTPASAAA IIPSSSETGI NAPLKPPTAL EAIMPPFFTA SFSNAKAAVV
101  PCVPQTLKPI SSRMRATESP TAGVGASDKS RIPNGIFSIF EASRPMSSPT
151  RVILKAVFFT TSATSVNVVA SEFSNAAFTT PGPDTPTLIT ASASPEP*NA
201  PAINGLSSTA LQNTTILAQP KPSGVISAVR LTVSPASLTA SILIPARVLP
251  ILMELHTISV VFIASGMERI NTSSEGDIPF CTNAEKPPIK DTPMALAALS
301  KVCATLT*
```

Computer analysis of this amino acid sequence gave the following results:

Homology with a predicted ORF from *N.meningitidis* (strain A)

ORF24 shows 96.4% identity over a 307 aa overlap with an ORF (ORF24a) from strain A of *N. meningitidis:*

```
                    10        20        30        40        50        60
orf24a.pep  MRTAVVLLLIMPMAASSAMMPEMVCAGVSPGTAIISXPTEQTAVIASSLSNVSTPASAAA
            |||||||||||||||||||||||||||||||||||| |||||||:|||||:|||||||||
orf24       MRTAVVLLLIMPMAASSAMMPEMVCAGVSPGTAIISKPTEQTAVMASSLSSVSTPASAAA
                    10        20        30        40        50        60

                    70        80        90       100       110       120
orf24a.pep  IIPSSSXTGINAPLKPPTALEAIMPPFFTASFSNAKAAVVPCVPQTLKPISSRMRATESP
            |||||| |||||||||||||||||||||||||||||||||||||||||||||||||||||
orf24       IIPSSSETGINAPLKPPTALEAIMPPFFTASFSNAKAAVVPCVPQTLKPISSRMRATESP
                    70        80        90       100       110       120

                   130       140       150       160       170       180
orf24a.pep  TAGVGASDKSRIPNGIFSIFEASRPMSSPTRVILKAVFFTTSATSVNVVASEFSNAAFTT
            ||||||||||||||||||||||||||||||||||||||||||||||||||||||||||||
orf24       TAGVGASDKSRIPNGIFSIFEASRPMSSPTRVILKAVFFTTSATSVNVVASEFSNAAFTT
                   130       140       150       160       170       180

                   190       200       210       220       230       240
orf24a.pep  PGPDTPTLITASASPEPXNAPAIXGLSSXALQNTTILAQPKPSSVISXVRLMVSPASLTA
            ||||||||||||||||||||||| ||||:|||||||||||||||:||| ||| ||||||||
orf24       PGPDTPTLITASASPEPXNAPAINGLSSTALQNTTILAQPKPSGVISAVRLTVSPASLTA
                   190       200       210       220       230       240

                   250       260       270       280       290       300
orf24a.pep  SILIPARVLPILMELHTISVVFIASGMERXNTSSEGDIPFCTSAEKPPIKDTPMALAALS
            ||||||||||||||||||||||||||||| |||||||||||:||||||||||||||||||
orf24       SILIPARVLPILMELHTISVVFIASGMERINTSSEGDIPFCTNAEKPPIKDTPMALAALS
                   250       260       270       280       290       300

orf24a.pep  KVCATLTX
            ||||||||
orf24       KVCATLTX
```

The complete length ORF24a nucleotide sequence <SEQ ID 677> is:

```
  1  ATGCGCACGG CAGTGGTTTT GCTGTTGATC ATGCCGATGG CGGCTTCGTC
 51  GGCAATGATG CCGGAAATGG TGTGCGCGGG TGTGTCGCCG GGAACGGCAA
101  TCATATCCAA NCCGACCGAA CAAACGGCGG TCATCGCTTC GAGTTTATCC
151  AACGTCAGCA CGCCTGCTTC GGCGGCGGCA ATCATACCTT CGTCTTCGGA
201  NACGGGGATA AACGCGCCAC TCAAACCGCC AACCGCGCTC GAAGCCATCA
251  TGCCGCCCTT TTTCACGGCA TCGTTCAGCA ATGCCAAAGC TGCTGTTGTG
301  CCGTGCGTAC CGCAGACGCT CAAACCCATT TCTTCAAGAA TGCGCGCCAC
351  CGAGTCGCCG ACGGCAGGGG TCGGTGCCAG CGACAAGTCG AGAATACCAA
401  ACGGGATATT CAGCATTTTT GAGGCTTCGC GGCCGATGAG TTCGCCCACG
451  CGGGTAATTT TGAAGGCGGT TTTCTTCACA ACTTCGGCAA CTTCGGTCAA
501  TGTCGTTGCA TCCGAATTTT CCAACGCGGC TTTTACGACA CCCGGGCCGG
551  ATACGCCGAC ATTAATCACA GCATCCGCTT CGCCTGAGCC GTGAAACGCG
601  CCCGCCATAN ACGGGTTGTC TTCCNCCGCG TTGCAGAACA CGACGATTTT
651  GGCGCAGCCG AAACCTTCTA GTGTGATTTC ANCCGTGCGT TTGATGGTTT
```

```
701  CGCCCGCCAG TCTGACCGCG TCCATATTGA TACCGGCGCG CGTACTGCCG
751  ATATTGATGG AGCTGCACAC GATATCAGTA GTCTTCATCG CTTCGGGAAT
801  GGAACGGATN AACACCTCGT CAGAAGGCGA CATACCTTTT TGCACCAGCG
851  CGGAAAAGCC GCCAATAAAA GACACGCCGA TGGCTTTGGC AGCCTTATCC
901  AAAGTTTGCG CCACGCTGAC GTAA
```

This encodes a protein having amino acid sequence <SEQ ID 678>:

```
  1  MRTAVVLLLI MPMAASSAMM PEMVCAGVSP GTAIISXPTE QTAVIASSLS
 51  NVSTPASAAA IIPSSSXTGI NAPLKPPTAL EAIMPPFFTA SFSNAKAAVV
101  PCVPQTLKPI SSRMRATESP TAGVGASDKS RIPNGIFSIF EASRPMSSPT
151  RVILKAVFFT TSATSVNVVA SEFSNAAFTT PGPDTPTLIT ASASPEP*NA
201  PAIXGLSSXA LQNTTILAQP KPSSVISXVR LMVSPASLTA SILIPARVLP
251  ILMELHTISV VFIASGMERX NTSSEGDIPF CTSAEKPPIK DTPMALAALS
301  KVCATLT*
```

It should be noted that this protein includes a stop codon at position 198.

ORF24a and ORF24-1 show 96.4% identity in 307 aa overlap:

```
                       10        20        30        40        50        60
orf24a.pep     MRTAVVLLLIMPMAASSAMMPEMVCAGVSPGTAIISXPTEQTAVIASSLSNVSTPASAAA
               |||||||||||||||||||||||||||||||||||| |||||||:|||||:||||||||
orf24-1        MRTAVVLLLIMPMAASSAMMPEMVCAGVSPGTAIISKPTEQTAVMASSLSSVSTPASAAA
                       10        20        30        40        50        60

                       70        80        90       100       110       120
orf24a.pep     IIPSSSXTGINAPLKPPTALEAIMPPFFTASFSNAKAAVVPCVPQTLKPISSRMRATESP
               |||||| |||||||||||||||||||||||||||||||||||||||||||||||||||||
orf24-1        IIPSSSETGINAPLKPPTALEAIMPPFFTASFSNAKAAVVPCVPQTLKPISSRMRATESP
                       70        80        90       100       110       120

                      130       140       150       160       170       180
orf24a.pep     TAGVGASDKSRIPNGIFSIFEASRPMSSPTRVILKAVFFTTSATSVNVVASEFSNAAFTT
               ||||||||||||||||||||||||||||||||||||||||||||||||||||||||||||
orf24-1        TAGVGASDKSRIPNGIFSIFEASRPMSSPTRVILKAVFFTTSATSVNVVASEFSNAAFTT
                      130       140       150       160       170       180

                      190       200       210       220       230       240
orf24a.pep     PGPDTPTLITASASPEPXNAPAIXGLSSXALQNTTILAQPKPSSVISXVRLMVSPASLTA
               ||||||||||||||||||||||| ||||:|||||||||||||||:||| ||| |||||||
orf24-1        PGPDTPTLITASASPEPXNAPAINGLSSTALQNTTILAQPKPSGVISAVRLTVSPASLTA
                      190       200       210       220       230       240

                      250       260       270       280       290       300
orf24a.pep     SILIPARVLPILMELHTISVVFIASGMERXNTSSEGDIPFCTSAEKPPIKDTPMALAALS
               ||||||||||||||||||||||||||||| |||||||||||:||||||||||||||||
orf24-1        SILIPARVLPILMELHTISVVFIASGMERINTSSEGDIPFCTNAEKPPIKDTPMALAALS
                      250       260       270       280       290       300

orf24a.pep     KVCATLTX
               ||||||||
orf24-1        KVCATLTX
```

Homology with a predicted ORF from *N.gonorrhoeae*

ORF24 shows 96.7% identity over a 121 aa overlap with a predicted ORF (ORF24ng) from *N.gonorrhoeae:*

```
orf24.pep     MRTAVVLLLIMPMAASSAMMPEMVCAGVSPGTAIISKPTEQTAVMASSLSSVSTPASAAA   60
              ||||||||||||||||||||||||||||||||||:||||||||||||||||:|||||||
orf24ng       MRTAVVLLLIMPMAASSAMMPEMVCAGVSPGTAIMSKPTEQTAVMASSLSSVNTPASAAA   60

orf24.pep     IIPSSSETGINAPLKPPTALEAIMPPFFTASFSNAKAAVVPCVPQTLKPIXSRMRATXSP  120
              |||||||||||||||||||||||||||||||||||||||||||||||||| |||||| ||
orf24ng       IIPSSSETGINAPLKPPTALEAIMPPFFTASFSNAKAAVVPCVPQTLKPISSRMRATESP  120

orf24.pep     TG                                                            122
              |:
orf24ng       TAGVGASDKSRMPNGIFSIFEASRPMSSPTRVILKAVFFTTSATSVRLTASEFSSAALTT  180
```

The complete length ORF24ng nucleotide sequence <SEQ ID 679> is:

```
  1  ATGCGCACGG CGGTGGTTTT GCTGTTGATC ATGCCGATGG CGGCTTCGTC
 51  GGCGATGATG CCGGAAATGG TGTGCGCGGG CGTGTCGCCG GGAACGGCAA
101  TCATGTCCAA ACCAACGGAG CAGACGGCGG TCATGGCTTC GAGTTTGTCC
151  AGCGTCAACA CGCCTGCCTC GGCGGCGGCA ATCATACCTT CGTCTTCGGA
201  AACGGGGATA AACGCGCCGC TCAAACCGCC GACCGCGCTG GAAGCCATCA
251  TGCCGCCCTT TTTCACGGCA TCGTTCAGCA ATGCCAAAGC TGCTGTTGTG
301  CCGTGCGTAC CGCAGACGCT CAAGCCCATT TCTTCAAGAA TGCGCGCCAC
351  CGAGTCGCCG ACGGCGGGGG TCGGTGCCAG CGACAAATCG AGAATGCCGA
401  ACGGGATATT CAGCATTTTT GAGGCTTCGC GACCGATGAG TTCGCCCACG
451  CGGGTGATTT TGAAAGCGGT TTTCTTCACG ACTTCGGCGA CCTCGGTCAG
501  GCTGACCGCG TCCGAATTTT CCAGCGCGGC TTTGACCACG CCTGGACCGG
551  ATACGCCGAC ATTAATCACA GCATCCGCTT CGCCCGAGCC GTGGAACGCA
601  CCCGCCATAA ACGGATTGTC TTCCACCGCG TTGCAGAACA CGACGATTTT
651  GGCGCAGCCG AAACCTTCGG GTGTGATTTC AGCCGTGCGT TTGATGGTTT
701  CGCCTGCCAG CTTGACCGCA TCCATATTGA TACCGGCACG CGTGCTGCCG
751  ATATTGATGG AGCTGCACAC GATATCGGTA GTTTTCATCG CTTCGGGAAC
801  GGAACGGATC AACACCTCAT CCGAAGGCGA CATACCTTTT TGCACCAGCG
851  CGGAAAAGCC GCCGATAAAG GACACGCCGA TGGCTTTGGC TGCCTTGTCC
901  AAAGTCTGCG CCACGCTGAC ATAA
```

This encodes a protein having amino acid sequence <SEQ ID 680>:

```
  1  MRTAVVLLLI MPMAASSAMM PEMVCAGVSP GTAIMSKPTE QTAVMASSLS
 51  SVNTPASAAA IIPSSSETGI NAPLKPPTAL EAIMPPFFTA SFSNAKAAVV
101  PCVPQTLKPI SSRMRATESP TAGVGASDKS RMPNGIFSIF EASRPMSSPT
151  RVILKAVFFT TSATSVRLTA SEFSSAALTT PGPDTPTLIT ASASPEPWNA
201  PAINGLSSTA LQNTTILAQP KPSGVISAVR LMVSPASLTA SILIPARVLP
251  ILMELHTISV VFIASGTERI NTSSEGDIPF CTSAEKPPIK DTPMALAALS
301  KVCATLT*
```

ORF24ng and ORF24-1 show 96.1% identity in 307 aa overlap:

```
                       10        20        30        40        50        60
orf24-1.pep   MRTAVVLLLIMPMAASSAMMPEMVCAGVSPGTAIISKPTEQTAVMASSLSSVSTPASAAA
              ||||||||||||||||||||||||||||||||||:||||||||||||||||:|||||||
orf24ng       MRTAVVLLLIMPMAASSAMMPEMVCAGVSPGTAIMSKPTEQTAVMASSLSSVNTPASAAA
                       10        20        30        40        50        60

                       70        80        90       100       110       120
orf24-1.pep   IIPSSSETGINAPLKPPTALEAIMPPFFTASFSNAKAAVVPCVPQTLKPISSRMRATESP
              ||||||||||||||||||||||||||||||||||||||||||||||||||||||||||||
orf24ng       IIPSSSETGINAPLKPPTALEAIMPPFFTASFSNAKAAVVPCVPQTLKPISSRMRATESP
                       70        80        90       100       110       120

                      130       140       150       160       170       180
orf24-1.pep   TAGVGASDKSRIPNGIFSIFEASRPMSSPTRVILKAVFFTTSATSVNVVASEFSNAAFTT
              |||||||||||:||||||||||||||||||||||||||||||||||| ::|||||:||:||
orf24ng       TAGVGASDKSRMPNGIFSIFEASRPMSSPTRVILKAVFFTTSATSVRLTASEFSSAALTT
                      130       140       150       160       170       180

                      190       200       210       220       230       240
orf24-1.pep   PGPDTPTLITASASPEPXNAPAINGLSSTALQNTTILAQPKPSGVISAVRLTVSPASLTA
              ||||||||||||||||| |||||||||||||||||||||||||||||||||| ||||||||
orf24ng       PGPDTPTLITASASPEPWNAPAINGLSSTALQNTTILAQPKPSGVISAVRLMVSPASLTA
                      190       200       210       220       230       240

                      250       260       270       280       290       300
orf24-1.pep   SILIPARVLPILMELHTISVVFIASGMERINTSSEGDIPFCTNAEKPPIKDTPMALAALS
              |||||||||||||||||||||||||| ||||||||||||||:||||||||||||||||||
orf24ng       SILIPARVLPILMELHTISVVFIASGTERINTSSEGDIPFCTSAEKPPIKDTPMALAALS
                      250       260       270       280       290       300

orf24-1.pep   KVCATLTX
              ||||||||

           orf24ng       KVCATLTX
```

Based on this analysis, including the presence of a putative leader sequence (first 18 aa - double-underlined) and putative transmembrane domains (single-underlined) in the gonococcal protein, it is predicted that the proteins from *N.meningitidis* and *N.gonorrhoeae,* and their epitopes, could be useful antigens for vaccines or diagnostics, or for raising antibodies.

**Example 81**

The following partial DNA sequence was identified in *N.meningitidis* <SEQ ID 681>:

```
  1  ..ACCGACGTGC AAAAAGAGTT GGTCGGCGAA CAACGCAAGT GGGCGCAGGA
 51    AAAAATCAGC AACTGCCGAC AAGCCGCCGC GCAGGCAGAC CGGCAGGAAT
101    ACGCCGAATA CCTCAAGCTG CAATGCGACA CGCGGATGAC GCGCGAACGG
151    ATACAGTATC TTCGCGGCTA TTCCATCGAT TAG
```

This corresponds to the amino acid sequence <SEQ ID 682; ORF25>:

```
  1  ..TDVQKELVGE QRKWAQEKIS NCRQAAAQAD RQEYAEYLKL QCDTRMTRER
 51    IQYLRGYSID *
```

Further work revealed the complete nucleotide sequence <SEQ ID 683>:

```
   1  ATGTATCGGA AACTCATTGC GCTGCCGTTT GCCCTGCTGC TTGCCGCTTG
  51  CGGCAGGGAA GAACCGCCCA AGGCATTGGA ATGCGCCAAC CCCGCCGTGT
 101  TGCAAGGCAT ACGCGGCAAT ATTCAGGAAA CGCTCACGCA GGAAGCGCGT
 151  TCTTTCGCGC GCGAAGACGG CAGGCAGTTT GTCGATGCCG ACAAAATTAT
 201  CGCCGCCGCC TACGGTTTGG CGTTTTCTTT GGAACACGCT TCGGAAACGC
 251  AGGAAGGCGG GCGCACGTTC TGTATCGCCG ATTTGAACAT TACCGTGCCG
 301  TCTGAAACGC TTGCCGATGC CAAGGCAAAC AGCCCCCTGT TGTACGGGGA
 351  AACTGCTTTG TCGGATATTG TGCGGCAGAA GACGGGCGGC AATGTCGAGT
 401  TTAAAGACGG CGTATTGACG GCAGCCGTCC GCTTCCTGCC CGTCAAAGAC
 451  GGTCAGACGG CATTTGTCGA CAACACGGTC GGTATGGCGG CGCAAACGCT
 501  GTCTGCCGCG CTGCTGCCTT ACGGCGTGAA GAGCATCGTG ATGATAGACG
 551  GCAAGGCGGT GAAAAAAGAA GACGCGGTCA GGATTTTGAG CGGAAAAGCC
 601  CGTGAAGAAG AACCGTCCAA ACCCACGCCC GAAGACATTT TGGAACACAA
 651  TGCCGCCGGC GGCGATGCGG GCGTACCCCA AGCCGCAGAA GGCGCGCCCG
 701  AACCGGAAAT CCTGCATCCT GACGACGGCG AGCGTGCCGA TACCGTTACC
 751  GTATCACGGG GCGAAGTGGA AGAGGCGCGC GTACAAAACC AGCGTGCGGA
 801  ATCCGAAATT ACCAAACTTT GGGGAGGACT CGATACCGAC GTGCAAAAAG
 851  AGTTGGTCGG CGAACAACGC AAGTGGGCGC AGGAAAAAAT CAGCAACTGC
 901  CGACAAGCCG CCGCGCAGGC AGACCGGCAG GAATACGCCG AATACCTCAA
 951  GCTGCAATGC GACACGCGGA TGACGCGCGA ACGGATACAG TATCTTCGCG
1001  GCTATTCCAT CGATTAG
```

This corresponds to the amino acid sequence <SEQ ID 684; ORF25-1>:

```
   1  MYRKLIALPF ALLLAACGRE EPPKALECAN PAVLQGIRGN IQETLTQEAR
  51  SFAREDGRQF VDADKIIAAA YGLAFSLEHA SETQEGGRTF CIADLNITVP
 101  SETLADAKAN SPLLYGETAL SDIVRQKTGG NVEFKDGVLT AAVRFLPVKD
 151  GQTAFVDNTV GMAAQTLSAA LLPYGVKSIV MIDGKAVKKE DAVRILSGKA
 201  REEEPSKPTP EDILEHNAAG GDAGVPQAAE GAPEPEILHP DDGERADTVT
 251  VSRGEVEEAR VQNQRAESEI TKLWGGLDTD VQKELVGEQR KWAQEKISNC
 301  RQAAAQADRQ EYAEYLKLQC DTRMTRERIQ YLRGYSID*
```

Computer analysis of this amino acid sequence gave the following results:

<u>Homology with a predicted ORF from *N.meningitidis* (strain A)</u>

ORF25 shows 98.3% identity over a 60aa overlap with an ORF (ORF25a) from strain A of *N. meningitidis:*

```
                                          10        20        30
orf25.pep                             TDVQKELVGEQRKWAQEKISNCRQAAAQAD
                                      |||||||||| |||||||||||||||||||
orf25a     VTVSRGEVEEARVQNQRAESEITKLWGGLDTDVQKELVGEXRKWAQEKISNCRQAAAQAD
          250       260       270       280       290       300

                   40        50        60
orf25.pep  RQEYAEYLKLQCDTRMTRERIQYLRGYSIDX
           ||||||||||||||||||||||||||||||
orf25a     RQEYAEYLKLQCDTRMTRERIQYLRGYSIDX
          310       320       330
```

The complete length ORF25a nucleotide sequence <SEQ ID 685> is:

```
   1 ATGTATCGGA AACTCATTGC GCTGCCGTTT GCCCTGCTGC TTGCCGCTTG
  51 CGGCAGGGAA GAACCGCCCA AGGCATTGGA ATGCGCCAAC CCCGCCGTGT
 101 TGCAANGCAT ACGCNGCAAT ATTCAGGAAA CGCTCACGCA GGAAGCGCGT
 151 TCTTTCGCGC GCGAAGACNG CANGCAGTTT GTCGATGCCG ACNAAATTAT
 201 CGCCGCCGCC TANGNTNNGN NGNTNTCTTT GGAACACGCT TCGGAAACGC
 251 AGGAAGGCGG GCGCACGTTC TGTNTCGCCG ATTTGAACAT TACCGTGCCG
 301 TCTGAAACGC TTGCCGATGC CAAGGCAAAC AGCCCCCTGC TGTACGGGGA
 351 AACCGCTTTG TCGGATATTG TGCGGCAGAA GACGGGCGGC AATGTCGAGT
 401 TTAAAGACGG CGTATTGACG GCAGCCGTCC GCTTCCTACC CGTCAAAGAC
 451 GGTCAGANGG CATTTGTCGA CAACACGGTC GGTATGGCGG CGCAAACGCT
 501 GTCTGCCGCG TTGCTGCCTT ACGGCGTGAA GAGCATCGTG ATGATAGACG
 551 GCAAGGCGGT AAAAAAAGAA GACGCGGTCA GGATTNTGAG CNGANAAGCC
 601 CGTGAANAAG AACCGTCCAA ANCCNNGCCC GAAGACATTT TGGAACATAA
 651 TGCCGCCGGA GGGGATGCAG ACGTACCCCA AGCCGGAGAA GACGCGCCCG
 701 AACCGGAAAT CCTGCATCCT GACGACGGCG AGCGTGCCGA TACCGTTACC
 751 GTATCACGGG GCGAAGTGGA AGAGGCGCGN GTACAAAACC AGCGTGCGGA
 801 ATCCGAAATT ACCAAACTTT GGGGAGGACT CGATACCGAC GTGCAAAAAG
 851 AGTTGGTCGG CGAANAACGC AAGTGGGCGC AGGAAAAAAT CAGCAACTGC
 901 CGACAAGCCG CCGCGCAGGC AGACCGGCAG GAATACGCCG AATACCTCAA
 951 GCTGCAATGC GACACGCGGA TGACGCGCGA ACGGATACAG TATCTTCGCG
1001 GCTATTCCAT CGATTAG
```

This encodes a protein having amino acid sequence <SEQ ID 686>:

```
   1 MYRKLIALPF ALLLAACGRE EPPKALECAN PAVLQXIRXN IQETLTQEAR
  51 SFAREDXXQF VDADXIIAAA XXXXXSLEHA SETQEGGRTF CXADLNITVP
 101 SETLADAKAN SPLLYGETAL SDIVRQKTGG NVEFKDGVLT AAVRFLPVKD
 151 GQXAFVDNTV GMAAQTLSAA LLPYGVKSIV MIDGKAVKKE DAVRIXSXXA
 201 REXEPSKXXP EDILEHNAAG GDADVPQAGE DAPEPEILHP DDGERADTVT
 251 VSRGEVEEAR VQNQRAESEI TKLWGGLDTD VQKELVGEXR KWAQEKISNC
 301 RQAAAQADRQ EYAEYLKLQC DTRMTRERIQ YLRGYSID*
```

ORF25a and ORF25-1 show 93.5% identity in 338 aa overlap:

```
                     10        20        30        40        50        60
orf25a.pep   MYRKLIALPFALLLAACGREEPPKALECANPAVLQXIRXNIQETLTQEARSFAREDXXQF
             ||||||||||||||||||||||||||||||||||| || ||||||||||||||||  ||
orf25-1      MYRKLIALPFALLLAACGREEPPKALECANPAVLQGIRGNIQETLTQEARSFAREDGRQF
                     10        20        30        40        50        60

                     70        80        90       100       110       120
orf25a.pep   VDADXIIAAAXXXXXSLEHASETQEGGRTFCXADLNITVPSETLADAKANSPLLYGETAL
             |||| |||||     |||||||||||||||| ||||||||||||||||||||||||||||
orf25-1      VDADKIIAAAYGLAFSLEHASETQEGGRTFCIADLNITVPSETLADAKANSPLLYGETAL
                     70        80        90       100       110       120

                    130       140       150       160       170       180
orf25a.pep   SDIVRQKTGGNVEFKDGVLTAAVRFLPVKDGQXAFVDNTVGMAAQTLSAALLPYGVKSIV
             ||||||||||||||||||||||||||||||||:|||||||||||||||||||||||||||
orf25-1      SDIVRQKTGGNVEFKDGVLTAAVRFLPVKDGQTAFVDNTVGMAAQTLSAALLPYGVKSIV
                    130       140       150       160       170       180

                    190       200       210       220       230       240
orf25a.pep   MIDGKAVKKEDAVRIXSXXAREXEPSKXXPEDILEHNAAGGDADVPQAGEDAPEPEILHP
             ||||||||||||||| |  ||| ||||  |||||||||||||| ||||:| |||||||||
orf25-1      MIDGKAVKKEDAVRILSGKAREEEPSKPTPEDILEHNAAGGDAGVPQAAEGAPEPEILHP
                    190       200       210       220       230       240
```

```
                      250       260       270       280       290       300
orf25a.pep    DDGERADTVTVSRGEVEEARVQNQRAESEITKLWGGLDTDVQKELVGEXRKWAQEKISNC
              |||||||||||||||||||||||||||||||||||||||||||||||| |||||||||||
orf25-1       DDGERADTVTVSRGEVEEARVQNQRAESEITKLWGGLDTDVQKELVGEQRKWAQEKISNC
                      250       260       270       280       290       300

                      310       320       330      339
orf25a.pep    RQAAAQADRQEYAEYLKLQCDTRMTRERIQYLRGYSIDX
              |||||||||||||||||||||||||||||||||||||||
orf25-1       RQAAAQADRQEYAEYLKLQCDTRMTRERIQYLRGYSIDX
                      310       320       330
```

Homology with a predicted ORF from *N.gonorrhoeae*

ORF25 shows 100% identity over a 60aa overlap with a predicted ORF (ORF25ng) from *N.gonorrhoeae:*

```
orf25.pep                                       TDVQKELVGEQRKWAQEKISNCRQAAAQAD   30
                                                ||||||||||||||||||||||||||||||
orf25ng     VTVSRGEVEEARVQNQRAESEITKLWGGLDTDVQKELVGEQRKWAQEKISNCRQAAAQAD  308

orf25.pep   RQEYAEYLKLQCDTRMTRERIQYLRGYSID   60
            ||||||||||||||||||||||||||||||
orf25ng     RQEYAEYLKLQCDTRMTRERIQYLRGYSID  338
```

The complete length ORF25ng nucleotide sequence <SEQ ID 687> is:

```
   1  ATGTATCGGA AACTCATTGC GCTGCCGTTT GCCCTGCTGC TTGCAGCGTG
  51  CGGCAGGGAA GAACCGCCCA AGGCGTTGGA ATGCGCCAAC CCCGCCGTGT
 101  TGCAGGACAT ACGCGGCAGT ATTCAGGAAA CGCTCACGCA GGAAGCGCGT
 151  TCTTTCGCGC GCGAAGACGG CAGGCAGTTT GTCGATGCCG ACAAAATTAT
 201  CGCCGCCGCC TACGGTTTGG CGTTTTCTTT GGAACACGCT TCGGAAACGC
 251  AGGAAGGCGG GCGCACGTTC TGTATCGCCG ATTTGAACAT TACCGTGCCG
 301  TCTGAAACGC TTGCCGATGC CGAGGCAAAC AGCCCCCTGC TGTATGGGGA
 351  AACGTCTTTG GCAGACATCG TGCAGCAGAA GACGGGCGGC AATGTCGAGT
 401  TTAAAGACGG CGTATTGACG GCAGCCGTCC GCTTCCTGCC CGCCAAAGAC
 451  GCTCGGACGG CATTTATCGA CAACACGGTC GGTATGGCGA CGCAAACGCT
 501  GTCTGCCGCG TTGCTGCCTT ACGGCGTGAA GAGCATCGTG ATGATAGACG
 551  GCAAGGCGGT GACAAAAGAA GACGCGGTCA GGGTTTTGAG CGGCAAAGCC
 601  CGTGAAGAAG AACCGTCCAA ACCCACCCCC GAAGACATTT TGGAACACAA
 651  TGCCGCCGGC GGCGATGCGG GCGTACCCCA AGCCGCAGAA GGCGCACCCG
 701  AACCCGAAAT CCTGCATCCC GACGACGTCG AGCGTGCCGA TACCGTTACC
 751  GTATCACGGG GCGAAGTGGA AGAGGCGCGC GTACAAAACC AACGTGCGGA
 801  ATCCGAAATT ACCAAACTTT GGGGAGGACT CGATACCGAC GTGCAAAAAG
 851  AGTTGGTCGG CGAACAGCGC AAGTGGGCGC AGGAAAAAAT CAGcaactgc
 901  cgACAAGCCG CCGCGCAGGC AGACCGGCAG GAATACGCCG AATACCTCAA
 951  GCTCCAATGC GACACGCGGA TGACGCGCGA ACggaTACAG TATCTTCGCG
1001  GCTATTCCAT CGATTAG
```

This encodes a protein having amino acid sequence <SEQ ID 688>:

```
  1  MYRKLIALPF ALLLAACGRE EPPKALECAN PAVLQDIRGS IQETLTQEAR
 51  SFAREDGRQF VDADKIIAAA YGLAFSLEHA SETQEGGRTF CIADLNITVP
101  SETLADAEAN SPLLYGETSL ADIVQQKTGG NVEFKDGVLT AAVRFLPAKD
151  ARTAFIDNTV GMATQTLSAA LLPYGVKSIV MIDGKAVTKE DAVRVLSGKA
201  REEEPSKPTP EDILEHNAAG GDAGVPQAAE GAPEPEILHP DDVERADTVT
251  VSRGEVEEAR VQNQRAESEI TKLWGGLDTD VQKELVGEQR KWAQEKISNC
301  RQAAAQADRQ EYAEYLKLQC DTRMTRERIQ YLRGYSID*
```

ORF25ng and ORF25-1 show 95.9% identity in 338 aa overlap:

```
                       10        20        30        40        50        60
orf25-1.pep  MYRKLIALPFALLLAACGREEPPKALECANPAVLQGIRGNIQETLTQEARSFAREDGRQF
             ||||||||||||||||||||||||||||||||||| |||:||||||||||||||||||||
orf25ng      MYRKLIALPFALLLAACGREEPPKALECANPAVLQDIRGSIQETLTQEARSFAREDGRQF
                       10        20        30        40        50        60

                       70        80        90       100       110       120
orf25-1.pep  VDADKIIAAAYGLAFSLEHASETQEGGRTFCIADLNITVPSETLADAKANSPLLYGETAL
             ||||||||||||||||||||||||||||||||||||||||||||||||:|||||||||||||:|
orf25ng      VDADKIIAAAYGLAFSLEHASETQEGGRTFCIADLNITVPSETLADAEANSPLLYGETSL
                       70        80        90       100       110       120

                      130       140       150       160       170       180
orf25-1.pep  SDIVRQKTGGNVEFKDGVLTAAVRFLPVKDGQTAFVDNTVGMAAQTLSAALLPYGVKSIV
             :|||:||||||||||||||||||||||:||::|||:|||||||:||||||||||||||||
orf25ng      ADIVQQKTGGNVEFKDGVLTAAVRFLPAKDARTAFIDNTVGMATQTLSAALLPYGVKSIV
                      130       140       150       160       170       180

                      190       200       210       220       230       240
orf25-1.pep  MIDGKAVKKEDAVRILSGKAREEEPSKPTPEDILEHNAAGGDAGVPQAAEGAPEPEILHP
             ||||||| ||||||:|||||||||||||||||||||||||||||||||||||||||||||
orf25ng      MIDGKAVTKEDAVRVLSGKAREEEPSKPTPEDILEHNAAGGDAGVPQAAEGAPEPEILHP
                      190       200       210       220       230       240

                      250       260       270       280       290       300
orf25-1.pep  DDGERADTVTVSRGEVEEARVQNQRAESEITKLWGGLDTDVQKELVGEQRKWAQEKISNC
             || |||||||||||||||||||||||||||||||||||||||||||||||||||||||||
orf25ng      DDVERADTVTVSRGEVEEARVQNQRAESEITKLWGGLDTDVQKELVGEQRKWAQEKISNC
                      250       260       270       280       290       300

                      310       320       330      339
orf25-1.pep  RQAAAQADRQEYAEYLKLQCDTRMTRERIQYLRGYSIDX
             |||||||||||||||||||||||||||||||||||||||
orf25ng      RQAAAQADRQEYAEYLKLQCDTRMTRERIQYLRGYSIDX
                      310       320       330
```

Based on this analysis, including the presence of a predicted prokaryotic membrane lipoprotein lipid attchment site (underlined) in the gonococcal protein, it was predicted that the proteins from *N.meningitidis* and *N.gonorrhoeae,* and their epitopes, could be useful antigens for vaccines or diagnostics, or for raising antibodies.

ORF25-1 (37kDa) was cloned in pET and pGex vectors and expressed in *E.coli,* as described above. The products of protein expression and purification were analyzed by SDS-PAGE. Figure 16A shows the results of affinity purification of the GST-fusion protein, and Figure 16B shows the results of expression of the His-fusion in *E.coli.* Purified His-fusion protein was used to immunise mice, whose sera were used for Western blot (Figure 16C), ELISA (positive result), and FACS analysis (Figure 16D). These experiments confirm that ORF25-1 is a surface-exposed protein, and that it is a useful immunogen.

Figure 16E shows plots of hydrophilicity, antigenic index, and AMPHI regions for ORF25-1.

**Example 82**

The following partial DNA sequence was identified in *N.meningitidis* <SEQ ID 689>

```
   1  ATGCAGCTGA TCGACTATTC ACATTCATTT TTCTCGGTTG TGCCACCCTT
  51  TTTGGCACTG GCACTTGCCG TCATTACCCG CCGCGTACTG CTGTCTTTAG
 101  GCATCGGTAT TCTGGwysGC GTTGCCTTTT TGGTCGGCGG CAACCCCGTC
 151  GACGGTCTGA CACACCTGAA AGACATGGTC GTCGGCTTGG CTTGGTCAGA
 201  CGsyGATTGG TCGCTGGGCA AACCAAAAAT CTTGGTTTTC CkGATACTTT
 251  TGGGTATTTT TACTTCCCTG CTGACCTACT CCGGCAGCAA T.........
                                    //
 851  .......... .......... .......... ........AC TTCGCTGGTA
 901  TTCGGCGGCA CTTGCGGCGT CTTTGCCGTC GTTCTCTGCA CGCTCGGCAC
 951  GATTAAAACC GCCGACTATC CCAAAGCCGT TTGGCAGGGT GCGAAATCTA
```

```
1001  TGTTCGGCGC AATCGCCATT TTAATCCTCG CTTGGCTCAT CAGTACGGTT
1051  GTCGGCGAAA TGCACACCGG CGATTACCTC TCCACACTGG TTGCGGGCAA
1101  CATCCATCCC GGCTTCCTGC CCGTCATCCT CTTCCTGCTC GCCAGCGTGA
1151  TGGCGTTTGC CACAGGCACA AGCTGGGGGA CGTTCGGCAT TATGCTGCCG
1201  ATTGCCGCCG CCATGGCGGT CAAAGTCGAA CCCGCGCTGA TTATCCCGTG
1251  TATGTCCGCA GTAATGGCGG GGGCGGTATG CGGCGACCAC TGCTCGCCCA
1301  TTTCCGACAC GACCATCCTG TCGTCCACCG GCGCGCGCTG CAACCACATC
1351  GACCACGTTA CCTCGCAACT GCCTTACGCC TTAACCGTTG CCGCCGCCGC
1401  CGCATCGGGC TACCTCGCAT TGGGTCTGAC AAAATCCGCG CTGTTGGGCT
1451  TTGGCACGAC AGGCATTGTA TTGGCGGTGC TGATTTTTCT GTTGAAAGAT
1501  AAAAAA..
```

This corresponds to the amino acid sequence <SEQ ID 690; ORF26>:

```
  1  MQLIDYSHSF FSVVPPFLAL ALAVITRRVL LSLGIGILXX VAFLVGGNPV
 51  DGLTHLKDMV VGLAWSDXDW SLGKPKILVF XILLGIFTSL LTYSGSN...
                                    //
251  .......... .......... .......... .......... ......TSLV
301  FGGTCGVFAV VLCTLGTIKT ADYPKAVWQG AKSMFGAIAI LILAWLISTV
351  VGEMHTGDYL STLVAGNIHP GFLPVILFLL ASVMAFATGT SWGTFGIMLP
401  IAAAMAVKVE PALIIPCMSA VMAGAVCGDH CSPISDTTIL SSTGARCNHI
451  DHVTSQLPYA LTVAAAAASG YLALGLTKSA LLGFGTTGIV LAVLIFLLKD
501  KK..
```

Further work revealed the complete nucleotide sequence <SEQ ID 691>:

```
   1 ATGCAGCTGA TCGACTATTC ACATTCATTT TTCTCGGTTG TGCCACCCTT
  51 TTTGGCACTG GCACTTGCCG TCATTACCCG CCGCGTACTG CTGTCTTTAG
 101 GCATCGGTAT TCTGGTCGGC GTTGCCTTTT TGGTCGGCGG CAACCCCGTC
 151 GACGGTCTGA CACACCTGAA AGACATGGTC GTCGGCTTGG CTTGGTCAGA
 201 CGGCGATTGG TCGCTGGGCA AACCAAAAAT CTTGGTTTTC CTGATACTTT
 251 TGGGTATTTT TACTTCCCTG CTGACCTACT CCGGCAGCAA TCAGGCGTTT
 301 GCCGACTGGG CAAAACGGCA CATTAAAAAC CGGCGCGGCG CGAAAATGCT
 351 GACCGCCTGC CTCGTGTTCG TAACCTTTAT CGACGACTAT TTCCACAGTC
 401 TCGCCGTCGG TGCGATTGCC CGCCCCGTTA CCGACAAGTT TAAAGTTTCC
 451 CGCACCAAAC TCGCCTACAT CCTCGACTCC ACTGCCGCTC CTATGTGCGT
 501 GCTGATGCCC GTTTCAAGCT GGGGCGCGTC GATTATCGCC ACGCTTGCCG
 551 GACTGCTCGT TACCTACAAA ATCACCGAAT ACACGCCGAT GGGGACGTTT
 601 GTCGCCATGA GCCTGATGAA CTATTACGCA CTGTTTGCCC TGATTATGGT
 651 GTTCGTCGTC GCATGGTTTT CCTTCGACAT CGGCTCGATG GCACGTTTCG
 701 AACAAGCCGC GTTGAACGAA GCCCACGATG AAACTGCCGT TTCAGACGCT
 751 ACCAAAGGTC GTGTTTACGC ACTGATTATT CCCGTTTTGG CCTTAATCGC
 801 CTCAACGGTT TCCGCCATGA TCTACACCGG CGCGCAGGCA AGCGAAACCT
 851 TCAGCATTTT GGGGGCATTT GAAAACACGG ACGTAAACAC TTCGCTGGTA
 901 TTCGGCGGCA CTTGCGGCGT CCTTGCCGTC GTTCTCTGCA CGCTCGGCAC
 951 GATTAAAACC GCCGACTATC CCAAAGCCGT TTGGCAGGGT GCGAAATCTA
1001 TGTTCGGCGC AATCGCCATT TTAATCCTCG CTTGGCTCAT CAGTACGGTT
1051 GTCGGCGAAA TGCACACCGG CGATTACCTC TCCACACTGG TTGCGGGCAA
1101 CATCCATCCC GGCTTCCTGC CCGTCATCCT CTTCCTGCTC GCCAGCGTGA
1151 TGGCGTTTGC CACAGGCACA AGCTGGGGGA CGTTCGGCAT TATGCTGCCG
1201 ATTGCCGCCG CCATGGCGGT CAAAGTCGAA CCCGCGCTGA TTATCCCGTG
1251 TATGTCCGCA GTAATGGCGG GGGCGGTATG CGGCGACCAC TGCTCGCCCA
1301 TTTCCGACAC GACCATCCTG TCGTCCACCG GCGCGCGCTG CAACCACATC
1351 GACCACGTTA CCTCGCAACT GCCTTACGCC TTAACCGTTG CCGCCGCCGC
1401 CGCATCGGGC TACCTCGCAT TGGGTCTGAC AAAATCCGCG CTGTTGGGCT
1451 TTGGCACGAC AGGCATTGTA TTGGCGGTGC TGATTTTTCT GTTGAAAGAT
1501 AAAAAACGCG CCAACGCCTG A
```

This corresponds to the amino acid sequence <SEQ ID 692; ORF26-1>:

```
  1 MQLIDYSHSF FSVVPPFLAL ALAVITRRVL LSLGIGILVG VAFLVGGNPV
 51 DGLTHLKDMV VGLAWSDGDW SLGKPKILVF LILLGIFTSL LTYSGSNQAF
101 ADWAKRHIKN RRGAKMLTAC LVFVTFIDDY FHSLAVGAIA RPVTDKFKVS
151 RTKLAYILDS TAAPMCVLMP VSSWGASIIA TLAGLLVTYK ITEYTPMGTF
201 VAMSLMNYYA LFALIMVFVV AWFSFDIGSM ARFEQAALNE AHDETAVSDA
251 TKGRVYALII PVLALIASTV SAMIYTGAQA SETFSILGAF ENTDVNTSLV
301 FGGTCGVLAV VLCTLGTIKT ADYPKAVWQG AKSMFGAIAI LILAWLISTV
351 VGEMHTGDYL STLVAGNIHP GFLPVILFLL ASVMAFATGT SWGTFGIMLP
401 IAAAMAVKVE PALIIPCMSA VMAGAVCGDH CSPISDTTIL SSTGARCNHI
451 DHVTSQLPYA LTVAAAAASG YLALGLTKSA LLGFGTTGIV LAVLIFLLKD
501 KKRANA*
```

Computer analysis of this amino acid sequence gave the following results:

Homology with the hypothetical transmembrane protein HI1586 of *H.influenzae* (accession number P44263)

ORF26 and HI1586 show 53% and 49% amino acid identity in 97 and 221 aa overlap at the N-terminus and C-terminus, respectively:

```
Orf26   1   MQLIDYSHSFFSVVPPFLALALAVITRRVXXXXXXXXXXXVAFLVGGNPVDGLTHLKDMV 60
            M+LID+S S +S+VP  LA+ LA+ TRRV              L         +L  V
HI1586 14   MELIDFSSSVWSIVPALLAIILAIATRRVLVSLSAGIIIGSLMLSDWQIGSAFNYLVKNV 73

Orf26  61   VGLAWSDXDWSLGKPKILVFXILLGIFTSLLTYSGSN 97
            V L ++D + +     I++F +LLG+ T+LLT SGSN
HI1586 74   VSLVYADGEIN-SNMNIVLFLLLLGVLTALLTVSGSN 109

                                        //

Orf26  86   IFTSLLTYSGS--NTSLVFGGTCGVFAVVLCTL--GTIKTADYPKAVWQGAKSMFGXXXX 141
            +F+ L T+  +   TSLV GG C +     L  +     +   +Y ++   G KSM G
HI1586 299  VFSVLGTFENTVVGTSLVVGGFCSIIISTLLIILDRQVSVPEYVRSWIVGIKSMSGAIAI 358

Orf26  142  XXXXXXXSTVVGEMHTGDYLSTLVAGNIHPGFLPVILFLLASVMAFATGTSWGTFGIMLP 201
                   + +VG+M TG YLS+LV+GNI   FLPVILF+L + MAF+TGTSWGTFGIMLP
HI1586 359  LFFAWTINKIVGDMQTGKYLSSLVSGNIPMQFLPVILFVLGAAMAFSTGTSWGTFGIMLP 418

Orf26  202  IAAAMAVKVEPALIIPCMSAVMAGAVCGDHCSPISDTTILSSTGARCNHIDHVTSQXXXX 261
            IAAAMA     P L++PC+SAVMAGAVCGDHCSP+SDTTILSSTGA+CNHIDHVT+Q
HI1586 419  IAAAMAANAAPELLLPCLSAVMAGAVCGDHCSPVSDTTILSSTGAKCNHIDHVTTQLPYA 478

Orf26  262  XXXXXXXXXXXXXXXXXKSALLGFGTTGIVLAVLIFLLKDK 302
                             S L GF  T + L V+IF +K +
HI1586 479  ATVATATSIGYIVVGFTYSGLAGFAATAVSLIVIIFAVKKR 519
```

Homology with a predicted ORF from *N.meningitidis* (strain A)

ORF26 shows 58.2% identity over a 502aa overlap with an ORF (ORF26a) from strain A of *N. meningitidis:*

```
                      10        20        30        40        50        60
orf26.pep      MQLIDYSHSFFSVVPPFLALALAVITRRVLLSLGIGILXXVAFLVGGNPVDGLTHLKDMV
               ||||||||||||||||||||||||||||||||||||||  ||||||||||||||||||||
orf26a         MQLIDYSHSFFSVVPPFLALALAVITRRVLLSLGIGILVGVAFLVGGNPVDGLTHLKDMV
                      10        20        30        40        50        60

                      70        80        90       99
orf26.pep      VGLAWSDXDWSLGKPKILVFXILLGIFTSLLTYSGSNXX---------------------
               ||||||| |||||||| ||| |||||||||||||||||
orf26a         VGLAWSDGDWSLGKPKXLVFLILLGIFTSLLTYSGSNQAFADWAKRHIKNRRGAKMLTAC
                      70        80        90       100       110       120

orf26.pep      ------------------------------------------------------------

orf26a         LVFVTFIDDYFHSLAVGAXARPVTDKFKVSRAKLAYILDSTAAPMCVLMPVSSWGASIIA
                     130       140       150       160       170       180

orf26.pep      ------------------------------------------------------------

orf26a         TLAGLLVTYKITEYTPMGTFVAMSLMNYYALFALIMVFVVAWFSFDIGSMARFEQAALNE
                     190       200       210       220       230       240

                                                            100       110
orf26.pep      --------------------------------------------------------TSLV
                                                                       ||||
orf26a         AHDETAVSDGSWGRVYALIIPVLALIASTVSAMIYTGAQASETFSILGAFENTDVNTSLV
                     250       260       270       280       290       300
```

```
              120       130       140       150       160       170
orf26.pep     FGGTCGVFAVVLCTLGTIKTADYPKAVWQGAKSMFGAIAILILAWLISTVVGEMHTGDYL
              ||||||:||||||||||| |||||||||||||||||||||||||||||||||||||||||
orf26a        FGGTCGVLAVVLCTLGTIKIADYPKAVWQGAKSMFGAIAILILAWLISTVVGEMHTGDYL
                     310       320       330       340       350       360

              180       190       200       210       220       230
orf26.pep     STLVAGNIHPGFLPVILFLLASVMAFATGTSWGTFGIMLPIAAAMAVKVEPALIIPCMSA
              |||||||||||||| |||||||||||||||||||||||||||||||||||:|:|||||||
orf26a        STLVAGNIHPGFLXVILFLLASVMAFATGTSWGTFGIMLPIAAAMAVKVDPSLIIPCMSA
                     370       380       390       400       410       420

              240       250       260       270       280       290
orf26.pep     VMAGAVCGDHCSPISDTTILSSTGARCNHIDHVTSQLPYALTVAAAAASGYLALGLTKSA
              ||||||||||||||||||||||||||||||||||||||||||||||||||||||||||||
orf26a        VMAGAVCGDHCSPISDTTILSSTGARCNHIDHVTSQLPYALTVAAAAASGYLALGLTKSA
                     430       440       450       460       470       480

              300       310
orf26.pep     LLGFGTTGIVLAVLIFLLKDKK
              |||||:||||||||||||||||
orf26a        LLGFGXTGIVLAVLIFLLKDKKRANAX
                     490       500
```

The complete length ORF26a nucleotide sequence <SEQ ID 693> is:

```
   1  ATGCAGCTGA TCGACTATTC ACATTCATTT TTCTCGGTTG TGCCACCCTT
  51  TTTGGCACTG GCACTTGCCG TCATTACCCG CCGCGTACTG CTGTCTTTAG
 101  GCATCGGTAT TCTGGTCGGC GTTGCCTTTT TGGTCGGCGG CAACCCCGTC
 151  GACGGTCTGA CACACCTGAA AGACATGGTC GTCGGCTTGG CTTGGTCAGA
 201  CGGCGATTGG TCGCTGGGCA AACCAAAANT CTTGGTTTTC CTGATACTTT
 251  TGGGTATTTT TACTTCCCTG CTGACCTACT CCGGCAGCAA TCAGGCGTTT
 301  GCCGACTGGG CAAAACGGCA CATTAAAAAC CGGCGCGGCG CGAAAATGCT
 351  GACCGCCTGC CTCGTGTTCG TAACCTTTAT CGACGACTAT TTCCACAGTC
 401  TCGCCGTCGG TGCGNTTGCC CGCCCCGTTA CCGACAAGTT TAAAGTTTCC
 451  CGCGCCAAAC TCGCCTACAT CCTCGACTCC ACTGCCGCGC CTATGTGCGT
 501  GCTGATGCCC GTTTCAAGCT GGGGCGCGTC GATTATCGCC ACGCTTGCCG
 551  GACTGCTCGT TACCTACAAA ATCACCGAAT ACACGCCGAT GGGGACGTTT
 601  GTCGCCATGA GCCTGATGAA CTATTACGCA CTGTTTGCCC TGATTATGGT
 651  GTTCGTCGTC GCATGGTTCT CCTTCGACAT CGGCTCGATG GCACGTTTCG
 701  AACAAGCCGC GTTGAACGAA GCCCACGATG AAACTGCCGT TTCAGACGGC
 751  AGCTGGGGCA GGGTTTACGC ATTGATTATT CCCGTTTTGG CCTTAATCGC
 801  CTCAACGGTT TCCGCCATGA TCTACACCGG TGCACAGGCA AGCGAAACCT
 851  TCAGCATTTT GGGTGCATTT GAAAATACGG ACGTGAACAC TTCGCTGGTA
 901  TTCGGCGGCA CTTGCGGCGT GCTTGCCGTC GTCCTCTGCA CGCTCGGCAC
 951  GATTAAAATC GCCGATTATC CCAAAGCCGT TTGGCAGGGT GCGAAATCCA
1001  TGTTCGGCGC AATCGCCATT TTAATCCTTG CCTGGCTCAT CAGTACGGTT
1051  GTCGGCGAAA TGCACACAGG CGACTACCTC TCCACGCTGG TTGCGGGCAA
1101  CATCCATCCC GGCTTCCTGN CCGTCATCCT TTTCCTGCTC GCCAGCGTGA
1151  TGGCGTTTGC CACAGGCACA AGCTGGGGGA CGTTCGGCAT CATGCTGCCG
1201  ATTGCCGCCG CCATGGCGGT CAAAGTCGAT CCCTCACTGA TTATCCCGTG
1251  TATGTCCGCC GTGATGGCGG GGGCGGTATG CGGCGACCAC TGCTCGCCCA
1301  TTTCCGACAC GACCATCCTG TCGTCCACCG GCGCGCGCTG CAACCACATC
1351  GACCACGTTA CNTCGCAACT GCCTTACGCC TTAACCGTTG CCGCCGCCGC
1401  CGCATCGGGN TACCTCGCAT TGGGTCTGAC AAAATCCGCG CTGTTGGGTT
1451  TTGGCANGAC AGGCATTGTA TTGGCGGTGC TGATTTTTCT GTTGAAAGAT
1501  AAAAAACGCG CCAACGCCTG A
```

This encodes a protein having amino acid sequence <SEQ ID 694>:

```
  1 MQLIDYSHSF FSVVPPFLAL ALAVITRRVL LSLGIGILVG VAFLVGGNPV
 51 DGLTHLKDMV VGLAWSDGDW SLGKPKXLVF LILLGIFTSL LTYSGSNQAF
101 ADWAKRHIKN RRGAKMLTAC LVFVTFIDDY FHSLAVGAXA RPVTDKFKVS
151 RAKLAYILDS TAAPMCVLMP VSSWGASIIA TLAGLLVTYK ITEYTPMGTF
201 VAMSLMNYYA LFALIMVFVV AWFSFDIGSM ARFEQAALNE AHDETAVSDG
251 SWGRVYALII PVLALIASTV SAMIYTGAQA SETFSILGAF ENTDVNTSLV
301 FGGTCGVLAV VLCTLGTIKI ADYPKAVWQG AKSMFGAIAI LILAWLISTV
351 VGEMHTGDYL STLVAGNIHP GFLXVILFLL ASVMAFATGT SWGTFGIMLP
401 IAAAMAVKVD PSLIIPCMSA VMAGAVCGDH CSPISDTTIL SSTGARCNHI
451 DHVTSQLPYA LTVAAAAASG YLALGLTKSA LLGFGXTGIV LAVLIFLLKD
501 KKRANA*
```

ORF26a and ORF26-1 show 97.8% identity in 506 aa overlap:

```
                       10        20        30        40        50        60
orf26a.pep    MQLIDYSHSFFSVVPPFLALALAVITRRVLLSLGIGILVGVAFLVGGNPVDGLTHLKDMV
              ||||||||||||||||||||||||||||||||||||||||||||||||||||||||||||
orf26-1       MQLIDYSHSFFSVVPPFLALALAVITRRVLLSLGIGILVGVAFLVGGNPVDGLTHLKDMV
                       10        20        30        40        50        60

                       70        80        90       100       110       120
orf26a.pep    VGLAWSDGDWSLGKPKXLVFLILLGIFTSLLTYSGSNQAFADWAKRHIKNRRGAKMLTAC
              |||||||||||||||| |||||||||||||||||||||||||||||||||||||||||||
orf26-1       VGLAWSDGDWSLGKPKILVFLILLGIFTSLLTYSGSNQAFADWAKRHIKNRRGAKMLTAC
                       70        80        90       100       110       120

                      130       140       150       160       170       180
orf26a.pep    LVFVTFIDDYFHSLAVGAXARPVTDKFKVSRAKLAYILDSTAAPMCVLMPVSSWGASIIA
              |||||||||||||||||| |||||||||||:|||||||||||||||||||||||||||||
orf26-1       LVFVTFIDDYFHSLAVGAIARPVTDKFKVSRTKLAYILDSTAAPMCVLMPVSSWGASIIA
                      130       140       150       160       170       180

                      190       200       210       220       230       240
orf26a.pep    TLAGLLVTYKITEYTPMGTFVAMSLMNYYALFALIMVFVVAWFSFDIGSMARFEQAALNE
              ||||||||||||||||||||||||||||||||||||||||||||||||||||||||||||
orf26-1       TLAGLLVTYKITEYTPMGTFVAMSLMNYYALFALIMVFVVAWFSFDIGSMARFEQAALNE
                      190       200       210       220       230       240

                      250       260       270       280       290       300
orf26a.pep    AHDETAVSDGSWGRVYALIIPVLALIASTVSAMIYTGAQASETFSILGAFENTDVNTSLV
              |||||||||:: ||||||||||||||||||||||||||||||||||||||||||||||||
orf26-1       AHDETAVSDATKGRVYALIIPVLALIASTVSAMIYTGAQASETFSILGAFENTDVNTSLV
                      250       260       270       280       290       300

                      310       320       330       340       350       360
orf26a.pep    FGGTCGVLAVVLCTLGTIKIADYPKAVWQGAKSMFGAIAILILAWLISTVVGEMHTGDYL
              |||||||||||||||||| |||||||||||||||||||||||||||||||||||||||||
orf26-1       FGGTCGVLAVVLCTLGTIKTADYPKAVWQGAKSMFGAIAILILAWLISTVVGEMHTGDYL
                      310       320       330       340       350       360

                      370       380       390       400       410       420
orf26a.pep    STLVAGNIHPGFLXVILFLLASVMAFATGTSWGTFGIMLPIAAAMAVKVDPSLIIPCMSA
              ||||||||||||| ||||||||||||||||||||||||||||||||||||:|:|||||||
orf26-1       STLVAGNIHPGFLPVILFLLASVMAFATGTSWGTFGIMLPIAAAMAVKVEPALIIPCMSA
                      370       380       390       400       410       420

                      430       440       450       460       470       480
orf26a.pep    VMAGAVCGDHCSPISDTTILSSTGARCNHIDHVTSQLPYALTVAAAAASGYLALGLTKSA
              ||||||||||||||||||||||||||||||||||||||||||||||||||||||||||||
orf26-1       VMAGAVCGDHCSPISDTTILSSTGARCNHIDHVTSQLPYALTVAAAAASGYLALGLTKSA
                      430       440       450       460       470       480

                      490       500
orf26a.pep    LLGFGXTGIVLAVLIFLLKDKKRANAX
              |||||:|||||||||||||||||||||
orf26-1       LLGFGTTGIVLAVLIFLLKDKKRANAX
                      490       500
```

Homology with a predicted ORF from *N.gonorrhoeae*

ORF26 shows 94.8% and 99% identity in 97 and 206 aa overlap at the N-terminus and C-terminus, respectively, with a predicted ORF (ORF26ng) from *N. gonorrhoeae:*

```
orf26.pep   MQLIDYSHSFFSVVPPFLALALAVITRRVLLSLGIGILXXVAFLVGGNPVDGLTHLKDMV   60
            ||||||||||||||||||||||||||||||||||||||  ||||||||||||||||||||
orf26ng     MQLIDYSHSFFSVVPPFLALALAVITRRVLLSLGIGILVGVAFLVGGNPVDGLTHLKDMV   60

orf26.pep   VGLAWSDXDWSLGKPKILVFXILLGIFTSLLTYSGSN                          97
            |||||:| |||||||||||| ||||||||||||||||
orf26ng     VGLAWADGDWSLGKPKILVFLILLGIFTSLLTYSGSNQAFADWAKRHIKNRCGAKMLTAC  120
```

```
orf26.pep                                TSLVFGGTCGVFAVVLCTLGTIKTADYPKA   326
                                         ||||||||||:||||||:||||||||||||
orf26ng     ASTVSAMIYTGAQASETFSILGAFENTDVNTSLVFGGTCGVLAVVLCTFGTIKTADYPKA   326

orf26.pep   VWQGAKSMFGAIAILILAWLISTVVGEMHTGDYLSTLVAGNIHPGFLPVILFLLASVMAF   386
            ||||||||||||||||||||||||||||||||||||||||||||||||||||||||||||
orf26ng     VWQGAKSMFGAIAILILAWLISTVVGEMHTGDYLSTLVAGNIHPGFLPVILFLLASVMAF   386

orf26.pep   ATGTSWGTFGIMLPIAAAMAVKVEPALIIPCMSAVMAGAVCGDHCSPISDTTILSSTGAR   446
            ||||||||||||||||||||||||||||||||||||||||||||||||||||||||||||
orf26ng     ATGTSWGTFGIMLPIAAAMAVKVEPALIIPCMSAVMAGAVCGDHCSPISDTTILSSTGAR   446

orf26.pep   CNHIDHVTSQLPYALTVAAAAASGYLALGLTKSALLGFGTTGIVLAVLIFLLKDKK       502
            ||||||||||||||||||||||||||||||||||||||||||||||||||||||||
orf26ng     CNHIDHVTSQLPYALTVAAAAASGYLALGLTKSALLGFGTTGIVLAVLIFLLKDKKRADV   506
```

The complete length ORF26ng nucleotide sequence <SEQ ID 695> is:

```
   1  ATGCAGCTGA TTGACTATTC ACATTCATTT TTCTCGGTTG TGCCACCCTT
  51  TTTGGCACTG GCACTTGCCG TCATTACCCG CCGCGTACTG CTGTCTTTAG
 101  GCATCGGTAT TTTGGTCGGC GTTGCCTTTT TGGTCGGCGG CAACCCCGTC
 151  GACGGTCTGA CACACCTGAA AGACATGGTC GTCGGCTTGG CTTGGGCAGA
 201  CGGCGATTGG TCGCTGGGCA AACCAAAAAT CTTGGTTTTC CTGATACTTT
 251  TGGGCATTTT CACTTCACTG CTGACCTACT CCGGCAGCAA TCAGGCGTTT
 301  GCCGACTGGG CAAAACGGCA CATTAAAAAC CGGTGCGGCG CGAAAATGCT
 351  GACCGCCTGC CTCGTGTTCG TAACCTTTAT CGACGACTAT TTCCACAGCC
 401  TCGCCGTCGG TGCGATTGCC CGCCCCGTTA CCGACAAGTT TAAAGTTTCC
 451  CGCGCCAAAC TCGCCTACAT CCTCGACTCC ACTGCCTCGC CCATGTGCGT
 501  GCTGATGCCC GTTTCAAGCT GGGGCGCGTC GATTATCGCC ACGCTTGCCG
 551  GATTGCTCGT TACCTACAAA ATTACCGAAT ACACGCCGAT GGGGACGTTT
 601  GTCGCCATGA GCCTGATGAA CTATTACGCG CTGTTTGCCC TGATTATGGT
 651  ATTCGTCGTC GCATGGTTCT CCTTCGACAT CGGCTCGAtg gCGCGTTTCG
 701  AACAGGCTGC GTTGAACGAA gcccaggacg aaaccgccgc tTCAGACgCT
 751  ACCAAAGGTC GTGTTTACGC ATTGATTATT CCCGTTTTGG CCTTAATCGC
 801  CTCAACGGTT TCCGCCATGA TCTACACCGG CGCGCAGGCA AGCGAAACCT
 851  TCAGCATTTT GGGGGCATTT GAAAATACCG ACGTAAACAC TTCGCTGGTA
 901  TTCGGCGGCA CTTGCGGCGT GCTTGCCGTC GTCCTCTGCA CGTTCGGCAC
 951  GATTAAAACC GCCGATTATC CCAAAGCCGT GTGGCAGGGT GCGAAATCCA
1001  TGTTCGGCGC AATCGCCATT TTAATCCTCG CCTGGCTCAT CAGTACGGTT
1051  GTCGGCGAAA TGCACACGGG CGACTACCTC TCCACGCTGG TTGCGGGCAA
1101  CATCCATCCC GGCTTCCTGC CCGTCATCCT CTTCCTGCTC GCCAGCGTGA
1151  TGGCGTTTGC CACAGGCACA AGCTGGGGGA CGTTCGGCAT TATGCTGCCG
1201  ATTGCCGCCG CCATGGCGGT CAAAGTCGAA CCCGCGCTGA TTAtcccGTG
1251  TATGTCCGCA GTAATGGCGG GGGCGGTATG CGGCGACCAC TGTTCGCCCA
1301  TCTCCGACAC GACCATCCTG TCGTCCACCG GCGCGCGCTG CAACCACATC
1351  GACCACGTTA CCTCGCAACT GCCTTATGCC CTGACGGTTG CCGCCGCCGC
1401  CGCATCGGGC TACCTCGCAT TGGGTCTGAC AAAATCCGCG CTGTTGGGCT
1451  TTGGCACGAC CGGTATTGTA TTGGCGGTGC TGATTTTTCT GTTGAAAGAT
1501  AAAAAACGCG CCGACGTTTG A
```

This encodes a protein having amino acid sequence <SEQ ID 696>:

```
  1 MQLIDYSHSF FSVVPPFLAL ALAVITRRVL LSLGIGILVG VAFLVGGNPV
 51 DGLTHLKDMV VGLAWADGDW SLGKPKILVF LILLGIFTSL LTYSGSNQAF
101 ADWAKRHIKN RCGAKMLTAC LVFVTFIDDY FHSLAVGAIA RPVTDKFKVS
151 RAKLAYILDS TASPMCVLMP VSSWGASIIA TLAGLLVTYK ITEYTPMGTF
201 VAMSLMNYYA LFALIMVFVV AWFSFDIGSM ARFEQAALNE AQDETAASDA
251 TKGRVYALII PVLALIASTV SAMIYTGAQA SETFSILGAF ENTDVNTSLV
301 FGGTCGVLAV VLCTFGTIKT ADYPKAVWQG AKSMFGAIAI LILAWLISTV
351 VGEMHTGDYL STLVAGNIHP GFLPVILFLL ASVMAFATGT SWGTFGIMLP
401 IAAAMAVKVE PALIIPCMSA VMAGAVCGDH CSPISDTTIL SSTGARCNHI
451 DHVTSQLPYA LTVAAAAASG YLALGLTKSA LLGFGTTGIV LAVLIFLLKD
501 KKRADV*
```

ORF26ng and ORF26-1 show 98.4% identity in 505 aa overlap:

```
                      10        20        30        40        50        60
orf26-1.pep  MQLIDYSHSFFSVVPPFLALALAVITRRVLLSLGIGILVGVAFLVGGNPVDGLTHLKDMV
             ||||||||||||||||||||||||||||||||||||||||||||||||||||||||||||
```

```
orf26ng      MQLIDYSHSFFSVVPPFLALALAVITRRVLLSLGIGILVGVAFLVGGNPVDGLTHLKDMV
                     10        20        30        40        50        60

                     70        80        90       100       110       120
orf26-1.pep  VGLAWSDGDWSLGKPKILVFLILLGIFTSLLTYSGSNQAFADWAKRHIKNRRGAKMLTAC
             |||||:||||||||||||||||||||||||||||||||||||||||||||| ||||||||
orf26ng      VGLAWADGDWSLGKPKILVFLILLGIFTSLLTYSGSNQAFADWAKRHIKNRCGAKMLTAC
                     70        80        90       100       110       120

                    130       140       150       160       170       180
orf26-1.pep  LVFVTFIDDYFHSLAVGAIARPVTDKFKVSRTKLAYILDSTAAPMCVLMPVSSWGASIIA
             |||||||||||||||||||||||||||||||:|||||||||:||||||||||||||||||
orf26ng      LVFVTFIDDYFHSLAVGAIARPVTDKFKVSRAKLAYILDSTASPMCVLMPVSSWGASIIA
                    130       140       150       160       170       180

                    190       200       210       220       230       240
orf26-1.pep  TLAGLLVTYKITEYTPMGTFVAMSLMNYYALFALIMVFVVAWFSFDIGSMARFEQAALNE
             ||||||||||||||||||||||||||||||||||||||||||||||||||||||||||||
orf26ng      TLAGLLVTYKITEYTPMGTFVAMSLMNYYALFALIMVFVVAWFSFDIGSMARFEQAALNE
                    190       200       210       220       230       240

                    250       260       270       280       290       300
orf26-1.pep  AHDETAVSDATKGRVYALIIPVLALIASTVSAMIYTGAQASETFSILGAFENTDVNTSLV
             |:||||:|||||||||||||||||||||||||||||||||||||||||||||||||||||
orf26ng      AQDETAASDATKGRVYALIIPVLALIASTVSAMIYTGAQASETFSILGAFENTDVNTSLV
                    250       260       270       280       290       300

                    310       320       330       340       350       360
orf26-1.pep  FGGTCGVLAVVLCTLGTIKTADYPKAVWQGAKSMFGAIAILILAWLISTVVGEMHTGDYL
             ||||||||||||||:|||||||||||||||||||||||||||||||||||||||||||||
orf26ng      FGGTCGVLAVVLCTFGTIKTADYPKAVWQGAKSMFGAIAILILAWLISTVVGEMHTGDYL
                    310       320       330       340       350       360

                    370       380       390       400       410       420
orf26-1.pep  STLVAGNIHPGFLPVILFLLASVMAFATGTSWGTFGIMLPIAAAMAVKVEPALIIPCMSA
             ||||||||||||||||||||||||||||||||||||||||||||||||||||||||||||
orf26ng      STLVAGNIHPGFLPVILFLLASVMAFATGTSWGTFGIMLPIAAAMAVKVEPALIIPCMSA
                    370       380       390       400       410       420

                    430       440       450       460       470       480
orf26-1.pep  VMAGAVCGDHCSPISDTTILSSTGARCNHIDHVTSQLPYALTVAAAAASGYLALGLTKSA
             ||||||||||||||||||||||||||||||||||||||||||||||||||||||||||||
orf26ng      VMAGAVCGDHCSPISDTTILSSTGARCNHIDHVTSQLPYALTVAAAAASGYLALGLTKSA
                    430       440       450       460       470       480

                    490       500
orf26-1.pep  LLGFGTTGIVLAVLIFLLKDKKRANAX
             ||||||||||||||||||||||||::
orf26ng      LLGFGTTGIVLAVLIFLLKDKKRADVX
                    490       500
```

In addition, ORF26 ng shows significant homology to a hypothetical *H. influenzae* protein:

```
sp|P44263|YF86_HAEIN   HYPOTHETICAL   PROTEIN   HI1586   >gi|1074850|pir||C64037
hypothetical
protein HI1586 - Haemophilus influenzae (strain Rd KW20) >gi|1574427 (U32832) H.
influenzae predicted coding region HI1586 [Haemophilus influenzae] Length = 519
 Score =  538 bits (1370), Expect = e-152
 Identities = 280/507 (55%), Positives = 346/507 (68%), Gaps = 7/507 (1%)

Query: 1   MQLIDYSHSFFSVVPPFLALALAVITRRXXXXXXXXXXXXXAFLVGGNPVDGLTHLKDMV 60
            M+LID+S S +S+VP  LA+ LA+ TRR               L          +L   V
Sbjct: 14  MELIDFSSSVWSIVPALLAIILAIATRRVLVSLSAGIIIGSLMLSDWQIGSAFNYLVKNV 73

Query: 61  VGLAWADGDWSLGKPKILVFLILLGIFTSLLTYSGSNQAFADWAKRHIKNRCGAKMLTAC 120
            V L +ADG+ +     I++FL+LLG+ T+LLT SGSN+AFA+WA+  IK R GAK+L A
Sbjct: 74  VSLVYADGEIN-SNMNIVLFLLLLGVLTALLTVSGSNRAFAEWAQSRIKGRRGAKLLAAS 132

Query: 121 LVFVTFIDDYFHSLAVGAIARPVTDKFKVSRAKLAYILDSTASPMCVLMPVSSWGASIIA 180
            LVFVTFIDDYFHSLAVGAIARPVTD+FKVSRAKLAYILDSTA+PMCV+MPVSSWGA II
Sbjct: 133 LVFVTFIDDYFHSLAVGAIARPVTDRFKVSRAKLAYILDSTAAPMCVMMPVSSWGAYIIT 192

Query: 181 TLAGLLVTYKITEYTPMGTFVAMSLMNYYALFALIMVFVVAWFSFDIGSMARFEQAALNE 240
             + GLL TY ITEYTP+G FVAMS MN+YA+F++IMVF VA+FSFDI SM R E+ AL
Sbjct: 193 LIGGLLATYSITEYTPIGAFVAMSSMNFYAIFSIIMVFFVAYFSFDIASMVRHEKLALKN 252

Query: 241 AQDETAASDATKGRVYALIIPVLALIASTVSAMIYTGAQA----SETFSILGAFENTDVN 296
             +D+      TKG+V  LI+P+L LI +TVS MIYTGA+A     + FS+LG FENT V
Sbjct: 253 TEDQLEEETGTKGQVRNLILPILVLIIATVSMMIYTGAEALAADGKVFSVLGTFENTVVG 312

Query: 297 TSLVFGGTCGVL--AVVLCTFGTIKTADYPKAVWQGAKSMFGXXXXXXXXXXSTVVGEM 354
             TSLV GG C ++   +++      +    +Y ++   G KSM G           + +VG+M
Sbjct: 313 TSLVVGGFCSIIISTLLIILDRQVSVPEYVRSWIVGIKSMSGAIAILFFAWTINKIVGDM 372

Query: 355 HTGDYLSTLVAGNIHPGFLPVILFLLASVMAFATGTSWGTFGIMLPIAAAMAVKVEPALI 414
             TG YLS+LV+GNI   FLPVILF+L + MAF+TGTSWGTFGIMLPIAAAMA    P L+
Sbjct: 373 QTGKYLSSLVSGNIPMQFLPVILFVLGAAMAFSTGTSWGTFGIMLPIAAAMAANAAPELL 432

Query: 415 IPCMSAVMAGAVCGDHCSPISDTTILSSTGARCNHIDHVTSQXXXXXXXXXXXXXXXXXX 474
            +PC+SAVMAGAVCGDHCSP+SDTTILSSTGA+CNHIDHVT+Q
Sbjct: 433 LPCLSAVMAGAVCGDHCSPVSDTTILSSTGAKCNHIDHVTTQLPYAATVATATSIGYIVV 492

Query: 475 XXXKSALLGFGTTGIVLAVLIFLLKDK 501
               S L GF  T + L V+IF +K +
Sbjct: 493 GFTYSGLAGFAATAVSLIVIIFAVKKR 519
```

Based on this analysis, it is predicted that these proteins from *N.meningitidis* and *N.gonorrhoeae,* and their epitopes, could be useful antigens for vaccines or diagnostics, or for raising antibodies.

**Example 83**

The following partial DNA sequence was identified in *N.meningitidis* <SEQ ID 697>:

```
  1  ..AAGCAATGGT ATGCCGACGN .AGTATCAAG ACGGAAATGG TTATGGTCAA
 51    CGATGAGCCT GCCAAAATTC TGACTTGGGA TGAAAGCGGC CGATTACTCT
101    CGGAACTGTC TATCCGCCAC CATCAACGCA ACGGGGTGGT TTTGGAGTGG
151    TATGAAGATG GTTCTAAAAA GAGCGAAGT. GTTTATCAGG ATGACAAGTT
201    GGTCAGGAAA ACCCAGTGGG ATAAGGATGG TTATTTAATC GAACCCTGA
```

This corresponds to the amino acid sequence <SEQ ID 698; ORF27>:

```
 1  ..KQWYADXSIK TEMVMVNDEP AKILTWDESG RLLSELSIRH HQRNGVVLEW
51    YEDGSKKSEX VYQDDKLVRK TQWDKDGYLI EP*
```

Further work revealed the complete nucleotide sequence <SEQ ID 699>:

```
  1  ATGAAAAAAT TATCTCGGAT TGTATTTTCA ACTGTCCTGT TGGGTTTTTC
 51  GGCCGCTTTG CCGGCGCAGA CCTATTCTGT TTATTTTAAT CAGAACGGAA
101  AGCTGACGGC GACGATGTCT TCTGCCGCTT ATATCAGGCA ATATAGTGTG
151  GTGGCGGGTA TTGCGCACGC GCAGGATTTT TATTATCCGT CGATGAAGAA
201  ATATTCTGAA CCTTATATCG TTGCTTCAAC GCAAATCAAA TCTTTTGTGC
251  CTACCCTGCA AAACGGTATG TTGATTTTGT GGCATTTTAA TGGTCAGAAA
301  AAAATGGCGG GGGGCTTCAG CAAGGGTAAG CCGGACGGGG AGTGGGTCAA
351  CTGGTATCCG AACGGTAAAA AATCTGCCGT TATGCCTTAT AAAAATGGCT
401  TGAGTGAGGG TACGGGATAC CGCTATTACC GTAACGGCGG CAAGGAAAGC
451  GAAATCCAGT TTAAGCAAAA TAAGGCAAAC GGCGTATGGA AGCAATGGTA
501  TGCCGACGGC AGTATCAAGA CGGAAATGGT TATGGTCAAC GATGAGCCTG
551  CCAAAATTCT GACTTGGGAT GAAAGCGGCC GATTACTCTC GGAACTGTCT
601  ATCCGCCACC ATCAACGCAA CGGGGTGGTT TTGGAGTGGT ATGAAGATGG
651  TTCTAAAAAG AGCGAAGCTG TTTATCAGGA TGACAAGTTG GTCAGGAAAA
701  CCCAGTGGGA TAAGGATGGT TATTTAATCG AACCCTGA
```

This corresponds to the amino acid sequence <SEQ ID 700; ORF27-1>:

```
  1  MKKLSRIVFS TVLLGFSAAL PAQTYSVYFN QNGKLTATMS SAAYIRQYSV
 51  VAGIAHAQDF YYPSMKKYSE PYIVASTQIK SFVPTLQNGM LILWHFNGQK
101  KMAGGFSKGK PDGEWVNWYP NGKKSAVMPY KNGLSEGTGY RYYRNGGKES
151  EIQFKQNKAN GVWKQWYADG SIKTEMVMVN DEPAKILTWD ESGRLLSELS
201  IRHHQRNGVV LEWYEDGSKK SEAVYQDDKL VRKTQWDKDG YLIEP*
```

Computer analysis of this amino acid sequence gave the following results:

Homology with a predicted ORF from *N.meningitidis* (strain A)

ORF27 shows 91.5% identity over a 82aa overlap with an ORF (ORF27a) from strain A of *N. meningitidis:*

```
                                                      10        20        30
orf27.pep                                     KQWYADXSIKTEMVMVNDEPAKILTWDESG
                                              |||||| :||||||||||||||||||||||
orf27a      LSEGTGXRYYRNGGKESEIQFKQNKANGVWKQWYADGNIKTEMVMVNDEPAKILTWDESG
                140       150       160       170       180       190

                    40        50        60        70        80
orf27.pep   RLLSELSIRHHQRNGVVLEWYEDGSKKSEXVYQDDKLVRKTQWDKDGYLIEPX
            ||||||||:|| |||||||||||||||| | ||||||||||||| ||||||||
orf27a      RLLSELSIHHHXRNGVVLEWYEDGSKKXEAVYQDDKLVRKTQWDXDGYLIEPX
                200       210       220       230       240
```

The complete length ORF27a nucleotide sequence <SEQ ID 701> is:

```
  1 ATGAAAAAAT TATCTCGGAT TGTATTTTCA ACTGTCCTGT TGGGTTTTTC
 51 GGCCGCTTTG CCGGCGCAGA NCTATTCTGT TTATTTTAAT CAGAACGGGA
101 AACTGACGGC GACGNTGTCT TCTGCCGCNT ATATCAGGCA ATATAGTGTG
151 GCGGAGGGTA TTGCGCACGC GCAGGANTTT TANTATCCGT CGATGAAGAA
201 ATATTCCGAA CCTTATATCG TTGCTTCAAC GCAAATCAAA TCTTTTGTGC
251 CTACCCTGCA AAACGGTATG TTGATTTTGT GGCATTTTAA NGGTCAGAAA
301 AAAATGGCNG GGGGCTTCAG CAAGGGTAAG CCGGACGGGG AGTGGGTCAA
351 CTGGTATCCG AACGGTAAAA AATCTGCCGT TATGCCTTAT AAAAATGGTT
401 TGAGTGAAGG TACGGGGTNN CGCTATTACC GTAACGGCGG CAAGGAAAGC
451 GAAATCCAGT TTAAACAGAA TAAGGCAAAC GGCGTATGGA AGCAATGGTA
501 TGCCGACGGC AATATCAAAA CGGAAATGGT TATGGTCAAT GATGAGCCTG
551 CCAAAATTCT GACATGGGAT GAAAGCGGTC GATTACTCTC GGAACTGTCT
601 ATCCATCATC ATNAACGTAA TGGAGTAGTC TTAGAGTGGT ATGAAGATGG
651 TTCTAAAAAG ANTGAAGCTG TTTATCAGGA TGATAAGTTG GTCAGGAAAA
701 CCCAGTGGGA TAANGATGGT TATTTAATCG AACCCTGA
```

This encodes a protein having amino acid sequence <SEQ ID 702>:

```
  1 MKKLSRIVFS TVLLGFSAAL PAQXYSVYFN QNGKLTATXS SAAYIRQYSV
 51 AEGIAHAQXF XYPSMKKYSE PYIVASTQIK SFVPTLQNGM LILWHFXGQK
101 KMAGGFSKGK PDGEWVNWYP NGKKSAVMPY KNGLSEGTGX RYYRNGGKES
151 EIQFKQNKAN GVWKQWYADG NIKTEMVMVN DEPAKILTWD ESGRLLSELS
201 IHHHXRNGVV LEWYEDGSKK XEAVYQDDKL VRKTQWDXDG YLIEP*
```

ORF27a and ORF27-1 show 94.7% identity in 245 aa overlap:

```
                    10        20        30        40        50        60
orf27a.pep  MKKLSRIVFSTVLLGFSAALPAQXYSVYFNQNGKLTATXSSAAYIRQYSVAEGIAHAQXF
            |||||||||||||||||||||||:|||||||||||||| |||||||||||: |||||| |
orf27-1     MKKLSRIVFSTVLLGFSAALPAQTYSVYFNQNGKLTATMSSAAYIRQYSVVAGIAHAQDF
                    10        20        30        40        50        60

                    70        80        90       100       110       120
orf27a.pep  XYPSMKKYSEPYIVASTQIKSFVPTLQNGMLILWHFXGQKKMAGGFSKGKPDGEWVNWYP
             ||||||||||||||||||||||||||||||||||| |||||||||||||||||||||||
orf27-1     YYPSMKKYSEPYIVASTQIKSFVPTLQNGMLILWHFNGQKKMAGGFSKGKPDGEWVNWYP
                    70        80        90       100       110       120

                   130       140       150       160       170       180
orf27a.pep  NGKKSAVMPYKNGLSEGTGXRYYRNGGKESEIQFKQNKANGVWKQWYADGNIKTEMVMVN
            ||||||||||||||||||| ||||||||||||||||||||||||||||||:|||||||||
orf27-1     NGKKSAVMPYKNGLSEGTGYRYYRNGGKESEIQFKQNKANGVWKQWYADGSIKTEMVMVN
                   130       140       150       160       170       180

                   190       200       210       220       230       240
orf27a.pep  DEPAKILTWDESGRLLSELSIHHHXRNGVVLEWYEDGSKKXEAVYQDDKLVRKTQWDXDG
            |||||||||||||||||||||:|| ||||||||||||||| |||||||||||||||| ||
orf27-1     DEPAKILTWDESGRLLSELSIRHHQRNGVVLEWYEDGSKKSEAVYQDDKLVRKTQWDKDG



                   190       200       210       220       230       240

orf27a.pep  YLIEPX
            ||||||
orf27-1     YLIEPX
```

Homology with a predicted ORF from *N.gonorrhoeae*

ORF27 shows 96.3% identity over 82 aa overlap with a predicted ORF (ORF27ng) from *N.gonorrhoeae:*

```
orf27.pep                                    KQWYADXSIKTEMVMVNDEPAKILTWDESG   30
                                             |||||| |||||||||||||||||||||||
orf27ng     LSEGTGYRYYRNGGKESEIQFKQNKANGVWKQWYADGSIKTEMVMVNDEPAKILTWDESG  193

orf27.pep   RLLSELSIRHHQRNGVVLEWYEDGSKKSEXVYQDDKLVRKTQWDKDGYLIEP   82
            |||||||||||:||||||||||||||||| ||||||||||||||||||||||
orf27ng     RLLSELSIRHHKRNGVVLEWYEDGSKKSEAVYQDDKLVRKTQWDKDGYLIEP  245
```

The complete length ORF27ng nucleotide sequence <SEQ ID 703> is:

```
  1  ATGAAGAAAT TATCTCGGAT TGTATTTTCA ATCGTACTGT TGGGTTTTTC
 51  GGCCGCTTTG CCGGCGCAGA CCTATTCTGT TTATTTTAAT CAGAACGGGA
101  AACTGACGGC GACGATGTCT TCTGCCGCTT ATATCAGGCA ATATAGTGTG
151  GCGGCGGGTA TCGCACACGC GCAGGATTTT TATTATCCGT CGATGAAGAA
201  ATATTCCGAA CCTTATATCG TTGCTTCAAC GCAAATCAAA TCTTTTGTGC
251  CTACCCTGCA AAACGGTATG TTGATTTTGT GGCATTTTAA TGGTCAGAAA
301  AAAATGGCGG GGGGCTTCAG CAAGGGTAAG CCGGACGGGG AATGGGTCAA
351  CTGGTATCCG AACGGTAAAA AATCTGCGGT TATGCCTTAT AAAAATGGCT
401  TGAGTGAGGG TACGGGATAC CGTTATTACC GTAACGGCGG CAAGGAAAGC
451  GAAATCCAGT TTAAGCAAAA TAAGGCGAAC GGCGTATGGA AGCAATGGTA
501  TGCCGATGGA AGTATCAAGA CGGAAATGGT TATGGTCAAC GATGAGCCTG
551  CCAAAATTCT GACTTGGGAT GAAAGCGGCC GATTACTTTC GGAACTGTCT
601  ATCCGCCACC ATAAACGCAA CGGGGTGGTT TTGGAGTGGT ATGAAGATGG
651  TTCTAAAAAG AGCGAGGCTG TTTATCAGGA TGACAAGTTG GTCAGGAAAA
701  CCCAATGGGA TAAGGATGGT TATTTAATCG AACCCTGA
```

This encodes a protein having amino acid sequence <SEQ ID 704>:

```
  1  MKKLSRIVFS IVLLGFSAAL PAQTYSVYFN QNGKLTATMS SAAYIRQYSV
 51  AAGIAHAQDF YYPSMKKYSE PYIVASTQIK SFVPTLQNGM LILWHFNGQK
101  KMAGGFSKGK PDGEWVNWYP NGKKSAVMPY KNGLSEGTGY RYYRNGGKES
151  EIQFKQNKAN GVWKQWYADG SIKTEMVMVN DEPAKILTWD ESGRLLSELS
201  IRHHKRNGVV LEWYEDGSKK SEAVYQDDKL VRKTQWDKDG YLIEP*
```

ORF27ng and ORF27-1 show 98.8% identity in 245 aa overlap:

```
                      10        20        30        40        50        60
orf27-1.pep   MKKLSRIVFSTVLLGFSAALPAQTYSVYFNQNGKLTATMSSAAYIRQYSVVAGIAHAQDF
              |||||||||| |||||||||||||||||||||||||||||||||||||||||:|||||||||
orf27ng       MKKLSRIVFSIVLLGFSAALPAQTYSVYFNQNGKLTATMSSAAYIRQYSVAAGIAHAQDF
                      10        20        30        40        50        60

                      70        80        90       100       110       120
orf27-1.pep   YYPSMKKYSEPYIVASTQIKSFVPTLQNGMLILWHFNGQKKMAGGFSKGKPDGEWVNWYP
              ||||||||||||||||||||||||||||||||||||||||||||||||||||||||||||
orf27ng       YYPSMKKYSEPYIVASTQIKSFVPTLQNGMLILWHFNGQKKMAGGFSKGKPDGEWVNWYP
                      70        80        90       100       110       120

                     130       140       150       160       170       180
orf27-1.pep   NGKKSAVMPYKNGLSEGTGYRYYRNGGKESEIQFKQNKANGVWKQWYADGSIKTEMVMVN
              ||||||||||||||||||||||||||||||||||||||||||||||||||||||||||||
orf27ng       NGKKSAVMPYKNGLSEGTGYRYYRNGGKESEIQFKQNKANGVWKQWYADGSIKTEMVMVN
                     130       140       150       160       170       180

                     190       200       210       220       230       240
orf27-1.pep   DEPAKILTWDESGRLLSELSIRHHQRNGVVLEWYEDGSKKSEAVYQDDKLVRKTQWDKDG
              ||||||||||||||||||||||||:|||||||||||||||||||||||||||||||||||
orf27ng       DEPAKILTWDESGRLLSELSIRHHKRNGVVLEWYEDGSKKSEAVYQDDKLVRKTQWDKDG
```

```
                    190       200       210       220       230       240

orf27-1.pep   YLIEPX
              ||||||
orf27ng       YLIEPX
```

Based on this analysis, including the putative leader sequence in the gonococcal protein, it was predicted that the proteins from *N.meningitidis* and *N.gonorrhoeae,* and their epitopes, could be useful antigens for vaccines or diagnostics, or for raising antibodies.

ORF27-1 (24.5kDa) was cloned in pET and pGex vectors and expressed in *E.coli,* as described above. The products of protein expression and purification were analyzed by SDS-PAGE. Figure 17A shows the results of affinity purification of the GST-fusion protein, and Figure 17B shows the results of expression of the His-fusion in *E. coli.* Purified GST-fusion protein was used to immunise mice, whose sera were used for ELISA, which gave a positive result, confirming that ORF27-1 is a surface-exposed protein and a useful immunogen.

**Example 84**

The following partial DNA sequence was identified in *N.meningitidis* <SEQ ID 705>:

```
  1  ATGAAATTTA CCAAGCACCC CGTCTGGGCA ATGGCGTTCC GCCCATTTTA
 51  TTCGCTGGCG GCTCTGTACG GCGCATTGTC CGTATTGCTG TGGGGTTTCG
101  GCTACACGGG AACGCACkAG CTGTCCGGTT TCTATTGGCA CGCGCATGAg
151  ATGATTTGGG GTTATGCCGG ACTGGTCGTC ATCGCCTTCC TGCTGACCGC
201  CGTCGCCACT TGGACGGGGC AGCCGCCCAC GCGGGGCGGC GTaTCTGGTC
251  GGCTTGACTA TCTTTTGGCT GGCTGCGCGG ATTGCCGCCT TTATCCCGGG
301  TTGGGGTGCG TCGGCAAGCG GCATACTCGG TACGCTGTTT TTCTGGTACG
351  GCGCGGTGTG CATGGCTTTG CCCGTTATCC GTTCGCAGAA TCAACGCAAC
401  TATGTTgCCG TGTTCGCGCT GTTCGTCTTG GGCGGCACGC ATGCGGCGTT
451  CCACGTCCAG CTGCACAACG GCAACCTAGG CGGACTCTTG AGCGGATTGC
501  AGTCGGGCTT GGTGATG
```

This corresponds to the amino acid sequence <SEQ ID 706; ORF47>:

```
  1  MKFTKHPVWA MAFRPFYSLA ALYGALSVLL WGFGYTGTHX LSGFYWHAHE
 51  MIWGYAGLVV IAFLLTAVAT WTGQPPTRGG VLVGLTIFWL AARIAAFIPG
101  WGASASGILG TLFFWYGAVC MALPVIRSQN QRNYVAVFAL FVLGGTHAAF
151  HVQLHNGNLG GLLSGLQSGL VM
```

Further work revealed the complete nucleotide sequence <SEQ ID 707>:

```
  1  ATGAAATTTA CCAAGCACCC CGTCTGGGCA ATGGCGTTCC GCCCATTTTA
 51  TTCGCTGGCG GCTCTGTACG GCGCATTGTC CGTATTGCTG TGGGGTTTCG
101  GCTACACGGG AACGCACGAG CTGTCCGGTT TCTATTGGCA CGCGCATGAG
151  ATGATTTGGG GTTATGCCGG ACTGGTCGTC ATCGCCTTCC TGCTGACCGC
201  CGTCGCCACT TGGACGGGGC AGCCGCCCAC GCGGGGCGGC GTTCTGGTCG
251  GCTTGACTAT CTTTTGGCTG GCTGCGCGGA TTGCCGCCTT TATCCCGGGT
301  TGGGGTGCGT CGGCAAGCGG CATACTCGGT ACGCTGTTTT TCTGGTACGG
351  CGCGGTGTGC ATGGCTTTGC CCGTTATCCG TTCGCAGAAT CAACGCAACT
401  ATGTTGCCGT GTTCGCGCTG TTCGTCTTGG GCGGCACGCA TGCGGCGTTC
451  CACGTCCAGC TGCACAACGG CAACCTAGGC GGACTCTTGA GCGGATTGCA
501  GTCGGGCTTG GTGATGGTGT CGGGTTTTAT CGGTCTGATT GGTACGCGGA
551  TTATTTCGTT TTTTACGTCC AAACGCTTGA ATGTGCCGCA GATTCCCAGT
601  CCGAAATGGG TGGCGCAGGC TTCGCTGTGG CTGCCCATGC TGACTGCCAT
651  GCTGATGGCG CACGGTGTGT TGGCTTGGCT GTCTGCCGTT TTTGCCTTTG
701  CGGCAGGTGT GATTTTTACC GTGCAGGTGT ACCGCTGGTG GTATAAACCC
751  GTGTTGAAAG AGCCGATGCT GTGGATTCTG TTTGCCGGCT ATCTGTTTAC
```

```
 801  CGGATTGGGG CTGATTGCGG TCGGCGCGTC TTATTTCAAA CCCGCTTTCC
 851  TCAATCTGGG TGTGCATCTG ATCGGGGTCG GCGGTATCGG CGTGCTGACT
 901  TTGGGCATGA TGGCGCGTAC CGCGCTTGGT CATACGGGCA ATCCGATTTA
 951  TCCGCCGCCC AAAGCCGTTC CCGTTGCGTT TTGGCTGATG ATGGCGGCAA
1001  CCGCCGTCCG TATGGTTGCC GTATTTTCTT CCGGCACTGC CTACACGCAC
1051  AGCATCCGCA CCTCTTCGGT TTTGTTTGCA CTCGCGCTTT TGGTGTATGC
1101  GTGGAAGTAT ATTCCTTGGC TGATTCGTCC GCGTTCGGAC GGCAGGCCCG
1151  GTTGA
```

This corresponds to the amino acid sequence <SEQ ID 708; ORF47-1>:

```
  1  MKFTKHPVWA MAFRPFYSLA ALYGALSVLL WGFGYTGTHE LSGFYWHAHE
 51  MIWGYAGLVV IAFLLTAVAT WTGQPPTRGG VLVGLTIFWL AARIAAFIPG
101  WGASASGILG TLFFWYGAVC MALPVIRSQN QRNYVAVFAL FVLGGTHAAF
151  HVQLHNGNLG GLLSGLQSGL VMVSGFIGLI GTRIISFFTS KRLNVPQIPS
201  PKWVAQASLW LPMLTAMLMA HGVLAWLSAV FAFAAGVIFT VQVYRWWYKP
251  VLKEPMLWIL FAGYLFTGLG LIAVGASYFK PAFLNLGVHL IGVGGIGVLT
301  LGMMARTALG HTGNPIYPPP KAVPVAFWLM MAATAVRMVA VFSSGTAYTH
351  SIRTSSVLFA LALLVYAWKY IPWLIRPRSD GRPG*
```

Computer analysis of this amino acid sequence predicts a leader peptide and also gave the following results:

Homology with a predicted ORF from *N.meningitidis* (strain A)

ORF47 shows 99.4% identity over a 172aa overlap with an ORF (ORF47a) from strain A of *N. meningitidis:*

```
                     10        20        30        40        50        60
orf47.pep   MKFTKHPVWAMAFRPFYSLAALYGALSVLLWGFGYTGTHXLSGFYWHAHEMIWGYAGLVV
            ||||||||||||||||||||||||||||||||||||||| ||||||||||||||||||||
orf47a      MKFTKHPVWAMAFRPFYSLAALYGALSVLLWGFGYTGTHELSGFYWHAHEMIWGYAGLVV
                     10        20        30        40        50        60

                     70        80        90       100       110       120
orf47.pep   IAFLLTAVATWTGQPPTRGGVLVGLTIFWLAARIAAFIPGWGASASGILGTLFFWYGAVC
            ||||||||||||||||||||||||||||||||||||||||||||||||||||||||||||
orf47a      IAFLLTAVATWTGQPPTRGGVLVGLTIFWLAARIAAFIPGWGASASGILGTLFFWYGAVC
                     70        80        90       100       110       120

                    130       140       150       160       170
orf47.pep   MALPVIRSQNQRNYVAVFALFVLGGTHAAFHVQLHNGNLGGLLSGLQSGLVM
            ||||||||||||||||||||||||||||||||||||||||||||||||||||
orf47a      MALPVIRSQNQRNYVAVFALFVLGGTHAAFHVQLHNGNLGGLLSGLQSGLVMVSGFIGLI
                    130       140       150       160       170       180

orf47a      GTRIISFFTSKRLNVPQIPSPKWVAQASLWLPMLTAMLMAHGVMPWLSAAFAFAAGVIFT
                    190       200       210       220       230       240
```

The complete length ORF47a nucleotide sequence <SEQ ID 709> is:

```
   1  ATGAAATTTA CCAAGCACCC CGTTTGGGCA ATGGCGTTCC GCCCGTTTTA
  51  TTCACTGGCG GCTCTGTACG GCGCATTGTC CGTATTGCTG TGGGGTTTCG
 101  GCTACACGGG AACGCACGAG CTGTCCGGTT TCTATTGGCA CGCGCATGAG
 151  ATGATTTGGG GTTATGCCGG ACTGGTCGTC ATCGCCTTCC TGCTGACCGC
 201  CGTCGCCACT TGGACGGGGC AGCCGCCCAC GCGGGGCGGC GTTCTGGTCG
 251  GCTTGACTAT CTTTTGGCTG GCTGCGCGGA TTGCCGCCTT TATCCCGGGT
 301  TGGGGTGCGT CGGCAAGCGG CATACTCGGT ACGCTGTTTT TCTGGTACGG
 351  CGCGGTGTGC ATGGCTTTGC CCGTTATCCG TTCGCAGAAT CAACGCAATT
 401  ATGTTGCCGT GTTCGCGCTG TTCGTCTTGG GCGGTACGCA CGCGGCGTTC
 451  CACGTCCAGC TGCACAACGG CAACCTAGGC GGACTCTTGA GCGGATTGCA
 501  GTCGGGCTTG GTGATGGTGT CGGGTTTTAT CGGTCTGATT GGTACGCGGA
 551  TTATTTCGTT TTTTACGTCC AAACGGTTGA ATGTGCCGCA GATTCCCAGT
 601  CCGAAATGGG TGGCGCAGGC TTCGCTGTGG CTGCCCATGC TGACCGCCAT
 651  GCTGATGGCG CACGGCGTGA TGCCTTGGCT GTCGGCGGCT TTCGCGTTTG
 701  CGGCAGGTGT GATTTTTACC GTGCAGGTGT ACCGCTGGTG GTATAAGCCT
 751  GTGTTGAAAG AGCCGATGCT GTGGATTCTG TTTGCCGGCT ATCTGTTTAC
 801  CGGATTGGGG CTGATTGCGG TCGGCGCGTC TTATTTCAAA CCCGCTTTCC

 851  TCAATCTGGG TGTGCATCTG ATCGGGGTCG GCGGTATCGG CGTGCTGACT
 901  TTGGGCATGA TGGCGCGTAC CGCGCTCGGT CATACGGGCA ATCCGATTTA
 951  TCCGCCGCCC AAAGCCGTTC CCGTTGCGTT TTGGCTGATG ATGGCGGCAA
1001  CCGCCGTCCG TATGGTTGCC GTATTTTCTT CCGGCACTGC CTACACGCAC
1051  AGCATACGCA CCTCTTCGGT TTTGTTTGCA CTCGCGCTTT TGGTGTATGC
1101  GTGGAAGTAT ATTCCTTGGC TGATTCGTCC GCGTTCGGAC GGCAGGCCCG
1151  GTTGA
```

This encodes a protein having amino acid sequence <SEQ ID 710>:

```
  1  MKFTKHPVWA MAFRPFYSLA ALYGALSVLL WGFGYTGTHE LSGFYWHAHE
 51  MIWGYAGLVV IAFLLTAVAT WTGQPPTRGG VLVGLTIFWL AARIAAFIPG
101  WGASASGILG TLFFWYGAVC MALPVIRSQN QRNYVAVFAL FVLGGTHAAF
151  HVQLHNGNLG GLLSGLQSGL VMVSGFIGLI GTRIISFFTS KRLNVPQIPS
201  PKWVAQASLW LPMLTAMLMA HGVMPWLSAA FAFAAGVIFT VQVYRWWYKP
251  VLKEPMLWIL FAGYLFTGLG LIAVGASYFK PAFLNLGVHL IGVGGIGVLT
301  LGMMARTALG HTGNPIYPPP KAVPVAFWLM MAATAVRMVA VFSSGTAYTH
351  SIRTSSVLFA LALLVYAWKY IPWLIRPRSD GRPG*
```

ORF47a and ORF47-1 show 99.2% identity in 384 aa overlap:

```
                       10        20        30        40        50        60
orf47a.pep   MKFTKHPVWAMAFRPFYSLAALYGALSVLLWGFGYTGTHELSGFYWHAHEMIWGYAGLVV
             ||||||||||||||||||||||||||||||||||||||||||||||||||||||||||||
orf47-1      MKFTKHPVWAMAFRPFYSLAALYGALSVLLWGFGYTGTHELSGFYWHAHEMIWGYAGLVV
                       10        20        30        40        50        60

                       70        80        90       100       110       120
orf47a.pep   IAFLLTAVATWTGQPPTRGGVLVGLTIFWLAARIAAFIPGWGASASGILGTLFFWYGAVC
             ||||||||||||||||||||||||||||||||||||||||||||||||||||||||||||
orf47-1      IAFLLTAVATWTGQPPTRGGVLVGLTIFWLAARIAAFIPGWGASASGILGTLFFWYGAVC
                       70        80        90       100       110       120

                      130       140       150       160       170       180
orf47a.pep   MALPVIRSQNQRNYVAVFALFVLGGTHAAFHVQLHNGNLGGLLSGLQSGLVMVSGFIGLI
             ||||||||||||||||||||||||||||||||||||||||||||||||||||||||||||
orf47-1      MALPVIRSQNQRNYVAVFALFVLGGTHAAFHVQLHNGNLGGLLSGLQSGLVMVSGFIGLI
                      130       140       150       160       170       180

                      190       200       210       220       230       240
orf47a.pep   GTRIISFFTSKRLNVPQIPSPKWVAQASLWLPMLTAMLMAHGVMPWLSAAFAFAAGVIFT
             ||||||||||||||||||||||||||||||||||||||||||: ||||:||||||||||
orf47-1      GTRIISFFTSKRLNVPQIPSPKWVAQASLWLPMLTAMLMAHGVLAWLSAVFAFAAGVIFT
                      190       200       210       220       230       240

                      250       260       270       280       290       300
orf47a.pep   VQVYRWWYKPVLKEPMLWILFAGYLFTGLGLIAVGASYFKPAFLNLGVHLIGVGGIGVLT
             ||||||||||||||||||||||||||||||||||||||||||||||||||||||||||||
orf47-1      VQVYRWWYKPVLKEPMLWILFAGYLFTGLGLIAVGASYFKPAFLNLGVHLIGVGGIGVLT
                      250       260       270       280       290       300

                      310       320       330       340       350       360
orf47a.pep   LGMMARTALGHTGNPIYPPPKAVPVAFWLMMAATAVRMVAVFSSGTAYTHSIRTSSVLFA
             ||||||||||||||||||||||||||||||||||||||||||||||||||||||||||||
orf47-1      LGMMARTALGHTGNPIYPPPKAVPVAFWLMMAATAVRMVAVFSSGTAYTHSIRTSSVLFA
                      310       320       330       340       350       360

                      370       380
orf47a.pep   LALLVYAWKYIPWLIRPRSDGRPGX
             ||||||||||||||||||||||||
orf47-1      LALLVYAWKYIPWLIRPRSDGRPGX
                      370       380
```

<u>Homology with a predicted ORF from *N.gonorrhoeae*</u>

ORF47 shows 97.1% identity over 172 aa overlap with a predicted ORF (ORF47ng) from *N.gonorrhoeae:*

```
ORF47      MKFTKHPVWAMAFRPFYSLAALYGALSVLLWGFGYTGTHELSGFYWHAHEMIWGYAGLVV   60
           ||||||||||||||||||||||||||||||||||||||||||||||||||||||||||||

ORF47ng    MKFTKHPVWAMAFRPFYSLAALYGALSVLLWGFGYTGTHELSGFYWHAHEMIWGYAGLVV   60

ORF47      IAFLLTAVATWTGQPPTRGGVLVGLTIFWLAARIAAFIPGWGASASGILGTLFFWYGAVC  120
           |||||||||||||||||||||||||| ||||||||||||||||:||||||||||||||||
ORF47ng    IAFLLTAVATWTGQPPTRGGVLVGLTAFWLAARIAAFIPGWGAAASGILGTLFFWYGAVC  120

ORF47      MALPVIRSQNQRNYVAVFALFVLGGTHAAFHVQLHNGNLGGLLSGLQSGLVM          172
           ||||||||||:||||||||:||||||||||||||||||||||||||||||||
ORF47ng    MALPVIRSQNRRNYVAVFAIFVLGGTHAAFHVQLHNGNLGGLLSGLQSGLVMVWGFIGLI  180
```

The ORF47ng nucleotide sequence <SEQ ID 711> is predicted to encode a protein comprising amino acid sequence <SEQ ID 712>:

```
  1  MKFTKHPVWA MAFRPFYSLA ALYGALSVLL WGFGYTGTHE LSGFYWHAHE
 51  MIWGYAGLVV IAFLLTAVAT WTGQPPTRGG VLVGLTAFWL AARIAAFIPG
101  WGAAASGILG TLFFWYGAVC MALPVIRSQN RRNYVAVFAI FVLGGTHAAF
151  HVQLHNGNLG GLLSGLQSGL VMVWGFIGLI GMKIISFFTS KRLKLPQIPS
201  PKWVAHASLW LPMLNAILMA HRVMPWLSAA FPFAAGVIFT VQVYAGGITP
251  IEETSCGSVA GICYRLGNSS G
```

The predicted leader peptide and transmembrane domains are identical (except for an Ile/Ala substitution at residue 87 and an Leu/Ile substitution at position 140) to sequences in the meningococcal protein (see also *Pseudomonas stutzeri* orf396, accession number e246540):

```
TM segments in ORF47ng
    INTEGRAL    Likelihood = -5.63    Transmembrane    52 -  68
    INTEGRAL    Likelihood = -3.88    Transmembrane   169 - 185
    INTEGRAL    Likelihood = -3.08    Transmembrane    82 -  98
    INTEGRAL    Likelihood = -1.91    Transmembrane   134 - 150
    INTEGRAL    Likelihood = -1.44    Transmembrane   107 - 123
    INTEGRAL    Likelihood = -1.38    Transmembrane   227 - 243
```

Further work revealed the complete gonococcal DNA sequence <SEQ ID 713>:

```
   1  ATGAAATTTA CCAAACATCC CGTCTGGGCA ATGGCGTTCC GCCCGTTTTA
  51  TTCACTGGCG GCACTGTACG GCGCATTGTC CGTATTGCTG TGGGGTTTCG
 101  GCTACACGGG AACGCACGAG CTGTCCGGTT TCTATTGGCA CGCGCATGAG
 151  ATGATTTGGG GTTATGCCGG TCTCGTCGTC ATCGCCTTCC TGCTGACCGC
 201  CGTCGCCACT TGGACGGGAC AGCCGCCCAC GAGGGGCGGC GTTCTGGTCG
 251  GCTTGACCGC CTTTTGGCTG GCTGCGCGGA TTGCCGCCTT TATCCCGGGT
 301  TGGGGTGCGG CGGCAAGCGG CATACTCGGT ACGCTGTTTT TCTGGTACGG
 351  CGCGGTGTGC ATGGCTTTGC CCGTTATCCG TtcgCAAAAC CGGCGCAACT
 401  ATGtcgCCGT ATTCGCAATA TTTGTGCTGG GCGGTACGCA TGCGgcgTTC
 451  CACGtccAgc tGCACAACGG CAACCTAGGC GGACTCTTGA GCGGATTGCA
 501  GTCGGGCCTG GTTATGGTGT CGGGCTTTAT CGGCCTGATT GGGATGAGGA
 551  TTATTTCGTT TTTTACGTCC AAACGGTTGA ACGTGCCGCA GATTCCCAGT
 601  CCGAAATGGG TGGCGCAGGC TTCGCTGTGG CTACCCATGC TGACCGCCAT
 651  ACTGATGGCG CACGGCGTGA TGCCTTGGCT GTCGGCGGCT TTCGCGTTTG
 701  CGGCGGGCGT GATTTTTACC GTACAGGTGT ACCGCTGGTG GTATAAACCC
 751  GTATTGAAAG AACCGATGCT GTGGATTCTG TTTGCCGGCT ATCTGTTTAC
 801  CGGATTGGGG CTGATTGCGG TCGGCGCGTC TTATTTCAAA CCTGCCTTCC
 851  TCAATCTGGG CGTACATCTG ATCGGGGTCG GCGGTATCGG CGTGCTGACT
 901  TTGGGCATGA TGGCGCGTAC CGCGCTCGGT CATACGGGCA ATTCGATTTA
 951  TCCGCCGCCC AAAGCCGTTC CCGTTGCGTT TTGGCTGATG ATGGCGGCAA
1001  CCGCCGTCCG TATGGTTGCC GTATTTTCTT CCGGCACTGC CTACACGCAC
1051  AGCATCCGCA CGTCTTCGGT TTTGTTTGCA CTCGCGCTGC TGGTGTATGC
1101  GTGGAAATAC ATTCCGTGGC TGATCCGTCC GCGTTCGGAC GGCAGGCCCG
1151  GTTGA
```

This encodes a protein having amino acid sequence <SEQ ID 714; ORF47ng-1>:

```
  1  MKFTKHPVWA MAFRPFYSLA ALYGALSVLL WGFGYTGTHE LSGFYWHAHE
 51  MIWGYAGLVV IAFLLTAVAT WTGQPPTRGG VLVGLTAFWL AARIAAFIPG
101  WGAAASGILG TLFFWYGAVC MALPVIRSQN RRNYVAVFAI FVLGGTHAAF
151  HVQLHNGNLG GLLSGLQSGL VMVSGFIGLI GMRIISFFTS KRLNVPQIPS
201  PKWVAQASLW LPMLTAILMA HGVMPWLSAA FAFAAGVIFT VQVYRWWYKP
251  VLKEPMLWIL FAGYLFTGLG LIAVGASYFK PAFLNLGVHL IGVGGIGVLT
301  LGMMARTALG HTGNSIYPPP KAVPVAFWLM MAATAVRMVA VFSSGTAYTH
```

```
351  SIRTSSVLFA LALLVYAWKY IPWLIRPRSD GRPG*
```

ORF47ng-1 and ORF47-1 show 97.4% identity in 384 aa overlap:

```
                      10        20        30        40        50        60
orf47-1.pep   MKFTKHPVWAMAFRPFYSLAALYGALSVLLWGFGYTGTHELSGFYWHAHEMIWGYAGLVV
              ||||||||||||||||||||||||||||||||||||||||||||||||||||||||||||
orf47ng-1     MKFTKHPVWAMAFRPFYSLAALYGALSVLLWGFGYTGTHELSGFYWHAHEMIWGYAGLVV
                      10        20        30        40        50        60

                      70        80        90       100       110       120
orf47-1.pep   IAFLLTAVATWTGQPPTRGGVLVGLTIFWLAARIAAFIPGWGASASGILGTLFFWYGAVC
              |||||||||||||||||||||||||| ||||||||||||||||:||||||||||||||||
orf47ng-1     IAFLLTAVATWTGQPPTRGGVLVGLTAFWLAARIAAFIPGWGAAASGILGTLFFWYGAVC
                      70        80        90       100       110       120

                     130       140       150       160       170       180
orf47-1.pep   MALPVIRSQNQRNYVAVFALFVLGGTHAAFHVQLHNGNLGGLLSGLQSGLVMVSGFIGLI
              ||||||||||:||||||||:||||||||||||||||||||||||||||||||||||||||
orf47ng-1     MALPVIRSQNRRNYVAVFAIFVLGGTHAAFHVQLHNGNLGGLLSGLQSGLVMVSGFIGLI
                     130       140       150       160       170       180

                     190       200       210       220       230       240
orf47-1.pep   GTRIISFFTSKRLNVPQIPSPKWVAQASLWLPMLTAMLMAHGVLAWLSAVFAFAAGVIFT
              | ||||||||||||||||||||||||||||||||||:||||||: |||:|||||||||||
orf47ng-1     GMRIISFFTSKRLNVPQIPSPKWVAQASLWLPMLTAILMAHGVMPWLSAAFAFAAGVIFT
                     190       200       210       220       230       240

                     250       260       270       280       290       300
orf47-1.pep   VQVYRWWYKPVLKEPMLWILFAGYLFTGLGLIAVGASYFKPAFLNLGVHLIGVGGIGVLT
              ||||||||||||||||||||||||||||||||||||||||||||||||||||||||||||
orf47ng-1     VQVYRWWYKPVLKEPMLWILFAGYLFTGLGLIAVGASYFKPAFLNLGVHLIGVGGIGVLT
                     250       260       270       280       290       300

                     310       320       330       340       350       360
orf47-1.pep   LGMMARTALGHTGNPIYPPPKAVPVAFWLMMAATAVRMVAVFSSGTAYTHSIRTSSVLFA
              |||||||||||||| |||||||||||||||||||||||||||||||||||||||||||||
orf47ng-1     LGMMARTALGHTGNSIYPPPKAVPVAFWLMMAATAVRMVAVFSSGTAYTHSIRTSSVLFA
                     310       320       330       340       350       360

                     370       380
orf47-1.pep   LALLVYAWKYIPWLIRPRSDGRPGX
              |||||||||||||||||||||||||
orf47ng-1     LALLVYAWKYIPWLIRPRSDGRPGX
                     370       380
```

Furthermore, ORF47ng-1 shows significant homology to an ORF from *Pseudomonas stutzeri:*

```
gnl|PID|e246540 (Z73914) ORF396 protein [Pseudomonas stutzeri] Length = 396
Score =  155 bits (389), Expect = 5e-37
Identities = 121/391 (30%), Positives = 169/391 (42%), Gaps = 21/391 (5%)

Query: 7   PVWAMAFRPFYSLAALYGALSVLLWGFGYTGTHELSGFY-------WHAHEMIWGYAGLV 59
            P+W +AFRPF+    +LY  L++ LW    +TG     GF        WH HEM++G+A  +
Sbjct: 14  PIWRLAFRPFFLAGSLYALLAIPLWVAAWTGLWP--GFQPTGGWLAWHRHEMLFGFAMAI 71

Query: 60  VIAFLLTAVATWTGQPPTRGGVLVGLTAFWLAARIAAFIPGWGAAASGILGTLFFWYGAV 119
            V  FLLTAV TWTGQ     G  LVGL A WLAAR+  ++ G  AA     L  LF
Sbjct: 72  VAGFLLTAVQTWTGQTAPSGNRLVGLAAVWLAARL-GWLFGLPAAWLAPLDLLFLVALVW 130

Query: 120 CMALPVIRSQNRRNYVAVFAIFVLGGTHAAFXXXXXXXXXXXXXXXXXXXXMVSGFIGL 179
             MA  +    + +RNY   V  + ++ G                         +V+  + L
Sbjct: 131 MMAQMLWAVRQKRNYPIVVVLSLMLGADVLILTGLLQGNDALQRQGVLAGLWLVAALMAL 190

Query: 180 IGMRIISFFTSKRLNVPQIPSP-KWVAQASLWLPMLTAILMAHGV----MPWLSAAFAFA 234
            IG R+I FFT + L        P W+  A L       + A+L A GV      P L   F  A
Sbjct: 191 IGGRVIPFFTQRGLGKVDAVKPWVWLDVALLVGTGVIALLHAFGVAMRPQPLLGLLFV-A 249

Query: 235 AGVIFTVQVYRWWYKPVLKEPMLWILFAGYLFTGLGLIAVGASYF-KPAFXXXXXXXXXX 293
             GV   +++ RW+ K + K  +LW L     L+  +     +  +F  A
Sbjct: 250 IGVGHLLRLMRWYDKGIWKVGLLWSLHVAMLWLVVAAFGLALWHFGLLAQSSPSLHALSV 309

Query: 294 XXXXXXXXXMMARTALGHTGNSIYPPPKAVPVAFWLXXXXXXXXXXXXFSSGTAYTHSIR 353
                      M+AR  LGHTG  +  P  +  AF L            F S       +
Sbjct: 310 GSMSGLILAMIARVTLGHTGRPLQLPAGIIG-AFVL---FNLGTAARVFLSVAWPVGGLW 365

Query: 354 TSSVLFALALLVYAWKYIPWLIRPRSDGRPG 384
              ++V + LA  +Y W+Y P L+  R DG PG
Sbjct: 366 LAAVCWTLAFALYVWRYAPMLVAARVDGHPG 396
```

Based on this analysis, it is predicted that the proteins from *N.meningitidis* and *N.gonorrhoeae,* and their epitopes, could be useful antigens for vaccines or diagnostics, or for raising antibodies.

**Example 85**

The following partial DNA sequence was identified in *N.meningitidis* <SEQ ID 715>:

```
  1 ..ATGCCGTCTG AAGGTTCAGA CGGCmTCGGT GyCGGGGAAy CAGAAGyGGT
 51   AGCGCATGCC CAATGAGACT TCGTGGGTTT TGAAGCGGGT GTTTTCCAAG
101   CGTCCCCAGT TGTGGTAACG GTATCCGGTG TCyAArGTCA GCTTGGGyGT
151   GATGTCGAAa CCGACACCGG CGATGACACC AAGACCyAmG CTGCTGATrC
201   TGTkGCTTTC GTGATAGGsA GGTTTGyTGG kmksAsyTTG TAyrATwkkG
251   CCTssCwsTG kAGmGCCkTk CkyTGGTkkA swGrwArTAG TCGTGGTTTy
301   TkTTyyCACC GAATGAACyT GATGTTTAAC GTGTCCGTAG GCGACGCGCG
351   CGCCGATATA GGGTTTGAAT TTATCGTTGA GTTTGAAATC GTAAATGGCG
401   GACAAGCCGA GAGAAGAAAC GGCGTGGAAG CTGCCGTTTC CCTGATGTTT
451   TGTTTGGGTT TCTTTGTAGT TGTTGTTTAT CTCTTCAGTA ACTTTTTTAG
501   TAGAAGAATT ACTTTCTTTC CATTTTCTGT AACTGGCATA ATCTGCCGCT
551   ATTCTCCAGC CGCCGAAATC ..
```

This corresponds to the amino acid sequence <SEQ ID 716; ORF67>:

```
  1  ..MPSEGSDGXG XGEXEXVAHA QXDFVGFEAG VFQASPVVVT VSGVXXQLGX
 51    DVETDTGDDT KTXAADXVAF VIGRFXGXXL YXXAXXXXAX XWXXXXSRGF
101    XXHRMNLMFN VSVGDARADI GFEFIVEFEI VNGGQAERRN GVEAAVSLMF
151    CLGFFVVVVY LFSNFFSRRI TFFPFSVTGI ICRYSPAAEI ..
```

Computer analysis of this amino acid sequence gave the following results:

Homology with a predicted ORF from *N.gonorrhoeae*

ORF67 shows 51.8% identity over 199 aa overlap with a predicted ORF (ORF67ng) from *N.gonorrhoeae:*

```
orf67.pep                                      MPSEGSDGXGXGEXEXVAHAQXDFVGFEAG   30
                                              |||||||| | || | |||||  |||||||
orf67ng         TNFEIAVLSGMTVRVFYCARPAPVNGGRLKMPSEGSDGIGIGESEAVAHAQRGFVGFEAG  146
                  90       100       110       120       130       140

orf67.pep       VFQASPVVVTVSGVXXQLGXDVETDTGDDTKTXAADXVAFVIGRFXGXXLYXXAXXXXAX   90
                |||||||||:|:||  | | || : :   ::: ||  |||:|| |    :        : 
orf67ng         VFQASPVVVAVAGVQGQAGRDVYAHARHRAEAQAAAAVAFLIGVFLRMSVRINRNCCVSI  206

orf67.pep       XWXXXXSRGFXXHRMNLMFNVSVGDARADIGFEFIVEFEIVNGGQAERRNGVEAAVSLMF  150
                :     |  :   |:: : :|||||||:||||||:|||||||||||||||||| || |||
orf67ng         TRVGGKSTCYFFSRIDAVSDVSVGDARTDIGFEFVVEFEIVNGGQAERRNGVECAVFLMF  266

orf67.pep       CLGFFVV--------VVYLFSNFFSRRITFF-PFSVTGIICRYSPAAEI             190
                 |  | |        ::  |: |: : | :  || |||||   :||||:
orf67ng         RLLVFYVKLVAAKSFIILSFQLFYVHGIFIVVPFPVTGIIRGDAPAAEVVADRHPGVDGM  326
```

The ORF67ng nucleotide sequence <SEQ ID 717> is predicted to encode a protein comprising amino acid sequence <SEQ ID 718>:

```
  1  MPSETVGSIV NVGVDESVGF SPPFPSIQHF YRFHRIHRIR LFRPPGPMQL
 51  NRHSHGSGNL GRGVWATVLS DKFPCGQVRI PACAGMTNFE IAVLSGMTVR
101  VFYCARPAPV NGGRLKMPSE GSDGIGIGES EAVAHAQRGF VGFEAGVFQA
151  SPVVVAVAGV QGQAGRDVYA HARHRAEAQA AAAVAFLIGV FLRMSVRINR
201  NCCVSITRVG GKSTCYFFSR IDAVSDVSVG DARTDIGFEF VVEFEIVNGG
251  QAERRNGVEC AVFLMFRLLV FYVKLVAAKS FIILSFQLFY VHGIFIVVPF
301  PVTGIIRGDA PAAEVVADRH PGVDGMRTDV SEIIAYRAYF VFAWSGWFRI
351  IVGNAFGGVG *
```

Based on the presence of a several putative transmembrane domains in the gonococcal protein, it is predicted that the proteins from *N.meningitidis* and *N.gonorrhoeae,* and their epitopes, could be useful antigens for vaccines or diagnostics, or for raising antibodies.

**Example 86**

The following partial DNA sequence was identified in *N.meningitidis* <SEQ ID 719>

```
  1  ATGTTTGCTT TTTTAGAAGC CTTTTTTGTC GAATACGGTT ATGCGGCTGT
 51  TTTTTTTGTA TTGGTCATCT GCGGTTTCGG CGTGCCGATT CCCGAGGATT
101  TGACCTTGGT AACAGGCGGC GTGATTTCGG GTATGGGTTA TACCAATCCG
151  CATATTATGT TTGCAGTCGG TATGCTCGGC GTATTGGTCG GGGACGGCAT
201  CATGTTCGCC GCCGGACGAA TTTGGGGGCA GArArTCCTA rGGTTCArAC
251  CTATTGCGsG CATCATGACG CCGrAACGTT ATGAGCAGGT TCAGGAAAAA
301  TTCGACAAAT ACGGTAACTG GGTCTTATTT GTCGCCCGTT TCCTGCCCGG
351  TTTGAGAACG GCCGTATTTG TTACAGCCGG TATCAGCCGC AAGGTTTCAT
401  ACTTGCGTTT TATCATTATG GATGGACTGG CCGCA...
```

This corresponds to the amino acid sequence <SEQ ID 720; ORF78>:

```
  1  MFAFLEAFFV EYGYAAVFFV LVICGFGVPI PEDLTLVTGG VISGMGYTNP
 51  HIMFAVGMLG VLVGDGIMFA AGRIWGQXXL XFXPIAXIMT PXRYEQVQEK
101  FDKYGNWVLF VARFLPGLRT AVFVTAGISR KVSYLRFIIM DGLAA...
```

Further work revealed the complete nucleotide sequence <SEQ ID 721>:

```
  1  ATGTTTGCTT TTTTAGAAGC CTTTTTTGTC GAATACGGTT ATGCGGCTGT
 51  TTTTTTTGTA TTGGTCATCT GCGGTTTCGG CGTGCCGATT CCCGAGGATT
101  TGACCTTGGT AACAGGCGGC GTGATTTCGG GTATGGGTTA TACCAATCCG
151  CATATTATGT TTGCAGTCGG TATGCTCGGC GTATTGGTCG GGGACGGCAT
201  CATGTTCGCC GCCGGACGAA TTTGGGGGCA GAAAATCCTA AGGTTCAAAC
251  CTATTGCGCG CATCATGACG CCGAAACGTT ATGAGCAGGT TCAGGAAAAA
301  TTCGACAAAT ACGGTAACTG GGTCTTATTT GTCGCCCGTT TCCTGCCCGG
351  TTTGAGAACG GCCGTATTTG TTACAGCCGG TATCAGCCGC AAGGTTTCAT
401  ACTTGCGTTT TATCATTATG GATGGACTGG CCGCACTGAT TTCCGTCCCT
451  ATTTGGATTT ATCTGGGCGA ATACGGTGCG CACAACATCG ATTGGCTGAT
501  GGCGAAAATG CACAGCCTGC AATCGGGTAT TTTTGTTATC TTGGGTATAG
551  GTGCGACCGT TGTCGCTTGG ATTTGGTGGA AAAAACGCCA ACGTATCCAG
601  TTTTACCGCA GCAAATTGAA AGAAAAGCGG GCGCAACGCA AAGCCGCCAA
651  GGCAGCCAAA AAAGCCGCGC AAAGCAAACA ATAA
```

This corresponds to the amino acid sequence <SEQ ID 722; ORF78-1>:

```
  1  MFAFLEAFFV EYGYAAVFFV LVICGFGVPI PEDLTLVTGG VISGMGYTNP
 51  HIMFAVGMLG VLVGDGIMFA AGRIWGQKIL RFKPIARIMT PKRYEQVQEK
101  FDKYGNWVLF VARFLPGLRT AVFVTAGISR KVSYLRFIIM DGLAALISVP
151  IWIYLGEYGA HNIDWLMAKM HSLQSGIFVI LGIGATVVAW IWWKKRQRIQ
201  FYRSKLKEKR AQRKAAKAAK KAAQSKQ*
```

Computer analysis of this amino acid sequence predicts several transmembrane domains, and also gave the following results:

Homology with the dedA homologue of *H.influenzae* (accession number P45280)

ORF78 and the dedA homologue show 58% aa identity in 144aa overlap:

```
Orf78:  4   FLEAFFVEYGYAAVFFVLVICGFGVPIPEDLTLVTGGVISGM--GYTNPHIMFAVGMLGV 61
            FL  FF EYGY AV FVL+ICGFGVPIPED+TLV+GGVI+G+     N H+M  V M+GV
DedA:   20  FLIGFFTEYGYWAVLFVLIICGFGVPIPEDITLVSGGVIAGLYPENVNSHLMLLVSMIGV 79

Orf78:  62  LVGDGIMFAAGRIWGQXXLXFXPIAXIMTPXRYEQVQEKFDKYGNWVLFVARFLPGLRTA 121
            L GD  M+   GRI+G    L F PI   I+T  R   V+EKF +YGN VLFVARFLPGLR
DedA:   80  LAGDSCMYWLGRIYGTKILRFRPIRRIVTLQRLRMVREKFSQYGNRVLFVARFLPGLRAP 139

Orf78:  122 VFVTAGISRKVSYLRFIIMDGLAA 145
            +++ +GI+R+VSY+RF+++D  AA
DedA:   140 IYMVSGITRRVSYVRFVLIDFCAA 163
```

Homology with a predicted ORF from *N.meningitidis* (strain A)

ORF78 shows 93.8% identity over a 145aa overlap with an ORF (ORF78a) from strain A of *N. meningitidis:*

```
                     10        20        30        40        50        60
orf78.pep    MFAFLEAFFVEYGYAAVFFVLVICGFGVPIPEDLTLVTGGVISGMGYTNPHIMFAVGMLG
             |||:||||||||||||||||||||||||||||||||||||||||||||||||||||||||
orf78a       MFALLEAFFVEYGYAAVFFVLVICGFGVPIPEDLTLVTGGVISGMGYTNPHIMFAVGMLG
                     10        20        30        40        50        60

                     70        80        90       100       110       120
orf78.pep    VLVGDGIMFAAGRIWGQXXLXFXPIAXIMTPXRYEQVQEKFDKYGNWVLFVARFLPGLRT
             |||||||||||||||||  | | ||| |||| || |||||||||||||||||||||||||
orf78a       VLVGDGIMFAAGRIWGQKILKFKPIARIMTPKRYAQVQEKFDKYGNWVLFVARFLPGLRT
                     70        80        90       100       110       120

                    130       140
orf78.pep    AVFVTAGISRKVSYLRFIIMDGLAA
             |||||||||||||||||:|||||||
orf78a       AVFVTAGISRKVSYLRFLIMDGLAALISVPVWIYLGEYGAHNIDWLMAKMHSLQSGIFIA
                    130       140       150       160       170       180
```

The complete length ORF78a nucleotide sequence <SEQ ID 723> is:

```
  1  ATGTTTGCCC TTTTGGAAGC CTTTTTTGTC GAATACGGCT ATGCGGCCGT
 51  GTTTTTCGTT TTGGTCATCT GCGGTTTCGG CGTGCCGATT CCCGAGGATT
101  TGACCTTGGT AACAGGCGGC GTGATTTCGG GTATGGGTTA TACCAATCCG
151  CATATTATGT TTGCAGTCGG TATGCTCGGC GTATTGGTCG GGGACGGCAT
201  CATGTTCGCC GCCGGACGCA TCTGGGGGCA GAAAATCCTC AAGTTCAAAC
251  CGATTGCGCG CATCATGACG CCGAAACGTT ACGCACAGGT TCAGGAAAAA
301  TTCGACAAAT ACGGCAACTG GGTGTTATTT GTCGCTCGTT TCCTGCCCGG
351  TTTGCGGACT GCCGTTTTCG TTACCGCCGG CATCAGCCGC AAAGTATCGT
401  ATCTGCGCTT TCTGATTATG GACGGGCTTG CCGCGCTGAT TTCCGTGCCC
451  GTTTGGATTT ACTTGGGCGA GTACGGCGCG CACAACATCG ATTGGCTGAT
501  GGCGAAAATG CACAGCCTGC AATCCGGCAT CTTCATCGCA TTGGGCGTGC
551  TGGCGGCGGC GCTGGCGTGG TTCTGGTGGC GCAAACGCCG ACATTATCAG
601  CTTTACCGCG CACAATTGAG CGAAAAACGC GCCAAACGCA AGGCGGAAAA
651  GGCAGCGAAA AAAGCGGCAC AGAAGCAGCA GTAA
```

This encodes a protein having amino acid sequence <SEQ ID 724>:

```
  1  MFALLEAFFV EYGYAAVFFV LVICGFGVPI PEDLTLVTGG VISGMGYTNP
 51  HIMFAVGMLG VLVGDGIMFA AGRIWGQKIL KFKPIARIMT PKRYAQVQEK
101  FDKYGNWVLF VARFLPGLRT AVFVTAGISR KVSYLRFLIM DGLAALISVP
151  VWIYLGEYGA HNIDWLMAKM HSLQSGIFIA LGVLAAALAW FWWRKRRHYQ
201  LYRAQLSEKR AKRKAEKAAK KAAQKQQ*
```

ORF78a and ORF78-1 show 89.0% identity in 227 aa overlap:

```
                    10        20        30        40        50        60
orf78a.pep  MFALLEAFFVEYGYAAVFFVLVICGFGVPIPEDLTLVTGGVISGMGYTNPHIMFAVGMLG
            |||:||||||||||||||||||||||||||||||||||||||||||||||||||||||||
orf78-1     MFAFLEAFFVEYGYAAVFFVLVICGFGVPIPEDLTLVTGGVISGMGYTNPHIMFAVGMLG
                    10        20        30        40        50        60

                    70        80        90       100       110       120
orf78a.pep  VLVGDGIMFAAGRIWGQKILKFKPIARIMTPKRYAQVQEKFDKYGNWVLFVARFLPGLRT
            ||||||||||||||||||||:||||||||||||| |||||||||||||||||||||||||
orf78-1     VLVGDGIMFAAGRIWGQKILRFKPIARIMTPKRYEQVQEKFDKYGNWVLFVARFLPGLRT
                    70        80        90       100       110       120

                   130       140       150       160       170       180
orf78a.pep  AVFVTAGISRKVSYLRFLIMDGLAALISVPVWIYLGEYGAHNIDWLMAKMHSLQSGIFIA
            |||||||||||||||||:|||||||||||||:|||||||||||||||||||||||||||:
orf78-1     AVFVTAGISRKVSYLRFIIMDGLAALISVPIWIYLGEYGAHNIDWLMAKMHSLQSGIFVI
                   130       140       150       160       170       180

                   190       200       210       220
orf78a.pep  LGVLAAALAWFWWRKRRHYQLYRAQLSEKRAKRKAEKAAKKAAQKQQX
            ||: |:::||:||:||:: |:||::|:||||:||| ||||||||::||
orf78-1     LGIGATVVAWIWWKKRQRIQFYRSKLKEKRAQRKAAKAAKKAAQSKQX
                   190       200       210       220
```

Homology with a predicted ORF from *N.gonorrhoeae*

ORF78 shows 97.4% identity over 38 aa overlap with a predicted ORF (ORF78ng) from *N. gonorrhoeae:*

```
orf78.pep   XXLXFXPIAXIMTPXRYEQVQEKFDKYGNWVLFVARFLPGLRTAVFVTAGISRKVSYLRF  137
                                        ||||||||||||||||||||||||||||||
orf78ng                                 YPVLFVARFLPGLRTAVFVTAGISRKVSYLRF   32

orf78.pep   IIMDGLAA                                                      145
            :|||||||
orf78ng     LIMDGLAALISVPVWIYLGEYGAHNIDWLMAKMHSLQSGIFIALGVLAAALAWFWWRKRR   92
```

The ORF78ng nucleotide sequence <SEQ ID 725> is predicted to encode a protein comprising amino acid sequence <SEQ ID 726>:

```
  1  ..YPVLFVARFL PGLRTAVFVT AGISRKVSYL RFLIMDGLAA LISVPVWIYL
 51    GEYGAHNIDW LMAKMHSLQS GIFIALGVLA AALAWFWWRK RRHYQLYRAQ
101    LSEKRAKRKA EKAAKKAAQK QQ*
```

Further work revealed the complete gonococcal nucleotide sequence <SEQ ID 727>:

```
  1 atgtttgccc tttTggaagc CTTTTTTGTC GAAtacggCt atgcGGCCGT
 51 GTTTTTCGTT TTGGTCATCT GCGGTTTCGG CGTGCCGATT CCCGAAGATT
101 TGACCTTGGT AACGGGCGGC GTGATTTCGG GTATGGGTTA TACCAATCCG
151 CATATTATGT TTGCGGTCGG TATGCTCGGC GTGTTGGCGG GCGACGGCGT
201 GATGTTTGCC GCCGGACGCA TCTGGGGGCA GAAAATCCTC AAGTTCAAAC
251 CGATTGCGCG CATCATGACG CCGAAACGTT ACGCGCAGGT TCAGGAAAAA
301 TTCGACAAAT ACGGCAACTG GGTTCTGTTT GTCGCCCGTT TCCTGCCGGG
351 TTTGCGGACT GCCGTTTTCG TTACCGCCGG CATCAGCCGC AAAGTATCGT
401 ATCTGCGCTT TCTGATTATG GACGGGCTGG CCGCGCTGAT TTCCGTGCCC
451 GTTTGGATTT ACTTGGGCGA GTACGGCGCG CACAACATCG ATTGGCTGAT
501 GGCGAAAATG CACAGCCTGC AATCGGGCAT CTTCATCGCA TTGGGCGTGC
551 TGGCGGCGGC GCTGGCGTGG TTCTGGTGGC GCAAACGCCG ACATTATCAG
601 CTTTACCGCG CACAATTGAG CGAAAAACGC GCCAAACGCA AGGCGGAAAA
651 GGCAGCGAAA AAAGCGGCAC AGAAGCAGCA GTAa
```

This corresponds to the amino acid sequence <SEQ ID 728; ORF78ng-1>:

```
  1 MFALLEAFFV EYGYAAVFFV LVICGFGVPI PEDLTLVTGG VISGMGYTNP
 51 HIMFAVGMLG VLAGDGVMFA AGRIWGQKIL KFKPIARIMT PKRYAQVQEK
101 FDKYGNWVLF VARFLPGLRT AVFVTAGISR KVSYLRFLIM DGLAALISVP
151 VWIYLGEYGA HNIDWLMAKM HSLQSGIFIA LGVLAAALAW FWWRKRRHYQ
201 LYRAQLSEKR AKRKAEKAAK KAAQKQQ*
```

ORF78ng-1 and ORF78-1 show 88.1 % identity in 227 aa overlap:

```
                     10        20        30        40        50        60
orf78-1.pep  MFAFLEAFFVEYGYAAVFFVLVICGFGVPIPEDLTLVTGGVISGMGYTNPHIMFAVGMLG
             |||:||||||||||||||||||||||||||||||||||||||||||||||||||||||||
orf78ng-1    MFALLEAFFVEYGYAAVFFVLVICGFGVPIPEDLTLVTGGVISGMGYTNPHIMFAVGMLG
                     10        20        30        40        50        60

                     70        80        90       100       110       120
orf78-1.pep  VLVGDGIMFAAGRIWGQKILRFKPIARIMTPKRYEQVQEKFDKYGNWVLFVARFLPGLRT
             ||:|||:||||||||||||:|||||||||||| |||||||||||||||||||||||||||
orf78ng-1    VLAGDGVMFAAGRIWGQKILKFKPIARIMTPKRYAQVQEKFDKYGNWVLFVARFLPGLRT
                     70        80        90       100       110       120

                    130       140       150       160       170       180
orf78-1.pep  AVFVTAGISRKVSYLRFIIMDGLAALISVPIWIYLGEYGAHNIDWLMAKMHSLQSGIFVI
             |||||||||||||||||:||||||||||||:|||||||||||||||||||||||||||:
orf78ng-1    AVFVTAGISRKVSYLRFLIMDGLAALISVPVWIYLGEYGAHNIDWLMAKMHSLQSGIFIA
                    130       140       150       160       170       180

                    190       200       210       220
orf78-1.pep  LGIGATVVAWIWWKKRQRIQFYRSKLKEKRAQRKAAKAAKKAAQSKQX
             ||: |:::||:||:||:: |:||::|:||||:||| ||||||||::||
orf78ng-1    LGVLAAALAWFWWRKRRHYQLYRAQLSEKRAKRKAEKAAKKAAQKQQX
                    190       200       210       220
```

Furthermore, orf78ng-1 shows homology to the dedA protein from *H.influenzae:*

```
sp|P45280|YG29_HAEIN HYPOTHETICAL PROTEIN HI1629 >gi|1073983|pir||D64133 dedA
protein (dedA) homolog - Haemophilus influenzae (strain Rd KW20)
>gi|1574476 (U32836) dedA protein (dedA) [Haemophilus influenzae] Length = 212
 Score =  223 bits (563), Expect = 7e-58
 Identities = 108/182 (59%), Positives = 140/182 (76%), Gaps = 2/182 (1%)

Query: 5    LEAFFVEYGYAAVFFVLVICGFGVPIPEDLTLVTGGVISGM--GYTNPHIMFAVGMLGVL 62
            L  FF EYGY AV FVL+ICGFGVPIPED+TLV+GGVI+G+     N H+M  V M+GVL
Sbjct: 21   LIGFFTEYGYWAVLFVLIICGFGVPIPEDITLVSGGVIAGLYPENVNSHLMLLVSMIGVL 80

Query: 63   AGDGVMFAAGRIWGQKILKFKPIARIMTPKRYAQVQEKFDKYGNWVLFVARFLPGLRTAV 122
            AGD  M+  GRI+G KIL+F+PI RI+T +R   V+EKF +YGN VLFVARFLPGLR  +
Sbjct: 81   AGDSCMYWLGRIYGTKILRFRPIRRIVTLQRLRMVREKFSQYGNRVLFVARFLPGLRAPI 140

Query: 123  FVTAGISRKVSYLRFLIMDGLAALISVPVWIYLGEYGAHNIDWLMAKMHSLQSGIFIALG 182
            ++ +GI+R+VSY+RF+++D  AA+ISVP+WIYLGE GA N+DWL  ++   Q  I+I +G
Sbjct: 141  YMVSGITRRVSYVRFVLIDFCAAIISVPIWIYLGELGAKNLDWLHTQIQKGQIVIYIFIG 200

Query: 183  VL 184
             L
Sbjct: 201  YL 202
```

Based on this analysis, including the presence of putative transmembrane domains, it is predicted that these proteins from *N.meningitidis* and *N.gonorrhoeae,* and their epitopes, could be useful antigens for vaccines or diagnostics, or for raising antibodies.

## Example 87

The following partial DNA sequence was identified in *N.meningitidis* <SEQ ID 729>:

```
  1  ATGAAAAAAT TATTGGCGGC CGTGATGATG GCAGGTTTGG CAGGCGCGGT
 51  TTCCGCCGCC GGAGTCCACG TTGAGGACGG CTGGGCGCGC ACCACCGTCG
101  AAGGTATGAA AATAGGCGGC GCGTTCATGA AAATCCACAA CGACGAAGCC
151  AAACAAGACT TTTTGCTCGG CGGAAGCAGC CCCGTTGCCG ACCGCGTCGA
201  AGTGCATACC CACATCAACG ACAACGGCGT GATGCGGATG CGCGAAGTCG
251  AAGGCGGCGT GCCTTTGGAA GCGAAATCCG TTACCGAACT CAAACCCGGC
301  AGCTATCATG TGATGTTTAT GGGTTTGAAA AAACAATTAA AAGAGGGCGA
351  TAAAATTCCC GTTACCCTGA AATTTAAAAA CGCCAAAGCG CAAACCGTCC
401  AACTGGAAGT CAAAATCGCG CCGATGCCGG CAATGAACCA C...
```

This corresponds to the amino acid sequence <SEQ ID 730; ORF79>:

```
  1  MKKLLAAVMM AGLAGAVSAA GVHVEDGWAR TTVEGMKIGG AFMKIHNDEA
 51  KQDFLLGGSS PVADRVEVHT HINDNGVMRM REVEGGVPLE AKSVTELKPG
101  SYHVMFMGLK KQLKEGDKIP VTLKFKNAKA QTVQLEVKIA PMPAMNH..
```

Further work revealed the complete nucleotide sequence <SEQ ID 731>:

```
  1  ATGAAAAAAT TATTGGCGGC CGTGATGATG GCAGGTTTGG CAGGCGCGGT
 51  TTCCGCCGCC GGAGTCCACG TTGAGGACGG CTGGGCGCGC ACCACCGTCG
101  AAGGTATGAA AATAGGCGGC GCGTTCATGA AAATCCACAA CGACGAAGCC
151  AAACAAGACT TTTTGCTCGG CGGAAGCAGC CCCGTTGCCG ACCGCGTCGA
201  AGTGCATACC CACATCAACG ACAACGGCGT GATGCGGATG CGCGAAGTCG
251  AAGGCGGCGT GCCTTTGGAA GCGAAATCCG TTACCGAACT CAAACCCGGC
301  AGCTATCATG TGATGTTTAT GGGTTTGAAA AAACAATTAA AAGAGGGCGA
351  TAAAATTCCC GTTACCCTGA AATTTAAAAA CGCCAAAGCG CAAACCGTCC
401  AACTGGAAGT CAAAATCGCG CCGATGCCGG CAATGAACCA CGGTCATCAC
451  CACGGCGAAG CGCATCAGCA CTAA
```

This corresponds to the amino acid sequence <SEQ ID 732; ORF79-1>:

```
  1  MKKLLAAVMM AGLAGAVSAA GVHVEDGWAR TTVEGMKIGG AFMKIHNDEA
 51  KQDFLLGGSS PVADRVEVHT HINDNGVMRM REVEGGVPLE AKSVTELKPG
101  SYHVMFMGLK KQLKEGDKIP VTLKFKNAKA QTVQLEVKIA PMPAMNHGHH
151  HGEAHQH*
```

Computer analysis of this amino acid sequence revealed a putative leader peptide and also gave the following results:

<u>Homology with a predicted ORF from *N.meningitidis* (strain A)</u>

ORF79 shows 94.6% identity over a 147aa overlap with an ORF (ORF79a) from strain A of *N. meningitidis:*

```
                    10        20        30        40        50        60
orf79.pep   MKKLLAAVMMAGLAGAVSAAGVHVEDGWARTTVEGMKIGGAFMKIHNDEAKQDFLLGGSS
            || ||||||||||||||||||:|||||||||||||||:||||||||||||||||||||||
orf79a      MKXLLAAVMMAGLAGAVSAAGIHVEDGWARTTVEGMKMGGAFMKIHNDEAKQDFLLGGSS
                    10        20        30        40        50        60

                    70        80        90       100       110       120
orf79.pep   PVADRVEVHTHINDNGVMRMREVEGGVPLEAKSVTELKPGSYHVMFMGLKKQLKEGDKIP
            |||||||||||||||||||||||||||||||||||||||||||||||| ||||| |||||
orf79a      PVADRVEVHTHINDNGVMRMREVEGGVPLEAKSVTELKPGSYHVMFMGXKKQLKXGDKIP
                    70        80        90       100       110       120

                   130       140
orf79.pep   VTLKFKNAKAQTVQLEVKIAPMPAMNH
            |||||||||||||||||| ||| ||:|
orf79a      VTLKFKNAKAQTVQLEVKTAPMSAMDHGHHHGEAHQHX
                   130       140       150
```

The complete length ORF79a nucleotide sequence <SEQ ID 733> is:

```
  1  ATGAAANAAC TATTGGCAGC CGTGATGATG GCAGGTTTGG CAGGCGCGGT
 51  TTCCGCCGCC GGAATCCACG TTGAGGACGG CTGGGCGCGC ACCACCGTCG
101  AAGGTATGAA AATGGGCGGC GCGTTCATGA AAATCCACAA CGACGAAGCC
151  AAACAAGACT TTTTGCTCGG CGGAAGCAGC CCTGTTGCCG ACCGCGTCGA
201  AGTGCATACC CATATCAATG ATAACGGTGT GATGCGGATG CGCGAAGTCG
251  AAGGCGGCGT GCCTTTGGAG GCGAAATCCG TTACCGAACT CAAACCCGGC
301  AGCTATCATG TCATGTTTAT GGGTNTGAAA AAACAATTAA AAGANGGCGA
351  CAAGATTCCC GTTACCCTGA AATTTAAAAA CGCCAAAGCA CAAACCGTCC
401  AACTGGAAGT CAAAACCGCG CCGATGTCGG CAATGGACCA CGGTCATCAC
451  CACGGCGAAG CGCATCAGCA CTAA
```

This encodes a protein having amino acid sequence <SEQ ID 734>:

```
  1  MKXLLAAVMM AGLAGAVSAA GIHVEDGWAR TTVEGMKMGG AFMKIHNDEA
 51  KQDFLLGGSS PVADRVEVHT HINDNGVMRM REVEGGVPLE AKSVTELKPG
101  SYHVMFMGXK KQLKXGDKIP VTLKFKNAKA QTVQLEVKTA PMSAMDHGHH
151  HGEAHQH*
```

ORF79a and ORF79-1 show 94.9% identity in 157 aa overlap:

```
                    10        20        30        40        50        60
orf79a.pep  MKXLLAAVMMAGLAGAVSAAGIHVEDGWARTTVEGMKMGGAFMKIHNDEAKQDFLLGGSS
            || |||||||||||||||||:|||||||||||||||:|||||||||||||||||||||||
orf79-1     MKKLLAAVMMAGLAGAVSAAGVHVEDGWARTTVEGMKIGGAFMKIHNDEAKQDFLLGGSS
                    10        20        30        40        50        60

                    70        80        90       100       110       120
orf79a.pep  PVADRVEVHTHINDNGVMRMREVEGGVPLEAKSVTELKPGSYHVMFMGXKKQLKXGDKIP
            |||||||||||||||||||||||||||||||||||||||||||||||| ||||| |||||
orf79-1     PVADRVEVHTHINDNGVMRMREVEGGVPLEAKSVTELKPGSYHVMFMGLKKQLKEGDKIP
                    70        80        90       100       110       120

                   130       140       150
orf79a.pep  VTLKFKNAKAQTVQLEVKTAPMSAMDHGHHHGEAHQHX
            |||||||||||||||||| ||| ||:||||||||||||
orf79-1     VTLKFKNAKAQTVQLEVKIAPMPAMNHGHHHGEAHQHX
                   130       140       150
```

Homology with a predicted ORF from *N.gonorrhoeae*

ORF79 shows 96.1% identity over 76 aa overlap with a predicted ORF (ORF79ng) from *N.gonorrhoeae:*

```
orf79.pep   FMKIHNDEAKQDFLLGGSSPVADRVEVHTHINDNGVMRMREVEGGVPLEAKSVTELKPGS  101
                                          ||||||||||||:||||||||||||||||
orf79ng                                   INDNGVMRMREVKGGVPLEAKSVTELKPGS   30

orf79.pep   YHVMFMGLKKQLKEGDKIPVTLKFKNAKAQTVQLEVKIAPMPAMNH            147
            |||||||||||||||||||||||||||||||||||||| ||| ||||
orf79ng     YHVMFMGLKKQLKEGDKIPVTLKFKNAKAQTVQLEVKTAPMSAMNHGHHHGEAHQH    86
```

An ORF79ng nucleotide sequence <SEQ ID 735> was predicted to encode a protein comprising amino acid sequence <SEQ ID 736>:

```
  1  ..INDNGVMRMR EVKGGVPLEA KSVTELKPGS YHVMFMGLKK QLKEGDKIPV
 51    TLKFKNAKAQ TVQLEVKTAP MSAMNHGHHH GEAHQH*
```

Further work revealed the complete gonococcal DNA sequence <SEQ ID 737>:

```
  1  ATGAAAAAAT TATTGGCAGC CGTGATGATG GCAGGTTTGG CAGGCGCGGT
 51  TTccgccgCc GGagTccAtG TCGAggACGG CTGGGCGCGc accaCTGtcg
101  aaggtATgaa aatggGCGGC GCgttCATga aaATCCACAA CGACGaaGcc
151  atacaaGACt ttgtgcTCgg CGGaagcatg cccgttgccg accgcGTCGA
201  AGTGCAtaca cacATCAACG ACAACGGCGT GATGCGTATG CGCGAAGTCA
251  AAGGCGGCGT GCCTTTGGAG GCGAAATCCG TTACCGAACT CAAACCCGGC
301  AGCTATCACG TGATGTTTAT GGGTTTGAAA AAACAACTGA AAGAGGGCGA
351  CAAGATTCCC GTTACCCTGA AATTTAAAAA CGCCAAAGCG CAAACCGTCC
401  AACTGGAAGT CAAAACCGCG CCGATGTCGG CAATGAACCA CGGTCATCAC
451  CACGGCGAAG CGCATCAGCA CTAA
```

This corresponds to the amino acid sequence <SEQ ID 738; ORF79ng-1>:

```
  1  MKKLLAAVMM AGLAGAVSAA GVHVEDGWAR TTVEGMKMGG AFMKIHNDEA
 51  IQDFVLGGSM PVADRVEVHT HINDNGVMRM REVKGGVPLE AKSVTELKPG
101  SYHVMFMGLK KQLKEGDKIP VTLKFKNAKA QTVQLEVKTA PMSAMNHGHH
151  HGEAHQH*
```

ORF79ng-1 and ORF79-1 show 95.5% identity in 157 aa overlap:

```
                    10        20        30        40        50        60
orf79-1.pep  MKKLLAAVMMAGLAGAVSAAGVHVEDGWARTTVEGMKIGGAFMKIHNDEAKQDFLLGGSS
             |||||||||||||||||||||||||||||||||||||:|||||||||||| |||:|||| 
orf79ng-1    MKKLLAAVMMAGLAGAVSAAGVHVEDGWARTTVEGMKMGGAFMKIHNDEAIQDFVLGGSM
                    10        20        30        40        50        60

                    70        80        90       100       110       120
orf79-1.pep  PVADRVEVHTHINDNGVMRMREVEGGVPLEAKSVTELKPGSYHVMFMGLKKQLKEGDKIP
             |||||||||||||||||||||||:||||||||||||||||||||||||||||||||||||
orf79ng-1    PVADRVEVHTHINDNGVMRMREVKGGVPLEAKSVTELKPGSYHVMFMGLKKQLKEGDKIP
                    70        80        90       100       110       120

                   130       140       150
orf79-1.pep  VTLKFKNAKAQTVQLEVKIAPMPAMNHGHHHGEAHQHX
             |||||||||||||||||| ||| ||||||||||||||
orf79ng-1    VTLKFKNAKAQTVQLEVKTAPMSAMNHGHHHGEAHQHX
                   130       140       150
```

Furthermore, ORF79ng-1 shows significant homology to a protein from *Aquifex aeolicus:*

```
gi|2983695 (AE000731) putative protein [Aquifex aeolicus] Length = 151
 Score = 63.6 bits (152), Expect = 6e-10
 Identities = 38/114 (33%), Positives = 58/114 (50%), Gaps = 1/114 (0%)

Query: 24  VEDGWARTTVEGMKMGGAFMKIHNDEAIQDFVLGGSMPVADRVEVHTHINDNGVMRMREV 83
            V+  W       G       M I N+     D+++G      +A RVE+H   + +N V +M
Sbjct: 27  VKHPWVMEPPPGPNTTMMGMIIVNEGDEPDYLIGAKTDIAQRVELHKTVIENDVAKMVPQ 86

Query: 84  KGGVPLEAKSVTELKPGSYHVMFMGLKKQLKEGDKIPVTLKFKNAKAQTVQLEV 137
            +  + +  K    E K   YHVM +GLKK++KEGDK+ V L F+ +    TV+  V
Sbjct: 87  ER-IEIPPKGKVEFKHHGYHVMIIGLKKRIKEGDKVKVELIFEKSGKITVEAPV 139
```

Based on this analysis, it is predicted that the proteins from *N.meningitidis* and *N.gonorrhoeae,* and their epitopes, could be useful antigens for vaccines or diagnostics, or for raising antibodies.

ORF79-1 (15.6kDa) was cloned in the pET vector and expressed in *E.coli,* as described above. The products of protein expression and purification were analyzed by SDS-PAGE. Figure 18A shows the results of affinity purification of the His-fusion protein. Purified His-fusion protein was used to immunise mice, whose sera were used for ELISA (positive result) and FACS analysis (Figure 18B) These experiments confirm that ORF79-1 is a surface-exposed protein, and that it is a useful immunogen.

**Example 88**

The following DNA sequence, believed to be complete, was identified in *N.meningitidis* <SEQ ID 739>:

```
  1  ATGACGGTAA CTGCGGCCGA AGGCGGCAAA GCTGCCAAGG CGTTAAAAAA
 51  ATATCTGATT ACGGGCATTT TGGTCTGGCT GCCGATTGCG GTAACGGTTT
101  GGGTGGTTTC CTATATCGTT TCCGCGTCCG ATCAGCTCGT CAACCTGCTG
151  CCGAAGCAAT GGCGGCCGCA ATATGTTTTG GGGTTTAATA TCCCGGGGCT
201  GGGCGTTATC GTTGCCATTG CCGTATTGTT TGTAACCGGA TTGTTTGCCG
251  CCAACGTATT GGGTCGGCAG ATCCTCGCCG CGTGGGACAG CCTGTTGGGG
301  CGGATTCCGG TTGTGAAAtC CATCTATTCG AGTGTGAAAA AAGTATCCGA
351  ATacgTGCTG TCCGACAGCA GCCGTTCGTT TAAAACGCCG GTACTCGTGC
401  CGTTTCCCCA GCCCGGTATT TGGACGATyG CTTTCGTGTC AGGGCAGGTG
451  TCGAATGCGG TTAAGGCCGC ATTGCCGAAs GACGGCGATT ATCTTTCCGT
501  GTATGTTCCG ACCACGCCGA ATCCGACCGG CGGTTACTAT ATTATGGTAA
551  AGAAAAGCGA TGTGCGCGAA CTCGATATGA GCGTGGACGA AsCATTGAAA
601  TATGTGATTT CGCTGGGTAT GGTCATCCCT GACGACCTGC CCGTCAAAAC
651  ATTGGCAsGA CCTATGCCGT CTGAAAAGGC GGATTTGCCC GAACAACAAT
701  AA
```

This corresponds to the amino acid sequence <SEQ ID 740; ORF98>:

```
  1  MTVTAAEGGK AAKALKKYLI TGILVWLPIA VTVWVVSYIV SASDQLVNLL
 51  PKQWRPQYVL GFNIPGLGVI VAIAVLFVTG LFAANVLGRQ ILAAWDSLLG
101  RIPVVKSIYS SVKKVSEYVL SDSSRSFKTP VLVPFPQPGI WTIAFVSGQV

151  SNAVKAALPX DGDYLSVYVP TTPNPTGGYY IMVKKSDVRE LDMSVDEXLK
201  YVISLGMVIP DDLPVKTLAX PMPSEKADLP EQQ*
```

Further work revealed the complete nucleotide sequence <SEQ ID 741>:

```
  1  ATGACGGAAC nTGCGGCCGA AGGCGGCAAA GCTGCCAArG CGTTAAAAAA
 51  ATATCTGATT ACGGGCATTT TGGTCTGGCT GCCGATTGCG GTAACGGTTT
101  GGGTGGTTTC CTATATCGTT TCCGCGTCCG ATCAGCTCGT CAACCTGCTG
151  CCGAAGCAAT GGCGGCCGCA ATATGTTTTG GGGTTTAATA TCCCGGGGCT
201  GGGCGTTATC GTTGCCATTG CCGTATTGTT TGTAACCGGA TTGTTTGCCG
251  CCAACGTATT GGGTCGGCAG ATCCTCGCCG CGTGGGACAG CCTGTTGGGG
301  CGGATTCCGG TTGTGAAATC CATCTATTCG AGTGTGAAAA AAGTATCCGA
351  ATCGCTGCTG TCCGACAGCA GCCGTTCGTT TAAAACGCCG GTACTCGTGC
401  CGTTTCCCCA GCCCGGTATT TGGACGATTG CTTTCGTGTC AGGGCAGGTG
451  TCGAATGCGG TTAAGGCCGC ATTGCCGAAG GACGGCGATT ATCTTTCCGT
501  GTATGTTCCG ACCACGCCGA ATCCGACCGG CGGTTACTAT ATTATGGTAA
551  AGAAAAGCGA TGTGCGCGAA CTCGATATGA GCGTGGACGA AGCATTGAAA
601  TATGTGATTT CGCTGGGTAT GGTCATCCCT GACGACCTGC CCGTCAAAAC
651  ATTGGCAGGA CCTATGCCGT CTGAAAAGGC GGATTTGCCC GAACAACAAT
701  AA
```

This corresponds to the amino acid sequence <SEQ ID 742; ORF98-1>:

```
  1  MTEXAAEGGK AAKALKKYLI TGILVWLPIA VTVWVVSYIV SASDQLVNLL
 51  PKQWRPQYVL GFNIPGLGVI VAIAVLFVTG LFAANVLGRQ ILAAWDSLLG
101  RIPVVKSIYS SVKKVSESLL SDSSRSFKTP VLVPFPQPGI WTIAFVSGQV
151  SNAVKAALPK DGDYLSVYVP TTPNPTGGYY IMVKKSDVRE LDMSVDEALK
201  YVISLGMVIP DDLPVKTLAG PMPSEKADLP EQQ*
```

Computer analysis of this amino acid sequence gave the following results:

Homology with a predicted ORF from *N.meningitidis* (strain A)

ORF98 shows 96.1 % identity over a 233aa overlap with an ORF (ORF98a) from strain A of *N. meningitidis:*

```
                      10        20        30        40        50        60
orf98.pep    MTVTAAEGGKAAKALKKYLITGILVWLPIAVTVWVVSYIVSASDQLVNLLPKQWRPQYVL
             ||  ||||||||||||||||||||||||||||||||||||||||||||||||||||||||
orf98a       MTEPAAEGGKAAKALKKYLITGILVWLPIAVTVWVVSYIVSASDQLVNLLPKQWRPQYVL
                      10        20        30        40        50        60

                      70        80        90       100       110       120
orf98.pep    GFNIPGLGVIVAIAVLFVTGLFAANVLGRQILAAWDSLLGRIPVVKSIYSSVKKVSEYVL
             |||||||||||||||||||||||||||||||||||||||||||||||||||||||| :|
orf98a       GFNIPGLGVIVAIAVLFVTGLFAANVLGRQILAAWDSLLGRIPVVKSIYSSVKKVSXSLL
                      70        80        90       100       110       120

                     130       140       150       160       170       180
orf98.pep    SDSSRSFKTPVLVPFPQPGIWTIAFVSGQVSNAVKAALPXDGDYLSVYVPTTPNPTGGYY
             |||||||||||||||| ||||||||||||||||||||| |||||||||||||||||||||
orf98a       SDSSRSFKTPVLVPFPQSGIWTIAFVSGQVSNAVKAALPKDGDYLSVYVPTTPNPTGGYY
                     130       140       150       160       170       180

                     190       200       210       220       230
orf98.pep    IMVKKSDVRELDMSVDEXLKYVISLGMVIPDDLPVKTLAXPMPSEKADLPEQQX
             ||||||||||||||||| |||||||||||||||||||||| ||||||||||||||
orf98a       IMVKKSDVRELDMSVDEALKYVISLGMVIPDDLPVKTLAGPMPSEKADLPEQQX
                     190       200       210       220       230
```

The complete length ORF98a nucleotide sequence <SEQ ID 743> is:

```
  1 ATGACGGAAC CTGCGGCCGA AGGCGGCAAA GCTGCCAAGG CGTTAAAAAA
 51 ATATCTGATT ACGGGCATTT TGGTCTGGCT GCCGATTGCG GTAACGGTTT
101 GGGTGGTTTC CTATATCGTT TCCGCGTCCG ATCAGCTCGT CAACCTGCTG
151 CCGAAGCAAT GGCGGCCGCA ATATGTTTTG GGGTTTAATA TCCCGGGGCT
201 GGGCGTTATC GTTGCCATTG CCGTATTGTT TGTAACCGGA TTATTTGCCG
251 CAAACGTATT GGGCCGGCAG ATTCTTGCCG CGTGGGACAG CTTGTTGGGG
301 CGGATTCCGG TTGTGAAGTC CATCTATTCG AGTGTGAAAA AAGTATCCGA

351 NTCGTTGCTG TCCGACAGCA GCCGTTCGTT TAAAACACCA GTACTCGTGC
401 CGTTTCCCCA ATCGGGTATT TGGACAATCG CATTCGTGTC CGGTCAGGTG
451 TCGAATGCGG TTAAGGCCGC ATTGCCGAAG GACGGCGATT ATCTTTCCGT
501 GTATGTTCCG ACCACGCCGA ATCCGACCGG CGGTTACTAT ATTATGGTAA
551 AGAAAAGCGA TGTGCGCGAA CTCGATATGA GCGTGGACGA AGCGTTGAAA
601 TATGTGATTT CGCTGGGTAT GGTCATCCCT GACGACCTGC CCGTCAAAAC
651 ATTGGCAGGA CCTATGCCGT CTGAAAAGGC GGATTTGCCC GAACAACAAT
701 AA
```

This encodes a protein having amino acid sequence <SEQ ID 744>:

```
  1 MTEPAAEGGK AAKALKKYLI TGILVWLPIA VTVWVVSYIV SASDQLVNLL
 51 PKQWRPQYVL GFNIPGLGVI VAIAVLFVTG LFAANVLGRQ ILAAWDSLLG
101 RIPVVKSIYS SVKKVSXSLL SDSSRSFKTP VLVPFPQSGI WTIAFVSGQV
151 SNAVKAALPK DGDYLSVYVP TTPNPTGGYY IMVKKSDVRE LDMSVDEALK
201 YVISLGMVIP DDLPVKTLAG PMPSEKADLP EQQ*
```

ORF98a and ORF98-1 show 98.7% identity in 233 aa overlap:

```
                  10        20        30        40        50        60
orf98a.pep  MTEPAAEGGKAAKALKKYLITGILVWLPIAVTVWVVSYIVSASDQLVNLLPKQWRPQYVL
            ||| ||||||||||||||||||||||||||||||||||||||||||||||||||||||||
orf98-1     MTEXAAEGGKAAKALKKYLITGILVWLPIAVTVWVVSYIVSASDQLVNLLPKQWRPQYVL
                  10        20        30        40        50        60

                  70        80        90       100       110       120
orf98a.pep  GFNIPGLGVIVAIAVLFVTGLFAANVLGRQILAAWDSLLGRIPVVKSIYSSVKKVSXSLL
            ||||||||||||||||||||||||||||||||||||||||||||||||||||||||| |||
orf98-1     GFNIPGLGVIVAIAVLFVTGLFAANVLGRQILAAWDSLLGRIPVVKSIYSSVKKVSESLL
                  70        80        90       100       110       120

                 130       140       150       160       170       180
orf98a.pep  SDSSRSFKTPVLVPFPQSGIWTIAFVSGQVSNAVKAALPKDGDYLSVYVPTTPNPTGGYY
            ||||||||||||||||| ||||||||||||||||||||||||||||||||||||||||||
orf98-1     SDSSRSFKTPVLVPFPQPGIWTIAFVSGQVSNAVKAALPKDGDYLSVYVPTTPNPTGGYY
                 130       140       150       160       170       180

                 190       200       210       220       230
orf98a.pep  IMVKKSDVRELDMSVDEALKYVISLGMVIPDDLPVKTLAGPMPSEKADLPEQQX
            ||||||||||||||||||||||||||||||||||||||||||||||||||||||
orf98-1     IMVKKSDVRELDMSVDEALKYVISLGMVIPDDLPVKTLAGPMPSEKADLPEQQX
                 190       200       210       220       230
```

Homology with a predicted ORF from *N.gonorrhoeae*

ORF98 shows 95.3% identity over a 233 aa overlap with a predicted ORF (ORF98ng) from *N.gonorrhoeae:*

```
                  10        20        30        40        50        60
orf98.pep   MTVTAAEGGKAAKALKKYLITGILVWLPIAVTVWVVSYIVSASDQLVNLLPKQWRPQYVL   60
            ||  ||||||||||||||||||||||||||||||||||||||||||||||||||||||||
orf98ng     MTEPAAEGGKAAKALKKYLITGILVWLPIAVTVWVVSYIVSASDQLVNLLPKQWRPQYVL   60

orf98.pep   GFNIPGLGVIVAIAVLFVTGLFAANVLGRQILAAWDSLLGRIPVVKSIYSSVKKVSEYVL  120
            ||||||||||||||||||||||||||||||||||||||| ||||||||||||||||| :|
orf98ng     GFNIPGLGVIVAIAVLFVTGLFAANVLGRQILAAWDSLLXRIPVVKSIYSSVKKVSESLL  120

orf98.pep   SDSSRSFKTPVLVPFPQPGIWTIAFVSGQVSNAVKAALPXDGDYLSVYVPTTPNPTGGYY  180
            ||||||||||||||||| ||||||||||||||||||||| ||||||||||||||||||||
orf98ng     SDSSRSFKTPVLVPFPQSGIWTIAFVSGQVSNAVKAALPQDGDYLSVYVPTTPNPTGGYY  180

orf98.pep   IMVKKSDVRELDMSVDEXLKYVISLGMVIPDDLPVKTLAXPMPSEKADLPEQQ  233
            ||||||||||||||||| |||||||||||||||||||||| ||| |||:|||||
orf98ng     IMVKKSDVRELDMSVDEALKYVISLGMVIPDDLPVKTLAGPMPPEKAELPEQQ  233
```

The complete length ORF98ng nucleotide sequence <SEQ ID 745> is predicted to encode a protein having amino acid sequence <SEQ ID 746>:

```
  1  MTEPAAEGGK AAKALKKYLI TGILVWLPIA VTVWVVSYIV SASDQLVNLL
 51  PKQWRPQYVL GFNIPGLGVI VAIAVLFVTG LFAANVLGRQ ILAAWDSLLX
101  RIPVVKSIYS SVKKVSESLL SDSSRSFKTP VLVPFPQSGI WTIAFVSGQV
151  SNAVKAALPQ DGDYLSVYVP TTPNPTGGYY IMVKKSDVRE LDMSVDEALK
201  YVISLGMVIP DDLPVKTLAG PMPPEKAELP EQQ*
```

Further work revealed the complete nucleotide sequence <SEQ ID 747>:

```
  1 ATGACGGAAC CTGCGGCCGA AGGCGGCAAA GCTGCCAAGG CGTTAAAAAA
 51 ATATCTGATT ACAGGCATTT TGGTCTGGCT GCCGATTGCG GTAACGGTTT
101 GGGTGGTTTC CTATATCGTT TCCGCGTCCG ACCAGCTTGT CAACCTGCTG
151 CCGAAGCAAT GGCGGCCGCA ATATGTTTTG GGGTTTAATA TCCCCGGGCT
201 CGGCGTTATT GTTGCCATTG CCGTATTGTT TGTAACCGGA TTATTTGCCG
251 CAAACGTGTT GGGCCGGCAG ATTCTTGCCG CGTGGGACAG CCTGTTgggg
301 cggaTTCCGG TTGTCAAATC CATCTATTCG AGTGTGAAAA AAGTATCCGA
351 ATCGCTGCTG TCCGACAGCA GCCGTTCGTT TAAAACGCCG GTACTCGTGC
401 CGTTTCCCCA ATCGGGTATT TGGACAATCG CATTCGTGTC CGGTCAGGTG
451 TCGAATGCGG TTAAGGCCGC ATTGCCGCAG GATGGCGATT ATCTTTCCGT
501 GTATGTCCCG ACCACGCCCA ACCCGACCGG CGGTTACTAT ATTATGGTAA
551 AGAAAAGCGA TGTGCGCGAA CTCGATATGA GCGTGGACGA AGCGTTGAAA
601 TATGTGATTT CGCTGGGTAT GGTCATCCCT GACGACCTGC CCGTCAAAAC
651 ATTGGCAGGA CCTATGCCGC CTGAAAAGGC GGAGTTGCCC GAACAACAAT
701 AA
```

This corresponds to the amino acid sequence <SEQ ID 748; ORF98ng-1>:

```
  1 MTEPAAEGGK AAKALKKYLI TGILVWLPIA VTVWVVSYIV SASDQLVNLL
 51 PKQWRPQYVL GFNIPGLGVI VAIAVLFVTG LFAANVLGRQ ILAAWDSLLG
101 RIPVVKSIYS SVKKVSESLL SDSSRSFKTP VLVPFPQSGI WTIAFVSGQV
151 SNAVKAALPQ DGDYLSVYVP TTPNPTGGYY IMVKKSDVRE LDMSVDEALK
201 YVISLGMVIP DDLPVKTLAG PMPPEKAELP EQQ*
```

ORF98ng-1 and ORF98-1 show 97.9% identity in 233 aa overlap:

```
                     10        20        30        40        50        60
orf98-1.pep  MTEXAAEGGKAAKALKKYLITGILVWLPIAVTVWVVSYIVSASDQLVNLLPKQWRPQYVL
             ||| ||||||||||||||||||||||||||||||||||||||||||||||||||||||||
orf98ng-1    MTEPAAEGGKAAKALKKYLITGILVWLPIAVTVWVVSYIVSASDQLVNLLPKQWRPQYVL
                     10        20        30        40        50        60

                     70        80        90       100       110       120
orf98-1.pep  GFNIPGLGVIVAIAVLFVTGLFAANVLGRQILAAWDSLLGRIPVVKSIYSSVKKVSESLL
             ||||||||||||||||||||||||||||||||||||||||||||||||||||||||||||
orf98ng-1    GFNIPGLGVIVAIAVLFVTGLFAANVLGRQILAAWDSLLGRIPVVKSIYSSVKKVSESLL
                     70        80        90       100       110       120

                    130       140       150       160       170       180
orf98-1.pep  SDSSRSFKTPVLVPFPQPGIWTIAFVSGQVSNAVKAALPKDGDYLSVYVPTTPNPTGGYY
             ||||||||||||||||| ||||||||||||||||||||:|||||||||||||||||||||
orf98ng-1    SDSSRSFKTPVLVPFPQSGIWTIAFVSGQVSNAVKAALPQDGDYLSVYVPTTPNPTGGYY
                    130       140       150       160       170       180

                    190       200       210       220       230
orf98-1.pep  IMVKKSDVRELDMSVDEALKYVISLGMVIPDDLPVKTLAGPMPSEKADLPEQQX
             ||||||||||||||||||||||||||||||||||||||||||| |||:||||||
orf98ng-1    IMVKKSDVRELDMSVDEALKYVISLGMVIPDDLPVKTLAGPMPPEKAELPEQQX
                    190       200       210       220       230
```

Based on this analysis, including the fact that the putative transmembrane domains in the gonococcal protein are identical to the sequences in the meningococcal protein, it is predicted that the proteins from *N.meningitidis* and *N.gonorrhoeae,* and their epitopes, could be useful antigens for vaccines or diagnostics, or for raising antibodies.

**Example 89**

The following partial DNA sequence was identified in *N.meningitidis* <SEQ ID 749>:

```
   1  ATgAAAACGG TAGTCTGGAT TGTCGTCCTG TTTGCCGCCG CCGTCGGACT
  51  GGCGCTGGCT TCGGGCATTT ACACCGGCGA CGTGTATATC GTACTCGGAC
 101  AGACCATGCT CAGAATCAAC CTGCACGCCT TTGTGTTAGG TTCGCTGATT
 151  GCCGTCGTGG TGTGGTATTT CTTGTTTAAA TTCATTATCG GsGgTACTCA
 201  ATATCCCCGA AAAGATGCAG CGTTTCGGTT CGGCnCGTAA AGGCCkCAAG
 251  ssCGsGCTTG CCTTGAACAA GGCGGGTTTG GCGTATTTTG AAGGGCGTTT
 301  TGAAAAGGCG GAACTAGAAG CCTCACGCGT GTTGGTCAAC AAAGtAGGCC
 351  GaGAGACAAC CGGACTTTGG CATTGATGCT GrGCGCGCAC GCCGCCGGAC
 401  AGATGGAAAA CATCGAssTG CGCGACCGTT ATCTTGCGGA AATCGCCAAA
 451  CTGCCGGAAA AACAGCAGCT TTCCCGTTAT CTTTTGTTGG CGGAATCGGC
 501  GTTGAACCGG CGCGATTACG AAGCGGCGGA AGCCAATCTT CATGCGGCGG
 551  CGAAGATGAA TGCCAACCTT ACGCGCCTCG TGCGTCTGCA .ATTCGTTAC
 601  GCTTTCGACA GGGGCGACGC GTTGCAGGTT CTGGCAAAAA CCGAAAAACT
 651  TTCCAAGGCG GGCGCGTTGG GCAAATCGGA AATGGAACGG TATCAAAATT
 701  GGGCATATCC GTCGCCAGCT GGCGGATGCT GCCGATGCCG CCGCTTTGAA
 751  AACCTGCCTG AAGCGGATTC CCGACAGCCT CAAAAACGGG GAATTGAGCG
 801  TATCGGTTGC GGAAAAGTAC GAACGTTTGG GACTGTATGC CGATGCGGTC
 851  AAATGGGTCA AACAGCATTA TCCGCAsAAC CGCCGCCCCG AGCTTTTGGA
 901  AGCCTTTGTC GAAAGCGTGC GCTTTTTGGG CGAGCGCGAA CAGCAGAAAG
 951  CCATCGATTT TGCCGATGCT TGGCTGAAAG AACAGCCCGA TAACGCGCTT
1001  CTGCTGATGT ATCTCGGTCG GCTCGCCTTC GGCCGCAAAC TTTGGGGCAA
1051  GGCAAAAGGC TACCTTGAAG CGAGCATTGC ATTAAAGCCG AGTATTTCCG
1101  CGCGTTTGGT TCTAACAAAG GTTTTCGACG AAATCGGAGA ACCGCAGAAG
1151  GCGGAGGCGC AC...
```

This corresponds to the amino acid sequence <SEQ ID 750; ORF100>:

```
  1  MKTVVWIVVL FAAAVGLALA SGIYTGDVYI VLGQTMLRIN LHAFVLGSLI
 51  AVVVWYFLFK FIIGVLNIPE KMQRFGSARK GXKXXLALNK AGLAYFEGRF
101  EKAELEASRV LVNKVGRDNR TLALMLXAHA AGQMENIXXR DRYLAEIAKL
151  PEKQQLSRYL LLAESALNRR DYEAAEANLH AAAKMNANLT RLVRLXIRYA
201  FDRGDALQVL AKTEKLSKAG ALGKSEMERY QNWAYRRQLA DAADAAALKT
251  CLKRIPDSLK NGELSVSVAE KYERLGLYAD AVKWVKQHYP XNRRPELLEA
301  FVESVRFLGE REQQKAIDFA DAWLKEQPDN ALLLMYLGRL AFGRKLWGKA
351  KGYLEASIAL KPSISARLVL TKVFDEIGEP QKAEAH...
```

Further work revealed the complete nucleotide sequence <SEQ ID 751>:

```
   1  ATGAAAACGG TAGTCTGGAT TGTCGTCCTG TTTGCCGCCG CCGTCGGACT
  51  GGCGCTGGCT TCGGGCATTT ACACCGGCGA CGTGTATATC GTACTCGGAC
 101  AGACCATGCT CAGAATCAAC CTGCACGCCT TTGTGTTAGG TTCGCTGATT
 151  GCCGTCGTGG TGTGGTATTT CTTGTTTAAA TTCATTATCG GCGTACTCAA
 201  TATCCCCGAA AAGATGCAGC GTTTCGGTTC GGCGCGTAAA GGCCGCAAGG
 251  CCGCGCTTGC CTTGAACAAG GCGGGTTTGG CGTATTTTGA AGGGCGTTTT
 301  GAAAAGGCGG AACTAGAAGC CTCACGCGTG TTGGTCAACA AAGAGGCCGG
 351  AGACAACCGG ACTTTGGCAT TGATGCTGGG CGCGCACGCC GCCGGACAGA
 401  TGGAAAACAT CGAGCTGCGC GACCGTTATC TTGCGGAAAT CGCCAAACTG
 451  CCGGAAAAAC AGCAGCTTTC CCGTTATCTT TTGTTGGCGG AATCGGCGTT
 501  GAACCGGCGC GATTACGAAG CGGCGGAAGC CAATCTTCAT GCGGCGGCGA
 551  AGATGAATGC CAACCTTACG CGCCTCGTGC GTCTGCAACT TCGTTACGCT
 601  TTCGACAGGG GCGACGCGTT GCAGGTTCTG GCAAAAACCG AAAAACTTTC
 651  CAAGGCGGGC GCGTTGGGCA AATCGGAAAT GGAACGGTAT CAAAATTGGG
 701  CATACCGCCG CCAGCTGGCG GATGCTGCCG ATGCCGCCGC TTTGAAAACC
 751  TGCCTGAAGC GGATTCCCGA CAGCCTCAAA AACGGGGAAT TGAGCGTATC
 801  GGTTGCGGAA AAGTACGAAC GTTTGGGACT GTATGCCGAT GCGGTCAAAT
 851  GGGTCAAACA GCATTATCCG CACAACCGCC GCCCCGAGCT TTTGGAAGCC
 901  TTTGTCGAAA GCGTGCGCTT TTTGGGCGAG CGCGAACAGC AGAAAGCCAT
 951  CGATTTTGCC GATGCTTGGC TGAAAGAACA GCCCGATAAC GCGCTTCTGC
1001  TGATGTATCT CGGTCGGCTC GCCTACGGCC GCAAACTTTG GGCAAGGCA
1051  AAAGGCTACC TTGAAGCGAG CATTGCATTA AAGCCGAGTA TTTCCGCGCG
1101  TTTGGTTCTA GCAAAGGTTT TCGACGAAAT CGGAGAACCG CAGAAGGCGG
1151  AGGCGCAGCG CAACTTGGTT TTGGAAGCCG TCTCCGATGA CGAACGTCAC
1201  GCAGCGTTAG AGCAGCATAG CTGA
```

This corresponds to the amino acid sequence <SEQ ID 752; ORF100-1>:

```
  1  MKTVVWIVVL FAAAVGLALA SGIYTGDVYI VLGQTMLRIN LHAFVLGSLI
 51  AVVVWYFLFK FIIGVLNIPE KMQRFGSARK GRKAALALNK AGLAYFEGRF
101  EKAELEASRV LVNKEAGDNR TLALMLGAHA AGQMENIELR DRYLAEIAKL
151  PEKQQLSRYL LLAESALNRR DYEAAEANLH AAAKMNANLT RLVRLQLRYA
201  FDRGDALQVL AKTEKLSKAG ALGKSEMERY QNWAYRRQLA DAADAAALKT

251  CLKRIPDSLK NGELSVSVAE KYERLGLYAD AVKWVKQHYP HNRRPELLEA
301  FVESVRFLGE REQQKAIDFA DAWLKEQPDN ALLLMYLGRL AYGRKLWGKA
351  KGYLEASIAL KPSISARLVL AKVFDEIGEP QKAEAQRNLV LEAVSDDERH
401  AALEQHS*
```

Computer analysis of this amino acid sequence gave the following results:

Homology with a predicted ORF from *N.meningitidis* strain A)

ORF100 shows 93.5% identity over a 386aa overlap with an ORF (ORF100a) from strain A of *N. meningitidis:*

```
                    10        20        30        40        50        60
orf100.pep  MKTVVWIVVLFAAAVGLALASGIYTGDVYIVLGQTMLRINLHAFVLGSLIAVVVWYFLFK
            |||||||||||||| |||||||| ||||||||||||||||||||||||||||||||||||
orf100a     MKTVVWIVVLFAAAXGLALASGIXTGDVYIVLGQTMLRINLHAFVLGSLIAVVVWYFLFK
                    10        20        30        40        50        60

                    70        80        90       100       110       120
orf100.pep  FIIGVLNIPEKMQRFGSARKGXKXXLALNKAGLAYFEGRFEKAELEASRVLVNKVGRDNR
            ||||||| |||||||||||||| |  |||||||||||||||||||||||||| || : |||
orf100a     FIIGVLNXPEKMQRFGSARKGRKAALALNKAGLAYFEGRFEKAELEASRVLGNKEAGDNR
                    70        80        90       100       110       120

                   130       140       150       160       170       180
orf100.pep  TLALMLXAHAAGQMENIXXRDRYLAEIAKLPEKQQLSRYLLLAESALNRRDYEAAEANLH
            |||||| ||||||||||  |||||||||||||||||||||||||||||||||||||||||
orf100a     TLALMLGAHAAGQMENIELRDRYLAEIAKLPEKQQLSRYLLLAESALNRRDYEAAEANLH
                   130       140       150       160       170       180

                   190       200       210       220       230       240
orf100.pep  AAAKMNANLTRLVRLXIRYAFDRGDALQVLAKTEKLSKAGALGKSEMERYQNWAYRRQLA
            |||||||||||||||| :||||||||||||||||||| ||||| |||||||||||||||| 
orf100a     AAAKMNANLTRLVRLQLRYAFDRGDALQVLAKTEKXSKAGAXGKSEMERYQNWAYRRQLX
                   190       200       210       220       230       240

                   250       260       270       280       290       300
orf100.pep  DAADAAALKTCLKRIPDSLKNGELSVSVAEKYERLGLYADAVKWVKQHYPXNRRPELLEA
            |||||||||||||||||||||||||||||||||||||||||||||||||| |||||||||
orf100a     DAADAAALKTCLKRIPDSLKNGELSVSVAEKYERLGLYADAVKWVKQHYPHNRRPELLEA
                   250       260       270       280       290       300

                   310       320       330       340       350       360
orf100.pep  FVESVRFLGEREQQKAIDFADAWLKEQPDNALLLMYLGRLAFGRKLWGKAKGYLEASIAL
            |||||||||||:||||||||||||||||||||||| ||||||:|||||||||||||||||
orf100a     FVESVRFLGERDQQKAIDFADAWLKEQPDNALLLXYLGRLAYGRKLWGKAKGYLEASIAL
                   310       320       330       340       350       360

                   370       380
orf100.pep  KPSISARLVLTKVFDEIGEPQKAEAH
            ||||||||||:||||| ||||||||:
orf100a     KPSISARLVLAKVFDETGEPQKAEAQRNLVLASVAEENRPSAETHX
                   370       380       390       400
```

The complete length ORF100a nucleotide sequence <SEQ ID 753> is:

```
   1  ATGAAAACGG TAGTCTGGAT TGTCGTCCTG TTTGCCGCCG CNNTCGGGCT
  51  GGCATTGGCG TCGGGCATTN ACACCGGCGA CGTGTATATC GTACTCGGAC
 101  AGACCATGCT CAGAATCAAC CTGCACGCCT TTGTGTTAGG TTCGCTGATT
 151  GCCGTCGTGG TGTGGTATTT CCTGTTCAAA TTCATCATCG GCGTACTCAA
 201  TANCCCCGAA AAGATGCAGC GTTTCGGTTC GGCGCGTAAA GGCCGCAAGG
 251  CCGCGCTTGC TTTGAACAAG GCGGGTTTGG CGTATTTTGA AGGGCGTTTT
 301  GAAAAGGCGG AACTTGAAGC CTCGCGCGTA TTGGGAAACA AAGAGGCGGG
 351  GGATAACCGG ACTTTGGCAT TGATGTTGGG CGCACATGCC GCCGGGCAGA
 401  TGGAAAACAT CGAGCTGCGC GACCGTTATC TTGCGGAAAT CGCCAAACTG
 451  CCGGAAAAGC AGCAGCTTTC CCGTTATCTT TTGTTGGCGG AATCGGCGTT
 501  GAACCGGCGC GATTACGAAG CGGCGGAAGC CAATCTTCAT GCGGCGGCGA
 551  AGATGAATGC CAACCTTACG CGCCTCGTGC GTCTGCAACT TCGTTACGCT
 601  TTCGACAGGG GCGACGCGTT GCAGGTTCTG GCAAAAACCG AAAAANTTTC
 651  CAAGGCGGGC GCGTNGGGCA AATCGGAAAT GGAACGGTAT CAAAATTGGG
```

```
 701  CATACCGCCG CCAGCTGNCG GATGCTGCCG ATGCCGCCGC TTTGAAAACC
 751  TGCCTGAAGC GGATTCCCGA CAGCCTCAAA AACGGGGAAT TGAGCGTATC
 801  GGTTGCGGAA AAGTACGAAC GTTTGGGACT GTATGCCGAT GCGGTCAAAT
 851  GGGTCAAACA GCATTATCCG CACAACCGCC GACCCGAACT TTTGGAAGCN
 901  TTTGTCGAAA GCGTGCGCTT TTTGGGCGAA CGCGATCAGC AGAAAGCCAT
 951  CGATTTTGCC GATGCTTGGC TGAAAGAACA GCCCGATAAT GCGCTTCTGC
1001  TGANGTATCT CGGTCGGCTC GCCTACGGCC GCAAACTTTG GGGCAAGGCA
1051  AAAGGCTACC TTGAAGCGAG CATTGCATTA AAGCCGAGTA TTTCCGCGCG
1101  TTTGGTTCTG GCAAAGGTTT TTGACGAAAC CGGAGAACCG CAGAAGGCGG
1151  AGGCGCAGCG CAACTTGGTT TTGGCAAGCG TTGCCGAGGA AAACCGNCCT
1201  TCCGCCGAAA CCCATTGA
```

This encodes a protein having amino acid sequence <SEQ ID 754>:

```
  1  MKTVVWIVVL FAAAXGLALA SGIXTGDVYI VLGQTMLRIN LHAFVLGSLI
 51  AVVVWYFLFK FIIGVLNXPE KMQRFGSARK GRKAALALNK AGLAYFEGRF
101  EKAELEASRV LGNKEAGDNR TLALMLGAHA AGQMENIELR DRYLAEIAKL
151  PEKQQLSRYL LLAESALNRR DYEAAEANLH AAAKMNANLT RLVRLQLRYA
201  FDRGDALQVL AKTEKXSKAG AXGKSEMERY QNWAYRRQLX DAADAAALKT
251  CLKRIPDSLK NGELSVSVAE KYERLGLYAD AVKWVKQHYP HNRRPELLEA
301  FVESVRFLGE RDQQKAIDFA DAWLKEQPDN ALLLXYLGRL AYGRKLWGKA
351  KGYLEASIAL KPSISARLVL AKVFDETGEP QKAEAQRNLV LASVAEENRP
401  SAETH*
```

ORF100a and ORF100-1 show 95.1% identity in 406 aa overlap:

```
                       10        20        30        40        50        60
orf100a.pep    MKTVVWIVVLFAAAXGLALASGIXTGDVYIVLGQTMLRINLHAFVLGSLIAVVVWYFLFK
               |||||||||||||| |||||||| ||||||||||||||||||||||||||||||||||||
orf100-1       MKTVVWIVVLFAAAVGLALASGIYTGDVYIVLGQTMLRINLHAFVLGSLIAVVVWYFLFK
                       10        20        30        40        50        60

                       70        80        90       100       110       120
orf100a.pep    FIIGVLNXPEKMQRFGSARKGRKAALALNKAGLAYFEGRFEKAELEASRVLGNKEAGDNR
               ||||||| |||||||||||||||||||||||||||||||||||||||||||| |||||||
orf100-1       FIIGVLNIPEKMQRFGSARKGRKAALALNKAGLAYFEGRFEKAELEASRVLVNKEAGDNR
                       70        80        90       100       110       120

                      130       140       150       160       170       180
orf100a.pep    TLALMLGAHAAGQMENIELRDRYLAEIAKLPEKQQLSRYLLLAESALNRRDYEAAEANLH
               ||||||||||||||||||||||||||||||||||||||||||||||||||||||||||||
orf100-1       TLALMLGAHAAGQMENIELRDRYLAEIAKLPEKQQLSRYLLLAESALNRRDYEAAEANLH
                      130       140       150       160       170       180

                      190       200       210       220       230       240
orf100a.pep    AAAKMNANLTRLVRLQLRYAFDRGDALQVLAKTEKXSKAGAXGKSEMERYQNWAYRRQLX
               ||||||||||||||||||||||||||||||||||| ||||| ||||||||||||||||| 
orf100-1       AAAKMNANLTRLVRLQLRYAFDRGDALQVLAKTEKLSKAGALGKSEMERYQNWAYRRQLA
                      190       200       210       220       230       240

                      250       260       270       280       290       300
orf100a.pep    DAADAAALKTCLKRIPDSLKNGELSVSVAEKYERLGLYADAVKWVKQHYPHNRRPELLEA
               ||||||||||||||||||||||||||||||||||||||||||||||||||||||||||||
orf100-1       DAADAAALKTCLKRIPDSLKNGELSVSVAEKYERLGLYADAVKWVKQHYPHNRRPELLEA
                      250       260       270       280       290       300

                      310       320       330       340       350       360
orf100a.pep    FVESVRFLGERDQQKAIDFADAWLKEQPDNALLLXYLGRLAYGRKLWGKAKGYLEASIAL
               ||||||||||:||||||||||||||||||||||| |||||||||||||||||||||||||
orf100-1       FVESVRFLGEREQQKAIDFADAWLKEQPDNALLLMYLGRLAYGRKLWGKAKGYLEASIAL
                      310       320       330       340       350       360

                      370       380       390       400
orf100a.pep    KPSISARLVLAKVFDETGEPQKAEAQRNLVLASVAEENRPSA-ETHX
               |||||||||||||||| ||||||||||||||  :|::::|  :|  |  |
orf100-1       KPSISARLVLAKVFDEIGEPQKAEAQRNLVLEAVSDDERHAALEQHSX
                      370       380       390       400
```

Homology with a predicted ORF from *N.gonorrhoeae*

ORF100 shows 93.3% identity over a 386 aa overlap with a predicted ORF (ORF100ng) from *N.gonorrhoeae:*

```
orf100.pep    MKTVVWIVVLFAAAVGLALASGIYTGDVYIVLGQTMLRINLHAFVLGSLIAVVVWYFLFK   60
              ||||||||||||||||||||||||||||||||||||||||||||||||||||||||||||
orf100ng      MKTVVWIVVLFAAAVGLALASGIYTGDVYIVLGQTMLRINLHAFVLGSLIAVVVWYFLFK   60

orf100.pep    FIIGVLNIPEKMQRFGSARKGXKXXLALNKAGLAYFEGRFEKAELEASRVLVNKVGRDNR  120
              ||||||||||:|:| |||||| |  |||||||||||||||||||||||||| || : |||
orf100ng      FIIGVLNIPENMRRSGSARKGRKAALALNKAGLAYFEGRFEKAELEASRVLGNKEAGDNR  120

orf100.pep    TLALMLXAHAAGQMENIXXRDRYLAEIAKLPEKQQLSRYLLLAESALNRRDYEAAEANLH  180
              |||||| ||||||||||  |||||||||||||||||||||||||||||||||||||||||
orf100ng      TLALMLGAHAAGQMENIELRDRYLAEIAKLPEKQQLSRYLLLAESALNRRDYEAAEANLH  180

orf100.pep    AAAKMNANLTRLVRLXIRYAFDRGDALQVLAKTEKLSKAGALGKSEMERYQNWAYRRQLA  240
              ||||||||||||||| :|||||||||||||||||||||||||||||||||||||||||:|
orf100ng      AAAKMNANLTRLVRLQLRYAFDRGDALQVLAKTEKLSKAGALGKSEMERYQNWAYRRQMA  240

orf100.pep    DAADAAALKTCLKRIPDSLKNGELSVSVAEKYERLGLYADAVKWVKQHYPXNRRPELLEA  300
              |||||||||||||||||||||||||||||||||||||||||||||||||| |||||||||
orf100ng      DAADAAALKTCLKRIPDSLKNGELSVSVAEKYERLGLYADAVKWVKQHYPHNRRPELLEA  300

orf100.pep    FVESVRFLGEREQQKAIDFADAWLKEQPDNALLLMYLGRLAFGRKLWGKAKGYLEASIAL  360
              |||||||||||||||||||||:|||||||||||||||||||:||||||||||||||||||
orf100ng      FVESVRFLGEREQQKAIDFADSWLKEQPDNALLLMYLGRLAYGRKLWGKAKGYLEASIAL  360

orf100.pep    KPSISARLVLTKVFDEIGEPQKAEAH                   386
              |||| |||||:||||| :: |||||:
orf100ng      KPSIPARLVLAKVFDETAQSQKAEAQRNLVLASVAGENRPSAETR  405
```

The complete length ORF100ng nucleotide sequence <SEQ ID 755> is:

```
   1  ATGAAAACGG TAGTCTGGAT TGTTGTCCTG TTTGCCGCCG CCGTCGGACT
  51  GGCGCTGGCT TCGGGCATTT ACACCGGCGA CGTGTATATC GTACTCGGAC
 101  AGACCATGCT CAGAATCAAC CTGCACGCCT TTGTGTTAGG TTCGCTGATT
 151  GCCGTCGTGG TGTGGTATTT CCTGTTTAAA TTCATCATCG GCGTACTCAA
 201  TATCCCCGAA AATATGCGGC GTTCCGGTTC GGCGCGGAAA GGCCGCAAGG
 251  CCGCGCTTGC CTTGAATAAG GCGGGTTTGG CGTATTTCGA AGGGCGTTTT
 301  GAAAAGGCGG AACTCGAAGC CTCTCGAGTG TTGGGCAACA AAGAGGCCGG
 351  AGACAACCGG ACTTTGGCAT TGATGCTGGG CGCGCACGCG GCAGGACAGA
 401  TGGAAAATAT CGAGCTGCGC GACCGTTATC TTGCGGAAAT CGCCAAACTG
 451  CCGGAAAAAC AGCAGCTTTC CCGCTATCTT CTGCTGGCGG AATCGGCGTT
 501  AAACCGGCGC GATTACGAAG CGGCGGAAGC CAATCTTCAT GCGGCGGCGA
 551  AGATGAATGC CAACCTTACG CGCCTCGTGC GTCTGCAACT TCGTTACGCC
 601  TTCGATCGGG GCGATGCGTT GCAGGTTCTG GCAAAAaccG AAAAACTTTC
 651  CAAGGCGGGC GCGTTGGGCA AATCGGAAAT GGAACGGTAT CAAAATTGGG
 701  CATACCGCCG CCAGATGGCG GATGCTGCCG ATGCCGCCGC TTTGAAAACC
 751  TGCCTGAAGC GGATTCCCGA CAGCCTCAAA AACGGGGAAT TGagcGTATC
 801  GGTTGCGGAA AAGTACGAAC GTTTGGGACT GTATGCCGAT GCGGTCAAAT
 851  GGGTCAAACA GCATTATCCG CACAACCGCC GCCCCGAGCT TTTGGAAGCC
 901  TTTGTCGAAA GCGTGCGCTT TTTGGGCGAG CGCGAACAGC AGAAAGCCAT
 951  CGATTTTGCC GATTCTTGGC TGAAAGAACA GCCCGATAAC GCGCTTCTGC
1001  TGATGTATCT CGGCCGGCTC GCCTACGGCC GCAAACTTTG GGGTAAGGCA
1051  AAAGGCTACC TTGAAGCGAG TATTGCACTG AAGCCGAGTA TTCCGGCGCG
1101  TTTGGTGTTG GCAAAGGTTT TTGACGAAAC CGCACAGTCG CAAAAAGCCG
1151  AAGCACAGCG CAACTTGGTT TTGGCAAGCG TTGCCGGGGA AAACCGCCCT
1201  TCCGCCGAAA CCCGTTGA
```

This encodes a protein having amino acid sequence <SEQ ID 756>:

```
  1  MKTVVWIVVL FAAAVGLALA SGIYTGDVYI VLGQTMLRIN LHAFVLGSLI
 51  AVVVWYFLFK FIIGVLNIPE NMRRSGSARK GRKAALALNK AGLAYFEGRF
101  EKAELEASRV LGNKEAGDNR TLALMLGAHA AGQMENIELR DRYLAEIAKL
151  PEKQQLSRYL LLAESALNRR DYEAAEANLH AAAKMNANLT RLVRLQLRYA
201  FDRGDALQVL AKTEKLSKAG ALGKSEMERY QNWAYRRQMA DAADAAALKT
251  CLKRIPDSLK NGELSVSVAE KYERLGLYAD AVKWVKQHYP HNRRPELLEA
301  FVESVRFLGE REQQKAIDFA DSWLKEQPDN ALLLMYLGRL AYGRKLWGKA
351  KGYLEASIAL KPSIPARLVL AKVFDETAQS QKAEAQRNLV LASVAGENRP
401  SAETR*
```

ORF100ng and ORF100-1 show 95.3% identity in 402 aa overlap:

```
                        10        20        30        40        50        60
orf100-1.pep    MKTVVWIVVLFAAAVGLALASGIYTGDVYIVLGQTMLRINLHAFVLGSLIAVVVWYFLFK
                ||||||||||||||||||||||||||||||||||||||||||||||||||||||||||||
orf100ng        MKTVVWIVVLFAAAVGLALASGIYTGDVYIVLGQTMLRINLHAFVLGSLIAVVVWYFLFK
                        10        20        30        40        50        60

                        70        80        90       100       110       120
orf100-1.pep    FIIGVLNIPEKMQRFGSARKGRKAALALNKAGLAYFEGRFEKAELEASRVLVNKEAGDNR
                ||||||||||:|:| |||||||||||||||||||||||||||||||||||||| ||||||
orf100ng        FIIGVLNIPENMRRSGSARKGRKAALALNKAGLAYFEGRFEKAELEASRVLGNKEAGDNR
                        70        80        90       100       110       120

                       130       140       150       160       170       180
orf100-1.pep    TLALMLGAHAAGQMENIELRDRYLAEIAKLPEKQQLSRYLLLAESALNRRDYEAAEANLH
                ||||||||||||||||||||||||||||||||||||||||||||||||||||||||||||
orf100ng        TLALMLGAHAAGQMENIELRDRYLAEIAKLPEKQQLSRYLLLAESALNRRDYEAAEANLH
                       130       140       150       160       170       180

                       190       200       210       220       230       240
orf100-1.pep    AAAKMNANLTRLVRLQLRYAFDRGDALQVLAKTEKLSKAGALGKSEMERYQNWAYRRQLA
                ||||||||||||||||||||||||||||||||||||||||||||||||||||||||||:|
orf100ng        AAAKMNANLTRLVRLQLRYAFDRGDALQVLAKTEKLSKAGALGKSEMERYQNWAYRRQMA
                       190       200       210       220       230       240

                       250       260       270       280       290       300
orf100-1.pep    DAADAAALKTCLKRIPDSLKNGELSVSVAEKYERLGLYADAVKWVKQHYPHNRRPELLEA
                ||||||||||||||||||||||||||||||||||||||||||||||||||||||||||||
orf100ng        DAADAAALKTCLKRIPDSLKNGELSVSVAEKYERLGLYADAVKWVKQHYPHNRRPELLEA
                       250       260       270       280       290       300

                       310       320       330       340       350       360
orf100-1.pep    FVESVRFLGEREQQKAIDFADAWLKEQPDNALLLMYLGRLAYGRKLWGKAKGYLEASIAL
                ||||||||||||||||||||:|||||||||||||||||||||||||||||||||||||||
orf100ng        FVESVRFLGEREQQKAIDFADSWLKEQPDNALLLMYLGRLAYGRKLWGKAKGYLEASIAL
                       310       320       330       340       350       360

                       370       380       390       400
orf100-1.pep    KPSISARLVLAKVFDEIGEPQKAEAQRNLVLEAVSDDERHAALEQHSX
                |||| |||||||||||  ::  ||||||||||  :|:  ::|  :|
orf100n        KPSIPARLVLAKVFDETAQSQKAEAQRNLVLASVAGENRPSAETRX
                       370       380       390       400
```

Based on this analysis, including the presence of a putative leader sequence, a putative transmembrane domain, and a RGD motif, it is predicted that the proteins from *N meningitidis* and *N.gonorrhoeae,* and their epitopes, could be useful antigens for vaccines or diagnostics, or for raising antibodies.

**Example 90**

The following DNA sequence, believed to be complete, was identified in *N. meningitidis* <SEQ ID 757>

```
  1  ATGATGTTTT CTTGGTTCAA GCTGTTTCAC TTGTTTTTTG TCATTTCGTG
 51  GTTTGCAGGG CTGTTTTACC TGCCGAGGAT TTTCGTCAAT ATGGCGATGA
101  TTGATGTGCC GCGCGGCAAT CCCGAGTATG TGCGTCTGTC GGGCATGGCG
151  GTGCGGCTGT ACCGTTTTAT GTCGCCGTTG GGCTTCGGCG CGGTCGTGTT
201  CGGCGCGGCG ATACCGTTTG CCGCCGGCTG GTGGGGCAGC GGCTGGGTAC
251  ACGTCAAACT GTGTTTGGGC TTGATGCTCT TGGCTTACCA GTTGTATTGC
301  GGCGTGCTGC TGCGCCGTTT TCAGGATTAC AGCAATGCTT TTTCACACCG
351  CTGGTACCGC GTGTTCAACG AAATCCCCGT GCTGCTGATG GTTGCCGCGC

401  TGTATsTGGT CGTGTTCAAA CCGTTTTGA
```

This corresponds to the amino acid sequence <SEQ ID 758; ORF102>:

```
  1  MMFSWFKLFH LFFVISWFAG LFYLPRIFVN MAMIDVPRGN PEYVRLSGMA
 51  VRLYRFMSPL GFGAVVFGAA IPFAAGWWGS GWVHVKLCLG LMLLAYQLYC
101  GVLLRRFQDY SNAFSHRWYR VFNEIPVLLM VAALYXVVFK PF*
```

Further work revealed the complete nucleotide sequence <SEQ ID 759>:

```
  1  ATGATGTTTT CTTGGTTCAA GCTGTTTCAC TTGTTTTTTG TCATTTCGTG
 51  GTTTGCAGGG CTGTTTTACC TGCCGAGGAT TTTCGTCAAT ATGGCGATGA
101  TTGATGTGCC GCGCGGCAAT CCCGAGTATG TGCGTCTGTC GGGCATGGCG
151  GTGCGGCTGT ACCGTTTTAT GTCGCCGTTG GGCTTCGGCG CGGTCGTGTT
201  CGGCGCGGCG ATACCGTTTG CCGCCGGCTG GTGGGGCAGC GGCTGGGTAC
251  ACGTCAAACT GTGTTTGGGC TTGATGCTCT TGGCTTACCA GTTGTATTGC
301  GGCGTGCTGC TGCGCCGTTT TCAGGATTAC AGCAATGCTT TTTCACACCG
351  CTGGTACCGC GTGTTCAACG AAATCCCCGT GCTGCTGATG GTTGCCGCGC
401  TGTATCTGGT CGTGTTCAAA CCGTTTTGA
```

This corresponds to the amino acid sequence <SEQ ID 760; ORF102-1>:

```
  1  MMFSWFKLFH LFFVISWFAG LFYLPRIFVN MAMIDVPRGN PEYVRLSGMA
 51  VRLYRFMSPL GFGAVVFGAA IPFAAGWWGS GWVHVKLCLG LMLLAYQLYC
101  GVLLRRFQDY SNAFSHRWYR VFNEIPVLLM VAALYLVVFK PF*
```

Computer analysis of this amino acid sequence gave the following results:

Homology with HP1484 hypothetical integral membrane protein *of H. pylori* (accession number AE000647)

ORF102 and HP1484 show 33% aa identity in 143aa overlap:

```
orf102  3    FSWFKLFHLFFVISWFAGLFYLPRIFVNMAMIDVPRGNPEYVRLSGMAVRLYRFMSPLGF 62
             F W K FH+  VISW A LFYLPR+FV  A     +     V++     +LY F++
HP1484  8    FLWVKAFHVIAVISWMAALFYLPRLFVYHAENAHKKEFVGVVQIQEK--KLYSFIASPAM 65

orf102  63   GAVVFGAAIPFAAG---WWGSGWVHVKLCLGLMLLAYQLYCGVLLRRFQDYSNAFSHRWY 119
             G  +     +          +   GW+H KL L ++LLAY  YC    +R  +       + R+Y
HP1484  66   GFTLITGILMLLIEPTLFKSGGWLHAKLALVVLLLAYHFYCKKCMRELEKDPTRRNARFY 125

orf102  120  RVFNEIPXXXXXXXXXXXXFKPF 142
             RVFNE P            KPF
HP1484  126  RVFNEAPTILMILIVILVVVKPF 148
```

Homology with a predicted ORF from *N.meningitidis* (strain A)

ORF102 shows 99.3% identity over a 142aa overlap with an ORF (ORF102a) from strain A of *N. meningitidis:*

```
                   10        20        30        40        50        60
orf102.pep  MMFSWFKLFHLFFVISWFAGLFYLPRIFVNMAMIDVPRGNPEYVRLSGMAVRLYRFMSPL
            ||||||||||||||||||||||||||||||||||||||||||||||||||||||||||||
orf102a     MMFSWFKLFHLFFVISWFAGLFYLPRIFVNMAMIDVPRGNPEYVRLSGMAVRLYRFMSPL
                   10        20        30        40        50        60

                   70        80        90       100       110       120
orf102.pep  GFGAVVFGAAIPFAAGWWGSGWVHVKLCLGLMLLAYQLYCGVLLRRFQDYSNAFSHRWYR
            ||||||||||||||||||||||||||||||||||||||||||||||||||||||||||||
orf102a     GFGAVVFGAAIPFAAGWWGSGWVHVKLCLGLMLLAYQLYCGVLLRRFQDYSNAFSHRWYR
                   70        80        90       100       110       120

                  130       140
orf102.pep  VFNEIPVLLMVAALYXVVFKPFX
            ||||||||||||||| |||||||
orf102a     VFNEIPVLLMVAALYLVVFKPFX
                  130       140
```

The complete length ORF102a nucleotide sequence <SEQ ID 761> is:

```
  1  ATGATGTTTT CTTGGTTCAA GCTGTTTCAC TTGTTTTTTG TCATTTCGTG
 51  GTTTGCAGGG CTGTTTTACC TGCCGAGGAT TTTCGTCAAT ATGGCGATGA
101  TTGATGTGCC GCGCGGCAAT CCCGAGTATG TGCGTCTGTC GGGCATGGCG
151  GTGCGGCTGT ACCGTTTTAT GTCGCCGTTG GCTTCGGCG CGGTCGTGTT
201  CGGCGCGGCG ATACCGTTTG CCGCCGGCTG GTGGGGCAGC GGCTGGGTAC
251  ACGTCAAACT GTGTTTGGGC TTGATGCTCT TGGCTTACCA GTTGTATTGC
301  GGCGTGCTGC TGCGCCGTTT TCAGGATTAC AGCAATGCTT TTTCACACCG
351  CTGGTACCGC GTGTTCAACG AAATCCCCGT GCTGCTGATG GTTGCCGCGC
401  TGTATCTGGT CGTGTTCAAA CCGTTTTGA
```

This encodes a protein having amino acid sequence <SEQ ID 762>:

```
  1  MMFSWFKLFH LFFVISWFAG LFYLPRIFVN MAMIDVPRGN PEYVRLSGMA
 51  VRLYRFMSPL GFGAVVFGAA IPFAAGWWGS GWVHVKLCLG LMLLAYQLYC
101  GVLLRRFQDY SNAFSHRWYR VFNEIPVLLM VAALYLVVFK PF*
```

ORF102a and ORF102-1 show complete identity in 142 aa overlap:

```
                       10        20        30        40        50        60
orf102a.pep    MMFSWFKLFHLFFVISWFAGLFYLPRIFVNMAMIDVPRGNPEYVRLSGMAVRLYRFMSPL
               ||||||||||||||||||||||||||||||||||||||||||||||||||||||||||||
orf102-1       MMFSWFKLFHLFFVISWFAGLFYLPRIFVNMAMIDVPRGNPEYVRLSGMAVRLYRFMSPL
                       10        20        30        40        50        60

                       70        80        90       100       110       120
orf102a.pep    GFGAVVFGAAIPFAAGWWGSGWVHVKLCLGLMLLAYQLYCGVLLRRFQDYSNAFSHRWYR
               ||||||||||||||||||||||||||||||||||||||||||||||||||||||||||||
orf102-1       GFGAVVFGAAIPFAAGWWGSGWVHVKLCLGLMLLAYQLYCGVLLRRFQDYSNAFSHRWYR
                       70        80        90       100       110       120

                      130       140
orf102a.pep    VFNEIPVLLMVAALYLVVFKPFX
               |||||||||||||||||||||||
orf102-1       VFNEIPVLLMVAALYLVVFKPFX
                      130       140
```

Homology with a predicted ORF from *N.gonorrhoeae*

ORF102 shows 97.9% identity over a 142 aa overlap with a predicted ORF (ORF102ng) from *N. gonorrhoeae:*

```
orf102.pep   MMFSWFKLFHLFFVISWFAGLFYLPRIFVNMAMIDVPRGNPEYVRLSGMAVRLYRFMSPL   60
             |||||||||||||||||||||||||||||||||||:||||||||||||||||||||||||
orf102ng     MMFSWFKLFHLFFVISWFAGLFYLPRIFVNMAMIDAPRGNPEYVRLSGMAVRLYRFMSPL   60

orf102.pep   GFGAVVFGAAIPFAAGWWGSGWVHVKLCLGLMLLAYQLYCGVLLRRFQDYSNAFSHRWYR  120
             |||||||||||||||| |||||||||||||||||||||||||||||||||||||||||||
orf102ng     GFGAVVFGAAIPFAAGRWGSGWVHVKLCLGLMLLAYQLYCGVLLRRFQDYSNAFSHRWYR  120

orf102.pep   VFNEIPVLLMVAALYXVVFKPF  142
             ||||||||||||||| ||||||
orf102ng     VFNEIPVLLMVAALYLVVFKPF  142
```

The complete length ORF102ng nucleotide sequence <SEQ ID 763> is:

```
  1  ATGATGTTTT CTTGGTTCAA GCTGTTTCAC TTGTTTTTTG TCATTTCGTG
 51  GTTTGCAGGG CTGTTTTACC TGCCGAGGAT TTTCGTCAAT ATGGCGATGA
101  TTGATGCGCC GCGCGGCAAT CCCGAGTATG TGCGCCTGTC GGGGATGGCG
151  GTGCGGTTGT ACCGTTTTAT GTCGCCTTTG GGTTTCGGCG CGGTCGTGTT
201  CGGCGCGGCG ATACCGTTTG CCGCcggccg GTGGGGCagc ggctggGTTC
251  ACGTCAAACT GTGTTTGGGC TTGATGCTCT TGGCTTATCA GTTGTATTGC
301  GGCGTGCTGC TGCGCCGTTT TCAGGATTAC AGCAATGCTT TTTCACACCG
351  CTGGTACCGC GTGTTCAAcg aAATCCCCGT GCTGCTGATG GTTGCCGCGC
401  TGTATCTGGT CGTGTTCAAA CCGTTTTGA
```

This encodes a protein having amino acid sequence <SEQ ID 764>:

```
  1  MMFSWFKLFH LFFVISWFAG LFYLPRIFVN MAMIDAPRGN PEYVRLSGMA
 51  VRLYRFMSPL GFGAVVFGAA IPFAAGRWGS GWVHVKLCLG LMLLAYQLYC
101  GVLLRRFQDY SNAFSHRWYR VFNEIPVLLM VAALYLVVFK PF*
```

ORF102ng and ORF102-1 show 98.6% identity in 142 aa overlap:

```
                       10        20        30        40        50        60
orf102-1.pep  MMFSWFKLFHLFFVISWFAGLFYLPRIFVNMAMIDVPRGNPEYVRLSGMAVRLYRFMSPL
              |||||||||||||||||||||||||||||||||||:||||||||||||||||||||||||
orf102ng      MMFSWFKLFHLFFVISWFAGLFYLPRIFVNMAMIDAPRGNPEYVRLSGMAVRLYRFMSPL
                       10        20        30        40        50        60

                       70        80        90       100       110       120
orf102-1.pep  GFGAVVFGAAIPFAAGWWGSGWVHVKLCLGLMLLAYQLYCGVLLRRFQDYSNAFSHRWYR
              |||||||||||||||| |||||||||||||||||||||||||||||||||||||||||||
orf102ng      GFGAVVFGAAIPFAAGRWGSGWVHVKLCLGLMLLAYQLYCGVLLRRFQDYSNAFSHRWYR
                       70        80        90       100       110       120

                      130       140
orf102-1.pep  VFNEIPVLLMVAALYLVVFKPFX
              ||||||||||||||||||||||
orf102ng      VFNEIPVLLMVAALYLVVFKPFX
                      130       140
```

In addition, ORF102ng shows significant homology to a membrane protein from *H.pylori:*

```
gi|2314656 (AE000647) conserved hypothetical integral membrane protein
[Helicobacter pylori] Length = 148
 Score = 79.2 bits (192), Expect = 1e-14
 Identities = 50/147 (34%), Positives = 68/147 (46%), Gaps = 13/147 (8%)

Query: 3   FSWFKLFHLFFVISWFAGLFYLPRIFVNMAMIDAPRGNPEYVRLSGMAVRLYRFMSPLGF 62
            F W K FH+  VISW A LFYLPR+FV  A     +     V++     +LY F++
Sbjct: 8   FLWVKAFHVIAVISWMAALFYLPRLFVYHAENAHKKEFVGVVQIQEK--KLYSFIASPAM 65

Query: 63  GAVVFGAAIP-------FAAGRWGSGWVHVKLCLGLMLLAYQLYCGVLLRRFQDYSNAFS 115
            G  +    +        F +G    GW+H KL L ++LLAY  YC   +R  +      +
Sbjct: 66  GFTLITGILMLLIEPTLFKSG----GWLHAKLALVVLLLAYHFYCKKCMRELEKDPTRRN 121

Query: 116 HRWYRVFNEIPXXXXXXXXXXXXFKPF 142
             R+YRVFNE P            KPF
Sbjct: 122 ARFYRVFNEAPTILMILIVILVVVKPF 148
```

Based on this analysis, it is predicted that these proteins from *N. meningitidis* and *N.gonorrhoeae,* and their epitopes, could be useful antigens for vaccines or diagnostics, or for raising antibodies.

**Example 91**

The following partial DNA sequence was identified in *N.meningitidis* <SEQ ID 765>:

```
  1   ATGGCAAAAA TGATGAAATG GGCGGCTGTT GCGGCGGTCG CGGCGGCAGC
 51   GGTTTGGGGC GGATGGTCTT AACTGAAGCC CGAGCCGCAC GTGCTTGATA
101   TTACGGAAAC GGTCAGGCGC GGC // .....
//..  ATTTCGTTTA CGATTTTGTC CGAACCGGAT ACGCCGATTA AGGCGAAGCT
 51   CGACAGCGTC GACCCCGGGC TGACCACGAT GTCGTCGGGC GGTTACAACA
101   GCAGTACGGA TACGGCTTCC AATGCGGTCT ACTATTATGC CCGTTCGTTT
151   GTGCCGAATC CGGACGGCAA ACTCGCCACG GGGATGACGA CGCAGAATAC
201   GGTTGAAATC GACGGCGTGA AAAATGTGCT GATTATTCCG TCGCTGACCG
251   TGAAAAATCG CGGCGGCAAG GCGTTTGTGC GCGTGTTGGG TGCGGACGGC
301   AAGGCGGCGG AACGCGAAAT CCGGACCGGT ATGAGAGACA GTATGAATAC
351   CGAAGTAAAA AGCGGGTTGA AAGAGGGGGA CAAAGTGGTC ATCTCCGAAA
401   TAACCGCCGC CGAGCAACAG GAAAGCGGCG AACGCGCCCT AGGCGGCCCG
451   CCGCGCCGAT AA
```

This corresponds to the amino acid sequence <SEQ ID 766; ORF85>:

```
  1  MAKMMKWAAV AAVAAAAVWG GWS.LKPEPH VLDITETVRR G.........
 51  .......... .......... .......... .......... ..........
101  .......... .......... .......... .......... ..........
151  .......... .......... .......... .......... ..........

201  .......... .......... .......... .........I SFTILSEPDT
251  PIKAKLDSVD PGLTTMSSGG YNSSTDTASN AVYYYARSFV PNPDGKLATG
301  MTTQNTVEID GVKNVLIIPS LTVKNRGGKA FVRVLGADGK AAEREIRTGM
351  RDSMNTEVKS GLKEGDKVVI SEITAAEQQE SGERALGGPP RR*
```

Further work revealed the further partial nucleotide sequence <SEQ ID 767>:

```
   1  ..GTATCGGTCG GCGCGCAGGC ATCGGGGCAG ATTAAGATAC TTTATGTCAA
  51    ACTCGGGCAA CAGGTTAAAA AGGGCGATTT GATTGCGGAA ATCAATTCGA
 101    CCTCGCAGAC CAATACGCTC AATACGGAAA AATCCAAGTT GGAAACGTAT
 151    CAGGCGAAGC TGGTGTCGGC ACAGATTGCA TTGGGCAGCG CGGAGAAGAA
 201    ATATAAGCGT CAGGCGGCGT TATGGAAGGA AAACGCGACT TCCAAAGAGG
 251    ATTTGGAAAG CGCGCAGGAT GCGTTTGCCG CCGCCAAAGC CAATGTTGCC
 301    GAGCTGAAGG CTTTAATCAG ACAGAGCAAA ATTTCCATCA ATACCGCCGA
 351    GTCGGAATTG GGCTACACGC GCATTACCGC AACGATGGAC GGCACGGTGG
 401    TGGCGATTCT CGTGGAAGAG GGGCAGACTG TGAACGCGGC GCAGTCTACG
 451    CCGACGATTG TCCAATTGGC GAATCTGGAT ATGATGTTGA ACAAAATGCA
 501    GATTGCCGAG GGCGATATTA CCAAGGTGAA GGCGGGGCAG GATATTTCGT
 551    TTACGATTTT GTCCGAACCG GATACGCCGA TTAAGGCGAA GCTCGACAGC
 601    GTCGACCCCG GGCTGACCAC GATGTCGTCG GGCGGTTACA ACAGCAGTAC
 651    GGATACGGCT TCCAATGCGG TCTACTATTA TGCCCGTTCG TTTGTGCCGA
 701    ATCCGGACGG CAAACTCGCC ACGGGGATGA CGACGCAGAA TACGGTTGAA
 751    ATCGACGGCG TGAAAAATGT GCTGATTATT CCGTCGCTGA CCGTGAAAAA
 801    TCGCGGCGGC AAGGCGTTTG TGCGCGTGTT GGGTGCGGAC GGCAAGGCGG
 851    CGGAACGCGA AATCCGGACC GGTATGAGAG ACAGTATGAA TACCGAAGTA
 901    AAAAGCGGGT TGAAAGAGGG GGACAAAGTG GTCATCTCCG AAATAACCGC
 951    CGCCGAGCAA CAGGAAAGCG GCGAACGCGC CCTAGGCGGC CCGCCGCGCC
1001    GATAA
```

This corresponds to the amino acid sequence <SEQ ID 768; ORF85-1>:

```
  1  ..VSVGAQASGQ IKILYVKLGQ QVKKGDLIAE INSTSQTNTL NTEKSKLETY
 51    QAKLVSAQIA LGSAEKKYKR QAALWKENAT SKEDLESAQD AFAAAKANVA
101    ELKALIRQSK ISINTAESEL GYTRITATMD GTVVAILVEE GQTVNAAQST
151    PTIVQLANLD MMLNKMQIAE GDITKVKAGQ DISFTILSEP DTPIKAKLDS
201    VDPGLTTMSS GGYNSSTDTA SNAVYYYARS FVPNPDGKLA TGMTTQNTVE
251    IDGVKNVLII PSLTVKNRGG KAFVRVLGAD GKAAEREIRT GMRDSMNTEV
301    KSGLKEGDKV VISEITAAEQ QESGERALGG PPRR*
```

Computer analysis of this amino acid sequence gave the following results:

Homology with a predicted ORF from *N.meningitidis* (strain A)

ORF85 shows 87.8% identity over a 41aa overlap and 99.3% identity over a 153aa overlap with an ORF (ORF85a) from strain A of *N. meningitidis:*

```
                    10        20         30        40
orf85.pep    MAKMMKWAAVAAVAAAAVWGGWS-LKPEPHVLDITETVRRG
             ||||||||||||||||||||||| |||||::  ||||||||
orf85a       MAKMMKWAAVAAVAAAAVWGGWSYLKPEPQAAYITETVRRGDISRTVSATGEISPSNLVS
                    10        20        30        40        50        60
                                     //
                                        80        90       100
orf85.pep    ..............................ISFTILSEPDTPIKAKLDSVDPGLTTMSSG
                                           ||||||||||||||||||||||||||||||
orf85a       TIVQLANLDMMLNKMQIAEGDITKVKAGQDISFTILSEPDTPIKAKLDSVDPGLTTMSSG
           210       220       230       240       250       260

           110       120       130       140       150       160
orf85.pep    GYNSSTDTASNAVYYYARSFVPNPDGKLATGMTTQNTVEIDGVKNVLIIPSLTVKNRGGK
             |||||||||||||||||||||||||||||||||||||||||||||||||||||||||||:
orf85a       GYNSSTDTASNAVYYYARSFVPNPDGKLATGMTTQNTVEIDGVKNVLIIPSLTVKNRGGR
           270       280       290       300       310       320

           170       180       190       200       210       220
orf85.pep    AFVRVLGADGKAAEREIRTGMRDSMNTEVKSGLKEGDKVVISEITAAEQQESGERALGGP
             ||||||||||||||||||||||||||||||||||||||||||||||||||||||||||||
orf85a       AFVRVLGADGKAAEREIRTGMRDSMNTEVKSGLKEGDKVVISEITAAEQQESGERALGGP
           330       340       350       360       370       380

                230
orf85.pep     PRRX
              ||||
orf85a        PRRX
             390
```

The complete length ORF85a nucleotide sequence <SEQ ID 769> is:

```
   1 ATGGCAAAAA TGATGAAATG GGCGGCTGTT GCGGCGGTCG CGGCGGCAGC
  51 GGTTTGGGGC GGATGGTCTT ATCTGAAGCC CGAGCCGCAG GCTGCTTATA
 101 TTACGGAAAC GGTCAGGCGC GGCGACATCA GCCGGACGGT TTCTGCAACA
 151 GGGGAGATTT CGCCGTCCAA CCTGGTATCG GTCGGCGCGC AGGCATCGGG
 201 GCAGATTAAG AAACTTTATG TCAAACTCGG GCAACAGGTT AAAAAGGGCG
 251 ATTTGATTGC GGAAATCAAT TCGACCTCGC AGACCAATAC GCTCAATACG
 301 GAAAAATCCA AATTGGAAAC GTATCAGGCG AAGCTGGTGT CGGCACAGAT
 351 TGCATTGGGC AGCGCGGAGA AGAAATATAA GCGTCAGGCG GCGTTGTGGA
 401 AGGATGATGC GACCGCTAAA GAAGATTTGG AAAGCGCACA GGATGCGCTT
 451 GCCGCCGCCA AAGCCAATGT TGCCGAGCTG AAGGCTCTAA TCAGACAGAG
 501 CAAAATTTCC ATCAATACCG CCGAGTCGGA ATTGGGCTAC ACGCGCATTA
 551 CCGCAACGAT GGACGGCACG GTGGTGGCGA TTCTCGTGGA AGAGGGGCAG
 601 ACTGTGAACG CGGCGCAGTC TACGCCGACG ATTGTCCAAT TGGCGAATCT
 651 GGATATGATG TTGAACAAAA TGCAGATTGC CGAGGGCGAT ATTACCAAGG
 701 TGAAGGCGGG GCAGGATATT TCGTTTACGA TTTTGTCCGA ACCGGATACG
 751 CCGATTAAGG CGAAGCTCGA CAGCGTCGAC CCCGGGCTGA CCACGATGTC
 801 GTCGGGCGGC TACAACAGCA GTACGGATAC GGCTTCCAAT GCGGTCTACT
 851 ATTATGCCCG TTCGTTTGTG CCGAATCCGG ACGGCAAACT CGCCACGGGG
 901 ATGACGACGC AGAATACGGT TGAAATCGAC GGTGTGAAAA ATGTGCTGAT
 951 TATTCCGTCG CTGACCGTGA AAAATCGCGG CGGCAGGGCG TTTGTGCGCG
1001 TGTTGGGTGC AGACGGCAAG GCGGCGGAAC GCGAAATCCG GACCGGTATG
1051 AGAGACAGTA TGAATACCGA AGTAAAAAGC GGGTTGAAAG AGGGGGACAA
1101 AGTGGTCATC TCCGAAATAA CCGCCGCCGA GCAGCAGGAA AGCGGCGAAC
1151 GCGCCCTAGG CGGCCCGCCG CGCCGATAA
```

This encodes a protein having amino acid sequence <SEQ ID 770>:

```
  1  MAKMMKWAAV AAVAAAAVWG GWSYLKPEPQ AAYITETVRR GDISRTVSAT
 51  GEISPSNLVS VGAQASGQIK KLYVKLGQQV KKGDLIAEIN STSQTNTLNT
101  EKSKLETYQA KLVSAQIALG SAEKKYKRQA ALWKDDATAK EDLESAQDAL
151  AAAKANVAEL KALIRQSKIS INTAESELGY TRITATMDGT VVAILVEEGQ
201  TVNAAQSTPT IVQLANLDMM LNKMQIAEGD ITKVKAGQDI SFTILSEPDT
251  PIKAKLDSVD PGLTTMSSGG YNSSTDTASN AVYYYARSFV PNPDGKLATG
301  MTTQNTVEID GVKNVLIIPS LTVKNRGGRA FVRVLGADGK AAEREIRTGM
351  RDSMNTEVKS GLKEGDKVVI SEITAAEQQE SGERALGGPP RR*
```

ORF85a and ORF85-1 show 98.2% identity in 334 aa overlap:

```
                     30        40        50        60        70        80
orf85a.pep   PQAAYITETVRRGDISRTVSATGEISPSNLVSVGAQASGQIKKLYVKLGQQVKKGDLIAE
                                           ||||||||||| ||||||||||||||||
orf85-1                                    VSVGAQASGQIKILYVKLGQQVKKGDLIAE
                                                   10        20        30

                     90       100       110       120       130       140
orf85a.pep   INSTSQTNTLNTEKSKLETYQAKLVSAQIALGSAEKKYKRQAALWKDDATAKEDLESAQD
             ||||||||||||||||||||||||||||||||||||||||||||||||::||:||||||||
orf85-1      INSTSQTNTLNTEKSKLETYQAKLVSAQIALGSAEKKYKRQAALWKENATSKEDLESAQD
                     40        50        60        70        80        90

                    150       160       170       180       190       200
orf85a.pep   ALAAAKANVAELKALIRQSKISINTAESELGYTRITATMDGTVVAILVEEGQTVNAAQST
             |:||||||||||||||||||||||||||||||||||||||||||||||||||||||||||
orf85-1      AFAAAKANVAELKALIRQSKISINTAESELGYTRITATMDGTVVAILVEEGQTVNAAQST
                    100       110       120       130       140       150

                    210       220       230       240       250       260
orf85a.pep   PTIVQLANLDMMLNKMQIAEGDITKVKAGQDISFTILSEPDTPIKAKLDSVDPGLTTMSS
             ||||||||||||||||||||||||||||||||||||||||||||||||||||||||||||
orf85-1      PTIVQLANLDMMLNKMQIAEGDITKVKAGQDISFTILSEPDTPIKAKLDSVDPGLTTMSS
                    160       170       180       190       200       210

                    270       280       290       300       310       320
orf85a.pep   GGYNSSTDTASNAVYYYARSFVPNPDGKLATGMTTQNTVEIDGVKNVLIIPSLTVKNRGG
             ||||||||||||||||||||||||||||||||||||||||||||||||||||||||||||
orf85-1      GGYNSSTDTASNAVYYYARSFVPNPDGKLATGMTTQNTVEIDGVKNVLIIPSLTVKNRGG
                    220       230       240       250       260       270

                    330       340       350       360       370       380
orf85a.pep   RAFVRVLGADGKAAEREIRTGMRDSMNTEVKSGLKEGDKVVISEITAAEQQESGERALGG
             :|||||||||||||||||||||||||||||||||||||||||||||||||||||||||||
orf85-1      KAFVRVLGADGKAAEREIRTGMRDSMNTEVKSGLKEGDKVVISEITAAEQQESGERALGG
                    280       290       300       310       320       330

                    390
orf85a.pep   PPRRX
             |||||
orf85-1      PPRRX
```

Figure 19D shows plots of hydrophilicity, antigenic index, and AMPHI regions for ORF85a..

<u>Homology with a predicted ORF from *N.gonorrhoeae*</u>

ORF85 shows a high degree of identity with a predicted ORF (ORF85ng) from *N.gonorrhoeae:*

```
ORF85        1 MAKMMKWAAVAAVAAAAVWGGWS.LKPEPHVLDITETVRRG......... 40
                ||||||||||||||||||||||| ||||||::  |||:||||
ORF85ng      1 MAKMMKWAAVAAVAAAAVWGGWSYLKPEPQAAYITEAVRRGDISRTVSAT 50

                     .         .         .         .         .
ORF85          ..................................ISFTILSEPDT 250
                                                 |||||||||||
ORF85ng    201 TVNAAQSTPTIVQLANLDMMLNKMQIAEGDITKVKAGQDISFTILSEPDT 250
                     .         .         .         .         .
ORF85      251 PIKAKLDSVDPGLTTMSSGGYNSSTDTASNAVYYYARSFVPNPDGKLATG 300
               ||||||||||||||||||||||||||||||||||||||||||||||||||
ORF85ng    251 PIKAKLDSVDPGLTTMSSGGYNSSTDTASNAVYYYARSFVPNPDGKLATG 300
                     .         .         .         .         .
ORF85      301 MTTQNTVEIDGVKNVLIIPSLTVKNRGGKAFVRVLGADGKAAEREIRTGM 350
               ||||||||||||||||:||||||||||||||||||||||||| |||||||
ORF85ng    301 MTTQNTVEIDGVKNVLLIPSLTVKNRGGKAFVRVLGADGKAVEREIRTGM 350
                     .         .         .         .
ORF85      152 RDSMNTEVKSGLKEGDKVVISEITAAEQQESGERALGGPPRR 393
               :|||||||||||||||||||||||||||||||||||||||||
ORF85ng    351 KDSMNTEVKSGLKEGDKVVISEITAAEQQESGERALGGPPRR 393
```

The complete length ORF85ng nucleotide sequence <SEQ ID 771> is:

```
   1  ATGGCAAAAA TGATGAAATG GGCGGCTGTT GCGGCGGTCG CGGCGGCaac
  51  GGTTTGGGGC GGATGGTCTT ATCTGAAGCC CGAACCGCAG GCTGCTTATA
 101  TTACGGAaac ggTCAGGCGC GGCGATATCA GCCGGACGGT TTCCGCGACG
 151  GgcgAGATTT CGCCGTCCAA CCTGGTATCG GTCGGCGCGC AGGCTTCGGG
 201  GCAGATTAAA AAGCTTTATG TCAAACTCGG GCAACAGGTC AAAAAGGGCG
 251  ATTTGATTGC GGAAATCAAT TCGACCACGC AGACCAACAC GATCGATATG
 301  GAAAAATCCA AATTGGAAAC GTATCAGGCG AAGCTGGTGT CGGCACAGAT
 351  TGCATTGGGC AGCGCGGAGA AGAAATATAA GCGTCAGGCG GCGTTGTGGA
 401  AGGATGATGC GACCTCTAAA GAAGATTTGG AAAGCGCGCA GGATGCGCTT
 451  GCCGCCGCCA AAGCCAATGT TGCCGAGTTG AAGGCTTTAA TCAGACAGAG
 501  CAAAATTTCC ATCAATACCG CCGAGTCGGA TTTGGGCTAC ACGCGCATTA
 551  CCGCGACGAT GGACGGCACG GTGGTGGCGA TTCCCGTGGA AGAGGGGCAG
 601  ACTGTGAACG CGGCGCAGTC TACGCCGACG ATTGTCCAAT TGGCGAATCT
 651  GGATATGATG TTGAACAAAA TGCAGATTGC CGAGGGCGAT ATTACCAAGG
 701  TGAAGGCGGG GCAGGATATT TCGTTTACGA TTTTGTCCGA ACCGGATACG
 751  CCGATTAAGG CGAAGCTCGA CAGCGTCGAC CCCGGGCTGA CCACGATGTC
 801  GTCGGGCGGC TACAACAGCA GTACGGATAC GGCTTCCAAT GCGGTCTATT
 851  ATTATGCCCG TTCGTTTGTG CCGAATCCGG ACGGCAAACT CGCCACGGGG
 901  ATGACGACGC AGAATACGGT TGAAATCGAC GGTGTGAAAA ATGTGTTGCT
 951  TATTCCGTCG CTGACCGTGA AAAATCGCGG CGGCAAGGCG TTCGTACGCG
1001  TGTTGGGTGC GGACGGCAAG GCAGTGGAAC GCGAAATCCG GACCGGTATG
1051  AAAGACAGTA TGAATACCGA AGTGAAAAGC GGGTTGAAAG AGGGGGACAA
1101  AGTGGTCATC TCCGAAATAA CCGCCGCCGA GCAGCAGGAA AGCGGCGAAC
1151  GCGCCCTAGG CGGCCCGCCG CGCCGATAA
```

This encodes a protein having amino acid sequence <SEQ ID 772>:

```
  1  MAKMMKWAAV AAVAAAAVWG GWSYLKPEPQ AAYITEAVRR GDISRTVSAT
 51  GEISPSNLVS VGAQASGQIK KLYVKLGQQV KKGDLIAEIN STTQTNTIDM
101  EKSKLETYQA KLVSAQIALG SAEKKYKRQA ALWKDDATSK EDLESAQDAL
151  AAAKANVAEL KALIRQSKIS INTAESDLGY TRITATMDGT VVAIPVEEGQ
201  TVNAAQSTPT IVQLANLDMM LNKMQIAEGD ITKVKAGQDI SFTILSEPDT
251  PIKAKLDSVD PGLTTMSSGG YNSSTDTASN AVYYYARSFV PNPDGKLATG
301  MTTQNTVEID GVKNVLLIPS LTVKNRGGKA FVRVLGADGK AVEREIRTGM
351  KDSMNTEVKS GLKEGDKVVI SEITAAEQQE SGERALGGPP RR*
```

ORF85ng and ORF85-1 show 96.1 % identity in 334 aa overlap:

```
            30        40        50        60        70        80
orf85ng     PQAAYITETVRRGDISRTVSATGEISPSNLVSVGAQASGQIKKLYVKLGQQVKKGDLIAE
                                          |||||||||||| ||||||||||||||||
orf85-1                                   VSVGAQASGQIKILYVKLGQQVKKGDLIAE
                                                  10        20        30

            90       100       110       120       130       140
orf85ng     INSTTQTNTIDMEKSKLETYQAKLVSAQIALGSAEKKYKRQAALWKDDATSKEDLESAQD
            ||||:||||:: |||||||||||||||||||||||||||||||||::||||||||||||
orf85-1     INSTSQTNTLNTEKSKLETYQAKLVSAQIALGSAEKKYKRQAALWKENATSKEDLESAQD
                    40        50        60        70        80        90

           150       160       170       180       190       200
orf85ng     ALAAAKANVAELKALIRQSKISINTAESDLGYTRITATMDGTVVAIPVEEGQTVNAAQST
            |:|||||||||||||||||||||||||||:|||||||||||||||||| ||||||||||||
orf85-1     AFAAAKANVAELKALIRQSKISINTAESELGYTRITATMDGTVVAILVEEGQTVNAAQST
                   100       110       120       130       140       150

           210       220       230       240       250       260
orf85ng     PTIVQLANLDMMLNKMQIAEGDITKVKAGQDISFTILSEPDTPIKAKLDSVDPGLTTMSS
            ||||||||||||||||||||||||||||||||||||||||||||||||||||||||||||
orf85-1     PTIVQLANLDMMLNKMQIAEGDITKVKAGQDISFTILSEPDTPIKAKLDSVDPGLTTMSS
                   160       170       180       190       200       210

           270       280       290       300       310       320
orf85ng     GGYNSSTDTASNAVYYYARSFVPNPDGKLATGMTTQNTVEIDGVKNVLLIPSLTVKNRGG
            ||||||||||||||||||||||||||||||||||||||||||||||||:|||||||||||
orf85-1     GGYNSSTDTASNAVYYYARSFVPNPDGKLATGMTTQNTVEIDGVKNVLIIPSLTVKNRGG
                   220       230       240       250       260       270

           330       340       350       360       370       380
orf85ng     KAFVRVLGADGKAVEREIRTGMKDSMNTEVKSGLKEGDKVVISEITAAEQQESGERALGG
            ||||||||||||:||||||||:||||||||||||||||||||||||||||||||||||||
orf85-1     KAFVRVLGADGKAAEREIRTGMRDSMNTEVKSGLKEGDKVVISEITAAEQQESGERALGG
                   280       290       300       310       320       330

           390
orf85ng     PPRRX
            |||||
orf85-1     PPRRX
```

In addition, ORF85ng shows significant homology to an *E.coli* membrane fusion protein:

```
gi|1787104 (AE000189) o380; 27% identical (27 gaps) to 332 residues from
membrane fusion protein precursor, MTRC_NEIGO SW: P43505 (412 aa) [Escherichia
coli] Length = 380
 Score =  193 bits (485), Expect = 2e-48
 Identities = 120/345 (34%), Positives = 182/345 (51%), Gaps = 13/345 (3%)

Query: 29  PQAAYITETVRRGDISRTVSATGEISPSNLVSVGAQASGQIKKLYVKLGQQVKKGDLIAE 88
            P   Y T  VR GD+ ++V ATG++      V VGAQ SGQ+K L V +G +VKK  L+
Sbjct: 41  PVPTYQTLIVRPGDLQQSVLATGKLDALRKVDVGAQVSGQLKTLSVAIGDKVKKDQLLGV 100

Query: 89  INSTTQTNTIDMEKSKLETYQAKLVSAQIALGSAEKKYKRQAALWKDDATSKEXXXXXXX 148
            I+      N I    ++ L     +A+    A+   L  A    Y RQ   L +   A S++
Sbjct: 101 IDPEQAENQIKEVEATLMELRAQRQQAEAELKLARVTYSRQQRLAQTKAVSQQDLDTAAT 160

Query: 149 XXXXXXXXXXXXXXIRQSKISINTAESDLGYTRITATMDGTVVAIPVEEGQTVNAAQST 208
                          I++++ S++TA+++L YTRI A M G V   I    +GQTV AAQ
Sbjct: 161 EMAVKQAQIGTIDAQIKRNQASLDTAKTNLDYTRIVAPMAGEVTQITTLQGQTVIAAQQA 220
```

```
Query: 209 PTIVQLANLDMMLNKMQIAEGDITKVKAGQDISFTILSEPDTPIKAKLDSVDPGLTTMSS 268
           P I+ LA++  ML K Q++E D+  +K GQ   FT+L +P T  + ++  V P
Sbjct: 221 PNILTLADMSAMLVKAQVSEADVIHLKPGQKAWFTVLGDPLTRYEGQIKDVLP------- 273

Query: 269 GGYNSSTDTASNAVYYYARSFVPNPDGKLATGMTTQNTVEIDGVKNVLLIPSLTVKNRGG 328
                + +  ++A++YYAR  VPNP+G L   MT Q  +++  VKNVL IP   + +  G
Sbjct: 274 -----TPEKVNDAIFYYARFEVPNPNGLLRLDMTAQVHIQLTDVKNVLTIPLSALGDPVG 328

Query: 329 KAFVRV-LGADGKAVEREIRTGMKDSMNTEVKSGLKEGDKVVISE 372
             +V L  +G+  ERE+  G ++  + E+  GL+ GD+VVI E
Sbjct: 329 DNRYKVKLLRNGETREREVTIGARNDTDVEIVKGLEAGDEVVIGE 373
```

Based on this analysis, it was predicted that the proteins from *N.meningitidis* and *N.gonorrhoeae,* and their epitopes, could be useful antigens for vaccines or diagnostics, or for raising antibodies.

ORF85-1 (40.4kDa) was cloned in the pGex vectors and expressed in *E.coli,* as described above. The products of protein expression and purification were analyzed by SDS-PAGE. Figure 19A shows the results of affinity purification of the GST-fusion protein. Purified GST-fusion protein was used to immunise mice, whose sera were used for Western blot (Figure 19B), FACS analysis (Figure 19C), and ELISA (positive result). These experiments confirm that ORF85-1 is a surface-exposed protein, and that it is a useful immunogen.

**Example 92**

The following partial DNA sequence was identified in *N.meningitidis* <SEQ ID 773>:

```
  1 ..ATTCCCGCCA CGATGACATT TGAACGCAGC GGCAATGCTT ACAAAATCGT
 51   TTCGACGATT AAAGTGCCGC TATACAATAT CCGTTTCGAG TCCGGCGGTA
101   CGGTTGTCGG CAATACCCTG CACCCTACCT ACTATAGAGA CATACGCAGG
151   GGCAAACTGT ATGCGGAAgc CAAATTCGCC GACgGcAGCG TAACTTACGG
201   CAAAGCGGGC GAGAGCAAAA CCGAGCAAAG CCCCAAGGCT ATGGATTTGT
251   TCACGCTTGC CTGGCAGTTG GCGGCAAATG ACGCGAAACT CCCCCCGGGG
301   CTGAAAATCA CCAACGGCAA AAAACTTTAT TCCGTCGGCG GTTTGAATAA
351   GGCGGGTACA GGAAAATACA GCATAGGCGG CGTGGAAACC GAAGTCGTCA
401   AATATCGGGT GCGGCGCGGC GACGATGCGG TAATGTATTT cTTCGCACCG
451   TCCCTGAACA ATATTCCGGC ACAAATCGGC TATACCGACG ACGGCAAAAC
501   CTATACGCTG AAACTCAAAT CGGTGCAGAT CAACGGCCAG GCAGCCAAAC
551   CGTAA
```

This corresponds to the amino acid sequence <SEQ ID 774; ORF120>:

```
  1 ..IPATMTFERS GNAYKIVSTI KVPLYNIRFE SGGTVVGNTL HPTYYRDIRR
 51   GKLYAEAKFA DGSVTYGKAG ESKTEQSPKA MDLFTLAWQL AANDAKLPPG
101   LKITNGKKLY SVGGLNKAGT GKYSIGGVET EVVKYRVRRG DDAVMYFFAP
151   SLNNIPAQIG YTDDGKTYTL KLKSVQINGQ AAKP*
```

Further work revealed the complete nucleotide sequence <SEQ ID 775>:

```
  1  ATGATGAAGA CTTTTAAAAA TATATTTTCC GCCGCCATTT TGTCCGCCGC
 51  CCTGCCGTGC GCGTATGCGG CAGGGCTGCC CCAATCCGCC GTGCTGCACT
101  ATTCCGGCAG CTACGGCATT CCCGCCACGA TGACATTTGA ACGCAGCGGC
151  AATGCTTACA AAATCGTTTC GACGATTAAA GTGCCGCTAT ACAATATCCG
201  TTTCGAGTCC GGCGGTACGG TTGTCGGCAA TACCCTGCAC CCTACCTACT
251  ATAGAGACAT ACGCAGGGGC AAACTGTATG CGGAAGCCAA ATTCGCCGAC
301  GGCAGCGTAA CTTACGGCAA AGCGGGCGAG AGCAAAACCG AGCAAAGCCC
351  CAAGGCTATG GATTTGTTCA CGCTTGCCTG GCAGTTGGCG GCAAATGACG
401  CGAAACTCCC CCCGGGGCTG AAAATCACCA ACGGCAAAAA ACTTTATTCC
451  GTCGGCGGTT TGAATAAGGC GGGTACAGGA AAATACAGCA TAGGCGGCGT
501  GGAAACCGAA GTCGTCAAAT ATCGGGTGCG GCGCGGCGAC GATGCGGTAA
551  TGTATTTCTT CGCACCGTCC CTGAACAATA TTCCGGCACA AATCGGCTAT

601  ACCGACGACG GCAAAACCTA TACGCTGAAA CTCAAATCGG TGCAGATCAA
651  CGGCCAGGCA GCCAAACCGT AA
```

This corresponds to the amino acid sequence <SEQ ID 776; ORF120-1>:

```
  1  MMKTFKNIFS AAILSAALPC AYAAGLPQSA VLHYSGSYGI PATMTFERSG
 51  NAYKIVSTIK VPLYNIRFES GGTVVGNTLH PTYYRDIRRG KLYAEAKFAD
101  GSVTYGKAGE SKTEQSPKAM DLFTLAWQLA ANDAKLPPGL KITNGKKLYS
151  VGGLNKAGTG KYSIGGVETE VVKYRVRRGD DAVMYFFAPS LNNIPAQIGY
201  TDDGKTYTLK LKSVQINGQA AKP*
```

Computer analysis of this amino acid sequence gave the following results:

Homology with a predicted ORF from *N.meningitidis* (strain A)

ORF120 shows 92.4% identity over a 184aa overlap with an ORF (ORF120a) from strain A of *N. meningitidis:*

```
                                                   10        20        30
orf120.pep                                 IPATMTFERSGNAYKIVSTIKVPLYNIRFE
                                           ||||  :      || |||||||||||||||
orf120a     SAAILSAALPCAYAAGLPXSAVLHYSGSYGIPATXXXXXXXNAXKIVSTIKVPLYNIRFE
           10        20        30        40        50        60

                    40        50        60        70        80        90
orf120.pep  SGGTVVGNTLHPTYYRDIRRGKLYAEAKFADGSVTYGKAGESKTEQSPKAMDLFTLAWQL
            ||||||||||||||||||||||||||||||||||||||      : ||||||||||||||
orf120a     SGGTVVGNTLHPTYYRDIRRGKLYAEAKFADGSVTYGKAXXXXXXQSPKAMDLFTLAWQL
           70        80        90       100       110       120

                   100       110       120       130       140       150
orf120.pep  AANDAKLPPGLKITNGKKLYSVGGLNKAGTGKYSIGGVETEVVKYRVRRGDDAVMYFFAP
            ||||||||||||||||||||||||||||||||||||||||||||||||||||||||||||
orf120a     AANDAKLPPGLKITNGKKLYSVGGLNKAGTGKYSIGGVETEVVKYRVRRGDDAVMYFFAP
          130       140       150       160       170       180

                   160       170       180
orf120.pep  SLNNIPAQIGYTDDGKTYTLKLKSVQINGQAAKPX
            |||||||||||||||||||||||||||||||||||
orf120a     SLNNIPAQIGYTDDGKTYTLKLKSVQINGQAAKPX
          190       200       210       220
```

The complete length ORF120a nucleotide sequence <SEQ ID 777> is:

```
  1  ATGATGAAGA CTTTTAAAAA TATATTTTCC GCCGCCATTT TGTCCGCCGC
 51  CCTGCCGTGC GCGTATGCGG CAGGGCTGCC CNAATCCGCC GTGCTGCACT
101  ATTCCGGCAG CTACGGCATT CCCGCCACNA NNANNTNNGN ACNNNGNGNC
151  AATGCTTNCA AAATCGTTTC GACGATTAAA GTGCCGCTAT ACAATATCCG
201  TTTCGAGTCC GGCGGTACGG TTGTCGGCAA TACCCTGCAC CCTACCTACT
251  ATAGAGACAT ACGCAGGGGC AAACTGTATG CGGAAGCCAA ATTCGCCGAC
301  GGCAGCGTAA CCTACGGCAA AGCGGNNNNN ANCNNNNNNG NGCAAAGCCC
351  CAAGGCTATG GATTTGTTCA CGCTTGCNTG GCAGTTGGCG GCAAATGACG
401  CGAAACTCCC CCCGGGGCTG AAAATCACCA ACGGCAAAAA ACTTTATTCC
451  GTCGGCGGTT TGAATAAGGC GGGTACAGGA AAATACAGCA TAGGCGGCGT
501  GGAAACCGAA GTCGTCAAAT ATCGGGTGCG GCGCGGCGAC GATGCGGTAA
551  TGTATTTCTT CGCACCGTCC CTGAACAATA TTCCGGCACA AATCGGCTAT
601  ACCGACGACG GCAAAACCTA TACGCTGAAA CTCAAATCGG TGCAGATCAA
651  CGGCCAGGCA GCCAAACCGT AA
```

This encodes a protein having amino acid sequence <SEQ ID 778>:

```
  1  MMKTFKNIFS AAILSAALPC AYAAGLPXSA VLHYSGSYGI PATXXXXXXX
 51  NAXKIVSTIK VPLYNIRFES GGTVVGNTLH PTYYRDIRRG KLYAEAKFAD
101  GSVTYGKAXX XXXXQSPKAM DLFTLAWQLA ANDAKLPPGL KITNGKKLYS
151  VGGLNKAGTG KYSIGGVETE VVKYRVRRGD DAVMYFFAPS LNNIPAQIGY
201  TDDGKTYTLK LKSVQINGQA AKP*
```

ORF120a and ORF120-1 show 93.3% identity in 223 aa overlap:

```
                        10        20        30        40        50        60
orf120a.pep     MMKTFKNIFSAAILSAALPCAYAAGLPXSAVLHYSGSYGIPATXXXXXXXNAXKIVSTIK
                |||||||||||||||||||||||||||| ||||||||||||||| :       || |||||||
orf120-1        MMKTFKNIFSAAILSAALPCAYAAGLPQSAVLHYSGSYGIPATMTFERSGNAYKIVSTIK
                        10        20        30        40        50        60

                        70        80        90       100       110       120
orf120a.pep     VPLYNIRFESGGTVVGNTLHPTYYRDIRRGKLYAEAKFADGSVTYGKAXXXXXXQSPKAM
                ||||||||||||||||||||||||||||||||||||||||||||||||      : ||||||
orf120-1        VPLYNIRFESGGTVVGNTLHPTYYRDIRRGKLYAEAKFADGSVTYGKAGESKTEQSPKAM
                        70        80        90       100       110       120

                       130       140       150       160       170       180
orf120a.pep     DLFTLAWQLAANDAKLPPGLKITNGKKLYSVGGLNKAGTGKYSIGGVETEVVKYRVRRGD
                ||||||||||||||||||||||||||||||||||||||||||||||||||||||||||||
orf120-1        DLFTLAWQLAANDAKLPPGLKITNGKKLYSVGGLNKAGTGKYSIGGVETEVVKYRVRRGD
                       130       140       150       160       170       180

                       190       200       210       220
orf120a.pep     DAVMYFFAPSLNNIPAQIGYTDDGKTYTLKLKSVQINGQAAKPX
                ||||||||||||||||||||||||||||||||||||||||||||
orf120-1        DAVMYFFAPSLNNIPAQIGYTDDGKTYTLKLKSVQINGQAAKPX
                       190       200       210       220
```

<u>Homology with a predicted ORF from *N.gonorrhoeae*</u>

ORF120 shows 97.8% identity over 184 aa overlap with a predicted ORF (ORF120ng) from *N.gonorrhoeae:*

```
orf120.pep                                  IPATMTFERSGNAYKIVSTIKVPLYNIRFE   30
                                            ||||||||||||||||||||||||||||||
orf120ng     SAAILSAALPCAYAARLPQSAVLHYSGSYGIPATMTFERSGNAYKIVSTIKVPLYNIRFE   69

orf120.pep   SGGTVVGNTLHPTYYRDIRRGKLYAEAKFADGSVTYGKAGESKTEQSPKAMDLFTLAWQL   90
             ||||||||||||:||:||||||||||||||||||||||||||||||||||||||||||||
orf120ng     SGGTVVGNTLHPAYYKDIRRGKLYAEAKFADGSVTYGKAGESKTEQSPKAMDLFTLAWQL  129

orf120.pep   AANDAKLPPGLKITNGKKLYSVGGLNKAGTGKYSIGGVETEVVKYRVRRGDDAVMYFFAP  150
             |||||||||||||||||||||||||||||||||||||||||||||||||||||:| |||||
orf120ng     AANDAKLPPGLKITNGKKLYSVGGLNKAGTGKYSIGGVETEVVKYRVRRGDDTVTYFFAP  189

orf120.pep   SLNNIPAQIGYTDDGKTYTLKLKSVQINGQAAKP  184
             ||||||||||||||||||||||||||||||||||
orf120ng     SLNNIPAQIGYTDDGKTYTLKLKSVQINGQAAKP  223
```

The complete length ORF120ng nucleotide sequence <SEQ ID 779> is:

```
  1  ATGATGAAGA CTTTTAAAAA TATATTTTCC GCCGCCATTT TGTCCGCCGC
 51  CCTGCCGTGC GCGTATGCGG CAAGGCTACC CCAATCCGCC GTGCTGCACT
101  ATTCCGGCAG CTACGGCATT CCCGCCACGA TGACATTTGA ACGCAGCGGC
151  AATGCTTACA AAATCGTTTC GACGATTAAA GTGCCGCTAT ACAATATCCG
201  TTTCGAATCC GGCGGTACGG TTGTCGGCAA TACCCTGCAC CCTGCCTACT
251  ATAAAGACAT ACGCAGGGGC AAACTGTATG CGGAAGCCAA ATTCGCCGAC
301  GGCAGCGTAA CCTACGGCAA AGCGGGCGAG AGCAAAACCG AGCAAAGCCC
351  CAAGGCTATG GATTTGTTCA CGCTTGCCTG GCAGTTGGCG GCAAATGACG
401  CGAAACTCCC CCCGGGTCTG AAAATCACCA ACGGCAAAAA ACTTTATTCC
451  GTCGGCGGCC TGAATAAGGC GGGTACGGGA AAATACAGCA TaggCGGCGT
501  GGAAACCGAA GTCGTCAAAT ATCGGGTGCG GCGCGGCGAC GATACGGTAA
551  CGTATTTCTT CGCACCGTCC CTGAACAATA TTCCGGCACA AATCGGCTAT
601  ACCGACGACG GCAAAACCTA TACGCTGAAG CTCAAATCGG TGCAGATCAA
651  CGGACAGGCC GCCAAACCGT AA
```

This encodes a protein having amino acid sequence <SEQ ID 780>:

```
  1  MMKTFKNIFS AAILSAALPC AYAARLPQSA VLHYSGSYGI PATMTFERSG
 51  NAYKIVSTIK VPLYNIRFES GGTVVGNTLH PAYYKDIRRG KLYAEAKFAD
101  GSVTYGKAGE SKTEQSPKAM DLFTLAWQLA ANDAKLPPGL KITNGKKLYS
151  VGGLNKAGTG KYSIGGVETE VVKYRVRRGD DTVTYFFAPS LNNIPAQIGY
201  TDDGKTYTLK LKSVQINGQA AKP*
```

In comparison with ORF120-1, ORF120ng shows 97.8% identity in 223 aa overlap:

```
                    10        20        30        40        50        60
orf120-1.pep  MMKTFKNIFSAAILSAALPCAYAAGLPQSAVLHYSGSYGIPATMTFERSGNAYKIVSTIK
              ||||||||||||||||||||||||| ||||||||||||||||||||||||||||||||||
orf120ng      MMKTFKNIFSAAILSAALPCAYAARLPQSAVLHYSGSYGIPATMTFERSGNAYKIVSTIK
                    10        20        30        40        50        60

                    70        80        90       100       110       120
orf120-1.pep  VPLYNIRFESGGTVVGNTLHPTYYRDIRRGKLYAEAKFADGSVTYGKAGESKTEQSPKAM
              ||||||||||||||||||||:||:||||||||||||||||||||||||||||||||||||
orf120ng      VPLYNIRFESGGTVVGNTLHPAYYKDIRRGKLYAEAKFADGSVTYGKAGESKTEQSPKAM
                    70        80        90       100       110       120

                   130       140       150       160       170       180
orf120-1.pep  DLFTLAWQLAANDAKLPPGLKITNGKKLYSVGGLNKAGTGKYSIGGVETEVVKYRVRRGD
              ||||||||||||||||||||||||||||||||||||||||||||||||||||||||||||
orf120ng      DLFTLAWQLAANDAKLPPGLKITNGKKLYSVGGLNKAGTGKYSIGGVETEVVKYRVRRGD
                   130       140       150       160       170       180

                   190       200       210       220
orf120-1.pep  DAVMYFFAPSLNNIPAQIGYTDDGKTYTLKLKSVQINGQAAKPX
              |:| ||||||||||||||||||||||||||||||||||||||||
orf120ng      DTVTYFFAPSLNNIPAQIGYTDDGKTYTLKLKSVQINGQAAKPX
                   190       200       210       220
```

This analysis, including the presence of a putative leader sequence in the gonococcal protein suggests that the proteins from *N.meningitidis* and *N.gonorrhoeae,* and their epitopes, could be useful antigens for vaccines or diagnostics, or for raising antibodies.

**Example 93**

The following partial DNA sequence was identified in *N.meningitidis* <SEQ ID 781>:

```
  1 ATGTATCGGA GGAAAGGGCG GGGCATCAAG CCGTGGATGG GTGCCGGTGC
 51 .GCGTTTGCC GCCTTGGTCT GGCTGGTTTT CGCGCTCGGC GATACTTTGA
101 CTCCGTTTGC GGTTGCGGCG GTGCTGGCGT ATGTATTGGA CCCTTTGGTC
151 GAATGGTTGC AGAAAAAGGG TTTGAACCGT GCATCCGCTT CGATGTCTGT
201 GATGGTGTTT TCCTTGATTT TGTTGTTGGC ATTATTGTTG ATTATCGTCC
251 CTATGCTGGT CGGGCAGTTC AACAATTTGG CATCGCGCCT GCCCCAATTA
301 ATCGGTTTTA TGCAGAACAC GCTGCTGCCG TGGTTGAAAA ATACAATCGG
351 CGGATATGTG GAAATCGATC AGGCATCTAT TATTGCGTGG CTTCAGGCGC
401 ATACGGGAGA GTTGAGCAAC GCGCTTAAGG CGTGGTTTCC CGTTTTGATG
451 AGGCAGGGCG GCAATATT..
```

This corresponds to the amino acid sequence <SEQ ID 782; ORF121>:

```
  1 MYRRKGRGIK PWMGAGXAFA ALVWLVFALG DTLTPFAVAA VLAYVLDPLV
 51 EWLQKKGLNR ASASMSVMVF SLILLLALLL IIVPMLVGQF NNLASRLPQL
101 IGFMQNTLLP WLKNTIGGYV EIDQASIIAW LQAHTGELSN ALKAWFPVLM
151 RQGGNI..
```

Further work revealed the complete nucleotide sequence <SEQ ID 783>:

```
   1  ATGTATCGGA GGAAAGGGCG GGGCATCAAG CCGTGGATGG GTGCCGGTGC
  51  GGCGTTTGCC GCCTTGGTCT GGCTGGTTTT CGCGCTCGGC GATACTTTGA
 101  CTCCGTTTGC GGTTGCGGCG GTGCTGGCGT ATGTATTGGA CCCTTTGGTC
 151  GAATGGTTGC AGAAAAAGGG TTTGAACCGT GCATCCGCTT CGATGTCTGT
 201  GATGGTGTTT TCCTTGATTT TGTTGTTGGC ATTATTGTTG ATTATCGTCC
 251  CTATGCTGGT CGGGCAGTTC AACAATTTGG CATCGCGCCT GCCCCAATTA
 301  ATCGGTTTTA TGCAGAACAC GCTGCTGCCG TGGTTGAAAA ATACAATCGG
 351  CGGATATGTG GAAATCGATC AGGCATCTAT TATTGCGTGG CTTCAGGCGC
 401  ATACGGGAGA GTTGAGCAAC GCGCTTAAGG CGTGGTTTCC CGTTTTGATG
 451  AGGCAGGGCG GCAATATTGT CAGCAGTATC GGCAACCTGC TGCTGCTTCC
 501  CTTGCTGCTT TACTATTTCC TGCTGGATTG GCAGCGGTGG TCGTGCGGCA
 551  TTGCCAAACT GGTTCCGAgG CGTTTTGCCG GTGCTTATAC GCGCATTACA
 601  GGCAATTTGA ACGAGGTATT GGGCGAATTT TTGCGCGGGC AGCTTCTGGT
```

```
 651  AATGCTGATT ATGGGCTTGG TTTACGGTTT GGGATTGGTG CTGGTCGGGC
 701  TGGATTCGGG GTTTGCCATC GGTATGCTTG CCGGTATTTT GGTGTTTGTC
 751  CCTTATCTCG GGGCGTTTAC GGGATTGCTG CTTGCCACCG TCGCCGCCTT
 801  GCTCCAGTTC GGTTCGTGGA ACGGCATCCT ATCGGTTTGG GCGGTTTTTG
 851  CCGTAGGACA GTTTCTCGAA AGTTTTTTCA TTACGCCGAA AATCGTGGGA
 901  GACCGTATCG GGCTGTCGCC GTTTTGGGTT ATCTTTTCGC TGATGGCGTT
 951  CGGGCAGCTG ATGGGCTTTG TCGGAATGTT GGCGGGATTG CCTTTGGCCG
1001  CCGTAACCTT GGTCTTGCTT CGCGAGGGCG TGCAGAAATA TTTTGCCGGC
1051  AGTTTTTACC GGGGCAGGTA G
```

This corresponds to the amino acid sequence <SEQ ID 784; ORF121-1>:

```
   1  MYRRKGRGIK PWMGAGAAFA ALVWLVFALG DTLTPFAVAA VLAYVLDPLV
  51  EWLQKKGLNR ASASMSVMVF SLILLLALLL IIVPMLVGQF NNLASRLPQL
 101  IGFMQNTLLP WLKNTIGGYV EIDQASIIAW LQAHTGELSN ALKAWFPVLM
 151  RQGGNIVSSI GNLLLLPLLL YYFLLDWQRW SCGIAKLVPR RFAGAYTRIT
 201  GNLNEVLGEF LRGQLLVMLI MGLVYGLGLV LVGLDSGFAI GMLAGILVFV
 251  PYLGAFTGLL LATVAALLQF GSWNGILSVW AVFAVGQFLE SFFITPKIVG
 301  DRIGLSPFWV IFSLMAFGQL MGFVGMLAGL PLAAVTLVLL REGVQKYFAG
 351  SFYRGR*
```

Computer analysis of this amino acid sequence gave the following results:

Homology with a predicted ORF from *N.meningitidis* (strain A)

ORF121 shows 98.7% identity over a 156aa overlap with an ORF (ORF 121 a) from strain A of *N. meningitidis:*

```
                         10        20        30        40        50        60
orf121.pep     MYRRKGRGIKPWMGAGXAFAALVWLVFALGDTLTPFAVAAVLAYVLDPLVEWLQKKGLNR
               ||||||||||||| || |||||||||||||||||||||||||||||||||||||||||||
orf121a        MYRRKGRGIKPWMDAGAAFAALVWLVFALGDTLTPFAVAAVLAYVLDPLVEWLQKKGLNR
                         10        20        30        40        50        60

                         70        80        90       100       110       120
orf121.pep     ASASMSVMVFSLILLLALLLIIVPMLVGQFNNLASRLPQLIGFMQNTLLPWLKNTIGGYV
               ||||||||||||||||||||||||||||||||||||||||||||||||||||||||||||
orf121a        ASASMSVMVFSLILLLALLLIIVPMLVGQFNNLASRLPQLIGFMQNTLLPWLKNTIGGYV
                         70        80        90       100       110       120

                        130       140       150
orf121.pep     EIDQASIIAWLQAHTGELSNALKAWFPVLMRQGGNI
               ||||||||||||||||||||||||||||||||||||
orf121a        EIDQASIIAWLQAHTGELSNALKAWFPVLMRQGGNIVSSIGNLLLLPLLLYYFLLDWQRW
                        130       140       150       160       170       180

orf121a        SCGIAKLVPRRFAGAYTRITGNLNEVLGEFLRGQLLVMLIMGLVYGLGLVLVGLDSGFAI
                        190       200       210       220       230       240
```

The complete length ORF121a nucleotide sequence <SEQ ID 785> is:

```
   1  ATGTATCGGA GGAAAGGGCG GGGCATCAAG CCGTGGATGG ATGCCGGTGC
  51  GGCGTTTGCC GCCTTGGTCT GGCTGGTTTT CGCGCTCGGC GATACTTTGA
 101  CTCCGTTTGC GGTTGCGGCG GTGCTGGCGT ATGTATTGGA CCCTTTGGTC
 151  GAATGGTTGC AGAAAAAGGG TTTGAACCGT GCATCCGCTT CGATGTCTGT
 201  GATGGTGTTT TCCTTGATTT TGTTGTTGGC ATTATTGTTG ATTATTGTCC
 251  CTATGCTGGT CGGGCAGTTC AACAATTTGG CATCGCGCCT GCCCCAATTA
 301  ATCGGTTTTA TGCAGAACAC GCTGCTGCCG TGGTTGAAAA ATACAATCGG
 351  CGGATATGTG GAAATCGATC AGGCATCTAT TATTGCGTGG CTTCAGGCGC
 401  ATACGGGCGA GTTGAGCAAC GCGCTTAAGG CGTGGTTTCC CGTTTTGATG
 451  AGGCAGGGCG GCAATATTGT CAGCAGTATC GGCAACCTGC TGCTGCTTCC
 501  CTTGCTGCTT TACTATTTCC TGCTGGATTG GCAGCGGTGG TCGTGCGGCA
 551  TTGCCAAACT GGTTCCGAGG CGTTTTGCCG GTGCTTATAC GCGCATTACA
 601  GGCAATTTGA ACGAGGTATT GGGCGAATTT TTGCGCGGGC AGCTTCTGGT
 651  GATGCTGATT ATGGGTTTGG TTTACGGCTT GGGGTTGGTG CTGGTCGGGC
 701  TGGATTCGGG GTTTGCAATC GGTATGGTTG CCGGTATTTT GGTTTTTGTT
 751  CCCTATTTGG GCGCGTTTAC AGGACTGCTG CTGGCAACCG TCGCCGCCTT
 801  GCTCCAGTTC GGTTCGTGGA ACGGCATCTT GGCTGTTTGG GCGGTTTTTG
 851  CCGTAGGACA GTTTCTCGAA AGTTTTTTCA TTACGCCGAA AATCGTGGGA
 901  GACCGTATCG GCCTGTCGCC GTTTTGGGTT ATCTTTTCGC TGATGGCGTT
 951  CGGGCAGCTG ATGGGCTTTG TCGGAATGTT GGCCGGATTG CCTTTGGCCG
1001  CCGTAACCTT GGTCTTGCTT CGCGAGGGCG TGCAGAAATA TTTTGCCGGC
1051  AGTTTTTACC GGGGCAGGTA G
```

This encodes a protein having amino acid sequence <SEQ ID 786>:

```
   1  MYRRKGRGIK PWMDAGAAFA ALVWLVFALG DTLTPFAVAA VLAYVLDPLV
  51  EWLQKKGLNR ASASMSVMVF SLILLLALLL IIVPMLVGQF NNLASRLPQL
 101  IGFMQNTLLP WLKNTIGGYV EIDQASIIAW LQAHTGELSN ALKAWFPVLM
 151  RQGGNIVSSI GNLLLLPLLL YYFLLDWQRW SCGIAKLVPR RFAGAYTRIT
 201  GNLNEVLGEF LRGQLLVMLI MGLVYGLGLV LVGLDSGFAI GMVAGILVFV
 251  PYLGAFTGLL LATVAALLQF GSWNGILAVW AVFAVGQFLE SFFITPKIVG
 301  DRIGLSPFWV IFSLMAFGQL MGFVGMLAGL PLAAVTLVLL REGVQKYFAG
 351  SFYRGR*
```

ORF121a and ORF121-1 show99.2% identity in 356 aa overlap:

```
                      10        20        30        40        50        60
orf121a.pep   MYRRKGRGIKPWMDAGAAFAALVWLVFALGDTLTPFAVAAVLAYVLDPLVEWLQKKGLNR
              ||||||||||||| ||||||||||||||||||||||||||||||||||||||||||||||
orf121-1      MYRRKGRGIKPWMGAGAAFAALVWLVFALGDTLTPFAVAAVLAYVLDPLVEWLQKKGLNR
                      10        20        30        40        50        60

                      70        80        90       100       110       120
orf121a.pep   ASASMSVMVFSLILLLALLLIIVPMLVGQFNNLASRLPQLIGFMQNTLLPWLKNTIGGYV
              ||||||||||||||||||||||||||||||||||||||||||||||||||||||||||||
orf121-1      ASASMSVMVFSLILLLALLLIIVPMLVGQFNNLASRLPQLIGFMQNTLLPWLKNTIGGYV
                      70        80        90       100       110       120

                     130       140       150       160       170       180
orf121a.pep   EIDQASIIAWLQAHTGELSNALKAWFPVLMRQGGNIVSSIGNLLLLPLLLYYFLLDWQRW
              ||||||||||||||||||||||||||||||||||||||||||||||||||||||||||||
orf121-1      EIDQASIIAWLQAHTGELSNALKAWFPVLMRQGGNIVSSIGNLLLLPLLLYYFLLDWQRW
                     130       140       150       160       170       180

                     190       200       210       220       230       240
orf121a.pep   SCGIAKLVPRRFAGAYTRITGNLNEVLGEFLRGQLLVMLIMGLVYGLGLVLVGLDSGFAI
              ||||||||||||||||||||||||||||||||||||||||||||||||||||||||||||
orf121-1      SCGIAKLVPRRFAGAYTRITGNLNEVLGEFLRGQLLVMLIMGLVYGLGLVLVGLDSGFAI
                     190       200       210       220       230       240

                     250       260       270       280       290       300
orf121a.pep   GMVAGILVFVPYLGAFTGLLLATVAALLQFGSWNGILAVWAVFAVGQFLESFFITPKIVG
              ||:|||||||||||||||||||||||||||||||||:|||||||||||||||||||||||
orf121-1      GMLAGILVFVPYLGAFTGLLLATVAALLQFGSWNGILSVWAVFAVGQFLESFFITPKIVG
                     250       260       270       280       290       300

                     310       320       330       340       350
orf121a.pep   DRIGLSPFWVIFSLMAFGQLMGFVGMLAGLPLAAVTLVLLREGVQKYFAGSFYRGRX
              |||||||||||||||||||||||||||||||||||||||||||||||||||||||||
orf121-1      DRIGLSPFWVIFSLMAFGQLMGFVGMLAGLPLAAVTLVLLREGVQKYFAGSFYRGRX
                     310       320       330       340       350
```

Homology with a predicted ORF from *N.gonorrhoeae*

ORF121 shows 97.4% identity over a 156 aa overlap with a predicted ORF (ORF121ng) from *N.gonorrhoeae:*

```
orf121.pep   MYRRKGRGIKPWMGAGXAFAALVWLVFALGDTLTPFAVAAVLAYVLDPLVEWLQKKGLNR   60
             |||||||||||||||| |||||||||:|||||||||||||||||||||||||||||||||
orf121ng     MYRRKGRGIKPWMGAGAAFAALVWLVYALGDTLTPFAVAAVLAYVLDPLVEWLQKKGLNR   60

orf121.pep   ASASMSVMVFSLILLLALLLIIVPMLVGQFNNLASRLPQLIGFMQNTLLPWLKNTIGGYV  120
             ||||||||||||||||||||||||||||||||||||||||||||||||||||||||||||
orf121ng     ASASMSVMVFSLILLLALLLIIVPMLVGQFNNLASRLPQLIGFMQNTLLPWLKNTIGGYV  120

orf121.pep   EIDQASIIAWLQAHTGELSNALKAWFPVLMRQGGNI                         156
             ||||||||||:||||||||||||||||||||:|||||
orf121ng     EIDQASIIAWFQAHTGELSNALKAWFPVLMKQGGNIVSTIGNLLLPPLLLYYFLLDWHRW  180
```

An ORF121ng nucleotide sequence <SEQ ID 787> was predicted to encode a protein having amino acid sequence <SEQ ID 788>:

```
  1  MYRRKGRGIK PWMGAGAAFA ALVWLVYALG DTLTPFAVAA VLAYVLDPLV
 51  EWLQKKGLNR ASASMSVMVF SLILLLALLL IIVPMLVGQF NNLASRLPQL
101  IGFMQNTLLP WLKNTIGGYV EIDQASIIAW FQAHTGELSN ALKAWFPVLM
151  KQGGNIVSTI GNLLLPPLLL YYFLLDWHRW SCGIPKLVPR RFAGAYTRIT
201  GNLNKVWGKF LRGQLLGETE RGAVVCRVGR ECWEGGGARS RPSDDGWPRW
251  GGG*
```

Further work revealed the following gonoccocal DNA sequence <SEQ ID 789>:

```
   1  ATGTATCGGA GAAAAGGACG GGGCATCAAG CCGTGGATGG GTGCCGGCGC
  51  GGCGTTTGCC GCCTTGGTCT GGCTGGTTTA CGCGCTCGGC GATACTTTGA
 101  CTCCGTTTGC GGTTGCGGCG GTGCTGGCGT ATGTGTTGGA CCCTTTGGTC
 151  GAATGGTTGC AGAAAAAGGG TTTGAACCGT GCATCCGCTT CGATGTCTGT
 201  GATGGTGTTT TCCTTGATTT TGTTGTTGGC ATTATTGTTG ATTATTGTCC
 251  CTATGCTGGT CGGGCAGTTC AATAATTTGG CATCTCGCCT GCCCCAATTA
 301  ATCGGTTTTA TGCAGAACAC GCTGCTGCCG TGGTTGAAAA ATACAATCGG
 351  CGGATATGTG GAAATCGATC AGGCATCTAT TATTGCGTGG TTTCAGGCGC
 401  ATACGGGCGA GTTGAGCAAC GCGCTTAAGG CGTGGTTTCC CGTTTTGATG
 451  AAACAGGGCG GCAATATTGT CAGCAGTATC GGCAACCTGC TGCTGCCGCC
 501  CTTGCTGCTT TACTATTTCC TGCTGGATTG GCAGCGGTGG TCGTGCGGCA
 551  TCGCCAAACT GGTTCCGAGG CGTTTTGCCG GTGCTTATAC GCGCATTACG
 601  GGTAATTTGA ACGAGGTATT GGGCGAATTT TTGCGCGGTC AGCTTCTGGT
 651  GATGCTGATT ATGGGCTTGG TTTACGGTTT GGGATTGATG CTAGTCGGAC
 701  TGGATTCGGG ATTTGCCATC GGTATGGTTG CCGGTATTTT GGTGTTTGTC
 751  CCCTATTTGG GTGCGTTTAC GGGATTGCTG CTTGCCACTG TTGCAGCCTT
 801  GCTCCAGTTC GGTTCGTGGA ACGGAATCTT GGCTGTTTGG GCGGTTTTTG
 851  CCGTCGGTCA GTTTCTCGAA AGTTTTTTCA TTACGCCGAA AATTGTAGGA
 901  GACCGTATCG GCCTGTCGCC GTTTTGGGTT ATCTTTTCGC TGATGGCGTT
 951  CGGAGAGCTG ATGGGCTTTG TCGGAATGTT GGCCGGATTG CCTTTGGCCG
1001  CCGTAACCTT GGTCTTGCTT CGCGAGGGCG CGCAGAAATA TTTTGCCGGC
1051  AGTTTTTACC GGGGCAGGTA G
```

This corresponds to the amino acid sequence <SEQ ID 790; ORF121ng-1>:

```
  1  MYRRKGRGIK PWMGAGAAFA ALVWLVYALG DTLTPFAVAA VLAYVLDPLV
 51  EWLQKKGLNR ASASMSVMVF SLILLLALLL IIVPMLVGQF NNLASRLPQL
101  IGFMQNTLLP WLKNTIGGYV EIDQASIIAW FQAHTGELSN ALKAWFPVLM
151  KQGGNIVSSI GNLLLPPLLL YYFLLDWQRW SCGIAKLVPR RFAGAYTRIT
201  GNLNEVLGEF LRGQLLVMLI MGLVYGLGLM LVGLDSGFAI GMVAGILVFV
251  PYLGAFTGLL LATVAALLQF GSWNGILAVW AVFAVGQFLE SFFITPKIVG
301  DRIGLSPFWV IFSLMAFGEL MGFVGMLAGL PLAAVTLVLL REGAQKYFAG
351  SFYRGR*
```

ORF121ng-1 and ORF121-1 show 97.5% identity in 356 aa overlap:

```
                      10        20        30        40        50        60
orf121-1.pep  MYRRKGRGIKPWMGAGAAFAALVWLVFALGDTLTPFAVAAVLAYVLDPLVEWLQKKGLNR
              ||||||||||||||||||||||||||:|||||||||||||||||||||||||||||||||
orf121ng-1    MYRRKGRGIKPWMGAGAAFAALVWLVYALGDTLTPFAVAAVLAYVLDPLVEWLQKKGLNR
                      10        20        30        40        50        60

                      70        80        90       100       110       120
orf121-1.pep  ASASMSVMVFSLILLLALLLIIVPMLVGQFNNLASRLPQLIGFMQNTLLPWLKNTIGGYV
              ||||||||||||||||||||||||||||||||||||||||||||||||||||||||||||
orf121ng-1    ASASMSVMVFSLILLLALLLIIVPMLVGQFNNLASRLPQLIGFMQNTLLPWLKNTIGGYV
                      70        80        90       100       110       120

                     130       140       150       160       170       180
orf121-1.pep  EIDQASIIAWLQAHTGELSNALKAWFPVLMRQGGNIVSSIGNLLLLPLLLYYFLLDWQRW
              ||||||||||:|||||||||||||||||||:|||||||||||||| ||||||||||||||
orf121ng-1    EIDQASIIAWFQAHTGELSNALKAWFPVLMKQGGNIVSSIGNLLLPPLLLYYFLLDWQRW
                     130       140       150       160       170       180

                     190       200       210       220       230       240
orf121-1.pep  SCGIAKLVPRRFAGAYTRITGNLNEVLGEFLRGQLLVMLIMGLVYGLGLVLVGLDSGFAI
              |||||||||||||||||||||||||||||||||||||||||||||||||:||||||||||
orf121ng-1    SCGIAKLVPRRFAGAYTRITGNLNEVLGEFLRGQLLVMLIMGLVYGLGLMLVGLDSGFAI


                     190       200       210       220       230       240

                     250       260       270       280       290       300
orf121-1.pep  GMLAGILVFVPYLGAFTGLLLATVAALLQFGSWNGILSVWAVFAVGQFLESFFITPKIVG
              ||:||||||||||||||||||||||||||||||||||:||||||||||||||||||||||
orf121ng-1    GMVAGILVFVPYLGAFTGLLLATVAALLQFGSWNGILAVWAVFAVGQFLESFFITPKIVG
                     250       260       270       280       290       300

                     310       320       330       340       350
orf121-1.pep  DRIGLSPFWVIFSLMAFGQLMGFVGMLAGLPLAAVTLVLLREGVQKYFAGSFYRGRX
              ||||||||||||||||||:||||||||||||||||||||||||:|||||||||||||
orf121ng-1    DRIGLSPFWVIFSLMAFGELMGFVGMLAGLPLAAVTLVLLREGAQKYFAGSFYRGRX
                     310       320       330       340       350
```

In addition, ORF121ng-1 shows homology to a permease from *H.influenzae:*

```
sp|P43969|PERM_HAEIN PUTATIVE PERMEASE PERM HOMOLOG Length = 349
Score = 69.9 bits (168), Expect = 2e-11
Identities = 67/317 (21%), Positives = 120/317 (37%), Gaps = 7/317 (2%)

Query: 26  VYALGDTLTPFAVAAVLAYVLDPLVEWL-QKKGLNRASASMSVMVFSXXXXXXXXXXXVP 84
            +Y  GD + P  +A VL+Y+L+  + +L Q     R  A++ +               VP
Sbjct: 32  IYFFGDLIAPLLIALVLSYLLEIPINFLNQYLKCPRMLATILIFGSFIGLAAVFFLVLVP 91

Query: 85  MLVGQFNNLASRLPQLIGFMQNTLLPWLKNTIGGYVE-IDQASIIAWFQAHTGELSNALK 143
            ML  Q  +L S LP +     N    WL N    Y E ID + + + F +    ++     +
Sbjct: 92  MLWNQTISLLSDLPAMF----NKSNEWLLNLPKNYPELIDYSMVDSIFNSVREKILGFGE 147
Query: 144 AWFPVLMKQGGNIVSSIGNXXXXXXXXXXXXXDWQRWSCGIAKLVPRRFAGAYTRITGNL 203

            +    + +     N+VS                       D          G+++ +P++    A+ R     +
Sbjct: 148 SAVKLSLASIMNLVSLGIYAFLVPLMMFFMLKDKSELLQGVSRFLPKNRNLAFXRWK-EM 206

Query: 204 NEVLGEFLRGQXXXXXXXXXXXXXXXXXXXDSGFAIGMVAGILVFVPYXXXXXXXXXXX 263
            + +  ++ G+                        +     +     G+ V VPY
Sbjct: 207 QQQISNYIHGKLLEILIVTLITYIIFLIFGLNYPLLLAFAVGLSVLVPYIGAVIVTIPVA 266

Query: 264 XXXXXQFGSWNGILAVWAVFAVGQFLESFFITPKIVGDRIGLSPFWVIFSLMAFGELMGF 323
                 QFG        +     FAV Q L+    + P +    + + L P   +I S++ FG L GF
Sbjct: 267 LVALFQFGISPTFWYIIIAFAVSQLLDGNLLVPYLFSEAVNLHPLIIIISVLIFGGLWGF 326

Query: 324 VGMLAGLPLAAVTLVLL 340
            G+    +PLA +    ++
Sbjct: 327 WGVFFAIPLATLVKAVI 343
```

Based on this analysis, including the presence of a putative leader sequence and transmembrane domains in the two proteins, it is predicted that the proteins from *N.meningitidis* and *N.gonorrhoeae,* and their epitopes, could be useful antigens for vaccines or diagnostics, or for raising antibodies.

**Example 94**

The following partial DNA sequence was identified in *N.meningitidis* <SEQ ID 791>:

```
  1  ..ACTGCTTTTT CGGCGGCGCT GCGCTTGAGT CCATCATGAC TCGTCATATT
 51    TTTGTCCTTT GGGAAACCGT ATCAACAAAC AGCCGCCATC TTAACATTTT
101    TTTGCACGTC CTGCCCGCCG CGTTCAAATG CGTACCAGCA ATACCGCCGC
151    CTGCGCCTCT ATGCCTTCCA TCCGCCCGAG ATAGCCGAGT TTTTCGTTGG
201    TTTTGCCTTT GATGTTGACG CACGAAATGT CTATGCCCAA ATCGGCGGCG
251    ATGTTGGCAC GCATTTGCGG AATGTGCGGC GCGAGTGTGG GTTTCTGTGC
301    AATCACGGTC GTATCGACAT TGACCGCCTG CCAACCCTGC GCCTGAACGC
351    TTTGATACGC CGCACGCAAA AGGACGCGGC TGTCCGCATC TTTGAACTCT
401    GCGGCGGTGT CGGGGAAATG GCTGCCGATA TCGCCCAAAC CTGCCGCACC
451    GAGCAGCGCG TCGGTAACGG CGTGCAGCAG CGCATCGGCA TCGGAGTGTC
501    CGAGCAGCCC TTTTTCAAAT GGGATTTCAA CTCCGCCAAG TATCAG..
```

This corresponds to the amino acid sequence <SEQ ID 792; ORF122>:

```
  1  ..TAFSAALRLS PSXLVIFLSF GKPYQQTAAI LTFFCTSCPP RSNAYQQYRR
 51    LRLYAFHPPE IAEFFVGFAF DVDARNVYAQ IGGDVGTHLR NVRRECGFLC
101    NHGRIDIDRL PTLRLNALIR RTQKDAAVRI FELCGGVGEM AADIAQTCRT
151    EQRVGNGVQQ RIGIGVSEQP FFKWDFNSAK YQ..
```

Further work revealed the complete nucleotide sequence <SEQ ID 793>:

```
  1 ATATCGTACT GGGCAAGCAG TTCGCCGGAT TTTTTGGAAG TAGATACCGC
 51 GCCTTTGATT TTTTTGCCGC TCTTACCCAA GGCTTCGATG AAAAAGTTGA
101 TGGTCGAGCC GGTACCGATG CCGATATATT CATTTTCGGG TACGAATTCG
151 ACTGCTTTTT CGGCGGCGAT GCGCTTGAGT TCGTCTTGTG TCGTCATATT
201 TTTGTCCTTT GGGAAACCGT ATCAACAAAC AGCCGCCATC TTAACATTTT
251 TTTGCACGTC CTGCCCGCCG CGTTCAAATG CGTACCAGCA ATACCGCCGC
301 CTGCGCCTCT ATGCCTTCCA TCCGCCCGAG ATAGCCGAGT TTTTCGTTGG
351 TTTTGCCTTT GATGTTGACG CACGAAATGT CTATGCCCAA ATCGGCGGCG
401 ATGTTGGCAC GCATTTGCGG AATGTGCGGC GCGAGTTTGG GTTTCTGTGC
451 AATCACGGTC GTATCGACAT TGACCGCCTG CCAACCCTGC GCCTGAACGC
501 TTTGATACGC CGCACGCAAA AGGACGCGGC TGTCCGCATC TTTGAACTCT
551 GCGGCGGTGT CGGGGAAATG GCTGCCGATA TCGCCCAAAC CTGCCGCACC
601 GAGCAGCGCG TCGGTAACGG CGTGCAGCAG CGCATCGGCA TCGGAGTGTC
651 CGAGCAGCCC TTTTTCAAAT GGGATTTCAA CTCCGCCAAG TATCAGCTTT
701 CTGCCTTCGG TCAGTTGGTG GACATCGTAG CCCTGTCCGA TACGGATGTT
751 CGTCATCGTT TGTGTTCCTG A
```

This corresponds to the amino acid sequence <SEQ ID 794; ORF122-1>:

```
  1 ISYWASSSPD FLEVDTAPLI FLPLLPKASM KKLMVEPVPM PIYSFSGTNS
 51 TAFSAAMRLS SSCVVIFLSF GKPYQQTAAI LTFFCTSCPP RSNAYQQYRR
101 LRLYAFHPPE IAEFFVGFAF DVDARNVYAQ IGGDVGTHLR NVRREFGFLC
151 NHGRIDIDRL PTLRLNALIR RTQKDAAVRI FELCGGVGEM AADIAQTCRT
201 EQRVGNGVQQ RIGIGVSEQP FFKWDFNSAK YQLSAFGQLV DIVALSDTDV
251 RHRLCS*
```

Computer analysis of this amino acid sequence gave the following results:

<u>Homology with a predicted ORF from *N.meningitidis* (strain A)</u>

ORF122 shows 94.0% identity over a 182aa overlap with an ORF (ORF122a) from strain A of *N. meningitidis:*

```
                                                10        20        30
orf122.pep                          TAFSAALRLSPSXLVIFLSFGKPYQQTAAI
                                    ||||||:||| | :|||||||||||||||
orf122a      FLPLLPKASMKKLMVEPVPMPMYSFSGTNSTAFSAAMRLSSSCVVIFLSFGKPYQQTAAI
                     30        40        50        60        70        80

                     40        50        60        70        80        90
orf122.pep   LTFFCTSCPPRSNAYQQYRRLRLYAFHPPEIAEFFVGFAFDVDARNVYAQIGGDVGTHLR
             |||| |||||||| |||||||||||| |||:|||||||| |||||||||||||||||
orf122a      LTFFXTSCPPRSNPYQQYRRLRLYAFHAPEITEFFVGFAFXVDARNVYAQIGGDVGTHLR
                     90       100       110       120       130       140

                    100       110       120       130       140       150
orf122.pep   NVRRECGFLCNHGRIDIDRLPTLRLNALIRRTQKDAAVRIFELCGGVGEMAADIAQTCRT
             |:||| |||||||||||||||||||||||||||||||||||||||||||||||||||||
orf122a      NMRREFGFLCNHGRIDIDRLPTLRLNALIRRTQKDAAVRIFELCGGVGEMAADIAQTCRT
                    150       160       170       180       190       200

                    160       170       180
orf122.pep   EQRVGNGVQQRIGIGVSEQPFFKWDFNSAKYQ
             ||||||||||||||||||||||||||||||||
orf122a      EQRVGNGVQQRIGIGVSEQPFFKWDFNSAKYQLSAFGQLVDIVALSDTDVRHRLCSX
                    210       220       230       240       250
```

The complete length ORF122a nucleotide sequence <SEQ ID 795> is:

```
  1  ATATCATATT GGGCAAGCAG TTCACTGGAT TTTTTGGAAG TAGATACCGC
 51  GCCTTTGATT TTTTTGCCGC TCTTACCCAA GGCTTCGATG AAAAAGTTGA
101  TGGTCGAACC GGTACCGATG CCGATGTATT CGTTTTCGGG TACGAATTCG
```

```
151  ACTGCNTTTT CGGCGGCGAT GCGCTTGAGT TCGTCTTGTG TCGTCATATT
201  TTTGTCCTTT GGGAAACCGT ATCAACAAAC AGCCGCCATC TTAACATTTT
251  TTNNNACGTC CTGCCCGCCG CGTTCAAATC CTTACCAGCA ATACCGCCGC
301  CTGCGACTCT ATGCCTTCCA TGCGCCCGAG ATAACCGAGT TTTTCGTTGG
351  TTTTGCCTTT GANGTTGACG CACGAAATGT CTATGCCCAA ATCGGCGGCG
401  ATGTTGGCAC GCATTTGCGG AATATGCGGC GCGAGTTTGG GTTTCTGTGC
451  AATCACGGTC GTATCGACAT TGACCGCCTG CCAACCCTGC GCCTGAACGC
501  TTTGATACGC CGCACGCAAA AGGACGCGGC TGTCCGCATC TTTGAACTCT
551  GCGGCGGTGT CGGGGAAATG GCTGCCGATA TCGCCCAAAC CTGCCGCACC
601  GAGCAGCGCG TCGGTAACGG CGTGCAGCAG CGCATCGGCA TCGGAGTGTC
651  CGAGCAGCCC TTTTTCAAAT GGGATTTCAA CTCCGCCAAG TATCAGCTTT
701  CTGCCTTCGG TCAGTTGGTG GACATCGTAG CCCTGTCCGA TACGGATGTT
751  CGTCATCGTT TGTGTTCCTG A
```

This encodes a protein having amino acid sequence <SEQ ID 796>:

```
  1  ISYWASSSLD FLEVDTAPLI FLPLLPKASM KKLMVEPVPM PMYSFSGTNS
 51  TAFSAAMRLS SSCVVIFLSF GKPYQQTAAI LTFFXTSCPP RSNPYQQYRR
101  LRLYAFHAPE ITEFFVGFAF XVDARNVYAQ IGGDVGTHLR NMRREFGFLC
151  NHGRIDIDRL PTLRLNALIR RTQKDAAVRI FELCGGVGEM AADIAQTCRT
201  EQRVGNGVQQ RIGIGVSEQP FFKWDFNSAK YQLSAFGQLV DIVALSDTDV
251  RHRLCS*
```

ORF122a and ORF122-1 show 96.9% identity in 256 aa overlap:

```
                      10        20        30        40        50        60
orf122a.pep   ISYWASSSLDFLEVDTAPLIFLPLLPKASMKKLMVEPVPMPMYSFSGTNSTAFSAAMRLS
              |||||||| ||||||||||||||||||||||||||||||||:||||||||||||||||||
orf122-1      ISYWASSSPDFLEVDTAPLIFLPLLPKASMKKLMVEPVPMPIYSFSGTNSTAFSAAMRLS
                      10        20        30        40        50        60

                      70        80        90       100       110       120
orf122a.pep   SSCVVIFLSFGKPYQQTAAILTFFXTSCPPRSNPYQQYRRLRLYAFHAPEITEFFVGFAF
              |||||||||||||||||||||||| |||||||| |||||||||||||| |||:|||||||
orf122-1      SSCVVIFLSFGKPYQQTAAILTFFCTSCPPRSNAYQQYRRLRLYAFHPPEIAEFFVGFAF
                      70        80        90       100       110       120

                     130       140       150       160       170       180
orf122a.pep   XVDARNVYAQIGGDVGTHLRNMRREFGFLCNHGRIDIDRLPTLRLNALIRRTQKDAAVRI
               |||||||||||||||||||:||||||||||||||||||||||||||||||||||||||||
orf122-1      DVDARNVYAQIGGDVGTHLRNVRREFGFLCNHGRIDIDRLPTLRLNALIRRTQKDAAVRI
                     130       140       150       160       170       180

                     190       200       210       220       230       240
orf122a.pep   FELCGGVGEMAADIAQTCRTEQRVGNGVQQRIGIGVSEQPFFKWDFNSAKYQLSAFGQLV
              ||||||||||||||||||||||||||||||||||||||||||||||||||||||||||||
orf122-1      FELCGGVGEMAADIAQTCRTEQRVGNGVQQRIGIGVSEQPFFKWDFNSAKYQLSAFGQLV
                     190       200       210       220       230       240

                     250
orf122a.pep   DIVALSDTDVRHRLCSX
              ||||||||||||||||
orf122-1      DIVALSDTDVRHRLCSX
                     250
```

Homology with a predicted ORF from *N.gonorrhoeae*

ORF122 shows 89.6% identity over a 182 aa overlap with a predicted ORF (ORF122ng) from *N.gonorrhoeae:*

```
orf122.pep                                        TAFSAALRLSPSXLVIFLSFGKPYQQTAAI   30
                                                  ||||||:||| | :|||||||||||||||
orf122ng   FLPLLPKASMKKLMVEPVPMPMYSFSGTNSTAFSAAMRLSSSCVVIFLSFGKPYQQTAAI   80

orf122.pep LTFFCTSCPPRSNAYQQYRRLRLYAFHPPEIAEFFVGFAFDVDARNVYAQIGGDVGTHLR   90
           ||||||| ||||| ||||||||||||||||||||||||||||:||||: :||||||||||
orf122ng   LTFFCTSWPPRSNPYQQYRRLRLYAFHPPEIAEFFVGFAFDIDARNIDTQIGGDVGTHLR  140

orf122.pep NVRRECGFLCNHGRIDIDRLPTLRLNALIRRTQKDAAVRIFELCGGVGEMAADIAQTCRT  150
           ||| | |||||||||||:||||||||||||||||||||||||||||||:||||:||||||
orf122ng   NVRCEFGFLCNHGRIDIDHLPTLRLNALIRRTQKDAAVRIFELCGGVGKMAADVAQTCRT  200

orf122.pep EQRVGNGVQQRIGIGVSEQPFFKWDFNSAKYQ                            182
           |||||||||||:|| : ||||||||||||||
orf122ng   EQRVGNGVQQRVGIRMPEQPFFKWDFNSAKYQLSAFGQLVDIVALSDTDIRHRLCS  256
```

The complete length ORF122ng nucleotide sequence <SEQ ID 797> is:

```
  1  ATGTCGTACC GGGCAAGCAG TTCGCCGGAT TTTTTGGAGG TTGAAACCGC
 51  GCCTTTGATT TTTTTACCGC TTTTGCCCAA GGCTTCGATG AAGAAATTGa
101  tgGTCGAACC GgtaCCGATG CCGATGTATT CGTTTTCGGG TACGAATTCG
151  ACTGCTTTTT CGGCGGCGAT GCGCttgAgt TCgtcttgcg TcgTCATATT
201  TTTAtccttt gGGAAaccct atcaAcaAAc agccgccatC TTAACATTTT
251  TTTGCACGtc ctggccgccg cgttcaAATc cgtaccaGca ataccgccgc
301  ctgcgcctCT AtgcCTTCCA TCCGCCCGAG ATAGCCGAGT TTTCGTTGG
351  TTTTGCCTTT GATatTGACG CACGAAATAT CGatacCCAa atcggcgGCG
401  ATGTTGGCAC GCATTTGCGG AATGTGCGGT GCGAGTTTGG GTTTCTGTGC
451  AATCACGGTC GTATCGACAT TGACCACCTG CCAACCCTGC GCCTGAACGC
501  TTTGATACGC CGCACGCAAA AGGACGCGGC TGTCCGCATC TTTGAACTCT
551  GCGGCGGTGT CGGGAAAATG GCTGCCGATG TCGCCCAAAC CTGCCGCACC
601  GAGCAGCgcg tcggtaaCGG CGTGCAGCAG cgcgTcgGCA TCCGAATGCC
651  CGAGCAGCCC TTTTTCAAAT GGGATTTCAA CTCCGCCAAG TATCAGCTTT
701  CTGCCTTCGG TCAATTGGTG GACATCGTAG CCCTGTCCGA TACGGATATT
751  CGTCATCGTT TGTGTTCCTG A
```

This encodes a protein having amino acid sequence <SEQ ID 798>:

```
  1  MSYRASSSPD FLEVETAPLI FLPLLPKASM KKLMVEPVPM PMYSFSGTNS
 51  TAFSAAMRLS SSCVVIFLSF GKPYQQTAAI LTFFCTSWPP RSNPYQQYRR
101  LRLYAFHPPE IAEFFVGFAF DIDARNIDTQ IGGDVGTHLR NVRCEFGFLC
151  NHGRIDIDHL PTLRLNALIR RTQKDAAVRI FELCGGVGKM AADVAQTCRT
201  EQRVGNGVQQ RVGIRMPEQP FFKWDFNSAK YQLSAFGQLV DIVALSDTDI
251  RHRLCS*
```

ORF122ng and ORF122-1 show 92.6% identity in 256 aa overlap:

```
                        10        20        30        40        50        60
orf122-1.pep    ISYWASSSPDFLEVDTAPLIFLPLLPKASMKKLMVEPVPMPIYSFSGTNSTAFSAAMRLS
                :|| ||||||||||:||||||||||||||||||||||||||:||||||||||||||||||
orf122ng        MSYRASSSPDFLEVETAPLIFLPLLPKASMKKLMVEPVPMPMYSFSGTNSTAFSAAMRLS
                        10        20        30        40        50        60

                        70        80        90       100       110       120
orf122-1.pep    SSCVVIFLSFGKPYQQTAAILTFFCTSCPPRSNAYQQYRRLRLYAFHPPEIAEFFVGFAF
                ||||||||||||||||||||||||||| ||||| ||||||||||||||||||||||||||
orf122ng        SSCVVIFLSFGKPYQQTAAILTFFCTSWPPRSNPYQQYRRLRLYAFHPPEIAEFFVGFAF
                        70        80        90       100       110       120

                       130       140       150       160       170       180
orf122-1.pep    DVDARNVYAQIGGDVGTHLRNVRREFGFLCNHGRIDIDRLPTLRLNALIRRTQKDAAVRI
                |:||||: :|||||||||||||| ||||||||||||||:|||||||||||||||||||||
orf122ng        DIDARNIDTQIGGDVGTHLRNVRCEFGFLCNHGRIDIDHLPTLRLNALIRRTQKDAAVRI
                       130       140       150       160       170       180

                       190       200       210       220       230       240
orf122-1.pep    FELCGGVGEMAADIAQTCRTEQRVGNGVQQRIGIGVSEQPFFKWDFNSAKYQLSAFGQLV
                ||||||||:||||:|||||||||||||||||:|| : |||||||||||||||||||||||
orf122ng        FELCGGVGKMAADVAQTCRTEQRVGNGVQQRVGIRMPEQPFFKWDFNSAKYQLSAFGQLV
                       190       200       210       220       230       240

                       250
orf122-1.pep    DIVALSDTDVRHRLCSX
                |||||||||:|||||||
orf122ng        DIVALSDTDIRHRLCSX
                       250
```

Based on this analysis, it is predicted that the proteins from *N.meningitidis* and *N.gonorrhoeae,* and their epitopes, could be useful antigens for vaccines or diagnostics, or for raising antibodies.

## Example 95

The following partial DNA sequence was identified in *N.meningitidis* <SEQ ID 799>:

```
  1  ..GCCGGCGCGA GTGCGAACAA CATTTCCGCG CGTTTTGCGG AAACACCCGT
 51    CGCTGTCAGC GTTACCCTGA TCGGCACGGT ACTTGCCGTC ATGCTGCCCG
101    TTACCGAATA TGAAAACTTC CTGCTGCTTA TCGGCTCGGT ATTTGCGCCG
151    ATGGGGCGGA TTTTGATTGC CGACTTTTTC GTCTTGAAAC GGCGTGA
```

This corresponds to the amino acid sequence <SEQ ID 800; ORFI25>:

```
 1  ..AGASANNISA RFAETPVAVS VTLIGTVLAV MLPVTEYENF LLLIGSVFAP
51    MGGFDCRLFR LETA*
```

Further work revealed the complete nucleotide sequence <SEQ ID 801>:

```
   1 ATGTCGGGCA ATGCCTCCTC TCCTTCATCT TCCTCCGCCA TCGGGCTGAT
  51 TTGGTTCGGC GCGGCGGTAT CGATTGCCGA AATCAGCACG GGTACGCTGC
 101 TTGCGCCTTT GGGCTGGCAG CGCGGTCTGG CGGCTCTACT TTTGGGTCAT
 151 GCCGTCGGCG GCGCGCTGTT TTTTGCGGCG GCGTATATCG GCGCACTGAC
 201 CGGACGCAGC TCGATGGAAA GCGTGCGCCT GTCGTTCGGC AAACGCGGTT
 251 CAGTGCTGTT TTCCGTGGCG AATATGCTGC AACTGGCCGG CTGGACGGCG
 301 GTGATGATTT ACGCCGGCGC AACGGTCAGC TCCGCTTTGG GCAAAGTGTT
 351 GTGGGACGGC GAATCTTTTG TCTGGTGGGC ATTGGCAAAC GGCGCGCTGA
 401 TTGTGCTGTG GCTGGTTTTC GGCGCACGCA AAACAGGCGG GCTGAAAACC
 451 GTTTCGATGC TGCTGATGCT GTTGGCGGTT CTGTGGCTGA GTGCCGAAGT
 501 CTTTTCCACG GCAGGCAGCA CCGCCGCACA GGTTTCAGAC GGCATGAGTT
 551 TCGGAACGGC AGTCGAGCTG TCCGCCGTGA TGCCGCTTTC CTGGCTGCCG
 601 CTTGCCGCCG ACTACACGCG CCACGCGCGC CGCCCGTTTG CGGCAACCCT
 651 GACGGCAACG CTCGCCTACA CGCTGACCGG CTGCTGGATG TATGCCTTGG
 701 GTTTGGCAGC GGCGTTGTTC ACCGGAGAAA CCGACGTGGC AAAAATCCTG
 751 CTGGGCGCAG GTTTGGGTGC GGCAGGCATT TTGGCGGTCG TCCTCTCCAC
 801 CGTTACCACA ACGTTTCTCG ATGCCTATTC CGCCGGCGCG AGTGCGAACA
 851 ACATTTCCGC GCGTTTTGCG GAAACACCCG TCGCTGTCGG CGTTACCCTG
 901 ATCGGCACGG TACTTGCCGT CATGCTGCCC GTTACCGAAT ATGAAAACTT
 951 CCTGCTGCTT ATCGGCTCGG TATTTGCGCC GATGGCGGCG GTTTTGATTG
1001 CCGACTTTTT CGTCTTGAAA CGGCGTGAGG AGATTGAAGG CTTTGACTTT
1051 GCCGGACTGG TTCTGTGGCT TGCGGGCTTC ATCCTCTACC GCTTCCTGCT
1101 CTCGTCCGGC TGGGAAAGCA GCATCGGTCT GACCGCCCCC GTAATGTCTG
1151 CCGTTGCCAT TGCCACCGTA TCGGTACGCC TTTTCTTTAA AAAAACCCAA
1201 TCTTTACAAA GGAACCCGTC ATGA
```

This corresponds to the amino acid sequence <SEQ ID 802; ORF125-1>:

```
   1 MSGNASSPSS SSAIGLIWFG AAVSIAEIST GTLLAPLGWQ RGLAALLLGH
  51 AVGGALFFAA AYIGALTGRS SMESVRLSFG KRGSVLFSVA NMLQLAGWTA
 101 VMIYAGATVS SALGKVLWDG ESFVWWALAN GALIVLWLVF GARKTGGLKT
 151 VSMLLMLLAV LWLSAEVFST AGSTAAQVSD GMSFGTAVEL SAVMPLSWLP
 201 LAADYTRHAR RPFAATLTAT LAYTLTGCWM YALGLAAALF TGETDVAKIL
 251 LGAGLGAAGI LAVVLSTVTT TFLDAYSAGA SANNISARFA ETPVAVGVTL
 301 IGTVLAVMLP VTEYENFLLL IGSVFAPMAA VLIADFFVLK RREEIEGFDF
 351 AGLVLWLAGF ILYRFLLSSG WESSIGLTAP VMSAVAIATV SVRLFFKKTQ
 401 SLQRNPS*
```

Computer analysis of this amino acid sequence gave the following results:

Homology with a predicted ORF from *N.meningitidis* (strain A)

ORF125 shows 76.5% identity over a 5 1 aa overlap with an ORF (ORF125a) from strain A of *N. meningitidis:*

```
                                                       10        20        30
orf125.pep                                     AGASANNISARFAETPVAVSVTLIGTVLAV
                                               ||:|||||||:::| |:||:|:::||:|||
orf125a      KILLGAGLGAAGILAVVLSTVTTTFLDAYSAGVSANNISAKLSEIPIAVAVAVVGTLLAV
             250       260       270       280       290       300

                     40        50        60

orf125.pep   MLPVTEYENFLLLIGSVFAPMGGFDCRLFRLETAX
             :||||||||||||||||||||:
orf125a      LLPVTEYENFLLLIGSVFAPMAAVLIADFFVLKRREEIEG
             310       320       330       340
```

The ORF125a partial nucleotide sequence <SEQ ID 803> is:

```
   1  ATGTCGGGCA ATGCCTCCTC TCNTTCATCT TCCGCCGCCA TCGGGCTGAT
  51  TTGGTTCGGC GCGGCGGTAT CGATTGCCGA AATCAGCACG GGTACACTGC
 101  TTGCGCCTTT GGGCTGGCAG CGCGGTCTGG CNGCTCTGCT TTTGGGTCAT
 151  GCCGTCGGCG GCGCGCTGTT TTTTGCGGCG GCGTATATCG GCGCACTGAC
 201  CGGACNCANC TCGATGGAAA GCGTGCGCCT GTCGTTCGGC AAACGCGGTT
 251  CAGTGCTGTT TTCCGTGGCG AATATGCTGC AACTGGCCGG CTGGACGGCG
 301  GTGATGATTT ACGCCGGCGC AACGGTCAGC TCCGCTTTGG GCAAAGTGTT
 351  GTGGGACGGC GAATCTTTTG TCTGGTGGGC ATTGGCAAAC GGCGCGCTGA
 401  TTGTGCTGTG GCTGGTTTTC GGCGCACGCA AAACAGGCGG GCTGAAAACC
 451  GTTTCGATGC TGCTGATGCT GTTGGCGGTT CTGTGGCTGA GTGCCGAANT
 501  NTTTTCCACG GCAGGCAGCA CCGCCGCANN GGTNNCAGAC GGCATGAGTT
 551  TCGGAACGGC AGTCGAGCTG TCCGCCGTNA TGCCGCTTTC TTGGCTGCCG
 601  CTGGCCGCCG ACTACACGCG CCACGCGCGC CGCCCGTTTG CGGCAACCCT
 651  GACGGCAACG CTCGCCTACA CGCTGACCGG CTGCTGGATG TATGCCTTGG
 701  GTTTGGCAGC GGCGTTGTTC ACCGGAGAAA CCGACGTGGC AAAAATCCTG
 751  CTGGGCGCAG GTTTGGGTGC GGCAGGCATT TTGGCGGTCG TCCTGTCGAC
 801  CGTTACCACC ACTTTTCTCG ATGCNTACTC CGCCGGCGTA AGTGCCAACA
 851  ATATTTCCGC CAAACTTTCG GAAATACCNA TCGCCGTTGC CGTCGCCGTT
 901  GTCGGCACAC TGCTTGCCGT CCTCCTGCCC GTTACCGAAT ATGAAAACTT
 951  CCTGCTGCTT ATCGGCTCGG TATTTGCGCC GATGGCGGCG GTTTTGATTG
1001  CCGACTTTTT CGTCTTGAAA CGGCGTGAGG AGATTGAAGG C..
```

This encodes a protein having the partial amino acid sequence <SEQ ID 804>:

```
   1  MSGNASSXSS SAAIGLIWFG AAVSIAEIST GTLLAPLGWQ RGLAALLLGH
  51  AVGGALFFAA AYIGALTGXX SMESVRLSFG KRGSVLFSVA NMLQLAGWTA
 101  VMIYAGATVS SALGKVLWDG ESFVWWALAN GALIVLWLVF GARKTGGLKT
 151  VSMLLMLLAV LWLSAEXFST AGSTAAXVXD GMSFGTAVEL SAVMPLSWLP
 201  LAADYTRHAR RPFAATLTAT LAYTLTGCWM YALGLAAALF TGETDVAKIL
 251  LGAGLGAAGI LAVVLSTVTT TFLDAYSAGV SANNISAKLS EIPIAVAVAV
 301  VGTLLAVLLP VTEYENFLLL IGSVFAPMAA VLIADFFVLK RREEIEG..
```

ORF125a and ORF125-1 show 94.5% identity in 347 aa overlap:

```
                        10         20         30         40         50         60
orf125a.pep  MSGNASSXSSSAAIGLIWFGAAVSIAEISTGTLLAPLGWQRGLAALLLGHAVGGALFFAA
             ||||||| |||:||||||||||||||||||||||||||||||||||||||||||||||||
orf125-1     MSGNASSPSSSSAIGLIWFGAAVSIAEISTGTLLAPLGWQRGLAALLLGHAVGGALFFAA
                        10         20         30         40         50         60

                        70         80         90        100        110        120
orf125a.pep  AYIGALTGXXSMESVRLSFGKRGSVLFSVANMLQLAGWTAVMIYAGATVSSALGKVLWDG
             ||||||||  ||||||||||||||||||||||||||||||||||||||||||||||||||
orf125-1     AYIGALTGRSSMESVRLSFGKRGSVLFSVANMLQLAGWTAVMIYAGATVSSALGKVLWDG
                        70         80         90        100        110        120

                       130        140        150        160        170        180
orf125a.pep  ESFVWWALANGALIVLWLVFGARKTGGLKTVSMLLMLLAVLWLSAEXFSTAGSTAAXVXD
             |||||||||||||||||||||||||||||||||||||||||||||| ||||||||| | |
orf125-1     ESFVWWALANGALIVLWLVFGARKTGGLKTVSMLLMLLAVLWLSAEVFSTAGSTAAQVSD
                       130        140        150        160        170        180

                       190        200        210        220        230        240
orf125a.pep  GMSFGTAVELSAVMPLSWLPLAADYTRHARRPFAATLTATLAYTLTGCWMYALGLAAALF
             ||||||||||||||||||||||||||||||||||||||||||||||||||||||||||||
orf125-1     GMSFGTAVELSAVMPLSWLPLAADYTRHARRPFAATLTATLAYTLTGCWMYALGLAAALF
                       190        200        210        220        230        240

                       250        260        270        280        290        300
orf125a.pep  TGETDVAKILLGAGLGAAGILAVVLSTVTTTFLDAYSAGVSANNISAKLSEIPIAVAVAV
             |||||||||||||||||||||||||||||||||||||||:|||||||:::| |:||:|::
orf125-1     TGETDVAKILLGAGLGAAGILAVVLSTVTTTFLDAYSAGASANNISARFAETPVAVGVTL
                       250        260        270        280        290        300

                       310        320        330        340
orf125a.pep  VGTLLAVLLPVTEYENFLLLIGSVFAPMAAVLIADFFVLKRREEIEG
             :||:|||:|||||||||||||||||||||||||||||||||||||||
orf125-1     IGTVLAVMLPVTEYENFLLLIGSVFAPMAAVLIADFFVLKRREEIEGFDFAGLVLWLAGF
                       310        320        330        340        350        360
```

Homology with a predicted ORF from *N.gonorrhoeae*

ORF125 shows 86.2% identity over a 65aa overlap with a predicted ORF (ORF125ng) from *N.gonorrhoeae:*

```
orf125.pep                                 AGASANNISARFAETPVAVSVTLIGTVLAV   30
                                           |||||||||||||| ||||:|||| |||||
orf125ng   KILLGAGLGITGILAVVLSTVTTTFLDTYSAGASANNISARFAEIPVAVGVTLIRTVLAV  308

orf125.pep MLPVTEYENFLLLIGSVFAPM-GGFDCRLFRLETA   64
           |||||||:|||||| |||:|| |||||||| |:||
orf125ng   MLPVTEYKNFLLLIRSVFGPMAGGFDCRLFCLKTA  343
```

An ORF125ng nucleotide sequence <SEQ ID 805> was predicted to encode a protein having amino acid sequence <SEQ ID 806>:

```
  1  MSGNASSPSS SAAIGLVWFG AAVSIAEIST GTLLAPLGWQ RGLAALLLGH
 51  AVGGALFFAA AYIGALTGRS SMESVRLSFG KCGSVLFSVA NMLQLAGWTA
101  VMIYVGATVS SALGKVLWDG ESFVWWALAN GALIVLWLVF GARRTGGLKT
151  VSMLLMLLAV LWLSVEVFAS SGTNAAPAVS DGMTFGTAVE LSAVMPLSWL
201  PLAADYTRQA RRPFAATLTA TLAYTLTGCW MYALGLAAAL FTGETDVAKI
251  LLGAGLGITG ILAVVLSTVT TTFLDTYSAG ASANNISARF AEIPVAVGVT
301  LIRTVLAVML PVTEYKNFLL LIRSVFGPMA GGFDCRLFCL KTA*
```

Further work revealed the following gonococcal DNA sequence <SEQ ID 807>:

```
   1  ATGTCGGGCA ATGCCTCCTC TCCTTCATCT TCCGCCGCCA TCGGGCTGGT
  51  TTGGTTCGGC GCGGCGGTAT CGATTGCCGA AATCAGCACG GGTACGCTGC
 101  TCGCCCCCTT GGGCTGGCAG CGCGGTCTGG CGGCCCTGCT TTTGGGTCAT
 151  GCCGTCGGCG GCGCGCTGTT TTTTGCGGCG GCGTATATCG GCGCACTGAC
 201  CGGACGCAGC TCGATGGAAA GTGTGCGCCT GTCGTTCGGC AAATGCGGTT
 251  CAGTGCTGTT TTCCGTGGCG AATATGCTGC AACTGGCCGG CTGGACGGCG
 301  GTGATGATTT ACGTCGGCGC AACGGTCAGC TCCGCTTTGG GCAAAGTGTT
 351  GTGGGACGGC GAATCCTTTG TCTGGTGGGC ATTGGCAAAC GGCGCACTGA
 401  TCGTGCTGTG GCTGGTTTTC GGCGCACGCA GAACGGGCGG GCTGAAAACC
 451  GTTTCGATGC TGCTGATGCT GCTTGCCGTG TTGTGGTTGA GCGTCGAAGT
 501  GTTCGCTTCG TCCGGCACAA ACGCCGCGCC CGCCGTTTCA GACGGCATGA
 551  CCTTCGGAAC GGCAGTCGAA CTGTCCGCCG TCATGCCGCT TTCCTGGCTG
 601  CCGCTGGCCG CCGACTACAC GCGCCAAGCA CGCCGCCCGT TTGCGGCAAC
 651  CCTGACGGCA ACGCTCGCCT ATACGCTGAC GGGCTGCTGG ATGTATGCCT
 701  TGGGTTTGGC GGCGGCTCTG TTTACCGGAG AAACCGACGT GGCGAAAATC
 751  CTGTTGGGCG CGGGCTTGGG CATAACGGGC ATTCTGGCAG TCGTCCTCTC
 801  CACCGTTACC ACAACGTTTC TCGATACCTA TTCCGCCGGC GCGAGTGCGA
 851  ACAACATTTC CGCGCGTTTT GCGGAAATAC CCGTCGCTGT CGGCGTTACC
 901  CTGATCGGCA CGGTGCTTGC CGTCATGCTG CCCGTTACCG AATATAAAAA
 951  CTTCCTGCTG CTTATCGGCT CGGTATTTGC GCCGATGGCG GCGGTTTTGA
1001  TTGCCGACTT TTTCGTCTTA AAACGGCGTG AGGAGATTGA AGGCTTTGAC
1051  TTTGCCGGAC TGGTTCTGTG GCTGGCAGGC TTCATCCTCT ACCGCTTCCT
1101  GCTCTCGTCC GGTTGGGAAA GCAGCATCGG TCTGACCGCC CCCGTAATGT
1151  CTGCCGTTGC CATTGCCACC GTATCGGTAC GCCTTTTCTT TAAAAAAACC
1201  CAATCTTTAC AAAGGAACCC GTCATGA
```

This corresponds to the amino acid sequence <SEQ ID 808; ORF125ng-1>:

```
   1  MSGNASSPSS SAAIGLVWFG AAVSIAEIST GTLLAPLGWQ RGLAALLLGH
  51  AVGGALFFAA AYIGALTGRS SMESVRLSFG KCGSVLFSVA NMLQLAGWTA
 101  VMIYVGATVS SALGKVLWDG ESFVWWALAN GALIVLWLVF GARRTGGLKT
 151  VSMLLMLLAV LWLSVEVFAS SGTNAAPAVS DGMTFGTAVE LSAVMPLSWL
 201  PLAADYTRQA RRPFAATLTA TLAYTLTGCW MYALGLAAAL FTGETDVAKI
 251  LLGAGLGITG ILAVVLSTVT TTFLDTYSAG ASANNISARF AEIPVAVGVT
 301  LIGTVLAVML PVTEYKNFLL LIGSVFAPMA AVLIADFFVL KRREEIEGFD
```

```
 351  FAGLVLWLAG FILYRFLLSS GWESSIGLTA PVMSAVAIAT VSVRLFFKKT
 401  QSLQRNPS*
```

ORF125ng-1 and ORF125-1 show 95.1% identity in 408 aa overlap:

```
                          10        20        30        40        50        60
orf125-1.pep      MSGNASSPSSSSAIGLIWFGAAVSIAEISTGTLLAPLGWQRGLAALLLGHAVGGALFFAA
                  |||||||||||:||||:|||||||||||||||||||||||||||||||||||||||||||
orf125ng-1        MSGNASSPSSSAAIGLVWFGAAVSIAEISTGTLLAPLGWQRGLAALLLGHAVGGALFFAA
                          10        20        30        40        50        60

                          70        80        90       100       110       120
orf125-1.pep      AYIGALTGRSSMESVRLSFGKRGSVLFSVANMLQLAGWTAVMIYAGATVSSALGKVLWDG
                  ||||||||||||||||||||| ||||||||||||||||||||||:|||||||||||||||
orf125ng-1        AYIGALTGRSSMESVRLSFGKCGSVLFSVANMLQLAGWTAVMIYVGATVSSALGKVLWDG
                          70        80        90       100       110       120

                         130       140       150       160       170       179
orf125-1.pep      ESFVWWALANGALIVLWLVFGARKTGGLKTVSMLLMLLAVLWLSAEVFSTAGSTAAQ-VS
                  |||||||||||||||||||||||:||||||||||||||||||||:|||:::|::||  ||
orf125ng-1        ESFVWWALANGALIVLWLVFGARRTGGLKTVSMLLMLLAVLWLSVEVFASSGTNAAPAVS
                         130       140       150       160       170       180

                180       190       200       210       220       230      239
orf125-1.pep      DGMSFGTAVELSAVMPLSWLPLAADYTRHARRPFAATLTATLAYTLTGCWMYALGLAAAL
                  |||:||||||||||||||||||||||||:|||||||||||||||||||||||||||||||
orf125ng-1        DGMTFGTAVELSAVMPLSWLPLAADYTRQARRPFAATLTATLAYTLTGCWMYALGLAAAL
                         190       200       210       220       230       240

                240       250       260       270       280       290      299
orf125-1.pep      FTGETDVAKILLGAGLGAAGILAVVLSTVTTTFLDAYSAGASANNISARFAETPVAVGVT
                  ||||||||||||||||| :||||||||||||||||:|||||||||||||||| |||||||
orf125ng-1        FTGETDVAKILLGAGLGITGILAVVLSTVTTTFLDTYSAGASANNISARFAEIPVAVGVT
                         250       260       270       280       290       300

                300       310       320       330       340       350      359
orf125-1.pep      LIGTVLAVMLPVTEYENFLLLIGSVFAPMAAVLIADFFVLKRREEIEGFDFAGLVLWLAG
                  |||||||||||||||:||||||||||||||||||||||||||||||||||||||||||||
orf125ng-1        LIGTVLAVMLPVTEYKNFLLLIGSVFAPMAAVLIADFFVLKRREEIEGFDFAGLVLWLAG
                         310       320       330       340       350       360

                360       370       380       390       400
orf125-1.pep      FILYRFLLSSGWESSIGLTAPVMSAVAIATVSVRLFFKKTQSLQRNPSX
                  |||||||||||||||||||||||||||||||||||||||||||||||||
orf125ng-1        FILYRFLLSSGWESSIGLTAPVMSAVAIATVSVRLFFKKTQSLQRNPSX
                         370       380       390       400
```

Based on this analysis, including the presence of putative leader sequence and transmembrane domains in the gonococcal protein, it is predicted that the proteins from *N.meningitidis* and *N.gonorrhoeae,* and their epitopes, could be useful antigens for vaccines or diagnostics, or for raising antibodies.

**Example 96**

The following partial DNA sequence was identified in *N.meningitidis* <SEQ ID 809>:

```
  1 ATGACCCGTA TCGCCATCCT CGGCGGCGGC CTCTCGGGAA GGCTGACCGC
 51 GTTGCAGCTT GCAGAACAAG GTTATCAGAT TGCACTTTTC GATAAAAGCT
101 GCCGCCGGGG CGAACACGCC GCCGCCTATG TAGCCGCCGC CATGCTCGCG
151 CCTGCAGCGG A.ACGGTCGA AGCCACGCCC GAAGTGGTCA GGCTGGGCAG
201 GCAGAGCATC CCGCTTTGGC GCGGCATCCG ATGCCGTCTG AACACGCACA
251 CGATGATGCA GGAAAACGGC AGCCTGATTG TATGGCACGG GCAGGACAAG
301 CCATTATCCA GCGAGTTCGT CCGCCATCTC AAACGCGGCG GCGT.ACGGA
351 TGACGAAATC GTCCGTTGGC GCGCCGACGA CATCGCCGAA CGCGAACCGC
401 AACTCGGCGG ACGTTTTTAA GACGGCATCT ACCTGCCGAC CGAAGC.CAG
451 CTCGACGGGC GGCAATTATA GTCTGCACTT GCCGACGCTT TGGACGAACT
```

```
 501  GAACGTCCCC TGCCATTGGG AACACGAATG CGTCCCCGAA GCCTGCAAG..
```

This corresponds to the amino acid sequence <SEQ ID 810; ORF126>:

```
   1  MTRIAILGGG LSGRLTALQL AEQGYQIALF DKSCRRGEHA AAYVAAAMLA
  51  PAAXTVEATP EVVRLGRQSI PLWRGIRCRL NTHTMMQENG SLIVWHGQDK
 101  PLSSEFVRHL KRGGXTDDEI VRWRADDIAE REPQLGGRFX DGIYLPTEXQ
 151  LDGRQLXSAL ADALDELNVP CHWEHECVPE ACK...
```

Further work revealed the complete nucleotide sequence <SEQ ID 811>:

```
   1  ATGACCCGTA TCGCCATCCT CGGCGGCGGC CTCTCGGGAA GGCTGACCGC
  51  GTTGCAGCTT GCAGAACAAG GTTATCAGAT TGCACTTTTC GATAAAGGCT
 101  GCCGCCGGGG CGAACACGCC GCCGCCTATG TTGCCGCCGC CATGCTCGCG
 151  CCTGCGGCGG AAGCGGTCGA AGCCACGCCC GAAGTGGTCA GGCTGGGCAG
 201  GCAGAGCATC CCGCTTTGGC GCGGCATCCG ATGCCGTCTG AACACGCACA
 251  CGATGATGCA GGAAAACGGC AGCCTGATTG TGTGGCACGG GCAGGACAAG
 301  CCATTATCCA GCGAGTTCGT CCGCCATCTC AAACGCGGCG GCGTAGCGGA
 351  TGACGAAATC GTCCGTTGGC GCGCCGACGA CATCGCCGAA CGCGAACCGC
 401  AACTCGGCGG ACGTTTTTCA GACGGCATCT ACCTGCCGAC CGAAGGCCAG
 451  CTCGACGGGC GGCAAATATT GTCTGCACTT GCCGACGCTT TGGACGAACT
 501  GAACGTCCCC TGCCATTGGG AACACGAATG CGTCCCCGAA GGCCTGCAAG
 551  CCCAATACGA CTGGCTGATC GACTGCCGCG GCTACGGCGC AAAAACCGCG
 601  TGGAACCAAT CCCCCGAGCA CACCAGCACC CTGCGCGGCA TACGCGGCGA
 651  AGTGGCGCGG GTTTACACAC CCGAAATCAC GCTCAACCGC CCCGTGCGTC
 701  TGCTCCATCC GCGTTATCCG CTCTACATCG CCCCGAAAGA AAACCACGTC
 751  TTCGTCATCG GCGCGACCCA AATCGAAAGC GAAAGCCAAG CCCCCGCCAG
 801  CGTGCGTTCA GGGTTGGAAC TCTTGTCCGC ACTCTATGCC ATCCACCCCG
 851  CCTTCGGCGA AGCCGACATC CTCGAAATCG CCACCGGCCT GCGCCCCACG
 901  CTCAACCACC ACAACCCCGA AATCCGTTAC AACCGCGCCC GACGCCTGAT
 951  TGAAATCAAC GGCCTTTTCC GCCACGGTTT CATGATCTCC CCCGCCGTAA
1001  CCGCCGCCGC CGCCAGATTG GCAGTGGCAC TGTTTGACGG AAAAGACGCG
1051  CCCGAACGCG ATAAAGAAAG CGGTTTGGCG TATATCCGAA GACAAGATTA
1101  A
```

This corresponds to the amino acid sequence <SEQ ID 812; ORF126-1>:

```
   1  MTRIAILGGG LSGRLTALQL AEQGYQIALF DKGCRRGEHA AAYVAAAMLA
  51  PAAEAVEATP EVVRLGRQSI PLWRGIRCRL NTHTMMQENG SLIVWHGQDK
 101  PLSSEFVRHL KRGGVADDEI VRWRADDIAE REPQLGGRFS DGIYLPTEGQ
 151  LDGRQILSAL ADALDELNVP CHWEHECVPE GLQAQYDWLI DCRGYGAKTA
 201  WNQSPEHTST LRGIRGEVAR VYTPEITLNR PVRLLHPRYP LYIAPKENHV
 251  FVIGATQIES ESQAPASVRS GLELLSALYA IHPAFGEADI LEIATGLRPT
 301  LNHHNPEIRY NRARRLIEIN GLFRHGFMIS PAVTAAAARL AVALFDGKDA
 351  PERDKESGLA YIRRQD*
```

Computer analysis of this amino acid sequence gave the following results:

Homology with a predicted ORF from *N.meningitidis* (strain A)

ORF126 shows 90.0% identity over a 180aa overlap with an ORF (ORF126a) from strain A of *N. meningitidis:*

```
                     10        20        30        40        50        60
orf126.pep   MTRIAILGGGLSGRLTALQLAEQGYQIALFDKSCRRGEHAAAYVAAAMLAPAAXTVEATP
             |||||||||||||||||||||||||||||||:|||||||||||||||||||| :|||||
orf126a      MTRIAILGGGLSGRLTALQLAEQGYQIALFDKGCRRGEHAAAYVAAAMLAPAAEAVEATP
                     10        20        30        40        50        60

                     70        80        90       100       110       120
orf126.pep   EVVRLGRQSIPLWRGIRCRLNTHTMMQENGSLIVWHGQDKPLSSEFVRHLKRGGXTDDEI
             ||||||| |||||||||:|:| :|| ||||||||||||||||:||||||||| :|| |
orf126a      EVVRLGRQXIPLWRGIRCHLKTPAMMXENGSLIVWHGQDKPLSNEFVRHLKRGGVADDXI
                     70        80        90       100       110       120

                    130       140       150       160       170       180
orf126.pep   VRWRADDIAEREPQLGGRFXDGIYLPTEXQLDGRQLXSALADALDELNVPCHWEHECVPE
             |||||||||||||||||| |||||||| ||||||: ||||||||||||||||||||:||
orf126a      VRWRADDIAEREPQLGGRFSDGIYLPTEGQLDGRQILSALADALDELNVPCHWEHECAPE

                 130       140       150       160       170       180
```

The complete length ORF126a nucleotide sequence <SEQ ID 813> is:

```
   1  ATGACCCGTA TCGCCATCCT CGGCGGCGGC CTCTCNGGAA GGCTGACCGC
  51  ACTGCAGCTT GCAGAACAAG GTTATCAGAT TGCACTTTTC GATAAAGGCT
 101  GCCGCCGGGG CGAACACGCC GCCGCCTATG TTGCCGCCGC CATGCTCGCG
 151  CCTGCGGCGG AAGCGGTCGA AGCCACGCCT GAAGTGGTCA GGCTGGGCAG
 201  GCAGANCATC CCGCTTTGGC GCGGCATCCG ATGCCATCTG AAAACGCCTG
 251  CCATGATGCA NGAAAACGGC AGCCTGATTG TGTGGCACGG GCAGGACAAA
 301  CCTTTATCCA ACGAGTTCGT CCGCCATCTC AAACGCGGCG GCGTAGCGGA
 351  TGACNAAATC GTCCGTTGGC GCGCCGACGA CATCGCCGAA CGCGAACCGC
 401  AACTCGGCGG ACGTTTTTCA GACGGCATCT ACCTGCCGAC CGAAGGCCAG
 451  CTCGACGGGC GGCAAATATT GTCTGCACTT GCCGACGCTT TGGACGAACT
 501  GAACGTCCCC TGCCATTGGG AACACGAATG TGCCCCCGAA GACTTGCAAG
 551  CCCAATACGA CTGGCTGATC GACTGCCGCG GCTACGGCGC AAAAACCGCG
 601  TGGAACCAAT CCCCCGANNA NACCAGCACC CTGCGCGGCA TACGCGGCGA
 651  AGTGGCGCGG GTTTACACAC CCGAAATCAC GCTCAACCGC CCCGTGCGCC
 701  TGCTACACCC GCGCTATCCG CTNTACATCG CCCCGAAAGA AAACCNCGTC
 751  TTCGTCATCG GCGCGACCCA AATCGAAAGC GAAAGCCAAG CACCTGCCAG
 801  CGTGCGTTCC GGGCTGGAAC TCTTATCCGC ACTCTATGCC GTCCACCCCG
 851  CCTTCGGCGA AGCCGACATC CTCGAAATCG CCACCGGCCT GCGCCCCACG
 901  CTCAATCACC ACAACCCCGA AATCCGTTAC AACCGCGCCC GACGCCTGAT
 951  TGAAATCAAC GGCCTTTTCC GCCACGGTTT CATGATCTCC CCCGCCGTAA
1001  CCGCCGCCGC CGTCAGATTG GCAGTGGCAC TGTTTGACGG AAAAGANGCG
1051  CCCGAACGCG ATGAAGAAAG CGGTTTGGCG TATATCCGAA GACAAGATTA
1101  A
```

This encodes a protein having amino acid sequence <SEQ ID 814>:

```
  1  MTRIAILGGG LSGRLTALQL AEQGYQIALF DKGCRRGEHA AAYVAAAMLA
 51  PAAEAVEATP EVVRLGRQXI PLWRGIRCHL KTPAMMXENG SLIVWHGQDK
101  PLSNEFVRHL KRGGVADDXI VRWRADDIAE REPQLGGRFS DGIYLPTEGQ
151  LDGRQILSAL ADALDELNVP CHWEHECAPE DLQAQYDWLI DCRGYGAKTA
201  WNQSPXXTST LRGIRGEVAR VYTPEITLNR PVRLLHPRYP LYIAPKENXV
251  FVIGATQIES ESQAPASVRS GLELLSALYA VHPAFGEADI LEIATGLRPT
301  LNHHNPEIRY NRARRLIEIN GLFRHGFMIS PAVTAAAVRL AVALFDGKXA
351  PERDEESGLA YIRRQD*
```

ORF126a and ORF126-1 show 95.4% identity in 366 aa overlap:

```
                         10        20        30        40        50        60
orf126a.pep    MTRIAILGGGLSGRLTALQLAEQGYQIALFDKGCRRGEHAAAYVAAAMLAPAAEAVEATP
               ||||||||||||||||||||||||||||||||||||||||||||||||||||||||||||
orf126-1       MTRIAILGGGLSGRLTALQLAEQGYQIALFDKGCRRGEHAAAYVAAAMLAPAAEAVEATP
                         10        20        30        40        50        60

                         70        80        90       100       110       120
orf126a.pep    EVVRLGRQXIPLWRGIRCHLKTPAMMXENGSLIVWHGQDKPLSNEFVRHLKRGGVADDXI
               |||||||| |||||||||:|:| :|| ||||||||||||||||:|||||||||||||| |
orf126-1       EVVRLGRQSIPLWRGIRCRLNTHTMMQENGSLIVWHGQDKPLSSEFVRHLKRGGVADDEI
                         70        80        90       100       110       120

                        130       140       150       160       170       180
orf126a.pep    VRWRADDIAEREPQLGGRFSDGIYLPTEGQLDGRQILSALADALDELNVPCHWEHECAPE
               |||||||||||||||||||||||||||||||||||||||||||||||||||||||||:||
orf126-1       VRWRADDIAEREPQLGGRFSDGIYLPTEGQLDGRQILSALADALDELNVPCHWEHECVPE
                        130       140       150       160       170       180

                        190       200       210       220       230       240
orf126a.pep    DLQAQYDWLIDCRGYGAKTAWNQSPXXTSTLRGIRGEVARVYTPEITLNRPVRLLHPRYP
                ||||||||||||||||||||||||  |||||||||||||||||||||||||||||||||
orf126-1       GLQAQYDWLIDCRGYGAKTAWNQSPEHTSTLRGIRGEVARVYTPEITLNRPVRLLHPRYP
                        190       200       210       220       230       240

                        250       260       270       280       290       300
orf126a.pep    LYIAPKENXVFVIGATQIESESQAPASVRSGLELLSALYAVHPAFGEADILEIATGLRPT
               |||||||| |||||||||||||||||||||||||||||||:|||||||||||||||||||
orf126-1       LYIAPKENHVFVIGATQIESESQAPASVRSGLELLSALYAIHPAFGEADILEIATGLRPT
                        250       260       270       280       290       300

                        310       320       330       340       350       360
orf126a.pep    LNHHNPEIRYNRARRLIEINGLFRHGFMISPAVTAAAVRLAVALFDGKXAPERDEESGLA
               |||||||||||||||||||||||||||||||||||||:|||||||||| |||||:|||||
orf126-1       LNHHNPEIRYNRARRLIEINGLFRHGFMISPAVTAAAARLAVALFDGKDAPERDKESGLA
                        310       320       330       340       350       360

orf126a.pep    YIRRQDX
               |||||||
orf126-1       YIRRQDX
```

<u>Homology with a predicted ORF from *N.gonorrhoeae*</u>

ORF126 shows 90% identity over a 180 aa overlap with a predicted ORF (ORF126ng) from *N.gonorrhoeae:*

```
orf126.pep   MTRIAILGGGLSGRLTALQLAEQGYQIALFDKSCRRGEHAAAYVAAAMLAPAAXTVEATP   60
             |||||:||||||||||||||||||||| ||||: |:||||||||||||||||| :|||||
orf126ng     MTRIAVLGGGLSGRLTALQLAEQGYQIELFDKGTRQGEHAAAYVAAAMLAPAAEAVEATP   60

orf126.pep   EVVRLGRQSIPLWRGIRCRLNTHTMMQENGSLIVWHGQDKPLSSEFVRHLKRGGXTDDEI  120
             ||:|||||||||||||||||||| ||||||||||||||||||||||||||||||| :||||
orf126ng     EVIRLGRQSIPLWRGIRCRLNTLTMMQENGSLIVWHGQDKPLSSEFVRHLKRGGVADDEI  120

orf126.pep   VRWRADDIAEREPQLGGRFXDGIYLPTEXQLDGRQLXSALADALDELNVPCHWEHECVPE  180
             ||||||:|||||||||||| |||||||| ||||||: ||||||||||||||||||||:|:
orf126ng     VRWRADEIAEREPQLGGRFSDGIYLPTEGQLDGRQILSALADALDELNVPCHWEHECAPQ  180
```

An ORF126ng nucleotide sequence <SEQ ID 815> was predicted to encode a protein having amino acid sequence <SEQ ID 816>:

```
  1  MTRIAVLGGG LSGRLTALQL AEQGYQIELF DKGTRQGEHA AAYVAAAMLA
 51  PAAEAVEATP EVIRLGRQSI PLWRGIRCRL NTLTMMQENG SLIVWHGQDK
101  PLSSEFVRHL KRGGVADDEI VRWRADEIAE REPQLGGRFS DGIYLPTEGQ
151  LDGRQILSAL ADALDELNVP CHWEHECAPQ DLQAQYDWVI DCRGYGAKTA
201  WNQSPEHTST LRGIRGEVRG FTRPKSRSTA PCACCTRAIR STSPRKKTTS
251  SSSARPKSKA KAKPPPAYVP GWNSYPRSMP STPPSAKPTS SKWRPGLRPT
301  LNHHNPEIRY SRERRLIEIN GLFRHGFMIS PAVTAAAVRL AVALFDGKDA
351  PERDEESGLA YIGRQD*
```

Further work revealed the following gonococcal DNA sequence <SEQ ID 817>:

```
   1  ATGACCCGTA TCGCCGTCCT CGGAGGCGGC CTTTCCGGAA GGCTGACCGC
  51  ATTGCAGCTT GCAGAACAAG GTTATCAGAT TGAACTTTTC GACAAGGGCA
 101  CCCGCCAAGG CGAACACGCC GCCGCCTATG TTGCCGCCGC GATGCTCGCG
 151  CCTGCGGCGG AAGCGGTCGA GGCAACGCCC GAAGTCATCA GGCTGGGCAG
 201  GCAGAGCATT CCGCTTTGGC GCGGCATCCG ATGCCGTCTG AACACGCTCA
 251  CGATGATGCA GGAAAACGGC AGCCTGATTG TGTGGCACGG GCAGGACAAG
 301  CCATTATCCA GCGAGTTCGT CCGCCATCTC AAACGCGGCG GCGTAGCGGA
 351  TGACGAAATC GTCCGTTGGC GCGCCGATGA AATCGCCGAA CGCGAACCGC
 401  AACTCGGCGG ACGTTTTTCA GACGGCATCT ACCTGCCGAC CGAAGGCCAG
 451  CTCGACGGGC GGCAAATATT GTCTGCACTT GCCGACGCTT TGGACGAACT
 501  GAACGTCCCT TGCCATTGGG AACACGAATG CGCCCCCCAA GACCTGCAAG
 551  CCCAATACGA CTGGGTAATC GACTGCCGGG GCTACGGCGC GAAAACCGCG
 601  TGGAACCAAT CCCCCGAGCA CACCAGCACC TTGCGCGGCA TACGCGGCGA
 651  AGTGGCGCGG GTTTACACGC CCGAAATCAC GCTCAACCGC CCCGTGCGCC
 701  TGCTGCACCC GCGCTATCCG CTCTACATCG CCCCGAAAGA AAACCACGTC
 751  TTCGTCATCG GCGCGACCCA AATCGAAAGC GAAAGCCAAG CCCCCGCCAG
 801  CGTACGTTCC GGGCTGGAAC TCTTATCCGC GCTCTATGCC GTCCACCCCG
 851  CCTTCGGCGA AGCCGACATC CTCGAAATCG CCGCCGGCCT GCGCCCCACG
 901  CTCAACCACC ACAACCCCGA AATCCGCTAC AGCCGCGAAC GCCGCCTCAT
 951  CGAAATCAAC GGCCTTTTCC GGCACGGCTT TATGATTTCC CCCGCCGTAA
1001  CCGCCGCCGC CGTCAGATTG GCAGTGGCAC TGTTTGACGG AAAAGACGCG
1051  CCCGAACGTG ATGAAGAAAG CGGTTTGGCG TATATCGGAA GACAAGATTA
1101  A
```

This corresponds to the amino acid sequence <SEQ ID 818; ORF126ng-1>:

```
  1  MTRIAVLGGG LSGRLTALQL AEQGYQIELF DKGTRQGEHA AAYVAAAMLA
 51  PAAEAVEATP EVIRLGRQSI PLWRGIRCRL NTLTMMQENG SLIVWHGQDK
101  PLSSEFVRHL KRGGVADDEI VRWRADEIAE REPQLGGRFS DGIYLPTEGQ
151  LDGRQILSAL ADALDELNVP CHWEHECAPQ DLQAQYDWVI DCRGYGAKTA
201  WNQSPEHTST LRGIRGEVAR VYTPEITLNR PVRLLHPRYP LYIAPKENHV
251  FVIGATQIES ESQAPASVRS GLELLSALYA VHPAFGEADI LEIAAGLRPT
301  LNHHNPEIRY SRERRLIEIN GLFRHGFMIS PAVTAAAVRL AVALFDGKDA
351  PERDEESGLA YIGRQD*
```

ORF126ng-1 and ORF126-1 show 95.1% identity in 366 aa overlap:

```
                     10        20        30        40        50        60
orf126-1.pep  MTRIAILGGGLSGRLTALQLAEQGYQIALFDKGCRRGEHAAAYVAAAMLAPAAEAVEATP
              |||||:||||||||||||||||||||| ||||| |:||||||||||||||||||||||||
orf126ng-1    MTRIAVLGGGLSGRLTALQLAEQGYQIELFDKGTRQGEHAAAYVAAAMLAPAAEAVEATP
                     10        20        30        40        50        60

                     70        80        90       100       110       120
orf126-1.pep  EVVRLGRQSIPLWRGIRCRLNTHTMMQENGSLIVWHGQDKPLSSEFVRHLKRGGVADDEI
              ||:||||||||||||||||||| |||||||||||||||||||||||||||||||||||||
orf126ng-1    EVIRLGRQSIPLWRGIRCRLNTLTMMQENGSLIVWHGQDKPLSSEFVRHLKRGGVADDEI
                     70        80        90       100       110       120

                    130       140       150       160       170       180
orf126-1.pep  VRWRADDIAEREPQLGGRFSDGIYLPTEGQLDGRQILSALADALDELNVPCHWEHECVPE
              ||||||:||||||||||||||||||||||||||||||||||||||||||||||||||:|:
orf126ng-1    VRWRADEIAEREPQLGGRFSDGIYLPTEGQLDGRQILSALADALDELNVPCHWEHECAPQ
                    130       140       150       160       170       180

                    190       200       210       220       230       240
orf126-1.pep  GLQAQYDWLIDCRGYGAKTAWNQSPEHTSTLRGIRGEVARVYTPEITLNRPVRLLHPRYP
               |||||||:||||||||||||||||||||||||||||||||||||||||||||||||||||
orf126ng-1    DLQAQYDWVIDCRGYGAKTAWNQSPEHTSTLRGIRGEVARVYTPEITLNRPVRLLHPRYP
                    190       200       210       220       230       240

                    250       260       270       280       290       300
orf126-1.pep  LYIAPKENHVFVIGATQIESESQAPASVRSGLELLSALYAIHPAFGEADILEIATGLRPT
              ||||||||||||||||||||||||||||||||||||||||:||||||||||||||:|||||
orf126ng-1    LYIAPKENHVFVIGATQIESESQAPASVRSGLELLSALYAVHPAFGEADILEIAAGLRPT
                    250       260       270       280       290       300

                    310       320       330       340       350       360
orf126-1.pep  LNHHNPEIRYNRARRLIEINGLFRHGFMISPAVTAAAARLAVALFDGKDAPERDKESGLA
              ||||||||||:| ||||||||||||||||||||||||:|||||||||||||||||:|||||
orf126ng-1    LNHHNPEIRYSRERRLIEINGLFRHGFMISPAVTAAAVRLAVALFDGKDAPERDEESGLA
                    310       320       330       340       350       360

orf126-1.pep  YIRRQDX
              || ||||
orf126ng-1    YIGRQDX
```

Furthermore, ORF126ng-1 shows homology to a putative *Rhizobium* oxidase flavoprotein:

```
gi|2627327 (AF004408) putative amino acid oxidase flavoprotein [Rhizobium etli]
Length = 327
 Score =  169 bits (423), Expect = 3e-41
 Identities = 112/329 (34%), Positives = 163/329 (49%), Gaps = 25/329 (7%)

Query: 3   RIAVLGGGLSGRLTALQLAEQGYQIELFDKGTRQGEHXXXXXXXXXXXXXXXXXXXXXXX 62
           RI V G G++G   A QL    G+++ L ++    G
Sbjct: 2   RILVNGAGVAGLTVAWQLYRHGFRVTLAERAGTVGA-GASGFAGGMLAPWCERESAEEPV 60

Query: 63  IRLGRQSIPLWRGIRCRLNTLTMMQENGSLIVWHGQDKPLSSEFVRHLKRGGVADDEIVR 122
           + LGR +    W              +    G+L+V  G+D       F R    G    DE+
Sbjct: 61  LTLGRLAADWWEAA-----LPGHVHRRGTLVVAGGRDTGELDRFSRRTS-GWEWLDEVA- 113

Query: 123 WRADEIAEREPQLGGRFSDGIYLPTEGQLDGRQILSALADALDELNVPCHWEHECAPQDL 182
                IA   EP L GRF    ++    E   LD RQ L+ALA   L++   +        +
Sbjct: 114 -----IAALEPDLAGRFRRALFFRQEAHLDPRQALAALAAGLEDARMRLTLG---VVGES 165

Query: 183 QAQYDWVIDCRGYGAKTAWNQSPEHTSTLRGIRGEVARVYTPEITLNRPVRLLHPRYPLY 242
```

```
                +D V+DC G                 LRG+RGE+  V T E++L+RPVRLLHPR+P+Y
Sbjct: 166 DVDHDRVVDCTGAA-------QIGRLPGLRGVRGEMLCVETTEVSLSRPVRLLHPRHPIY 218

Query: 243 IAPKENHVFVIGATQIESESQAPASVRSGLELLSALYAVHPAFGEADILEIAAGLRPTLN 302
            I P++ + F++GAT IES+   P + RS +ELL+A YA+HPAFGEA + E  AG+RP
Sbjct: 219 IVPRDKNRFMVGATMIESDDGGPITARSLMELLNAAYAMHPAFGEARVTETGAGVRPAYP 278

Query: 303 HHNPEIRYSRERRLIEINGLFRHGFMISP 331
            + P  R ++E R + +NGL+RHGF+++P
Sbjct: 279 DNLP--RVTQEGRTLHVNGLYRHGFLLAP 305
```

This analysis suggests that the proteins from *N. meningitidis* and *N.gonorrhoeae,* and their epitopes, could be useful antigens for vaccines or diagnostics, or for raising antibodies.

## Example 97

The following DNA sequence, believed to be complete, was identified in *N.meningitidis* <SEQ ID 819>:

```
  1  ATGACTGATA ATCGGGGGTT TACGCTGGTT GAATTAATAT CAGTGGTCTT
 51  GATATTGTCT GTACTTGCTT TAATTGTTTA TCCGAGCTAT CGCAATTATG
101  TTGAGAAAGC AAAGATAAAT GCAGTGCGGG CAGCCTTGTT AGAAAATGCA
151  CATTTTATGG AAAAGTTTTA TCTGCAGAAT GGGAGGTTTA AACAAACATC
201  TACCAAGTGG CCAAGTTTGC CGATTAAAGA GGCAGAAGGC TTTTGTATCC
251  GTTTGAATGG AATCGtCGCG CGGG..GCTT TAGACAGTAA ATTCATGTTG
301  AAGGCGGTAG CCATAGATAA AGATAAAAAT CCTTTTATTA TTAAGATGAA
351  TGAAAATCTA GTAACCTTTA aTTTGCAAGA AGTCCGCCAG TTCGTGTAGT
401  GACGGGCTGG ATTATTTTAA AGGAAATGAT AAGGACTGCA AGTTACTTAA
451  GTAG
```

This corresponds to the amino acid sequence <SEQ ID 820; ORF127>:

```
  1  MTDNRGFTLV ELISVVLILS VLALIVYPSY RNYVEKAKIN AVRAALLENA
 51  HFMEKFYLQN GRFKQTSTKW PSLPIKEAEG FCIRLNGIVA RXALDSKFML
101  KAVAIDKDKN PFIIKMNENL VTFICKKSAS SCSDGLDYFK GNDKDCKLLK
151  *
```

Further work revealed the following DNA sequence <SEQ ID 821>:

```
  1  ATGACTGATA ATCGGGGGTT TACGCTGGTT GAATTAATAT CAGTGGTCTT
 51  GATATTGTCT GTACTTGCTT TAATTGTTTA TCCGAGCTAT CGCAATTATG
101  TTGAGAAAGC AAAGATAAAT GCAGTGCGGG CAGCCTTGTT AGAAAATGCA
151  CATTTTATGG AAAAGTTTTA TCTGCAGAAT GGGAGGTTTA AACAAACATC
201  TACCAAGTGG CCAAGTTTGC CGATTAAAGA GGCAGAAGGC TTTTGTATCC
251  GTTTGAATGG AATCGCGCGC GGGGCTTTAG ACAGTAAATT CATGTTGAAG
301  GCGGTAGCCA TAGATAAAGA TAAAAATCCT TTTATTATTA AGATGAATGA
351  AAATCTAGTA ACCTTTATTT GCAAGAAGTC CGCCAGTTCG TGTAGTGACG
401  GGCTGGATTA TTTTAAAGGA AATGATAAGG ACTGCAAGTT ACTTAAGTAG
```

This corresponds to the amino acid sequence <SEQ ID 822; ORF127-1>:

```
  1  MTDNRGFTLV ELISVVLILS VLALIVYPSY RNYVEKAKIN AVRAALLENA
 51  HFMEKFYLQN GRFKQTSTKW PSLPIKEAEG FCIRLNGIAR GALDSKFMLK
101  AVAIDKDKNP FIIKMNENLV TFICKKSASS CSDGLDYFKG NDKDCKLLK*
```

Computer analysis of this amino acid sequence gave the following results:

Homology with a predicted ORF from *N.meningitidis* (strain A)

ORF127 shows 98.0% identity over a 150aa overlap with an ORF (ORF127a) from strain A of *N. meningitidis:*

```
                       10        20        30        40        50        60
orf127.pep     MTDNRGFTLVELISVVLILSVLALIVYPSYRNYVEKAKINAVRAALLENAHFMEKFYLQN
               ||||||||||||||||||||||||||||||||||||||||||:|||||||||||||||||
orf127a        MTDNRGFTLVELISVVLILSVLALIVYPSYRNYVEKAKINTVRAALLENAHFMEKFYLQN
                       10        20        30        40        50        60

                       70        80        90       100       110       120
orf127.pep     GRFKQTSTKWPSLPIKEAEGFCIRLNGIVARXALDSKFMLKAVAIDKDKNPFIIKMNENL
               |||||||||||||||||||||||||||| || ||||||||||||||||||||||||||||
orf127a        GRFKQTSTKWPSLPIKEAEGFCIRLNGI-ARGALDSKFMLKAVAIDKDKNPFIIKMNENL
                       70        80         90       100       110

                      130       140       150
orf127.pep     VTFICKKSASSCSDGLDYFKGNDKDCKLLKX
               |||||||||||||||||||||||||||||||
orf127a        VTFICKKSASSCSDGLDYFKGNDKDCKLLKX
                    120       130       140       150
```

The complete length ORF127a nucleotide sequence <SEQ ID 823> is:

```
  1  ATGACTGATA ATCGGGGGTT TACGCTGGTT GAATTAATAT CAGTGGTCTT
 51  GATATTGTCT GTACTTGCTT TAATTGTTTA TCCGAGCTAT CGCAATTATG
101  TTGAGAAAGC AAAGATAAAT ACAGTGCGGG CAGCCTTGTT AGAAAATGCA
151  CATTTTATGG AAAAGTTTTA TCTGCAGAAT GGGAGATTTA AACAAACATC
201  TACCAAATGG CCAAGTTTGC CGATTAAAGA GGCAGAAGGC TTTTGTATCC
251  GTTTGAATGG AATCGCGCGC GGGGCCTTAG ACAGTAAATT CATGTTGAAG
301  GCGGTAGCCA TAGATAAAGA TAAAAATCCT TTTATTATTA AGATGAATGA
351  AAATCTAGTA ACCTTTATTT GCAAGAAGTC CGCCAGTTCG TGTAGTGACG
401  GGCTGGATTA TTTTAAAGGA AATGATAAGG ACTGCAAGTT ACTTAAGTAG
```

This encodes a protein having amino acid sequence <SEQ ID 824>:

```
  1  MTDNRGFTLV ELISVVLILS VLALIVYPSY RNYVEKAKIN TVRAALLENA
 51  HFMEKFYLQN GRFKQTSTKW PSLPIKEAEG FCIRLNGIAR GALDSKFMLK
101  AVAIDKDKNP FIIKMNENLV TFICKKSASS CSDGLDYFKG NDKDCKLLK*
```

ORF127a and ORF127-1 show 99.3% identity in 149 aa overlap:

```
                       10        20        30        40        50        60
orf127a.pep    MTDNRGFTLVELISVVLILSVLALIVYPSYRNYVEKAKINTVRAALLENAHFMEKFYLQN
               ||||||||||||||||||||||||||||||||||||||||||:|||||||||||||||||
orf127-1       MTDNRGFTLVELISVVLILSVLALIVYPSYRNYVEKAKINAVRAALLENAHFMEKFYLQN
                       10        20        30        40        50        60

                     70        80        90       100       110       120
orf127a.pep    GRFKQTSTKWPSLPIKEAEGFCIRLNGIARGALDSKFMLKAVAIDKDKNPFIIKMNENLV
               ||||||||||||||||||||||||||||||||||||||||||||||||||||||||||||
orf127-1       GRFKQTSTKWPSLPIKEAEGFCIRLNGIARGALDSKFMLKAVAIDKDKNPFIIKMNENLV
                       70        80        90       100       110       120

                     130       140       150
orf127a.pep    TFICKKSASSCSDGLDYFKGNDKDCKLLKX
               ||||||||||||||||||||||||||||||
orf127-1       TFICKKSASSCSDGLDYFKGNDKDCKLLKX
                       130       140       150
```

Homology with a predicted ORF from *N.gonorrhoeae*

ORF127 shows 97.3% identity over a 150 aa overlap with a predicted ORF (ORF127ng) from *N.gonorrhoeae:*

```
orf127.pep   MTDNRGFTLVELISVVLILSVLALIVYPSYRNYVEKAKINAVRAALLENAHFMEKFYLQN   60
             ||||||||||||||||||||||||||||||||||||||||||||:|||||||||||||||
orf127ng     MTDNRGFTLVELISVVLILSVLALIVYPSYRNYVEKAKINAVRAAFLENAHFMEKFYLQN   60

orf127.pep   GRFKQTSTKWPSLPIKEAEGFCIRLNGIVARXALDSKFMLKAVAIDKDKNPFIIKMNENL  120
             |||||||||||||||||||||||||||| || |||||||||||||||||||||||||||||
orf127ng     GRFKQTSTKWPSLPIKEAEGFCIRLNGI-ARGALDSKFMLKAVAIDKDKNPFIIKMNENL  119

orf127.pep   VTFICKKSASSCSDGLDYFKGNDKDCKLLK  150
             ||||||||||||||| ||||||||||||||
orf127ng     VTFICKKSASSCSDRLDYFKGNDKDCKLLK  149
```

The complete length ORF127ng nucleotide sequence <SEQ ID 825> is:

```
  1 ATGACTGATA ATCGGGGGTT TACACTGGTT GAATTAATAT CAGTGGTCTT
 51 GATATTGTCT GTACTTGCTT TAATTGTTTA TCCGAGCTAT CGCAATTATG
101 TTGAGAAAGC AAAGATAAAT GCAGTGCGGG CAGCCTTGTT AGAAAATGCA
151 CATTTTATGG AAAAGTTTTA TCTGCAGAAT GGGAGATTTA AACAAACATC
201 TACCAAATGG CCAAGTTTGC CGATTAAAGA GGCAGAAGGC TTTTGTATCC
251 GTTTGAATGG AATCGCGCGC GGGGCTTTAG ACAGTAAATT CATGTTGAAG
301 GCGGTAGCCA TAGATAAAGA TAAAAATCCT TTTATTATTA AGATGAATGA
351 AAATCTAGTA ACCTTTATTT GCAAGAAGTC CGCCAGTTCG TGTAGTGACG
401 GGCTGGATTA TTTTAAAGGA AATGATAAGG ACTGCAAGTT ACTTAAGTAG
```

This encodes a protein having amino acid sequence <SEQ ID 826>:

```
  1 MTDNRGFTLV ELISVVLILS VLALIVYPSY RNYVEKAKIN AVRAAFLENA
 51 HFMEKFYLQN GRFKQTSTKW PSLPIKEAEG FCIRLNGIAR GALDSKFMLK
101 AVAIDKDKNP FIIKMNENLV TFICKKSASS CSDRLDYFKG NDKDCKLLK*
```

ORF127ng and ORF127-1 show 100.0% identity in 149 aa overlap:

```
                       10        20        30        40        50        60
orf127-1.pep   MTDNRGFTLVELISVVLILSVLALIVYPSYRNYVEKAKINAVRAALLENAHFMEKFYLQN
               ||||||||||||||||||||||||||||||||||||||||||||||||||||||||||||
orf127ng-1     MTDNRGFTLVELISVVLILSVLALIVYPSYRNYVEKAKINAVRAALLENAHFMEKFYLQN
                       10        20        30        40        50        60

                       70        80        90       100       110       120
orf127-1.pep   GRFKQTSTKWPSLPIKEAEGFCIRLNGIARGALDSKFMLKAVAIDKDKNPFIIKMNENLV
               ||||||||||||||||||||||||||||||||||||||||||||||||||||||||||||
orf127ng-1     GRFKQTSTKWPSLPIKEAEGFCIRLNGIARGALDSKFMLKAVAIDKDKNPFIIKMNENLV
                       70        80        90       100       110       120

                      130       140       150
orf127-1.pep   TFICKKSASSCSDGLDYFKGNDKDCKLLKX
               ||||||||||||||||||||||||||||||
orf127ng-1     TFICKKSASSCSDGLDYFKGNDKDCKLLKX
                      130       140       150
```

This analysis, including the fact that the predicted transmembrane domain is shared by the meningococcal and gonococcal proteins, suggests that the proteins from *N.meningitidis* and *N.gonorrhoeae,* and their epitopes, could be useful antigens for vaccines or diagnostics, or for raising antibodies.

## Example 98

The following partial DNA sequence was identified in *N.meningitidis* <SEQ ID 827>

```
  1 ..GTGTCGCTGG CTTCGGTGAT TGCCTCTCAA ATCTTCCTTT ACGAAGATTT
 51   CAACCAAATG CGGAAAACCC GTGGAGCTAT CTGCGGTTTT CTTGTCCAAT
101   ATTTATCTGG GGTTTCAGCA GGGGTATTTC GATTTGAGTG CCGACGAGAA
151   CCCCGTACTG CATATCTGGT CTTTGGCAGT AGAGGAACAG TATTACCTCC
201   TGTATCCCCT TTTGCTGATA TTTTGCTGCA AAAAAACCAA ATCGCTACGG
251   GTGCTGCGTA ACATCAGCAT CATCCTGTTT TTGATTTTGA CTGCCTCATC
301   GTTTTTGCCA AGCGGGTTTT ATACCGACAT CCTCAACCAA CCCAATACTT
351   ATTACCTTTC GACACTGAGG TTTCCCGAGC TGTTGGCAGG TTCGCTGCTG
401   GCGGTTTACG GGCAAACGCA AAACGGCAGA CGGCAAACAG CAAATGGAAA
451   ACGGCAGTTG CTTTCATCAC TCTGCTTCGG CGCATTGCTT GCCTGCCTGT
501   TCGTGATTGA CAAACACAAT CCGTTTATCC CGGGAATGAC CCTGCTCCTT
551   CCCTGCCTGC TGACGGCACT GCTTATCCGG AGTATGCAAT ACGGGACACT
601   TCCGACCCGC ATCCTGTCGG CAAGCCCCAT CGTATTTGTC GGCAAAATCT
651   CTTATTCCCT ATACCTGTAC CATTGGATTT TTATTGCTTT CGCTCCGCTC
701   ATTAGAGGCG GGAAACAGCT CGGACTGCCT GCCG..
```

This corresponds to the amino acid sequence <SEQ ID 828; ORF128>:

```
  1 ..VSLASVIASQ IFLYEDFNQM RKTVELSAVF LSNIYLGFQQ GYFDLSADEN
 51   PVLHIWSLAV EEQYYLLYPL LLIFCCKKTK SLRVLRNISI ILFLILTASS
101   FLPSGFYTDI LNQPNTYYLS TLRFPELLAG SLLAVYGQTQ NGRRQTANGK
151   RQLLSSLCFG ALLACLFVID KHNPFIPGMT LLLPCLLTAL LIRSMQYGTL
201   PTRILSASPI VFVGKISYSL YLYHWIFIAF APLIRGGKQL GLPA..
```

Further work revealed the complete nucleotide sequence <SEQ ID 829>:

```
   1 ATGCAAGCTG TCCGATACAG ACCGGAAATT GACGGATTGC GGGCCGTCGC
  51 CGTGCTATCC GTCATGATTT TCCACCTGAA TAACCGCTGG CTGCCCGGAG
 101 GATTCCTGGG GGTGGACATT TTCTTTGTCA TCTCAGGATT CCTCATTACC
 151 GGCATCATTC TTTCTGAAAT ACAGAACGGT TCTTTTTCTT TCCGGGATTT
 201 TTATACCCGC AGGATTAAGC GGATTTATCC TGCCTTTATT GCGGCCGTGT
 251 CGCTGGCTTC GGTGATTGCC TCTCAAATCT TCCTTTACGA AGATTTCAAC
 301 CAAATGCGGA AAACCGTGGA GCTTTCTGCG GTTTTCTTGT CCAATATTTA
 351 TCTGGGGTTT CAGCAGGGGT ATTTCGATTT GAGTGCCGAC GAGAACCCCG
 401 TACTGCATAT CTGGTCTTTG GCAGTAGAGG AACAGTATTA CCTCCTGTAT
 451 CCCCTTTTGC TGATATTTTG CTGCAAAAAA ACCAAATCGC TACGGGTGCT
 501 GCGTAACATC AGCATCATCC TGTTTTTGAT TTTGACTGCC TCATCGTTTT
 551 TGCCAAGCGG GTTTTATACC GACATCCTCA ACCAACCCAA TACTTATTAC
 601 CTTTCGACAC TGAGGTTTCC CGAGCTGTTG GCAGGTTCGC TGCTGGCGGT
 651 TTACGGGCAA ACGCAAAACG GCAGACGGCA AACAGCAAAT GGAAAACGGC
 701 AGTTGCTTTC ATCACTCTGC TTCGGCGCAT TGCTTGCCTG CCTGTTCGTG
 751 ATTGACAAAC ACAATCCGTT TATCCCGGGA ATGACCCTGC TCCTTCCCTG
 801 CCTGCTGACG GCACTGCTTA TCCGGAGTAT GCAATACGGG ACACTTCCGA
 851 CCCGCATCCT GTCGGCAAGC CCCATCGTAT TTGTCGGCAA AATCTCTTAT
 901 TCCCTATACC TGTACCATTG GATTTTTATT GCTTTCGCCC ATTACATTAC
 951 AGGCGACAAA CAGCTCGGAC TGCCTGCCGT ATCGGCGGTT GCCGCGTTGA
1001 CGGCCGGATT TTCCCTGTTG AGTTATTATT TGATTGAACA GCCGCTTAGA
1051 AAACGGAAGA TGACCTTCAA AAAGGCATTT TTCTGCCTCT ATCTCGCCCC
1101 GTCCCTGATA CTTGTCGGTT ACAACCTGTA CGCAAGGGGG ATATTGAAAC
1151 AGGAACACCT CCGCCCGTTG CCCGGCGCGC CCCTTGCTGC GGAAAATCAT
1201 TTTCCGGAAA CCGTCCTGAC CCTCGGCGAC TCGCACGCCG GACACCTGAG
1251 GGGGTTTCTG GATTATGTCG GCAGCCGGGA AGGGTGGAAA GCCAAAATCC
1301 TGTCCCTCGA TTCGGAGTGT TTGGTTTGGG TAGATGAGAA GCTGGCAGAC
1351 AACCCGTTAT GTCGAAAATA CCGGGATGAA GTTGAAAAAG CCGAAGCCGT
1401 TTTCATTGCC CAATTCTATG ATTTGAGGAT GGGCGGCCAG CCTGTGCCGA
1451 GATTTGAAGC GCAATCCTTC CTAATACCCG GGTTCCCAGC CCGATTCAGG
1501 GAAACCGTCA AAAGGATAGC CGCCGTCAAA CCCGTCTATG TTTTTGCAAA
1551 CAACACATCA ATCAGCCGTT CGCCCCTGAG GGAGGAAAAA TTGAAAAGAT
1601 TTGCCGCAAA CCAATATCTC CGCCCCATTC AGGCTATGGG CGACATCGGC
1651 AAGAGCAATC AGGCGGTCTT TGATTTGATT AAAGATATTC CCAATGTGCA
1701 TTGGGTGGAC GCACAAAAAT ACCTGCCCAA AAACACGGTC GAAATATACG
1751 GCCGCTATCT TTACGGCGAC CAAGACCACC TGACCTATTT CGGTTCTTAT
1801 TATATGGGGC GGGAATTCCA CAAACACGAA CGCCTGCTTA AATCTTCCCA
1851 CGGCGGCGCA TTGCAGTAG
```

This corresponds to the amino acid sequence <SEQ ID 830; ORF128-1>:

```
   1 MQAVRYRPEI DGLRAVAVLS VMIFHLNNRW LPGGFLGVDI FFVISGFLIT
  51 GIILSEIQNG SFSFRDFYTR RIKRIYPAFI AAVSLASVIA SQIFLYEDFN
 101 QMRKTVELSA VFLSNIYLGF QQGYFDLSAD ENPVLHIWSL AVEEQYYLLY
 151 PLLLIFCCKK TKSLRVLRNI SIILFLILTA SSFLPSGFYT DILNQPNTYY
 201 LSTLRFPELL AGSLLAVYGQ TQNGRRQTAN GKRQLLSSLC FGALLACLFV
 251 IDKHNPFIPG MTLLLPCLLT ALLIRSMQYG TLPTRILSAS PIVFVGKISY
 301 SLYLYHWIFI AFAHYITGDK QLGLPAVSAV AALTAGFSLL SYYLIEQPLR
 351 KRKMTFKKAF FCLYLAPSLI LVGYNLYARG ILKQEHLRPL PGAPLAAENH
 401 FPETVLTLGD SHAGHLRGFL DYVGSREGWK AKILSLDSEC LVWVDEKLAD
 451 NPLCRKYRDE VEKAEAVFIA QFYDLRMGGQ PVPRFEAQSF LIPGFPARFR
 501 ETVKRIAAVK PVYVFANNTS ISRSPLREEK LKRFAANQYL RPIQAMGDIG
 551 KSNQAVFDLI KDIPNVHWVD AQKYLPKNTV EIYGRYLYGD QDHLTYFGSY
 601 YMGREFHKHE RLLKSSHGGA LQ*
```

Computer analysis of this amino acid sequence gave the following results:

Homology with hypothetical integral membrane protein HI0392 of *H.influenzae* (accession number U32723)

ORF128 and HI0392 show 52% aa identity in 180aa overlap:

```
Orf128: 1   VSLASVIASQIFLYEDFNQMRKTVELSAVFLSNIYLGFQQGYFDLSADENPVLHIWSLAV 60
            ++L S IAS IF+Y DFN++RKT+EL+  FLSN YLG  QGYFDLSA+ENPVLHIWSLAV
HI0392: 46  MALVSFIASAIFIYNDFNKLRKTIELAIAFLSNFYLGLTQGYFDLSANENPVLHIWSLAV 105

Orf128: 61  EEQXXXXXXXXXIFCCKKTKSLRVLRNISIILFLILTASSFLPSGFYTDILNQPNTYYLS 120
            E Q         I   KK + ++VL  I++ILF IL A+SF+ + FY ++L+QPN YYLS
HI0392: 106 EGQYYLIFPLILILAYKKFREVKVLFIITLILFFILLATSFVSANFYKEVLHQPNIYYLS 165

Orf128: 121 TLRFPELLAGSLLAVYGQTQNGRRQTANGKRQLLSSLCFGALLACLFVIDKHNPFIPGMT 180
             LRFPELL GSLLA+Y    N + Q +      +L+ L      L +CLF+++ +  FIPG+T
HI0392: 166 NLRFPELLVGSLLAIYHNLSN-KVQLSKQVNNILAILSTLLLFSCLFLMNNNIAFIPGIT 224
```

Homology with a predicted ORF from *N.meningitidis* (strain A)

ORF128 shows 98.0% identity over a 244aa overlap with an ORF (ORF128a) from strain A of *N. meningitidis:*

```
                                                     10        20        30
orf128.pep                               VSLASVIASQIFLYEDFNQMRKTVELSAVF
                                         ||||||||||||||||||||||||||||||
orf128a     ILSEIQNGSFSFRDFYTRRIKRIYPAFIAAVSLASVIASQIFLYEDFNQMRKTVELSAVF
                    60        70        80        90       100       110

                    40        50        60        70        80        90
orf128.pep  LSNIYLGFQQGYFDLSADENPVLHIWSLAVEEQYYLLYPLLLIFCCKKTKSLRVLRNISI
            ||||||||||||||||||||||||||||||||||||||||||||||||||||||||||||
orf128a     LSNIYLGFQQGYFDLSADENPVLHIWSLAVEEQYYLLYPLLLIFCCKKTKSLRVLRNISI
                   120       130       140       150       160       170

                   100       110       120       130       140       150
orf128.pep  ILFLILTASSFLPSGFYTDILNQPNTYYLSTLRFPELLAGSLLAVYGQTQNGRRQTANGK
            ||||||||:|||||||||||||||||||||||||||||||||||||||||||||||||||
orf128a     ILFLILTATSFLPSGFYTDILNQPNTYYLSTLRFPELLAGSLLAVYGQTQNGRRQTANGK
                   180       190       200       210       220       230

                   160       170       180       190       200       210
orf128.pep  RQLLSSLCFGALLACLFVIDKHNPFIPGMTLLLPCLLTALLIRSMQYGTLPTRILSASPI
            ||||||||||||||||||||||||||||||||||||||||||||||||||||||||||||
orf128a     RQLLSSLCFGALLACLFVIDKHNPFIPGMTLLLPCLLTALLIRSMQYGTLPTRILSASPI
                   240       250       260       270       280       290

                   220       230       240
orf128.pep  VFVGKISYSLYLYHWIFIAFAPLIRGGKQLGLPA
            ||||||||||||||||||||    | | |||||||
orf128a     VFVGKISYSLYLYHWIFIAFAHYITGDKQLGLPAVSAVAALTAGFSLLSYYLIEQPLRKR
                   300       310       320       330       340       350

orf128a     KMTFKKAFFCLYLAPSLILVGYNLYARGILKQEHLRPLPGAPLAAENHFPETVLTLGDSH
                   360       370       380       390       400       410
```

The complete length ORF128a nucleotide sequence <SEQ ID 831> is:

```
   1 ATGCAAGCTG TCCGATACAG ACCGGAAATT GACGGATTGC GGGCCGTCGC
  51 CGTGCTATCC GTCATGATTT TCCACCTGAA TAACCGCTGG CTGCCCGGAG
 101 GATTCCTGGG GGTGGACATT TTCTTTGTCA TCTCAGGATT CCTCATTACC
 151 GGCATCATTC TTTCTGAAAT ACAGAACGGT TCTTTTTCTT TCCGGGATTT
 201 TTATACCCGC AGGATTAAGC GGATTTATCC TGCTTTTATT GCGGCCGTGT
 251 CGCTGGCTTC GGTGATTGCC TCTCAAATCT TCCTTTACGA AGATTTCAAC
 301 CAAATGCGGA AAACCGTGGA GCTTTCTGCG GTTTTCTTGT CCAATATTTA
 351 TCTGGGGTTT CAGCAGGGGT ATTTCGATTT GAGTGCCGAC GAGAACCCCG
 401 TACTGCATAT CTGGTCTTTG GCAGTAGAGG AACAGTATTA CCTCCTGTAT
 451 CCTCTTTTGC TGATATTTTG CTGCAAAAAA ACAAAATCGC TACGGGTGCT
 501 GCGTAACATC AGCATCATCC TATTTCTGAT TTTGACTGCC ACATCGTTTT
 551 TGCCAAGCGG GTTTTATACC GATATTCTCA ACCAACCCAA TACTTATTAC
 601 CTTTCGACAC TGAGGTTTCC CGAGCTGTTG GCAGGTTCGC TGCTGGCGGT
 651 TTACGGGCAA ACGCAAAACG GCAGACGGCA AACAGCAAAT GGAAAACGGC
 701 AGTTGCTTTC ATCACTCTGC TTCGGCGCAT TGCTTGCCTG CCTGTTCGTG
 751 ATTGACAAAC ACAATCCGTT TATCCCGGGA ATGACCCTGC TCCTTCCCTG
 801 CCTGCTGACG GCACTGCTTA TCCGGAGTAT GCAATACGGG ACACTTCCGA
 851 CCCGCATCCT GTCGGCAAGC CCCATCGTAT TTGTCGGCAA AATCTCTTAT

 901 TCCCTATACC TGTACCATTG GATTTTTATT GCTTTCGCCC ATTACATTAC
 951 AGGCGACAAA CAGCTCGGAC TGCCTGCCGT ATCGGCGGTT GCCGCGTTGA
1001 CGGCCGGATT TTCCCTGTTG AGTTATTATT TGATTGAACA GCCGCTTAGA
1051 AAACGGAAGA TGACCTTCAA AAAGGCATTT TTCTGCCTCT ATCTCGCCCC
1101 GTCCCTGATA CTTGTCGGTT ACAACCTGTA CGCAAGGGGG ATATTGAAAC
1151 AGGAACACCT CCGCCCGTTG CCCGGCGCGC CCCTTGCTGC GGAAAATCAT
1201 TTTCCGGAAA CCGTCCTGAC CCTCGGCGAC TCGCACGCCG GACACCTGCG
1251 GGGGTTTCTG GATTATGTCG GCAGCCGGGA AGGGTGGAAA GCCAAAATCC
1301 TGTCCCTCGA TTCGGAGTGT TTGGTTTGGG TAGATGAGAA GCTGGCAGAC
1351 AACCCGTTAT GTCGAAAATA CCGGGATGAA GTTGAAAAAG CCGAAGCCGT
1401 TTTCATTGCC CAATTCTATG ATTTGAGGAT GGGCGGCCAG CCCGTGCCGA
1451 GATTTGAAGC GCAATCCTTC CTAATACCCG GGTTCCCAGC CCGATTCAGG
1501 GAAACCGTCA AAAGGATAGC CGCCGTCAAA CCCGTCTATG TTTTTGCAAA
1551 CAACACATCA ATCAGCCGTT CGCCCCTGAG GGAGGAAAAA TTGAAAAGAT
1601 TTGCCGCAAA CCAATATCTC CGCCCCATTC AGGCTATGGG CGACATCGGC
1651 AAGAGCAATC AGGCGGTCTT TGATTTGATT AAAGATATTC CCAATGTGCA
1701 TTGGGTGGAC GCACAAAAAT ACCTGCCCAA AAACACGGTC GAAATATACG
1751 GCCGCTATCT TTACGGCGAC CAAGACCACC TGACCTATTT CGGTTCTTAT
1801 TATATGGGGC GGGAATTTCA CAAACACGAA CGCCTGCTTA AATCTTCTCG
1851 CGACGGCGCA TTGCAGTAG
```

This encodes a protein having amino acid sequence <SEQ ID 832>:

```
  1 MQAVRYRPEI DGLRAVAVLS VMIFHLNNRW LPGGFLGVDI FFVISGFLIT
 51 GIILSEIQNG SFSFRDFYTR RIKRIYPAFI AAVSLASVIA SQIFLYEDFN
101 QMRKTVELSA VFLSNIYLGF QQGYFDLSAD ENPVLHIWSL AVEEQYYLLY
151 PLLLIFCCKK TKSLRVLRNI SIILFLILTA TSFLPSGFYT DILNQPNTYY
201 LSTLRFPELL AGSLLAVYGQ TQNGRRQTAN GKRQLLSSLC FGALLACLFV
251 IDKHNPFIPG MTLLLPCLLT ALLIRSMQYG TLPTRILSAS PIVFVGKISY
301 SLYLYHWIFI AFAHYITGDK QLGLPAVSAV AALTAGFSLL SYYLIEQPLR
351 KRKMTFKKAF FCLYLAPSLI LVGYNLYARG ILKQEHLRPL PGAPLAAENH
401 FPETVLTLGD SHAGHLRGFL DYVGSREGWK AKILSLDSEC LVWVDEKLAD
451 NPLCRKYRDE VEKAEAVFIA QFYDLRMGGQ PVPRFEAQSF LIPGFPARFR
501 ETVKRIAAVK PVYVFANNTS ISRSPLREEK LKRFAANQYL RPIQAMGDIG
551 KSNQAVFDLI KDIPNVHWVD AQKYLPKNTV EIYGRYLYGD QDHLTYFGSY
601 YMGREFHKHE RLLKSSRDGA LQ*
```

ORF128a and ORF128-1 show 99.5% identity in 622 aa overlap:

```
orf128a.pep   MQAVRYRPEIDGLRAVAVLSVMIFHLNNRWLPGGFLGVDIFFVISGFLITGIILSEIQNG
              |||||:||||||||||||||||||||||||||||||||||||||||||||||||||||||
orf128-1      MQAVRYRPEIDGLRAVAVLSVMIFHLNNRWLPGGFLGVDIFFVISGFLITGIILSEIQNG

orf128a.pep   SFSFRDFYTRRIKRIYPAFIAAVSLASVIASQIFLYEDFNQMRKTVELSAVFLSNIYLGF
              ||||||||||||||||||||||||||||||||||||||||||||||||||||||||||||
orf128-1      SFSFRDFYTRRIKRIYPAFIAAVSLASVIASQIFLYEDFNQMRKTVELSAVFLSNIYLGF

orf128a.pep   QQGYFDLSADENPVLHIWSLAVEEQYYLLYPLLLIFCCKKTKSLRVLRNISIILFLILTA
              ||||||||||||||||||||||||||||||||||||||||||||||||||||||||||||
orf128-1      QQGYFDLSADENPVLHIWSLAVEEQYYLLYPLLLIFCCKKTKSLRVLRNISIILFLILTA

orf128a.pep   TSFLPSGFYTDILNQPNTYYLSTLRFPELLAGSLLAVYGQTQNGRRQTANGKRQLLSSLC
              :|||||||||||||||||||||||||||||||||||||||||||||||||||||||||||
orf128-1      SSFLPSGFYTDILNQPNTYYLSTLRFPELLAGSLLAVYGQTQNGRRQTANGKRQLLSSLC

orf128a.pep   FGALLACLFVIDKHNPFIPGMTLLLPCLLTALLIRSMQYGTLPTRILSASPIVFVGKISY
              ||||||||||||||||||||||||||||||||||||||||||||||||||||||||||||
orf128-1      FGALLACLFVIDKHNPFIPGMTLLLPCLLTALLIRSMQYGTLPTRILSASPIVFVGKISY

orf128a.pep   SLYLYHWIFIAFAHYITGDKQLGLPAVSAVAALTAGFSLLSYYLIEQPLRKRKMTFKKAF
              ||||||||||||||||||||||||||||||||||||||||||||||||||||||||||||
orf128-1      SLYLYHWIFIAFAHYITGDKQLGLPAVSAVAALTAGFSLLSYYLIEQPLRKRKMTFKKAF

orf128a.pep   FCLYLAPSLILVGYNLYARGILKQEHLRPLPGAPLAAENHFPETVLTLGDSHAGHLRGFL
              ||||||||||||||||||||||||||||||||||||||||||||||||||||||||||||
orf128-1      FCLYLAPSLILVGYNLYARGILKQEHLRPLPGAPLAAENHFPETVLTLGDSHAGHLRGFL

orf128a.pep   DYVGSREGWKAKILSLDSECLVWVDEKLADNPLCRKYRDEVEKAEAVFIAQFYDLRMGGQ
              ||||||||||||||||||||||||||||||||||||||||||||||||||||||||||||
orf128-1      DYVGSREGWKAKILSLDSECLVWVDEKLADNPLCRKYRDEVEKAEAVFIAQFYDLRMGGQ

orf128a.pep   PVPRFEAQSFLIPGFPARFRETVKRIAAVKPVYVFANNTSISRSPLREEKLKRFAANQYL
              ||||||||||||||||||||||||||||||||||||||||||||||||||||||||||||
orf128-1      PVPRFEAQSFLIPGFPARFRETVKRIAAVKPVYVFANNTSISRSPLREEKLKRFAANQYL

orf128a.pep   RPIQAMGDIGKSNQAVFDLIKDIPNVHWVDAQKYLPKNTVEIYGRYLYGDQDHLTYFGSY
              ||||||||||||||||||||||||||||||||||||||||||||||||||||||||||||
orf128-1      RPIQAMGDIGKSNQAVFDLIKDIPNVHWVDAQKYLPKNTVEIYGRYLYGDQDHLTYFGSY

orf128a.pep   YMGREFHKHERLLKSSRDGALQX
              ||||||||||||||||: |||||
orf128-1      YMGREFHKHERLLKSSHGGALQX
```

Homology with a predicted ORF from *N.gonorrhoeae*

ORF128 shows 93.4% identity over 244 aa overlap with a predicted ORF (ORF128ng) from *N. gonorrhoeae:*

```
orf128.pep                                 VSLASVIASQIFLYEDFNQMRKTVELSAVF   30
                                           |||||||||||||||||||||||:|||:||
orf128ng      ILSEIQNGSFSFRDFYTRRIKRIYPAFIAAVSLASVIASQIFLYEDFNQMRKTIELSTVF  112

orf128.pep    LSNIYLGFQQGYFDLSADENPVLHIWSLAVEEQYYLLYPLLLIFCCKKTKSLRVLRNISI   90
              ||||||||: ||||||||||||||||||||||||||||||||||| ||||||||||||||
orf128ng      LSNIYLGFRLGYFDLSADENPVLHIWSLAVEEQYYLLYPLLLIFCYKKTKSLRVLRNISI  172

orf128.pep    ILFLILTASSFLPSGFYTDILNQPNTYYLSTLRFPELLAGSLLAVYGQTQNGRRQTANGK  150
              |||||||||||||:||||||||||||||||||||||||:||||||||||||||||| |||
orf128ng      ILFLILTASSFLPAGFYTDILNQPNTYYLSTLRFPELLVGSLLAVYGQTQNGRRQTENGK  232

orf128.pep    RQLLSSLCFGALLACLFVIDKHNPFIPGMTLLLPCLLTALLIRSMQYGTLPTRILSASPI  210
              ||||| |||||||:||||||||:|||||:|||||||||||||||||||||||||||||||
orf128ng      RQLLSLLCFGALLVCLFVIDKHDPFIPGITLLLPCLLTALLIRSMQYGTLPTRILSASPI  292

orf128.pep    VFVGKISYSLYLYHWIFIAFAPLIRGGKQLGLPA                            244
              |||||||||||||||||||||  | | |||||||
orf128ng      VFVGKISYSLYLYHWIFIAFAHYITGDKQLGLPAVSAVAALTAGFSLLSYYLIEQPLRKR  352
```

The complete length ORF128ng nucleotide sequence <SEQ ID 833> is:

```
   1  ATGCAAGCTG TCCGATACAG GCCTGAAATT GACGGATTGC GGGCCGTCGC
  51  CGTGCTATCC GTCATTATTT TCCACCTGAA TAACCGCTGG CTGCCCGGAG
 101  GATTCCTGGG GGTGGACATT TTCTTTGTCA TCTCGGGATT CCTCATTACC
 151  AACATCATTC TTTCTGAAAT ACAGAACGGT TCTTTTTCTT TCCGGGATTT
 201  TTATACCCGC AGGATTAAGC GGATTTATCC TGCTTTTATT GCGGCCGTGT
 251  CCCTGGCTTC GGTGATTGCT TCTCAAATCT TCCTTTACGA AGATTTCAAC
 301  CAAATGAGGA AAACCATAGA GCTTTCTACG GTTTTTTTGT CCAATATTTA
 351  TTTGGGGTTC CGATTGGGGT ATTTCGATTT GAGTGCCGAC GAGAACCCCG
 401  TACTGCATAT CTGGTCTTTG GCGGTAGAGG AACAGTATTA CCTCCTGTAT
 451  CCTCTTTTGC TGATATTCTG TTACAAAAAA ACCAAATCAC TACGGGTGCT
 501  GCGTAATATC AGCATCATCC TGTTTCTGAT TTTGACCGCA TCATCGTTTT
 551  TGCCGGCCGG GTTTTATACC GACATCCTCA ACCAACCcaa TACTTATTAC
 601  CTTTCGACAC TGAGGTTTCC CGAGCTGTTG GTGGGTTCGC TGTTGGCGGT
 651  TTACGGGCAA ACGCAAAACG GCAGACGGCA AACAGAAAAT GGAAAACGGC
 701  AGTTGCTTTC ATTACTCTGT TTCGGCGCat tgCTTGTCTG CCTGTTCGTG
 751  ATCGACAAAC ACGATCCGTT TATCCCGGGA ATAACCCTGC TCCTTCCCTG
 801  CCTGCTGACG GCGCTGCTTA TCCGGAGTAT GCAATACGGG ACACTTCCGA
 851  CCCGCATCCT GTCGGCAAGC CCCATCGTAT TTGTCGGCAA AATCTCTTAT
 901  TCCCTATACC TGTACCATTG GATTTTTATT GCCTTCGCCC ATTACATTAC
 951  AGGCGACAAA CAGCTCGGAC TGCCTGCCGT ATCGGCGGTT GCCGCGTTGA
1001  CGGCCGGATT TTCCCTGTTG AGCTATTATT TGATTGAACA GCCGCTTAGA
1051  AAACGGAAGA TGACCTTCAA AAAGGCATTT TTCTGCCTTT ATCTCGCCCC
1101  GTCCCTGATG CTTGTCGGTT ACAACCTGTA TTCAAGAGGG ATATTGAAAC
1151  AGGAACACCT CCGCCCGCTG CCCGGCACGC CCGTTGCTGC GGAAAATAAT
1201  TTTCCGGAAA CCGTCTTGAC CCTCGGCGAC TCGCACGCCG GACACCTGCG
1251  GGGGTTTCTG GATTATGTCG GCGGCAGGGA AGGGTGGAAA GCTAAAATCC
1301  TGTCCCTCGA TTCGGAGTGT TTGGTTTGGG TGGATGAGAA GCTGGCAGAC
1351  AACCCGTTGT GCCGAAAATA CCGGGATGAA GTTGAAAAAG CCGAAGCTGT
1401  TTTCATTGCC CAATTCTATG ATTTGAGGAT GGGCGGCCAG CCCGTGCCGA
1451  GATTTGAAGC GCAATCCTTC CTGATACCCG GGTTCAAAGC CCGATTCAGG
1501  GAAACCGTCA AGAGGATAGC CGCCGTCAAA CCTGTATATG TTTTTGCAAA
1551  CAATACATCA ATCAGCCGTT CTCCCTTGAG GGAGGAAAAA TTGAAAAGAT

1601  TTGCTATAAA CCAATACCTC CGGCCTATTC GGGCTATGGG CGACATCGGC
1651  AAGAGCAATC AGGCGGTCTT TGATTTGGTT AAAGATATTC CCAATGTGCA
1701  TTGGGTGGAC GCACAAAAAT ACCTGCCCAA AAACACGGTC GAAATACACG
1751  GACGCTATCT TTACGGCGAC CAAGACCACC TGACCTATTT CGGTTCTTAT
1801  TATATGGGGC GGGAATTTCA CAAACACGAA CGCCTGCTCA AGCATTCCCG
1851  AGGCGGCGCA TTGCAGTAG
```

This encodes a protein having amino acid sequence <SEQ ID 834>:

```
  1 MQAVRYRPEI DGLRAVAVLS VIIFHLNNRW LPGGFLGVDI FFVISGFLIT
 51 NIILSEIQNG SFSFRDFYTR RIKRIYPAFI AAVSLASVIA SQIFLYEDFN
101 QMRKTIELST VFLSNIYLGF RLGYFDLSAD ENPVLHIWSL AVEEQYYLLY
151 PLLLIFCYKK TKSLRVLRNI SIILFLILTA SSFLPAGFYT DILNQPNTYY
201 LSTLRFPELL VGSLLAVYGQ TQNGRRQTEN GKRQLLSLLC FGALLVCLFV
251 IDKHDPFIPG ITLLLPCLLT ALLIRSMQYG TLPTRILSAS PIVFVGKISY
301 SLYLYHWIFI AFAHYITGDK QLGLPAVSAV AALTAGFSLL SYYLIEQPLR
351 KRKMTFKKAF FCLYLAPSLM LVGYNLYSRG ILKQEHLRPL PGTPVAAENN
401 FPETVLTLGD SHAGHLRGFL DYVGGREGWK AKILSLDSEC LVWVDEKLAD
451 NPLCRKYRDE VEKAEAVFIA QFYDLRMGGQ PVPRFEAQSF LIPGFKARFR
501 ETVKRIAAVK PVYVFANNTS ISRSPLREEK LKRFAINQYL RPIRAMGDIG
551 KSNQAVFDLV KDIPNVHWVD AQKYLPKNTV EIHGRYLYGD QDHLTYFGSY
601 YMGREFHKHE RLLKHSRGGA LQ*
```

ORF128ng and ORF128-1 show 95.7% identity in 622 aa overlap:

```
orf128-1.pep  MQAVRYRPEIDGLRAVAVLSVMIFHLNNRWLPGGFLGVDIFFVISGFLITGIILSEIQNG
              |||||||||||||||||||||:||||||||||||||||||||||||||||:|||||||||
orf128ng      MQAVRYRPEIDGLRAVAVLSVIIFHLNNRWLPGGFLGVDIFFVISGFLITNIILSEIQNG

orf128-1.pep  SFSFRDFYTRRIKRIYPAFIAAVSLASVIASQIFLYEDFNQMRKTVELSAVFLSNIYLGF
              |||||||||||||||||||||||||||||||||||||||||||||:|||:||||||||||
orf128ng      SFSFRDFYTRRIKRIYPAFIAAVSLASVIASQIFLYEDFNQMRKTIELSTVFLSNIYLGF

orf128-1.pep  QQGYFDLSADENPVLHIWSLAVEEQYYLLYPLLLIFCCKKTKSLRVLRNISIILFLILTA
              : ||||||||||||||||||||||||||||||||||| ||||||||||||||||||||||
orf128ng      RLGYFDLSADENPVLHIWSLAVEEQYYLLYPLLLIFCYKKTKSLRVLRNISIILFLILTA

orf128-1.pep  SSFLPSGFYTDILNQPNTYYLSTLRFPELLAGSLLAVYGQTQNGRRQTANGKRQLLSSLC
              |||||:||||||||||||||||||||||||:||||||||||||||||| ||||||| |||
orf128ng      SSFLPAGFYTDILNQPNTYYLSTLRFPELLVGSLLAVYGQTQNGRRQTENGKRQLLSLLC

orf128-1.pep  FGALLACLFVIDKHNPFIPGMTLLLPCLLTALLIRSMQYGTLPTRILSASPIVFVGKISY
              |||||:||||||||:|||||:|||||||||||||||||||||||||||||||||||||||
orf128ng      FGALLVCLFVIDKHDPFIPGITLLLPCLLTALLIRSMQYGTLPTRILSASPIVFVGKISY

orf128-1.pep  SLYLYHWIFIAFAHYITGDKQLGLPAVSAVAALTAGFSLLSYYLIEQPLRKRKMTFKKAF
              ||||||||||||||||||||||||||||||||||||||||||||||||||||||||||||
orf128ng      SLYLYHWIFIAFAHYITGDKQLGLPAVSAVAALTAGFSLLSYYLIEQPLRKRKMTFKKAF

orf128-1.pep  FCLYLAPSLILVGYNLYARGILKQEHLRPLPGAPLAAENHFPETVLTLGDSHAGHLRGFL
              |||||||||:|||||||:||||||||||||||:|:||||:||||||||||||||||||||
orf128ng      FCLYLAPSLMLVGYNLYSRGILKQEHLRPLPGTPVAAENNFPETVLTLGDSHAGHLRGFL

orf128-1.pep  DYVGSREGWKAKILSLDSECLVWVDEKLADNPLCRKYRDEVEKAEAVFIAQFYDLRMGGQ
              ||||:|||||||||||||||||||||||||||||||||||||||||||||||||||||||
orf128ng      DYVGGREGWKAKILSLDSECLVWVDEKLADNPLCRKYRDEVEKAEAVFIAQFYDLRMGGQ

orf128-1.pep  PVPRFEAQSFLIPGFPARFRETVKRIAAVKPVYVFANNTSISRSPLREEKLKRFAANQYL
              ||||||||||||||| ||||||||||||||||||||||||||||||||||||||| ||||
orf128ng      PVPRFEAQSFLIPGFKARFRETVKRIAAVKPVYVFANNTSISRSPLREEKLKRFAINQYL

orf128-1.pep  RPIQAMGDIGKSNQAVFDLIKDIPNVHWVDAQKYLPKNTVEIYGRYLYGDQDHLTYFGSY
              |||:|||||||||||||||:||||||||||||||||||||||:|||||||||||||||||
orf128ng      RPIRAMGDIGKSNQAVFDLVKDIPNVHWVDAQKYLPKNTVEIHGRYLYGDQDHLTYFGSY

orf128-1.pep  YMGREFHKHERLLKSSHGGALQX
              |||||||||||||| |:||||||
orf128ng      YMGREFHKHERLLKHSRGGALQX
                             610       620
```

In addition, ORF218ng shows homology to a hypothetical *H. influenzae* protein:

```
sp|P43993|Y392_HAEIN   HYPOTHETICAL   PROTEIN   HI0392   >gi|1074385|pir||B64007
hypothetical protein HI0392 - Haemophilus influenzae (strain Rd KW20)
>gi|1573364 (U32723) H. influenzae predicted coding region HI0392 [Haemophilus
influenzae] Length = 245
 Score =  239 bits (604), Expect = 3e-62
 Identities = 124/225 (55%), Positives = 152/225 (67%), Gaps = 1/225 (0%)

Query: 38  VDIFFVISGFLITNIILSEIQNGSFSFRDFYTRRIKRIYPXXXXXXXXXXXXXXXXFLYE 97
            +DIFFVISGFLIT II++EIQ  SFS + FYTRRIKRIYP                F+Y
Sbjct: 1   MDIFFVISGFLITGIIITEIQQNSFSLKQFYTRRIKRIYPAFITVMALVSFIASAIFIYN 60

Query: 98  DFNQMRKTIELSTVFLSNIYLGFRLGYFDLSADENPVLHIWSLAVEEQXXXXXXXXXIFC 157
            DFN++RKTIEL+  FLSN YLG   GYFDLSA+ENPVLHIWSLAVE Q         I
Sbjct: 61  DFNKLRKTIELAIAFLSNFYLGLTQGYFDLSANENPVLHIWSLAVEGQYYLIFPLILILA 120

Query: 158 YKKTKSLRVLRNISIILFLILTASSFLPAGFYTDILNQPNTYYLSTLRFPELLVGSLLAV 217
            YKK + ++VL  I++ILF IL A+SF+ A FY ++L+QPN YYLS LRFPELLVGSLLA+
Sbjct: 121 YKKFREVKVLFIITLILFFILLATSFVSANFYKEVLHQPNIYYLSNLRFPELLVGSLLAI 180

Query: 218 YGQTQNGRRQTENGKRQLLSLLCFGALLVCLFVIDKHDPFIPGIT 262
            Y    N + Q        +L++L     L   CLF+++ +  FIPGIT
Sbjct: 181 YHNLSN-KVQLSKQVNNILAILSTLLLFSCLFLMNNNIAFIPGIT 224
```

This analysis, including the identification of several putative transmembrane domains, suggests that these proteins from *N.meningitidis* and *N.gonorrhoeae,* and their epitopes, could be useful antigens for vaccines or diagnostics, or for raising antibodies.

**Example 99**

The following partial DNA sequence was identified in *N.meningitidis* <SEQ ID 835>:

```
  1  ..ATTATTTACG AATACCGCTG GATGTTTCTT TACGGCGCAC TGACGACCTT
 51    GGGGCTGACG GTCGTGGCAA C.GCGGGCGG TTCGGTATTG GGTCTGTTGT
101    TGGCGTTGGC GCGCCTGATT CACTTGGAAA AAGCCGGTGC GCCGATGCGC
151    GTGCTGGCGT GGGCGTTGCG TAAAGTTTCG CTGCTGTATG TTACGCTGTT
201    CCGGGGTACG CCGCTGTTTG TGCAGATTGT GATTTGGGCG TATGTGTGGT
251    TTCCGTTTTT CGTC..
```

This corresponds to the amino acid sequence <SEQ ID 836; ORF129>:

```
  1  ..IIYEYRWMFL YGALTTLGLT VVAXAGGSVL GLLLALARLI HLEKAGAPMR
 51    VLAWALRKVS LLYVTLFRGT PLFVQIVIWA YVWFPFFV..
```

Further work revealed the complete nucleotide sequence <SEQ ID 837>:

```
  1  ATGGATTTTC GTTTTGACAT TATTTACGAA TACCGCTGGA TGTTTCTTTA
 51  CGGCGCACTG ACGACCTTGG GGCTGACGGT CGTGGCAACG GCGGGCGGTT
101  CGGTATTGGG TCTGTTGTTG GCGTTGGCGC GCCTGATTCA CTTGGAAAAA
151  GCCGGTGCGC CGATGCGCGT GCTGGCGTGG GCGTTGCGTA AAGTTTCGCT
201  GCTGTATGTT ACGCTGTTCC GGGGTACGCC GCTGTTTGTG CAGATTGTGA
251  TTTGGGCGTA TGTGTGGTTT CCGTTTTTCG TCCATCCTTC AGACGGCATT
301  TTGGTCAGCG GCGAGGCGGC AATCGCGCTG CGTCGCGGAT ACGGGCCGCT
351  GATTGCCGGT TCTTTGGCAC TGATCGCCAA CTCGGGGGCG TATATCTGTG
401  AGATTTTCCG CGCGGGCATC CAGTCTATAG ACAAAGGACA GATGGAGGCG
451  GCGCGTTCTT TGGGGCTGAC CTATCCGCAG GCGATGCGCT ATGTGATTCT
501  GCCGCAGGCA TTGCGCCGCA TGCTGCCGCC TTTGGCGAGC GAGTTCATCA
551  CGCTCTTGAA AGACAGCTCG CTGCTGTCGG TCATTGCTGT GGCGGAGTTG
601  GCGTATGTTC AGAATACGAT TACGGGCCGG TATTCGGTTT ATGAAGAACC
651  GCTTTACACC GTCGCCCTGA TTTATCTGTT GATGACGACT TTCTTAGGCT
701  GGATATTCCT GCGTTTGGAA AAACGTTACA ATCCGCAACA CCGCTGA
```

This corresponds to the amino acid sequence <SEQ ID 838; ORF129-1>:

```
  1  MDFRFDIIYE YRWMFLYGAL TTLGLTVVAT AGGSVLGLLL ALARLIHLEK
 51  AGAPMRVLAW ALRKVSLLYV TLFRGTPLFV QIVIWAYVWF PFFVHPSDGI
101  LVSGEAAIAL RRGYGPLIAG SLALIANSGA YICEIFRAGI QSIDKGQMEA
151  ARSLGLTYPQ AMRYVILPQA LRRMLPPLAS EFITLLKDSS LLSVIAVAEL

201  AYVQNTITGR YSVYEEPLYT VALIYLLMTT FLGWIFLRLE KRYNPQHR*
```

Computer analysis of this amino acid sequence gave the following results:

Homology with a predicted ORF from *N.meningitidis* (strain A)

ORF129 shows 98.9% identity over a 88aa overlap with an ORF (ORF129a) from strain A of *N. meningitidis:*

```
                           10        20        30        40        50
orf129.pep           IIYEYRWMFLYGALTTLGLTVVAXAGGSVLGLLLALARLIHLEKAGAPMRVLAW
                     |||||||||||||||||||||||:||||||||||||||||||||||||||||||
orf129a       MDFRFDIIYEYRWMFLYGALTTLGLTVVATAGGSVLGLLLALARLIHLEKAGAPMRVLAW
                      10        20        30        40        50        60

                  60        70        80
orf129.pep    ALRKVSLLYVTLFRGTPLFVQIVIWAYVWFPFFV
              ||||||||||||||||||||||||||||||||||
orf129a       ALRKVSLLYVTLFRGTPLFVQIVIWAYVWFPFFVHPSDGILVSGEAAIALRRGYGPLIAG
                      70        80        90       100       110       120

orf129a       SLALIANSGAYICEIFRAGIQSIDKGQMEAARSLGLTYPQAMRYVILPQALRRMLPPLAS
                     130       140       150       160       170       180
```

The complete length ORF129a nucleotide sequence <SEQ ID 839> is:

```
  1  ATGGATTTTC GTTTTGACAT TATTTACGAA TACCGCTGGA TGTTTCTTTA
 51  CGGCGCACTG ACGACCTTGG GGCTGACGGT CGTGGCGACG GCGGGCGGTT
101  CGGTATTGGG TCTGTTGTTG GCGTTGGCGC GCCTGATTCA CTTGGAAAAA
151  GCCGGTGCGC CGATGCGCGT GCTGGCGTGG GCGTTGCGTA AGGTTTCGCT
201  GCTGTATGTT ACGCTGTTCC GGGGTACGCC GCTGTTTGTG CAGATTGTGA
251  TTTGGGCGTA TGTGTGGTTT CCGTTTTTCG TCCATCCTTC AGACGGCATT
301  TTGGTTAGCG GCGAGGCGGC AATCGCGCTG CGTCGCGGAT ACGGGCCGCT
351  GATTGCCGGT TCTTTGGCAC TGATCGCCAA CTCGGGGGCG TATATCTGTG
401  AGATTTTCCG CGCGGGCATC CAGTCTATAG ACAAAGGACA GATGGAGGCG
451  GCGCGTTCTT TGGGGCTGAC CTATCCGCAG GCGATGCGCT ATGTGATTCT
501  GCCGCAGGCA TTGCGCCGTA TGCTGCCGCC TTTGGCGAGC GAGTTCATCA
551  CGCTCTTGAA AGACAGCTCG CTGCTGTCGG TCATTGCTGT GGCGGAGTTG
601  GCGTATGTTC AGAATACGAT TACGGGCCGG TATTCGGTTT ATGAAGAACC
651  GCTTTACACC GTCGCCCTGA TTTATCTGTT GATGACGACT TTCTTAGGCT
701  GGATATTCCT GCGTTTGGAA AAACGTTACA ATCCGCAACA CCGCTGA
```

This encodes a protein having amino acid sequence <SEQ ID 840>:

```
  1  MDFRFDIIYE YRWMFLYGAL TTLGLTVVAT AGGSVLGLLL ALARLIHLEK
 51  AGAPMRVLAW ALRKVSLLYV TLFRGTPLFV QIVIWAYVWF PFFVHPSDGI
101  LVSGEAAIAL RRGYGPLIAG SLALIANSGA YICEIFRAGI QSIDKGQMEA
151  ARSLGLTYPQ AMRYVILPQA LRRMLPPLAS EFITLLKDSS LLSVIAVAEL
201  AYVQNTITGR YSVYEEPLYT VALIYLLMTT FLGWIFLRLE KRYNPQHR*
```

ORF129a and ORF129-1 show 100.0% identity in 248 aa overlap:

```
orf129a.pep  MDFRFDIIYEYRWMFLYGALTTLGLTVVATAGGSVLGLLLALARLIHLEKAGAPMRVLAW
             ||||||||||||||||||||||||||||||||||||||||||||||||||||||||||||
orf129-1     MDFRFDIIYEYRWMFLYGALTTLGLTVVATAGGSVLGLLLALARLIHLEKAGAPMRVLAW

orf129a.pep  ALRKVSLLYVTLFRGTPLFVQIVIWAYVWFPFFVHPSDGILVSGEAAIALRRGYGPLIAG
             ||||||||||||||||||||||||||||||||||||||||||||||||||||||||||||
orf129-1     ALRKVSLLYVTLFRGTPLFVQIVIWAYVWFPFFVHPSDGILVSGEAAIALRRGYGPLIAG

orf129a.pep  SLALIANSGAYICEIFRAGIQSIDKGQMEAARSLGLTYPQAMRYVILPQALRRMLPPLAS
             ||||||||||||||||||||||||||||||||||||||||||||||||||||||||||||
orf129-1     SLALIANSGAYICEIFRAGIQSIDKGQMEAARSLGLTYPQAMRYVILPQALRRMLPPLAS

orf129a.pep  EFITLLKDSSLLSVIAVAELAYVQNTITGRYSVYEEPLYTVALIYLLMTTFLGWIFLRLE
             ||||||||||||||||||||||||||||||||||||||||||||||||||||||||||||
orf129-1     EFITLLKDSSLLSVIAVAELAYVQNTITGRYSVYEEPLYTVALIYLLMTTFLGWIFLRLE

orf129a.pep  KRYNPQHRX
             |||||||||
orf129-1     KRYNPQHRX
```

Homology with a predicted ORF from *N.gonorrhoeae*

ORF129 shows 98.9% identity over a 88 aa overlap with a predicted ORF (ORF129ng) from *N.gonorrhoeae:*

```
orf129.pep         IIYEYRWMFLYGALTTLGLTVVAXAGGSVLGLLLALARLIHLEKAGAPMRVLAW   54
                   ||||||||||||||||||||||:|||||||||||||||||||||||||||||||
orf129ng     MDFRFDIIYEYRWMFLYGALTTLGLTVVATAGGSVLGLLLALARLIHLEKAGAPMRVLAW   60

orf129.pep   ALRKVSLLYVTLFRGTPLFVQIVIWAYVWFPFFV                           88
             ||||||||||||||||||||||||||||||||||
orf129ng     ALRKVSLLYVTLFRGTPLFVQIVIWAYVWFPFFVILHTAFLGNAMRQSRRVPDKGRWIAG  120
```

An ORF129ng nucleotide sequence <SEQ ID 841 > was predicted to encode a protein having amino acid sequence <SEQ ID 842>:

```
  1  MDFRFDIIYE YRWMFLYGAL TTLGLTVVAT AGGSVLGLLL ALARLIHLEK
 51  AGAPMRVLAW ALRKVSLLYV TLFRGTPLFV QIVIWAYVWF PFFVILHTAF
101  LGNAMRQSRR VPDKGRWIAG SLELNCQPRG RKTRGEFPPG ESNLGTEPRN
151  PLSMGQRRFP GCENWYPPQN FIKK*
```

Further work revealed the following gonococcal sequence <SEQ ID 843>:

```
  1  ATGGATTTTc gtTTTGACAT TATTTAcgaA TACCGCTGGA TGTTTCTTTA
 51  CGGCGCACTG Acgaccttgg ggctgacggt cgtggcgacg gCGGGCGGTT
101  CGGtattggG TCTGTTGTTG GCGTTGGCGC GCCTGATTCA CTTGGAAAAA
151  GCCGGTGCGC CGATGCGCGT GCTGGCGTGG GCGTTGCGTA AGGTTTCGCT
201  GCTGTACGTT ACCCTGTTCC GGGGTACGCC GCTGTTTGTG CAGATTGTGA
251  TTTGGGCGTA TGTGTGGTTT CCGTTTTTCG TCCATCCTTC AGACGGCATT
301  TTGGTCAGCG GCGAGGCGGC AATCGCGCTG CGTCGCGGAT ACGGGCCGCT
351  GATTGCCGGT TCTTTGGCAC TGATCGCCAA CTCGGGGGCG TATATCTGTG
401  AGATTTTCCG CGCGGGCATC CAGTCTATAG ACAAAGGACA GATGGAGGCG
451  GCGTGTTCTT TGGGACTGAC CTATCCGCAG GCGATGCGCT ATGTGATTCT
501  GCCGCAGGCA TTGCGCCGTA TGCTGCCGCC TTTGGCGAGC GAGTTCATCA
551  CGCTCTTGAA AGACAGCTCG CTGCTGTCGG TCATTGCTGT GGCGGAGTTG
601  GCGTATGTTC AGAATACGAT TACGGGCCGG TATTCGGTTT ATGAAGAACC
651  GCTTTACACC GCCGCCCTGA TTTATCTGTT GATGACGACT TTCTTAGGCT
701  GGATATTCCT GCGTTTGGAA AAACGTTACA ATCCGCAACA CCGCTGA
```

This corresponds to the amino acid sequence <SEQ ID 844; ORF129ng-1>:

```
  1  MDFRFDIIYE YRWMFLYGAL TTLGLTVVAT AGGSVLGLLL ALARLIHLEK
 51  AGAPMRVLAW ALRKVSLLYV TLFRGTPLFV QIVIWAYVWF PFFVHPSDGI
101  LVSGEAAIAL RRGYGPLIAG SLALIANSGA YICEIFRAGI QSIDKGQMEA
151  ARSLGLTYPQ AMRYVILPQA LRRMLPPLAS EFITLLKDSS LLSVIAVAEL
201  AYVQNTITGR YSVYEEPLYT VALIYLLMTT FLGWIFLRLE KRYNPQHR*
```

ORF129ng-1 and ORF129-1 show 99.2% identity in 248 aa overlap:

```
orf129-1.pep MDFRFDIIYEYRWMFLYGALTTLGLTVVATAGGSVLGLLLALARLIHLEKAGAPMRVLAW
             ||||||||||||||||||||||||||||||||||||||||||||||||||||||||||||
orf129ng-1   MDFRFDIIYEYRWMFLYGALTTLGLTVVATAGGSVLGLLLALARLIHLEKAGAPMRVLAW

orf129-1.pep ALRKVSLLYVTLFRGTPLFVQIVIWAYVWFPFFVHPSDGILVSGEAAIALRRGYGPLIAG
             ||||||||||||||||||||||||||||||||||||||||||||||||||||||||||||
orf129ng-1   ALRKVSLLYVTLFRGTPLFVQIVIWAYVWFPFFVHPSDGILVSGEAAIALRRGYGPLIAG

orf129-1.pep SLALIANSGAYICEIFRAGIQSIDKGQMEAARSLGLTYPQAMRYVILPQALRRMLPPLAS
             |||||||||||||||||||||||||||||| |||||||||||||||||||||||||||||
orf129ng-1   SLALIANSGAYICEIFRAGIQSIDKGQMEAACSLGLTYPQAMRYVILPQALRRMLPPLAS

orf129-1.pep EFITLLKDSSLLSVIAVAELAYVQNTITGRYSVYEEPLYTVALIYLLMTTFLGWIFLRLE
             ||||||||||||||||||||||||||||||||||||||||:|||||||||||||||||||
orf129ng-1   EFITLLKDSSLLSVIAVAELAYVQNTITGRYSVYEEPLYTAALIYLLMTTFLGWIFLRLE

orf129-1.pep KRYNPQHRX

             |||||||||
orf129ng-1   KRYNPQHRX
```

In addition, ORF129ng-1 is homologous to an ABC transporter from *A.fulgidus:*

```
2650409(AE001090) glutamine ABC transporter, permease protein (glnP)
[Archaeoglobus fulgidus]Length = 224
 Score =  132 bits (329), Expect = 2e-30
 Identities = 86/178 (48%), Positives = 103/178 (57%), Gaps = 18/178 (10%)

Query: 65  VSLLYVTLFRGTPLFVQIVIWAYVWFPFFVHPSDGILVSGEAAIALRRGYGPLIAGSLAL 124
            +S  YV + RGTPL VQI+I       +F  P+ GI +  E A           G +AL
Sbjct: 58  ISTAYVEVIRGTPLLVQILI------VYFGLPAIGINLQPEPA------------GIIAL 99

Query: 125 IANSGAYICEIFRAGIQSIDKGQMEAACSLGLTYPQAMRYVILPQALRRMLPPLASEFIT 184
               SGAYI EI RAGI+SI  GQMEAA SLG+TY QAMRYVI PQA R +LP L +EFI
Sbjct: 100 SICSGAYIAEIVRAGIESIPIGQMEAARSLGMTYLQAMRYVIFPQAFRNILPALGNEFIA 159

Query: 185 LLKDSSLLSVIAVAELAYVQNTITGRYSVYEEPLYTAALIYLLMTTFLGWIFLRLEKR 242
            LLKDSSLLSVI++ EL  V   I         P    AL YL+MT  L  +    +K+
Sbjct: 160 LLKDSSLLSVISIVELTRVGRQIVNTTFNAWTPFLGVALFYLMMTIPLSRLVAYSQKK 217
```

This analysis, including the identification of transmembrane domains in the two proteins, suggests that the proteins from *N.meningitidis* and *N.gonorrhoeae,* and their epitopes, could be useful antigens for vaccines or diagnostics, or for raising antibodies.

**Example 100**

The following partial DNA sequence was identified in *N.meningitidis* <SEQ ID 845>:

```
  1  ..CTGAAAGAAT GCCGTCTGAA AGACCCTGTT TTTATTCCAA ATATCGTTTA
 51    TAAGAACATC GCCATTACTT TCCTGCTCTT GCACGCCGCC GCCGAACTTT
101    GGCTGCCCGC GCAAACCGCC GGTTTTACCG CGCTCGCCGT CGGCTTCATC
151    CTGCTCGCCA AGCTGCGTGA gCTTCACCAT CACGAACTCT TACGTAAACA
201    cTACGTCCGC ACTTATTACy TGCTCCAACT CTTTGCCGCC GCAGgcTAgT
251    TTGTGGACAG GCGCGGCGwA ATTACAAAAC CTGCCCGCyT CCGCGCCCCT
301    GCACCTGATT ACCCTCGGCG GCATGATGGG CGGCGTGATG ATGGTGTGGc
351    TGACCGCCGG ACTGTGGCAC AGCGGCTTTA CCAAACTCGA CTACCCCAAA
401    CTCTGCCGCA TTGCCGTCCC CATCCTTTTC GCCGCCGCCG TCTCGCGCGC
451    TTTCTTGrTG AACGTGAACC CGrTATTTTT CATTACCGTT CCTGCGATTC
501    TGACCGCCGC CGTATTCGTA CTGTATCTTT TCrCGTTTAT ACCGATATTT
551    CGGGCGAATG CGTTTACAGA CGATCCGGAr TAr
```

This corresponds to the amino acid sequence <SEQ ID 846; ORF130>:

```
  1  ..LKECRLKDPV FIPNIVYKNI AITFLLLHAA AELWLPAQTA GFTALAVGFI
 51    LLAKLRELHH HELLRKHYVR TYYLLQLFAA AGSLWTGAAX LQNLPASAPL
101    HLITLGGMMG GVMMVWLTAG LWHSGFTKLD YPKLCRIAVP ILFAAAVSRA
151    FLXNVNPXFF ITVPAILTAA VFVLYLFXFI PIFRANAFTD DPE*
```

Further work revealed the complete nucleotide sequence <SEQ ID 847>:

```
   1 ATGCGGCCGT TTTTCGTCGG CGCGGCGGTG CTTGCCATAC TCGGTGCGCT
  51 GGTGTTTTTC ATCAACCCCG GTGCCATCGT CCTGCACCGC CAAATTTTCT
 101 TGGAACTTAT GCTGCCGGCG GCATACGGCG GTTTTTTGAC TGCGGCTTTG
 151 TTGGACTGGA CGGGTTTTTC GGGTAACCTG AAACCTGTCG CGACTTTGAT
 201 GGCGGCATTA TTGCTCGCCG CATCCGCTAT ACTGCCCTTT TCGCCGCAAA
 251 CTGCCTCGTT TTTCGTCGCC GCCTATTGGC TGGTGTTGCT GCTGTTCTGC
 301 GCCCGGCTGA TTTGGCTAGA CCGAAACACC GACAACTTCG CCCTGCTAAT
 351 GTTACTTGCC GCGTTCACTG TTTTTCAGAC GGCATATGCC GTCAGCGGCG
 401 ATTTGAACCT GTTGCGCGCG CAAGTGCATC TAAATATGGC GGCGGTGATG
 451 TTCGTATCCG TGCGCGTCAG TATTCTTTTG GGCGCGGAAG CCCTGAAAGA
 501 ATGCCGTCTG AAAGACCCTG TTTTTATTCC AAATATCGTT TATAAAAACA
 551 TCGCCATTAC TTTCCTGCTC TTGCACGCCG CCGCCGAACT TTGGCTGCCC
 601 GCGCAAACCG CCGGTTTTAC CGCGCTCGCC GTCGGCTTCA TCCTGCTCGC
 651 CAAGCTGCGT GAGCTTCACC ATCACGAACT CTTACGTAAA CACTACGTCC
 701 GCACTTATTA CCTGCTCCAA CTCTTTGCCG CCGCAGGCTA TTTGTGGACA

 751 GGCGCGGCGA AATTACAAAA CCTGCCCGCC TCCGCGCCCC TGCACCTGAT
 801 TACCCTCGGC GGCATGATGG GCGGCGTGAT GATGGTGTGG CTGACCGCCG
 851 GACTGTGGCA CAGCGGCTTT ACCAAACTCG ACTACCCCAA ACTCTGCCGC
 901 ATTGCCGTCC CCATCCTTTT CGCCGCCGCC GTCTCGCGCG CTTTCTTGAT
 951 GAACGTGAAC CCGATATTTT TCATTACCGT TCCTGCGATT CTGACCGCCG
1001 CCGTATTCGT ACTGTATCTT TTCACGTTTA TACCGATATT TCGGGCGAAT
1051 GCGTTTACAG ACGATCCGGA ATAA
```

This corresponds to the amino acid sequence <SEQ ID 848; ORF130-1>:

```
   1 MRPFFVGAAV LAILGALVFF INPGAIVLHR QIFLELMLPA AYGGFLTAAL
  51 LDWTGFSGNL KPVATLMAAL LLAASAILPF SPQTASFFVA AYWLVLLLFC
 101 ARLIWLDRNT DNFALLMLLA AFTVFQTAYA VSGDLNLLRA QVHLNMAAVM
 151 FVSVRVSILL GAEALKECRL KDPVFIPNIV YKNIAITFLL LHAAAELWLP
 201 AQTAGFTALA VGFILLAKLR ELHHHELLRK HYVRTYYLLQ LFAAAGYLWT
 251 GAAKLQNLPA SAPLHLITLG GMMGGVMMVW LTAGLWHSGF TKLDYPKLCR
 301 IAVPILFAAA VSRAFLMNVN PIFFITVPAI LTAAVFVLYL FTFIPIFRAN
 351 AFTDDPE*
```

Computer analysis of this amino acid sequence gave the following results:

Homology with a predicted ORF from *N. meningitidis* (strain A)

ORF130 shows 94.3% identity over a 193aa overlap with an ORF (ORF130a) from strain A of *N. meningitidis:*

```
                                                      10         20         30
orf130.pep                               LKECRLKDPVFIPNIVYKNIAITFLLLHAA
                                         ||||||||||||||:|||||||||||||||
orf130a      LNLLRAQVHLNMAAVMFVSVRVSILLGAEALKECRLKDPVFIPNVVYKNIAITFLLLHAA
                   140       150       160       170       180       190

                        40         50         60         70         80         90
orf130.pep   AELWLPAQTAGFTALAVGFILLAKLRELHHHELLRKHYVRTYYLLQLFAAAGSLWTGAAX
             ||||||||||||:||||||||||||||||||||||||||||||||||||| |||||
orf130a      AELWLPAQTAGFTSLAVGFILLAKLRELHHHELLRKHYVRTYYLLQLFAAAGYLWTGAAK
                   200       210       220       230       240       250

                       100        110        120        130        140        150
orf130.pep   LQNLPASAPLHLITLGGMMGGVMMVWLTAGLWHSGFTKLDYPKLCRIAVPILFAAAVSRA
             ||||||||||||||||||||:|||||||||||||||||||||||||||||||||||||||
orf130a      LQNLPASAPLHLITLGGMMGSVMMVWLTAGLWHSGFTKLDYPKLCRIAVPILFAAAVSRA
                   260       270       280       290       300       310

                       160        170        180        190
orf130.pep   FLXNVNPXFFITVPAILTAAVFVLYLFXFIPIFRANAFTDDPEX
             | |||| |||||||||||||||||||::|:||||||||||||||
orf130a      VLMNVNPIFFITVPAILTAAVFVLYLLTFVPIFRANAFTDDPEX
                   320       330       340       350
```

The complete length ORF130a nucleotide sequence <SEQ ID 849> is:

```
   1  ATGCGGCCGT TTTTCGTCGG CGCGGCGGTG CTTGCCATAC TCGGTGCGCT
  51  GGTGTTTTTC ATCAACCCCG GTGCCATCGT CCTGCACCGC CAAATTTTCT
 101  TGGAACTTAT GCTGCCGGCG GCATACGGCG GTTTTTTGAC TGCGGCTTTG
 151  TTGGACTGGA CGGGTTTTTC GGGTAACCTG AAACCTGTCG CGACTTTGAT
 201  GGCGGCATTA TTGCTCGCCG CATCCGCTAT ACTGCCCTTT TCGCCGCAAA
 251  CTGCCTCGTT TTTCGTCGCC GCCTATTGGC TGGTGTTGCT GCTGTTCTGC
 301  GCCCGGCTGA TTTGGCTAGA CCGAAACACC GACAACTTCG CCCTGCTAAT
 351  GTTACTTGCC GCGTTCACTG TTTTTCAGAC GGCATATGCC GTCAGCGGCG
 401  ATTTGAACCT GTTGCGCGCG CAAGTGCATC TAAATATGGC GGCGGTGATG
 451  TTCGTATCCG TGCGCGTCAG TATTCTTTTG GGCGCGGAAG CCCTGAAAGA
 501  ATGCCGTCTG AAAGACCCAG TATTCATCCC CAATGTCGTC TATAAAAACA
 551  TCGCCATTAC CTTCCTGCTC CTGCACGCCG CCGCCGAACT TTGGCTGCCT
 601  GCGCAAACCG CCGGTTTTAC CTCGCTCGCC GTCGGCTTTA TCCTGCTTGC
 651  CAAGCTGCGT GAGCTTCACC ATCACGAACT CCTGCGCAAA CACTACGTCC
 701  GCACTTATTA CCTGCTCCAA CTCTTTGCCG CCGCAGGCTA TTTGTGGACA
 751  GGCGCGGCGA AATTACAAAA CCTGCCCGCC TCCGCGCCCC TGCACCTGAT
 801  TACCCTCGGT GGCATGATGG GCAGCGTGAT GATGGTGTGG CTGACTGCCG
 851  GACTGTGGCA CAGCGGCTTT ACCAAGCTCG ACTACCCGAA ACTCTGCCGC

 901  ATCGCCGTCC CCATCCTNTT CGCCGCCGCC GTTTCGCGCG CTGTTTTAAT
 951  GAACGTAAAC CCGATATTCT TCATCACCGT CCCCGCAATT CTGACCGCCG
1001  CCGTGTTCGT GCTTTACCTG CTGACATTCG TACCGATCTT TCGGGCGAAC
1051  GCGTTTACAG ACGATCCGGA ATAA
```

This encodes a protein having amino acid sequence <SEQ ID 850>:

```
  1  MRPFFVGAAV LAILGALVFF INPGAIVLHR QIFLELMLPA AYGGFLTAAL
 51  LDWTGFSGNL KPVATLMAAL LLAASAILPF SPQTASFFVA AYWLVLLLFC
101  ARLIWLDRNT DNFALLMLLA AFTVFQTAYA VSGDLNLLRA QVHLNMAAVM
151  FVSVRVSILL GAEALKECRL KDPVFIPNVV YKNIAITFLL LHAAAELWLP
201  AQTAGFTSLA VGFILLAKLR ELHHHELLRK HYVRTYYLLQ LFAAAGYLWT
251  GAAKLQNLPA SAPLHLITLG GMMGSVMMVW LTAGLWHSGF TKLDYPKLCR
301  IAVPILFAAA VSRAVLMNVN PIFFITVPAI LTAAVFVLYL LTFVPIFRAN
351  AFTDDPE*
```

ORF130a and ORF130-1 show 98.3% identity in 357 aa overlap:

```
orf130a.pep   MRPFFVGAAVLAILGALVFFINPGAIVLHRQIFLELMLPAAYGGFLTAALLDWTGFSGNL
              ||||||||||||||||||||||||||||||||||||||||||||||||||||||||||||
orf130-1      MRPFFVGAAVLAILGALVFFINPGAIVLHRQIFLELMLPAAYGGFLTAALLDWTGFSGNL

orf130a.pep   KPVATLMAALLLAASAILPFSPQTASFFVAAYWLVLLLFCARLIWLDRNTDNFALLMLLA
              ||||||||||||||||||||||||||||||||||||||||||||||||||||||||||||
orf130-1      KPVATLMAALLLAASAILPFSPQTASFFVAAYWLVLLLFCARLIWLDRNTDNFALLMLLA

orf130a.pep   AFTVFQTAYAVSGDLNLLRAQVHLNMAAVMFVSVRVSILLGAEALKECRLKDPVFIPNVV
              ||||||||||||||||||||||||||||||||||||||||||||||||||||||||||:|
orf130-1      AFTVFQTAYAVSGDLNLLRAQVHLNMAAVMFVSVRVSILLGAEALKECRLKDPVFIPNIV

orf130a.pep   YKNIAITFLLLHAAAELWLPAQTAGFTSLAVGFILLAKLRELHHHELLRKHYVRTYYLLQ
              ||||||||||||||||||||||||||:|||||||||||||||||||||||||||||||||
orf130-1      YKNIAITFLLLHAAAELWLPAQTAGFTALAVGFILLAKLRELHHHELLRKHYVRTYYLLQ

orf130a.pep   LFAAAGYLWTGAAKLQNLPASAPLHLITLGGMMGSVMMVWLTAGLWHSGFTKLDYPKLCR
              |||||||||||||||||||||||||||||||||||:||||||||||||||||||||||||
orf130-1      LFAAAGYLWTGAAKLQNLPASAPLHLITLGGMMGGVMMVWLTAGLWHSGFTKLDYPKLCR

orf130a.pep   IAVPILFAAAVSRAVLMNVNPIFFITVPAILTAAVFVLYLLTFVPIFRANAFTDDPE
              ||||||||||||| |||||||||||||||||||||||||:||:||||||||||||||
orf130-1      IAVPILFAAAVSRAFLMNVNPIFFITVPAILTAAVFVLYLFTFIPIFRANAFTDDPE
```

<u>Homology with a predicted ORF from *N.gonorrhoeae*</u>

ORF130 shows 91.7% identity over a 193 aa overlap with a predicted ORF (ORF130ng) from *N. gonorrhoeae:*

```
orf130.pep                                LKECRLKDPVFIPNIVYKNIAITFLLLHAA   30
                                          ||||||||||||||::||||||| ||||||
orf130ng     LNLLRAQVHLNMAAVMFVSVRVSVLLGTETLKECRLKDPVFIPNVIYKNIAIT-LLLHAA  201

orf130.pep   AELWLPAQTAGFTALAVGFILLAKLRELHHHELLRKHYVRTYYLLQLFAAAGSLWTGAAX   90
             |||||||||||||||||||||||||||||||||||||||||||||||||||| ||||||
orf130ng     AELWLPAQTAGFTALAVGFILLAKLRELHHHELLRKHYVRTYYLLQLFAAAGYLWTGAAK  261

orf130.pep   LQNLPASAPLHLITLGGMMGGVMMVWLTAGLWHSGFTKLDYPKLCRIAVPILFAAAVSRA  150
             ||||||||||||||||| |||||||||||||||||||||||||||| ||||:|||||
orf130ng     LQNLPASAPLHLITLGGMTGGVMMVWLTAGLWHSGFTKLDYPKLCRIAVSILFASAVSRA  321

orf130.pep   FLXNVNPXFFITVPAILTAAVFVLYLFXFIPIFRANAFTDDPE  193
              | |||| |||||| |||||||:|||::|:|||||||||||||
orf130ng     VLMNVNPIFFITVPEILTAAVFMLYLLTFVPIFRANAFTDDPE  364
```

An ORF130ng nucleotide sequence <SEQ ID 851> was predicted to encode a protein having amino acid sequence <SEQ ID 852>:

```
1   MNKFFTHPMR PFFVGAAVLA ILGALVFFHQ PRRYHPAPPN FLGTYAAGCI
51  RRFFDYRFVG PDGFFRQPET CRYFDGGVVA CCGCFIAVFT ATCRIFRRRL

101 LAGVAAVLRL ADLARRQHRT LRSVDVTAAF TVFQTAYAVS GDLNLLRAQV
151 HLNMAAVMFV SVRVSVLLGT ETLKECRLKD PVFIPNVIYK NIAITLLLHA
201 AAELWLPAQT AGFTALAVGF ILLAKLRELH HHELLRKHYV RTYYLLQLFA
251 AAGYLWTGAA KLQNLPASAP LHLITLGGMT GGVMMVWLTA GLWHSGFTKL
301 DYPKLCRIAV SILFASAVSR AVLMNVNPIF FITVPEILTA AVFMLYLLTF
351 VPIFRANAFT DDPE*
```

Further work revealed the following gonococcal DNA sequence <SEQ ID 853>:

```
   1 ATGCGCCCGT TTTTCGTCGG TGCGGCAGTA CTTGCCATAC TCGGTGCGTT
  51 GGTGTTTTTT ATCAACCCCG GCGCTATCAT CCTGCACCGC CAAATTTTCT
 101 TGGAACTTAT GCTGCCGGCT GCATACGGCG GTTTTTTGAC TACCGCTTTG
 151 TTGGACCGGA CGGGTTTTTC AGGCAACCTG AAACCTGCCG CTACTTTGAT
 201 GGCGGTGTTG TTGCTTGTTG CGGCTGTTTT ATTGCCGTTT TTACCGCAAC
 251 TTGCCGCATT TTTCGTCGCC GCCTATTGGC TGGTGTTGCT GCTGTTCTGC
 301 GCCTGGCTGA TTTGGCTCGA CCGCAACACC GACAACTTCG CTCTGTTGAT
 351 GTTACTTGCC GCATTTACCG TTTTTCAGAC GGCCTATGCC GTCAGCGGCG
 401 ATTTGAACTT ACTGCGCGCG CAAGTGCATT TGAATATGGC GGCGGTCATG
 451 TTCGTATCCG TCCGCGTCAG CGTCCTTTTG GGCACGGAAA CCCTGAAAGA
 501 ATGCCGTCTG AAAGACCCCG TATTCATCCC CAACGTTATC TATAAAAACA
 551 TCGCCATCAC CCTGCTGCTG CACGCCGCCG CCGAACTTTG GCTGCCCGCG
 601 CAAACCGCCG GTTTTACTGC GCTTGCCGTC GGCTTCATCC TGCTCGCCAA
 651 GCTGCGCGAA CTGCACCATC ACGAACTCTT ACGCAAACAC TACGTCCGCA
 701 CTTATTACCT GCTCCAGCTC TTTGCCGCCG CAGGTTATCT GTGGACAGGC
 751 GCGGCGAAAC TGCAAAACCT GCCCGCCTCC GCGCCCCTGC ACCTGATTAC
 801 CCTCGGCGGC ATGACGGGTG GCGTGATGAT GGTGTGGCTG ACTGCCGGAC
 851 TGTGGCACAG CGGCTTTACC AAACTCGACT ACCCGAAACT CTGCCGCATC
 901 GCCGTCTCCA TCCTTTTCGC CTCCGCCGTT TCGCGCGCTG TTTTAATGAA
 951 CGTGAATCCG ATATTCTTCA TCACCGTTCC CGAGATTCTG ACCGCCGCCG
1001 TGTTCATGCT TTACCTGCTG ACGTTCGTAC CGATTTTTCG AGCGAACGCG
1051 TTTACAGACG ATCCGGAATA A
```

This corresponds to the amino acid sequence <SEQ ID 854; ORF130ng-1>:

```
  1 MRPFFVGAAV LAILGALVFF INPGAIILHR QIFLELMLPA AYGGFLTTAL
 51 LDRTGFSGNL KPAATLMAVL LLVAAVLLPF LPQLAAFFVA AYWLVLLLFC
101 AWLIWLDRNT DNFALLMLLA AFTVFQTAYA VSGDLNLLRA QVHLNMAAVM
151 FVSVRVSVLL GTETLKECRL KDPVFIPNVI YKNIAITLLL HAAAELWLPA
201 QTAGFTALAV GFILLAKLRE LHHHELLRKH YVRTYYLLQL FAAAGYLWTG
251 AAKLQNLPAS APLHLITLGG MTGGVMMVWL TAGLWHSGFT KLDYPKLCRI
301 AVSILFASAV SRAVLMNVNP IFFITVPEIL TAAVFMLYLL TFVPIFRANA
351 FTDDPE*
```

ORF130ng-1 and ORF130-1 show 92.4% identity in 357 aa overlap:

```
orf130-1.pep   MRPFFVGAAVLAILGALVFFINPGAIVLHRQIFLELMLPAAYGGFLTAALLDWTGFSGNL
               ||||||||||||||||||||||||||:|||||||||||||||||||:|||| ||||||||
orf130ng-1     MRPFFVGAAVLAILGALVFFINPGAIILHRQIFLELMLPAAYGGFLTTALLDRTGFSGNL

orf130-1.pep   KPVATLMAALLLAASAILPFSPQTASFFVAAYWLVLLLFCARLIWLDRNTDNFALLMLLA
               ||:|||||:|||:|:::||| || |:||||||||||||||| ||||||||||||||||||
orf130ng-1     KPAATLMAVLLLVAAVLLPFLPQLAAFFVAAYWLVLLLFCAWLIWLDRNTDNFALLMLLA

orf130-1.pep   AFTVFQTAYAVSGDLNLLRAQVHLNMAAVMFVSVRVSILLGAEALKECRLKDPVFIPNIV
               |||||||||||||||||||||||||||||||||||||:|||:|:||||||||||||||::
orf130ng-1     AFTVFQTAYAVSGDLNLLRAQVHLNMAAVMFVSVRVSVLLGTETLKECRLKDPVFIPNVI

orf130-1.pep   YKNIAITFLLLHAAAELWLPAQTAGFTALAVGFILLAKLRELHHHELLRKHYVRTYYLLQ
               ||||||| ||||||||||||||||||||||||||||||||||||||||||||||||||||
orf130ng-1     YKNIAIT-LLLHAAAELWLPAQTAGFTALAVGFILLAKLRELHHHELLRKHYVRTYYLLQ

orf130-1.pep   LFAAAGYLWTGAAKLQNLPASAPLHLITLGGMMGGVMMVWLTAGLWHSGFTKLDYPKLCR
               |||||||||||||||||||||||||||||||| |||||||||||||||||||||||||||
orf130ng-1     LFAAAGYLWTGAAKLQNLPASAPLHLITLGGMTGGVMMVWLTAGLWHSGFTKLDYPKLCR

orf130-1.pep   IAVPILFAAAVSRAFLMNVNPIFFITVPAILTAAVFVLYLFTFIPIFRANAFTDDPEX
               ||| ||||:||||| ||||||||||||| |||||||:|||:|:|||||||||||||||
orf130ng-1     IAVSILFASAVSRAVLMNVNPIFFITVPEILTAAVFMLYLLTFVPIFRANAFTDDPEX
```

Based on this analysis, it is predicted that the proteins from *N.meningitidis* and *N.gonorrhoeae,* and their epitopes, could be useful antigens for vaccines or diagnostics, or for raising antibodies.

**Example 101**

The following partial DNA sequence was identified in *N.meningitidis* <SEQ ID 855>:

```
  1  ATGGAAATTC GGGCAATAAA ATATACGGCA ATGGCTGCGT TGCTTGCATT
 51  TACGGTTGCA GGCTGCCGGC TGGCGGGGTG GTATGAGTGT TCGTCCCTCA
101  CCGGCTGGTG TAAGCCGAGA AAACCGGCTG CCATCGATTT TTGGGATATT
151  GGCGGCGAGA GTCCGCCGTC TTTAGGGGAC TACGAGATAC CGCTTTCAGA
201  CGGCAATAGT TCCGTCAGGG CAAACGAATA TGAATCCGCA CAACAATCTT
251  ACTTTTACAG GAAAATAGGG AAGTTTGAAG C.TGCGGGCT GGATTGGCGT
301  ACGCGTGACG GCAAACCTTT GATTGAGACG TTCAAACAGG GAGGATTTGA
351  CTGCTTGGAA AAG..
```

This corresponds to the amino acid sequence <SEQ ID 856; ORF131>:

```
  1  MEIRAIKYTA MAALLAFTVA GCRLAGWYEC SSLTGWCKPR KPAAIDFWDI
 51  GGESPPSLGD YEIPLSDGNS SVRANEYESA QQSYFYRKIG KFEXCGLDWR
101  TRDGKPLIET FKQGGFDCLE K..
```

Further work revealed the complete nucleotide sequence <SEQ ID 857>:

```
  1  ATGGAAATTC GGGCAATAAA ATATACGGCA ATGGCTGCGT TGCTTGCATT
 51  TACGGTTGCA GGCTGCCGGC TGGCGGGGTG GTATGAGTGT TCGTCCCTCA
101  CCGGCTGGTG TAAGCCGAGA AAACCGGCTG CCATCGATTT TTGGGATATT
151  GGCGGCGAGA GTCCGCCGTC TTTAGGGGAC TACGAGATAC CGCTTTCAGA
201  CGGCAATCGT TCCGTCAGGG CAAACGAATA TGAATCCGCA CAACAATCTT
251  ACTTTTACAG GAAAATAGGG AAGTTTGAAG CCTGCGGGCT GGATTGGCGT
301  ACGCGTGACG GCAAACCTTT GATTGAGACG TTCAAACAGG GAGGATTTGA
351  CTGCTTGGAA AAGCAGGGGT TGCGGCGCAA CGGTCTGTCC GAGCGCGTCC
401  GATGGTAA
```

This corresponds to the amino acid sequence <SEQ ID 858; ORF131-1>:

```
  1  MEIRAIKYTA MAALLAFTVA GCRLAGWYEC SSLTGWCKPR KPAAIDFWDI
 51  GGESPPSLGD YEIPLSDGNR SVRANEYESA QQSYFYRKIG KFEACGLDWR
101  TRDGKPLIET FKQGGFDCLE KQGLRRNGLS ERVRW*
```

Computer analysis of this amino acid sequence gave the following results:

Homology with a predicted ORF from *N.meningitidis* (strain A)

ORF131 shows 95.0% identity over a 121 aa overlap with an ORF (ORF 131 a) from strain A of *N. meningitidis:*

```
                      10        20        30        40        50        60
orf131.pep    MEIRAIKYTAMAALLAFTVAGCRLAGWYECSSLTGWCKPRKPAAIDFWDIGGESPPSLGD
              |||||||||||||||||||||||||||||||||:||||||||||||||||||||||||| |
orf131a       MEIRAIKYTAMAALLAFTVAGCRLAGWYECSSLSGWCKPRKPAAIDFWDIGGESPPSLED
                      10        20        30        40        50        60

                      70        80        90       100       110       120
orf131.pep    YEIPLSDGNSSVRANEYESAQQSYFYRKIGKFEXCGLDWRTRDGKPLIETFKQGGFDCLE
              ||||||||| |||||||||||||||||||||||| ||||||||||||||||||| |||||:
orf131a       YEIPLSDGNRSVRANEYESAQQSYFYRKIGKFEACGLDWRTRDGKPLIETFKQEGFDCLK
                      70        80        90       100       110       120

orf131.pep    K
              |
orf131a       KQGLRRNGLSERVRWX
                     130
```

The complete length ORF131a nucleotide sequence <SEQ ID 859> is:

```
  1  ATGGAAATTC GGGCAATAAA ATATACGGCA ATGGCTGCGT TGCTTGCATT
 51  TACGGTTGCA GGCTGCCGGT TGGCAGGTTG GTATGAGTGT TCGTCCCTGT
101  CCGGCTGGTG TAAGCCGAGA AAACCTGCCG CCATCGATTT TTGGGATATT
151  GGCGGCGAGA GTCCTCCGTC TTTAGAGGAC TACGAGATAC CGCTTTCAGA
201  CGGCAATCGT TCCGTCAGGG CAAACGAATA TGAATCCGCA CAACAATCTT
251  ACTTTTACAG GAAAATAGGG AAGTTTGAAG CCTGCGGGTT GGATTGGCGT
301  ACGCGTGACG GCAAACCTTT GATTGAGACG TTCAAACAGG AAGGTTTTGA
351  TTGTTTGAAA AAGCAGGGGT TGCGGCGCAA CGGTCTGTCC GAGCGCGTCC
401  GATGGTAA
```

This encodes a protein having amino acid sequence <SEQ ID 860>:

```
  1  MEIRAIKYTA MAALLAFTVA GCRLAGWYEC SSLSGWCKPR KPAAIDFWDI
 51  GGESPPSLED YEIPLSDGNR SVRANEYESA QQSYFYRKIG KFEACGLDWR
101  TRDGKPLIET FKQEGFDCLK KQGLRRNGLS ERVRW*
```

ORF131a and ORF131-1 show 97.0% identity in 13 5 aa overlap:

```
orf131a.pep   MEIRAIKYTAMAALLAFTVAGCRLAGWYECSSLSGWCKPRKPAAIDFWDIGGESPPSLED
              |||||||||||||||||||||||||||||||||:|||||||||||||||||||||||| |
orf131-1      MEIRAIKYTAMAALLAFTVAGCRLAGWYECSSLTGWCKPRKPAAIDFWDIGGESPPSLGD

orf131a.pep   YEIPLSDGNRSVRANEYESAQQSYFYRKIGKFEACGLDWRTRDGKPLIETFKQEGFDCLK
              ||||||||||||||||||||||||||||||||||||||||||||||||||||| |||||:
orf131-1      YEIPLSDGNRSVRANEYESAQQSYFYRKIGKFEACGLDWRTRDGKPLIETFKQGGFDCLE

orf131a.pep   KQGLRRNGLSERVRWX
              |||||||||||||||
orf131-1      KQGLRRNGLSERVRWX
```

Homology with a predicted ORF from *N.gonorrhoeae*

ORF131 shows 89.3% identity over 121 aa overlap with a predicted ORF (ORF131ng) from *N.gonorrhoeae:*

```
orf131.pep   MEIRAIKYTAMAALLAFTVAGCRLAGWYECSSLTGWCKPRKPAAIDFWDIGGESPPSLGD    60
             ||||:||||| |||:||||||||||||||| ||:||||||||||||||||||||| || |
orf131ng     MEIRVIKYTATAALFAFTVAGCRLAGWYECLSLSGWCKPRKPAAIDFWDIGGESPLSLED    60

orf131.pep   YEIPLSDGNSSVRANEYESAQQSYFYRKIGKFEXCGLDWRTRDGKPLIETFKQGGFDCLE   120
             ||||||||| |||||||||||:||||||||||| |||||||||||||:| ||| ||||||
orf131ng     YEIPLSDGNRSVRANEYESAQKSYFYRKIGKFEACGLDWRTRDGKPLVERFKQEGFDCLE   120

orf131.pep   K              121
             |
orf131ng     KQGLRRNGLSERVRW  134
```

A complete length ORF131ng nucleotide sequence <SEQ ID 861> was predicted to encode a protein having amino acid sequence <SEQ ID 862>:

```
  1   MEIRVIKYTA TAALFAFTVA GCRLAGWYEC LSLSGWCKPR KPAAIDFWDI
 51   GGESPLSLED YEIPLSDGNR SVRANEYESA QKSYFYRKIG KFEACGLDWR
101   TRDGKPLVER FKQEGFDCLE KQGLRRNGLS ERVRW*
```

Further work revealed the following gonococcal DNA sequence <SEQ ID 863>:

```
  1   ATGGAAATTC GGGTAATAAA ATATACGGCA ACGGCTGCGT TGTTTGCATT
 51   TACGGTTGCA GGCTGCCGGC TGGCGGGGTG GTATGAGTGT TCGTCCTTGT
101   CCGGCTGGTG TAAGCCGAGA AAACCTGCCG CCATCGATTT TTGGGATATT
151   GGCGGCGAGA GtccgctGTC TTTAGAGGAC TACGAGATAC CGCTTTCAGA
201   CGGCAATCGT TCCGTCAGGG CAAACGAATA TGAATCCGCG CAAAAATCTT
251   ACTTTTATAG GAAAATAGGG AAGTTTGAAG CCTGCGGGTT GGATTGGCGT
301   ACGCGTGACG GCAAACCTTT GGTTGAGAGG TTCAAACAGG AAGGTTTCGA
351   CTGTTTGGAA AAGCAGGGGT TGCGGCGCAA CGGCCTGTCC GAGCGCGTCC
401   GATGGTAA
```

This corresponds to the amino acid sequence <SEQ ID 864; ORF131ng-1>:

```
  1   MEIRVIKYTA TAALFAFTVA GCRLAGWYEC SSLSGWCKPR KPAAIDFWDI
 51   GGESPLSLED YEIPLSDGNR SVRANEYESA QKSYFYRKIG KFEACGLDWR
101   TRDGKPLVER FKQEGFDCLE KQGLRRNGLS ERVRW*
```

ORF131ng-1 and ORF131-1 show 92.6% identity in 135 aa overlap:

```
orf131ng-1.pep MEIRVIKYTATAALFAFTVAGCRLAGWYECSSLSGWCKPRKPAAIDFWDIGGESPLSLED
               ||||:||||| |||:||||||||||||||||||:||||||||||||||||||||| || |
orf131-1       MEIRAIKYTAMAALLAFTVAGCRLAGWYECSSLTGWCKPRKPAAIDFWDIGGESPPSLGD

orf131ng-1.pep YEIPLSDGNRSVRANEYESAQKSYFYRKIGKFEACGLDWRTRDGKPLVERFKQEGFDCLE
               |||||||||||||||||||||:|||||||||||||||||||||||||:| ||| ||||||
orf131-1       YEIPLSDGNRSVRANEYESAQQSYFYRKIGKFEACGLDWRTRDGKPLIETFKQGGFDCLE

orf131ng-1.pep KQGLRRNGLSERVRWX
               ||||||||||||||||
orf131-1       KQGLRRNGLSERVRWX
```

Based on the presence of a predicted prokaryotic membrane lipoprotein lipid attachment site, it is predicted that the proteins from *N.meningitidis* and *N.gonorrhoeae,* and their epitopes, could be useful antigens for vaccines or diagnostics, or for raising antibodies.

**Example 102**

The following partial DNA sequence was identified in *N.meningitidis* <SEQ ID 865>

```
  1 ATGAAACACA TCCATATTAT CGGTATCGGC GGCACGTTTA TGGGCGGGCT
 51 TGCCGCCATT GCCAAAGAAG CGGGGTTTGA AGTCAGCGGT TGCGACGCGA
101 AGATGTATCC GCCGATGAGC ACCCAGCTCG AAGCCTTGGG TATAGACGTG
151 TATGAAGGCT TCGATGCCGC TCAGTTGGAC GAATTTAAAG CCGACGTTTA
201 CGTTATCGGC AATGTCGCCA AGCGCGGGAT GGATGTGGTT GAAGCGATTT
251 TGAACCTCGG CCTGCCtTAT ATtTcCGGCC CGCAATGGCT GTCGGAAAAC
301 GTGCTGCACC ATCATTGGGT ACTCGGTGTG GCGGGGACgC ACGGCAAAAC
351 GACCACCGCC TCCATGCTCG CATGGGTCTT GGAATATgCC GGCCTCGCGC
401 CGGGCTTCCT TATtGGCGGC GTACC.GGAA AATttCGGCG TTTCCGCCCG
451 CCTGCCGCAA ACGCCGCGCC AAGACCCGAA CAGCCAATCG CCGTTTTTcG
501 TCATCGAAGC CGACGAATAC GACACCGCCT TTtTCGACAA ACGTTCTAAA
551 TtCGTGCATT ACCGTCCGCG TACCGCCGTG TTGAACAATC TGGAATTCGA
601 CCACGCCGAC ATCTTTGCCG ACTTGGGCGC GATACAGACc CAGTTCCACT
651 ACCTCGTGCG TACCGTGCCG TCTGAAGGCT TAATCGTCTG CAACGGACGG
701 CAGCAAAGCC TGCAAGATAC TTTGGACAAA GGCTGCTGGA CGCCGGTGGA
751 AAAATTCGGC ACGGAACACG GCTGGCA..
```

This corresponds to the amino acid sequence <SEQ ID 866; ORF132>:

```
  1 MKHIHIIGIG GTFMGGLAAI AKEAGFEVSG CDAKMYPPMS TQLEALGIDV
 51 YEGFDAAQLD EFKADVYVIG NVAKRGMDVV EAILNLGLPY ISGPQWLSEN
101 VLHHHWVLGV AGTHGKTTTA SMLAWVLEYA GLAPGFLIGG VXGKFRRFRP
151 PAANAAPRPE QPIAVFRHRS RRIRHRLFRQ TFXIRALPSA YRRVEQSGIR
201 PRRHLCRLGR DTDPVPLPRA YRAVXRLNRL QRTAAKPARY FGQRLLDAGG
251 KIRHGTRLA..
```

Further work revealed the complete nucleotide sequence <SEQ ID 867>:

```
   1 ATGAAACACA TCCATATTAT CGGTATCGGC GGCACGTTTA TGGGCGGGCT
  51 TGCCGCCATT GCCAAAGAAG CGGGGTTTGA AGTCAGCGGT TGCGACGCGA
 101 AGATGTATCC GCCGATGAGC ACCCAGCTCG AAGCCTTGGG TATAGACGTG
 151 TATGAAGGCT TCGATGCCGC TCAGTTGGAC GAATTTAAAG CCGACGTTTA
 201 CGTTATCGGC AATGTCGCCA AGCGCGGGAT GGATGTGGTT GAAGCGATTT
 251 TGAACCTCGG CCTGCCTTAT ATTTCCGGCC CGCAATGGCT GTCGGAAAAC
 301 GTGCTGCACC ATCATTGGGT ACTCGGTGTG GCGGGGACGC ACGGCAAAAC
 351 GACCACCGCC TCCATGCTCG CATGGGTCTT GGAATATGCC GGCCTCGCGC
 401 CGGGCTTCCT TATTGGCGGC GTACCGGAAA ATTTCGGCGT TTCCGCCCGC
 451 CTGCCGCAAA CGCCGCGCCA AGACCCGAAC AGCCAATCGC CGTTTTTCGT
 501 CATCGAAGCC GACGAATACG ACACCGCCTT TTTCGACAAA CGTTCTAAAT
 551 TCGTGCATTA CCGTCCGCGT ACCGCCGTGT TGAACAATCT GGAATTCGAC
 601 CACGCCGACA TCTTTGCCGA CTTGGGCGCG ATACAGACCC AGTTCCACTA
```

```
 651 CCTCGTGCGT ACCGTGCCGT CTGAAGGCTT AATCGTCTGC AACGGACGGC
 701 AGCAAAGCCT GCAAGATACT TTGGACAAAG GCTGCTGGAC GCCGGTGGAA
 751 AAATTCGGCA CGGAACACGG CTGGCAGGCC GGCGAAGCCA ATGCCGACGG
 801 CTCGTTCGAC GTGTTGCTCG ACGGCAAAAC CGCCGGACGC GTCAAATGGG
 851 ATTTGATGGG CAGGCACAAC CGCATGAACG CGCTCGCCGT CATTGCCGCC
 901 GCGCGTCATG TCGGTGTCGA TATTCAGACC GCCTGCGAAG CCTTGGGCGC
 951 GTTTAAAAAC GTCAAACGCC GGATGGAAAT CAAAGGCACG GCAAACGGCA
1001 TCACCGTTTA CGACGACTTC GCCCACCACC CGACCGCCAT CGAAACCACG
1051 ATTCAAGGTT TGCGCCAACG CGTCGGCGGC GCGCGCATCC TCGCCGTCCT
1101 CGAACCGCGT TCCAACACGA TGAAGCTGGG CACGATGAAG TCCGCCCTGC
1151 CTGTAAGCCT CAAAGAAGCC GACCAAGTGT TCTGCTACGC CGGCGGCGTG
1201 GACTGGGACG TCGCCGAAGC CCTCGCGCCT TTGGGCGGCA GGCTGAACGT
1251 CGGCAAAGAC TTCGATGCCT TCGTTGCCGA AATCGTGAAA AACGCCGAAG
1301 TAGGCGACCA TATTTTGGTG ATGAGCAACG GCGGTTTCGG CGGAATACAC
1351 GGAAAGCTGC TGGAAGCTTT GAGATAG
```

This corresponds to the amino acid sequence <SEQ ID 868; ORF132-1>:

```
  1 MKHIHIIGIG GTFMGGLAAI AKEAGFEVSG CDAKMYPPMS TQLEALGIDV
 51 YEGFDAAQLD EFKADVYVIG NVAKRGMDVV EAILNLGLPY ISGPQWLSEN
101 VLHHHWVLGV AGTHGKTTTA SMLAWVLEYA GLAPGFLIGG VPENFGVSAR
151 LPQTPRQDPN SQSPFFVIEA DEYDTAFFDK RSKFVHYRPR TAVLNNLEFD
201 HADIFADLGA IQTQFHYLVR TVPSEGLIVC NGRQQSLQDT LDKGCWTPVE
251 KFGTEHGWQA GEANADGSFD VLLDGKTAGR VKWDLMGRHN RMNALAVIAA
301 ARHVGVDIQT ACEALGAFKN VKRRMEIKGT ANGITVYDDF AHHPTAIETT
351 IQGLRQRVGG ARILAVLEPR SNTMKLGTMK SALPVSLKEA DQVFCYAGGV
401 DWDVAEALAP LGGRLNVGKD FDAFVAEIVK NAEVGDHILV MSNGGFGGIH
451 GKLLEALR*
```

Computer analysis of this amino acid sequence gave the following results:

<u>Homology with the hypothetical o457 protein of *E.coli* (accession number U14003)</u>

ORF132 and o457 show 58% aa identity in 140 aa overlap:

```
Orf132: 4   IHIIGIGGTFMGGLAAIAKEAGFEVSGCDAKMYPPMSTQLEALGIDVYEGFDAAQLDEFK 63
            IHI+GI GTFMGGLA +A++ G EV+G DA +YPPMST LE  GI++ +G+DA+QL+  +
o457:   3   IHILGICGTFMGGLAMLARQLGHEVTGSDANVYPPMSTLLEKQGIELIQGYDASQLEP-Q 61

Orf132: 64  ADVYVIGNVAKRGMDVVEAILNLGLPYISGPQWLSENVLHHHWVLGVAGTHGKTTTASML 123
             D+ +IGN   RG   VEA+L   +PY+SGPQWL + VL   WVL VAGTHGKTTTA M
o457:   62  PDLVIIGNAMTRGNPCVEAVLEKNIPYMSGPQWLHDFVLRDRWVLAVAGTHGKTTTAGMA 121

Orf132: 124 AWVLEYAGLAPGFLIGGVXG 143
             W+LE  G  PGF+IGGV G
o457:   122 TWILEQCGYKPGFVIGGVPG 141
```

<u>Homology with a predicted ORF from *N.meningitidis* (strain A)</u>

ORF132 shows 74.6% identity over a 189aa overlap with an ORF (ORF132a) from strain A of *N. meningitidis:*

```
                      10        20        30        40        50        60
orf132.pep    MKHIHIIGIGGTFMGGLAAIAKEAGFEVSGCDAKMYPPMSTQLEALGIDVYEGFDAAQLD
              |||||||||||||||:|||||||||| ||||||||||||||||||| ||||||:||||
orf132a       MKHIHIIGIGGTFMGGIAAIAKEAGFEXSGCDAKMYPPMSTQLEALGIGVYEGFDTAQLD
                      10        20        30        40        50        60

                      70        80        90       100       110       120
orf132.pep    EFKADVYVIGNVAKRGMDVVEAILNLGLPYISGPQWLSENVLHHHWVLGVAGTHGKTTTA
              |||||||||||||||||||||||||| ||||||||||||:|| ||||| |||| ||||||||
orf132a       EFKADVYVIGNVAKRGMDVVEAILNRGLPYISGPQWLAENXLHHHWXLGVAXTHGKTTTA
                      70        80        90       100       110       120

                     130       140       150       160
orf132.pep    SMLAWVLEYAGLAPGFLIGGVXGKFR---RFRPPAANAAPRPEQPI----------AVFR
              ||||||||||||||| |||| :|    |: | :    |  ::|:           | |
orf132a       SMLAWVLEYAGLAPGFXIGGVPENFSVSARL-PQTPRQDPNSQSPFFVIEADEYDTAFFD
                     130       140       150       160       170

                170       180       190       200       210       220
orf132.pep    HRSRRIRHRLFRQTFXIRALPSAYRRVEQSGIRPRRHLCRLGRDTDPVPLPRAYRAVXRL
              :||: :::|
orf132a       KRSKFVHYRPRTAVLNNLEFDHADIFADLGAIQTQFHHLVRTVPSEGLIVCNGRQQSLQD
            180       190       200       210       220       230
```

The complete length ORF132a nucleotide sequence <SEQ ID 869> is:

```
   1  ATGAAACACA TCCACATTAT CGGTATCGGC GGCACGTTTA TGGGTGGGAT
  51  TGCCGCCATT GCCAAAGAAG CAGGGTTTGA ANTCAGCGGT TGCGATGCGA
 101  AGATGTATCC GCCGATGAGC ACCCAGCTCG AAGCCTTGGG CATAGGCGTG
 151  TATGAAGGCT TCGACACCGC GCAGTTGGAC GAATTTAAAG CCGACGTTTA
 201  CGTTATCGGC AATGTCGCCA AGCGCGGGAT GGATGTGGTT GAAGCGATTT
 251  TGAACCGTGG GCTGCCTTAT ATTTCCGGCC CGCAATGGCT GGCTGAAAAC
 301  NTGCTGCACC ATCATTGGNN ACTCGGCGTG GCGGNGACGC ACGGCAAAAC
 351  GACCACCGCG TCTATGCTCG CGTGGGTTTT GGAATATGCC GGACTCGCAC
 401  CGGGCTTCNT TATCGGCGGC GTACCGGAAA ACTTCAGCGT TTCCGCCCGC
 451  CTGCCGCAAA CGCCGCGCCA AGACCCGAAC AGCCAATCGC CGTTTTTCGT
 501  CATTGAAGCC GACGAATACG ACACCGCGTT TTTCGACAAA CGCTCCAAAT
 551  TCGTGCATTA CCGTCCGCGT ACCGCCGTGT TGAACAATCT GGAATTCGAC
 601  CACGCCGACA TCTTCGCCGA TTTGGGCGCG ATACAGACCC AGTTCCACCA
 651  CCTCGTGCGT ACCGTGCCGT CTGAAGGCCT CATCGTCTGC AACGGACGGC
 701  AGCAAAGCCT GCAAGACACT TTGGACAAAG GCTGCTGGAC GCCGGTGGAA
 751  AAATTCGGCA CGGAACACGG CTGGCAGGCC GGCGAAGCCA ATGCCGATGG
 801  CTCGTTCGAC GTGTTGCTTG ACGGCAAAAA AGCCGGACAC GTCGCTTGGA
 851  GTTTGATGGG CGGACACAAC CGCATGAACG CGCTCGCNGT CATCGCCGCC
 901  GCGCGTCATG CCGGAGTNGA CATTCAGACG GCCTGCGAAG CCTTGAGCAC
 951  GTTTAAAAAC GTCAAACGCC GCATGGAAAT CAAAGGCACG GCAAACGGTA
1001  TCACCGTTTA CGACGACTTC GCCCACCATC CGACCGCTAT CGAAACCACG
1051  ATTCAAGGTT TGCGCCAGCG CGTCGGCGGC GCGCGCATCC TCGCCGTCCT
1101  CGAACCGCGT TCCAATACGA TGAAGCTGGG TACGATGAAA GCCGCCCTGC
1151  CCGCAAGCCT CAAAGAAGCC GACCAAGTGT TCTGNTACGC CGGCGGCGCG
1201  GACTGGGACG TTGCCGAAGC CCTCGCGCCT TTGGGCGGCA GGCTGCACGT
1251  CGGCAAAGAC TTCGATGCCT TCGTTGCCGA AATCGTGAAA AACGCCGAAG
1301  CAGGCGACCA TATTTTGGTG ATGAGCAACG GCGGTTTCGG CGGAATACAC
1351  ACCAAACTGC TGGACGCTTT GAGATAG
```

This encodes a protein having amino acid sequence <SEQ ID 870>:

```
   1  MKHIHIIGIG GTFMGGIAAI AKEAGFEXSG CDAKMYPPMS TQLEALGIGV
  51  YEGFDTAQLD EFKADVYVIG NVAKRGMDVV EAILNRGLPY ISGPQWLAEN
 101  XLHHHWXLGV AXTHGKTTTA SMLAWVLEYA GLAPGFXIGG VPENFSVSAR
 151  LPQTPRQDPN SQSPFFVIEA DEYDTAFFDK RSKFVHYRPR TAVLNNLEFD
 201  HADIFADLGA IQTQFHHLVR TVPSEGLIVC NGRQQSLQDT LDKGCWTPVE
 251  KFGTEHGWQA GEANADGSFD VLLDGKKAGH VAWSLMGGHN RMNALAVIAA
 301  ARHAGVDIQT ACEALSTFKN VKRRMEIKGT ANGITVYDDF AHHPTAIETT
 351  IQGLRQRVGG ARILAVLEPR SNTMKLGTMK AALPASLKEA DQVFXYAGGA
 401  DWDVAEALAP LGGRLHVGKD FDAFVAEIVK NAEAGDHILV MSNGGFGGIH
 451  TKLLDALR*
```

ORF132a and ORF132-1 show 93.9% identity in 458 aa overlap:

```
orf132a.pep  MKHIHIIGIGGTFMGGIAAIAKEAGFEXSGCDAKMYPPMSTQLEALGIGVYEGFDTAQLD
             |||||||||||||||:|||||||||| |||||||||||||||||||| ||||||:||||
orf132-1     MKHIHIIGIGGTFMGGLAAIAKEAGFEVSGCDAKMYPPMSTQLEALGIDVYEGFDAAQLD

orf132a.pep  EFKADVYVIGNVAKRGMDVVEAILNRGLPYISGPQWLAENXLHHHWXLGVAXTHGKTTTA
             |||||||||||||||||||||||| |||||||||||:|| ||||| |||| ||||||||
orf132-1     EFKADVYVIGNVAKRGMDVVEAILNLGLPYISGPQWLSENVLHHHWVLGVAGTHGKTTTA

orf132a.pep  SMLAWVLEYAGLAPGFXIGGVPENFSVSARLPQTPRQDPNSQSPFFVIEADEYDTAFFDK
             |||||||||||||||| ||||||||:|||||||||||||||||||||||||||||||||||
orf132-1     SMLAWVLEYAGLAPGFLIGGVPENFGVSARLPQTPRQDPNSQSPFFVIEADEYDTAFFDK

orf132a.pep  RSKFVHYRPRTAVLNNLEFDHADIFADLGAIQTQFHHLVRTVPSEGLIVCNGRQQSLQDT
             ||||||||||||||||||||||||||||||||||||:|||||||||||||||||||||||
orf132-1     RSKFVHYRPRTAVLNNLEFDHADIFADLGAIQTQFHYLVRTVPSEGLIVCNGRQQSLQDT

orf132a.pep  LDKGCWTPVEKFGTEHGWQAGEANADGSFDVLLDGKKAGHVAWSLMGGHNRMNALAVIAA
             |||||||||||||||||||||||||||||||||||| ||:| |:||| |||||||||||
```

```
orf132-1        LDKGCWTPVEKFGTEHGWQAGEANADGSFDVLLDGKTAGRVKWDLMGRHNRMNALAVIAA

orf132a.pep     ARHAGVDIQTACEALSTFKNVKRRMEIKGTANGITVYDDFAHHPTAIETTIQGLRQRVGG
                |||:|||||||||||::|||||||||||||||||||||||||||||||||||||||||||
orf132-1        ARHVGVDIQTACEALGAFKNVKRRMEIKGTANGITVYDDFAHHPTAIETTIQGLRQRVGG

orf132a.pep     ARILAVLEPRSNTMKLGTMKAALPASLKEADQVFXYAGGADWDVAEALAPLGGRLHVGKD
                ||||||||||||||||||||:|||:||||||||| ||||:||||||||||||||||:|||
orf132-1        ARILAVLEPRSNTMKLGTMKSALPVSLKEADQVFCYAGGVDWDVAEALAPLGGRLNVGKD

orf132a.pep     FDAFVAEIVKNAEAGDHILVMSNGGFGGIHTKLLDALRX
                |||||||||||||:|||||||||||||||| |||:|||
orf132-1        FDAFVAEIVKNAEVGDHILVMSNGGFGGIHGKLLEALRX
```

Homology with a predicted ORF from *N.gonorrhoeae*

ORF132 shows 89.6% identity over 259 aa overlap with a predicted ORF (ORF132ng) from *N. gonorrhoeae:*

```
orf132.pep   MKHIHIIGIGGTFMGGLAAIAKEAGFEVSGCDAKMYPPMSTQLEALGIDVYEGFDAAQLD   60
             ||||||||||||||||:|||||||||:||||||||||||||||||||| |:||||||||:
orf132ng     MKHIHIIGIGGTFMGGIAAIAKEAGFKVSGCDAKMYPPMSTQLEALGIGVHEGFDAAQLE   60

orf132.pep   EFKADVYVIGNVAKRGMDVVEAILNLGLPYISGPQWLSENVLHHHWVLGVAGTHGKTTTA  120
             ||:||:|||||||:||||||||||| ||||||||||||:|||||||||||||||||||||
orf132ng     EFQADIYVIGNVARRGMDVVEAILNRGLPYISGPQWLAENVLHHHWVLGVAGTHGKTTTA  120

orf132.pep   SMLAWVLEYAGLAPGFLIGGVXGKFRRFRPPAANAAPRPEQPIAVFRHRSRRIRHRLFRQ  180
             ||||||||||||||||||||| |||||||||:|||| |||| ||||||||||||||||||
orf132ng     SMLAWVLEYAGLAPGFLIGGVPGKFRRFRPPTANAASRPEQQIAVFRHRSRRIRHRLFRQ  180

orf132.pep   TFXIRALPSAYRRVEQSGIRPRRHLCRLGRDTDPVPLPRAYRAVXRLNRLQRTAAKPARY  240
             |: ||||  |||||||||||||||| |||||||||| |||:|:: | :||||||||||||
orf132ng     TLQIRALSPAYRRVEQSGIRPRRHLRRLGRDTDPVPPPRAHRTIRRPHRLQRTAAKPARY  240

orf132.pep   FGQRLLDAGGKIRHGTRLA    259
             |||||||||||||| ||||
orf132ng     FGQRLLDAGGKIRHRTRLADW  261
```

An ORF132ng nucleotide sequence <SEQ ID 871> was predicted to encode a protein having amino acid sequence <SEQ ID 872>:

```
  1  MKHIHIIGIG GTFMGGIAAI AKEAGFKVSG CDAKMYPPMS TQLEALGIGV
 51  HEGFDAAQLE EFQADIYVIG NVARRGMDVV EAILNRGLPY ISGPQWLAEN
101  VLHHHWVLGV AGTHGKTTTA SMLAWVLEYA GLAPGFLIGG VPGKFRRFRP
151  PTANAASRPE QQIAVFRHRS RRIRHRLFRQ TLQIRALSPA YRRVEQSGIR
201  PRRHLRRLGR DTDPVPPPRA HRTIRRPHRL QRTAAKPARY FGQRLLDAGG
251  KIRHRTRLAD W*
```

Further work revealed the following gonococcal DNA sequence <SEQ ID 873>:

```
   1 ATGAAACACA TCCACATTAT CGGTATCGGC GGCACGTTTA TGGGCGGGAT
  51 TGCCGCCATT GCCAAAGAAG CCGGGTTCAA AGTCAGCGGT TGCGACGCGA
 101 AGATGTATCC GCCGATGAGC ACCCAGCTCG AAGCCTTGGG CATAGGCGTA
 151 CACGAAGGCT TCGATGCCGC GCAGTTGGAA GAATTTCAAG CCGATATTTA
 201 CGTCATCGGC AATGTCGCCA GGCGCGGGAT GGATGTGGTC GAGGCGATTT
 251 TGAACCGTGG GCTGCCTTAT ATTTCCGGCC CGCAATGGCT GGCTGAAAac
 301 GTGCtgcacc atcaTTGGgt ACTCGGCGTG GcagggaCGC ACGGcaaAac
 351 gaccaCcGcg tCCATGCTCG CCTGGGTCTT GGAATATGCC GGACTCGCGC
 401 CGGGCTTCCT CATCGGCGGt gtaccggaAA ATTTCGGCGT TTCCGCCCGC
 451 CTACCGCAAA CGCCGCGTCA AGACCCGAAC AGCAAATCGC CGTTTTTCGT
 501 CATCGAAGCC GACGAATACG ACACCGCCTT TTTCGACAAA CGCTCCAAAT
 551 TCGTGCATTA TCGCCCGCGT ACCGCCGTGT TGAACAATCT GGAATTCGAC
 601 CACGCCGACA TCTTCGCCGA CTTGGGCGCG ATACAGACCC AGTTCCACCA
 651 CCTCGTGCGC ACCGTACCAT CCGAAGGCCT CATCGTCTGC AACGGACAGC
 701 AGCAAAGCCT GCAAGATACT TTGGACAAAG GCTGCTGGAC GCCGGTGGAA
 751 AAATTCGGCA CCGGACACGG CTGGCAGATT GGTGAAGTCA ATGCCGACGG
 801 CTCGTTCGAC GTATTGCTTG ACGGCAAAAA AGCCGGACAC GTCGCATGGG

 851 ATTTGATGGG CGGACACAAC CGCATGAACG CGCTCGCCGT CATCGCTGCC
 901 GCACGCCATG CCGGAGTCGA TGTTCAGACG GCCTGCGAAG CCTTGGGTGC
 951 GTTTAAAAAC GTCAAACGCC GCATGGAAAT CAAAGGCACG GCAAACGGCA
1001 TCACCGTTTA CGACGATTTC GCCCACCACC CGACCGCCAT CGAAACCACG
1051 ATTCAAGGTT TGCGCCAACG TGTCGGCGGC GCGCGCATCC TCGCCGTCCT
1101 CGAGCCGCGT TCCAACACCA TGAAACTCGG CACGATGAAG TCCGCCCTGC
1151 CCGCAAGCCT CAAAGAAGCC GACCAAGTGT TCTGCTACGC CGGCGGCGCG
1201 GACTGGGACG TTGCCGAAGC CCTCGCGCCT TTGGGCTGCA GGCTGCGCGT
1251 CGGTAAAGAT TTCGATACCT TCGTTGCCGA AATTGTGAAA AACGCCCGAA
1301 CCGGCGACCA TATTTTGGTG ATGAGCAACG GCGGTTTCGG CGGAATACAC
1351 ACCAAACTGC TGGACGCTTT GAGATAG
```

This corresponds to the amino acid sequence <SEQ ID 874; ORF132ng-1>:

```
  1 MKHIHIIGIG GTFMGGIAAI AKEAGFKVSG CDAKMYPPMS TQLEALGIGV
 51 HEGFDAAQLE EFQADIYVIG NVARRGMDVV EAILNRGLPY ISGPQWLAEN
101 VLHHHWVLGV AGTHGKTTTA SMLAWVLEYA GLAPGFLIGG VPENFGVSAR
151 LPQTPRQDPN SKSPFFVIEA DEYDTAFFDK RSKFVHYRPR TAVLNNLEFD
201 HADIFADLGA IQTQFHHLVR TVPSEGLIVC NGQQQSLQDT LDKGCWTPVE
251 KFGTGHGWQI GEVNADGSFD VLLDGKKAGH VAWDLMGGHN RMNALAVIAA
301 ARHAGVDVQT ACEALGAFKN VKRRMEIKGT ANGITVYDDF AHHPTAIETT
351 IQGLRQRVGG ARILAVLEPR SNTMKLGTMK SALPASLKEA DQVFCYAGGA
401 DWDVAEALAP LGCRLRVGKD FDTFVAEIVK NARTGDHILV MSNGGFGGIH
451 TKLLDALR*
```

ORF132ng-1 and ORF132-1 show 93.2% identity in 458 aa overlap:

```
orf132ng-1.pep  MKHIHIIGIGGTFMGGIAAIAKEAGFKVSGCDAKMYPPMSTQLEALGIGVHEGFDAAQLE
                ||||||||||||||||:|||||||||:|||||||||||||||||||||| |:|||||||:
orf132-1        MKHIHIIGIGGTFMGGLAAIAKEAGFEVSGCDAKMYPPMSTQLEALGIDVYEGFDAAQLD

orf132ng-1.pep  EFQADIYVIGNVARRGMDVVEAILNRGLPYISGPQWLAENVLHHHWVLGVAGTHGKTTTA
                ||:||:|||||||:|||||||||||| |||||||||||:||||||||||||||||||||||
orf132-1        EFKADVYVIGNVAKRGMDVVEAILNLGLPYISGPQWLSENVLHHHWVLGVAGTHGKTTTA

orf132ng-1.pep  SMLAWVLEYAGLAPGFLIGGVPENFGVSARLPQTPRQDPNSKSPFFVIEADEYDTAFFDK
                |||||||||||||||||||||||||||||||||||||||||:||||||||||||||||||
orf132-1        SMLAWVLEYAGLAPGFLIGGVPENFGVSARLPQTPRQDPNSQSPFFVIEADEYDTAFFDK

orf132ng-1.pep  RSKFVHYRPRTAVLNNLEFDHADIFADLGAIQTQFHHLVRTVPSEGLIVCNGQQQSLQDT
                ||||||||||||||||||||||||||||||||||||:|||||||||||||||:|||||||
orf132-1        RSKFVHYRPRTAVLNNLEFDHADIFADLGAIQTQFHYLVRTVPSEGLIVCNGRQQSLQDT

orf132ng-1.pep  LDKGCWTPVEKFGTGHGWQIGEVNADGSFDVLLDGKKAGHVAWDLMGGHNRMNALAVIAA
                |||||||||||||| |||| ||:||||||||||||| ||:| ||||| ||||||||||||
orf132-1        LDKGCWTPVEKFGTEHGWQAGEANADGSFDVLLDGKTAGRVKWDLMGRHNRMNALAVIAA

orf132ng-1.pep  ARHAGVDVQTACEALGAFKNVKRRMEIKGTANGITVYDDFAHHPTAIETTIQGLRQRVGG
                |||:|||:||||||||||||||||||||||||||||||||||||||||||||||||||||
orf132-1        ARHVGVDIQTACEALGAFKNVKRRMEIKGTANGITVYDDFAHHPTAIETTIQGLRQRVGG

orf132ng-1.pep  ARILAVLEPRSNTMKLGTMKSALPASLKEADQVFCYAGGADWDVAEALAPLGCRLRVGKD
                ||||||||||||||||||||||||:||||||||||||||:|||||||||||| || ||||
orf132-1        ARILAVLEPRSNTMKLGTMKSALPVSLKEADQVFCYAGGVDWDVAEALAPLGGRLNVGKD

orf132ng-1.pep  FDTFVAEIVKNARTGDHILVMSNGGFGGIHTKLLDALRX
                ||:|||||||||::|||||||||||||||| |||:||||
orf132-1        FDAFVAEIVKNAEVGDHILVMSNGGFGGIHGKLLEALRX
```

In addition, ORF132ng-1 is homologous to a hypothetical *E.coli* protein:

```
pir||S56459 hypothetical protein o457 - Escherichia coli >gi|537075 (U14003)
ORF_o457 [Escherichia coli] >gi|1790680 (AE000494) hypothetical 48.5 kD protein
in fbp-pmba intergenic region [Escherichia coli] Length = 457
 Score =  474 bits (1207), Expect = e-133
 Identities = 249/439 (56%), Positives = 294/439 (66%), Gaps = 13/439 (2%)

Query: 22  KEAGFKVSGCDAKMYPPMSTQLEALGIGVHEGFDAAQLEEFQADIYVIGNVARRGMDVVE 81
            ++ G +V+G DA +YPPMST LE  GI + +G+DA+QLE  Q D+ +IGN   RG   VE
Sbjct: 21  RQLGHEVTGSDANVYPPMSTLLEKQGIELIQGYDASQLEP-QPDLVIIGNAMTRGNPCVE 79
```

```
Query: 82  AILNRGLPYISGPQWLAENVLHHHWVLGVAGTHGKTTTASMLAWVLEYAGLAPGFLIGGV 141
           A+L + +PY+SGPQWL + VL   WVL VAGTHGKTTTA M  W+LE  G  PGF+IGGV
Sbjct: 80  AVLEKNIPYMSGPQWLHDFVLRDRWVLAVAGTHGKTTTAGMATWILEQCGYKPGFVIGGV 139

Query: 142 PENFGVSARLPQTPRQDPNSKSPFFVIEADEYDTAFFDKRSKFVHYRPRTAVLNNLEFDH 201
           P NF VSA L          +S FFVIEADEYD AFFDKRSKFVHY PRT +LNNLEFDH
Sbjct: 140 PGNFEVSAHL---------GESDFFVIEADEYDCAFFDKRSKFVHYCPRTLILNNLEFDH 190

Query: 202 ADIFADLGAIQTQFHHLVRTVPSEGLIVCNGQQQSLQDTLDKGCWTPVEKFGTGHGWQIG 261
           ADIF DL AIQ QFHHLVR VP +G I+      +L+ T+  GCW+  E  G    WQ
Sbjct: 191 ADIFDDLKAIQKQFHHLVRIVPGQGRIIWPENDINLKQTMAMGCWSEQELVGEQGHWQAK 250

Query: 262 EVNADGS-FDVLLDGKKAGHVAWDLMGGHNRMNALAVIAAARHAGVDVQTACEALGAFKN 320
           ++  D S ++VLLDG+K G V W L+G HN  N L  IAAARH GV    A  ALG+F N
Sbjct: 251 KLTTDASEWEVLLDGEKVGEVKWSLVGEHNMHNGLMAIAAARHVGVAPADAANALGSFIN 310

Query: 321 VKRRMEIKGTANGITVYDDFAHHPTAIETTIQGLRQRVGG-ARILAVLEPRSNTMKLGTM 379
            +RR+E++G ANG+TVYDDFAHHPTAI  T+  LR +VGG ARI+AVLEPRSNTMK+G
Sbjct: 311 ARRRLELRGEANGVTVYDDFAHHPTAILATLAALRGKVGGTARIIAVLEPRSNTMKMGIC 370

Query: 380 KSALPASLKEADQVF-CYAGGADWDVAEALAPLGCRLRVGKDFDTFVAEIVKNARTGDHI 438
           K  L  SL  AD+VF         W VAE              D DT    +VK A+ GDHI
Sbjct: 371 KDDLAPSLGRADEVFLLQPAHIPWQVAEVAEACVQPAHWSGDVDTLADMVVKTAQPGDHI 430

Query: 439 LVMSNGGFGGIHTKLLDAL 457
           LVMSNGGFGGIH KLLD L
Sbjct: 431 LVMSNGGFGGIHQKLLDGL 449
```

Based on this analysis, it was predicted that these proteins from *N.meningitidis* and *N.gonorrhoeae,* and their epitopes, could be useful antigens for vaccines or diagnostics, or for raising antibodies. ORF132-1 (26.4kDa) was cloned in pET and pGex vectors and expressed in *E. coli,* as described above. The products of protein expression and purification were analyzed by SDS-PAGE. Figure 20A shows the results of affinity purification of the His-fusion protein, and Figure 20B shows the results of expression of the GST-fusion in *E.coli.* Purified His-fusion protein was used to immunise mice, whose sera were used for FACS analysis (Figure 20C) and ELISA (positive result). These experiments confirm that ORF132 is a surface-exposed protein, and that it is a useful immunogen.

**Example 103**

The following partial DNA sequence was identified in *N.meningitidis* <SEQ ID 875>

```
  1 ..CCGGGCTATT ACGGCTCGGA TGACGAATTT AAGCGGGCAT TCGGAGAAAA
 51   CTCGCCGACA TmCAAGAAAC ATTGCAACCG GAGCTGCGGG ATTTATGAAC
101   CCGTATTGAA AAAATACGGC AAAAAGCGCG CCAACAACCA TTCGGTCAGC
151   ATTAGTGCGG ACTTCGGCGA TTATTTCATG CCGTTCGCCA GCTATTCGCG
201   CACACACCGT ATGCCCAACA TCCAAGAAAT GTATTTTTCC CAAATCGGCG
251   ACTCCGGCGT TCACACCGCC TTAAAACCAG AGCGCGCAAA CACTTGGCAA
301   TTTGGCTTCr ATACCTATAA AAAAGGATTG TTAAAACAAG ATGATACATT
351   AGGATTAAAA CTGGTCGGCT ACCGCAGCCG CATCGACAAC TACATCCACA
401   ACGTTTACGG GAAATGGTGG GATTTGAACG GGGATATTCC GAGCTGGGTC
451   AGCAGCACCG GGCTTGCCTA CACCATCCAA CATCGCrATT TCAwAGACAA
501   AGTGCATCAA nnnnnnnnnn nnnnnnnnnn nnnnTACGAT TATGGGCGTT
551   TTTTCACCAA CCTTTCTTAC GCCTATCAAA AAAGCACGCA ACCGACCAAC
601   TTCAGCGATG CGAGCGAATC GCCCAACAAT GCGTCCAAAG AAGACCAACT
651   CAAACAAGGT TATGGGTTGA GCAGGGTTTC CGCCCTGCCG CGAGATTACG
701   GACGTTTGGA AGTCGGTACG CGCTGGTTGG GCAACAAACT GACTTTGGGC
751   GGCGCGATGC GCTATTTCGG CAAGAGCATC CGCGCGACGG CTGAAGAACG
801   CTATATCGAC GGCACCAACG GGGGAAATAC CAGCAATTTC CGGCAACTGG
851   GCAAGCGTTC CATCAAACAA ACCGAAACTC TTGCCCGCCA GCCTTTGATT
901   TTwGATTTTa ACGCCGCTTA CGAGCCGAAG AAAAACCTTA TTTTCCGCGC
```

```
 951    CGAAGTCAAA AATCTGTTCG ACAGGCGTTA TATCGATCCG CTCGATGCGG
1001    GCAATGATGC GGCAAC.GAG CGTTATTACA GCTCGTTCGA CCCGAAAGAC
1051    AAGGACrrAG ACGTAACGTG TAATGCTGAT AAAACGTTGT GCaACGGCAA
1101    ATACGGCGGC ACAAGCAAAA GCGTATTGAC CAATTTTGCA CGCGGACGCA
1151    CCTTTTTgAT GACGATGAGC TACAAGTTTT AA
```

This corresponds to the amino acid sequence <SEQ ID 876; ORFI33>:

```
  1  ..PGYYGSDDEF KRAFGENSPT XKKHCNRSCG IYEPVLKKYG KKRANNHSVS
 51    ISADFGDYFM PFASYSRTHR MPNIQEMYFS QIGDSGVHTA LKPERANTWQ
101    FGFXTYKKGL LKQDDTLGLK LVGYRSRIDN YIHNVYGKWW DLNGDIPSWV
151    SSTGLAYTIQ HRXFXDKVHQ XXXXXXXXYD YGRFFTNLSY AYQKSTQPTN
201    FSDASESPNN ASKEDQLKQG YGLSRVSALP RDYGRLEVGT RWLGNKLTLG
251    GAMRYFGKSI RATAEERYID GTNGGNTSNF RQLGKRSIKQ TETLARQPLI
301    XDFNAAYEPK KNLIFRAEVK NLFDRRYIDP LDAGNDAAXE RYYSSFDPKD
351    KDXDVTCNAD KTLCNGKYGG TSKSVLTNFA RGRTFLMTMS YKF*
```

Further work revealed the further partial DNA sequence <SEQ ID 877>:

```
   1 GAGGCGCAGA TACAGGTTTT GGAAGATGTG CACGTCAAGG CGAAGCGCGT
  51 ACCGAAAGAC AAAAAAGTGT TTACCGATGC GCGTGCCGTA TCGACCCGTC
 101 AGGATATATT CAAATCCAGC GAAAACCTCG ACAACATCGT ACGCAGCATC
 151 CCCGGTGCGT TTACACAGCA AGATAAAAGC TCGGGCATTG TGTCTTTGAA
 201 TATTCGCGGC GACAGCGGGT TCGGGCGGGT CAATACGATG GTGGACGGCA
 251 TCACGCAGAC CTTTTATTCG ACTTCTACCG ATGCGGGCAG GGCAGGCGGT
 301 TCATCTCAAT TCGGTGCATC TGTCGACAGC AATTTTATTG CCGGACTGGA
 351 TGTCGTCAAA GGCAGCTTCA GCGGCTCGGC AGGCATCAAC AGCCTTGCCG
 401 GTTCGGCGAA TCTGCGGACT TTAGGCGTGG ATGACGTCGT TCAGGGCAAT
 451 AATACCTACG GCCTGCTGCT AAAAGGTCTG ACCGGCACCA ATTCAACCAA
 501 AGGTAATGCG ATGGCGGCGA TAGGTGCGCG CAAATGGCTG GAAAGCGGAG
 551 CATCTGTCGG TGTGCTTTAC GGGCACAGCA GGCGCAGCGT GGCGCAAAAT
 601 TACCGCGTGG GCGGCGGCGG GCAGCACATC GGAAATTTTG GCGCGGAATA
 651 TTTGGAACGG CGCAAGCAGC GATATTTTGT ACAAGAGGGT GCTTTGAAAT
 701 TCAATTCCGA CAGCGGAAAA TGGGAGCGGG ATTTACAAAG GCAACAGTGG
 751 AAATACAAGC CGTATAAAAA TTACAACAAC CAAGAACTAC AaAAATACAT
 801 CGAAGAGCAT GACAAAAGCT GGCGGGAAAA CCTg.CaCCG CAATACGACA
 851 TTACCCCCAT CGATCCGTCC AGCCTGAAGC AGCAGTCGGC AGGCAATCTG
 901 TTTAAATTGG AATACGACGG CGTATTCAAT AAATACACGG CGCAATTTCG
 951 CGATTTAAAC ACCAAAATCG GCAGCCGCAA AATCATCAAC CGCAATTATC
1001 AGTTCAATTA CGGTTTGTCT TTGAACCCGT ATACCAACCT CAATCTGACC
1051 GCAGCCTACA ATTCGGGCAG GCAGAAATAT CCGAAAGGGT CGAAGTTTAC
1101 AGGCTGGGGG CTTTTAAAGG ATTTTGAAAC CTACAACAAC GCGAAAATCC
1151 TCGACCTCAA CAACACCGCC ACCTTCCGGC TGCCCCGCGA AACCGAGTTG
1201 CAAACCACTT TGGGCTTCAA TTATTTCCAC AACGAATACG GCAAAAACCG
1251 CTTTCCTGAA GAATTGGGGC TGTTTTTCGA CGGTCCTGAT CAGGACAACG
1301 GGCTTTATTC CTATTTGGGG CGGTTTAAGG GCGATAAAGG GCTGCTGCCC
1351 CAAAAATCAA CCATTGTCCA ACCGGCCGGC AGCCAATATT TCAACACGTT
1401 CTACTTCGAT GCCGCGCTCA AAAAAGACAT TTACCGCTTA AACTACAGCA
1451 CCAATACCGT CGGCTACCGT TTCGGCGGCG AATATACGGG CTATTACGGC
1501 TCGGATGACG AATTTAAGCG GGCATTCGGA GAAAACTCGC CGACATACAA
1551 GAAACATTGC AACCGGAGCT GCGGGATTTA TGAACCCGTA TTGAAAAAAT
1601 ACGGCAAAAA GCGCGCCAAC AACCATTCGG TCAGCATTAG TGCGGACTTC
1651 GGCGATTATT TCATGCCGTT CGCCAGCTAT TCGCGCACAC ACCGTATGCC
1701 CAACATCCAA GAAATGTATT TTTCCCAAAT CGGCGACTCC GGCGTTCACA
1751 CCGCCTTAAA ACCAGAGCGC GCAAACACTT GGCAATTTGG CTTCAATACC
1801 TATAAAAAAG GATTGTTAAA ACAAGATGAT ACATTAGGAT TAAAACTGGT
1851 CGGCTACCGC AGCCGCATCG ACAACTACAT CCACAACGTT TACGGGAAAT
1901 GGTGGGATTT GAACGGGGAT ATTCCGAGCT GGGTCAGCAG CACCGGGCTT
1951 GCCTACACCA TCCAACATCG CAATTTCAAA GACAAAGTGC ACAAACACGG
2001 TTTTGAGTTG GAGCTGAATT ACGATTATGG GCGTTTTTTC ACCAACCTTT
2051 CTTACGCCTA TCAAAAAAGC ACGCAACCGA CCAACTTCAG CGATGCGAGC
2101 GAATCGCCCA ACAATGCGTC CAAAGAAGAC CAACTCAAAC AAGGTTATGG
2151 GTTGAGCAGG GTTTCCGCCC TGCCGCGAGA TTACGGACGT TTGGAAGTCG
2201 GTACGCGCTG GTTGGGCAAC AAACTGACTT TGGGCGGCGC GATGCGCTAT
2251 TTCGGCAAGA GCATCCGCGC GACGGCTGAA GAACGCTATA TCGACGGCAC
2301 CAACGGGGGA AATACCAGCA ATTTCCGGCA ACTGGGCAAG CGTTCCATCA
2351 AACAAACCGA AACTCTTGCC CGCCAGCCTT TGATTTTTGA TTTTTACGCC
2401 GCTTACGAGC CGAAGAAAAA CCTTATTTTC CGCGCCGAAG TCAAAAATCT
2451 GTTCGACAGG CGTTATATCG ATCCGCTCGA TGCGGGCAAT GATGCGGCAA
2501 CGCAGCGTTA TTACAGCTCG TTCGACCCGA AAGACAAGGA CGAAGACGTA
2551 ACGTGTAATG CTGATAAAAC GTTGTGCAAC GGCAAATACG GCGGCACAAG
2601 CAAAAGCGTA TTGACCAATT TTGCACGCGG ACGCACCTTT TTGATGACGA
2651 TGAGCTACAA GTTTTAA
```

This corresponds to the amino acid sequence <SEQ ID 878; ORF133-1>:

```
  1 EAQIQVLEDV HVKAKRVPKD KKVFTDARAV STRQDIFKSS ENLDNIVRSI
 51 PGAFTQQDKS SGIVSLNIRG DSGFGRVNTM VDGITQTFYS TSTDAGRAGG
101 SSQFGASVDS NFIAGLDVVK GSFSGSAGIN SLAGSANLRT LGVDDVVQGN
151 NTYGLLLKGL TGTNSTKGNA MAAIGARKWL ESGASVGVLY GHSRRSVAQN
201 YRVGGGGQHI GNFGAEYLER RKQRYFVQEG ALKFNSDSGK WERDLQRQQW
251 KYKPYKNYNN QELQKYIEEH DKSWRENLXP QYDITPIDPS SLKQQSAGNL
301 FKLEYDGVFN KYTAQFRDLN TKIGSRKIIN RNYQFNYGLS LNPYTNLNLT
351 AAYNSGRQKY PKGSKFTGWG LLKDFETYNN AKILDLNNTA TFRLPRETEL
401 QTTLGFNYFH NEYGKNRFPE ELGLFFDGPD QDNGLYSYLG RFKGDKGLLP
451 QKSTIVQPAG SQYFNTFYFD AALKKDIYRL NYSTNTVGYR FGGEYTGYYG
501 SDDEFKRAFG ENSPTYKKHC NRSCGIYEPV LKKYGKKRAN NHSVSISADF
551 GDYFMPFASY SRTHRMPNIQ EMYFSQIGDS GVHTALKPER ANTWQFGFNT
601 YKKGLLKQDD TLGLKLVGYR SRIDNYIHNV YGKWWDLNGD IPSWVSSTGL
651 AYTIQHRNFK DKVHKHGFEL ELNYDYGRFF TNLSYAYQKS TQPTNFSDAS
701 ESPNNASKED QLKQGYGLSR VSALPRDYGR LEVGTRWLGN KLTLGGAMRY
751 FGKSIRATAE ERYIDGTNGG NTSNFRQLGK RSIKQTETLA RQPLIFDFYA
801 AYEPKKNLIF RAEVKNLFDR RYIDPLDAGN DAATQRYYSS FDPKDKDEDV
851 TCNADKTLCN GKYGGTSKSV LTNFARGRTF LMTMSYKF*
```

Computer analysis of this amino acid sequence gave the following results:

Homology with with the probable TonB-dependent receptor HI121 of *H.influenzae* (accession number U32801)

ORF133 and HI121 show 57% aa identity in 363aa overlap:

```
Orf133: 31  IYEPVLKKYGKKRANNHSVSISADFGDYFMPFASYSRTHRMPNIQEMYFSQIGDSGVHTA 90
            I EP+L K G K+A NHS ++SA+  DYFMPF +YSRTHRMPNIQEM+FSQ+ ++GV+TA
HI121:  563 INEPILHKSGHKKAFNHSATLSAELSDYFMPFFTYSRTHRMPNIQEMFFSQVSNAGVNTA 622

Orf133: 91  LKPERANTWQFGFXTYKKGLLKQDDTLGLKLVGYRSRIDNYIHNVYGKWWDLNGDIPSWV 150
            LKPE+++T+Q GF TYKKGL  QDD LG+KLVGYRS I NYIHNVYG WW     +P+W
HI121:  623 LKPEQSDTYQLGFNTYKKGLFTQDDVLGVKLVGYRSFIKNYIHNVYGVWW--RDGMPTWA 680

Orf133: 151 SSTGLAYTIQHRXFXDKVHXXXXXXXXXYDYGRFFTNLSYAYQKSTQPTNFSDASESPNN 210
             S G  YTI H+ +   V          YD GRFF N+SYAYQ++ QPTN++DAS  PNN
HI121:  681 ESNGFKYTIAHQNYKPIVKKSGVELEINYDMGRFFANVSYAYQRTNQPTNYADASPRPNN 740

Orf133: 211 ASKEDQLKQGYGLSRVSALPRDYGRLEVGTRWLGNKLTLGGAMRYFGKSIRATAEERYID 270
            AS+ED LKQGYGLSRVS LP+DYGRLE+GTRW   KLTLG A RY+GKS RAT EE YI+
HI121:  741 ASQEDILKQGYGLSRVSMLPKDYGRLELGTRWFDQKLTLGLAARYYGKSKRATIEEEYIN 800

Orf133: 271 GTNGGNTSNFRQLGKRSIKQTETLARQPLIXDFNAAYEPKKNLIFRAEVKNLFDRRYIDP 330
            G+     +  R+    ++K+TE + +QP+I D + +YEP K+LI +AEV+NL D+RY+DP
HI121:  801 GSR-FKKNTLRRENYYAVKKTEDIKKQPIILDLHVSYEPIKDLIIKAEVQNLLDKRYVDP 859

Orf133: 331 LDAGNDAAXERYYSSFDPKDKDXDVTCNADKTLCNGKYGGTSKSVLTNFARGRTFLMTMS 390
            LDAGNDAA +RYYSS      +  + C  D + C    GG+ K+VL NFARGRT++++++
HI121:  860 LDAGNDAASQRYYSSL-----NNSIECAQDSSAC----GGSDKTVLYNFARGRTYILSLN 910

Orf133: 391 YKF 393
            YKF
HI121:  911 YKF 913
```

Homology with a predicted ORF from *N. meningitidis* (strain A)

ORF133 shows 90.8% identity over a 392aa overlap with an ORF (ORF133a) from strain A of *N. meningitidis:*

```
                                                                10        20        30
orf133.pep                                               PGYYGSDDEFKRAFGENSPTXKKHCNRSCGI
                                                          ||| |||||||||||||||  ||||:||||
orf133a          FYFDAALKKDIYRLNYSTNTVGYRFGGXYTGYYXSDDEFKRAFGENSPTYXKHCNQSCGI
                450       460       470       480       490       500

                  40        50        60        70        80        90
orf133.pep YEPVLKKYGKKRANNHSVSISADFGDYFMPFASYSRTHRMPNIQEMYFSQIGDSGVHTAL
           ||||||||||||||||||||||||||||||||||||||||||||||||||||||||||||
orf133a    YEPVLKKYGKKRANNHSVSISADFGDYFMPFASYSRTHRMPNIQEMYFSQIGDSGVHTAL
         510       520       530       540       550       560

                 100       110       120       130       140       150
orf133.pep KPERANTWQFGFXTYKKGLLKQDDTLGLKLVGYRSRIDNYIHNVYGKWWDLNGDIPSWVS
           |||||||||||| |||||||||||| ||||||||||||| |||||||||||||||:|||||
orf133a    KPERANTWQFGFNTYKKGLLKQDDILGLKLVGYRSRIDXYIHNVYGKWWDLNGNIPSWVS
         570       580       590       600       610       620

                 160       170       180       190       200       210
orf133.pep STGLAYTIQHRXFXDKVHQXXXXXXXXXYDYGRFFTNLSYAYQKSTQPTNFSDASESPNNA
           ||||||||||| | ||||:          ||| ||||||||||||||||||||||||||||
orf133a    STGLAYTIQHRNFKDKVHKHGFELELNYDYXRFFTNLSYAYQKSTQPTNFSDASESPNNA
         630       640       650       660       670       680

                 220       230       240       250       260       270
orf133.pep SKEDQLKQGYGLSRVSALPRDYGRLEVGTRWLGNKLTLGGAMRYFGKSIRATAEERYIDG
           |||||||||||||||||||||||||||||||||||||||||||||||||||||||||||
orf133a    SKEDQLKQGYGLSRVSALPRDYGRLEVGTRWLGNKLTLGGAMRYFGKSIRATAEERYIDX
         690       700       710       720       730       740

                 280       290       300       310       320       330
orf133.pep TNGGNTSNFRQLGKRSIKQTETLARQPLIXDFNAAYEPKKNLIFRAEVKNLFDRRYIDPL
           |||  |||||||||||| ||||||||||| |  ||||||| ||||||||||||||||||||
orf133a    TNGXXTSNFRQLGKRSIXQTETLARQPLIFDXYAAYEPKKXLIFRAEVKNLFDRRYIDPL
         750       760       770       780       790       800

                 340       350       360       370       380       390
orf133.pep DAGNDAAXERYYSSFDPKDKDXDVTCNADKTLCNGKYGGTSKSVLTNFARGRTFLMTMSY
           |||||||::|||||||||||||  :|||| |:|||||||||||||||||||| |||:||||
orf133a    DAGNDAATQRYYSSFDPKDKDEEVTCNDDNTLCNGKYGGTSKSVLTNFARGXTFLITMSY
         810       820       830       840       850       860

orf133.pep KFX
           |||
orf133a    KFX
         870
```

A partial ORF133a nucleotide sequence <SEQ ID 879> is:

```
   1  AAAGACAAAA AAGTGTTTAC CGATGCGCGT GCCGTATCGA CCCGTCAGGA
  51  TATATTCAAA TCCANCGAAA ACCTCGACAA CATCGTACGC ANCATCCCCG
 101  GTGCGTTTAC ACANCAANAT AAAAGCTCGG GCNTTGTGTC TTTGAATATT
 151  CGCNGCGACA GCGGGTTCGG GCGGGTCAAT ACNATGGTNG ACGGCATCAC
 201  NCANACCTTT TATTCGACTT CTACCGATGC GGGCAGGGCA GGCGGTTCAT
 251  CTCAATTCGG TGCATCTGTC GACAGCAATT TTATNGCCGG ACTGGATGTC
 301  GTCAAAGGCA GCTTCAGCGG CTCGGCAGGC ATCAACAGCC TTGCCGGTTC
 351  GGCGAATCTG CGGACTTTAN GCGTGGATGA TGTCGTTCAG GGCAATANTA
 401  CNTACGGCCT GCTGCTAAAA GGTCTGACCG GCACCAATTC AACCAAAGGT
 451  AATGCGATGG CGGCGATAGG TGCGCGCAAA TGGCTGGAAA GCGGAGCATC
 501  TGTCGGTGTG CTTTACGGGC ACAGCAGGCG CAGCGTGGCG CAAAATTACC
 551  GCGTGGGCGG CGGCGGGCAG CACATCGGAA ATTTTGGCGC GGAATATCTG
 601  GAACGACGCA AGCAACGATA TTTTGAGCAA GAAGGCGGGT TGAAATTCAA
 651  TTCCAACAGC GGAAAATGGG AGCGGGATTT CCAAAAGTCG TACTGGAAAA
 701  CCAAGTGGTA TCAAAAATAC GATGCCCCCC AAGAACTGCA AAAATACATC
 751  GAAGGTCATG ATAAAAGCTG GCGGGAAAAC CTGGCGCCGC AATACGACAT
 801  CACCCCCATC GATCCGTCCA GCCTGAAGCN GCAGTCGGCA GGCAACCTGT
 851  TTAAATTGGA ATACGACGGC GTATTCAATA AATACACGGC GCAATTTCGC
 901  GATTTAAACA CCAAAATCGG CAGCCGCAAA ATCATCAACC GCAATTATCA
 951  ATTCAATTAC GGTTTGTCTT TGAACCCGTA TACCAACCTC AATCTGACCG
1001  CAGCCTACAA TTCGGGCAGG CAGAAATATC CGAAAGGGTC GAAGTTTACA
1051  GGCTGGGGGC TTTTNAAAGA TTTTGAAACC TACAACAACG CAAAAATCCT
1101  CGACCTCANC AACACCTCCA CCTTCCGGCT GCCCCGTGAA ACCGAGTTGC
1151  AAACCACTTT GGGCTTCAAT TATTTCCACA ACGAATACGG CAAAAACCGC
1201  TTTCCTGAAG AATTGGGGCT GTTTTTCGAC GGTCCGGATC ANGACAACGG
1251  GCTTTATTCC TATTTGGGGC GGTTTAAGGG CGATAAAGGG CTGCTGCCCC
1301  AAAAATCAAC CATTGTCCAA CCGGCCGGCA GCCAATATTT CAACACGTTC
1351  TACTTCGATG CCGCGCTCAA AAAAGACATT TACCGCTTAA ACTACAGCAC
```

```
1401  CAATACCGTC GGCTACCGTT TCGGCGGCNA ATATACGGGC TATTACNGCT
1451  CGGATGACGA ATTTAAGCGG GCATTCGGAG AAAACTCGCC GACATACANG
1501  AAACATTGCA ACCAGAGCTG CGGAATTTAT GAACCCGTAT TGAAAAAATA
1551  CGGCAAAAAG CGCGCCAACA ACCATTCGGT CAGCATTAGT GCGGACTTCG
1601  GCGATTATTT CATGCCGTTC GCCAGCTATT CGCGCACACA CCGTATGCCC
1651  AACATCCAAG AAATGTATTT TTCCCAAATC GGCGACTCCG GCGTTCACAC
1701  CGCCTTAAAA CCAGAGCGCG CAAACACTTG GCAATTTGGC TTCAATACCT
1751  ATAAAAAAGG ATTGTTAAAA CAAGATGATA TATTAGGATT AAAACTGGTC
1801  GGCTACCGCA GCCGCATCGA CNACTACATC CACAACGTTT ACGGGAAATG
1851  GTGGGATTTG AACGGGAATA TTCCGAGCTG GGTCAGCAGC ACCGGGCTTG
1901  CCTACACCAT CCAACACCGC AATTTCAAAG ACAAAGTGCA CAAACACGGT
1951  TTTGAGTTGG AGCTGAATTA CGATTATNGG CGTTTTTTCA CCAACCTTTC
2001  TTACGCCTAT CAAAAAAGCA CGCAACCGAC CAACTTCAGC GATGCGAGCG
2051  AATCGCCCAA CAATGCGTCC AAAGAAGACC AACTCAAACA AGGTTATGGG
2101  TTGAGCAGGG TTTCCGCCCT GCCGCGAGAT TACGGACGTT TGGAAGTCGG
2151  TACGCGCTGG TTGGGCAACA AACTGACTTT GGGCGGCGCG ATGCGCTATT
2201  TCGGCAAGAG CATCCGCGCG ACGGCTGAAG AACGCTATAT CGACGNCACC
2251  AATGGGGNAN NTACCAGCAA TTTCCGGCAA CTGGGCAAGC GTTCCATCAN
2301  ACAAACCGAA ACCCTTGCCC GCCAGCCTTT GATTTTTGAT TTNTACGCCG
2351  CTTACGAGCC GAAGAAAAAN CTTATTTTCC GCGCCGAAGT CAAAAATCTG
2401  TTCGACAGGC GTTATATCGA TCCGCTCGAT GCGGGCAATG ATGCGGCAAC
2451  GCAGCGTTAT TACAGTTCGT TCGACCCGAA AGACAAGGAC GAAGAAGTAA
2501  CGTGTAATGA TGATAACACG TTATGCAACG GCAAATACGG CGGCACAAGC
2551  AAAAGCGTAT TGACCAATTT TGCACGCGGA CNCACCTTTT TGATAACGAT
2601  GAGCTACAAG TTTTAA
```

This encodes a protein having (partial) amino acid sequence <SEQ ID 880>:

```
  1 KDKKVFTDAR AVSTRQDIFK SXENLDNIVR XIPGAFTXQX KSSGXVSLNI
 51 RXDSGFGRVN TMVDGITXTF YSTSTDAGRA GGSSQFGASV DSNFXAGLDV
101 VKGSFSGSAG INSLAGSANL RTLXVDDVVQ GNXTYGLLLK GLTGTNSTKG
151 NAMAAIGARK WLESGASVGV LYGHSRRSVA QNYRVGGGGQ HIGNFGAEYL
201 ERRKQRYFEQ EGGLKFNSNS GKWERDFQKS YWKTKWYQKY DAPQELQKYI
251 EGHDKSWREN LAPQYDITPI DPSSLKXQSA GNLFKLEYDG VFNKYTAQFR
301 DLNTKIGSRK IINRNYQFNY GLSLNPYTNL NLTAAYNSGR QKYPKGSKFT
351 GWGLXKDFET YNNAKILDLX NTSTFRLPRE TELQTTLGFN YFHNEYGKNR
401 FPEELGLFFD GPDXDNGLYS YLGRFKGDKG LLPQKSTIVQ PAGSQYFNTF
451 YFDAALKKDI YRLNYSTNTV GYRFGGXYTG YYXSDDEFKR AFGENSPTYX
501 KHCNQSCGIY EPVLKKYGKK RANNHSVSIS ADFGDYFMPF ASYSRTHRMP
551 NIQEMYFSQI GDSGVHTALK PERANTWQFG FNTYKKGLLK QDDILGLKLV
601 GYRSRIDXYI HNVYGKWWDL NGNIPSWVSS TGLAYTIQHR NFKDKVHKHG
651 FELELNYDYX RFFTNLSYAY QKSTQPTNFS DASESPNNAS KEDQLKQGYG
701 LSRVSALPRD YGRLEVGTRW LGNKLTLGGA MRYFGKSIRA TAEERYIDXT
751 NGXXTSNFRQ LGKRSIXQTE TLARQPLIFD XYAAYEPKKX LIFRAEVKNL
801 FDRRYIDPLD AGNDAATQRY YSSFDPKDKD EEVTCNDDNT LCNGKYGGTS
851 KSVLTNFARG XTFLITMSYK F*
```

ORF133a and ORF133-1 show 94.3% identity in 871 aa overlap:

```
                                         10        20        30        40
orf133a.pep                      KDKKVFTDARAVSTRQDIFKSXENLDNIVRXIPGAFTXQXKS
                                 ||||||||||||||||||||| |||||||| |||||| | ||
orf133-1       EAQIQVLEDVHVKAKRVPKDKKVFTDARAVSTRQDIFKSSENLDNIVRSIPGAFTQQDKS
                       10        20        30        40        50        60

                     50        60        70        80        90       100
orf133a.pep    SGXVSLNIRXDSGFGRVNTMVDGITXTFYSTSTDAGRAGGSSQFGASVDSNFXAGLDVVK
               || |||||| ||||||||||||||| |||||||||||||||||||||||||| |||||||
orf133-1       SGIVSLNIRGDSGFGRVNTMVDGITQTFYSTSTDAGRAGGSSQFGASVDSNFIAGLDVVK
                       70        80        90       100       110       120

                    110       120       130       140       150       160
orf133a.pep    GSFSGSAGINSLAGSANLRTLXVDDVVQGNXTYGLLLKGLTGTNSTKGNAMAAIGARKWL
               ||||||||||||||||||||| |||||||| |||||||||||||||||||||||||||||
orf133-1       GSFSGSAGINSLAGSANLRTLGVDDVVQGNNTYGLLLKGLTGTNSTKGNAMAAIGARKWL
                      130       140       150       160       170       180

                    170       180       190       200       210       220
orf133a.pep    ESGASVGVLYGHSRRSVAQNYRVGGGGQHIGNFGAEYLERRKQRYFEQEGGLKFNSNSGK
               |||||||||||||||||||||||||||||||||||||||||||||| |||:|||||:|||
orf133-1       ESGASVGVLYGHSRRSVAQNYRVGGGGQHIGNFGAEYLERRKQRYFVQEGALKFNSDSGK
                      190       200       210       220       230       240
```

```
                       230       240       250       260       270       280
orf133a.pep     WERDFQKSYWKTKWYQKYDAPQELQKYIEGHDKSWRENLAPQYDITPIDPSSLKXQSAGN
                ||||:|:: || | |::|:  |||||||| ||||||||| |||||||||||||| |||||
orf133-1        WERDLQRQQWKYKPYKNYNN-QELQKYIEEHDKSWRENLXPQYDITPIDPSSLKQQSAGN
                         250       260       270       280       290

                       290       300       310       320       330       340
orf133a.pep     LFKLEYDGVFNKYTAQFRDLNTKIGSRKIINRNYQFNYGLSLNPYTNLNLTAAYNSGRQK
                ||||||||||||||||||||||||||||||||||||||||||||||||||||||||||||
orf133-1        LFKLEYDGVFNKYTAQFRDLNTKIGSRKIINRNYQFNYGLSLNPYTNLNLTAAYNSGRQK
              300       310       320       330       340       350

                       350       360       370       380       390       400
orf133a.pep     YPKGSKFTGWGLXKDFETYNNAKILDLXNTSTFRLPRETELQTTLGFNYFHNEYGKNRFP
                |||||||||||| |||||||||||||| ||:|||||||||||||||||||||||||||||
orf133-1        YPKGSKFTGWGLLKDFETYNNAKILDLNNTATFRLPRETELQTTLGFNYFHNEYGKNRFP
              360       370       380       390       400       410

                       410       420       430       440       450       460
orf133a.pep     EELGLFFDGPDXDNGLYSYLGRFKGDKGLLPQKSTIVQPAGSQYFNTFYFDAALKKDIYR
                ||||||||||| ||||||||||||||||||||||||||||||||||||||||||||||||
orf133-1        EELGLFFDGPDQDNGLYSYLGRFKGDKGLLPQKSTIVQPAGSQYFNTFYFDAALKKDIYR
              420       430       440       450       460       470

                       470       480       490       500       510       520
orf133a.pep     LNYSTNTVGYRFGGXYTGYYXSDDEFKRAFGENSPTYXKHCNQSCGIYEPVLKKYGKKRA
                |||||||||||||| ||||| |||||||||||||||| ||||:|||||||||||||||||
orf133-1        LNYSTNTVGYRFGGEYTGYYGSDDEFKRAFGENSPTYKKHCNRSCGIYEPVLKKYGKKRA
              480       490       500       510       520       530

                       530       540       550       560       570       580
orf133a.pep     NNHSVSISADFGDYFMPFASYSRTHRMPNIQEMYFSQIGDSGVHTALKPERANTWQFGFN
                ||||||||||||||||||||||||||||||||||||||||||||||||||||||||||||
orf133-1        NNHSVSISADFGDYFMPFASYSRTHRMPNIQEMYFSQIGDSGVHTALKPERANTWQFGFN
              540       550       560       570       580       590

                       590       600       610       620       630       640
orf133a.pep     TYKKGLLKQDDILGLKLVGYRSRIDXYIHNVYGKWWDLNGNIPSWVSSTGLAYTIQHRNF
                ||||||||||| ||||||||||||| ||||||||||||||:|||||||||||||||||||
orf133-1        TYKKGLLKQDDTLGLKLVGYRSRIDNYIHNVYGKWWDLNGDIPSWVSSTGLAYTIQHRNF
              600       610       620       630       640       650

                       650       660       670       680       690       700
orf133a.pep     KDKVHKHGFELELNYDYXRFFTNLSYAYQKSTQPTNFSDASESPNNASKEDQLKQGYGLS
                ||||||||||||||||| ||||||||||||||||||||||||||||||||||||||||||
orf133-1        KDKVHKHGFELELNYDYGRFFTNLSYAYQKSTQPTNFSDASESPNNASKEDQLKQGYGLS
              660       670       680       690       700       710

                       710       720       730       740       750       760
orf133a.pep     RVSALPRDYGRLEVGTRWLGNKLTLGGAMRYFGKSIRATAEERYIDXTNGXXTSNFRQLG
                |||||||||||||||||||||||||||||||||||||||||||||| |||  ||||||||
orf133-1        RVSALPRDYGRLEVGTRWLGNKLTLGGAMRYFGKSIRATAEERYIDGTNGGNTSNFRQLG
              720       730       740       750       760       770

                       770       780       790       800       810       820
orf133a.pep     KRSIXQTETLARQPLIFDXYAAYEPKKXLIFRAEVKNLFDRRYIDPLDAGNDAATQRYYS
                |||| ||||||||||||| |||||||| ||||||||||||||||||||||||||||||||
orf133-1        KRSIKQTETLARQPLIFDFYAAYEPKKNLIFRAEVKNLFDRRYIDPLDAGNDAATQRYYS
              780       790       800       810       820       830

                       830       840       850       860       870
orf133a.pep     SFDPKDKDEEVTCNDDNTLCNGKYGGTSKSVLTNFARGXTFLITMSYKFX
                |||||||||:|||| |:|||||||||||||||||||||| ||:|||||||
orf133-1        SFDPKDKDEDVTCNADKTLCNGKYGGTSKSVLTNFARGRTFLMTMSYKFX
              840       850       860       870       880
```

Homology with a predicted ORF from *N. gonorrhoeae*

ORF133 shows 92.3% identity over 392 aa overlap with a predicted ORF (ORF133ng) from *N. gonorrhoeae:*

```
orf133.pep                                      PGYYGSDDEFKRAFGENSPTXKKHCNRSCGI   31
                                                |||||::|||||||||||: |:||: |||:
orf133ng     FYFDAALKKDIYRLNYSTNAINYRFGGEYTGYYGSENEFKRAFGENSPAYKEHCDPSCGL  560

orf133.pep   YEPVLKKYGKKRANNHSVSISADFGDYFMPFASYSRTHRMPNIQEMYFSQIGDSGVHTAL   91
             |||||||||||||||||||||||||||||||:||||||||||||||||||||||||||||
orf133ng     YEPVLKKYGKKRANNHSVSISADFGDYFMPFAGYSRTHRMPNIQEMYFSQIGDSGVHTAL  620

orf133.pep   KPERANTWQFGFXTYKKGLLKQDDTLGLKLVGYRSRIDNYIHNVYGKWWDLNGDIPSWVS  151
             |||||||||||| |||||||||| ||||||||||||||||||||||||||||||||||||:
orf133ng     KPERANTWQFGFNTYKKGLLKQDDILGLKLVGYRSRIDNYIHNVYGKWWDLNGDIPSWVG  680

orf133.pep   STGLAYTIQHRXFXDKVHQXXXXXXXXYDYGRFFTNLSYAYQKSTQPTNFSDASESPNNA  211
             ||||||||:|| | ||||:         ||||||||||||||||||||||||||||||||
orf133ng     STGLAYTIRHRNFKDKVHKHGFELELNYDYGRFFTNLSYAYQKSTQPTNFSDASESPNNA  740

orf133.pep   SKEDQLKQGYGLSRVSALPRDYGRLEVGTRWLGNKLTLGGAMRYFGKSIRATAEERYIDG  271
             ||||||||||||||||||||||||||||||||||||||||||||||||||||||||||||
orf133ng     SKEDQLKQGYGLSRVSALPRDYGRLEVGTRWLGNKLTLGGAMRYFGKSIRATAEERYIDG  800

orf133.pep   TNGGNTSNFRQLGKRSIKQTETLARQPLIXDFNAAYEPKKNLIFRAEVKNLFDRRYIDPL  331
             |||||||| |||||||||||||||||||| || ||||||||||||||||||||||||||||
orf133ng     TNGGNTSNVRQLGKRSIKQTETLARQPLIFDFYAAYEPKKNLIFRAEVKNLFDRRYIDPL  860

orf133.pep   DAGNDAAXERYYSSFDPKDKDXDVTCNADKTLCNGKYGGTSKSVLTNFARGRTFLMTMSY  391
             |||||||::||||||||||||| ||||||||||||||||||||||||||||||||||||||
orf133ng     DAGNDAATQRYYSSFDPKDKDEDVTCNADKTLCNGKYGGTSKSVLTNFARGRTFLMTMSY  920

orf133.pep   KF  393
             ||
orf133ng     KF  922
```

The complete length ORF133ng nucleotide sequence <SEQ ID 881> is predicted to encode a protein having amino acid sequence <SEQ ID 882>:

```
  1  MRSSFRLKPI CFYLMGVMLY HHSYAEDAGR AGSEAQIQVL EDVHVKAKRV
 51  PKDKKVFTDA RAVSTRQDVF KSGENLDNIV RSIPGAFTQQ DKSSGIVSLN
101  IRGDSGFGRV NTMVDGITQT FYSTSTDAGR AGGSSQFGAS VDSNFIAGLD
151  VVKGSFSGSA GINSLAGSAN LRTLGVDDVV QGNNTYGLLL KGLTGTNSTK
201  GNAMAAIGAR KWLESGASVG VLYGHSRRGV AQNYRVGGGG QHIGNFGEEY
251  LERRKQQYFV QEGGLKFNAG SGKWERDLQR QYWKTKWYKK YEDPQELQKY
301  IEEHDKSWRE NLAPQYDITP IDPSGLKQQS AGNLLNLEYD GVFNKYTAQF
351  RDLNTRIGSR KIINRNYQFN YGLSLNPYTN LNLTAAYNSG RQKYPKGAKF
401  TGWGLLKDFE TYNNAKILDL NNTATFRLPR ETELQTTLGF NYFHNEYGKN
451  RFPEELGLFF DGPDQDNGLY SYLGRFKGDK GLLPQKSTIV QPAGSQYFNT
501  FYFDAALKKD IYRLNYSTNA INYRFGGEYT GYYGSENEFK RAFGENSPAY
551  KEHCDPSCGL YEPVLKKYGK KRANNHSVSI SADFGDYFMP FAGYSRTHRM
601  PNIQEMYFSQ IGDSGVHTAL KPERANTWQF GFNTYKKGLL KQDDILGLKL
651  VGYRSRIDNY IHNVYGKWWD LNGDIPSWVG STGLAYTIRH RNFKDKVHKH
701  GFELELNYDY GRFFTNLSYA YQKSTQPTNF SDASESPNNA SKEDQLKQGY
751  GLSRVSALPR DYGRLEVGTR WLGNKLTLGG AMRYFGKSIR ATAEERYIDG
801  TNGGNTSNVR QLGKRSIKQT ETLARQPLIF DFYAAYEPKK NLIFRAEVKN
851  LFDRRYIDPL DAGNDAATQR YYSSFDPKDK DEDVTCNADK TLCNGKYGGT
901  SKSVLTNFAR GRTFLMTMSY KF*
```

A variant was also identified, being encoded by the gonococcal DNA sequence <SEQ ID 883>:

```
   1 ATGAGATCTT CTTTCCGGTT GAAGCCGATT TGTTTTTATC TTATGGGTGT
  51 TATGCTATAT CATCATAGTT ATGCCGAAGA TGCAGGGCGC GCGGGCAGCG
 101 AGGCGCAGAT ACAGGTTTTG GAAGATGTGC ACGTCAAGGC GAAGCGCGTA
 151 CCGAAAGACA AAAAAGTGTT TACCGATGCG CGTGCCGTAT CGACCCGTca
 201 gGATGTGTTC AAATCCGGCG AAAACCTCGA CAACATCGTA CGCAGCATAC
 251 CCGGTGCGTT TACACAGCAA GATAAAAGCT CGGGCATTGT GTCTTTGAAT
```

```
 301 ATTCGCGGCG ACAGCGGGTT CGGGCGGGTC AATACGATGG TGGACGGCAT
 351 CACGCAGACC TTTTATTCGA CTTCTACCGA TGCGGGCAGG GCAGGCGGTT
 401 CATCTCAATT CGGTGCATCT GTCGACAGCA ATTTTATTGC CGGACTGGAT
 451 GTCGTCAAAG GCAGCTTCAG CGGCTCGGCA GGCATCAACA GCCTTGCCGG
 501 TTCGGCGAAT CTGCGGACTT TAGGCGTGGA TGACGTCGTT CAGGGCAATA
 551 ATACCTACGG CCTGCTGCTA AAAGGTCTGA CCGGCACCAA TTCAACCAAA
 601 GGTAATGCGA TGGCGGCGAT AGGTGCGCGC AAATGGCTGG AAAGCGGAGC
 651 GTCTGTCGGT GTGCTTTACG GGCACAGCAG GCGCGGCGTG GCGCAAAATT
 701 ACCGCGTGGG CGGCGGCGGG CAGCACATCG GAAATTTTGG TGAAGAATAT
 751 CTGGAACGGC GCAAACAGCA ATATTTTGTA CAAGAGGGTG GTTTGAAATT
 801 CAATGCCGGC AGCGGAAAAT GGGAACGGGA TTTGCAAAGG CAATACTGGA
 851 AAACAAAGTG GTATAAAAAA TACGAAGACC CCCAAGAACT GCAAAAATAC
 901 ATCGAAGAGC ATGATAAAAG CTGGCGGGAA AACCTGGCGC CGCAATACGA
 951 CATCACCCCC ATCGATCCGT CCGGCCTGAA GCAGCAGTCG GCAGGCAATC
1001 TGTTTAAATT GGAATACGAC GGCGTATTCA ATAAATACAC GGCGCAATTT
1051 CGCGATTTAA ACACCAGAAT CGGCAGCCGC AAAATCATCA ACCGCAATTA
1101 TCAATTCAAT TACGGTTTGT CTTTGAACCC GTATACCAAC CTCAATCTGA
1151 CCGCAGCCTA CAATTCGGGC AGGCAGAAAT ATCCGAAAGG GGCGAAGTTT
1201 ACAGGCTGGG GGCTTTTAAA AGATTTTGAA ACCTACAACA ACGCGAAAAT
1251 CCTCGACCTC AACAACACCG CCACCTTCCG GCTGCCCCGC GAAACCGAGT
1301 TGCAAACCAC TTTGGGCTTC AATTATTTCC ACAACGAATA CGGCAAAAAC
1351 CGCTTTCCTG AAGAATTGGG GCTGTTTTTC GACGGTCCTG ATCAGGACAA
1401 CGGGCTTTAT TCCTATTTGG GGCGGTTTAA GGGCGATAAA GGGCTGTTGC
1451 CTCAAAAATC AACCATTGTC CAACCGGCCG GCAGCCAATA TTTCAACACG
1501 TTCTACTTCG ATGCCGCGCT CAAAAAAGAC ATTTACCGCT TAAACTACAG
1551 CACCAATGCA ATCAACTACC GTTTCGGCGG CGAATATACG GGCTATTACG
1601 GCTCGGAAAA CGAATTTAAG CGGGCATTCG GAGAAAACTC GCCGGCATAC
1651 AAGGAACATT GCGACCCGAG CTGCGGGCTT TATGAACCCG TATTGAAAAA
1701 ATACGGCAAA AAGCGCGCCA ACAACCATTC GGTCAGCATT AGTGCGGACT
1751 TCGGCGATTA TTTCATGCCG TTCGCCGGCT ATTCGCGCAC ACACCGTATG
1801 CCCAACATCC AAGAAATGTA TTTTTCCCAA ATCGGCGACT CCGGCGTTCA
1851 CACCGCCTTA AAACCAGAGC GCGCAAACAC TTGGCAATTT GGCTTCAATA
1901 CCTATAAAAA AGGATTGTTA AAACAAGATG ATATATTAGG ATTGAAACTG
1951 GTCGGCTACC GCAGCCGCAT TGACAACTAC ATCCACAACG TTTACGGAAA
2001 ATGGTGGGAT TTGAACGGGG ATATTCCGAG CTGGGTCGGC AGCACCGGGC
2051 TTGCCTACAC CATCCGACAC CGCAATTTCA AAGACAAAGT GCACAAACAC
2101 GGTTTTGAGC TGGAGCTGAA TTACGATTAT GGGCGTTTTT TCACCAACCT
2151 TTCTTACGCC TATCAAAAAA GCACGCAACC GACCAATTTC AGCGATGCGA
2201 GCGAATCGCC CAACAATGCC tccaaAGAAG ACCAACTCAA ACAAGGTTAT
2251 GGGCTGAGCA GGGTTTCCGC CCTGCCGCGA GATTACGGAC GTTTGGAAGT
2301 CGGTACGCGC TGGTTGGGCA ACAAACTGAC TTTGGGCGGC GCGAtgcGCT
2351 ATTTCGGCAA GAGCATCCGC GCGACGGCTG AAGAACGCTA TATCGACGGC
2401 ACCAACGGGG GAAATACCAG CAATGTCCGG CAACTGGGCA AGCGTTCCAT
2451 CAAACAAACC GAAACCCTTG CCCGACAGCC TTTGATTTTT GATTTTTACG
2501 CCGCTTACGA GCCGAAGAAA AACCTTATTT TCCGCGCCGA AGTCAAAAAC
2551 CTGTTCGACA GGCGTTATAT CGATCCGCTC GATGCGGGCA ATGATGCGGC
2601 AACGCAGCGT TATTACAGCT CGTTCGACCC GAAAGACAAG GACGAAGACG
2651 TAACGTGTAA TGCTGATAAA ACGTTGTGCA ACGGCAAATA CGGCGGCACA
2701 AGCAAAAGCG TATTGACCAA TTTCGCACGC GGACGCACCT TCTTGATGAC
2751 GATGAGCTAC AAGTTTTAA
```

This corresponds to the amino acid sequence <SEQ ID 884; ORF133ng-1>:

```
  1 MRSSFRLKPI CFYLMGVMLY HHSYAEDAGR AGSEAQIQVL EDVHVKAKRV
 51 PKDKKVFTDA RAVSTRQDVF KSGENLDNIV RSIPGAFTQQ DKSSGIVSLN
101 IRGDSGFGRV NTMVDGITQT FYSTSTDAGR AGGSSQFGAS VDSNFIAGLD
151 VVKGSFSGSA GINSLAGSAN LRTLGVDDVV QGNNTYGLLL KGLTGTNSTK
201 GNAMAAIGAR KWLESGASVG VLYGHSRRGV AQNYRVGGGG QHIGNFGEEY
251 LERRKQQYFV QEGGLKFNAG SGKWERDLQR QYWKTKWYKK YEDPQELQKY
301 IEEHDKSWRE NLAPQYDITP IDPSGLKQQS AGNLFKLEYD GVFNKYTAQF
351 RDLNTRIGSR KIINRNYQFN YGLSLNPYTN LNLTAAYNSG RQKYPKGAKF
401 TGWGLLKDFE TYNNAKILDL NNTATFRLPR ETELQTTLGF NYFHNEYGKN
451 RFPEELGLFF DGPDQDNGLY SYLGRFKGDK GLLPQKSTIV QPAGSQYFNT
501 FYFDAALKKD IYRLNYSTNA INYRFGGEYT GYYGSENEFK RAFGENSPAY
551 KEHCDPSCGL YEPVLKKYGK KRANNHSVSI SADFGDYFMP FAGYSRTHRM
601 PNIQEMYFSQ IGDSGVHTAL KPERANTWQF GFNTYKKGLL KQDDILGLKL
651 VGYRSRIDNY IHNVYGKWWD LNGDIPSWVG STGLAYTIRH RNFKDKVHKH
701 GFELELNYDY GRFFTNLSYA YQKSTQPTNF SDASESPNNA SKEDQLKQGY
751 GLSRVSALPR DYGRLEVGTR WLGNKLTLGG AMRYFGKSIR ATAEERYIDG
801 TNGGNTSNVR QLGKRSIKQT ETLARQPLIF DFYAAYEPKK NLIFRAEVKN
851 LFDRRYIDPL DAGNDAATQR YYSSFDPKDK DEDVTCNADK TLCNGKYGGT
901 SKSVLTNFAR GRTFLMTMSY KF*
```

ORF133ng-1 and ORF133-1 show 96.2% identity in 889 aa overlap:

```
                       10        20        30        40        50        60
orf133ng-1.pep SFRLKPICFYLMGVMLYHHSYAEDAGRAGSEAQIQVLEDVHVKAKRVPKDKKVFTDARAV
                                             ||||||||||||||||||||||||||||||
orf133-1                                     EAQIQVLEDVHVKAKRVPKDKKVFTDARAV
                                                     10        20        30

                       70        80        90       100       110       120
orf133ng-1.pep STRQDVFKSGENLDNIVRSIPGAFTQQDKSSGIVSLNIRGDSGFGRVNTMVDGITQTFYS
               |||||:|||:||||||||||||||||||||||||||||||||||||||||||||||||||
orf133-1       STRQDIFKSSENLDNIVRSIPGAFTQQDKSSGIVSLNIRGDSGFGRVNTMVDGITQTFYS
                       40        50        60        70        80        90

                      130       140       150       160       170       180
orf133ng-1.pep TSTDAGRAGGSSQFGASVDSNFIAGLDVVKGSFSGSAGINSLAGSANLRTLGVDDVVQGN
               ||||||||||||||||||||||||||||||||||||||||||||||||||||||||||||
orf133-1       TSTDAGRAGGSSQFGASVDSNFIAGLDVVKGSFSGSAGINSLAGSANLRTLGVDDVVQGN
                      100       110       120       130       140       150

                      190       200       210       220       230       240
orf133ng-1.pep NTYGLLLKGLTGTNSTKGNAMAAIGARKWLESGASVGVLYGHSRRGVAQNYRVGGGGQHI
               |||||||||||||||||||||||||||||||||||||||||||||:||||||||||||||
orf133-1       NTYGLLLKGLTGTNSTKGNAMAAIGARKWLESGASVGVLYGHSRRSVAQNYRVGGGGQHI
                      160       170       180       190       200       210

                      250       260       270       280       290       300
orf133ng-1.pep GNFGEEYLERRKQQYFVQEGGLKFNAGSGKWERDLQRQYWKTKWYKKYEDPQELQKYIEE
               |||| ||||||||:||||||:||||: ||||||||||| || | ||:|:: |||||||||
orf133-1       GNFGAEYLERRKQRYFVQEGALKFNSDSGKWERDLQRQQWKYKPYKNYNN-QELQKYIEE
                      220       230       240       250       260

                      310       320       330       340       350       360
orf133ng-1.pep HDKSWRENLAPQYDITPIDPSGLKQQSAGNLFKLEYDGVFNKYTAQFRDLNTRIGSRKII
               ||||||||| |||||||||||:||||||||||||||||||||||||||||||:|||||||
orf133-1       HDKSWRENLXPQYDITPIDPSSLKQQSAGNLFKLEYDGVFNKYTAQFRDLNTKIGSRKII
             270       280       290       300       310       320

                      370       380       390       400       410       420
orf133ng-1.pep NRNYQFNYGLSLNPYTNLNLTAAYNSGRQKYPKGAKFTGWGLLKDFETYNNAKILDLNNT
               ||||||||||||||||||||||||||||||||||:|||||||||||||||||||||||||
orf133-1       NRNYQFNYGLSLNPYTNLNLTAAYNSGRQKYPKGSKFTGWGLLKDFETYNNAKILDLNNT
             330       340       350       360       370       380

                      430       440       450       460       470       480
orf133ng-1.pep ATFRLPRETELQTTLGFNYFHNEYGKNRFPEELGLFFDGPDQDNGLYSYLGRFKGDKGLL
               ||||||||||||||||||||||||||||||||||||||||||||||||||||||||||||
orf133-1       ATFRLPRETELQTTLGFNYFHNEYGKNRFPEELGLFFDGPDQDNGLYSYLGRFKGDKGLL
             390       400       410       420       430       440

                      490       500       510       520       530       540
orf133ng-1.pep PQKSTIVQPAGSQYFNTFYFDAALKKDIYRLNYSTNAINYRFGGEYTGYYGSENEFKRAF
               ||||||||||||||||||||||||||||||||||||:::|||||||||||||::||||||
orf133-1       PQKSTIVQPAGSQYFNTFYFDAALKKDIYRLNYSTNTVGYRFGGEYTGYYGSDDEFKRAF
             450       460       470       480       490       500

                      550       560       570       580       590       600
orf133ng-1.pep GENSPAYKEHCDPSCGLYEPVLKKYGKKRANNHSVSISADFGDYFMPFAGYSRTHRMPNI
               |||||:||:||: |||:||||||||||||||||||||||||||||||||:||||||||||
orf133-1       GENSPTYKKHCNRSCGIYEPVLKKYGKKRANNHSVSISADFGDYFMPFASYSRTHRMPNI
             510       520       530       540       550       560

                      610       620       630       640       650       660
orf133ng-1.pep QEMYFSQIGDSGVHTALKPERANTWQFGFNTYKKGLLKQDDILGLKLVGYRSRIDNYIHN
               ||||||||||||||||||||||||||||||||||||||||| ||||||||||||||||||
orf133-1       QEMYFSQIGDSGVHTALKPERANTWQFGFNTYKKGLLKQDDTLGLKLVGYRSRIDNYIHN
             570       580       590       600       610       620

                      670       680       690       700       710       720
orf133ng-1.pep VYGKWWDLNGDIPSWVGSTGLAYTIRHRNFKDKVHKHGFELELNYDYGRFFTNLSYAYQK
               ||||||||||||||||:||||||||:||||||||||||||||||||||||||||||||||  .
```

```
orf133-1         VYGKWWDLNGDIPSWVSSTGLAYTIQHRNFKDKVHKHGFELELNYDYGRFFTNLSYAYQK
               630       640       650       660       670       680

                     730       740       750       760       770       780
orf133ng-1.pep   STQPTNFSDASESPNNASKEDQLKQGYGLSRVSALPRDYGRLEVGTRWLGNKLTLGGAMR
                 ||||||||||||||||||||||||||||||||||||||||||||||||||||||||||||
orf133-1         STQPTNFSDASESPNNASKEDQLKQGYGLSRVSALPRDYGRLEVGTRWLGNKLTLGGAMR
               690       700       710       720       730       740

                     790       800       810       820       830       840
orf133ng-1.pep   YFGKSIRATAEERYIDGTNGGNTSNVRQLGKRSIKQTETLARQPLIFDFYAAYEPKKNLI
                 ||||||||||||||||||||||||| ||||||||||||||||||||||||||||||||||
orf133-1         YFGKSIRATAEERYIDGTNGGNTSNFRQLGKRSIKQTETLARQPLIFDFYAAYEPKKNLI
               750       760       770       780       790       800

                     850       860       870       880       890       900
orf133ng-1.pep   FRAEVKNLFDRRYIDPLDAGNDAATQRYYSSFDPKDKDEDVTCNADKTLCNGKYGGTSKS
                 ||||||||||||||||||||||||||||||||||||||||||||||||||||||||||||
orf133-1         FRAEVKNLFDRRYIDPLDAGNDAATQRYYSSFDPKDKDEDVTCNADKTLCNGKYGGTSKS
               810       820       830       840       850       860

                     910       920
orf133ng-1.pep   VLTNFARGRTFLMTMSYKFX
                 ||||||||||||||||||||
orf133-1         VLTNFARGRTFLMTMSYKFX
               870       880
```

In addition, ORF133ng-1 is homologous to a TonB-dependent receptor in *H.influenzae:*

```
sp|P45114|YC17_HAEIN PROBABLE TONB-DEPENDENT RECEPTOR HI1217 PRECURSOR
>gi|1075372|pir||G64110 transferrin binding protein 1 precursor (tbp1) homolog -
Haemophilus influenzae (strain Rd KW20) >gi|1574147 (U32801) transferrin binding
protein 1 precursor (tbp1) [Haemophilus influenzae] Length = 913
 Score =  930 bits (2377), Expect = 0.0
 Identities = 476/921 (51%), Positives = 619/921 (66%), Gaps = 72/921 (7%)

Query: 38  QVLEDVHVKAKRVPKDKKVFTDARAVSTRQDVFKSGENLDNIVRSIPGAFTQQDKSSGIV 97
            + L  + V  K +  DKK FT+A+A STR++VFK  + +D ++RSIPGAFTQQDK SG+V
Sbjct: 29  ETLGQIDVVEKVISNDKKPFTEAKAKSTRENVFKETQTIDQVIRSIPGAFTQQDKGSGVV 88

Query: 98  SLNIRGDSGFGRVNTMVDGITQTFYSTSTDAGRAGGSSQFGASVDSNFIAGLDVVKGSFS 157
            S+NIRG++G GRVNTMVDG+TQTFYST+ D+G++GGSSQFGA++D NFIAG+DV K +FS
Sbjct: 89  SVNIRGENGLGRVNTMVDGVTQTFYSTALDSGQSGGSSQFGAAIDPNFIAGVDVNKSNFS 148

Query: 158 GSAGINSLAGSANLRTLGVDDVVQXXXXXXXXXXXXXXXXXXXXXAMAAIGARKWLESGA 217
            G++GIN+LAGSAN RTLGV+DV+                       M     RKWL++G
Sbjct: 149 GASGINALAGSANFRTLGVNDVITDDKPFGIILKGMTGSNATKSNFMTMAAGRKWLDNGG 208

Query: 218 SVGVLYGHSRRGVAQNYRVGGGGQHIGNFGEEYLERRKQQYFVQEGGLKFNAGSGKWERD 277
             VGV+YG+S+R V+Q+YR+ GGG+ + + G++ L + K+ YF +  G   N   G+W  D
Sbjct: 209 YVGVVYGYSQREVSQDYRI-GGGERLASLGQDILAKEKEAYF-RNAGYILNP-EGQWTPD 265

Query: 278 LQRQYWK-----------TKWY--------------------KKYEDPQELQK---YIEE 303
            L +++W              +Y                    KK +D ++LQK    IEE
Sbjct: 266 LSKKHWSCNKPDYQKNGDCSYYRIGSAAKTRREILQELLTNGKKPKDIEKLQKGNDGIEE 325

Query: 304 HDKSWRENLAPQYDITPIDPSGLKQQSAGNLFKLEYDGVFNKYTAQFRDLNTRIGSRKII 363
             DKS+  N   QY + PI+P  L+ +S  +L K EY        AQ R L+ +IGSRKI
Sbjct: 326 TDKSFERN-KDQYSVAPIEPGSLQSRSRSHLLKFEYGDDHQNLGAQLRTLDNKIGSRKIE 384

Query: 364 NRNYQFNYGLSLNPYTNLNLTAAYNSGRQKYPKGAKFTGWGLLKDFETYNNAKILDLNNT 423
            NRNYQ NY  + N Y +LNL AA+N G+  YPKG  F GW +     T N A I+D+NN+
Sbjct: 385 NRNYQVNYNFNNNSYLDLNLMAAHNIGKTIYPKGGFFAGWQVADKLITKNVANIVDINNS 444

Query: 424 ATFRLPRETELQTTLGFNYFHNEYGKNRFPEELGLFFDGPDQDNGLYSY--LGRFKGDKG 481
             TF LP+E +L+TTLGFNYF NEY KNRFPEEL LF++    D GLYS+   GR+ G K
Sbjct: 445 HTFLLPKEIDLKTTLGFNYFTNEYSKNRFPEELSLFYNDASHDQGLYSHSKRGRYSGTKS 504

Query: 482 LLPQKSTIVQPAGSQYFNTFYFDAALKKDIYRLNYSTNAINYRFGGEYTGYYGSENEFKR 541
            LLPQ+S I+QP+G Q F T YFD AL K IY LNYS N  +Y F GEY GY
Sbjct: 505 LLPQRSVILQPSGKQKFKTVYFDTALSKGIYHLNYSVNFTHYAFNGEYVGY--------- 555
```

```
Query: 542 AFGENSPAYKEHCDPSCGLYEPVLKKYGKKRANNHSVSISADFGDYFMPFAGYSRTHRMP 601
              EN+   +          + EP+L K G K+A NHS ++SA+  DYFMPF  YSRTHRMP
Sbjct: 556 ---ENTAGQQ--------INEPILHKSGHKKAFNHSATLSAELSDYFMPFFTYSRTHRMP 604

Query: 602 NIQEMYFSQIGDSGVHTALKPERANTWQFGFNTYKKGLLKQDDILGLKLVGYRSRIDNYI 661
           NIQEM+FSQ+ ++GV+TALKPE+++T+Q GFNTYKKGL  QDD+LG+KLVGYRS I NYI
Sbjct: 605 NIQEMFFSQVSNAGVNTALKPEQSDTYQLGFNTYKKGLFTQDDVLGVKLVGYRSFIKNYI 664

Query: 662 HNVYGKWWDLNGDIPSWVGSTGLAYTIRHRNFKDKVHKHGFELELNYDYGRFFTNLSYAY 721
           HNVYG WW     +P+W  S G  YTI H+N+K  V K G ELE+NYD GRFF N+SYAY
Sbjct: 665 HNVYGVWW--RDGMPTWAESNGFKYTIAHQNYKPIVKKSGVELEINYDMGRFFANVSYAY 722

Query: 722 QKSTQPTNFSDASESPNNASKEDQLKQGYGLSRVSALPRDYGRLEVGTRWLGNKLTLGGA 781
           Q++ QPTN++DAS  PNNAS+ED LKQGYGLSRVS LP+DYGRLE+GTRW   KLTLG A
Sbjct: 723 QRTNQPTNYADASPRPNNASQEDILKQGYGLSRVSMLPKDYGRLELGTRWFDQKLTLGLA 782

Query: 782 MRYFGKSIRATAEERYIDGTNGGNTSNVRQLGKRSIKQTETLARQPLIFDFYAAYEPKKN 841
            RY+GKS RAT EE YI+G+     + +R+    ++K+TE + +QP+I D + +YEP K+
Sbjct: 783 ARYYGKSKRATIEEEYINGSR-FKKNTLRRENYYAVKKTEDIKKQPIILDLHVSYEPIKD 841

Query: 842 LIFRAEVKNLFDRRYIDPLDAGNDAATQRYYSSFDPKDKDEDVTCNADKTLCNGKYGGTS 901
           LI +AEV+NL D+RY+DPLDAGNDAA+QRYYSS       +  + C  D + C    GG+
Sbjct: 842 LIIKAEVQNLLDKRYVDPLDAGNDAASQRYYSSL-----NNSIECAQDSSAC----GGSD 892

Query: 902 KSVLTNFARGRTFLMTMSYKF 922
           K+VL NFARGRT++++++YKF
Sbjct: 893 KTVLYNFARGRTYILSLNYKF 913
```

The underlined motif in the gonococcal protein (also present in the meningococcal protein) is predicted to be an ATP/GTP-binding site motif A (P-loop), and the analysis suggests that these proteins from *N.meningitidis* and *N.gonorrhoeae,* and their epitopes, could be useful antigens for vaccines or diagnostics, or for raising antibodies.

## Example 104

The following partial DNA sequence was identified in *N.meningitidis* <SEQ ID 885>

```
  1 ATGAACCTGA TTTCACGTTA CATCATCCGT CAAATGGCGG TTATGGCGGT
 51 TTACGCGCTC CTTGCCTTCC TCGCTTTGTA CAGCTTTTTT GAAATCCTGT
101 ACGAAACCGG CAACCTCGGC AAAGGCAGTT ACGGCATATG GGAAATGCTG
151 GGCTACACCG CCCTCAAAAT GCCCGCCCGC GCCTACGAAC TGATTCCCCT
201 CGCCGTCCTT ATCGGCGGAC TGGTCTCCCT CAGCCAGCTT GCCGCCGGCA
251 GCGAACTGAC CGTCATCAAA GCCAGCGGCA TGAGCACCAA AAAGCTGCTG
301 TTGATTCTGT CGCAGTTCGG TTTTATTTTT GCTATTGCCA CCGTCGCGCT
351 CGGCGAATGG GTTGCGCCCA CACTGAGCCA AAAAGCCGAA AACATCAAAG
401 CCGCCGCCAT CAACGGCAAA ATCAGCACCG GCAATACCGG CCTTTGGCTG
451 AAAGAAAAAA ACAGCGTGAT CAATGTGCGC GAAATGTTGC CCGACCAT..
```

This corresponds to the amino acid sequence <SEQ ID 886; ORF112>:

```
  1 MNLISRYIIR QMAVMAVYAL LAFLALYSFF EILYETGNLG KGSYGIWEML
 51 GYTALKMPAR AYELIPLAVL IGGLVSLSQL AAGSELTVIK ASGMSTKKLL
101 LILSQFGFIF AIATVALGEW VAPTLSQKAE NIKAAAINGK ISTGNTGLWL
151 KEKNSVINVR EMLPDH...
```

Further work revealed further partal nucleotide sequence <SEQ ID 887>:

```
  1  ATGAACCTGA TTTCACGTTA CATCATCCGT CAAATGGCGG TTATGGCGGT
 51  TTACGCGCTC CTTGCCTTCC TCGCTTTGTA CAGCTTTTTT GAAATCCTGT
101  ACGAAACCGG CAACCTCGGC AAAGGCAGTT ACGGCATATG GGAAATGCTG
151  gGCTACACCG CCCTCAAAAT GCCCGCCCGC GCCTACGAAC TGATTCCCCT
201  CGCCGTCCTT ATCGGCGGAC TGGTCTCCCT CAGCCAGCTT GCCGCCGGCA
251  GCGAACTGAC CGTCATCAAA GCCAGCGGCA TGAGCACCAA AAAGCTGCTG
301  TTGATTCTGT CGCAGTTCGG TTTTATTTTT GCTATTGCCA CCGTCGCGCT
351  CGGCGAATGG GTTGCGCCCA CACTGAGCCA AAAAGCCGAA AACATCAAAG
401  CCGCCGCCAT CAACGGCAAA ATCAGCACCG GCAATACCGG CCTTTGGCTG
```

```
451  AAAGAAAAAA ACAGCrTkAT CAATGTGCGC GAAATGTTGC CCGACCATAC
501  GCTTTTGGGC ATCAAAATTT GGGCGCGCAA CGATAAAAAC GAATTGGCAG
551  AGGCAGTGGA AGCCGATTCC GCCGTTTTGA ACAGCGACGG CAGTTGGCAG
601  TTGAAAAACA TCCGCCGCAG CACGCTTGGC GAAGACAAAG TCGAGGTCTC
651  TATTGCGGCT GAAGAAAACT GGCCGATTTC CGTCAAACGC AACCTGATGG
701  ACGTATTGCT CGTCAAACCC GACCAAATGT CCGTCGGCGA ACTGACCACC
751  TACATCCGCC ACCTCCAAAA CAACAGCCAA AACACCCGAA TCTACGCCAT
801  CGCATGGTGG CGCAAATTGG TTTACCCCGC CGCAGCCTGG GTGATGGCGC
851  TCGTCGCCTT TGCCTTTACC CCGCAAACCA CCCGCCACGG CAATATGGGC
901  TTAAAACTCT TCGGCGGCAT CTGTsTCGGA TTGCTGTTCC ACCTTGCCGG
951  ACGGCTCTTT GGGTTTACCA GCCAACTCGG...
```

This corresponds to the amino acid sequence <SEQ ID 888; ORF112-1>:

```
  1  MNLISRYIIR QMAVMAVYAL LAFLALYSFF EILYETGNLG KGSYGIWEML
 51  GYTALKMPAR AYELIPLAVL IGGLVSLSQL AAGSELTVIK ASGMSTKKLL
101  LILSQFGFIF AIATVALGEW VAPTLSQKAE NIKAAAINGK ISTGNTGLWL
151  KEKNSXINVR EMLPDHTLLG IKIWARNDKN ELAEAVEADS AVLNSDGSWQ
201  LKNIRRSTLG EDKVEVSIAA EENWPISVKR NLMDVLLVKP DQMSVGELTT
251  YIRHLQNNSQ NTRIYAIAWW RKLVYPAAAW VMALVAFAFT PQTTRHGNMG
301  LKLFGGICXG LLFHLAGRLF GFTSQL...
```

Computer analysis of this amino acid sequence predicts two transmembrane domains and gave the following results:

Homology with a predicted ORF from *N.meningitidis* (strain A)

ORF112 shows 96.4% identity over a 166aa overlap with an ORF (ORF112a) from strain A of *N. meningitidis:*

```
                    10        20        30        40        50        60
orf112.pep  MNLISRYIIRQMAVMAVYALLAFLALYSFFEILYETGNLGKGSYGIWEMLGYTALKMPAR
            ||||||||||||||||||||||||||||||||||||||||||||||||| ||||||| ||
orf112a     MNLISRYIIRQMAVMAVYALLAFLALYSFFEILYETGNLGKGSYGIWEMXGYTALKMXAR
                    10        20        30        40        50        60

                    70        80        90       100       110       120
orf112.pep  AYELIPLAVLIGGLVSLSQLAAGSELTVIKASGMSTKKLLLILSQFGFIFAIATVALGEW
            ||||:||||||||||| |||||||||:|||||||||||||||||||||||||||||||||
orf112a     AYELMPLAVLIGGLVSXSQLAAGSELXVIKASGMSTKKLLLILSQFGFIFAIATVALGEW
                    70        80        90       100       110       120

                   130       140       150       160
orf112.pep  VAPTLSQKAENIKAAAINGKISTGNTGLWLKEKNSVINVREMLPDH
            |||||||||||||||||||||||||||||||||||:||||||||||
orf112a     VAPTLSQKAENIKAAAINGKISTGNTGLWLKEKNSIINVREMLPDHTLLGIKIWARNDKN
                   130       140       150       160       170       180

orf112a     ELAEAVEADSAVLNSDGSWQLKNIRRSTLGEDKVEVSIAAEEXWPISVKRNLMDVLLVKP
                   190       200       210       220       230       240
```

The ORF112a nucleotide sequence <SEQ ID 889> is:

```
   1  ATGAACCTGA TTTCACGTTA CATCATCCGT CAAATGGCGG TTATGGCGGT
  51  TTACGCGCTC CTTGCCTTCC TCGCTTTGTA CAGCTTTTTT GAAATCCTGT
 101  ACGAAACCGG CAACCTCGGC AAAGGCAGTT ACGGCATATG GGAAATGNTG
 151  GGNTACACCG CCCTCAAAAT GNCCGCCCGC GCCTACGAAC TGATGCCCCT
 201  CGCCGTCCTT ATCGGCGGAC TGGTCTCTNT CAGCCAGCTT GCCGCCGGCA
 251  GCGAACTGAN CGTCATCAAA GCCAGCGGCA TGAGCACCAA AAAGCTGCTG
 301  TTGATTCTGT CGCAGTTCGG TTTTATTTTT GCTATTGCCA CCGTCGCGCT
 351  CGGCGAATGG GTTGCGCCCA CACTGAGCCA AAAAGCCGAA AACATCAAAG
 401  CCGCGGCCAT CAACGGCAAA ATCAGTACCG GCAATACCGG CCTTTGGCTG
 451  AAAGAAAAAA ACAGCATTAT CAATGTGCGC GAAATGTTGC CCGACCATAC
 501  CCTGCTGGGC ATTAAAATCT GGGCCCGCAA CGATAAAAAC GAACTGGCAG
 551  AGGCAGTGGA AGCCGATTCC GCCGTTTTGA ACAGCGACGG CAGTTGGCAG
 601  TTGAAAAACA TCCGCCGCAG CACGCTTGGC GAAGACAAAG TCGAGGTCTC
 651  TATTGCGGCT GAAGAAAANT GGCCGATTTC CGTCAAACGC AACCTGATGG
 701  ACGTATTGCT CGTCAAACCC GACCAAATGT CCGTCGGCGA ACTGACCACC

 751  TACATCCGCC ACCTCCAAAN NNACAGCCAA AACACCCGAA TCTACGCCAT
 801  CGCATGGTGG CGCAAATTGG TTTACCCCGC CGCAGCCTGG GTGATGGCGC
 851  TCGTCGCCTT TGCCTTTACC CCGCAAACCA CCCGCCACGG CAATATGGGC
 901  TTAAAANTCT TCGGCGGCAT CTGTCTCGGA TTGCTGTTCC ACCTTGCCGG
 951  NCGGCTCTTC NGGTTTACCA GCCAACTCTA CGGCATCCCG CCCTTCCTCG
1001  NCGGCGCACT ACCTACCATA GCCTTCGCCT TGCTCGCCGT TTGGCTGATA
1051  CGCAAACAGG AAAAACGCTA A
```

This encodes a protein having the amino acid sequence <SEQ ID 890>:

```
   1  MNLISRYIIR QMAVMAVYAL LAFLALYSFF EILYETGNLG KGSYGIWEMX
  51  GYTALKMXAR AYELMPLAVL IGGLVSXSQL AAGSELXVIK ASGMSTKKLL
 101  LILSQFGFIF AIATVALGEW VAPTLSQKAE NIKAAAINGK ISTGNTGLWL
 151  KEKNSIINVR EMLPDHTLLG IKIWARNDKN ELAEAVEADS AVLNSDGSWQ
 201  LKNIRRSTLG EDKVEVSIAA EEXWPISVKR NLMDVLLVKP DQMSVGELTT
 251  YIRHLQXXSQ NTRIYAIAWW RKLVYPAAAW VMALVAFAFT PQTTRHGNMG
 301  LKXFGGICLG LLFHLAGRLF XFTSQLYGIP PFLXGALPTI AFALLAVWLI
 351  RKQEKR*
```

ORF112a and ORF112-1 show 96.3% identity in 326 aa overlap:

```
orf112a.pep     MNLISRYIIRQMAVMAVYALLAFLALYSFFEILYETGNLGKGSYGIWEMXGYTALKMXAR
                ||||||||||||||||||||||||||||||||||||||||||||||||| ||||||| ||
orf112-1        MNLISRYIIRQMAVMAVYALLAFLALYSFFEILYETGNLGKGSYGIWEMLGYTALKMPAR

orf112a.pep     AYELMPLAVLIGGLVSXSQLAAGSELXVIKASGMSTKKLLLILSQFGFIFAIATVALGEW
                ||||:||||||||||| |||||||||:|||||||||||||||||||||||||||||||||
orf112-1        AYELIPLAVLIGGLVSLSQLAAGSELTVIKASGMSTKKLLLILSQFGFIFAIATVALGEW

orf112a.pep     VAPTLSQKAENIKAAAINGKISTGNTGLWLKEKNSIINVREMLPDHTLLGIKIWARNDKN
                ||||||||||||||||||||||||||||||||||| ||||||||||||||||||||||||
orf112-1        VAPTLSQKAENIKAAAINGKISTGNTGLWLKEKNSXINVREMLPDHTLLGIKIWARNDKN

orf112a.pep     ELAEAVEADSAVLNSDGSWQLKNIRRSTLGEDKVEVSIAAEEXWPISVKRNLMDVLLVKP
                |||||||||||||||||||||||||||||||||||||||||| |||||||||||||||||
orf112-1        ELAEAVEADSAVLNSDGSWQLKNIRRSTLGEDKVEVSIAAEENWPISVKRNLMDVLLVKP

orf112a.pep     DQMSVGELTTYIRHLQXXSQNTRIYAIAWWRKLVYPAAAWVMALVAFAFTPQTTRHGNMG
                ||||||||||||||||  ||||||||||||||||||||||||||||||||||||||||||
orf112-1        DQMSVGELTTYIRHLQNNSQNTRIYAIAWWRKLVYPAAAWVMALVAFAFTPQTTRHGNMG

orf112a.pep     LKXFGGICLGLLFHLAGRLFXFTSQLYGIPPFLXGALPTIAFALLAVWLIRKQEKRX
                || ||||| ||||||||||| |||||
orf112-1        LKLFGGICXGLLFHLAGRLFGFTSQL
```

<u>Homology with a predicted ORF from *N.gonorrhoeae*</u>

ORF112 shows 95.8% identity over 166aa overlap with a predicted ORF (ORF112ng) from *N. gonorrhoeae:*

```
orf112.pep   MNLISRYIIRQMAVMAVYALLAFLALYSFFEILYETGNLGKGSYGIWEMLGYTALKMPAR  60
             ||||||||||||||||||||||||||||||||||||||||||||||||||||||||||||
orf112ng     MNLISRYIIRQMAVMAVYALLAFLALYSFFEILYETGNLGKGSYGIWEMLGYTALKMPAR  60

orf112.pep   AYELIPLAVLIGGLVSLSQLAAGSELTVIKASGMSTKKLLLILSQFGFIFAIATVALGEW 120
             ||||:|||||||||:|||||||||||:||||||||||||||||||||||||||:||||||
orf112ng     AYELMPLAVLIGGLASLSQLAAGSELAVIKASGMSTKKLLLILSQFGFIFAIAAVALGEW 120

orf112.pep   VAPTLSQKAENIKAAAINGKISTGNTGLWLKEKNSVINVREMLPDH               166
             |||||||||||||||||||||||||||||||||:|:|||| |||||
orf112ng     VAPTLSQKAENIKAAAINGKISTGNTGLWLKEKTSIINVRGMLPDHTLLGIKIWARNDKN 180
```

The complete length ORF112ng nucleotide sequence <SEQ ID 891> is:

```
  1 ATGAACCTGA TTTCACGTTA CATCATCCGC CAAATGGCGG TTATGGCGGT
 51 TTACGCGCTC CTTGCCTTCC TCGCTTTGTA CAGCTTTTTT GAAATCCTGT
101 ACGAAACCGG CAACCTCGGC AAAGGCAGTT ACGGCATATG GGAAATGCTG
151 GGCTACACCG CCCTCAAAAT GCCCGCCCGC GCCTACGAAC TCATGCCCCT
201 CGCCGTCCTC ATCGGCGGAC TGGCCTCTCT CAGCCAGCTT GCCGCCGGCA
251 GCGAACTGGC CGTCATCAAA GCCAGCGGCA TGAGCACCAA AAAGCTGCTG
```

```
 301  TTGATTCTGT CTCAGTTCGG TTTTATTTTT GCTATTGCCG CCGTCGCGCT
 351  CGGCGAATGG GTTGCGCCCA CGCTGAGCCA AAAAGCCGAA AACATCAAag
 401  cCGCCGCCAt taacggCAAA ATCAGCAccg gcAATACCGG CCTTTggcTG
 451  AAAGAAAAAa ccAGCATTAT CAATGTGcGc GGAATGTTGC CCGACCATAC
 501  GCTTTTGGGC ATCAAAATTT GGGCGCGCAA CGATAAAAAC GAATTGGCAG
 551  AGGCAGTGGA AGCCGATTCC GCCGTTTTGA ACAGCGACGG CAGCTGGCAG
 601  TTGAAAAACA TCCGCCGCAG CATCATGGGT ACAGACAAAA TCGAAACATC
 651  cgCCGCCGCC GAAGAAACTT gGCCGATTGC CGTCAGACGC AACCTGATGG
 701  ACGTATTGCT CGTCAAGCCC GACCAAATGT CCGTCGGCGA GCTGACCACC
 751  TACATCCGCC ACCTCCAAAA CAACAGCCAA AACACCCAAA TCTACGCCAT
 801  CGCATGGTGG CGTAAACTCG TTTACCCCGT CGCCGCATGG GTCATGGCGC
 851  TCGTTGCCTT CGCCTTTACG CCGCAAACCA CGCGCCACGG CAATATGGGC
 901  TTAAAACTCT TCGGCGGCAT CTGTCTCGGA TTGCTGTTCC ACCTTGCCGG
 951  CAGGCTCTTC GGGTTTACCA GCCAACTCTA CGGCACCCCA CCCTTCCTCG
1001  CCGGCGCACT GCCTACCATA GCCTTCGCCT TGCTCGCTGT TTGGCTGATA
1051  CGCAAACAGG AAAAACGTTG A
```

This encodes a protein having amino acid sequence <SEQ ID 892>:

```
  1  MNLISRYIIR QMAVMAVYAL LAFLALYSFF EILYETGNLG KGSYGIWEML
 51  GYTALKMPAR AYELMPLAVL IGGLASLSQL AAGSELAVIK ASGMSTKKLL
101  LILSQFGFIF AIAAVALGEW VAPTLSQKAE NIKAAAINGK ISTGNTGLWL
151  KEKTSIINVR GMLPDHTLLG IKIWARNDKN ELAEAVEADS AVLNSDGSWQ
201  LKNIRRSIMG TDKIETSAAA EETWPIAVRR NLMDVLLVKP DQMSVGELTT
251  YIRHLQNNSQ NTQIYAIAWW RKLVYPVAAW VMALVAFAFT PQTTRHGNMG
301  LKLFGGICLG LLFHLAGRLF GFTSQLYGTP PFLAGALPTI AFALLAVWLI
351  RKQEKR*
```

ORF112ng and ORF112-1 show 94.2% identity in 326 aa overlap:

```
                   10        20        30        40        50        60
orf112ng   MNLISRYIIRQMAVMAVYALLAFLALYSFFEILYETGNLGKGSYGIWEMLGYTALKMPAR
           ||||||||||||||||||||||||||||||||||||||||||||||||||||||||||||
orf112-1   MNLISRYIIRQMAVMAVYALLAFLALYSFFEILYETGNLGKGSYGIWEMLGYTALKMPAR
                   10        20        30        40        50        60

                   70        80        90       100       110       120
orf112ng   AYELMPLAVLIGGLASLSQLAAGSELAVIKASGMSTKKLLLILSQFGFIFAIAAVALGEW
           ||||:||||||||||:||||||||||||:|||||||||||||||||||||||||||:||||||
orf112-1   AYELIPLAVLIGGLVSLSQLAAGSELTVIKASGMSTKKLLLILSQFGFIFAIATVALGEW
                   70        80        90       100       110       120

                  130       140       150       160       170       180
orf112ng   VAPTLSQKAENIKAAAINGKISTGNTGLWLKEKTSIINVRGMLPDHTLLGIKIWARNDKN
           ||||||||||||||||||||||||||||||||||:| |||| ||||||||||||||||||
orf112-1   VAPTLSQKAENIKAAAINGKISTGNTGLWLKEKNSXINVREMLPDHTLLGIKIWARNDKN
                  130       140       150       160       170       180

                  190       200       210       220       230       240
orf112ng   ELAEAVEADSAVLNSDGSWQLKNIRRSIMGTDKIETSAAAEETWPIAVRRNLMDVLLVKP
           ||||||||||||||||||||||||||| :| ||:|:| ||||:|||:|:||||||||||
orf112-1   ELAEAVEADSAVLNSDGSWQLKNIRRSTLGEDKVEVSIAAEENWPISVKRNLMDVLLVKP
                  190       200       210       220       230       240

                  250       260       270       280       290       300
orf112ng   DQMSVGELTTYIRHLQNNSQNTQIYAIAWWRKLVYPVAAWVMALVAFAFTPQTTRHGNMG
           |||||||||||||||||||||:||||||||||||:||||||||||||||||||||||||
orf112-1   DQMSVGELTTYIRHLQNNSQNTRIYAIAWWRKLVYPAAAWVMALVAFAFTPQTTRHGNMG
                  250       260       270       280       290       300

                  310       320       330       340       350
orf112ng   LKLFGGICLGLLFHLAGRLFGFTSQLYGTPPFLAGALPTIAFALLAVWLIRKQEKRX
           |||||||| ||||||||||||||||
orf112-1   LKLFGGICXGLLFHLAGRLFGFTSQL
                  310       320
```

This analysis suggests that these proteins from *N.meningitidis* and *N.gonorrhoeae,* and their epitopes, could be useful antigens for vaccines or diagnostics, or for raising antibodies.

It will be appreciated that the invention has been described by means of example only, and that modifications may be made whilst remaining within the spirit and scope of the invention.

**TABLE I - PCR primers**

| ORF | Primer | Sequence | Restriction sites |
|---|---|---|---|
| **ORF 1** | Forward | CGCGGATCCGCTAGC-GGACACACTTATTTCGG | BamHI-NheI |
| | Reverse | CCCGCTCGAG-CCAGCGGTAGCCTAATT | XhoI |
| **ORF 2** | Forward | GCGGATCCCATATG-TTTGATTTCGGTTTGGG | BamHI-NdeI |
| | Reverse | CCCGCTCGAG-GACGGCATAACGGCG | XhoI |
| **ORF 2-1** | Forward | GCGGATCCCATATG-TTTGATTTCGGTTTGGG | BamHI-NdeI |
| | Reverse | CCCGCTCGAG-TGATTTACGGACGCGCA | XhoI |
| **ORF 4** | Forward | GCGGATCCCATATG-TGCGGAGGTCAAAAAGAC | BamHI-NdeI |
| | Reverse | CCCGCTCGAG-TTTGGCTGCGCCTTC | XhoI |
| **ORF 5** | Forward | GGAATTCCATATGGCCATGG-TGGAAGGCGCACAACC | NdeI-NcoI |
| | Forward | CGGGATCC-ATGGAAGGCGCACAAC | BamHI |
| | Reverse | CCCGCTCGAG-GACTGTGCAAAAACGG | XhoI |
| **ORF 6** | Forward | CGCGGATCCCATATG-ACCCGTCAATCTCTGCA | BamHI-NdeI |
| | Reverse | CCCGCTCGAG-TGCGCCGAACACTTTC | XhoI |
| **ORF 7** | Forward | CGCGGATCCGCTAGC-GCGCTGCTTTTTGTTCC | BamHI-NheI |
| | Reverse | CCCGCTCGAG-TTTCAAAATATATTTGCGGA | XhoI |
| **ORF 8** | Forward | GCGGATCCCATATG-GCTCAACTGCTTCGTAC | BamHI-NdeI |
| | Reverse | CCCGCTCGAG-AGCAGGCTTTGGCGC | XhoI |
| **ORF 9** | Forward | CGCGGATCCCATATG-CCGAAGGAAGTCGGAAA | BamHI-NdeI |
| | Reverse | CCCGCTCGAG-TTTCCGAGGTTTTCGGG | XhoI |
| **ORF 10** | Forward | GCGGATCCCATATG-GACACAAAAGAAATCCTC | BamHI-NdeI |
| | Reverse | CCCGCTCGAG- TAATGGGAAACCTTGTTTT | XhoI |
| **ORF 11** | Forward | GCGGATCCCATATG-GCGGTCAACCTCTACG | BamHI-NdeI |
| | Reverse | CCCGCTCGAG-GGAAACGACTTCGCC | XhoI |
| **ORF 13** | Forward | CGCGGATCCCATATG-GCTCTGCTTTCCGCGC | BamHI-NdeI |
| | Reverse | CCCGCTCGAG-AGGGTGTGTGATAATAAG | XhoI |
| **ORF 15** | Forward | GGAATTCCATATGGCCATGG-GCGGGACACTGACAG | NdeI-NcoI |
| | Forward | CGGGATCC-TGCGGGACACTGACAGG | BamHI |
| | Reverse | CCCGCTCGAG-AGGTTGGCCTTGTCTATG | XhoI |
| **ORF 17** | Forward | GGAATTCCATATGGCCATGG -TTGCCGGCCTGTTCG | NdeI-NcoI |
| | Forward | CGGGATCC-ATTGCCGGCCTGTTCG | BamHI |
| | Reverse | CCCGCTCGAG-AAGCAGGTTGTACAGC | XhoI |

(continued)

| ORF | Primer | Sequence | Restriction sites |
|---|---|---|---|
| **ORF 18** | Forward | GCGGATCCCATATG-ATTTTGCTGCATTTGGAT | BamHI-NdeI |
| | Reverse | CCCGCTCGAG-TCTTCCAATTTCTGAAAGC | XhoI |
| **ORF 19** | Forward | GGAATTCCATATGGCCATGG -TCGCCAGTGTTTTTACC | NdeI-NcoI |
| | Forward | CGGGATCC-TTCGCCAGTGTTTTTACCG | BamHI |
| | Reverse | CCCGCTCGAG-GGTGTTTTTGAAGCTGCC | XhoI |
| **ORF 20** | Forward | GGAATTCCATATGGCCATGG -TCGGCGCGGGTATG | NdeI-NcoI |
| | Forward | CGGGATCC-TTCGGCGCGGGTATG | BamHI |
| | Reverse | CCCGCTCGAG-CGGCGAGCGAGAGCA | XhoI |
| **ORF 22** | Forward | GGAATTCCATATGGCCATGG-TGATTAAAATCAAAAAAGGTCT | NdeI-NcoI |
| | Forward | CGGGATCC-ATGATTAAAATCAAAAAAGGTCTAAACC | BamHI |
| | Reverse | CCCGCTCGAG-ATTATGATAGCGGCCC | XhoI |
| **ORF 23** | Forward | CGCGGATCCCATATG-GATGTTTCTGTTTCAGAC | BamHI-NdeI |
| | Reverse | CCCGCTCGAG-TTTAAACCGATAGGTAAACG | XhoI |
| **ORF 24** | Forward | GGAATTCCATATGGCCATGG -TGATGCCGGAAATGGTG | NdeI-NcoI |
| | Forward | CGGGATCC-ATGATGCCGGAAATGGTG | BamHI |
| | Reverse | CCCGCTCGAG-TGTCAGCGTGGCGCA | XhoI |
| **ORF 25** | Forward | GCGGATCCCATATG-TATCGCAAACTGATTGC | BamHI-NdeI |
| | Reverse | CCCGCTCGAG-ATCGATGGAATAGCCG | XhoI |
| **ORF 26** | Forward | GCGGATCCCATATG -CAGCTGATCGACTATTC | BamHI-NdeI |
| | Reverse | CCCGCTCGAG-GACATCGGCGCGTTTT | XhoI |
| **ORF 27** | Forward | GGAATTCCATATGGCCATGG-AGACCTATTCTGTTTA | NdeI-NcoI |
| | Forward | CGGGATCC- CAGACCTATTCTGTTTATTTTAATC | BamHI |
| | Reverse | CCCGCTCGAG-GGGTTCGATTAAATAACCAT | XhoI |
| **ORF 28** | Forward | GGAATTCCATATGGCCATGG-ACGGCTGTACGTTGATGT | NdeI-NcoI |
| | Forward | CGGGATCC-AACGGCTGTACGTTGATG | BamHI |
| | Reverse | CCCGCTCGAG-TTTGTCAGAGGAATTCGCG | XhoI |
| **ORF 29** | Forward | GCGGATCCCATATG -AACGGTTTGGATGCCCG | BamHI-NdeI |
| | Forward | CGCGGATCCGCTAGC-AACGGTTTGGATGCCCG | BamHI-NheI |
| | Reverse | CCCGCTCGAG-TTTGTCTAAGTTCCTGATATG | XhoI |
| **ORF 32** | Forward | CGCGGATCCCATATG-AATACTCCTCCTTTTG | BamHI-NdeI |
| | Reverse | CCCGCTCGAG-GCGTATTTTTTGATGCTTTG | XhoI |
| **ORF 33** | Forward | GCGGATCCCATATG -ATTGATAGGGATCGTATG | BamHI-NdeI |
| | Reverse | CCCGCTCGAG-TTGATCTTTCAAACGGCC | XhoI |

(continued)

| ORF | Primer | Sequence | Restriction sites |
|---|---|---|---|
| **ORF 35** | Forward | GCGGATCCCATATG-TTCAGAGCTCAGCTT | BamHI-NdeI |
| | Forward | CGCGGATCCGCTAGC-TTCAGAGCTCAGCTT | BamHI-NheI |
| | Reverse | CCCGCTCGAG-AAACAGCCATTTGAGCGA | XhoI |
| **ORF 37** | Forward | GCGGATCCCATATG-GATGACGTATCGGATTTT | BamHI-NdeI |
| | Reverse | CCCGCTCGAG-ATAGCCCGCTTTCAGG | XhoI |
| **ORF 58** | Forward | CGCGGATCCGCTAGC-TCCGAACGCGAGTGGAT | BamHI-NheI |
| | Reverse | CCCGCTCGAG-AGCATTGTCCAAGGGGAC | XhoI |
| **ORF 65** | Forward | GGAATTCCATATGGCCATGG -TGCTGTATCTGAATCAAG | NdeI-NcoI |
| | Forward | CGGGATCC-TTGCTGTATCTGAATCAAGG | BamHI |
| | Reverse | CCCGCTCGAG-CCGCATCGGCAGACA | XhoI |
| **ORF 66** | Forward | GCGGATCCCATATG-TACGCATTTACCGCCG | BamHI-NdeI |
| | Reverse | CCCGCTCGAG-TGGATTTTGCAGAGATGG | XhoI |
| **ORF 72** | Forward | CGCGGATCCCATATG- AATGCAGTAAAAATATCTGA | BamHI-NdeI |
| | Reverse | CCCGCTCGAG-GCCTGAGACCTTTGCAA | XhoI |
| **ORF 73** | Forward | GCGGATCCCATATG-AGATTTTTCGGTATCGG | BamHI-NdeI |
| | Reverse | CCCGCTCGAG-TTCATCTTTTTCATGTTCG | XhoI |
| **ORF 75** | Forward | GCGGATCCCATATG- TCTGTCTTTCAAACGGC | BamHI-NdeI |
| | Reverse | CCCGCTCGAG-TTTGTTTTTGCAAGACAG | XhoI |
| **ORF 76** | Forward | GATCAGCTAGCCATATG-AAACAGAAAAAAACCGC | NheI-NdeI |
| | Reverse | CGGGATCC-TTACGGTTTGACACCGTT | BamHI |
| **ORF 79** | Forward | CGCGGATCCCATATG-GTTTCCGCCGCCG | BamHI-NdeI |
| | Reverse | CCCGCTCGAG-GTGCTGATGCGCTTCG | XhoI |
| **ORF 83** | Forward | GCGGATCCCATATG-AAAACCCTGCTGCTGC | BamHI-NdeI |
| | Reverse | CCCGCTCGAG-GCCGCCTTTGCGGC | XhoI |
| **ORF 84** | Forward | GCGGATCCCATATG-GCAGAGATCTGTTTG | BamHI-NdeI |
| | Reverse | CCCGCTCGAG-GTTTGCCGATCCGACCA | XhoI |
| **ORF 85** | Forward | CGCGGATCCCATATG- GCGGTTTGGGGCGGA | BamHI-NdeI |
| | Reverse | CCCGCTCGAG-TCGGCGCGGCGGGC | XhoI |
| **ORF 89** | Forward | GGAATTCCATATGGCCATGG-CCATACCTTCTTATCA | NdeI-NcoI |
| | Forward | CGGGATCC-GCCATACCTTCTTATCAGAG | BamHI |
| | Reverse | CCCGCTCGAG-TTTTTTGCGATTAGAAAAAGC | XhoI |
| **ORF 97** | Forward | GCGGATCCCATATG-CATCCTGCCAGCGAAC | BamHI-NdeI |
| | Reverse | CCCGCTCGAG-TTCGCCTACGGTTTTTTG | XhoI |

(continued)

| ORF | Primer | Sequence | Restriction sites |
|---|---|---|---|
| **ORF 98** | Forward | GCGGATCCCATATG-ACGGTAACTGCGG | BamHI-NdeI |
| | Reverse | CCCGCTCGAG-TTGTTGTTCGGGCAAATC | XhoI |
| **ORF 100** | Forward | GCGGATCCCATATG-TCGGGCATTTACACCG | BamHI-NdeI |
| | Reverse | CCCGCTCGAG-ACGGGTTTCGGCGGAA | XhoI |
| **ORF 101** | Forward | GCGGATCCCATATG-ATTTATCAAAGAAACCTC | BamHI-NdeI |
| | Reverse | CCCGCTCGAG-TTTTCCGCCTTTCAATGT | XhoI |
| **ORF 102** | Forward | GCGGATCCCATATG-GCAGGGCTGTTTTACC | BamHI-NdeI |
| | Reverse | CCCGCTCGAG-AAACGGTTTGAACACGAC | XhoI |
| **ORF 103** | Forward | GCGGATCCCATATG-AACCACGACATCAC | BamHI-NdeI |
| | Reverse | CCCGCTCGAG-CAGCCACAGGACGGC | XhoI |
| **ORF 104** | Forward | GCGGATCCCATATG-ACGTGGGGAACGC | BamHI-NdeI |
| | Reverse | CCCGCTCGAG-GCGGCGTTTGAACGGC | XhoI |
| **ORF 105** | Forward | GCGGATCCCATATG-ACCAAATTTCAAACCCCTC | BamHI-NdeI |
| | Reverse | CCCGCTCGAG-TAAACGAATGCCGTCCAG | XhoI |
| **ORF 106** | Forward | GCGGATCCCATATG-AGGATAACCGACGGCG | BamHI-NdeI |
| | Reverse | CCCGCTCGAG-TTTGTTCCCGATGATGTT | XhoI |
| **ORF 109** | Forward | GCGGATCCCATATG-GAAGATTTATATATAATACTCG | BamHI-NdeI |
| | Reverse | CCCGCTCGAG-ATCAGCTTCGAACCGAAG | XhoI |
| **ORF110** | Forward | AAAGAATTC-ATGAGTAAATCCCGTAGATCTCCC | EcoRI |
| | Reverse | AAACTGCAG-GGAAAACCACATCCGCACTCTGCC | PStI |
| **ORF111** | Forward | AAAGAATTC-GCACCGCAAAAGGCAAAAACCGCA | EcoRI |
| | Reverse | AAACTGCAG-TCTGCGCGTTTTCGGGCAGGGTGG | PstI |
| **ORF113** | Forward | AAAGAATTC-ATGAACAAAACCCTCTATCGTGTGATTTTCAACCG | EcoRI |
| | Reverse | AAACTGCAG-TTACGAATGCCTGCTTGCTCGACCGTACTG | PstI |
| **ORF115** | Forward | AAAGAATTC-TTGCTTGTGCAAACAGAAAAAGACGG | EcoRI |
| | Reverse | AAAAAAGTCGAC-CTATTTTTTAGGGGC*T*TTTGC*T*TGTTTGAAAAGCCTGCC | SalI |
| **ORF119** | Forward | AAAGAATTC-TACAACATGTATCAGGAAAACCAATACCG | EcoRI |
| | Reverse | AAACTGCAG-TTATGAAAACAGGCGCAGGGCGGTTTTGCC | PstI |
| **ORF120** | Forward | AAAGAATTC-GCAAGGCTACCCCAATCCGCCGTG | EcoRI |
| | Reverse | AAACTGCAG-CGGTTTGGCTGCCTGGCCGTTGAT | PstI |
| **ORF121** | Forward | AAAGAATTC-GCCTTGGTCTGGCTGGTTTTCGC | EcoRI |
| | Reverse | AAACTGCAG-TCATCCGCCACCCCACCTCGGCCATCCATC | PstI |
| **ORF122** | Forward | AAAAAAGTCGAC-ATGTCTTACCGCGCAAGCAGTTCTCC | SalI |

(continued)

| ORF | Primer | Sequence | Restriction sites |
|---|---|---|---|
| | Reverse | AAACTGCAG-TCAGGAACACAAACGATGACGAATATCCGTATC | PstI |
| **ORF125** | Forward | AAAGAATTC-GCGCTGTTTTTTGCGGCGGCGTAT | EcoRI |
| | Reverse | AAACTGCAG-CGCCGTTTCAAGACGAAAAAGTCG | PstI |
| **ORF126** | Forward | AAAGAATTC-GCGGAAACGGTCGAAG | EcoRI |
| | Reverse | AAACTGCAG-TTAATCTTGTCTTCCGATATAC | PstI |
| **ORF127** | Forward | AAAGAATTC-ATGACTGATAATCGGGGGTTTACG | EcoRI |
| | Reverse | AAAAAAGTCGAC-CTTAAGTAACTTGCAGTCCTTATC | SalI |
| **ORF128** | Forward | AAAGAATTC-ATGCAAGCTGTCCGCTACAGGCC | EcoRI |
| | Reverse | AAACTGCAG-CTA*T*TGCAATGCGCCGCCGCGGGAATG*T*TGAGCAGGCG | PstI |
| **ORF129** | Forward | AAAGAATTC-ATGGATTTTCGTTTTGACATTATTTACGAATACCG | EcoRI |
| | Reverse | AAACTGCAG-TTATTTTTTGATGAAATTTTGGGGCGG | PstI |
| **ORF130** | Forward | AAAGAATTC-GCAGTACTTGCCATTCTCGGTGCG | EcoRI |
| | Reverse | AAACTGCAG-CTCCGGATCGTCTGTAAACGCATT | PstI |
| **ORF 131** | Forward | GCGGATCCCATATG-GAAATTCGGGCAATAAAAT | BamHI-NdeI |
| | Reverse | CCCGCTCGAG-CCAGCGGACGCGTTC | XhoI |
| **ORF 132** | Forward | GCGGATCCCATATG-AAAGAAGCGGGGTTTG | BamHI-NdeI |
| | Reverse | CCCGCTCGAG-CCAATCTGCCAGCCGT | XhoI |
| **ORF 133** | Forward | CGCGGATCCCATATG-GAAGATGCAGGGCGCG | BamHI-NdeI |
| | Reverse | CCCGCTCGAG-AAACTTGTAGCTCATCGT | XhoI |
| **ORF 134** | Forward | GCGGATCCCATATG-TCTGTGCAAGCAGTATTG | BamHI-NdeI |
| | Reverse | CCCGCTCGAG-ATCCTGTGCCAATGCG | XhoI |
| **ORF 135** | Forward | GCGGATCCCATATG-CCGTCTGAAAAAGCTTT | BamHI-NdeI |
| | Reverse | CCCGCTCGAG-AAATACCGCTGAGGATG | XhoI |
| **ORF 136** | Forward | CGCGGATCCGCTAGC-ATGAAGCGGCGTATAGCC | BamHI-NheI |
| | Reverse | CCCGCTCGAG-TTCCGAATATTTGGAACTTTT | XhoI |
| **ORF 137** | Forward | CGCGGATCCCATATG-GGCACGGCGGGAAATA | BamHI-NdeI |
| | Reverse | CCCGCTCGAG-ATAACGGTATGCCGCC | XhoI |
| **ORF 138** | Forward | GCGGATCCCATATG-TTTCGTTTACAATTCAGGC | BamHI-NdeI |
| | Reverse | CCCGCTCGAG-CGGCGTTTTATAGCGG | XhoI |
| **ORF 139** | Forward | GCGGATCCCATATG-GCTTTTTTGGCGGTAATG | BamHI-NdeI |
| | Reverse | CCCGCTCGAG-TAACGTTTCCGTGCGTTT | XhoI |
| **ORF 140** | Forward | GCGGATCCCATATG-TTGCCCACAGGCAGC | BamHI-NdeI |
| | Reverse | CCCGCTCGAG-GACGATGGCAAACAGC | XhoI |

(continued)

| ORF | Primer | Sequence | Restriction sites |
|---|---|---|---|
| **ORF 141** | Forward | GCGGATCCCATATG-CCGTCTGAAGCAGTCT | BamHI-NdeI |
| | Reverse | CCCGCTCGAG-ATCTGTTGTTTTTAAAATATT | XhoI |
| **ORF 142** | Forward | GCGGATCCCATATG-GATAATTCTGGTAGTGAAG | BamHI-NdeI |
| | Reverse | CCCGCTCGAG-AAACGTATAGCCTACCT | XhoI |
| **ORF 143** | Forward | GCGGATCCCATATG-GATACCGCTTTGAACCT | BamHI-NdeI |
| | Reverse | CCCGCTCGAG-AATGGCTTCCGCAATATG | XhoI |
| **ORF 144** | Forward | GCGGATCCCATATG-ACCTTTTTACAACGTTTGC | BamHI-NdeI |
| | Reverse | CCCGCTCGAG-AGATTGTTGTTGTTTTTTCG | XhoI |
| **ORF 147** | Forward | GCGGATCCCATATG-TCTGTCTTTCAAACGGC | BamHI-NdeI |
| | Reverse | CCCGCTCGAG-TTTGTTTTTGCAAGACAG | XhoI |

NB:
- restriction sites are underlined
- for ORFs 110-130, where the ORF itself carries an *EcoR*I site *(eg.* ORF122), a *Sal*I site was used in the forward primer instead. Similarly, where the ORF carries a *Pst*I site *(eg.* ORFs 115 and 127), a *Sal*I site was used in the reverse primer.

TABLE II - Summary of cloning, expression and purification

| ORF | PCR/cloning | His-fusion expression | GST-fusion expression | Purification |
|---|---|---|---|---|
| **orf 1** | + | + | + | His-fusion |
| **orf 2** | + | + | + | GST-fusion |
| **orf 2.1** | + | n.d. | + | GST-fusion |
| **orf 4** | + | + | + | His-fusion |
| **orf 5** | + | n.d. | + | GST-fusion |
| **orf 6** | + | + | + | GST-fusion |
| **orf 7** | + | + | + | GST-fusion |
| **orf 8** | + | n.d. | n.d. | |
| **orf 9** | + | + | + | GST-fusion |
| **orf 10** | + | n.d. | n.d. | |
| **orf 11** | + | n.d. | n.d. | |
| **orf 13** | + | n.d. | + | GST-fusion |
| **orf 15** | + | + | + | GST-fusion |
| **orf 17** | + | n.d. | n.d. | |
| **orf 18** | + | n.d. | n.d. | |
| **orf 19** | + | n.d. | n.d. | |
| **orf 20** | + | n.d. | n.d. | |
| **orf 22** | + | + | + | GST-fusion |
| **orf 23** | + | + | + | His-fusion |
| **orf 24** | + | n.d. | n.d. | |
| **orf 25** | + | + | + | His-fusion |
| **orf 26** | + | n.d. | n.d. | |
| **orf 27** | + | + | + | GST-fusion |
| **orf 28** | + | + | + | GST-fusion |
| **orf 29** | + | n.d. | n.d. | |
| **orf 32** | + | + | + | His-fusion |
| **orf 33** | + | n.d. | n.d. | |
| **orf 35** | + | n.d. | n.d. | |
| **orf 37** | + | + | + | GST-fusion |
| **orf 58** | + | n.d. | n.d. | |
| **orf 65** | + | n.d. | n.d. | |
| **orf 66** | + | n.d. | n.d. | |
| **orf 72** | + | + | n.d. | His-fusion |
| **orf 73** | + | n.d. | + | n.d. |
| **orf 75** | + | n.d. | n.d. | |
| **orf 76** | + | + | n.d. | His-fusion |
| **orf 79** | + | + | n.d. | His-fusion |

(continued)

| ORF | PCR/cloning | His-fusion expression | GST-fusion expression | Purification |
|---|---|---|---|---|
| **orf 83** | + | n.d. | + | n.d. |
| **orf 84** | + | n.d. | n.d. | |
| **orf 85** | + | n.d. | + | GST-fusion |
| **orf 89** | + | n.d. | + | GST-fusion |
| **orf 97** | + | + | + | GST-fusion |
| **orf** 98 | + | n.d. | n.d. | |
| **orf 100** | + | n.d. | n.d. | |
| **orf 101** | + | n.d. | n.d. | |
| **orf 102** | + | n.d. | n.d. | |
| **orf 103** | + | n.d. | n.d. | |
| **orf 104** | + | n.d. | n.d. | |
| **orf 105** | + | n.d. | n.d. | |
| **orf 106** | + | + | + | His-fusion |
| **orf 109** | + | n.d. | n.d. | |
| **orf 110** | + | n.d. | n.d. | |
| **orf 111** | + | + | n.d. | His-fusion |
| **orf 113** | + | + | n.d. | His-fusion |
| **orf 115** | n.d. | n.d. | n.d. | |
| **orf 119** | + | + | n.d. | His-fusion |
| **orf 120** | + | + | n.d. | His-fusion |
| **orf 121** | + | n.d. | n.d. | |
| **orf 122** | + | + | n.d. | His-fusion |
| **orf 125** | + | + | n.d. | His-fusion |
| **orf 126** | + | + | n.d. | His-fusion |
| **orf 127** | + | + | n.d. | His-fusion |
| **orf 128** | + | n.d. | n.d. | |
| **orf 129** | + | + | n.d. | His-fusion |
| **orf 130** | + | n.d. | n.d. | |
| **orf 131** | + | + | + | n.d. |
| **orf 132** | + | + | + | His-fusion |
| **orf 133** | + | n.d. | + | GST-fusion |
| **orf 134** | + | n.d. | n.d. | |
| **orf 135** | + | n.d. | n.d. | |
| **orf 136** | + | n.d. | n.d. | |
| **orf 137** | + | n.d. | + | GST-fusion |
| **orf 138** | + | n.d. | + | GST-fusion |
| **orf 139** | + | n.d. | n.d. | |
| **orf 140** | + | n.d. | n.d. | |

(continued)

| ORF | PCR/cloning | His-fusion expression | GST-fusion expression | Purification |
|---|---|---|---|---|
| **orf 141** | + | n.d. | n.d. | |
| **orf 142** | + | n.d. | n.d. | |
| **orf 143** | + | n.d. | n.d. | |
| **orf 144** | + | n.d. | + | n.d. |
| **orf 147** | + | n.d. | n.d. | |

## Claims

1. A protein comprising an amino acid sequence selected from the group consisting of SEQ IDs 2, 4, 6, and 8.

2. A nucleic acid molecule which encodes a protein according to claim 1.

3. A nucleic acid molecule according to claim 2, comprising a nucleotide sequence selected from the group consisting of SEQ IDs 1, 3, 5, and 7.

4. A protein comprising an amino acid sequence selected from the group consisting of SEQ IDs 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392, 394, 396, 398, 400, 402, 404, 406, 408, 410, 412, 414, 416, 418, 420, 422, 424, 426, 428, 430, 432, 434, 436, 438, 440, 442, 444, 446, 448, 450, 452, 454, 456, 458, 460, 462, 464, 466, 468, 470, 472, 474, 476, 478, 480, 482, 484, 486, 488, 490, 492, 494, 496, 498, 500, 502, 504, 506, 508, 510, 512, 514, 516, 518, 520, 522, 524, 526, 528, 530, 532, 534, 536, 538, 540, 542, 544, 546, 548, 550, 552, 554, 556, 558, 560, 562, 564, 566, 568, 570, 572, 574, 576, 578, 580, 582, 584, 586, 588, 590, 592, 594, 596, 598, 600, 602, 604, 606, 608, 610, 612, 614, 616, 618, 620, 622, 624, 626, 628, 630, 632, 634, 636, 638, 640, 642, 644, 646, 648, 650, 652, 654, 656, 658, 660, 662, 664, 666, 668, 670, 672, 674, 676, 678, 680, 682, 684, 686, 688, 690, 692, 694, 696, 698, 700, 702, 704, 706, 708, 710, 712, 714, 716, 718, 720, 722, 724, 726, 728, 730, 732, 734, 736, 738, 740, 742, 744, 746, 748, 750, 752, 754, 756, 758, 760, 762, 764, 766, 768, 770, 772, 774, 776, 778, 780, 782, 784, 786, 788, 790, 792, 794, 796, 798, 800, 802, 804, 806, 808, 810, 812, 814, 816, 818, 820, 822, 824, 826, 828, 830, 832, 834, 836, 838, 840, 842, 844, 846, 848, 850, 852, 854, 856, 858, 860, 862, 864, 866, 868, 870, 872, 874, 876, 878, 880, 882, 884, 886, 888, 890, & 892..

5. A protein having 50% or greater sequence identity to a protein according to claim 4.

6. A protein comprising a fragment of an amino acid sequence selected from the group consisting of SEQ IDs 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342. 344, 346, 348, 350, 352, 354, 356, 358, 360, 362, 364, 366, 368, 370, 372, 374, 376, 378, 380, 382, 384, 386, 388, 390, 392, 394, 396, 398, 400, 402, 404, 406, 408, 410, 412, 414, 416, 418, 420, 422, 424, 426, 428, 430, 432, 434, 436, 438, 440, 442, 444, 446, 448, 450, 452, 454, 456, 458, 460, 462, 464, 466, 468, 470, 472, 474, 476, 478, 480, 482, 484, 486, 488, 490, 492, 494, 496, 498, 500, 502, 504, 506, 508, 510, 512, 514, 516, 518, 520, 522, 524, 526, 528, 530, 532, 534, 536, 538, 540, 542, 544, 546, 548, 550, 552, 554, 556, 558, 560, 562, 564, 566, 568, 570, 572, 574, 576, 578, 580, 582, 584, 586, 588, 590, 592, 594, 596, 598, 600, 602, 604, 606, 608, 610, 612, 614, 616, 618, 620, 622, 624, 626, 628, 630, 632, 634, 636, 638, 640, 642, 644, 646, 648, 650, 652, 654, 656, 658, 660, 662, 664, 666, 668, 670, 672, 674, 676, 678, 680, 682, 684, 686, 688, 690, 692, 694, 696, 698, 700, 702, 704, 706, 708, 710, 712, 714, 716, 718, 720, 722, 724, 726, 728, 730, 732, 734, 736, 738, 740, 742, 744, 746, 748, 750, 752, 754, 756, 758, 760, 762, 764, 766, 768, 770, 772, 774, 776, 778, 780, 782, 784, 786, 788, 790, 792, 794, 796, 798, 800, 802, 804, 806, 808, 810, 812, 814, 816, 818, 820, 822, 824, 826, 828, 830, 832, 834, 836, 838, 840, 842, 844, 846, 848, 850, 852, 854, 856, 858, 860, 862, 864, 866, 868, 870, 872, 874, 876, 878, 880, 882, 884, 886, 888, 890, & 892..

**7.** An antibody which binds to a protein according to any one of claims 4 to 6.

**8.** A nucleic acid molecule which encodes a protein according to any one of claims 4 to 6.

**9.** A nucleic acid molecule according to claim 8, comprising a nucleotide sequence selected from the group consisting of SEQ IDs 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89,91,93,95,97,99,101,103, 105,107,109,111, 113,115,117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391, 393, 395, 397, 399, 401, 403, 405, 407, 409, 411, 413, 415, 417, 419, 421, 423, 425, 427, 429, 431, 433, 435, 437, 439, 441, 443, 445, 447, 449, 451, 453, 455, 457, 459, 461, 463, 465, 467, 469, 471, 473, 475, 477, 479, 481, 483, 485, 487, 489, 491, 493, 495, 497, 499, 501, 503, 505, 507, 509, 511, 513, 515, 517, 519, 521, 523, 525, 527, 529, 531, 533, 535, 537, 539, 541, 543, 545, 547, 549, 551, 553, 555, 557, 559, 561, 563, 565, 567, 569, 571, 573, 575, 577, 579, 581, 583, 585, 587, 589, 591, 593, 595, 597, 599, 601, 603, 605, 607, 609, 611, 613, 615, 617, 619, 621, 623, 625, 627, 629, 631, 633, 635, 637, 639, 641, 643, 645, 647, 649, 651, 653, 655, 657, 659, 661, 663, 665, 667, 669, 671, 673, 675, 677, 679, 681, 683, 685, 687, 689, 691, 693, 695, 697, 699, 701, 703, 705, 707, 709, 711, 713, 715, 717, 719, 721, 723, 725, 727, 729, 731, 733, 735, 737, 739, 741, 743, 745, 747, 749, 751, 753, 755, 757, 759, 761, 763, 765, 767, 769, 771, 773, 775, 777, 779, 781, 783, 785, 787, 789, 791, 793, 795, 797, 799, 801, 803, 805, 807, 809, 811, 813, 815, 817, 819, 821, 823, 825, 827, 829, 831, 833, 835, 837, 839, 841, 843, 845, 847, 849, 851, 853, 855, 857, 859, 861, 863, 865, 867, 869, 871, 873, 875, 877, 879, 881, 883, 885, 887, 889, & 891..

**10.** A nucleic acid molecule comprising a fragment of a nucleotide sequence selected from the group consisting of SEQ IDs l, 3, 5, 7, 9, 1 l, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295,297,299,301,303,305,307,309,311,313,315,317,319,321,323,325,327,329,331,333, 335,337,339,341,343,345,347,349,351,353,355,357,359,361,363,365,367,369,371,373, 375,377,379,381,383,385,387,389,391,393,395,397,399,401,403,405,407,409,411,413, 415,417,419,421,423,425,427,429,431,433,435,437,439,441,443,445,447,449,451,453, 455,457,459,461,463,465,467,469,471,473,475,477,479,481,483,485,487,489,491,493, 495,497,499,501,503,505,507,509,511,513,515,517,519,521,523,525,527,529,531,533, 535, 537, 539, 541, 543, 545, 547, 549, 551, 553, 555, 557, 559, 561, 563, 565, 567, 569, 571, 573, 575, 577, 579, 581, 583, 585, 587, 589, 591, 593, 595, 597, 599, 601, 603, 605, 607, 609, 611, 613, 615,617,619,621,623,625,627,629,631,633,635,637,639,641,643,645,647,649,651,653, 655, 657, 659, 661, 663, 665, 667, 669, 671, 673, 675, 677, 679, 681, 683, 685, 687, 689, 691, 693, 695,697,699,701,703,705,707,709,711,713,715,717,719,721,723,725,727,729,731,733, 735,737,739,741,743,745,747,749,751,753,755,757,759,761,763,765,767,769,771,773, 775,777,779,781,783,785,787,789,791,793,795,797,799,801,803,805,807,809,811,813, 815, 817, 819, 821, 823, 825, 827, 829, 831, 833, 835, 837, 839, 841, 843, 845, 847, 849, 851, 853, 855, 857, 859, 861, 863, 865, 867, 869, 871, 873, 875, 877, 879, 881, 883, 885, 887, 889, & 891.

**11.** A nucleic acid molecule comprising a nucleotide sequence complementary to a nucleic acid molecule according to any one of claims 8 to 10.

**12.** A nucleic acid molecule comprising a nucleotide sequences having 50% or greater sequence identity to a nucleic acid molecule according to any one of claims 8-11.

**13.** A nucleic acid molecule which can hybridise to a nucleic acid molecule according to any one of claims 8-12 under high stringency conditions.

**14.** A composition comprising a protein, a nucleic acid molecule, or an antibody according to any preceding claim.

**15.** A composition according to claim 14 being a vaccine composition or a diagnostic composition.

**16.** A composition according to claim 14 or claim 15 for use as a pharmaceutical.

**17.** The use of a composition according to claim 14 in the manufacture of a medicament for the treatment or prevention of infection due to Neisserial bacteria.

**FIGURE 1**

FIG. 1A

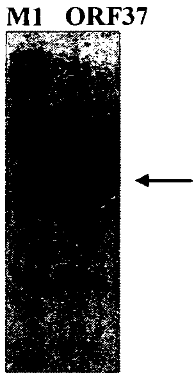


FIG. 1B

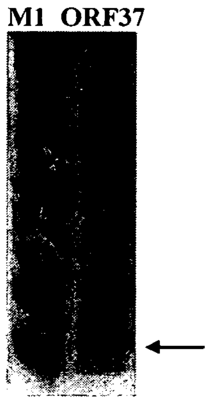


FIG. 1C

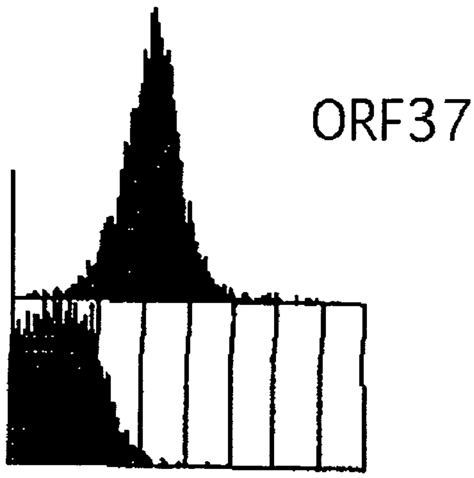


FIG. 1D

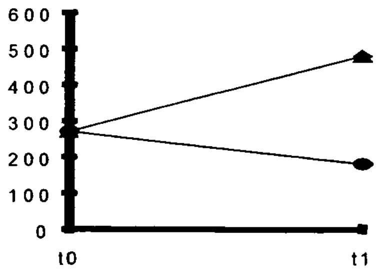

FIG 1E

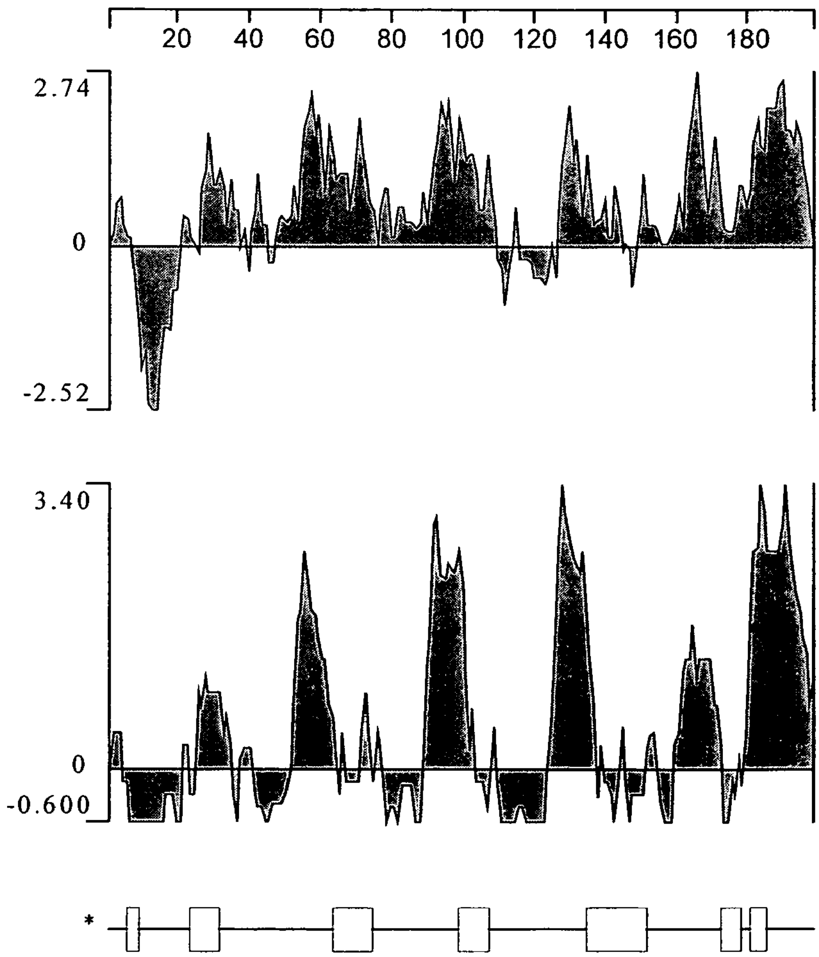

# FIGURE 2

*FIG. 2A*

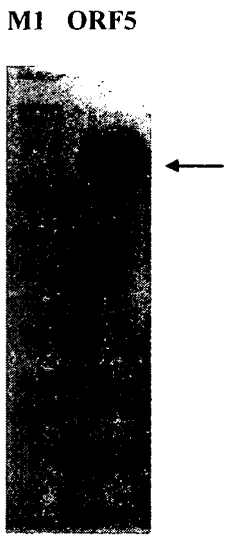


*FIG. 2B*

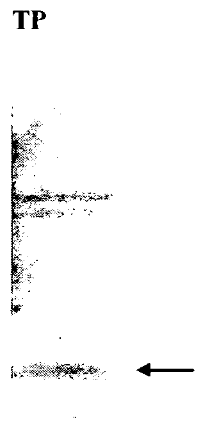

# FIGURE 3

*FIG. 3A*

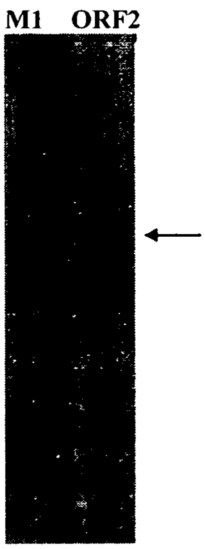


*FIG. 3B*

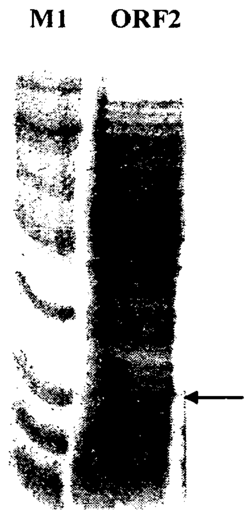


*FIG. 3C*

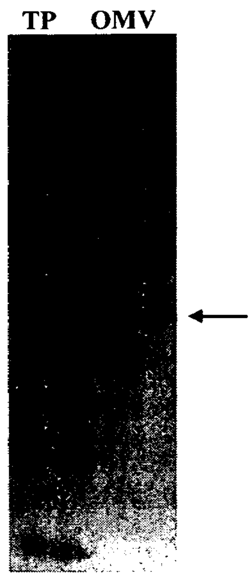


*FIG. 3D*

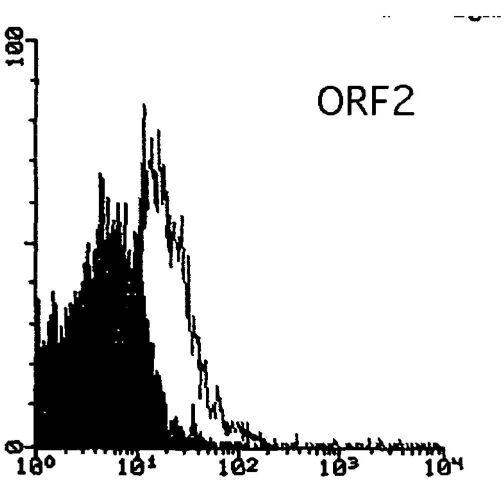

# FIGURE 4

**FIG. 4A**

M1 ORF15

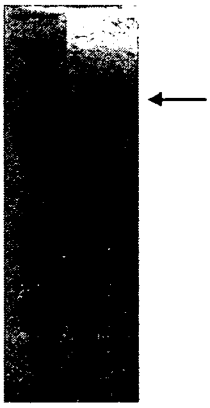

**FIG. 4B**

M2 ORF15

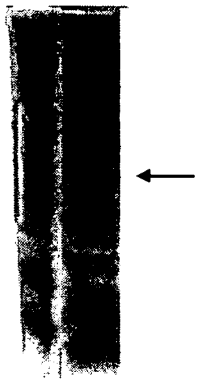

**FIG 4C**

TP OMV

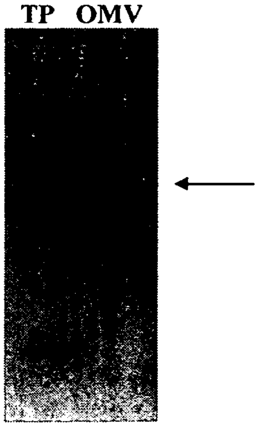

## FIGURE 5

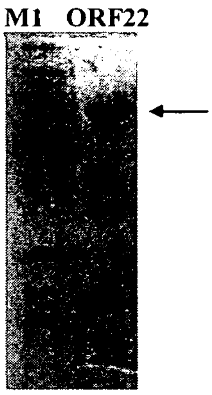


FIG. 5A

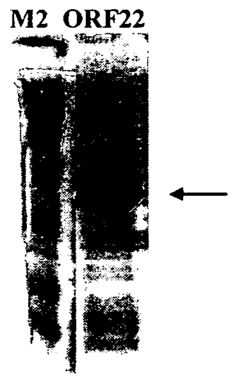


FIG. 5B

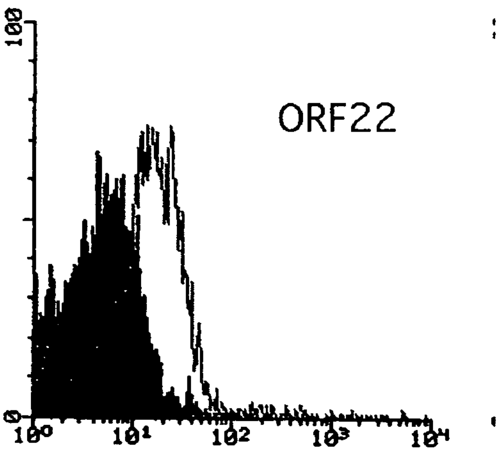


FIG. 5C

# FIGURE 6

*FIG. 6A*

M1 ORF28

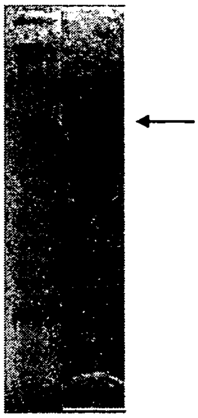

*FIG. 6B*

M2 ORF28

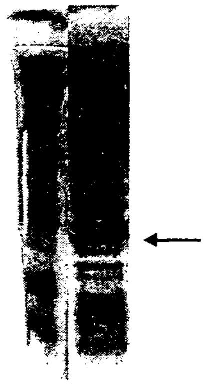

# FIGURE 7

*FIG. 7A*

M1 ORF32

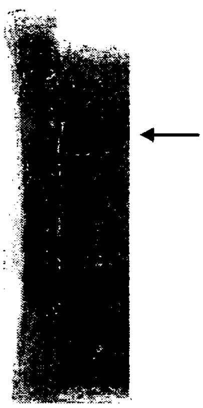

*FIG. 7B*

M1 ORF32

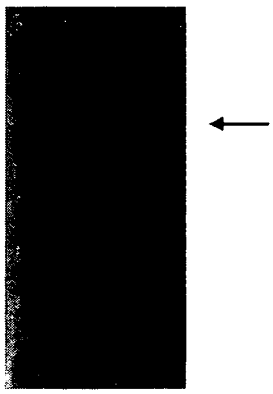

## *FIGURE 8*

*FIG. 8A*

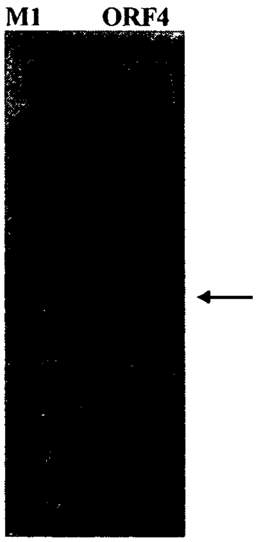


*FIG. 8B*

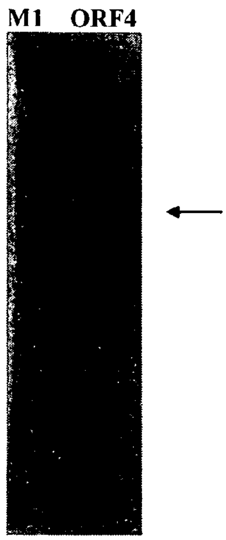

FIG. 8C

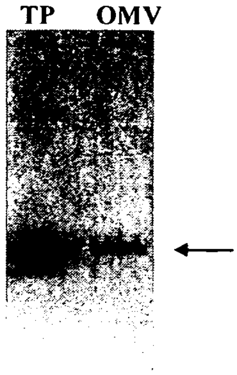


FIG. 8D

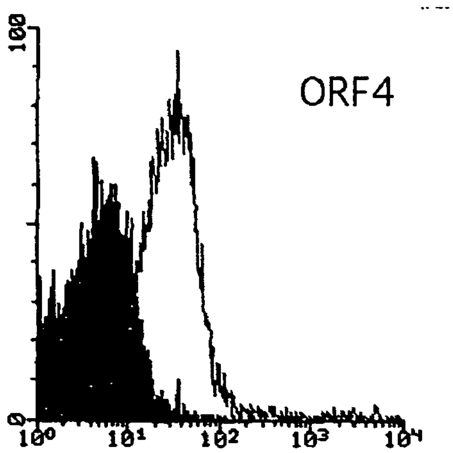

*FIG. 8E*

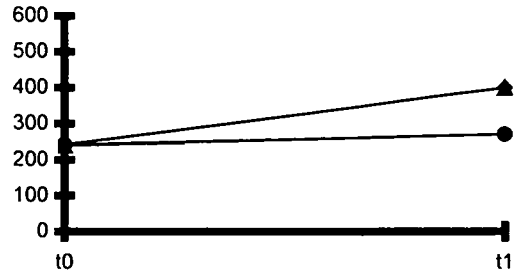
600
500
400
300
200
100
0
t0
t1

*FIG. 8F*

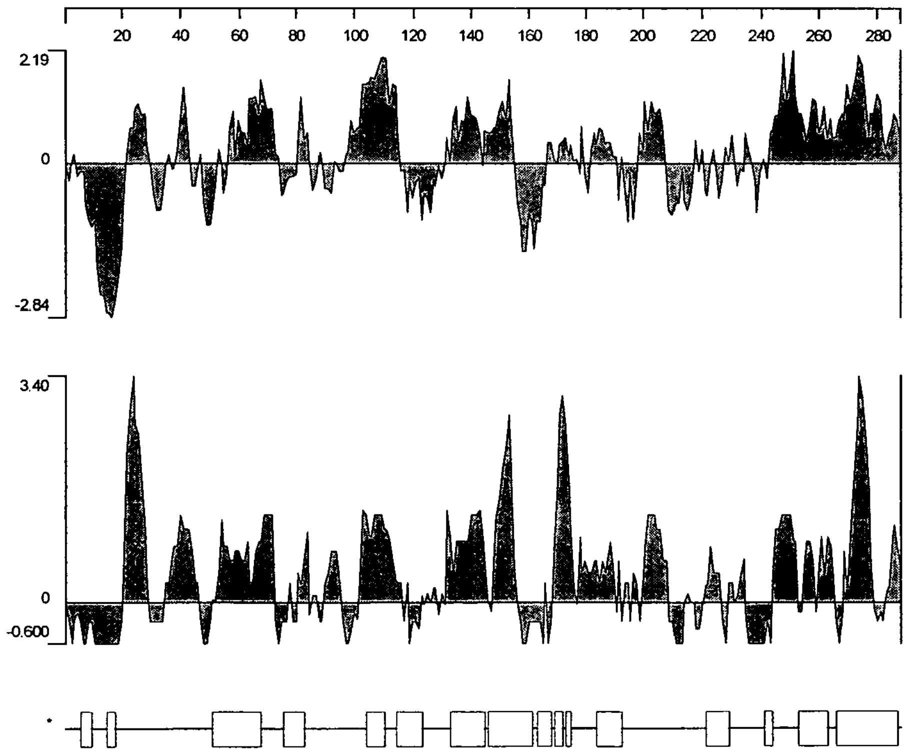
20
40
60
80
100
120
140
160
180
200
220
240
260
280
2.19
0
-2.84
3.40
0
-0.600

## *FIGURE 9*

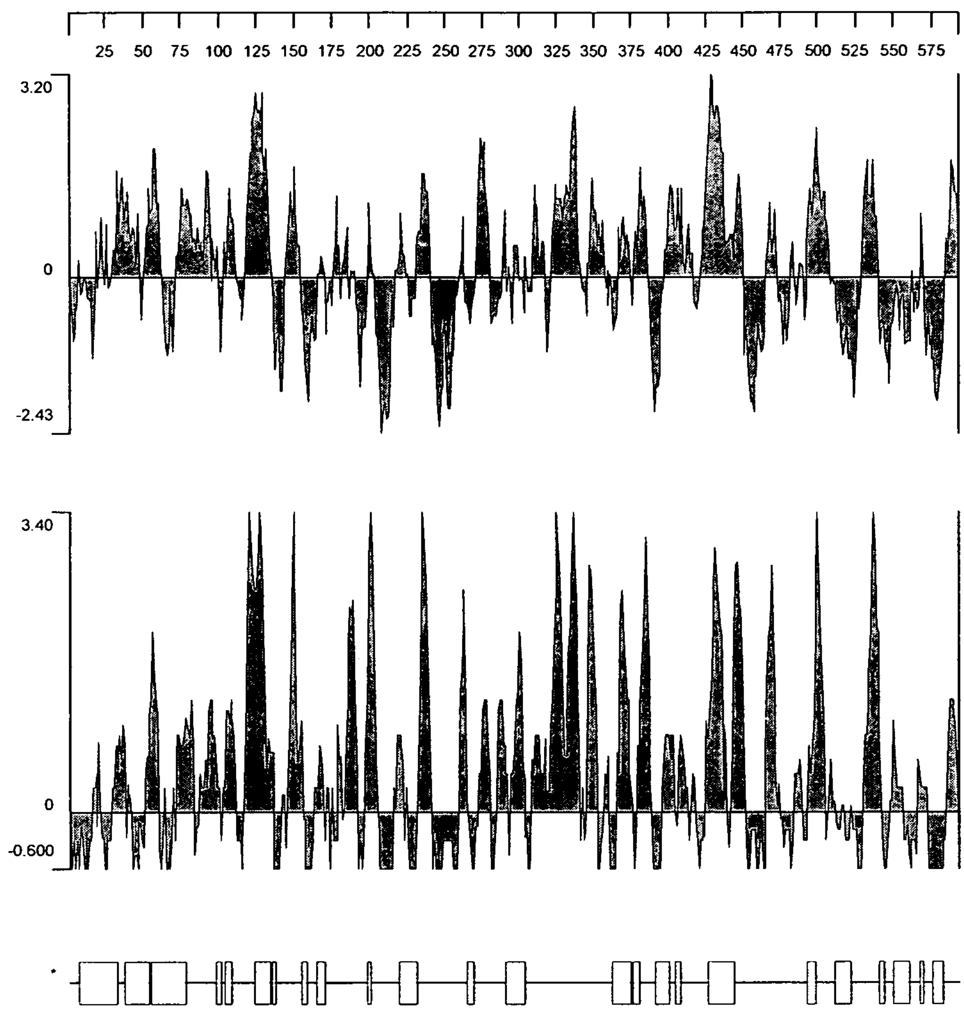
25 50 75 100 125 150 175 200 225 250 275 300 325 350 375 400 425 450 475 500 525 550 575
3.20
0
-2.43
3.40
0
-0.600

## FIGURE 10

**FIG. 10A**

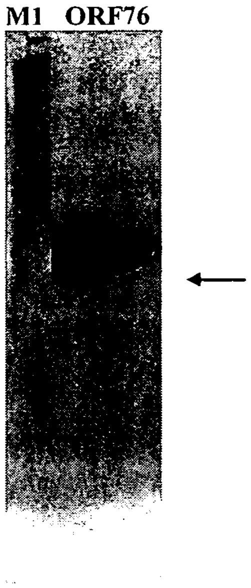


**FIG. 10B**

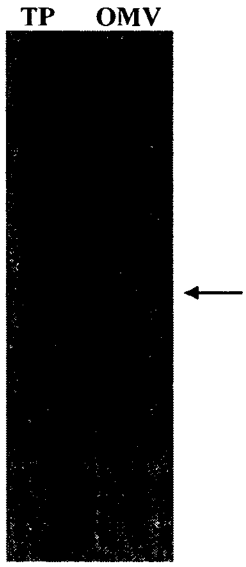


**FIG. 10C**

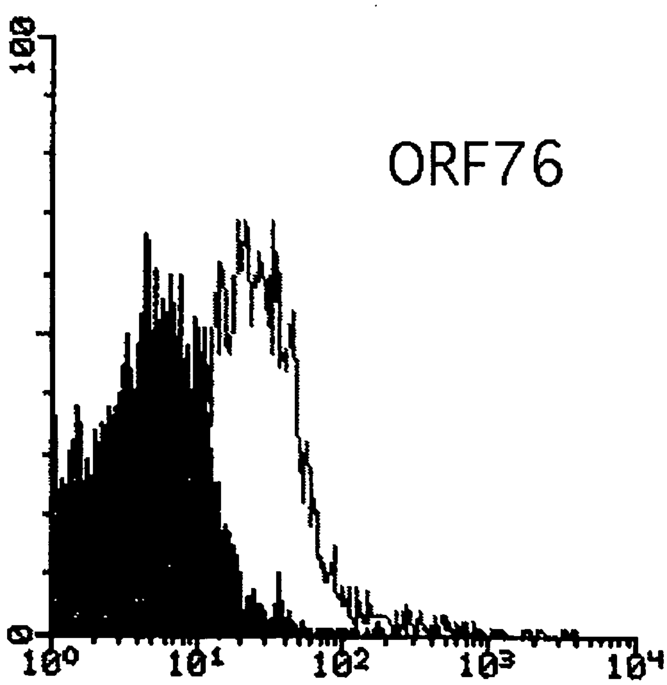

## FIGURE 11

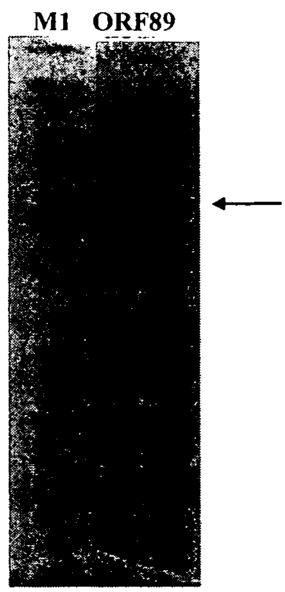

# FIGURE 12

*FIG. 12A*

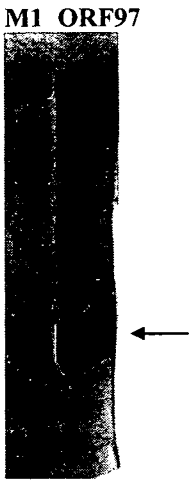


*FIG. 12B*

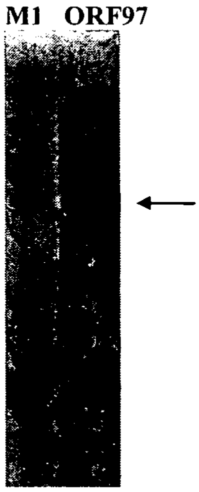


*FIG. 12C*

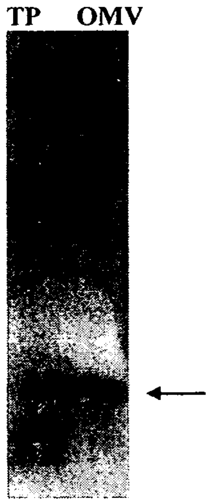


*FIG. 12D*

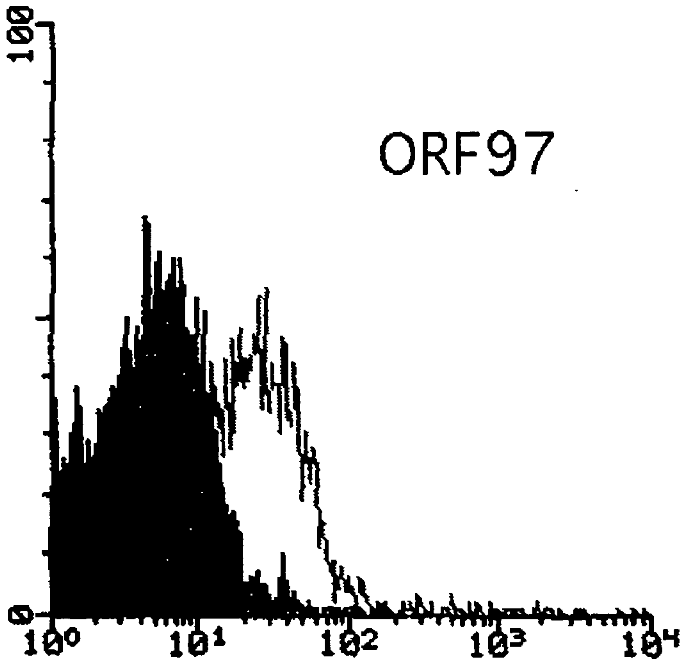

*FIG. 12E*

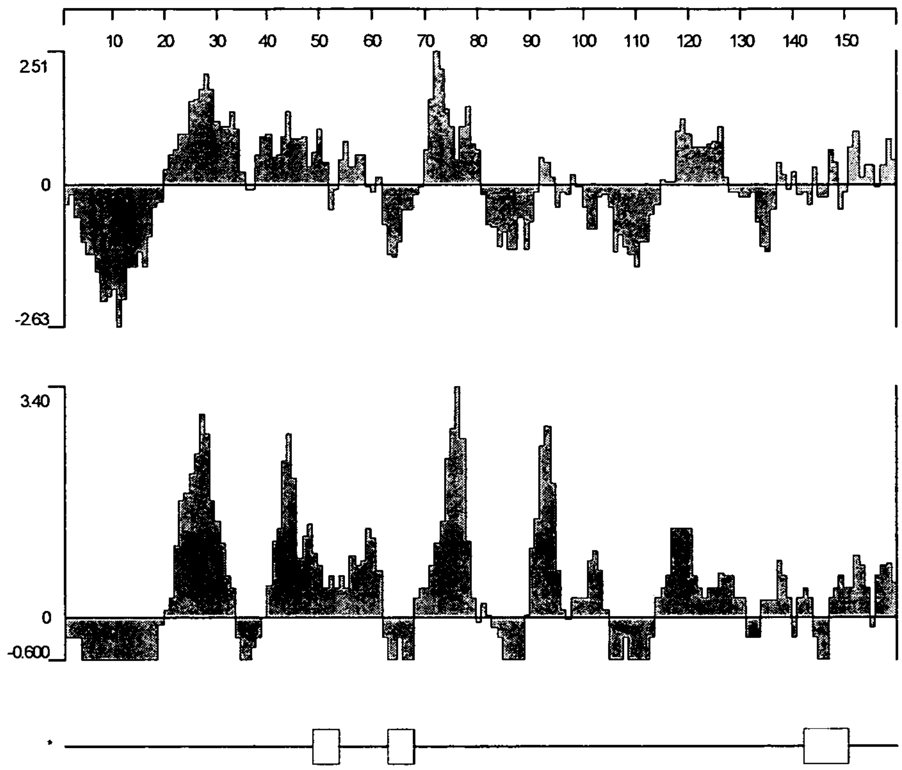
10
20
30
40
50
60
70
80
90
100
110
120
130
140
150
2.51
0
-2.63
3.40
0
-0.600

# FIGURE 13

FIG. 13A

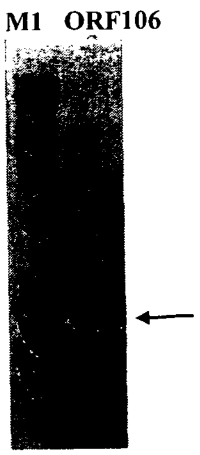


FIG. 13B

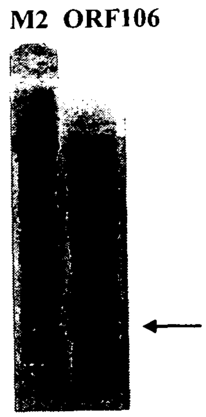


FIG. 13C

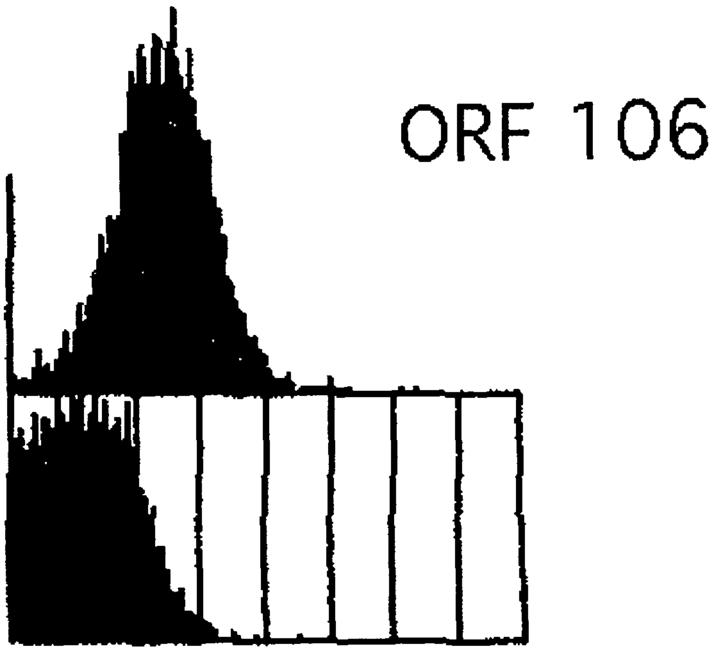

# FIGURE 14

FIG. 14A

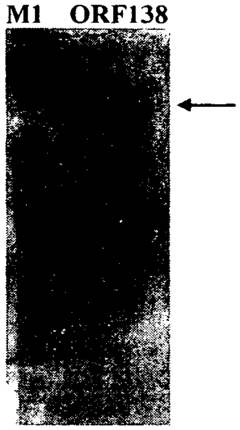


FIG. 14B

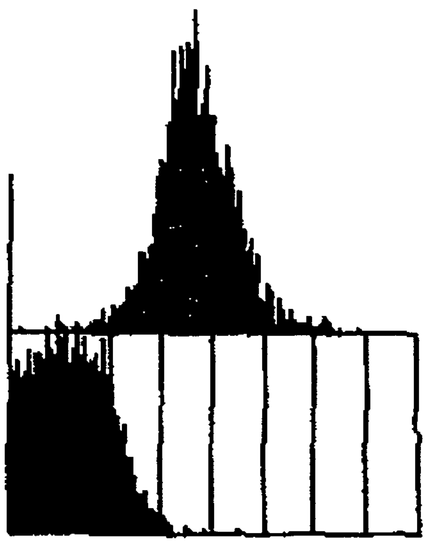

## FIGURE 15

***FIG. 15A***

M1 ORF23

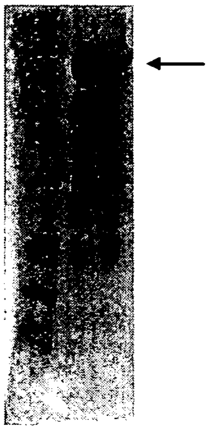

***FIG. 15B***

M2 ORF23

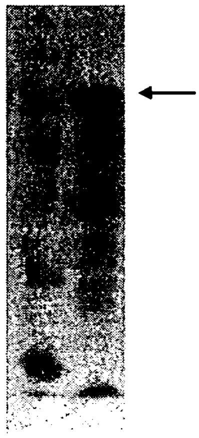

***FIG 15C***

TP OMV

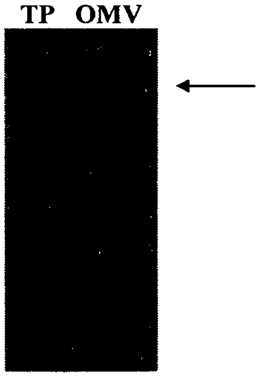

## FIGURE 16

FIG. 16A

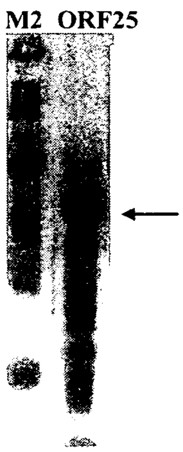


FIG. 16B

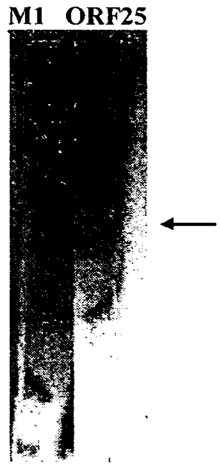


FIG. 16C

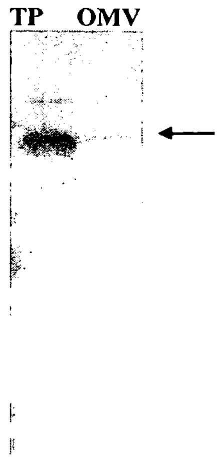


FIG. 16D

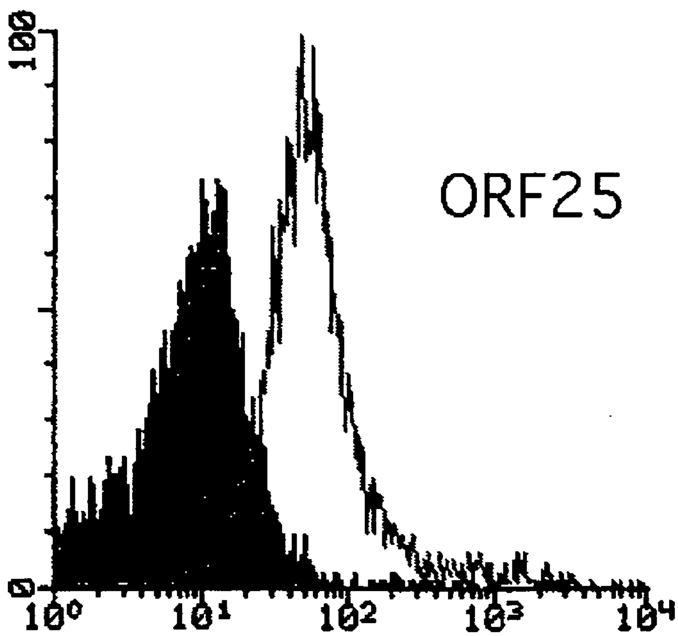

*FIG. 16E*

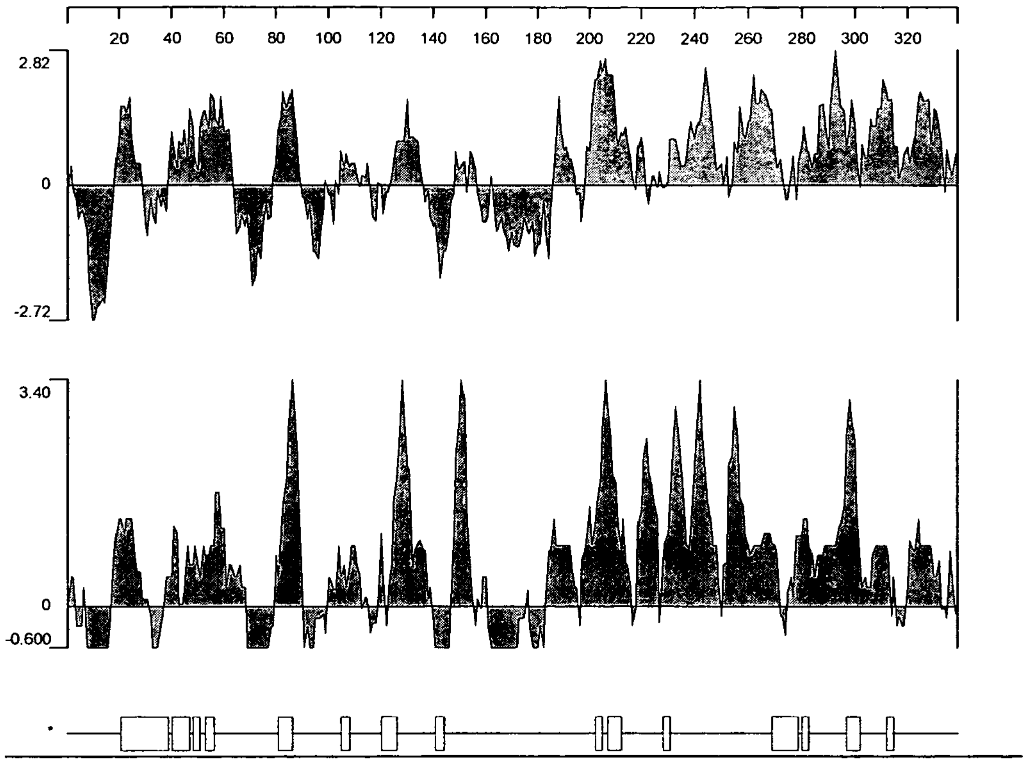
20
40
60
80
100
120
140
160
180
200
220
240
260
280
300
320
2.82
0
-2.72
3.40
0
-0.600

***FIGURE 17***

***FIG. 17A***

M1 ORF27

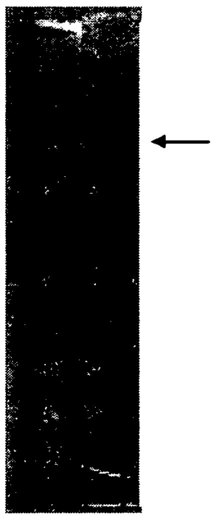

***FIG. 17B***

M2 ORF27

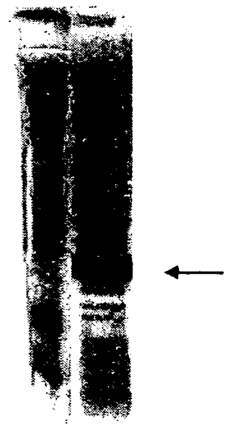

# FIGURE 18

FIG. 18A

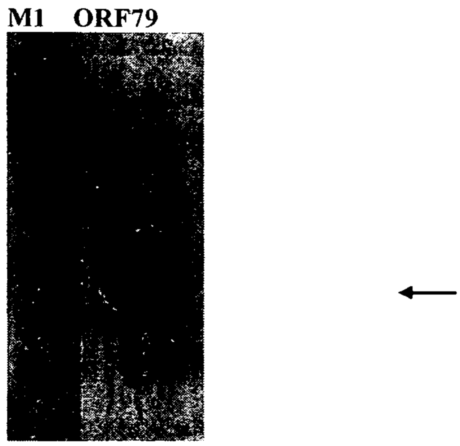


FIG. 18B

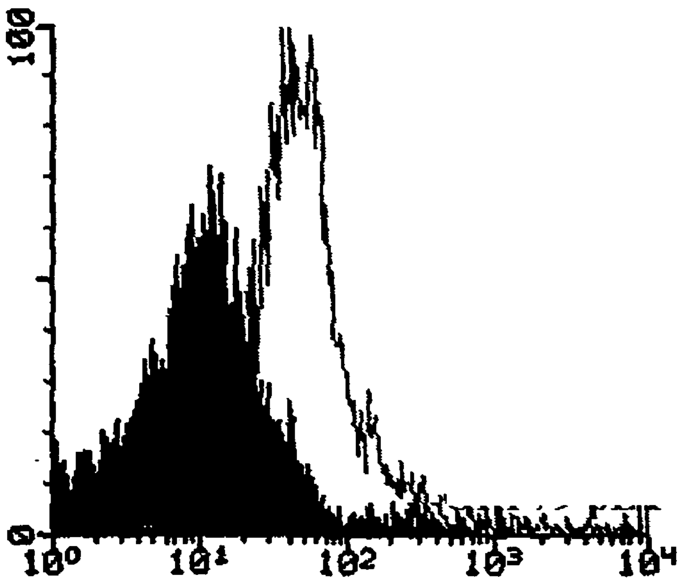

# FIGURE 19

*FIG. 19A*

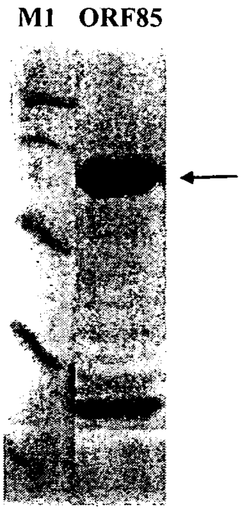


*FIG. 19B*

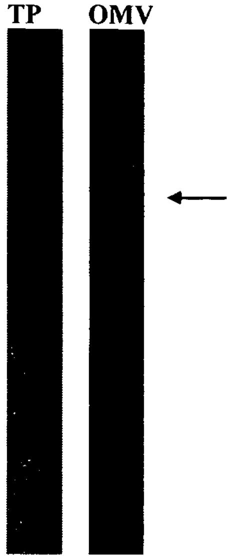


*FIG. 19C*

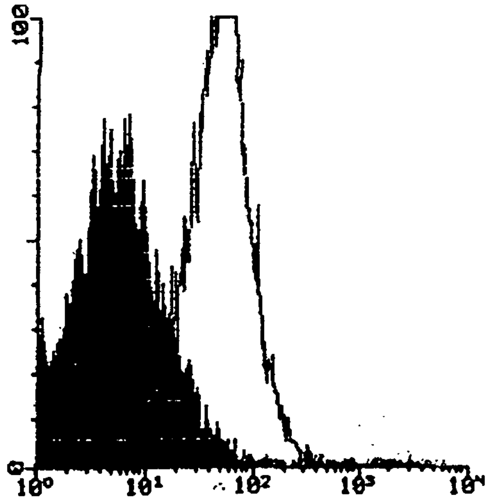

FIG 19D

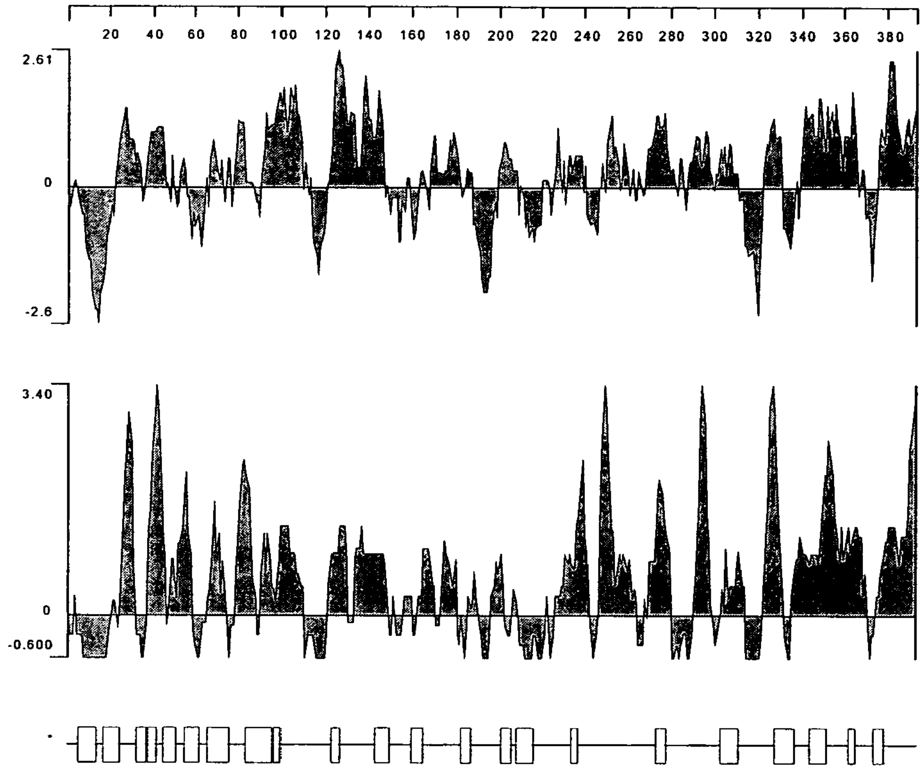
20
40
60
80
100
120
140
160
180
200
220
240
260
280
300
320
340
360
380
2.61
0
-2.6
3.40
0
-0.600

# FIGURE 20

FIG. 20A

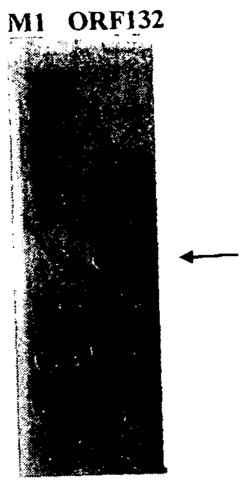


FIG. 20B

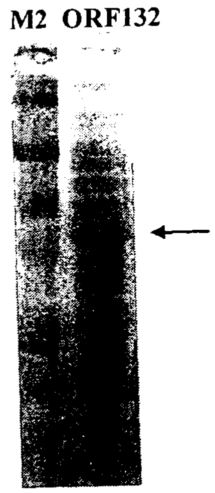


FIG. 20C

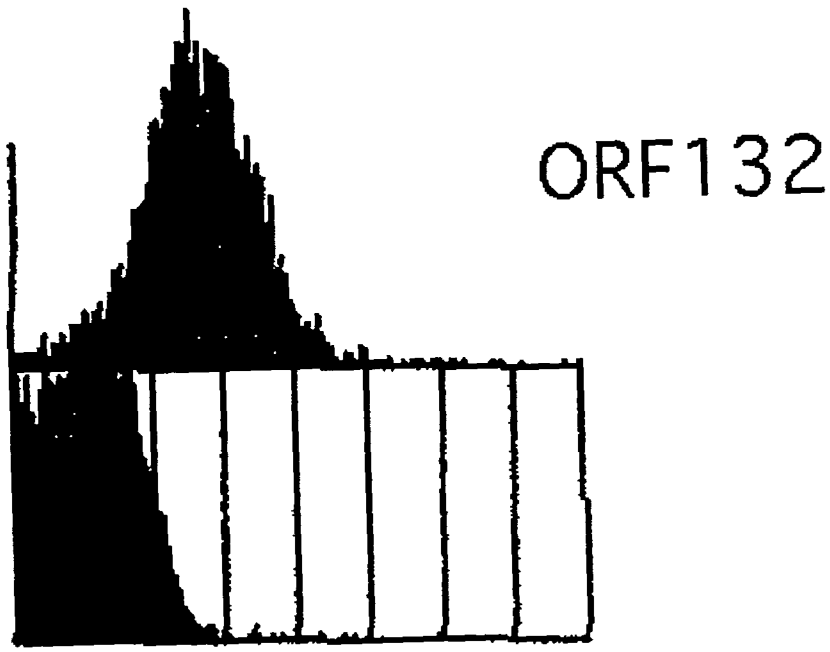

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0467714 A
- WO 9629412 A
- GB 9723516 A
- GB 9724190 A
- GB 9724386 A
- GB 9725158 A
- GB 9726147 A
- GB 9800759 A
- GB 9819016 A
- US 5753235 A
- EP 127839 A
- EP 155476 A
- WO 89046699 A
- US 5693506 A
- US 5659122 A
- US 5608143 A
- US 4738921 A
- EP 0036776 A
- EP 0121775 A
- US 4689406 A
- US 4551433 A
- EP 0267851 A
- EP 0219237 A
- EP 0324647 A
- US 4336336 A
- EP 0244042 A
- EP 0127328 A
- EP 0036259 A
- EP 0063953 A
- WO 8404541 A
- EP 0136829 A
- EP 0136907 A
- US 4745056 A
- EP 0284044 A
- EP 0329203 A
- US 4876197 A
- US 4880734 A
- EP 0164556 A
- EP 0196056 A
- WO 88024066 A
- EP 0012873 A
- JP 62096086 B
- US 4588684 A
- EP 0060057 A
- US 4546083 A
- US 4870008 A
- EP 0324274 A
- WO 8902463 A
- US 4837148 A
- US 4929555 A
- WO 9820734 A
- WO 9014837 A
- US 5591624 A
- WO 9637626 A
- WO 9530763 A
- WO 9205266 A
- GB 2200651 A
- EP 0415731 A
- EP 0345242 A
- EP 0334301 A
- WO 8902468 A
- WO 8905349 A
- WO 8909271 A
- WO 9002806 A
- WO 9007936 A
- WO 9403622 A
- WO 9325698 A
- WO 9325234 A
- WO 9311230 A
- WO 9310218 A
- WO 9102805 A
- WO 9102825 A
- WO 9507994 A
- US 5219740 A
- US 4405712 A
- US 4861719 A
- US 4980289 A
- US 4777127 A
- WO 9307283 A
- WO 9306223 A
- WO 9307282 A
- WO 9412649 A
- WO 9303769 A
- WO 9319191 A
- WO 9428938 A
- WO 9511984 A
- WO 9500655 A
- WO 9527071 A
- WO 9529993 A
- WO 9534671 A
- WO 9605320 A
- WO 9408026 A
- WO 9411506 A
- WO 9424299 A
- WO 9514102 A
- WO 9524297 A
- WO 9502697 A
- WO 9428152 A

- WO 9509241 A
- WO 9525807 A
- WO 9505835 A
- WO 9418922 A
- WO 9509654 A
- WO 9309239 A
- US 5478745 A
- US 4797368 A, Carter
- US 5139941 A, Muzyczka
- US 5474935 A, Chartejee
- WO 94288157 A, Kotin
- US 5354678 A
- US 5173414 A
- US 5252479 A
- US 5288641 A
- EP 0176170 A, Roizman
- WO 9504139 A
- WO 9009441 A
- WO 9207945 A
- EP 0453242 A, Breakefield
- US 5091309 A
- US 5217879 A
- WO 9210578 A
- US 08405627 B
- WO 9421792 A
- US 679640 A
- US 4603112 A
- US 4769330 A
- WO 8901973 A
- US 5166057 A
- EP 0386882 A
- EP 0440219 A
- US 08366787 B
- US 08240030 B
- US 08404796 B
- US 5149655 A
- US 5206152 A
- WO 9211033 A
- US 60023867 B
- WO 9011092 A
- US 5580859 A
- US 5422120 A
- WO 9513796 A
- WO 9423697 A
- WO 9114445 A
- EP 524968 A
- US 023867 P
- US 4762915 A
- EP 0524968 A
- WO 9314778 A
- US 9714465 W, Zuckermann
- US 4683195 A
- US 4683202 A

**Non-patent literature cited in the description**

- Vaccines Against Gonococcal Infection. **MEITZNER ; COHEN.** New Generation Vaccines. Marcel Dekker, 1997, 817-842
- **LIEBERMAN et al.** Safety and Immunogenicity of a Serogroups A/C Neisseria meningitidis Oligosaccharide-Protein Conjugate Vaccine in Young Children. *JAMA,* 1996, vol. 275 (19), 1499-1503
- **SCHUCHAT et al.** Bacterial Meningitis in the United States in 1995. *N Engl J Med,* 1997, vol. 337 (14), 970-976
- *Morbidity and Mortality weekly report,* 1997, vol. 46 (RR-5
- **ZOLLINGER WD.** New and Improved Vaccines Against Meningococcal Disease. *New Generation Vaccines,* 469-488
- **COSTANTINO et al.** Development and phase I clinical testing of a conjugate vaccine against meningococcus A and C. *Vaccine,* 1992, vol. 10, 691-698
- **ROMERO ; OUTSCHOORN.** Current status of Meningococcal group B vaccine candidates: capsular or non-capsular. *Clin Microbiol Rev,* 1994, vol. 7 (4), 559-575
- **POOLMAN JT.** Development of a meningococcal vaccine. *Infect. Agents Dis.,* 1992, vol. 4, 13-28
- **ALA'ALDEEN ; BORRIELLO.** The meningococcal transferrin-binding proteins 1 and 2 are both surface exposed and generate bactericidal antibodies capable of killing homologous and heterologous strains. *Vaccine,* 1996, vol. 14 (1), 49-53
- **SAMBROOK.** Molecular Cloning; A Laboratory Manual. 1989
- DNA Cloning. 1985, vol. I and ii
- Oligonucleotide Synthesis. 1984
- Nucleic Acid Hybridization. 1984
- Transcription and Translation. 1984
- Animal Cell Culture. 1986
- Immobilized Cells and Enzymes. IRL Press, 1986
- **B. PERBAL.** A Practical Guide to Molecular Cloning. 1984
- Methods in Enzymology. Academic Press, Inc, vol. 154 & 15
- Gene Transfer Vectors for Mammalian Cells. Cold Spring Harbor Laboratory, 1987
- Immunochemical Methods in Cell and Molecular Biology. Academic Press
- **SCOPES.** Protein Purification: Principles and Practice. Springer-Verlag, 1987
- Handbook of Experimental Immunology. 1986, vol. I-IV

- Expression of Cloned Genes in Mammalian Cells. **SAMBROOK et al.** Molecular Cloning: A Laboratory Manual. 1989
- **MANIATIS et al.** *Science,* 1987, vol. 236, 1237
- **ALBERTS et al.** Molecular Biology of the Cell. 1989
- **DIJKEMA et al.** *EMBO J.,* 1985, vol. 4, 761
- **GORMAN et al.** *Proc. Natl. Acad Sci.,* 1982, vol. 79, 6777
- **BOSHART et al.** *Cell,* 1985, vol. 41, 521
- **SASSONE-CORSI ; BORELLI.** *Trends Genet.,* 1986, vol. 2, 215
- **BIRNSTIEL et al.** *Cell,* 1985, vol. 41, 349
- Termination and 3' end processing of eukaryotic RNA. **PROUDFOOT ; WHITELAW.** Transcription and splicing. 1988
- **PROUDFOOT.** *Trends Biochem. Sci.,* 1989, vol. 14, 105
- Expression of cloned genes in cultured mammalian cells. **SAMBROOK et al.** Molecular Cloning: A Laboratory Manual. 1989
- **GLUZMAN.** *Cell,* 1981, vol. 23, 175
- **KAUFMAN et al.** *Mol. Cell. Biol.,* 1989, vol. 9, 946
- **SHIMIZU et al.** *Mol. Cell. Biol.,* 1986, vol. 6, 1074
- **SUMMERS ; SMITH.** Texas Agricultural Experiment Station Bulletin No. 1555. 1987
- **LUCKOW ; SUMMERS.** *virology,* 1989, vol. 17, 31
- **MILLER et al.** *Ann. Rev. Microbiol.,* 1988, vol. 42, 177
- The Regulation of Baculovirus Gene Expression. **FRIESEN et al.** The Molecular Biology of Baculoviruses. 1986
- **VLAK et al.** *J Gen. Virol.,* 1988, vol. 69, 765
- **CARBONELL et al.** *Gene,* 1988, vol. 73, 409
- **MAEDA et al.** *Nature,* 1985, vol. 315, 592
- **LEBACQ-VERHEYDEN et al.** *Molec. Cell. Biol.,* 1988, vol. 8, 3129
- **SMITH et al.** *Proc. Nat'l Acad. Sci. USA,* 1985, vol. 82, 8404
- **MIYAJIMA et al.** *Gene,* 1987, vol. 58, 273
- **MARTIN et al.** *DNA,* 1988, vol. 7, 99
- **SMITH et al.** *Mol. Cell. Biol.,* 1983, vol. 3, 2156
- **MILLER et al.** *Bioessays,* 1989, vol. 4, 91
- Current Protocols in Microbiology. 1990, vol. 2
- **CARBONELL et al.** *J Virol.,* 1985, vol. 56, 153
- **WRIGHT.** *Nature,* 1986, vol. 321, 718
- **FRASER et al.** *In Vitro Cell. Dev. Biol.,* 1989, vol. 25, 225
- **ZENK.** *Phytochemistry,* 1991, vol. 30, 3861-3863
- **VAULCOMBE et al.** *Mol. Gen. Genet.,* 1987, vol. 209, 33-40
- **CHANDLER et al.** *Plant Molecular Biology,* 1984, vol. 3, 407-418
- **ROGERS.** *J Biol. Chem.,* 1985, vol. 260, 3731-3738
- **ROTHSTEIN et al.** *Gene,* 1987, vol. 55, 353-356
- **WHITTIER et al.** *Nucleic Acids Research,* 1987, vol. 15, 2515-2535
- **WIRSEL et al.** *Molecular Microbiology,* 1989, vol. 3, 3-14
- **YU et al.** *Gene,* 1992, vol. 122, 247-253
- Gibberellins. **R.L. JONES ; J. MACMILLIN.** Advanced Plant Physiology. Pitman Publishing Limited, 1984, 21-52
- **SHEEN.** *Plant Cell,* 1990, vol. 2, 1027-1038
- **MAAS et al.** *EMBOJ,* 1990, vol. 9, 3447-3452
- **BENKEL ; HICKEY.** *Proc. Natl. Acad Sci.,* 1987, vol. 84, 1337-1339
- **WILMINK ; DONS.** *Plant Mol. Biol. Reptr,* 1993, vol. 11 (2), 165-185
- **REED ; MANIATIS.** *Cell,* 1985, vol. 41, 95-105
- **CROSSWAY.** *Mol. Gen. Genet,* 1985, vol. 202, 179-185
- **KRENS et al.** *Nature,* 1982, vol. 296, 72-74
- **KLEIN et al.** *Nature,* 1987, vol. 327, 70-73
- **KNUDSEN ; MULLER.** *Planta,* 1991, vol. 185, 330-336
- **FRALEY et al.** *Proc. Natl. Acad. Sci. USA,* 1982, vol. 79, 1859-1863
- **FROMM et al.** *Proc. Natl Acad. Sci. USA,* 1985, vol. 82, 5824
- **RAIBAUD et al.** *Annu. Rev. Genet.,* 1984, vol. 18, 173
- **CHANG et al.** *Nature,* 1977, vol. 198, 1056
- **GOEDDEL et al.** *Nuc. Acids Res.,* 1980, vol. 8, 4057
- **YELVERTON et al.** *Nucl. Acids Res.,* 1981, vol. 9, 731
- The cloning of interferon and other mistakes. **WEISSMANN.** Interferon. 1981, vol. 3
- **SHIMATAKE et al.** *Nature,* 1981, vol. 292, 128
- **AMANN et al.** *Gene,* 1983, vol. 25, 167
- **DE BOER et al.** *Proc. Natl. Acad. Sci.,* 1983, vol. 80, 21
- **STUDIER et al.** *J. Mol. Biol.,* 1986, vol. 189, 113
- **TABOR et al.** *Proc Natl. Acad. Sci.,* 1985, vol. 82, 1074
- **SHINE et al.** *Nature,* 1975, vol. 254, 34
- Genetic signals and nucleotide sequences in messenger RNA. **STEITZ et al.** Biological Regulation and Development: Gene Expression. 1979
- Expression of cloned genes in Escherichia coli. **SAMBROOK et al.** Molecular Cloning: A Laboratory Manual. 1989
- **NAGAI et al.** *Nature,* 1984, vol. 309, 810

- **JIA et al.** *Gene,* 1987, vol. 60, 197
- **ALLEN et al.** *J. Biotechnol.,* 1987, vol. 5, 93
- **MAKOFF et al.** *J. Gen. Microbiol.,* 1989, vol. 135, 11
- **MILLER et al.** *Bio/Technology,* 1989, vol. 7, 698
- **MASUI et al.** *Experimental Manipulation of Gene Expression,* 1983
- **GHRAYEB et al.** *EMBO J,* 1984, vol. 3, 2437
- **OKA et al.** *Proc. Natl. Acad. Sci.,* 1985, vol. 82, 7212
- **PALVA et al.** *Proc. Natl. Acad. Sci. USA,* 1982, vol. 79, 5582
- **DAVIES et al.** *Annu. Rev. Microbiol.,* 1978, vol. 32, 469
- **AMANN et al.** *Gene,* 1985, vol. 40, 183
- **STUDIER et al.** *J Mol. Biol.,* 1986, vol. 189, 113
- **POWELL et al.** *Appl. Environ. Microbiol.,* 1988, vol. 54, 655
- **MASSON et al.** *FEMS Microbiol. Lett.,* 1989, vol. 60, 273
- **MILLER et al.** *Proc. Natl. Acad. Sci.,* 1988, vol. 85, 856
- **WANG et al.** *J Bacteriol.,* 1990, vol. 172, 949
- **COHEN et al.** *Proc. Natl. Acad. Sci.,* 1973, vol. 69, 2110
- **DOWER et al.** *Nucleic Acids Res.,* 1988, vol. 16, 6127
- An improved method for transformation of Escherichia coli with ColEl-derived plasmids. **KUSHNER.** Genetic Engineering: Proceedings of the International Symposium on Genetic Engineering. 1978
- **MANDEL et al.** *J Mol. Biol.,* 1970, vol. 53, 159
- **TAKETO.** *Biochim. Biophys. Acta,* 1988, vol. 949, 318
- **CHASSY et al.** *FEMS Microbiol. Lett.,* 1987, vol. 44
- **FIEDLER et al.** *Anal. Biochem,* 1988, vol. 170, 38
- **AUGUSTIN et al.** *FEMS Microbiol. Lett.,* 1990, vol. 66, 203
- **BARANY et al.** *J. Bacteriol.,* 1980, vol. 144, 698
- Transformation of Streptococcus lactis by electroporation. **HARLANDER.** Streptococcal Genetics. 1987
- **PERRY et al.** *Infect Immun.,* 1981, vol. 32, 1295
- **SOMKUTI et al.** *Proc. 4th Evr. Cong. Biotechnology,* 1987, vol. 1, 412
- **MYANOHARA et al.** *Proc. Natl. Acad. Sci. USA,* 1983, vol. 80, 1
- **COHEN et al.** *Proc. Natl. Acad. Sci. USA,* 1980, vol. 77, 1078
- **HENIKOFF et al.** *Nature,* 1981, vol. 283, 835
- **HOLLENBERG et al.** *Curr. Topics Microbiol. Immunol.,* 1981, vol. 96, 119
- The Expression of Bacterial Antibiotic Resistance Genes in the Yeast Saccharomyces cerevisiae. **HOLLENBERG et al.** Plasmids of Medical, Environmental and Commercial Importance. 1979
- **MERCERAU-PUIGALON et al.** *Gene,* 1980, vol. 11, 163
- **PANTHIER et al.** *Curr. Genet.,* 1980, vol. 2, 109
- **BOTSTEIN et al.** *Gene,* 1979, vol. 8, 17-24
- **BRAKE et al.** *Proc. Natl. Acad. Sci USA,* 1984, vol. 81, 4642-4646
- **STINCHCOMB et al.** *J. Mol. Biol.,* 1982, vol. 158, 157
- **ORR-WEAVER et al.** *Methods in Enzymol.,* 1983, vol. 101, 228-245
- **RINE et al.** *Proc. Natl. Acad. Sci. USA,* 1983, vol. 80, 6750
- **BUTT et al.** *Microbiol, Rev.,* 1987, vol. 51, 351
- **KURTZ et al.** *Mol. Cell. Biol.,* 1986, vol. 6, 142
- **KUNZE et al.** *J. Basic Microbiol.,* 1985, vol. 25, 141
- **GLEESON et al.** *J. Gen. Microbiol.,* 1986, vol. 132, 3459
- **ROGGENKAMP et al.** *Mol. Gen. Genet.,* 1986, vol. 202, 302
- **DAS et al.** *J. Bacteriol.,* 1984, vol. 158, 1165
- **DE LOUVENCOURT et al.** *J Bacteriol.,* 1983, vol. 154, 737
- **VAN DEN BERG et al.** *Bio/Technology,* 1990, vol. 8, 135
- **CREGG et al.** *Mol. Cell. Biol.,* 1985, vol. 5, 3376
- **HINNEN et al.** *Proc. Natl. Acad. Sci. USA,* 1978, vol. 75, 1929
- **ITO et al.** *J. Bacteriol.,* 1983, vol. 153, 163
- **BEACH ; NURSE.** *Nature,* 1981, vol. 300, 706
- **DAVIDOW et al.** *Curr. Genet.,* 1985, vol. 10, 380471
- **GAILLARDIN et al.** *Curr. Genet.,* 1985, vol. 10, 49
- **DE LOUVENCOURT et al.** *J. Bacteriol.,* 1983, vol. 154, 1165
- **HINNEN et al.** *Proc. Natl. Acad Sci. USA,* 1978, vol. 75, 1929
- **DAVIDOW et al.** *Curr. Genet.,* 1985, vol. 10, 39
- **KOHLER ; MILSTEIN.** *Nature,* 1975, vol. 256, 495-96
- Remington's Pharmaceutical Sciences. Mack Pub. Co, 1991
- Vaccine design: the subunit and adjuvant approach. Plenum Press, 1995

- **ROBINSON ; TORRES.** *Seminars in Immunology,* 1997, vol. 9, 271-283
- **DONNELLY et al.** *Annu Rev Immunol,* 1997, vol. 15, 617-648
- **JOLLY.** *Cancer Gene Therapy,* 1994, vol. 1, 51-64
- **KIMURA.** *Human Gene Therapy,* 1994, vol. 5, 845-852
- **CONNELLY.** *Human Gene Therapy,* 1995, vol. 6, 185-193
- **KAPLITT.** *Nature Genetics,* 1994, vol. 6, 148-153
- **O'NEILL.** *J Virol.,* 1985, vol. 53, 160
- **KELLY.** *J Virol.,* 1983, vol. 45, 291
- RNA Tumor Viruses. Cold Spring Harbor Laboratory, 1985
- **HARTLEY ; ROWE.** *J Virol,* 1976, vol. 19, 19-25
- **VILE.** *Cancer Res,* 1993, vol. 53, 3860-3864
- **VILE.** *Cancer Res,* 1993, vol. 53, 962-967
- **RAM.** *Cancer Res,* 1993, vol. 53, 83-88
- **TAKAMIYA.** *J Neurosci Res,* 1992, vol. 33, 493-503
- **BABA.** *J Neurosurg,* 1993, vol. 79, 729-735
- **MANN.** *Cell,* 1983, vol. 33, 153
- **CANE.** *Proc Natl Acad Sci,* 1984, vol. 81, 6349
- **MILLER.** *Human Gene Therapy,* 1990, vol. 1
- **BERKNER.** *Biotechniques,* 1988, vol. 6, 616
- **ROSENFELD.** *Science,* 1991, vol. 252, 431
- **CURIEL.** *Hum. Gene Ther.,* 1992, vol. 3, 147-154
- **NAHREINI.** *Gene,* 1993, vol. 124, 257-262
- **SAMULSKI.** *J. Virol.,* 1987, vol. 61, 3096
- **SU.** *Human Gene Therapy,* 1996, vol. 7, 463-470
- **GELLER.** *Science,* 1988, vol. 241, 1667-1669
- **FINK.** *Human Gene Therapy,* 1992, vol. 3, 11-19
- **EVANS.** *Nature,* 1989, vol. 339, 385
- **SABIN.** *J. Biol. Standardization,* 1973, vol. 1, 115
- **ARNOLD.** *J Cell Biochem,* 1990, L401
- **FISHER-HOCH.** *Proc Natl Acad Sci,* 1989, vol. 86, 317
- **FLEXNER.** *Ann NY Acad Sci,* 1989, vol. 569, 86
- **FLEXNER.** *vaccine,* 1990, vol. 8, 17
- **MULLIGAN.** *Nature,* 1979, vol. 277, 108
- **MADZAK.** *J Gen Virol,* 1992, vol. 73, 1533
- **ENAMI.** *Proc Natl Acad Sci,* 1990, vol. 87, 3802-3805
- **ENAMI ; PALESE.** *J Virol,* 1991, vol. 65, 2711-2713
- **LUYTJES.** *Cell,* 1989, vol. 59, 110
- **MCMICHAEL.** *NEJ Med,* 1983, vol. 309, 13
- **YAP.** *Nature,* 1978, vol. 273, 238
- *Nature,* 1979, vol. 277, 108
- **BUCHSCHACHER.** *J Virol.,* 1992, vol. 66, 2731
- **HAMRE.** *Proc Soc Exp Biol Med,* 1966, vol. 121, 190
- **CURIEL.** *Hum Gene Ther,* 1992, vol. 3, 147-154
- **WU.** *J Biol Chem,* 1989, vol. 264, 16985-16987
- **PHILIP.** *Mol Cell Biol,* 1994, vol. 14, 2411-2418
- **WOFFENDIN.** *Proc Natl Acad Sci,* 1994, vol. 91, 1581-1585
- **WU ; WU.** *J. Biol. Chem.,* 1987, vol. 262, 4429-4432
- **HUCKED.** *Biochem Pharmacol,* 1990, vol. 40, 253-263
- **PLANK.** *Bioconjugate Chem,* 1992, vol. 3, 533-539
- **WOFFENDIN et al.** *Proc. Natl. Acad. Sci. USA,* 1994, vol. 91 (24), 11581-11585
- **STRYER.** Biochemistry. W.H. Freeman, 1975, 236-240
- **SZOKA.** *Biochem Biophys Acta,* 1980, vol. 600, 1
- **BAYER.** *Biochem Biophys Acta,* 1979, vol. 550, 464
- **RIVNAY.** *Meth Enzymol,* 1987, vol. 149, 119
- **WANG.** *Proc Natl Acad Sci,* 1987, vol. 84, 7851
- **PLANT.** *Anal Biochem,* 1989, vol. 176, 420
- **HUG ; SLEIGHT.** *Biochim. Biophys. Acta,* 1991, vol. 1097, 1-17
- **STRAUBINGER.** *Meth. Enzymol.,* 1983, vol. 101, 512-527
- **FELGNER.** *Proc. Natl. Acad. Sci. USA,* 1987, vol. 84, 7413-7416
- **MALONE.** *Proc. Natl. Acad. Sci. USA,* 1989, vol. 86, 6077-6081
- **DEBS.** *J Biol. Chem.,* 1990, vol. 265, 10189-10192
- **SZOKA.** *Proc. Natl. Acad Sci. USA,* 1978, vol. 75, 4194-4198
- **STRAUBINGER.** *Meth. Immunol.,* 1983, vol. 101, 512-527
- **SZOKA.** *Proc. Natl. Acad. Sci. USA,* 1978, vol. 75, 4194-4198
- **PAPAHADJOPOULOS.** *Biochim. Biophys. Acta,* 1975, vol. 394, 483
- **WILSON.** *Cell,* 1979, vol. 17, 77
- **DEAMER ; BANGHAM.** *Biochim. Biophys. Acta,* 1976, vol. 443, 629
- **OSTRO.** *Biochem. Biophys. Res. Commun.,* 1977, vol. 76, 836
- **FRALEY.** *Proc. Natl. Acad. Sci. USA,* 1979, vol. 76, 3348
- **ENOCH ; STRITTMATTER.** *Proc. Natl. Acad. Sci. USA,* 1979, vol. 76, 145

- **FRALEY.** *J. Biol. Chem.,* 1980, vol. 255, 10431
- **SZOKA ; PAPAHADJOPOULOS.** *Proc. Natl. Acad. Sci. USA,* 1978, vol. 75, 145
- **SCHAEFER-RIDDER.** *Science,* 1982, vol. 215, 166
- **BRESLOW.** *Annu Rev. Biochem,* 1985, vol. 54, 699
- **LAW.** *Adv. Exp Med. Biol.,* 1986, vol. 151, 162
- **CHEN.** *J Biol Chem,* 1986, vol. 261, 12918
- **KANE.** *Proc Natl Acad Sci USA,* 1980, vol. 77, 2465
- **UTERMANN.** *Hum Genet,* 1984, vol. 65, 232
- *Meth. Enzymol.,* 1986, vol. 128
- **PITAS.** *J Biochem.,* 1980, vol. 255, 5454-5460
- **MAHEY.** *J Clin. Invest,* 1979, vol. 64, 743-750
- **ATKINSON.** *Annu Rev Biophys Chem,* 1986, vol. 15, 403
- **RADDING ; 1958.** *Biochim Biophys Acta,* vol. 30, 443
- **MEINKOTH ; WAHL.** *Anal. Biochem.,* 1984, vol. 138, 267-284
- **MATTEUCCI et al.** *J. Am. Chem. Soc.,* 1981, vol. 103, 3185
- **URDEA.** *Proc. Natl. Acad. Sci. USA,* 1983, vol. 80, 7461
- **AGRAWAL ; IYER.** *Curr Opin Biotechnol,* 1995, vol. 6, 12-19
- **AGRAWAL.** *TIBTECH,* 1996, vol. 14, 376-387
- **COREY.** *TIBTECH,* 1997, vol. 15, 224-229
- **BUCHARDT et al.** *TIBTECH,* 1993, vol. 11, 384-386
- **MULLIS et al.** *Meth. Enzymol.,* 1987, vol. 155, 335-350
- **GAO et al.** *J. Immunol.,* 1989, vol. 143, 3007
- **ROBERTS et al.** *AIDS Res Hum Retrovir,* 1996, vol. 12, 593
- **QUAKYI et al.** *Scand J Immunol,* 1992, vol. 11, 9
- **ALTSCHUL et al.** Gapped BLAST and PSI-BLAST: a new generation of protein database search programs. *Nucleic Acids Research,* 1997, vol. 25, 2289-3402
- **ESPOSTI et al.** Critical evaluation of the hydropathy of membrane proteins. *Eur J Biochem,* 1990, vol. 190, 207-219